(19) Europäisches Patentamt / European Patent Office / Office européen des brevets

(11) EP 4 534 106 A1

(12) # EUROPEAN PATENT APPLICATION
published in accordance with Art. 153(4) EPC

(43) Date of publication:
**09.04.2025 Bulletin 2025/15**

(21) Application number: **23816183.0**

(22) Date of filing: **02.06.2023**

(51) International Patent Classification (IPC):
***A61K 47/68*** (2017.01) ***A61K 31/337*** (2006.01)
***A61K 31/357*** (2006.01) ***A61K 31/407*** (2006.01)
***A61K 31/4375*** (2006.01) ***A61K 31/4745*** (2006.01)
***A61K 31/475*** (2006.01) ***A61K 31/513*** (2006.01)
***A61K 31/537*** (2006.01) ***A61K 31/5513*** (2006.01)
***A61K 31/5517*** (2006.01) ***A61K 31/675*** (2006.01)
***A61K 31/7034*** (2006.01) ***A61K 31/7036*** (2006.01)
***A61K 31/706*** (2006.01) ***A61K 45/00*** (2006.01)
***A61P 35/00*** (2006.01)

(52) Cooperative Patent Classification (CPC):
**A61K 31/337; A61K 31/357; A61K 31/407; A61K 31/4375; A61K 31/4745; A61K 31/475; A61K 31/513; A61K 31/537; A61K 31/5513; A61K 31/5517; A61K 31/675; A61K 31/7034; A61K 31/7036; A61K 31/706; A61K 45/00;** (Cont.)

(86) International application number:
**PCT/JP2023/021638**

(87) International publication number:
**WO 2023/234427 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.06.2022 JP 2022091112**
**10.02.2023 JP 2023019436**

(71) Applicant: **UBE Corporation**
**Ube-shi, Yamaguchi 755-8633 (JP)**

(72) Inventors:
- **TSUZAKI, Yasunori**
**Ube-shi, Yamaguchi 755-8633 (JP)**
- **MIZUNO, Gen**
**Ube-shi, Yamaguchi 755-8633 (JP)**
- **KASHIWAGI, Takamasa**
**Ube-shi, Yamaguchi 755-8633 (JP)**
- **TANAKA, Masayuki**
**Ube-shi, Yamaguchi 755-8633 (JP)**
- **SHIMIZU, Hayato**
**Ube-shi, Yamaguchi 755-8633 (JP)**
- **NONOUCHI, Shimpei**
**Ube-shi, Yamaguchi 755-8633 (JP)**
- **MATSUSHITA, Takashi**
**Ube-shi, Yamaguchi 755-8633 (JP)**
- **KIMURA, Tomio**
**Tokyo 108-0075 (JP)**

(74) Representative: **Hoffmann Eitle**
**Patent- und Rechtsanwälte PartmbB**
**Arabellastraße 30**
**81925 München (DE)**

(54) **ANTIBODY-MULTIDRUG CONJUGATE PRECURSOR AND SYNTHETIC INTERMEDIATE THEREOF**

(57) Provided is a conjugate precursor for obtaining a more useful antitumor drug by further advancing a conventional antibody-drug conjugate.

An antibody-multidrug conjugate precursor is represented by General Formula (I) below:

EP 4 534 106 A1

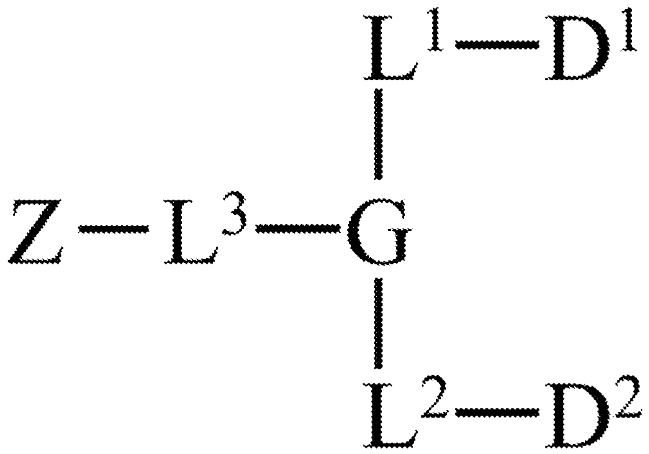

(I)

[in the formula, symbols have the meanings described in the specification], or a salt thereof, and a synthetic intermediate thereof or a salt thereof.

Fig. 1

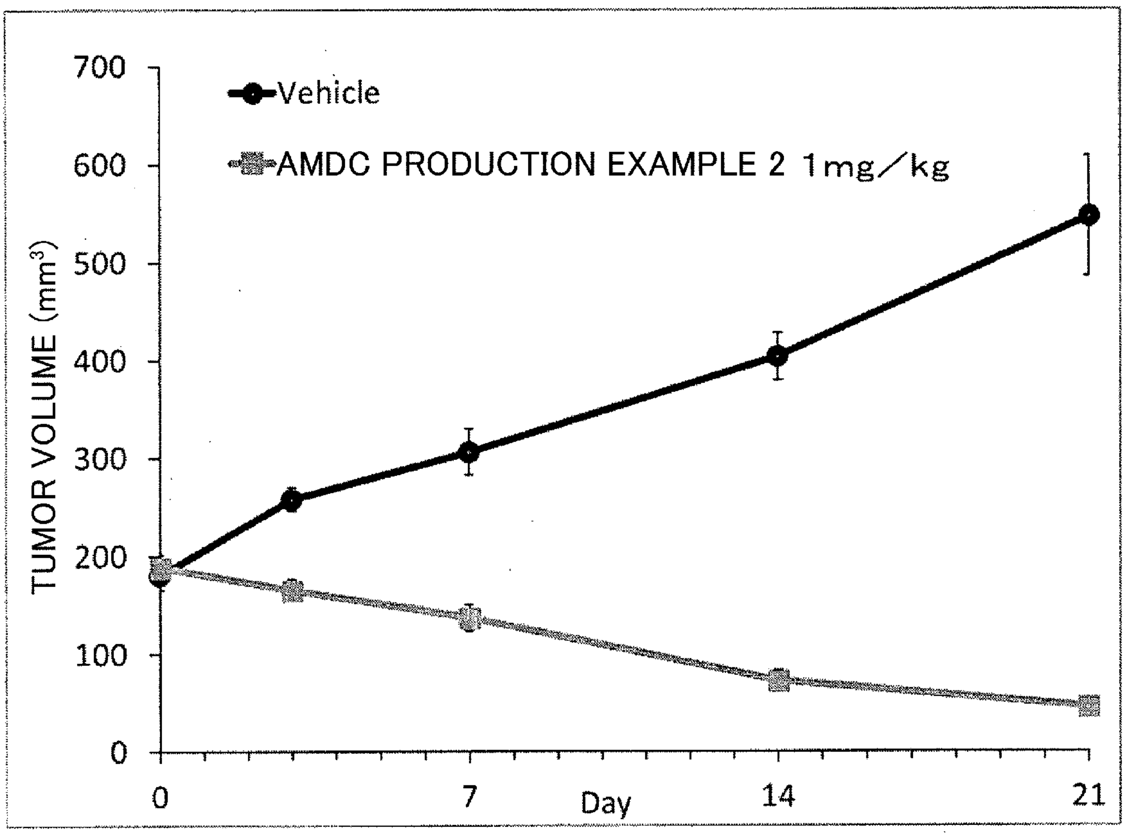


(52) Cooperative Patent Classification (CPC): (Cont.)
**A61K 47/68; A61P 35/00**

## Description

[Technical Field]

The present invention relates to an "antibody-multidrug conjugate (AMDC) precursor (hereinafter also referred to as a conjugate precursor) useful for synthesizing an AMDC used as an antitumor drug, and a synthetic intermediate thereof (hereinafter also referred to as a conjugate precursor synthesis intermediate).

[Background Art]

An antibody-drug conjugate (Antibody Drug Conjugate; ADC) in which a cytotoxic drug (payload) is conjugated to an antibody that binds to an antigen expressed on the surface of a tumor cell and can be internalized in the cell, can be expected to "selectively deliver the drug to the cancer cell, release and accumulate the drug in the cancer cell, and kill the cancer cell" (NPL 1 to 3). As examples of ADC, Adcetris (brentuximab vedotin) (PTL 1) in which monomethyl auristatin E is conjugated to an anti-CD30 antibody is approved as a therapeutic drug for Hodgkin's lymphoma and anaplastic large cell lymphoma, Kadcyla (trastuzumab emtansine) in which emtansine is conjugated to an anti-HER2 antibody is approved as a therapeutic drug for HER2-positive metastatic breast cancer, Enhertu (trastuzumab deruxtecan) in which deruxtecan is conjugated to an anti-HER2 antibody is approved as a therapeutic drug for HER2-positive metastatic breast cancer (PTL 2), Trodelvy (sacituzumab govitecan) in which SN-38 is conjugated to an anti-TROP-2 antibody is approved as a therapeutic drug for TROP-2-positive metastatic breast cancer, Blenrep (belantamab mafodotin) in which MMAF is conjugated to an anti-CD269 (BCMA) antibody is approved as a therapeutic drug for multiple myeloma, Tivdak (tisotumab vedotin) in which monomethyl auristatin E is conjugated to an anti-CD142 antibody is approved as a therapeutic drug for metastatic cervical cancer, and Zynlonta (loncastuximab tesirine) in which pyrrolobenzodiazepine dimer is conjugated to an anti-CD19 antibody is approved as a therapeutic drug for B cell lymphoma (NPL 4 to 10). In addition, many novel ADCs are under clinical development as antitumor drugs (NPL 11 to 14).

Meanwhile, almost all effective cancer chemotherapies are designed to overcome the difference in drug sensitivity within heterogeneous tumor cell populations, and treatment with a combination of multiple drugs is widely performed (NPL 15). This strategy has also been applied to ADCs, and currently, clinical tests are performed using combinations of approved unconjugated anticancer agents and ADCs (NPL 16). Accordingly, it is expected that ADCs in which multiple antitumor drugs having different mechanisms of action are bound to one antibody via a linker will exhibit better efficacy qualitatively and become more useful antitumor drugs. In addition, when multiple antitumor compounds having the same mechanism of action are bound to one antibody via a linker, it is expected that ADCs will exhibit better efficacy strongly and become more useful antitumor drugs.

[Citation List]

[Patent Literature]

[PTL 1] WO 2003/043583
[PTL 2] Japanese Patent Application Publication No. 2016-196484
[PTL 3] WO 2022/022649
[PTL 4] CN 113941007
[PTL 5] WO 2021/148500
[PTL 6] WO 2021/209007
[PTL 7] WO 2021/212638
[PTL 8] WO 2022/199429


[Non Patent Literature]

[NPL 1] Bioconjugate Chem., 2010, 21, 5-13
[NPL 2] Current Opin. Chem. Biol., 2010, 14, 529-537
[NPL 3] Expert Opin. Biol. Ther., 2004, 4, 1445-1452
[NPL 4] Biotechnol Lett., 2016, 38, 1655-1664.
[NPL 5] Chem Pharm Bull., 2019, 67, 173-185.

[NPL 6] N Engl J Med., 2012, 367, 1783-1791
[NPL 7] Expert Opin. Biol. Ther., 2020, 8, 871-875
[NPL 8] Drugs Today, 2021, 57, 653-663
[NPL 9] Drugs, 2021, 81, 2141-2147
[NPL 10] Drugs, 2021, 81, 1229-1233
[NPL 11] Clin Cancer Res., 2019, 25, 5441-5448.
[NPL 12] Molecules. 2020, 25, 4764
[NPL 13] Methods Mol Biol., 2020, 2078, 1-22
[NPL 14] Nat. Rev. Drug Discov., 2017, 16, 315
[NPL 15] Nat.Biotecnol., 2015, 33, 733-736
[NPL 16] N Engl J Med., 2018, 378, 331-344.
[NPL 17] Bioconjugate Chem. 2016, 27, 1030-1039
[NPL 18] Org. Biomol. Chem. 2016, 14, 6165-6178
[NPL 19] Nat. Commun. 2015, 6, 6645
[NPL 20] Angew. Chem. Int. Ed., 2017, 56, 733 -737
[NPL 21] Bioorg. Med. Chem. Lett., 2018, 28, 3617-3621
[NPL 22] Data in Brief, 2018, 21, 2208-2220
[NPL 23] Antib. Ther. 2019 Oct; 2(4): 71-78
[NPL 24] Nat. Commun. 2018, 9, 2512

[Summary of Invention]

[Technical Problem]

The present invention provides an ADC having a linker that is modified with at least one modification group such as a lactonyl group and a phosphoryl group and that is linked to two or more drugs.

[Solution to Problem]

To develop useful antitumor drugs, the present inventors have devised an "antibody-multidrug conjugate (antibody multi-drugs conjugate; AMDC) in which antibodies are bound to multiple antitumor drugs via a branched (for example, two-branched or Y-shaped) linker" and devised a useful conjugate precursor (a precursor compound that reacts with functional groups present in antibodies to form antibody-multidrug conjugates) for obtaining said AMDCs, and successfully synthesized the same, and elucidated a feature in which said AMDCs exhibit excellent pharmacokinetics (especially blood retention). In addition, the present inventors have devised and successfully synthesized a conjugate precursor having one or both of a lactonyl group or a phosphoryl group in said Y-shaped AMDC linker or an antitumor drug as a conjugate precursor for obtaining "highly hydrophilic Y-shaped AMDCs that are stable in blood," and elucidated a feature in which said AMDCs exhibit excellent pharmacokinetics (particularly high stability in blood compared to that of AMDCs without said groups). In addition, the present inventors have found that Y-shaped AMDCs synthesized using said precursor are effective in in vitro and in vivo tests, thereby confirming the usefulness of the conjugated precursor of the present invention. This present invention also includes a "conjugate precursor synthesis intermediate" useful for synthesizing said conjugate precursor.

AMDCs in which multiple drugs are bound to one antibody via a linker are disclosed in PTL 3 to 8 and NPL 17 to 24, and some of the documents disclose a Y-shaped linker, but none of the documents discloses both a lactonyl group and a phosphoryl group in the linker or the antitumor drug.

The present invention provides the following [1] to [35].

[1] An antibody-multidrug conjugate precursor (hereinafter also referred to as a conjugate precursor (I)) represented by General formula (I) below:

[C1]

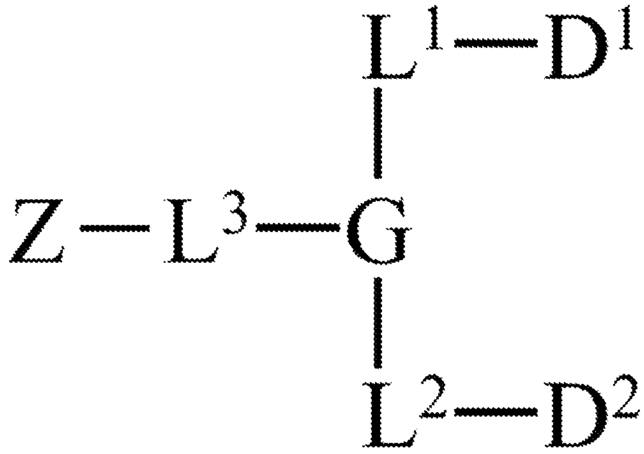

(I)

[in the formula,

Z is a reactive group capable of reacting with a functional group present in an antibody (hereinafter also referred to as a "functional group in an antibody") (hereinafter also simply referred to as a "reactive group"),
G is a group represented by Formula (i), (ii), (iii), or (iv) below,

[C2]

(i) ; 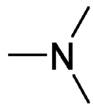

[C3]

(ii) ; 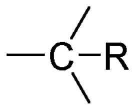

[C4]

(iii) ; 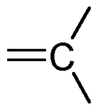

[C5]

(iv) ; 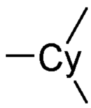

[in the formulae, R is a hydrogen atom, a hydroxyl group, an amino group, an alkyl group, or an alkyloxy group, and Cy is a cycloalkyl ring, a cycloalkenyl ring, an aryl ring, a heteroaryl ring, or a heterocyclyl ring],
$L^1$ is a linker linking G to $D^1$,
$L^2$ is a linker linking G to $D^2$,
$L^3$ is a linker linking G to Z,

$L^1$, $L^2$, and $L^3$ may be identical to or different from each other (hereinafter, $L^1$, $L^2$, and $L^3$ are also collectively referred to as "linker moieties"),

$D^1$ and $D^2$ are residues in which one hydrogen atom or one hydroxyl group is removed from any position of an antitumor drug molecule or an analog thereof (hereinafter also referred to as "antitumor drug residues"), or a derivative thereof, and which may be identical to or different from each other,

one or more lactonyl groups and one or more phosphoryls are independently present as substituents or protecting groups at any position of the antitumor drug residues or linker moieties, a total number of said lactonyl groups is 1 to 10, the lactonyl groups may be identical to or different from each other when said number of the lactonyl groups is two or more, and a total number of said phosphoryl groups is 1 to 10], or a salt thereof.

[2] The conjugate precursor (I) according to [1], or a salt thereof, in which Z is a maleimidyl group (Formula (v) below), an $\alpha$-halogenomethylcarbonyl group (Formula (vi) below), an ethynylphosphonamidate group (Formula (vii) below), a carboxy group, an active ester of a carboxy group, a sulfhydryl group, a hydroxyl group, an amino group, an alkynyl group, a cycloalkynyl group, or an azide group ($-N_3$ group),

Formula (v)

[C6]

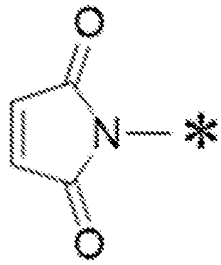

Formula (vi) [C7] Hal-$CH_2$-C(=O)-*

Formula (vii)

[C8]

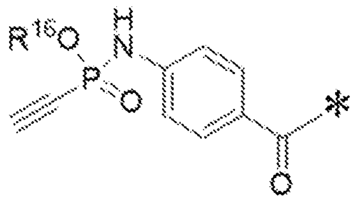

[in the formulae, * is a point of attachment to $L^3$, Hal is a fluorine atom, a chlorine atom, a bromine atom, or an iodine atom, and $R^{16}$ is a methyl group, an ethyl group, or a - $CH_2CH_2OCH_2CH_2OH$ group] .

[3] The conjugate precursor (I) according to [1] or [2], or a salt thereof, in which Z is a maleimidyl group (the Formula (v)), an $\alpha$-halogenomethylcarbonyl group (the Formula (vi)), an ethynylphosphonamidate group (the Formula (vii)), a carboxy group, or an active ester of a carboxy group.

[4] The conjugate precursor (I) according to any one of [1] to [3], or a salt thereof, in which Z is a maleimidyl group (the Formula (v)), an $\alpha$-halogenomethylcarbonyl group (the Formula (vi)), a carboxy group, or an active ester of a carboxy group.

[5] The conjugate precursor (I) according to any one of [1] to [4], or a salt thereof, in which G is a group represented by Formula (i), Formula (ii), or Formula (iii).

[6] The conjugate precursor (I) according to any one of [1] to [5], or a salt thereof, in which G is a group represented by Formula (i).

[7] The antibody multidrug conjugate precursor (I) according to any one of [1] to [6], or a salt thereof, wherein

$L^1$, $L^2$, and $L^3$ are each independently an optionally substituted alkylene group;

one or more methylene group(s) in the chain of said alkylene group is/are optionally replaced with one or more divalent group(s) independently selected from the group consisting of -C($R^1$)($R^2$)-; -O-; -N($R^3$)-; -N($R^3$)-N($R^3$)-; -S-; - Se-; -Si($R^4$)($R^5$)-; -S-S-; -Se-Se-; -SOm-; -SeOn-; - C(=C($R^6$)($R^7$))-; -C(=O)-; -C(=S)-; -C(=N($R^8$))-; -C(=N-OR$^9$)-; - C(=N-N($R^{10}$)($R^{11}$))-; -P(=O)($R^{12}$)-; -P(=O)(OR$^{13}$)-; -O-P(=O)($R^{12}$)-O-; -O-P(=O)(OR$^{13}$)-O-; -C($R^{14}$)=; =C($R^{14}$)-; -C($R^{14}$)=C($R^{14}$)-; - N=; =N-; -C≡C-; -(O-C($R^1$)($R^2$)-C($R^1$)($R^2$))$_{1-30}$-; -(C($R^1$)($R^2$)-C($R^1$)($R^2$)-0)$_{1-30}$-; an optionally substituted alkenylene group, an optionally substituted alkynylene group, an optionally substituted cycloalkylene group; an optionally substituted cycloalkenylene group; an arylene group; an optionally substituted heteroarylene group; and an optionally substituted heterocyclylene group;

$R^1$, $R^2$, $R^3$, $R^4$, $R^5$, $R^6$, $R^7$, $R^8$, $R^9$, $R^{10}$, $R^{11}$, $R^{12}$, $R^{13}$, and $R^{14}$ are each independently a group selected from the group consisting of a hydrogen atom, an optionally substituted alkyl group, an optionally substituted alkenyl group, an optionally substituted alkynyl group, an optionally substituted cycloalkyl group, an optionally substituted cycloalkenyl group, , an aryl group, an optionally substituted heteroaryl group, and an optionally substituted heterocyclyl group,

when $R^3$ is an alkyl group, said alkyl group is optionally combined with an alkyl group in an adjacent methylene group to form a cyclic structure; and

m and n are each independently an integer of 0 to 2.

[8] The antibody multidrug conjugate precursor (I) according to [7], or a salt thereof,

wherein $L^1$, $L^2$, and $L^3$ are each independently an optionally substituted alkylene group; and

one or more methylene groups in the chain of the alkylene group are replaced with one or more divalent groups independently selected from a following formula group: formula group

- C($R^1$)(alkyl) -;
- C($R^{14}$)=;
- O-;
- N($R^3$)-;
- N=;
- N($R^3$)-C($R^1$)($R^2$)- (wherein, when $R^3$, and $R^1$ or $R^2$ are alkyl groups, $R^3$ and $R^1$ or $R^2$ are optionally combined to form a cyclic structure);
- N($R^3$)-C($R^1$)($R^2$)-C(=O)- (wherein, when $R^3$, and $R^1$ or $R^2$ are alkyl groups, $R^3$ and $R^1$ or $R^2$ are optionally combined to form a cyclic structure);
- O-C($R^1$)($R^2$)-:
- S-C($R^1$)($R^2$)-;
- N($R^3$)-N($R^3$)-;
- N($R^3$)-O-C($R^1$)($R^2$)-;
- S-;
- Si($R^4$)($R^3$)-;
- S-S-;
- SOm-;
- C(=O)-;
- C($R^1$)($R^2$)-C(=O)-;
- C(=C($R^6$)($R^7$))-;
- C(=N($R^8$))-;
- C(=N-OR$^9$)-;
- C(=N-N($R^{10}$)($R^{11}$))-;
- P(=O)($R^{12}$)-;
- P (=O)($R^{12}$)-O-;
- O-P(=O)($R^{12}$)-O-;
- P(=O)(OR$^{13}$)-O-;
- P(=O)(OR$^{13}$)-N($R^3$)-;
- O-P(=O)(OR$^{13}$)-O-;
- C($R^{14}$)=C($R^{14}$)-;
- C($R^{14}$)=C($R^{14}$)-P(=O)(OR$^{13}$)-N($R^3$)-;
- C($R^{14}$)=N-;
- C($R^{14}$)=N-N($R^3$)-;
- C=C-;
- O-C($R^1$)($R^2$)-C($R^1$)($R^2$)-;

- O-C($R^1$)($R^2$)-C($R^1$)($R^2$)-O-;
- (O-C($R^1$)($R^2$)-C($R^1$)($R^2$))$_{2-30}$-;
- C(=O)-O-;
- C(=O)-S-;
- C(=O)-N($R^3$)-;
- C(=O)-N($R^3$)-O-;
- C(=O)-N($R^3$)-C($R^1$)($R^2$)- (wherein, when $R^3$, and $R^1$ or $R^2$ are alkyl groups, $R^3$ and $R^1$ or $R^2$ are optionally combined to form a cyclic structure);
- C(=S)-O-;
- C(=S)-S-;
- O-C(=O)-O-;
- O-C(=O)-N($R^3$)-;
- S-C(=O)-N($R^3$)-;
- N($R^3$)-C(=O)-N($R^3$)-;

an optionally substituted arylene group;
an optionally substituted heteroarylene group; and
an optionally substituted heterocyclylene group.

[9] The antibody multidrug conjugate precursor (I) according to [7], or a salt thereof, wherein

$L^1$, $L^2$, and $L^3$ are each independently an optionally substituted alkylene group; and
one or more methylene groups in the chain of the alkylene group are replaced with one or more divalent groups independently selected from a following formula group: formula group

- C(H or $C_1$-$C_4$ alkyl) =;
- O-;
- N(H or $C_1$-$C_4$ alkyl)=-;
- N=;
- S-;
- C(=O)-;
- O-Si($CH_3$)($CH_3$)-O-;
- $CH_2$-C(=O)-;
- C(=O)-NH-;
- C(=O)-O-;
- C(=O)-S-;
- O-C(=O)-O-;
- N(H or H or $C_1$-$C_4$ alky)-C(=O)-O-;
- NH-NH-;
- N(H or $C_1$-$C_4$ alky) -O-$CH_2$-;
- C(=O)-($CH_2$)$_{1-20}$-C(=O)-;
- C(=O)-($CH_2$)$_{1-10}$-O-($CH_2$)$_{1-10}$-C(=O)-;
- C(=O)-$CH_2$-(O-$CH_2CH_3$)$_{1-20}$-O-$CH_2$-C(=O)-;
- (O-$CH_2CH_2$)$_{1-20}$-;
- C(=O)-N(H or H or $C_1$-$C_4$ alky)-$CH_2CH_2$-N(H or H or $C_1$-$C_4$ alky) - C(=O)-;

[C9]

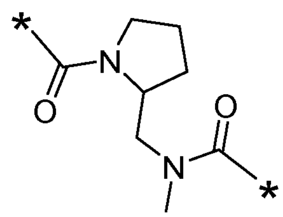

[wherein * is a point of attachment to an adjacent group]

- $C(=O)-N(H$ or $C_1-C_4$ alkyl) $-CH_2-$;
- $P(=O)(OH)-O-$;
- $O-P(=O)(OH)-O-$;
- $O-P(=O)(OH)-O-P(=O)$ $(OH)-O-$;
- $O-P(=O)(OH)-O-P(=O)$ $(OH)-O-P(=O)$ $(OH)-O-$;
- $CH(CH_2-NH_2)-$;
- $CH(CH_2-NH-C(=O))-$;
- $CH(CH_2-NH-C(=O)-(CH_2CH_2-O-)_{1-20}-CH_3)-$;
- $CH(CH_2-NH-C(=O)-(CH_2)_{0-20}$-lactonyl)-;
- $CH(CH_2-NH-C(=O)-(CH_2CH_2-O-)_{1-20}-CH_2$-lactonyl)-;
- $CH(CH_2-NH-C(=O)-(CH_2)_{1-20}$-phosphoryl)-;
- $CH(CH_2-NH-C(=O)-(CH_2CH_2-O-)_{1-20}-CH_2$-phosphoryl)-;
- $CH(CH_2-NH-(CH_2)_{1-20}$-phosphoryl)-;
- (cis) $-CH=CH-P(=O)(O-CH_2CH_3$ or $O-CH_2CH_2-OH)-NH-$ (phenylene) - $C(=O)-$;
- $C(=O)$-(cyclohexylene)-;
- (succinimidylene)-;
- Gly-;
- Ala-;
- Val-;
- Leu-;
- Ile-;
- Phe-;
- Ser-;
- Cys-;
- Asp-;
- Glu-;
- Orn-;
- Lys-;
- Cit-;
- Arg-;
- His-;
- Pro-;
- GlyGly-;
- PheLys-;
- ValLys-;
- ValCit-;
- ValAla-;
- AspValCit-;
- GluValCit-;
- LysValCit-;
- SerValCit-;
- AspValAla-;
- GluValAla-;
- LysValAla-;
- SerValAla-;
- GlyGlyPheGly-;
- AspGlyGlyPheGly-;
- GluGlyGlyPheGly-;
- LysGlyGlyPheGly-;
- SerGlyGlyPheGly-;
- AspAspAspAspAsp-

(in said amino acid residues and the amino acid residues in the peptides,

the carboxy group(s) in the side chain(s) of Asp and Glu may be converted into a lactonyl ester, a lactonylalkyl ester, or a phosphorylalkyl ester or may be converted into an unsubstituted amide, a monoalkyl amide, or a dialkyl amide, and the amino groups in said amide moieties may have lactonyl group(s), lactonylalkyl group(s), or phosphorylalkyl group(s) as substituent(s),

the amino group(s) in the side chain(s) of Lys and Orn may have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s), and
the hydroxyl group in the side chain of Ser and the sulfhydryl group in the side chain of Cys optionally may have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s));

- N(H or $C_1$-$C_4$ alkyl) - (optionally substituted phenylene)-$CH_2$-O-C(=O)-;
- O-(optionally substituted phenylene)-$CH_2$-O-C(=O)-;
- N(H or $C_1$-$C_4$ alkyl)-(optionally substituted pyridylene)-$CH_2$-O-C(=O)-;

[C10]

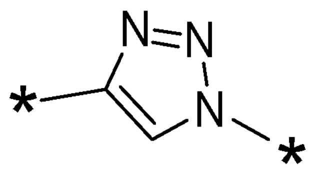

;

[C11]

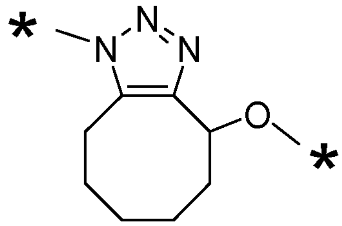

;

[C12]

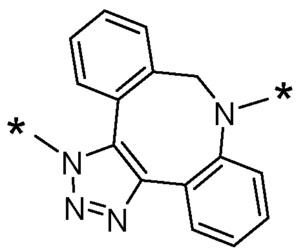

;

[C13]

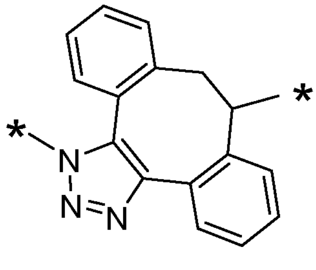

;

and

[C14]

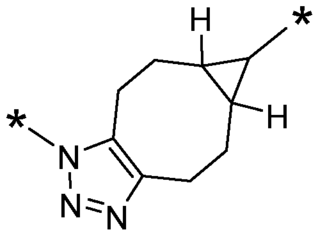

[in the formulae, * is a point of attachment to an adjacent group].

[10] The conjugate precursor (I) according to [7], or a salt thereof, in which $L^1$, $L^2$, and $L^3$ are each independently an optionally substituted alkylene group; and
one or more methylene groups in the chain of said alkylene group are substituted with one or more divalent groups independently selected from a formula group below, formula group

- $CH_2$-N($CH_3$)-$CH_2$-;
- $CH_2$-C(=O)-;
- C(=O)-NH-$CH_2$-;
- C(=O)-$(CH_2)_{1-10}$-(O-$CH_2CH_2$)$_{1-20}$-O-$(CH_2)_{1-10}$-C(=O)-;
- C(=O)-$(CH_2)_{1-10}$-(O-$CH_2CH_2$)$_{1-20}$-O-$(CH_2)_{1-10}$-$CH_2$-NH-;
- NH-$CH_2$-$(CH_2)_{1-10}$-(O-$CH_2CH_3$)$_{1-20}$-O-$(CH_2)_{1-10}$-$CH_2$-NH-;
- C(=O)-N($CH_3$)-$CH_2CH_2$-N($CH_3$)-C(=O)-;

[C15]

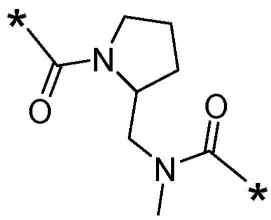

[in the formulae, * is a point of attachment to an adjacent group] ;

- P(=O)(OH)-O-;
- O-P(=O)(OH)-O-;
- O-P(=O)(OH)-O-P(=O) (OH)-O-;
- O-P(=O)(OH)-O-P(=O) (OH)-O-P(=O) (OH)-O-;
- CH($CH_2$-$NH_2$)-;
- CH($CH_2$-NH($CH_3$))-;
- CH($CH_2$-N$(CH_3)_2$)-;
- CH($CH_2$-NH-C(=O)-($CH_2CH_2$-O-)$_{0-20}$-$CH_3$)-;
- CH($CH_2$-NH-C(=O)-$(CH_2)_{0-20}$-lactonyl)-;
- CH($CH_2$-NH-C(=O)-($CH_2CH_2$-O-)$_{1-20}$-$(CH_2)_{1-10}$-$CH_2$-lactonyl)-;
- CH($CH_2$-NH-C(=O)-($CH_2CH_2$-O-)$_{1-20}$-$(CH_2)_{1-10}$-$CH_2$-NH-lactonyl)-;
- CH($CH_2$-NH-C(=O)-($CH_2CH_2$-O-)$_{1-20}$-$(CH_2)_{1-10}$-$CH_2$-NH-C(=O)-lactonyl)-;
- CH($CH_2$-NH-C(=O)-($CH_2CH_2$-O-)$_{1-20}$-$(CH_2)_{1-10}$-C(=O)-NH-lactonyl)-;
- CH($CH_2$-NH-$(CH_2)_{1-20}$-$(O)_{0-1}$-phosphoryl)-;
- CH($CH_2$-NH-C(=O)-$(CH_2)_{1-20}$-phosphoryl)-;
- CH($CH_2$-NH-C(=O)-$(CH_2)_{1-20}$-C(=O)-NH-$(CH_2)_{1-10}$-phosphoryl)-;
- CH($CH_2$-NH-C(=O)-($CH_2CH_2$-O-)$_{1-20}$-$(CH_2)_{1-10}$-$CH_2$-phosphoryl)-;
- CH($CH_2$-NH-C(=O)-($CH_2CH_2$-O-)$_{1-20}$-$(CH_2)_{1-10}$-C(=O)-NH-$(CH_2)_{1-10}$-phosphoryl) -;

- $CH(CH_2-NH-C(=O)-(CH_2CH_2-O-)_{1-20}-(CH_2)_{1-10}-C(=O)-NH-(CH_2)_{1-10}-C(H, OH, Cl, NH_2, \text{or } C_1-C_4 \text{ alkyl})(P(=O)(OH)_2)_2)$-;
- (cis)-CH=CH-P(=O)(O-$CH_2CH_3$)-NH-(phenylene)-C(=O)-;
- C(=O)-(cyclohexylene)-;
- (succinimidylene)-;
- Ser-;
- Cys-;
- Asp-;
- Glu-;
- Orn-;
- Lys-;
- ValLys-;
- ValCit-;
- ValAla-;
- GlyGly-C(=O)-NH-(optionally substituted phenylene)-$CH_2$-;
- PheLys-C(=O)-NH-(optionally substituted phenylene)-$CH_2$-;
- ValLys-C(=O)-NH-(optionally substituted phenylene)-$CH_2$-;
- ValCit-C(=O)-NH-(optionally substituted phenylene)-$CH_2$-;
- ValAla-C(=O)-NH-(optionally substituted phenylene)-$CH_2$-;
- AspValCit-C(=O)-NH-(optionally substituted phenylene)-$CH_2$-;
- GluValCit-C(=O)-NH-(optionally substituted phenylene)-$CH_2$-;
- LysValCit-C(=O)-NH-(optionally substituted phenylene)-$CH_2$-;
- SerValCit-C(=O)-NH-(optionally substituted phenylene)-$CH_2$-;
- AspValAla-C(=O)-NH-(optionally substituted phenylene)-$CH_2$-;
- GluValAla-C(=O)-NH-(optionally substituted phenylene)-$CH_2$-;
- LysValAla-C(=O)-NH-(optionally substituted phenylene)-$CH_2$-;
- SerValAla-C(=O)-NH-(optionally substituted phenylene)-$CH_2$-;
- GlyGlyPheGly-C(=O)-NH-(optionally substituted phenylene) -$CH_2$-;
- AspGlyGlyPheGly-C(=O)-NH-(optionally substituted phenylene) -$CH_2$-;
- GluGlyGlyPheGly-C(=O)-NH-(optionally substituted phenylene) -$CH_2$-;
- LysGlyGlyPheGly-C(=O)-NH-(optionally substituted phenylene) -$CH_2$-;
- SerGlyGlyPheGly-C(=O)-NH-(optionally substituted phenylene) -$CH_2$-;
- AspAspAspAspAsp-C(=O)-NH-(optionally substituted phenylene) -$CH_2$-;
- GlyGly-C(=O)-NH-(optionally substituted phenylene)-$CH_2$-O-C(=O)-;
- PheLys-C(=O)-NH-(optionally substituted phenylene)-$CH_2$-O-C(=O)-;
- ValLys-C(=O)-NH-(optionally substituted phenylene)-$CH_2$-O-C(=O)-;
- ValCit-C(=O)-NH-(optionally substituted phenylene)-$CH_2$-O-C(=O)-;
- ValAla-C(=O)-NH-(optionally substituted phenylene)-$CH_2$-O-C(=O)-;
- AspValCit-C(=O)-NH-(optionally substituted phenylene)-$CH_2$-O-C(=O)-;
- GluValCit-C(=O)-NH-(optionally substituted phenylene)-$CH_2$-O-C(=O)-;
- LysValCit-C(=O)-NH-(optionally substituted phenylene)-$CH_2$-O-C(=O)-;
- SerValCit-C(=O)-NH-(optionally substituted phenylene)-$CH_2$-O-C(=O)-;
- AspValAla-C(=O)-NH-(optionally substituted phenylene)-$CH_2$-O-C(=O)-;
- GluValAla-C(=O)-NH-(optionally substituted phenylene)-$CH_2$-O-C(=O)-;
- LysValAla-C(=O)-NH-(optionally substituted phenylene)-$CH_2$-O-C(=O)-;
- SerValAla-C(=O)-NH-(optionally substituted phenylene)-$CH_2$-O-C(=O)-;
- GlyGlyPheGly-C(=O)-NH-(optionally substituted phenylene)-$CH_2$-O-C(=O)-;
- AspGlyGlyPheGly-C(=O)-NH-(optionally substituted phenylene)-$CH_2$-O-C(=O)-;
- GluGlyGlyPheGly-C(=O)-NH-(optionally substituted phenylene) -$CH_2$-O-C(=O)-;
- LysGlyGlyPheGly-C(=O)-NH-(optionally substituted phenylene) -$CH_2$-O-C(=O)-;
- SerGlyGlyPheGly-C(=O)-NH-(optionally substituted phenylene)-$CH_2$-O-C(=O)-;
- AspAspAspAspAsp-C(=O)-NH-(optionally substituted phenylene)-$CH_2$-O-C(=O)-;
- GlyGlyPheGly-C(=O)-NH-$CH_2$-;
- AspGlyGlyPheGly-C(=O)-NH-$CH_2$-

(in said amino acid residues and the amino acid residues in the peptides,
the carboxy group(s) in the side chain(s) of Asp and Glu is/are converted into O-(lactonyl) ester, O-(lactonylalkyl) ester, O-(phosphorylalkyl) ester, N-(lactonyl)amide, N-(lactonylalkyl)amide, or N-(phosphorylalkyl)amide,
the amino group(s) in the side chain(s) of Lys and Orn has/have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s), and
the hydroxyl group in the side chain of Ser and the sulfhydryl group in the side chain of Cys optionally have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s));

[C16]

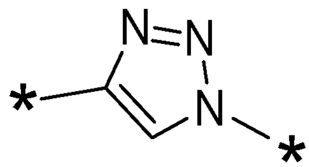

;

[C17]

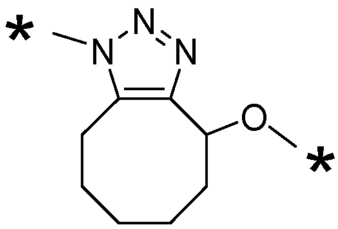

;

[C18]

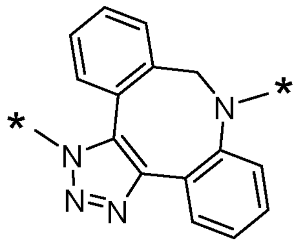

;

[C19]

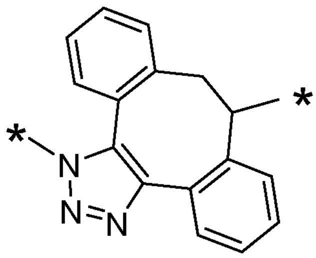

;

and

[C20]

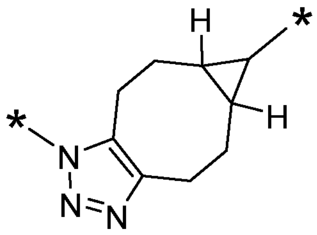

[in the formulae, * is a point of attachment to an adjacent group].

[11] The conjugate precursor (I) according to any one of [1] to [10], or a salt thereof, in which $D^1$ and $D^2$ are residues of antitumor drug molecules independently selected from the group consisting of camptothecin; MMAE; maytansine; PBD (parabenzodiazepine) dimer; eribulin; 5-fluorouracil (5-FU); 5-bromo-N-(2,3-dihydroxypropoxy)-3,4-difluoro-2-[(2-fluoro-4-iodophenyl)amino]-benzamide (PD-318088); pimasertib (AS-703026); 3-[(2R)-2,3-dihydroxypropyl]-6-fluoro-5-(2-fluoro-4-iodoanilino)-8-methylpyrido[2,3-d]pyrimidine-4,7-dione (TAK-733); samotolisib (LY-3023414); calicheamicin; paclitaxel; docetaxel; mitomycin C; bleomycin; cyclocytidine; vincristine; vinblastine; daunomycin; doxorubicin; dolastatin 10; superdox; ciprofloxacin; and cadrofloxacin (CS-940); or analogs or derivatives thereof; or derivatives of said analogs.

[12] The conjugate precursor (I) according to any one of [1] to [11], or a salt thereof, in which $D^1$ and $D^2$ are residues of antitumor drug molecules independently selected from the group consisting of camptothecin; MMAE; maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; and superdox; or analogs or derivatives thereof; or derivatives of said analogs.

[13] The conjugate precursor (I) according to any one of [1] to [12], or a salt thereof, in which $D^1$ and $D^2$ are residues of antitumor drug molecules independently selected from the group consisting of camptothecin; MMAE; maytansine; PBD (parabenzodiazepine) dimer; and eribulin; or analogs or derivatives thereof; or derivatives of said analogs.

[14] The conjugate precursor (I) according to any one of [1] to [13], or a salt thereof, in which $D^1$ and $D^2$ are residues of identical antitumor drugs or analogs or derivatives of the antitumor drugs; or derivatives of said analogs.

[15] The conjugate precursor (I) according to any one of [1] to [13], or a salt thereof, in which $D^1$ and $D^2$ are residues of different antitumor drugs or analogs or derivatives thereof; or derivatives of said analogs.

[16] The conjugate precursor (I) according to any one of [1] to [15], or a salt thereof, in which at least one of $D^1$ and $D^2$ is a residue of an antitumor drug or an analog of the antitumor drug which has a hydroxyl group in the molecule and in which at least one hydroxyl group is phosphorylated; or a derivative of said analog.

[17] The conjugate precursor (I) according to any one of [1] to [16], or a salt thereof, in which one or more lactonyl groups and one or more phosphoryls are independently present as substituents or protecting groups at any position of the antitumor drug residues or the linker moieties, the total number of said lactonyl groups is 1 to 8, the lactonyl groups may be identical to or different from each other when said number of the lactonyl groups is two or more, and the total number of said phosphoryl groups is 1 to 4.

[18] The conjugate precursor (I) according to any one of [1] to [17], or a salt thereof, in which one or more lactonyl groups and one or more phosphoryls are independently present as substituents or protecting groups at any position of the antitumor drug residues or the linker moieties, and

apart from the above, when at least any one of the antitumor drug residues and the linker moieties has one or more groups selected from, as substituents or protecting groups, a phosphorylalkylcarbonyl group ($-C(=O)$-alkylene-$P(=O)(OH)_2$); a dialkylamino group; and a cyclic amino group that forms a ring together with the dialkyl group at any position independently and there are a plurality of said groups in total, the groups may be identical to or different from each other.

[19] A conjugate precursor (I) selected from a group consisting of

Example 1

[C21]

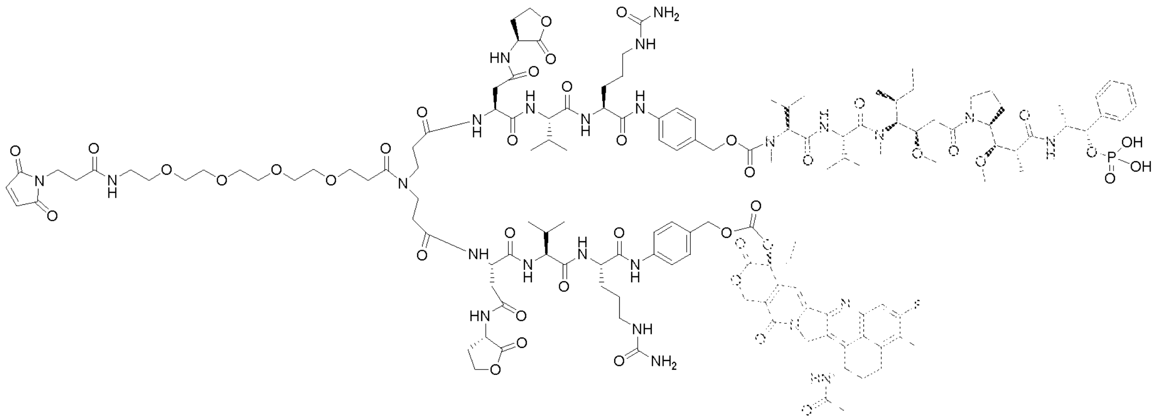

Example 2

[C22]

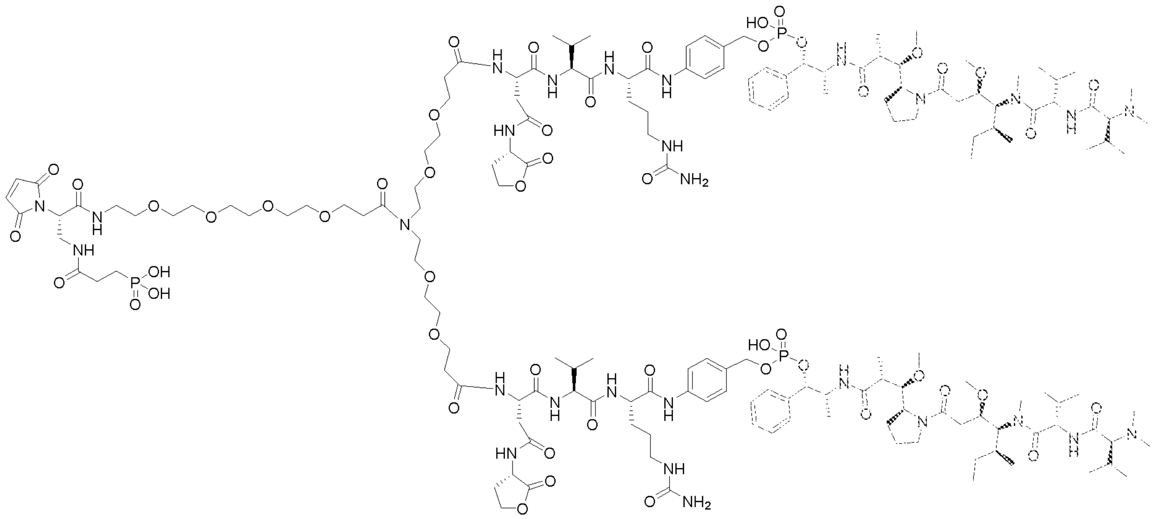

Example 3

[C23]

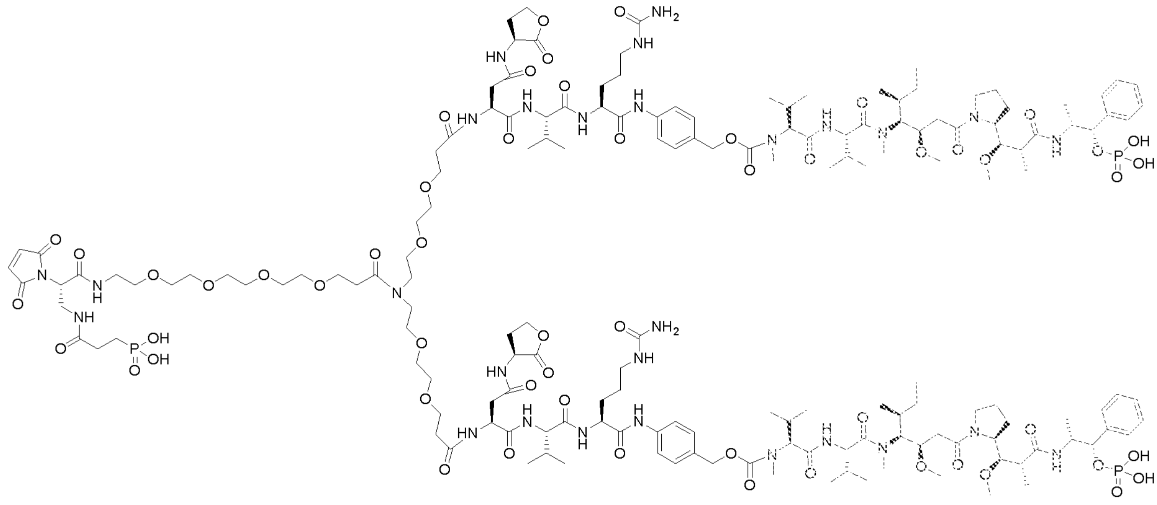

Example 4

[C24]

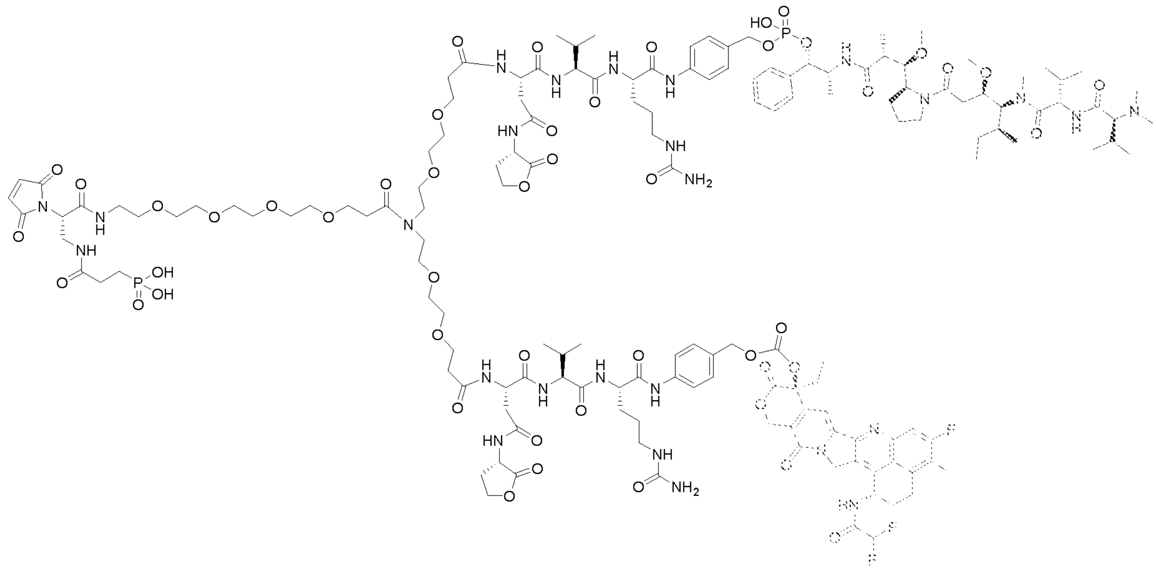

Example 5

[C25]

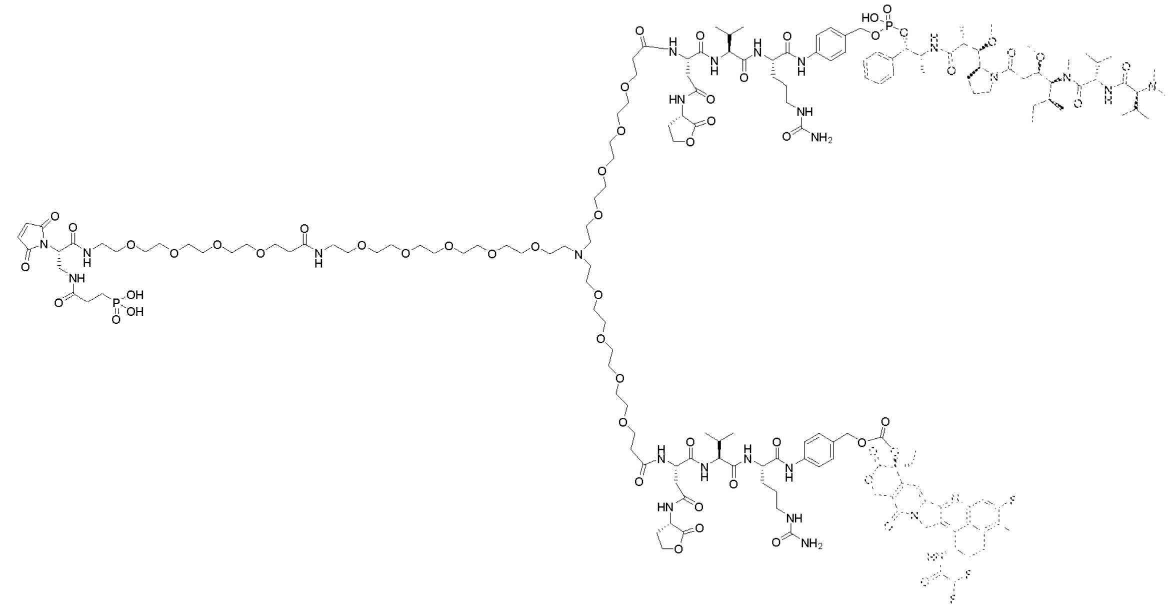

Example 6

[C26]

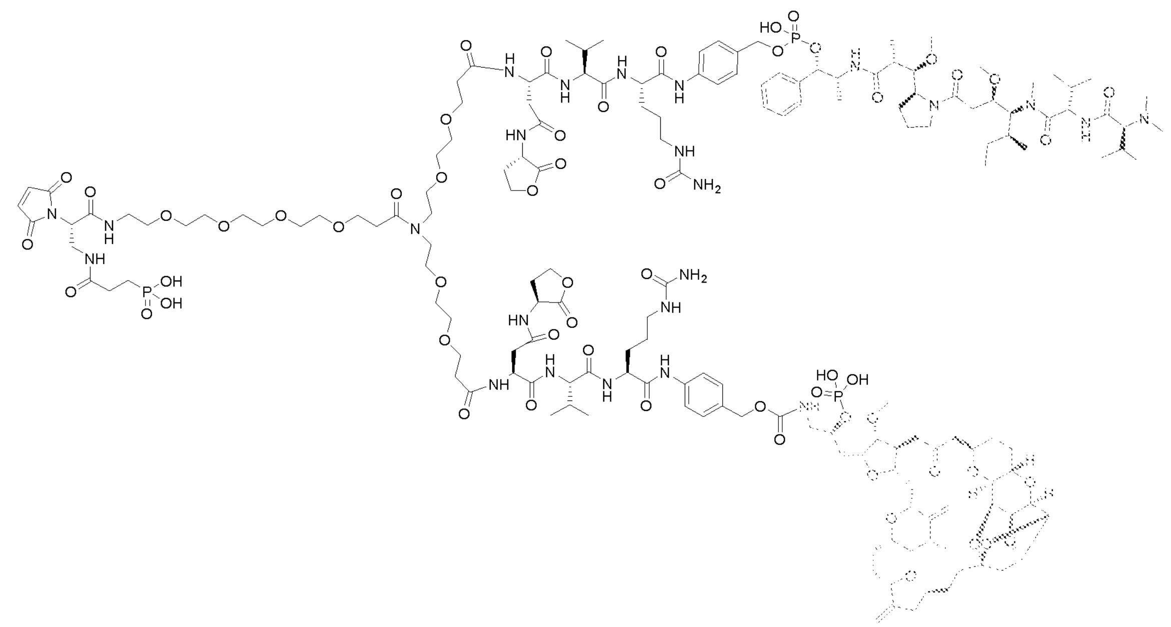

Example 7

[C27]

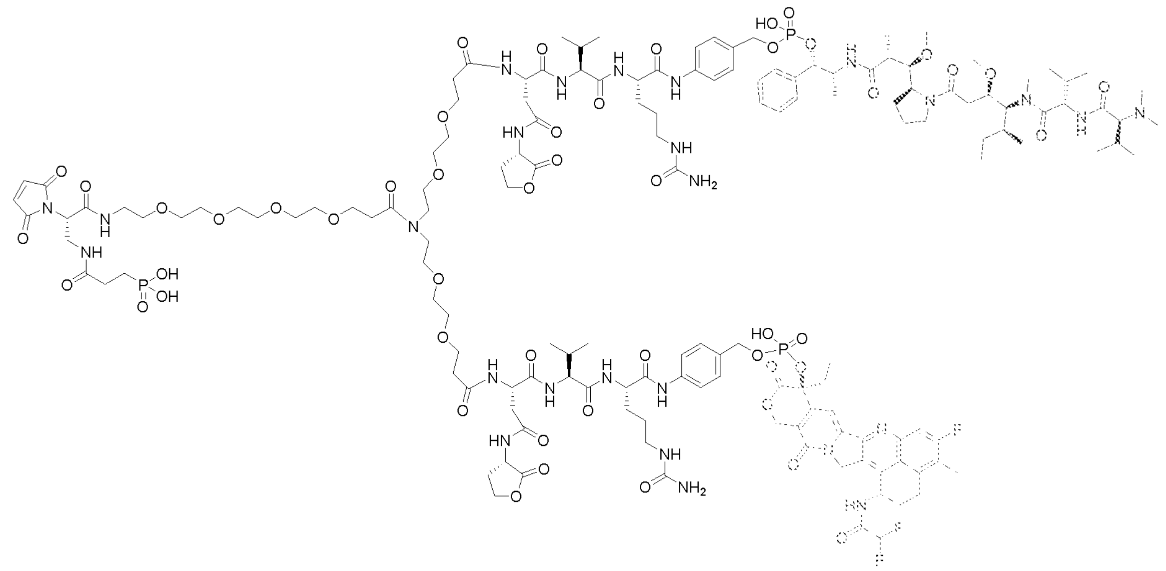

[in the formulae, steric configurations of the amino acid residues in the linkers are all L-shaped], or a salt thereof.

[20] A synthetic intermediate (II-1) of a conjugate precursor (I) represented by General Formula (II-1) below (hereinafter also referred to as conjugate precursor synthesis intermediate (II-1)):

[C28]

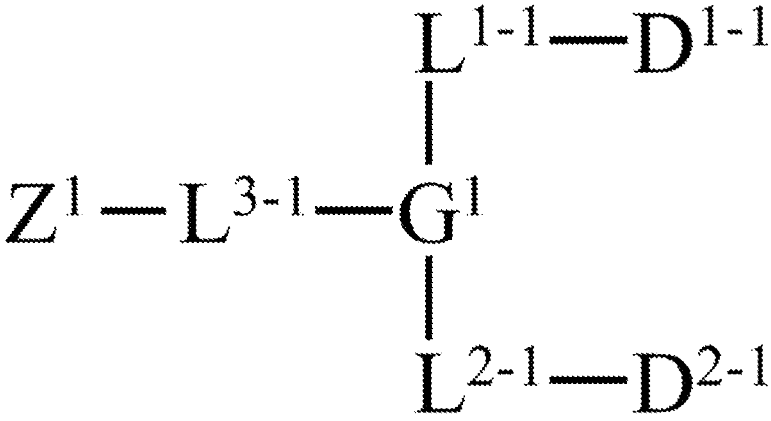

(II-1)

[in the formula,

$Z^1$ is the "reactive group" as defined in [1] above; or a group for bonding said "reactive group" or the antitumor drug residue as defined in [1] above to the linker (hereinafter also simply referred to as a "bonding group"), and said bonding group may be a protective body protected by a protecting group (hereinafter also simply referred to as a "protective body"),
$G^1$ is a group represented by the Formula (i), (ii), (iii), or (iv),
$L^{1-1}$ is a linker linking $G^1$ to $D^{1-1}$,
$L^{2-1}$ is a linker linking $G^1$ to $D^{2-1}$,
$L^{3-1}$ is a linker linking $G^1$ to $Z^1$,
$L^{1-1}$, $L^{2-1}$, and $L^{3-1}$ may be identical to or different from each other (hereinafter, $L^{1-1}$, $L^{2-1}$, and $L^{3-1}$ are also collectively referred to as "linker moieties"),
$D^{1-1}$ and $D^{2-1}$ are antitumor drug residues as defined in [1] above or bonding groups or protective bodies thereof,
$D^{1-1}$ and $D^{2-1}$ may be identical to or different from each other,
when there are one or more groups independently selected from a lactonyl group; a phosphoryl group; a phosphorylalkylcarbonyl group (-C(=O)-alkylene-P(=O)(OH)$_2$); dialkylamino group; and a cyclic amino group that forms a ring together with the dialkyl group at any position(s) of the antitumor drug residues or the linker

moieties and there are a plurality of said groups in total, the groups may be identical to or different from each other, and
when $Z^1$ is a reactive group, at least one of $D^{1-1}$ and $D^{2-1}$ is a bonding group], or a salt thereof.

[21] The conjugate precursor synthesis intermediate (II-1) according to [20], or a salt thereof, in which $G^1$ is a group selected from the formulae according to [5] or [6] above.
[22] The conjugate precursor synthesis intermediate (II-1) according to [20] or [21], or a salt thereof, in which $L^{1-1}$, $L^{2-1}$, and $L^{3-1}$ are each independently an optionally substituted alkylene group; and
one or more methylene groups in the chain of said alkylene group are each independently substituted with one or more groups selected from the divalent groups according to any one of [7] to [10] above.
[23] The conjugate precursor synthesis intermediate (II-1) according to any one of [20] to [22], or a salt thereof, in which $Z^1$ is a bonding group which is a hydroxyl group, a nitro group, a cyano group, an amide group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.
[24] The conjugate precursor synthesis intermediate (II-1) according to any one of [20] to [23], or a salt thereof, in which $Z^1$ is a bonding group which is a hydroxyl group, a nitro group, a cyano group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.
[25] The conjugate precursor synthesis intermediate (II-1) according to any one of [20] to [24], or a salt thereof, in which $Z^1$ is a bonding group which is a hydroxyl group, a nitro group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.
[26] The conjugate precursor synthesis intermediate (II-1) according to any one of [20] to [25], or a salt thereof, in which $Z^1$ is a bonding group which is a hydroxyl group, a nitro group, an oxo group, an azide group, an amino group, a monoalkylamino group which may have a substituent, an imino group (also including -N= and an imino group of a cyclic amine), a carboxy group, a phosphoryl group, an alkynyl group, or a cycloalkynyl group; or a protective body thereof.
[27] The conjugate precursor synthesis intermediate (II-1) according to any one of [20] to [26], or a salt thereof, in which $Z^1$ is a group selected from the bonding groups according to any one of [23] to [26] above, and
one of $D^{1-1}$ and $D^{2-1}$ is a group selected from the antitumor drug residues according to any one of [11] to [16], and the other is a group selected from the bonding groups according to any one of [23] to [26] above.
[28] The conjugate precursor synthesis intermediate (II-1) according to any one of [20] to [26], or a salt thereof, in which $Z^1$ is a group selected from the bonding groups according to any one of [23] to [26] above, and
both of $D^{1-1}$ and $D^{2-1}$ are each independently a group selected from the antitumor drug residues according to any one of [11] to [16] above.
[29] The conjugate precursor synthesis intermediate (II-1) according to any one of [20] to [26], or a salt thereof, in which all of $Z^1$, $D^{1-1}$, and $D^{2-1}$ are each independently a group selected from the bonding groups according to any one of [23] to [26] above.
[30] The conjugate precursor synthesis intermediate (II-1) according to any one of [20] to [22], or a salt thereof, in which $Z^1$ is the reactive group as defined in [1] above, and
one of $D^{1-1}$ and $D^{2-1}$ is a group selected from the antitumor drug residues according to any one of [11] to [16], and the other is a group selected from the bonding groups according to any one of [23] to [26] above.
[31] The conjugate precursor synthesis intermediate (II-1) according to any one of [20] to [22], or a salt thereof, in which $Z^1$ is the reactive group as defined in [1] above, and
both of $D^{1-1}$ and $D^{2-1}$ are each independently a group selected from the bonding groups according to any one of [23] to [26] above.
[32] The conjugate precursor synthesis intermediate (II-1) according to any one of [20] to [31], or a salt thereof, in which one or more lactonyl groups and one or more phosphoryl groups are independently present as substituents or protecting groups at any position of the antitumor drug residues or the linker moieties, a total number of said lactonyl groups is 1 to 8, the lactonyl groups may be identical to or different from each other when said number of the lactonyl groups is two or more, and a total number of said phosphoryl groups is 1 to 4.
[33] The conjugate precursor synthesis intermediate (II-1) according to any one of [20] to [32], or a salt thereof, in which one or more lactonyl groups and one or more phosphoryl groups are independently present as substituents or protecting groups at any position of the antitumor drug residues or the linker moieties, and
apart from the above, when there are one or more groups each independently selected from a phosphorylalkylcarbonyl group (-C(=O)-alkylene-P(=O)$(OH)_2$); dialkylamino group; and a cyclic amino group that forms a ring together with the dialkyl group at any position(s) of the antitumor drug residues or the linker moieties and there are a plurality of said groups in total, the groups may be identical to or different from each other.

[34] A conjugate precursor synthesis intermediate (II-2) represented by General Formula (II-2) below:

[C29]

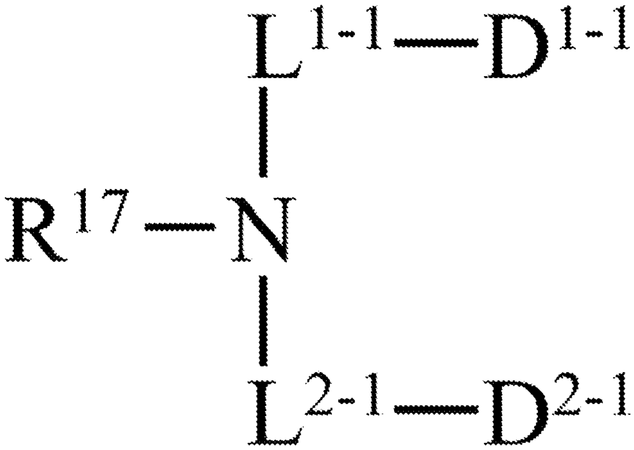

(II-2)

[in the formula,

$L^{1-1}$ and $L^{2-1}$ are each independently an optionally substituted alkylene group, and one or more methylene groups in the chain of said alkylene group are each independently substituted with one or more groups selected from the divalent groups according to any one of [7] to [10],

$D^{1-1}$ and $D^{2-1}$ are groups selected from the antitumor drug residues according to any one of [11] to [16] above or groups selected from the bonding groups according to any one of [23] to [26], and $D^{1-1}$ and $D^{2-1}$ may be identical to or different from each other,

when there are one or more groups independently selected from a lactonyl group; a phosphoryl group; a phosphorylalkylcarbonyl group (-C(=O)-alkylene-P(=O)$(OH)_2$); dialkylamino group; and a cyclic amino group that forms a ring together with the dialkyl group as substituents or protecting groups at any position(s) of said $L^{1-1}$ and $L^{2-1}$ or antitumor drug residues and there are a plurality of said groups in total, the groups may be identical to or different from each other, and

$R^{17}$ is a hydrogen atom or a protecting group], or a salt thereof.

[35] Conjugate precursor synthesis intermediates (II-1) and (II-2) selected from the group consisting of

Example 1-15

[C30]

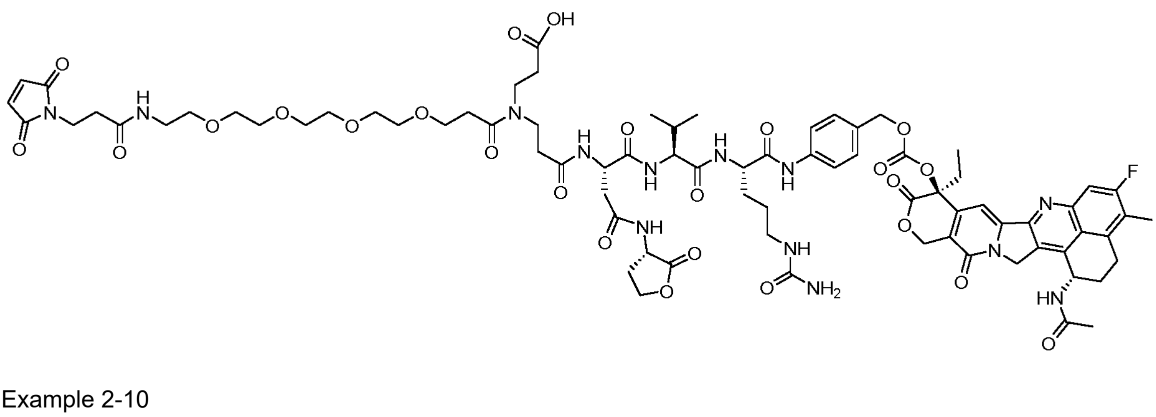

Example 2-10

[C31]

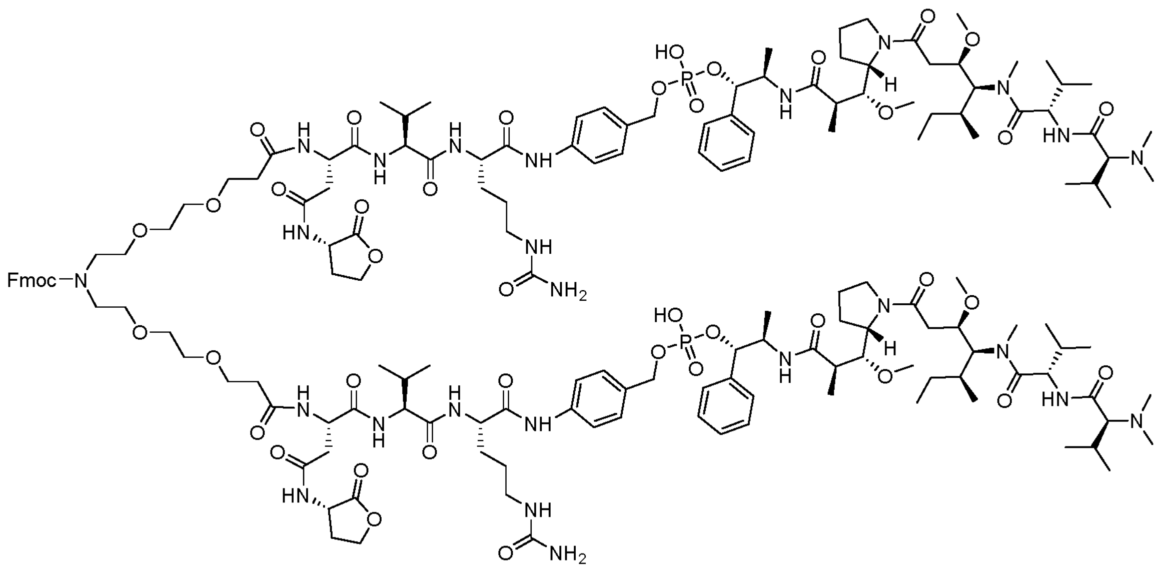

Example 3-1

[C32]

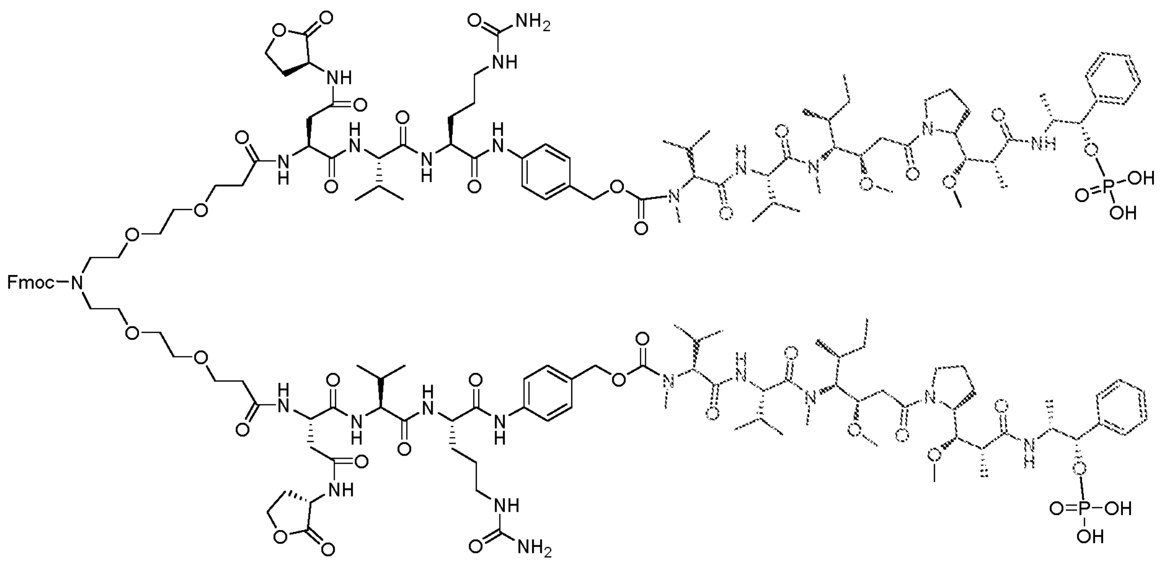

Example 4-3

[C33]

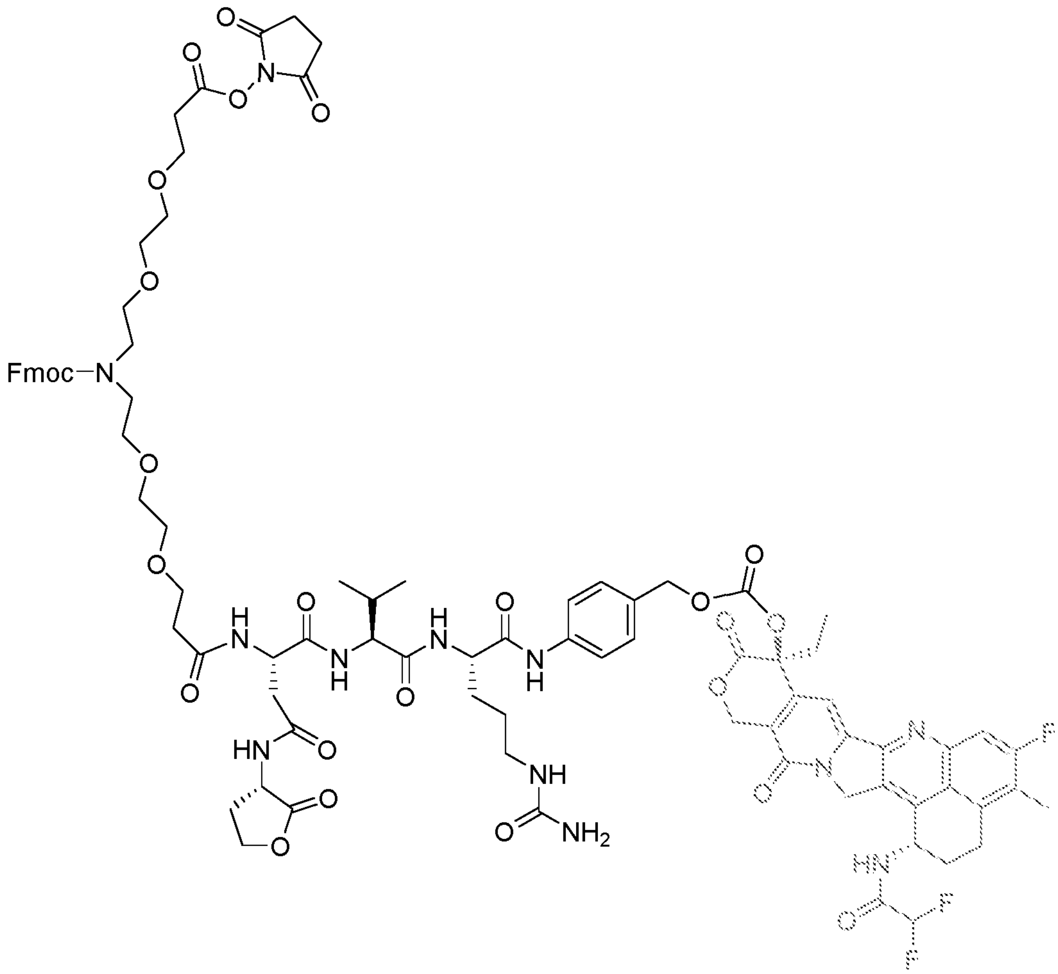

Example 4-4

[C34]

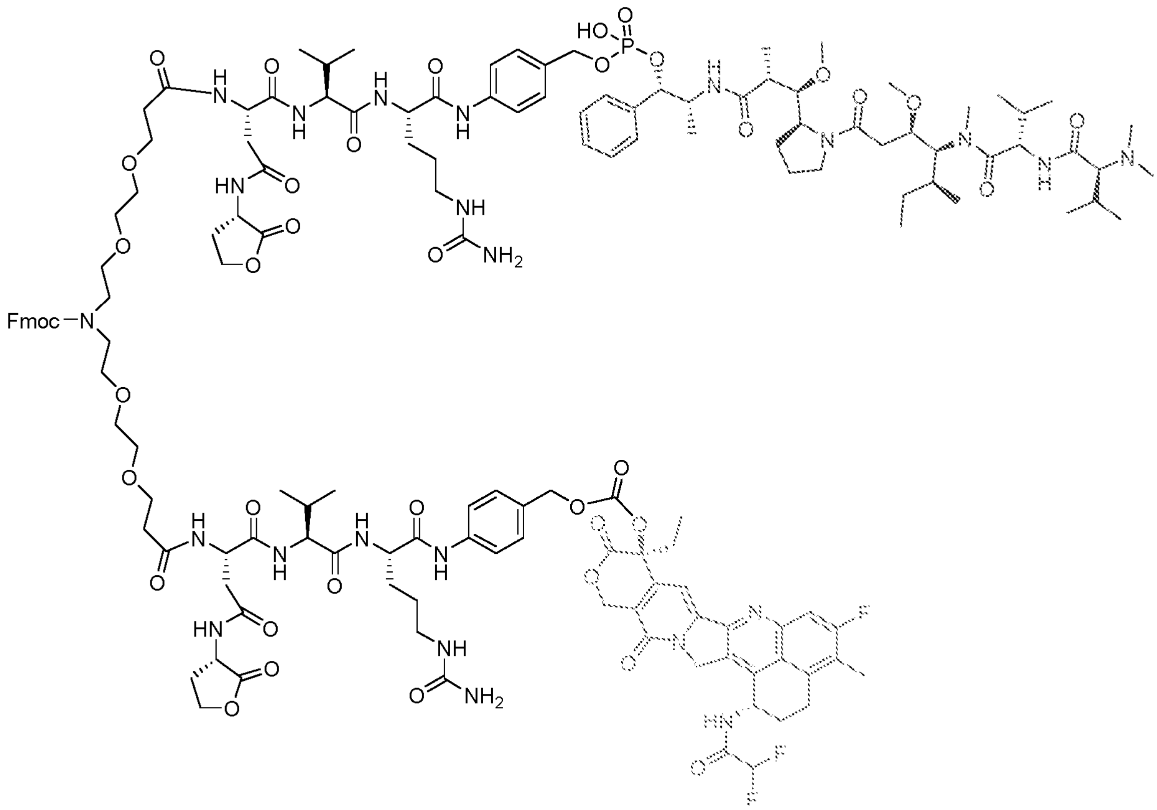

Example 5-2

[C35]

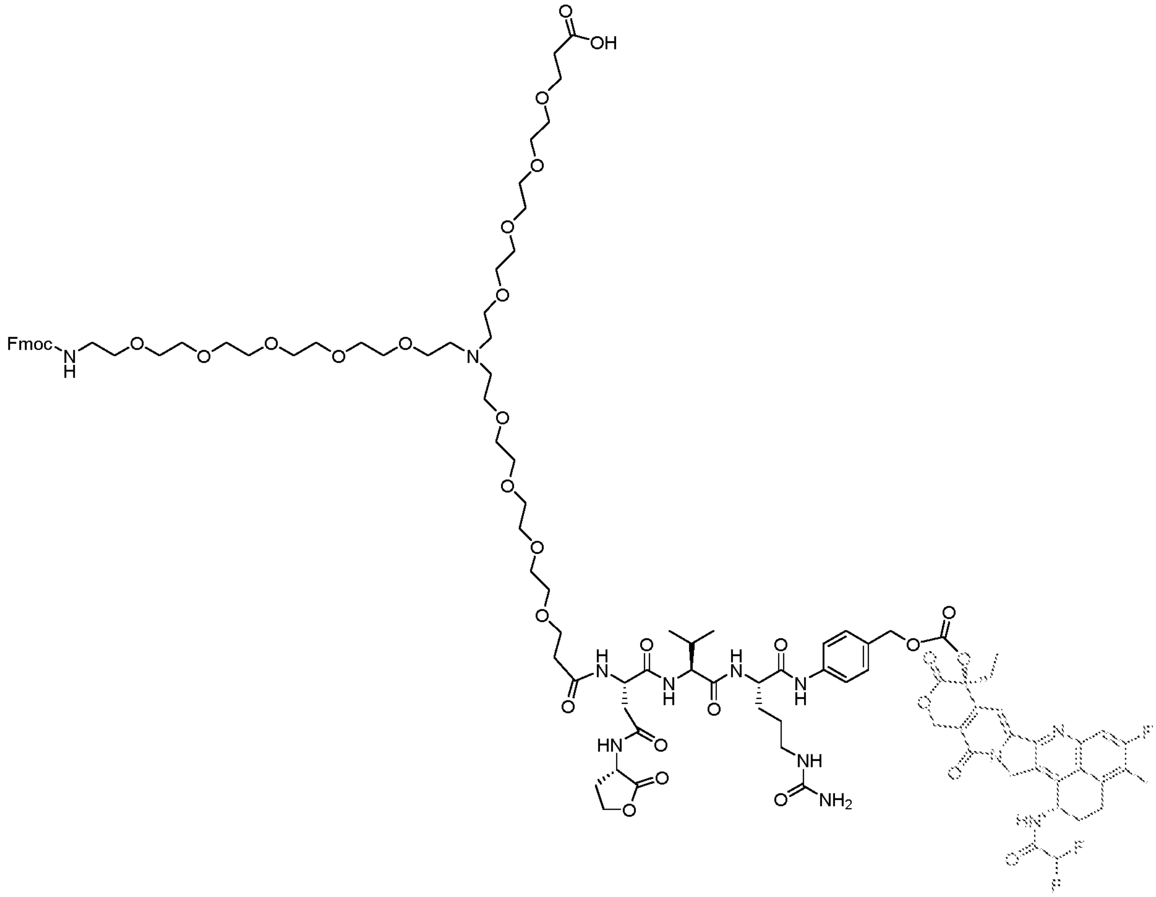

Example 5-3

[C36]

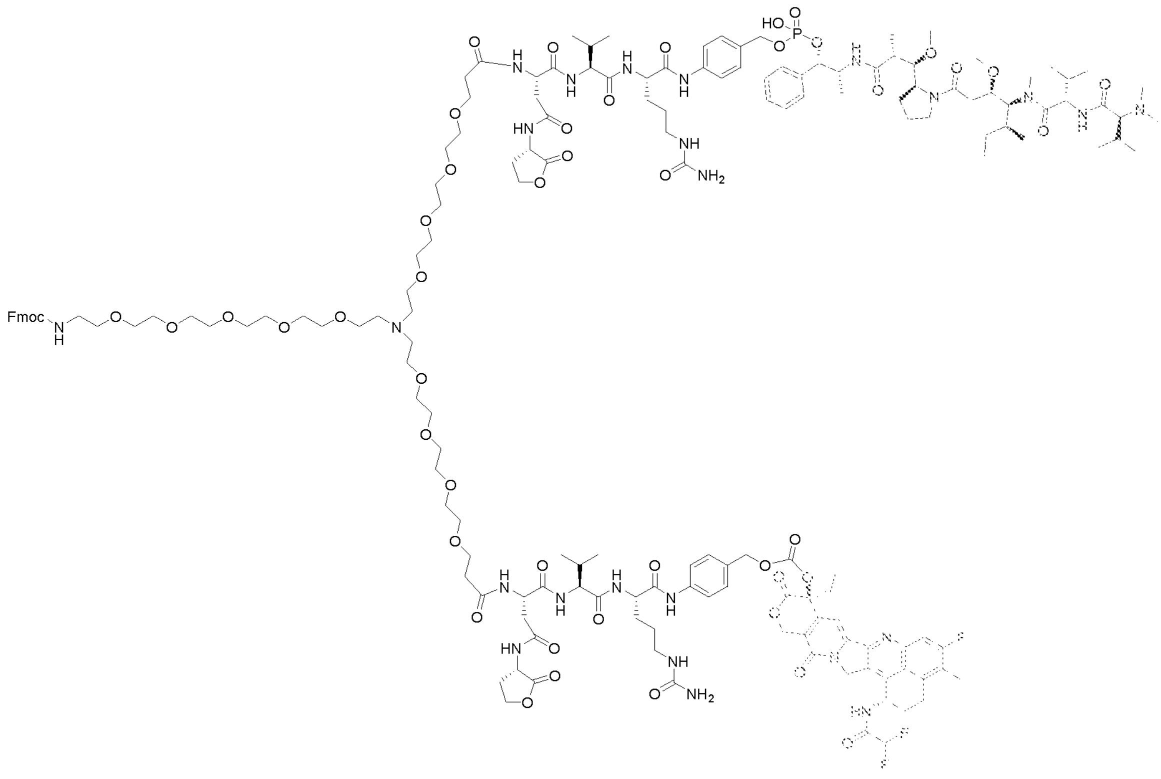

Example 6-4

[C37]

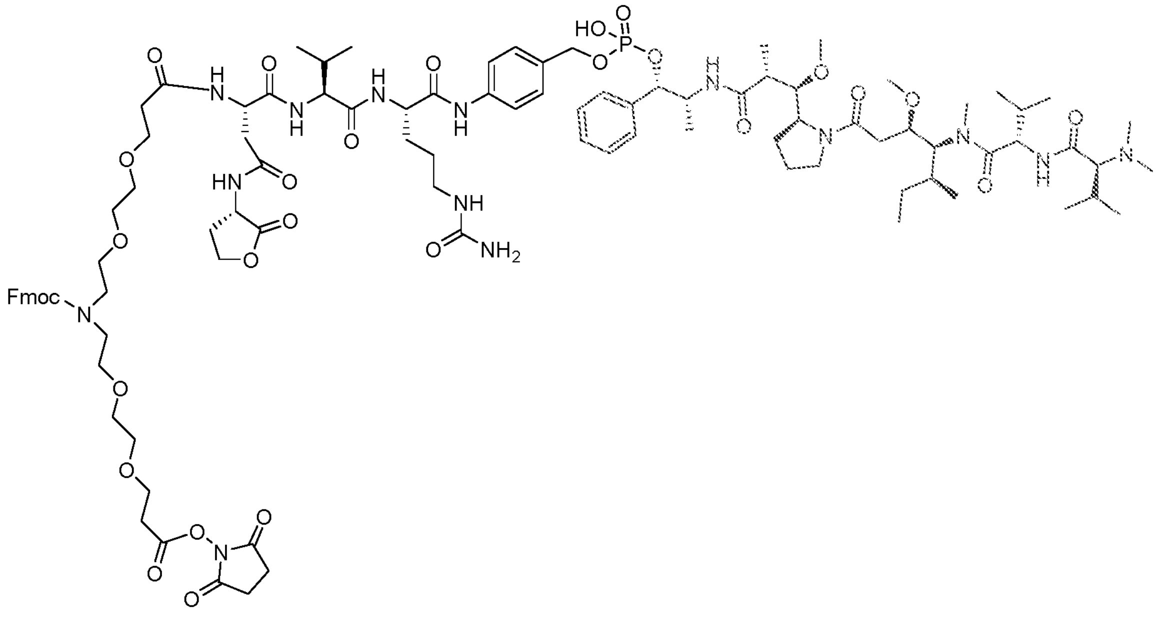

Example 6-5

[C38]

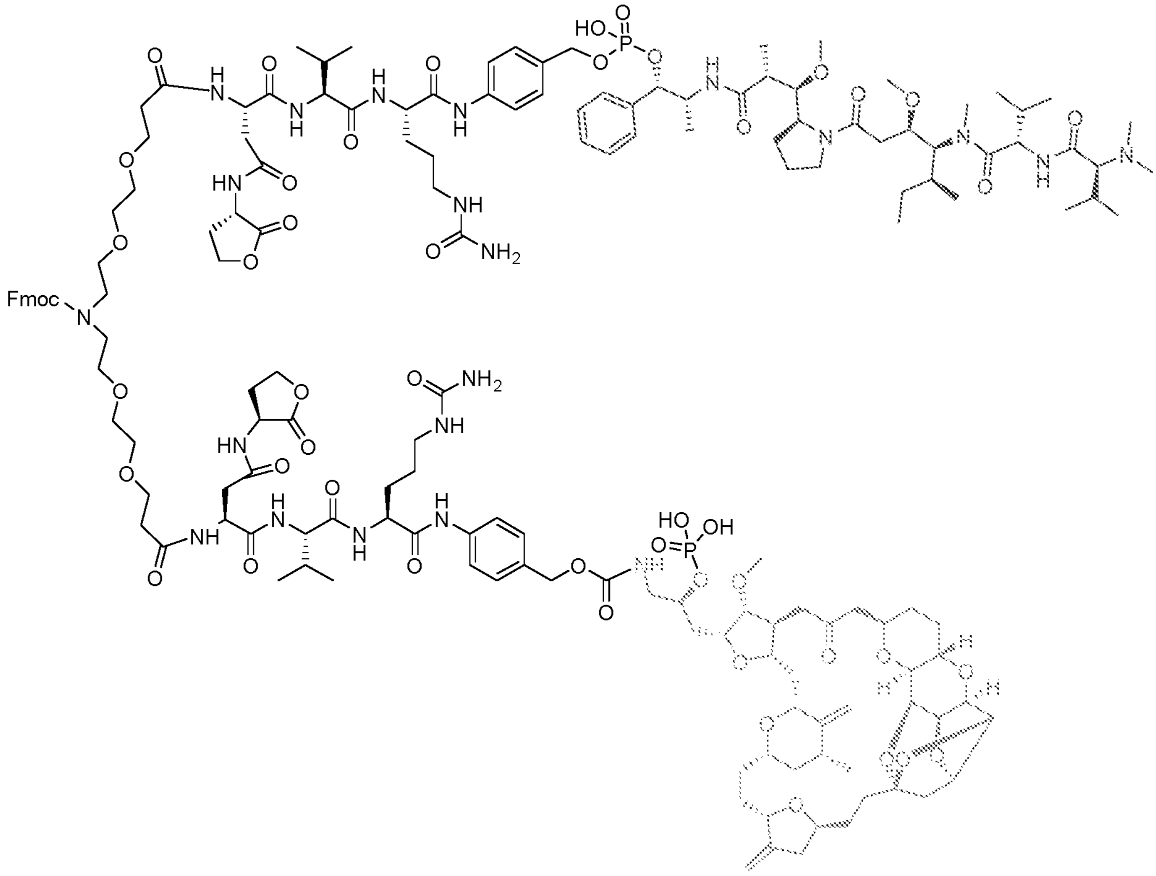

Example 7-6

[C39]

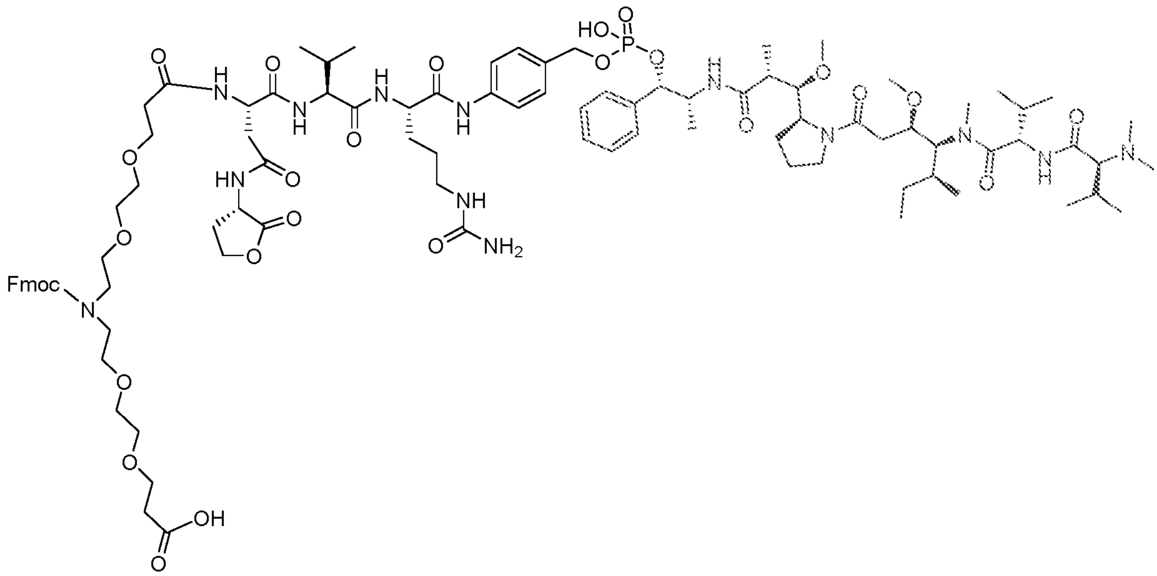

Example 7-7

[C40]

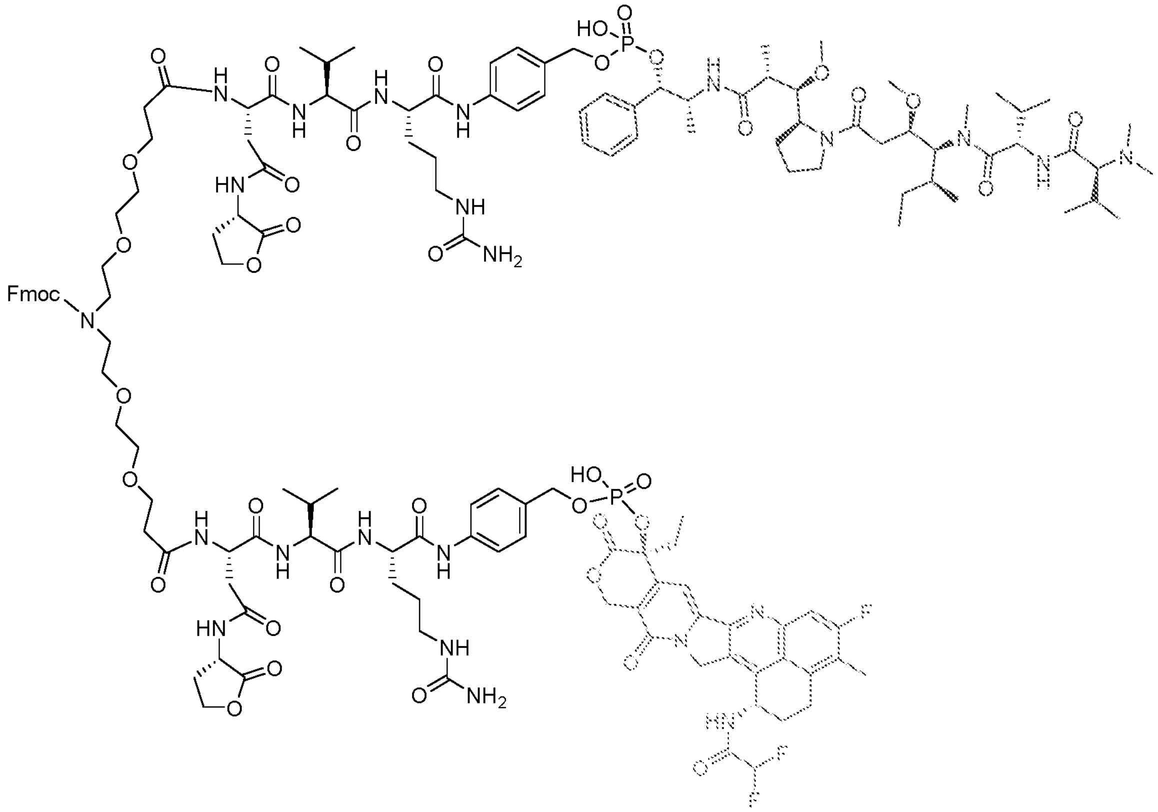

[in the formulae, steric configurations of amino acid residues in linkers are all L-shaped], or a salt thereof].

Hereinafter, aspects of the present invention will be described. Aspects created by optionally selecting and combining the following each aspect and aspects created by optionally combining the following each aspect with any aspect(s), embodiment(s), or the like described herein are also encompassed by the present invention.

[Aspect A1]

The conjugate precursor (I) or a salt thereof, in which, in General Formula (I):

[C41]

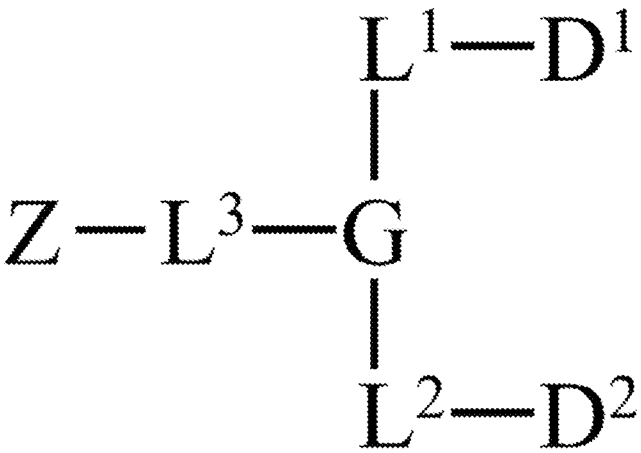

(I)

,

Z is a reactive group capable of reacting with an antibody (hereinafter also simply referred to as a "reactive group").

[Aspect A2]

The conjugate precursor (I) according to [Aspect A1], or a salt thereof, in which Z is a maleimidyl group (Formula (v) below, an α-halogenomethylcarbonyl group (Formula (vi) below), an ethynylphosphonamidate group (Formula (vii) below), a carboxy group, an active ester of a carboxy group, a sulfhydryl group, a hydroxyl group, an amino group, an alkynyl group, a cycloalkynyl group, or an azide group ($-N_3$ group).

[Aspect A3]

The conjugate precursor (I) according to [Aspect A1] or [Aspect A2], or a salt thereof, in which Z is a maleimidyl group (Formula (v) above, an α-halogenomethylcarbonyl group (Formula (vi) above), an ethynylphosphonamidate group (Formula (vii) above), a carboxy group, or an active ester of a carboxy group.

[Aspect A4]

The conjugate precursor (I) according to any one of [Aspect A1] to [Aspect A3], or a salt thereof, in which Z is a maleimidyl group (Formula (v)), an α-halogenomethylcarbonyl group (Formula (vi)), a carboxy group, or an active ester of a carboxy group.

[Aspect B1]

The conjugate precursor (I) according to any one of [Aspect A1] to [Aspect A4], or a salt thereof, in which G is a group represented by Formula (i), Formula (ii), or Formula (iii).

[Aspect B2]

The conjugate precursor (I) according to any one of [Aspect A1] to [Aspect A4] and [Aspect B1], or a salt thereof, in which G is a group represented by Formula (i).

[Aspect C1]

The conjugate precursor (I) according to any one of [Aspect A1] to [Aspect A4], [Aspect B1] and [Aspect B2], or a salt thereof,

wherein $L^1$, $L^2$, and $L^3$ are each independently an optionally substituted alkylene group;
one or more methylene groups in the chain of the alkylene group are optionally replaced with one or more divalent groups independently selected from the group consisting of - $C(R^1)(R^2)$-; -O-; $-N(R^3)-$; $-N(R^3)-N(R^3)-$; -S-; -Se-; $-Si(R^4)(R^5)-$; -S-S-; -Se-Se-; -SOm-; -SeOn-; $-C(=C(R^6)(R^7))-$; -C(=O)-; - C(=S)-; $-C(=N(R^8))-$; $-C(=N-OR^9)-$; $-C(=N-N(R^{10})(R^{11}))-$; - $P(=O)(R^{12})-$; $-P(=O)(OR^{13})-$; $-O-P(=O)(R^{12})-O-$; $-O-P(=O)(OR^{13})-O-$; $-C(R^{14})=$; $=C(R^{14})-$; $-C(R^{14})=C(R^{14})-$; -N=; =N-; -C≡C-; $-(O-C(R^1)(R^2)-C(R^1)(R^2))_{1-30}-$; $-(C(R^1)(R^2)-C(R^1)(R^2)-0)_{1-30}-$; an optionally substituted alkenylene group, an optionally substituted alkynylene group, and an optionally substituted cycloalkylene group; an optionally substituted cycloalkenylene group; an optionally substituted arylene group; an optionally substituted heteroarylene group; and an optionally substituted heterocyclylene group,
$R^1$, $R^2$, $R^3$, $R^4$, $R^5$, $R^6$, $R^7$, $R^8$, $R^9$, $R^{10}$, $R^{11}$, $R^{12}$, $R^{13}$, and $R^{14}$ are each independently a group selected from the group consisting of a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, and an optionally substituted heterocyclyl group, and when $R^3$ is an alkyl group, the alkyl group is optionally combined with an alkyl group in an adjacent methylene group to form a cyclic structure, and
m and n are each independently an integer of 0 to 2.

[Aspect C2]

The conjugate precursor (I) according to [Aspect C1], or a salt thereof, in which $L^1$, $L^2$, and $L^3$ are each independently an optionally substituted alkylene group; and
one or more methylene groups in the chain of said alkylene group are substituted with one or more divalent groups independently selected from a formula group below, formula group

- $C(R^1)(alkyl)$-;
- $C(R^{14})$=;
- O-;
- $N(R^3)$-;
- N=;
- $N(R^3)-C(R^1)(R^2)$- (wherein, when $R^3$, and $R^1$ or $R^2$ are alkyl groups, $R^3$ and $R^1$ or $R^2$ are optionally combined to form a cyclic structure);
- $=N(R^3)-C(R^1)(R^2)-C(=O)$- (wherein, when $R^3$, and $R^1$ or $R^2$ are alkyl groups, $R^3$ and $R^1$ or $R^2$ are optionally combined to form a cyclic structure);
- $O-C(R^1)(R^2)$-:
- $S-C(R^1)(R^2)$-:
- $N(R^3)-N(R^3)$-;
- $N(R^3)-O-C(R^1)(R^2)$-;
- S-;
- $Si(R^4)(R^3)$-;
- S-S-;
- SOm-;
- C(=O)-;
- $C(R^1)(R^2)-C(=O)$-;
- $C (=C(R^6)(R^7)$-;
- $C(=N(R^8))$-;
- $C(=N-OR^9)$-;
- $C(=N-N(R^{10})(R^{11}))$-;
- $P(=O)(R^{12})$-;
- $P(=O)(R^{12})$-O-;
- $O-P(=O)(R^{12})$-O-;
- $P(=O)(OR^{13})$-O-;
- $P(=O)(OR^{13})-N(R^3)$-;
- $O-P(=O)(OR^{13})$-O-;
- $C(R^{14})=C(R^{14})$-;
- $C(R^{14})=C (R^{14})-P(=O)(OR^{13})-N(R^3)$-;
- $C(R^{14})$=N-;
- $C(R^{14})=N-N (R^3)$-;
- C≡C-;
- $O-C(R^1)(R^2)-C(R^1)(R^2)$-;
- $O-C(R^1)(R^2)-C(R^1)(R^2)$-O-;
- $(O-C(R^1)(R^2)-C(R^1)(R^2))_{2-30}$-;
- C(=O)-O-;
- C(=O)-S-;
- $C(=O)-N(R^3)$-;
- C(=O)-N(R3)-O-;
- $C(=O)-N(R^3)-C(R^1)(R^2)$- (wherein, when $R^3$, and $R^1$ or $R^2$ are alkyl groups, $R^3$ and $R^1$ or $R^2$ are optionally combined to form a cyclic structure);
- C(=S)-O-;
- C(=S)-S-;
- O-C(=O)-O-;
- $O-C(=O)-N(R^3)$-;
- $S-C(=O)-N(R^3)$-;
- $N(R^3)-C(=O)-N(R^3)$-;

an optionally substituted arylene group;
an optionally substituted heteroarylene group; and
an optionally substituted heterocyclylene group.

[Aspect C3]

The conjugate precursor (I) according to [Aspect C1], or a salt thereof, in which $L^1$, $L^2$, and $L^3$ are each independently an optionally substituted alkylene group; and

one or more methylene groups in the chain of said alkylene group are substituted with one or more divalent groups independently selected from a formula group below, formula group

-C(H or $C_1$-$C_4$ alkyl)=;
-O-;
N(H or $C_1$-$C_4$ alkyl) -;
-N=;
-S-;
-C(=O)-;
-O-Si($CH_3$)($CH_3$)-O-;
-$CH_2$-C(=O)-;
-C(=O)-NH-;
-C(=O)-O-;
-C(=O)-S-;
-O-C(=O)-O-;
-N(H or $C_1$-$C_4$ alkyl) -C (=O) -O-;
-NH-NH-;
-N(H or $C_1$-$C_4$ alkyl) -O-$CH_2$-;
-C(=O)-($CH_2$)$_{1-20}$-C(=O)-;
-C(=O)-($CH_2$)$_{1-10}$-O-($CH_2$)$_{1-10}$-C(=O)-;
-C(=O)-$CH_2$-(O-$CH_2CH_2$)$_{1-20}$-O-$CH_2$-C(=O)-;
- (O-$CH_2CH_2$)1-20-;
-C(=O)-N(H or $C_1$-$C_4$ alkyl)-$CH_2CH_2$-N(H or H or $C_1$-$C_4$ alkyl) - C(=O)-;

[C42]

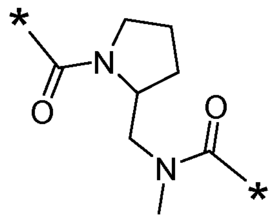

[wherein * is a point of attachment to an adjacent group];
-C(=O)-N(H or H or $C_1$-$C_4$ alkyl)-$CH_2$-;
-P(=O) (OH)-O-;
-O-P(=O) (OH)-O-;
-O-P(=O) (OH)-O-P(=O) (OH)-O-;
-O-P(=O) (OH)-O-P(=O) (OH)-O-P(=O) (OH)-O-;
-CH($CH_2$-$NH_2$) -;
-CH($CH_2$-NH-C(=O)-;
-CH($CH_2$-NH-C(=O)-($CH_2CH_2$-O-)$_{1-20}$-$CH_3$)-;
-CH($CH_2$-NH-C(=O)-($CH_2$)$_{0-20}$-lactonyl)-;
-CH($CH_2$-NH-C(=O)-($CH_2CH_2$-O-)$_{1-20}$-$CH_2$-lactonyl)-;
-CH($CH_2$-NH-C(=O)-($CH_2$)$_{1-20}$-phosphoryl)-;
-CH($CH_2$-NH-C(=O)-($CH_2CH_2$-O-)$_{1-20}$-$CH_2$-phosphoryl)-;
-CH($CH_2$-NH-($CH_2$)$_{1-20}$-phosphoryl)-;
- (cis) -CH=CH-P(=O)(O-$CH_2CH_3$ or O-$CH_2CH_2$-OH) -NH- (phenylene) - C(=O)-;
-C(=O)-(cyclohexylene)-;
-(succinimidylene)-;
-Gly-;
-Ala-;
-Val-;
-Leu-;
-Ile-;
-Phe-;

-Ser-;
-Cys-;
-Asp-;
-Glu-;
-Orn-;
-Lys-;
-Cit-;
-Arg-;
-His-;
-Pro-;
-GlyGly-;
-PheLys-;
-ValLys-;
-ValCit-;
-ValAla-;
-AspValCit-;
-GluValCit-;
-LysValCit-;
-SerValCit-;
-AspValAla-;
-GluValAla-;
-LysValAla-;
-SerValAla-;
-GlyGlyPheGly-;
-AspGlyGlyPheGly-;
-GluGlyGlyPheGly-;
-LysGlyGlyPheGly-;
-SerGlyGlyPheGly-;
-AspAspAspAspAsp-
(in said amino acid residues and the amino acid residues in the peptides,

the carboxy group(s) in the side chain(s) of Asp and Glu may be converted into a lactonyl ester, a lactonylalkyl ester, or a phosphorylalkyl ester or may be converted into an unsubstituted amide, a monoalkyl amide, or a dialkyl amide, and the amino groups in said amide moieties may have lactonyl group(s), lactonylalkyl group(s), or phosphorylalkyl group(s) as substituent(s),
the amino group(s) in the side chain(s) of Lys and Orn may have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s), and
the hydroxyl group in the side chain of Ser and the sulfhydryl group in the side chain of Cys optionally may have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s));

-N(H or $C_1$-$C_4$ alkyl) - (optionally substituted phenylene)-$CH_2$-O-C(=O)-;
-O-(optionally substituted phenylene)-$CH_2$-O-C(=O)-;
-N(H or $C_1$-$C_4$ alkyl) - (optionally substituted pyridylene)-$CH_2$-O-C(=O)-;

[C43]

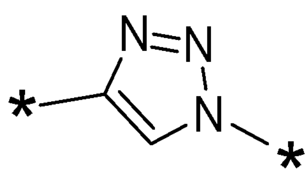

;

[C44]

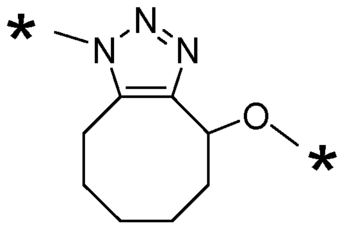

;

[C45]

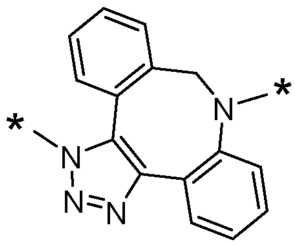

;

[C46]

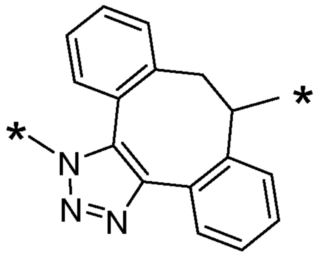

;

and

[C47]

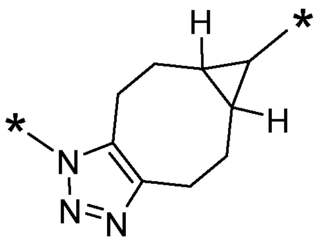

;

[in the formulae, * is a point of attachment to an adjacent group].

[Aspect C4]

The conjugate precursor (I) according to [Aspect C1], or a salt thereof, in which $L^1$, $L^2$, and $L^3$ are each independently an optionally substituted alkylene group; and

one or more methylene groups in the chain of said alkylene group are substituted with one or more divalent groups independently selected from a formula group below, formula group

- $CH_2$-N($CH_3$)-$CH_2$-;
- $CH_2$-C(=O)-;
- C(=O)-NH-$CH_2$-;
- C(=O)-$(CH_2)_{1-10}$-$(O-CH_2CH_2)_{1-20}$-O-$(CH_2)_{1-10}$-C(=O)-;
- C(=O)-$(CH_2)_{1-10}$-$(O-CH_2CH_2)_{1-20}$-O-$(CH_2)_{1-10}$-$CH_2$-NH-;
- NH-$CH_2$-$(CH_2)_{1-10}$-$(O-CH_2CH_2)_{1-20}$-O-$(CH_2)_{1-10}$-$CH_2$-NH-;
- C(=O)-N($CH_3$)-$CH_2CH_2$-N($CH_3$)-C(=O)-;

[C48]

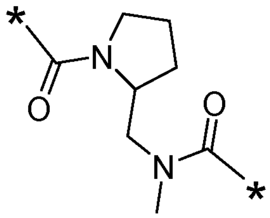

[in the formulae, * is a point of attachment to an adjacent group];

- P(=O)(OH)-O-;
- O-P(=O) (OH)-O-;
- O-P(=O) (OH)-O-P(=O) (OH)-O-;
- O-P(=O) (OH)-O-P(=O) (OH)-O-P(=O) (OH)-O-;
- CH($CH_2$-$NH_2$)-;
- CH($CH_2$-NH($CH_3$))-;
- CH($CH_2$-N$(CH_3)_2$)-;
- CH($CH_2$-NH-C(=O)-$(CH_2CH_2\text{-}O\text{-})_{0-20}$-$CH_3$)-;
- CH($CH_2$-NH-C(=O)-$(CH_2)_{0-20}$-lactonyl)-;
- CH($CH_2$-NH-C(=O)-$(CH_2CH_2\text{-}O\text{-})_{1-20}$-$(CH_2)_{1-10}$-$CH_2$-lactonyl)-;
- CH($CH_2$-NH-C(=O)-$(CH_2CH_2\text{-}O\text{-})_{1-20}$-$(CH_2)_{1-10}$-$CH_2$-NH-lactonyl)-;
- CH($CH_2$-NH-C(=O)-$(CH_2CH_2\text{-}O\text{-})_{1-20}$-$(CH_2)_{1-10}$-$CH_2$-NH-C(=O)-lactonyl)-;
- CH($CH_2$-NH-C(=O)-$(CH_2CH_2\text{-}O\text{-})_{1-20}$-$(CH_2)_{1-10}$-C(=O)-NH-lactonyl)-;
- CH($CH_2$-NH-$(CH_2)_{1-20}$-$(O)_{0-1}$-phosphoryl)-;
- CH($CH_2$-NH-C(=O)-$(CH_2)_{1-20}$-phosphoryl)-;
- CH($CH_2$-NH-C(=O)-$(CH_2)_{1-20}$-C(=O)-NH-$(CH_2)_{1-10}$-phosphoryl)-;
- CH($CH_2$-NH-C(=O)-$(CH_2CH_2\text{-}O\text{-})_{1-20}$-$(CH_2)_{1-10}$-$CH_2$-phosphoryl)-;
- CH($CH_2$-NH-C(=O)-$(CH_2CH_2\text{-}O\text{-})_{1-20}$-$(CH_2)_{1-10}$-C(=O)-NH-$(CH_2)_{1-10}$-phosphoryl) -;
- CH($CH_2$-NH-C(=O)-$(CH_2CH_2\text{-}O\text{-})_{1-20}$-$(CH_2)_{1-10}$-C(=O)-NH-$(CH_2)_{1-10}$-C(H, OH, Cl, $NH_2$, or $C_1$-$C_4$ alkyl)(P(=O)$(OH)_2)_2$)-;
- (cis) -CH=CH-P(=O)(O-$CH_2CH_3$)-NH-(phenylene)-C(=O)-;
- C(=O)-(cyclohexylene)-;
- (succinimidylene)-;
- Ser-;
- Cys-;
- Asp-;
- Glu-;
- Orn-;
- Lys-;
- ValLys-;
- ValCit-;
- ValAla-;
- GlyGly-C(=O)-NH-(optionally substituted phenylene)-$CH_2$-;
- PheLys-C(=O)-NH-(optionally substituted phenylene)-$CH_2$-;
- ValLys-C(=O)-NH-(optionally substituted phenylene)-$CH_2$-;
- ValCit-C(=O)-NH-(optionally substituted phenylene)-$CH_2$-;
- ValAla-C(=O)-NH-(optionally substituted phenylene)-$CH_2$-;

- AspValCit-C(=O)-NH-(optionally substituted phenylene)-$CH_2$-;
- GluValCit-C(=O)-NH-(optionally substituted phenylene)-$CH_2$-;
- LysValCit-C(=O)-NH-(optionally substituted phenylene)-$CH_2$-;
- SerValCit-C(=O)-NH-(optionally substituted phenylene)-$CH_2$-;
- AspValAla-C(=O)-NH-(optionally substituted phenylene)-$CH_2$-;
- GluValAla-C(=O)-NH-(optionally substituted phenylene)-$CH_2$-;
- LysValAla-C(=O)-NH-(optionally substituted phenylene)-$CH_2$-;
- SerValAla-C(=O)-NH-(optionally substituted phenylene)-$CH_2$-;
- GlyGlyPheGly-C(=O)-NH-(optionally substituted phenylene)-$CH_2$-;
- AspGlyGlyPheGly-C(=O)-NH-(optionally substituted phenylene) -$CH_2$-;
- GluGlyGlyPheGly-C(=O)-NH-(optionally substituted phenylene) -$CH_2$-;
- LysGlyGlyPheGly-C(=O)-NH-(optionally substituted phenylene) -$CH_2$-;
- SerGlyGlyPheGly-C(=O)-NH-(optionally substituted phenylene) -$CH_2$-;
- AspAspAspAspAsp-C(=O)-NH-(optionally substituted phenylene) -$CH_2$-;
- GlyGly-C(=O)-NH-(optionally substituted phenylene)-$CH_2$-O-C(=O)-;
- PheLys-C(=O)-NH-(optionally substituted phenylene)-$CH_2$-O-C(=O)-;
- ValLys-C(=O)-NH-(optionally substituted phenylene)-$CH_2$-O-C(=O)-;
- ValCit-C(=O)-NH-(optionally substituted phenylene)-$CH_2$-O-C(=O)-;
- ValAla-C(=O)-NH-(optionally substituted phenylene)-$CH_2$-O-C(=O)-;
- AspValCit-C(=O)-NH-(optionally substituted phenylene)-$CE_2$-O-C(=O)-;
- GluValCit-C(=O)-NH-(optionally substituted phenylene)-$CE_2$-O-C(=O)-;
- LysValCit-C(=O)-NH-(optionally substituted phenylene)-$CE_2$-O-C(=O)-;
- SerValCit-C(=O)-NH-(optionally substituted phenylene)-$CE_2$-O-C(=O)-;
- AspValAla-C(=O)-NH-(optionally substituted phenylene)-$CE_2$-O-C(=O)-;
- GluValAla-C(=O)-NH-(optionally substituted phenylene)-$CE_2$-O-C(=O)-;
- LysValAla-C(=O)-NH-(optionally substituted phenylene)-$CE_2$-O-C(=O)-;
- SerValAla-C(=O)-NH-(optionally substituted phenylene)-$CE_2$-O-C(=O)-;
- GlyGlyPheGly-C(=O)-NH-(optionally substituted phenylene)-$CH_2$-O-C(=O)-;
- AspGlyGlyPheGly-C(=O)-NH-(optionally substituted phenylene)-$CH_2$-O-C(=O)-;
- GluGlyGlyPheGly-C(=O)-NH-(optionally substituted phenylene)-$CH_2$-O-C(=O)-;
- LysGlyGlyPheGly-C(=O)-NH-(optionally substituted phenylene)-$CH_2$-O-C(=O)-;
- SerGlyGlyPheGly-C(=O)-NH-(optionally substituted phenylene)-$CH_2$-O-C(=O)-;
- AspAspAspAspAsp-C(=O)-NH-(optionally substituted phenylene)-$CH_2$-O-C(=O)-;
- GlyGlyPheGly-C(=O)-NH-$CH_2$-;
- AspGlyGlyPheGly-C(=O)-NH-$CH_2$-

(in said amino acid residues and the amino acid residues in the peptides,

the carboxy group(s) in the side chain(s) of Asp and Glu is/are converted into O-(lactonyl) ester, O-(lactonylalkyl) ester, O-(phosphorylalkyl) ester, N-(lactonyl)amide, **N-**(lactonylalkyl)amide, or N-(phosphorylalkyl)amide,
the amino group(s) in the side chain(s) of Lys and Orn has/have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s), and
the hydroxyl group in the side chain of Ser and the sulfhydryl group in the side chain of Cys optionally have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s));

[C49]

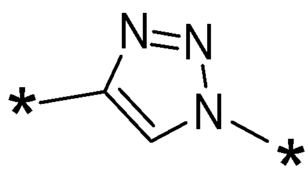

;

[C50]

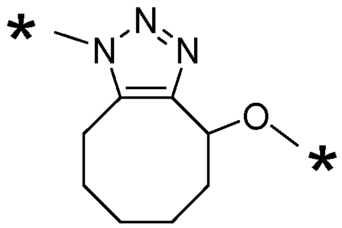

;

[C51]

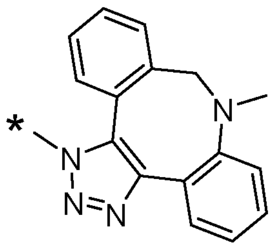

;

[C52]

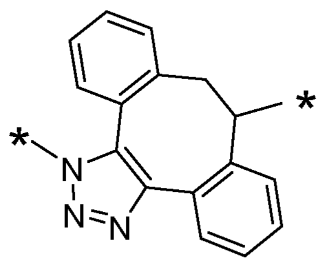

;

and

[C53]

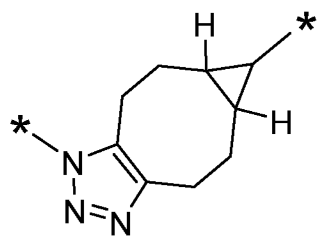

[in the formulae, * is a point of attachment to an adjacent group].

[Aspect D1]

The conjugate precursor (I) according to any one of [Aspect A1] to [Aspect A4], [Aspect B1], [Aspect B2], and [Aspect C1] to [Aspect C4], or a salt thereof, in which $D^1$ and $D^2$ are residues of antitumor drug molecules independently selected from the group consisting of camptothecin; MMAE; maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; ciprofloxacin; and cadrofloxacin

(CS-940); or analogs or derivatives thereof; or derivatives of said analogs.

[Aspect D2]

The conjugate precursor (I) according to any one of [Aspect A1] to [Aspect A4], [Aspect B1], [Aspect B2], [Aspect C1] to [Aspect C4], and [Aspect D1], or a salt thereof, in which $D^1$ and $D^2$ are residues of antitumor drug molecules independently selected from the group consisting of camptothecin; MMAE; maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; and superdox; or analogs or derivatives thereof; or derivatives of said analogs.

[Aspect D3]

The conjugate precursor (I) according to any one of [Aspect A1] to [Aspect A4], [Aspect B1], [Aspect B2], [Aspect C1] to [Aspect C4], [Aspect D1], and [Aspect D2], or a salt thereof, in which $D^1$ and $D^2$ are residues of antitumor drug molecules independently selected from the group consisting of camptothecin; MMAE; maytansine; PBD (parabenzodiazepine) dimer; and eribulin; or analogs or derivatives thereof; or derivatives of said analogs.

[Aspect D4]

The conjugate precursor (I) according to any one of [Aspect A1] to [Aspect A4], [Aspect B1], [Aspect B2], [Aspect C1] to [Aspect C4], and [Aspect D1] to [Aspect D3], or a salt thereof, in which $D^1$ and $D^2$ are residues of identical antitumor drugs or analogs or derivatives of the antitumor drugs; or derivatives of said analogs.

[Aspect D5]

The conjugate precursor (I) according to any one of [Aspect A1] to [Aspect A4], [Aspect B1], [Aspect B2], [Aspect C1] to [Aspect C4], and [Aspect D1] to [Aspect D3], or a salt thereof, in which $D^1$ and $D^2$ are residues of different antitumor drugs or analogs or derivatives thereof; or derivatives of said analogs.

[Aspect D6]

The conjugate precursor (I) according to any one of [Aspect A1] to [Aspect A4], [Aspect B1], [Aspect B2], [Aspect C1] to [Aspect C4], and [Aspect D1] to [Aspect D3], or a salt thereof, in which at least one of $D^1$ and $D^2$ is a residue of an "antitumor drug or an analog of the antitumor drug which has a hydroxyl group in the molecule and in which at least one hydroxyl group is phosphorylated; or a derivative of said analog."

[Aspect E1]

The conjugate precursor (I) according to any one of [Aspect A1] to [Aspect A4], [Aspect B1], [Aspect B2], [Aspect C1] to [Aspect C4], and [Aspect D1] to [Aspect D6], or a salt thereof, in which one or more lactonyl groups and one or more phosphoryl groups are independently present as substituents or protecting groups at any position of the antitumor drug residues or the linker moieties, the total number of said lactonyl groups is 1 to 10, the lactonyl groups may be identical to or different from each other when said number of the lactonyl groups is two or more, and the total number of said phosphoryl groups is 1 to 10.

[Aspect E2]

The conjugate precursor (I) according to any one of [Aspect A1] to [Aspect A4], [Aspect B1], [Aspect B2], [Aspect C1] to [Aspect C4], and [Aspect D1] to [Aspect D6], or a salt thereof, in which one or more lactonyl groups and one or more phosphoryl groups are independently present as substituents or protecting groups at any position of the antitumor drug residues or the linker moieties, the total number of said lactonyl groups is 1 to 8, the lactonyl groups may be identical to or different from each other when said number of the lactonyl groups is two or more, and the total number of said phosphoryl groups is 1 to 4.

[Aspect E3]

The conjugate precursor (I) according to any one of [Aspect A1] to [Aspect A4], [Aspect B1], [Aspect B2], [Aspect

C1] to [Aspect C4], [Aspect D1] to [Aspect D6], [Aspect E1], and [Aspect E2], or a salt thereof, in which one or more lactonyl groups and one or more phosphoryl groups are independently present as substituents or protecting groups at any position of the antitumor drug residues or the linker moieties, and
apart from the above, when there are one or more groups independently selected from a lactonyl group; a phosphoryl group; a phosphorylalkylcarbonyl group (-C(=O)-alkylene-P(=O)(OH)$_2$); dialkylamino group; and a cyclic amino group that forms a ring together with the dialkyl group at any position(s) of the antitumor drug residues or the linker moieties and there are a plurality of said groups in total, they may be identical to or different from each other.

[Aspect F1]

A conjugate precursor synthesis intermediate (II-1) or a salt thereof, in which, in General Formula (II-1) below:

[C54]

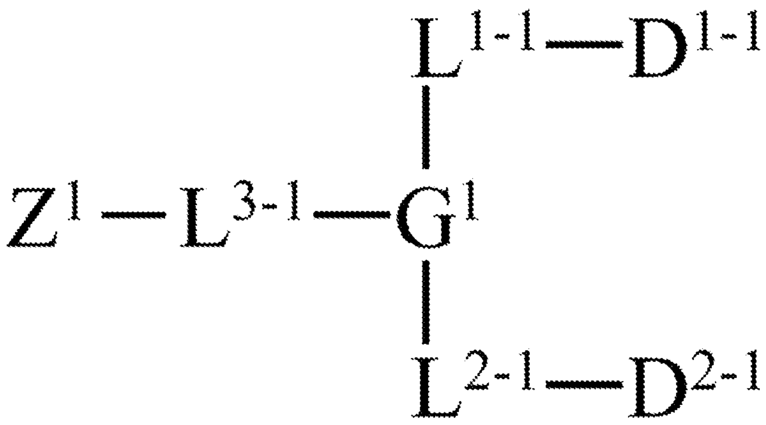

(II-1)

,

$G^1$ is a group selected from the formulae according to [Aspect B1] or [Aspect B2] above.

[Aspect F2]

The conjugate precursor synthesis intermediate (II-1) according to [Aspect F1], or a salt thereof, in which

$L^{1-1}$, $L^{2-1}$, and $L^{3-1}$ are each independently an optionally substituted alkylene group; and
one or more methylene groups in the chain of said alkylene group are each independently substituted with one or more groups selected from the divalent groups according to [Aspect C1] to [Aspect C4] above.

[Aspect G1]

The conjugate precursor synthesis intermediate (II-1) according to [Aspect F1] or [Aspect F2], or a salt thereof, in which $Z^1$ is a bonding group which is a hydroxyl group, a nitro group, a cyano group, an amide group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.

[Aspect G2]

The conjugate precursor synthesis intermediate (II-1) according to any one of [Aspect F1], [Aspect F2], and [Aspect G1], or a salt thereof, in which $Z^1$ is a bonding group which is a hydroxyl group, a nitro group, a cyano group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.

[Aspect G3]

The conjugate precursor synthesis intermediate (II-1) according to any one of [Aspect F1], [Aspect F2], [Aspect G1], and [Aspect G2], or a salt thereof, in which $Z^1$ is a bonding group which is a hydroxyl group, a nitro group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.

[Aspect G4]

The conjugate precursor synthesis intermediate (II-1) according to any one of [Aspect F1], [Aspect F2], and [Aspect G1] to [Aspect G3], or a salt thereof, in which $Z^1$ is a bonding group which is a hydroxyl group, a nitro group, an oxo group, an azide group, an amino group, a monoalkylamino group which may have a substituent, an imino group (also including -N= and an imino group of a cyclic amine), a carboxy group, a phosphoryl group, an alkynyl group, or a cycloalkynyl group; or a protective body thereof.

[Aspect H1]

The conjugate precursor synthesis intermediate (II-1) according to any one of [Aspect F1], [Aspect F2], and [Aspect G1] to [Aspect G4], or a salt thereof, in which $Z^1$ is a group selected from the bonding groups according to [Aspect G1] to [Aspect G4] above, and
either $D^{1-1}$ or $D^{2-1}$ is a group selected from the antitumor drug residues according to [Aspect D1] to [Aspect D6] above, and the other is a group selected from the bonding groups according to [Aspect G1] to [Aspect G4] above.

[Aspect H2]

The conjugate precursor synthesis intermediate (II-1) according to any one of [Aspect F1], [Aspect F2], and [Aspect G1] to [Aspect G4], or a salt thereof, in which $Z^1$ is a group selected from the bonding groups according to [Aspect G1] to [Aspect G4] above, and
both of $D^{1-1}$ and $D^{2-1}$ are each independently a group selected from the antitumor drug residues according to [Aspect D1] to [Aspect D6] above.

[Aspect H3]

The conjugate precursor synthesis intermediate (II-1) according to any one of [Aspect F1], [Aspect F2], and [Aspect G1] to [Aspect G4], or a salt thereof, in which all of $Z^1$, $D^{1-1}$, and $D^{2-1}$ are each independently a group selected from the bonding groups according to [Aspect G1] to [Aspect G4] above.

[Aspect H4]

The conjugate precursor synthesis intermediate (II-1) according to any one of [Aspect F1], [Aspect F2], and [Aspect G1] to [Aspect G4], or a salt thereof, in which $Z^1$ is the reactive group as defined in [Aspect A1] above, and either $D^{1-1}$ or $D^{2-1}$ is a group selected from the antitumor drug residues according to [Aspect D1] to [Aspect D6] above, and the other is a group selected from the bonding groups according to [Aspect G1] to [Aspect G4] above.

[Aspect H5]

The conjugate precursor synthesis intermediate (II-1) according to any one of [Aspect F1], [Aspect F2], and [Aspect G1] to [Aspect G4], or a salt thereof, in which $Z^1$ is the reactive group as defined in [Aspect A1] above, and both of $D^{1-1}$ and $D^{2-1}$ are each independently a group selected from the bonding groups according to [Aspect G1] to [Aspect G4] above.

[Aspect I1]

The conjugate precursor intermediate (II-1) according to any one of [Aspect F1], [Aspect F2], [Aspect G1] to [Aspect G4], and [Aspect H1] to [Aspect H5], or a salt thereof, in which when there are one or more groups independently selected from a lactonyl group; a phosphoryl group; a phosphorylalkylcarbonyl group ($-C(=O)$-alkylene-$P(=O)(OH)_2$); dialkylamino group; and a cyclic amino group that forms a ring together with the dialkyl group at any position(s) of the

antitumor drug residues or the linker moieties and there are a plurality of said groups in total, they may be identical to or different from each other.

[Aspect I2]

The conjugate precursor intermediate (II-1) according to any one of [Aspect F1], [Aspect F2], [Aspect G1] to [Aspect G4], [Aspect H1] to [Aspect H5], and [Aspect I1], or a salt thereof, in which one or more lactonyl groups and one or more phosphoryl groups are independently present as substituents or protecting groups at any position of the antitumor drug residues or the linker moieties, the total number of said lactonyl groups is 1 to 8, the lactonyl groups may be identical to or different from each other when said number of the lactonyl groups is two or more, and the total number of said phosphoryl groups is 1 to 4.

[Aspect I3]

The conjugate precursor intermediate (II-1) according to any one of [Aspect F1], [Aspect F2], [Aspect G1] to [Aspect G4], [Aspect H1] to [Aspect H5], [Aspect I1], and [Aspect I2], or a salt thereof, in which one or more lactonyl groups and one or more phosphoryl groups are independently present as substituents or protecting groups at any position of the antitumor drug residues or the linker moieties, and
apart from the above, when there are one or more groups independently selected from a lactonyl group; a phosphoryl group; a phosphorylalkylcarbonyl group (-C(=O)-alkylene-P(=O) $(OH)_2$); dialkylamino group; and a cyclic amino group that forms a ring together with the dialkyl group at any position(s) of the antitumor drug residues or the linker moieties and there are a plurality of said groups in total, they may be identical to or different from each other.

[Aspect J1]

A conjugate precursor intermediate (II-2) represented by General Formula (II-2) below:

[C55]

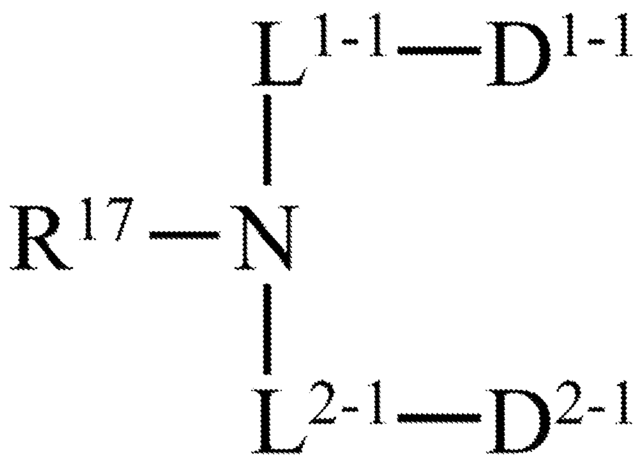

(II–2)

[in the formula,

$L^{1-1}$ and $L^{2-1}$ are each independently an optionally substituted alkylene group, and one or more methylene groups in the chain of said alkylene group are each independently substituted with one or more groups selected from the divalent groups according to [Aspect C1] to [Aspect C4],
$D^{1-1}$ and $D^{2-1}$ are groups selected from the antitumor drug residues according to [Aspect D1] to [Aspect D6] above or groups selected from the bonding groups according to [Aspect G1] to [Aspect G4], and these $D^{1-1}$ and $D^{2-1}$ may be identical to or different from each other, when there are one or more groups independently selected from a lactonyl group; a phosphoryl group (-P(=O) $(OH)_2$); a phosphorylalkylcarbonyl group (-C(=O)-alkylene-P(=O) $(OH)_2$); dialkylamino group; and a cyclic amino group that forms a ring together with the dialkyl group at any position(s) of said $L^{1-1}$ and $L^{2-1}$ or antitumor drug residues and there are a plurality of said groups in total, they may be identical to or different from each other, and $R^{17}$ is a hydrogen atom or a protecting group], or a salt thereof.

In the divalent group described above, if a part of $L^1$, $L^2$, $L^3$, $L^{1-1}$, $L^{2-1}$ and $L^{3-1}$ of the general formule (I) and (II) is left-right asymmetric or can be left-right asymmetric, said divalent group may face either the left, right, top, or bottom direction on the paper surface. For example, when -N($R^3$)-C($R^1$) ($R^2$)- exists as a part of $L^1$, -N($R^3$)- may be in the B side and -C($R^1$)($R^2$)- may be in the D side, or -C($R^1$)($R^2$)- may be in the B side and -N($R^3$)- may be in the D side.

Further, "three-letter alphabet" is a symbol for indicating the following amino acid, and especially in the present description, it indicates the divalent residue of each amino acid (-NH-CH($R^{15}$)-C(=O)-). In said formula, $R^{15}$ is a hydrogen atom (Gly), a methyl group (Ala), an isopropyl group (Val), an isobutyl group (Leu), a (butan-2-yl) group (Ile), a phenylmethyl group (Phe), a hydroxymethyl group (Ser), a sulfhydrylmethyl group (Cys), a carboxymethyl group (Asp), a 2-carboxyethyl group (Glu), a 3-aminopropyl group (Orn), a 4-aminobutyl group (Lys), a 3-(ureido)propyl group (Cit), a 3-guanidinopropyl group (Arg), or a (1H-imidazol-4-yl)methyl group (His) (wherein each amino acid in parentheses indicates the corresponding amino acid). Also, in the above formula (-NH-CH($R^{15}$)-C(=O)-), when $R^{15}$ is a propyl group and said propyl group is combined with the nitrogen atom of "-NH-" in the formula to form a 5-membered ring, a cyclic amino acid (Pro; proline) is formed.

Gly; glycine
Ala; alanine
Val; valine
Leu; leucin
Ile; isoleucine
Phe; phenylalanine
Ser; serine
Cys: cysteine
Asp; aspartic acid
Glu; glutamic acid
Orn; ornithine
Lys; lysine
Cit; citrulline
Arg; arginine
His; histidine
Pro; proline

When the "linker moieties" or the "antitumor drug residues" of the conjugate precursor represented by General Formula (I) of the present invention and the conjugate precursor synthesis intermediates represented by General formulae (II-1) and (II-2) of the present invention include amino acid residue(s) having asymmetric center(s) and optical isomer(s) may be generated, both D-type and L-type optical isomers are also encompassed by the present invention.

[Aspect K1]

A precursor of an antibody-drug conjugate (ADC) containing an antibody and a drug, or a salt thereof, in which

the ADC is one in which an antibody is directly or indirectly linked to a drug via a linker,
the precursor has a linker and a drug (for example, an antitumor drug molecule, an analog or derivative thereof, and a derivative of the analog), and
the linker has a reactive group (a linkage moiety with an antibody) that can react with a functional group (for example, a thiol group) in an antibody, and is modified by a solubilizing group, for example, a group selected from the group consisting of a lactonyl group, an optionally substituted $C_1$-$C_6$ aminoalkyl group, and a phosphoryl group (especially a solubilizing group).

[Aspect K2]

The precursor or a salt thereof according to [Aspect K1], wherein the linker is modified with a lactonyl group and a phosphoryl group.

[Aspect K3]

The precursor or a salt thereof according to [Aspect K1] or [Aspect K2],

wherein the linker is a branched linker, and

the branched linker has at least one branch, at least a first linker moiety ($L^1$), a second linker moiety ($L^2$) and a third linker moiety $L^3$),
the third linker is interposed between the antibody and the branch,
the first linker moiety and the second linker moiety are each linked to the branch at one end via covalent bond, and each linked at the other end to a first antitumor drug molecule or an analog thereof or a derivative; a derivative of the analog and a second antitumor drug molecule or an analog thereof or a derivative thereof; or a derivative of the analog via a covalent bond, {here, the first linker moiety and the second linker moiety are identical to or different from each other, and the first antitumor drug molecule and the second antitumor drug molecule are identical to or different from each other}.

[Aspect K4]

The precursor or a salt thereof according to any one of [Aspect K1] to [Aspect K3],
wherein the first linker moiety, the second linker moiety and the third linker moiety each contain a polyalkylene glycol (preferably, polyethylene glycol) block.

[Aspect K5]

The precursor or a salt thereof according to any of [Aspect K1] to [Aspect K4]
wherein the third linker moiety is modified with a lactonyl group and/or a phosphoryl group.

[Aspect K6]

The precursor or a salt thereof according to any one of [Aspect K1] to [Aspect K5],
wherein one or both of the first linker moiety and the second linker moiety are modified with a lactonyl group and/or a phosphoryl group.

[Aspect K7]

The precursor or a salt thereof according to any one of [Aspect K1] to [Aspect K5],
wherein the first linker moiety and the second linker moiety are modified with a lactonyl group and a phosphoryl group.

[Aspect K8]

The precursor or a salt thereof according to any one of [Aspect K1] to [Aspect K7],
wherein the first linker moiety or the second linker moiety and an antitumor drug molecule or an analog thereof or a derivative thereof; or a derivative of the analog are covalently linked via -O-P(=O) (OH)-O-.

[Aspect K9]

The precursor or a salt thereof according to any one of [Aspect K1] to [Aspect K8]
wherein the first linker moiety and the second linker moiety, and an antitumor drug molecule binding thereto, an analog thereof or a derivative thereof; or a derivative of the analog are covalently linked via -O-P(=O)(OH)-O-.

[Aspect K10]

The precursor or a salt thereof according to [Aspect K8] or [Aspect K9],
wherein the antitumor drug molecule or an analog thereof or a derivative thereof; or a derivative of the analog has a secondary amine, and the secondary amine is covalently linked to a second linker moiety via -O-P(=O) (OH)-O-.

[Aspect K11]

The precursor or a salt thereof according to any one of [Aspect K1] to [Aspect K10],
wherein the second linker moiety and the third linker moiety have an oligopeptide moiety that is cleaved in the tumor tissue.

[Aspect K12]

The precursor or a salt thereof according to [Aspect K11],

wherein the oligopeptide moiety contains a valine-citrulline (Val-Cit) dipeptide.

[Aspect K13]

The precursor or a salt thereof according to any one of [Aspect K1] to [Aspect K12],

wherein one, two or all selected from the group consisting of the first linker moiety, the second linker moiety, and the third linker moiety contain a polyalkylene glycol block (preferably a polyethylene glycol block),
the second linker moiety and the third linker moiety contain a valine-citrulline (Val-Cit) dipeptide, and
one, two or all selected from the group consisting of the first linker moiety, the second linker moiety, and the third linker moiety are modified with a phosphoryl group and/or a lactonyl group.

[Aspect K14]

The precursor or a salt thereof according to any one of [Aspect K1] to [Aspect K13],
wherein the antitumor drug molecule is modified with a phosphoryl group or a phosphate group, or has a phosphoryl group or a phosphate group.

[Aspect K15]

The precursor or a salt thereof according to any one of [Aspect K1] to [Aspect K14] above, which exhibits superior long-term blood retention compared to a precursor having MC-Val-Cit-PAB-MMAE when linked to the identical antibody.

[Aspect K16]

The precursor or a salt thereof according to any of the above, in which, when linked to the antibody, the concentration in blood after 3 days of administration is 10% or higher than the concentration in blood after 5 minutes of administration.

[Aspect K17]

The precursor or a salt thereof according to any of the above, in which, when linked to the antibody, the concentration in blood after 3 days of administration is 20% or higher than the concentration in blood after 5 minutes of administration.

[Aspect K18]

The precursor or a salt thereof according to any of the above, in which, when linked to the antibody, the concentration in blood after 3 days of administration is 25% or higher than the concentration in blood after 5 minutes of administration.

[Aspect K19]

The precursor or a salt thereof according to any of the above, in which, when linked to the antibody, the concentration in blood after 3 days of administration is 30% or higher than the concentration in blood after 5 minutes of administration.

[Aspect K20]

The precursor or a salt thereof according to any of the above, in which, when linked to the antibody, the concentration in blood after 3 days of administration is 14% or higher than the concentration in blood after 5 minutes of administration.

[Aspect K21]

The precursor or a salt thereof according to any of the above, in which the linker includes a linkage moiety with an antibody, a polyethylene glycol moiety, a cleavable moiety, a moiety linking the cleavable moiety to a drug moiety, and a drug.

[Aspect K22]

The precursor or a salt thereof according to any of the above, in which the linkage moiety with an antibody contains a maleimidyl group.

[Aspect K23]

The precursor or a salt thereof according to any of the above, in which the polyethylene glycol moiety contains 3 to 20 (preferably 3 to 10, for example, 3 to 6) ethylene glycol units.

[Aspect K24]

The precursor or a salt thereof according to any of the above, in which the cleavable moiety contains a valine-citrulline dipeptide.

[Aspect K25]

The precursor or a salt thereof according to any of the above, in which the linkage moiety with an antibody contains a maleimidyl group,

the polyethylene glycol block contains 3 to 20 (preferably 3 to 10, for example, 3 to 6) ethylene glycol units, and the cleavable moiety contains a valine-citrulline dipeptide.

[Aspect K26]

The precursor or a salt thereof according to any of the above, in which the linkage moiety with an antibody has an additional solubilizing group.

[Aspect K27]

The precursor or a salt thereof according to any of the above, in which the cleavable portion has a solubilizing group in the side chain.

[Aspect K28]

The precursor or a salt thereof according to any of the above, in which the moiety linking the cleavable moiety to a drug has an additional solubilizing group.

[Aspect K29]

The precursor or a salt thereof according to any of the above, in which the drug moiety has an additional solubilizing group.

[Aspect K30]

The precursor or a salt thereof according to [Aspect K26] above, in which the additional solubilizing group is a group having phosphonic acid in the side chain.

[Aspect K31]

The precursor or a salt thereof according to [Aspect K27] above, in which the solubilizing group is an amino group, phosphonic acid, or a lactonyl group.

[Aspect K32]

The precursor or a salt thereof according to [Aspect K28] above, in which the additional solubilizing group is a phosphate ester (including a phosphate diester).

[Aspect K33]

Use in production of the precursor or a salt thereof of any of the above of each moiety (especially the moiety having a solubilizing group) specified in [Aspect K21] to [Aspect K32] above.

[Aspect L1]

A compound having Formula (I) above, or a salt thereof, in which the formula has $R^{18}$ and $R^{19}$ instead of $D^1$ and $D^2$, respectively, and $R^{18}$ and $R^{19}$ are independently hydrogen atoms, protecting groups, or bonding groups {the definition of each group in the formula is as described above}.

[Aspect L2]

A compound having Formula (II-1) above, or a salt thereof, in which the formula has $R^{18}$ and $R^{19}$ instead of $D^{1-1}$ and $D^{2-1}$, respectively, and $R^{18}$ and $R^{19}$ are independently hydrogen atoms, protecting groups, or bonding groups {the definition of each group in the formula is as described above}.

[Aspect L3]

A compound having Formula (II-2) above, or a salt thereof, in which the formula has $R^{18}$ and $R^{19}$ instead of $D^{1-1}$ and $D^{2-1}$, respectively, and $R^{18}$ and $R^{19}$ are independently hydrogen atoms, protecting groups, or bonding groups {the definition of each group in the formula is as described above}.

[Aspect L4]

A method for producing an antibody-drug conjugate (ADC) containing an antibody and a drug, the method including:
reacting the compounds or salts thereof according to any one of [Aspect L1] to [Aspect L3] with an antibody and an antitumor drug molecule, an analog or derivative thereof, and a derivative of said analog to obtain ADCs.

[Advantageous Effects of Invention]

The antibody-multidrug conjugate obtained by reacting the conjugate precursor of the present invention (for example, the precursor (I)) or a salt thereof with an antibody has high stability in blood compared to an antibody-multidrug conjugate without said modification, and therefore is expected to exhibit a sustained antitumor effect, which is preferable. The antibody-multidrug conjugate obtained by reacting the conjugate of the present invention (for example, the precursor (I)) or a salt thereof with an antibody can be used as a cancer therapeutic agent having superior antitumor effects and safety by being selectively delivered to a target tumor cell after being administered to a living body to release an antitumor drug in said cell.

[Brief Description of Drawings]

[Fig. 1]
Fig. 1 shows the antitumor effect of AMDC (1 mg/kg body weight) of AMDC Production Example 2.
[Fig. 2]
Fig. 2 shows the antitumor effect of AMDC (1 mg/kg body weight) of AMDC Production Example 3.
[Fig. 3]
Fig. 3 shows the antitumor effect of AMDC (1 mg/kg body weight) of AMDC Production Example 4.
[Fig. 4]
Fig. 4 shows the antitumor effect of AMDC (1 mg/kg body weight) of AMDC Production Example 5.
[Fig. 5]
Fig. 5 shows the antitumor effect of AMDC (1 mg/kg body weight) of AMDC Production Example 6.
[Fig. 6]
Fig. 6 shows the antitumor effect of AMDC (3 mg/kg body weight) of AMDC Production Example 4.
[Fig. 7]
Fig. 7 shows the antitumor effect of AMDC (3 mg/kg body weight) of AMDC Production Example 5.

[Fig. 8]

Fig. 8 shows the SEC analysis HPLC chart of AMDC of AMDC Production Example 2.

[Description of Embodiments]

Hereinafter, the definitions of terms used herein are described and each of them is specifically illustrated, but the present invention is not limited thereto. In addition, various compounds (antibodies and intermediates), drug molecules, substituents, protecting groups, and the like defined or illustrated below are optionally selected and combined to prepare a conjugate precursor (I) and conjugate precursor synthesis intermediates (II-1) and (II-2) of the present invention, or salts thereof of the present invention.

Unless otherwise specified, the following terms and phrases described herein have the following meanings. In addition, when trade names are used herein, the trade names include product formulations, generic drugs, and active pharmaceutical components of the trade name products unless otherwise indicated. In addition, terms referred to in singular form do not exclude a plural form. In addition, the term "comprising" has the meaning of both "consisting of" and "containing".

The term "antibody" as described herein refers to an intact monoclonal antibody, a polyclonal antibody, a monospecific antibody, multispecific antibody (for example, a bispecific antibody), a modified antibody, an unmodified antibody, an unaltered antibody, a full-length antibody, an intact antibody, and an antibody fragment exhibiting desired biological activity (for example, an antigen-binding fragment of an antibody, in particular, a fragment thereof exhibiting the same binding characteristics as an antibody; however, an antibody fragment has a required number of binding sites for linkers to which drugs are attached). An antibody can be bound to an antigen which is expressed on the surface of a tumor cell and can be internalized in the cell, in other words, the antibody can target a tumor cell. In an antibody-multidrug conjugate (AMDC) generated by a reaction of the conjugate precursor (I) of the present invention with an antibody, since the antibody binds to a cytotoxic agent (for example, an antitumor active drug) via a linker and the mechanism is such that the antitumor drug is released from the AMDC in a cell after delivery to the tumor cell as an AMDC, it is preferable that said antibody have one or more properties including a property of being able to recognize tumor cells, a property being able to specifically bind to tumor cells, a property being able to be incorporated and internalized into tumor cells, and a property being able to impair tumor cells.

Native antibodies are tetramers composed of two identical pairs of immunoglobulin chains, each pair having one light chain and one heavy chain. In each pair, light chain and heavy chain variable regions (VL and VH) together are primarily responsible for antigen binding. Light chain and heavy chain variable domains are composed of three hypervariable regions called "complementarity-determining regions" or "CDRs" and a framework area interrupted by four hypervariable regions. The constant region can be recognized by the immune system and can interact with the immune system. The antibody can be of any type (for example, IgG, IgE, IgM, IgD, IgY and IgA), class (for example, IgG1, IgG2, IgG3, IgG4, IgA1 and IgA2) or subclass, and particularly, IgG1, IgG2, IgG3, and IgG4. The antibody can be derived from any suitable species. In some aspects, the antibody is derived from a human or mouse. The antibody may be, for example, a human antibody, a humanized antibody or a chimeric antibody.

The term "monoclonal antibody" in description herein refers to an antibody obtained from a substantially homogeneous antibody population. That is, individual antibodies contained in the population are identical except for a small number of naturally occurring mutations. Monoclonal antibodies are highly specific and target a single antigenic moiety. The term "monoclonal" indicates an antibody property of "being obtained from a substantially homogeneous antibody population", and does not mean that antibodies should be produced by any specific method.

An "intact antibody" includes an antigen-binding variable region, and light chain constant domains (CL) and heavy chain constant domains CH1, CH2, CH3 and CH4 suitable for an antibody class. The constant domain may be a native sequence constant domain (for example, a human native sequence constant domain) or its amino acid sequence variant.

An "antibody fragment" includes a part of an intact antibody, including its antigen-binding region or variable region. The antibody fragment can be any antigen-binding fragment of an antibody, although not particularly limited, and examples thereof include Fab, Fab', $F(ab')_2$, and scFv. In order to be used in the present invention, it is necessary for the antibody fragment to have a functional group (for example, a sulfhydryl group and an amino group) or a disulfide bond that can produce a sulfhydryl group by reduction so that it can bind to drug moieties via a linker.

An "antigen" is a substance to which an antibody binds or specifically binds (for example, a cell surface antigen, and particularly a cell surface protein, a cell surface glycoprotein, or a cell surface glycan).

"Specific binding" and "specifically bind" mean that an antibody or an antibody derivative binds to a corresponding target antigen in a highly selective manner and does not bind to other abundant antigens. Typically, antibodies and antibody derivatives bind with an affinity of at least about $1\times10^{-7}$ M, and preferably $10^{-8}$ to $10^{-9}$ M, $10^{-10}$ M, $10^{-11}$ M, or $10^{-12}$ M, and bind to a predetermined antigen with an affinity that is at least twice an affinity for a non-specific antigen (for example, bovine serum albumin (BSA), casein) other than a predetermined antigen or a closely related antigen.

In a specific embodiment, known antibodies for cancer treatment can be used. Immunospecific antibodies to cancer cell antigens are commercially available or can be produced by any method known to those skilled in the art, for example, recombinant expression techniques. Nucleotide sequences encoding immunospecific antibodies to cancer cell antigens can be obtained, for example, from GenBank databases or similar databases, or from documents, or by conventional cloning and sequence determining.

Examples of antibodies that can be used for treating cancer include, but are not limited to, the humanized anti-HER2 monoclonal antibody Herceptin (registered trademark) (trastuzumab; Genentech) (antibodies for treating patients with metastatic breast cancer); Rituxan (registered trademark) (rituximab; Genentech) (chimeric anti-CD20 monoclonal antibodies for treating patients with non-Hodgkin's lymphoma); OvaRex (AltaRex Corporation, MA) (mouse antibody/anti-MUC16 (CA125) antibodies for treating ovarian cancer); Panorex (Glaxo Wellcome, NC) (mice antibody/anti-EPCAM antibodies for treating colorectal cancer); Cetuximab Erbitux (Imclone Systems Inc., NY) (IgG chimeric antibody/anti-EGFR antibodies for treating epidermal growth factor positive cancer such as head and neck cancer); Vitaxin (Med-Immune, Inc., MD) (anti-Integrin alphavbeta 3 antibodies for treating breast cancer, lung cancer, and the like); Campath (Leukosite, MA) (anti-CD52 antibodies for treating chronic lymphogenous leukemia (CLL)) Smart M195 (Protein Design Labs, Inc., CA) (humanized anti-CD33 IgG antibodies for treating acute myeloid leukemia (AML); LymphoCide (Immunomedics, Inc., NJ) (humanized anti-CD22 IgG antibodies for treating non-Hodgkin's lymphoma); Smart ID10 (Protein Design Labs, Inc., CA) (humanized anti-HLA-DR antibodies for treating non-Hodgkin's lymphoma); Oncolym (Techniclone, Inc., CA) (radiolabeled murine anti-HLA-Dr10 antibodies for treating non-Hodgkin's lymphoma), Allomune (BioTransplant, CA) (humanized anti-CD2 mAb for treating Hodgkin's disease and non-Hodgkin's lymphoma); Avastin (Genentech, Inc., CA) (anti-VEGF humanized antibodies for treating lung cancer and colorectal cancer); Epratuzamab (Immunomedics, Inc., NJ and Amgen, CA) (anti-CD22 antibodies for treating non-Hodgkin's lymphoma); Patritumab (Daiichi Sankyo Co., Ltd.) (anti-HER3 humanized antibodies for treating lung cancer and breast cancer); and CEA-Cide (Labetuzumab Immunomedics, NJ) (humanized anti-CEA antibodies for treating colorectal cancer).

Examples of other antibodies useful for treating cancer include, but are not limited to, antibodies to the following antigens. Examples thereof include cancer-testis antigens such as MAGE-A1, MAGE-A2, MAGE-A3, MAGE-A4, NY-ESO-1, PRAME, CT83, BAGE, XAGE, GAGE-1 and SSX2, differentiation antigens such as gp100, Melan A, Tyrosinase, CD20, CD22, CD33, CD79b, PSA and PAP, overexpressed antigens such as HER2, MUC-1, PSMA, CEA, survivin and WT-1, viral antigens such as HPV viral proteins (E6, E7, etc.) and Epstein-Barr virus (EBV) proteins (EBV-LMP2, etc.), mutated gene product antigens (Neoantigens) such as p53, K-ras and BCR-ABL, and CEA and $\alpha$-fetoprotein (AFP) oncofetal antigens.

Specific examples of antibodies used in the reaction with the conjugate precursor (I) of the present invention are given below, but the present invention is not limited thereto.

The antibodies include an anti-HER2 antibody, an anti-EGFR antibody, an anti-Lymphocyte Antigen 6E (Ly-6E) antibody, an anti-Hepatocyte Growth Factor Receptor (HGFR) antibody, an anti-CD22 antibody, an anti-Folate Receptor alpha (FR$\alpha$) antibody, an anti-PSMA (FOLH1) antibody, an anti-CD30 (TNFRSF8) antibody, an anti-TROP-2 antibody, an anti-CD19 antibody, an anti-5T4 antibody, an anti-Mesothelin antibody, an anti-CD20 antibody, an anti-CD33 antibody, an anti-CDB7-H3 antibody, an anti-CD269 (BCMA) antibody, an anti-CD142 antibody, an anti-CD70 antibody, an anti-CD123 antibody, an anti-TAG-72 antibody, an anti-TFRC antibody, an anti-EPHA2 antibody, an anti-EPHA3 antibody, an anti-c-KIT antibody, an anti-PD-L1 antibody, an anti-Epithelial Cell Adhesion Molecule (EPCAM) antibody, an anti-AXL antibody, an anti-Interleukin-1 Receptor Accessory Protein antibody, an anti-Mucin-16 (CA125) antibody, an anti-MUC1 antibody, an anti-GPC3 antibody, an anti-CLEC12A (CLL1) antibody, an anti-LRRC15 antibody, an anti-ADAM9 antibody, an anti-Sodium-Dependent Phosphate Transport Protein 2B (SLC34A2) antibody, an anti-CD25 antibody, an anti-ROR1 antibody, an anti-TweakR antibody, an anti-CD74 antibody, an anti-CEACAM5 antibody, an anti-CD105 antibody, an anti-CD79b antibody, an anti-CD147 antibody, an anti-Sialyl-Tn antibody, an anti-GCC antibody, an anti-HER3 antibody, an anti-Integrin antibody, an anti-EGFR variant III antibody, an anti-Claudin 6 antibody, an anti-Leucine-Rich Repeat-Containing G-Protein Coupled Receptor 5 antibody, an anti-PTK7 antibody, an anti-LYPD3 antibody, an anti-ASCT2 antibody, an anti-ASPH antibody, an anti-GPC1 antibody, an anti-CD174 antibody, an anti-CD38 antibody, an anti-ANGPT2 antibody, an anti-CD44 antibody, an anti-DDL3 antibody, an anti-FGFR2 antibody, an anti-KAAG1 antibody, an anti-CD73 antibody, an anti-CLDN18.2 antibody, an anti-PRLR antibody, an anti-SEZ6 antibody, an anti-Neprilysin antibody, an anti-Neural Cell Adhesion Molecule 1 antibody, an anti-CD166 (ALCAM) antibody, an anti-CD24 antibody, an anti-CD27 antibody, an anti-CDH3 antibody, an anti-EPHA4 antibody, an anti-FGFR3 antibody, an anti-GFRAL antibody, an anti-Globo H antibody, an anti-IL-13Ra2 antibody, an anti-STEAP-1 antibody, an anti-TMCC3 antibody, an anti-CD209 antibody, an anti-CD37 antibody, an anti-CD48 antibody, an anti-CDCP1 antibody, an anti-CD46 antibody, an anti-CD200 antibody, an anti-CTLA4 antibody, an anti-CXCR5 antibody, an anti-FLT3 antibody, an anti-SDC1 (CD138) antibody, an anti-CLDN6/9 antibody, an anti-CEACAM6 antibody, an anti-CA9 antibody, an anti-EDNRB antibody, an anti-GD3 Ganglioside antibody, an anti-MICA/B antibody, an anti-JAG1/2 antibody, an anti-TM4SF1 antibody, an anti-uPAR antibody, an anti-Carbonic anhydrase IX antibody, an anti-ALK1 antibody, an anti-IGF-1R antibody, an anti-KDR antibody, an anti-CEACAM8 antibody, an anti-IL5R antibody, an anti-CD205 (Ly75) antibody, an anti-MSR1 antibody, an anti-KREMEN2 antibody, an anti-SSEA-4 antibody, an anti-CD228 antibody, an anti-CD5 antibody, an anti-DLL4 antibody, an anti-FAP antibody, an anti-Notch3 antibody, an anti-AG7 antibody, an anti-Guanylate Cyclases antibody, an anti-Nectin-4 antibody, an anti-CD226 (DNAM-1) antibody, an anti-FcRL5 antibody, an anti-HLA-DR antibody, an anti-ITGB3 antibody, an anti-DLK1 antibody, an anti-CD157 (BST1) antibody, an anti-CD56 (NCAM1) antibody, an anti-MICA (MHC class I Polypeptide-Related Sequence A) antibody, an anti-SSEA-1 antibody, an anti-TRAIL-R2 (DR5) antibody, an anti-GPNMB antibody, an anti-CCR5 antibody, an anti-LAMP1 antibody, an anti-LGALS3BP antibody, an anti-ROR2 antibody, an anti-DLL3 antibody, an anti-ETBR antibody, an anti-LIV-1 antibody, an anti-Integrin $\alpha v\beta 6$ antibody, an anti-TIM-1 antibody, an anti-AGS-16 (ENPP3) antibody, an anti-SLITRK6 antibody, an anti-GD2 antibody, an anti-CD52 antibody, an anti-CCR4 antibody, an anti-VEGFR2 antibody, an anti-PDGFR antibody, an anti-FGFR antibody, an anti-SLAMF7 antibody, an anti-GD2 Ganglioside antibody, an anti-EPHA3 antibody, an anti-Integrin $\alpha v\beta 3$ antibody, an anti-AGS-5 antibody, an anti-CA19-9 antibody, an anti-PSA antibody, an anti-MAGE3 antibody, an anti-Transferrin receptor 1 (CD71) antibody, an anti-CEACAM1 antibody, an anti-SLC3A2 (CD98) antibody, an anti-ACVR1 antibody, an anti-AG-7 antibody, an anti-AMHR2 antibody, an anti-ABCB1 antibody, an anti-C16orf54 antibody, an anti-CathepsinD antibody, an anti-CCR7 antibody, an anti-SLC44A4 antibody, an anti-CD300LF antibody, an anti-DPEP3 antibody, an anti-FucGM1 antibody, an anti-GPR20 antibody, an anti-ITGB6 antibody, an anti-Lewis-A-like carbohydrate antibody, an anti-prolactin receptor antibody, an anti-Sialyl Tn antibody, an anti-SLAMF6 antibody, an anti-SLAMF7 antibody, an anti-TRA-1-60 antibody, an anti-matriptase antibody, an anti-B7-H4 antibody, an anti-Cripto antibody, an anti-CD99 antibody, an anti-CanAg antibody, an anti-A33 antibody, an anti-$\alpha 10\beta 1$ integrin antibody, an anti-ALPP antibody, an anti-CD248 antibody, and an anti-GPRC5D antibody.

In addition, examples of antibodies expressed by generic names include trastuzumab, rituximab, palivizumab, infliximab, basiliximab, tocilizumab, bevacizumab, adalimumab, cetuximab, ranibizumab, omalizumab, eculizumab, ustekinumab, golimumab, canakinumab, denosumab, mogamulizumab, natalizumab, nivolumab, secukinumab, ipilimumab, evolocumab, mepolizumab, alirocumab, ixekizumab, brodalumab, idarucizumab, pembrolizumab, sarilumab, bezlotoxumab, belimumab, avelumab, dupilumab, atezolizumab, benralizumab, emicizumab, guselkumab, durvalumab, vedolizumab, romosozumab, risankizumab, ravulizumab, burosumab, brolucizumab, tildrakizumab, satralizumab, galcanezumab, fremanezumab, erenumab, dinutuximab, anifrolumab, sotrovimab, bimekizumab, faricimab, nemolizumab, lanadelumab, ibritumomab, brentuximab, inotuzumab, polatuzumab, enfortumab, panitumumab, ofatumumab, pertuzumab, alemtuzumab, ramucirumab, elotuzumab, daratumumab, obinutuzumab, blinatumomab, necitumumab, isatuximab, inebilizumab, dinutuximab, gemtuzumab, lorvotuzumab, promiximab, tamrintamab, talquetamab, cofetuzumab, belantamab, talquetamab, elranatamab, felzartamab, mezagitamab, naratuximab, otlertuzumab, lilotomab, teprotumumab, ganitumab, dalotuzumab, cixutumumab, robatumumab, istiratumab, figitumumab, sibrotuzumab, mecbotamab, tilvestamab, mipasetamab, enapotamab, oregovomab, abagovomab, ubamatamab, sofituzumab, upifitamab, lifastuzumab, dacliximab, daclizumab, ipilimumab, camidanlumab, arcitumomab, altumomab, tusamitamab, labetuzumab, cibisatamab, patritumab, duligotumab, zenocutuzumab, seribantumab, barecetamab, lumretuzumab, elgemtumab, disitamab, margetuximab, zanidatamab, coprelotamab, inetetamab, gancotamab, anbenitamab, zenocutuzumab, ertumaxomab, runimotamab, amivantamab, nimotuzumab, zalutumumab, futuximab, modotuximab, depatuxizumab, duligotumab, tomuzotuximab, imgatuzumab, matuzumab, serclutamab, petosemtamab, isatuximab, etevritamab, suciraslimab, epratuzumab, bectumomab, pinatuzumab, mirvetuximab, farletuzumab, rosopatamb, capromab, iratumumab, sacituzumab, datopotamab, coltuximab, denintuzumab, duvortuxizumab, loncastuximab, obexelimab, tafasitamab, taplitumomab, ublituximab, tositumomab, ocrelizumab, ocaratuzumab, zuberitamab, divozilimab, imvotamab, epcoritamab, glofitamab, odronextamab, plamotamab, mosunetuzumab, amatuximab, anetumab, belantamab, alnuctamab, elranatamab, linvoseltamab, pacanalotamab, pavurutamab, teclistamab, vibecotamab, talacotuzumab, pivekimab, flotetuzumab, tucotuzumab, catumaxomab, solitomab, oportuzumab, edrecolomab, citatuzumab, and adecatumumab, as well as, antibodies having one to six (preferably six) complementarity-determining regions (CDR) of any of these antibodies.

The antibodies described herein also include modified antibodies. The modified antibodies refer to antibodies that have been chemically or biologically modified. Chemical modifications include binding of chemical moieties to the amino acid framework, chemical modifications of N-linked or O-linked carbohydrate chains, and the like. Examples of biological modifications include those that are subjected to post-translational modifications (for example, glycosylation to N-linkage or O-linkage, N-terminal or C-terminal processing, deamidation, aspartic acid isomerization, and methionine oxidation) and addition of a methionine residue to the N-terminal by expression using prokaryotic host cells. In addition, such modifications also include labels that allow antibodies or antigens to be detected or isolated, for example, enzyme labels, fluorescent labels, and affinity labels. Such modified antibodies of the present invention are useful for improving stability and blood retention of original antibodies, reducing antigenicity, and detecting or isolating such antibodies or antigens.

The term "modified antibody" as described herein refers to a chemically or biologically modified antibody in which a linker is extended from a functional group (such as a hydroxyl group, a sulfhydryl group, an amino group, or a carboxy group) present in the original antibody, and a "sulfhydryl group, a hydroxyl group, an amino group, a maleimidyl group (Formula (v) above), an $\alpha$-halogenomethylcarbonyl group (Formula (vi) above), an ethynylphosphonamidate group (Formula (vii) above), a carboxy group, an active ester of a carboxy group, an azide group ($-N_3$ group), an alkynyl group, a

cycloalkynyl group, or the like" is added to the terminal of the linker. In the present specification, the above-defined "antibody" and the above-defined "modified antibody" are optionally collectively referred to as an "antibody."

In addition, antibody-dependent cytotoxic activity can be enhanced by regulating (glycosylating, defucosylating, etc.) glycan modifications bound to the antibodies described herein. Known techniques for regulating glycan modification of antibodies include, but are not limited to, those in WO 99/54342, WO 00/61739, and WO 02/31140. Antibodies used in the reaction with the conjugate precursor (I) of the present invention also include antibodies that have been subjected to glycosylation modification.

The term "functional groups in antibodies" as described herein refers to groups present in the above-defined "antibody" and "modified antibody," which react with reactive groups present in the conjugate precursor (I) and conjugate precursor synthesis intermediate (II-1) of the present invention to form antibody-multidrug conjugates. Specific examples thereof include a sulfhydryl group, a hydroxyl group, an amino group, a maleimidyl group (Formula (v) above), an α-halogenomethylcarbonyl group (Formula (vi) above), an ethynylphosphonamidate group (Formula (vii) above), a carboxy group, an active ester of a carboxy group, an azide group ($-N_3$ group), an alkynyl group, and a cycloalkynyl group.

The term "reactive groups" as described herein refers to groups present in the conjugate precursor (I) of the present invention and conjugate precursor intermediate (II-1) of the present invention, which react with the above-defined "functional groups in antibodies" to form antibody-multidrug conjugates. Specific examples thereof include a maleimidyl group (Formula (v) above), an α-halogenomethylcarbonyl group (Formula (vi) above), an ethynylphosphonamidate group (Formula (vii) above), a carboxy group, an active ester of a carboxy group, a sulfhydryl group, a hydroxyl group, an amino group, an alkynyl group, a cycloalkynyl group, and an azide group ($-N_3$ group).

The term "bonding groups" as described herein refers to groups present at the ends of linker components of the conjugate precursor synthesis intermediates (II-1) and (II-2) of the present invention for bonding said "reactive groups" or "antitumor drug residues" described below to the "linker moieties" through an etherification reaction, a thioetherification reaction, a carboxyesterification reaction, a phosphate (mono- or di-)esterification reaction, an amidation reaction, a reductive amination reaction, a disulfidation reaction, a click reaction, or the like. Specific examples thereof include a hydroxyl group, a nitro group, a cyano group, an amide group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; and a protective body thereof. In said group of the groups, nitro groups can be precursors of amino groups, cyano groups can be precursors of amino groups and amide groups, and amide groups can be precursors of amino groups and carboxy groups, and therefore, they are also treated as "bonding groups" herein.

The term "antitumor drug molecule" as described herein is not particularly limited as long as it is a drug molecule which has an antitumor effect or cytotoxicity regardless of its action mechanism and has a substituent or partial structure that can bind to linkers ($L^1$ and $L^2$ in the conjugate precursor (I) and $L^{1-1}$ and $L^{2-1}$ in the conjugate precursor synthesis intermediates (II-1) and (II-2)). The antitumor drug molecule is preferably released by the cleavage of some or all of the linkers in a tumor cell to exert its antitumor effect.

The term "antitumor drug residues" as described herein refers to residues in which one hydrogen atom or one hydroxyl group has been removed from any position of said "antitumor drug molecule or an analog thereof, or a derivative thereof." The atom to be removed or residues can be selected as appropriate by those skilled in the art.

Examples of antitumor drug molecules include antitumor drug molecules independently selected from the group consisting of camptothecin; MMAE; maytansine; a parabenzodiazepine (PBD) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; ciprofloxacin; cadrofloxacin (CS-940); and analogs or derivatives thereof, or derivatives of the analogs.

The term "analog" of "an antitumor drug molecule or an analog thereof, or a derivative thereof" in description herein refers to a compound having a chemical structure and activity (that is, antitumor activity of antitumor drug molecules) similar to that of each antitumor drug molecule, and for example "analog of camptothecin" refers to a compound having a similar chemical structure to camptothecin and the same type I topoisomerase inhibitory action as camptothecin. Examples of analogs include antitumor drug molecules modified with a solubilizing group.

In this specification, the "solubilizing group" is a group that ionizes or forms a hydrogen bond with a water molecule and improves water solubility of a compound (that is, improves water solubility of a compound relative to one without the solubilizing group). Examples of solubilizing groups include a lactonyl group (which may be, for example, β-propiolactone, γ-butyrolactone, or δ-valerolactone, and preferably γ-butyrolactone), a phosphoryl group ($-OPO_3H_2$), a phosphonic acid group ($-PO_3H_2$), a carboxyl group, a sulfonic acid group ($-SO_3H$), a nitric acid group ($-OSO_3H$), and a $C_1$-$C_6$ alkyl group having one or more optionally substituted heteroatoms (for example, O, S, or N). Examples of $C_1$-$C_6$ alkyl groups having one or more heteroatoms (for example, O, S, or N) include an aminohexyl group, an aminopentyl group, an aminobutyl group, an aminopropyl group, an aminoethyl group, and an aminomethyl group. In addition, a $C_1$-$C_6$ alkyl group having one or more heteroatoms (for example, O, S, or N) may be substituted, and particularly, optionally substituted with one, two, three or more optionally susbsituted methyl groups. Examples of $C_1$-$C_6$ alkyl groups having one or more

optionally substituted heteroatoms (for example, O, S, or N) include an aminohexyl group, aminopentyl group, aminobutyl group, aminopropyl group, aminoethyl group, and aminomethyl group substituted with one, two, three or more methyl groups, and for example, a dimethylaminobutyl group may be exemplified. Examples of substitutions for a methyl group include a hydroxyl group and a halogen (for example, Cr, F, and Br). In a preferable aspect, the solubilizing group is a phosphoryl group ($-OPO_3H_2$) or a phosphonic acid group ($-PO_3H_2$).

The term of "derivative" in "an antitumor drug molecule or an analog thereof, or a derivative thereof" as described herein refers to a derivative in which a functional group present in said antitumor drug molecule such as a hydroxy group, a sulfhydryl group (-SH), an amino group ($-NH_2$ and -NH- (also including -NH- in an amide group and -NH- in the ring of a heteroaryl group or a heterocyclyl group)), and a carboxy group is protected by a "protecting group", or a glycoside derivative produced by reacting said functional group with a saccharide. The protecting group of said derivative may be deprotected in vivo by various chemical reactions, biochemical reactions, or metabolic reactions (for example, pH fluctuations, hydrolysis reactions, and redox reactions), converted to a mother compound (original antitumor drug molecule), and exhibit antitumor activity in some cases, or may not be deprotected and exhibit antitumor activity in its intact form in other cases.

Said "protecting group" is optionally selected from the protecting groups disclosed in, for example, Greene's Protective Groups in Organic Synthesis 5th Edition, P. G. M. Wuts, JohnWiley & Sons Inc. (2014) and the like.

Examples of the protecting group of hydroxy group, which is a functional group, include alkyloxyalkyl groups such as a methyloxymethyl group, a methoxyethyloxymethyl group, and an ethoxy-2-ethyl group; arylalkyloxymethyl groups such as a benzyloxymethyl group; aryloxymethyl groups such as a phenyloxymethyl group; alkylthiomethyl groups such as a methylthiomethyl group; arylalkylthiomethyl groups such as a benzylthiomethyl group; arylthiomethyl groups such as a phenylthiomethyl group; aminomethyl groups in which the N atom is optionally protected by an alkyl group or a below-described "protecting group of amino group"; arylmethyl groups such as a benzyl group, a 4-methoxybenzyl group, and a triphenylmethyl group; alkylcarbonyl groups such as a methylcarbonyl group and an ethylcarbonyl group; alkenylcarbonyl groups such as an allylcarbonyl group; alkynylcarbonyl groups such as a propargylcarbonyl group: arylcarbonyl groups such as a phenylcarbonyl group; heteroarylcarbonyl groups such as a pyridine-2, 3, or 4-ylcarbonyl group; heterocyclylcarbonyl groups such as a tetrahydrofuran-2 or 3-ylcarbonyl group and a 5-oxotetrahydrofuran-2-ylcarbonyl group; alkyloxycarbonyl groups such as a tert-butyloxycarbonyl group, a methyloxycarbonyl group, and an ethyloxycarbonyl group; an allyloxycarbonyl group; allyloxycarbonyl groups such as a pheny oxycarbonyl group, a 4-Nitro pheny oxycarbonyl group, and a 4-methoxy pheny oxycarbonyl group; arylmethyloxycarbonyl groups such as a 9-fluorenylmethyloxycarbonyl group, a benzyloxycarbonyl group, a 4-methoxybenzyloxycarbonyl group, a 4 (or 2)-nitrobenzyloxycarbonyl group, a 4 (or 2)-aminobenzyloxycarbonyl group or an N-alkylcarbonylated or N-arylcarbonylated derivative thereof, and a 4 (or 2)-hydroxybenzyloxycarbonyl group or an O-alkylcarbonylated or O-arylcarbonylated derivative thereof; alkylaminocarbonyl groups such as a tert-butylaminocarbonyl group, a methylaminocarbonyl group, and an ethylaminocarbonyl group; silyl groups such as a trimethylsilyl group, a trimethylsilylethoxymethyl group, a triisopropylsilyl group, a triisopropylsilyl group, a tert-butyldimethylsilyl group, and a tert-butyldiphenylsilyl group; tetrahydropyranyl groups such as a tetrahydropyranyl group and a 2 (or 4)-methoxytetrahydropyranyl group. Also, said hydroxy group is optionally protected as an ester with formic acid, boric acid, phosphoric acid, phosphonic acid, phosphinic acid, sulfuric acid, sulfonic acid, sulfinic acid, sulfenic acid, an amino acid, or a peptide.

In one embodiment, the protecting group of hydroxy group is an alkyloxyalkyl group; an arylalkyloxymethyl group; an aminomethyl group in which the N atom is optionally protected by an alkyl group or a below-described "protecting group of amino group"; an alkylcarbonyl group; an alkenylcarbonyl group; an alkynylcarbonyl group: an arylcarbonyl group; an alkyloxycarbonyl group; an allyloxycarbonyl group; an allyloxycarbonyl group; an arylmethyloxycarbonyl group; an alkylaminocarbonyl group; a tetrahydropyranyl group; a tetrahydrofuranyl group; a silyl group, a lactonyl group; a lactonylalkyl group; a lactonylcarbonyl group; a lactonylalkylcarbonyl group; a phosphoryl group; a phosphorylalkyl group; or a phosphorylalkylcarbonyl group.

Examples of protecting groups of sulfhydryl groups which are functional groups include the protecting groups described as the protecting group of hydroxyl groups, but in addition, disulfide bonds (-S-S-) can also be protecting groups.

Examples of protecting groups for a carboxy group that is a functional group include alkyl groups such as a methyl group, an ethyl group, and a tert-butyl group ; an allyl group; arylmethyl groups such as abenzyl group ; a lactonyl group; and silyl groups such as a trimethylsilyl group, a trimethylsilylethoxymethyl group, a tert-butyldimethylsilyl group, and a tert-butyldiphenylsilyl group. In addition, the carboxy group may also be protected as an amide component (for example, an amide component with ammonia, alkylamine, dialkylamine, lactonylamine, amino acid ester, amino acid amide or the like), and the amino group of the amide moiety may have, as a substituent, a lactonyl group, a lactonylalkyl group, or a phosphorylalkyl group.

In one embodiment, the protecting group for a carboxy group is an alkyl group; an allyl group; an arylmethyl group; a lactonyl group; a lactonylalkyl group; or a phosphorylalkyl group.

The functional groups, a sulfenic acid group, a sulfinic acid group, a sulfonic acid group, a phosphinic acid group, a phosphonic acid group, and a boric acid group can also use the same protecting groups as those of the above carboxy

group.

Examples of the protecting group of amino group, which is a functional group, include alkyloxycarbonyl groups such as a tert-butyloxycarbonyl group, a methyloxycarbonyl group, and an ethyloxycarbonyl group; an allyloxycarbonyl group; allyloxycarbonyl groups such as a pheny oxycarbonyl group, a 4-nitro pheny oxycarbonyl group, and a 4-methoxy pheny oxycarbonyl group, aryloxycarbonyl groups such as a phenyloxycarbonyl group, a 4-nitrophenyloxycarbonyl group, and a 4-methoxyphenyloxycarbonyl group; arylmethyloxycarbonyl groups such as a 9-fluorenylmethyloxycarbonyl group, a benzyloxycarbonyl group, a 4-methoxybenzyloxycarbonyl group, a 4 (or 2)-nitrobenzyloxycarbonyl group, a 4 (or 2)-aminobenzyloxycarbonyl group or an N-alkylcarbonylated or N-arylcarbonylated derivative thereof, a 4 (or 2)-hydroxybenzyloxycarbonyl group or an O-alkylcarbonylated or O-arylcarbonylated derivative thereof; arylmethyl groups such as a benzyl group and a triphenylmethyl group; alkyloxyalkyl groups such as a methyloxymethyl group, a methoxyethyloxymethyl group, and an ethoxy-2-ethyl group; arylalkyloxymethyl groups such as a benzyloxymethyl group; aryloxymethyl groups such as a phenyloxymethyl group; alkylthiomethyl groups such as a methylthiomethyl group; arylalkylthiomethyl groups such as a benzylthiomethyl group; arylthiomethyl groups such as a phenyloxymethyl group; an aminomethyl group in which the N atom is optionally protected by an alkyl group or a protecting group of amino group; alkylcarbonyl groups such as a methylcarbonyl group and an ethylcarbonyl group; alkenylcarbonyl groups such as an allylcarbonyl group; alkynylcarbonyl groups such as a propargylcarbonyl group; arylcarbonyl groups such as a phenylcarbonyl group; heteroarylcarbonyl groups such as a pyridine-2, 3, or 4-ylcarbonyl group; heterocyclylcarbonyl groups such as a tetrahydrofuran-2 or 3-ylcarbonyl group and a 5-oxotetrahydrofuran-2-ylcarbonyl group; silyl groups such as a trimethylsilyl group, a trimethylsilylethoxymethyl group, a triisopropylsilyl group, a tert-butyldimethylsilyl group, and a tert-butyldiphenylsilyl group; or arylsulfonyl groups such as a 2,4-dinitrobenzenesulfonyl group and a 4-nitrobenzenesulfonyl group; and protecting groups of amino group usually used in the peptide synthesis. Also, said amino group is optionally protected as an amide with formic acid, phosphoric acid, phosphonic acid, phosphinic acid, sulfuric acid, sulfonic acid, sulfinic acid, sulfenic acid, an amino acid, or a peptide.

In one embodiment, the protecting group of amino group is an alkyloxycarbonyl group; an allyloxycarbonyl group; an arylmethyloxycarbonyl group; an alkylcarbonyl group; an alkenylcarbonyl group; an arylcarbonyl group; a silyl group, a lactonyl group; a lactonylalkyl group; a lactonylcarbonyl group; a lactonylalkylcarbonyl group; a phosphoryl group; a phosphorylalkyl group; a phosphorylalkylcarbonyl group; a bisphosphorylmethyl group (-C(H, OH, $NH_2$, Cl, or alkyl)$(P(=O)(OH)_2)_2$); an aminoalkylcarbonyl group; an alkylaminoalkylcarbonyl group; a dialkylaminoalkylcarbonyl group; a cyclic aminoalkylcarbonyl group formed by combining said dialkyl group; or a trialkylammoniumalkylcarbonyl group.

The protection by or removal of these protecting groups is carried out according to a method usually used (for example, see Greene's Protective Groups in Organic Synthesis 5th Edition, P. G. M. Wuts, JohnWiley & Sons Inc. (2014)).

The functional groups may be protected as glycosides by reacting with various sugars as described above. That is, they may be protected by glycosyl groups. Specific examples of glycosyl groups include a D-glucosyl group, a D-galactosyl group, a D-mannosyl group, and a D-glucuronosyl group, and functional groups (-OH, -SH, $-NH_2$, -NH-, and -COOH groups, etc.) of these sugar moieties may also be protected as described above. These glycosides and their protected forms can be produced according to conventional methods (for example, Comprehensive Glycoscience From Chemistry to Systems Biology, Hans Kamerling (2007)) commonly used in the field of glycochemistry.

Moreover, among said functional groups, a hydroxy group, a sulfhydryl group (-SH), and an amino group ($-NH_2$ and -NH- (also including -NH- in an amide group and -NH- in the ring of a heteroaryl group or a heterocyclyl group)) are also optionally protected as an ester, a thio ester, an amide, or an imide, respectively, with various sugar acids (for example, gluconic acid, glucuronic acid, and galacturonic acid).

The "protecting groups" of various functional groups in the "derivative" in "an antitumor drug molecule or an analog thereof, or a derivative thereof" as described herein are described above, and they can also be applied to linker moieties, a bonding group,, a protecting group, or a substituent.

The methods for selecting, attaching, and detaching said protecting groups are also applied to the production of the following compounds of General Formulae (I), (II-1), and (II-2) (that is, the conjugate precursor and the conjugate precursor synthesis intermediates).

Specific examples of the "antitumor drug molecule or an analog thereof, or a derivative thereof" as described herein are shown in Exemplification 1 below, but the present invention is not limited thereto.

Exemplification 1. Exemplification of "antitumor drug molecule or analog thereof, or derivative thereof"

[C56]

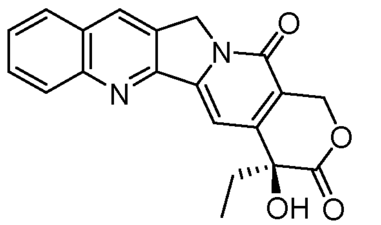

camptothecin

[C57]

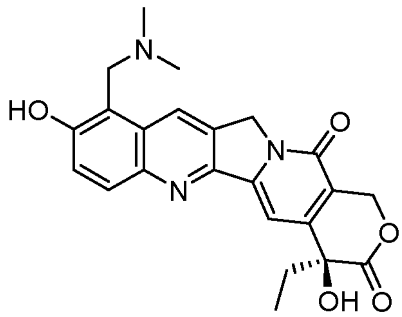

topotecan

[C58]

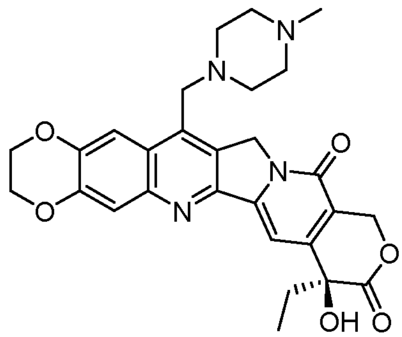

lurtotecan

[C59]

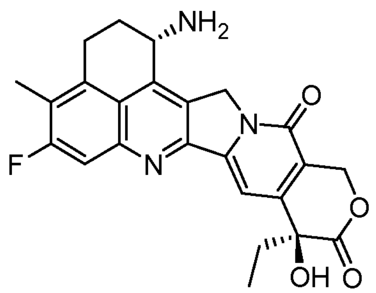

exatecan

[C60]

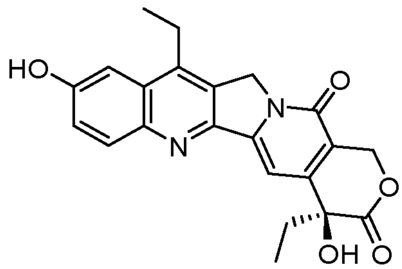

SN-38

[C61]

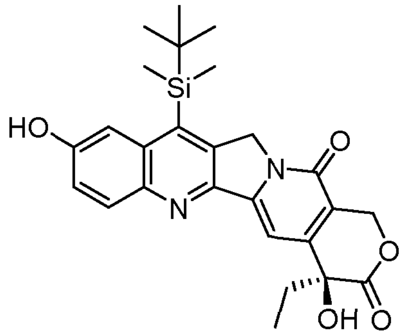

DB-67

[C62]

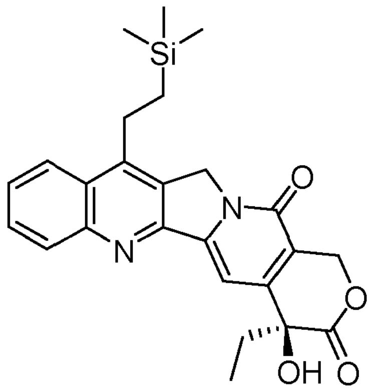

BNP-1350

[C63]

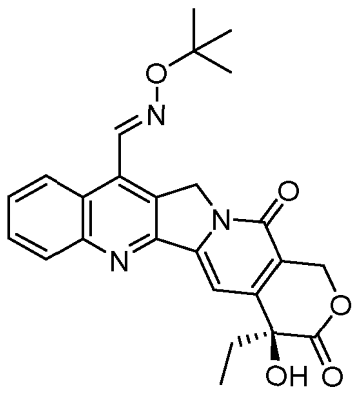

ST-1481

[C64]

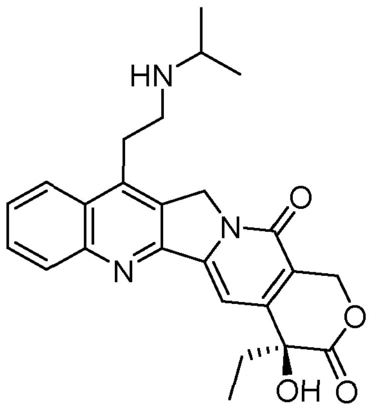

CKD-602

[C65]

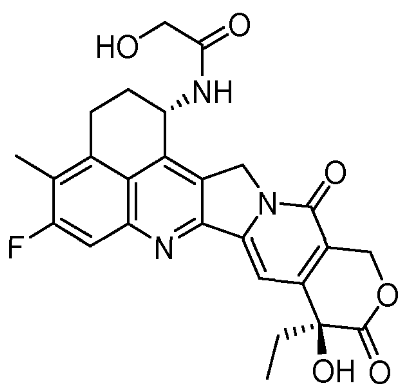

Dxd(N-(2-hydroxyacetyl)exatecan)

[C66]

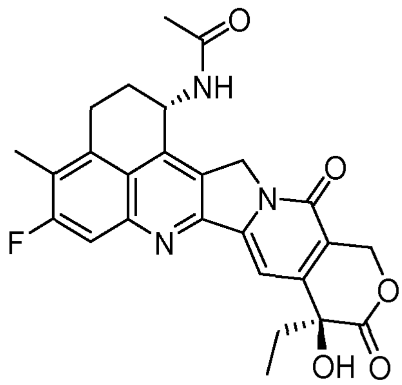

U-001

[C67]

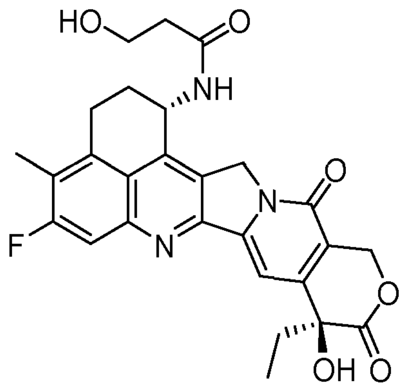

U-002

[C68]

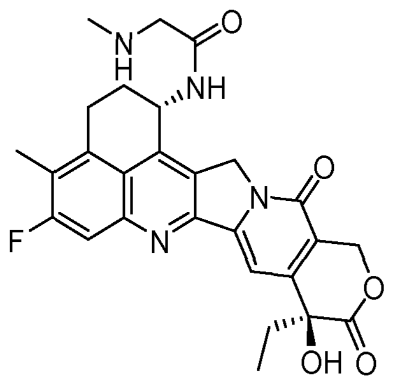

U-003

[C69]

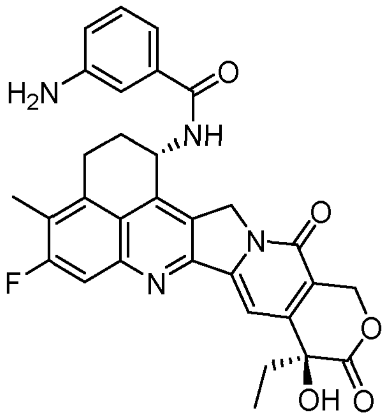

U-004

[C70]

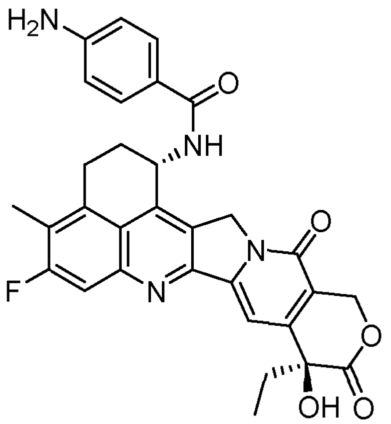

U-005

[C71]

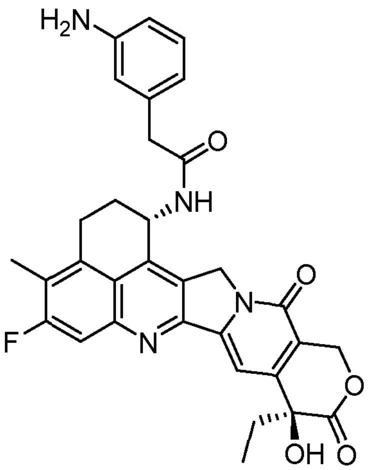

U-006

[C72]

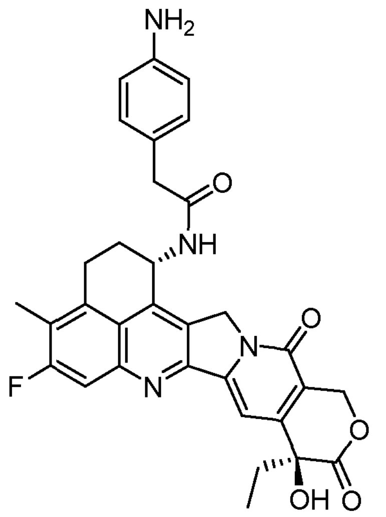

U-007

[C73]

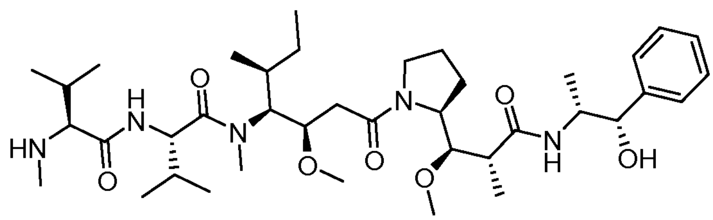

MMAE (monomethyl auristatin E)

[C74]

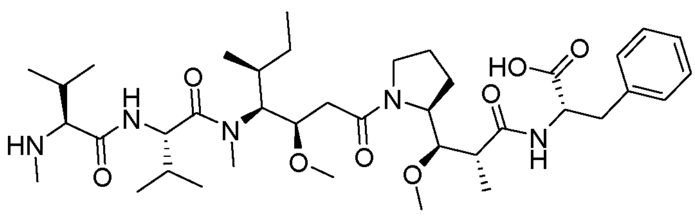

MMAF(monomethyl auristatin F)

[C75]

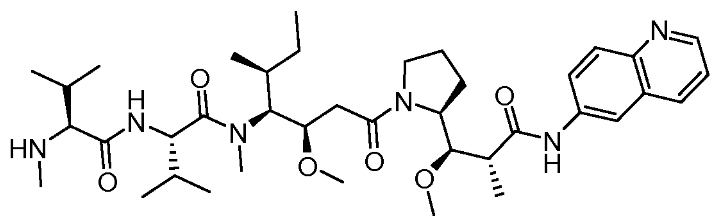

MMAQ(monomethyl auristatin Q)

[C76]

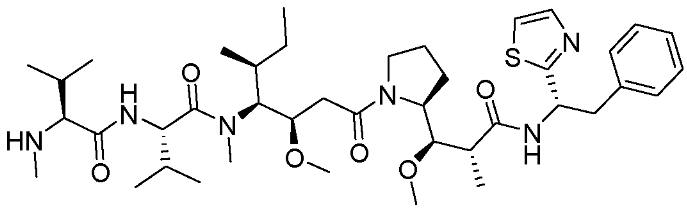

MMAD(monomethyl dolastatin 10)

[C77]

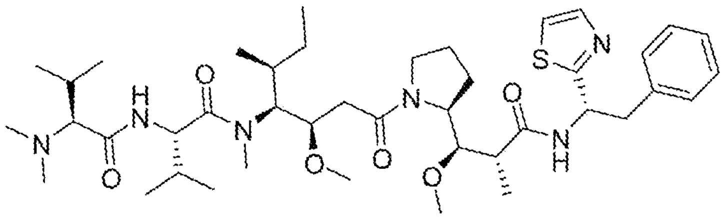

dolastatin 10

[C78]

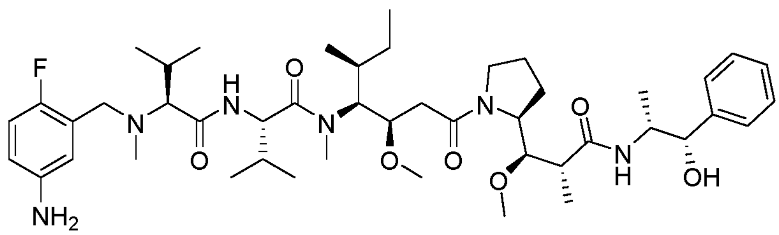

U-008

[C79]

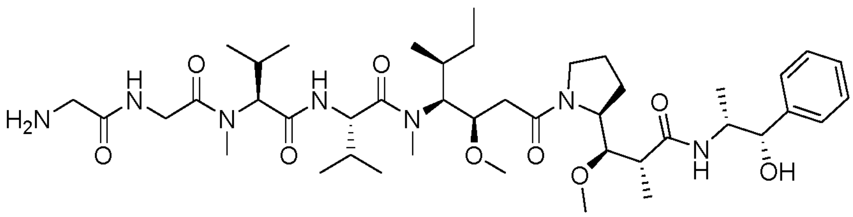

U-009

[C80]

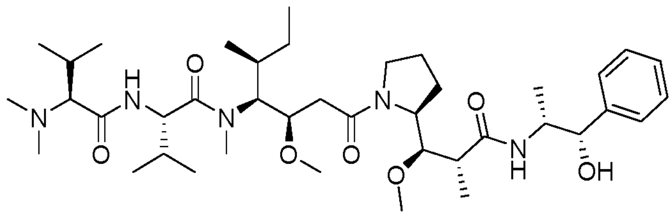

U-010

[C81]

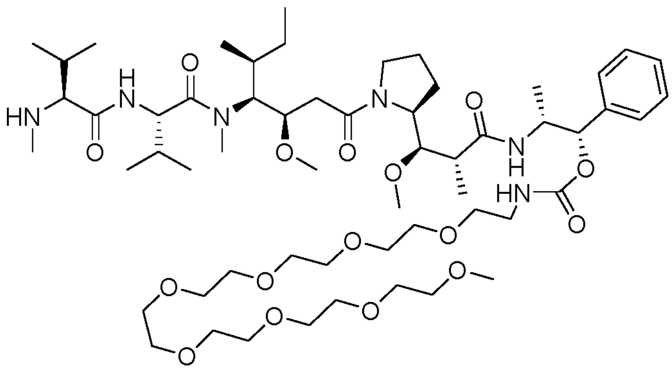

U-011

[C82]

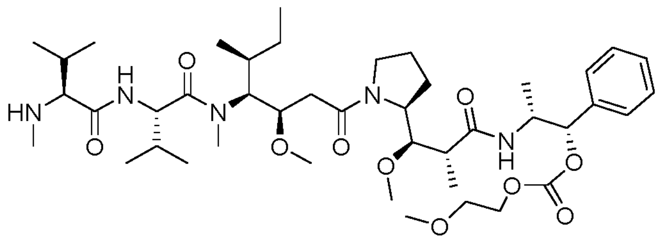

U-012

[C83]

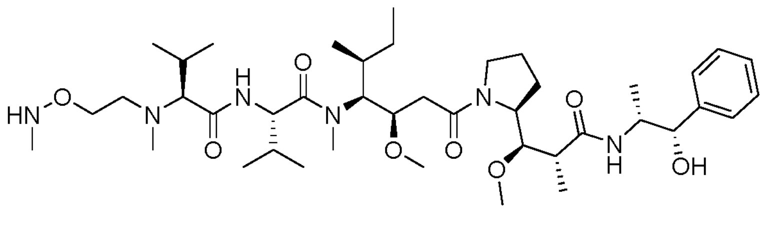

U-013

[C84]

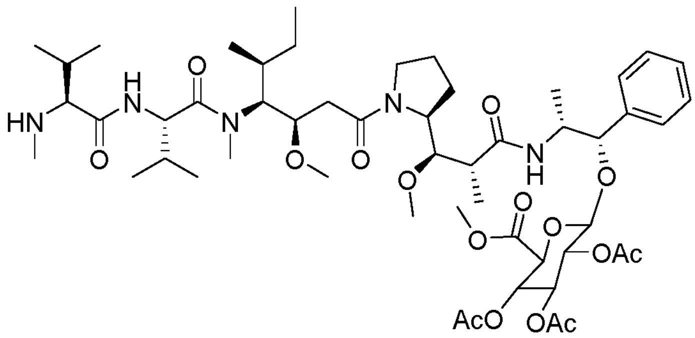

U-014

[C85]

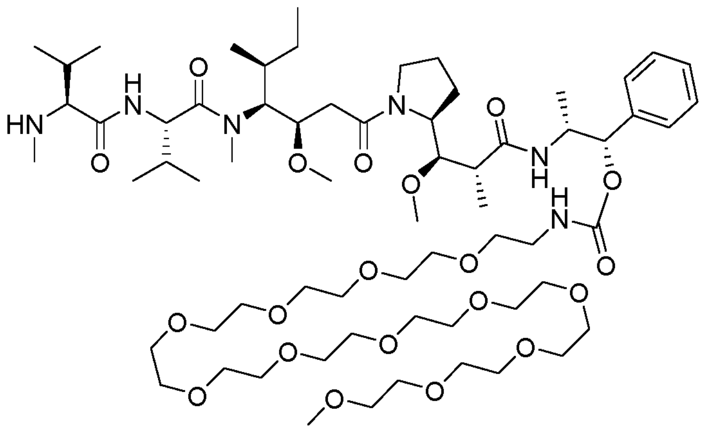

U-015

[C86]

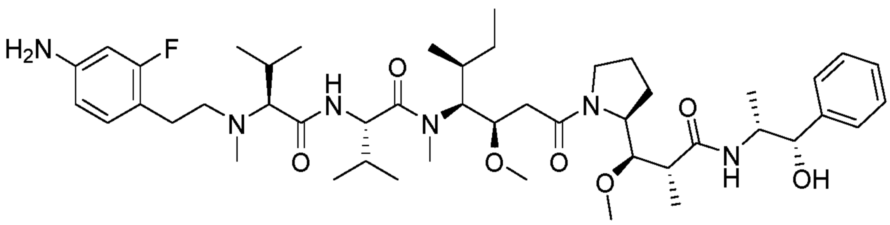

U-016

[C87]

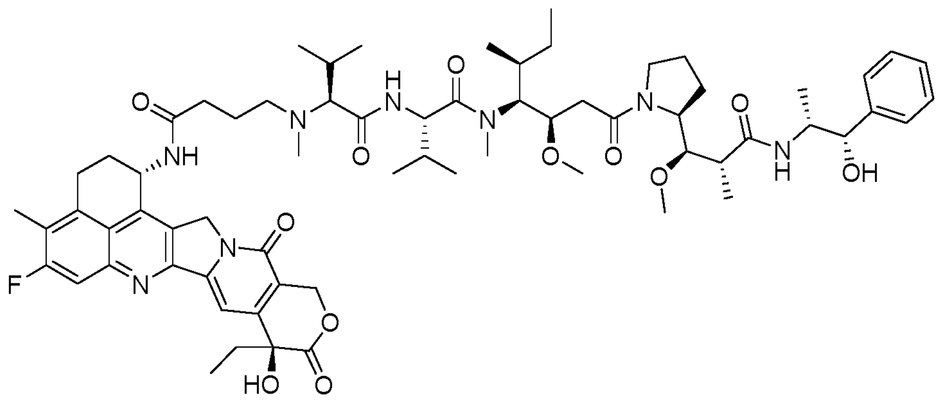

U-017

[C88]

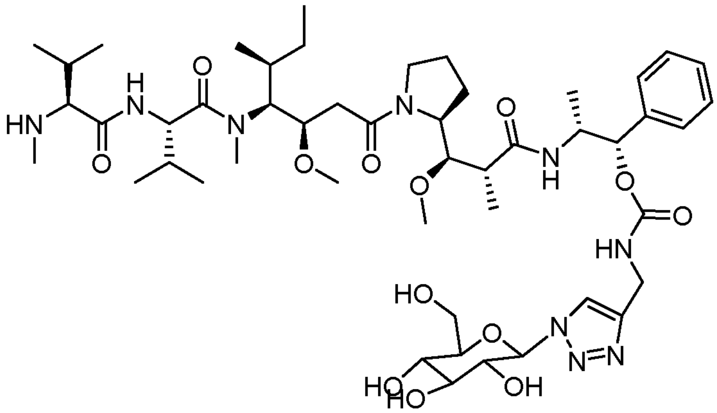

U-018

[C89]

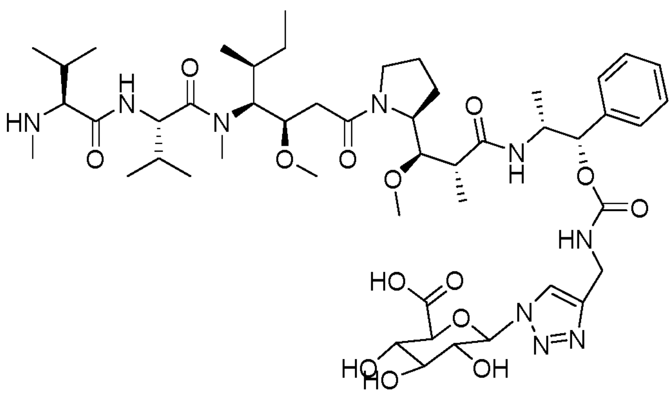

U-019

[C90]

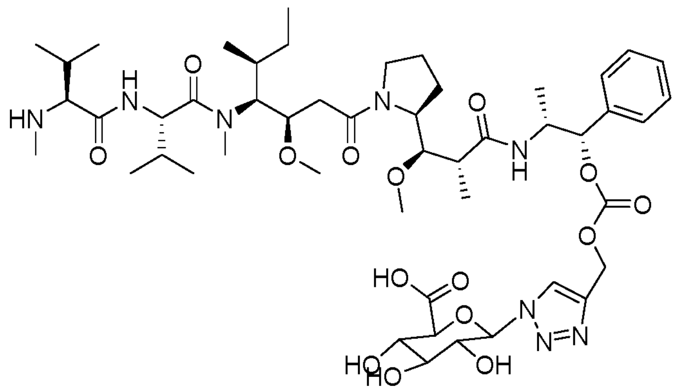

U-020

[C91]

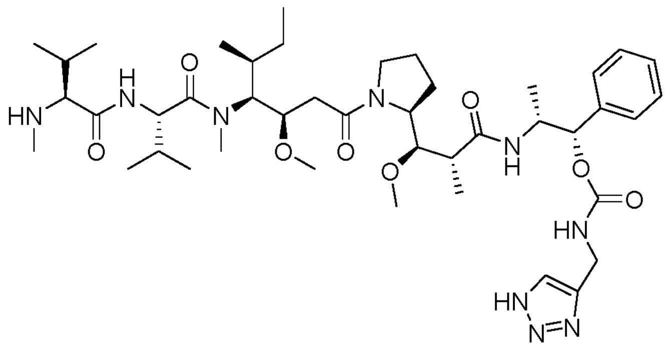

U-021

[C92]

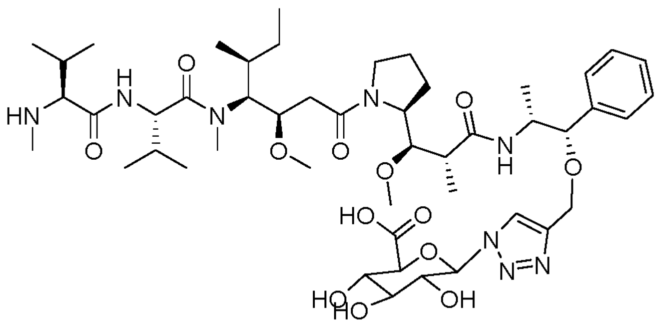

U-022

[93]

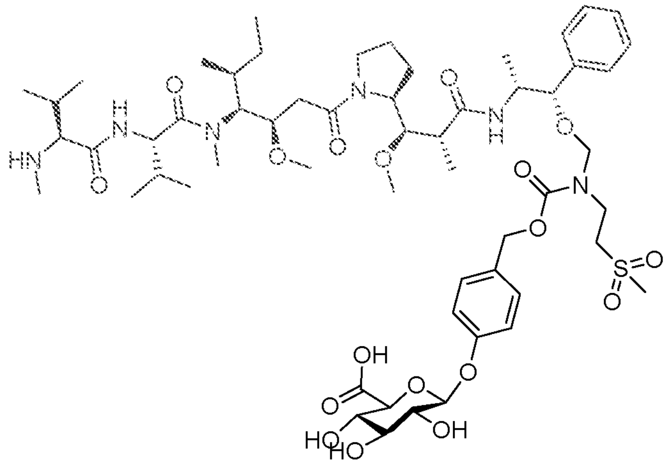

U-023

[C94]

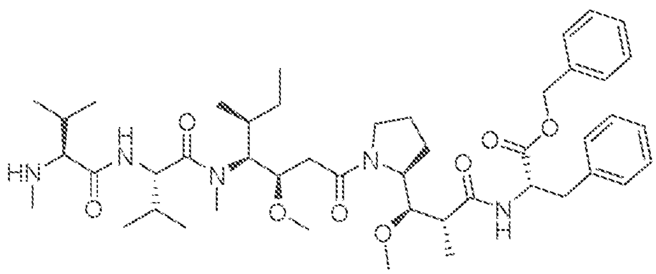

U-024

[C95]

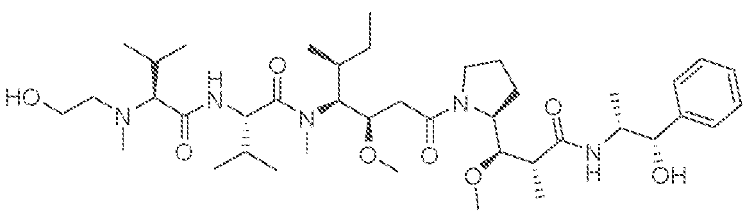

U-025

[C96]

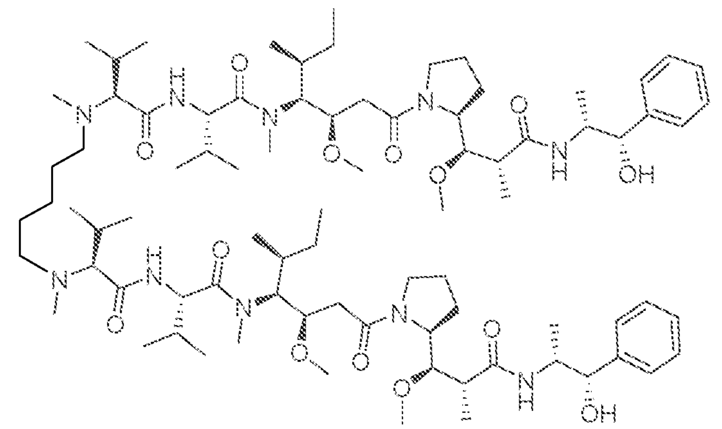

U-026

[C97]

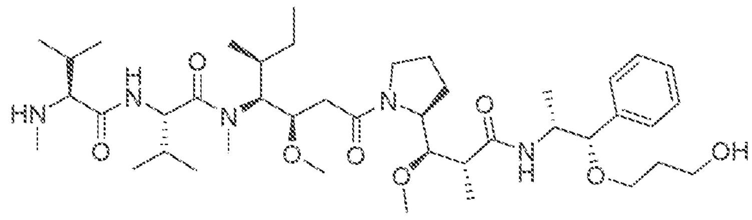

U-027

[C98]

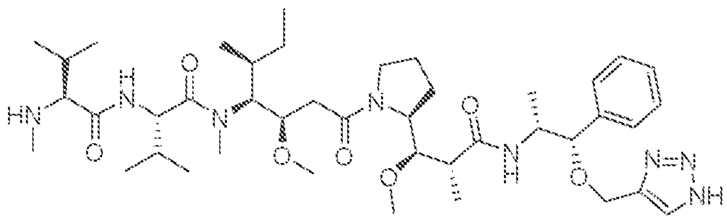

U-028

[C99]

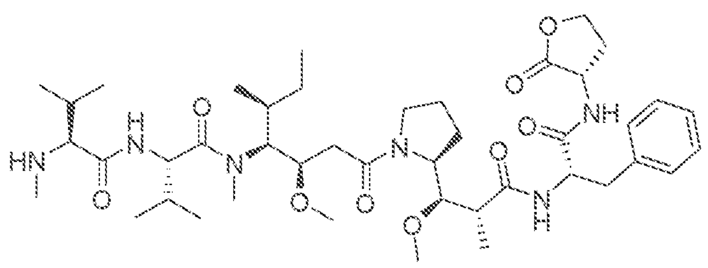

U-029

[C100]

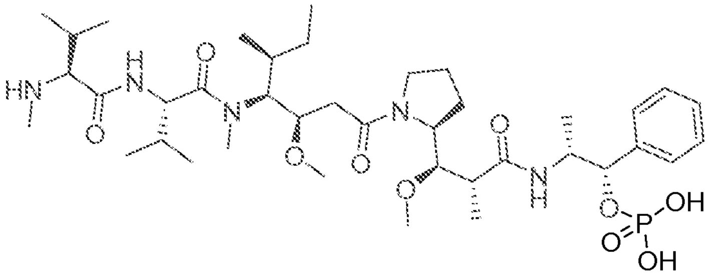

U-030

[C101]

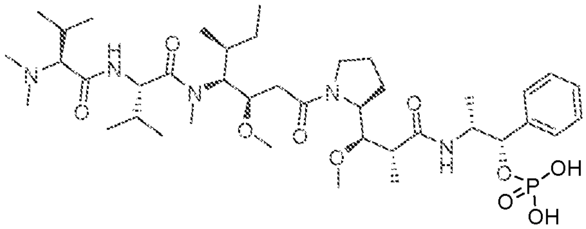

U-031

[C102]

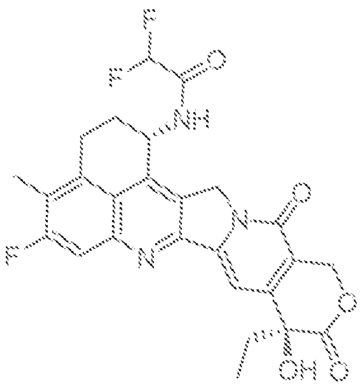

U-032

[C103]

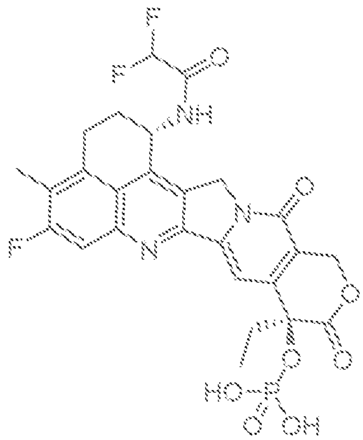

U-033

[C104]

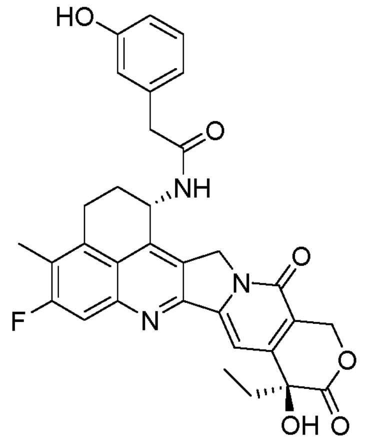

U-034

[C105]

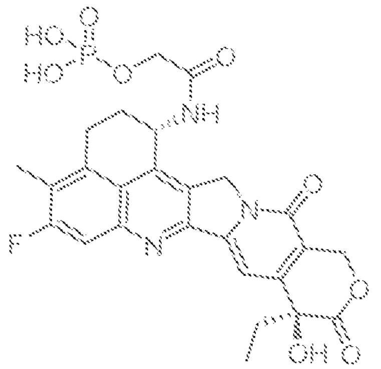

U-035

[C106]

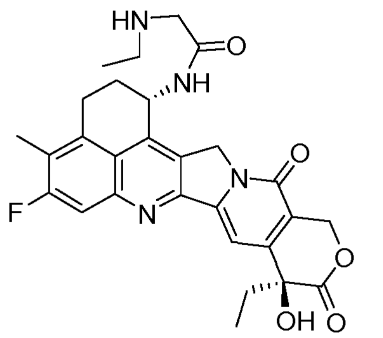

U-036

[C107]

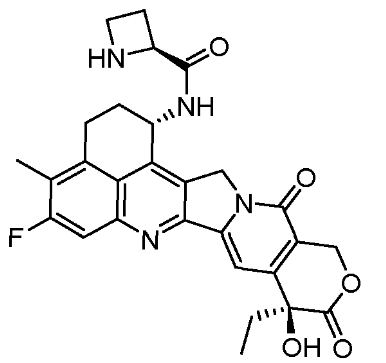

U-037

[C108]

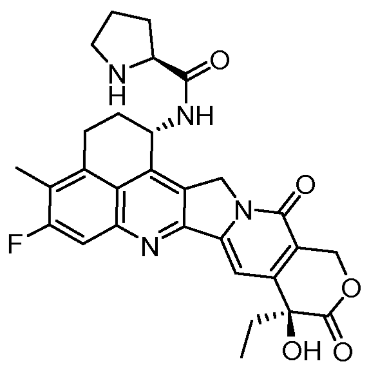

U-038

[C109]

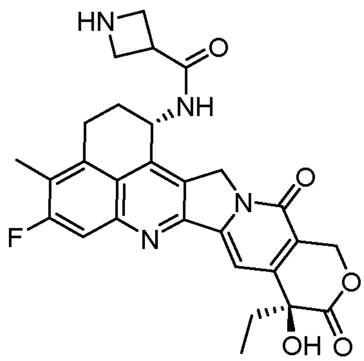

U-039

[C110]

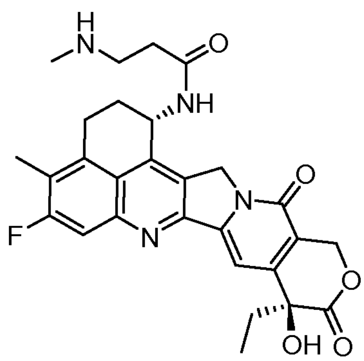

U-040

[C111]

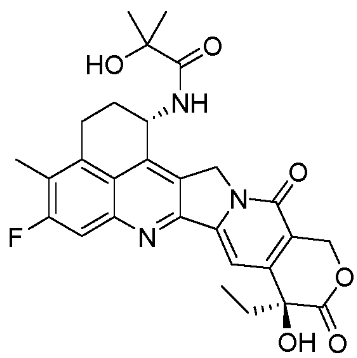

U-041

[C112]

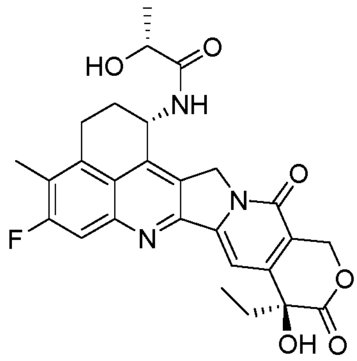

U-042

[C113]

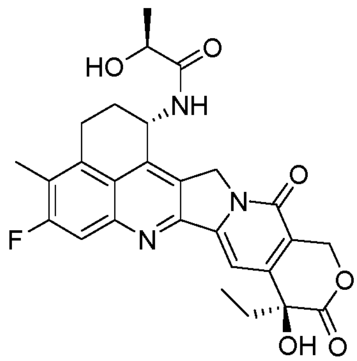

U-043

[C114]

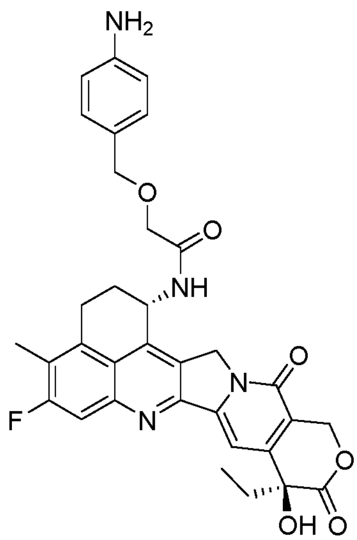

U-044

[C115]

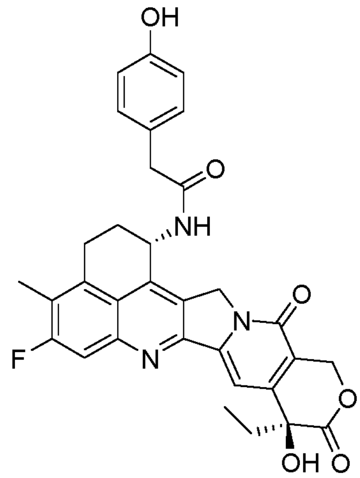

U-045

[C116]

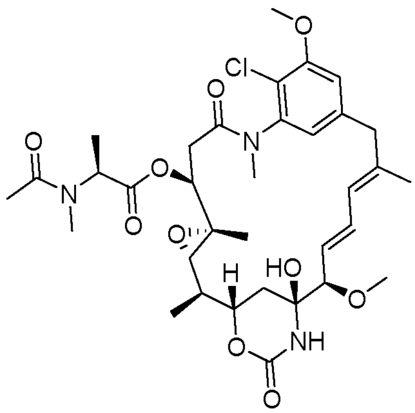

maytansine

[C117]

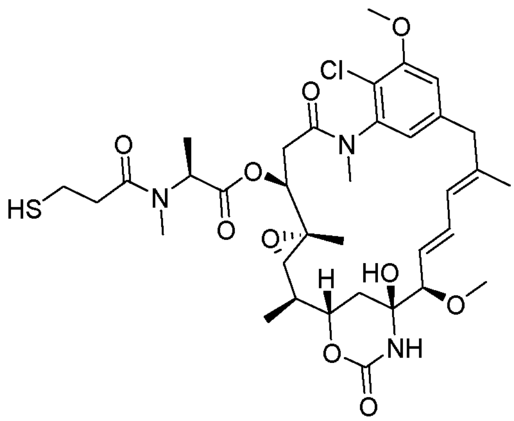

DM-1 (mertansine)

[C118]

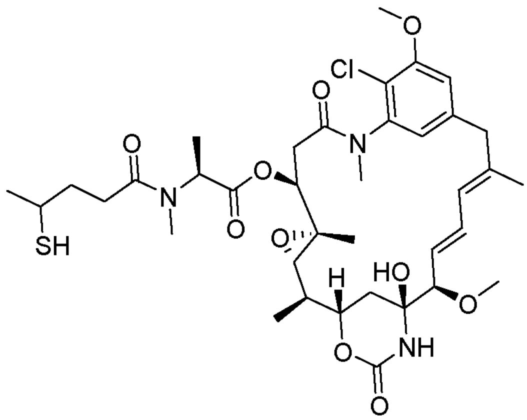

DM-3

[C119]

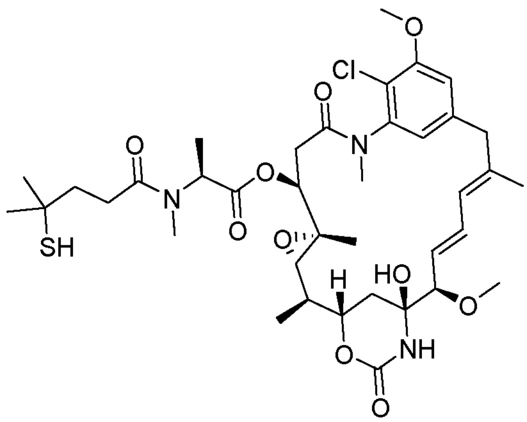

DM-4

[C120]

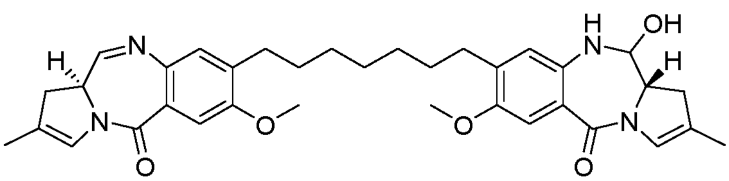

PBD dimer

[C121]

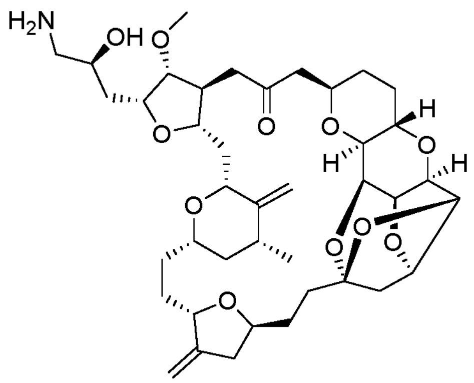

eribulin

[C122]

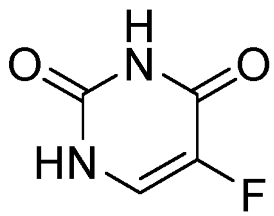

5-FU

[C123]

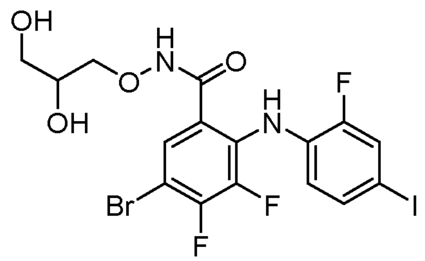

PD-318088

[C124]

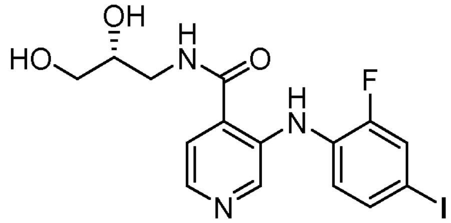

AS-703026

[C125]

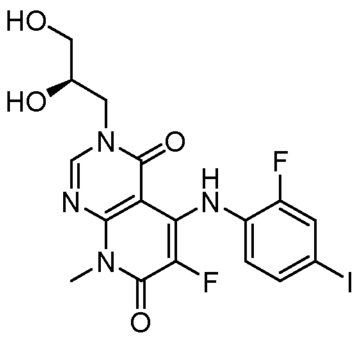

TAK-733

[C126]

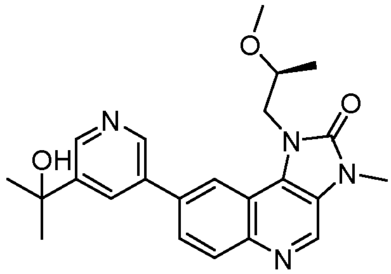

LY-3023414

[C127]

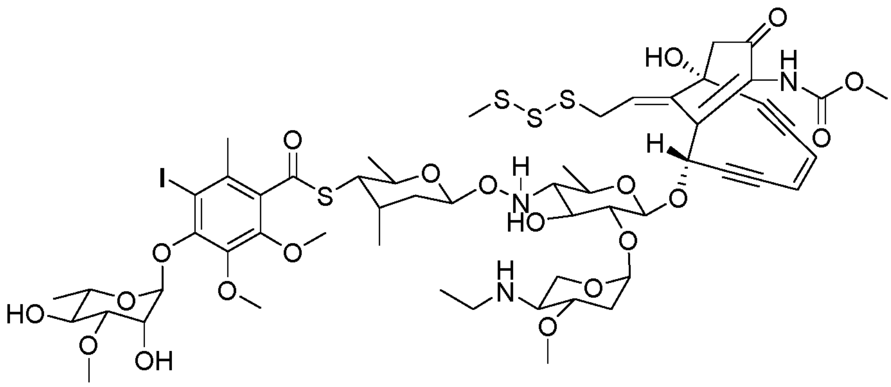

calicheamicin gamma 1

[C128]

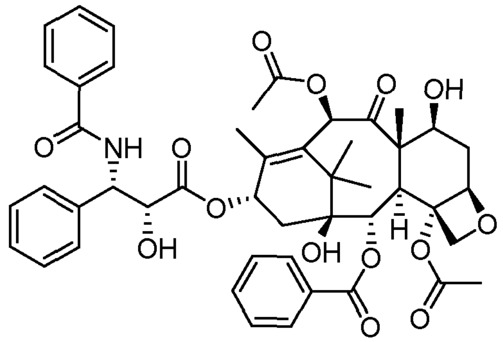

paclitaxel

[C129]

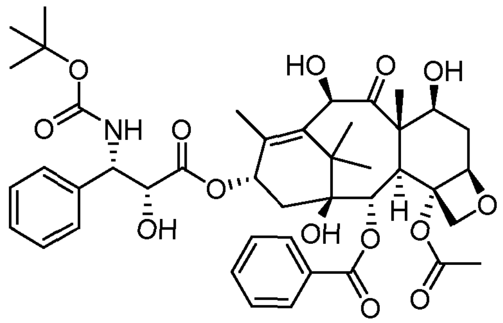

docetaxel

[C130]

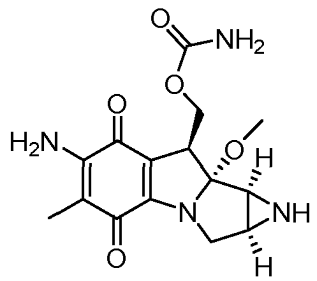

mitomycin C

[C131]

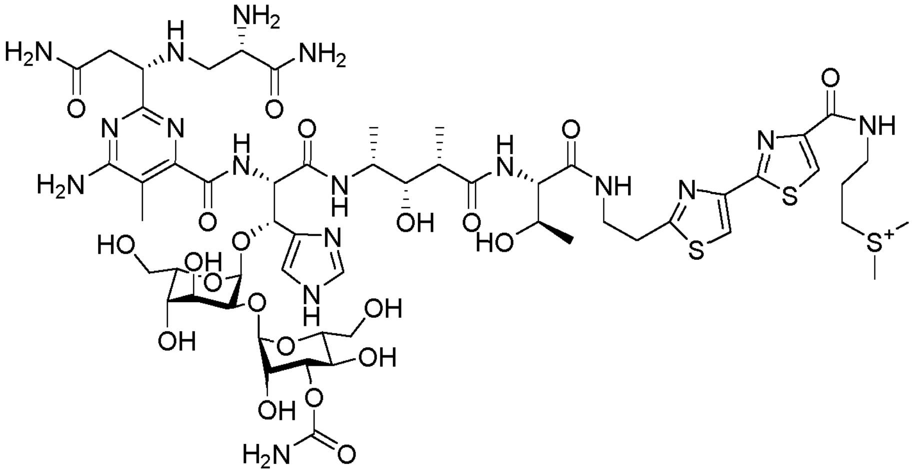

bleomycin A2

[C132]

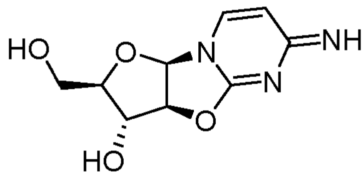

cyclocytidine

[C133]

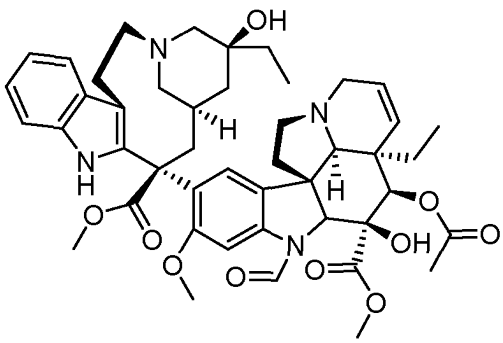

vincristine

[C134]

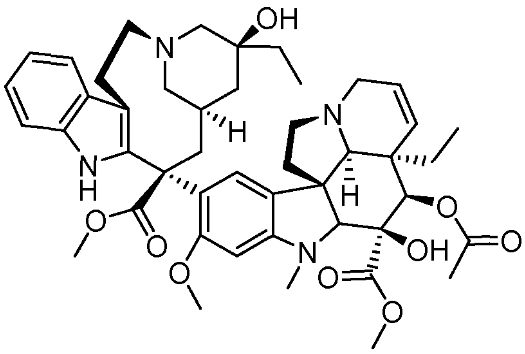

vinblastine

[C135]

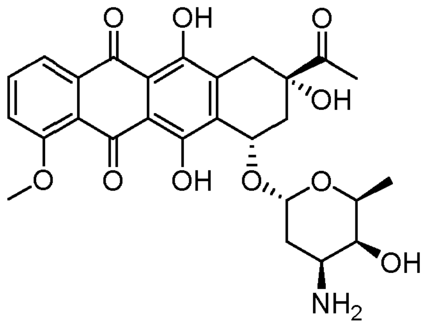

daunomycin

[C136]

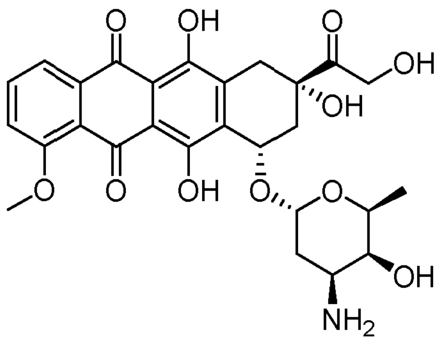

doxorubicin

[C137]

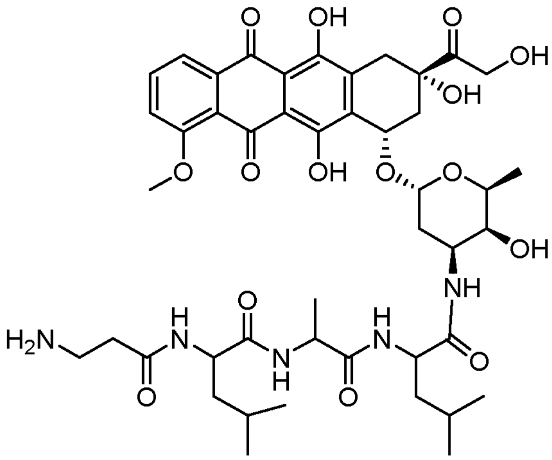

superdox

[C138]

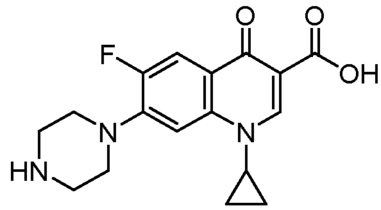

ciprofloxacin

[C139]

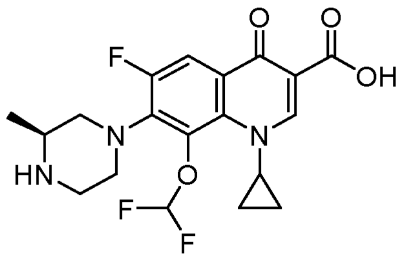

cadrofloxacin (CS-940)

[C140]

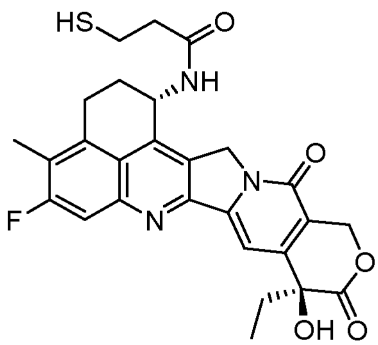

U-046

[C141]

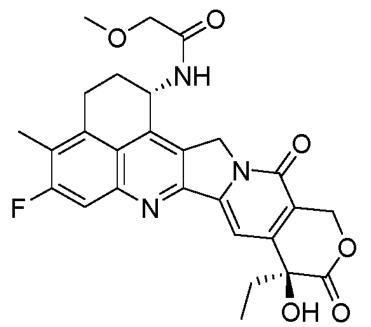

U-047

[C142]

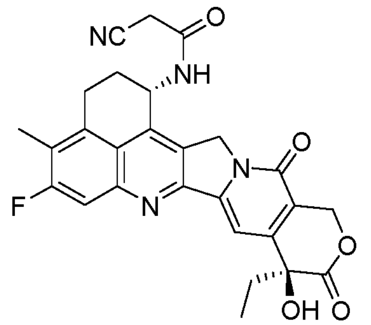

U-048

[C143]

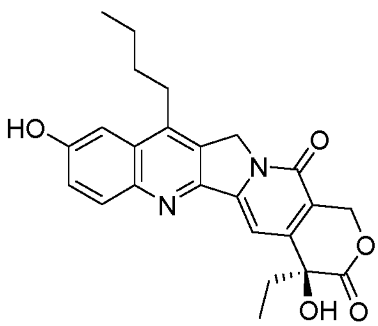

U-049

[C144]

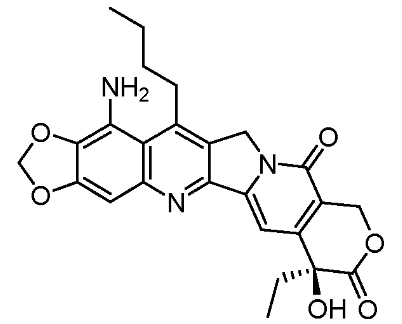

U-050

[C145]

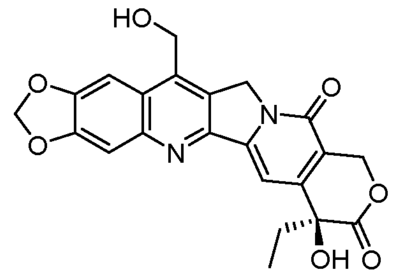

U-051

[C146]

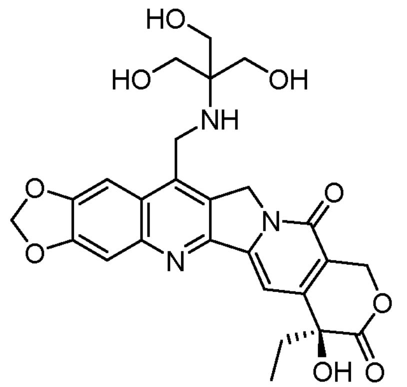

U-052

[C147]

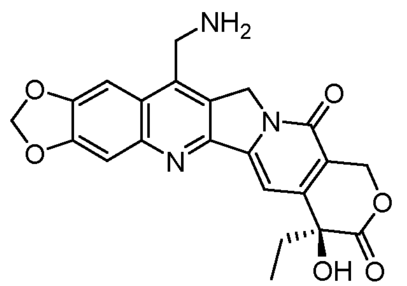

U-053

[C148]

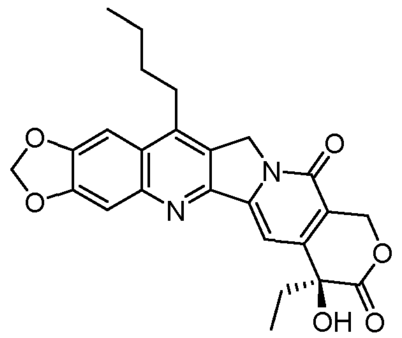

U-054

[C149]

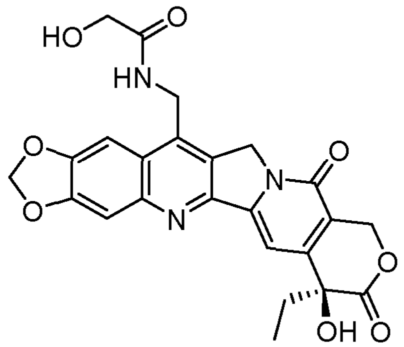

U-055

[C150]

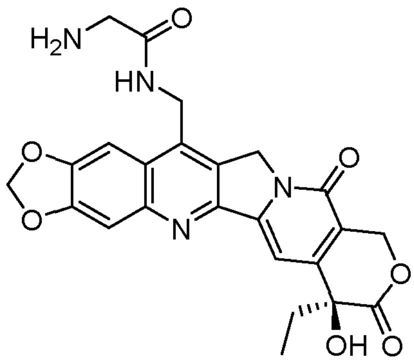

U-056

[C151]

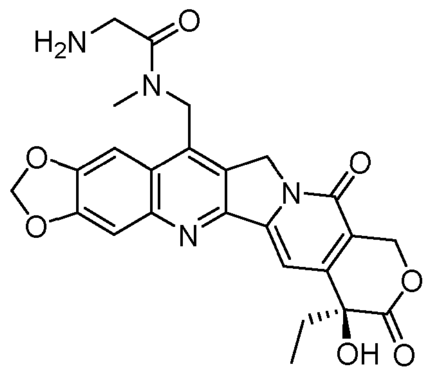

U-057

[C152]

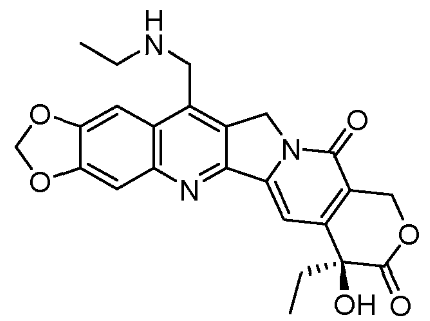

U-058

[C153]

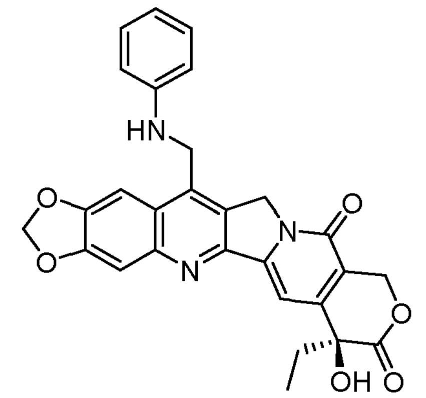

U-059

[C154]

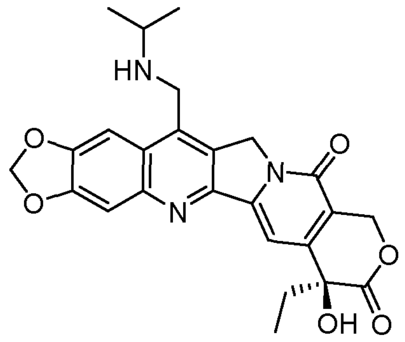

U-060

[C155]

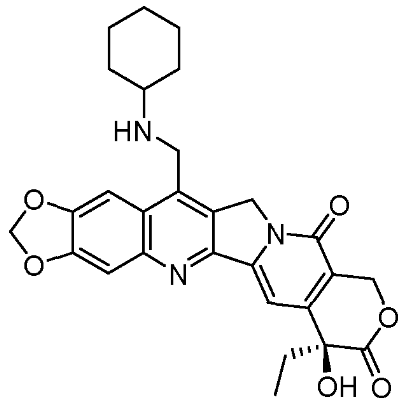

U-061

[C156]

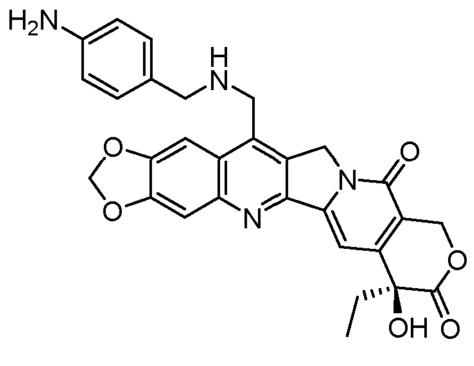

U-062

[C157]

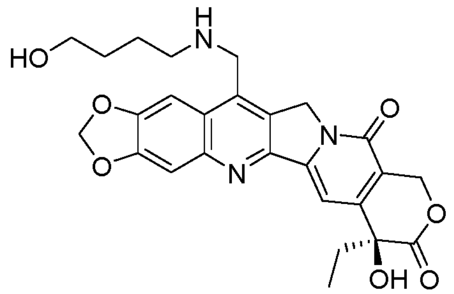

U-063

[C158]

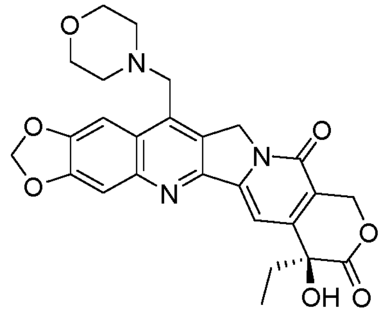

U-064

[C159]

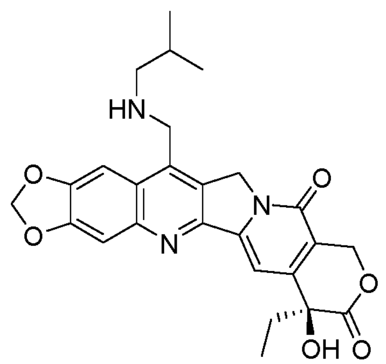

U-065

[C160]

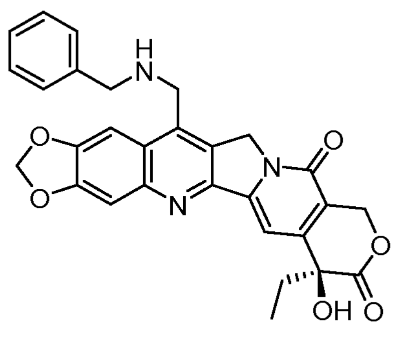

U-066

[C161]

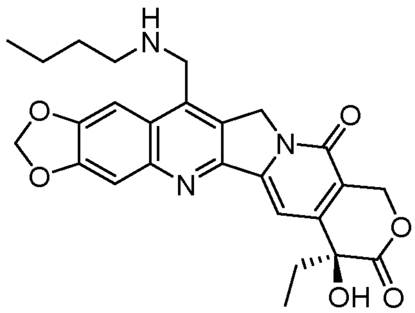

U-067

[C162]

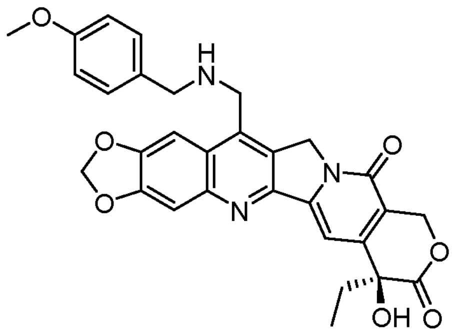

U-068

[C163]

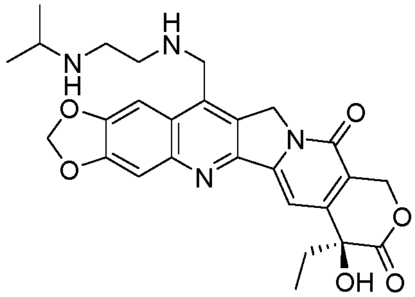

U-069

[C164]

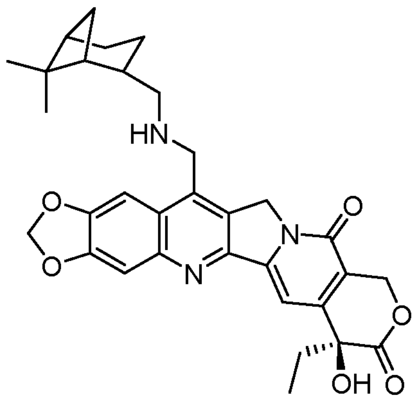

U-070

[C165]

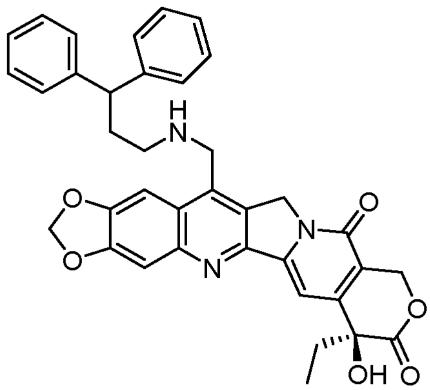

U-071

[C166]

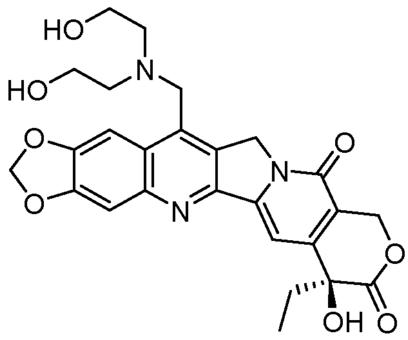

U-072

[C167]

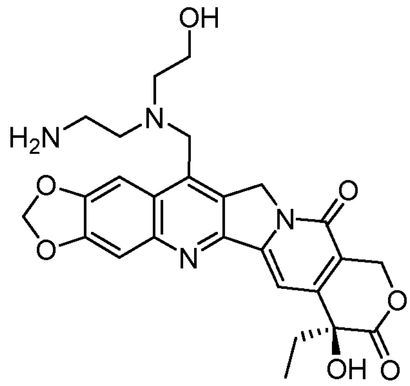

U-073

[C168]

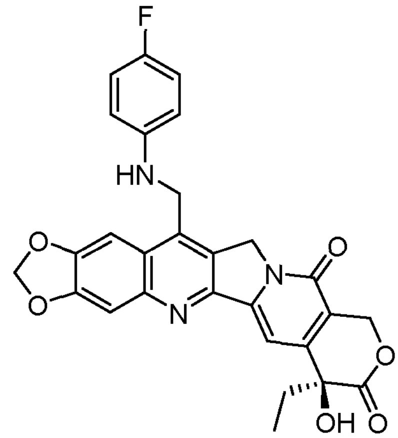

U-074

[C169]

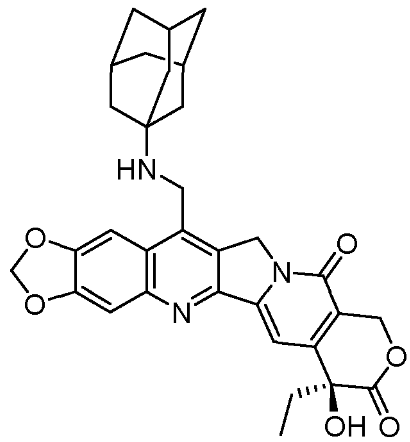

U-075

[C170]

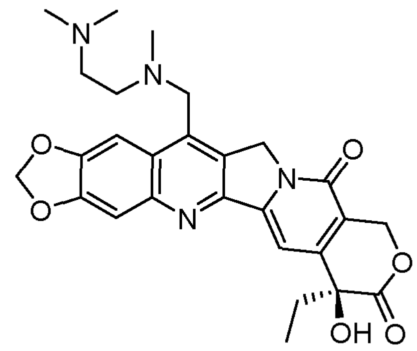

U-076

[C171]

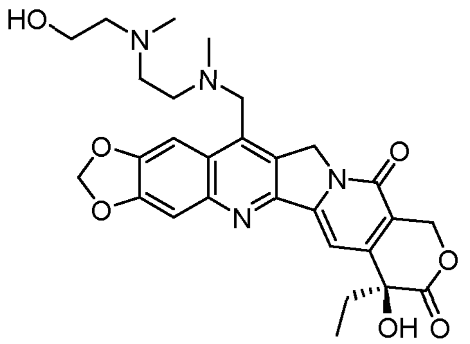

U-077

[C172]

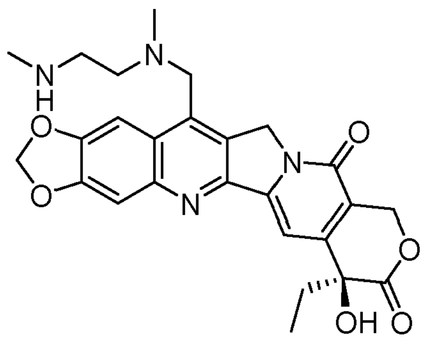

U-078

[C173]

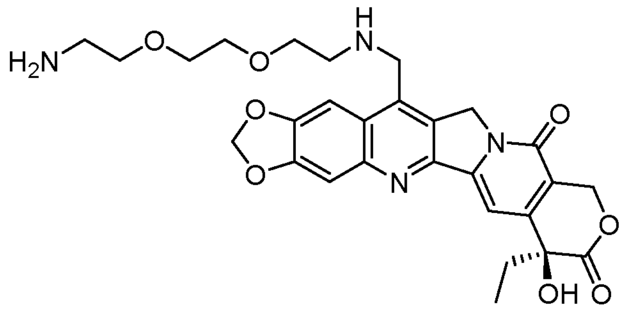

U-079

[C174]

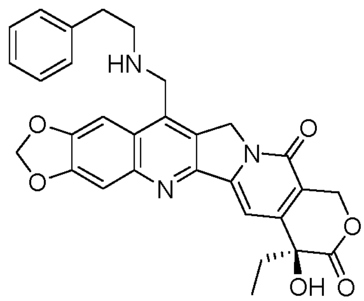

U-080

[C175]

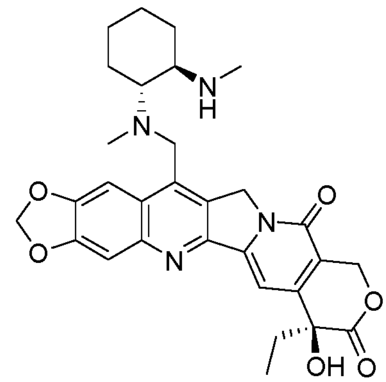

U-081

[C176]

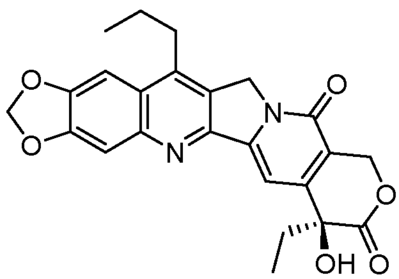

U-082

[C177]

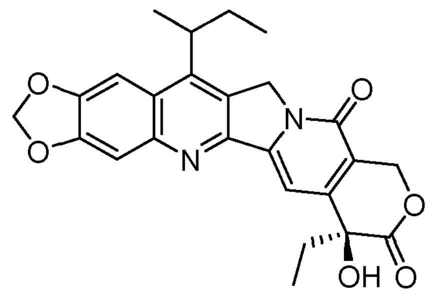

U-083

[C178]

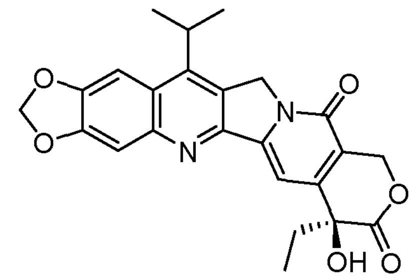

U-084

[C179]

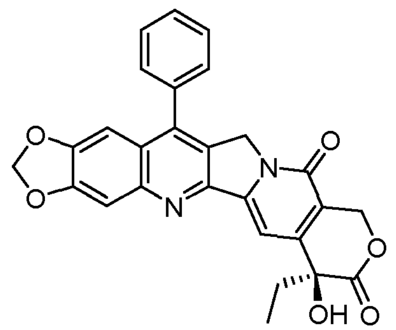

U-085

[C180]

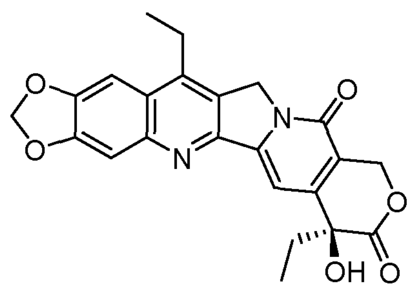

U-086

[C181]

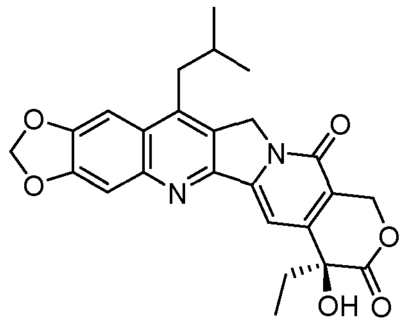

U-087

[C182]

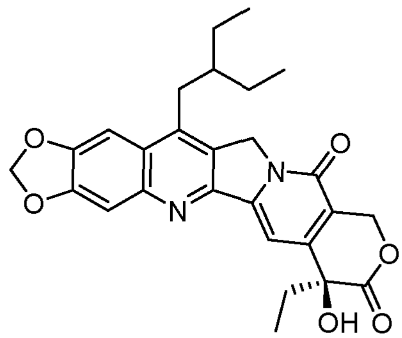

U-088

[C183]

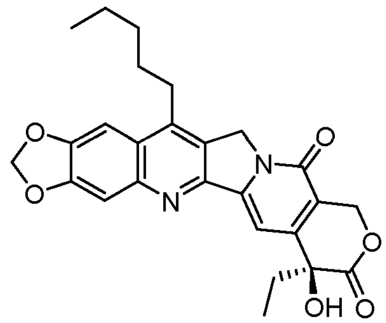

U-089

[C184]

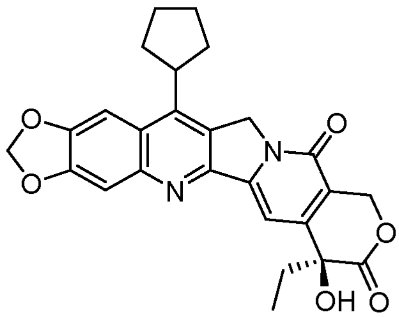

U-090

[C185]

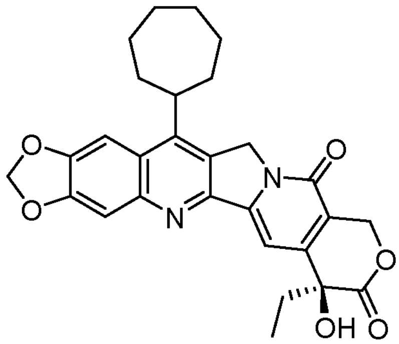

U-091

[C186]

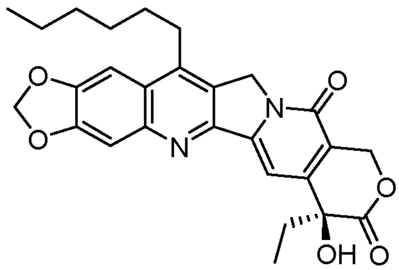

U-092

[C187]

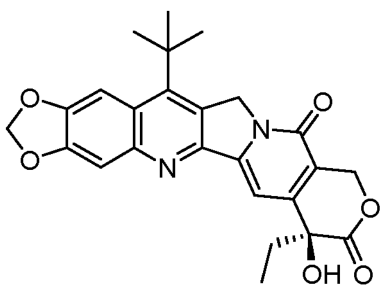

U-093

[C188]

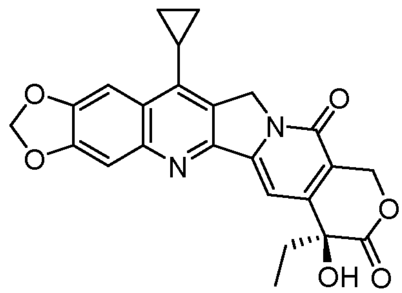

U-094

[C189]

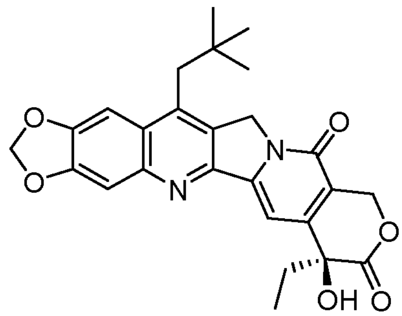

U-095

[C190]

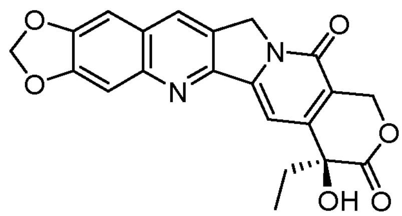

U-096

[C191]

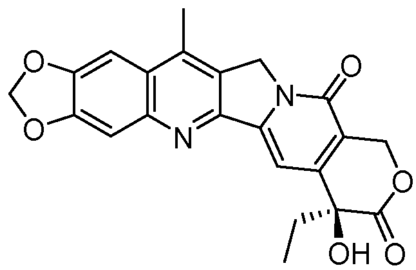

U-097

[C192]

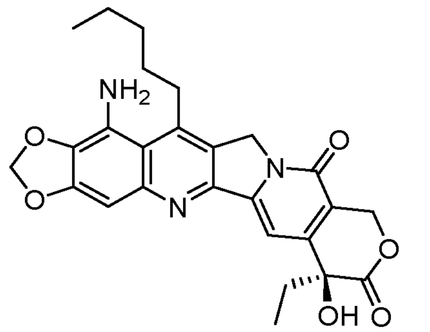

U-098

[C193]

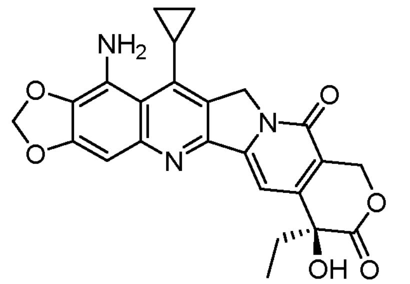

U-099

[C194]

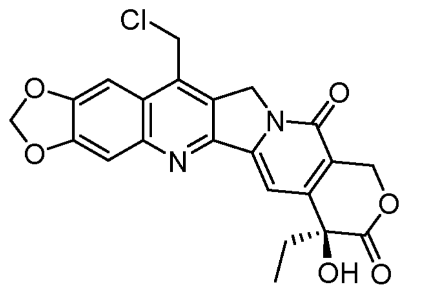

U-100

[C195]

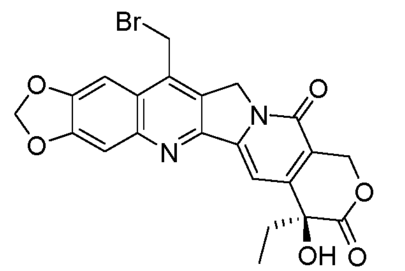

U-101

[C196]

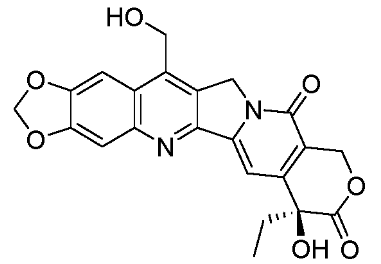

U-102

[C197]

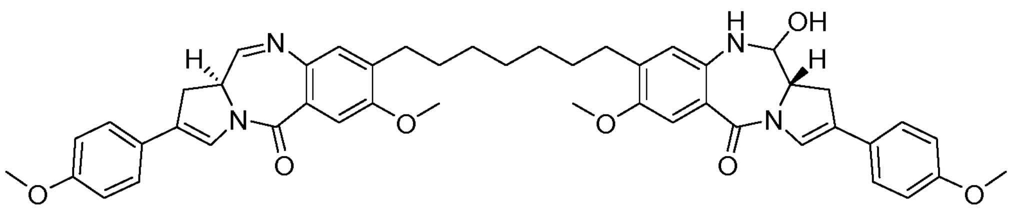

U-103

[C198]

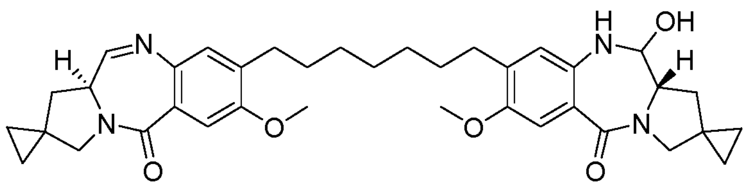

U-104

The term "active ester of a carboxy group" as described herein refers to one obtained by making a carboxy group into an ester with highly acidic alcohols to enhance its reactivity, and examples thereof include N-hydroxysuccinimidyl ester, sulfosuccinimidyl ester, N-hydroxyphthalimidyl ester, N-hydroxysulfophthalimidyl ester, ortho-nitrophenyl ester, para-nitrophenyl ester, 2,4-dinitrophenyl ester, 3-sulfonyl-4-nitrophenyl ester, 3-carboxy-4-nitrophenyl ester, and a pentafluorophenyl ester. However, the present invention is not limited thereto.

The term "alkyl group" as described herein refers to a group in which one hydrogen atom is removed from a straight or branched saturated hydrocarbon (i.e., alkane) having 1 to 10 carbon atom(s). Further, even if it is not specified, said alkyl group also encompasses "an optionally substituted alkyl group".

Examples of the substituent of an optionally substituted alkyl group include one or more substituent(s) independently selected from the following Group A.

Group A

a group consisting of:

halogen atom (fluorine atom, chlorine atom, bromine atom, or iodine atom);
alkyloxy group;
hydroxy group;
sulfhydryl group;
amino group;
alkylamino group;
dialkylamino group (wherein two alkyl groups are optionally combined to form a heterocyclyl group containing an N atom);
trialkyl ammonium group;
carboxy group;
cyano group;

nitro group;
alkylcarbonyl group;
alkenylcarbonyl group;
alkynylcarbonyl group;
arylcarbonyl group;
heteroarylcarbonyl group;
heterocyclylcarbonyl group;
lactonylcarbonyl group;
phosphorylalkylcarbonyl group;
alkyloxycarbonyl group;
aryloxycarbonyl group;
heteroaryloxycarbonyl group;
aminocarbonyl group;
aminocarbonyl group wherein the amino moiety is an amino group of any amino acid or peptide;
N-alkylaminocarbonyl group;
N,N-dialkylaminocarbonyl group (wherein two alkyl groups are optionally combined to form a heterocyclyl group containing an N atom);
aminocarbonyloxy group;
N-alkylaminocarbonyloxy group;
N,N-dialkylaminocarbonyloxy group (wherein two alkyl groups are optionally combined to form a heterocyclyl group containing an N atom);
alkyloxycarbonyloxy group;
aryloxycarbonyloxy group;
heteroaryloxycarbonyloxy group;
ureido group;
N-alkylureido group;
N,N-dialkylureido group (wherein two alkyl groups are optionally combined to form a heterocyclyl group containing an N atom);
N,N,N'-trialkylureido group (wherein two alkyl group(s) on the same N atom are optionally combined to form a heterocyclyl group containing an N atom);
guanidino group;
amidino group;
hydrazino group;
alkylhydrazino group;
N,N-dialkylhydrazino group;
N,N'-dialkylhydrazino group;
alkylthio group;
alkylsulfinyl group;
alkylsulfonyl group;
arylthio group;
arylsulfinyl group;
arylsulfonyl group;
heteroarylthio group;
heteroarylsulfinyl group;
heteroarylsulfonyl group;
sulfenic acid group;
sulfinic acid group;
sulfonic acid group;
sulfuric acid group;
sulfinamide group;
sulfonamide group;
phosphine oxide group;
phosphinic acid group;
phosphoryl group (phosphonic acid group $-P(=O)(OH)_2$);
phosphoric acid group (phosphate group $-O-P(=O)(OH)_2$);
boric acid group;
alkenyl group;
alkynyl group;

cycloalkyl group;
cycloalkenyl group;
aryl group;
heteroaryl group;
heterocyclyl group;
lactonyl group;
and Y is:

- hydrogen atom;
- alkyl group;
- halogen atom (fluorine atom, chlorine atom, bromine atom, or iodine atom);
- alkyloxy group;
- alkylthio group;
- hydroxy group;
- sulfhydryl group;
- amino group;
- alkylamino group;
- dialkylamino group (wherein two alkyl groups are optionally combined to form a heterocyclyl group containing an N atom);
- carboxy group;
- trialkyl ammonium group;
- cyano group;
- nitro group;
- alkylcarbonyl group;
- alkenylcarbonyl group;
- alkynylcarbonyl group;
- arylcarbonyl group;
- heteroarylcarbonyl group;
- heterocyclylcarbonyl group;
- lactonylcarbonyl group;
- phosphorylalkylcarbonyl group;
- alkyloxycarbonyl group;
- aryloxycarbonyl group;
- heteroaryloxycarbonyl group;
- aminocarbonyl group;
- aminocarbonyl group wherein the amino group moiety is an amino group of any amino acid or peptide;
- N-alkylaminocarbonyl group;
- N,N-dialkylaminocarbonyl group (wherein two alkyl groups are optionally combined to form a heterocyclyl group containing an N atom);
- aminocarbonyloxy group;
- N-alkylaminocarbonyloxy group;
- N,N-dialkylaminocarbonyloxy group (wherein two alkyl groups are optionally combined to form a heterocyclyl group containing an N atom);
- alkyloxycarbonyloxy group;
- aryloxycarbonyloxy group;
- heteroaryloxycarbonyloxy group;
- ureido group;
- N-alkylureido group;
- N,N-dialkylureido group (wherein two alkyl groups are optionally combined to form a heterocyclyl group containing an N atom);
- N,N,N'-trialkylureido group (wherein two alkyl group(s) on the same N atom are optionally combined to form a heterocyclyl group containing an N atom);
- guanidino group;
- amidino group;
- hydrazino group;
- alkylhydrazino group;
- N,N-dialkylhydrazino group;
- N,N'-dialkylhydrazino group;

alkylthio group;
alkylsulfinyl group;
alkylsulfonyl group;
arylthio group;
arylsulfinyl group;
arylsulfonyl group;
heteroarylthio group;
heteroarylsulfinyl group;
heteroarylsulfonyl group;
sulfenic acid group;
sulfinic acid group;
sulfonic acid group;
sulfuric acid group;
sulfinamide group;
sulfonamide group;
phosphine oxide group;
phosphinic acid group;
phosphoryl group (phosphonic acid group $-P(=O)(OH)_2$); bisphosphorylmethyl group (-C(H, OH, $NH_2$, Cl, or optionally
substituted alkyl) $(P(=O)(OH)_2)_2$);
phosphoric acid group (phosphate group $-O-P(=O)(OH)_2$);
boric acid group;
alkyl group;
alkenyl group;
alkynyl group;
cycloalkyl group;
cycloalkenyl group;
aryl group;
heteroaryl group;
heterocyclyl group; or
lactonyl group).

Specific examples of said alkyl group include a methyl group, an ethyl group, a n-propyl group, an isopropyl group, a n-butyl group, an isobutyl group, a sec-butyl group, a tert-butyl group, a n-pentyl group, a n-hexyl group, a n-heptyl group, a n-octyl group, a n-nonyl group, and a n-decyl group.

When the term of "alkane" is used in the present description, said alkane means "a straight or branched saturated hydrocarbon having 1 to 10 carbon atom(s)" formed by adding one hydrogen atom to the above-defined "alkyl group".

The term of "alkenyl group" as described herein refers to a straight or branched unsaturated hydrocarbon group comprising one or more double bond(s) (-C=C-) in the chain of the above-defined alkyl group.

Examples of the substituent of an optionally substituted alkenyl group include one or more substituent(s) independently selected from the Group A.

Specific examples of said alkenyl group include a methylidene group ($=CH_2$ group), an ethenyl group, a 1-propenyl group, a 2-propenyl group, a 1-butenyl group, a 2-butenyl group, a 3-butenyl group, a 1,3-butadienyl group, a 1-methyl-2-propenyl group, a 1,1-dimethyl-2-propenyl group, a 1-pentenyl group, a 2-pentenyl group, a 3-pentenyl group, a 4-pentenyl group, a 1-methyl-2-butenyl group, a 3-methyl-1-butenyl group, a 1-hexenyl group, a 2-hexenyl group, a 3-hexenyl group, a 4-hexenyl group, a 5-hexenyl group, a 1-methyl-2-pentenyl group, a 3-methyl-1-pentenyl group, a 2-heptenyl group, a 4-octenyl group, a 1-nonenyl group, and a 2-decenyl group.

The term of "alkynyl group" as described herein refers to a straight or branched unsaturated hydrocarbon group comprising one or more triple bond(s) (C triple bonds c) in the chain of the above-defined alkyl group (provided that the number of carbon atom is 2 to 10).

Examples of the substituent of an optionally substituted alkynyl group include one or more substituent(s) independently selected from the Group A.

Specific examples of said alkynyl group include an ethynyl group, a 1-propynyl group, a 2-propynyl group, a 1-butynyl group, a 2-butynyl group, a 3-butynyl group, a 1-methyl-2-propynyl group, a 1,1-dimethyl-2-propynyl group, a 1-pentynyl group, a 2-pentynyl group, a 3-pentynyl group, a 4-pentynyl group, a 1-methyl-2-butynyl group, a 3-methyl-1-butynyl group, a 1-hexynyl group, a 2-hexynyl group, a 3-hexynyl group, a 4-hexynyl group, a 5-hexynyl group, a 1-methyl-2-pentynyl group, a 3-methyl-1-pentynyl group, a 2-heptynyl group, a 4-octynyl group, a 1-nonynyl group, and a 2-decynyl group.

The term of "alkyloxy group" as described herein refers to a monovalent group in which the above-defined alkyl group having 1 to 10 carbon atom(s) is bound to an oxy group (alkyl-O- group). Specific examples of said alkyloxy group include a methoxy group, an ethoxy group, a n-propoxy group, an isopropoxy group, a n-butyloxy group, an isobutyloxy group, a sec-butyloxy group, a tert-butyloxy group, a n-pentyloxy group, a n-hexyloxy group, a n-heptyloxy group, a n-octyloxy group, a n-nonyloxy group, and a n-decyloxy group.

The term of "alkylthio group" as described herein refers to a monovalent group in which the above-defined alkyl group having 1 to 10 carbon atom(s) is bound to a thio group (alkyl-S- group). Specific examples of said alkylthio group include a methylthio group, an ethylthio group, a n-propylthio group, an isopropylthio group, a n-butylthio group, an isobutylthio group, a sec-butylthio group, a tert-butylthio group, a n-pentylthio group, a n-hexylthio group, a n-heptylthio group, a n-octylthio group, a n-nonylthio group, and a n-decylthio group.

Examples of the substituent of an optionally substituted alkyloxy group and an alkylthio group include one or more substituent(s) independently selected from the Group A.

The term of "cycloalkyl group" as described herein refers to a group in which one hydrogen atom is removed from a monocyclic hydrocarbon (i.e., cycloalkane) having 3 to 10 carbon atoms and a group in which one hydrogen atom is removed from a bicyclic hydrocarbon (i.e., bicyclic cycloalkane) having 4 to 10 carbon atoms.

Examples of the substituent of an optionally substituted cycloalkyl group include one or more substituent(s) independently selected from an oxo group (=O); a thioxo group (=S); an imino group (=N($R^8$)); an oxime group (=N-O$R^9$); a hydrazono group (=N-N($R^{10}$)($R^{11}$)); an alkyl group; and the Group A (hereinafter the group consisting of an oxo group; a thioxo group; an imino group; an oxime group; a hydrazono group; an alkyl group; and the Group A is referred to as "Group B") (wherein $R^8$, $R^9$, $R^{10}$, and $R^{11}$ are each independently a hydrogen atom, an alkyl group, an alkenyl group, a cycloalkyl group, a cycloalkenyl group, an aryl group, a heteroaryl group, or a heterocyclyl group).

Specific examples of said cycloalkyl group include monocyclic cycloalkyl groups having 3 to 10 carbon atoms such as a cyclopropyl group, a cyclobutyl group, a cyclopentyl group, a cyclohexyl group, a cycloheptyl group, a cyclooctyl group, a cyclononyl group, and a cyclodecyl group; and bicyclic cycloalkyl groups having 4 to 10 carbon atoms such as a bicyclo[1.1.0]butyl group, a bicyclo[2.1.0]pentyl group, a bicyclo[3.1.0]hexyl group, a bicyclo[3.2.0]heptyl group, a bicyclo[2.2.1]heptyl group, a bicyclo[3.3.0]octyl group, a bicyclo[3.2.1]octyl group, a bicyclo[2.2.2]octyl group, a bicyclo[6.1.0]nonyl group, and a bicyclo[4.4.0]decyl group.

When the term of "cycloalkane" is used in the present description, said cycloalkane means "a monocyclic hydrocarbon having 3 to 10 carbon atoms and a bicyclic hydrocarbon having 4 to 10 carbon atoms" in which one hydrogen atom is added to the above-defined "cycloalkyl group".

The term of "cycloalkenyl group" as described herein refers to a monocyclic or bicyclic unsaturated hydrocarbon group comprising one or more double bond(s) (-C=C-) in the ring of the above-defined cycloalkyl group.

Examples of the substituent of an optionally substituted cycloalkenyl group include one or more substituent(s) independently selected from the Group B.

Specific examples of said cycloalkenyl group include monocyclic cycloalkenyl groups having 3 to 10 carbon atoms such as a 1-cyclopropenyl group, a 1-cyclobutenyl group, a 1-cyclopentenyl group, a 2-cyclopentenyl group, a 1-cyclohexenyl group, a 2-cyclohexenyl group, a 3-cyclohexenyl group, a 1,3-cyclohexadienyl group, a 1,4-cyclohexadienyl group, a 1-cycloheptenyl group, a 2-cyclooctenyl group, a 3-cyclooctenyl group, a 4-cyclooctenyl group, a 1-cyclononenyl group, and a 1-cyclodecenyl group; and bicyclic cycloalkenyl groups having 4 to 10 carbon atoms such as a bicyclo[1.1.0]but-1-enyl group, a bicyclo[2.1.0]pent-2-enyl group, a bicyclo[3.1.0]hex-2-enyl group, a bicyclo[3.2.0]hept-3-enyl group, a bicyclo[2.2.1]hept-2-enyl group, a bicyclo[3.3.0]oct-2-enyl group, a bicyclo[3.2.1]oct-2-enyl group, a bicyclo[2.2.2]oct-2-enyl group, a bicyclo[6.1.0]non-4-enyl group, and a bicyclo[4.4.0]dec-3-enyl group.

The term of "aryl group" as described herein refers to a group in which one hydrogen atom is removed from a monocyclic or bicyclic aromatic hydrocarbon (i.e., arene) having 6 to 11 ring carbon atoms.

Examples of the substituent of an optionally substituted aryl group include one or more substituent(s) independently selected from an alkyl group and the Group A (hereinafter the group consisting of an alkyl group and the Group A is referred to as "Group C").

Specific examples of said aryl group include monocyclic aryl groups such as a phenyl group; and optionally partially saturated bicyclic aryl groups having 9 to 11 ring carbon atoms ($C_9$ to $C_{11}$) such as a naphthyl group, a tetrahydronaphthyl group, an indenyl group, and an indanyl group.

When the term of "arene" is used in the present description, said arene means "a monocyclic aromatic hydrocarbon having 6 to 11 ring carbon atoms or a bicyclic aromatic hydrocarbon having 9 to 11 ring carbon atoms" formed by adding one hydrogen atom to the above-defined "aryl group".

The term of "heteroaryl group" as described herein refers to a group in which one hydrogen atom is removed from a 5 to 14 membered monocyclic, bicyclic, or tricyclic aromatic heterocyclic ring comprising 1 to 4 heteroatom(s) selected from an oxygen atom, a sulfur atom, and a nitrogen atom other than carbon atom(s) (i.e., heteroarene).

Examples of the substituent of an optionally substituted heteroaryl group include one or more substituent(s) independently selected from the Group C.

Specific examples of said heteroaryl group include 5 to 6 membered monocyclic heteroaryl groups comprising 1 to 4 heteroatom(s) selected from an oxygen atom, a sulfur atom, and a nitrogen atom other than carbon atom(s) such as a pyrrolyl group, a furyl group, a thienyl group, a pyrazolyl group, an imidazolyl group, an oxazolyl group, an isoxazolyl group, a thiazolyl group, an isothiazolyl group, a thiadiazolyl group, a triazolyl group, a tetrazolyl group, a pyridyl group, a pyrazinyl group, a pyrimidinyl group, a pyridazinyl group, and a triazinyl group; 8 to 11 membered bicyclic heteroaryl groups comprising 1 to 4 heteroatom(s) selected from an oxygen atom, a sulfur atom, and a nitrogen atom other than carbon atom(s) such as an indolyl group, an isoindolyl group, an indazolyl group, a tetrahydroindazolyl group, a benzofuranyl group, a dihydrobenzofuranyl group, a dihydroisobenzofuranyl group, a benzothiophenyl group, a dihydrobenzothiophenyl group, a dihydroisobenzothiophenyl group, a benzooxazolyl group, a dihydrobenzooxazolyl group, a benzothiazolyl group, a dihydrobenzothiazolyl group, a quinolyl group, a tetrahydroquinolyl group, an isoquinolyl group, a tetrahydroisoquinolyl group, a naphthyridinyl group, a tetrahydronaphthyridinyl group, a quinoxalinyl group, a tetrahydroquinoxalinyl group, and a quinazolinyl group; and 11 to 14 membered tricyclic heteroaryl groups comprising 1 to 4 heteroatom(s) selected from an oxygen atom, a sulfur atom, and a nitrogen atom other than carbon atom(s) such as a dibenzofuran group, a dibenzopyrrole group, a dibenzothiophene group, and a dibenzopyridine group.

When the term of "heteroarene" is used in the present description, said heteroarene means "a 5 to 14 membered monocyclic, bicyclic, or tricyclic aromatic heterocyclic ring comprising 1 to 4 heteroatom(s) selected from an oxygen atom, a sulfur atom, and a nitrogen atom other than carbon atom(s)" formed by adding one hydrogen atom to the above-defined "heteroaryl group".

The term of "heterocyclyl group" as described herein refers to a monovalent group formed by removing one hydrogen atom from a 3 to 12 membered monocyclic nonaromatic heterocyclic ring comprising 1 to 4 heteroatom(s) selected from an oxygen atom, a sulfur atom, and a nitrogen atom other than carbon atom(s) (i.e., heterocyclene).

Examples of the substituent of an optionally substituted heterocyclyl group include one or more substituent(s) independently selected from an oxo group (=O); a thioxo group (=S); an imino group (=$NR^9$; wherein $R^9$ is the same as defined above); a hydrazono group (=N-N($R^{10}$)($R^{11}$); wherein $R^{10}$ and $R^{11}$ are the same as defined above); an alkyl group; and the Group A (hereinafter the group consisting of an oxo group; a thioxo group; an imino group; a hydrazono group; an alkyl group; and the Group A is referred to as "Group D").

Specific examples of said heterocyclyl group include an azetidinyl group, an oxetanyl group, a thietanyl group, a pyrrolidinyl group, a piperidinyl group, a piperidinyl group, a tetrahydrofuranyl group, a tetrahydropyranyl group, a tetrahydrothienyl group, a piperazinyl group, a morpholinyl group, a perhydroazepinyl group, an azacyclooctyl group, an azacyclooct-3-enyl group, an azacycloocta-4-enyl group, an azacyclononyl group, an azacyclodecyl group, 6 to 12 membered azabicycloalkyl groups (for example, an azabicyclohexyl group, an azabicycloheptyl group, an azabicyclooctyl group, an azabicyclononyl group, an azabicyclodecyl group, an azabicycloundecyl group, or an azabicyclododecyl group), 6 to 12 membered azabicycloalkenyl groups (for example, an azabicyclohexenyl group, an azabicycloheptenyl group, an azabicyclooctenyl group, an azabicyclononenyl group, an azabicyclodecenyl group, an azabicycloundecenyl group, or an azabicyclododecenyl group), and 6 to 12 membered azaspiroalkyl groups (for example, an azaspirohexyl group, an azaspiroheptyl group, an azaspirooctyl group, an azaspirononyl group, an azaspirodecyl group, an azaspiroundecyl group, or an azaspirododecyl group).

When the term of "heterocyclene" is used in the present description, said heterocyclene means "a 4 to 12 membered monocyclic nonaromatic heterocyclic ring" comprising 1 to 4 heteroatom(s) selected from an oxygen atom, a sulfur atom, and a nitrogen atom other than carbon atom(s) formed by adding one hydrogen atom to the above-defined "heterocyclyl group".

The above cyclic compounds, i.e., cycloalkane, arene, heteroarene, and heterocyclene are each optionally fused to any other cyclic compound(s) to form bi- to tetracyclic compounds, and monovalent groups or divalent groups may be produced by removing one or two hydrogen atom(s) from these bi- to tetracyclic compounds.

The term "lactonyl group" as described herein refers to a kind of said heterocyclyl group in which a heterocyclyl group has an oxygen atom in the ring as a heteroatom and the carbon atom adjacent to said oxygen atom is substituted with an oxo group. Examples of said lactonyl group include an α-acetolactonyl group, a β-propiolactonyl group, a γ-butyrolactonyl group, a δ-valerolactonyl group, an ε-caprolactonyl group, a γ-nonalactonyl group, a γ-decalactonyl group, a γ-undecalactonyl group, a glucono-δ-lactonyl group, and a pantolactonyl group (monovalent residue of pantolactone), and each of which is optionally substituted like said heterocyclyl group, and optionally fused to any other cyclic compound(s) to form a bi- to tetracyclic compound.

Further, said lactonyl group can be a substituent bound to a carbon atom or a heteroatom (such as N, O, S, Se, or P) present in linker moieties or antitumor drug residues in the conjugate precursor or the conjugate precursor synthesis intermediates of the present invention represented by General Formula (I), (II-1), or (II-2).

The term of "alkylene group" as described herein means a divalent group formed by removing any one hydrogen atom from the above-defined "alkyl group". Also, when said alkyl group has substituent(s), the term of "alkylene group" means a divalent group formed by removing any one additional hydrogen atom from an alkyl chain carbon atom other than said substituent(s).

Here, in the present invention, in General Formulae (I), (II-1) and (II-2), $L^1$, $L^2$, $L^3$, $L1^{-1}$, $L^{1-2}$ and $L^{3-1}$ are each independently an optionally substituted alkylene group,
one or more methylene groups in the chain of the alkylene group may be replaced with one or more divalent groups independently selected from the group consisting of - $C(R^1)(R^2)$ -; -O-; -$N(R^3)$-; -$N(R^3)$-$N(R^3)$-; -S-; -Se-; -$Si(R^4)(R^5)$-; -S-S-; -Se-Se-; -SOm-; -SeOn-; -C(=C($R^6$)($R^7$))-; -C(=O)-; - C(=S)-; -C(=N($R^8$))-; -C(=N-O$R^9$)-; -C(=N-N($R^{10}$)($R^{11}$))-; - P(=O)($R^{12}$)-; -P(=O)(O$R^{13}$)-; -O-P(=O)($R^{12}$)-O-; -O-P(=O)(O$R^{13}$)-O-; -C($R^{14}$)=; =C($R^{14}$)-; -C($R^{14}$)=C($R^{14}$)-; -N=;=N-; -C≡C-; -$(O-C(R^1)(R^2)-C(R^1)(R^2))_{1-30}$-; -$(C(R^1)(R^2)-C(R^1)(R^2)-0)_{1-30}$- (wherein $R^1$, $R^2$, $R^3$, $R^4$, $R^5$, $R^6$, $R^7$, $R^8$, $R^9$, $R^{10}$, $R^{11}$, $R^{12}$, $R^{13}$, and $R^{14}$ are independently a group selected from among a hydrogen atom, an alkyl group, an alkenyl group, an alkynyl group, a cycloalkyl group, a cycloalkenyl group, an aryl group, a heteroaryl group, and a heterocyclyl group, and when $R^3$ is an alkyl group, the alkyl group is optionally combined with an alkyl group on an adjacent methylene group to form a cyclic structure, and m and n are independently an integer of 0 to 2); an optionally substituted alkenylene group, an optionally substituted alkynylene group, an optionally substituted cycloalkylene group; an optionally substituted cycloalkenylene group; an optionally substituted arylene group; an optionally substituted heteroarylene group; and an optionally substituted heterocyclylene group.

Examples of the substituent of an optionally substituted alkylene group include one or more substituent(s) independently selected from the Group A.

Specific examples of said alkylene group include a methylene group, an ethylene group, a methylmethylene group, a trimethylene group, an ethylmethylene group, a dimethylmethylene group, a n-propylene group, a n-butylene group, a n-pentylene group, a n-hexylene group, a n-heptylene group, a n-octylene group, a n-nonylene group, a n-decylene group, a n-undecylene group, a n-dodecylene group, a n-tridecylene group, a n-tetradecylene group, a n-pentadecylene group, a n-hexadecylene group, a n-heptadecynylene group, a n-octadecylene group, a n-nonadecylene group, a n-icosenylene group, a n-triacontylene group, a n-tetracontylene group, and a n-pentacontylene group.

The term of "alkenylene group" as described herein refers to a group constituting the linker chain represented by $L^1$, $L^2$, $L^3$, $L1^{-1}$, $L^{2-1}$ and $L^{3-1}$ in the above general formulae (II-1) and (II-2), and means a divalent group formed by removing any one hydrogen atom from the above-defined "alkenyl group". Also, when said alkenyl group has substituent(s), the term of "alkenylene group" means a divalent group formed by removing any one additional hydrogen atom from an alkenyl chain carbon atom other than said substituent(s).

Examples of the substituent of an optionally substituted alkenylene group include one or more substituent(s) independently selected from the Group A.

Specific examples of said alkenylene group include an ethenylene group, a 1-propenylene group, a 2-propenylene group, a 1-butenylene group, a 2-butenylene group, a 3-butenylene group, a 1,3-butadienylene group, a 1-methyl-2-propenylene group, a 1,1-dimethyl-2-propenylene group, a 1-pentenylene group, a 2-pentenylene group, a 3-pentenylene group, a 4-pentenylene group, a 1-methyl-2-butenylene group, a 3-methyl-1-butenylene group, a 1-hexenylene group, a 2-hexenylene group, a 3-hexenylene group, a 4-hexenylene group, a 5-hexenylene group, a 1-methyl-2-pentenylene group, a 3-methyl-1-pentenylene group, a 2-heptenylene group, a 4-octenylene group, a 1-nonenylene group, a 2-decenylene group, a 1-undecenylene group, a 3-dodecenylene group, a 2-tridecenylene group, a 4-tetradecenylene group, a 1-pentadecenylene group, a 2-hexadecenylene group, a 3-heptadecenylene group, a 2-octadecenylene group, a 1-nonadecenylene group, and a 1-icosenylene group.

The term of "alkynylene group" as described herein refers to a group constituting the linker chain represented by $L^1$, $L^2$, $L^3$, $L1^{-1}$, $L^{2-1}$ and $L^{3-1}$ in the above general formulae (II-1) and (II-2), and means a divalent group formed by removing any one hydrogen atom from the above-defined "alkynyl group". Also, when said alkynyl group has substituent(s), the term of "alkynylene group" means a divalent group formed by removing any one additional hydrogen atom from the alkynyl chain carbon atom(s) other than said substituent(s).

Examples of the substituent of an optionally substituted alkynylene group include one or more substituent(s) independently selected from the Group A.

Specific examples of said alkynylene group include an ethynylene group, a 1-propynylene group, a 2-propynylene group, a 1-butynylene group, a 2-butynylene group, a 3-butynylene group, a 1-methyl-2-propynylene group, a 1,1-dimethyl-2-propynylene group, a 1-pentynylene group, a 2-pentynylene group, a 3-pentynylene group, a 4-pentynylene group, a 1-methyl-2-butynylene group, a 3-methyl-1-butynylene group, a 1-hexynylene group, a 2-hexynylene group, a 3-hexynylene group, a 4-hexynylene group, a 5-hexynylene group, a 1-methyl-2-pentynylene group, a 3-methyl-1-pentynylene group, a 2-heptynylene group, a 4-octynylene group, a 1-nonynylene group, a 2-decynylene group, a 1-undecynylene group, a 3-dodecynylene group, a 2-tridecynylene group, a 4-tetradecynylene group, a 1-pentadecynylene group, a 2-hexadecynylene group, a 3-heptadecynylene group, a 2-octadecynylene group, a 1-nonadecynylene group, and a 1-icosinylene group.

The term of "cycloalkylene group" as described herein refers to a group constituting the linker chain represented by $L^1$, $L^2$, $L^3$, $L1^{-1}$, $L^{2-1}$ and $L^{3-1}$ in the above general formulae (II-1) and (II-2), and means a divalent group formed by removing any one hydrogen atom from the above-defined "cycloalkyl group". Also, when said cycloalkyl group has substituent(s), the term of "cycloalkylene group" means a divalent group formed by removing any one additional hydrogen

atom from a cycloalkyl ring carbon atom other than said substituent(s).

Examples of the substituent of an optionally substituted cycloalkylene group include one or more substituent(s) independently selected from the Group B.

Specific examples of said cycloalkylene group include monocyclic cycloalkylene groups having 3 to 10 carbon atoms such as a cyclopropylene group, a cyclobutylene group, a cyclopentylene group, a cyclohexylene group, a cycloheptylene group, a cyclooctylene group, a cyclononylene group, and a cyclodecylene group; and bicyclic cycloalkylene groups having 4 to 10 carbon atoms such as a bicyclo[1.1.0]butylene group, a bicyclo[2.1.0]pentylene group, a bicyclo[3.1.0]hexylene group, a bicyclo[3.2.0]heptylene group, a bicyclo[2.2.1]heptylene group, a bicyclo[3.3.0]octylene group, a bicyclo[3.2.1]octylene group, a bicyclo[2.2.2]octylene group, a bicyclo[6.1.0]nonylene group, and a bicyclo[4.4.0]decylene group.

The term of "cycloalkenylene group" as described herein refers to a group constituting the linker chain represented by $L^1$, $L^2$, $L^3$, $L1^{-1}$, $L^{2-1}$ and $L^{3-1}$ in the above general formula (I), and means a divalent group formed by removing any one hydrogen atom from the above-defined "cycloalkenyl group". Also, when said cycloalkenyl group has substituent(s), the term of "cycloalkenylene group" means a divalent group formed by removing any one additional hydrogen atom from a cycloalkenyl ring carbon atom other than said substituent(s).

Examples of the substituent of an optionally substituted cycloalkenylene group include one or more substituent(s) independently selected from the Group B.

Specific examples of said cycloalkenylene group include monocyclic cycloalkenylene groups having 3 to 10 carbon atoms such as a 1-cyclopropenylene group, a 1-cyclobutenylene group, a 1-cyclopentenylene group, a 2-cyclopentenylene group, a 1-cyclohexenylene group, a 2-cyclohexenylene group, a 3-cyclohexenylene group, a 1, 3-cyclohexadienylene group, a 1,4-cyclohexadienylene group, a 1-cycloheptenylene group, a 2-cyclooctenylene group, a 3-cyclooctenylene group, a 4-cyclooctenylene group, a 1-cyclononenylene group, and a 1-cyclodecenylene group; and bicyclic cycloalkenyl groups having 4 to 10 carbon atoms such as a bicyclo[1.1.0]but-1-enylene group, a bicyclo[2.1.0]pent-2-enylene group, a bicyclo[3.1.0]hex-2-enylene group, a bicyclo[3.2.0]hept-3-enylene group, a bicyclo[2.2.1]hept-2-enylene group, a bicyclo[3.3.0]oct-2-enylene group, a bicyclo[3.2.1]oct-2-enylene group, a bicyclo[2.2.2]oct-2-enylene group, a bicyclo[6.1.0]non-4-enylene group, and a bicyclo[4.4.0]dec-3-enylene group.

The term of "arylene group" as described herein refers to a group constituting the linker chain represented by $L^1$, $L^2$, $L^3$, $L1^{-1}$, $L^{2-1}$ and $L^{3-1}$ in the above general formula (I), and means a divalent group formed by removing any one hydrogen atom from the above-defined "aryl group". Also, when said aryl group has substituent(s), the term of "arylene group" means a divalent group formed by removing any one additional hydrogen atom from an aryl ring carbon atom other than said substituent(s).

Examples of the substituent of an optionally substituted arylene group include one or more substituent(s) independently selected from the Group C.

Specific examples of said arylene group include monocyclic arylene groups having 6 to 11 ring carbon atoms such as a phenylene group; and optionally partially saturated bicyclic arylene groups having 9 to 11 ring carbon atoms such as a naphthylene group, a tetrahydronaphthylene group, an indenylene group, and an indanylene group.

The term of "heteroarylene group" as described herein refers to a group constituting the linker chain represented by $L^1$, $L^2$, $L^3$, $L1^{-1}$, $L^{2-1}$ and $L^{3-1}$ in the above general formula (I), and means a divalent group formed by removing any one hydrogen atom from the above defined "heteroaryl group". Also, when said heteroarylene group has substituent(s), the term of "heteroarylene group" means a divalent group formed by removing any one additional hydrogen atom from a heteroaryl ring atom other than said substituent(s).

Examples of the substituent of an optionally substituted heteroarylene group include one or more substituent(s) independently selected from the Group C.

Specific examples of said heteroarylene group include 5 to 6 membered monocyclic heteroarylene groups comprising 1 to 4 heteroatom(s) selected from an oxygen atom, a sulfur atom, and a nitrogen atom other than carbon atom(s) such as a pyrrolylene group, a furylene group, a thienylene group, a pyrazolylene group, an imidazolylene group, an oxazolylene group, an isoxazolylene group, a thiazolylene group, an isothiazolylene group, a thiadiazolylene group, triazolylene group, a tetrazolylene group, a pyridylene group, a pyrazinylene group, a pyrimidinylene group, a pyridazinylene group, and a triazinylene group; and 8 to 11 membered bicyclic heteroarylene groups comprising 1 to 4 heteroatom(s) selected from an oxygen atom, a sulfur atom, and a nitrogen atom other than carbon atom(s) such as an indolylene group, an isoindolylene group, an indazolylene group, a tetrahydroindazolylene group, a benzofuranylene group, a dihydrobenzofuranylene group, a dihydroisobenzofuranylene group, a benzothiophenylene group, a dihydrobenzothiophenylene group, a dihydroisobenzothiophenylene group, a benzoxazolylene group, a dihydrobenzoxazolylene group, a benzothiazolylene group, a dihydrobenzothiazolylene group, a quinolylene group, a tetrahydroquinolylene group, an isoquinolylene group, a tetrahydroisoquinolylene group, a naphthyridinylene group, a tetrahydronaphthyridinylene group, a quinoxalinylene group, a tetrahydroquinoxalinylene group, and a quinazolinylene group.

The term "heterocyclylene group" as described herein refers to a group constituting the linker chains represented by $L^1$, $L^2$, $L^3$, $L^{1-1}$, $L^{2-1}$, and $L^{3-1}$ in General Formulae (I), (II-1), and (II-2) above, and means a divalent group formed by

removing any one hydrogen atom from the above-defined "heterocyclyl group".

Examples of the substituent of an optionally substituted heterocyclylene group include one or more substituent(s) independently selected from the Group D.

Specific examples of said heterocyclylene group include an azetidinylene group, an oxetanylene group, a thietanylene group, a pyrrolidinylene group, a piperidinylene group, a piperidinylene group, a tetrahydrofurylene group, a tetrahydropyranylene group, a tetrahydrothienylene group, a piperazinylene group, a morpholinylene group, a perhydroazepinylene group, an azacyclooctylene group, an azacyclooct-3-enylene group, an azacyclooct-4-enylene group, an azacyclononylene group, an azacyclodecylene group, 6 to 12 membered azabicycloalkylene groups (for example, an azabicyclohexylene group, an azabicycloheptylene group, an azabicyclooctylene group, an azabicyclononylene group, an azabicyclodecylene group, an azabicycloundecylene group, or an azabicyclododecylene group), 6 to 12 membered azabicycloalkenylene groups (for example, an azabicyclohexenylene group, an azabicycloheptenylene group, an azabicyclooctenylene group, an azabicyclononenylene group, an azabicyclodecenylene group, an azabicycloundecenylene group, or an azabicyclododecenylene group), 6 to 12 membered azaspiroalkylene groups (for example, an azaspirohexylene group, an azaspiroheptylene group, an azaspirooctylene group, an azaspirononylene group, an azaspirodecylene group, an azaspiroundecylene group, or an azaspirododecylene group).

Said heterocyclylene group is optionally fused to one or more above aryl ring(s) and/or above heteroaryl ring(s) to form a bicyclic to tetracyclic heterocyclylene group.

Also, when said alkyl group, said alkenyl group, said alkynyl group, said cycloalkyl group, said cycloalkenyl group, said aryl group, said heteroaryl group, said heterocyclyl group, said alkylene group, said alkenylene group, said alkynylene group, said cycloalkylene group, said cycloalkenylene group, said arylene group, said heteroarylene group, and said heterocyclylene group have functional group(s) such as a hydroxy group, a sulfhydryl group, an amino group ($-NH_2$ and -NH- (also including -NH- in the ring of an heteroaryl group and a heterocyclyl group)), a carboxy group, a sulfenic acid group, a sulfinic acid group, a sulfonic acid group, a phosphinic acid group, a phosphonic acid group, or a boric acid group as substituent(s), said group(s) is/are optionally protected by the above protecting group(s) defined in relation to "an antitumor drug molecule or an analog thereof, or a derivative thereof".

Examples of the "salt" as described herein include salts with alkali metals such as lithium, sodium, and potassium; salts with alkaline earth metals such as magnesium and calcium; a salt with aluminum or zinc; salts with amines such as ammonia, choline, diethanolamine, lysine, ethylenediamine, tert-butylamine, tert-octylamine, tris(hydroxymethyl)aminomethane, N-methyl-glucosamine, triethanolamine, and dehydroabietylamine; salts with inorganic acids such as hydrogen chloride, hydrogen bromide, hydrogen iodide, sulfuric acid, nitric acid, and phosphoric acid; salts with organic acids such as formic acid, acetic acid, trifluoroacetic acid, propionic acid, oxalic acid, malonic acid, succinic acid, fumaric acid, maleic acid, lactic acid, malic acid, tartaric acid, citric acid, methanesulfonic acid, ethanesulfonic acid, and benzenesulfonic acid; and salts with acidic amino acids such as aspartic acid and glutamic acid. Further, the term of "salt" also includes intramolecular salts.

All of the conjugate precursor (I) and the conjugate precursor synthesis intermediates (II-1) and (II-2), and a salt thereof of the present invention may be obtained as a hydrate or a solvate, and the present invention encompasses all of them.

Specific examples of the conjugate precursor (I) of the present invention will be shown in Exemplification 2 below, and specific examples of the conjugate precursor synthesis intermediates (II-1) and (II-2) of the present invention will be shown in Exemplification 3 below, but the present invention is not limited thereto.

Exemplification 2. Exemplification of "conjugate precursor (I)"

Exemplification 2-1

[C199]

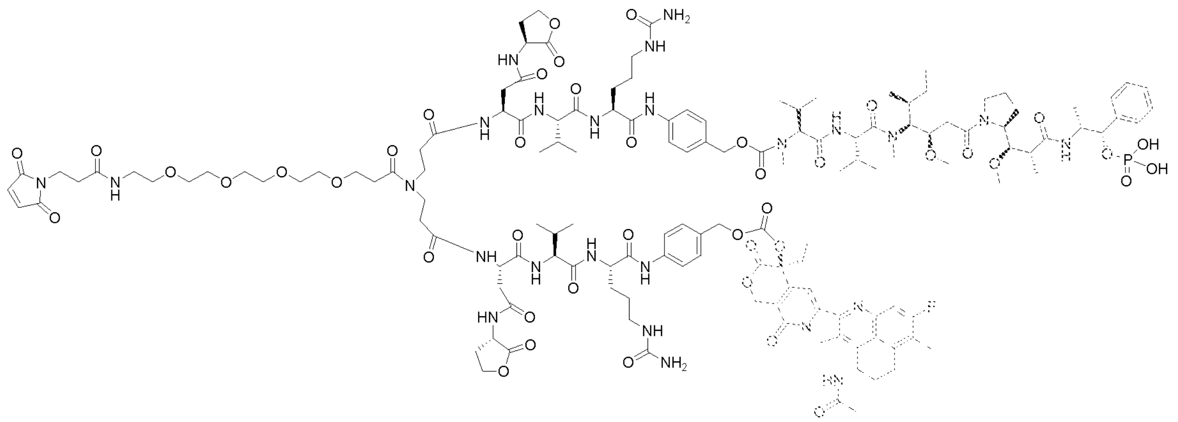

Exemplification 2-2

[C200]

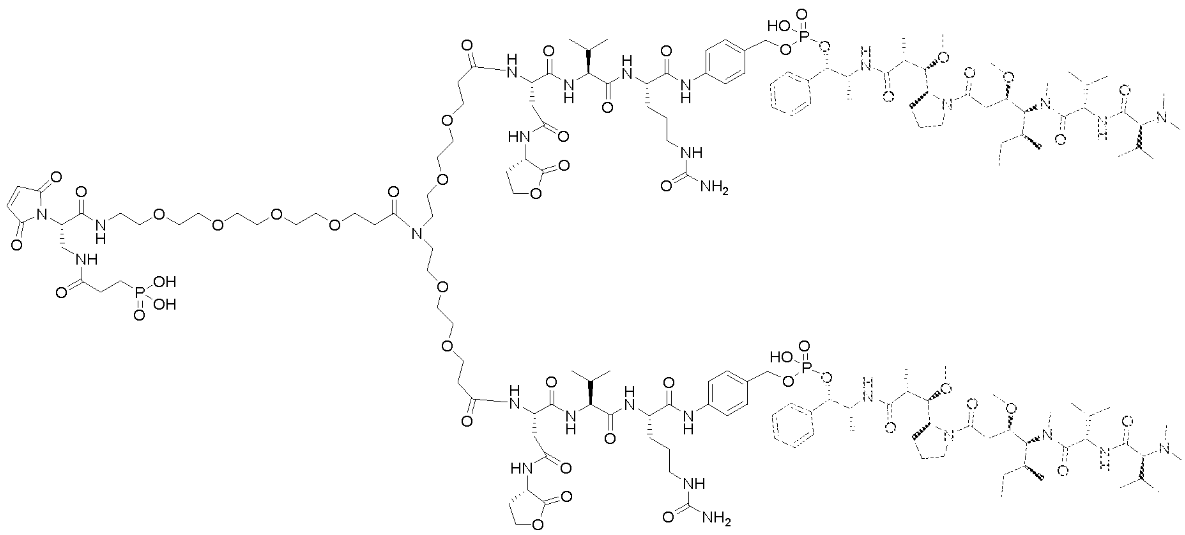

Exemplification 2-3

[C201]

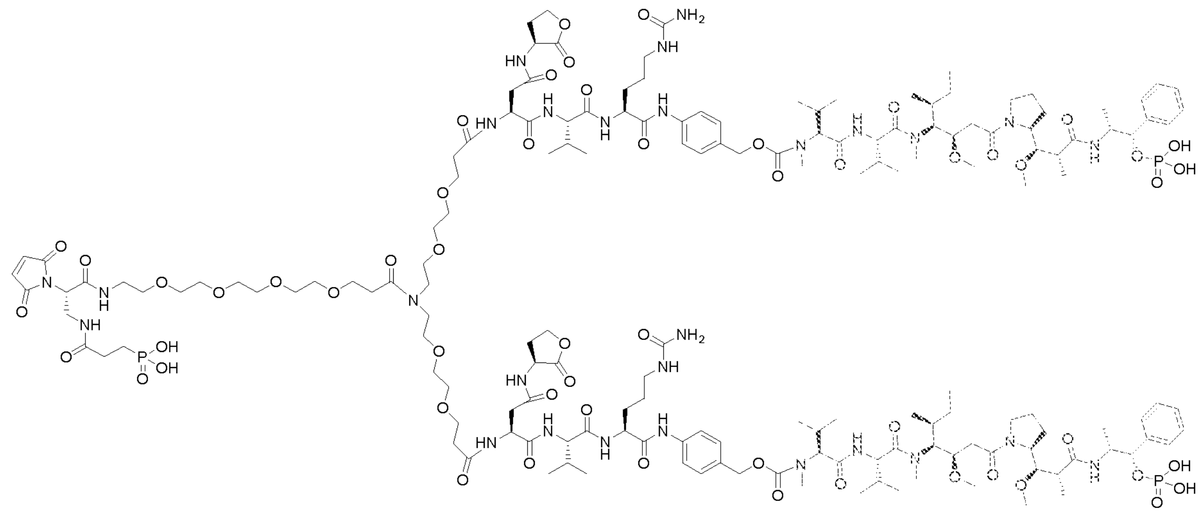

Exemplification 2-4

[C202]

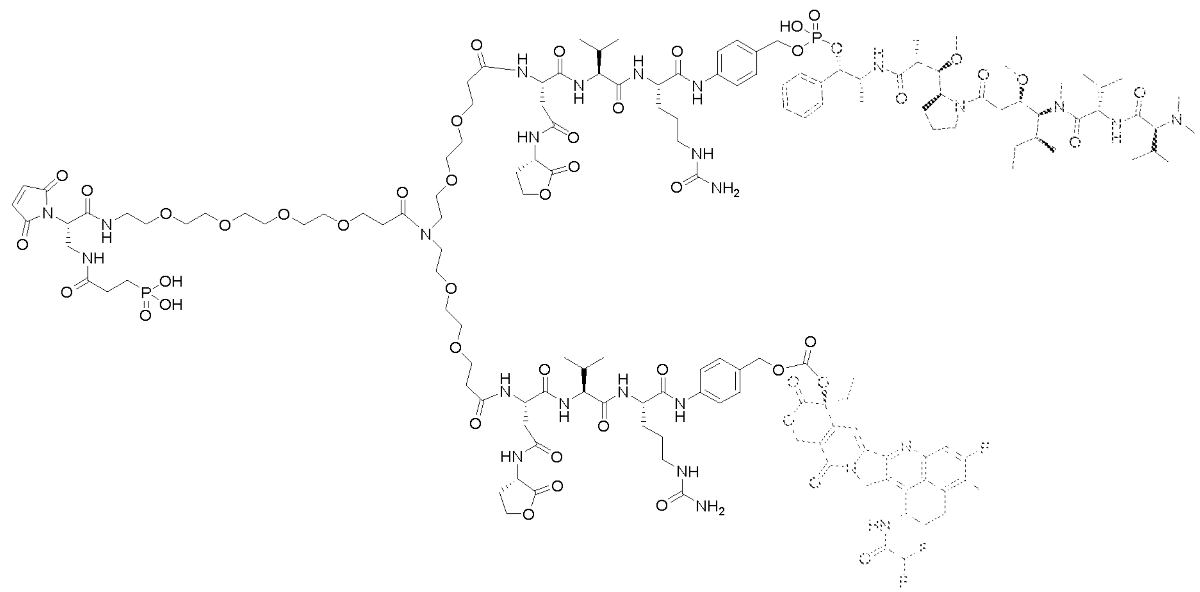

Exemplification 2-5

[C203]

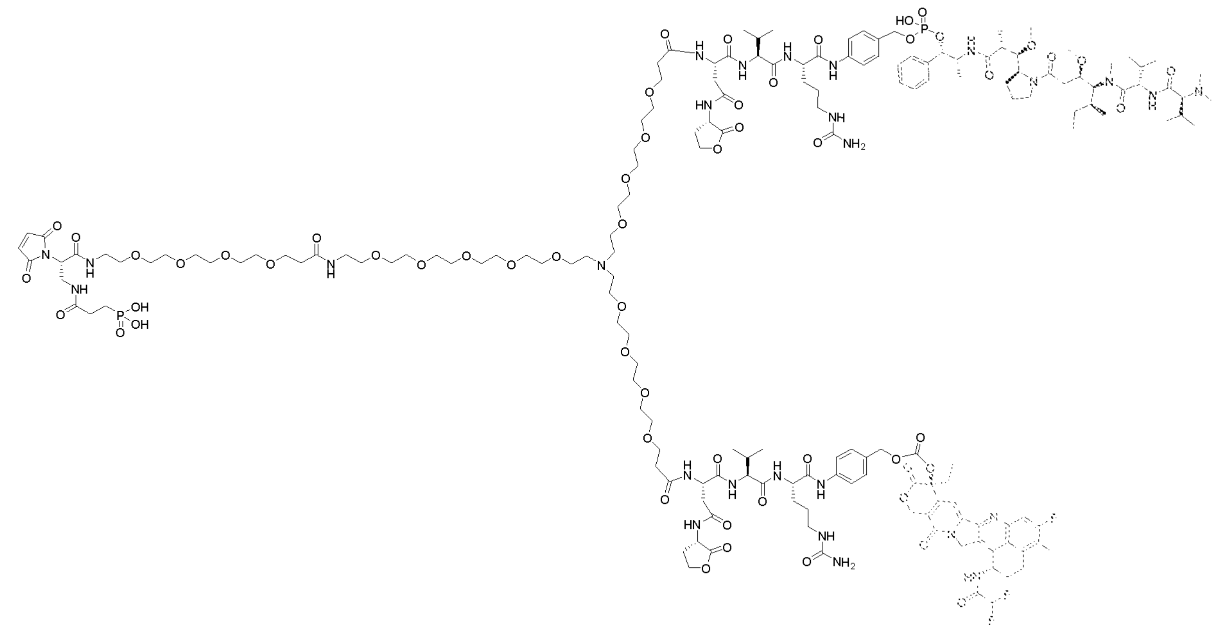

Exemplification 2-6

[C204]

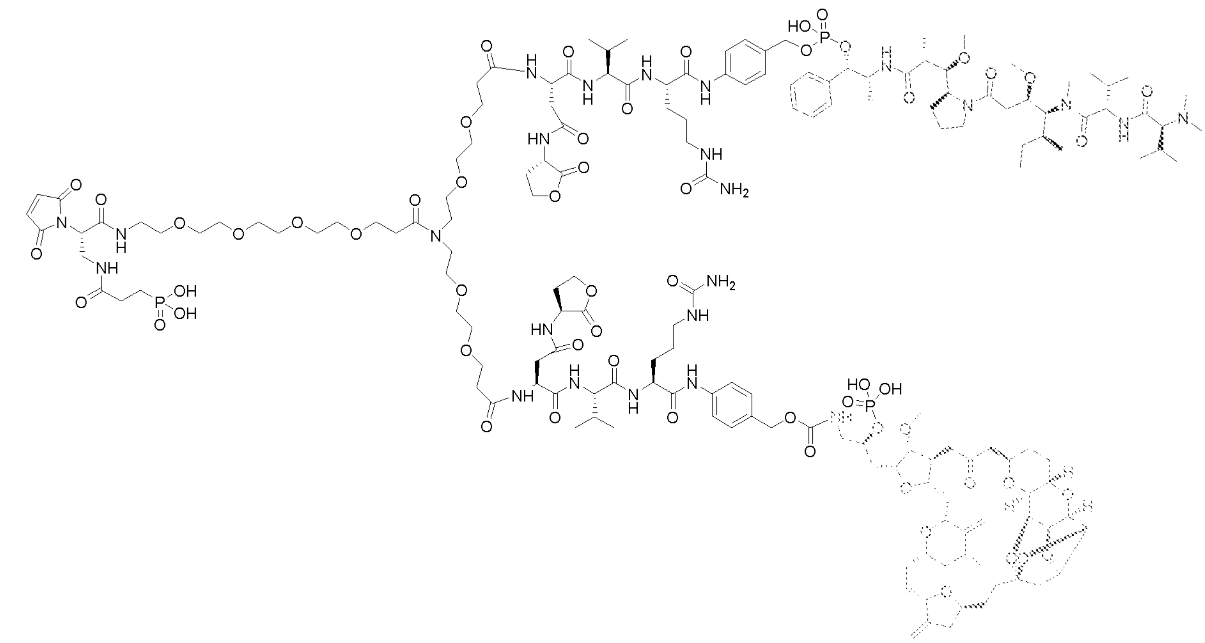

Exemplification 2-7

[C205]

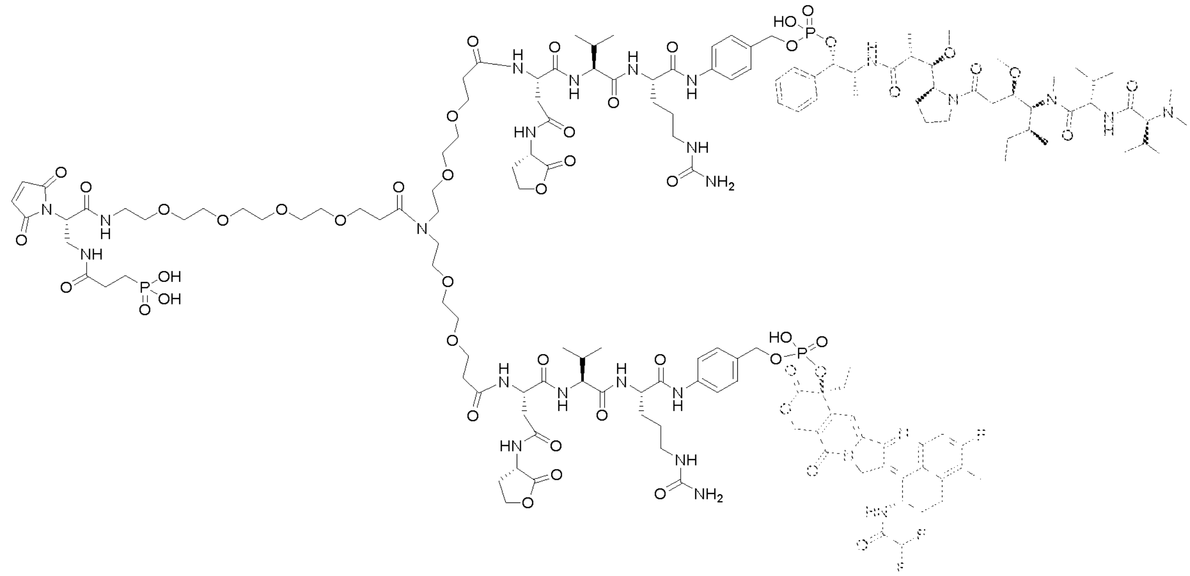

Exemplification 3. Exemplification of "conjugate precursor synthesis intermediates (II-1) and (II-2)" Exemplification 3-1

Exemplification 3-1-(1)-A

[C206]

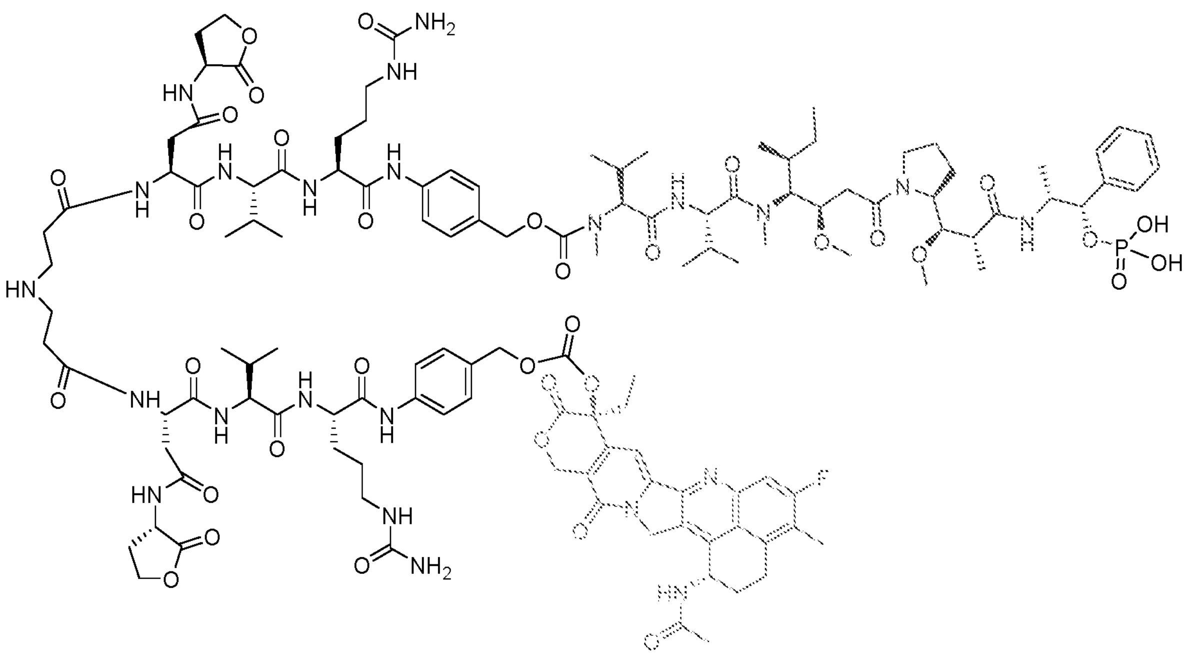

Exemplification 3-1-(1)-B

[C207]

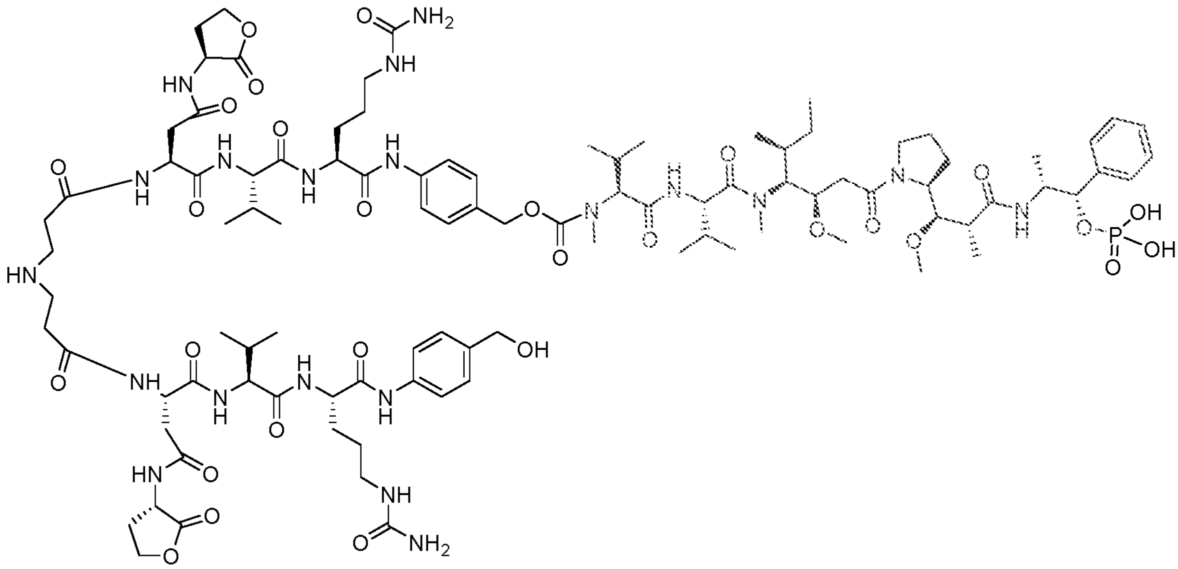

Exemplification 3-1-(1)-C

[C208]

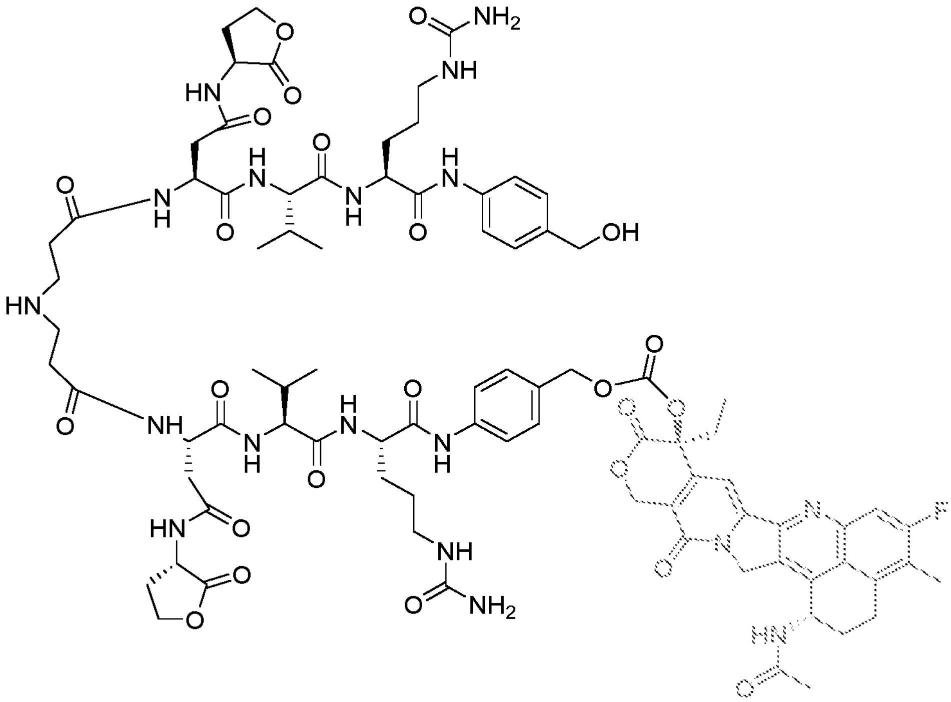

Exemplification 3-1-(2)

[C209]

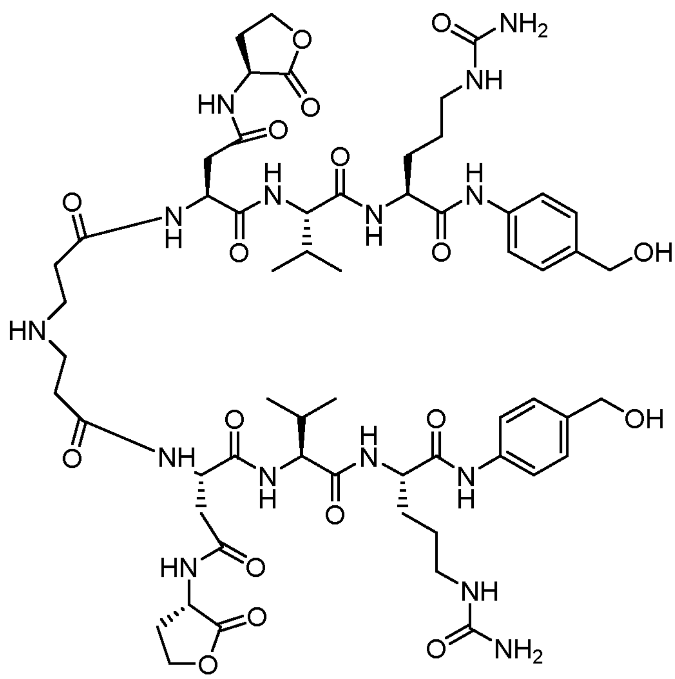

Exemplification 3-1-(3)-A

[C210]

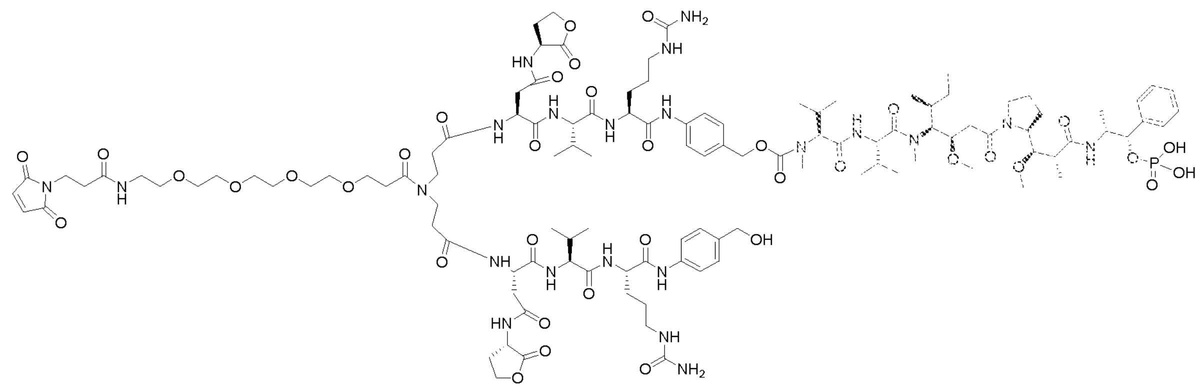

Exemplification 3-1-(3)-B

[C211]

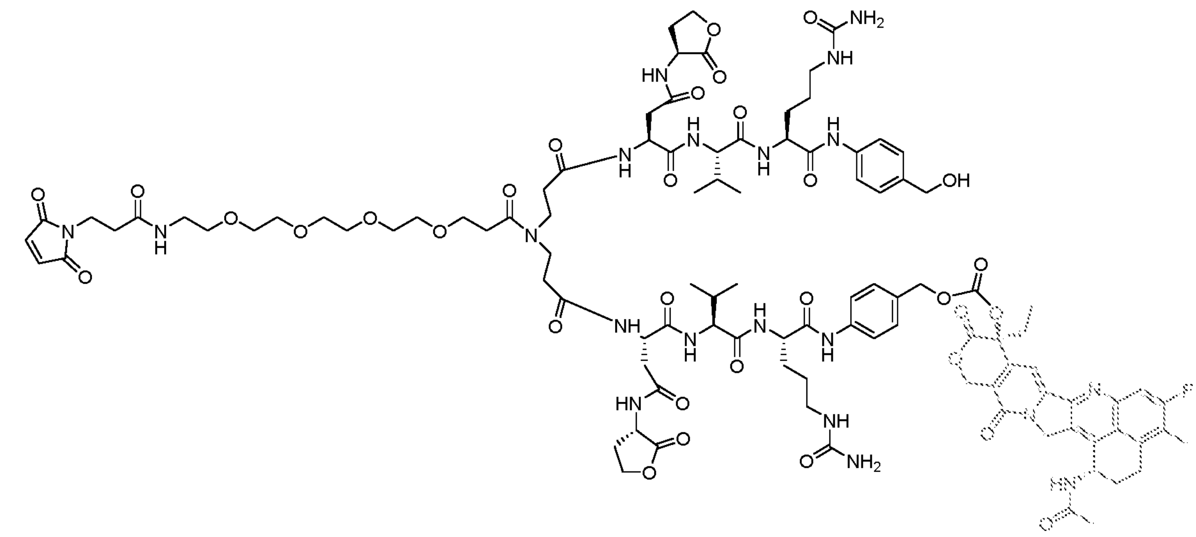

Exemplification 3-1-(3)-C

[C212]

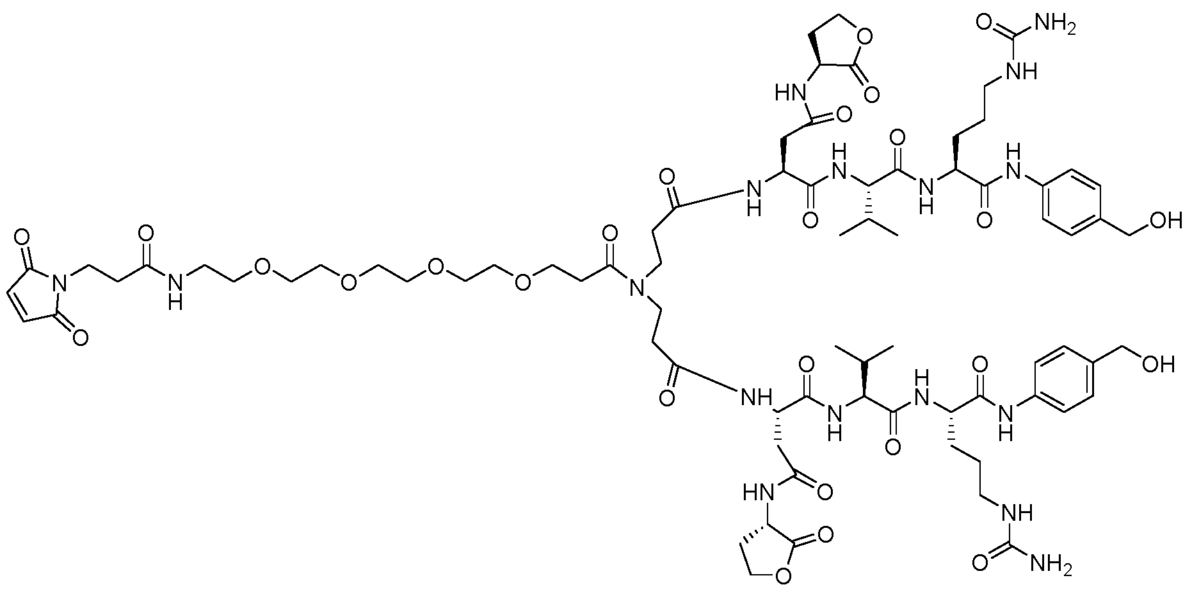

Exemplification 3-2-(1)-A

[C213]

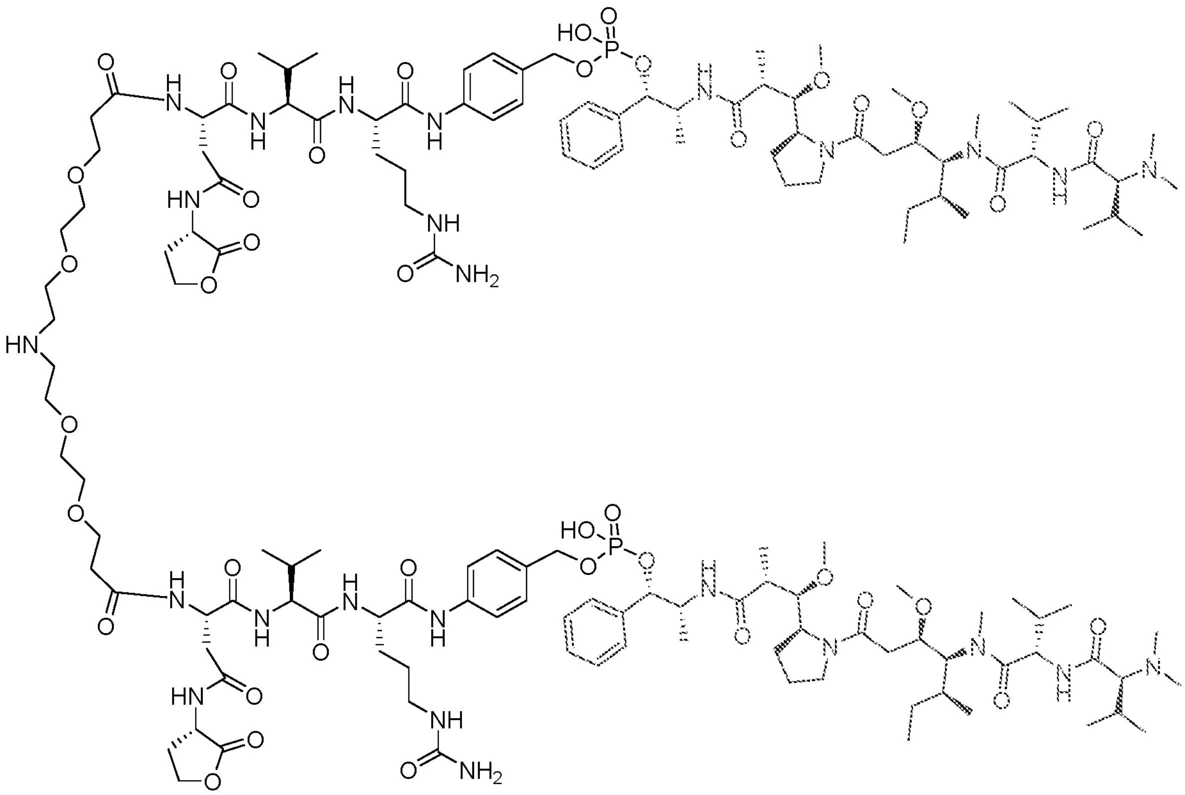

Exemplification 3-2-(1)-B

[C214]

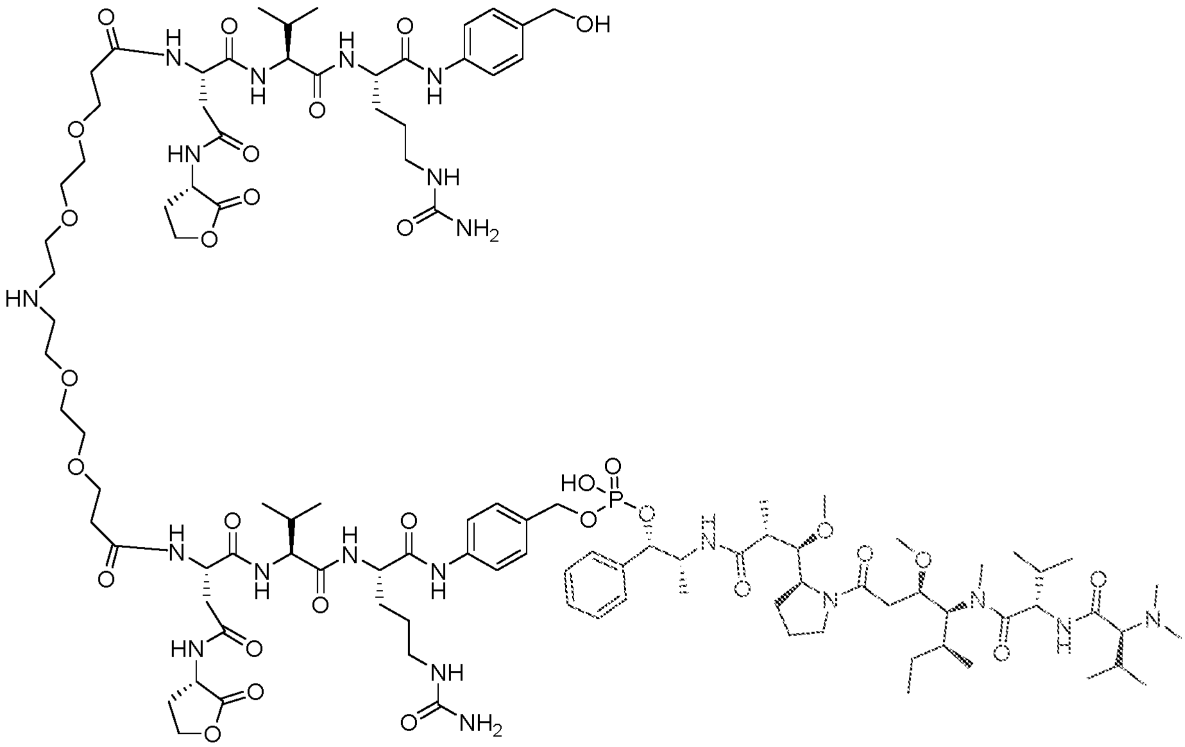

Exemplification 3-2-(2)

[C215]

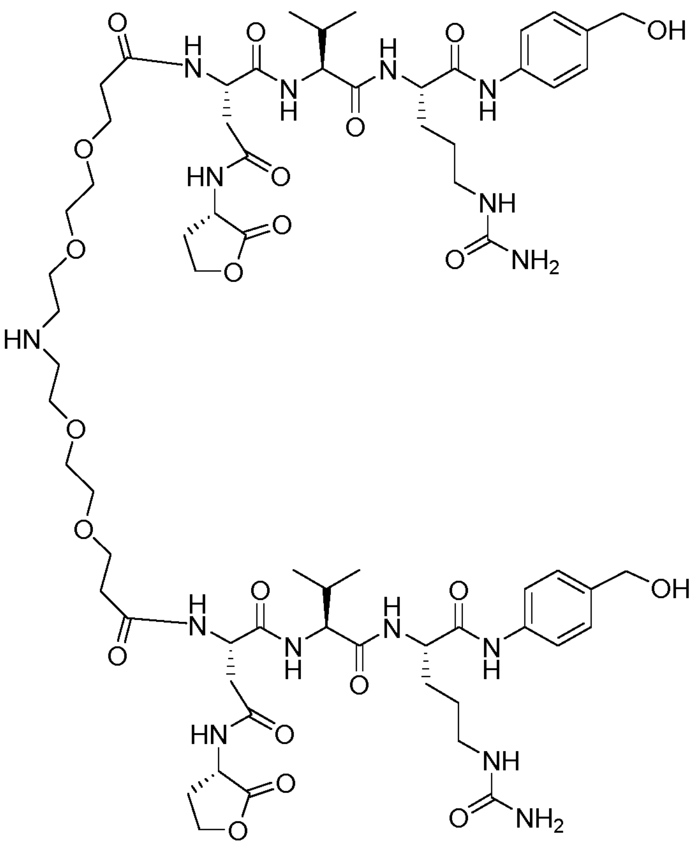

Exemplification 3-2-(3)-A

[C216]

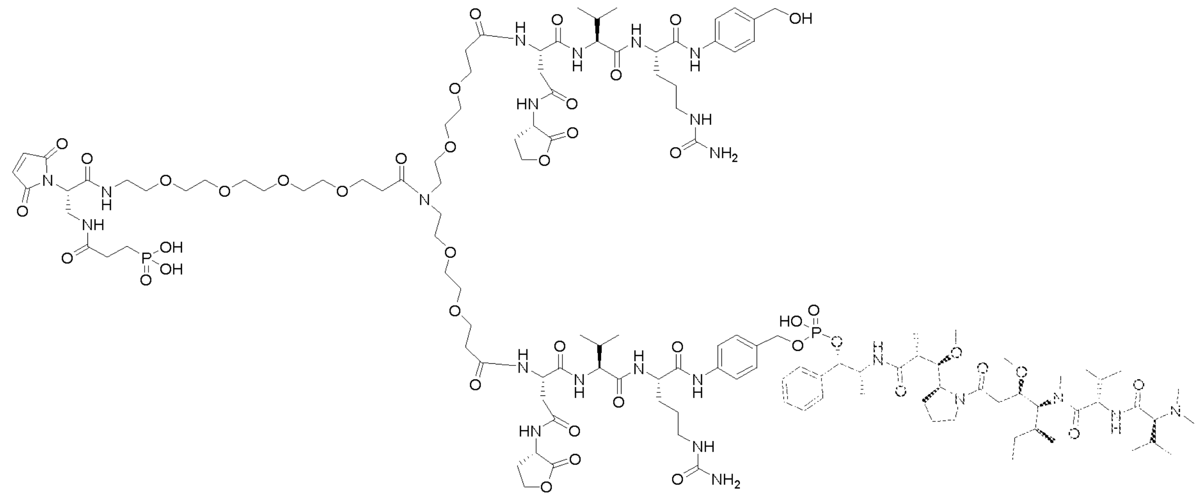

Exemplification 3-2-(3)-B

[C217]

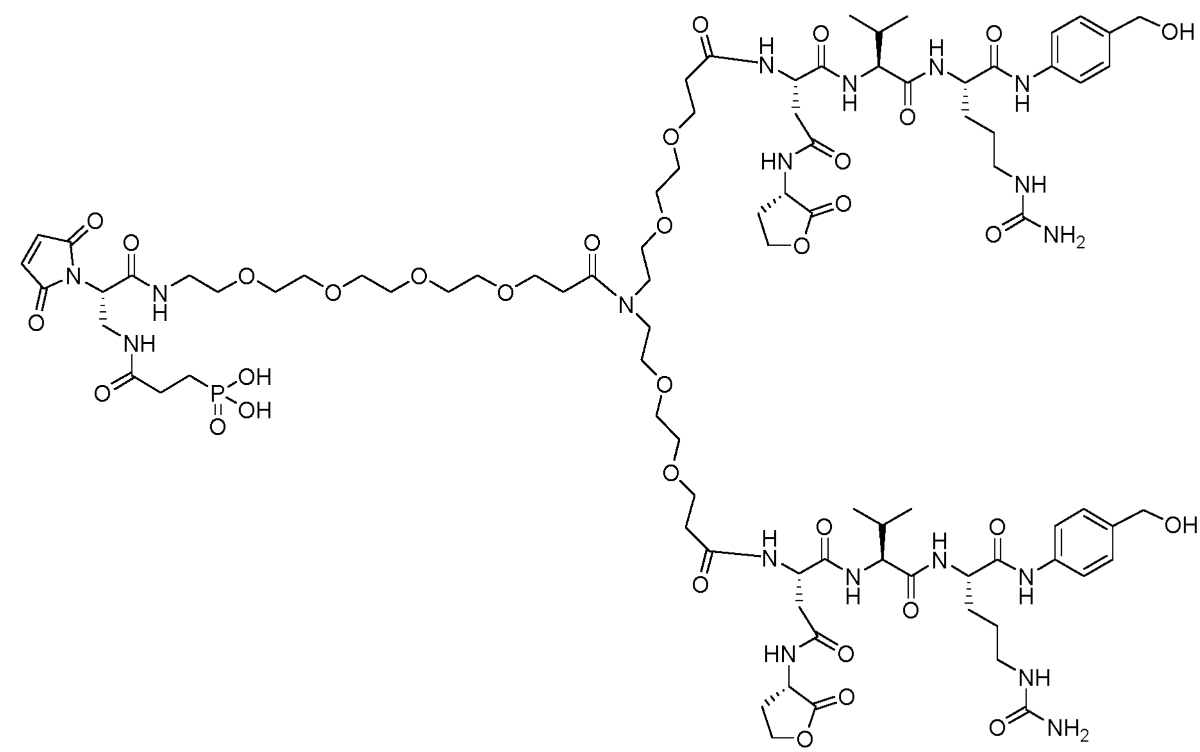

Exemplification 3-3-(1)-A

[C218]

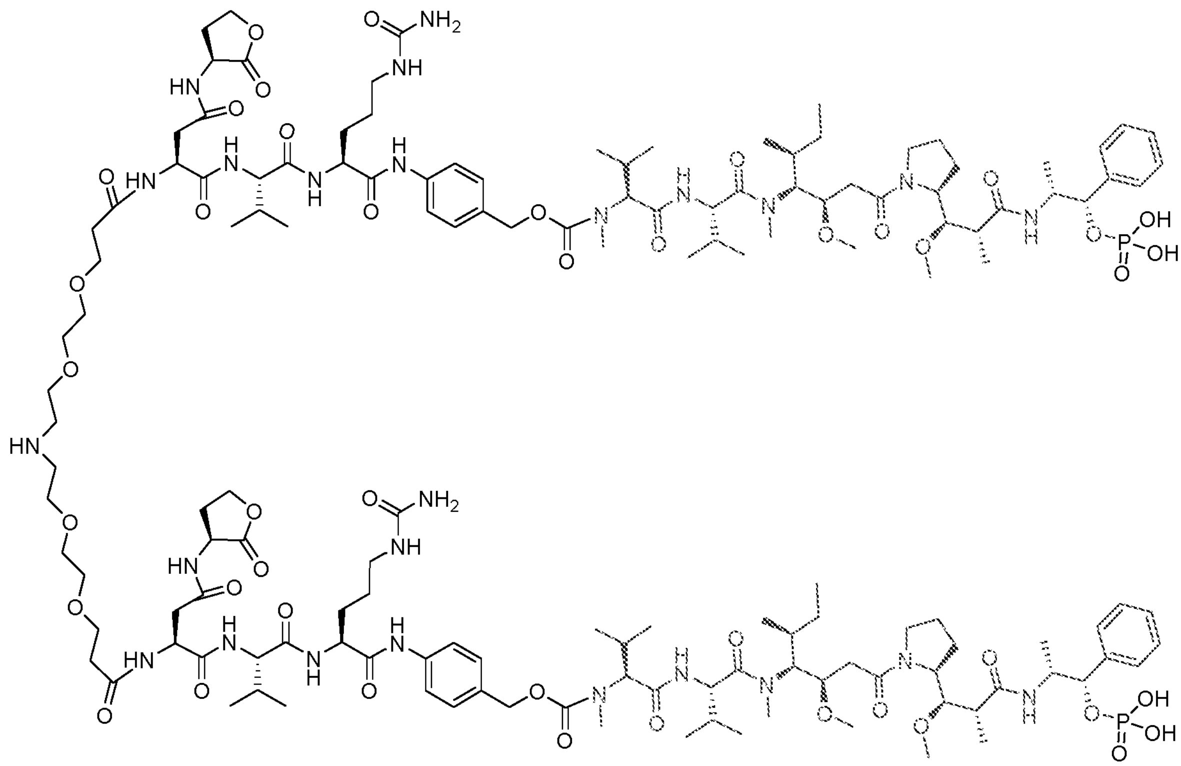

Exemplification 3-3-(1)-B

[C219]

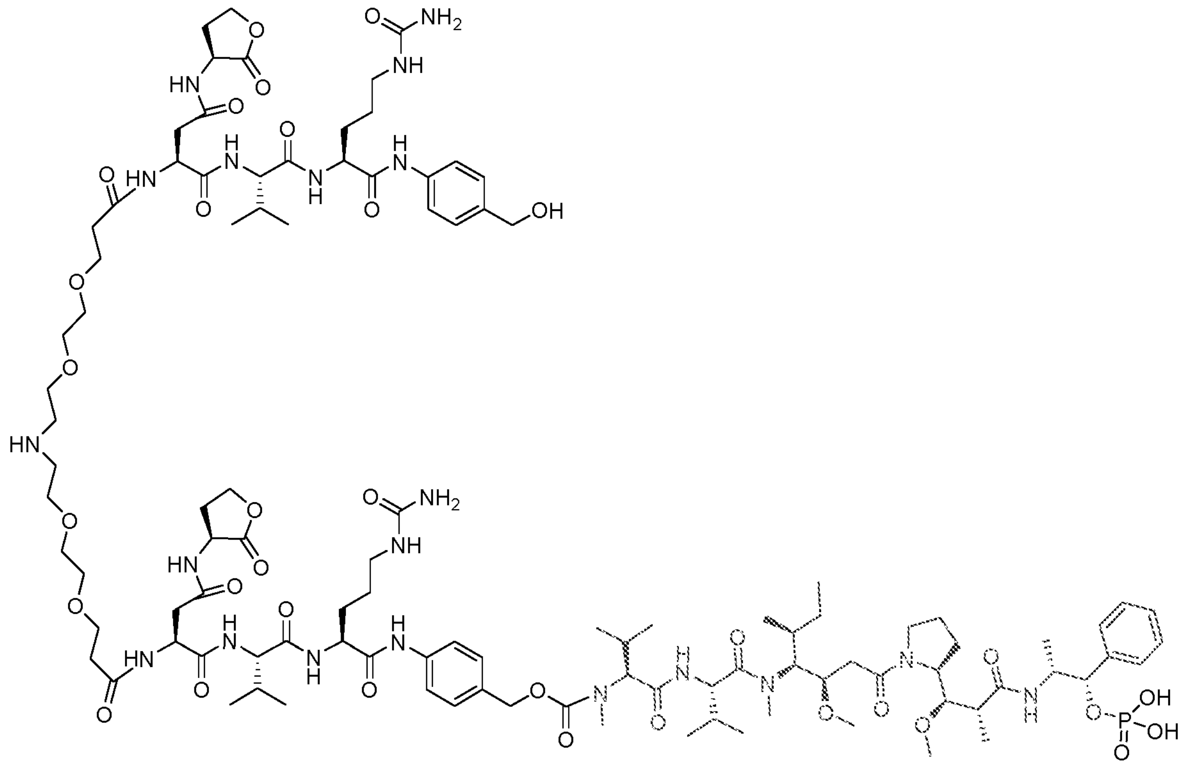

Exemplification 3-3-(3)

[C220]

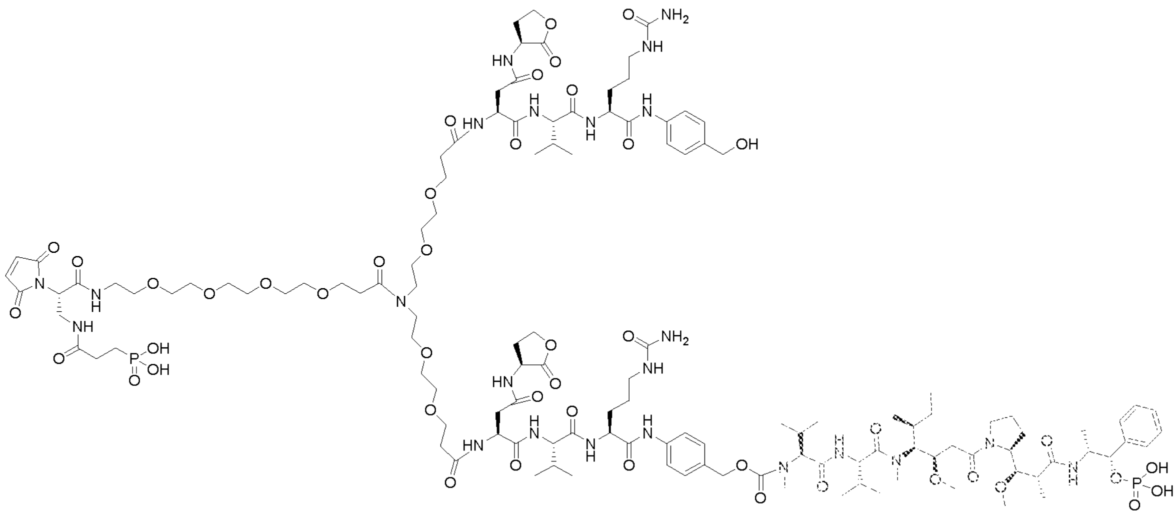

Exemplification 3-4-(1)-A

[C221]

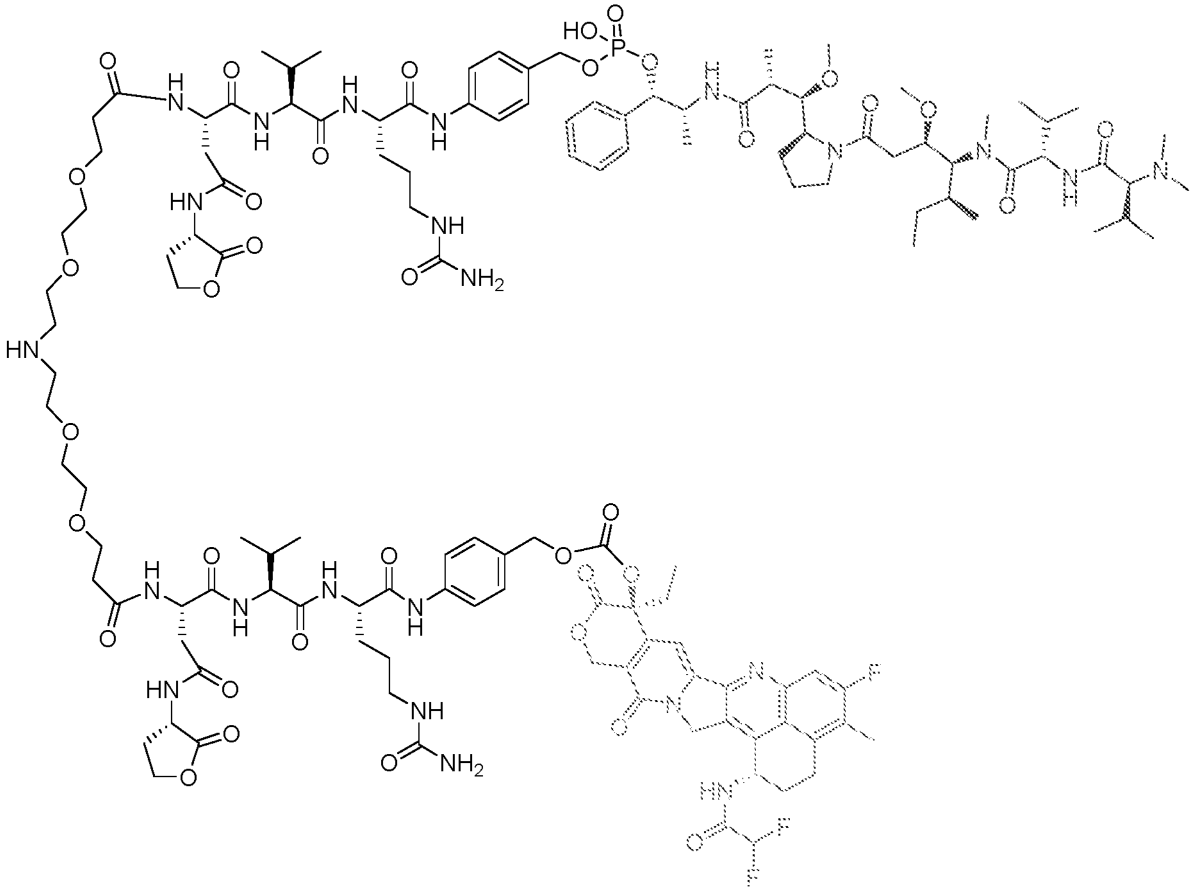

Exemplification 3-4-(1)-B

[C222]

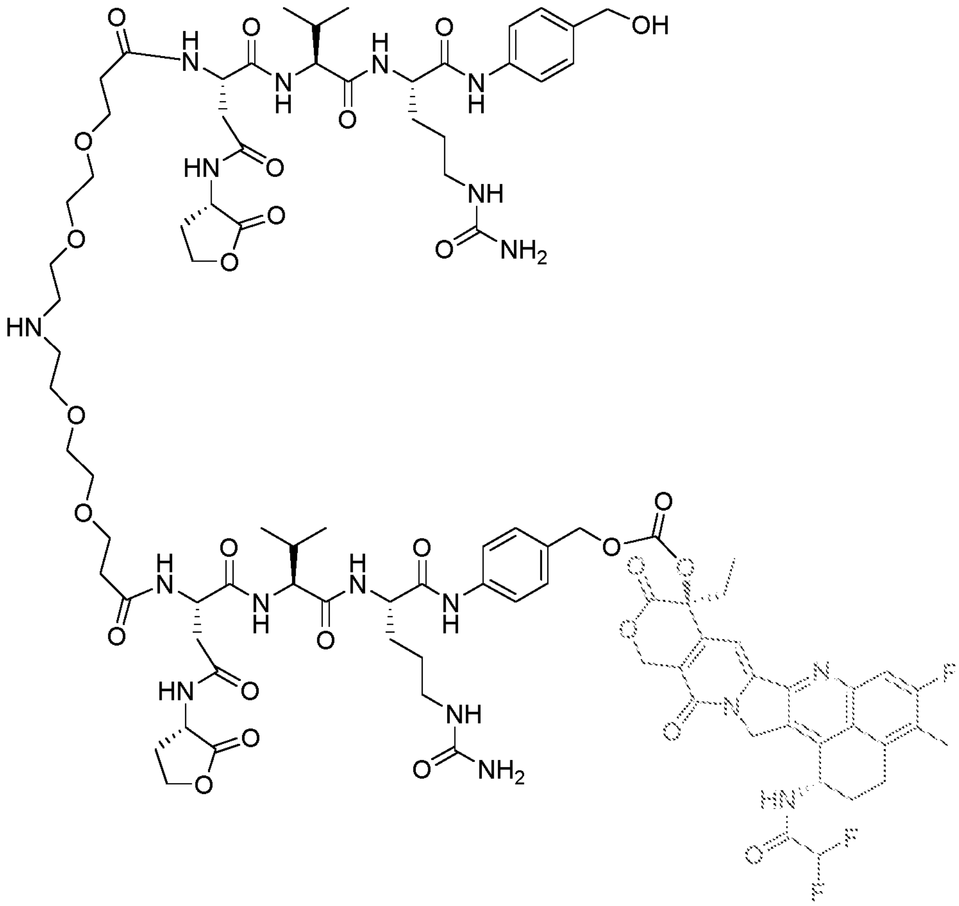

Exemplification 3-4-(3)-A

[C223]

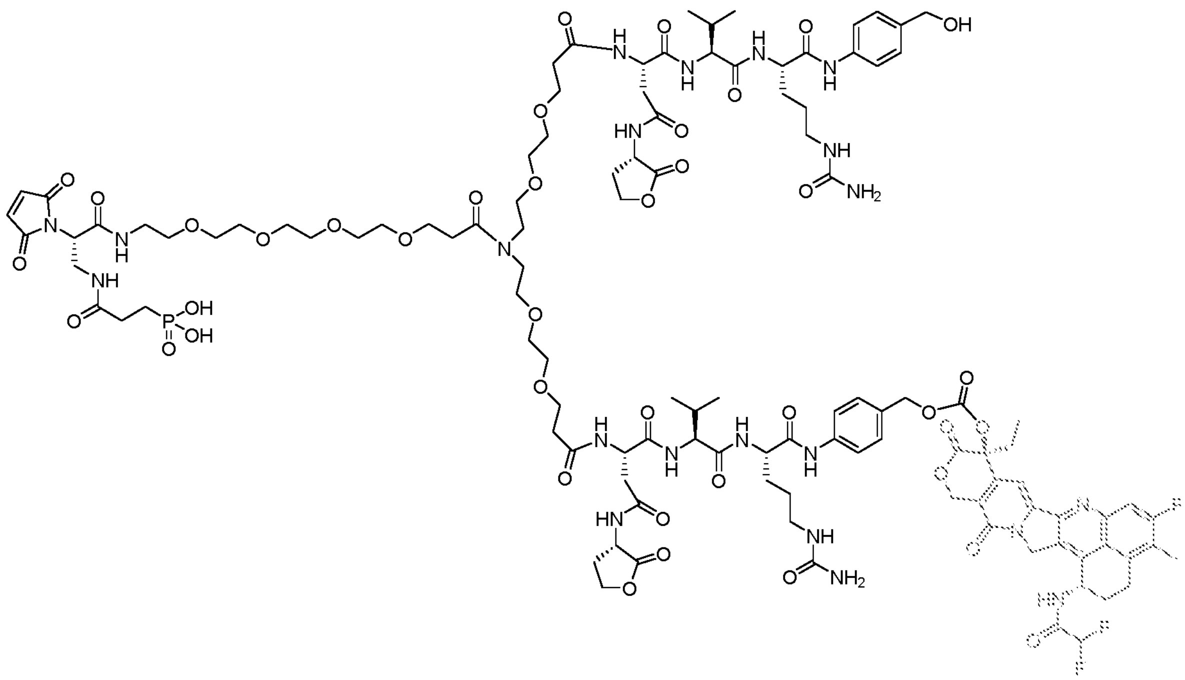

Exemplification 3-5-(1)-A

[C224]

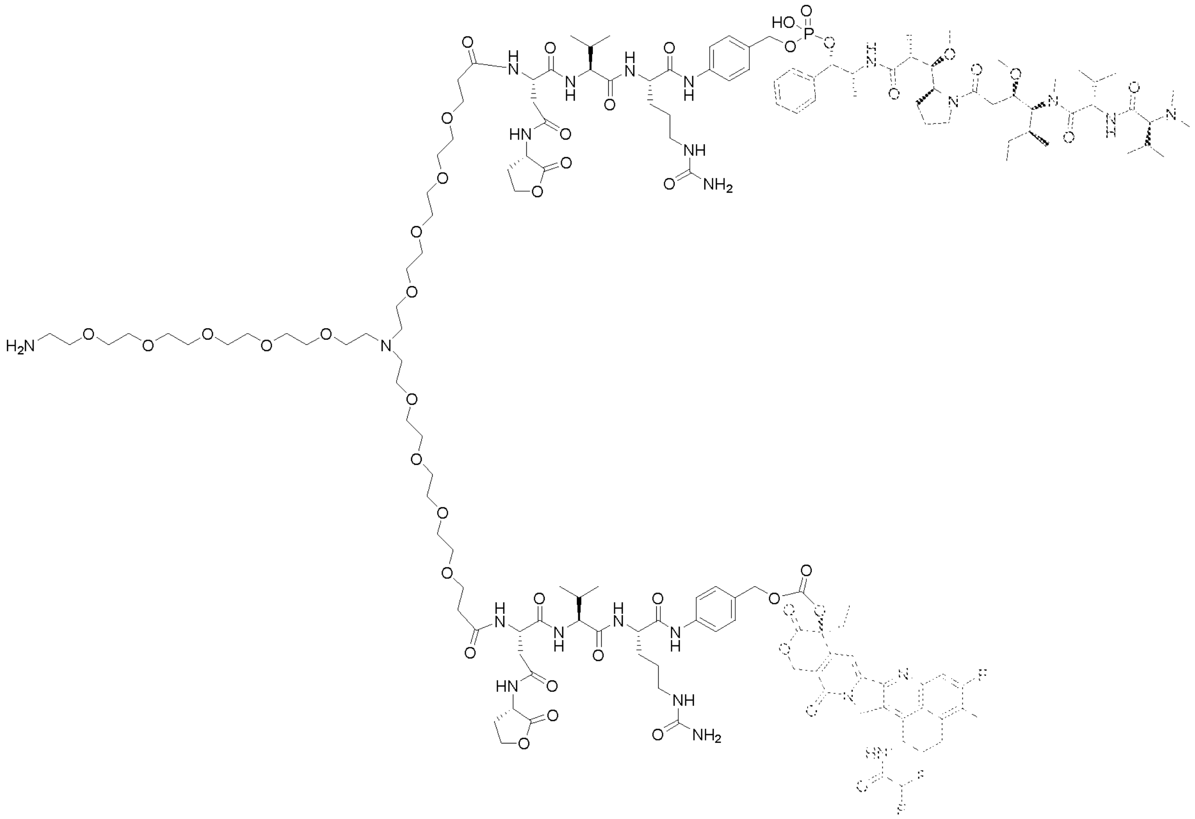

Exemplification 3-5-(1)-B

[C225]

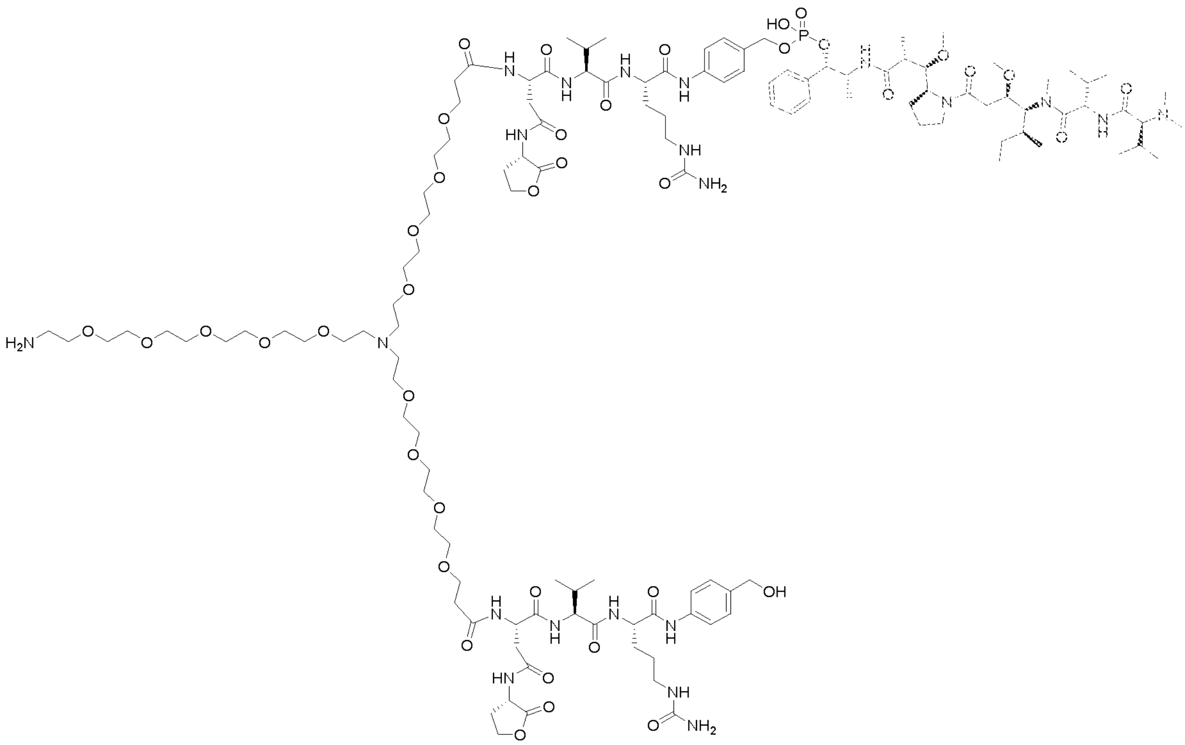

Exemplification 3-5-(1)-C

[C226]

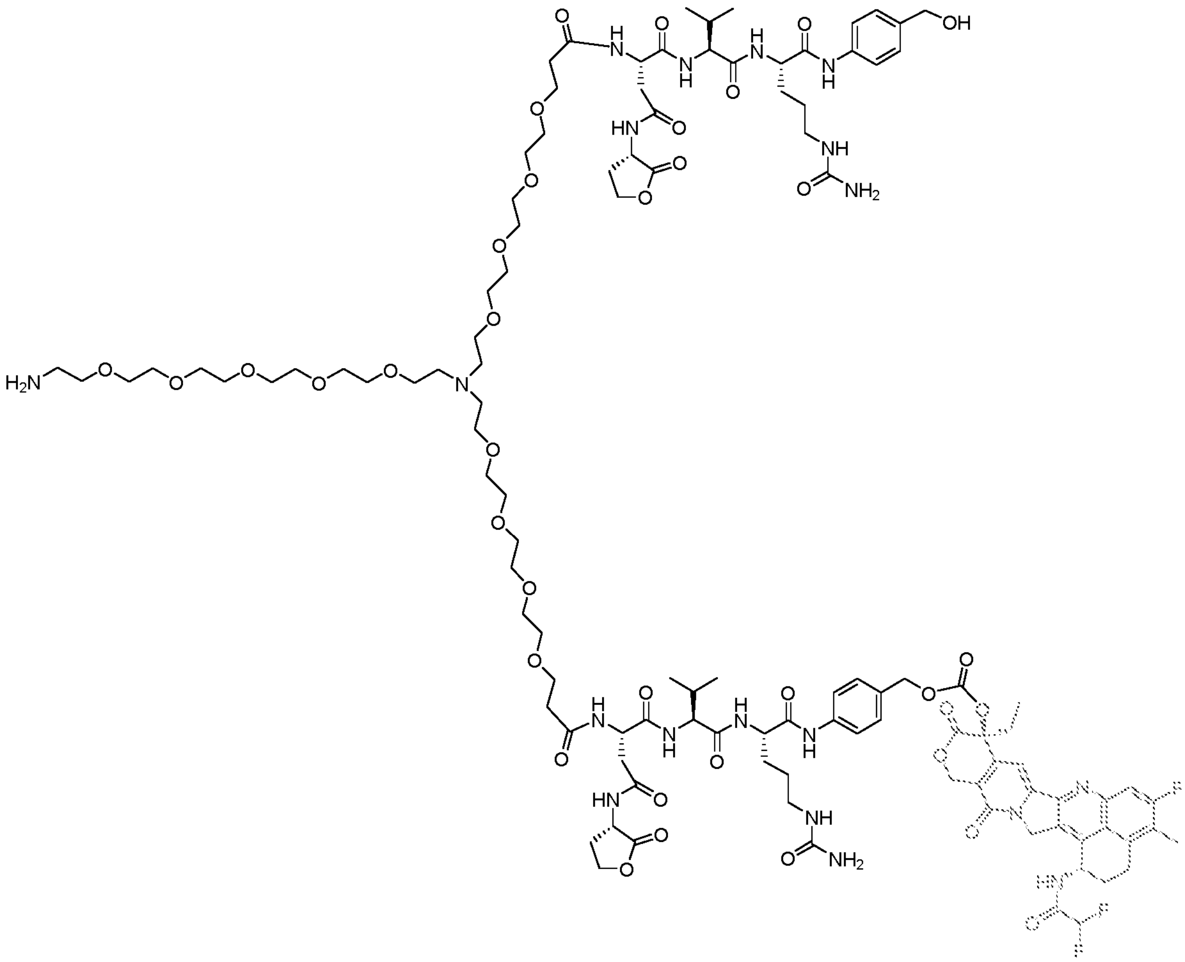

Exemplification 3-5-(2)

[C227]

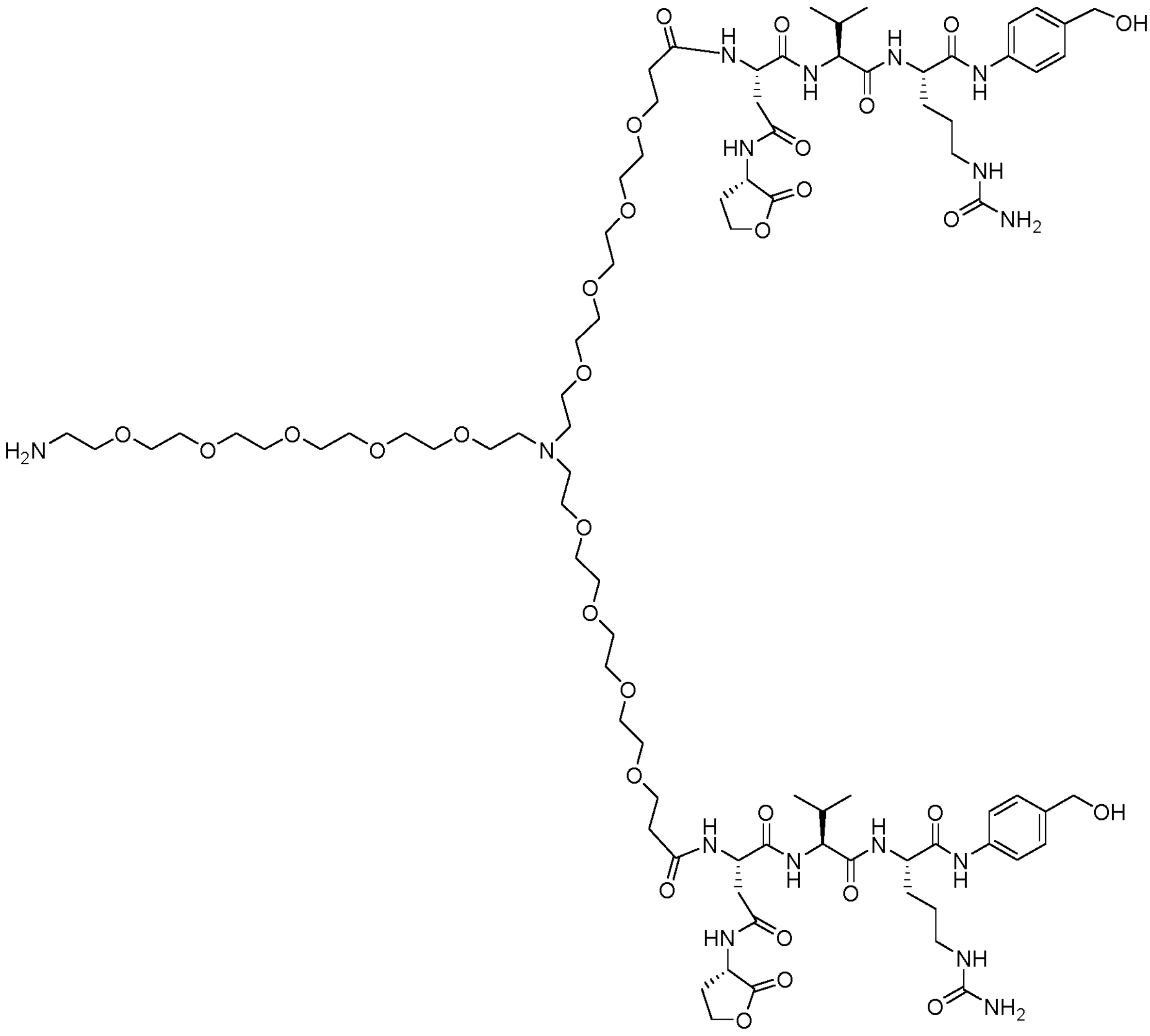

Exemplification 3-5-(3)-A

[C228]

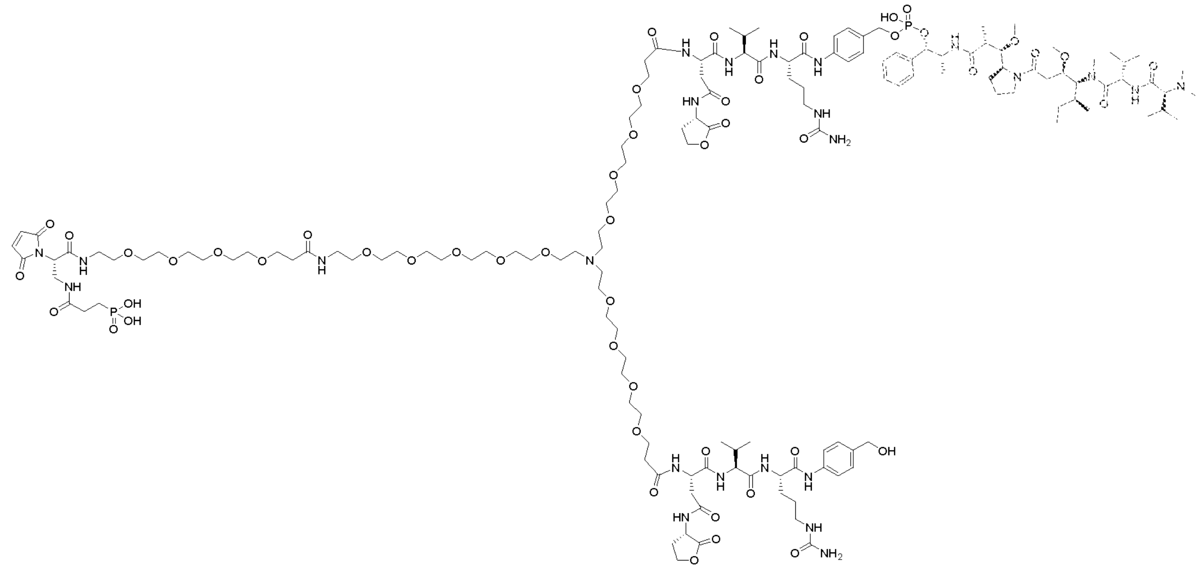

Exemplification 3-5-(3)-B

[C229]

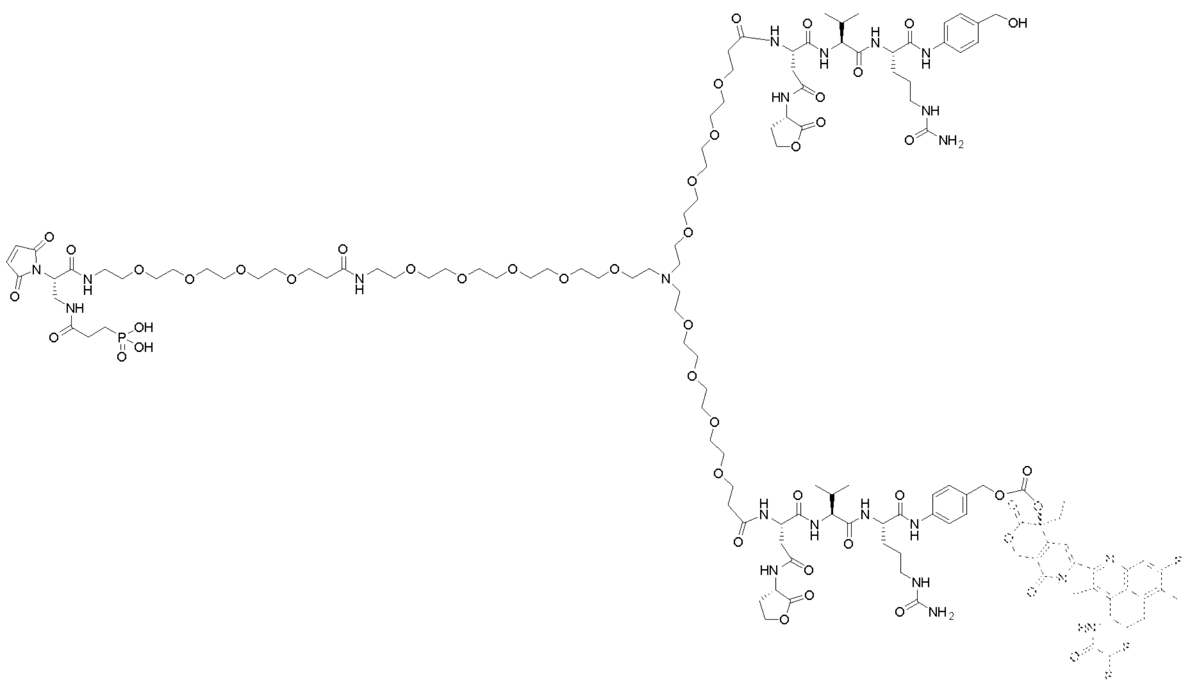

Exemplification 3-5-(3)-C

[C230]

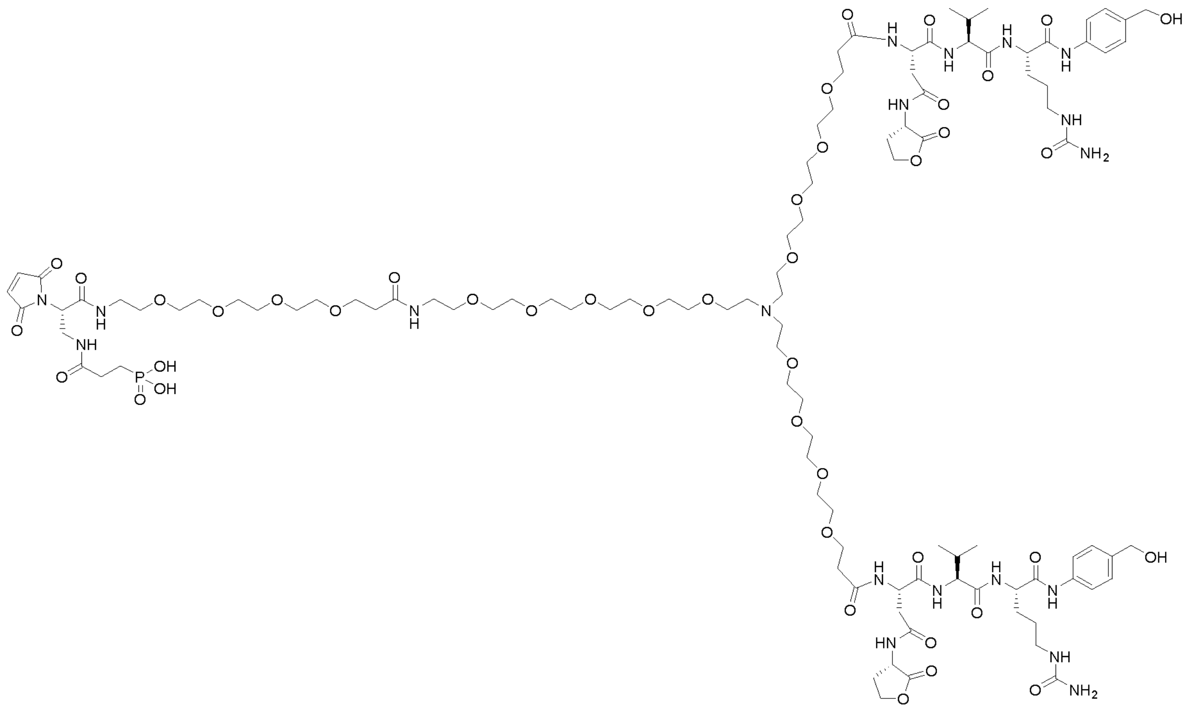

Exemplification 3-6-(1)-A

[C231]

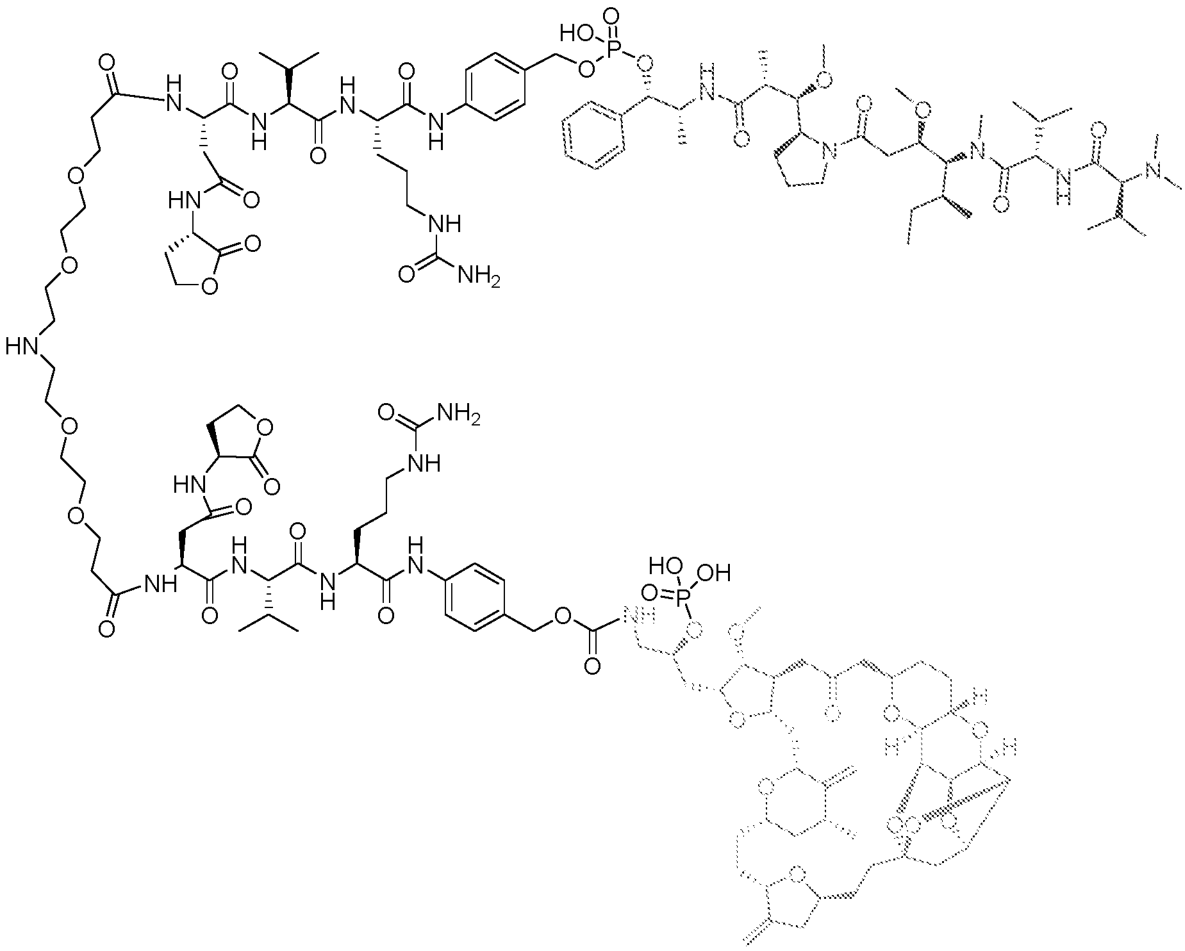

Exemplification 3-6-(1)-B

[C232]

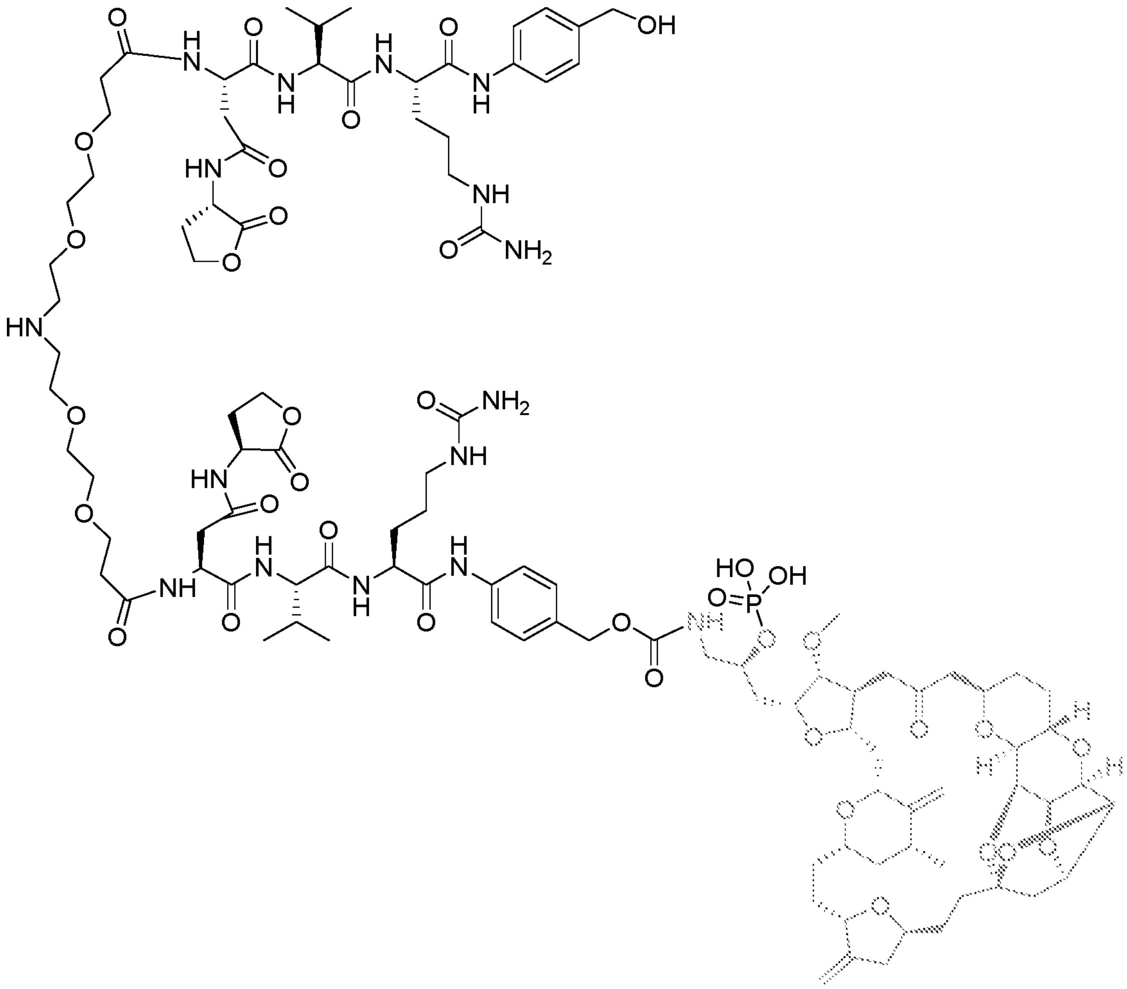

Exemplification 3-6-(3)-A

[C233]

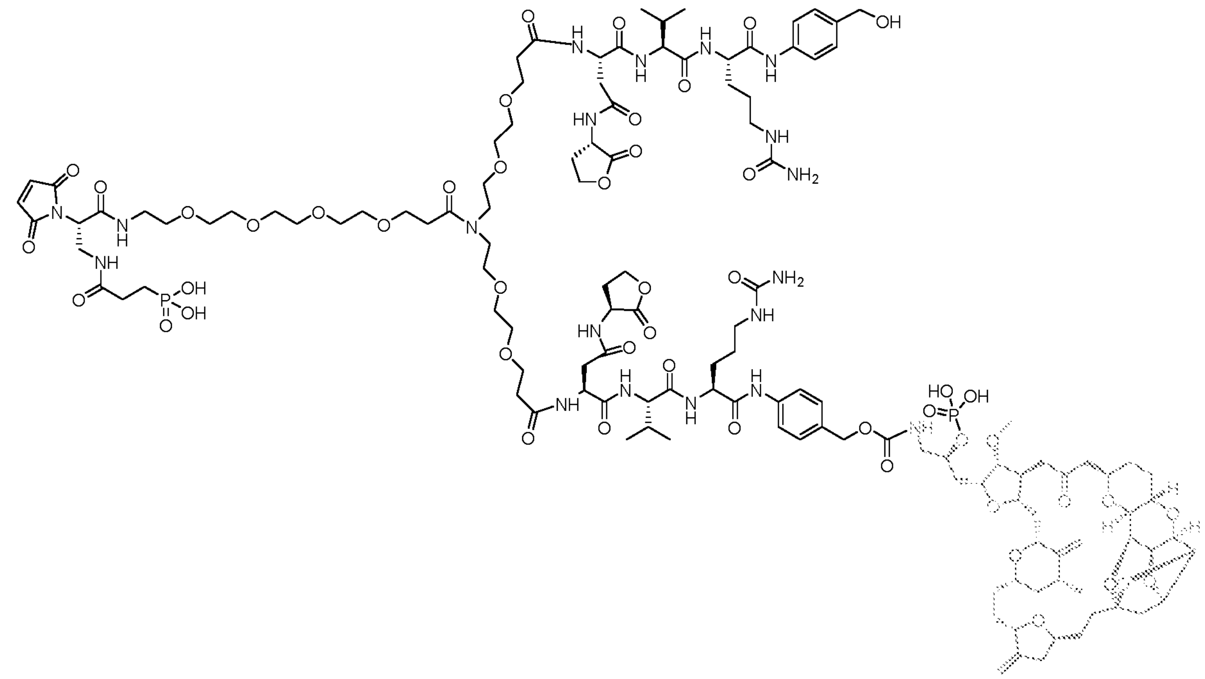

Exemplification 3-7-(1)-A

[C234]

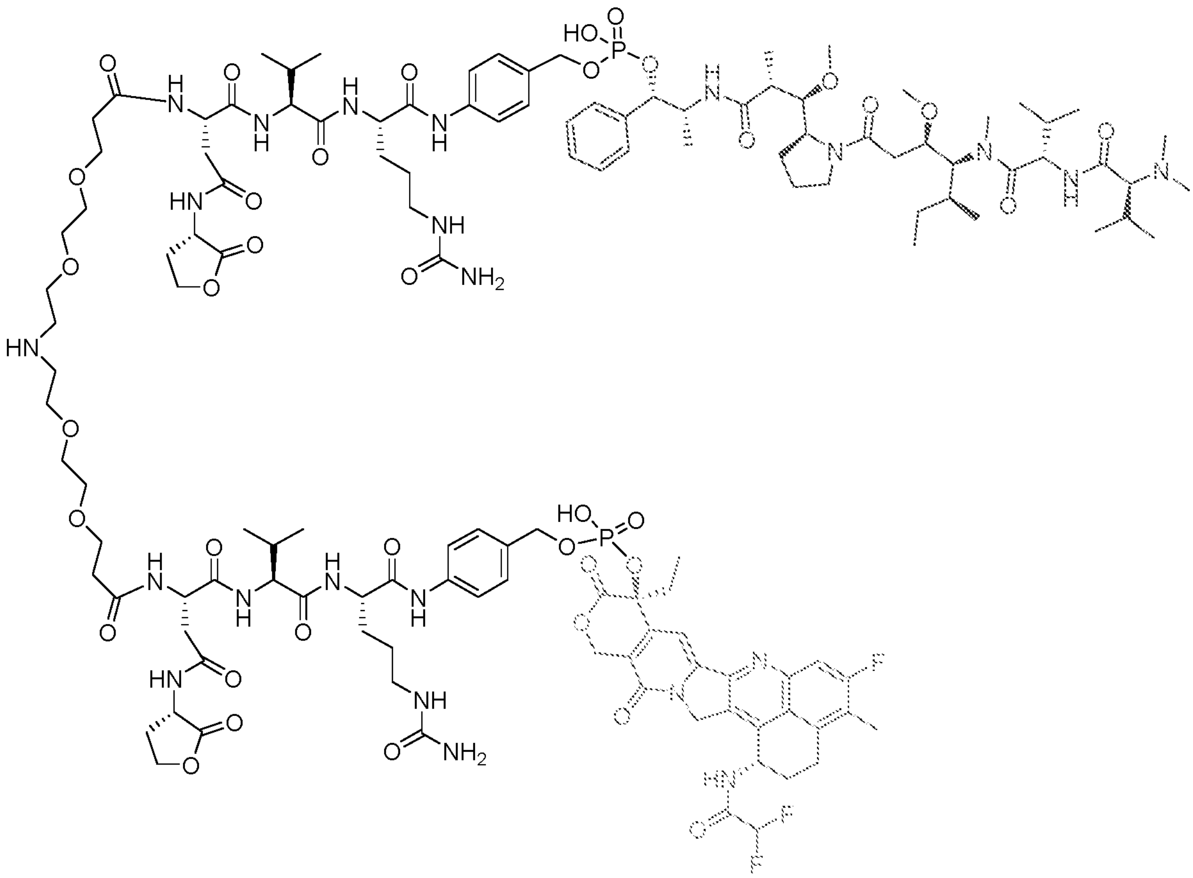

Exemplification 3-7-(1)-B

[C235]

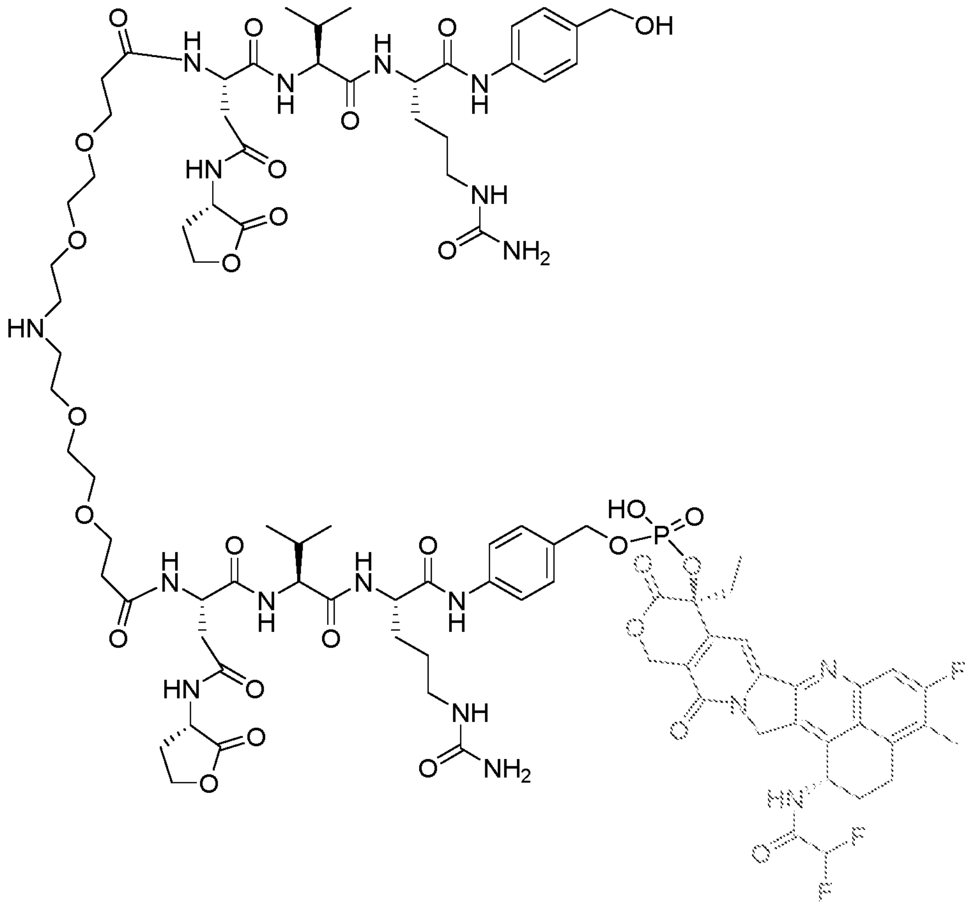

Exemplification 3-7-(3)-A

[C236]

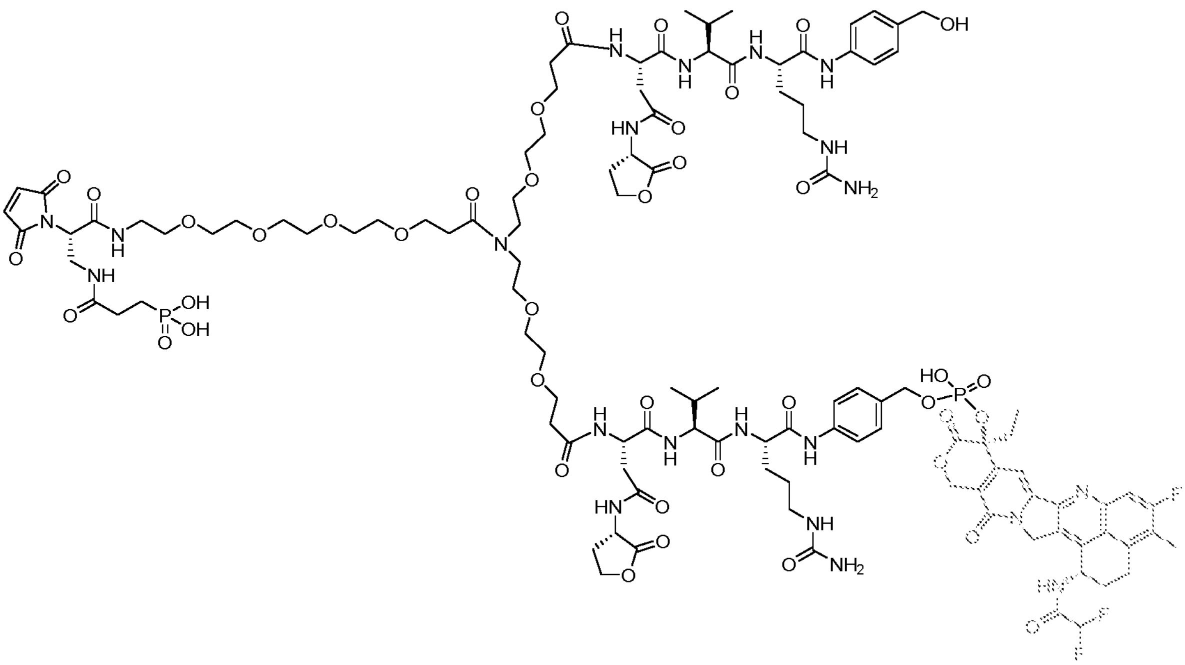

Further compounds of Exemplification 2 (exemplification precursor compounds) may be represented by Formula (X).

[C237]

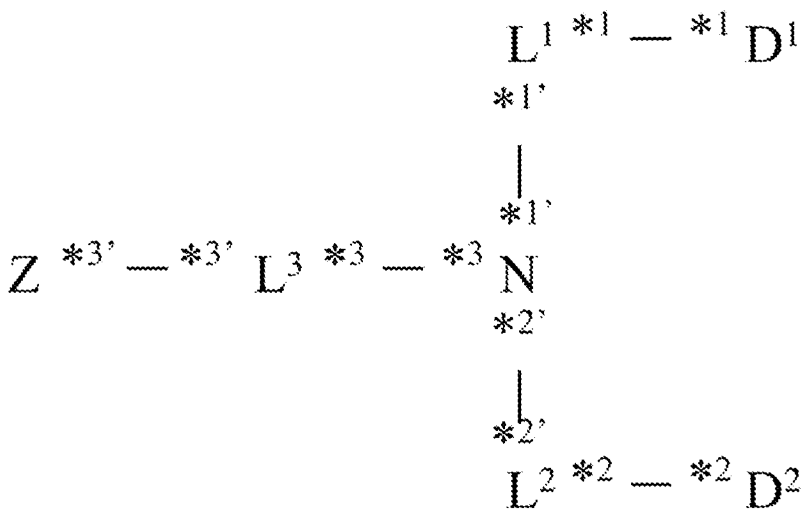

(X)

{where,

Z is Z in the table below,
$L^1$ is any one selected from the group consisting of $L^{1-a}$ to $L^{1-b}$,
$L^2$ is any one selected from the group consisting of $L^{2-a}$ to $L^{2-d}$,
$L^3$ is any one selected from the group consisting of $L^{3-a}$ to $L^{3-i}$,
$D^1$ is any one selected from the group consisting of $D^{1-a}$ to $D^{1-b}$,
$D^2$ is any one selected from the group consisting of $D^{2-aa}$ to $D^{2-at}$, and
*1 is linked to *1, *1’ is linked to *1’, *2 is linked to *2, *2’ is linked to *2’, *3 is linked to *3, and *3’ is linked to *3’}.

An additional exemplification precursor compound is represented by Formula (XI).

[C238]

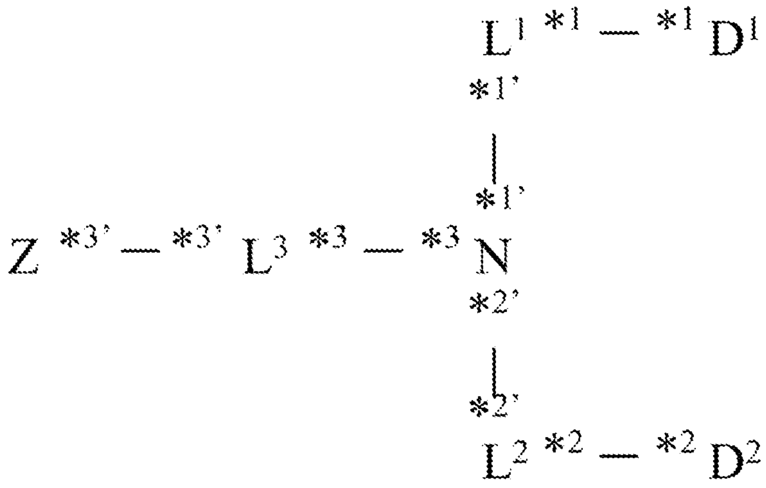

(XI)

{where,

Z is Z in the table below,
$L^1$ is any one selected from the group consisting of $L^{1-a}$ to $L^{1-b}$,
$L^2$ is any one selected from the group consisting of $L^{2-b}$ to $L^{2-h}$,
$L^3$ is any one selected from the group consisting of $L^{3-a}$ to $L^{3-i}$,
$D^1$ is any one selected from the group consisting of $D^{1-a}$ to $D^{1-b}$,
$D^2$ is any one selected from the group consisting of $D^{2-ba}$ to $D^{2-bg}$, and
*1 is linked to *1, *1' is linked to *1', *2 is linked to *2, *2' is linked to *2', *3 is linked to *3, and *3' is linked to *3'}.

An additional exemplification precursor compound is represented by Formula (XII).

[C239]

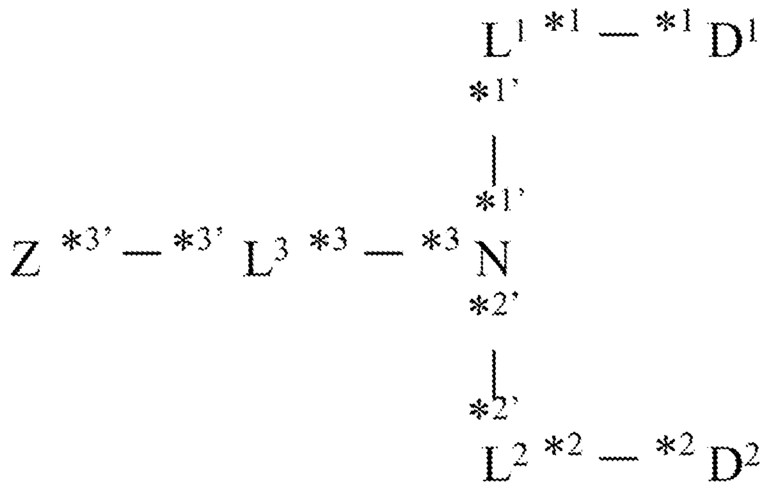

(XII)

{where,

Z is Z in the table below,

$L^1$ is any one selected from the group consisting of $L^{1\text{-}a}$ to $L^{1\text{-}b}$,
$L^2$ is any one selected from the group consisting of $L^{2\text{-}b}$, $L^{2\text{-}b}$, $L^{2\text{-}i}$ to $L^{2\text{-}n}$,
$L^3$ is any one selected from the group consisting of $L^{3\text{-}a}$ to $L^{3\text{-}i}$,
$D^1$ is any one selected from the group consisting of $D^{1\text{-}a}$ to $D^{1\text{-}b}$,
$D^2$ is any one selected from the group consisting of $D^{2\text{-}ca}$ to $D^{2\text{-}ch}$, and
*1 is linked to *1, *1' is linked to *1', *2 is linked to *2, *2' is linked to *2', *3 is linked to *3, and *3' is linked to *3'}.

An additional exemplification precursor compound is represented by Formula (XIII).

[C240]

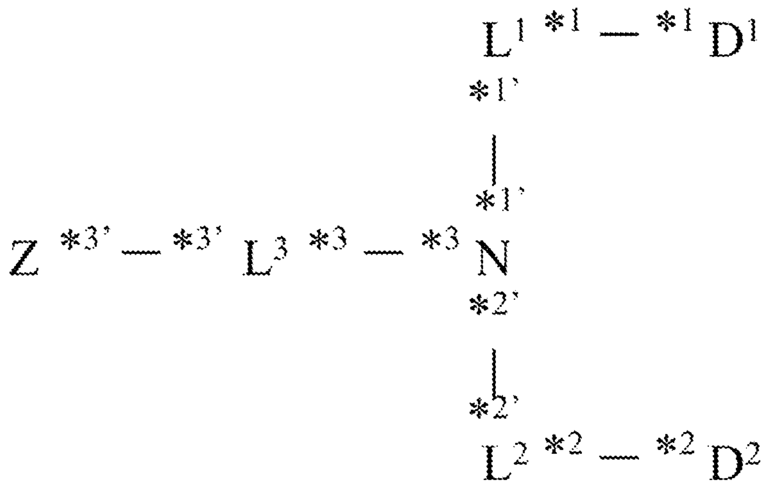

(XIII)

{where,

Z is Z in the table below,
$L^1$ is any one selected from the group consisting of $L^{1\text{-}a}$ to $L^{1\text{-}b}$,
$L^2$ is any one selected from the group consisting of $L^{2\text{-}o}$ to $L^{2\text{-}r}$,
$L^3$ is any one selected from the group consisting of $L^{3\text{-}a}$ to $L^{3\text{-}e}$, and $L^{3\text{-}j}$ to $L^{3\text{-}m}$,
$D^1$ is any one selected from the group consisting of $D^{1\text{-}a}$ to $D^{1\text{-}b}$,
$D^2$ is $D^{2\text{-}da}$. and
*1 is linked to *1, *1' is linked to *1', *2 is linked to *2, *2' is linked to *2', *3 is linked to *3, and *3' is linked to *3'}.

[Table 1]

| Name of group | Structure |
|---|---|
| Z | 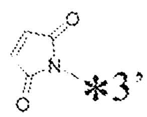 |

(continued)

| Name of group | Structure |
|---|---|
| $L^{1-a}$ | 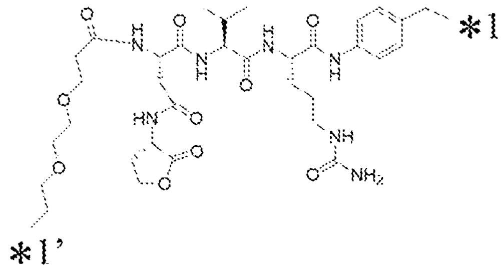 $L^{1-a}$ |
| $L^{1-b}$ | 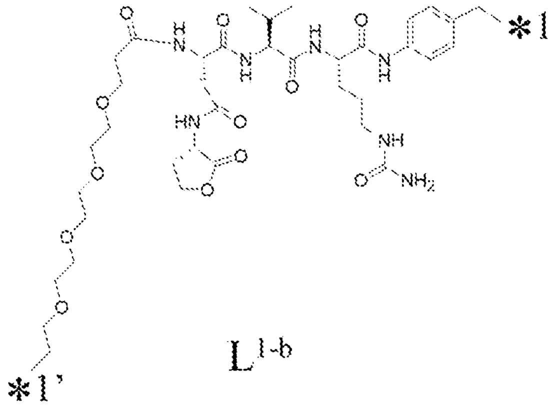 $L^{1-b}$ |
| $L^{2-a}$ | 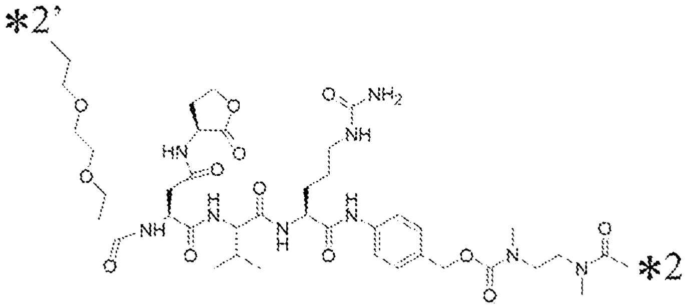 $L^{2-a}$ |
| $L^{2-b}$ | 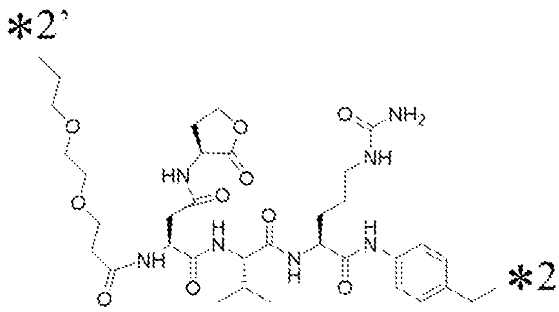 $L^{2-b}$ |

(continued)

| Name of group | Structure |
|---|---|
| $L^{2-c}$ | 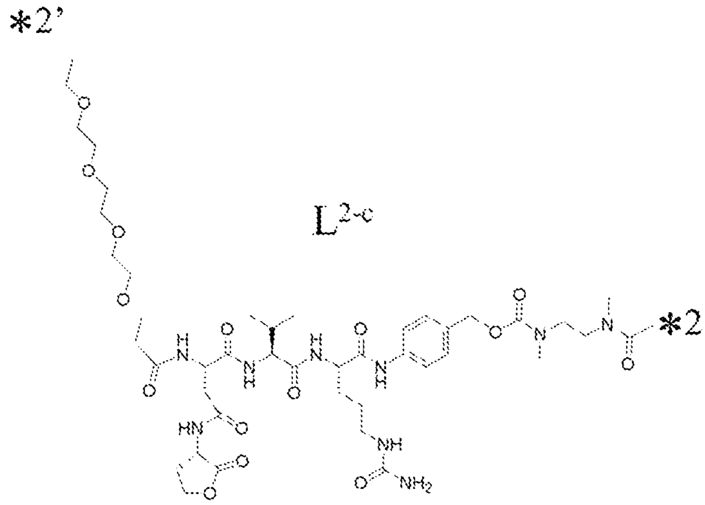 |
| $L^{2-d}$ | 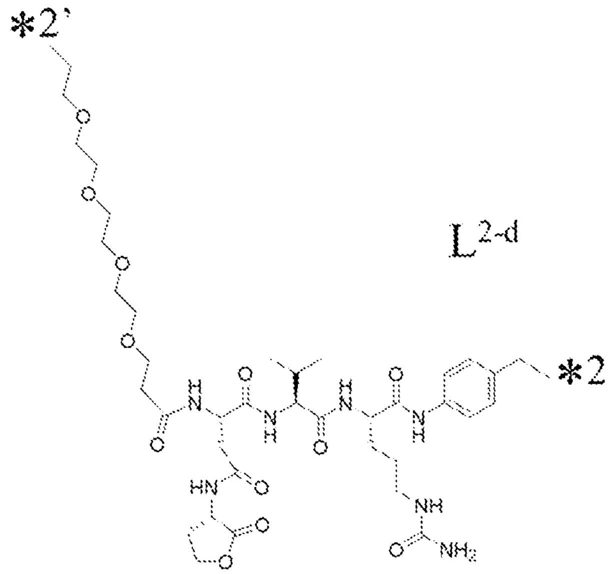 |
| $L^{2-e}$ | 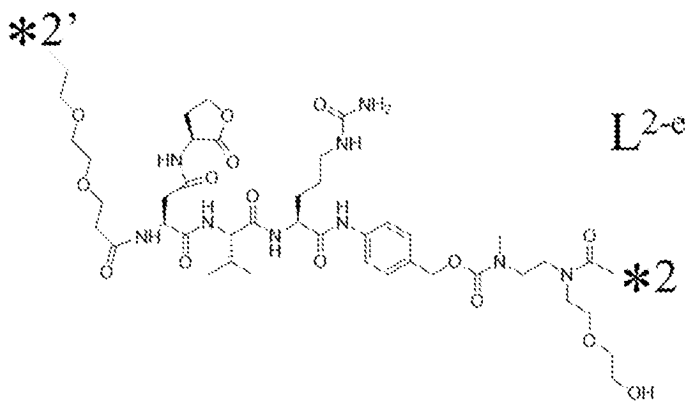 |

(continued)

| Name of group | Structure |
|---|---|
| $L^{2-f}$ | 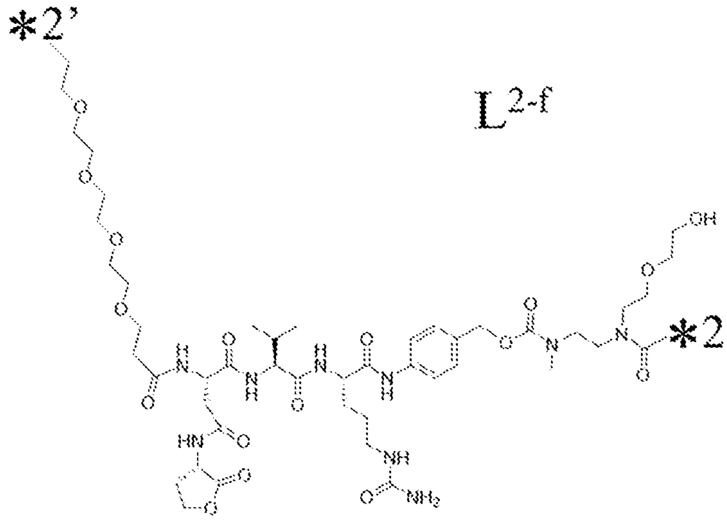 |
| $L^{2-g}$ | 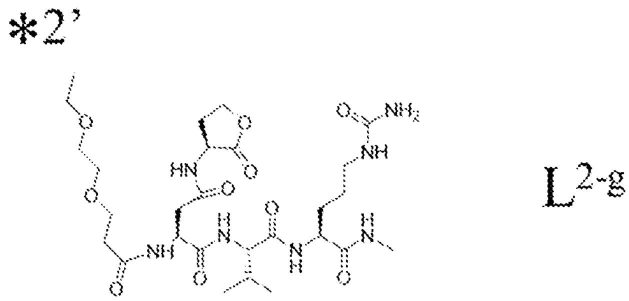 |
| $L^{2-h}$ | 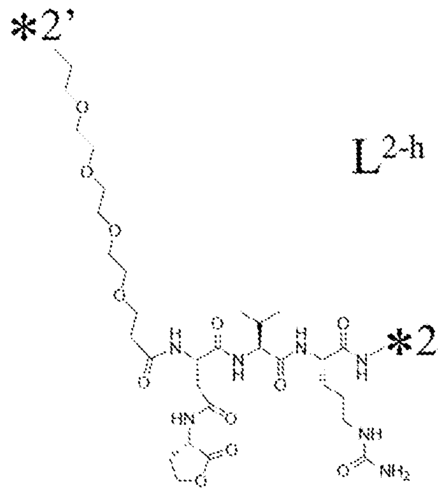 |
| $L^{2-i}$ | 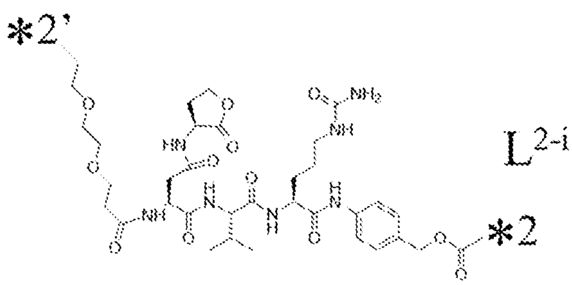 |

(continued)

| Name of group | Structure |
|---|---|
| $L^{2\text{-}j}$ | 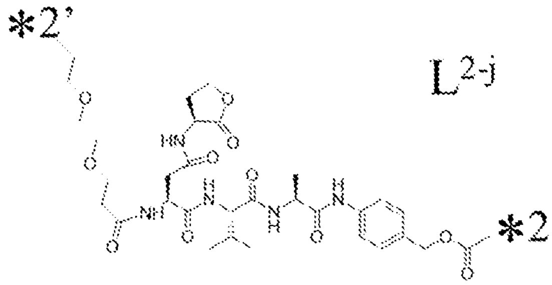 |
| $L^{2\text{-}k}$ | 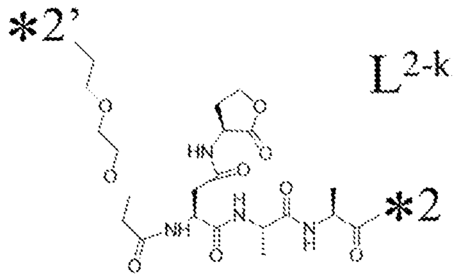 |
| $L^{2\text{-}l}$ | 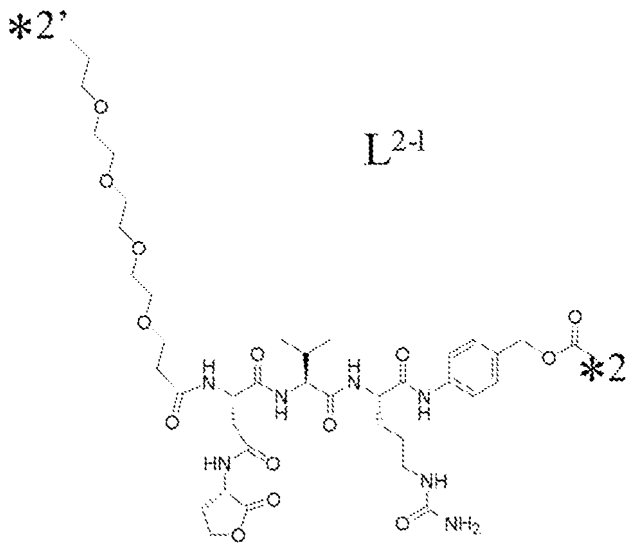 |
| $L^{2\text{-}m}$ | 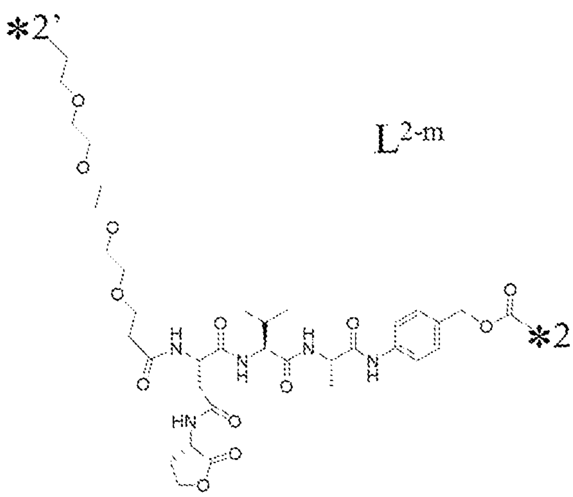 |

(continued)

| Name of group | Structure |
|---|---|
| $L^{2-n}$ | 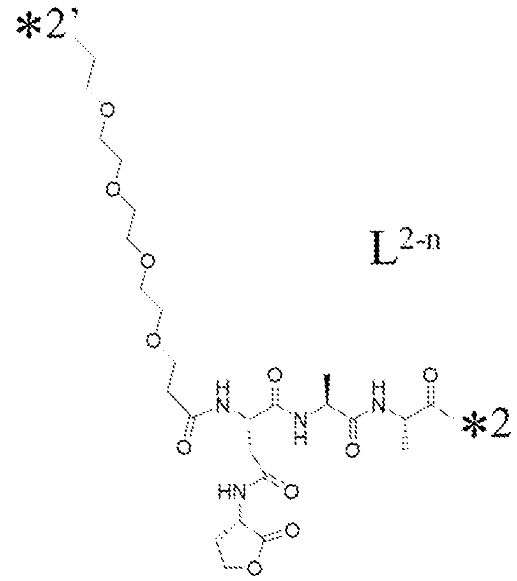 |
| $L^{2-o}$ | 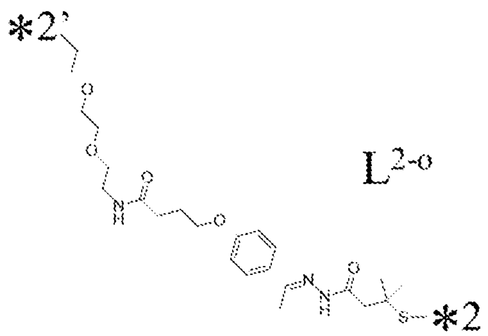 |
| $L^{2-p}$ | 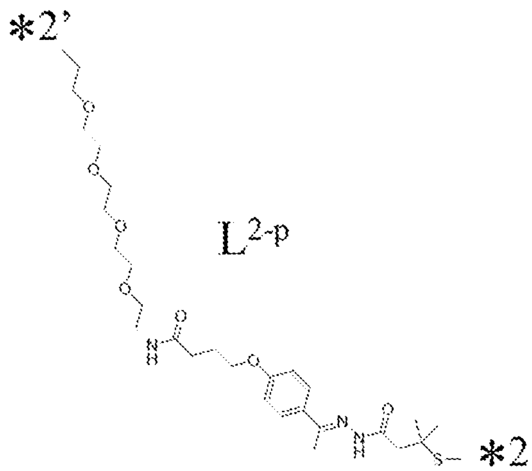 |

(continued)

| Name of group | Structure |
|---|---|
| $L^{2-q}$ | 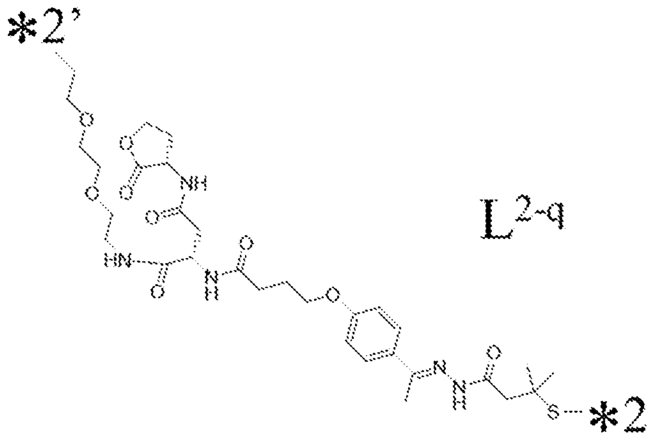 |
| $L^{2-r}$ | 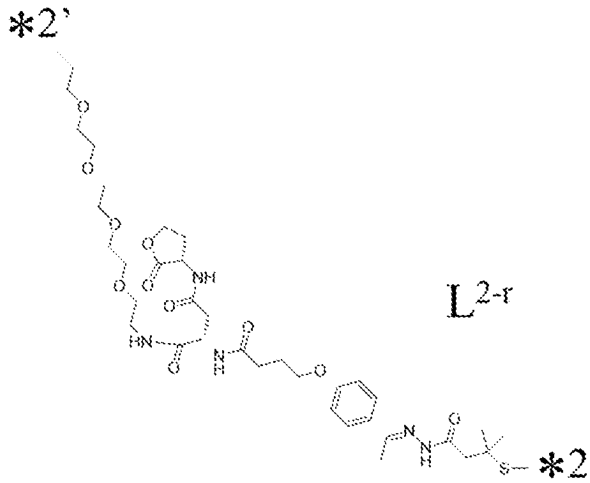 |
| $L^{3-a}$ | 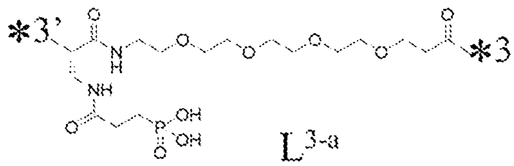 |
| $L^{3-b}$ | 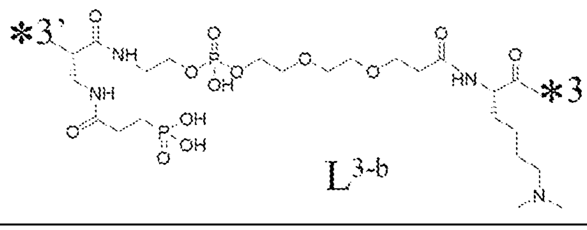 |
| $L^{3-c}$ | 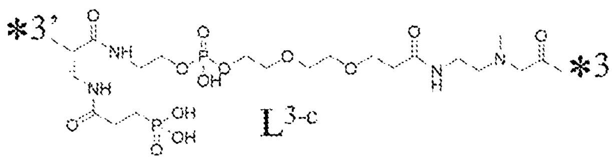 |

(continued)

| Name of group | Structure |
| --- | --- |
| $L^{3-d}$ | 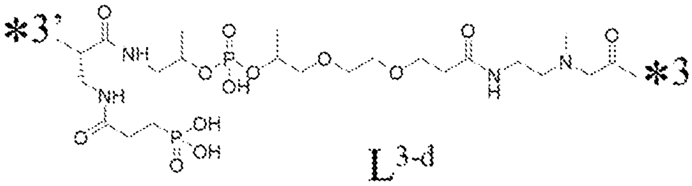 $L^{3-d}$ |
| $L^{3-e}$ | 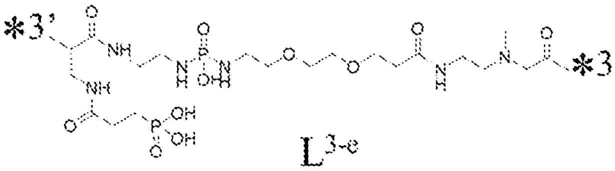 $L^{3-e}$ |
| $L^{3-f}$ | 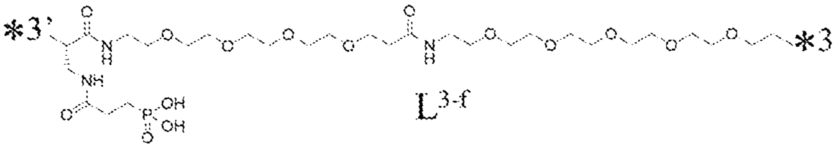 $L^{3-f}$ |
| $L^{3-g}$ | 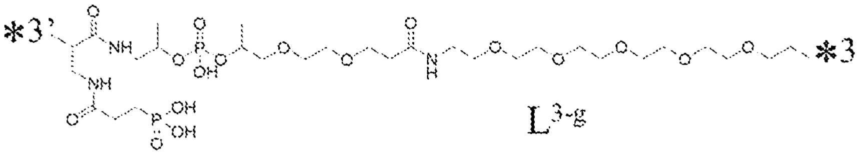 $L^{3-g}$ |
| $L^{3-h}$ | 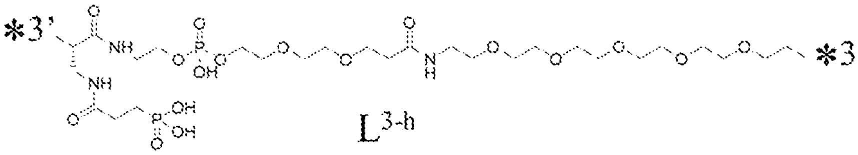 $L^{3-h}$ |
| $L^{3-i}$ | 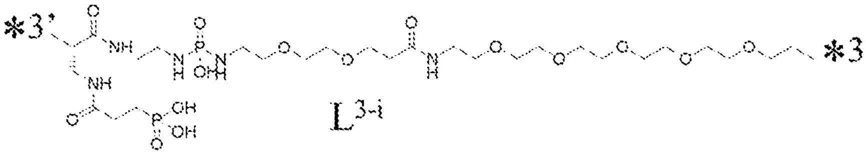 $L^{3-i}$ |
| $L^{3-j}$ | 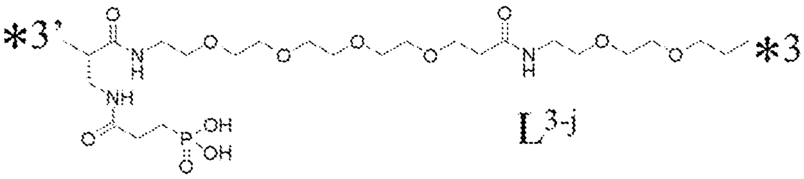 $L^{3-j}$ |
| $L^{3-k}$ | 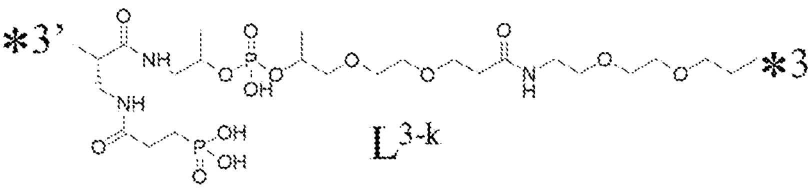 $L^{3-k}$ |
| $L^{3-l}$ | 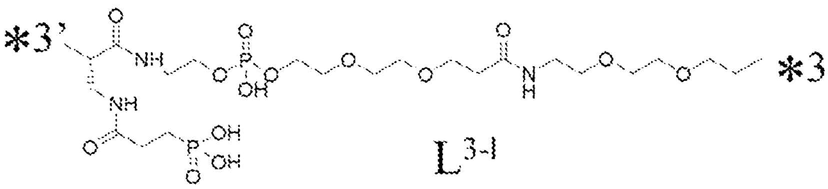 $L^{3-l}$ |

(continued)

| Name of group | Structure |
|---|---|
| $L^{3-m}$ | 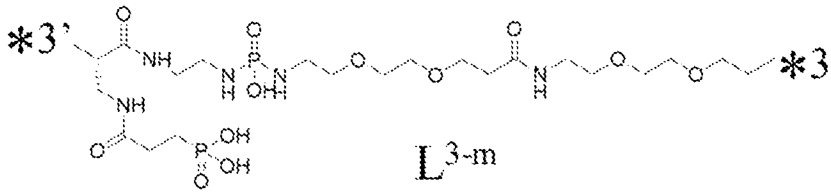 $L^{3-m}$ |
| $D^{1-a}$ | 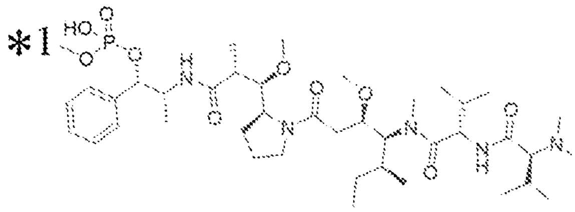 $D^{1-a}$ |
| $D^{1-b}$ | 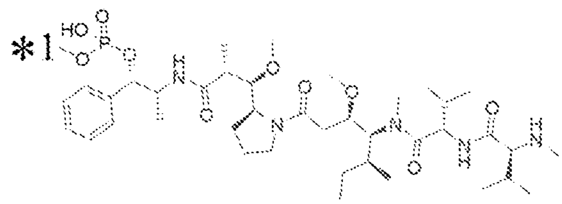 $D^{1-b}$ |
| $D^{2-aa}$ | 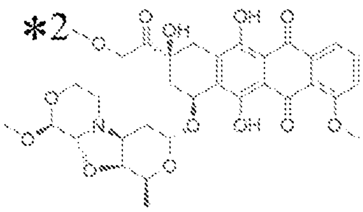 $D^{2-aa}$ |
| $D^{2-ab}$ | 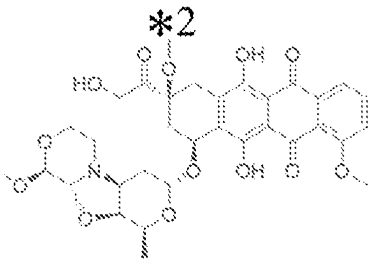 $D^{2-ab}$ |
| $D^{2-ac}$ | 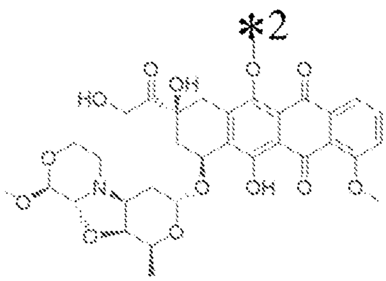 $D^{2-ac}$ |

(continued)

| Name of group | Structure |
|---|---|
| $D^{2\text{-}ad}$ | 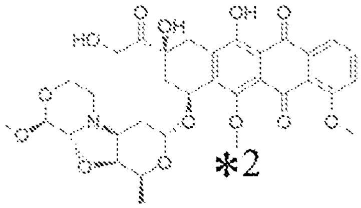 $D^{2\text{-}ad}$ |
| $D^{2\text{-}ae}$ | 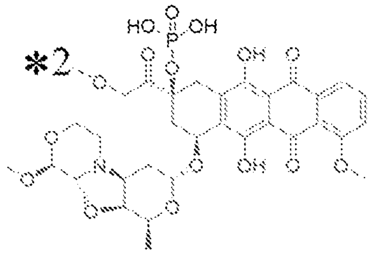 $D^{2\text{-}ae}$ |
| $D^{2\text{-}af}$ | 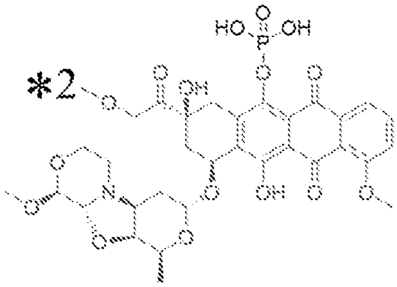 $D^{2\text{-}af}$ |
| $D^{2\text{-}ag}$ | 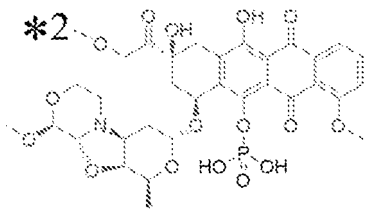 $D^{2\text{-}ag}$ |
| $D^{2\text{-}ah}$ | 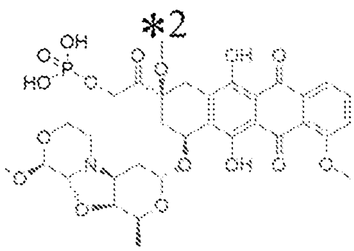 $D^{2\text{-}ah}$ |
| $D^{2\text{-}ai}$ | 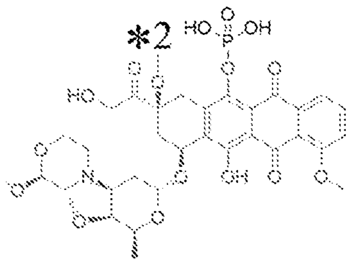 $D^{2\text{-}ai}$ |

(continued)

| Name of group | Structure |
|---|---|
| $D^{2-aj}$ | 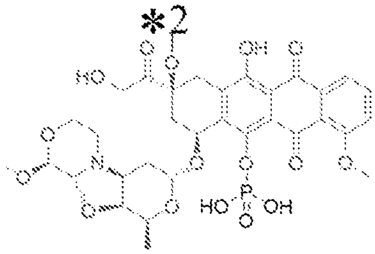 $D^{2-aj}$ |
| $D^{2-ak}$ | 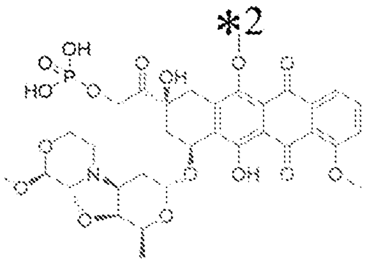 $D^{2-ak}$ |
| $D^{2-al}$ | 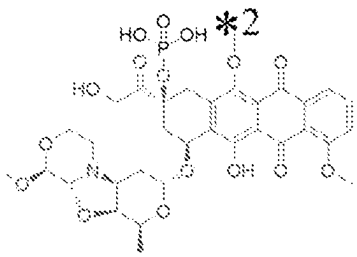 $D^{2-al}$ |
| $D^{2-am}$ | 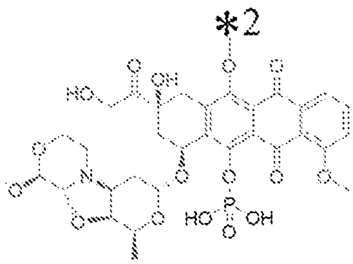 $D^{2-am}$ |
| $D^{2-an}$ | 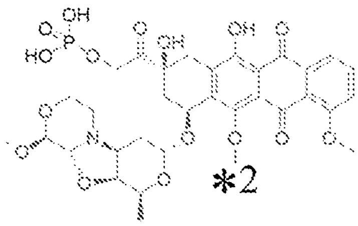 $D^{2-an}$ |
| $D^{2-ao}$ | 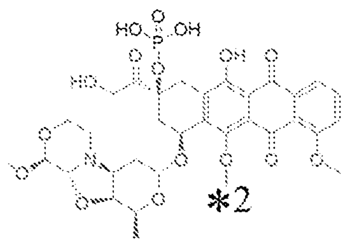 $D^{2-ao}$ |

(continued)

| Name of group | Structure |
|---|---|
| D$^{2-ap}$ | 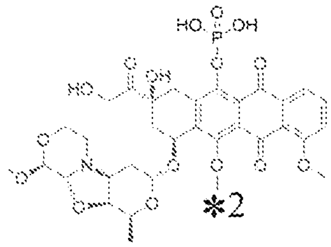 D$^{2-ap}$ |
| D$^{2-aq}$ | 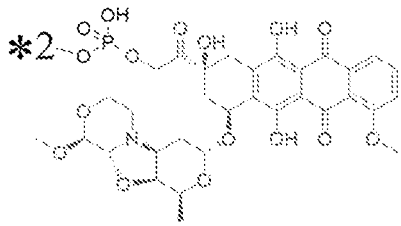 D$^{2-aq}$ |
| D$^{2-ar}$ | 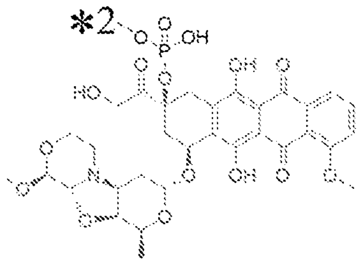 D$^{2-ar}$ |
| D$^{2-as}$ | 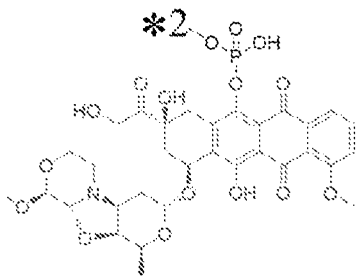 D$^{2-as}$ |
| D$^{2-at}$ | 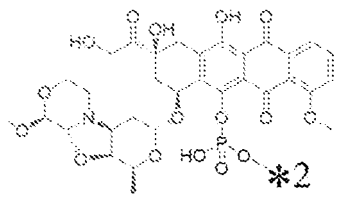 D$^{2-at}$ |
| D$^{2-ba}$ | 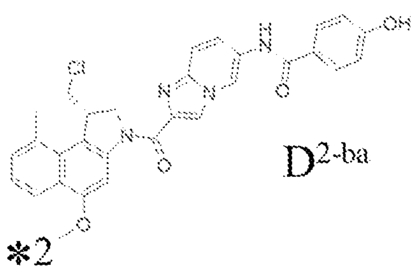 D$^{2-ba}$ |

(continued)

| Name of group | Structure |
|---|---|
| $D^{2-bb}$ | 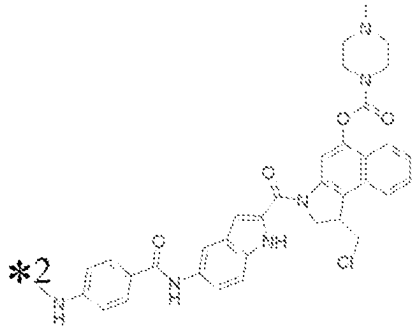 $D^{2-bb}$ |
| $D^{2-be}$ | 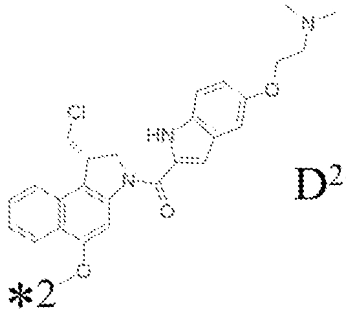 $D^{2-bc}$ |
| $D^{2-bd}$ | 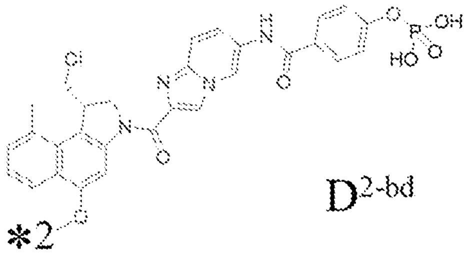 $D^{2-bd}$ |
| $D^{2-be}$ | 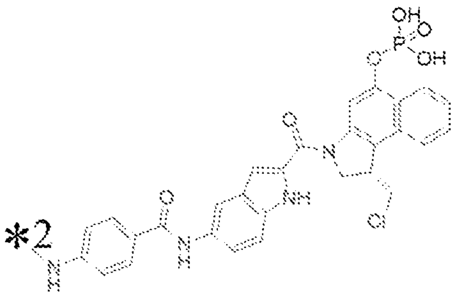 $D^{2-be}$ |
| $D^{2-bf}$ | 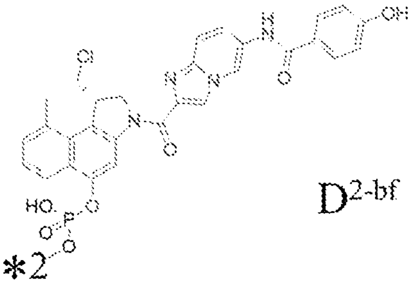 $D^{2-bf}$ |

(continued)

| Name of group | Structure |
|---|---|
| $D^{2\text{-bg}}$ | 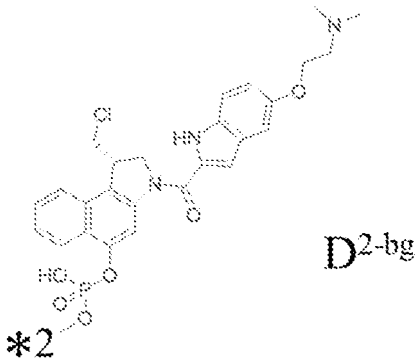 $D^{2\text{-bg}}$ |
| $D^{2\text{-ca}}$ | 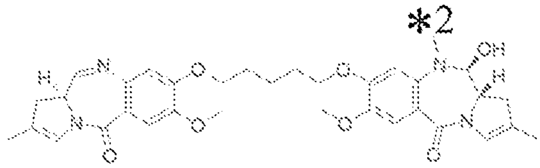 $D^{2\text{-ca}}$ |
| $D^{2\text{-cb}}$ | 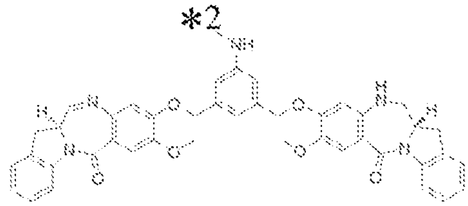 $D^{2\text{-cb}}$ |
| $D^{2\text{-cc}}$ | 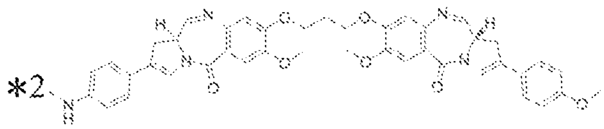 $D^{2\text{-cc}}$ |
| $D^{2\text{-cd}}$ | 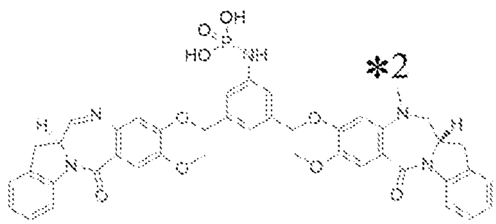 $D^{2\text{-cd}}$ |
| $D^{2\text{-ce}}$ | 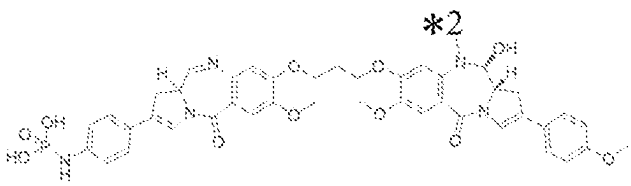 $D^{2\text{-ce}}$ |
| $D^{2\text{-cf}}$ | 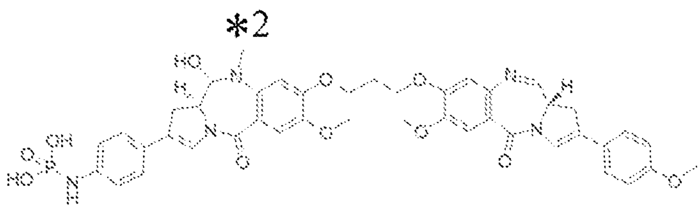 $D^{2\text{-cf}}$ |

(continued)

| Name of group | Structure |
|---|---|
| $D^{2-cg}$ | 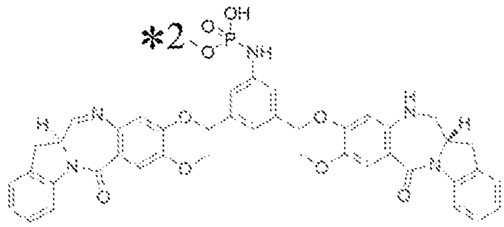 $D^{2-cg}$ |
| $D^{2-ch}$ | 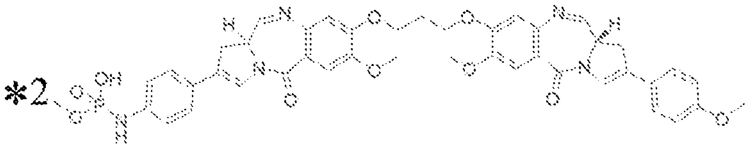 $D^{2-ch}$ |
| $D^{2-da}$ | 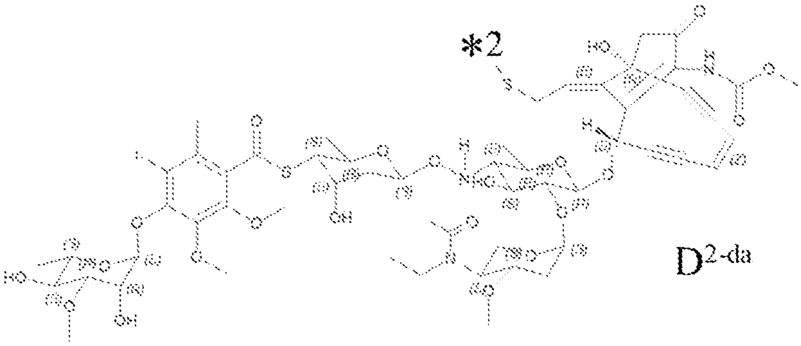 $D^{2-da}$ |

The present invention also provides exemplification precursor compounds (Exemplification 4-1 to Exemplification 4-1440) {in the following formula, Z, $L^{1-a}$ to $L^{1-b}$, $L^{2-a}$ to $L^{2-d}$, $L^{3-a}$ to $L^{3-i}$, $D^{1-a}$ to $D^{1-b}$, and $D^{2-aa}$ to $D^{2-at}$ are as defined above}.

Exemplification 4-1: Z-$L^{3-a}$-N(-$L^{1-a}$-$D^{1-a}$)(-$L^{2-a}$-$D^{2-aa}$),
Exemplification 4-2: Z-$L^{3-a}$-N(-$L^{1-a}$-$D^{1-a}$)(-$L^{2-a}$-$D^{2-ab}$),
Exemplification 4-3: Z-$L^{3-a}$-N(-$L^{1-a}$-$D^{1-a}$)(-$L^{2-a}$-$D^{2-ac}$),
Exemplification 4-4: Z-$L^{3-a}$-N(-$L^{1-a}$-$D^{1-a}$)(-$L^{2-a}$-$D^{2-ad}$),
Exemplification 4-5: Z-$L^{3-a}$-N(-$L^{1-a}$-$D^{1-a}$)(-$L^{2-a}$-$D^{2-ae}$),
Exemplification 4-6: Z-$L^{3-a}$-N(-$L^{1-a}$-$D^{1-a}$)(-$L^{2-a}$-$D^{2-af}$),
Exemplification 4-7: Z-$L^{3-a}$-N(-$L^{1-a}$-$D^{1-a}$)(-$L^{2-a}$-$D^{2-ag}$),
Exemplification 4-8: Z-$L^{3-a}$-N(-$L^{1-a}$-$D^{1-a}$)(-$L^{2-a}$-$D^{2-ah}$),
Exemplification 4-9: Z-$L^{3-a}$-N(-$L^{1-a}$-$D^{1-a}$)(-$L^{2-a}$-$D^{2-ai}$),
Exemplification 4-10: Z-$L^{3-a}$-N(-$L^{1-a}$-$D^{1-a}$)(-$L^{2-a}$-$D^{2-aj}$),
Exemplification 4-11: Z-$L^{3-a}$-N(-$L^{1-a}$-$D^{1-a}$)(-$L^{2-a}$-$D^{2-ak}$),
Exemplification 4-12: Z-$L^{3-a}$-N(-$L^{1-a}$-$D^{1-a}$)(-$L^{2-a}$-$D^{2-al}$),
Exemplification 4-13: Z-$L^{3-a}$-N(-$L^{1-a}$-$D^{1-a}$)(-$L^{2-a}$-$D^{2-am}$),
Exemplification 4-14: Z-$L^{3-a}$-N(-$L^{1-a}$-$D^{1-a}$)(-$L^{2-a}$-$D^{2-an}$),
Exemplification 4-15: Z-$L^{3-a}$-N(-$L^{1-a}$-$D^{1-a}$)(-$L^{2-a}$-$D^{2-ac}$),
Exemplification 4-16: Z-$L^{3-a}$-N(-$L^{1-a}$-$D^{1-a}$)(-$L^{2-a}$-$D^{2-ap}$),
Exemplification 4-17: Z-$L^{3-a}$-N(-$L^{1-a}$-$D^{1-a}$)(-$L^{2-b}$-$D^{2-aq}$),
Exemplification 4-18: Z-$L^{3-a}$-N(-$L^{1-a}$-$D^{1-a}$)(-$L^{2-b}$-$D^{2-ar}$),
Exemplification 4-19: Z-$L^{3-a}$-N(-$L^{1-a}$-$D^{1-a}$)(-$L^{2-b}$-$D^{2-as}$),
Exemplification 4-20: Z-$L^{3-a}$-N(-$L^{1-a}$-$D^{1-a}$)(-$L^{2-b}$-$D^{2-at}$),
Exemplification 4-21: Z-$L^{3-a}$-N(-$L^{1-a}$-$D^{1-a}$)(-$L^{2-c}$-$D^{2-aa}$),
Exemplification 4-22: Z-$L^{3-a}$-N(-$L^{1-a}$-$D^{1-a}$)(-$L^{2-c}$-$D^{2-ab}$),
Exemplification 4-23: Z-$L^{3-a}$-N(-$L^{1-a}$-$D^{1-a}$)(-$L^{2-c}$-$D^{2-ac}$),
Exemplification 4-24: Z-$L^{3-a}$-N(-$L^{1-a}$-$D^{1-a}$)(-$L^{2-c}$-$D^{2-ad}$),
Exemplification 4-25: Z-$L^{3-a}$-N(-$L^{1-a}$-$D^{1-a}$)(-$L^{2-c}$-$D^{2-ae}$),

Exemplification 4-26: Z-$L^{3-a}$-N(-$L^{1-a}$-$D^{1-a}$)(-$L^{2-c}$-$D^{2-af}$),

Exemplification 4-27: Z-$L^{3-a}$-N(-$L^{1-a}$-$D^{1-a}$)(-$L^{2-c}$-$D^{2-ag}$),

Exemplification 4-28: Z-$L^{3-a}$-N(-$L^{1-a}$-$D^{1-a}$)(-$L^{2-c}$-$D^{2-ah}$),

Exemplification 4-29: Z-$L^{3-a}$-N(-$L^{1-a}$-$D^{1-a}$)(-$L^{2-c}$-$D^{2-ai}$),

Exemplification 4-30: Z-$L^{3-a}$-N(-$L^{1-a}$-$D^{1-a}$)(-$L^{2-c}$-$D^{2-aj}$),

Exemplification 4-31: Z-$L^{3-a}$-N(-$L^{1-a}$-$D^{1-a}$)(-$L^{2-c}$-$D^{2-ak}$),

Exemplification 4-32: Z-$L^{3-a}$-N(-$L^{1-a}$-$D^{1-a}$)(-$L^{2-c}$-$D^{2-al}$),

Exemplification 4-33: Z-$L^{3-a}$-N(-$L^{1-a}$-$D^{1-a}$)(-$L^{2-c}$-$D^{2-am}$),

Exemplification 4-34: Z-$L^{3-a}$-N(-$L^{1-a}$-$D^{1-a}$)(-$L^{2-c}$-$D^{2-an}$),

Exemplification 4-35: Z-$L^{3-a}$-N(-$L^{1-a}$-$D^{1-a}$)(-$L^{2-c}$-$D^{2-ao}$),

Exemplification 4-36: Z-$L^{3-a}$-N(-$L^{1-a}$-$D^{1-a}$)(-$L^{2-c}$-$D^{2-ap}$),

Exemplification 4-37: Z-$L^{3-a}$-N(-$L^{1-a}$-$D^{1-a}$)(-$L^{2-d}$-$D^{2-aq}$),

Exemplification 4-38: Z-$L^{3-a}$-N(-$L^{1-a}$-$D^{1-a}$)(-$L^{2-d}$-$D^{2-ar}$),

Exemplification 4-39: Z-$L^{3-a}$-N(-$L^{1-a}$-$D^{1-a}$)(-$L^{2-d}$-$D^{2-as}$),

Exemplification 4-40: Z-$L^{3-a}$-N(-$L^{1-a}$-$D^{1-a}$)(-$L^{2-d}$-$D^{2-at}$),

Exemplification 4-41: Z-$L^{3-a}$-N(-$L^{1-b}$-$D^{1-a}$)(-$L^{2-a}$-$D^{2-aa}$),

Exemplification 4-42: Z-$L^{3-a}$-N(-$L^{1-b}$-$D^{1-a}$)(-$L^{2-a}$-$D^{2-a}$b),

Exemplification 4-43: Z-$L^{3-a}$-N(-$L^{1-b}$-$D^{1-a}$)(-$L^{2-a}$-$D^{2-ac}$),

Exemplification 4-44: Z-$L^{3-a}$-N(-$L^{1-b}$-$D^{1-a}$)(-$L^{2-a}$-$D^{2-ad}$),

Exemplification 4-45: Z-$L^{3-a}$-N(-$L^{1-b}$-$D^{1-a}$)(-$L^{2-a}$-$D^{2-ae}$),

Exemplification 4-46: Z-$L^{3-a}$-N(-$L^{1-b}$-$D^{1-a}$)(-$L^{2-a}$-$D^{2-af}$),

Exemplification 4-47: Z-$L^{3-a}$-N(-$L^{1-b}$-$D^{1-a}$)(-$L^{2-a}$-$D^{2-ag}$),

Exemplification 4-48: Z-$L^{3-a}$-N(-$L^{1-b}$-$D^{1-a}$)(-$L^{2-a}$-$D^{2-ah}$),

Exemplification 4-49: Z-$L^{3-a}$-N(-$L^{1-b}$-$D^{1-a}$)(-$L^{2-a}$-$D^{2-ai}$),

Exemplification 4-50: Z-$L^{3-a}$-N(-$L^{1-b}$-$D^{1-a}$)(-$L^{2-a}$-$D^{2-aj}$),

Exemplification 4-51: Z-$L^{3-a}$-N(-$L^{1-b}$-$D^{1-a}$)(-$L^{2-a}$-$D^{2-ak}$),

Exemplification 4-52: Z-$L^{3-a}$-N(-$L^{1-b}$-$D^{1-a}$)(-$L^{2-a}$-$D^{2-al}$),

Exemplification 4-53: Z-$L^{3-a}$-N(-$L^{1-b}$-$D^{1-a}$)(-$L^{2-a}$-$D^{2-am}$),

Exemplification 4-54: Z-$L^{3-a}$-N(-$L^{1-b}$-$D^{1-a}$)(-$L^{2-a}$-$D^{2-an}$),

Exemplification 4-55: Z-$L^{3-a}$-N(-$L^{1-b}$-$D^{1-a}$)(-$L^{2-a}$-$D^{2-ac}$),

Exemplification 4-56: Z-$L^{3-a}$-N(-$L^{1-b}$-$D^{1-a}$)(-$L^{2-a}$-$D^{2-ap}$),

Exemplification 4-57: Z-$L^{3-a}$-N(-$L^{1-b}$-$D^{1-a}$)(-$L^{2-b}$-$D^{2-aq}$),

Exemplification 4-58: Z-$L^{3-a}$-N(-$L^{1-b}$-$D^{1-a}$)(-$L^{2-b}$-$D^{2-ar}$),

Exemplification 4-59: Z-$L^{3-a}$-N(-$L^{1-b}$-$D^{1-a}$)(-$L^{2-b}$-$D^{2-as}$),

Exemplification 4-60: Z-$L^{3-a}$-N(-$L^{1-b}$-$D^{1-a}$)(-$L^{2-b}$-$D^{2-at}$),

Exemplification 4-61: Z-$L^{3-a}$-N(-$L^{1-b}$-$D^{1-a}$)(-$L^{2-b}$-$D^{2-aa}$),

Exemplification 4-62: Z-$L^{3-a}$-N(-$L^{1-b}$-$D^{1-a}$)(-$L^{2-b}$-$D^{2-ab}$),

Exemplification 4-63: Z-$L^{3-a}$-N(-$L^{1-b}$-$D^{1-a}$)(-$L^{2-c}$-$D^{2-ac}$),

Exemplification 4-64: Z-$L^{3-a}$-N(-$L^{1-b}$-$D^{1-a}$)(-$L^{2-c}$-$D^{2-ad}$),

Exemplification 4-65: Z-$L^{3-a}$-N(-$L^{1-b}$-$D^{1-a}$)(-$L^{2-c}$-$D^{2-ae}$),

Exemplification 4-66: Z-$L^{3-a}$-N(-$L^{1-b}$-$D^{1-a}$)(-$L^{2-c}$-$D^{2-af}$),

Exemplification 4-67 : Z-$L^{3-a}$-N(-$L^{1-b}$-$D^{1-a}$)(-$L^{2-c}$-$D^{2-ag}$),

Exemplification 4-68 : Z-$L^{3-a}$-N(-$L^{1-b}$-$D^{1-a}$)(-$L^{2-c}$-$D^{2-ah}$),

Exemplification 4-69: Z-$L^{3-a}$-N(-$L^{1-b}$-$D^{1-a}$)(-$L^{2-c}$-$D^{2-ai}$),

Exemplification 4-70: Z-$L^{3-a}$-N(-$L^{1-b}$-$D^{1-a}$)(-$L^{2-c}$-$D^{2-aj}$),

Exemplification 4-71: Z-$L^{3-a}$-N(-$L^{1-b}$-$D^{1-a}$)(-$L^{2-c}$-$D^{2-ak}$),

Exemplification 4-72: Z-$L^{3-a}$-N(-$L^{1-b}$-$D^{1-a}$)(-$L^{2-c}$-$D^{2-al}$),

Exemplification 4-73: Z-$L^{3-a}$-N(-$L^{1-b}$-$D^{1-a}$)(-$L^{2-c}$-$D^{2-am}$),

Exemplification 4-74: Z-$L^{3-a}$-N(-$L^{1-b}$-$D^{1-a}$)(-$L^{2-c}$-$D^{2-an}$),

Exemplification 4-75: Z-$L^{3-a}$-N(-$L^{1-b}$-$D^{1-a}$)(-$L^{2-c}$-$D^{2-ao}$),

Exemplification 4-76: Z-$L^{3-a}$-N(-$L^{1-b}$-$D^{1-a}$)(-$L^{2-c}$-$D^{2-ap}$),

Exemplification 4-77: Z-$L^{3-a}$-N(-$L^{1-b}$-$D^{1-a}$)(-$L^{2-d}$-$D^{2-aq}$),

Exemplification 4-78: Z-$L^{3-a}$-N(-$L^{1-b}$-$D^{1-a}$)(-$L^{2-d}$-$D^{2-ar}$),

Exemplification 4-79: Z-$L^{3-a}$-N(-$L^{1-b}$-$D^{1-a}$)(-$L^{2-d}$-$D^{2-as}$),

Exemplification 4-80: Z-$L^{3-a}$-N(-$L^{1-b}$-$D^{1-a}$)(-$L^{2-d}$-$D^{2-at}$),

Exemplification 4-81: Z-$L^{3-a}$-N(-$L^{1-a}$-$D^{1-b}$)(-$L^{2-a}$-$D^{2-aa}$),

Exemplification 4-82: Z-$L^{3-a}$-N(-$L^{1-a}$-$D^{1-b}$)(-$L^{2-a}$-$D^{2-a}$b),

Exemplification 4-83: Z-$L^{3-a}$-N(-$L^{1-a}$-$D^{1-b}$)(-$L^{2-a}$-$D^{2-ac}$),

Exemplification 4-84: Z-$L^{3-a}$-N(-$L^{1-a}$-$D^{1-b}$)(-$L^{2-a}$-$D^{2-ad}$),
Exemplification 4-85: Z-$L^{3-a}$-N(-$L^{1-a}$-$D^{1-b}$)(-$L^{2-a}$-$D^{2-ae}$),
Exemplification 4-86: Z-$L^{3-a}$-N(-$L^{1-a}$-$D^{1-b}$)(-$L^{2-a}$-$D^{2-af}$),
Exemplification 4-87: Z-$L^{3-a}$-N(-$L^{1-a}$-$D^{1-b}$)(-$L^{2-a}$-$D^{2-ag}$),
Exemplification 4-88: Z-$L^{3-a}$-N(-$L^{1-a}$-$D^{1-b}$)(-$L^{2-a}$-$D^{2-ah}$),
Exemplification 4-89: Z-$L^{3-a}$-N(-$L^{1-a}$-$D^{1-b}$)(-$L^{2-a}$-$D^{2-ai}$),
Exemplification 4-90: Z-$L^{3-a}$-N(-$L^{1-a}$-$D^{1-b}$)(-$L^{2-a}$-$D^{2-aj}$),
Exemplification 4-91: Z-$L^{3-a}$-N(-$L^{1-a}$-$D^{1-b}$)(-$L^{2-a}$-$D^{2-ak}$),
Exemplification 4-92: Z-$L^{3-a}$-N(-$L^{1-a}$-$D^{1-b}$)(-$L^{2-a}$-$D^{2-al}$),
Exemplification 4-93: Z-$L^{3-a}$-N(-$L^{1-a}$-$D^{1-b}$)(-$L^{2-a}$-$D^{2-am}$),
Exemplification 4-94 : Z-$L^{3-a}$-N(-$L^{1-a}$-$D^{1-b}$)(-$L^{2-a}$-$D^{2-an}$),
Exemplification 4-95: Z-$L^{3-a}$-N(-$L^{1-a}$-$D^{1-b}$)(-$L^{2-a}$-$D^{2-ac}$),
Exemplification 4-96: Z-$L^{3-a}$-N(-$L^{1-a}$-$D^{1-b}$)(-$L^{2-a}$-$D^{2-ap}$),
Exemplification 4-97: Z-$L^{3-a}$-N(-$L^{1-a}$-$D^{1-b}$)(-$L^{2-b}$-$D^{2-aq}$),
Exemplification 4-98: Z-$L^{3-a}$-N(-$L^{1-a}$-$D^{1-b}$)(-$L^{2-b}$-$D^{2-ar}$),
Exemplification 4-99: Z-$L^{3-a}$-N(-$L^{1-a}$-$D^{1-b}$)(-$L^{2-b}$-$D^{2-as}$),
Exemplification 4-100: Z-$L^{3-a}$-N(-$L^{1-a}$-$D^{1-b}$)(-$L^{2-b}$-$D^{2-at}$),
Exemplification 4-101: Z-$L^{3-a}$-N(-$L^{1-a}$-$D^{1-b}$)(-$L^{2-c}$-$D^{2-an}$),
Exemplification 4-102: Z-$L^{3-a}$-N(-$L^{1-a}$-$D^{1-b}$)(-$L^{2-b}$-$D^{2-ab}$),
Exemplification 4-103: Z-$L^{3-a}$-N(-$L^{1-a}$-$D^{1-b}$)(-$L^{2-c}$-$D^{2-ao}$),
Exemplification 4-104: Z-$L^{3-a}$-N(-$L^{1-a}$-$D^{1-b}$)(-$L^{2-c}$-$D^{2-ad}$),
Exemplification 4-105: Z-$L^{3-a}$-N(-$L^{1-a}$-$D^{1-b}$)(-$L^{2-c}$-$D^{2-ae}$),
Exemplification 4-106: Z-$L^{3-a}$-N(-$L^{1-a}$-$D^{1-b}$)(-$L^{2-c}$-$D^{2-af}$),
Exemplification 4-107: Z-$L^{3-a}$-N(-$L^{1-a}$-$D^{1-b}$)(-$L^{2-c}$-$D^{2-ag}$),
Exemplification 4-108: Z-$L^{3-a}$-N(-$L^{1-a}$-$D^{1-b}$)(-$L^{2-c}$-$D^{2-ah}$),
Exemplification 4-109: Z-$L^{3-a}$-N(-$L^{1-a}$-$D^{1-b}$)(-$L^{2-c}$-$D^{2-ai}$),
Exemplification 4-110: Z-$L^{3-a}$-N(-$L^{1-a}$-$D^{1-b}$)(-$L^{2-c}$-$D^{2-aj}$),
Exemplification 4-111: Z-$L^{3-a}$-N(-$L^{1-a}$-$D^{1-b}$)(-$L^{2-c}$-$D^{2-ak}$),
Exemplification 4-112: Z-$L^{3-a}$-N(-$L^{1-a}$-$D^{1-b}$)(-$L^{2-c}$-$D^{2-al}$),
Exemplification 4-113: Z-$L^{3-a}$-N(-$L^{1-a}$-$D^{1-b}$)(-$L^{2-c}$-$D^{2-am}$),
Exemplification 4-114: Z-$L^{3-a}$-N(-$L^{1-a}$-$D^{1-b}$)(-$L^{2-c}$-$D^{2-an}$),
Exemplification 4-115: Z-$L^{3-a}$-N(-$L^{1-a}$-$D^{1-b}$)(-$L^{2-c}$-$D^{2-ao}$),
Exemplification 4-116: Z-$L^{3-a}$-N(-$L^{1-a}$-$D^{1-b}$)(-$L^{2-c}$-$D^{2-ap}$),
Exemplification 4-117: Z-$L^{3-a}$-N(-$L^{1-a}$-$D^{1-b}$)(-$L^{2-d}$-$D^{2-aq}$),
Exemplification 4-118: Z-$L^{3-a}$-N(-$L^{1-a}$-$D^{1-b}$)(-$L^{2-d}$-$D^{2-ar}$),
Exemplification 4-119: Z-$L^{3-a}$-N(-$L^{1-a}$-$D^{1-b}$)(-$L^{2-d}$-$D^{2-as}$),
Exemplification 4-120: Z-$L^{3-a}$-N(-$L^{1-a}$-$D^{1-b}$)(-$L^{2-d}$-$D^{2-at}$),
Exemplification 4-121: Z-$L^{3-a}$-N(-$L^{1-b}$-$D^{1-b}$)(-$L^{2-a}$-$D^{2-aa}$),
Exemplification 4-122: Z-$L^{3-a}$-N(-$L^{1-b}$-$D^{1-b}$)(-$L^{2-a}$-$D^{2-ab}$),
Exemplification 4-123: Z-$L^{3-a}$-N(-$L^{1-b}$-$D^{1-b}$)(-$L^{2-a}$-$D^{2-ac}$),
Exemplification 4-124: Z-$L^{3-a}$-N(-$L^{1-b}$-$D^{1-b}$)(-$L^{2-a}$-$D^{2-ad}$),
Exemplification 4-125: Z-$L^{3-a}$-N(-$L^{1-b}$-$D^{1-b}$)(-$L^{2-a}$-$D^{2-ae}$),
Exemplification 4-126: Z-$L^{3-a}$-N(-$L^{1-b}$-$D^{1-b}$)(-$L^{2-a}$-$D^{2-af}$),
Exemplification 4-127: Z-$L^{3-a}$-N(-$L^{1-b}$-$D^{1-b}$)(-$L^{2-a}$-$D^{2-ag}$),
Exemplification 4-128: Z-$L^{3-a}$-N(-$L^{1-b}$-$D^{1-b}$)(-$L^{2-a}$-$D^{2-ah}$),
Exemplification 4-129: Z-$L^{3-a}$-N(-$L^{1-b}$-$D^{1-b}$)(-$L^{2-a}$-$D^{2-ai}$),
Exemplification 4-130: Z-$L^{3-a}$-N(-$L^{1-b}$-$D^{1-b}$)(-$L^{2-a}$-$D^{2-aj}$),
Exemplification 4-131: Z-$L^{3-a}$-N(-$L^{1-b}$-$D^{1-b}$)(-$L^{2-a}$-$D^{2-ak}$),
Exemplification 4-132: Z-$L^{3-a}$-N(-$L^{1-b}$-$D^{1-b}$)(-$L^{2-a}$-$D^{2-al}$),
Exemplification 4-133: Z-$L^{3-a}$-N(-$L^{1-b}$-$D^{1-b}$)(-$L^{2-a}$-$D^{2-am}$),
Exemplification 4-134: Z-$L^{3-a}$-N(-$L^{1-b}$-$D^{1-b}$)(-$L^{2-a}$-$D^{2-an}$),
Exemplification 4-135: Z-$L^{3-a}$-N(-$L^{1-b}$-$D^{1-b}$)(-$L^{2-a}$-$D^{2-ao}$),
Exemplification 4-136: Z-$L^{3-a}$-N(-$L^{1-b}$-$D^{1-b}$)(-$L^{2-a}$-$D^{2-ap}$),
Exemplification 4-137: Z-$L^{3-a}$-N(-$L^{1-b}$-$D^{1-b}$)(-$L^{2-b}$-$D^{2-aq}$),
Exemplification 4-138: Z-$L^{3-a}$-N(-$L^{1-b}$-$D^{1-b}$)(-$L^{2-b}$-$D^{2-ar}$),
Exemplification 4-139: Z-$L^{3-a}$-N(-$L^{1-b}$-$D^{1-b}$)(-$L^{2-b}$-$D^{2-as}$),
Exemplification 4-140: Z-$L^{3-a}$-N(-$L^{1-b}$-$D^{1-b}$)(-$L^{2-b}$-$D^{2-at}$),
Exemplification 4-141: Z-$L^{3-a}$-N(-$L^{1-b}$-$D^{1-b}$)(-$L^{2-c}$-$D^{2-an}$),

Exemplification 4-142: Z-$L^{3-a}$-N(-$L^{1-b}$-$D^{1-b}$)(-$L^{2-b}$-$D^{2-ab}$),
Exemplification 4-143: Z-$L^{3-a}$-N(-$L^{1-b}$-$D^{1-b}$)(-$L^{2-c}$-$D^{2-ao}$),
Exemplification 4-144: Z-$L^{3-a}$-N(-$L^{1-b}$-$D^{1-b}$)(-$L^{2-c}$-$D^{2-ad}$),
Exemplification 4-145: Z-$L^{3-a}$-N(-$L^{1-b}$-$D^{1-b}$)(-$L^{2-c}$-$D^{2-ae}$),
Exemplification 4-146: Z-$L^{3-a}$-N(-$L^{1-b}$-$D^{1-b}$)(-$L^{2-c}$-$D^{2-af}$),
Exemplification 4-147: Z-$L^{3-a}$-N(-$L^{1-b}$-$D^{1-b}$)(-$L^{2-c}$-$D^{2-ag}$),
Exemplification 4-148: Z-$L^{3-a}$-N(-$L^{1-b}$-$D^{1-b}$)(-$L^{2-c}$-$D^{2-ah}$),
Exemplification 4-149: Z-$L^{3-a}$-N(-$L^{1-b}$-$D^{1-b}$)(-$L^{2-c}$-$D^{2-ai}$),
Exemplification 4-150: Z-$L^{3-a}$-N(-$L^{1-b}$-$D^{1-b}$)(-$L^{2-c}$-$D^{2-aj}$),
Exemplification 4-151: Z-$L^{3-a}$-N(-$L^{1-b}$-$D^{1-b}$)(-$L^{2-c}$-$D^{2-ak}$),
Exemplification 4-152: Z-$L^{3-a}$-N(-$L^{1-b}$-$D^{1-b}$)(-$L^{2-c}$-$D^{2-al}$),
Exemplification 4-153: Z-$L^{3-a}$-N(-$L^{1-b}$-$D^{1-b}$)(-$L^{2-c}$-$D^{2-am}$),
Exemplification 4-154: Z-$L^{3-a}$-N(-$L^{1-b}$-$D^{1-b}$)(-$L^{2-c}$-$D^{2-an}$),
Exemplification 4-155: Z-$L^{3-a}$-N(-$L^{1-b}$-$D^{1-b}$)(-$L^{2-c}$-$D^{2-ao}$),
Exemplification 4-156: Z-$L^{3-a}$-N(-$L^{1-b}$-$D^{1-b}$)(-$L^{2-c}$-$D^{2-ap}$),
Exemplification 4-157: Z-$L^{3-a}$-N(-$L^{1-b}$-$D^{1-b}$)(-$L^{2-d}$-$D^{2-aq}$),
Exemplification 4-158: Z-$L^{3-a}$-N(-$L^{1-b}$-$D^{1-b}$)(-$L^{2-d}$-$D^{2-ar}$),
Exemplification 4-159: Z-$L^{3-a}$-N(-$L^{1-b}$-$D^{1-b}$)(-$L^{2-d}$-$D^{2-as}$),
Exemplification 4-160: Z-$L^{3-a}$-N(-$L^{1-b}$-$D^{1-b}$)(-$L^{2-d}$-$D^{2-at}$),
Exemplification 4-161: Z-$L^{3-b}$-N(-$L^{1-a}$-$D^{1-a}$)(-$L^{2-a}$-$D^{2-aa}$),
Exemplification 4-162: Z-$L^{3-b}$-N(-$L^{1-a}$-$D^{1-a}$)(-$L^{2-a}$-$D^{2-ab}$),
Exemplification 4-163: Z-$L^{3-b}$-N(-$L^{1-a}$-$D^{1-a}$)(-$L^{2-a}$-$D^{2-ac}$),
Exemplification 4-164: Z-$L^{3-b}$-N(-$L^{1-a}$-$D^{1-a}$)(-$L^{2-a}$-$D^{2-ad}$),
Exemplification 4-165: Z-$L^{3-b}$-N(-$L^{1-a}$-$D^{1-a}$)(-$L^{2-a}$-$D^{2-ae}$),
Exemplification 4-166: Z-$L^{3-b}$-N(-$L^{1-a}$-$D^{1-a}$)(-$L^{2-a}$-$D^{2-af}$),
Exemplification 4-167: Z-$L^{3-b}$-N(-$L^{1-a}$-$D^{1-a}$)(-$L^{2-a}$-$D^{2-ag}$),
Exemplification 4-168: Z-$L^{3-b}$-N(-$L^{1-a}$-$D^{1-a}$)(-$L^{2-a}$-$D^{2-ah}$),
Exemplification 4-169: Z-$L^{3-b}$-N(-$L^{1-a}$-$D^{1-a}$)(-$L^{2-a}$-$D^{2-ai}$),
Exemplification 4-170: Z-$L^{3-b}$-N(-$L^{1-a}$-$D^{1-a}$)(-$L^{2-a}$-$D^{2-aj}$),
Exemplification 4-171: Z-$L^{3-b}$-N(-$L^{1-a}$-$D^{1-a}$)(-$L^{2-a}$-$D^{2-ak}$),
Exemplification 4-172: Z-$L^{3-b}$-N(-$L^{1-a}$-$D^{1-a}$)(-$L^{2-a}$-$D^{2-al}$),
Exemplification 4-173: Z-$L^{3-b}$-N(-$L^{1-a}$-$D^{1-a}$)(-$L^{2-a}$-$D^{2-am}$),
Exemplification 4-174: Z-$L^{3-b}$-N(-$L^{1-a}$-$D^{1-a}$)(-$L^{2-a}$-$D^{2-an}$),
Exemplification 4-175: Z-$L^{3-b}$-N(-$L^{1-a}$-$D^{1-a}$)(-$L^{2-a}$-$D^{2-ac}$),
Exemplification 4-176: Z-$L^{3-b}$-N(-$L^{1-a}$-$D^{1-a}$)(-$L^{2-a}$-$D^{2-ap}$),
Exemplification 4-177: Z-$L^{3-b}$-N(-$L^{1-a}$-$D^{1-a}$)(-$L^{2-b}$-$D^{2-aq}$),
Exemplification 4-178: Z-$L^{3-b}$-N(-$L^{1-a}$-$D^{1-a}$)(-$L^{2-b}$-$D^{2-ar}$),
Exemplification 4-179: Z-$L^{3-b}$-N(-$L^{1-a}$-$D^{1-a}$)(-$L^{2-b}$-$D^{2-as}$),
Exemplification 4-180: Z-$L^{3-b}$-N(-$L^{1-a}$-$D^{1-a}$)(-$L^{2-b}$-$D^{2-at}$),
Exemplification 4-181: Z-$L^{3-b}$-N(-$L^{1-a}$-$D^{1-a}$)(-$L^{2-c}$-$D^{2-an}$),
Exemplification 4-182: Z-$L^{3-b}$-N(-$L^{1-a}$-$D^{1-a}$)(-$L^{2-b}$-$D^{2-ab}$),
Exemplification 4-183: Z-$L^{3-b}$-N(-$L^{1-a}$-$D^{1-a}$)(-$L^{2-c}$-$D^{2-ao}$),
Exemplification 4-184: Z-$L^{3-b}$-N(-$L^{1-a}$-$D^{1-a}$)(-$L^{2-c}$-$D^{2-ad}$),
Exemplification 4-185: Z-$L^{3-b}$-N(-$L^{1-a}$-$D^{1-a}$)(-$L^{2-c}$-$D^{2-ae}$),
Exemplification 4-186: Z-$L^{3-b}$-N(-$L^{1-a}$-$D^{1-a}$)(-$L^{2-c}$-$D^{2-af}$),
Exemplification 4-187: Z-$L^{3-b}$-N(-$L^{1-a}$-$D^{1-a}$)(-$L^{2-c}$-$D^{2-ag}$),
Exemplification 4-188: Z-$L^{3-b}$-N(-$L^{1-a}$-$D^{1-a}$)(-$L^{2-c}$-$D^{2-ah}$),
Exemplification 4-189: Z-$L^{3-b}$-N(-$L^{1-a}$-$D^{1-a}$)(-$L^{2-c}$-$D^{2-ai}$),
Exemplification 4-190: Z-$L^{3-b}$-N(-$L^{1-a}$-$D^{1-a}$)(-$L^{2-c}$-$D^{2-aj}$),
Exemplification 4-191: Z-$L^{3-b}$-N(-$L^{1-a}$-$D^{1-a}$)(-$L^{2-c}$-$D^{2-ak}$),
Exemplification 4-192: Z-$L^{3-b}$-N(-$L^{1-a}$-$D^{1-a}$)(-$L^{2-c}$-$D^{2-al}$),
Exemplification 4-193: Z-$L^{3-b}$-N(-$L^{1-a}$-$D^{1-a}$)(-$L^{2-c}$-$D^{2-am}$),
Exemplification 4-194: Z-$L^{3-b}$-N(-$L^{1-a}$-$D^{1-a}$)(-$L^{2-c}$-$D^{2-an}$),
Exemplification 4-195: Z-$L^{3-b}$-N(-$L^{1-a}$-$D^{1-a}$)(-$L^{2-c}$-$D^{2-ao}$),
Exemplification 4-196: Z-$L^{3-b}$-N(-$L^{1-a}$-$D^{1-a}$)(-$L^{2-c}$-$D^{2-ap}$),
Exemplification 4-197: Z-$L^{3-b}$-N(-$L^{1-a}$-$D^{1-a}$)(-$L^{2-d}$-$D^{2-aq}$),
Exemplification 4-198: Z-$L^{3-b}$-N(-$L^{1-a}$-$D^{1-a}$)(-$L^{2-d}$-$D^{2-ar}$),
Exemplification 4-199: Z-$L^{3-b}$-N(-$L^{1-a}$-$D^{1-a}$)(-$L^{2-d}$-$D^{2-as}$),

Exemplification 4-200: $Z-L^{3-b}-N(-L^{1-a}-D^{1-a})(-L^{2-d}-D^{2-at})$,
Exemplification 4-201: $Z-L^{3-b}-N(-L^{1-b}-D^{1-a})(-L^{2-a}-D^{2-aa})$,
Exemplification 4-202: $Z-L^{3-b}-N(-L^{1-b}-D^{1-a})(-L^{2-a}-D^{2-a}b)$,
Exemplification 4-203: $Z-L^{3-b}-N(-L^{1-b}-D^{1-a})(-L^{2-a}-D^{2-ac})$,
Exemplification 4-204: $Z-L^{3-b}-N(-L^{1-b}-D^{1-a})(-L^{2-a}-D^{2-ad})$,
Exemplification 4-205: $Z-L^{3-b}-N(-L^{1-b}-D^{1-a})(-L^{2-a}-D^{2-ae})$,
Exemplification 4-206: $Z-L^{3-b}-N(-L^{1-b}-D^{1-a})(-L^{2-a}-D^{2-af})$,
Exemplification 4-207: $Z-L^{3-b}-N(-L^{1-b}-D^{1-a})(-L^{2-a}-D^{2-ag})$,
Exemplification 4-208: $Z-L^{3-b}-N(-L^{1-b}-D^{1-a})(-L^{2-a}-D^{2-ah})$,
Exemplification 4-209: $Z-L^{3-b}-N(-L^{1-b}-D^{1-a})(-L^{2-a}-D^{2-ai})$,
Exemplification 4-210: $Z-L^{3-b}-N(-L^{1-b}-D^{1-a})(-L^{2-a}-D^{2-aj})$,
Exemplification 4-211: $Z-L^{3-b}-N(-L^{1-b}-D^{1-a})(-L^{2-a}-D^{2-ak})$,
Exemplification 4-212: $Z-L^{3-b}-N(-L^{1-b}-D^{1-a})(-L^{2-a}-D^{2-al})$,
Exemplification 4-213: $Z-L^{3-b}-N(-L^{1-b}-D^{1-a})(-L^{2-a}-D^{2-am})$,
Exemplification 4-214: $Z-L^{3-b}-N(-L^{1-b}-D^{1-a})(-L^{2-a}-D^{2-an})$,
Exemplification 4-215: $Z-L^{3-b}-N(-L^{1-b}-D^{1-a})(-L^{2-a}-D^{2-ac})$,
Exemplification 4-216: $Z-L^{3-b}-N(-L^{1-b}-D^{1-a})(-L^{2-a}-D^{2-ap})$,
Exemplification 4-217: $Z-L^{3-b}-N(-L^{1-b}-D^{1-a})(-L^{2-b}-D^{2-aq})$,
Exemplification 4-218: $Z-L^{3-b}-N(-L^{1-b}-D^{1-a})(-L^{2-b}-D^{2-ar})$,
Exemplification 4-219: $Z-L^{3-b}-N(-L^{1-b}-D^{1-a})(-L^{2-b}-D^{2-as})$,
Exemplification 4-220: $Z-L^{3-b}-N(-L^{1-b}-D^{1-a})(-L^{2-b}-D^{2-at})$,
Exemplification 4-221: $Z-L^{3-b}-N(-L^{1-b}-D^{1-a})(-L^{2-b}-D^{2-aa})$,
Exemplification 4-222: $Z-L^{3-b}-N(-L^{1-b}-D^{1-a})(-L^{2-b}-D^{2-ab})$,
Exemplification 4-223: $Z-L^{3-b}-N(-L^{1-b}-D^{1-a})(-L^{2-c}-D^{2-ao})$,
Exemplification 4-224: $Z-L^{3-b}-N(-L^{1-b}-D^{1-a})(-L^{2-c}-D^{2-ad})$,
Exemplification 4-225: $Z-L^{3-b}-N(-L^{1-b}-D^{1-a})(-L^{2-c}-D^{2-ae})$,
Exemplification 4-226: $Z-L^{3-b}-N(-L^{1-b}-D^{1-a})(-L^{2-c}-D^{2-af})$,
Exemplification 4-227: $Z-L^{3-b}-N(-L^{1-b}-D^{1-a})(-L^{2-c}-D^{2-ag})$,
Exemplification 4-228: $Z-L^{3-b}-N(-L^{1-b}-D^{1-a})(-L^{2-c}-D^{2-ah})$,
Exemplification 4-229: $Z-L^{3-b}-N(-L^{1-b}-D^{1-a})(-L^{2-c}-D^{2-ai})$,
Exemplification 4-230: $Z-L^{3-b}-N(-L^{1-b}-D^{1-a})(-L^{2-c}-D^{2-aj})$,
Exemplification 4-231: $Z-L^{3-b}-N(-L^{1-b}-D^{1-a})(-L^{2-c}-D^{2-ak})$,
Exemplification 4-232: $Z-L^{3-b}-N(-L^{1-b}-D^{1-a})(-L^{2-c}-D^{2-al})$,
Exemplification 4-233: $Z-L^{3-b}-N(-L^{1-b}-D^{1-a})(-L^{2-c}-D^{2-am})$,
Exemplification 4-234: $Z-L^{3-b}-N(-L^{1-b}-D^{1-a})(-L^{2-c}-D^{2-an})$,
Exemplification 4-235: $Z-L^{3-b}-N(-L^{1-b}-D^{1-a})(-L^{2-c}-D^{2-ao})$,
Exemplification 4-236: $Z-L^{3-b}-N(-L^{1-b}-D^{1-a})(-L^{2-c}-D^{2-ap})$,
Exemplification 4-237: $Z-L^{3-b}-N(-L^{1-b}-D^{1-a})(-L^{2-d}-D^{2-aq})$,
Exemplification 4-238: $Z-L^{3-b}-N(-L^{1-b}-D^{1-a})(-L^{2-d}-D^{2-ar})$,
Exemplification 4-239: $Z-L^{3-b}-N(-L^{1-b}-D^{1-a})(-L^{2-d}-D^{2-as})$,
Exemplification 4-240: $Z-L^{3-b}-N(-L^{1-b}-D^{1-a})(-L^{2-d}-D^{2-at})$,
Exemplification 4-241: $Z-L^{3-b}-N(-L^{1-a}-D^{1-b})(-L^{2-a}-D^{2-aa})$,
Exemplification 4-242: $Z-L^{3-b}-N(-L^{1-a}-D^{1-b})(-L^{2-a}-D^{2-ab})$,
Exemplification 4-243: $Z-L^{3-b}-N(-L^{1-a}-D^{1-b})(-L^{2-a}-D^{2-ac})$,
Exemplification 4-244: $Z-L^{3-b}-N(-L^{1-a}-D^{1-b})(-L^{2-a}-D^{2-ad})$,
Exemplification 4-245: $Z-L^{3-b}-N(-L^{1-a}-D^{1-b})(-L^{2-a}-D^{2-ae})$,
Exemplification 4-246: $Z-L^{3-b}-N(-L^{1-a}-D^{1-b})(-L^{2-a}-D^{2-af})$,
Exemplification 4-247: $Z-L^{3-b}-N(-L^{1-a}-D^{1-b})(-L^{2-a}-D^{2-ag})$,
Exemplification 4-248: $Z-L^{3-b}-N(-L^{1-a}-D^{1-b})(-L^{2-a}-D^{2-ah})$,
Exemplification 4-249: $Z-L^{3-b}-N(-L^{1-a}-D^{1-b})(-L^{2-a}-D^{2-ai})$,
Exemplification 4-250: $Z-L^{3-b}-N(-L^{1-a}-D^{1-b})(-L^{2-a}-D^{2-aj})$,
Exemplification 4-251: $Z-L^{3-b}-N(-L^{1-a}-D^{1-b})(-L^{2-a}-D^{2-ak})$,
Exemplification 4-252: $Z-L^{3-b}-N(-L^{1-a}-D^{1-b})(-L^{2-a}-D^{2-al})$,
Exemplification 4-253: $Z-L^{3-b}-N(-L^{1-a}-D^{1-b})(-L^{2-a}-D^{2-am})$,
Exemplification 4-254: $Z-L^{3-b}-N(-L^{1-a}-D^{1-b})(-L^{2-a}-D^{2-an})$,
Exemplification 4-255: $Z-L^{3-b}-N(-L^{1-a}-D^{1-b})(-L^{2-a}-D^{2-ao})$,
Exemplification 4-256: $Z-L^{3-b}-N(-L^{1-a}-D^{1-b})(-L^{2-a}-D^{2-ap})$,
Exemplification 4-257: $Z-L^{3-b}-N(-L^{1-a}-D^{1-b})(-L^{2-b}-D^{2-aq})$,

Exemplification 4-258: $Z-L^{3-b}-N(-L^{1-a}-D^{1-b})(-L^{2-b}-D^{2-ar})$,
Exemplification 4-259: $Z-L^{3-b}-N(-L^{1-a}-D^{1-b})(-L^{2-b}-D^{2-as})$,
Exemplification 4-260: $Z-L^{3-b}-N(-L^{1-a}-D^{1-b})(-L^{2-b}-D^{2-at})$,
Exemplification 4-261: $Z-L^{3-b}-N(-L^{1-a}-D^{1-b})(-L^{2-c}-D^{2-aa})$,
Exemplification 4-262: $Z-L^{3-b}-N(-L^{1-a}-D^{1-b})(-L^{2-c}-D^{2-ab})$,
Exemplification 4-263: $Z-L^{3-b}-N(-L^{1-a}-D^{1-b})(-L^{2-c}-D^{2-c})$,
Exemplification 4-264: $Z-L^{3-b}-N(-L^{1-a}-D^{1-b})(-L^{2-c}-D^{2-ad})$,
Exemplification 4-265: $Z-L^{3-b}-N(-L^{1-a}-D^{1-b})(-L^{2-c}-D^{2-ae})$,
Exemplification 4-266: $Z-L^{3-b}-N(-L^{1-a}-D^{1-b})(-L^{2-c}-D^{2-af})$,
Exemplification 4-267: $Z-L^{3-b}-N(-L^{1-a}-D^{1-b})(-L^{2-c}-D^{2-ag})$,
Exemplification 4-268: $Z-L^{3-b}-N(-L^{1-a}-D^{1-b})(-L^{2-c}-D^{2-ah})$,
Exemplification 4-269: $Z-L^{3-b}-N(-L^{1-a}-D^{1-b})(-L^{2-c}-D^{2-ai})$,
Exemplification 4-270: $Z-L^{3-b}-N(-L^{1-a}-D^{1-b})(-L^{2-c}-D^{2-aj})$,
Exemplification 4-271: $Z-L^{3-b}-N(-L^{1-a}-D^{1-b})(-L^{2-c}-D^{2-ak})$,
Exemplification 4-272: $Z-L^{3-b}-N(-L^{1-a}-D^{1-b})(-L^{2-c}-D^{2-al})$,
Exemplification 4-273: $Z-L^{3-b}-N(-L^{1-a}-D^{1-b})(-L^{2-c}-D^{2-am})$,
Exemplification 4-274: $Z-L^{3-b}-N(-L^{1-a}-D^{1-b})(-L^{2-c}-D^{2-an})$,
Exemplification 4-275: $Z-L^{3-b}-N(-L^{1-a}-D^{1-b})(-L^{2-c}-D^{2-ao})$,
Exemplification 4-276: $Z-L^{3-b}-N(-L^{1-a}-D^{1-b})(-L^{2-c}-D^{2-ap})$,
Exemplification 4-277: $Z-L^{3-b}-N(-L^{1-a}-D^{1-b})(-L^{2-d}-D^{2-aq})$,
Exemplification 4-278: $Z-L^{3-b}-N(-L^{1-a}-D^{1-b})(-L^{2-d}-D^{2-ar})$,
Exemplification 4-279: $Z-L^{3-b}-N(-L^{1-a}-D^{1-b})(-L^{2-d}-D^{2-as})$,
Exemplification 4-280: $Z-L^{3-b}-N(-L^{1-a}-D^{1-b})(-L^{2-d}-D^{2-at})$,
Exemplification 4-281: $Z-L^{3-b}-N(-L^{1-b}-D^{1-b})(-L^{2-a}-D^{2-aa})$,
Exemplification 4-282: $Z-L^{3-b}-N(-L^{1-b}-D^{1-b})(-L^{2-a}-D^{2-ab})$,
Exemplification 4-283: $Z-L^{3-b}-N(-L^{1-b}-D^{1-b})(-L^{2-a}-D^{2-ac})$,
Exemplification 4-284: $Z-L^{3-b}-N(-L^{1-b}-D^{1-b})(-L^{2-a}-D^{2-ad})$,
Exemplification 4-285: $Z-L^{3-b}-N(-L^{1-b}-D^{1-b})(-L^{2-a}-D^{2-ae})$,
Exemplification 4-286: $Z-L^{3-b}-N(-L^{1-b}-D^{1-b})(-L^{2-a}-D^{2-af})$,
Exemplification 4-287: $Z-L^{3-b}-N(-L^{1-b}-D^{1-b})(-L^{2-a}-D^{2-ag})$,
Exemplification 4-288: $Z-L^{3-b}-N(-L^{1-b}-D^{1-b})(-L^{2-a}-D^{2-ah})$,
Exemplification 4-289: $Z-L^{3-b}-N(-L^{1-b}-D^{1-b})(-L^{2-a}-D^{2-ai})$,
Exemplification 4-290: $Z-L^{3-b}-N(-L^{1-b}-D^{1-b})(-L^{2-a}-D^{2-aj})$,
Exemplification 4-291: $Z-L^{3-b}-N(-L^{1-b}-D^{1-b})(-L^{2-a}-D^{2-ak})$,
Exemplification 4-292: $Z-L^{3-b}-N(-L^{1-b}-D^{1-b})(-L^{2-a}-D^{2-al})$,
Exemplification 4-293: $Z-L^{3-b}-N(-L^{1-b}-D^{1-b})(-L^{2-a}-D^{2-am})$,
Exemplification 4-294: $Z-L^{3-b}-N(-L^{1-b}-D^{1-b})(-L^{2-a}-D^{2-an})$,
Exemplification 4-295: $Z-L^{3-b}-N(-L^{1-b}-D^{1-b})(-L^{2-a}-D^{2-ao})$,
Exemplification 4-296: $Z-L^{3-b}-N(-L^{1-b}-D^{1-b})(-L^{2-a}-D^{2-ap})$,
Exemplification 4-297: $Z-L^{3-b}-N(-L^{1-b}-D^{1-b})(-L^{2-b}-D^{2-aq})$,
Exemplification 4-298: $Z-L^{3-b}-N(-L^{1-b}-D^{1-b})(-L^{2-b}-D^{2-ar})$,
Exemplification 4-299: $Z-L^{3-b}-N(-L^{1-b}-D^{1-b})(-L^{2-b}-D^{2-as})$,
Exemplification 4-300: $Z-L^{3-b}-N(-L^{1-b}-D^{1-b})(-L^{2-b}-D^{2-at})$,
Exemplification 4-301: $Z-L^{3-b}-N(-L^{1-b}-D^{1-b})(-L^{2-c}-D^{2-aa})$,
Exemplification 4-302: $Z-L^{3-b}-N(-L^{1-b}-D^{1-b})(-L^{2-c}-D^{2-ab})$,
Exemplification 4-303: $Z-L^{3-b}-N(-L^{1-b}-D^{1-b})(-L^{2-c}-D^{2-ac})$,
Exemplification 4-304: $Z-L^{3-b}-N(-L^{1-b}-D^{1-b})(-L^{2-c}-D^{2-ad})$,
Exemplification 4-305: $Z-L^{3-b}-N(-L^{1-b}-D^{1-b})(-L^{2-c}-D^{2-ae})$,
Exemplification 4-306: $Z-L^{3-b}-N(-L^{1-b}-D^{1-b})(-L^{2-c}-D^{2-af})$,
Exemplification 4-307: $Z-L^{3-b}-N(-L^{1-b}-D^{1-b})(-L^{2-c}-D^{2-ag})$,
Exemplification 4-308: $Z-L^{3-b}-N(-L^{1-b}-D^{1-b})(-L^{2-c}-D^{2-ah})$,
Exemplification 4-309: $Z-L^{3-b}-N(-L^{1-b}-D^{1-b})(-L^{2-c}-D^{2-ai})$,
Exemplification 4-310: $Z-L^{3-b}-N(-L^{1-b}-D^{1-b})(-L^{2-c}-D^{2-aj})$,
Exemplification 4-311: $Z-L^{3-b}-N(-L^{1-b}-D^{1-b})(-L^{2-c}-D^{2-ak})$,
Exemplification 4-312: $Z-L^{3-b}-N(-L^{1-b}-D^{1-b})(-L^{2-c}-D^{2-al})$,
Exemplification 4-313: $Z-L^{3-b}-N(-L^{1-b}-D^{1-b})(-L^{2-c}-D^{2-am})$,
Exemplification 4-314: $Z-L^{3-b}-N(-L^{1-b}-D^{1-b})(-L^{2-c}-D^{2-an})$,
Exemplification 4-315: $Z-L^{3-b}-N(-L^{1-b}-D^{1-b})(-L^{2-c}-D^{2-ao})$,

Exemplification 4-316: Z-$L^{3-b}$-N(-$L^{1-b}$-$D^{1-b}$)(-$L^{2-c}$-$D^{2-ap}$),
Exemplification 4-317: Z-$L^{3-b}$-N(-$L^{1-b}$-$D^{1-b}$)(-$L^{2-d}$-$D^{2-aq}$),
Exemplification 4-318: Z-$L^{3-b}$-N(-$L^{1-b}$-$D^{1-b}$)(-$L^{2-d}$-$D^{2-ar}$),
Exemplification 4-319: Z-$L^{3-b}$-N(-$L^{1-b}$-$D^{1-b}$)(-$L^{2-d}$-$D^{2-as}$),
Exemplification 4-320: Z-$L^{3-b}$-N(-$L^{1-b}$-$D^{1-b}$)(-$L^{2-d}$-$D^{2-at}$),
Exemplification 4-321: Z-$L^{3-c}$-N(-$L^{1-a}$-$D^{1-a}$)(-$L^{2-a}$-$D^{2-aa}$),
Exemplification 4-322: Z-$L^{3-c}$-N(-$L^{1-a}$-$D^{1-a}$)(-$L^{2-a}$-$D^{2-ab}$),
Exemplification 4-323: Z-$L^{3-c}$-N(-$L^{1-a}$-$D^{1-a}$)(-$L^{2-a}$-$D^{2-ac}$),
Exemplification 4-324: Z-$L^{3-c}$-N(-$L^{1-a}$-$D^{1-a}$)(-$L^{2-a}$-$D^{2-ad}$),
Exemplification 4-325: Z-$L^{3-c}$-N(-$L^{1-a}$-$D^{1-a}$)(-$L^{2-a}$-$D^{2-ae}$),
Exemplification 4-326: Z-$L^{3-c}$-N(-$L^{1-a}$-$D^{1-a}$)(-$L^{2-a}$-$D^{2-af}$),
Exemplification 4-327: Z-$L^{3-c}$-N(-$L^{1-a}$-$D^{1-a}$)(-$L^{2-a}$-$D^{2-ag}$),
Exemplification 4-328: Z-$L^{3-c}$-N(-$L^{1-a}$-$D^{1-a}$)(-$L^{2-a}$-$D^{2-ah}$),
Exemplification 4-329: Z-$L^{3-c}$-N(-$L^{1-a}$-$D^{1-a}$)(-$L^{2-a}$-$D^{2-ai}$),
Exemplification 4-330: Z-$L^{3-c}$-N(-$L^{1-a}$-$D^{1-a}$)(-$L^{2-a}$-$D^{2-aj}$),
Exemplification 4-331: Z-$L^{3-c}$-N(-$L^{1-a}$-$D^{1-a}$)(-$L^{2-a}$-$D^{2-ak}$),
Exemplification 4-332: Z-$L^{3-c}$-N(-$L^{1-a}$-$D^{1-a}$)(-$L^{2-a}$-$D^{2-al}$),
Exemplification 4-333: Z-$L^{3-c}$-N(-$L^{1-a}$-$D^{1-a}$)(-$L^{2-a}$-$D^{2-am}$),
Exemplification 4-334: Z-$L^{3-c}$-N(-$L^{1-a}$-$D^{1-a}$)(-$L^{2-a}$-$D^{2-an}$),
Exemplification 4-335: Z-$L^{3-c}$-N(-$L^{1-a}$-$D^{1-a}$)(-$L^{2-a}$-$D^{2-ac}$),
Exemplification 4-336: Z-$L^{3-c}$-N(-$L^{1-a}$-$D^{1-a}$)(-$L^{2-a}$-$D^{2-ap}$),
Exemplification 4-337: Z-$L^{3-c}$-N(-$L^{1-a}$-$D^{1-a}$)(-$L^{2-b}$-$D^{2-aq}$),
Exemplification 4-338: Z-$L^{3-c}$-N(-$L^{1-a}$-$D^{1-a}$)(-$L^{2-b}$-$D^{2-ar}$),
Exemplification 4-339: Z-$L^{3-c}$-N(-$L^{1-a}$-$D^{1-a}$)(-$L^{2-b}$-$D^{2-as}$),
Exemplification 4-340: Z-$L^{3-c}$-N(-$L^{1-a}$-$D^{1-a}$)(-$L^{2-b}$-$D^{2-at}$),
Exemplification 4-341: Z-$L^{3-c}$-N(-$L^{1-a}$-$D^{1-a}$)(-$L^{2-c}$-$D^{2-an}$),
Exemplification 4-342: Z-$L^{3-c}$-N(-$L^{1-a}$-$D^{1-a}$)(-$L^{2-c}$-$D^{2-ab}$),
Exemplification 4-343: Z-$L^{3-c}$-N(-$L^{1-a}$-$D^{1-a}$)(-$L^{2-c}$-$D^{2-ac}$),
Exemplification 4-344: Z-$L^{3-c}$-N(-$L^{1-a}$-$D^{1-a}$)(-$L^{2-c}$-$D^{2-ad}$),
Exemplification 4-345: Z-$L^{3-c}$-N(-$L^{1-a}$-$D^{1-a}$)(-$L^{2-c}$-$D^{2-ae}$),
Exemplification 4-346: Z-$L^{3-c}$-N(-$L^{1-a}$-$D^{1-a}$)(-$L^{2-c}$-$D^{2-af}$),
Exemplification 4-347: Z-$L^{3-c}$-N(-$L^{1-a}$-$D^{1-a}$)(-$L^{2-c}$-$D^{2-ag}$),
Exemplification 4-348: Z-$L^{3-c}$-N(-$L^{1-a}$-$D^{1-a}$)(-$L^{2-c}$-$D^{2-ah}$),
Exemplification 4-349: Z-$L^{3-c}$-N(-$L^{1-a}$-$D^{1-a}$)(-$L^{2-c}$-$D^{2-ai}$),
Exemplification 4-350: Z-$L^{3-c}$-N(-$L^{1-a}$-$D^{1-a}$)(-$L^{2-c}$-$D^{2-aj}$),
Exemplification 4-351: Z-$L^{3-c}$-N(-$L^{1-a}$-$D^{1-a}$)(-$L^{2-c}$-$D^{2-ak}$),
Exemplification 4-352: Z-$L^{3-c}$-N(-$L^{1-a}$-$D^{1-a}$)(-$L^{2-c}$-$D^{2-al}$),
Exemplification 4-353: Z-$L^{3-c}$-N(-$L^{1-a}$-$D^{1-a}$)(-$L^{2-c}$-$D^{2-am}$),
Exemplification 4-354: Z-$L^{3-c}$-N(-$L^{1-a}$-$D^{1-a}$)(-$L^{2-c}$-$D^{2-an}$),
Exemplification 4-355: Z-$L^{3-c}$-N(-$L^{1-a}$-$D^{1-a}$)(-$L^{2-c}$-$D^{2-ao}$),
Exemplification 4-356: Z-$L^{3-c}$-N(-$L^{1-a}$-$D^{1-a}$)(-$L^{2-c}$-$D^{2-ap}$),
Exemplification 4-357: Z-$L^{3-c}$-N(-$L^{1-a}$-$D^{1-a}$)(-$L^{2-d}$-$D^{2-aq}$),
Exemplification 4-358: Z-$L^{3-c}$-N(-$L^{1-a}$-$D^{1-a}$)(-$L^{2-d}$-$D^{2-ar}$),
Exemplification 4-359: Z-$L^{3-c}$-N(-$L^{1-a}$-$D^{1-a}$)(-$L^{2-d}$-$D^{2-as}$),
Exemplification 4-360: Z-$L^{3-c}$-N(-$L^{1-a}$-$D^{1-a}$)(-$L^{2-d}$-$D^{2-at}$),
Exemplification 4-361: Z-$L^{3-c}$-N(-$L^{1-b}$-$D^{1-a}$)(-$L^{2-a}$-$D^{2-aa}$),
Exemplification 4-362: Z-$L^{3-c}$-N(-$L^{1-b}$-$D^{1-a}$)(-$L^{2-a}$-$D^{2-ab}$),
Exemplification 4-363: Z-$L^{3-c}$-N(-$L^{1-b}$-$D^{1-a}$)(-$L^{2-a}$-$D^{2-ac}$),
Exemplification 4-364: Z-$L^{3-c}$-N(-$L^{1-b}$-$D^{1-a}$)(-$L^{2-a}$-$D^{2-ad}$),
Exemplification 4-365: Z-$L^{3-c}$-N(-$L^{1-b}$-$D^{1-a}$)(-$L^{2-a}$-$D^{2-ae}$),
Exemplification 4-366: Z-$L^{3-c}$-N(-$L^{1-b}$-$D^{1-a}$)(-$L^{2-a}$-$D^{2-af}$),
Exemplification 4-367: Z-$L^{3-c}$-N(-$L^{1-b}$-$D^{1-a}$)(-$L^{2-a}$-$D^{2-ag}$),
Exemplification 4-368: Z-$L^{3-c}$-N(-$L^{1-b}$-$D^{1-a}$)(-$L^{2-a}$-$D^{2-ah}$),
Exemplification 4-369: Z-$L^{3-c}$-N(-$L^{1-b}$-$D^{1-a}$)(-$L^{2-a}$-$D^{2-ai}$),
Exemplification 4-370: Z-$L^{3-c}$-N(-$L^{1-b}$-$D^{1-a}$)(-$L^{2-a}$-$D^{2-aj}$),
Exemplification 4-371: Z-$L^{3-c}$-N(-$L^{1-b}$-$D^{1-a}$)(-$L^{2-a}$-$D^{2-ak}$),
Exemplification 4-372: Z-$L^{3-c}$-N(-$L^{1-b}$-$D^{1-a}$)(-$L^{2-a}$-$D^{2-al}$),
Exemplification 4-373: Z-$L^{3-c}$-N(-$L^{1-b}$-$D^{1-a}$)(-$L^{2-a}$-$D^{2-am}$),

Exemplification 4-374: Z-$L^{3-c}$-N(-$L^{1-b}$-$D^{1-a}$)(-$L^{2-a}$-$D^{2-an}$),
Exemplification 4-375: Z-$L^{3-c}$-N(-$L^{1-b}$-$D^{1-a}$)(-$L^{2-a}$-$D^{2-ac}$),
Exemplification 4-376: Z-$L^{3-c}$-N(-$L^{1-b}$-$D^{1-a}$)(-$L^{2-a}$-$D^{2-ap}$),
Exemplification 4-377: Z-$L^{3-c}$-N(-$L^{1-b}$-$D^{1-a}$)(-$L^{2-b}$-$D^{2-aq}$),
Exemplification 4-378: Z-$L^{3-c}$-N(-$L^{1-b}$-$D^{1-a}$)(-$L^{2-b}$-$D^{2-ar}$),
Exemplification 4-379: Z-$L^{3-c}$-N(-$L^{1-b}$-$D^{1-a}$)(-$L^{2-b}$-$D^{2-as}$),
Exemplification 4-380: Z-$L^{3-c}$-N(-$L^{1-b}$-$D^{1-a}$)(-$L^{2-b}$-$D^{2-at}$),
Exemplification 4-381: Z-$L^{3-c}$-N(-$L^{1-b}$-$D^{1-a}$)(-$L^{2-c}$-$D^{2-aa}$),
Exemplification 4-382: Z-$L^{3-c}$-N(-$L^{1-b}$-$D^{1-a}$)(-$L^{2-c}$-$D^{2-ab}$),
Exemplification 4-383: Z-$L^{3-c}$-N(-$L^{1-b}$-$D^{1-a}$)(-$L^{2-c}$-$D^{2-ac}$),
Exemplification 4-384: Z-$L^{3-c}$-N(-$L^{1-b}$-$D^{1-a}$)(-$L^{2-c}$-$D^{2-ad}$),
Exemplification 4-385: Z-$L^{3-c}$-N(-$L^{1-b}$-$D^{1-a}$)(-$L^{2-c}$-$D^{2-ae}$),
Exemplification 4-386: Z-$L^{3-c}$-N(-$L^{1-b}$-$D^{1-a}$)(-$L^{2-c}$-$D^{2-af}$),
Exemplification 4-387: Z-$L^{3-c}$-N(-$L^{1-b}$-$D^{1-a}$)(-$L^{2-c}$-$D^{2-ag}$),
Exemplification 4-388: Z-$L^{3-c}$-N(-$L^{1-b}$-$D^{1-a}$)(-$L^{2-c}$-$D^{2-ah}$),
Exemplification 4-389: Z-$L^{3-c}$-N(-$L^{1-b}$-$D^{1-a}$)(-$L^{2-c}$-$D^{2-ai}$),
Exemplification 4-390: Z-$L^{3-c}$-N(-$L^{1-b}$-$D^{1-a}$)(-$L^{2-c}$-$D^{2-aj}$),
Exemplification 4-391: Z-$L^{3-c}$-N(-$L^{1-b}$-$D^{1-a}$)(-$L^{2-c}$-$D^{2-ak}$),
Exemplification 4-392: Z-$L^{3-c}$-N(-$L^{1-b}$-$D^{1-a}$)(-$L^{2-c}$-$D^{2-al}$),
Exemplification 4-393: Z-$L^{3-c}$-N(-$L^{1-b}$-$D^{1-a}$)(-$L^{2-c}$-$D^{2-am}$),
Exemplification 4-394: Z-$L^{3-c}$-N(-$L^{1-b}$-$D^{1-a}$)(-$L^{2-c}$-$D^{2-an}$),
Exemplification 4-395: Z-$L^{3-c}$-N(-$L^{1-b}$-$D^{1-a}$)(-$L^{2-c}$-$D^{2-ao}$),
Exemplification 4-396: Z-$L^{3-c}$-N(-$L^{1-b}$-$D^{1-a}$)(-$L^{2-c}$-$D^{2-ap}$),
Exemplification 4-397: Z-$L^{3-c}$-N(-$L^{1-b}$-$D^{1-a}$)(-$L^{2-d}$-$D^{2-aq}$),
Exemplification 4-398: Z-$L^{3-c}$-N(-$L^{1-b}$-$D^{1-a}$)(-$L^{2-d}$-$D^{2-ar}$),
Exemplification 4-399: Z-$L^{3-c}$-N(-$L^{1-b}$-$D^{1-a}$)(-$L^{2-d}$-$D^{2-as}$),
Exemplification 4-400: Z-$L^{3-c}$-N(-$L^{1-b}$-$D^{1-a}$)(-$L^{2-d}$-$D^{2-at}$),
Exemplification 4-401: Z-$L^{3-c}$-N(-$L^{1-a}$-$D^{1-b}$)(-$L^{2-a}$-$D^{2-aa}$),
Exemplification 4-402: Z-$L^{3-c}$-N(-$L^{1-a}$-$D^{1-b}$)(-$L^{2-a}$-$D^{2-ab}$),
Exemplification 4-403: Z-$L^{3-c}$-N(-$L^{1-a}$-$D^{1-b}$)(-$L^{2-a}$-$D^{2-ac}$),
Exemplification 4-404: Z-$L^{3-c}$-N(-$L^{1-a}$-$D^{1-b}$)(-$L^{2-a}$-$D^{2-ad}$),
Exemplification 4-405: Z-$L^{3-c}$-N(-$L^{1-a}$-$D^{1-b}$)(-$L^{2-a}$-$D^{2-ae}$),
Exemplification 4-406: Z-$L^{3-c}$-N(-$L^{1-a}$-$D^{1-b}$)(-$L^{2-a}$-$D^{2-af}$),
Exemplification 4-407: Z-$L^{3-c}$-N(-$L^{1-a}$-$D^{1-b}$)(-$L^{2-a}$-$D^{2-ag}$),
Exemplification 4-408: Z-$L^{3-c}$-N(-$L^{1-a}$-$D^{1-b}$)(-$L^{2-a}$-$D^{2-ah}$),
Exemplification 4-409: Z-$L^{3-c}$-N(-$L^{1-a}$-$D^{1-b}$)(-$L^{2-a}$-$D^{2-ai}$),
Exemplification 4-410: Z-$L^{3-c}$-N(-$L^{1-a}$-$D^{1-b}$)(-$L^{2-a}$-$D^{2-aj}$),
Exemplification 4-411: Z-$L^{3-c}$-N(-$L^{1-a}$-$D^{1-b}$)(-$L^{2-a}$-$D^{2-ak}$),
Exemplification 4-412: Z-$L^{3-c}$-N(-$L^{1-a}$-$D^{1-b}$)(-$L^{2-a}$-$D^{2-al}$),
Exemplification 4-413: Z-$L^{3-c}$-N(-$L^{1-a}$-$D^{1-b}$)(-$L^{2-a}$-$D^{2-am}$),
Exemplification 4-414: Z-$L^{3-c}$-N(-$L^{1-a}$-$D^{1-b}$)(-$L^{2-a}$-$D^{2-an}$),
Exemplification 4-415: Z-$L^{3-c}$-N(-$L^{1-a}$-$D^{1-b}$)(-$L^{2-a}$-$D^{2-ac}$),
Exemplification 4-416: Z-$L^{3-c}$-N(-$L^{1-a}$-$D^{1-b}$)(-$L^{2-a}$-$D^{2-ap}$),
Exemplification 4-417: Z-$L^{3-c}$-N(-$L^{1-a}$-$D^{1-b}$)(-$L^{2-b}$-$D^{2-aq}$),
Exemplification 4-418: Z-$L^{3-c}$-N(-$L^{1-a}$-$D^{1-b}$)(-$L^{2-b}$-$D^{2-ar}$),
Exemplification 4-419: Z-$L^{3-c}$-N(-$L^{1-a}$-$D^{1-b}$)(-$L^{2-b}$-$D^{2-as}$),
Exemplification 4-420: Z-$L^{3-c}$-N(-$L^{1-a}$-$D^{1-b}$)(-$L^{2-b}$-$D^{2-at}$),
Exemplification 4-421: Z-$L^{3-c}$-N(-$L^{1-a}$-$D^{1-b}$)(-$L^{2-c}$-$D^{2-an}$),
Exemplification 4-422: Z-$L^{3-c}$-N(-$L^{1-a}$-$D^{1-b}$)(-$L^{2-c}$-$D^{2-ab}$),
Exemplification 4-423: Z-$L^{3-c}$-N(-$L^{1-a}$-$D^{1-b}$)(-$L^{2-c}$-$D^{2-ac}$),
Exemplification 4-424: Z-$L^{3-c}$-N(-$L^{1-a}$-$D^{1-b}$)(-$L^{2-c}$-$D^{2-ad}$),
Exemplification 4-425: Z-$L^{3-c}$-N(-$L^{1-a}$-$D^{1-b}$)(-$L^{2-c}$-$D^{2-ae}$),
Exemplification 4-426: Z-$L^{3-c}$-N(-$L^{1-a}$-$D^{1-b}$)(-$L^{2-c}$-$D^{2-af}$),
Exemplification 4-427: Z-$L^{3-c}$-N(-$L^{1-a}$-$D^{1-b}$)(-$L^{2-c}$-$D^{2-ag}$),
Exemplification 4-428: Z-$L^{3-c}$-N(-$L^{1-a}$-$D^{1-b}$)(-$L^{2-c}$-$D^{2-ah}$),
Exemplification 4-429: Z-$L^{3-c}$-N(-$L^{1-a}$-$D^{1-b}$)(-$L^{2-c}$-$D^{2-ai}$),
Exemplification 4-430: Z-$L^{3-c}$-N(-$L^{1-a}$-$D^{1-b}$)(-$L^{2-c}$-$D^{2-aj}$),
Exemplification 4-431: Z-$L^{3-c}$-N(-$L^{1-a}$-$D^{1-b}$)(-$L^{2-c}$-$D^{2-ak}$),

Exemplification 4-432: Z-$L^{3-c}$-N(-$L^{1-a}$-$D^{1-b}$)(-$L^{2-c}$-$D^{2-al}$),
Exemplification 4-433: Z-$L^{3-c}$-N(-$L^{1-a}$-$D^{1-b}$)(-$L^{2-c}$-$D^{2-am}$),
Exemplification 4-434: Z-$L^{3-c}$-N(-$L^{1-a}$-$D^{1-b}$)(-$L^{2-c}$-$D^{2-an}$),
Exemplification 4-435: Z-$L^{3-c}$-N(-$L^{1-a}$-$D^{1-b}$)(-$L^{2-c}$-$D^{2-ao}$),
Exemplification 4-436: Z-$L^{3-c}$-N(-$L^{1-a}$-$D^{1-b}$)(-$L^{2-c}$-$D^{2-ap}$),
Exemplification 4-437: Z-$L^{3-c}$-N(-$L^{1-a}$-$D^{1-b}$)(-$L^{2-d}$-$D^{2-aq}$),
Exemplification 4-438: Z-$L^{3-c}$-N(-$L^{1-a}$-$D^{1-b}$)(-$L^{2-d}$-$D^{2-ar}$),
Exemplification 4-439: Z-$L^{3-c}$-N(-$L^{1-a}$-$D^{1-b}$)(-$L^{2-d}$-$D^{2-as}$),
Exemplification 4-440: Z-$L^{3-c}$-N(-$L^{1-a}$-$D^{1-b}$)(-$L^{2-d}$-$D^{2-at}$),
Exemplification 4-441: Z-$L^{3-c}$-N(-$L^{1-b}$-$D^{1-b}$)(-$L^{2-a}$-$D^{2-aa}$),
Exemplification 4-442: Z-$L^{3-c}$-N(-$L^{1-b}$-$D^{1-b}$)(-$L^{2-a}$-$D^{2-ab}$),
Exemplification 4-443: Z-$L^{3-c}$-N(-$L^{1-b}$-$D^{1-b}$)(-$L^{2-a}$-$D^{2-ac}$),
Exemplification 4-444: Z-$L^{3-c}$-N(-$L^{1-b}$-$D^{1-b}$)(-$L^{2-a}$-$D^{2-ad}$),
Exemplification 4-445: Z-$L^{3-c}$-N(-$L^{1-b}$-$D^{1-b}$)(-$L^{2-a}$-$D^{2-ae}$),
Exemplification 4-446: Z-$L^{3-c}$-N(-$L^{1-b}$-$D^{1-b}$)(-$L^{2-a}$-$D^{2-af}$),
Exemplification 4-447: Z-$L^{3-c}$-N(-$L^{1-b}$-$D^{1-b}$)(-$L^{2-a}$-$D^{2-ag}$),
Exemplification 4-448: Z-$L^{3-c}$-N(-$L^{1-b}$-$D^{1-b}$)(-$L^{2-a}$-$D^{2-ah}$),
Exemplification 4-449: Z-$L^{3-c}$-N(-$L^{1-b}$-$D^{1-b}$)(-$L^{2-a}$-$D^{2-ai}$),
Exemplification 4-450: Z-$L^{3-c}$-N(-$L^{1-b}$-$D^{1-b}$)(-$L^{2-a}$-$D^{2-aj}$),
Exemplification 4-451: Z-$L^{3-c}$-N(-$L^{1-b}$-$D^{1-b}$)(-$L^{2-a}$-$D^{2-ak}$),
Exemplification 4-452: Z-$L^{3-c}$-N(-$L^{1-b}$-$D^{1-b}$)(-$L^{2-a}$-$D^{2-al}$),
Exemplification 4-453: Z-$L^{3-c}$-N(-$L^{1-b}$-$D^{1-b}$)(-$L^{2-a}$-$D^{2-am}$),
Exemplification 4-454: Z-$L^{3-c}$-N(-$L^{1-b}$-$D^{1-b}$)(-$L^{2-a}$-$D^{2-an}$),
Exemplification 4-455: Z-$L^{3-c}$-N(-$L^{1-b}$-$D^{1-b}$)(-$L^{2-a}$-$D^{2-ac}$),
Exemplification 4-456: Z-$L^{3-c}$-N(-$L^{1-b}$-$D^{1-b}$)(-$L^{2-a}$-$D^{2-ap}$),
Exemplification 4-457: Z-$L^{3-c}$-N(-$L^{1-b}$-$D^{1-b}$)(-$L^{2-b}$-$D^{2-aq}$),
Exemplification 4-458: Z-$L^{3-c}$-N(-$L^{1-b}$-$D^{1-b}$)(-$L^{2-b}$-$D^{2-ar}$),
Exemplification 4-459: Z-$L^{3-c}$-N(-$L^{1-b}$-$D^{1-b}$)(-$L^{2-b}$-$D^{2-as}$),
Exemplification 4-460: Z-$L^{3-c}$-N(-$L^{1-b}$-$D^{1-b}$)(-$L^{2-b}$-$D^{2-at}$),
Exemplification 4-461: Z-$L^{3-c}$-N(-$L^{1-b}$-$D^{1-b}$)(-$L^{2-c}$-$D^{2-aa}$),
Exemplification 4-462: Z-$L^{3-c}$-N(-$L^{1-b}$-$D^{1-b}$)(-$L^{2-c}$-$D^{2-ab}$),
Exemplification 4-463: Z-$L^{3-c}$-N(-$L^{1-b}$-$D^{1-b}$)(-$L^{2-c}$-$D^{2-ac}$),
Exemplification 4-464: Z-$L^{3-c}$-N(-$L^{1-b}$-$D^{1-b}$)(-$L^{2-c}$-$D^{2-ad}$),
Exemplification 4-465: Z-$L^{3-c}$-N(-$L^{1-b}$-$D^{1-b}$)(-$L^{2-c}$-$D^{2-ae}$),
Exemplification 4-466: Z-$L^{3-c}$-N(-$L^{1-b}$-$D^{1-b}$)(-$L^{2-c}$-$D^{2-af}$),
Exemplification 4-467: Z-$L^{3-c}$-N(-$L^{1-b}$-$D^{1-b}$)(-$L^{2-c}$-$D^{2-ag}$),
Exemplification 4-468: Z-$L^{3-c}$-N(-$L^{1-b}$-$D^{1-b}$)(-$L^{2-c}$-$D^{2-ah}$),
Exemplification 4-469: Z-$L^{3-c}$-N(-$L^{1-b}$-$D^{1-b}$)(-$L^{2-c}$-$D^{2-ai}$),
Exemplification 4-470: Z-$L^{3-c}$-N(-$L^{1-b}$-$D^{1-b}$)(-$L^{2-c}$-$D^{2-aj}$),
Exemplification 4-471: Z-$L^{3-c}$-N(-$L^{1-b}$-$D^{1-b}$)(-$L^{2-c}$-$D^{2-ak}$),
Exemplification 4-472: Z-$L^{3-c}$-N(-$L^{1-b}$-$D^{1-b}$)(-$L^{2-c}$-$D^{2-al}$),
Exemplification 4-473: Z-$L^{3-c}$-N(-$L^{1-b}$-$D^{1-b}$)(-$L^{2-c}$-$D^{2-am}$),
Exemplification 4-474: Z-$L^{3-c}$-N(-$L^{1-b}$-$D^{1-b}$)(-$L^{2-c}$-$D^{2-an}$),
Exemplification 4-475: Z-$L^{3-c}$-N(-$L^{1-b}$-$D^{1-b}$)(-$L^{2-c}$-$D^{2-ao}$),
Exemplification 4-476: Z-$L^{3-c}$-N(-$L^{1-b}$-$D^{1-b}$)(-$L^{2-c}$-$D^{2-ap}$),
Exemplification 4-477: Z-$L^{3-c}$-N(-$L^{1-b}$-$D^{1-b}$)(-$L^{2-d}$-$D^{2-aq}$),
Exemplification 4-478: Z-$L^{3-c}$-N(-$L^{1-b}$-$D^{1-b}$)(-$L^{2-d}$-$D^{2-ar}$),
Exemplification 4-479: Z-$L^{3-c}$-N(-$L^{1-b}$-$D^{1-b}$)(-$L^{2-d}$-$D^{2-as}$),
Exemplification 4-480: Z-$L^{3-c}$-N(-$L^{1-b}$-$D^{1-b}$)(-$L^{2-d}$-$D^{2-at}$),
Exemplification 4-481: Z-$L^{3-d}$-N(-$L^{1-a}$-$D^{1-a}$)(-$L^{2-a}$-$D^{2-aa}$),
Exemplification 4-482: Z-$L^{3-d}$-N(-$L^{1-a}$-$D^{1-a}$)(-$L^{2-a}$-$D^{2-ab}$),
Exemplification 4-483: Z-$L^{3-d}$-N(-$L^{1-a}$-$D^{1-a}$)(-$L^{2-a}$-$D^{2-ac}$),
Exemplification 4-484: Z-$L^{3-d}$-N(-$L^{1-a}$-$D^{1-a}$)(-$L^{2-a}$-$D^{2-ad}$),
Exemplification 4-485: Z-$L^{3-d}$-N(-$L^{1-a}$-$D^{1-a}$)(-$L^{2-a}$-$D^{2-ae}$),
Exemplification 4-486: Z-$L^{3-d}$-N(-$L^{1-a}$-$D^{1-a}$)(-$L^{2-a}$-$D^{2-af}$),
Exemplification 4-487: Z-$L^{3-d}$-N(-$L^{1-a}$-$D^{1-a}$)(-$L^{2-a}$-$D^{2-ag}$),
Exemplification 4-488: Z-$L^{3-d}$-N(-$L^{1-a}$-$D^{1-a}$)(-$L^{2-a}$-$D^{2-ah}$),
Exemplification 4-489: Z-$L^{3-d}$-N(-$L^{1-a}$-$D^{1-a}$)(-$L^{2-a}$-$D^{2-ai}$),

Exemplification 4-490: $Z-L^{3-d}-N(-L^{1-a}-D^{1-a})(-L^{2-a}-D^{2-al})$,
Exemplification 4-491: $Z-L^{3-d}-N(-L^{1-a}-D^{1-a})(-L^{2-a}-D^{2-ak})$,
Exemplification 4-492: $Z-L^{3-d}-N(-L^{1-a}-D^{1-a})(-L^{2-a}-D^{2-al})$,
Exemplification 4-493: $Z-L^{3-d}-N(-L^{1-a}-D^{1-a})(-L^{2-a}-D^{2-am})$,
Exemplification 4-494: $Z-L^{3-d}-N(-L^{1-a}-D^{1-a})(-L^{2-a}-D^{2-an})$,
Exemplification 4-495: $Z-L^{3-d}-N(-L^{1-a}-D^{1-a})(-L^{2-a}-D^{2-ac})$,
Exemplification 4-496: $Z-L^{3-d}-N(-L^{1-a}-D^{1-a})(-L^{2-a}-D^{2-ap})$,
Exemplification 4-497: $Z-L^{3-d}-N(-L^{1-a}-D^{1-a})(-L^{2-b}-D^{2-aq})$,
Exemplification 4-498: $Z-L^{3-d}-N(-L^{1-a}-D^{1-a})(-L^{2-b}-D^{2-ar})$,
Exemplification 4-499: $Z-L^{3-d}-N(-L^{1-a}-D^{1-a})(-L^{2-b}-D^{2-as})$,
Exemplification 4-500: $Z-L^{3-d}-N(-L^{1-a}-D^{1-a})(-L^{2-b}-D^{2-at})$,
Exemplification 4-501: $Z-L^{3-d}-N(-L^{1-a}-D^{1-a})(-L^{2-c}-D^{2-aa})$,
Exemplification 4-502: $Z-L^{3-d}-N(-L^{1-a}-D^{1-a})(-L^{2-c}-D^{2-ab})$,
Exemplification 4-503: $Z-L^{3-d}-N(-L^{1-a}-D^{1-a})(-L^{2-c}-D^{2-ac})$,
Exemplification 4-504: $Z-L^{3-d}-N(-L^{1-a}-D^{1-a})(-L^{2-c}-D^{2-ad})$,
Exemplification 4-505: $Z-L^{3-d}-N(-L^{1-a}-D^{1-a})(-L^{2-c}-D^{2-ae})$,
Exemplification 4-506: $Z-L^{3-d}-N(-L^{1-a}-D^{1-a})(-L^{2-c}-D^{2-af})$,
Exemplification 4-507: $Z-L^{3-d}-N(-L^{1-a}-D^{1-a})(-L^{2-c}-D^{2-ag})$,
Exemplification 4-508: $Z-L^{3-d}-N(-L^{1-a}-D^{1-a})(-L^{2-c}-D^{2-ah})$,
Exemplification 4-509: $Z-L^{3-d}-N(-L^{1-a}-D^{1-a})(-L^{2-c}-D^{2-ai})$,
Exemplification 4-510: $Z-L^{3-d}-N(-L^{1-a}-D^{1-a})(-L^{2-c}-D^{2-aj})$,
Exemplification 4-511: $Z-L^{3-d}-N(-L^{1-a}-D^{1-a})(-L^{2-c}-D^{2-ak})$,
Exemplification 4-512: $Z-L^{3-d}-N(-L^{1-a}-D^{1-a})(-L^{2-c}-D^{2-al})$,
Exemplification 4-513: $Z-L^{3-d}-N(-L^{1-a}-D^{1-a})(-L^{2-c}-D^{2-am})$,
Exemplification 4-514: $Z-L^{3-d}-N(-L^{1-a}-D^{1-a})(-L^{2-c}-D^{2-an})$,
Exemplification 4-515: $Z-L^{3-d}-N(-L^{1-a}-D^{1-a})(-L^{2-c}-D^{2-ao})$,
Exemplification 4-516: $Z-L^{3-d}-N(-L^{1-a}-D^{1-a})(-L^{2-c}-D^{2-ap})$,
Exemplification 4-517: $Z-L^{3-d}-N(-L^{1-a}-D^{1-a})(-L^{2-d}-D^{2-aq})$,
Exemplification 4-518: $Z-L^{3-d}-N(-L^{1-a}-D^{1-a})(-L^{2-d}-D^{2-ar})$,
Exemplification 4-519: $Z-L^{3-d}-N(-L^{1-a}-D^{1-a})(-L^{2-d}-D^{2-as})$,
Exemplification 4-520: $Z-L^{3-d}-N(-L^{1-a}-D^{1-a})(-L^{2-d}-D^{2-at})$,
Exemplification 4-521: $Z-L^{3-d}-N(-L^{1-b}-D^{1-a})(-L^{2-a}-D^{2-aa})$,
Exemplification 4-522: $Z-L^{3-d}-N(-L^{1-b}-D^{1-a})(-L^{2-a}-D^{2-ab})$,
Exemplification 4-523: $Z-L^{3-d}-N(-L^{1-b}-D^{1-a})(-L^{2-a}-D^{2-ac})$,
Exemplification 4-524: $Z-L^{3-d}-N(-L^{1-b}-D^{1-a})(-L^{2-a}-D^{2-ad})$,
Exemplification 4-525: $Z-L^{3-d}-N(-L^{1-b}-D^{1-a})(-L^{2-a}-D^{2-ae})$,
Exemplification 4-526: $Z-L^{3-d}-N(-L^{1-b}-D^{1-a})(-L^{2-a}-D^{2-af})$,
Exemplification 4-527: $Z-L^{3-d}-N(-L^{1-b}-D^{1-a})(-L^{2-a}-D^{2-ag})$,
Exemplification 4-528: $Z-L^{3-d}-N(-L^{1-b}-D^{1-a})(-L^{2-a}-D^{2-ah})$,
Exemplification 4-529: $Z-L^{3-d}-N(-L^{1-b}-D^{1-a})(-L^{2-a}-D^{2-ai})$,
Exemplification 4-530: $Z-L^{3-d}-N(-L^{1-b}-D^{1-a})(-L^{2-a}-D^{2-aj})$,
Exemplification 4-531: $Z-L^{3-d}-N(-L^{1-b}-D^{1-a})(-L^{2-a}-D^{2-ak})$,
Exemplification 4-532: $Z-L^{3-d}-N(-L^{1-b}-D^{1-a})(-L^{2-a}-D^{2-al})$,
Exemplification 4-533: $Z-L^{3-d}-N(-L^{1-b}-D^{1-a})(-L^{2-a}-D^{2-am})$,
Exemplification 4-534: $Z-L^{3-d}-N(-L^{1-b}-D^{1-a})(-L^{2-a}-D^{2-an})$,
Exemplification 4-535: $Z-L^{3-d}-N(-L^{1-b}-D^{1-a})(-L^{2-a}-D^{2-ac})$,
Exemplification 4-536: $Z-L^{3-d}-N(-L^{1-b}-D^{1-a})(-L^{2-a}-D^{2-ap})$,
Exemplification 4-537: $Z-L^{3-d}-N(-L^{1-b}-D^{1-a})(-L^{2-b}-D^{2-aq})$,
Exemplification 4-538: $Z-L^{3-d}-N(-L^{1-b}-D^{1-a})(-L^{2-b}-D^{2-ar})$,
Exemplification 4-539: $Z-L^{3-d}-N(-L^{1-b}-D^{1-a})(-L^{2-b}-D^{2-as})$,
Exemplification 4-540: $Z-L^{3-d}-N(-L^{1-b}-D^{1-a})(-L^{2-b}-D^{2-at})$,
Exemplification 4-541: $Z-L^{3-d}-N(-L^{1-b}-D^{1-a})(-L^{2-c}-D^{2-aa})$,
Exemplification 4-542: $Z-L^{3-d}-N(-L^{1-b}-D^{1-a})(-L^{2-c}-D^{2-ab})$,
Exemplification 4-543: $Z-L^{3-d}-N(-L^{1-b}-D^{1-a})(-L^{2-c}-D^{2-ac})$,
Exemplification 4-544: $Z-L^{3-d}-N(-L^{1-b}-D^{1-a})(-L^{2-c}-D^{2-ad})$,
Exemplification 4-545: $Z-L^{3-d}-N(-L^{1-b}-D^{1-a})(-L^{2-c}-D^{2-ae})$,
Exemplification 4-546: $Z-L^{3-d}-N(-L^{1-b}-D^{1-a})(-L^{2-c}-D^{2-af})$,
Exemplification 4-547: $Z-L^{3-d}-N(-L^{1-b}-D^{1-a})(-L^{2-c}-D^{2-ag})$,

Exemplification 4-548: Z-$L^{3-d}$-N(-$L^{1-b}$-$D^{1-a}$)(-$L^{2-c}$-$D^{2-ah}$),
Exemplification 4-549: Z-$L^{3-d}$-N(-$L^{1-b}$-$D^{1-a}$)(-$L^{2-c}$-$D^{2-ai}$),
Exemplification 4-550: Z-$L^{3-d}$-N(-$L^{1-b}$-$D^{1-a}$)(-$L^{2-c}$-$D^{2-aj}$),
Exemplification 4-551: Z-$L^{3-d}$-N(-$L^{1-b}$-$D^{1-a}$)(-$L^{2-c}$-$D^{2-ak}$),
Exemplification 4-552: Z-$L^{3-d}$-N(-$L^{1-b}$-$D^{1-a}$)(-$L^{2-c}$-$D^{2-al}$),
Exemplification 4-553: Z-$L^{3-d}$-N(-$L^{1-b}$-$D^{1-a}$)(-$L^{2-c}$-$D^{2-am}$),
Exemplification 4-554: Z-$L^{3-d}$-N(-$L^{1-b}$-$D^{1-a}$)(-$L^{2-c}$-$D^{2-an}$),
Exemplification 4-555: Z-$L^{3-d}$-N(-$L^{1-b}$-$D^{1-a}$)(-$L^{2-c}$-$D^{2-ao}$),
Exemplification 4-556: Z-$L^{3-d}$-N(-$L^{1-b}$-$D^{1-a}$)(-$L^{2-c}$-$D^{2-ap}$),
Exemplification 4-557: Z-$L^{3-d}$-N(-$L^{1-b}$-$D^{1-a}$)(-$L^{2-d}$-$D^{2-aq}$),
Exemplification 4-558: Z-$L^{3-d}$-N(-$L^{1-b}$-$D^{1-a}$)(-$L^{2-d}$-$D^{2-ar}$),
Exemplification 4-559: Z-$L^{3-d}$-N(-$L^{1-b}$-$D^{1-a}$)(-$L^{2-d}$-$D^{2-as}$),
Exemplification 4-560: Z-$L^{3-d}$-N(-$L^{1-b}$-$D^{1-a}$)(-$L^{2-d}$-$D^{2-at}$),
Exemplification 4-561: Z-$L^{3-d}$-N(-$L^{1-a}$-$D^{1-b}$)(-$L^{2-a}$-$D^{2-aa}$),
Exemplification 4-562: Z-$L^{3-d}$-N(-$L^{1-a}$-$D^{1-b}$)(-$L^{2-a}$-$D^{2-ab}$),
Exemplification 4-563: Z-$L^{3-d}$-N(-$L^{1-a}$-$D^{1-b}$)(-$L^{2-a}$-$D^{2-ac}$),
Exemplification 4-564: Z-$L^{3-d}$-N(-$L^{1-a}$-$D^{1-b}$)(-$L^{2-a}$-$D^{2-ad}$),
Exemplification 4-565: Z-$L^{3-d}$-N(-$L^{1-a}$-$D^{1-b}$)(-$L^{2-a}$-$D^{2-ae}$),
Exemplification 4-566: Z-$L^{3-d}$-N (-$L^{1-a}$-$D^{1-b}$)(-$L^{2-a}$-$D^{2-af}$),
Exemplification 4-567: Z-$L^{3-d}$-N (-$L^{1-a}$-$D^{1-b}$)(-$L^{2-a}$-$D^{2-ag}$),
Exemplification 4-568: Z-$L^{3-d}$-N (-$L^{1-a}$-$D^{1-b}$)(-$L^{2-a}$-$D^{2-ah}$),
Exemplification 4-569: Z-$L^{3-d}$-N (-$L^{1-a}$-$D^{1-b}$)(-$L^{2-a}$-$D^{2-ai}$),
Exemplification 4-570: Z-$L^{3-d}$-N (-$L^{1-a}$-$D^{1-b}$)(-$L^{2-a}$-$D^{2-aj}$),
Exemplification 4-571: Z-$L^{3-d}$-N (-$L^{1-a}$-$D^{1-b}$)(-$L^{2-a}$-$D^{2-ak}$),
Exemplification 4-572: Z-$L^{3-d}$-N (-$L^{1-a}$-$D^{1-b}$)(-$L^{2-a}$-$D^{2-al}$),
Exemplification 4-573: Z-$L^{3-d}$-N (-$L^{1-a}$-$D^{1-b}$)(-$L^{2-a}$-$D^{2-am}$),
Exemplification 4-574: Z-$L^{3-d}$-N (-$L^{1-a}$-$D^{1-b}$)(-$L^{2-a}$-$D^{2-an}$),
Exemplification 4-575: Z-$L^{3-d}$-N (-$L^{1-a}$-$D^{1-b}$)(-$L^{2-a}$-$D^{2-ac}$),
Exemplification 4-576: Z-$L^{3-d}$-N (-$L^{1-a}$-$D^{1-b}$)(-$L^{2-a}$-$D^{2-ap}$),
Exemplification 4-577: Z-$L^{3-d}$-N (-$L^{1-a}$-$D^{1-b}$)(-$L^{2-b}$-$D^{2-aq}$),
Exemplification 4-578: Z-$L^{3-d}$-N (-$L^{1-a}$-$D^{1-b}$)(-$L^{2-b}$-$D^{2-ar}$),
Exemplification 4-579: Z-$L^{3-d}$-N (-$L^{1-a}$-$D^{1-b}$)(-$L^{2-b}$-$D^{2-as}$),
Exemplification 4-580: Z-$L^{3-d}$-N (-$L^{1-a}$-$D^{1-b}$)(-$L^{2-b}$-$D^{2-at}$),
Exemplification 4-581: Z-$L^{3-d}$-N (-$L^{1-a}$-$D^{1-b}$)(-$L^{2-c}$-$D^{2-aa}$),
Exemplification 4-582: Z-$L^{3-d}$-N (-$L^{1-a}$-$D^{1-b}$)(-$L^{2-c}$-$D^{2-ab}$),
Exemplification 4-583: Z-$L^{3-d}$-N (-$L^{1-a}$-$D^{1-b}$)(-$L^{2-c}$-$D^{2-ac}$),
Exemplification 4-584: Z-$L^{3-d}$-N (-$L^{1-a}$-$D^{1-b}$)(-$L^{2-c}$-$D^{2-ad}$),
Exemplification 4-585: Z-$L^{3-d}$-N (-$L^{1-a}$-$D^{1-b}$)(-$L^{2-c}$-$D^{2-ae}$),
Exemplification 4-586: Z-$L^{3-d}$-N (-$L^{1-a}$-$D^{1-b}$)(-$L^{2-c}$-$D^{2-af}$),
Exemplification 4-587: Z-$L^{3-d}$-N (-$L^{1-a}$-$D^{1-b}$)(-$L^{2-c}$-$D^{2-ag}$),
Exemplification 4-588: Z-$L^{3-d}$-N (-$L^{1-a}$-$D^{1-b}$)(-$L^{2-c}$-$D^{2-ah}$),
Exemplification 4-589: Z-$L^{3-d}$-N (-$L^{1-a}$-$D^{1-b}$)(-$L^{2-c}$-$D^{2-ai}$),
Exemplification 4-590: Z-$L^{3-d}$-N (-$L^{1-a}$-$D^{1-b}$)(-$L^{2-c}$-$D^{2-aj}$),
Exemplification 4-591: Z-$L^{3-d}$-N (-$L^{1-a}$-$D^{1-b}$)(-$L^{2-c}$-$D^{2-ak}$),
Exemplification 4-592: Z-$L^{3-d}$-N (-$L^{1-a}$-$D^{1-b}$)(-$L^{2-c}$-$D^{2-al}$),
Exemplification 4-593: Z-$L^{3-d}$-N (-$L^{1-a}$-$D^{1-b}$)(-$L^{2-c}$-$D^{2-am}$),
Exemplification 4-594: Z-$L^{3-d}$-N (-$L^{1-a}$-$D^{1-b}$)(-$L^{2-c}$-$D^{2-an}$),
Exemplification 4-595: Z-$L^{3-d}$-N (-$L^{1-a}$-$D^{1-b}$)(-$L^{2-c}$-$D^{2-ao}$),
Exemplification 4-596: Z-$L^{3-d}$-N (-$L^{1-a}$-$D^{1-b}$)(-$L^{2-c}$-$D^{2-ap}$),
Exemplification 4-597: Z-$L^{3-d}$-N (-$L^{1-a}$-$D^{1-b}$)(-$L^{2-d}$-$D^{2-aq}$),
Exemplification 4-598: Z-$L^{3-d}$-N (-$L^{1-a}$-$D^{1-b}$)(-$L^{2-d}$-$D^{2-ar}$),
Exemplification 4-599: Z-$L^{3-d}$-N (-$L^{1-a}$-$D^{1-b}$)(-$L^{2-d}$-$D^{2-as}$),
Exemplification 4-600: Z-$L^{3-d}$-N (-$L^{1-a}$-$D^{1-b}$)(-$L^{2-d}$-$D^{2-at}$),
Exemplification 4-601: Z-$L^{3-d}$-N (-$L^{1-b}$-$D^{1-b}$)(-$L^{2-a}$-$D^{2-an}$),
Exemplification 4-602: Z-$L^{3-d}$-N (-$L^{1-b}$-$D^{1-b}$)(-$L^{2-a}$-$D^{2-ab}$),
Exemplification 4-603: Z-$L^{3-d}$-N (-$L^{1-b}$-$D^{1-b}$)(-$L^{2-a}$-$D^{2-ac}$),
Exemplification 4-604: Z-$L^{3-d}$-N (-$L^{1-b}$-$D^{1-b}$)(-$L^{2-a}$-$D^{2-ad}$),
Exemplification 4-605: Z-$L^{3-d}$-N (-$L^{1-b}$-$D^{1-b}$)(-$L^{2-a}$-$D^{2-ae}$),

Exemplification 4-606: Z-$L^{3-d}$-N (-$L^{1-b}$-$D^{1-b}$)(-$L^{2-a}$-$D^{2-af}$),
Exemplification 4-607: Z-$L^{3-d}$-N (-$L^{1-b}$-$D^{1-b}$)(-$L^{2-a}$-$D^{2-ag}$),
Exemplification 4-608: Z-$L^{3-d}$-N (-$L^{1-b}$-$D^{1-b}$)(-$L^{2-a}$-$D^{2-ah}$),
Exemplification 4-609: Z-$L^{3-d}$-N (-$L^{1-b}$-$D^{1-b}$)(-$L^{2-a}$-$D^{2-ai}$),
Exemplification 4-610: Z-$L^{3-d}$-N (-$L^{1-b}$-$D^{1-b}$)(-$L^{2-a}$-$D^{2-aj}$),
Exemplification 4-611: Z-$L^{3-d}$-N (-$L^{1-b}$-$D^{1-b}$)(-$L^{2-a}$-$D^{2-ak}$),
Exemplification 4-612: Z-$L^{3-d}$-N (-$L^{1-b}$-$D^{1-b}$)(-$L^{2-a}$-$D^{2-al}$),
Exemplification 4-613: Z-$L^{3-d}$-N (-$L^{1-b}$-$D^{1-b}$)(-$L^{2-a}$-$D^{2-am}$),
Exemplification 4-614: Z-$L^{3-d}$-N (-$L^{1-b}$-$D^{1-b}$)(-$L^{2-a}$-$D^{2-an}$),
Exemplification 4-615: Z-$L^{3-d}$-N (-$L^{1-b}$-$D^{1-b}$)(-$L^{2-a}$-$D^{2-ao}$),
Exemplification 4-616: Z-$L^{3-d}$-N (-$L^{1-b}$-$D^{1-b}$)(-$L^{2-a}$-$D^{2-ap}$),
Exemplification 4-617: Z-$L^{3-d}$-N (-$L^{1-b}$-$D^{1-b}$)(-$L^{2-b}$-$D^{2-aq}$),
Exemplification 4-618: Z-$L^{3-d}$-N (-$L^{1-b}$-$D^{1-b}$)(-$L^{2-b}$-$D^{2-ar}$),
Exemplification 4-619: Z-$L^{3-d}$-N (-$L^{1-b}$-$D^{1-b}$)(-$L^{2-b}$-$D^{2-as}$),
Exemplification 4-620: Z-$L^{3-d}$-N (-$L^{1-b}$-$D^{1-b}$)(-$L^{2-b}$-$D^{2-at}$),
Exemplification 4-621: Z-$L^{3-d}$-N (-$L^{1-b}$-$D^{1-b}$)(-$L^{2-c}$-$D^{2-aa}$),
Exemplification 4-622: Z-$L^{3-d}$-N (-$L^{1-b}$-$D^{1-b}$)(-$L^{2-c}$-$D^{2-ab}$),
Exemplification 4-623: Z-$L^{3-d}$-N (-$L^{1-b}$-$D^{1-b}$)(-$L^{2-c}$-$D^{2-ao}$),
Exemplification 4-624: Z-$L^{3-d}$-N (-$L^{1-b}$-$D^{1-b}$)(-$L^{2-c}$-$D^{2-ad}$),
Exemplification 4-625: Z-$L^{3-d}$-N (-$L^{1-b}$-$D^{1-b}$)(-$L^{2-c}$-$D^{2-ae}$),
Exemplification 4-626: Z-$L^{3-d}$-N (-$L^{1-b}$-$D^{1-b}$)(-$L^{2-c}$-$D^{2-af}$),
Exemplification 4-627: Z-$L^{3-d}$-N (-$L^{1-b}$-$D^{1-b}$)(-$L^{2-c}$-$D^{2-ag}$),
Exemplification 4-628: Z-$L^{3-d}$-N (-$L^{1-b}$-$D^{1-b}$)(-$L^{2-c}$-$D^{2-ah}$),
Exemplification 4-629: Z-$L^{3-d}$-N (-$L^{1-b}$-$D^{1-b}$)(-$L^{2-c}$-$D^{2-ai}$),
Exemplification 4-630: Z-$L^{3-d}$-N (-$L^{1-b}$-$D^{1-b}$)(-$L^{2-c}$-$D^{2-aj}$),
Exemplification 4-631: Z-$L^{3-d}$-N (-$L^{1-b}$-$D^{1-b}$)(-$L^{2-c}$-$D^{2-ak}$),
Exemplification 4-632: Z-$L^{3-d}$-N (-$L^{1-b}$-$D^{1-b}$)(-$L^{2-c}$-$D^{2-al}$),
Exemplification 4-633: Z-$L^{3-d}$-N (-$L^{1-b}$-$D^{1-b}$)(-$L^{2-c}$-$D^{2-am}$),
Exemplification 4-634: Z-$L^{3-d}$-N (-$L^{1-b}$-$D^{1-b}$)(-$L^{2-c}$-$D^{2-aa}$),
Exemplification 4-635: Z-$L^{3-d}$-N (-$L^{1-b}$-$D^{1-b}$)(-$L^{2-c}$-$D^{2-ao}$),
Exemplification 4-636: Z-$L^{3-d}$-N (-$L^{1-b}$-$D^{1-b}$)(-$L^{2-c}$-$D^{2-ap}$),
Exemplification 4-637: Z-$L^{3-d}$-N (-$L^{1-b}$-$D^{1-b}$)(-$L^{2-d}$-$D^{2-aq}$),
Exemplification 4-638: Z-$L^{3-d}$-N (-$L^{1-b}$-$D^{1-b}$)(-$L^{2-d}$-$D^{2-ar}$),
Exemplification 4-639: Z-$L^{3-d}$-N (-$L^{1-b}$-$D^{1-b}$)(-$L^{2-d}$-$D^{2-as}$),
Exemplification 4-640: Z-$L^{3-d}$-N (-$L^{1-b}$-$D^{1-b}$)(-$L^{2-d}$-$D^{2-at}$),
Exemplification 4-641: Z-$L^{3-e}$-N(-$L^{1-a}$-$D^{1-a}$)(-$L^{2-a}$-$D^{2-aa}$),
Exemplification 4-642: Z-$L^{3-e}$-N(-$L^{1-a}$-$D^{1-a}$)(-$L^{2-a}$-$D^{2-ab}$),
Exemplification 4-643: Z-$L^{3-e}$-N(-$L^{1-a}$-$D^{1-a}$)(-$L^{2-a}$-$D^{2-ac}$),
Exemplification 4-644: Z-$L^{3-e}$-N(-$L^{1-a}$-$D^{1-a}$)(-$L^{2-a}$-$D^{2-ad}$),
Exemplification 4-645: Z-$L^{3-e}$-N(-$L^{1-a}$-$D^{1-a}$)(-$L^{2-a}$-$D^{2-ae}$),
Exemplification 4-646: Z-$L^{3-e}$-N(-$L^{1-a}$-$D^{1-a}$)(-$L^{2-a}$-$D^{2-af}$),
Exemplification 4-647: Z-$L^{3-e}$-N(-$L^{1-a}$-$D^{1-a}$)(-$L^{2-a}$-$D^{2-ag}$),
Exemplification 4-648: Z-$L^{3-e}$-N(-$L^{1-a}$-$D^{1-a}$)(-$L^{2-a}$-$D^{2-ah}$),
Exemplification 4-649: Z-$L^{3-e}$-N(-$L^{1-a}$-$D^{1-a}$)(-$L^{2-a}$-$D^{2-ai}$),
Exemplification 4-650: Z-$L^{3-e}$-N(-$L^{1-a}$-$D^{1-a}$)(-$L^{2-a}$-$D^{2-aj}$),
Exemplification 4-651: Z-$L^{3-e}$-N(-$L^{1-a}$-$D^{1-a}$)(-$L^{2-a}$-$D^{2-ak}$),
Exemplification 4-652: Z-$L^{3-e}$-N(-$L^{1-a}$-$D^{1-a}$)(-$L^{2-a}$-$D^{2-al}$),
Exemplification 4-653: Z-$L^{3-e}$-N(-$L^{1-a}$-$D^{1-a}$)(-$L^{2-a}$-$D^{2-am}$),
Exemplification 4-654: Z-$L^{3-e}$-N(-$L^{1-a}$-$D^{1-a}$)(-$L^{2-a}$-$D^{2-an}$),
Exemplification 4-655: Z-$L^{3-e}$-N(-$L^{1-a}$-$D^{1-a}$)(-$L^{2-a}$-$D^{2-ao}$),
Exemplification 4-656: Z-$L^{3-e}$-N(-$L^{1-a}$-$D^{1-a}$)(-$L^{2-a}$-$D^{2-ap}$),
Exemplification 4-657: Z-$L^{3-e}$-N(-$L^{1-a}$-$D^{1-a}$)(-$L^{2-b}$-$D^{2-aq}$),
Exemplification 4-658: Z-$L^{3-e}$-N(-$L^{1-a}$-$D^{1-a}$)(-$L^{2-b}$-$D^{2-ar}$),
Exemplification 4-659: Z-$L^{3-e}$-N(-$L^{1-a}$-$D^{1-a}$)(-$L^{2-b}$-$D^{2-as}$),
Exemplification 4-660: Z-$L^{3-e}$-N(-$L^{1-a}$-$D^{1-a}$)(-$L^{2-b}$-$D^{2-at}$),
Exemplification 4-661: Z-$L^{3-e}$-N(-$L^{1-a}$-$D^{1-a}$)(-$L^{2-c}$-$D^{2-aa}$),
Exemplification 4-662: Z-$L^{3-e}$-N(-$L^{1-a}$-$D^{1-a}$)(-$L^{2-c}$-$D^{2-ab}$),
Exemplification 4-663: Z-$L^{3-e}$-N(-$L^{1-a}$-$D^{1-a}$)(-$L^{2-c}$-$D^{2-ao}$),

Exemplification 4-664: $Z-L^{3-e}-N(-L^{1-a}-D^{1-a})(-L^{2-c}-D^{2-ad})$,

Exemplification 4-665: $Z-L^{3-e}-N(-L^{1-a}-D^{1-a})(-L^{2-c}-D^{2-ae})$,

Exemplification 4-666: $Z-L^{3-e}-N(-L^{1-a}-D^{1-a})(-L^{2-c}-D^{2-af})$,

Exemplification 4-667: $Z-L^{3-e}-N(-L^{1-a}-D^{1-a})(-L^{2-c}-D^{2-ag})$,

Exemplification 4-668: $Z-L^{3-e}-N\ (-_{L}{}^{l}\text{-a-}_{D}\text{l-a})(-L^{2-c}-D^{2-ah})$,

Exemplification 4-669: $Z-L^{3-e}-N(-L^{1-a}-D^{1-a})(-L^{2-c}-D^{2-ai})$,

Exemplification 4-670: $Z-L^{3-e}-N(-L^{1-a}-D^{1-a})(-L^{2-c}-D^{2-a1})$,

Exemplification 4-671: $Z-L^{3-e}-N(-L^{1-a}-D^{1-a})(-L^{2-c}-D^{2-ak})$,

Exemplification 4-672: $Z-L^{3-e}-N(-L^{1-a}-D^{1-a})(-L^{2-c}-D^{2-a1})$,

Exemplification 4-673: $Z-L^{3-e}-N(-L^{1-a}-D^{1-a})(-L^{2-c}-D^{2-am})$,

Exemplification 4-674: $Z-L^{3-e}-N(-L^{1-a}-D^{1-a})(-L^{2-c}-D^{2-an})$,

Exemplification 4-675: $Z-L^{3-e}-N(-L^{1-a}-D^{1-a})(-L^{2-c}-D^{2-ao})$,

Exemplification 4-676: $Z-L^{3-e}-N(-L^{1-a}-D^{1-a})(-L^{2-c}-D^{2-ap})$,

Exemplification 4-677: $Z-L^{3-e}-N(-L^{1-a}-D^{1-a})(-L^{2-d}-D^{2-aq})$,

Exemplification 4-678: $Z-L^{3-e}-N(-L^{1-a}-D^{1-a})(-L^{2-d}-D^{2-ar})$,

Exemplification 4-679: $Z-L^{3-e}-N(-L^{1-a}-D^{1-a})(-L^{2-d}-D^{2-as})$,

Exemplification 4-680: $Z-L^{3-e}-N(-L^{1-a}-D^{1-a})(-L^{2-d}-D^{2-at})$,

Exemplification 4-681: $Z-L^{3-e}-N(-L^{1-b}-D^{1-a})(-L^{2-a}-D^{2-an})$,

Exemplification 4-682: $Z-L^{3-e}-N(-L^{1-b}-D^{1-a})(-L^{2-a}-D^{2-ab})$,

Exemplification 4-683: $Z-L^{3-e}-N(-L^{1-b}-D^{1-a})(-L^{2-a}-D^{2-ac})$,

Exemplification 4-684: $Z-L^{3-e}-N(-L^{1-b}-D^{1-a})(-L^{2-a}-D^{2-ad}$,

Exemplification 4-685: $Z-L^{3-e}-N(-L^{1-b}-D^{1-a})(-L^{2-a}-D^{2-ae})$,

Exemplification 4-686: $Z-L^{3-e}-N(-L^{1-b}-D^{1-a})(-L^{2-a}-D^{2-af})$,

Exemplification 4-687: $Z-L^{3-e}-N(-L^{1-b}-D^{1-a})(-L^{2-a}-D^{2-ag})$,

Exemplification 4-688: $Z-L^{3-e}-N(-L^{1-b}-D^{1-a})(-L^{2-a}-D^{2-ah})$,

Exemplification 4-689: $Z-L^{3-e}-N(-L^{1-b}-D^{1-a})(-L^{2-a}-D^{2-ai})$,

Exemplification 4-690: $Z-L^{3-e}-N(-L^{1-b}-D^{1-a})(-L^{2-a}-D^{2-aj})$,

Exemplification 4-691: $Z-L^{3-e}-N(-L^{1-b}-D^{1-a})(-L^{2-a}-D^{2-ak})$,

Exemplification 4-692: $Z-L^{3-e}-N(-L^{1-b}-D^{1-a})(-L^{2-a}-D^{2-al})$,

Exemplification 4-693: $Z-L^{3-e}-N(-L^{1-b}-D^{1-a})(-L^{2-a}-D^{2-am})$,

Exemplification 4-694: $Z-L^{3-e}-N(-L^{1-b}-D^{1-a})(-L^{2-a}-D^{2-an})$,

Exemplification 4-695: $Z-L^{3-e}-N(-L^{1-b}-D^{1-a})(-L^{2-a}-D^{2-ao})$,

Exemplification 4-696: $Z-L^{3-e}-N(-L^{1-b}-D^{1-a})(-L^{2-a}-D^{2-ap})$,

Exemplification 4-697: $Z-L^{3-e}-N(-L^{1-b}-D^{1-a})(-L^{2-b}-D^{2-aq})$,

Exemplification 4-698: $Z-L^{3-e}-N(-L^{1-b}-D^{1-a})(-L^{2-b}-D^{2-ar})$,

Exemplification 4-699: $Z-L^{3-e}-N(-L^{1-b}-D^{1-a})(-L^{2-b}-D^{2-as})$,

Exemplification 4-700: $Z-L^{3-e}-N(-L^{1-b}-D^{1-a})(-L^{2-b}-D^{2-at})$,

Exemplification 4-701: $Z-L^{3-e}-N(-L^{1-b}-D^{1-a})(-L^{2-c}-D^{2-aa})$,

Exemplification 4-702: $Z-L^{3-e}-N(-L^{1-b}-D^{1-a})(-L^{2-c}-D^{2-ab})$,

Exemplification 4-703: $Z-L^{3-e}-N(-L^{1-b}-D^{1-a})(-L^{2-c}-D^{2-ao})$,

Exemplification 4-704: $Z-L^{3-e}-N(-L^{1-b}-D^{1-a})(-L^{2-c}-D^{2-ad})$,

Exemplification 4-705: $Z-L^{3-e}-N(-L^{1-b}-D^{1-a})(-L^{2-c}-D^{2-ae})$,

Exemplification 4-706: $Z-L^{3-e}-N(-L^{1-b}-D^{1-a})(-L^{2-c}-D^{2-af})$,

Exemplification 4-707: $Z-L^{3-e}-N(-L^{1-b}-D^{1-a})(-L^{2-c}-D^{2-ag})$,

Exemplification 4-708: $Z-L^{3-e}-N(-L^{1-b}-D^{1-a})(-L^{2-c}-D^{2-ah})$,

Exemplification 4-709: $Z-L^{3-e}-N(-L^{1-b}-D^{1-a})(-L^{2-c}-D^{2-ai})$,

Exemplification 4-710: $Z-L^{3-e}-N(-L^{1-b}-D^{1-a})(-L^{2-c}-D^{2-a1})$,

Exemplification 4-711: $Z-L^{3-e}-N(-L^{1-b}-D^{1-a})(-L^{2-c}-D^{2-ak})$,

Exemplification 4-712: $Z-L^{3-e}-N(-L^{1-b}-D^{1-a})(-L^{2-c}-D^{2-al})$,

Exemplification 4-713: $Z-L^{3-e}-N(-L^{1-b}-D^{1-a})(-L^{2-c}-D^{2-am})$,

Exemplification 4-714: $Z-L^{3-e}-N(-L^{1-b}-D^{1-a})(-L^{2-c}-D^{2-an})$,

Exemplification 4-715: $Z-L^{3-e}-N(-L^{1-b}-D^{1-a})(-L^{2-c}-D^{2-ao})$,

Exemplification 4-716: $Z-L^{3-e}-N(-L^{1-b}-D^{1-a})(-L^{2-c}-D^{2-ap})$,

Exemplification 4-717: $Z-L^{3-e}-N(-L^{1-b}-D^{1-a})(-L^{2-d}-D^{2-aq})$,

Exemplification 4-718: $Z-L^{3-e}-N(-L^{1-b}-D^{1-a})(-L^{2-d}-D^{2-ar})$,

Exemplification 4-719: $Z-L^{3-e}-N(-L^{1-b}-D^{1-a})(-L^{2-d}-D^{2-as})$,

Exemplification 4-720: $Z-L^{3-e}-N(-L^{1-b}-D^{1-a})(-L^{2-d}-D^{2-at})$,

Exemplification 4-721: $Z-L^{3-e}-N(-L^{1-a}-D^{1-b})(-L^{2-a}-D^{2-aa})$,

Exemplification 4-722: $Z-L^{3-e}-N(-L^{1-a}-D^{1-b})(-L^{2-a}-D^{2-ab})$,
Exemplification 4-723: $Z-L^{3-e}-N(-L^{1-a}-D^{1-b})(-L^{2-a}-D^{2-ac})$,
Exemplification 4-724: $Z-L^{3-e}-N(-L^{1-a}-D^{1-b})(-L^{2-a}-D^{2-ad})$,
Exemplification 4-725: $Z-L^{3-e}-N(-L^{1-a}-D^{1-b})(-L^{2-a}-D^{2-ae})$,
Exemplification 4-726: $Z-L^{3-e}-N(-L^{1-a}-D^{1-b})(-L^{2-a}-D^{2-af})$,
Exemplification 4-727: $Z-L^{3-e}-N(-L^{1-a}-D^{1-b})(-L^{2-a}-D^{2-ag})$,
Exemplification 4-728: $Z-L^{3-e}-N(-L^{1-a}-D^{1-b})(-L^{2-a}-D^{2-ah})$,
Exemplification 4-729: $Z-L^{3-e}-N(-L^{1-a}-D^{1-b})(-L^{2-a}-D^{2-ai})$,
Exemplification 4-730: $Z-L^{3-e}-N(-L^{1-a}-D^{1-b})(-L^{2-a}-D^{2-aj})$,
Exemplification 4-731: $Z-L^{3-e}-N(-L^{1-a}-D^{1-b})(-L^{2-a}-D^{2-ak})$,
Exemplification 4-732: $Z-L^{3-e}-N(-L^{1-a}-D^{1-b})(-L^{2-a}-D^{2-al})$,
Exemplification 4-733: $Z-L^{3-e}-N(-L^{1-a}-D^{1-b})(-L^{2-a}-D^{2-am})$,
Exemplification 4-734: $Z-L^{3-e}-N(-L^{1-a}-D^{1-b})(-L^{2-a}-D^{2-an})$,
Exemplification 4-735: $Z-L^{3-e}-N(-L^{1-a}-D^{1-b})(-L^{2-a}-D^{2-ao})$,
Exemplification 4-736: $Z-L^{3-e}-N(-L^{1-a}-D^{1-b})(-L^{2-a}-D^{2-ap})$,
Exemplification 4-737: $Z-L^{3-e}-N(-L^{1-a}-D^{1-b})(-L^{2-b}-D^{2-aq})$,
Exemplification 4-738: $Z-L^{3-e}-N(-L^{1-a}-D^{1-b})(-L^{2-b}-D^{2-ar})$,
Exemplification 4-739: $Z-L^{3-e}-N(-L^{1-a}-D^{1-b})(-L^{2-b}-D^{2-as})$,
Exemplification 4-740: $Z-L^{3-e}-N(-L^{1-a}-D^{1-b})(-L^{2-b}-D^{2-at})$,
Exemplification 4-741: $Z-L^{3-e}-N(-L^{1-a}-D^{1-b})(-L^{2-c}-D^{2-aa})$,
Exemplification 4-742: $Z-L^{3-e}-N(-L^{1-a}-D^{1-b})(-L^{2-c}-D^{2-ab})$,
Exemplification 4-743: $Z-L^{3-e}-N(-L^{1-a}-D^{1-b})(-L^{2-c}-D^{2-ao})$,
Exemplification 4-744: $Z-L^{3-e}-N(-L^{1-a}-D^{1-b})(-L^{2-c}-D^{2-ad})$,
Exemplification 4-745: $Z-L^{3-e}-N(-L^{1-a}-D^{1-b})(-L^{2-c}-D^{2-ae})$,
Exemplification 4-746: $Z-L^{3-e}-N(-L^{1-a}-D^{1-b})(-L^{2-c}-D^{2-af})$,
Exemplification 4-747: $Z-L^{3-e}-N(-L^{1-a}-D^{1-b})(-L^{2-c}-D^{2-ag})$,
Exemplification 4-748: $Z-L^{3-e}-N(-L^{1-a}-D^{1-b})(-L^{2-c}-D^{2-ah})$,
Exemplification 4-749: $Z-L^{3-e}-N(-L^{1-a}-D^{1-b})(-L^{2-c}-D^{2-ai})$,
Exemplification 4-750: $Z-L^{3-e}-N(-L^{1-a}-D^{1-b})(-L^{2-c}-d^{2-aj})$,
Exemplification 4-751: $Z-L^{3-e}-N(-L^{1-a}-D^{1-b})(-L^{2-c}-D^{2-ak})$,
Exemplification 4-752: $Z-L^{3-e}-N(-L^{1-a}-D^{1-b})(-L^{2-c}-D^{2-a1})$,
Exemplification 4-753: $Z-L^{3-e}-N(-L^{1-a}-D^{1-b})(-L^{2-c}-D^{2-am})$,
Exemplification 4-754: $Z-L^{3-e}-N(-L^{1-a}-D^{1-b})(-L^{2-c}-D^{2-an})$,
Exemplification 4-755: $Z-L^{3-e}-N(-L^{1-a}-D^{1-b})(-L^{2-c}-D^{2-ao})$,
Exemplification 4-756: $Z-L^{3-e}-N(-L^{1-a}-D^{1-b})(-L^{2-c}-D^{2-ap})$,
Exemplification 4-757: $Z-L^{3-e}-N(-L^{1-a}-D^{1-b})(-L^{2-d}-D^{2-aq})$,
Exemplification 4-758: $Z-L^{3-e}-N(-L^{1-a}-D^{1-b})(-L^{2-d}-D^{2-ar})$,
Exemplification 4-759: $Z-L^{3-e}-N(-L^{1-a}-D^{1-b})(-L^{2-d}-D^{2-as})$,
Exemplification 4-760: $Z-L^{3-e}-N(-L^{1-a}-D^{1-b})(-L^{2-d}-D^{2-at})$,
Exemplification 4-761: $Z-L^{3-e}-N(-L^{1-b}-D^{1-b})(-L^{2-a}-D^{2-aa})$,
Exemplification 4-762: $Z-L^{3-e}-N(-L^{1-b}-D^{1-b})(-L^{2-a}-D^{2-ab})$,
Exemplification 4-763: $Z-L^{3-e}-N(-L^{1-b}-D^{1-b})(-L^{2-a}-D^{2-ac})$,
Exemplification 4-764: $Z-L^{3-e}-N(-L^{1-b}-D^{1-b})(-L^{2-a}-D^{2-ad})$,
Exemplification 4-765: $Z-L^{3-e}-N(-L^{1-b}-D^{1-b})(-L^{2-a}-D^{2-ae})$,
Exemplification 4-766: $Z-L^{3-e}-N(-L^{1-b}-D^{1-b})(-L^{2-a}-D^{2-af})$,
Exemplification 4-767: $Z-L^{3-e}-N(-L^{1-b}-D^{1-b})(-L^{2-a}-D^{2-ag})$,
Exemplification 4-768: $Z-L^{3-e}-N(-L^{1-b}-D^{1-b})(-L^{2-a}-D^{2-ah})$,
Exemplification 4-769: $Z-L^{3-e}-N(-L^{1-b}-D^{1-b})(-L^{2-a}-D^{2-ai})$,
Exemplification 4-770: $Z-L^{3-e}-N(-L^{1-b}-D^{1-b})(-L^{2-a}-D^{2-aj})$,
Exemplification 4-771: $Z-L^{3-e}-N(-L^{1-b}-D^{1-b})(-L^{2-a}-D^{2-ak})$,
Exemplification 4-772: $Z-L^{3-e}-N(-L^{1-b}-D^{1-b})(-L^{2-a}-D^{2-al})$,
Exemplification 4-773: $Z-L^{3-e}-N(-L^{1-b}-D^{1-b})(-L^{2-a}-D^{2-am})$,
Exemplification 4-774: $Z-L^{3-e}-N(-L^{1-b}-D^{1-b})(-L^{2-a}-D^{2-an})$,
Exemplification 4-775: $Z-L^{3-e}-N(-L^{1-b}-D^{1-b})(-L^{2-a}-D^{2-ao})$,
Exemplification 4-776: $Z-L^{3-e}-N(-L^{1-b}-D^{1-b})(-L^{2-a}-D^{2-ap})$,
Exemplification 4-777: $Z-L^{3-e}-N(-L^{1-b}-D^{1-b})(-L^{2-b}-D^{2-aq})$,
Exemplification 4-778: $Z-L^{3-e}-N(-L^{1-b}-D^{1-b})(-L^{2-b}-D^{2-ar})$,
Exemplification 4-779: $Z-L^{3-e}-N(-L^{1-b}-D^{1-b})(-L^{2-b}-D^{2-as})$,

Exemplification 4-780: $Z-L^{3-e}-N(-L^{1-b}-D^{1-b})(-L^{2-b}-D^{2-at})$,
Exemplification 4-781: $Z-L^{3-e}-N(-L^{1-b}-D^{1-b})(-L^{2-c}-D^{2-aa})$,
Exemplification 4-782: $Z-L^{3-e}-N(-L^{1-b}-D^{1-b})(-L^{2-c}-D^{2-ab})$,
Exemplification 4-783: $Z-L^{3-e}-N(-L^{1-b}-D^{1-b})(-L^{2-c}-D^{2-ao})$,
Exemplification 4-784: $Z-L^{3-e}-N(-L^{1-b}-D^{1-b})(-L^{2-c}-D^{2-ad})$,
Exemplification 4-785: $Z-L^{3-e}-N(-L^{1-b}-D^{1-b})(-L^{2-c}-D^{2-ae})$,
Exemplification 4-786: $Z-L^{3-e}-N(-L^{1-b}-D^{1-b})(-L^{2-c}-D^{2-af})$,
Exemplification 4-787: $Z-L^{3-e}-N(-L^{1-b}-D^{1-b})(-L^{2-c}-D^{2-ag})$,
Exemplification 4-788: $Z-L^{3-e}-N(-L^{1-b}-D^{1-b})(-L^{2-c}-D^{2-ah})$,
Exemplification 4-789: $Z-L^{3-e}-N(-L^{1-b}-D^{1-b})(-L^{2-c}-D^{2-ai})$,
Exemplification 4-790: $Z-L^{3-e}-N(-L^{1-b}-D^{1-b})(-L^{2-c}-D^{2-a1})$,
Exemplification 4-791: $Z-L^{3-e}-N(-L^{1-b}-D^{1-b})(-L^{2-c}-D^{2-ak})$,
Exemplification 4-792: $Z-L^{3-e}-N(-L^{1-b}-D^{1-b})(-L^{2-c}-D^{2-a1})$,
Exemplification 4-793: $Z-L^{3-e}-N(-L^{1-b}-D^{1-b})(-L^{2-c}-D^{2-am})$,
Exemplification 4-794: $Z-L^{3-e}-N(-L^{1-b}-D^{1-b})(-L^{2-c}-D^{2-an})$,
Exemplification 4-795: $Z-L^{3-e}-N(-L^{1-b}-D^{1-b})(-L^{2-c}-D^{2-ao})$,
Exemplification 4-796: $Z-L^{3-e}-N(-L^{1-b}-D^{1-b})(-L^{2-c}-D^{2-ap})$,
Exemplification 4-797: $Z-L^{3-e}-N(-L^{1-b}-D^{1-b})(-L^{2-d}-D^{2-aq})$,
Exemplification 4-798: $Z-L^{3-e}-N(-L^{1-b}-D^{1-b})(-L^{2-d}-D^{2-ar})$,
Exemplification 4-799: $Z-L^{3-e}-N(-L^{1-b}-D^{1-b})(-L^{2-d}-D^{2-as})$,
Exemplification 4-800: $Z-L^{3-e}-N(-L^{1-b}-D^{1-b})(-L^{2-d}-D^{2-at})$,
Exemplification 4-801: $Z-L^{3-f}-N(-L^{1-a}-D^{1-a})(-L^{2-a}-D^{2-aa})$,
Exemplification 4-802: $Z-L^{3-f}-N(-L^{1-a}-D^{1-a})(-L^{2-a}-D^{2-ab})$,
Exemplification 4-803: $Z-L^{3-f}-N(-L^{1-a}-D^{1-a})(-L^{2-a}-D^{2-ac})$,
Exemplification 4-804: $Z-L^{3-f}-N(-L^{1-a}-D^{1-a})(-L^{2-a}-D^{2-ad})$,
Exemplification 4-805: $Z-L^{3-f}-N(-L^{1-a}-D^{1-a})(-L^{2-a}-D^{2-ae})$,
Exemplification 4-806: $Z-L^{3-f}-N(-L^{1-a}-D^{1-a})(-L^{2-a}-D^{2-af})$,
Exemplification 4-807: $Z-L^{3-f}-N(-L^{1-a}-D^{1-a})(-L^{2-a}-D^{2-ag})$,
Exemplification 4-808: $Z-L^{3-f}-N(-L^{1-a}-D^{1-a})(-L^{2-a}-D^{2-ah})$,
Exemplification 4-809: $Z-L^{3-f}-N(-L^{1-a}-D^{1-a})(-L^{2-a}-D^{2-ai})$,
Exemplification 4-810: $Z-1^{3-f}-N(-L^{1-a}-D^{1-a})(-L^{2-a}-D^{2-ai})$,
Exemplification 4-811: $Z-1^{3-f}-N(-L^{1-a}-D^{1-a})(-L^{2-a}-D^{2-ak})$,
Exemplification 4-812: $Z-L^{3-f}-N(-L^{1-a}-D^{1-a})(-L^{2-a}-D^{2-al})$,
Exemplification 4-813: $Z-L^{3-f}-N(-L^{1-a}-D^{1-a})(-L^{2-a}-D^{2-am})$,
Exemplification 4-814: $Z-L^{3-f}-N(-L^{1-a}-D^{1-a})(-L^{2-a}-D^{2-an})$,
Exemplification 4-815: $Z-L^{3-f}-N(-L^{1-a}-D^{1-a})(-L^{2-a}-D^{2-ao})$,
Exemplification 4-816: $Z-L^{3-f}-N(-L^{1-a}-D^{1-a})(-L^{2-a}-D^{2-ap})$,
Exemplification 4-817: $Z-L^{3-f}-N(-L^{1-a}-D^{1-a})(-L^{2-b}-D^{2-aq})$,
Exemplification 4-818: $Z-L^{3-f}-N(-L^{1-a}-D^{1-a})(-L^{2-b}-D^{2-ar})$,
Exemplification 4-819: $Z-L^{3-f}-N(-L^{1-a}-D^{1-a})(-L^{2-b}-D^{2-as})$,
Exemplification 4-820: $Z-L^{3-f}-N(-L^{1-a}-D^{1-a})(-L^{2-b}-D^{2-at})$,
Exemplification 4-821: $Z-L^{3-f}-N(-L^{1-a}-D^{1-a})(-L^{2-c}-D^{2-aa})$,
Exemplification 4-822: $Z-L^{3-f}-N(-L^{1-a}-D^{1-a})(-L^{2-c}-D^{2-ab})$,
Exemplification 4-823: $Z-L^{3-f}-N(-L^{1-a}-D^{1-a})(-L^{2-c}-D^{2-ao})$,
Exemplification 4-824: $Z-L^{3-f}-N(-L^{1-a}-D^{1-a})(-I^{2-c}-D^{2-ad})$,
Exemplification 4-825: $Z-L^{3-f}-N(-L^{1-a}-D^{1-a})(-L^{2-c}-D^{2-ae})$,
Exemplification 4-826: $Z-L^{3-f}-N(-L^{1-a}-D^{1-a})(-L^{2-c}-D^{2-af})$,
Exemplification 4-827: $Z-L^{3-f}-N(-L^{1-a}-D^{1-a})(-L^{2-c}-D^{2-ag})$,
Exemplification 4-828: $Z-L^{3-f}-N(-L^{1-a}-D^{1-a})(-L^{2-c}-D^{2-ah})$,
Exemplification 4-829: $Z-L^{3-f}-N(-L^{1-a}-D^{1-a})(-L^{2-c}-D^{2-ai})$,
Exemplification 4-830: $Z-L^{3-f}-N(-L^{1-a}-D^{1-a})(-L^{2-c}-D^{2-a1})$,
Exemplification 4-831: $Z-L^{3-f}-N(-L^{1-a}-D^{1-a})(-L^{2-c}-D^{2-ak})$,
Exemplification 4-832: $Z-L^{3-f}-N(-L^{1-a}-D^{1-a})(-L^{2-c}-D^{2-a1})$,
Exemplification 4-833: $Z-L^{3-f}-N(-L^{1-a}-D^{1-a})(-L^{2-c}-D^{2-am})$,
Exemplification 4-834: $Z-L^{3-f}-N(-L^{1-a}-D^{1-a})(-L^{2-c}-D^{2-an})$,
Exemplification 4-835: $Z-L^{3-f}-N(-L^{1-a}-D^{1-a})(-L^{2-c}-D^{2-ao})$,
Exemplification 4-836: $Z-L^{3-f}-N(-L^{1-a}-D^{1-a})(-L^{2-c}-D^{2-ap})$,
Exemplification 4-837: $Z-L^{3-f}-N(-L^{1-a}-D^{1-a})(-L^{2-d}-D^{2-aq})$,

Exemplification 4-838: $Z-L^{3-f}-N(-L^{1-a}-D^{1-a})(-L^{2-d}-D^{2-ar})$,
Exemplification 4-839: $Z-L^{3-f}-N(-L^{1-a}-D^{1-a})(-L^{2-d}-D^{2-as})$,
Exemplification 4-840: $Z-L^{3-f}-N(-L^{1-a}-D^{1-a})(-L^{2-d}-D^{2-at})$,
Exemplification 4-841: $Z-L^{3-f}-N(-L^{1-b}-D^{1-a})(-L^{2-a}-D^{2-aa})$,
Exemplification 4-842: $Z-L^{3-f}-N(-L^{1-b}-D^{1-a})(-L^{2-a}-D^{2-ab})$,
Exemplification 4-843: $Z-L^{3-f}-N(-L^{1-b}-D^{1-a})(-L^{2-a}-D^{2-ac})$,
Exemplification 4-844: $Z-L^{3-f}-N(-L^{1-b}-D^{1-a})(-L^{2-a}-D^{2-ad})$,
Exemplification 4-845: $Z-L^{3-f}-N(-L^{1-b}-D^{1-a})(-L^{2-a}-D^{2-ae})$,
Exemplification 4-846: $Z-L^{3-f}-N(-L^{1-b}-D^{1-a})(-L^{2-a}-D^{2-af})$,
Exemplification 4-847: $Z-L^{3-f}-N(-L^{1-b}-D^{1-a})(-L^{2-a}-D^{2-ag})$,
Exemplification 4-848: $Z-L^{3-f}-N(-L^{1-b}-D^{1-a})(-L^{2-a}-D^{2-ah})$,
Exemplification 4-849: $Z-L^{3-f}-N(-L^{1-b}-D^{1-a})(-L^{2-a}-D^{2-ai})$,
Exemplification 4-850: $Z-L^{3-f}-N(-L^{1-b}-D^{1-a})(-L^{2-a}-D^{2-aj})$,
Exemplification 4-851: $Z-L^{3-f}-N(-L^{1-b}-D^{1-a})(-L^{2-a}-D^{2-ak})$,
Exemplification 4-852: $Z-L^{3-f}-N(-L^{1-b}-D^{1-a})(-L^{2-a}-D^{2-al})$,
Exemplification 4-853: $Z-L^{3-f}-N(-L^{1-b}-D^{1-a})(-L^{2-a}-D^{2-am})$,
Exemplification 4-854: $Z-L^{3-f}-N(-L^{1-b}-D^{1-a})(-L^{2-a}-D^{2-an})$,
Exemplification 4-855: $Z-L^{3-f}-N(-L^{1-b}-D^{1-a})(-L^{2-a}-D^{2-ao})$,
Exemplification 4-856: $Z-L^{3-f}-N(-L^{1-b}-D^{1-a})(-L^{2-a}-D^{2-ap})$,
Exemplification 4-857: $Z-L^{3-f}-N(-L^{1-b}-D^{1-a})(-L^{2-b}-D^{2-aq})$,
Exemplification 4-858: $Z-L^{3-f}-N(-L^{1-b}-D^{1-a})(-L^{2-b}-D^{2-ar})$,
Exemplification 4-859: $Z-L^{3-f}-N(-L^{1-b}-D^{1-a})(-L^{2-b}-D^{2-as})$,
Exemplification 4-860: $Z-L^{3-f}-N(-L^{1-b}-D^{1-a})(-L^{2-b}-D^{2-at})$,
Exemplification 4-861: $Z-L^{3-f}-N(-L^{1-b}-D^{1-a})(-L^{2-c}-D^{2-aa})$,
Exemplification 4-862: $Z-L^{3-f}-N(-L^{1-b}-D^{1-a})(-L^{2-c}-D^{2-ab})$,
Exemplification 4-863: $Z-L^{3-f}-N(-L^{1-b}-D^{1-a})(-L^{2-c}-D^{2-ao})$,
Exemplification 4-864: $Z-L^{3-f}-N(-L^{1-b}-D^{1-a})(-L^{2-c}-D^{2-ad})$,
Exemplification 4-865: $Z-L^{3-f}-N(-L^{1-b}-D^{1-a})(-L^{2-c}-D^{2-ae})$,
Exemplification 4-866: $Z-L^{3-f}-N(-L^{1-b}-D^{1-a})(-L^{2-c}-D^{2-af})$,
Exemplification 4-867: $Z-L^{3-f}-N(-L^{1-b}-D^{1-a})(-L^{2-c}-D^{2-ag})$,
Exemplification 4-868: $Z-L^{3-f}-N(-L^{1-b}-D^{1-a})(-L^{2-c}-D^{2-ah})$,
Exemplification 4-869: $Z-L^{3-f}-N(-L^{1-b}-D^{1-a})(-L^{2-c}-D^{2-ai})$,
Exemplification 4-870: $Z-L^{3-f}-N(-L^{1-b}-D^{1-a})(-L^{2-c}-D^{2-aj})$,
Exemplification 4-871: $Z-L^{3-f}-N(-L^{1-b}-D^{1-a})(-L^{2-c}-D^{2-ak})$,
Exemplification 4-872: $Z-L^{3-f}-N(-L^{1-b}-D^{1-a})(-L^{2-c}-D^{2-a1})$,
Exemplification 4-873: $Z-L^{3-f}-N(-L^{1-b}-D^{1-a})(-L^{2-c}-D^{2-am})$,
Exemplification 4-874: $Z-L^{3-f}-N(-L^{1-b}-D^{1-a})(-L^{2-c}-D^{2-an})$,
Exemplification 4-875: $Z-L^{3-f}-N(-L^{1-b}-D^{1-a})(-L^{2-c}-D^{2-ao})$,
Exemplification 4-876: $Z-L^{3-f}-N(-L^{1-b}-D^{1-a})(-L^{2-c}-D^{2-ap})$,
Exemplification 4-877: $Z-L^{3-f}-N(-L^{1-b}-D^{1-a})(-L^{2-d}-D^{2-aq})$,
Exemplification 4-878: $Z-L^{3-f}-N(-L^{1-b}-D^{1-a})(-L^{2-d}-D^{2-ar})$,
Exemplification 4-879: $Z-L^{3-f}-N(-L^{1-b}-D^{1-a})(-L^{2-d}-D^{2-as})$,
Exemplification 4-880: $Z-L^{3-f}-N(-L^{1-b}-D^{1-a})(-L^{2-d}-D^{2-at})$,
Exemplification 4-881: $Z-L^{3-f}-N(-L^{1-a}-D^{1-b})(-L^{2-a}-D^{2-aa})$,
Exemplification 4-882: $Z-L^{3-f}-N(-L^{1-a}-D^{1-b})(-L^{2-a}-D^{2-ab})$,
Exemplification 4-883: $Z-L^{3-f}-N(-L^{1-a}-D^{1-b})(-L^{2-a}-D^{2-ac})$,
Exemplification 4-884: $Z-L^{3-f}-N(-L^{1-a}-D^{1-b})(-L^{2-a}-D^{2-ad})$,
Exemplification 4-885: $Z-L^{3-f}-N(-L^{1-a}-D^{1-b})(-L^{2-a}-D^{2-ae})$,
Exemplification 4-886: $Z-L^{3-f}-N(-L^{1-a}-D^{1-b})(-L^{2-a}-D^{2-af})$,
Exemplification 4-887: $Z-L^{3-f}-N(-L^{1-a}-D^{1-b})(-L^{2-a}-D^{2-ag})$,
Exemplification 4-888: $Z-L^{3-f}-N(-L^{1-a}-D^{1-b})(-L^{2-a}-D^{2-ah})$,
Exemplification 4-889: $Z-L^{3-f}-N(-L^{1-a}-D^{1-b})(-L^{2-a}-D^{2-ai})$,
Exemplification 4-890: $Z-L^{3-f}-N(-L^{1-a}-D^{1-b})(-L^{2-a}-D^{2-aj})$,
Exemplification 4-891: $Z-L^{3-f}-N(-L^{1-a}-D^{1-b})(-L^{2-a}-D^{2-ak})$,
Exemplification 4-892: $Z-L^{3-f}-N(-L^{1-a}-D^{1-b})(-L^{2-a}-D^{2-al})$,
Exemplification 4-893: $Z-L^{3-f}-N(-L^{1-a}-D^{1-b})(-L^{2-a}-D^{2-am})$,
Exemplification 4-894: $Z-L^{3-f}-N(-L^{1-a}-D^{1-b})(-L^{2-a}-D^{2-an})$,
Exemplification 4-895: $Z-1^{3-f}-N(-L^{1-a}-D^{1-b})(-L^{2-a}-D^{2-ao})$ ,

Exemplification 4-896: $Z-L^{3-f}-N(-L^{1-a}-D^{1-b})(-L^{2-a}-D^{2-ap})$,
Exemplification 4-897: $Z-L^{3-f}-N(-L^{1-a}-D^{1-b})(-L^{2-b}-D^{2-aq})$,
Exemplification 4-898: $Z-L^{3-f}-N(-L^{1-a}-D^{1-b})(-L^{2-b}-D^{2-ar})$,
Exemplification 4-899: $Z-L^{3-f}-N(-L^{1-a}-D^{1-b})(-L^{2-b}-D^{2-as})$,
Exemplification 4-900: $Z-L^{3-f}-N(-L^{1-a}-D^{1-b})(-L^{2-b}-D^{2-at})$,
Exemplification 4-901: $Z-1^{3-f}-N(-L^{1}-a-D^{1-b})(-L^{2-c}-D^{2-aa})$,
Exemplification 4-902: $Z-L^{3-f}-N(-L^{1-a}-D^{1-b})(-L^{2-c}-D^{2-ab})$,
Exemplification 4-903: $Z-L^{3-f}-N(-L^{1-a}-D^{1-b})(-L^{2-c}-D^{2-ac})$,
Exemplification 4-904: $Z-L^{3-f}-N(-L^{1-a}-D^{1-b})(-L^{2-c}-D^{2-ad})$,
Exemplification 4-905: $Z-1^{3-f}-N(-L^{1-a}-D^{1-b})(-L^{2-c}-D^{2-ae})$,
Exemplification 4-906: $Z-L^{3-f}-N(-L^{1-a}-D^{1-b})(-L^{2-c}-D^{2-af})$,
Exemplification 4-907: $Z-L^{3-f}-N(-L^{1-a}-D^{1-b})(-L^{2-c}-D^{2-ag})$,
Exemplification 4-908: $Z-L^{3-f}-N(-L^{1-a}-D^{1-b})(-L^{2-c}-D^{2-ah})$,
Exemplification 4-909: $Z-L^{3-f}-N(-L^{1-a}-D^{1-b})(-L^{2-c}-D^{2-ai})$,
Exemplification 4-910: $Z-L^{3-f}-N(-L^{1-a}-D^{1-b})(-L^{2-c}-D^{2-aj})$,
Exemplification 4-911: $Z-L^{3-f}-N(-L^{1-a}-D^{1-b})(-L^{2-c}-D^{2-ak})$,
Exemplification 4-912: $Z-L^{3-f}-N(-L^{1-a}-D^{1-b})(-L^{2-c}-D^{2-al})$,
Exemplification 4-913: $Z-L^{3-f}-N(-L^{1-a}-D^{1-b})(-L^{2-c}-D^{2-am})$,
Exemplification 4-914: $Z-L^{3-f}-N(-L^{1-a}-D^{1-b})(-L^{2-c}-D^{2-an})$,
Exemplification 4-915: $Z-L^{3-f}-N(-L^{1-a}-D^{1-b})(-L^{2-c}-D^{2-ao})$,
Exemplification 4-916: $Z-L^{3-f}-N(-L^{1-a}-D^{1-b})(-L^{2-c}-D^{2-ap})$,
Exemplification 4-917: $Z-L^{3-f}-N(-L^{1-a}-D^{1-b})(-L^{2-d}-D^{2-aq})$,
Exemplification 4-918: $Z-L^{3-f}-N(-L^{1-a}-D^{1-b})(-L^{2-d}-D^{2-ar})$,
Exemplification 4-919: $Z-L^{3-f}-N(-L^{1-a}-D^{1-b})(-L^{2-d}-D^{2-as})$,
Exemplification 4-920: $Z-L^{3-f}-N(-L^{1-a}-D^{1-b})(-L^{2-d}-D^{2-at})$,
Exemplification 4-921: $Z-L^{3-f}-N(-L^{1-b}-D^{1-b})(-L^{2-a}-D^{2-aa})$,
Exemplification 4-922: $Z-L^{3-f}-N(-L^{1-b}-D^{1-b})(-L^{2-a}-D^{2-ab})$,
Exemplification 4-923: $Z-L^{3-f}-N\ (-L^{1-b}-D^{1-b})(-L^{2-a}-D^{2-ac})$,
Exemplification 4-924: $Z-L^{3-f}-N(-L^{1-b}-D^{1-b})(-L^{2-a}-D^{2-ad})$,
Exemplification 4-925: $Z-L^{3-f}-N(-L^{1-b}-D^{1-b})(-L^{2-a}-D^{2-ae})$,
Exemplification 4-926: $Z-L^{3-f}-N(-L^{1-b}-D^{1-b})(-L^{2-a}-D^{2-af})$,
Exemplification 4-927: $Z-L^{3-f}-N(-L^{1-b}-D^{1-b})(-L^{2-a}-D^{2-ag})$,
Exemplification 4-928: $Z-L^{3-f}-N(-L^{1-b}-D^{1-b})(-L^{2-a}-D^{2-ah})$,
Exemplification 4-929: $Z-L^{3-f}-N(-L^{1-b}-D^{1-b})(-L^{2-a}-D^{2-ai})$,
Exemplification 4-930: $Z-L^{3-f}-N(-L^{1-b}-D^{1-b})(-L^{2-a}-D^{2-aj})$,
Exemplification 4-931: $Z-L^{3-f}-N(-L^{1-b}-D^{1-b})(-L^{2-a}-D^{2-ak})$,
Exemplification 4-932: $Z-L^{3-f}-N(-L^{1-b}-D^{1-b})(-L^{2-a}-D^{2-al})$,
Exemplification 4-933: $Z-L^{3-f}-N(-L^{1-b}-D^{1-b})(-L^{2-a}-D^{2-am})$,
Exemplification 4-934: $Z-L^{3-f}-N(-L^{1-b}-D^{1-b})(-L^{2-a}-D^{2-an})$,
Exemplification 4-935: $Z-L^{3-f}-N(-L^{1-b}-D^{1-b})(-L^{2-a}-D^{2-ao})$,
Exemplification 4-936: $Z-L^{3-f}-N(-L^{1-b}-D^{1-b})(-L^{2-a}-D^{2-ap})$,
Exemplification 4-937: $Z-L^{3-f}-N(-L^{1-b}-D^{1-b})(-L^{2-b}-D^{2-aq})$,
Exemplification 4-938: $Z-L^{3-f}-N(-L^{1-b}-D^{1-b})(-L^{2-b}-D^{2-ar})$,
Exemplification 4-939: $Z-L^{3-f}-N(-L^{1-b}-D^{1-b})(-L^{2-b}-D^{2-as})$,
Exemplification 4-940: $Z-L^{3-f}-N(-L^{1-b}-D^{1-b})(-L^{2-b}-D^{2-at})$,
Exemplification 4-941: $Z-L^{3-f}-N(-L^{1-b}-D^{1-b})(-L^{2-c}-D^{2-aa})$,
Exemplification 4-942: $Z-L^{3-f}-N(-L^{1-b}-D^{1-b})(-L^{2-c}-D^{2-ab})$,
Exemplification 4-943: $Z-L^{3-f}-N(-L^{1-b}-D^{1-b})(-L^{2-c}-D^{2-ac})$,
Exemplification 4-944: $Z-L^{3-f}-N(-L^{1-b}-D^{1-b})(-L^{2-c}-D^{2-ad})$,
Exemplification 4-945: $Z-L^{3-f}-N(-L^{1-b}-D^{1-b})(-L^{2-c}-D^{2-ae})$,
Exemplification 4-946: $Z-L^{3-f}-N(-L^{1-b}-D^{1-b})(-L^{2-c}-D^{2-af})$,
Exemplification 4-947: $Z-L^{3-f}-N(-L^{1-b}-D^{1-b})(-L^{2-c}-D^{2-ag})$,
Exemplification 4-948: $Z-L^{3-f}-N(-L^{1-b}-D^{1-b})(-L^{2-c}-D^{2-ah})$,
Exemplification 4-949: $Z-L^{3-f}-N(-L^{1-b}-D^{1-b})(-L^{2-c}-D^{2-ai})$,
Exemplification 4-950: $Z-L^{3-f}-N(-L^{1-b}-D^{1-b})(-L^{2-c}-D^{2-aj})$,
Exemplification 4-951: $Z-L^{3-f}-N(-L^{1-b}-D^{1-b})(-L^{2-c}-D^{2-ak})$,
Exemplification 4-952: $Z-L^{3-f}-N(-L^{1-b}-D^{1-b})(-L^{2-c}-D^{2-al})$,
Exemplification 4-953: $Z-L^{3-f}-N(-L^{1-b}-D^{1-b})(-L^{2-c}-D^{2-am})$,

Exemplification 4-954: Z-$L^{3-f}$-N(-$L^{1-b}$-$D^{1-b}$)(-$L^{2-c}$-$D^{2-an}$),
Exemplification 4-955: Z-$L^{3-f}$-N(-$L^{1-b}$-$D^{1-b}$)(-$L^{2-c}$-$D^{2-ao}$),
Exemplification 4-956: Z-$L^{3-f}$-N(-$L^{1-b}$-$D^{1-b}$)(-$L^{2-c}$-$D^{2-ap}$),
Exemplification 4-957: Z-$L^{3-f}$-N(-$L^{1-b}$-$D^{1-b}$)(-$L^{2-d}$-$D^{2-aq}$),
Exemplification 4-958: Z-$L^{3-f}$-N(-$L^{1-b}$-$D^{1-b}$)(-$L^{2-d}$-$D^{2-ar}$),
Exemplification 4-959: Z-$L^{3-f}$-N(-$L^{1-b}$-$D^{1-b}$)(-$L^{2-d}$-$D^{2-as}$),
Exemplification 4-960: Z-$L^{3-f}$-N(-$L^{1-b}$-$D^{1-b}$)(-$L^{2-d}$-$D^{2-at}$),
Exemplification 4-961: Z-$L^{3-g}$-N(-$L^{1-a}$-$D^{1-a}$)(-$L^{2-a}$-$D^{2-aa}$),
Exemplification 4-962: Z-$L^{3-g}$-N(-$L^{1-a}$-$D^{1-a}$)(-$L^{2-a}$-$D^{2-ab}$),
Exemplification 4-963: Z-$L^{3-g}$-N(-$L^{1-a}$-$D^{1-a}$)(-$L^{2-a}$-$D^{2-ac}$),
Exemplification 4-964: Z-$L^{3-g}$-N(-$L^{1-a}$-$D^{1-a}$)(-$L^{2-a}$-$D^{2-ad}$),
Exemplification 4-965: Z-$L^{3-g}$-N(-$L^{1-a}$-$D^{1-a}$)(-$L^{2-a}$-$D^{2-ae}$),
Exemplification 4-966: Z-$L^{3-g}$-N(-$L^{1-a}$-$D^{1-a}$)(-$L^{2-a}$-$D^{2-af}$),
Exemplification 4-967: Z-$L^{3-g}$-N(-$L^{1-a}$-$D^{1-a}$)(-$L^{2-a}$-$D^{2-ag}$),
Exemplification 4-968: Z-$L^{3-g}$-N(-$L^{1-a}$-$D^{1-a}$)(-$L^{2-a}$-$D^{2-ah}$),
Exemplification 4-969: Z-$L^{3-g}$-N(-$L^{1-a}$-$D^{1-a}$)(-$L^{2-a}$-$D^{2-ai}$),
Exemplification 4-970: Z-$L^{3-g}$-N(-$L^{1-a}$-$D^{1-a}$)(-$L^{2-a}$-$D^{2-aj}$),
Exemplification 4-971: Z-$L^{3-g}$-N(-$L^{1-a}$-$D^{1-a}$)(-$L^{2-a}$-$D^{2-ak}$),
Exemplification 4-972: Z-$L^{3-g}$-N(-$L^{1-a}$-$D^{1-a}$)(-$L^{2-a}$-$D^{2-al}$),
Exemplification 4-973: Z-$L^{3-g}$-N(-$L^{1-a}$-$D^{1-a}$)(-$L^{2-a}$-$D^{2-am}$),
Exemplification 4-974: Z-$L^{3-g}$-N(-$L^{1-a}$-$D^{1-a}$)(-$L^{2-a}$-$D^{2-an}$),
Exemplification 4-975: Z-$L^{3-g}$-N(-$L^{1-a}$-$D^{1-a}$)(-$L^{2-a}$-$D^{2-ao}$),
Exemplification 4-976: Z-$L^{3-g}$-N(-$L^{1-a}$-$D^{1-a}$)(-$L^{2-a}$-$D^{2-ap}$),
Exemplification 4-977: Z-$L^{3-g}$-N(-$L^{1-a}$-$D^{1-a}$)(-$L^{2-b}$-$D^{2-aq}$),
Exemplification 4-978: Z-$L^{3-g}$-N(-$L^{1-a}$-$D^{1-a}$)(-$L^{2-b}$-$D^{2-ar}$),
Exemplification 4-979: Z-$L^{3-g}$-N(-$L^{1-a}$-$D^{1-a}$)(-$L^{2-b}$-$D^{2-as}$),
Exemplification 4-980: Z-$L^{3-g}$-N(-$L^{1-a}$-$D^{1-a}$)(-$L^{2-b}$-$D^{2-at}$),
Exemplification 4-981: Z-$L^{3-g}$-N(-$L^{1-a}$-$D^{1-a}$)(-$L^{2-c}$-$D^{2-aa}$),
Exemplification 4-982: Z-$L^{3-g}$-N(-$L^{1-a}$-$D^{1-a}$)(-$L^{2-c}$-$D^{2-ab}$),
Exemplification 4-983: Z-$L^{3-g}$-N(-$L^{1-a}$-$D^{1-a}$)(-$L^{2-c}$-$D^{2-ac}$),
Exemplification 4-984: Z-$L^{3-g}$-N(-$L^{1-a}$-$D^{1-a}$)(-$L^{2-c}$-$D^{2-ad}$),
Exemplification 4-985: Z-$L^{3-g}$-N(-$L^{1-a}$-$D^{1-a}$)(-$L^{2-c}$-$D^{2-ae}$),
Exemplification 4-986: Z-$L^{3-g}$-N(-$L^{1-a}$-$D^{1-a}$)(-$L^{2-c}$-$D^{2-af}$),
Exemplification 4-987: Z-$L^{3-g}$-N(-$L^{1-a}$-$D^{1-a}$)(-$L^{2-c}$-$D^{2-ag}$),
Exemplification 4-988: Z-$L^{3-g}$-N(-$L^{1-a}$-$D^{1-a}$)(-$L^{2-c}$-$D^{2-ah}$),
Exemplification 4-989: Z-$L^{3-g}$-N(-$L^{1-a}$-$D^{1-a}$)(-$L^{2-c}$-$D^{2-ai}$),
Exemplification 4-990: Z-$L^{3-g}$-N(-$L^{1-a}$-$D^{1-a}$)(-$L^{2-c}$-$D^{2-aj}$),
Exemplification 4-991: Z-$L^{3-g}$-N(-$L^{1-a}$-$D^{1-a}$)(-$L^{2-c}$-$D^{2-ak}$),
Exemplification 4-992: Z-$L^{3-g}$-N(-$L^{1-a}$-$D^{1-a}$)(-$L^{2-c}$-$D^{2-al}$),
Exemplification 4-993: Z-$L^{3-g}$-N(-$L^{1-a}$-$D^{1-a}$)(-$L^{2-c}$-$D^{2-am}$),
Exemplification 4-994: Z-$L^{3-g}$-N(-$L^{1-a}$-$D^{1-a}$)(-$L^{2-c}$-$D^{2-an}$),
Exemplification 4-995: Z-$L^{3-g}$-N(-$L^{1-a}$-$D^{1-a}$)(-$L^{2-c}$-$D^{2-ao}$),
Exemplification 4-996: Z-$L^{3-g}$-N(-$L^{1-a}$-$D^{1-a}$)(-$L^{2-c}$-$D^{2-ap}$),
Exemplification 4-997: Z-$L^{3-g}$-N(-$L^{1-a}$-$D^{1-a}$)(-$L^{2-d}$-$D^{2-aq}$),
Exemplification 4-998: Z-$L^{3-g}$-N(-$L^{1-a}$-$D^{1-a}$)(-$L^{2-d}$-$D^{2-ar}$),
Exemplification 4-999: Z-$L^{3-g}$-N(-$L^{1-a}$-$D^{1-a}$)(-$L^{2-d}$-$D^{2-as}$),
Exemplification 4-1000: Z-$L^{3-g}$-N(-$L^{1-a}$-$D^{1-a}$)(-$L^{2-d}$-$D^{2-at}$),
Exemplification 4-1001: Z-$L^{3-g}$-N(-$L^{1-b}$-$D^{1-a}$)(-$L^{2-a}$-$D^{2-aa}$),
Exemplification 4-1002: Z-$L^{3-g}$-N(-$L^{1-b}$-$D^{1-a}$)(-$L^{2-a}$-$D^{2-ab}$),
Exemplification 4-1003: Z-$L^{3-g}$-N(-$L^{1-b}$-$D^{1-a}$)(-$L^{2-a}$-$D^{2-ac}$),
Exemplification 4-1004: Z-$L^{3-g}$-N(-$L^{1-b}$-$D^{1-a}$)(-$L^{2-a}$-$D^{2-ad}$),
Exemplification 4-1005: Z-$L^{3-g}$-N(-$L^{1-b}$-$D^{1-a}$)(-$L^{2-a}$-$D^{2-ae}$),
Exemplification 4-1006: Z-$L^{3-g}$-N(-$L^{1-b}$-$D^{1-a}$)(-$L^{2-a}$-$D^{2-af}$),
Exemplification 4-1007: Z-$L^{3-g}$-N(-$L^{1-b}$-$D^{1-a}$)(-$L^{2-a}$-$D^{2-ag}$),
Exemplification 4-1008: Z-$L^{3-g}$-N(-$L^{1-b}$-$D^{1-a}$)(-$L^{2-a}$-$D^{2-ah}$),
Exemplification 4-1009: Z-$L^{3-g}$-N(-$L^{1-b}$-$D^{1-a}$)(-$L^{2-a}$-$D^{2-ai}$),
Exemplification 4-1010: Z-$L^{3-g}$-N(-$L^{1-b}$-$D^{1-a}$)(-$L^{2-a}$-$D^{2-aj}$),
Exemplification 4-1011: Z-$L^{3-g}$-N(-$L^{1-b}$-$D^{1-a}$)(-$L^{2-a}$-$D^{2-ak}$),

Exemplification 4-1012: $Z-L^{3-g}-N(-L^{1-b}-D^{1-a})(-L^{2-a}-D^{2-al})$,

Exemplification 4-1013: $Z-L^{3-g}-N(-L^{1-b}-D^{1-a})(-L^{2-a}-D^{2-am})$,

Exemplification 4-1014: $Z-L^{3-g}-N(-L^{1-b}-D^{1-a})(-L^{2-a}-D^{2-an})$,

Exemplification 4-1015: $Z-L^{3-g}-N(-L^{1-b}-D^{1-a})(-L^{2-a}-D^{2-ao})$,

Exemplification 4-1016: $Z-L^{3-g}-N(-L^{1-b}-D^{1-a})(-L^{2-a}-D^{2-ap})$,

Exemplification 4-1017: $Z-L^{3-g}-N(-L^{1-b}-D^{1-a})(-L^{2-b}-D^{2-aq})$,

Exemplification 4-1018: $Z-L^{3-g}-N(-L^{1-b}-D^{1-a})(-L^{2-b}-D^{2-ar})$,

Exemplification 4-1019: $Z-L^{3-g}-N(-L^{1-b}-D^{1-a})(-L^{2-b}-D^{2-as})$,

Exemplification 4-1020: $Z-L^{3-g}-N(-L^{1-b}-D^{1-a})(-L^{2-b}-D^{2-at})$,

Exemplification 4-1021: $Z-L^{3-g}-N(-L^{1-b}-D^{1-a})(-L^{2-c}-D^{2-aa})$,

Exemplification 4-1022: $Z-L^{3-g}-N(-L^{1-b}-D^{1-a})(-L^{2-c}-D^{2-ab})$,

Exemplification 4-1023: $Z-L^{3-g}-N(-L^{1-b}-D^{1-a})(-L^{2-c}-D^{2-ac})$,

Exemplification 4-1024: $Z-L^{3-g}-N(-L^{1-b}-D^{1-a})(-L^{2-c}-D^{2-ad})$,

Exemplification 4-1025: $Z-L^{3-g}-N(-L^{1-b}-D^{1-a})(-L^{2-c}-D^{2-ae})$,

Exemplification 4-1026: $Z-L^{3-g}-N(-L^{1-b}-D^{1-a})(-L^{2-c}-D^{2-af})$,

Exemplification 4-1027: $Z-L^{3-g}-N(-L^{1-b}-D^{1-a})(-L^{2-c}-D^{2-ag})$,

Exemplification 4-1028: $Z-L^{3-g}-N(-L^{1-b}-D^{1-a})(-L^{2-c}-D^{2-ah})$,

Exemplification 4-1029: $Z-L^{3-g}-N(-L^{1-b}-D^{1-a})(-L^{2-c}-D^{2-ai})$,

Exemplification 4-1030: $Z-L^{3-g}-N(-L^{1-b}-D^{1-a})(-L^{2-c}-D^{2-al})$,

Exemplification 4-1031: $Z-L^{3-g}-N(-L^{1-b}-D^{1-a})(-L^{2-c}-D^{2-ak})$,

Exemplification 4-1032: $Z-L^{3-g}-N(-L^{1-b}-D^{1-a})(-L^{2-c}-D^{2-al})$,

Exemplification 4-1033: $Z-L^{3-g}-N(-L^{1-b}-D^{1-a})(-L^{2-c}-D^{2-am})$,

Exemplification 4-1034 : $Z-L^{3-g}-N(-L^{1-b}-D^{1-a})(-L^{2-c}-D^{2-an})$,

Exemplification 4-1035: $Z-L^{3-g}-N(-L^{1-b}-D^{1-a})(-L^{2-c}-D^{2-ao})$,

Exemplification 4-1036: $Z-L^{3-g}-N(-L^{1-b}-D^{1-a})(-L^{2-c}-D^{2-ap})$,

Exemplification 4-1037 : $Z-L^{3-g}-N(-L^{1-b}-D^{1-a})(-L^{2-d}-D^{2-aq})$,

Exemplification 4-1038 : $Z-L^{3-g}-N(-L^{1-b}-D^{1-a})(-L^{2-d}-D^{2-ar})$,

Exemplification 4-1039: $Z-L^{3-g}-N(-L^{1-b}-D^{1-a})(-L^{2-d}-D^{2-as})$,

Exemplification 4-1040: $Z-L^{3-g}-N(-L^{1-b}-D^{1-a})(-L^{2-d}-D^{2-at})$,

Exemplification 4-1041: $Z-L^{3-g}-N(-L^{1-a}-D^{1-b})(-L^{2-a}-D^{2-aa})$,

Exemplification 4-1042 : $Z-L^{3-g}-N(-L^{1-a}-D^{1-b})(-L^{2-a}-D^{2-ab})$,

Exemplification 4-1043: $Z-L^{3-g}-N(-L^{1-a}-D^{1-b})(-L^{2-a}-D^{2-ac})$,

Exemplification 4-1044: $Z-L^{3-g}-N(-L^{1-a}-D^{1-b})(-L^{2-a}-D^{2-ad})$,

Exemplification 4-1045: $Z-L^{3-g}-N(-L^{1-a}-D^{1-b})(-L^{2-a}-D^{2-ae})$,

Exemplification 4-1046: $Z-L^{3-g}-N(-L^{1-a}-D^{1-b})(-L^{2-a}-D^{2-af})$,

Exemplification 4-1047 : $Z-L^{3-g}-N(-L^{1-a}-D^{1-b})(-L^{2-a}-D^{2-ag})$,

Exemplification 4-1048: $Z-L^{3-g}-N(-L^{1-a}-D^{1-b})(-L^{2-a}-D^{2-ah})$,

Exemplification 4-1049: $Z-L^{3-g}-N(-L^{1-a}-D^{1-b})(-L^{2-a}-D^{2-ai})$,

Exemplification 4-1050: $Z-L^{3-g}-N(-L^{1-a}-D^{1-b})(-L^{2-a}-D^{2-aj})$,

Exemplification 4-1051: $Z-L^{3-g}-N(-L^{1-a}-D^{1-b})(-L^{2-a}-D^{2-ak})$,

Exemplification 4-1052: $Z-L^{3-g}-N(-L^{1-a}-D^{1-b})(-L^{2-a}-D^{2-al})$,

Exemplification 4-1053: $Z-L^{3-g}-N(-L^{1-a}-D^{1-b})(-L^{2-a}-D^{2-am})$,

Exemplification 4-1054 : $Z-L^{3-g}-N(-L^{1-a}-D^{1-b})(-L^{2-a}-D^{2-an})$,

Exemplification 4-1055: $Z-L^{3-g}-N(-L^{1-a}-D^{1-b})(-L^{2-a}-D^{2-ao})$,

Exemplification 4-1056: $Z-L^{3-g}-N(-L^{1-a}-D^{1-b})(-L^{2-a}-D^{2-ap})$,

Exemplification 4-1057: $Z-L^{3-g}-N(-L^{1-a}-D^{1-b})(-L^{2-b}-D^{2-aq})$,

Exemplification 4-1058: $Z-L^{3-g}-N(-L^{1-a}-D^{1-b})(-L^{2-b}-D^{2-ar})$,

Exemplification 4-1059: $Z-L^{3-g}-N(-L^{1-a}-D^{1-b})(-L^{2-b}-D^{2-as})$,

Exemplification 4-1060: $Z-L^{3-g}-N(-L^{1-a}-D^{1-b})(-L^{2-b}-D^{2-at})$,

Exemplification 4-1061: $Z-L^{3-g}-N(-L^{1-a}-D^{1-b})(-L^{2-c}-D^{2-aa})$,

Exemplification 4-1062: $Z-L^{3-g}-N(-L^{1-a}-D^{1-b})(-L^{2-c}-D^{2-ab})$,

Exemplification 4-1063: $Z-L^{3-g}-N(-L^{1-a}-D^{1-b})(-L^{2-c}-D^{2-ac})$,

Exemplification 4-1064: $Z-L^{3-g}-N(-L^{1-a}-D^{1-b})(-L^{2-c}-D^{2-ad})$,

Exemplification 4-1065: $Z-L^{3-g}-N(-L^{1-a}-D^{1-b})(-L^{2-c}-D^{2-ae})$,

Exemplification 4-1066: $Z-L^{3-g}-N(-L^{1-a}-D^{1-b})(-L^{2-c}-D^{2-af})$,

Exemplification 4-1067: $Z-L^{3-g}-N(-L^{1-a}-D^{1-b})(-L^{2-c}-D^{2-ag})$,

Exemplification 4-1068: $Z-L^{3-g}-N(-L^{1-a}-D^{1-b})(-L^{2-c}-D^{2-ah})$,

Exemplification 4-1069: $Z-L^{3-g}-N(-L^{1-a}-D^{1-b})(-L^{2-c}-D^{2-ai})$,

Exemplification 4-1070: $Z-L^{3-g}-N(-L^{1-a}-D^{1-b})(-L^{2-c}-D^{2-aj})$,
Exemplification 4-1071: $Z-L^{3-g}-N(-L^{1-a}-D^{1-b})(-L^{2-c}-D^{2-ak})$,
Exemplification 4-1072: $Z-L^{3-g}-N(-L^{1-a}-D^{1-b})(-L^{2-c}-D^{2-al})$,
Exemplification 4-1073: $Z-L^{3-g}-N(-L^{1-a}-D^{1-b})(-L^{2-c}-D^{2-am})$,
Exemplification 4-1074: $Z-L^{3-g}-N(-L^{1-a}-D^{1-b})(-L^{2-c}-D^{2-an})$,
Exemplification 4-1075: $Z-L^{3-g}-N(-L^{1-a}-D^{1-b})(-L^{2-c}-D^{2-ao})$,
Exemplification 4-1076: $Z-L^{3-g}-N(-L^{1-a}-D^{1-b})(-L^{2-c}-D^{2-ap})$,
Exemplification 4-1077: $Z-L^{3-g}-N(-L^{1-a}-D^{1-b})(-L^{2-d}-D^{2-aq})$,
Exemplification 4-1078: $Z-L^{3-g}-N(-L^{1-a}-D^{1-b})(-L^{2-d}-D^{2-ar})$,
Exemplification 4-1079: $Z-L^{3-g}-N(-L^{1-a}-D^{1-b})(-L^{2-d}-D^{2-as})$,
Exemplification 4-1080: $Z-L^{3-g}-N(-L^{1-a}-D^{1-b})(-L^{2-d}-D^{2-at})$,
Exemplification 4-1081: $Z-L^{3-g}-N(-L^{1-b}-D^{1-b})(-L^{2-a}-D^{2-aa})$,
Exemplification 4-1082: $Z-L^{3-g}-N(-L^{1-b}-D^{1-b})(-L^{2-a}-D^{2-ab})$,
Exemplification 4-1083: $Z-L^{3-g}-N(-L^{1-b}-D^{1-b})(-L^{2-a}-D^{2-ac})$,
Exemplification 4-1084: $Z-L^{3-g}-N(-L^{1-b}-D^{1-b})(L^{2-a}-D^{2-ad})$,
Exemplification 4-1085: $Z-L^{3-g}-N(-L^{1-b}-D^{1-b})(-L^{2-a}-D^{2-ae})$,
Exemplification 4-1086: $Z-L^{3-g}-N(-L^{1-b}-D^{1-b})(-L^{2-a}-D^{2-af})$,
Exemplification 4-1087: $Z-L^{3-g}-N(-L^{1-b}-D^{1-b})(-L^{2-a}-D^{2-ag})$,
Exemplification 4-1088: $Z-L^{3-g}-N(-L^{1-b}-D^{1-b})(-L^{2-a}-D^{2-ah})$,
Exemplification 4-1089: $Z-L^{3-g}-N(-L^{1-b}-D^{1-b})(-L^{2-a}-D^{2-ai})$,
Exemplification 4-1090: $Z-L^{3-g}-N(-L^{1-b}-D^{1-b})(-L^{2-a}-D^{2-aj})$,
Exemplification 4-1091: $Z-L^{3-g}-N(-L^{1-b}-D^{1-b})(L^{2-a}-D^{2-ak})$,
Exemplification 4-1092: $Z-L^{3-g}-N(-L^{1-b}-D^{1-b})(-L^{2-a}-D^{2-al})$,
Exemplification 4-1093: $Z-L^{3-g}-N(-L^{1-b}-D^{1-b})(-L^{2-a}-D^{2-am})$,
Exemplification 4-1094: $Z-L^{3-g}-N(-L^{1-b}-D^{1-b})(-L^{2-a}-D^{2-an})$,
Exemplification 4-1095: $Z-L^{3-g}-N(-L^{1-b}-D^{1-b})(-L^{2-a}-D^{2-ao})$,
Exemplification 4-1096: $Z-L^{3-g}-N(-L^{1-b}-D^{1-b})(-L^{2-a}-D^{2-ap})$,
Exemplification 4-1097: $Z-L^{3-g}-N(-L^{1-b}-D^{1-b})(-L^{2-b}-D^{2-aq})$,
Exemplification 4-1098: $Z-L^{3-g}-N(-L^{1-b}-D^{1-b})(-L^{2-b}-D^{2-ar})$,
Exemplification 4-1099: $Z-L^{3-g}-N(-L^{1-b}-D^{1-b})(-L^{2-b}-D^{2-as})$,
Exemplification 4-1100: $Z-L^{3-g}-N(-L^{1-b}-D^{1-b})(-L^{2-b}-D^{2-at})$,
Exemplification 4-1101: $Z-L^{3-g}-N(-L^{1-b}-D^{1-b})(-L^{2-c}-D^{2-aa})$,
Exemplification 4-1102: $Z-L^{3-g}-N(-L^{1-b}-D^{1-b})(-L^{2-c}-D^{2-ab})$,
Exemplification 4-1103: $Z-L^{3-g}-N(-L^{1-b}-D^{1-b})(-L^{2-c}-D^{2-ac})$,
Exemplification 4-1104: $Z-L^{3-g}-N(-L^{1-b}-D^{1-b})(-L^{2-c}-D^{2-ad})$,
Exemplification 4-1105: $Z-L^{3-g}-N(-L^{1-b}-D^{1-b})(-L^{2-c}-D^{2-ae})$,
Exemplification 4-1106: $Z-L^{3-g}-N(-L^{1-b}-D^{1-b})(-L^{2-c}-D^{2-af})$,
Exemplification 4-1107: $Z-L^{3-g}-N(-L^{1-b}-D^{1-b})(-L^{2-c}-D^{2-ag})$,
Exemplification 4-1108: $Z-L^{3-g}-N(-L^{1-b}-D^{1-b})(-L^{2-c}-D^{2-ah})$,
Exemplification 4-1109: $Z-L^{3-g}-N(-L^{1-b}-D^{1-b})(-L^{2-c}-D^{2-ai})$,
Exemplification 4-1110: $Z-L^{3-g}-N(-L^{1-b}-D^{1-b})(-L^{2-c}-D^{2-al})$,
Exemplification 4-1111: $Z-L^{3-g}-N(-L^{1-b}-D^{1-b})(L^{2-c}-D^{2-ak})$,
Exemplification 4-1112: $Z-L^{3-g}-N(-L^{1-b}-D^{1-b})(-L^{2-c}-D^{2-al})$,
Exemplification 4-1113: $Z-L^{3-g}-N(-L^{1-b}-D^{1-b})(-L^{2-c}-D^{2-am})$,
Exemplification 4-1114: $Z-L^{3-g}-N(-L^{1-b}-D^{1-b})(-L^{2-c}-D^{2-an})$,
Exemplification 4-1115: $Z-L^{3-g}-N(-L^{1-b}-D^{1-b})(-L^{2-c}-D^{2-ao})$,
Exemplification 4-1116: $Z-L^{3-g}-N(-L^{1-b}-D^{1-b})(-L^{2-c}-D^{2-ap})$,
Exemplification 4-1117: $Z-L^{3-g}-N(-L^{1-b}-D^{1-b})(-L^{2-d}-D^{2-aq})$,
Exemplification 4-1118: $Z-L^{3-g}-N(-L^{1-b}-D^{1-b})(-L^{2-d}-D^{2-ar})$,
Exemplification 4-1119: $Z-L^{3-g}-N(-L^{1-b}-D^{1-b})(-L^{2-d}-D^{2-as})$,
Exemplification 4-1120: $Z-L^{3-g}-N(-L^{1-b}-D^{1-b})(-L^{2-d}-D^{2-at})$,
Exemplification 4-1121: $Z-L^{3-h}-N(-L^{1-a}-D^{1-a})(-L^{2-a}-D^{2-aa})$,
Exemplification 4-1122: $Z-L^{3-h}-N(-L^{1-a}-D^{1-a})(-L^{2-a}-D^{2-ab})$,
Exemplification 4-1123: $Z-L^{3-h}-N(-L^{1-a}-D^{1-a})(-L^{2-a}-D^{2-ac})$,
Exemplification 4-1124: $Z-L^{3-h}-N(-L^{1-a}-D^{1-a})(-L^{2-a}-D^{2-ad})$,
Exemplification 4-1125: $Z-L^{3-h}-N(-L^{1-a}-D^{1-a})(-L^{2-a}-D^{2-ae})$,
Exemplification 4-1126: $Z-L^{3-h}-N(-L^{1-a}-D^{1-a})(-L^{2-a}-D^{2-af})$,
Exemplification 4-1127: $Z-L^{3-h}-N(-L^{1-a}-D^{1-a})(-L^{2-a}-D^{2-ag})$,

Exemplification 4-1128: Z-L$^{3-h}$-N(-L$^{1-a}$-D$^{1-a}$)(-L$^{2-a}$-D$^{2-ah}$),
Exemplification 4-1129: Z-L$^{3-h}$-N(-L$^{1-a}$-D$^{1-a}$)(-L$^{2-a}$-D$^{2-ai}$),
Exemplification 4-1130: Z-L$^{3-h}$-N(-L$^{1-a}$-D$^{1-a}$)(-L$^{2-a}$-D$^{2-aj}$),
Exemplification 4-1131: Z-L$^{3-h}$-N(-L$^{1-a}$-D$^{1-a}$)(-L$^{2-a}$-D$^{2-ak}$),
Exemplification 4-1132: Z-L$^{3-h}$-N(-L$^{1-a}$-D$^{1-a}$)(-L$^{2-a}$-D$^{2-al}$),
Exemplification 4-1133: Z-L$^{3-h}$-N(-L$^{1-a}$-D$^{1-a}$)(-L$^{2-a}$-D$^{2-am}$),
Exemplification 4-1134: Z-L$^{3-h}$-N(-L$^{1-a}$-D$^{1-a}$)(-L$^{2-a}$-D$^{2-an}$),
Exemplification 4-1135: Z-L$^{3-h}$-N(-L$^{1-a}$-D$^{1-a}$)(-L$^{2-a}$-D$^{2-ao}$),
Exemplification 4-1136: Z-L$^{3-h}$-N(-L$^{1-a}$-D$^{1-a}$)(-L$^{2-a}$-D$^{2-ap}$),
Exemplification 4-1137: Z-L$^{3-h}$-N(-L$^{1-a}$-D$^{1-a}$)(-L$^{2-b}$-D$^{2-aq}$),
Exemplification 4-1138: Z-L$^{3-h}$-N(-L$^{1-a}$-D$^{1-a}$)(-L$^{2-b}$-D$^{2-ar}$),
Exemplification 4-1139: Z-L$^{3-h}$-N(-L$^{1-a}$-D$^{1-a}$)(-L$^{2-b}$-D$^{2-as}$),
Exemplification 4-1140: Z-L$^{3-h}$-N(-L$^{1-a}$-D$^{1-a}$)(-L$^{2}$b-D$^{2-at}$),
Exemplification 4-1141: Z-L$^{3-h}$-N(-L$^{1-a}$-D$^{1-a}$)(-L$^{2-c}$-D$^{2-aa}$),
Exemplification 4-1142: Z-L$^{3-h}$-N(-L$^{1-a}$-D$^{1-a}$)(-L$^{2-c}$-D$^{2-ab}$),
Exemplification 4-1143: Z-L$^{3-h}$-N(-L$^{1-a}$-D$^{1-a}$)(-L$^{2-c}$-D$^{2-ac}$),
Exemplification 4-1144: Z-L$^{3-h}$-N(-L$^{1-a}$-D$^{1-a}$)(-L$^{2-c}$-D$^{2-ad}$),
Exemplification 4-1145: Z-L$^{3-h}$-N(-L$^{1-a}$-D$^{1-a}$)(-L$^{2-c}$-D$^{2-ae}$),
Exemplification 4-1146: Z-L$^{3-h}$-N(-L$^{1-a}$-D$^{1-a}$)(-L$^{2-c}$-D$^{2-af}$),
Exemplification 4-1147: Z-L$^{3-h}$-N(-L$^{1-a}$-D$^{1-a}$)(-L$^{2-c}$-D$^{2-ag}$),
Exemplification 4-1148: Z-L$^{3-h}$-N(-L$^{1-a}$-D$^{1-a}$)(-L$^{2-c}$-D$^{2-ah}$),
Exemplification 4-1149: Z-L$^{3-h}$-N(-L$^{1-a}$-D$^{1-a}$)(-L$^{2-c}$-D$^{2-ai}$),
Exemplification 4-1150: Z-L$^{3-h}$-N(-L$^{1-a}$-D$^{1-a}$)(-L$^{2-c}$-D$^{2-ai}$),
Exemplification 4-1151: Z-L$^{3-h}$-N(-L$^{1-a}$-D$^{1-a}$)(-L$^{2-c}$-D$^{2-ak}$),
Exemplification 4-1152: Z-L$^{3-h}$-N(-L$^{1-a}$-D$^{1-a}$)(-L$^{2-c}$-D$^{2-al}$),
Exemplification 4-1153: Z-L$^{3-h}$-N(-L$^{1-a}$-D$^{1-a}$)(-L$^{2-c}$-D$^{2-am}$),
Exemplification 4-1154: Z-L$^{3-h}$-N(-L$^{1-a}$-D$^{1-a}$)(-L$^{2-c}$-D$^{2-an}$),
Exemplification 4-1155: Z-L$^{3-h}$-N(-L$^{1-a}$-D$^{1-a}$)(-L$^{2-c}$-D$^{2-ao}$),
Exemplification 4-1156: Z-L$^{3-h}$-N(-L$^{1-a}$-D$^{1-a}$)(-L$^{2-c}$-D$^{2-ap}$),
Exemplification 4-1157: Z-L$^{3-h}$-N(-L$^{1-a}$-D$^{1-a}$)(-L$^{2-d}$-D$^{2-aq}$),
Exemplification 4-1158: Z-L$^{3-h}$-N(-L$^{1-a}$-D$^{1-a}$)(-L$^{2-d}$-D$^{2-ar}$),
Exemplification 4-1159: Z-L$^{3-h}$-N(-L$^{1-a}$-D$^{1-a}$)(-L$^{2-d}$-D$^{2-as}$),
Exemplification 4-1160: Z-L$^{3-h}$-N(-L$^{1-a}$-D$^{1-a}$)(-L$^{2-d}$-D$^{2-at}$),
Exemplification 4-1161: Z-L$^{3-h}$-N(-L$^{1-b}$-D$^{1-a}$)(-L$^{2-a}$-D$^{2-aa}$),
Exemplification 4-1162: Z-L$^{3-h}$-N(-L$^{1-b}$-D$^{1-a}$)(-L$^{2-a}$-D$^{2-ab}$),
Exemplification 4-1163: Z-L$^{3-h}$-N(-L$^{1-b}$-D$^{1-a}$)(-L$^{2-a}$-D$^{2-ac}$),
Exemplification 4-1164: Z-L$^{3-h}$-N(-L$^{1-b}$-D$^{1-a}$)(-L$^{2-a}$-D$^{2-ad}$),
Exemplification 4-1165: Z-L$^{3-h}$-N(-L$^{1-b}$-D$^{1-a}$)(-L$^{2-a}$-D$^{2-ae}$),
Exemplification 4-1166: Z-L$^{3-h}$-N(-L$^{1-b}$-D$^{1-a}$)(-L$^{2-a}$-D$^{2-af}$),
Exemplification 4-1167: Z-L$^{3-h}$-N(-L$^{1-b}$-D$^{1-a}$)(-L$^{2-a}$-D$^{2-ag}$),
Exemplification 4-1168: Z-L$^{3-h}$-N(-L$^{1-b}$-D$^{1-a}$)(-L$^{2-a}$-D$^{2-ah}$),
Exemplification 4-1169: Z-L$^{3-h}$-N(-L$^{1-b}$-D$^{1-a}$)(-L$^{2-a}$-D$^{2-ai}$),
Exemplification 4-1170: Z-L$^{3-h}$-N(-L$^{1-b}$-D$^{1-a}$)(-L$^{2-a}$-D$^{2-aj}$),
Exemplification 4-1171: Z-L$^{3-h}$-N(-L$^{1-b}$-D$^{1-a}$)(-L$^{2-a}$-D$^{2-ak}$),
Exemplification 4-1172: Z-L$^{3-h}$-N(-L$^{1-b}$-D$^{1-a}$)(-L$^{2-a}$-D$^{2-al}$),
Exemplification 4-1173: Z-L$^{3-h}$-N(-L$^{1-b}$-D$^{1-a}$)(-L$^{2-a}$-D$^{2-am}$),
Exemplification 4-1174: Z-L$^{3-h}$-N(-L$^{1-b}$-D$^{1-a}$)(-L$^{2-a}$-D$^{2-an}$),
Exemplification 4-1175: Z-L$^{3-h}$-N(-L$^{1-b}$-D$^{1-a}$)(-L$^{2-a}$-D$^{2-ao}$),
Exemplification 4-1176: Z-L$^{3-h}$-N(-L$^{1-b}$-D$^{1-a}$)(-L$^{2-a}$-D$^{2-ap}$),
Exemplification 4-1177: Z-L$^{3-h}$-N(-L$^{1-b}$-D$^{1-a}$)(-L$^{2-b}$-D$^{2-aq}$),
Exemplification 4-1178: Z-L$^{3-h}$-N(-L$^{1-b}$-D$^{1-a}$)(-L$^{2-b}$-D$^{2-ar}$),
Exemplification 4-1179: Z-L$^{3-h}$-N(-L$^{1-b}$-D$^{1-a}$)(-L$^{2-b}$-D$^{2-as}$),
Exemplification 4-1180: Z-L$^{3-h}$-N(-L$^{1-b}$-D$^{1-a}$)(-L$^{2-b}$-D$^{2-at}$),
Exemplification 4-1181: Z-L$^{3-h}$-N(-L$^{1-b}$-D$^{1-a}$)(-L$^{2-c}$-D$^{2-aa}$),
Exemplification 4-1182: Z-L$^{3-h}$-N(-L$^{1-b}$-D$^{1-a}$)(-L$^{2-c}$-D$^{2-ab}$),
Exemplification 4-1183: Z-L$^{3-h}$-N(-L$^{1-b}$-D$^{1-a}$)(-L$^{2-c}$-D$^{2-ac}$),
Exemplification 4-1184: Z-L$^{3-h}$-N(-L$^{1-b}$-D$^{1-a}$)(-L$^{2-c}$-D$^{2-ad}$),
Exemplification 4-1185: Z-L$^{3-h}$-N(-L$^{1-b}$-D$^{1-a}$)(-L$^{2-c}$-D$^{2-ae}$),

Exemplification 4-1186: Z-$L^{3-h}$-N(-$L^{1-b}$-$D^{1-a}$)(-$L^{2-c}$-$D^{2-af}$),
Exemplification 4-1187: Z-$L^{3-h}$-N(-$L^{1-b}$-$D^{1-a}$)(-$L^{2-c}$-$D^{2-ag}$),
Exemplification 4-1188: Z-$L^{3-h}$-N(-$L^{1-b}$-$D^{1-a}$)(-$L^{2-c}$-$D^{2-ah}$),
Exemplification 4-1189: Z-$L^{3-h}$-N(-$L^{1-b}$-$D^{1-a}$)(-$L^{2-c}$-$D^{2-ai}$),
Exemplification 4-1190: Z-$L^{3-h}$-N(-$L^{1-b}$-$D^{1-a}$)(-$L^{2-c}$-$D^{2-aj}$),
Exemplification 4-1191: Z-$L^{3-h}$-N(-$L^{1-b}$-$D^{1-a}$)(-$L^{2-c}$-$D^{2-ak}$),
Exemplification 4-1192: Z-$L^{3-h}$-N(-$L^{1-b}$-$D^{1-a}$)(-$L^{2-c}$-$D^{2-al}$),
Exemplification 4-1193: Z-$L^{3-h}$-N(-$L^{1-b}$-$D^{1-a}$)(-$L^{2-c}$-$D^{2-am}$),
Exemplification 4-1194: Z-$L^{3-h}$-N(-$L^{1-b}$-$D^{1-a}$)(-$L^{2-c}$-$D^{2-an}$),
Exemplification 4-1195: Z-$L^{3-h}$-N(-$L^{1-b}$-$D^{1-a}$)(-$L^{2-c}$-$D^{2-ao}$),
Exemplification 4-1196: Z-$L^{3-h}$-N(-$L^{1-b}$-$D^{1-a}$)(-$L^{2-c}$-$D^{2-ap}$),
Exemplification 4-1197: Z-$L^{3-h}$-N(-$L^{1-b}$-$D^{1-a}$)(-$L^{2-d}$-$D^{2-aq}$),
Exemplification 4-1198: Z-$L^{3-h}$-N(-$L^{1-b}$-$D^{1-a}$)(-$L^{2-d}$-$D^{2-ar}$),
Exemplification 4-1199: Z-$L^{3-h}$-N(-$L^{1-b}$-$D^{1-a}$)(-$L^{2-d}$-$D^{2-as}$),
Exemplification 4-1200: Z-$L^{3-h}$-N(-$L^{1-b}$-$D^{1-a}$)(-$L^{2-d}$-$D^{2-at}$),
Exemplification 4-1201: Z-$L^{3-h}$-N (-$L^{1-a}$-$D^{1-b}$)(-$L^{2-a}$-$D^{2-aa}$),
Exemplification 4-1202: Z-$L^{3-h}$-N(-$L^{1-a}$-$D^{1-b}$)(-$L^{2-a}$-$D^{2-ab}$),
Exemplification 4-1203: Z-$L^{3-h}$-N(-$L^{1-a}$-$D^{1-b}$)(-$L^{2-a}$-$D^{2-ac}$),
Exemplification 4-1204: Z-$L^{3-h}$-N(-$L^{1-a}$-$D^{1-b}$)(-$L^{2-a}$-$D^{2-ad}$),
Exemplification 4-1205: Z-$L^{3-h}$-N(-$L^{1-a}$-$D^{1-b}$)(-$L^{2-a}$-$D^{2-ae}$),
Exemplification 4-1206: Z-$L^{3-h}$-N(-$L^{1-a}$-$D^{1-b}$)(-$L^{2-a}$-$D^{2-af}$),
Exemplification 4-1207: Z-$L^{3-h}$-N(-$L^{1-a}$-$D^{1-b}$)(-$L^{2-a}$-$D^{2-ag}$),
Exemplification 4-1208: Z-$L^{3-h}$-N(-$L^{1-a}$-$D^{1-b}$)(-$L^{2-a}$-$D^{2-ah}$),
Exemplification 4-1209: Z-$L^{3-h}$-N(-$L^{1-a}$-$D^{1-b}$)(-$L^{2-a}$-$D^{2-ai}$),
Exemplification 4-1210: Z-$L^{3-h}$-N(-$L^{1-a}$-$D^{1-b}$)(-$L^{2-a}$-$D^{2-aj}$),
Exemplification 4-1211: Z-$L^{3-h}$-N(-$L^{1-a}$-$D^{1-b}$)(-$L^{2-a}$-$D^{2-ak}$),
Exemplification 4-1212: Z-$L^{3-h}$-N(-$L^{1-a}$-$D^{1-b}$)(-$L^{2-a}$-$D^{2-al}$),
Exemplification 4-1213: Z-$L^{3-h}$-N(-$L^{1-a}$-$D^{1-b}$)(-$L^{2-a}$-$D^{2-am}$),
Exemplification 4-1214: Z-$L^{3-h}$-N(-$L^{1-a}$-$D^{1-b}$)(-$L^{2-a}$-$D^{2-an}$),
Exemplification 4-1215: Z-$L^{3-h}$-N(-$L^{1-a}$-$D^{1-b}$)(-$L^{2-a}$-$D^{2-ao}$),
Exemplification 4-1216: Z-$L^{3-h}$-N(-$L^{1-a}$-$D^{1-b}$)(-$L^{2-a}$-$D^{2-ap}$),
Exemplification 4-1217: Z-$L^{3-h}$-N(-$L^{1-a}$-$D^{1-b}$)(-$L^{2-b}$-$D^{2-aq}$),
Exemplification 4-1218: Z-$L^{3-h}$-N(-$L^{1-a}$-$D^{1-b}$)(-$L^{2-b}$-$D^{2-ar}$),
Exemplification 4-1219: Z-$L^{3-h}$-N(-$L^{1-a}$-$D^{1-b}$)(-$L^{2-b}$-$D^{2-as}$),
Exemplification 4-1220: Z-$L^{3-h}$-N(-$L^{1-a}$-$D^{1-b}$)(-$L^{2-b}$-$D^{2-at}$),
Exemplification 4-1221: Z-$L^{3-h}$-N(-$L^{1-a}$-$D^{1-b}$)(-$L^{2-c}$-$D^{2-aa}$),
Exemplification 4-1222: Z-$L^{3-h}$-N(-$L^{1-a}$-$D^{1-b}$)(-$L^{2-c}$-$D^{2-ab}$),
Exemplification 4-1223: Z-$L^{3-h}$-N(-$L^{1-a}$-$D^{1-b}$)(-$L^{2-c}$-$D^{2-ac}$),
Exemplification 4-1224: Z-$L^{3-h}$-N(-$L^{1-a}$-$D^{1-b}$)(-$L^{2-c}$-$D^{2-ad}$),
Exemplification 4-1225: Z-$L^{3-h}$-N(-$L^{1-a}$-$D^{1-b}$)(-$L^{2-c}$-$D^{2-ae}$),
Exemplification 4-1226: Z-$L^{3-h}$-N(-$L^{1-a}$-$D^{1-b}$)(-$L^{2-c}$-$D^{2-af}$),
Exemplification 4-1227: Z-$L^{3-h}$-N(-$L^{1-a}$-$D^{1-b}$)(-$L^{2-c}$-$D^{2-ag}$),
Exemplification 4-1228: Z-$L^{3-h}$-N(-$L^{1-a}$-$D^{1-b}$)(-$L^{2-c}$-$D^{2-ah}$),
Exemplification 4-1229: Z-$L^{3-h}$-N(-$L^{1-a}$-$D^{1-b}$)(-$L^{2-c}$-$D^{2-ai}$),
Exemplification 4-1230: Z-$L^{3-h}$-N(-$L^{1-a}$-$D^{1-b}$)(-$L^{2-c}$-$D^{2-aj}$),
Exemplification 4-1231: Z-$L^{3-h}$-N(-$L^{1-a}$-$D^{1-b}$)(-$L^{2-c}$-$D^{2-ak}$),
Exemplification 4-1232: Z-$L^{3-h}$-N(-$L^{1-a}$-$D^{1-b}$)(-$L^{2-c}$-$D^{2-al}$),
Exemplification 4-1233: Z-$L^{3-h}$-N(-$L^{1-a}$-$D^{1-b}$)(-$L^{2-c}$-$D^{2-am}$),
Exemplification 4-1234: Z-$L^{3-h}$-N(-$L^{1-a}$-$D^{1-b}$)(-$L^{2-c}$-$D^{2-an}$),
Exemplification 4-1235: Z-$L^{3-h}$-N(-$L^{1-a}$-$D^{1-b}$)(-$L^{2-c}$-$D^{2-ao}$),
Exemplification 4-1236: Z-$L^{3-h}$-N(-$L^{1-a}$-$D^{1-b}$)(-$L^{2-c}$-$D^{2-ap}$),
Exemplification 4-1237: Z-$L^{3-h}$-N(-$L^{1-a}$-$D^{1-b}$)(-$L^{2-d}$-$D^{2-aq}$),
Exemplification 4-1238: Z-$L^{3-h}$-N(-$L^{1-a}$-$D^{1-b}$)(-$L^{2-d}$-$D^{2-ar}$),
Exemplification 4-1239: Z-$L^{3-h}$-N(-$L^{1-a}$-$D^{1-b}$)(-$L^{2-d}$-$D^{2-as}$),
Exemplification 4-1240: Z-$L^{3-h}$-N(-$L^{1-a}$-$D^{1-b}$)(-$L^{2-d}$-$D^{2-at}$),
Exemplification 4-1241: Z-$L^{3-h}$-N(-$L^{1-b}$-$D^{1-b}$)(-$L^{2-a}$-$D^{2-aa}$),
Exemplification 4-1242: Z-$L^{3-h}$-N(-$L^{1-b}$-$D^{1-b}$)(-$L^{2-a}$-$D^{2-ab}$),
Exemplification 4-1243: Z-$L^{3-h}$-N(-$L^{1-b}$-$D^{1-b}$)(-$L^{2-a}$-$D^{2-ac}$),

Exemplification 4-1244: $Z-L^{3-h}-N(-L^{1-b}-D^{1-b})(-L^{2-a}-D^{2-ad})$,
Exemplification 4-1245: $Z-L^{3-h}-N(-L^{1-b}-D^{1-b})(-L^{2-a}-D^{2-ae})$,
Exemplification 4-1246: $Z-L^{3-h}-N(-L^{1-b}-D^{1-b})(-L^{2-a}-D^{2-af})$,
Exemplification 4-1247: $Z-L^{3-h}-N(-L^{1-b}-D^{1-b})(-L^{2-a}-D^{2-ag})$,
Exemplification 4-1248: $Z-L^{3-h}-N(-L^{1-b}-D^{1-b})(-L^{2-a}-D^{2-ah})$,
Exemplification 4-1249: $Z-L^{3-h}-N(-L^{1-b}-D^{1-b})(-L^{2-a}-D^{2-ai})$,
Exemplification 4-1250: $Z-L^{3-h}-N(-L^{1-b}-D^{1-b})(-L^{2-a}-D^{2-aj})$,
Exemplification 4-1251: $Z-L^{3-h}-N(-L^{1-b}-D^{1-b})(-L^{2-a}-D^{2-ak})$,
Exemplification 4-1252: $Z-L^{3-h}-N(-L^{1-b}-D^{1-b})(-L^{2-a}-D^{2-al})$,
Exemplification 4-1253: $Z-L^{3-h}-N(-L^{1-b}-D^{1-b})(-L^{2-a}-D^{2-am})$,
Exemplification 4-1254: $Z-L^{3-h}-N(-L^{1-b}-D^{1-b})(-L^{2-a}-D^{2-an})$,
Exemplification 4-1255: $Z-L^{3-h}-N(-L^{1-b}-D^{1-b})(-L^{2-a}-D^{2-ao})$,
Exemplification 4-1256: $Z-L^{3-h}-N(-L^{1-b}-D^{1-b})(-L^{2-a}-D^{2-ap})$,
Exemplification 4-1257: $Z-L^{3-h}-N(-L^{1-b}-D^{1-b})(-L^{2-b}-D^{2-aq})$,
Exemplification 4-1258: $Z-L^{3-h}-N(-L^{1-b}-D^{1-b})(-L^{2-b}-D^{2-ar})$,
Exemplification 4-1259: $Z-L^{3-h}-N(-L^{1-b}-D^{1-b})(-L^{2-b}-D^{2-as})$,
Exemplification 4-1260: $Z-L^{3-h}-N(-L^{1-b}-D^{1-b})(-L^{2-b}-D^{2-at})$,
Exemplification 4-1261: $Z-L^{3-h}-N(-L^{1-b}-D^{1-b})(-L^{2-c}-D^{2-aa})$,
Exemplification 4-1262: $Z-L^{3-h}-N(-L^{1-b}-D^{1-b})(-L^{2-c}-D^{2-ab})$,
Exemplification 4-1263: $Z-L^{3-h}-N(-L^{1-b}-D^{1-b})(-L^{2-c}-D^{2-ac})$,
Exemplification 4-1264: $Z-L^{3-h}-N(-L^{1-b}-D^{1-b})(-L^{2-c}-D^{2-ad})$,
Exemplification 4-1265: $Z-L^{3-h}-N(-L^{1-b}-D^{1-b})(-L^{2-c}-D^{2-ae})$,
Exemplification 4-1266: $Z-L^{3-h}-N(-L^{1-b}-D^{1-b})(-L^{2-c}-D^{2-af})$,
Exemplification 4-1267: $Z-L^{3-h}-N(-L^{1-b}-D^{1-b})(-L^{2-c}-D^{2-ag})$,
Exemplification 4-1268: $Z-L^{3-h}-N(-L^{1-b}-D^{1-b})(-L^{2-c}-D^{2-ah})$,
Exemplification 4-1269: $Z-L^{3-h}-N(-L^{1-b}-D^{1-b})(-L^{2-c}-D^{2-ai})$,
Exemplification 4-1270: $Z-L^{3-h}-N(-L^{1-b}-D^{1-b})(-L^{2-c}-D^{2-aj})$,
Exemplification 4-1271: $Z-L^{3-h}-N(-L^{1-b}-D^{1-b})(-L^{2-c}-D^{2-ak})$,
Exemplification 4-1272: $Z-L^{3-h}-N(-L^{1-b}-D^{1-b})(-L^{2-c}-D^{2-al})$,
Exemplification 4-1273: $Z-L^{3-h}-N(-L^{1-b}-D^{1-b})(-L^{2-c}-D^{2-am})$,
Exemplification 4-1274: $Z-L^{3-h}-N(-L^{1-b}-D^{1-b})(-L^{2-c}-D^{2-an})$,
Exemplification 4-1275: $Z-L^{3-h}-N(-L^{1-b}-D^{1-b})(-L^{2-c}-D^{2-ao})$,
Exemplification 4-1276: $Z-L^{3-h}-N(-L^{1-b}-D^{1-b})(-L^{2-c}-D^{2-ap})$,
Exemplification 4-1277: $Z-L^{3-h}-N(-L^{1-b}-D^{1-b})(-L^{2-d}-D^{2-aq})$,
Exemplification 4-1278: $Z-L^{3-h}-N(-L^{1-b}-D^{1-b})(-L^{2-d}-D^{2-ar})$,
Exemplification 4-1279: $Z-L^{3-h}-N(-L^{1-b}-D^{1-b})(-L^{2-d}-D^{2-as})$,
Exemplification 4-1280: $Z-L^{3-h}-N(-L^{1-b}-D^{1-b})(-L^{2-d}-D^{2-at})$,
Exemplification 4-1281: $Z-L^{3-i}-N(-L^{1-a}-D^{1-a})(-L^{2-a}-D^{2-aa})$,
Exemplification 4-1282: $Z-L^{3-i}-N(-L^{1-a}-D^{1-a})(-L^{2-a}-D^{2-ab})$,
Exemplification 4-1283: $Z-L^{3-i}-N(-L^{1-a}-D^{1-a})(-L^{2-a}-D^{2-ac})$,
Exemplification 4-1284: $Z-L^{3-i}-N(-L^{1-a}-D^{1-a})(-L^{2-a}-D^{2-ad})$,
Exemplification 4-1285: $Z-L^{3-i}-N(-L^{1-a}-D^{1-a})(-L^{2-a}-D^{2-ae})$,
Exemplification 4-1286: $Z-L^{3-i}-N(-L^{1-a}-D^{1-a})(-L^{2-a}-D^{2-af})$,
Exemplification 4-1287: $Z-L^{3-i}-N(-L^{1-a}-D^{1-a})(-L^{2-a}-D^{2-ag})$,
Exemplification 4-1288: $Z-L^{3-i}-N(-L^{1-a}-D^{1-a})(-L^{2-a}-D^{2-ah})$,
Exemplification 4-1289: $Z-L^{3-i}-N(-L^{1-a}-D^{1-a})(-L^{2-a}-D^{2-ai})$,
Exemplification 4-1290: $Z-L^{3-i}-N(-L^{1-a}-D^{1-a})(-L^{2-a}-D^{2-aj})$,
Exemplification 4-1291: $Z-L^{3-i}-N(-L^{1-a}-D^{1-a})(-L^{2-a}-D^{2-ak})$,
Exemplification 4-1292: $Z-L^{3-i}-N(-L^{1-a}-D^{1-a})(-L^{2-a}-D^{2-al})$,
Exemplification 4-1293: $Z-L^{3-i}-N(-L^{1-a}-D^{1-a})(-L^{2-a}-D^{2-am})$,
Exemplification 4-1294: $Z-L^{3-i}-N(-L^{1-a}-D^{1-a})(-L^{2-a}-D^{2-an})$,
Exemplification 4-1295: $Z-L^{3-i}-N(-L^{1-a}-D^{1-a})(-L^{2-a}-D^{2-ao})$,
Exemplification 4-1296: $Z-L^{3-i}-N(-L^{1-a}-D^{1-a})(-L^{2-a}-D^{2-ap})$,
Exemplification 4-1297: $Z-L^{3-i}-N(-L^{1-a}-D^{1-a})(-L^{2-b}-D^{2-aq})$,
Exemplification 4-1298: $Z-L^{3-i}-N(-L^{1-a}-D^{1-a})(-L^{2-b}-D^{2-ar})$,
Exemplification 4-1299: $Z-L^{3-i}-N(-L^{1-a}-D^{1-a})(-L^{2-b}-D^{2-as})$,
Exemplification 4-1300: $Z-L^{3-i}-N(-L^{1-a}-D^{1-a})(-L^{2-b}-D^{2-at})$,
Exemplification 4-1301: $Z-L^{3-i}-N(-L^{1-a}-D^{1-a})(-L^{2-c}-D^{2-aa})$,

Exemplification 4-1302: $Z-L^{3-i}-N(-L^{1-a}-D^{1-a})(-L^{2-c}-D^{2-ab})$,
Exemplification 4-1303: $Z-L^{3-i}-N(-L^{1-a}-D^{1-a})(-L^{2-c}-D^{2-ac})$,
Exemplification 4-1304: $Z-L^{3-i}-N(-L^{1-a}-D^{1-a})(-L^{2-c}-D^{2-ad})$,
Exemplification 4-1305: $Z-L^{3-i}-N(-L^{1-a}-D^{1-a})(-L^{2-c}-D^{2-ae})$,
Exemplification 4-1306: $Z-L^{3-i}-N(-L^{1-a}-D^{1-a})(-L^{2-c}-D^{2-af})$,
Exemplification 4-1307: $Z-L^{3-i}-N(-L^{1-a}-D^{1-a})(-L^{2-c}-D^{2-ag})$,
Exemplification 4-1308: $Z-L^{3-i}-N(-L^{1-a}-D^{1-a})(-L^{2-c}-D^{2-ah})$,
Exemplification 4-1309: $Z-L^{3-i}-N(-L^{1-a}-D^{1-a})(-L^{2-c}-D^{2-ai})$,
Exemplification 4-1310: $Z-L^{3-i}-N(-L^{1-a}-D^{1-a})(-L^{2-c}-D^{2-aj})$,
Exemplification 4-1311: $Z-L^{3-i}-N(-L^{1-a}-D^{1-a})(-L^{2-c}-D^{2-ak})$,
Exemplification 4-1312: $Z-L^{3-i}-N(-L^{1-a}-D^{1-a})(-L^{2-c}-D^{2-al})$,
Exemplification 4-1313: $Z-L^{3-i}-N(-L^{1-a}-D^{1-a})(-L^{2-c}-D^{2-am})$,
Exemplification 4-1314: $Z-L^{3-i}-N(-L^{1-a}-D^{1-a})(-L^{2-c}-D^{2-an})$,
Exemplification 4-1315: $Z-L^{3-i}-N(-L^{1-a}-D^{1-a})(-L^{2-c}-D^{2-ao})$,
Exemplification 4-1316: $Z-L^{3-i}-N(-L^{1-a}-D^{1-a})(-L^{2-c}-D^{2-ap})$,
Exemplification 4-1317: $Z-L^{3-i}-N(-L^{1-a}-D^{1-a})(-L^{2-d}-D^{2-aq})$,
Exemplification 4-1318: $Z-L^{3-i}-N(-L^{1-a}-D^{1-a})(-L^{2-d}-D^{2-ar})$,
Exemplification 4-1319: $Z-L^{3-i}-N(-L^{1-a}-D^{1-a})(-L^{2-d}-D^{2-as})$,
Exemplification 4-1320: $Z-L^{3-i}-N(-L^{1-a}-D^{1-a})(-L^{2-d}-D^{2-at})$,
Exemplification 4-1321: $Z-L^{3-i}-N(-L^{1-b}-D^{1-a})(-L^{2-a}-D^{2-aa})$,
Exemplification 4-1322: $Z-L^{3-i}-N(-L^{1-b}-D^{1-a})(-L^{2-a}-D^{2-ab})$,
Exemplification 4-1323: $Z-L^{3-i}-N(-L^{1-b}-D^{1-a})(-L^{2-a}-D^{2-ac})$,
Exemplification 4-1324: $Z-L^{3-i}-N(-L^{1-b}-D^{1-a})(-L^{2-a}-D^{2-ad})$,
Exemplification 4-1325: $Z-L^{3-i}-N(-L^{1-b}-D^{1-a})(-L^{2-a}-D^{2-ae})$,
Exemplification 4-1326: $Z-L^{3-i}-N(-L^{1-b}-D^{1-a})(-L^{2-a}-D^{2-af})$,
Exemplification 4-1327: $Z-L^{3-i}-N(-L^{1-b}-D^{1-a})(-L^{2-a}-D^{2-ag})$,
Exemplification 4-1328: $Z-L^{3-i}-N(-L^{1-b}-D^{1-a})(-L^{2-a}-D^{2-ah})$,
Exemplification 4-1329: $Z-L^{3-i}-N(-L^{1-b}-D^{1-a})(-L^{2-a}-D^{2-ai})$,
Exemplification 4-1330: $Z-L^{3-i}-N(-L^{1-b}-D^{1-a})(-L^{2-a}-D^{2-aj})$,
Exemplification 4-1331: $Z-L^{3-i}-N(-L^{1-b}-D^{1-a})(-L^{2-a}-D^{2-ak})$,
Exemplification 4-1332: $Z-L^{3-i}-N(-L^{1-b}-D^{1-a})(-L^{2-a}-D^{2-al})$,
Exemplification 4-1333: $Z-L^{3-i}-N(-L^{1-b}-D^{1-a})(-L^{2-a}-D^{2-am})$,
Exemplification 4-1334: $Z-L^{3-i}-N(-L^{1-b}-D^{1-a})(-L^{2-a}-D^{2-an})$,
Exemplification 4-1335: $Z-L^{3-i}-N(-L^{1-b}-D^{1-a})(-L^{2-a}-D^{2-ao})$,
Exemplification 4-1336: $Z-L^{3-i}-N(-L^{1-b}-D^{1-a})(-L^{2-a}-D^{2-ap})$,
Exemplification 4-1337: $Z-L^{3-i}-N(-L^{1-b}-D^{1-a})(-L^{2-b}-D^{2-aq})$,
Exemplification 4-1338: $Z-L^{3-i}-N(-L^{1-b}-D^{1-a})(-L^{2-b}-D^{2-ar})$,
Exemplification 4-1339: $Z-L^{3-i}-N(-L^{1-b}-D^{1-a})(-L^{2-b}-D^{2-as})$,
Exemplification 4-1340: $Z-L^{3-i}-N(-L^{1-b}-D^{1-a})(-L^{2-b}-D^{2-at})$,
Exemplification 4-1341: $Z-L^{3-i}-N(-L^{1-b}-D^{1-a})(-L^{2-c}-D^{2-aa})$,
Exemplification 4-1342: $Z-L^{3-i}-N(-L^{1-b}-D^{1-a})(-L^{2-c}-D^{2-ab})$,
Exemplification 4-1343: $Z-L^{3-i}-N(-L^{1-b}-D^{1-a})(-L^{2-c}-D^{2-ac})$,
Exemplification 4-1344: $Z-L^{3-i}-N(-L^{1-b}-D^{1-a})(-L^{2-c}-D^{2-ad})$,
Exemplification 4-1345: $Z-L^{3-i}-N(-L^{1-b}-D^{1-a})(-L^{2-c}-D^{2-ae})$,
Exemplification 4-1346: $Z-L^{3-i}-N(-L^{1-b}-D^{1-a})(-L^{2-c}-D^{2-af})$,
Exemplification 4-1347: $Z-L^{3-i}-N(-L^{1-b}-D^{1-a})(-L^{2-c}-D^{2-ag})$,
Exemplification 4-1348: $Z-L^{3-i}-N(-L^{1-b}-D^{1-a})(-L^{2-c}-D^{2-ah})$,
Exemplification 4-1349: $Z-L^{3-i}-N(-L^{1-b}-D^{1-a})(-L^{2-c}-D^{2-ai})$,
Exemplification 4-1350: $Z-L^{3-i}-N(-L^{1-b}-D^{1-a})(-L^{2-c}-D^{2-aj})$,
Exemplification 4-1351: $Z-L^{3-i}-N(-L^{1-b}-D^{1-a})(-L^{2-c}-D^{2-ak})$,
Exemplification 4-1352: $Z-L^{3-i}-N(-L^{1-b}-D^{1-a})(-L^{2-c}-D^{2-al})$,
Exemplification 4-1353: $Z-L^{3-i}-N(-L^{1-b}-D^{1-a})(-L^{2-c}-D^{2-am})$,
Exemplification 4-1354: $Z-L^{3-i}-N(-L^{1-b}-D^{1-a})(-L^{2-c}-D^{2-an})$,
Exemplification 4-1355: $Z-L^{3-i}-N(-L^{1-b}-D^{1-a})(-L^{2-c}-D^{2-ao})$,
Exemplification 4-1356: $Z-L^{3-i}-N(-L^{1-b}-D^{1-a})(-L^{2-c}-D^{2-ap})$,
Exemplification 4-1357: $Z-L^{3-i}-N(-L^{1-b}-D^{1-a})(-L^{2-d}-D^{2-aq})$,
Exemplification 4-1358: $Z-L^{3-i}-N(-L^{1-b}-D^{1-a})(-L^{2-d}-D^{2-ar})$,
Exemplification 4-1359: $Z-L^{3-i}-N(-L^{1-b}-D^{1-a})(-L^{2-d}-D^{2-as})$,

Exemplification 4-1360: Z-$L^{3-i}$-N(-$L^{1-b}$-$D^{1-a}$)(-$L^{2-d}$-$D^{2-at}$),
Exemplification 4-1361: Z-$L^{3-i}$-N(-$L^{1-a}$-$D^{1-b}$)(-$L^{2-a}$-$D^{2-aa}$),
Exemplification 4-1362: Z-$L^{3-i}$-N(-$L^{1-a}$-$D^{1-b}$)(-$L^{2-a}$-$D^{2-ab}$),
Exemplification 4-1363: Z-$L^{3-i}$-N(-$L^{1-a}$-$D^{1-b}$)(-$L^{2-a}$-$D^{2-ac}$),
Exemplification 4-1364: Z-$L^{3-i}$-N(-$L^{1-a}$-$D^{1-b}$)(-$L^{2-a}$-$D^{2-ad}$),
Exemplification 4-1365: Z-$L^{3-i}$-N(-$L^{1-a}$-$D^{1-b}$)(-$L^{2-a}$-$D^{2-ae}$),
Exemplification 4-1366: Z-$L^{3-i}$-N(-$L^{1-a}$-$D^{1-b}$)(-$L^{2-a}$-$D^{2-af}$),
Exemplification 4-1367: Z-$L^{3-i}$-N(-$L^{1-a}$-$D^{1-b}$)(-$L^{2-a}$-$D^{2-ag}$),
Exemplification 4-1368: Z-$L^{3-i}$-N(-$L^{1-a}$-$D^{1-b}$)(-$L^{2-a}$-$D^{2-ah}$),
Exemplification 4-1369: Z-$L^{3-i}$-N(-$L^{1-a}$-$D^{1-b}$)(-$L^{2-a}$-$D^{2-ai}$),
Exemplification 4-1370: Z-$L^{3-i}$-N(-$L^{1-a}$-$D^{1-b}$)(-$L^{2-a}$-$D^{2-aj}$),
Exemplification 4-1371: Z-$L^{3-i}$-N(-$L^{1-a}$-$D^{1-b}$)(-$L^{2-a}$-$D^{2-ak}$),
Exemplification 4-1372: Z-$L^{3-i}$-N(-$L^{1-a}$-$D^{1-b}$)(-$L^{2-a}$-$D^{2-al}$),
Exemplification 4-1373: Z-$L^{3-i}$-N(-$L^{1-a}$-$D^{1-b}$)(-$L^{2-a}$-$D^{2-am}$),
Exemplification 4-1374: Z-$L^{3-i}$-N(-$L^{1-a}$-$D^{1-b}$)(-$L^{2-a}$-$D^{2-an}$),
Exemplification 4-1375: Z-$L^{3-i}$-N(-$L^{1-a}$-$D^{1-b}$)(-$L^{2-a}$-$D^{2-ao}$),
Exemplification 4-1376: Z-$L^{3-i}$-N(-$L^{1-a}$-$D^{1-b}$)(-$L^{2-a}$-$D^{2-ap}$),
Exemplification 4-1377: Z-$L^{3-i}$-N(-$L^{1-a}$-$D^{1-b}$)(-$L^{2-b}$-$D^{2-aq}$),
Exemplification 4-1378: Z-$L^{3-i}$-N(-$L^{1-a}$-$D^{1-b}$)(-$L^{2-b}$-$D^{2-ar}$),
Exemplification 4-1379: Z-$L^{3-i}$-N(-$L^{1-a}$-$D^{1-b}$)(-$L^{2-b}$-$D^{2-as}$),
Exemplification 4-1380: Z-$L^{3-i}$-N(-$L^{1-a}$-$D^{1-b}$)(-$L^{2-b}$-$D^{2-at}$),
Exemplification 4-1381: Z-$L^{3-i}$-N(-$L^{1-a}$-$D^{1-b}$)(-$L^{2-c}$-$D^{2-aa}$),
Exemplification 4-1382: Z-$L^{3-i}$-N(-$L^{1-a}$-$D^{1-b}$)(-$L^{2-c}$-$D^{2-ab}$),
Exemplification 4-1383: Z-$L^{3-i}$-N(-$L^{1-a}$-$D^{1-b}$)(-$L^{2-c}$-$D^{2-ac}$),
Exemplification 4-1384: Z-$L^{3-i}$-N(-$L^{1-a}$-$D^{1-b}$)(-$L^{2-c}$-$D^{2-ad}$),
Exemplification 4-1385: Z-$L^{3-i}$-N(-$L^{1-a}$-$D^{1-b}$)(-$L^{2-c}$-$D^{2-ae}$),
Exemplification 4-1386: Z-$L^{3-i}$-N(-$L^{1-a}$-$D^{1-b}$)(-$L^{2-c}$-$D^{2-af}$),
Exemplification 4-1387: Z-$L^{3-i}$-N(-$L^{1-a}$-$D^{1-b}$)(-$L^{2-c}$-$D^{2-ag}$),
Exemplification 4-1388: Z-$L^{3-i}$-N(-$L^{1-a}$-$D^{1-b}$)(-$L^{2-c}$-$D^{2-ah}$),
Exemplification 4-1389: Z-$L^{3-i}$-N(-$L^{1-a}$-$D^{1-b}$)(-$L^{2-c}$-$D^{2-ai}$),
Exemplification 4-1390: Z-$L^{3-i}$-N(-$L^{1-a}$-$D^{1-b}$)(-$L^{2-c}$-$D^{2-aj}$),
Exemplification 4-1391: Z-$L^{3-i}$-N(-$L^{1-a}$-$D^{1-b}$)(-$L^{2-c}$-$D^{2-ak}$),
Exemplification 4-1392: Z-$L^{3-i}$-N(-$L^{1-a}$-$D^{1-b}$)(-$L^{2-c}$-$D^{2-al}$),
Exemplification 4-1393: Z-$L^{3-i}$-N(-$L^{1-a}$-$D^{1-b}$)(-$L^{2-c}$-$D^{2-am}$),
Exemplification 4-1394: Z-$L^{3-i}$-N(-$L^{1-a}$-$D^{1-b}$)(-$L^{2-c}$-$D^{2-an}$),
Exemplification 4-1395: Z-$L^{3-i}$-N(-$L^{1-a}$-$D^{1-b}$)(-$L^{2-c}$-$D^{2-ao}$),
Exemplification 4-1396: Z-$L^{3-i}$-N(-$L^{1-a}$-$D^{1-b}$)(-$L^{2-c}$-$D^{2-ap}$),
Exemplification 4-1397: Z-$L^{3-i}$-N(-$L^{1-a}$-$D^{1-b}$)(-$L^{2-d}$-$D^{2-aq}$),
Exemplification 4-1398: Z-$L^{3-i}$-N(-$L^{1-a}$-$D^{1-b}$)(-$L^{2-d}$-$D^{2-ar}$),
Exemplification 4-1399: Z-$L^{3-i}$-N(-$L^{1-a}$-$D^{1-b}$)(-$L^{2-d}$-$D^{2-as}$),
Exemplification 4-1400: Z-$L^{3-i}$-N(-$L^{1-a}$-$D^{1-b}$)(-$L^{2-d}$-$D^{2-at}$),
Exemplification 4-1401: Z-$L^{3-i}$-N(-$L^{1-b}$-$D^{1-b}$)(-$L^{2-a}$-$D^{2-aa}$),
Exemplification 4-1402: Z-$L^{3-i}$-N(-$L^{1-b}$-$D^{1-b}$)(-$L^{2-a}$-$D^{2-ab}$),
Exemplification 4-1403: Z-$L^{3-i}$-N(-$L^{1-b}$-$D^{1-b}$)(-$L^{2-a}$-$D^{2-ac}$),
Exemplification 4-1404: Z-$L^{3-i}$-N(-$L^{1-b}$-$D^{1-b}$)(-$L^{2-a}$-$D^{2-ad}$),
Exemplification 4-1405: Z-$L^{3-i}$-N(-$L^{1-b}$-$D^{1-b}$)(-$L^{2-a}$-$D^{2-ae}$),
Exemplification 4-1406: Z-$L^{3-i}$-N(-$L^{1-b}$-$D^{1-b}$)(-$L^{2-a}$-$D^{2-af}$),
Exemplification 4-1407: Z-$L^{3-i}$-N(-$L^{1-b}$-$D^{1-b}$)(-$L^{2-a}$-$D^{2-ag}$),
Exemplification 4-1408: Z-$L^{3-i}$-N(-$L^{1-b}$-$D^{1-b}$)(-$L^{2-a}$-$D^{2-ah}$),
Exemplification 4-1409: Z-$L^{3-i}$-N(-$L^{1-b}$-$D^{1-b}$)(-$L^{2-a}$-$D^{2-ai}$),
Exemplification 4-1410: Z-$L^{3-i}$-N(-$L^{1-b}$-$D^{1-b}$)(-$L^{2-a}$-$D^{2-aj}$),
Exemplification 4-1411: Z-$L^{3-i}$-N(-$L^{1-b}$-$D^{1-b}$)(-$L^{2-a}$-$D^{2-ak}$),
Exemplification 4-1412: Z-$L^{3-i}$-N(-$L^{1-b}$-$D^{1-b}$)(-$L^{2-a}$-$D^{2-al}$),
Exemplification 4-1413: Z-$L^{3-i}$-N(-$L^{1-b}$-$D^{1-b}$)(-$L^{2-a}$-$D^{2-am}$),
Exemplification 4-1414: Z-$L^{3-i}$-N(-$L^{1-b}$-$D^{1-b}$)(-$L^{2-a}$-$D^{2-an}$),
Exemplification 4-1415: Z-$L^{3-i}$-N(-$L^{1-b}$-$D^{1-b}$)(-$L^{2-a}$-$D^{2-ao}$),
Exemplification 4-1416: Z-$L^{3-i}$-N(-$L^{1-b}$-$D^{1-b}$)(-$L^{2-a}$-$D^{2-ap}$),
Exemplification 4-1417: Z-$L^{3-i}$-N(-$L^{1-b}$-$D^{1-b}$)(-$L^{2-b}$-$D^{2-aq}$),

Exemplification 4-1418: Z-$L^{3-i}$-N(-$L^{1-b}$-$D^{1-b}$)(-$L^{2-b}$-$D^{2-ar}$),
Exemplification 4-1419: Z-$L^{3-i}$-N(-$L^{1-b}$-$D^{1-b}$)(-$L^{2-b}$-$D^{2-as}$),
Exemplification 4-1420: Z-$L^{3-i}$-N(-$L^{1-b}$-$D^{1-b}$)(-$L^{2-b}$-$D^{2-at}$),
Exemplification 4-1421: Z-$L^{3-i}$-N(-$L^{1-b}$-$D^{1-b}$)(-$L^{2-c}$-$D^{2-an}$),
Exemplification 4-1422: Z-$L^{3-i}$-N(-$L^{1-b}$-$D^{1-b}$)(-$L^{2-c}$-$D^{2-ab}$),
Exemplification 4-1423: Z-$L^{3-i}$-N(-$L^{1-b}$-$D^{1-b}$)(-$L^{2-c}$-$D^{2-ac}$),
Exemplification 4-1424: Z-$L^{3-i}$-N(-$L^{1-b}$-$D^{1-b}$)(-$L^{2-c}$-$D^{2-ad}$),
Exemplification 4-1425: Z-$L^{3-i}$-N(-$L^{1-b}$-$D^{1-b}$)(-$L^{2-c}$-$D^{2-ae}$),
Exemplification 4-1426: Z-$L^{3-i}$-N(-$L^{1-b}$-$D^{1-b}$)(-$L^{2-c}$-$D^{2-af}$),
Exemplification 4-1427: Z-$L^{3-i}$-N(-$L^{1-b}$-$D^{1-b}$)(-$L^{2-c}$-$D^{2-ag}$),
Exemplification 4-1428: Z-$L^{3-i}$-N(-$L^{1-b}$-$D^{1-b}$)(-$L^{2-c}$-$D^{2-ah}$),
Exemplification 4-1429: Z-$L^{3-i}$-N(-$L^{1-b}$-$D^{1-b}$)(-$L^{2-c}$-$D^{2-ai}$),
Exemplification 4-1430: Z-$L^{3-i}$-N(-$L^{1-b}$-$D^{1-b}$)(-$L^{2-c}$-$D^{2-aj}$),
Exemplification 4-1431: Z-$L^{3-i}$-N(-$L^{1-b}$-$D^{1-b}$)(-$L^{2-c}$-$D^{2-ak}$),
Exemplification 4-1432: Z-$L^{3-i}$-N(-$L^{1-b}$-$D^{1-b}$)(-$L^{2-c}$-$D^{2-al}$),
Exemplification 4-1433: Z-$L^{3-i}$-N(-$L^{1-b}$-$D^{1-b}$)(-$L^{2-c}$-$D^{2-am}$),
Exemplification 4-1434: Z-$L^{3-i}$-N(-$L^{1-b}$-$D^{1-b}$)(-$L^{2-c}$-$D^{2-an}$),
Exemplification 4-1435: Z-$L^{3-i}$-N(-$L^{1-b}$-$D^{1-b}$)(-$L^{2-c}$-$D^{2-ao}$),
Exemplification 4-1436: Z-$L^{3-i}$-N(-$L^{1-b}$-$D^{1-b}$)(-$L^{2-c}$-$D^{2-ap}$),
Exemplification 4-1437: Z-$L^{3-i}$-N(-$L^{1-b}$-$D^{1-b}$)(-$L^{2-d}$-$D^{2-aq}$),
Exemplification 4-1438: Z-$L^{3-i}$-N(-$L^{1-b}$-$D^{1-b}$)(-$L^{2-d}$-$D^{2-ar}$),
Exemplification 4-1439: Z-$L^{3-i}$-N(-$L^{1-b}$-$D^{1-b}$)(-$L^{2-d}$-$D^{2-as}$),
Exemplification 4-1440: Z-$L^{3-i}$-N(-$L^{1-b}$-$D^{1-b}$)(-$L^{2-d}$-$D^{2-at}$).

The present invention also provides exemplification precursor compounds (Exemplification 5-1 to Exemplification 5-504) {in the following formula, Z, $L^{1-a}$ to $L^{1-b}$, $L^{2-b}$, $L^{2-d}$ to $L^{2-h}$, $L^{3-a}$ to $L^{3-i}$, $D^{1-a}$ to $D^{1-b}$, and $D^{2-ba}$ to $D^{2-bg}$ are as defined above}.

Exemplification 5-1: Z-$L^{3-a}$-N(-$L^{1-a}$-$D^{1-a}$)($L^{2-e}$-$D^{2-ba}$),
Exemplification 5-2: Z-$L^{3-a}$-N(-$L^{1-a}$-$D^{1-a}$)($L^{2-g}$-$D^{2-bb}$),
Exemplification 5-3: Z-$L^{3-a}$-N(-$L^{1-a}$-$D^{1-a}$)($L^{2-e}$-$D^{2-bc}$),
Exemplification 5-4: Z-$L^{3-a}$-N(-$L^{1-a}$-$D^{1-a}$)($L^{2-e}$-$D^{2-bd}$),
Exemplification 5-5: Z-$L^{3-a}$-N(-$L^{1-a}$-$D^{1-a}$)($L^{2-g}$-$D^{2-be}$),
Exemplification 5-6: Z-$L^{3-a}$-N(-$L^{1-a}$-$D^{1-a}$)($L^{2}$-b-$D^{2-bf}$),
Exemplification 5-7: Z-$L^{3-a}$-N(-$L^{1-a}$-$D^{1-a}$)($L^{2-b}$-$D^{2-bg}$),
Exemplification 5-8: Z-$L^{3-a}$-N(-$L^{1-a}$-$D^{1-a}$)($L^{2-f}$-$D^{2-ba}$),
Exemplification 5-9: Z-$L^{3-a}$-N(-$L^{1-a}$-$D^{1-a}$)($L^{2-h}$-$D^{2-bb}$),
Exemplification 5-10: Z-$L^{3-a}$-N(-$L^{1-a}$-$D^{1-a}$)($L^{2-f}$-$D^{2-bc}$),
Exemplification 5-11: Z-$L^{3-a}$-N(-$L^{1-a}$-$D^{1-a}$)($L^{2-f}$-$D^{2-bd}$),
Exemplification 5-12: Z-$L^{3-a}$-N(-$L^{1-a}$-$D^{1-a}$)($L^{2-h}$-$D^{2-be}$),
Exemplification 5-13: Z-$L^{3-a}$-N(-$L^{1-a}$-$D^{1-a}$)($L^{2-d}$-$D^{2-bf}$),
Exemplification 5-14: Z-$L^{3-a}$-N(-$L^{1-a}$-$D^{1-a}$)($L^{2-d}$-$D^{2-bg}$),
Exemplification 5-15: Z-$L^{3-a}$-N(-$L^{1-a}$-$D^{1-b}$)($L^{2-e}$-$D^{2-ba}$),
Exemplification 5-16: Z-$L^{3-a}$-N(-$L^{1-a}$-$D^{1-b}$)($L^{2-g}$-$D^{2-bb}$),
Exemplification 5-17: Z-$L^{3-a}$-N(-$L^{1-a}$-$D^{1-b}$)($L^{2-e}$-$D^{2-bc}$),
Exemplification 5-18: Z-$L^{3-a}$-N(-$L^{1-a}$-$D^{1-b}$)($L^{2-e}$-$D^{2-bd}$),
Exemplification 5-19: Z-$L^{3-a}$-N(-$L^{1-a}$-$D^{1-b}$)($L^{2-g}$-$D^{2-be}$),
Exemplification 5-20: Z-$L^{3-a}$-N(-$L^{1-a}$-$D^{1-b}$)($L^{2}$-b-$D^{2-bf}$),
Exemplification 5-21: Z-$L^{3-a}$-N(-$L^{1-a}$-$D^{1-b}$)($L^{2-b}$-$D^{2-bg}$),
Exemplification 5-22: Z-$L^{3-a}$-N(-$L^{1-a}$-$D^{1-b}$)($L^{2-f}$-$D^{2-ba}$),
Exemplification 5-23: Z-$L^{3-a}$-N(-$L^{1-a}$-$D^{1-b}$)($L^{2-h}$-$D^{2-bb}$),
Exemplification 5-24: Z-$L^{3-a}$-N(-$L^{1-a}$-$D^{1-b}$)($L^{2-f}$-$D^{2-bc}$),
Exemplification 5-25: Z-$L^{3-a}$-N(-$L^{1-a}$-$D^{1-b}$)($L^{2-f}$-$D^{2-bd}$),
Exemplification 5-26: Z-$L^{3-a}$-N(-$L^{1-a}$-$D^{1-b}$)($L^{2-h}$-$D^{2-be}$),
Exemplification 5-27: Z-$L^{3-a}$-N(-$L^{1-a}$-$D^{1-b}$)($L^{2-d}$-$D^{2-bf}$),
Exemplification 5-28: Z-$L^{3-a}$-N(-$L^{1-a}$-$D^{1-b}$)($L^{2-d}$-$D^{2-bg}$),
Exemplification 5-29: Z-$L^{3-a}$-N(-$L^{1-b}$-$D^{1-a}$)($L^{2-e}$-$D^{2-ba}$),
Exemplification 5-30: Z-$L^{3-a}$-N(-$L^{1-b}$-$D^{1-a}$)($L^{2-g}$-$D^{2-bb}$),

Exemplification 5-31: $Z-L^{3-a}-N(-L^{1-b}-D^{1-a})(L^{2-e}-D^{2-bc})$,
Exemplification 5-32: $Z-L^{3-a}-N(-L^{1-b}-D^{1-a})(L^{2-e}-D^{2-bd})$,
Exemplification 5-33: $Z-L^{3-a}-N(-L^{1-b}-D^{1-a})(L^{2-g}-D^{2-be})$,
Exemplification 5-34: $Z-L^{3-a}-N(-L^{1-b}-D^{1-a})(L^{2}-b-D^{2-bf})$,
Exemplification 5-35: $Z-L^{3-a}-N(-L^{1-b}-D^{1-a})(L^{2-b}-D^{2-bg})$,
Exemplification 5-36: $Z-L^{3-a}-N(-L^{1-b}-D^{1-a})(L^{2-f}-D^{2-ba})$,
Exemplification 5-37: $Z-L^{3-a}-N(-L^{1-b}-D^{1-a})(L^{2-h}-D^{2-bb})$,
Exemplification 5-38: $Z-L^{3-a}-N(-L^{1-b}-D^{1-a})(L^{2-f}-D^{2-bc})$,
Exemplification 5-39: $Z-L^{3-a}-N(-L^{1-b}-D^{1-a})(L^{2-f}-D^{2-bd})$,
Exemplification 5-40: $Z-L^{3-a}-N(-L^{1-b}-D^{1-a})(L^{2-h}-D^{2-be})$,
Exemplification 5-41: $Z-L^{3-a}-N(-L^{1-b}-D^{1-a})(L^{2-d}-D^{2-bf})$,
Exemplification 5-42: $Z-L^{3-a}-N(-L^{1-b}-D^{1-a})(L^{2-d}-D^{2-bg})$,
Exemplification 5-43: $Z-L^{3-a}-N(-L^{1-b}-D^{1-b})(L^{2-e}-D^{2-ba})$,
Exemplification 5-44: $Z-L^{3-a}-N(-L^{1-b}-D^{1-b})(L^{2-g}-D^{2-bb})$,
Exemplification 5-45: $Z-L^{3-a}-N(-L^{1-b}-D^{1-b})(L^{2-e}-D^{2-bc})$,
Exemplification 5-46: $Z-L^{3-a}-N(-L^{1-b}-D^{1-b})(L^{2-e}-D^{2-bd})$,
Exemplification 5-47: $Z-L^{3-a}-N(-L^{1-b}-D^{1-b})(L^{2-g}-D^{2-be})$,
Exemplification 5-48: $Z-L^{3-a}-N(-L^{1-b}-D^{1-b})(L^{2}-b-D^{2-bf})$,
Exemplification 5-49: $Z-L^{3-a}-N(-L^{1-b}-D^{1-b})(L^{2-b}-D^{2-bg})$,
Exemplification 5-50: $Z-L^{3-a}-N(-L^{1-b}-D^{1-b})(L^{2-f}-D^{2-ba})$,
Exemplification 5-51: $Z-L^{3-a}-N(-L^{1-b}-D^{1-b})(L^{2-h}-D^{2-bb})$,
Exemplification 5-52: $Z-L^{3-a}-N(-L^{1-b}-D^{1-b})(L^{2-f}-D^{2-bc})$,
Exemplification 5-53: $Z-L^{3-a}-N(-L^{1-b}-D^{1-b})(L^{2-f}-D^{2-bd})$,
Exemplification 5-54: $Z-L^{3-a}-N(-L^{1-b}-D^{1-b})(L^{2-h}-D^{2-be})$,
Exemplification 5-55: $Z-L^{3-a}-N(-L^{1-b}-D^{1-b})(L^{2-d}-D^{2-bf})$,
Exemplification 5-56: $Z-L^{3-a}-N(-L^{1-b}-D^{1-b})(L^{2-d}-D^{2-bg})$,
Exemplification 5-57: $Z-L^{3-b}-N(-L^{1-a}-D^{1-a})(L^{2-e}-D^{2-ba})$,
Exemplification 5-58: $Z-L^{3-b}-N(-L^{1-a}-D^{1-a})(L^{2-g}-D^{2-bb})$,
Exemplification 5-59: $Z-L^{3-b}-N(-L^{1-a}-D^{1-a})(L^{2-e}-D^{2-bc})$,
Exemplification 5-60: $Z-L^{3-b}-N(-L^{1-a}-D^{1-a})(L^{2-e}-D^{2-bd})$,
Exemplification 5-61: $Z-L^{3-b}-N(-L^{1-a}-D^{1-a})(L^{2-g}-D^{2-be})$,
Exemplification 5-62: $Z-L^{3-b}-N(-L^{1-a}-D^{1-a})(L^{2-b}-D^{2-bf})$,
Exemplification 5-63: $Z-L^{3-b}-N(-L^{1-a}-D^{1-a})(L^{2-b}-D^{2-bg})$,
Exemplification 5-64: $Z-L^{3-b}-N(-L^{1-a}-D^{1-a})(L^{2-f}-D^{2-ba})$,
Exemplification 5-65: $Z-L^{3-b}-N(-L^{1-a}-D^{1-a})(L^{2-h}-D^{2-bb})$,
Exemplification 5-66: $Z-L^{3-b}-N(-L^{1-a}-D^{1-a})(L^{2-f}-D^{2-bc})$,
Exemplification 5-67: $Z-L^{3-b}-N(-L^{1-a}-D^{1-a})(L^{2-f}-D^{2-bd})$,
Exemplification 5-68: $Z-L^{3-b}-N(-L^{1-a}-D^{1-a})(L^{2-h}-D^{2-be})$,
Exemplification 5-69: $Z-L^{3-b}-N(-L^{1-a}-D^{1-a})(L^{2-d}-D^{2-bf})$,
Exemplification 5-70: $Z-L^{3-b}-N(-L^{1-a}-D^{1-a})(L^{2-d}-D^{2-bg})$,
Exemplification 5-71: $Z-L^{3-b}-N(-L^{1-a}-D^{1-b})$ $(L^{2-e}-D^{2-ba})$,
Exemplification 5-72: $Z-L^{3-b}-N(-L^{1-a}-D^{1-b})$ $(L^{2-g}-D^{2-bb})$,
Exemplification 5-73: $Z-L^{3-b}-N(-L^{1-a}-D^{1-b})$ $(L^{2-e}-D^{2-bc})$,
Exemplification 5-74: $Z-L^{3-b}-N(-L^{1-a}-D^{1-b})$ $(L^{2-e}-D^{2-bd})$,
Exemplification 5-75: $Z-L^{3-b}-N(-L^{1-a}-D^{1-b})(L^{2-g}-D^{2-be})$,
Exemplification 5-76: $Z-L^{3-b}-N(-L^{1-a}-D^{1-b})(L^{2-b}-D^{2-bf})$,
Exemplification 5-77: $Z-L^{3-b}-N(-L^{1-a}-D^{1-b})(L^{2-b}-D^{2-bg})$,
Exemplification 5-78: $Z-L^{3-b}-N(-L^{1-a}-D^{1-b})(L^{2-f}-D^{2-ba})$,
Exemplification 5-79: $Z-L^{3-b}-N(-L^{1-a}-D^{1-b})$ $(L^{2-h}-D^{2-bb})$,
Exemplification 5-80: $Z-L^{3-b}-N(-L^{1-a}-D^{1-b})(L^{2-f}-D^{2-bc})$,
Exemplification 5-81: $Z-L^{3-b}-N(-L^{1-a}-D^{1-b})(L^{2-f}-D^{2-bd})$,
Exemplification 5-82: $Z-L^{3-b}-N(-L^{1-a}-D^{1-b})(L^{2-h}-D^{2-be})$,
Exemplification 5-83: $Z-L^{3-b}-N(-L^{1-a}-D^{1-b})(L^{2-d}-D^{2-bf})$,
Exemplification 5-84: $Z-L^{3-b}-N(-L^{1-a}-D^{1-b})(L^{2-d}-D^{2-bg})$,
Exemplification 5-85: $Z-L^{3-b}-N(-L^{1-b}-D^{1-a})$ $(L^{2-e}-D^{2-ba})$,
Exemplification 5-86: $Z-L^{3-b}-N(-L^{1-b}-D^{1-a})$ $(L^{2-g}-D^{2-bb})$,
Exemplification 5-87: $Z-L^{3-b}-N(-L^{1-b}-D^{1-a})$ $(L^{2-e}-D^{2-bc})$,
Exemplification 5-88: $Z-L^{3-b}-N(-L^{1-b}-D^{1-a})(L^{2-e}-D^{2-bd})$,

Exemplification 5-89: Z-$L^{3-b}$-N(-$L^{1-b}$-$D^{1-a}$) ($L^{2-g}$-$D^{2-be}$),
Exemplification 5-90: Z-$L^{3-b}$-N(-$L^{1-b}$-$D^{1-a}$) ($L^{2-b}$-$D^{2-bf}$),
Exemplification 5-91: Z-$L^{3-b}$-N(-$L^{1-b}$-$D^{1-a}$) ($L^{2-b}$-$D^{2-bg}$),
Exemplification 5-92: Z-$L^{3-b}$-N(-$L^{1-b}$-$D^{1-a}$) ($L^{2-f}$-$D^{2-ba}$),
Exemplification 5-93: Z-$L^{3-b}$-N(-$L^{1-b}$-$D^{1-a}$) ($L^{2-h}$-$D^{2-bb}$),
Exemplification 5-94: Z-$L^{3-b}$-N(-$L^{1-b}$-$D^{1-a}$) ($L^{2-f}$-$D^{2-bc}$),
Exemplification 5-95: Z-$L^{3-b}$-N(-$L^{1-b}$-$D^{1-a}$) ($L^{2-f}$-$D^{2-bd}$),
Exemplification 5-96: Z-$L^{3-b}$-N(-$L^{1-b}$-$D^{1-a}$)($L^{2-h}$-$D^{2-be}$),
Exemplification 5-97 : Z-$L^{3-b}$-N(-$L^{1-b}$-$D^{1-a}$) ($L^{2-d}$-$D^{2-bf}$),
Exemplification 5-98 : Z-$L^{3-b}$-N(-$L^{1-b}$-$D^{1-a}$) ($L^{2-d}$-$D^{2-bg}$),
Exemplification 5-99: Z-$L^{3-b}$-N(-$L^{1-b}$-$D^{1-b}$)($L^{2-e}$-$D^{2-ba}$),
Exemplification 5-100: Z-$L^{3-b}$-N(-$L^{1-b}$-$D^{1-b}$)($L^{2-g}$-$D^{2-bb}$),
Exemplification 5-101: Z-$L^{3-b}$-N(-$L^{1-b}$-$D^{1-b}$)($L^{2-e}$-$D^{2-bc}$),
Exemplification 5-102: Z-$L^{3-b}$-N(-$L^{1-b}$-$D^{1-b}$)($L^{2-e}$-$D^{2-bd}$),
Exemplification 5-103: Z-$L^{3-b}$-N(-$L^{1-b}$-$D^{1-b}$)($L^{2-g}$-$D^{2-be}$),
Exemplification 5-104: Z-$L^{3-b}$-N(-$L^{1-b}$-$D^{1-b}$)($L^{2-b}$-$D^{2-bf}$),
Exemplification 5-105: Z-$L^{3-b}$-N(-$L^{1-b}$-$D^{1-b}$)($L^{2-b}$-$D^{2-bg}$),
Exemplification 5-106: Z-$L^{3-b}$-N(-$L^{1-b}$-$D^{1-b}$)($L^{2-f}$-$D^{2-ba}$),
Exemplification 5-107: Z-$L^{3-b}$-N(-$L^{1-b}$-$D^{1-b}$)($L^{2-h}$-$D^{2-bb}$),
Exemplification 5-108 : Z-$L^{3-b}$-N(-$L^{1-b}$-$D^{1-b}$)($L^{2-f}$-$D^{2-bc}$),
Exemplification 5-109: Z-$L^{3-b}$-N(-$L^{1-b}$-$D^{1-b}$)($L^{2-f}$-$D^{2-bd}$),
Exemplification 5-110: Z-$L^{3-b}$-N(-$L^{1-b}$-$D^{1-b}$)($L^{2-h}$-$D^{2-be}$),
Exemplification 5-111: Z-$L^{3-b}$-N(-$L^{1-b}$-$D^{1-b}$)($L^{2-d}$-$D^{2-bf}$),
Exemplification 5-112: Z-$L^{3-b}$-N(-$L^{1-b}$-$D^{1-b}$)($L^{2-d}$-$D^{2-b9}$),
Exemplification 5-113: Z-$L^{3-c}$-N(-$L^{1-a}$-$D^{1-a}$)($L^{2-e}$-$D^{2-ba}$),
Exemplification 5-114: Z-$L^{3-c}$-N(-$L^{1-a}$-$D^{1-a}$)($L^{2-g}$-$D^{2-bb}$),
Exemplification 5-115: Z-$L^{3-c}$-N(-$L^{1-a}$-$D^{1-a}$)($L^{2-e}$-$D^{2-bc}$),
Exemplification 5-116: Z-$L^{3-c}$-N(-$L^{1-a}$-$D^{1-a}$)($L^{2-e}$-$D^{2-bd}$),
Exemplification 5-117: Z-$L^{3-c}$-N(-$L^{1-a}$-$D^{1-a}$)($L^{2-g}$-$D^{2-be}$),
Exemplification 5-118 : Z-$L^{3-c}$-N(-$L^{1-a}$-$D^{1-a}$)($L^{2-b}$-$D^{2-bf}$),
Exemplification 5-119: Z-$L^{3-c}$-N(-$L^{1-a}$-$D^{1-a}$)($L^{2-b}$-$D^{2-bg}$),
Exemplification 5-120: Z-$L^{3-c}$-N(-$L^{1-a}$-$D^{1-a}$)($L^{2-f}$-$D^{2-ba}$),
Exemplification 5-121: Z-$L^{3-c}$-N(-$L^{1-a}$-$D^{1-a}$)($L^{2-h}$-$D^{2-bb}$),
Exemplification 5-122: Z-$L^{3-c}$-N(-$L^{1-a}$-$D^{1-a}$)($L^{2-f}$-$D^{2-bc}$),
Exemplification 5-123: Z-$L^{3-c}$-N(-$L^{1-a}$-$D^{1-a}$)($L^{2-f}$-$D^{2-bd}$),
Exemplification 5-124: Z-$L^{3-c}$-N(-$L^{1-a}$-$D^{1-a}$)($L^{2-h}$-$D^{2-be}$),
Exemplification 5-125: Z-$L^{3-c}$-N(-$L^{1-a}$-$D^{1-a}$)($L^{2-d}$-$D^{2-bf}$),
Exemplification 5-126: Z-$L^{3-c}$-N(-$L^{1-a}$-$D^{1-a}$)($L^{2-d}$-$D^{2-bg}$),
Exemplification 5-127: Z-$L^{3-c}$-N(-$L^{1-a}$-$D^{1-b}$)($L^{2-e}$-$D^{2-ba}$),
Exemplification 5-128: Z-$L^{3-c}$-N(-$L^{1-a}$-$D^{1-b}$)($L^{2-g}$-$D^{2-bb}$),
Exemplification 5-129: Z-$L^{3-c}$-N(-$L^{1-a}$-$D^{1-b}$)($L^{2-e}$-$D^{2-bc}$),
Exemplification 5-130: Z-$L^{3-c}$-N(-$L^{1-a}$-$D^{1-b}$) ($L^{2-e}$-$D^{2-bd}$),
Exemplification 5-131: Z-$L^{3-c}$-N(-$L^{1-a}$-$D^{1-b}$)($L^{2-g}$-$D^{2-be}$), Exemplification 5-132: Z-$L^{3-c}$-N(-$L^{1-a}$-$D^{1-b}$) ($L^{2-b}$-$D^{2-bf}$), Exemplification 5-133: Z-$L^{3-c}$-N(-$L^{1-a}$-$D^{1-b}$) ($L^{2-b}$-$D^{2-bg}$), Exemplification 5-134: Z-$L^{3-c}$-N(-$L^{1-a}$-$D^{1-b}$) ($L^{2-f}$-$D^{2-ba}$), Exemplification 5-135: Z-$L^{3-c}$-N(-$L^{1-a}$-$D^{1-b}$) ($L^{2-h}$-$D^{2-bb}$), Exemplification 5-136: Z-$L^{3-c}$-N(-$L^{1-a}$-$D^{1-b}$) ($L^{2-f}$-$D^{2-bc}$), Exemplification 5-137: Z-$L^{3-c}$-N(-$L^{1-a}$-$D^{1-b}$) ($L^{2-f}$-$D^{2-bd}$), Exemplification 5-138: Z-$L^{3-c}$-N(-$L^{1-a}$-$D^{1-b}$) ($L^{2-h}$-$D^{2-be}$), Exemplification 5-139: Z-$L^{3-c}$-N(-$L^{1-a}$-$D^{1-b}$) ($L^{2-d}$-$D^{2-bf}$), Exemplification 5-140: Z-$L^{3-c}$-N(-$L^{1-a}$-$D^{1-b}$) ($L^{2-d}$-$D^{2-bg}$), Exemplification 5-141: Z-$L^{3-c}$-N(-$L^{1-b}$-$D^{1-a}$)($L^{2-e}$-$D^{2-ba}$), Exemplification 5-142: Z-$L^{3-c}$-N(-$L^{1-b}$-$D^{1-a}$) ($L^{2-g}$-$D^{2-bb}$), Exemplification 5-143: Z-$L^{3-c}$-N(-$L^{1-b}$-$D^{1-a}$) ($L^{2-e}$-$D^{2-bc}$), Exemplification 5-144: Z-$L^{3-c}$-N(-$L^{1-b}$-$D^{1-a}$) ($L^{2-e}$-$D^{2-bd}$), Exemplification 5-145: Z-$L^{3-c}$-N(-$L^{1-b}$-$D^{1-a}$)($L^{2-g}$-$D^{2-be}$), Exemplification 5-146: Z-$L^{3-c}$-N(-$L^{1-b}$-$D^{1-a}$)($L^{2-b}$-$D^{2-bf}$), Exemplification 5-147: Z-$L^{3-c}$-N(-$L^{1-b}$-$D^{1-a}$)($L^{2-b}$-$D^{2-bg}$), Exemplification 5-148: Z-$L^{3-c}$-N(-$L^{1-b}$-$D^{1-a}$)($L^{2-f}$-$D^{2-ba}$), Exemplification 5-149: Z-$L^{3-c}$-N(-$L^{1-b}$-$D^{1-a}$)($L^{2-h}$-$D^{2-bb}$), Exemplification 5-150: Z-$L^{3-c}$-N(-$L^{1-b}$-$D^{1-a}$)($L^{2-f}$-$D^{2-bc}$), Exemplification 5-151: Z-$L^{3-c}$-N(-$L^{1-b}$-$D^{1-a}$)($L^{2-f}$-$D^{2-bd}$), Exemplification 5-152: Z-$L^{3-c}$-N(-$L^{1-b}$-$D^{1-a}$) ($L^{2-h}$-$D^{2-be}$), Exemplification 5-153: Z-$L^{3-c}$-N(-$L^{1-b}$-$D^{1-a}$) ($L^{2-d}$-$D^{2-bf}$), Exemplification 5-154: Z-$L^{3-c}$-N(-$L^{1-b}$-$D^{1-a}$)($L^{2-d}$-$D^{2-bg}$), Exemplification 5-155: Z-$L^{3-c}$-N(-$L^{1-b}$-$D^{1-b}$)($L^{2-e}$-$D^{2-ba}$), Exemplification 5-156: Z-$L^{3-c}$-N(-$L^{1-b}$-$D^{1-b}$)($L^{2-g}$-$D^{2-bb}$), Exemplification 5-157: Z-$L^{3-c}$-N(-$L^{1-b}$-$D^{1-b}$)($L^{2-e}$-$D^{2-bc}$),
Exemplification 5-158: Z-$L^{3-c}$-N(-$L^{1-b}$-$D^{1-b}$)($L^{2-e}$-$D^{2-bd}$),
Exemplification 5-159: Z-$L^{3-c}$-N(-$L^{1-b}$-$D^{1-b}$)($L^{2-g}$-$D^{2-be}$),

Exemplification 5-160: Z-$L^{3-c}$-N(-$L^{1-b}$-$D^{1-b}$)($L^{2-b}$-$D^{2-bf}$),
Exemplification 5-161: Z-$L^{3-c}$-N(-$L^{1-b}$-$D^{1-b}$)($L^{2-b}$-$D^{2-bg}$),
Exemplification 5-162: Z-$L^{3-c}$-N(-$L^{1-b}$-$D^{1-b}$)($L^{2-f}$-$D^{2-ba}$),
Exemplification 5-163: Z-$L^{3-c}$-N(-$L^{1-b}$-$D^{1-b}$)($L^{2-h}$-$D^{2-bb}$),
Exemplification 5-164: Z-$L^{3-c}$-N(-$L^{1-b}$-$D^{1-b}$)($L^{2-f}$-$D^{2-bc}$),
Exemplification 5-165: Z-$L^{3-c}$-N(-$L^{1-b}$-$D^{1-b}$)($L^{2-f}$-$D^{2-bd}$),
Exemplification 5-166: Z-$L^{3-c}$-N(-$L^{1-b}$-$D^{1-b}$)($L^{2-h}$-$D^{2-be}$),
Exemplification 5-167: Z-$L^{3-c}$-N(-$L^{1-b}$-$D^{1-b}$)($L^{2-d}$-$D^{2-bf}$),
Exemplification 5-168: Z-$L^{3-c}$-N(-$L^{1-b}$-$D^{1-b}$)($L^{2-d}$-$D^{2-bg}$),
Exemplification 5-169: Z-$L^{3-d}$-N(-$L^{1-a}$-$D^{1-a}$)($L^{2-e}$-$D^{2-ba}$),
Exemplification 5-170: Z-$L^{3-d}$-N(-$L^{1-a}$-$D^{1-a}$)($L^{2-g}$-$D^{2-bb}$),
Exemplification 5-171: Z-$L^{3-d}$-N(-$L^{1-a}$-$D^{1-a}$)($L^{2-e}$-$D^{2-bc}$),
Exemplification 5-172: Z-$L^{3-d}$-N(-$L^{1-a}$-$D^{1-a}$)($L^{2-e}$-$D^{2-bd}$),
Exemplification 5-173: Z-$L^{3-d}$-N(-$L^{1-a}$-$D^{1-a}$)($L^{2-g}$-$D^{2-be}$),
Exemplification 5-174: Z-$L^{3-d}$-N(-$L^{1-a}$-$D^{1-a}$)($L^{2-b}$-$D^{2-bf}$),
Exemplification 5-175: Z-$L^{3-d}$-N(-$L^{1-a}$-$D^{1-a}$)($L^{2-b}$-$D^{2-bg}$),
Exemplification 5-176: Z-$L^{3-d}$-N(-$L^{1-a}$-$D^{1-a}$)($L^{2-f}$-$D^{2-ba}$),
Exemplification 5-177: Z-$L^{3-d}$-N(-$L^{1-a}$-$D^{1-a}$)($L^{2-h}$-$D^{2-bb}$),
Exemplification 5-178: Z-$L^{3-d}$-N(-$L^{1-a}$-$D^{1-a}$)($L^{2-f}$-$D^{2-bc}$),
Exemplification 5-179: Z-$L^{3-d}$-N(-$L^{1-a}$-$D^{1-a}$)($L^{2-f}$-$D^{2-bd}$),
Exemplification 5-180: Z-$L^{3-d}$-N(-$L^{1-a}$-$D^{1-a}$)($L^{2-h}$-$D^{2-be}$),
Exemplification 5-181: Z-$L^{3-d}$-N(-$L^{1-a}$-$D^{1-a}$)($L^{2-d}$-$D^{2-bf}$),
Exemplification 5-182: Z-$L^{3-d}$-N(-$L^{1-a}$-$D^{1-a}$)($L^{2-d}$-$D^{2-bg}$),
Exemplification 5-183: Z-$L^{3-d}$-N(-$L^{1-a}$-$D^{1-b}$) ($L^{2-e}$-$D^{2-ba}$),
Exemplification 5-184: Z-$L^{3-d}$-N(-$L^{1-a}$-$D^{1-b}$) ($L^{2-g}$-$D^{2-bb}$),
Exemplification 5-185: Z-$L^{3-d}$-N(-$L^{1-a}$-$D^{1-b}$)($L^{2-e}$-$D^{2-bc}$),
Exemplification 5-186: Z-$L^{3-d}$-N(-$L^{1-a}$-$D^{1-b}$) ($L^{2-e}$-$D^{2-bd}$),
Exemplification 5-187: Z-$L^{3-d}$-N(-$L^{1-a}$-$D^{1-b}$) ($L^{2-g}$-$D^{2-be}$),
Exemplification 5-188: Z-$L^{3-d}$-N(-$L^{1-a}$-$D^{1-b}$) ($L^{2-b}$-$D^{2-bf}$),
Exemplification 5-189: Z-$L^{3-d}$-N(-$L^{1-a}$-$D^{1-b}$) ($L^{2-b}$-$D^{2-bg}$),
Exemplification 5-190: Z-$L^{3-d}$-N(-$L^{1-a}$-$D^{1-b}$)($L^{2-f}$-$D^{2-ba}$),
Exemplification 5-191: Z-$L^{3-d}$-N(-$L^{1-a}$-$D^{1-b}$) ($L^{2-h}$-$D^{2-bb}$),
Exemplification 5-192: Z-$L^{3-d}$-N(-$L^{1-a}$-$D^{1-b}$) ($L^{2-f}$-$D^{2-bc}$),
Exemplification 5-193: Z-$L^{3-d}$-N(-$L^{1-a}$-$D^{1-b}$) ($L^{2-f}$-$D^{2-bd}$),
Exemplification 5-194: Z-$L^{3-d}$-N(-$L^{1-a}$-$D^{1-b}$) ($L^{2-h}$-$D^{2-be}$),
Exemplification 5-195: Z-$L^{3-d}$-N(-$L^{1-a}$-$D^{1-b}$) ($L^{2-d}$-$D^{2-bf}$),
Exemplification 5-196: Z-$L^{3-d}$-N(-$L^{1-a}$-$D^{1-b}$) ($L^{2-d}$-$D^{2-bg}$),
Exemplification 5-197: Z-$L^{3-d}$-N(-$L^{1-b}$-$D^{1-a}$) ($L^{2-e}$-$D^{2-ba}$),
Exemplification 5-198: Z-$L^{3-d}$-N(-$L^{1-b}$-$D^{1-a}$)($L^{2-g}$-$D^{2-bb}$),
Exemplification 5-199: Z-$L^{3-d}$-N(-$L^{1-b}$-$D^{1-a}$)($L^{2-e}$-$D^{2-bc}$),
Exemplification 5-200: Z-$L^{3-d}$-N(-$L^{1-b}$-$D^{1-a}$) ($L^{2-e}$-$D^{2-bd}$),
Exemplification 5-201: Z-$L^{3-d}$-N(-$L^{1-b}$-$D^{1-a}$)($L^{2-g}$-$D^{2-be}$),
Exemplification 5-202: Z-$L^{3-d}$-N(-$L^{1-b}$-$D^{1-a}$) ($L^{2-b}$-$D^{2-bf}$),
Exemplification 5-203: Z-$L^{3-d}$-N(-$L^{1-b}$-$D^{1-a}$) ($L^{2-b}$-$D^{2-bg}$),
Exemplification 5-204: Z-$L^{3-d}$-N(-$L^{1-b}$-$D^{1-a}$) ($L^{2-f}$-$D^{2-ba}$),
Exemplification 5-205: Z-$L^{3-d}$-N(-$L^{1-b}$-$D^{1-a}$) ($L^{2-h}$-$D^{2-bb}$),
Exemplification 5-206: Z-$L^{3-d}$-N(-$L^{1-b}$-$D^{1-a}$) ($L^{2-f}$-$D^{2-bc}$),
Exemplification 5-207: Z-$L^{3-d}$-N(-$L^{1-b}$-$D^{1-a}$) ($L^{2-f}$-$D^{2-bd}$),
Exemplification 5-208: Z-$L^{3-d}$-N(-$L^{1-b}$-$D^{1-a}$) ($L^{2-h}$-$D^{2-be}$),
Exemplification 5-209: Z-$L^{3-d}$-N(-$L^{1-b}$-$D^{1-a}$) ($L^{2-d}$-$D^{2-bf}$),
Exemplification 5-210: Z-$L^{3-d}$-N(-$L^{1-b}$-$D^{1-a}$) ($L^{2-d}$-$D^{2-bg}$),
Exemplification 5-211: Z-$L^{3-d}$-N(-$L^{1-b}$-$D^{1-b}$)($L^{2-e}$-$D^{2-ba}$),
Exemplification 5-212: Z-$L^{3-d}$-N(-$L^{1-b}$-$D^{1-b}$)($L^{2-g}$-$D^{2-bb}$),
Exemplification 5-213: Z-$L^{3-d}$-N(-$L^{1-b}$-$D^{1-b}$)($L^{2-e}$-$D^{2-bc}$),
Exemplification 5-214: Z-$L^{3-d}$-N(-$L^{1-b}$-$D^{1-b}$)($L^{2-e}$-$D^{2-bd}$),
Exemplification 5-215: Z-$L^{3-d}$-N(-$L^{1-b}$-$D^{1-b}$)($L^{2-g}$-$D^{2-be}$),
Exemplification 5-216: Z-$L^{3-d}$-N(-$L^{1-b}$-$D^{1-b}$)($L^{2-b}$-$D^{2-bf}$),
Exemplification 5-217: Z-$L^{3-d}$-N(-$L^{1-b}$-$D^{1-b}$)($L^{2-b}$-$D^{2-bg}$),

Exemplification 5-218: Z-$L^{3-d}$-N(-$L^{1-b}$-$D^{1-b}$)($L^{2-f}$-$D^{2-ba}$),
Exemplification 5-219: Z-$L^{3-d}$-N(-$L^{1-b}$-$D^{1-b}$)($L^{2-h}$-$D^{2-bb}$),
Exemplification 5-220: Z-$L^{3-d}$-N(-$L^{1-b}$-$D^{1-b}$)($L^{2-f}$-$D^{2-bc}$),
Exemplification 5-221: Z-$L^{3-d}$-N(-$L^{1-b}$-$D^{1-b}$)($L^{2-f}$-$D^{2-bd}$),
Exemplification 5-222: Z-$L^{3-d}$-N(-$L^{1-b}$-$D^{1-b}$)($L^{2-h}$-$D^{2-be}$),
Exemplification 5-223: Z-$L^{3-d}$-N(-$L^{1-b}$-$D^{1-b}$)($L^{2-d}$-$D^{2-bf}$),
Exemplification 5-224: Z-$L^{3-d}$-N(-$L^{1-b}$-$D^{1-b}$)($L^{2-d}$-$D^{2-bg}$),
Exemplification 5-225: Z-$L^{3-e}$-N(-$L^{1-a}$-$D^{1-a}$)($L^{2-e}$-$D^{2-ba}$),
Exemplification 5-226: Z-$L^{3-e}$-N(-$L^{1-a}$-$D^{1-a}$)($L^{2-g}$-$D^{2-bb}$),
Exemplification 5-227: Z-$L^{3-e}$-N(-$L^{1-a}$-$D^{1-a}$)($L^{2-e}$-$D^{2-bc}$),
Exemplification 5-228: Z-$L^{3-e}$-N(-$L^{1-a}$-$D^{1-a}$)($L^{2-e}$-$D^{2-bd}$),
Exemplification 5-229: Z-$L^{3-e}$-N(-$L^{1-a}$-$D^{1-a}$)($L^{2-g}$-$D^{2-be}$),
Exemplification 5-230: Z-$L^{3-e}$-N(-$L^{1-a}$-$D^{1-a}$)($L^{2-b}$-$D^{2-bf}$),
Exemplification 5-231: Z-$L^{3-e}$-N(-$L^{1-a}$-$D^{1-a}$)($L^{2-b}$-$D^{2-bg}$),
Exemplification 5-232: Z-$L^{3-e}$-N(-$L^{1-a}$-$D^{1-a}$)($L^{2-f}$-$D^{2-ba}$),
Exemplification 5-233: Z-$L^{3-e}$-N(-$L^{1-a}$-$D^{1-a}$)($L^{2-h}$-$D^{2-bb}$),
Exemplification 5-234: Z-$L^{3-e}$-N(-$L^{1-a}$-$D^{1-a}$)($L^{2-f}$-$D^{2-bc}$),
Exemplification 5-235: Z-$L^{3-e}$-N(-$L^{1-a}$-$D^{1-a}$)($L^{2-f}$-$D^{2-bd}$),
Exemplification 5-236: Z-$L^{3-e}$-N(-$L^{1-a}$-$D^{1-a}$)($L^{2-h}$-$D^{2-be}$),
Exemplification 5-237: Z-$L^{3-e}$-N(-$L^{1-a}$-$D^{1-a}$)($L^{2-d}$-$D^{2-bf}$),
Exemplification 5-238: Z-$L^{3-e}$-N(-$L^{1-a}$-$D^{1-a}$)($L^{2-d}$-$D^{2-bg}$),
Exemplification 5-239: Z-$L^{3-e}$-N(-$L^{1-a}$-$D^{1-b}$) ($L^{2-e}$-$D^{2-ba}$),
Exemplification 5-240: Z-$L^{3-e}$-N(-$L^{1-a}$-$D^{1-b}$)($L^{2-g}$-$D^{2-bb}$),
Exemplification 5-241: Z-$L^{3-e}$-N(-$L^{1-a}$-$D^{1-b}$) ($L^{2-e}$-$D^{2-bc}$),
Exemplification 5-242: Z-$L^{3-e}$-N(-$L^{1-a}$-$D^{1-b}$)($L^{2-e}$-$D^{2-bd}$),
Exemplification 5-243: Z-$L^{3-e}$-N(-$L^{1-a}$-$D^{1-b}$) ($L^{2-g}$-$D^{2-be}$),
Exemplification 5-244: Z-$L^{3-e}$-N(-$L^{1-a}$-$D^{1-b}$) ($L^{2-b}$-$D^{2-bf}$),
Exemplification 5-245: Z-$L^{3-e}$-N(-$L^{1-a}$-$D^{1-b}$) ($L^{2-b}$-$D^{2-bg}$),
Exemplification 5-246: Z-$L^{3-e}$-N(-$L^{1-a}$-$D^{1-b}$) ($L^{2-f}$-$D^{2-ba}$),
Exemplification 5-247: Z-$L^{3-e}$-N(-$L^{1-a}$-$D^{1-b}$) ($L^{2-h}$-$D^{2-bb}$),
Exemplification 5-248: Z-$L^{3-e}$-N(-$L^{1-a}$-$D^{1-b}$) ($L^{2-f}$-$D^{2-bc}$),
Exemplification 5-249: Z-$L^{3-e}$-N(-$L^{1-a}$-$D^{1-b}$) ($L^{2-f}$-$D^{2-bd}$),
Exemplification 5-250: Z-$L^{3-e}$-N(-$L^{1-a}$-$D^{1-b}$) ($L^{2-h}$-$D^{2-be}$),
Exemplification 5-251: Z-$L^{3-e}$-N(-$L^{1-a}$-$D^{1-b}$) ($L^{2-d}$-$D^{2-bf}$),
Exemplification 5-252: Z-$L^{3-e}$-N(-$L^{1-a}$-$D^{1-b}$) ($L^{2-d}$-$D^{2-bg}$),
Exemplification 5-253: Z-$L^{3-e}$-N(-$L^{1-b}$-$D^{1-a}$) ($L^{2-e}$-$D^{2-ba}$),
Exemplification 5-254: Z-$L^{3-e}$-N(-$L^{1-b}$-$D^{1-a}$)($L^{2-g}$-$D^{2-bb}$),
Exemplification 5-255: Z-$L^{3-e}$-N(-$L^{1-b}$-$D^{1-a}$) ($L^{2-e}$-$D^{2-bc}$),
Exemplification 5-256: Z-$L^{3-e}$-N(-$L^{1-b}$-$D^{1-a}$) ($L^{2-e}$-$D^{2-bd}$),
Exemplification 5-257: Z-$L^{3-e}$-N(-$L^{1-b}$-$D^{1-a}$) ($L^{2-g}$-$D^{2-be}$),
Exemplification 5-258: Z-$L^{3-e}$-N(-$L^{1-b}$-$D^{1-a}$)($L^{2-b}$-$D^{2-bf}$),
Exemplification 5-259: Z-$L^{3-e}$-N(-$L^{1-b}$-$D^{1-a}$)($L^{2-b}$-$D^{2-bg}$),
Exemplification 5-260: Z-$L^{3-e}$-N(-$L^{1-b}$-$D^{1-a}$)($L^{2-f}$-$D^{2-ba}$),
Exemplification 5-261: Z-$L^{3-e}$-N(-$L^{1-b}$-$D^{1-a}$) ($L^{2-h}$-$D^{2-bb}$),
Exemplification 5-262: Z-$L^{3-e}$-N(-$L^{1-b}$-$D^{1-a}$) ($L^{2-f}$-$D^{2-bc}$),
Exemplification 5-263: Z-$L^{3-e}$-N(-$L^{1-b}$-$D^{1-a}$) ($L^{2-f}$-$D^{2-bd}$),
Exemplification 5-264: Z-$L^{3-e}$-N(-$L^{1-b}$-$D^{1-a}$) ($L^{2-h}$-$D^{2-be}$),
Exemplification 5-265: Z-$L^{3-e}$-N(-$L^{1-b}$-$D^{1-a}$) ($L^{2-d}$-$D^{2-bf}$),
Exemplification 5-266: Z-$L^{3-e}$-N(-$L^{1-b}$-$D^{1-a}$) ($L^{2-d}$-$D^{2-bg}$),
Exemplification 5-267: Z-$L^{3-e}$-N(-$L^{1-b}$-$D^{1-b}$)($L^{2-e}$-$D^{2-ba}$),
Exemplification 5-268: Z-$L^{3-e}$-N(-$L^{1-b}$-$D^{1-b}$)($L^{2-g}$-$D^{2-bb}$),
Exemplification 5-269: Z-$L^{3-e}$-N(-$L^{1-b}$-$D^{1-b}$)($L^{2-e}$-$D^{2-bc}$),
Exemplification 5-270: Z-$L^{3-e}$-N(-$L^{1-b}$-$D^{1-b}$)($L^{2-e}$-$D^{2-bd}$),
Exemplification 5-271: Z-$L^{3-e}$-N(-$L^{1-b}$-$D^{1-b}$)($L^{2-g}$-$D^{2-be}$),
Exemplification 5-272: Z-$L^{3-e}$-N(-$L^{1-b}$-$D^{1-b}$)($L^{2-b}$-$D^{2-bf}$),
Exemplification 5-273: Z-$L^{3-e}$-N(-$L^{1-b}$-$D^{1-b}$)($L^{2-b}$-$D^{2-bg}$),
Exemplification 5-274: Z-$L^{3-e}$-N(-$L^{1-b}$-$D^{1-b}$)($L^{2-f}$-$D^{2-ba}$),
Exemplification 5-275: Z-$L^{3-e}$-N(-$L^{1-b}$-$D^{1-b}$)($L^{2-h}$-$D^{2-bb}$),

Exemplification 5-276: Z-L$^{3-e}$-N(-L$^{1-b}$-D$^{1-b}$)(L$^{2-f}$-D$^{2-bc}$),
Exemplification 5-277: Z-L$^{3-e}$-N(-L$^{1-b}$-D$^{1-b}$)(L$^{2-f}$-D$^{2-bd}$),
Exemplification 5-278: Z-L$^{3-e}$-N(-L$^{1-b}$-D$^{1-b}$)(L$^{2-h}$-D$^{2-be}$),
Exemplification 5-279: Z-L$^{3-e}$-N(-L$^{1-b}$-D$^{1-b}$)(L$^{2-d}$-D$^{2-bf}$),
Exemplification 5-280: Z-L$^{3-e}$-N(-L$^{1-b}$-D$^{1-b}$)(L$^{2-d}$-D$^{2-bg}$),
Exemplification 5-281: Z-1$^{3-f}$-N(-L$^{1-a}$-D$^{1-a}$)(L$^{2-e}$-D$^{2-ba}$),
Exemplification 5-282: Z-L$^{3-f}$-N(-L$^{1-a}$-D$^{1-a}$)(L$^{2-g}$-D$^{2-bb}$),
Exemplification 5-283: Z-1$^{3-f}$-N(-L$^{1-a}$-D$^{1-a}$)(L$^{2-e}$-D$^{2-bc}$),
Exemplification 5-284: Z-L$^{3-f}$-N(-L$^{1-a}$-D$^{1-a}$)(L$^{2-e}$-D$^{2-bd}$),
Exemplification 5-285: Z-L$^{3-f}$-N(-L$^{1-a}$-D$^{1-a}$)(L$^{2-g}$-D$^{2-be}$),
Exemplification 5-286: Z-L$^{3-f}$-N(-L$^{1-a}$-D$^{1-a}$)(L$^{2-b}$-D$^{2-bf}$),
Exemplification 5-287: Z-L$^{3-f}$-N(-L$^{1-a}$-D$^{1-a}$)(L$^{2-b}$-D$^{2-bg}$),
Exemplification 5-288: Z-L$^{3-f}$-N(-L$^{1-a}$-D$^{1-a}$)(L$^{2-f}$-D$^{2-ba}$),
Exemplification 5-289: Z-L$^{3-f}$-N(-L$^{1-a}$-D$^{1-a}$)(L$^{2-h}$-D$^{2-bb}$),
Exemplification 5-290: Z-L$^{3-f}$-N(-L$^{1-a}$-D$^{1-a}$)(L$^{2-f}$-D$^{2-bc}$),
Exemplification 5-291: Z-L$^{3-f}$-N(-L$^{1-a}$-D$^{1-a}$)(L$^{2-f}$-D$^{2-bd}$),
Exemplification 5-292: Z-L$^{3-f}$-N(-L$^{1-a}$-D$^{1-a}$)(L$^{2-h}$-D$^{2-be}$),
Exemplification 5-293: Z-L$^{3-f}$-N(-L$^{1-a}$-D$^{1-a}$)(L$^{2-d}$-D$^{2-bf}$),
Exemplification 5-294: Z-L$^{3-f}$-N(-L$^{1-a}$-D$^{1-a}$)(L$^{2-d}$-D$^{2-bg}$),
Exemplification 5-295: Z-L$^{3-f}$-N(-L$^{1-a}$-D$^{1-b}$)(L$^{2-e}$-D$^{2-ba}$),
Exemplification 5-296: Z-L$^{3-f}$-N(-L$^{1-a}$-D$^{1-b}$)(L$^{2-g}$-D$^{2-bb}$),
Exemplification 5-297: Z-L$^{3-f}$-N(-L$^{1-a}$-D$^{1-b}$)(L$^{2-e}$-D$^{2-bc}$),
Exemplification 5-298: Z-L$^{3-f}$-N(-L$^{1-a}$-D$^{1-b}$)(L$^{2-e}$-D$^{2-bd}$),
Exemplification 5-299: Z-L$^{3-f}$-N(-L$^{1-a}$-D$^{1-b}$)(L$^{2-g}$-D$^{2-be}$),
Exemplification 5-300: Z-L$^{3-f}$-N(-L$^{1-a}$-D$^{1-b}$)(L$^{2-b}$-D$^{2-bf}$),
Exemplification 5-301: Z-L$^{3-f}$-N(-L$^{1-a}$-D$^{1-b}$)(L$^{2-b}$-D$^{2-bg}$),
Exemplification 5-302: Z-L$^{3-f}$-N(-L$^{1-a}$-D$^{1-b}$)(L$^{2-f}$-D$^{2-ba}$),
Exemplification 5-303: Z-L$^{3-f}$-N(-L$^{1-a}$-D$^{1-b}$)(L$^{2-h}$-D$^{2-bb}$),
Exemplification 5-304: Z-L$^{3-f}$-N(-L$^{1-a}$-D$^{1-b}$)(L$^{2-f}$-D$^{2-bc}$),
Exemplification 5-305: Z-L$^{3-f}$-N(-L$^{1-a}$-D$^{1-b}$)(L$^{2-f}$-D$^{2-bd}$),
Exemplification 5-306: Z-L$^{3-f}$-N(-L$^{1-a}$-D$^{1-b}$)(L$^{2-h}$-D$^{2-be}$),
Exemplification 5-307: Z-L$^{3-f}$-N(-L$^{1-a}$-D$^{1-b}$)(L$^{2-d}$-D$^{2-bf}$),
Exemplification 5-308: Z-L$^{3-f}$-N(-L$^{1-a}$-D$^{1-b}$)(L$^{2-d}$-D$^{2-bg}$),
Exemplification 5-309: Z-L$^{3-f}$-N(-L$^{1-b}$-D$^{1-a}$)(L$^{2-e}$-D$^{2-ba}$),
Exemplification 5-310: Z-L$^{3-f}$-N(-L$^{1-b}$-D$^{1-a}$)(L$^{2-g}$-D$^{2-bb}$),
Exemplification 5-311: Z-L$^{3-f}$-N(-L$^{1-b}$-D$^{1-a}$)(L$^{2-e}$-D$^{2-bc}$),
Exemplification 5-312: Z-L$^{3-f}$-N(-L$^{1-b}$-D$^{1-a}$)(L$^{2-e}$-D$^{2-bd}$),
Exemplification 5-313: Z-L$^{3-f}$-N(-L$^{1-b}$-D$^{1-a}$)(L$^{2-g}$-D$^{2-be}$),
Exemplification 5-314: Z-L$^{3-f}$-N(-L$^{1-b}$-D$^{1-a}$)(L$^{2-b}$-D$^{2-bf}$),
Exemplification 5-315: Z-L$^{3-f}$-N(-L$^{1-b}$-D$^{1-a}$)(L$^{2-b}$-D$^{2-bg}$),
Exemplification 5-316: Z-L$^{3-f}$-N(-L$^{1-b}$-D$^{1-a}$)(L$^{2-f}$-D$^{2-ba}$),
Exemplification 5-317: Z-L$^{3-f}$-N(-L$^{1-b}$-D$^{1-a}$)(L$^{2-h}$-D$^{2-bb}$),
Exemplification 5-318: Z-L$^{3-f}$-N(-L$^{1-b}$-D$^{1-a}$)(L$^{2-f}$-D$^{2-bc}$),
Exemplification 5-319: Z-L$^{3-f}$-N(-L$^{1-b}$-D$^{1-a}$)(L$^{2-f}$-D$^{2-bd}$),
Exemplification 5-320: Z-L$^{3-f}$-N(-L$^{1-b}$-D$^{1-a}$)(L$^{2-h}$-D$^{2-be}$),
Exemplification 5-321: Z-L$^{3-f}$-N(-L$^{1-b}$-D$^{1-a}$)(L$^{2-d}$-D$^{2-bf}$),
Exemplification 5-322: Z-L$^{3-f}$-N(-L$^{1-b}$-D$^{1-a}$)(L$^{2-d}$-D$^{2-bg}$),
Exemplification 5-323: Z-L$^{3-f}$-N(-L$^{1-b}$-D$^{1-b}$)(L$^{2-e}$-D$^{2-ba}$),
Exemplification 5-324: Z-L$^{3-f}$-N(-L$^{1-b}$-D$^{1-b}$)(L$^{2-g}$-D$^{2-bb}$),
Exemplification 5-325: Z-L$^{3-f}$-N(-L$^{1-b}$-D$^{1-b}$)(L$^{2-e}$-D$^{2-bc}$),
Exemplification 5-326: Z-L$^{3-f}$-N(-L$^{1-b}$-D$^{1-b}$)(L$^{2-e}$-D$^{2-bd}$),
Exemplification 5-327: Z-L$^{3-f}$-N(-L$^{1-b}$-D$^{1-b}$)(L$^{2-g}$-D$^{2-be}$),
Exemplification 5-328: Z-L$^{3-f}$-N(-L$^{1-b}$-D$^{1-b}$)(L$^{2-b}$-D$^{2-bf}$),
Exemplification 5-329: Z-L$^{3-f}$-N(-L$^{1-b}$-D$^{1-b}$)(L$^{2-b}$-D$^{2-bg}$),
Exemplification 5-330: Z-L$^{3-f}$-N(-L$^{1-b}$-D$^{1-b}$)(L$^{2-f}$-D$^{2-ba}$),
Exemplification 5-331: Z-L$^{3-f}$-N(-L$^{1-b}$-D$^{1-b}$)(L$^{2-h}$-D$^{2-bb}$),
Exemplification 5-332: Z-L$^{3-f}$-N(-L$^{1-b}$-D$^{1-b}$)(L$^{2-f}$-D$^{2-bc}$),
Exemplification 5-333: Z-L$^{3-f}$-N(-L$^{1-b}$-D$^{1-b}$)(L$^{2-f}$-D$^{2-bd}$),

Exemplification 5-334: Z-$L^{3-f}$-N(-$L^{1-b}$-$D^{1-b}$)($L^{2-h}$-$D^{2-be}$),
Exemplification 5-335: Z-$L^{3-f}$-N(-$L^{1-b}$-$D^{1-b}$)($L^{2-d}$-$D^{2-bf}$),
Exemplification 5-336: Z-$L^{3-f}$-N(-$L^{1-b}$-$D^{1-b}$)($L^{2-d}$-$D^{2-bg}$),
Exemplification 5-337: Z-$L^{3-g}$-N(-$L^{1-a}$-$D^{1-a}$)($L^{2-e}$-$D^{2-ba}$),
Exemplification 5-338: Z-$L^{3-g}$-N(-$L^{1-a}$-$D^{1-a}$)($L^{2-g}$-$D^{2-bb}$),
Exemplification 5-339: Z-$L^{3-g}$-N(-$L^{1-a}$-$D^{1-a}$)($L^{2-e}$-$D^{2-bc}$),
Exemplification 5-340: Z-$L^{3-g}$-N(-$L^{1-a}$-$D^{1-a}$)($L^{2-e}$-$D^{2-bd}$),
Exemplification 5-341: Z-$L^{3-g}$-N(-$L^{1-a}$-$D^{1-a}$)($L^{2-g}$-$D^{2-be}$),
Exemplification 5-342: Z-$L^{3-g}$-N(-$L^{1-a}$-$D^{1-a}$)($L^{2-b}$-$D^{2-bf}$),
Exemplification 5-343: Z-$L^{3-g}$-N(-$L^{1-a}$-$D^{1-a}$)($L^{2-b}$-$D^{2-bg}$),
Exemplification 5-344: Z-$L^{3-g}$-N(-$L^{1-a}$-$D^{1-a}$)($L^{2-f}$-$D^{2-ba}$),
Exemplification 5-345: Z-$L^{3-g}$-N(-$L^{1-a}$-$D^{1-a}$)($L^{2-h}$-$D^{2-bb}$),
Exemplification 5-346: Z-$L^{3-g}$-N(-$L^{1-a}$-$D^{1-a}$)($L^{2-f}$-$D^{2-bc}$),
Exemplification 5-347: Z-$L^{3-g}$-N(-$L^{1-a}$-$D^{1-a}$)($L^{2-f}$-$D^{2-bd}$),
Exemplification 5-348: Z-$L^{3-g}$-N(-$L^{1-a}$-$D^{1-a}$)($L^{2-h}$-$D^{2-be}$),
Exemplification 5-349: Z-$L^{3-g}$-N(-$L^{1-a}$-$D^{1-a}$)($L^{2-d}$-$D^{2-bf}$),
Exemplification 5-350: Z-$L^{3-g}$-N(-$L^{1-a}$-$D^{1-a}$)($L^{2-d}$-$D^{2-bg}$),
Exemplification 5-351: Z-$L^{3-g}$-N(-$L^{1-a}$-$D^{1-b}$)($L^{2-e}$-$D^{2-ba}$),
Exemplification 5-352: Z-$L^{3-g}$-N(-$L^{1-a}$-$D^{1-b}$)($L^{2-g}$-$D^{2-bb}$),
Exemplification 5-353: Z-$L^{3-g}$-N(-$L^{1-a}$-$D^{1-b}$)($L^{2-e}$-$D^{2-bc}$),
Exemplification 5-354: Z-$L^{3-g}$-N(-$L^{1-a}$-$D^{1-b}$)($L^{2-e}$-$D^{2-bd}$),
Exemplification 5-355: Z-$L^{3-g}$-N(-$L^{1-a}$-$D^{1-b}$)($L^{2-g}$-$D^{2-be}$),
Exemplification 5-356: Z-$L^{3-g}$-N(-$L^{1-a}$-$D^{1-b}$)($L^{2-b}$-$D^{2-bf}$),
Exemplification 5-357: Z-$L^{3-g}$-N(-$L^{1-a}$-$D^{1-b}$)($L^{2-b}$-$D^{2-bg}$),
Exemplification 5-358: Z-$L^{3-g}$-N(-$L^{1-a}$-$D^{1-b}$)($L^{2-f}$-$D^{2-ba}$),
Exemplification 5-359: Z-$L^{3-g}$-N(-$L^{1-a}$-$D^{1-b}$)($L^{2-h}$-$D^{2-bb}$),
Exemplification 5-360: Z-$L^{3-g}$-N(-$L^{1-a}$-$D^{1-b}$)($L^{2-f}$-$D^{2-bc}$),
Exemplification 5-361: Z-$L^{3-g}$-N(-$L^{1-a}$-$D^{1-b}$)($L^{2-f}$-$D^{2-bd}$),
Exemplification 5-362: Z-$L^{3-g}$-N(-$L^{1-a}$-$D^{1-b}$)($L^{2-h}$-$D^{2-be}$),
Exemplification 5-363: Z-$L^{3-g}$-N(-$L^{1-a}$-$D^{1-b}$)($L^{2-d}$-$D^{2-bf}$),
Exemplification 5-364: Z-$L^{3-g}$-N(-$L^{1-a}$-$D^{1-b}$)($L^{2-d}$-$D^{2-bg}$),
Exemplification 5-365: Z-$L^{3-g}$-N(-$L^{1-b}$-$D^{1-a}$)($L^{2-e}$-$D^{2-ba}$),
Exemplification 5-366: Z-$L^{3-g}$-N(-$L^{1-b}$-$D^{1-a}$)($L^{2-g}$-$D^{2-bb}$),
Exemplification 5-367: Z-$L^{3-g}$-N(-$L^{1-b}$-$D^{1-a}$)($L^{2-e}$-$D^{2-bc}$),
Exemplification 5-368: Z-$L^{3-g}$-N(-$L^{1-b}$-$D^{1-a}$)($L^{2-e}$-$D^{2-bd}$),
Exemplification 5-369: Z-$L^{3-g}$-N(-$L^{1-b}$-$D^{1-a}$)($L^{2-g}$-$D^{2-be}$),
Exemplification 5-370: Z-$L^{3-g}$-N(-$L^{1-b}$-$D^{1-a}$)($L^{2-b}$-$D^{2-bf}$),
Exemplification 5-371: Z-$L^{3-g}$-N (-$L^{1-b}$-$D^{1-a}$)($L^{2-b}$-$D^{2-bg}$),
Exemplification 5-372: Z-$L^{3-g}$-N(-$L^{1-b}$-$D^{1-a}$)($L^{2-f}$-$D^{2-ba}$),
Exemplification 5-373: Z-$L^{3-g}$-N(-$L^{1-b}$-$D^{1-a}$)($L^{2-h}$-$D^{2-bb}$),
Exemplification 5-374: Z-$L^{3-g}$-N(-$L^{1-b}$-$D^{1-a}$)($L^{2-f}$-$D^{2-bc}$),
Exemplification 5-375: Z-$L^{3-g}$-N(-$L^{1-b}$-$D^{1-a}$)($L^{2-f}$-$D^{2-bd}$),
Exemplification 5-376: Z-$L^{3-g}$-N(-$L^{1-b}$-$D^{1-a}$)($L^{2-h}$-$D^{2-be}$),
Exemplification 5-377: Z-$L^{3-g}$-N(-$L^{1-b}$-$D^{1-a}$)($L^{2-d}$-$D^{2-bf}$),
Exemplification 5-378: Z-$L^{3-g}$-N (-$L^{1-b}$-$D^{1-a}$)($L^{2-d}$-$D^{2-bg}$),
Exemplification 5-379: Z-$L^{3-g}$-N(-$L^{1-b}$-$D^{1-b}$)($L^{2-e}$-$D^{2-ba}$),
Exemplification 5-380: Z-$L^{3-g}$-N (-$L^{1-b}$-$D^{1-b}$)($L^{2-g}$-$D^{2-bb}$),
Exemplification 5-381: Z-$L^{3-g}$-N(-$L^{1-b}$-$D^{1-b}$)($L^{2-e}$-$D^{2-bc}$),
Exemplification 5-382: Z-$L^{3-g}$-N (-$L^{1-b}$-$D^{1-b}$)($L^{2-e}$-$D^{2-bd}$),
Exemplification 5-383: Z-$L^{3-g}$-N(-$L^{1-b}$-$D^{1-b}$)($L^{2-g}$-$D^{2-be}$),
Exemplification 5-384: Z-$L^{3-g}$-N (-$L^{1-b}$-$D^{1-b}$)($L^{2-b}$-$D^{2-bf}$),
Exemplification 5-385: Z-$L^{3-g}$-N(-$L^{1-b}$-$D^{1-b}$)($L^{2-b}$-$D^{2-bg}$),
Exemplification 5-386: Z-$L^{3-g}$-N(-$L^{1-b}$-$D^{1-b}$)($L^{2-f}$-$D^{2-ba}$),
Exemplification 5-387: Z-$L^{3-g}$-N (-$L^{1-b}$-$D^{1-b}$)($L^{2-h}$-$D^{2-bb}$),
Exemplification 5-388: Z-$L^{3-g}$-N(-$L^{1-b}$-$D^{1-b}$)($L^{2-f}$-$D^{2-bc}$),
Exemplification 5-389: Z-$L^{3-g}$-N (-$L^{1-b}$-$D^{1-b}$)($L^{2-f}$-$D^{2-bd}$),
Exemplification 5-390: Z-$L^{3-g}$-N(-$L^{1-b}$-$D^{1-b}$)($L^{2-h}$-$D^{2-be}$),
Exemplification 5-391: Z-$L^{3-g}$-N (-$L^{1-b}$-$D^{1-b}$)($L^{2-d}$-$D^{2-bf}$),

Exemplification 5-392: $Z-L^{3-g}-N(-L^{1-b}-D^{1-b})(L^{2-d}-D^{2-bg})$,
Exemplification 5-393: $Z-L^{3-h}-N(-L^{1-a}-D^{1-a})(L^{2-e}-D^{2-ba})$,
Exemplification 5-394: $Z-L^{3-h}-N(-L^{1-a}-D^{1-a})(L^{2-g}-D^{2-bb})$,
Exemplification 5-395: $Z-L^{3-h}-N(-L^{1-a}-D^{1-a})(L^{2-e}-D^{2-bc})$,
Exemplification 5-396: $Z-L^{3-h}-N(-L^{1-a}-D^{1-a})(L^{2-e}-D^{2-bd})$,
Exemplification 5-397: $Z-L^{3-h}-N(-L^{1-a}-D^{1-a})(L^{2-g}-D^{2-be})$,
Exemplification 5-398: $Z-L^{3-h}-N(-L^{1-a}-D^{1-a})(L^{2-b}-D^{2-bf})$,
Exemplification 5-399: $Z-L^{3-h}-N(-L^{1-a}-D^{1-a})(L^{2-b}-D^{2-bg})$,
Exemplification 5-400: $Z-L^{3-h}-N(-L^{1-a}-D^{1-a})(L^{2-f}-D^{2-ba})$,
Exemplification 5-401: $Z-L^{3-h}-N(-L^{1-a}-D^{1-a})(L^{2-h}-D^{2-bb})$,
Exemplification 5-402: $Z-L^{3-h}-N(-L^{1-a}-D^{1-a})(L^{2-f}-D^{2-bc})$,
Exemplification 5-403: $Z-L^{3-h}-N(-L^{1-a}-D^{1-a})(L^{2-f}-D^{2-bd})$,
Exemplification 5-404: $Z-L^{3-h}-N(-L^{1-a}-D^{1-a})(L^{2-h}-D^{2-be})$,
Exemplification 5-405: $Z-L^{3-h}-N(-L^{1-a}-D^{1-a})(L^{2-d}-D^{2-bf})$,
Exemplification 5-406: $Z-L^{3-h}-N(-L^{1-a}-D^{1-a})(L^{2-d}-D^{2-bg})$,
Exemplification 5-407: $Z-L^{3-h}-N(-L^{1-a}-D^{1-b})(L^{2-e}-D^{2-ba})$,
Exemplification 5-408: $Z-L^{3-h}-N(-L^{1-a}-D^{1-b})(L^{2-g}-D^{2-bb})$,
Exemplification 5-409: $Z-L^{3-h}-N(-L^{1-a}-D^{1-b})(L^{2-e}-D^{2-bc})$,
Exemplification 5-410: $Z-L^{3-h}-N(-L^{1-a}-D^{1-b})(L^{2-e}-D^{2-bd})$,
Exemplification 5-411: $Z-L^{3-h}-N(-L^{1-a}-D^{1-b})(L^{2-g}-D^{2-be})$,
Exemplification 5-412: $Z-L^{3-h}-N(-L^{1-a}-D^{1-b})(L^{2-b}-D^{2-bf})$,
Exemplification 5-413: $Z-L^{3-h}-N(-L^{1-a}-D^{1-b})(L^{2-b}-D^{2-bg})$,
Exemplification 5-414: $Z-L^{3-h}-N(-L^{1-a}-D^{1-b})(L^{2-f}-D^{2-ba})$,
Exemplification 5-415: $Z-L^{3-h}-N(-L^{1-a}-D^{1-b})(L^{2-h}-D^{2-bb})$,
Exemplification 5-416: $Z-L^{3-h}-N(-L^{1-a}-D^{1-b})(L^{2-f}-D^{2-bc})$,
Exemplification 5-417: $Z-L^{3-h}-N(-L^{1-a}-D^{1-b})(L^{2-f}-D^{2-bd})$,
Exemplification 5-418: $Z-L^{3-h}-N(-L^{1-a}-D^{1-b})(L^{2-h}-D^{2-be})$,
Exemplification 5-419: $Z-L^{3-h}-N(-L^{1-a}-D^{1-b})(L^{2-d}-D^{2-bf})$,
Exemplification 5-420: $Z-L^{3-h}-N(-L^{1-a}-D^{1-b})(L^{2-d}-D^{2-bg})$,
Exemplification 5-421: $Z-L^{3-h}-N(-L^{1-b}-D^{1-a})(L^{2-e}-D^{2-ba})$,
Exemplification 5-422: $Z-L^{3-h}-N(-L^{1-b}-D^{1-a})(L^{2-g}-D^{2-bb})$,
Exemplification 5-423: $Z-L^{3-h}-N(-L^{1-b}-D^{1-a})(L^{2-e}-D^{2-bc})$,
Exemplification 5-424: $Z-L^{3-h}-N(-L^{1-b}-D^{1-a})(L^{2-e}-D^{2-bd})$,
Exemplification 5-425: $Z-L^{3-h}-N(-L^{1-b}-D^{1-a})(L^{2-g}-D^{2-be})$,
Exemplification 5-426: $Z-L^{3-h}-N(-L^{1-b}-D^{1-a})(L^{2-b}-D^{2-bf})$,
Exemplification 5-427: $Z-L^{3-h}-N(-L^{1-b}-D^{1-a})(L^{2-b}-D^{2-bg})$,
Exemplification 5-428: $Z-L^{3-h}-N(-L^{1-b}-D^{1-a})(L^{2-f}-D^{2-ba})$,
Exemplification 5-429: $Z-L^{3-h}-N(-L^{1-b}-D^{1-a})(L^{2-h}-D^{2-bb})$,
Exemplification 5-430: $Z-L^{3-h}-N(-L^{1-b}-D^{1-a})(L^{2-f}-D^{2-bc})$,
Exemplification 5-431: $Z-L^{3-h}-N(-L^{1-b}-D^{1-a})(L^{2-f}-D^{2-bd})$,
Exemplification 5-432: $Z-L^{3-h}-N(-L^{1-b}-D^{1-a})(L^{2-h}-D^{2-be})$,
Exemplification 5-433: $Z-L^{3-h}-N(-L^{1-b}-D^{1-a})(L^{2-d}-D^{2-bf})$,
Exemplification 5-434: $Z-L^{3-h}-N(-L^{1-b}-D^{1-a})(L^{2-d}-D^{2-bg})$,
Exemplification 5-435: $Z-L^{3-h}-N(-L^{1-b}-D^{1-b})(L^{2-e}-D^{2-ba})$,
Exemplification 5-436: $Z-L^{3-h}-N(-L^{1-b}-D^{1-b})(L^{2-g}-D^{2-bb})$,
Exemplification 5-437: $Z-L^{3-h}-N(-L^{1-b}-D^{1-b})(L^{2-e}-D^{2-bc})$,
Exemplification 5-438: $Z-L^{3-h}-N(-L^{1-b}-D^{1-b})(L^{2-e}-D^{2-bd})$,
Exemplification 5-439: $Z-L^{3-h}-N(-L^{1-b}-D^{1-b})(L^{2-g}-D^{2-be})$,
Exemplification 5-440: $Z-L^{3-h}-N(-L^{1-b}-D^{1-b})(L^{2-b}-D^{2-bf})$,
Exemplification 5-441: $Z-L^{3-h}-N(-L^{1-b}-D^{1-b})(L^{2-b}-D^{2-bg})$,
Exemplification 5-442: $Z-L^{3-h}-N(-L^{1-b}-D^{1-b})(L^{2-f}-D^{2-ba})$,
Exemplification 5-443: $Z-L^{3-h}-N(-L^{1-b}-D^{1-b})(L^{2-h}-D^{2-bb})$,
Exemplification 5-444: $Z-L^{3-h}-N(-L^{1-b}-D^{1-b})(L^{2-f}-D^{2-bc})$,
Exemplification 5-445: $Z-L^{3-h}-N(-L^{1-b}-D^{1-b})(L^{2-f}-D^{2-bd})$,
Exemplification 5-446: $Z-L^{3-h}-N(-L^{1-b}-D^{1-b})(L^{2-h}-D^{2-be})$,
Exemplification 5-447: $Z-L^{3-h}-N(-L^{1-b}-D^{1-b})(L^{2-d}-D^{2-bf})$,
Exemplification 5-448: $Z-L^{3-h}-N(-L^{1-b}-D^{1-b})(L^{2-d}-D^{2-bg})$,
Exemplification 5-449: $Z-L^{3-i}-N(-L^{1-a}-D^{1-a})(L^{2-e}-D^{2-ba})$,

Exemplification 5-450: Z-$L^{3-i}$-N(-$L^{1-a}$-$D^{1-a}$)($L^{2-g}$-$D^{2-bb}$),
Exemplification 5-451: Z-$L^{3-i}$-N(-$L^{1-a}$-$D^{1-a}$)($L^{2-e}$-$D^{2-bc}$),
Exemplification 5-452: Z-$L^{3-i}$-N(-$L^{1-a}$-$D^{1-a}$)($L^{2-e}$-$D^{2-bd}$),
Exemplification 5-453: Z-$L^{3-i}$-N(-$L^{1-a}$-$D^{1-a}$)($L^{2-g}$-$D^{2-be}$),
Exemplification 5-454: Z-$L^{3-i}$-N(-$L^{1-a}$-$D^{1-a}$)($L^{2-b}$-$D^{2-bf}$),
Exemplification 5-455: Z-$L^{3-i}$-N(-$L^{1-a}$-$D^{1-a}$)($L^{2-b}$-$D^{2-bg}$),
Exemplification 5-456: Z-$L^{3-i}$-N(-$L^{1-a}$-$D^{1-a}$)($L^{2-f}$-$D^{2-ba}$),
Exemplification 5-457: Z-$L^{3-i}$-N(-$L^{1-a}$-$D^{1-a}$)($L^{2-h}$-$D^{2-bb}$),
Exemplification 5-458: Z-$L^{3-i}$-N(-$L^{1-a}$-$D^{1-a}$)($L^{2-f}$-$D^{2-bc}$),
Exemplification 5-459: Z-$L^{3-i}$-N(-$L^{1-a}$-$D^{1-a}$)($L^{2-f}$-$D^{2-bd}$),
Exemplification 5-460: Z-$L^{3-i}$-N(-$L^{1-a}$-$D^{1-a}$)($L^{2-h}$-$D^{2-be}$),
Exemplification 5-461: Z-$L^{3-i}$-N(-$L^{1-a}$-$D^{1-a}$)($L^{2-d}$-$D^{2-bf}$),
Exemplification 5-462: Z-$L^{3-i}$-N(-$L^{1-a}$-$D^{1-a}$)($L^{2-d}$-$D^{2-bg}$),
Exemplification 5-463: Z-$L^{3-i}$-N(-$L^{1-a}$-$D^{1-b}$)($L^{2-e}$-$D^{2-ba}$),
Exemplification 5-464: Z-$L^{3-i}$-N(-$L^{1-a}$-$D^{1-b}$)($L^{2-g}$-$D^{2-bb}$),
Exemplification 5-465: Z-$L^{3-i}$-N(-$L^{1-a}$-$D^{1-b}$)($L^{2-e}$-$D^{2-bc}$),
Exemplification 5-466: Z-$L^{3-i}$-N(-$L^{1-a}$-$D^{1-b}$)($L^{2-e}$-$D^{2-bd}$),
Exemplification 5-467: Z-$L^{3-i}$-N(-$L^{1-a}$-$D^{1-b}$)($L^{2-g}$-$D^{2-be}$),
Exemplification 5-468: Z-$L^{3-i}$-N(-$L^{1-a}$-$D^{1-b}$)($L^{2-b}$-$D^{2-bf}$),
Exemplification 5-469: Z-$L^{3-i}$-N(-$L^{1-a}$-$D^{1-b}$)($L^{2-b}$-$D^{2-bg}$),
Exemplification 5-470: Z-$L^{3-i}$-N(-$L^{1-a}$-$D^{1-b}$)($L^{2-f}$-$D^{2-ba}$),
Exemplification 5-471: Z-$L^{3-i}$-N(-$L^{1-a}$-$D^{1-b}$)($L^{2-h}$-$D^{2-bb}$),
Exemplification 5-472: Z-$L^{3-i}$-N(-$L^{1-a}$-$D^{1-b}$)($L^{2-f}$-$D^{2-bc}$),
Exemplification 5-473: Z-$L^{3-i}$-N(-$L^{1-a}$-$D^{1-b}$)($L^{2-f}$-$D^{2-bd}$),
Exemplification 5-474: Z-$L^{3-i}$-N(-$L^{1-a}$-$D^{1-b}$)($L^{2-h}$-$D^{2-be}$),
Exemplification 5-475: Z-$L^{3-i}$-N(-$L^{1-a}$-$D^{1-b}$)($L^{2-d}$-$D^{2-bf}$),
Exemplification 5-476: Z-$L^{3-i}$-N(-$L^{1-a}$-$D^{1-b}$)($L^{2-d}$-$D^{2-bg}$),
Exemplification 5-477: Z-$L^{3-i}$-N(-$L^{1-b}$-$D^{1-a}$)($L^{2-e}$-$D^{2-ba}$),
Exemplification 5-478: Z-$L^{3-i}$-N(-$L^{1-b}$-$D^{1-a}$)($L^{2-g}$-$D^{2-bb}$),
Exemplification 5-479: Z-$L^{3-i}$-N(-$L^{1-b}$-$D^{1-a}$)($L^{2-e}$-$D^{2-bc}$),
Exemplification 5-480: Z-$L^{3-i}$-N(-$L^{1-b}$-$D^{1-a}$)($L^{2-e}$-$D^{2-bd}$),
Exemplification 5-481: Z-$L^{3-i}$-N(-$L^{1-b}$-$D^{1-a}$)($L^{2-g}$-$D^{2-be}$),
Exemplification 5-482: Z-$L^{3-i}$-N(-$L^{1-b}$-$D^{1-a}$)($L^{2-b}$-$D^{2-bf}$),
Exemplification 5-483: Z-$L^{3-i}$-N(-$L^{1-b}$-$D^{1-a}$)($L^{2-b}$-$D^{2-bg}$),
Exemplification 5-484: Z-$L^{3-i}$-N(-$L^{1-b}$-$D^{1-a}$)($L^{2-f}$-$D^{2-ba}$),
Exemplification 5-485: Z-$L^{3-i}$-N(-$L^{1-b}$-$D^{1-a}$)($L^{2-h}$-$D^{2-bb}$),
Exemplification 5-486: Z-$L^{3-i}$-N(-$L^{1-b}$-$D^{1-a}$)($L^{2-f}$-$D^{2-bc}$),
Exemplification 5-487: Z-$L^{3-i}$-N(-$L^{1-b}$-$D^{1-a}$)($L^{2-f}$-$D^{2-bd}$),
Exemplification 5-488: Z-$L^{3-i}$-N(-$L^{1-b}$-$D^{1-a}$)($L^{2-h}$-$D^{2-be}$),
Exemplification 5-489: Z-$L^{3-i}$-N(-$L^{1-b}$-$D^{1-a}$)($L^{2-d}$-$D^{2-bf}$),
Exemplification 5-490: Z-$L^{3-i}$-N(-$L^{1-b}$-$D^{1-a}$)($L^{2-d}$-$D^{2-bg}$),
Exemplification 5-491: Z-$L^{3-i}$-N(-$L^{1-b}$-$D^{1-b}$)($L^{2-e}$-$D^{2-ba}$),
Exemplification 5-492: Z-$L^{3-i}$-N(-$L^{1-b}$-$D^{1-b}$)($L^{2-g}$-$D^{2-bb}$),
Exemplification 5-493: Z-$L^{3-i}$-N(-$L^{1-b}$-$D^{1-b}$)($L^{2-e}$-$D^{2-bc}$),
Exemplification 5-494: Z-$L^{3-i}$-N(-$L^{1-b}$-$D^{1-b}$)($L^{2-e}$-$D^{2-bd}$),
Exemplification 5-495: Z-$L^{3-i}$-N(-$L^{1-b}$-$D^{1-b}$)($L^{2-g}$-$D^{2-be}$),
Exemplification 5-496: Z-$L^{3-i}$-N(-$U^{1-b}$-$D^{1-b}$)($L^{2-b}$-$D^{2-bf}$),
Exemplification 5-497: Z-$L^{3-i}$-N (-$L^{1-b}$-$D^{1-b}$) ($L^{2-b}$-$D^{2-bg}$),
Exemplification 5-498: Z-$L^{3-i}$-N(-$L^{1-b}$-$D^{1-b}$)($L^{2-f}$-$D^{2-ba}$),
Exemplification 5-499: Z-$L^{3-i}$-N(-$L^{1-b}$-$D^{1-b}$)($L^{2-h}$-$D^{2-bb}$),
Exemplification 5-500: Z-$L^{3-i}$-N(-$L^{1-b}$-$D^{1-b}$)($L^{2-f}$-$D^{2-bc}$),
Exemplification 5-501: Z-$L^{3-i}$-N(-$L^{1-b}$-$D^{1-b}$)($L^{2-f}$-$D^{2-bd}$),
Exemplification 5-502: Z-$L^{3-i}$-N(-$L^{1-b}$-$D^{1-b}$)($L^{2-h}$-$D^{2-be}$),
Exemplification 5-503: Z-$L^{3-i}$-N(-$L^{1-b}$-$D^{1-b}$)($L^{2-d}$-$D^{2-bf}$),
Exemplification 5-504: Z-$L^{3-i}$-N(-$L^{1-b}$-$D^{1-b}$)($L^{2-d}$-$D^{2-bg}$).

The present invention also provides exemplification precursor compounds (Exemplification 6-1 to Exemplification 6-936) {in the following formula, Z, $L^{1-a}$ to $L^{1-b}$, $L^{2-b}$, $L^{2-d}$, $L^{2-i}$ to $L^{2-n}$, $L^{3-a}$ to $L^{3-1}$, $D^{1-a}$ to $D^{1-b}$, and $D^{2-ca}$ to $D^{2-ch}$ are as

defined above}.

Exemplification 6-1: Z-$L^{3-a}$-N(-$L^{1-a}$-$D^{1-a}$)($L^{2-i}$-$D^{2-ca}$),
Exemplification 6-2: Z-$L^{3-a}$-N(-$L^{1-a}$-$D^{1-a}$)($L^{2-i}$-$D^{2-cc}$),
Exemplification 6-3: Z-$L^{3-a}$-N(-$L^{1-a}$-$D^{1-a}$)($L^{2-i}$-$D^{2-cd}$),
Exemplification 6-4: Z-$L^{3-a}$-N(-$L^{1-a}$-$D^{1-a}$)($L^{2-i}$-$D^{2-ce}$),
Exemplification 6-5: Z-$L^{3-a}$-N(-$L^{1-a}$-$D^{1-a}$)($L^{2-i}$-$D^{2-cf}$),
Exemplification 6-6: Z-$L^{3-a}$-N(-$L^{1-a}$-$D^{1-a}$)($L^{2-j}$-$D^{2-ca}$),
Exemplification 6-7: Z-$L^{3-a}$-N(-$L^{1-a}$-$D^{1-a}$)($L^{2-j}$-$D^{2-cc}$),
Exemplification 6-8: Z-$L^{3-a}$-N(-$L^{1-a}$-$D^{1-a}$)($L^{2-j}$-$D^{2-cd}$),
Exemplification 6-9: Z-$L^{3-a}$-N(-$L^{1-a}$-$D^{1-a}$)($L^{2-3}$-$D^{2-ce}$),
Exemplification 6-10: Z-$L^{3-2}$-N(-$L^{1-a}$-$D^{1-a}$)($L^{2-j}$-$D^{2-cf}$),
Exemplification 6-11: Z-$L^{3-a}$-N(-$L^{1-a}$-$D^{1-a}$)($L^{2-k}$-$D^{2-cb}$),
Exemplification 6-12: Z-$L^{3-a}$-N(-$L^{1-a}$-$D^{1-a}$)($L^{2-1}$-$D^{2-ca}$),
Exemplification 6-13: Z-$L^{3-a}$-N(-$L^{1-a}$-$D^{1-a}$)($L^{2-1}$-$D^{2-cc}$),
Exemplification 6-14 : Z-$L^{3-a}$-N(-$L^{1-a}$-$D^{1-a}$)($L^{2-1}$-$D^{2-cd}$),
Exemplification 6-15: Z-$L^{3-a}$-N(-$L^{1-a}$-$D^{1-a}$)($L^{2-1}$-$D^{2-ce}$),
Exemplification 6-16: Z-$L^{3-a}$-N(-$L^{1-a}$-$D^{1-a}$)($L^{2-1}$-$D^{2-cf}$),
Exemplification 6-17 : Z-$L^{3-a}$-N(-$L^{1-a}$-$D^{1-a}$)($L^{2-m}$-$D^{2-ca}$),
Exemplification 6-18 : Z-$L^{3-a}$-N(-$L^{1-a}$-$D^{1-a}$)($L^{2-m}$-$D^{2-cc}$),
Exemplification 6-19: Z-$L^{3-a}$-N(-$L^{1-a}$-$D^{1-a}$)($L^{2-m}$-$D^{2-cd}$),
Exemplification 6-20: Z-$L^{3-a}$-N(-$L^{1-a}$-$D^{1-a}$)($L^{2-m}$-$D^{2-ce}$),
Exemplification 6-21: Z-$L^{3-a}$-N(-$L^{1-a}$-$D^{1-a}$)($L^{2-m}$-$D^{2-cf}$),
Exemplification 6-22: Z-$L^{3-a}$-N(-$L^{1-a}$-$D^{1-a}$)($L^{2-n}$-$D^{2-cb}$),
Exemplification 6-23: Z-$L^{3-a}$-N(-$L^{1-a}$-$D^{1-a}$)($L^{2-b}$-$D^{2-cg}$),
Exemplification 6-24: Z-$L^{3-a}$-N(-$L^{1-a}$-$D^{1-a}$)($L^{2-b}$-$D^{2-ch}$),
Exemplification 6-25: Z-$L^{3-a}$-N(-$L^{1-a}$-$D^{1-a}$)($L^{2-d}$-$D^{2-cg}$),
Exemplification 6-26: Z-$L^{3-a}$-N(-$L^{1-a}$-$D^{1-a}$)($L^{2-d}$-$D^{2-ch}$),
Exemplification 6-27: Z-$L^{3-a}$-N(-$L^{1-a}$-$D^{1-b}$)($L^{2-i}$-$D^{2-ca}$),
Exemplification 6-28: Z-$L^{3-a}$-N(-$L^{1-a}$-$D^{1-b}$)($L^{2-i}$-$D^{2-cc}$),
Exemplification 6-29: Z-$L^{3-a}$-N(-$L^{1-a}$-$D^{1-b}$)($L^{2-i}$-$D^{2-cd}$),
Exemplification 6-30: Z-$L^{3-a}$-N(-$L^{1-a}$-$D^{1-b}$)($L^{2-i}$-$D^{2-ce}$),
Exemplification 6-31: Z-$L^{3-a}$-N(-$L^{1-a}$-$D^{1-b}$)($L^{2-i}$-$D^{2-cf}$),
Exemplification 6-32: Z-$L^{3-a}$-N(-$L^{1-a}$-$D^{1-b}$)($L^{2-3}$-$D^{2-ca}$),
Exemplification 6-33: Z-$L^{3-a}$-N(-$L^{1-a}$-$D^{1-b}$)($L^{2-j}$-$D^{2-cc}$),
Exemplification 6-34 : Z-$L^{3-a}$-N(-$L^{1-a}$-$D^{1-b}$)($L^{2-j}$-$D^{2-cd}$),
Exemplification 6-35: Z-$L^{3-a}$-N(-$L^{1-a}$-$D^{1-b}$)($L^{2-j}$-$D^{2-ce}$),
Exemplification 6-36: Z-$L^{3-a}$-N(-$L^{1-a}$-$D^{1-b}$)($L^{2-j}$-$D^{2-cf}$),
Exemplification 6-37 : Z-$L^{3-a}$-N(-$L^{1-a}$-$D^{1-b}$)($L^{2-k}$-$D^{2-cb}$),
Exemplification 6-38 : Z-$L^{3-a}$-N(-$L^{1-a}$-$D^{1-b}$)($L^{2-1}$-$D^{2-ca}$),
Exemplification 6-39: Z-$L^{3-a}$-N(-$L^{1-a}$-$D^{1-b}$)($L^{2-1}$-$D^{2-cc}$),
Exemplification 6-40: Z-$L^{3-a}$-N(-$L^{1-a}$-$D^{1-b}$)($L^{2-1}$-$D^{2-cd}$),
Exemplification 6-41: Z-$L^{3-a}$-N(-$L^{1-a}$-$D^{1-b}$)($L^{2-1}$-$D^{2-ce}$),
Exemplification 6-42: Z-$L^{3-a}$-N(-$L^{1-a}$-$D^{1-b}$)($L^{2-1}$-$D^{2-cf}$),
Exemplification 6-43: Z-$L^{3-a}$-N(-$L^{1-a}$-$D^{1-b}$)($L^{2-m}$-$D^{2-ca}$),
Exemplification 6-44: Z-$L^{3-a}$-N(-$L^{1-a}$-$D^{1-b}$)($L^{2-m}$-$D^{2-cc}$),
Exemplification 6-45: Z-$L^{3-a}$-N(-$L^{1-a}$-$D^{1-b}$)($L^{2-m}$-$D^{2-cd}$),
Exemplification 6-46: Z-$L^{3-a}$-N(-$L^{1-a}$-$D^{1-b}$)($L^{2-m}$-$D^{2-ce}$),
Exemplification 6-47: Z-$L^{3-a}$-N(-$L^{1-a}$-$D^{1-b}$)($L^{2-m}$-$D^{2-cf}$),
Exemplification 6-48: Z-$L^{3-a}$-N(-$L^{1-a}$-$D^{1-b}$)($L^{2-n}$-$D^{2-cb}$),
Exemplification 6-49: Z-$L^{3-a}$-N(-$L^{1-a}$-$D^{1-b}$)($L^{2-b}$-$D^{2-cg}$),
Exemplification 6-50: Z-$L^{3-a}$-N(-$L^{1-a}$-$D^{1-b}$)($L^{2-b}$-$D^{2-ch}$),
Exemplification 6-51: Z-$L^{3-a}$-N(-$L^{1-a}$-$D^{1-b}$)($L^{2-d}$-$D^{2-cg}$),
Exemplification 6-52: Z-$L^{3-a}$-N(-$L^{1-a}$-$D^{1-b}$)($L^{2-d}$-$D^{2-ch}$),
Exemplification 6-53: Z-$1^{3-a}$-N(-$L^{1-b}$-$D^{1-a}$)($L^{2-i}$-$D^{2-ca}$),
Exemplification 6-54: Z-$L^{3-a}$-N(-$L^{1-b}$-$D^{1-a}$)($L^{2-i}$-$D^{2-cc}$),
Exemplification 6-55: Z-$L^{3-a}$-N(-$L^{1-b}$-$D^{1-a}$)($L^{2-i}$-$D^{2-cd}$),
Exemplification 6-56: Z-$L^{3-a}$-N(-$L^{1-b}$-$D^{1-a}$)($L^{2-i}$-$D^{2-ce}$),

Exemplification 6-57: Z-$L^{3-a}$-N(-$L^{1-b}$-$D^{1-a}$)($L^{2-i}$-$D^{2-cf}$),

Exemplification 6-58: Z-$L^{3-a}$-N(-$L^{1-b}$-$D^{1-a}$)($L^{2-3}$-$D^{2-ca}$),

Exemplification 6-59: Z-$L^{3-a}$-N(-$L^{1-b}$-$D^{1-a}$)($L^{2-j}$-$D^{2-cc}$),

Exemplification 6-60: Z-$L^{3-a}$-N(-$L^{1-b}$-$D^{1-a}$)($L^{2-j}$-$D^{2-cd}$),

Exemplification 6-61: Z-$L^{3-a}$-N(-$L^{1-b}$-$D^{1-2}$)($L^{2-3}$-$D^{2-ce}$),

Exemplification 6-62: Z-$L^{3-a}$-N(-$L^{1-b}$-$D^{1-a}$)($L^{2-j}$-$D^{2-cf}$),

Exemplification 6-63: Z-$L^{3-a}$-N(-$L^{1-b}$-$D^{1-a}$)($L^{2-k}$-$D^{2-cb}$),

Exemplification 6-64: Z-$L^{3-a}$-N(-$L^{1-b}$-$D^{1-a}$)($L^{2-1}$-$D^{2-ca}$),

Exemplification 6-65: Z-$L^{3-a}$-N(-$L^{1-b}$-$D^{1-a}$)($L^{2-1}$-$D^{2-cc}$),

Exemplification 6-66: Z-$L^{3-a}$-N(-$L^{1-b}$-$D^{1-a}$)($L^{2-1}$-$D^{2-cd}$),

Exemplification 6-67 : Z-$L^{3-a}$-N(-$L^{1-b}$-$D^{1-a}$)($L^{2-1}$-$D^{2-ce}$),

Exemplification 6-68 : Z-$L^{3-a}$-N(-$L^{1-b}$-$D^{1-a}$)($L^{2-1}$-$D^{2-cf}$),

Exemplification 6-69: Z-$L^{3-a}$-N(-$L^{1-b}$-$D^{1-a}$)($L^{2-m}$-$D^{2-ca}$),

Exemplification 6-70: Z-$L^{3-a}$-N(-$L^{1-b}$-$D^{1-a}$)($L^{2-m}$-$D^{2-cc}$),

Exemplification 6-71: Z-$L^{3-a}$-N(-$L^{1-b}$-$D^{1-a}$)($L^{2-m}$-$D^{2-cd}$),

Exemplification 6-72: Z-$L^{3-a}$-N(-$L^{1-b}$-$D^{1-a}$)($L^{2-m}$-$D^{2-ce}$),

Exemplification 6-73: Z-$L^{3-a}$-N(-$L^{1-b}$-$D^{1-a}$)($L^{2-m}$-$D^{2-cf}$),

Exemplification 6-74: Z-$L^{3-a}$-N(-$L^{1-b}$-$D^{1-a}$)($L^{2-n}$-$D^{2-cb}$),

Exemplification 6-75: Z-$L^{3-a}$-N(-$L^{1-b}$-$D^{1-a}$)($L^{2-b}$-$D^{2-cg}$),

Exemplification 6-76: Z-$L^{3-a}$-N(-$L^{1-b}$-$D^{1-a}$)($L^{2-b}$-$D^{2-ch}$),

Exemplification 6-77 : Z-$L^{3-a}$-N(-$L^{1-b}$-$D^{1-2}$)($L^{2-d}$-$D^{2-cg}$),

Exemplification 6-78 : Z-$L^{3-a}$-N(-$L^{1-b}$-$D^{1-2}$)($L^{2-d}$-$D^{2-ch}$),

Exemplification 6-79: Z-$L^{3-a}$-N(-$L^{1-b}$-$D^{1-b}$)($L^{2-i}$-$D^{2-ca}$),

Exemplification 6-80: Z-$L^{3-a}$-N(-$L^{1-b}$-$D^{1-b}$)($L^{2-i}$-$D^{2-cc}$),

Exemplification 6-81: Z-$L^{3-a}$-N(-$L^{1-b}$-$D^{1-b}$)($L^{2-i}$-$D^{2-cd}$),

Exemplification 6-82: Z-$L^{3-a}$-N(-$L^{1-b}$-$D^{1-b}$)($L^{2-i}$-$D^{2-ce}$),

Exemplification 6-83: Z-$L^{3-a}$-N(-$L^{1-b}$-$D^{1-b}$)($L^{2-i}$-$D^{2-cf}$),

Exemplification 6-84: Z-$L^{3-a}$-N(-$L^{1-b}$-$D^{1-b}$)($L^{2-3}$-$D^{2-ca}$),

Exemplification 6-85: Z-$L^{3-a}$-N(-$L^{1-b}$-$D^{1-b}$)($L^{2-j}$-$D^{2-cc}$),

Exemplification 6-86: Z-$L^{3-a}$-N(-$L^{1-b}$-$D^{1-b}$)($L^{2-j}$-$D^{2-ca}$),

Exemplification 6-87: Z-$L^{3-a}$-N(-$L^{1-b}$-$D^{1-b}$)($L^{2-j}$-$D^{2-ce}$),

Exemplification 6-88: Z-$L^{3-a}$-N(-$L^{1-b}$-$D^{1-b}$)($L^{2-j}$-$D^{2-cf}$),

Exemplification 6-89: Z-$L^{3-a}$-N(-$L^{1-b}$-$D^{1-b}$)($L^{2-k}$-$D^{2-cb}$),

Exemplification 6-90: Z-$L^{3-a}$-N(-$L^{1-b}$-$D^{1-b}$)($L^{2-1}$-$D^{2-ca}$),

Exemplification 6-91: Z-$L^{3-a}$-N(-$L^{1-b}$-$D^{1-b}$)($L^{2-1}$-$D^{2-cc}$),

Exemplification 6-92: Z-$L^{3-a}$-N(-$L^{1-b}$-$D^{1-b}$)($L^{2-1}$-$D^{2-cd}$),

Exemplification 6-93: Z-$L^{3-a}$-N(-$L^{1-b}$-$D^{1-b}$)($L^{2-1}$-$D^{2-ce}$),

Exemplification 6-94 : Z-$L^{3-a}$-N(-$L^{1-b}$-$D^{1-b}$)($L^{2-1}$-$D^{2-cf}$),

Exemplification 6-95: Z-$L^{3-a}$-N(-$L^{1-b}$-$D^{1-b}$)($L^{2-m}$-$D^{2-ca}$),

Exemplification 6-96: Z-$L^{3-a}$-N(-$L^{1-b}$-$D^{1-b}$)($L^{2-m}$-$D^{2-cc}$),

Exemplification 6-97: Z-$L^{3-a}$-N(-$L^{1-b}$-$D^{1-b}$)($L^{2-m}$-$D^{2-cd}$),

Exemplification 6-98 : Z-$L^{3-a}$-N(-$L^{1-b}$-$D^{1-b}$)($L^{2-m}$-$D^{2-ce}$),

Exemplification 6-99: Z-$L^{3-a}$-N(-$L^{1-b}$-$D^{1-b}$)($L^{2-m}$-$D^{2-cf}$),

Exemplification 6-100: Z-$L^{3-a}$-N(-$L^{1-b}$-$D^{1-b}$)($L^{2-n}$-$D^{2-cb}$),

Exemplification 6-101: Z-$L^{3-a}$-N(-$L^{1-b}$-$D^{1-b}$)($L^{2-b}$-$D^{2-cg}$),

Exemplification 6-102: Z-$L^{3-a}$-N(-$L^{1-b}$-$D^{1-b}$)($L^{2-b}$-$D^{2-ch}$),

Exemplification 6-103: Z-$L^{3-a}$-N(-$L^{1-b}$-$D^{1-b}$)($L^{2-d}$-$D^{2-cg}$),

Exemplification 6-104: Z-$L^{3-a}$-N(-$L^{1-b}$-$D^{1-b}$)($L^{2-d}$-$D^{2-ch}$),

Exemplification 6-105: Z-$L^{3-b}$-N(-$L^{1-a}$-$D^{1-a}$)($L^{2-i}$-$D^{2-ca}$),

Exemplification 6-106: Z-$L^{3-b}$-N(-$L^{1-a}$-$D^{1-a}$)($L^{2-i}$-$D^{2-cc}$),

Exemplification 6-107: Z-$L^{3-b}$-N(-$L^{1-a}$-$D^{1-a}$)($L^{2-i}$-$D^{2-cd}$),

Exemplification 6-108: Z-$L^{3-b}$-N(-$L^{1-a}$-$D^{1-a}$)($L^{2-i}$-$D^{2-ce}$),

Exemplification 6-109: Z-$L^{3-b}$-N(-$L^{1-a}$-$D^{1-a}$)($L^{2-i}$-$D^{2-cf}$),

Exemplification 6-110: Z-$L^{3-b}$-N(-$L^{1-a}$-$D^{1-a}$)($L^{2-3}$-$D^{2-ca}$),

Exemplification 6-111: Z-$L^{3-b}$-N(-$L^{1-a}$-$D^{1-a}$)($L^{2-j}$-$D^{2-cc}$),

Exemplification 6-112: Z-$L^{3-b}$-N(-$L^{1-a}$-$D^{1-a}$)($L^{2-j}$-$D^{2-cd}$),

Exemplification 6-113: Z-$L^{3-b}$-N(-$L^{1-a}$-$D^{1-a}$)($L^{2-j}$-$D^{2-ce}$),

Exemplification 6-114 : Z-$L^{3-b}$-N(-$L^{1-a}$-$D^{1-a}$)($L^{2-j}$-$D^{2-cf}$),

Exemplification 6-115: $Z-L^{3-b}-N(-L^{1-a}-D^{1-a})(L^{2-k}-D^{2-cb})$,
Exemplification 6-116: $Z-L^{3-b}-N(-L^{1-a}-D^{1-a})(L^{2-1}-D^{2-ca})$,
Exemplification 6-117: $Z-L^{3-b}-N(-L^{1-a}-D^{1-a})(L^{2-1}-D^{2-cc})$,
Exemplification 6-118: $Z-L^{3-b}-N(-L^{1-a}-D^{1-a})(L^{2-1}-D^{2-cd})$,
Exemplification 6-119: $Z-L^{3-b}-N(-L^{1-a}-D^{1-a})(L^{2-1}-D^{2-ce})$,
Exemplification 6-120: $Z-L^{3-b}-N(-L^{1-a}-D^{1-a})(L^{2-1}-D^{2-cf})$,
Exemplification 6-121: $Z-L^{3-b}-N(-L^{1-a}-D^{1-a})(L^{2-m}-D^{2-ca})$,
Exemplification 6-122: $Z-L^{3-b}-N(-L^{1-a}-D^{1-a})(L^{2-m}-D^{2-cc})$,
Exemplification 6-123: $Z-L^{3-b}-N(-L^{1-a}-D^{1-a})(L^{2-m}-D^{2-cd})$,
Exemplification 6-124: $Z-L^{3-b}-N(-L^{1-a}-D^{1-a})(L^{2-m}-D^{2-ce})$,
Exemplification 6-125: $Z-L^{3-b}-N(-L^{1-a}-D^{1-a})(L^{2-m}-D^{2-cf})$,
Exemplification 6-126: $Z-L^{3-b}-N(-L^{1-a}-D^{1-a})(L^{2-n}-D^{2-cb})$,
Exemplification 6-127: $Z-L^{3-b}-N(-L^{1-a}-D^{1-a})(L^{2-b}-D^{2-cg})$,
Exemplification 6-128: $Z-L^{3-b}-N(-L^{1-a}-D^{1-a})(L^{2-b}-D^{2-ch})$,
Exemplification 6-129: $Z-L^{3-b}-N(-L^{1-a}-D^{1-a})(L^{2-d}-D^{2-cg})$,
Exemplification 6-130: $Z-L^{3-b}-N(-L^{1-a}-D^{1-a})(L^{2-d}-D^{2-ch})$,
Exemplification 6-131: $Z-L^{3-b}-N(-L^{1-a}-D^{1-b})(L^{2-i}-D^{2-ca})$,
Exemplification 6-132: $Z-L^{3-b}-N(-L^{1-a}-D^{1-b})(L^{2-i}-D^{2-cc})$,
Exemplification 6-133: $Z-L^{3-b}-N(-L^{1-a}-D^{1-b})(L^{2-i}-D^{2-cd})$,
Exemplification 6-134: $Z-L^{3-b}-N(-L^{1-a}-D^{1-b})(L^{2-i}-D^{2-ce})$,
Exemplification 6-135: $Z-L^{3-b}-N(-L^{1-a}-D^{1-b})(L^{2-i}-D^{2-cf})$,
Exemplification 6-136: $Z-L^{3-b}-N(-L^{1-a}-D^{1-b})(L^{2-3}-D^{2-ca})$,
Exemplification 6-137: $Z-L^{3-b}-N(-L^{1-a}-D^{1-b})(L^{2-j}-D^{2-cc})$,
Exemplification 6-138: $Z-L^{3-b}-N(-L^{1-a}-D^{1-b})(L^{2-j}-D^{2-cd})$,
Exemplification 6-139: $Z-L^{3-b}-N(-L^{1-a}-D^{1-b})(L^{2-j}-D^{2-ce})$,
Exemplification 6-140: $Z-L^{3-b}-N(-L^{1-a}-D^{1-b})(L^{2-j}-D^{2-cf})$,
Exemplification 6-141: $Z-L^{3-b}-N(-L^{1-a}-D^{1-b})(L^{2-k}-D^{2-cb})$,
Exemplification 6-142: $Z-L^{3-b}-N(-L^{1-a}-D^{1-b})(L^{2-1}-D^{2-ca})$,
Exemplification 6-143: $Z-L^{3-b}-N(-L^{1-a}-D^{1-b})(L^{2-1}-D^{2-cc})$,
Exemplification 6-144: $Z-L^{3-b}-N(-L^{1-a}-D^{1-b})(L^{2-1}-D^{2-cd})$,
Exemplification 6-145: $Z-L^{3-b}-N(-L^{1-a}-D^{1-b})(L^{2-1}-D^{2-ce})$,
Exemplification 6-146: $Z-L^{3-b}-N(-L^{1-a}-D^{1-b})(L^{2-1}-D^{2-cf})$,
Exemplification 6-147: $Z-L^{3-b}-N(-L^{1-a}-D^{1-b})(L^{2-m}-D^{2-ca})$,
Exemplification 6-148: $Z-L^{3-b}-N(-L^{1-a}-D^{1-b})(L^{2-m}-D^{2-cc})$,
Exemplification 6-149: $Z-L^{3-b}-N(-L^{1-a}-D^{1-b})(L^{2-m}-D^{2-cd})$,
Exemplification 6-150: $Z-L^{3-b}-N(-L^{1-a}-D^{1-b})(L^{2-m}-D^{2-ce})$,
Exemplification 6-151: $Z-L^{3-b}-N(-L^{1-a}-D^{1-b})(L^{2-m}-D^{2-cf})$,
Exemplification 6-152: $Z-L^{3-b}-N(-L^{1-a}-D^{1-b})(L^{2-n}-D^{2-cb})$,
Exemplification 6-153: $Z-L^{3-b}-N(-L^{1-a}-D^{1-b})(L^{2-b}-D^{2-cg})$,
Exemplification 6-154: $Z-L^{3-b}-N(-L^{1-a}-D^{1-b})(L^{2-b}-D^{2-ch})$,
Exemplification 6-155: $Z-L^{3-b}-N(-L^{1-a}-D^{1-b})(L^{2-d}-D^{2-cg})$,
Exemplification 6-156: $Z-L^{3-b}-N(-L^{1-a}-D^{1-b})(L^{2-d}-D^{2-ch})$,
Exemplification 6-157: $Z-L^{3-b}-N(-L^{1-b}-D^{1-a})(L^{2-i}-D^{2-ca})$,
Exemplification 6-158: $Z-L^{3-b}-N(-L^{1-b}-D^{1-a})(L^{2-i}-D^{2-cc})$,
Exemplification 6-159: $Z-L^{3-b}-N(-L^{1-b}-D^{1-a})(L^{2-i}-D^{2-cd})$,
Exemplification 6-160: $Z-L^{3-b}-N(-L^{1-b}-D^{1-a})(L^{2-i}-D^{2-ce})$,
Exemplification 6-161: $Z-L^{3-b}-N(-L^{1-b}-D^{1-a})(L^{2-i}-D^{2-cf})$,
Exemplification 6-162: $Z-L^{3-b}-N(-L^{1-b}-D^{1-a})(L^{2-3}-D^{2-ca})$,
Exemplification 6-163: $Z-L^{3-b}-N(-L^{1-b}-D^{1-2})(L^{2-j}-D^{2-cc})$,
Exemplification 6-164: $Z-L^{3-b}-N(-L^{1-b}-D^{1-a})(L^{2-j}-D^{2-cd})$,
Exemplification 6-165: $Z-L^{3-b}-N(-L^{1-b}-D^{1-a})(L^{2-j}-D^{2-ce})$,
Exemplification 6-166: $Z-L^{3-b}-N(-L^{1-b}-D^{1-a})(L^{2-3}-D^{2-of})$,
Exemplification 6-167: $Z-L^{3-b}-N(-L^{1-b}-D^{1-2})(L^{2-k}-D^{2-cb})$,
Exemplification 6-168: $Z-L^{3-b}-N(-L^{1-b}-D^{1-a})(L^{2-1}-D^{2-ca})$,
Exemplification 6-169: $Z-L^{3-b}-N(-L^{1-b}-D^{1-a})(L^{2-1}-D^{2-cc})$,
Exemplification 6-170: $Z-L^{3-b}-N(-L^{1-b}-D^{1-a})(L^{2-1}-D^{2-cd})$,
Exemplification 6-171: $Z-L^{3-b}-N(-L^{1-b}-D^{1-2})(L^{2-1}-D^{2-ce})$,
Exemplification 6-172: $Z-L^{3-b}-N(-L^{1-b}-D^{1-a})(L^{2-1}-D^{2-cf})$,

Exemplification 6-173: Z-L$^{3-b}$-N(-L$^{1-b}$-D$^{1-a}$)(L$^{2-m}$-D$^{2-ca}$),
Exemplification 6-174: Z-L$^{3-b}$-N(-L$^{1-b}$-D$^{1-a}$)(L$^{2-m}$-D$^{2-cc}$),
Exemplification 6-175: Z-L$^{3-b}$-N(-L$^{1-b}$-D$^{1-a}$)(L$^{2-m}$-D$^{2-cd}$),
Exemplification 6-176: Z-L$^{3-b}$-N(-L$^{1-b}$-D$^{1-2}$)(L$^{2-m}$-D$^{2-ce}$),
Exemplification 6-177: Z-L$^{3-b}$-N(-L$^{1-b}$-D$^{1-a}$)(L$^{2-m}$-D$^{2-cf}$),
Exemplification 6-178: Z-L$^{3-b}$-N(-L$^{1-b}$-D$^{1-a}$)(L$^{2-n}$-D$^{2-cb}$),
Exemplification 6-179: Z-L$^{3-b}$-N(-L$^{1-b}$-D$^{1-2}$)(L$^{2-b}$-D$^{2-cg}$),
Exemplification 6-180: Z-L$^{3-b}$-N(-L$^{1-b}$-D$^{1-a}$)(L$^{2-b}$-D$^{2-ch}$,
Exemplification 6-181: Z-L$^{3-b}$-N(-L$^{1-b}$-D$^{1-2}$)(L$^{2-d}$-D$^{2-cg}$),
Exemplification 6-182: Z-L$^{3-b}$-N(-L$^{1-b}$-D$^{1-a}$)(L$^{2-d}$-D$^{2-ch}$),
Exemplification 6-183: Z-L$^{3-b}$-N(-L$^{1-b}$-D$^{1-b}$)(L$^{2-i}$-D$^{2-ca}$),
Exemplification 6-184: Z-L$^{3-b}$-N(-L$^{1-b}$-D$^{1-b}$)(L$^{2-i}$-D$^{2-cc}$),
Exemplification 6-185: Z-L$^{3-b}$-N(-L$^{1-b}$-D$^{1-b}$)(L$^{2-i}$-D$^{2-cd}$),
Exemplification 6-186: Z-L$^{3-b}$-N(-L$^{1-b}$-D$^{1-b}$)(L$^{2-i}$-D$^{2-ce}$),
Exemplification 6-187: Z-L$^{3-b}$-N(-L$^{1-b}$-D$^{1-b}$)(L$^{2-i}$-D$^{2-cf}$),
Exemplification 6-188: Z-L$^{3-b}$-N(-L$^{1-b}$-D$^{1-b}$)(L$^{2-3}$-D$^{2-ca}$),
Exemplification 6-189: Z-L$^{3-b}$-N(-L$^{1-b}$-D$^{1-b}$)(L$^{2-j}$-D$^{2-cc}$),
Exemplification 6-190: Z-L$^{3-b}$-N(-L$^{1-b}$-D$^{1-b}$)(L$^{2-j}$-D$^{2-cd}$),
Exemplification 6-191: Z-L$^{3-b}$-N(-L$^{1-b}$-D$^{1-b}$)(L$^{2-j}$-D$^{2-ce}$),
Exemplification 6-192: Z-L$^{3-b}$-N(-L$^{1-b}$-D$^{1-b}$)(L$^{2-j}$-D$^{2-cf}$),
Exemplification 6-193: Z-L$^{3-b}$-N(-L$^{1-b}$-D$^{1-b}$)(L$^{2-k}$-D$^{2-cb}$),
Exemplification 6-194: Z-L$^{3-b}$-N(-L$^{1-b}$-D$^{1-b}$)(L$^{2-1}$-D$^{2-ca}$),
Exemplification 6-195: Z-L$^{3-b}$-N(-L$^{1-b}$-D$^{1-b}$)(L$^{2-l}$-D$^{2-cc}$),
Exemplification 6-196: Z-L$^{3-b}$-N(-L$^{1-b}$-D$^{1-b}$)(L$^{2-l}$-D$^{2-cd}$),
Exemplification 6-197: Z-L$^{3-b}$-N(-L$^{1-b}$-D$^{1-b}$)(L$^{2-l}$-D$^{2-ce}$),
Exemplification 6-198: Z-L$^{3-b}$-N(-L$^{1-b}$-D$^{1-b}$)(L$^{2-l}$-D$^{2-cf}$),
Exemplification 6-199: Z-L$^{3-b}$-N(-L$^{1-b}$-D$^{1-b}$)(L$^{2-m}$-D$^{2-ca}$),
Exemplification 6-200: Z-L$^{3-b}$-N(-L$^{1-b}$-D$^{1-b}$)(L$^{2-m}$-D$^{2-cc}$),
Exemplification 6-201: Z-L$^{3-b}$-N(-L$^{1-b}$-D$^{1-b}$)(L$^{2-m}$-D$^{2-cd}$),
Exemplification 6-202: Z-L$^{3-b}$-N(-L$^{1-b}$-D$^{1-b}$)(L$^{2-m}$-D$^{2-ce}$),
Exemplification 6-203: Z-L$^{3-b}$-N(-L$^{1-b}$-D$^{1-b}$)(L$^{2-m}$-D$^{2-cf}$),
Exemplification 6-204: Z-L$^{3-b}$-N(-L$^{1-b}$-D$^{1-b}$)(L$^{2-n}$-D$^{2-cb}$),
Exemplification 6-205: Z-L$^{3-b}$-N(-L$^{1-b}$-D$^{1-b}$)(L$^{2-b}$-D$^{2-cg}$),
Exemplification 6-206: Z-L$^{3-b}$-N(-L$^{1-b}$-D$^{1-b}$)(L$^{2-b}$-D$^{2-ch}$),
Exemplification 6-207: Z-L$^{3-b}$-N(-L$^{1-b}$-D$^{1-b}$)(L$^{2-d}$-D$^{2-cg}$),
Exemplification 6-208: Z-L$^{3-b}$-N(-L$^{1-b}$-D$^{1-b}$)(L$^{2-d}$-D$^{2-ch}$),
Exemplification 6-209: Z-L$^{3-c}$-N(-L$^{1-a}$-D$^{1-a}$)(L$^{2-i}$-D$^{2-ca}$),
Exemplification 6-210: Z-L$^{3-c}$-N(-L$^{1-a}$-D$^{1-a}$)(L$^{2-i}$-D$^{2-cc}$),
Exemplification 6-211: Z-L$^{3-c}$-N(-L$^{1-a}$-D$^{1-a}$)(L$^{2-i}$-D$^{2-cd}$),
Exemplification 6-212: Z-L$^{3-c}$-N(-L$^{1-a}$-D$^{1-a}$)(L$^{2-i}$-D$^{2-ce}$),
Exemplification 6-213: Z-L$^{3-c}$-N(-L$^{1-a}$-D$^{1-a}$)(L$^{2-i}$-D$^{2-cf}$),
Exemplification 6-214: Z-L$^{3-c}$-N(-L$^{1-a}$-D$^{1-a}$)(L$^{2-j}$-D$^{2-ca}$),
Exemplification 6-215: Z-L$^{3-c}$-N(-L$^{1-a}$-D$^{1-a}$)(L$^{2-j}$-D$^{2-cc}$),
Exemplification 6-216: Z-L$^{3-c}$-N(-L$^{1-a}$-D$^{1-a}$)(L$^{2-j}$-D$^{2-cd}$),
Exemplification 6-217: Z-L$^{3-c}$-N(-L$^{1-a}$-D$^{1-a}$)(L$^{2-j}$-D$^{2-ce}$),
Exemplification 6-218: Z-L$^{3-c}$-N(-L$^{1-a}$-D$^{1-a}$)(L$^{2-j}$-D$^{2-cf}$),
Exemplification 6-219: Z-L$^{3-c}$-N(-L$^{1-a}$-D$^{1-a}$)(L$^{2-k}$-D$^{2-cb}$),
Exemplification 6-220: Z-L$^{3-c}$-N(-L$^{1-a}$-D$^{1-a}$)(L$^{2-l}$-D$^{2-ca}$),
Exemplification 6-221: Z-L$^{3-c}$-N(-L$^{1-a}$-D$^{1-a}$)(L$^{2-l}$-D$^{2-cc}$),
Exemplification 6-222: Z-L$^{3-c}$-N(-L$^{1-a}$-D$^{1-a}$)(L$^{2-l}$-D$^{2-cd}$),
Exemplification 6-223: Z-L$^{3-c}$-N(-L$^{1-a}$-D$^{1-a}$)(L$^{2-l}$-D$^{2-ce}$),
Exemplification 6-224: Z-L$^{3-c}$-N(-L$^{1-a}$-D$^{1-a}$)(L$^{2-l}$-D$^{2-cf}$),
Exemplification 6-225: Z-L$^{3-c}$-N(-L$^{1-a}$-D$^{1-a}$)(L$^{2-m}$-D$^{2-ca}$),
Exemplification 6-226: Z-L$^{3-c}$-N(-L$^{1-a}$-D$^{1-a}$)(L$^{2-m}$-D$^{2-cc}$),
Exemplification 6-227: Z-L$^{3-c}$-N(-L$^{1-a}$-D$^{1-a}$)(L$^{2-m}$-D$^{2-cd}$),
Exemplification 6-228: Z-L$^{3-c}$-N(-L$^{1-a}$-D$^{1-a}$)(L$^{2-m}$-D$^{2-ce}$),
Exemplification 6-229: Z-L$^{3-c}$-N(-L$^{1-a}$-D$^{1-a}$)(L$^{2-m}$-D$^{2-cf}$),
Exemplification 6-230: Z-L$^{3-c}$-N(-L$^{1-a}$-D$^{1-a}$)(L$^{2-n}$-D$^{2-cb}$),

Exemplification 6-231: $Z-L^{3-c}-N(-L^{1-a}-D^{1-a})(L^{2-b}-D^{2-cg})$,
Exemplification 6-232: $Z-L^{3-c}-N(-L^{1-a}-D^{1-a})(L^{2-b}-D^{2-ch})$,
Exemplification 6-233: $Z-L^{3-c}-N(-L^{1-a}-D^{1-a})(L^{2-d}-D^{2-cg})$,
Exemplification 6-234: $Z-L^{3-c}-N(-L^{1-a}-D^{1-a})(L^{2-d}-D^{2-ch})$,
Exemplification 6-235: $Z-L^{3-c}-N(-L^{1-a}-D^{1-b})(L^{2-i}-D^{2-ca})$,
Exemplification 6-236: $Z-L^{3-c}-N(-L^{1-a}-D^{1-b})(L^{2-i}-D^{2-cc})$,
Exemplification 6-237: $Z-L^{3-c}-N(-L^{1-a}-D^{1-b})(L^{2-i}-D^{2-cd})$,
Exemplification 6-238: $Z-L^{3-c}-N(-L^{1-a}-D^{1-b})(L^{2-i}-D^{2-ce})$,
Exemplification 6-239: $Z-L^{3-c}-N(-L^{1-a}-D^{1-b})(L^{2-i}-D^{2-cf})$,
Exemplification 6-240: $Z-L^{3-c}-N(-L^{1-a}-D^{1-b})(L^{2-j}-D^{2-ca})$,
Exemplification 6-241: $Z-L^{3-c}-N(-L^{1-a}-D^{1-b})(L^{2-j}-D^{2-cc})$,
Exemplification 6-242: $Z-L^{3-c}-N(-L^{1-a}-D^{1-b})(L^{2-j}-D^{2-cd})$,
Exemplification 6-243: $Z-L^{3-c}-N(-L^{1-a}-D^{1-b})(L^{2-j}-D^{2-ce})$,
Exemplification 6-244: $Z-L^{3-c}-N(-L^{1-a}-D^{1-b})(L^{2-j}-D^{2-cf})$,
Exemplification 6-245: $Z-L^{3-c}-N(-L^{1-a}-D^{1-b})(L^{2-k}-D^{2-cb})$,
Exemplification 6-246: $Z-L^{3-c}-N(-L^{1-a}-D^{1-b})(L^{2-l}-D^{2-ca})$,
Exemplification 6-247: $Z-L^{3-c}-N(-L^{1-a}-D^{1-b})(L^{2-l}-D^{2-cc})$,
Exemplification 6-248: $Z-L^{3-c}-N(-L^{1-a}-D^{1-b})(L^{2-l}-D^{2-cd})$,
Exemplification 6-249: $Z-L^{3-c}-N(-L^{1-a}-D^{1-b})(L^{2-l}-D^{2-ce})$,
Exemplification 6-250: $Z-L^{3-c}-N(-L^{1-a}-D^{1-b})(L^{2-l}-D^{2-cf})$,
Exemplification 6-251: $Z-L^{3-c}-N(-L^{1-a}-D^{1-b})(L^{2-m}-D^{2-ca})$,
Exemplification 6-252: $Z-L^{3-c}-N(-L^{1-a}-D^{1-b})(L^{2-m}-D^{2-cc})$,
Exemplification 6-253: $Z-L^{3-c}-N(-L^{1-a}-D^{1-b})(L^{2-m}-D^{2-cd})$,
Exemplification 6-254: $Z-L^{3-c}-N(-L^{1-a}-D^{1-b})(L^{2-m}-D^{2-ce})$,
Exemplification 6-255: $Z-L^{3-c}-N(-L^{1-a}-D^{1-b})(L^{2-m}-D^{2-cf})$,
Exemplification 6-256: $Z-L^{3-c}-N(-L^{1-a}-D^{1-b})(L^{2-n}-D^{2-cb})$,
Exemplification 6-257: $Z-L^{3-c}-N(-L^{1-a}-D^{1-b})(L^{2-b}-D^{2-cg})$,
Exemplification 6-258: $Z-L^{3-c}-N(-L^{1-a}-D^{1-b})(L^{2-b}-D^{2-ch})$,
Exemplification 6-259: $Z-L^{3-c}-N(-L^{1-a}-D^{1-b})(L^{2-d}-D^{2-cg})$,
Exemplification 6-260: $Z-L^{3-c}-N(-L^{1-a}-D^{1-b})(L^{2-d}-D^{2-ch})$,
Exemplification 6-261: $Z-L^{3-c}-N(-L^{1-b}-D^{1-a})(L^{2-i}-D^{2-ca})$,
Exemplification 6-262: $Z-L^{3-c}-N(-L^{1-b}-D^{1-a})(L^{2-i}-D^{2-cc})$,
Exemplification 6-263: $Z-L^{3-c}-N(-L^{1-b}-D^{1-a})(L^{2-i}-D^{2-cd})$,
Exemplification 6-264: $Z-L^{3-c}-N(-L^{1-b}-D^{1-a})(L^{2-i}-D^{2-ce})$,
Exemplification 6-265: $Z-L^{3-c}-N(-L^{1-b}-D^{1-a})(L^{2-i}-D^{2-cf})$,
Exemplification 6-266: $Z-L^{3-c}-N(-L^{1-b}-D^{1-a})(L^{2-j}-D^{2-ca})$,
Exemplification 6-267: $Z-L^{3-c}-N(-L^{1-b}-D^{1-a})(L^{2-j}-D^{2-cc})$,
Exemplification 6-268: $Z-L^{3-c}-N(-L^{1-b}-D^{1-a})(L^{2-j}-D^{2-cd})$,
Exemplification 6-269: $Z-L^{3-c}-N(-L^{1-b}-D^{1-a})(L^{2-j}-D^{2-ce})$,
Exemplification 6-270: $Z-L^{3-c}-N(-L^{1-b}-D^{1-a})(L^{2-j}-D^{2-cf})$,
Exemplification 6-271: $Z-L^{3-c}-N(-L^{1-b}-D^{1-a})(L^{2-k}-D^{2-cb})$,
Exemplification 6-272: $Z-L^{3-c}-N(-L^{1-b}-D^{1-a})(L^{2-l}-D^{2-ca})$,
Exemplification 6-273: $Z-L^{3-c}-N(-L^{1-b}-D^{1-a})(L^{2-1}-D^{2-cc})$,
Exemplification 6-274: $Z-L^{3-c}-N(-L^{1-b}-D^{1-a})(L^{2-1}-D^{2-cd})$,
Exemplification 6-275: $Z-L^{3-c}-N(-L^{1-b}-D^{1-a})(L^{2-l}-D^{2-ce})$,
Exemplification 6-276: $Z-L^{3-c}-N(-L^{1-b}-D^{1-a})(L^{2-l}-D^{2-cf})$,
Exemplification 6-277: $Z-L^{3-c}-N(-L^{1-b}-D^{1-a})(L^{2-m}-D^{2-ca})$,
Exemplification 6-278: $Z-L^{3-c}-N(-L^{1-b}-D^{1-a})(L^{2-m}-D^{2-cc})$,
Exemplification 6-279: $Z-L^{3-c}-N(-L^{1-b}-D^{1-a})(L^{2-m}-D^{2-cd})$,
Exemplification 6-280: $Z-L^{3-c}-N(-L^{1-b}-D^{1-a})(L^{2-m}-D^{2-ce})$,
Exemplification 6-281: $Z-L^{3-c}-N(-L^{1-b}-D^{1-a})(L^{2-m}-D^{2-cf})$,
Exemplification 6-282: $Z-L^{3-c}-N(-L^{1-b}-D^{1-a})(L^{2-n}-D^{2-cb})$,
Exemplification 6-283: $Z-L^{3-c}-N(-L^{1-b}-D^{1-a})(L^{2-b}-D^{2-cg})$,
Exemplification 6-284: $Z-L^{3-c}-N(-L^{1-b}-D^{1-a})(L^{2-b}-D^{2-ch})$,
Exemplification 6-285: $Z-L^{3-c}-N(-L^{1-b}-D^{1-a})(L^{2-d}-D^{2-cg})$,
Exemplification 6-286: $Z-L^{3-c}-N(-L^{1-b}-D^{1-a})(L^{2-d}-D^{2-ch})$,
Exemplification 6-287: $Z-L^{3-c}-N(-L^{1-b}-D^{1-b})(L^{2-i}-D^{2-ca})$,
Exemplification 6-288: $Z-L^{3-c}-N(-L^{1-b}-D^{1-b})(L^{2-i}-D^{2-cc})$,

Exemplification 6-289: $Z-L^{3-c}-N(-L^{1-b}-D^{1-b})(L^{2-i}-D^{2-cd})$,
Exemplification 6-290: $Z-L^{3-c}-N(-L^{1-b}-D^{1-b})(L^{2-i}-D^{2-ce})$,
Exemplification 6-291: $Z-L^{3-c}-N(-L^{1-b}-D^{1-b})(L^{2-i}-D^{2-cf})$,
Exemplification 6-292: $Z-L^{3-c}-N(-L^{1-b}-D^{1-b})(L^{2-j}-D^{2-ca})$,
Exemplification 6-293: $Z-L^{3-c}-N(-L^{1-b}-D^{1-b})(L^{2-j}-D^{2-cc})$,
Exemplification 6-294: $Z-L^{3-c}-N(-L^{1-b}-D^{1-b})(L^{2-j}-D^{2-cd})$,
Exemplification 6-295: $Z-L^{3-c}-N(-L^{1-b}-D^{1-b})(L^{2-j}-D^{2-ce})$,
Exemplification 6-296: $Z-L^{3-c}-N(-L^{1-b}-D^{1-b})(L^{2-j}-D^{2-cf})$,
Exemplification 6-297: $Z-L^{3-c}-N(-L^{1-b}-D^{1-b})(L^{2-k}-D^{2-cb})$,
Exemplification 6-298: $Z-L^{3-c}-N(-L^{1-b}-D^{1-b})(L^{2-l}-D^{2-ca})$,
Exemplification 6-299: $Z-L^{3-c}-N(-L^{1-b}-D^{1-b})(L^{2-l}-D^{2-cc})$,
Exemplification 6-300: $Z-L^{3-c}-N(-L^{1-b}-D^{1-b})(L^{2-l}-D^{2-cd})$,
Exemplification 6-301: $Z-L^{3-c}-N(-L^{1-b}-D^{1-b})(L^{2-l}-D^{2-ce})$,
Exemplification 6-302: $Z-L^{3-c}-N(-L^{1-b}-D^{1-b})(L^{2-l}-D^{2-cf})$,
Exemplification 6-303: $Z-L^{3-c}-N(-L^{1-b}-D^{1-b})(L^{2-m}-D^{2-ca})$,
Exemplification 6-304: $Z-L^{3-c}-N(-L^{1-b}-D^{1-b})(L^{2-m}-D^{2-cc})$,
Exemplification 6-305: $Z-L^{3-c}-N(-L^{1-b}-D^{1-b})(L^{2-m}-D^{2-cd})$,
Exemplification 6-306: $Z-L^{3-c}-N(-L^{1-b}-D^{1-b})(L^{2-m}-D^{2-ce})$,
Exemplification 6-307: $Z-L^{3-c}-N(-L^{1-b}-D^{1-b})(L^{2-m}-D^{2-cf})$,
Exemplification 6-308: $Z-L^{3-c}-N(-L^{1-b}-D^{1-b})(L^{2-n}-D^{2-cb})$,
Exemplification 6-309: $Z-L^{3-c}-N(-L^{1-b}-D^{1-b})(L^{2-b}-D^{2-cg})$,
Exemplification 6-310: $Z-L^{3-c}-N(-L^{1-b}-D^{1-b})(L^{2-b}-D^{2-ch})$,
Exemplification 6-311: $Z-L^{3-c}-N(-L^{1-b}-D^{1-b})(L^{2-d}-D^{2-cg})$,
Exemplification 6-312: $Z-L^{3-c}-N(-L^{1-b}-D^{1-b})(L^{2-d}-D^{2-ch})$,
Exemplification 6-313: $Z-L^{3-d}-N(-L^{1-a}-D^{1-a})(L^{2-i}-D^{2-ca})$,
Exemplification 6-314: $Z-L^{3-d}-N(-L^{1-a}-D^{1-a})(L^{2-i}-D^{2-cc})$,
Exemplification 6-315: $Z-L^{3-d}-N(-L^{1-a}-D^{1-a})(L^{2-i}-D^{2-cd})$,
Exemplification 6-316: $Z-L^{3-d}-N(-L^{1-a}-D^{1-a})(L^{2-i}-D^{2-ce})$,
Exemplification 6-317: $Z-L^{3-d}-N(-L^{1-a}-D^{1-a})(L^{2-i}-D^{2-cf})$,
Exemplification 6-318: $Z-L^{3-d}-N(-L^{1-a}-D^{1-a})(L^{2-j}-D^{2-ca})$,
Exemplification 6-319: $Z-L^{3-d}-N(-L^{1-a}-D^{1-a})(L^{2-j}-D^{2-cc})$,
Exemplification 6-320: $Z-L^{3-d}-N(-L^{1-a}-D^{1-a})(L^{2-j}-D^{2-cd})$,
Exemplification 6-321: $Z-L^{3-d}-N(-L^{1-a}-D^{1-a})(L^{2-j}-D^{2-ce})$,
Exemplification 6-322: $Z-L^{3-d}-N(-L^{1-a}-D^{1-a})(L^{2-j}-D^{2-cf})$,
Exemplification 6-323: $Z-L^{3-d}-N(-L^{1-a}-D^{1-a})(L^{2-k}-D^{2-cb})$,
Exemplification 6-324: $Z-L^{3-d}-N(-L^{1-a}-D^{1-a})(L^{2-l}-D^{2-ca})$,
Exemplification 6-325: $Z-L^{3-d}-N(-L^{1-a}-D^{1-a})(L^{2-l}-D^{2-cc})$,
Exemplification 6-326: $Z-L^{3-d}-N(-L^{1-a}-D^{1-a})(L^{2-l}-D^{2-cd})$,
Exemplification 6-327: $Z-L^{3-d}-N(-L^{1-a}-D^{1-a})(L^{2-l}-D^{2-ce})$,
Exemplification 6-328: $Z-L^{3-d}-N(-L^{1-a}-D^{1-a})(L^{2-l}-D^{2-cf})$,
Exemplification 6-329: $Z-L^{3-d}-N(-L^{1-a}-D^{1-a})(L^{2-m}-D^{2-ca})$,
Exemplification 6-330: $Z-L^{3-d}-N(-L^{1-a}-D^{1-a})(L^{2-m}-D^{2-cc})$,
Exemplification 6-331: $Z-L^{3-d}-N(-L^{1-a}-D^{1-a})(L^{2-m}-D^{2-cd})$,
Exemplification 6-332: $Z-L^{3-d}-N(-L^{1-a}-D^{1-a})(L^{2-m}-D^{2-ce})$,
Exemplification 6-333: $Z-L^{3-d}-N(-L^{1-a}-D^{1-a})(L^{2-m}-D^{2-cf})$,
Exemplification 6-334: $Z-L^{3-d}-N(-L^{1-a}-D^{1-a})(L^{2-n}-D^{2-cb})$,
Exemplification 6-335: $Z-L^{3-d}-N(-L^{1-a}-D^{1-a})(L^{2-b}-D^{2-cg})$,
Exemplification 6-336: $Z-L^{3-d}-N(-L^{1-a}-D^{1-a})(L^{2-b}-D^{2-ch})$,
Exemplification 6-337: $Z-L^{3-d}-N(-L^{1-a}-D^{1-a})(L^{2-d}-D^{2-cg})$,
Exemplification 6-338: $Z-L^{3-d}-N(-L^{1-a}-D^{1-a})(L^{2-d}-D^{2-ch})$,
Exemplification 6-339: $Z-L^{3-d}-N(-L^{1-a}-D^{1-b})(L^{2-i}-D^{2-ca})$,
Exemplification 6-340: $Z-L^{3-d}-N(-L^{1-a}-D^{1-b})(L^{2-i}-D^{2-cc})$,
Exemplification 6-341: $Z-L^{3-d}-N(-L^{1-a}-D^{1-b})(L^{2-i}-D^{2-cd})$,
Exemplification 6-342: $Z-L^{3-d}-N(-L^{1-a}-D^{1-b})(L^{2-i}-D^{2-ce})$,
Exemplification 6-343: $Z-L^{3-d}-N(-L^{1-a}-D^{1-b})(L^{2-i}-D^{2-cf})$,
Exemplification 6-344: $Z-L^{3-d}-N(-L^{1-a}-D^{1-b})(L^{2-j}-D^{2-ca})$,
Exemplification 6-345: $Z-L^{3-d}-N(-L^{1-a}-D^{1-b})(L^{2-j}-D^{2-cc})$,
Exemplification 6-346: $Z-L^{3-d}-N(-L^{1-a}-D^{1-b})(L^{2-j}-D^{2-cd})$,

Exemplification 6-347: Z-$L^{3-d}$-N(-$L^{1-a}$-$D^{1-b}$)($L^{2-j}$-$D^{2-ce}$),
Exemplification 6-348: Z-$L^{3-d}$-N(-$L^{1-a}$-$D^{1-b}$)($L^{2-j}$-$D^{2-cf}$),
Exemplification 6-349: Z-$L^{3-d}$-N(-$L^{1-a}$-$D^{1-b}$)($L^{2-k}$-$D^{2-cb}$),
Exemplification 6-350: Z-$L^{3-d}$-N(-$L^{1-a}$-$D^{1-b}$)($L^{2-l}$-$D^{2-ca}$),
Exemplification 6-351: Z-$L^{3-d}$-N(-$L^{1-a}$-$D^{1-b}$)($L^{2-l}$-$D^{2-cc}$),
Exemplification 6-352: Z-$L^{3-d}$-N(-$L^{1-a}$-$D^{1-b}$)($L^{2-l}$-$D^{2-cd}$),
Exemplification 6-353: Z-$L^{3-d}$-N(-$L^{1-a}$-$D^{1-b}$)($L^{2-l}$-$D^{2-ce}$),
Exemplification 6-354: Z-$L^{3-d}$-N(-$L^{1-a}$-$D^{1-b}$)($L^{2-l}$-$D^{2-cf}$),
Exemplification 6-355: Z-$L^{3-d}$-N(-$L^{1-a}$-$D^{1-b}$)($L^{2-m}$-$D^{2-ca}$),
Exemplification 6-356: Z-$L^{3-d}$-N(-$L^{1-a}$-$D^{1-b}$)($L^{2-m}$-$D^{2-cc}$),
Exemplification 6-357: Z-$L^{3-d}$-N(-$L^{1-a}$-$D^{1-b}$)($L^{2-m}$-$D^{2-cd}$),
Exemplification 6-358: Z-$L^{3-d}$-N(-$L^{1-a}$-$D^{1-b}$)($L^{2-m}$-$D^{2-ce}$),
Exemplification 6-359: Z-$L^{3-d}$-N(-$L^{1-a}$-$D^{1-b}$)($L^{2-m}$-$D^{2-cf}$),
Exemplification 6-360: Z-$L^{3-d}$-N(-$L^{1-a}$-$D^{1-b}$)($L^{2-n}$-$D^{2-cb}$),
Exemplification 6-361: Z-$L^{3-d}$-N(-$L^{1-a}$-$D^{1-b}$)($L^{2-b}$-$D^{2-cg}$),
Exemplification 6-362: Z-$L^{3-d}$-N(-$L^{1-a}$-$D^{1-b}$)($L^{2-b}$-$D^{2-ch}$),
Exemplification 6-363: Z-$L^{3-d}$-N(-$L^{1-a}$-$D^{1-b}$)($L^{2-d}$-$D^{2-cg}$),
Exemplification 6-364: Z-$L^{3-d}$-N(-$L^{1-a}$-$D^{1-b}$)($L^{2-d}$-$D^{2-ch}$),
Exemplification 6-365: Z-$L^{3-d}$-N(-$L^{1-b}$-$D^{1-a}$)($L^{2-i}$-$D^{2-ca}$),
Exemplification 6-366: Z-$L^{3-d}$-N(-$L^{1-b}$-$D^{1-a}$)($L^{2-i}$-$D^{2-cc}$),
Exemplification 6-367: Z-$L^{3-d}$-N(-$L^{1-b}$-$D^{1-a}$)($L^{2-i}$-$D^{2-cd}$),
Exemplification 6-368: Z-$L^{3-d}$-N(-$L^{1-b}$-$D^{1-a}$)($L^{2-i}$-$D^{2-ce}$),
Exemplification 6-369: Z-$L^{3-d}$-N(-$L^{1-b}$-$D^{1-a}$)($L^{2-i}$-$D^{2-cf}$),
Exemplification 6-370: Z-$L^{3-d}$-N(-$L^{1-b}$-$D^{1-a}$)($L^{2-j}$-$D^{2-ca}$),
Exemplification 6-371: Z-$L^{3-d}$-N(-$L^{1-b}$-$D^{1-a}$)($L^{2-j}$-$D^{2-cc}$),
Exemplification 6-372: Z-$L^{3-d}$-N(-$L^{1-b}$-$D^{1-a}$)($L^{2-j}$-$D^{2-cd}$),
Exemplification 6-373: Z-$L^{3-d}$-N(-$L^{1-b}$-$D^{1-a}$)($L^{2-j}$-$D^{2-ce}$),
Exemplification 6-374: Z-$L^{3-d}$-N(-$L^{1-b}$-$D^{1-a}$)($L^{2-j}$-$D^{2-cf}$),
Exemplification 6-375: Z-$L^{3-d}$-N(-$L^{1-b}$-$D^{1-a}$)($L^{2-k}$-$D^{2-cb}$),
Exemplification 6-376: Z-$L^{3-d}$-N(-$L^{1-b}$-$D^{1-a}$)($L^{2-l}$-$D^{2-ca}$),
Exemplification 6-377: Z-$L^{3-d}$-N(-$L^{1-b}$-$D^{1-a}$)($L^{2-l}$-$D^{2-cc}$),
Exemplification 6-378: Z-$L^{3-d}$-N(-$L^{1-b}$-$D^{1-a}$)($L^{2-l}$-$D^{2-cd}$),
Exemplification 6-379: Z-$L^{3-d}$-N(-$L^{1-b}$-$D^{1-a}$)($L^{2-l}$-$D^{2-ce}$),
Exemplification 6-380: Z-$L^{3-d}$-N(-$L^{1-b}$-$D^{1-a}$)($L^{2-l}$-$D^{2-cf}$),
Exemplification 6-381: Z-$L^{3-d}$-N(-$L^{1-b}$-$D^{1-a}$)($L^{2-m}$-$D^{2-ca}$),
Exemplification 6-382: Z-$L^{3-d}$-N(-$L^{1-b}$-$D^{1-a}$)($L^{2-m}$-$D^{2-cc}$),
Exemplification 6-383: Z-$L^{3-d}$-N(-$L^{1-b}$-$D^{1-a}$)($L^{2-m}$-$D^{2-cd}$),
Exemplification 6-384: Z-$L^{3-d}$-N(-$L^{1-b}$-$D^{1-a}$)($L^{2-m}$-$D^{2-ce}$),
Exemplification 6-385: Z-$L^{3-d}$-N(-$L^{1-b}$-$D^{1-a}$)($L^{2-m}$-$D^{2-cf}$),
Exemplification 6-386: Z-$L^{3-d}$-N(-$L^{1-b}$-$D^{1-a}$)($L^{2-n}$-$D^{2-cb}$),
Exemplification 6-387: Z-$L^{3-d}$-N(-$L^{1-b}$-$D^{1-a}$)($L^{2-b}$-$D^{2-cg}$),
Exemplification 6-388: Z-$L^{3-d}$-N(-$L^{1-b}$-$D^{1-a}$)($L^{2-b}$-$D^{2-ch}$),
Exemplification 6-389: Z-$L^{3-d}$-N(-$L^{1-b}$-$D^{1-a}$)($L^{2-d}$-$D^{2-cg}$),
Exemplification 6-390: Z-$L^{3-d}$-N(-$L^{1-b}$-$D^{1-a}$)($L^{2-d}$-$D^{2-ch}$),
Exemplification 6-391: Z-$L^{3-d}$-N(-$L^{1-b}$-$D^{1-b}$)($L^{2-i}$-$D^{2-ca}$),
Exemplification 6-392: Z-$L^{3-d}$-N(-$L^{1-b}$-$D^{1-b}$)($L^{2-i}$-$D^{2-cc}$),
Exemplification 6-393: Z-$L^{3-d}$-N(-$L^{1-b}$-$D^{1-b}$)($L^{2-i}$-$D^{2-cd}$),
Exemplification 6-394: Z-$L^{3-d}$-N(-$L^{1-b}$-$D^{1-b}$)($L^{2-i}$-$D^{2-ce}$),
Exemplification 6-395: Z-$L^{3-d}$-N(-$L^{1-b}$-$D^{1-b}$)($L^{2-i}$-$D^{2-cf}$),
Exemplification 6-396: Z-$L^{3-d}$-N(-$L^{1-b}$-$D^{1-b}$)($L^{2-j}$-$D^{2-ca}$),
Exemplification 6-397: Z-$L^{3-d}$-N(-$L^{1-b}$-$D^{1-b}$)($L^{2-j}$-$D^{2-cc}$),
Exemplification 6-398: Z-$L^{3-d}$-N(-$L^{1-b}$-$D^{1-b}$)($L^{2-j}$-$D^{2-cd}$),
Exemplification 6-399: Z-$L^{3-d}$-N(-$L^{1-b}$-$D^{1-b}$)($L^{2-j}$-$D^{2-ce}$),
Exemplification 6-400: Z-$L^{3-d}$-N(-$L^{1-b}$-$D^{1-b}$)($L^{2-j}$-$D^{2-cf}$),
Exemplification 6-401: Z-$L^{3-d}$-N(-$L^{1-b}$-$D^{1-b}$)($L^{2-k}$-$D^{2-cb}$),
Exemplification 6-402: Z-$L^{3-d}$-N(-$L^{1-b}$-$D^{1-b}$)($L^{2-l}$-$D^{2-ca}$),
Exemplification 6-403: Z-$L^{3-d}$-N(-$L^{1-b}$-$D^{1-b}$)($L^{2-l}$-$D^{2-cc}$),
Exemplification 6-404: Z-$L^{3-d}$-N(-$L^{1-b}$-$D^{1-b}$)($L^{2-l}$-$D^{2-cd}$),

Exemplification 6-405: $Z-L^{3-d}-N(-L^{1-b}-D^{1-b})(L^{2-l}-D^{2-ce})$,
Exemplification 6-406: $Z-L^{3-d}-N(-L^{1-b}-D^{1-b})(L^{2-l}-D^{2-cf})$,
Exemplification 6-407: $Z-L^{3-d}-N(-L^{1-b}-D^{1-b})(L^{2-m}-D^{2-ca})$,
Exemplification 6-408: $Z-L^{3-d}-N(-L^{1-b}-D^{1-b})(L^{2-m}-D^{2-cc})$,
Exemplification 6-409: $Z-L^{3-d}-N(-L^{1-b}-D^{1-b})(L^{2-m}-D^{2-cd})$,
Exemplification 6-410: $Z-L^{3-d}-N(-L^{1-b}-D^{1-b})(L^{2-m}-D^{2-ce})$,
Exemplification 6-411: $Z-L^{3-d}-N(-L^{1-b}-D^{1-b})(L^{2-m}-D^{2-cf})$,
Exemplification 6-412: $Z-L^{3-d}-N(-L^{1-b}-D^{1-b})(L^{2-n}-D^{2-cb})$,
Exemplification 6-413: $Z-L^{3-d}-N(-L^{1-b}-D^{1-b})(L^{2-b}-D^{2-cg})$,
Exemplification 6-414: $Z-L^{3-d}-N(-L^{1-b}-D^{1-b})(L^{2-b}-D^{2-ch})$,
Exemplification 6-415: $Z-L^{3-d}-N(-L^{1-b}-D^{1-b})(L^{2-d}-D^{2-cg})$,
Exemplification 6-416: $Z-L^{3-d}-N(-L^{1-b}-D^{1-b})(L^{2-d}-D^{2-ch})$,
Exemplification 6-417: $Z-L^{3-e}-N(-L^{1-a}-D^{1-a})(L^{2-i}-D^{2-ca})$,
Exemplification 6-418: $Z-L^{3-e}-N(-L^{1-a}-D^{1-a})(L^{2-i}-D^{2-cc})$,
Exemplification 6-419: $Z-L^{3-e}-N(-L^{1-a}-D^{1-a})(L^{2-i}-D^{2-cd})$,
Exemplification 6-420: $Z-L^{3-e}-N(-L^{1-a}-D^{1-a})(L^{2-i}-D^{2-ce})$,
Exemplification 6-421: $Z-L^{3-e}-N(-L^{1-a}-D^{1-a})(L^{2-i}-D^{2-cf})$,
Exemplification 6-422: $Z-L^{3-e}-N(-L^{1-a}-D^{1-a})(L^{2-j}-D^{2-ca})$,
Exemplification 6-423: $Z-L^{3-e}-N(-L^{1-a}-D^{1-a})(L^{2-j}-D^{2-cc})$,
Exemplification 6-424: $Z-L^{3-e}-N(-L^{1-a}-D^{1-a})(L^{2-j}-D^{2-cd})$,
Exemplification 6-425: $Z-L^{3-e}-N(-L^{1-a}-D^{1-a})(L^{2-j}-D^{2-ce})$,
Exemplification 6-426: $Z-L^{3-e}-N(-L^{1-a}-D^{1-a})(L^{2-j}-D^{2-cf})$,
Exemplification 6-427: $Z-L^{3-e}-N(-L^{1-a}-D^{1-a})(L^{2-k}-D^{2-cb})$,
Exemplification 6-428: $Z-L^{3-e}-N(-L^{1-a}-D^{1-a})(L^{2-l}-D^{2-ca})$,
Exemplification 6-429: $Z-L^{3-e}-N(-L^{1-a}-D^{1-a})(L^{2-l}-D^{2-cc})$,
Exemplification 6-430: $Z-L^{3-e}-N(-L^{1-a}-D^{1-a})(L^{2-l}-D^{2-cd})$,
Exemplification 6-431: $Z-L^{3-e}-N(-L^{1-a}-D^{1-a})(L^{2-l}-D^{2-ce})$,
Exemplification 6-432: $Z-L^{3-e}-N(-L^{1-a}-D^{1-a})(L^{2-l}-D^{2-cf})$,
Exemplification 6-433: $Z-L^{3-e}-N(-L^{1-a}-D^{1-a})(L^{2-m}-D^{2-ca})$,
Exemplification 6-434: $Z-L^{3-e}-N(-L^{1-a}-D^{1-a})(L^{2-m}-D^{2-cc})$,
Exemplification 6-435: $Z-L^{3-e}-N(-L^{1-a}-D^{1-a})(L^{2-m}-D^{2-cd})$,
Exemplification 6-436: $Z-L^{3-e}-N(-L^{1-a}-D^{1-a})(L^{2-m}-D^{2-ce})$,
Exemplification 6-437: $Z-L^{3-e}-N(-L^{1-a}-D^{1-a})(L^{2-m}-D^{2-cf})$,
Exemplification 6-438: $Z-L^{3-e}-N(-L^{1-a}-D^{1-a})(L^{2-n}-D^{2-cb})$,
Exemplification 6-439: $Z-L^{3-e}-N(-L^{1-a}-D^{1-a})(L^{2-b}-D^{2-cg})$,
Exemplification 6-440: $Z-L^{3-e}-N(-L^{1-a}-D^{1-a})(L^{2-b}-D^{2-ch})$,
Exemplification 6-441: $Z-L^{3-e}-N(-L^{1-a}-D^{1-a})(L^{2-d}-D^{2-cg})$,
Exemplification 6-442: $Z-L^{3-e}-N(-L^{1-a}-D^{1-a})(L^{2-d}-D^{2-ch})$,
Exemplification 6-443: $Z-L^{3-e}-N(-L^{1-a}-D^{1-b})(L^{2-i}-D^{2-ca})$,
Exemplification 6-444: $Z-L^{3-e}-N(-L^{1-a}-D^{1-b})(L^{2-i}-D^{2-cc})$,
Exemplification 6-445: $Z-L^{3-e}-N(-L^{1-a}-D^{1-b})(L^{2-i}-D^{2-cd})$,
Exemplification 6-446: $Z-L^{3-e}-N(-L^{1-a}-D^{1-b})(L^{2-i}-D^{2-ce})$,
Exemplification 6-447: $Z-L^{3-e}-N(-L^{1-a}-D^{1-b})(L^{2-i}-D^{2-cf})$,
Exemplification 6-448: $Z-L^{3-e}-N(-L^{1-a}-D^{1-b})(L^{2-j}-D^{2-ca})$,
Exemplification 6-449: $Z-L^{3-e}-N(-L^{1-a}-D^{1-b})(L^{2-j}-D^{2-cc})$,
Exemplification 6-450: $Z-L^{3-e}-N(-L^{1-a}-D^{1-b})(L^{2-j}-D^{2-cd})$,
Exemplification 6-451: $Z-L^{3-e}-N(-L^{1-a}-D^{1-b})(L^{2-j}-D^{2-ce})$,
Exemplification 6-452: $Z-L^{3-e}-N(-L^{1-a}-D^{1-b})(L^{2-j}-D^{2-cf})$,
Exemplification 6-453: $Z-L^{3-e}-N(-L^{1-a}-D^{1-b})(L^{2-k}-D^{2-cb})$,
Exemplification 6-454: $Z-L^{3-e}-N(-L^{1-a}-D^{1-b})(L^{2-l}-D^{2-ca})$,
Exemplification 6-455: $Z-L^{3-e}-N(-L^{1-a}-D^{1-b})(L^{2-l}-D^{2-cc})$,
Exemplification 6-456: $Z-L^{3-e}-N(-L^{1-a}-D^{1-b})(L^{2-l}-D^{2-cd})$,
Exemplification 6-457: $Z-L^{3-e}-N(-L^{1-a}-D^{1-b})(L^{2-l}-D^{2-ce})$,
Exemplification 6-458: $Z-L^{3-e}-N(-L^{1-a}-D^{1-b})(L^{2-l}-D^{2-cf})$,
Exemplification 6-459: $Z-L^{3-e}-N(-L^{1-a}-D^{1-b})(L^{2-m}-D^{2-ca})$,
Exemplification 6-460: $Z-L^{3-e}-N(-L^{1-a}-D^{1-b})(L^{2-m}-D^{2-cc})$,
Exemplification 6-461: $Z-L^{3-e}-N(-L^{1-a}-D^{1-b})(L^{2-m}-D^{2-cd})$,
Exemplification 6-462: $Z-L^{3-e}-N(-L^{1-a}-D^{1-b})(L^{2-m}-D^{2-ce})$,

Exemplification 6-463: Z-$L^{3-e}$-N(-$L^{1-a}$-$D^{1-b}$)($L^{2-m}$-$D^{2-cf}$),
Exemplification 6-464: Z-$L^{3-e}$-N(-$L^{1-a}$-$D^{1-b}$)($L^{2-n}$-$D^{2-cb}$),
Exemplification 6-465: Z-$L^{3-e}$-N(-$L^{1-a}$-$D^{1-b}$)($L^{2-b}$-$D^{2-cg}$),
Exemplification 6-466: Z-$L^{3-e}$-N(-$L^{1-a}$-$D^{1-b}$)($L^{2-b}$-$D^{2-ch}$),
Exemplification 6-467: Z-$L^{3-e}$-N(-$L^{1-a}$-$D^{1-b}$)($L^{2-d}$-$D^{2-cg}$),
Exemplification 6-468: Z-$L^{3-e}$-N(-$L^{1-a}$-$D^{1-b}$)($L^{2-d}$-$D^{2-ch}$),
Exemplification 6-469: Z-$L^{3-e}$-N(-$L^{1-b}$-$D^{1-a}$)($L^{2-i}$-$D^{2-ca}$),
Exemplification 6-470: Z-$L^{3-e}$-N(-$L^{1-b}$-$D^{1-a}$)($L^{2-i}$-$D^{2-cc}$),
Exemplification 6-471: Z-$L^{3-e}$-N(-$L^{1-b}$-$D^{1-a}$)($L^{2-i}$-$D^{2-cd}$),
Exemplification 6-472: Z-$L^{3-e}$-N(-$L^{1-b}$-$D^{1-a}$)($L^{2-i}$-$D^{2-ce}$),
Exemplification 6-473: Z-$L^{3-e}$-N(-$L^{1-b}$-$D^{1-a}$)($L^{2-i}$-$D^{2-cf}$),
Exemplification 6-474: Z-$L^{3-e}$-N(-$L^{1-b}$-$D^{1-a}$)($L^{2-j}$-$D^{2-ca}$),
Exemplification 6-475: Z-$L^{3-e}$-N(-$L^{1-b}$-$D^{1-a}$)($L^{2-j}$-$D^{2-cc}$),
Exemplification 6-476: Z-$L^{3-e}$-N(-$L^{1-b}$-$D^{1-a}$)($L^{2-j}$-$D^{2-cd}$),
Exemplification 6-477: Z-$L^{3-e}$-N(-$L^{1-b}$-$D^{1-a}$)($L^{2-j}$-$D^{2-ce}$),
Exemplification 6-478: Z-$L^{3-e}$-N(-$L^{1-b}$-$D^{1-a}$)($L^{2-j}$-$D^{2-cf}$),
Exemplification 6-479: Z-$L^{3-e}$-N(-$L^{1-b}$-$D^{1-a}$)($L^{2-k}$-$D^{2-cb}$),
Exemplification 6-480: Z-$L^{3-e}$-N(-$L^{1-b}$-$D^{1-a}$)($L^{2-l}$-$D^{2-ca}$),
Exemplification 6-481: Z-$L^{3-e}$-N(-$L^{1-b}$-$D^{1-a}$)($L^{2-l}$-$D^{2-cc}$),
Exemplification 6-482: Z-$L^{3-e}$-N(-$L^{1-b}$-$D^{1-a}$)($L^{2-l}$-$D^{2-cd}$),
Exemplification 6-483: Z-$L^{3-e}$-N(-$L^{1-b}$-$D^{1-a}$)($L^{2-l}$-$D^{2-ce}$),
Exemplification 6-484: Z-$L^{3-e}$-N(-$L^{1-b}$-$D^{1-a}$)($L^{2-l}$-$D^{2-cf}$),
Exemplification 6-485: Z-$L^{3-e}$-N(-$L^{1-b}$-$D^{1-a}$)($L^{2-m}$-$D^{2-ca}$),
Exemplification 6-486: Z-$L^{3-e}$-N(-$L^{1-b}$-$D^{1-a}$)($L^{2-m}$-$D^{2-cc}$),
Exemplification 6-487: Z-$L^{3-e}$-N(-$L^{1-b}$-$D^{1-a}$)($L^{2-m}$-$D^{2-cd}$),
Exemplification 6-488: Z-$L^{3-e}$-N(-$L^{1-b}$-$D^{1-a}$)($L^{2-m}$-$D^{2-ce}$),
Exemplification 6-489: Z-$L^{3-e}$-N(-$L^{1-b}$-$D^{1-a}$)($L^{2-m}$-$D^{2-cf}$),
Exemplification 6-490: Z-$L^{3-e}$-N(-$L^{1-b}$-$D^{1-a}$)($L^{2-n}$-$D^{2-cb}$),
Exemplification 6-491: Z-$L^{3-e}$-N(-$L^{1-b}$-$D^{1-a}$)($L^{2-b}$-$D^{2-cg}$),
Exemplification 6-492: Z-$L^{3-e}$-N(-$L^{1-b}$-$D^{1-a}$)($L^{2-b}$-$D^{2-ch}$),
Exemplification 6-493: Z-$L^{3-e}$-N(-$L^{1-b}$-$D^{1-a}$)($L^{2-d}$-$D^{2-cg}$),
Exemplification 6-494: Z-$L^{3-e}$-N(-$L^{1-b}$-$D^{1-a}$)($L^{2-d}$-$D^{2-ch}$),
Exemplification 6-495: Z-$L^{3-e}$-N(-$L^{1-b}$-$D^{1-b}$)($L^{2-i}$-$D^{2-ca}$),
Exemplification 6-496: Z-$L^{3-e}$-N(-$L^{1-b}$-$D^{1-b}$)($L^{2-i}$-$D^{2-cc}$),
Exemplification 6-497: Z-$L^{3-e}$-N(-$L^{1-b}$-$D^{1-b}$)($L^{2-i}$-$D^{2-cd}$),
Exemplification 6-498: Z-$L^{3-e}$-N(-$L^{1-b}$-$D^{1-b}$)($L^{2-i}$-$D^{2-ce}$),
Exemplification 6-499: Z-$L^{3-e}$-N(-$L^{1-b}$-$D^{1-b}$)($L^{2-i}$-$D^{2-cf}$),
Exemplification 6-500: Z-$L^{3-e}$-N(-$L^{1-b}$-$D^{1-b}$)($L^{2-j}$-$D^{2-ca}$),
Exemplification 6-501: Z-$L^{3-e}$-N(-$L^{1-b}$-$D^{1-b}$)($L^{2-j}$-$D^{2-cc}$),
Exemplification 6-502: Z-$L^{3-e}$-N(-$L^{1-b}$-$D^{1-b}$)($L^{2-j}$-$D^{2-cd}$),
Exemplification 6-503: Z-$L^{3-e}$-N(-$L^{1-b}$-$D^{1-b}$)($L^{2-j}$-$D^{2-ce}$),
Exemplification 6-504: Z-$L^{3-e}$-N(-$L^{1-b}$-$D^{1-b}$)($L^{2-j}$-$D^{2-cf}$),
Exemplification 6-505: Z-$L^{3-e}$-N(-$L^{1-b}$-$D^{1-b}$)($L^{2-k}$-$D^{2-cb}$),
Exemplification 6-506: Z-$L^{3-e}$-N(-$L^{1-b}$-$D^{1-b}$)($L^{2-l}$-$D^{2-ca}$),
Exemplification 6-507: Z-$L^{3-e}$-N(-$L^{1-b}$-$D^{1-b}$)($L^{2-l}$-$D^{2-cc}$),
Exemplification 6-508: Z-$L^{3-e}$-N(-$L^{1-b}$-$D^{1-b}$)($L^{2-l}$-$D^{2-cd}$),
Exemplification 6-509: Z-$L^{3-e}$-N(-$L^{1-b}$-$D^{1-b}$)($L^{2-l}$-$D^{2-ce}$),
Exemplification 6-510: Z-$L^{3-e}$-N(-$L^{1-b}$-$D^{1-b}$)($L^{2-l}$-$D^{2-cf}$),
Exemplification 6-511: Z-$L^{3-e}$-N(-$L^{1-b}$-$D^{1-b}$)($L^{2-m}$-$D^{2-ca}$),
Exemplification 6-512: Z-$L^{3-e}$-N(-$L^{1-b}$-$D^{1-b}$)($L^{2-m}$-$D^{2-cc}$),
Exemplification 6-513: Z-$L^{3-e}$-N(-$L^{1-b}$-$D^{1-b}$)($L^{2-m}$-$D^{2-cd}$),
Exemplification 6-514: Z-$L^{3-e}$-N(-$L^{1-b}$-$D^{1-b}$)($L^{2-m}$-$D^{2-ce}$),
Exemplification 6-515: Z-$L^{3-e}$-N(-$L^{1-b}$-$D^{1-b}$)($L^{2-m}$-$D^{2-cf}$),
Exemplification 6-516: Z-$L^{3-e}$-N(-$L^{1-b}$-$D^{1-b}$)($L^{2-n}$-$D^{2-cb}$),
Exemplification 6-517: Z-$L^{3-e}$-N(-$L^{1-b}$-$D^{1-b}$)($L^{2-b}$-$D^{2-cg}$),
Exemplification 6-518: Z-$L^{3-e}$-N(-$L^{1-b}$-$D^{1-b}$)($L^{2-b}$-$D^{2-ch}$),
Exemplification 6-519: Z-$L^{3-e}$-N(-$L^{1-b}$-$D^{1-b}$)($L^{2-d}$-$D^{2-cg}$),
Exemplification 6-520: Z-$L^{3-e}$-N(-$L^{1-b}$-$D^{1-b}$)($L^{2-d}$-$D^{2-ch}$),

Exemplification 6-521: Z-$L^{3-f}$-N(-$L^{1-a}$-$D^{1-a}$)($L^{2-i}$-$D^{2-ca}$),
Exemplification 6-522: Z-$L^{3-f}$-N(-$L^{1-a}$-$D^{1-a}$)($L^{2-i}$-$D^{2-cc}$),
Exemplification 6-523: Z-$L^{3-f}$-N(-$L^{1-a}$-$D^{1-a}$)($L^{2-i}$-$D^{2-cd}$),
Exemplification 6-524: Z-$L^{3-f}$-N(-$L^{1-a}$-$D^{1-a}$)($L^{2-i}$-$D^{2-ce}$),
Exemplification 6-525: Z-$L^{3-f}$-N(-$L^{1-a}$-$D^{1-a}$)($L^{2-i}$-$D^{2-cf}$),
Exemplification 6-526: Z-$L^{3-f}$-N(-$L^{1-a}$-$D^{1-a}$)($L^{2-j}$-$D^{2-ca}$),
Exemplification 6-527: Z-$L^{3-f}$-N(-$L^{1-a}$-$D^{1-a}$)($L^{2-j}$-$D^{2-cc}$),
Exemplification 6-528: Z-$L^{3-f}$-N(-$L^{1-a}$-$D^{1-a}$)($L^{2-j}$-$D^{2-cd}$),
Exemplification 6-529: Z-$L^{3-f}$-N(-$L^{1-a}$-$D^{1-a}$)($L^{2-j}$-$D^{2-ce}$),
Exemplification 6-530: Z-$L^{3-f}$-N(-$L^{1-a}$-$D^{1-a}$)($L^{2-j}$-$D^{2-cf}$),
Exemplification 6-531: Z-$L^{3-f}$-N(-$L^{1-a}$-$D^{1-a}$)($L^{2-k}$-$D^{2-cb}$),
Exemplification 6-532: Z-$L^{3-f}$-N(-$L^{1-a}$-$D^{1-a}$)($L^{2-l}$-$D^{2-ca}$),
Exemplification 6-533: Z-$L^{3-f}$-N(-$L^{1-a}$-$D^{1-a}$)($L^{2-l}$-$D^{2-cc}$),
Exemplification 6-534: Z-$L^{3-f}$-N(-$L^{1-a}$-$D^{1-a}$)($L^{2-l}$-$D^{2-cd}$),
Exemplification 6-535: Z-$L^{3-f}$-N(-$L^{1-a}$-$D^{1-a}$)($L^{2-l}$-$D^{2-ce}$),
Exemplification 6-536: Z-$L^{3-f}$-N(-$L^{1-a}$-$D^{1-a}$)($L^{2-l}$-$D^{2-cf}$),
Exemplification 6-537: Z-$L^{3-f}$-N(-$L^{1-a}$-$D^{1-a}$)($L^{2-m}$-$D^{2-ca}$),
Exemplification 6-538: Z-$L^{3-f}$-N(-$L^{1-a}$-$D^{1-a}$)($L^{2-m}$-$D^{2-cc}$),
Exemplification 6-539: Z-$L^{3-f}$-N(-$L^{1-a}$-$D^{1-a}$)($L^{2-m}$-$D^{2-cd}$),
Exemplification 6-540: Z-$L^{3-f}$-N(-$L^{1-a}$-$D^{1-a}$)($L^{2-m}$-$D^{2-ce}$),
Exemplification 6-541: Z-$L^{3-f}$-N(-$L^{1-a}$-$D^{1-a}$)($L^{2-m}$-$D^{2-cf}$),
Exemplification 6-542: Z-$L^{3-f}$-N(-$L^{1-a}$-$D^{1-a}$)($L^{2-n}$-$D^{2-cb}$),
Exemplification 6-543: Z-$L^{3-f}$-N(-$L^{1-a}$-$D^{1-a}$)($L^{2-b}$-$D^{2-cg}$),
Exemplification 6-544: Z-$L^{3-f}$-N(-$L^{1-a}$-$D^{1-a}$)($L^{2-b}$-$D^{2-ch}$),
Exemplification 6-545: Z-$L^{3-f}$-N(-$L^{1-a}$-$D^{1-a}$)($L^{2-d}$-$D^{2-cg}$),
Exemplification 6-546: Z-$L^{3-f}$-N(-$L^{1-a}$-$D^{1-a}$)($L^{2-d}$-D2$^{-ch}$),
Exemplification 6-547: Z-$L^{3-f}$-N(-$L^{1-a}$-$D^{1-b}$)($L^{2-i}$-$D^{2-ca}$),
Exemplification 6-548: Z-$L^{3-f}$-N(-$L^{1-a}$-$D^{1-b}$)($L^{2-i}$-$D^{2-cc}$),
Exemplification 6-549: Z-$L^{3-f}$-N(-$L^{1-a}$-$D^{1-b}$)($L^{2-i}$-$D^{2-cd}$),
Exemplification 6-550: Z-$L^{3-f}$-N(-$L^{1-a}$-$D^{1-b}$)($L^{2-i}$-$D^{2-ce}$),
Exemplification 6-551: Z-$L^{3-f}$-N(-$L^{1-a}$-$D^{1-b}$)($L^{2-i}$-$D^{2-cf}$),
Exemplification 6-552: Z-$L^{3-f}$-N(-$L^{1-a}$-$D^{1-b}$)($L^{2-3}$-$D^{2-ca}$),
Exemplification 6-553: Z-$L^{3-f}$-N(-$L^{1-a}$-$D^{1-b}$)($L^{2-j}$-$D^{2-cc}$),
Exemplification 6-554: Z-$L^{3-f}$-N(-$L^{1-a}$-$D^{1-b}$)($L^{2-j}$-$D^{2-cd}$),
Exemplification 6-555: Z-$L^{3-f}$-N(-$L^{1-a}$-$D^{1-b}$)($L^{2-j}$-$D^{2-ce}$),
Exemplification 6-556: Z-$L^{3-f}$-N(-$L^{1-a}$-$D^{1-b}$)($L^{2-j}$-$D^{2-cf}$),
Exemplification 6-557: Z-$L^{3-f}$-N(-$L^{1-a}$-$D^{1-b}$)($L^{2-k}$-$D^{2-cb}$),
Exemplification 6-558: Z-$L^{3-f}$-N(-$L^{1-a}$-$D^{1-b}$)($L^{2-1}$-$D^{2-cb}$),
Exemplification 6-559: Z-$L^{3-f}$-N(-$L^{1-a}$-$D^{1-b}$)($L^{2-1}$-$D^{2-cc}$),
Exemplification 6-560: Z-$L^{3-f}$-N(-$L^{1-a}$-$D^{1-b}$)($L^{2-1}$-$D^{2-cd}$),
Exemplification 6-561: Z-$L^{3-f}$-N(-$L^{1-a}$-$D^{1-b}$)($L^{2-1}$-$D^{2-ce}$),
Exemplification 6-562: Z-$L^{3-f}$-N(-$L^{1-a}$-$D^{1-b}$)($L^{2-1}$-$D^{2-cf}$),
Exemplification 6-563: Z-$L^{3-f}$-N(-$L^{1-a}$-$D^{1-b}$)($L^{2-m}$-$D^{2-ca}$),
Exemplification 6-564: Z-$L^{3-f}$-N(-$L^{1-a}$-$D^{1-b}$)($L^{2-m}$-$D^{2-cc}$),
Exemplification 6-565: Z-$L^{3-f}$-N(-$L^{1-a}$-$D^{1-b}$)($L^{2-m}$-$D^{2-cd}$),
Exemplification 6-566: Z-$L^{3-f}$-N(-$L^{1-a}$-$D^{1-b}$)($L^{2-m}$-$D^{2-ce}$),
Exemplification 6-567: Z-$L^{3-f}$-N(-$L^{1-a}$-$D^{1-b}$)($L^{2-m}$-$D^{2-cf}$),
Exemplification 6-568: Z-$L^{3-f}$-N(-$L^{1-a}$-$D^{1-b}$)($L^{2-n}$-$D^{2-cb}$),
Exemplification 6-569: Z-$L^{3-f}$-N(-$L^{1-a}$-$D^{1-b}$)($L^{2-b}$-$D^{2-cg}$),
Exemplification 6-570: Z-$L^{3-f}$-N(-$L^{1-a}$-$D^{1-b}$)($L^{2-b}$-$D^{2-ch}$),
Exemplification 6-571: Z-$L^{3-f}$-N(-$L^{1-a}$-$D^{1-b}$)($L^{2-d}$-$D^{2-cg}$),
Exemplification 6-572: Z-$L^{3-f}$-N(-$L^{1-a}$-$D^{1-b}$)($L^{2-d}$-D2$^{-ch}$),
Exemplification 6-573: Z-$L^{3-f}$-N(-$L^{1-b}$-$D^{1-a}$)($L^{2-i}$-$D^{2-ca}$),
Exemplification 6-574: Z-$L^{3-f}$-N(-$L^{1-b}$-$D^{1-a}$)($L^{2-i}$-$D^{2-cc}$),
Exemplification 6-575: Z-$L^{3-f}$-N(-$L^{1-b}$-$D^{1-a}$)($L^{2-i}$-$D^{2-cd}$),
Exemplification 6-576: Z-$L^{3-f}$-N(-$L^{1-b}$-$D^{1-a}$)($L^{2-i}$-$D^{2-ce}$),
Exemplification 6-577: Z-$L^{3-f}$-N(-$L^{1-b}$-$D^{1-a}$)($L^{2-i}$-$D^{2-cf}$),
Exemplification 6-578: Z-$L^{3-f}$-N(-$L^{1-b}$-$D^{1-a}$)($L^{2-3}$-$D^{2-ca}$),

Exemplification 6-579: Z-$L^{3-f}$-N(-$L^{1-b}$-$D^{1-a}$)($L^{2-j}$-$D^{2-cc}$),
Exemplification 6-580: Z-$L^{3-f}$-N(-$L^{1-b}$-$D^{1-a}$) ($L^{2-j}$-$D^{2-cd}$),
Exemplification 6-581: Z-$L^{3-f}$-N(-$L^{1-b}$-$D^{1-a}$)($L^{2-j}$-$D^{2-ce}$),
Exemplification 6-582: Z-$L^{3-f}$-N(-$L^{1-b}$-$D^{1-a}$)($L^{2-j}$-$D^{2-cf}$),
Exemplification 6-583: Z-$L^{3-f}$-N(-$L^{1-b}$-$D^{1-a}$)($L^{2-k}$-$D^{2-cb}$),
Exemplification 6-584: Z-$L^{3-f}$-N(-$L^{1-b}$-$D^{1-a}$)($L^{2-1}$-$D^{2-cb}$),
Exemplification 6-585: Z-$L^{3-f}$-N(-$L^{1-b}$-$D^{1-a}$)($L^{2-1}$-$D^{2-cc}$),
Exemplification 6-586: Z-$L^{3-f}$-N(-$L^{1-b}$-$D^{1-a}$)($L^{2-1}$-$D^{2-cd}$),
Exemplification 6-587: Z-$L^{3-f}$-N(-$L^{1-b}$-$D^{1-a}$)($L^{2-1}$-$D^{2-ce}$),
Exemplification 6-588: Z-$L^{3-f}$-N(-$L^{1-b}$-$D^{1-a}$)($L^{2-1}$-$D^{2-cf}$),
Exemplification 6-589: Z-$L^{3-f}$-N(-$L^{1-b}$-$D^{1-a}$)($L^{2-m}$-$D^{2-ca}$),
Exemplification 6-590: Z-$L^{3-f}$-N(-$L^{1-b}$-$D^{1-a}$)($L^{2-m}$-$D^{2-cc}$),
Exemplification 6-591: Z-$L^{3-f}$-N(-$L^{1-b}$-$D^{1-a}$)($L^{2-m}$-$D^{2-cd}$),
Exemplification 6-592: Z-$L^{3-f}$-N(-$L^{1-b}$-$D^{1-a}$)($L^{2-m}$-$D^{2-ce}$),
Exemplification 6-593: Z-$L^{3-f}$-N(-$L^{1-b}$-$D^{1-a}$)($L^{2-m}$-$D^{2-cf}$),
Exemplification 6-594: Z-$L^{3-f}$-N(-$L^{1-b}$-$D^{1-a}$)($L^{2-n}$-$D^{2-cb}$),
Exemplification 6-595: Z-$L^{3-f}$-N(-$L^{1-b}$-$D^{1-a}$) ($L^{2-b}$-$D^{2-cg}$),
Exemplification 6-596: Z-$L^{3-f}$-N(-$L^{1-b}$-$D^{1-a}$)($L^{2-b}$-$D^{2-ch}$),
Exemplification 6-597: Z-$L^{3-f}$-N(-$L^{1-b}$-$D^{1-a}$) ($L^{2-d}$-$D^{2-cg}$),
Exemplification 6-598: Z-$L^{3-f}$-N(-$L^{1-b}$-$D^{1-a}$)($L^{2-d}$-$D2^{-ch}$),
Exemplification 6-599: Z-$L^{3-f}$-N(-$L^{1-b}$-$D^{1-b}$)($L^{2-i}$-$D^{2-ca}$),
Exemplification 6-600: Z-$L^{3-f}$-N(-$L^{1-b}$-$D^{1-b}$)($L^{2-i}$-$D^{2-cc}$),
Exemplification 6-601: Z-$L^{3-f}$-N(-$L^{1-b}$-$D^{1-b}$)($L^{2-i}$-$D^{2-cd}$),
Exemplification 6-602: Z-$L^{3-f}$-N(-$L^{1-b}$-$D^{1-b}$)($L^{2-i}$-$D^{2-ce}$),
Exemplification 6-603: Z-$L^{3-f}$-N(-$L^{1-b}$-$D^{1-b}$)($L^{2-i}$-$D^{2-cf}$),
Exemplification 6-604: Z-$L^{3-f}$-N(-$L^{1-b}$-$D^{1-b}$)($L^{2-3}$-$D^{2-ca}$),
Exemplification 6-605: Z-$L^{3-f}$-N(-$L^{1-b}$-$D^{1-b}$)($L^{2-j}$-$D^{2-cc}$),
Exemplification 6-606: Z-$L^{3-f}$-N(-$L^{1-b}$-$D^{1-b}$)($L^{2-j}$-$D^{2-cd}$),
Exemplification 6-607: Z-$L^{3-f}$-N(-$L^{1-b}$-$D^{1-b}$)($L^{2-j}$-$D^{2-ce}$),
Exemplification 6-608: Z-$L^{3-f}$-N(-$L^{1-b}$-$D^{1-b}$)($L^{2-j}$-$D2^{-cf}$),
Exemplification 6-609: Z-$L^{3-f}$-N(-$L^{1-b}$-$D^{1-b}$)($L^{2-k}$-$D^{2-cb}$),
Exemplification 6-610: Z-$L^{3-f}$-N(-$L^{1-b}$-$D^{1-b}$)($L^{2-1}$-$D^{2-cb}$),
Exemplification 6-611: Z-$L^{3-f}$-N(-$L^{1-b}$-$D^{1-b}$)($L^{2-1}$-$D^{2-cc}$),
Exemplification 6-612: Z-$L^{3-f}$-N(-$L^{1-b}$-$D^{1-b}$)($L^{2-1}$-$D^{2-cd}$),
Exemplification 6-613: Z-$L^{3-f}$-N(-$L^{1-b}$-$D^{1-b}$)($L^{2-1}$-$D^{2-ce}$),
Exemplification 6-614: Z-$L^{3-f}$-N(-$L^{1-b}$-$D^{1-b}$)($L^{2-1}$-$D^{2-cf}$),
Exemplification 6-615: Z-$L^{3-f}$-N(-$L^{1-b}$-$D^{1-b}$)($L^{2-m}$-$D^{2-ca}$),
Exemplification 6-616: Z-$L^{3-f}$-N(-$L^{1-b}$-$D^{1-b}$)($L^{2-m}$-$D^{2-cc}$),
Exemplification 6-617: Z-$L^{3-f}$-N(-$L^{1-b}$-$D^{1-b}$)($L^{2-m}$-$D^{2-cd}$),
Exemplification 6-618: Z-$L^{3-f}$-N(-$L^{1-b}$-$D^{1-b}$)($L^{2-m}$-$D^{2-ce}$),
Exemplification 6-619: Z-$L^{3-f}$-N(-$L^{1-b}$-$D^{1-b}$)($L^{2-m}$-$D^{2-cf}$),
Exemplification 6-620: Z-$L^{3-f}$-N(-$L^{1-b}$-$D^{1-b}$)($L^{2-n}$-$D^{2-cb}$),
Exemplification 6-621: Z-$L^{3-f}$-N(-$L^{1-b}$-$D^{1-b}$)($L^{2-b}$-$D^{2-cg}$),
Exemplification 6-622: Z-$L^{3-f}$-N(-$L^{1-b}$-$D^{1-b}$)($L^{2-b}$-$D^{2-ch}$),
Exemplification 6-623: Z-$L^{3-f}$-N(-$L^{1-b}$-$D^{1-b}$)($L^{2-d}$-$D^{2-cg}$),
Exemplification 6-624: Z-$L^{3-f}$-N(-$L^{1-b}$-$D^{1-b}$)($L^{2-d}$-$D2^{-ch}$),
Exemplification 6-625: Z-$L^{3-g}$-N(-$L^{1-a}$-$D^{1-a}$)($L^{2-i}$-$D^{2-ca}$),
Exemplification 6-626: Z-$L^{3-g}$-N(-$L^{1-a}$-$D^{1-a}$)($L^{2-i}$-$D^{2-cc}$),
Exemplification 6-627: Z-$L^{3-g}$-N(-$L^{1-a}$-$D^{1-a}$)($L^{2-i}$-$D^{2-cd}$),
Exemplification 6-628: Z-$L^{3-g}$-N(-$L^{1-a}$-$D^{1-a}$)($L^{2-i}$-$D^{2-ce}$),
Exemplification 6-629: Z-$L^{3-g}$-N(-$L^{1-a}$-$D^{1-a}$)($L^{2-i}$-$D^{2-cf}$),
Exemplification 6-630: Z-$L^{3-g}$-N(-$L^{1-a}$-$D^{1-a}$)($L^{2-3}$-$D^{2-ca}$),
Exemplification 6-631: Z-$L^{3-g}$-N(-$L^{1-a}$-$D^{1-a}$)($L^{2-j}$-$D^{2-cc}$),
Exemplification 6-632: Z-$L^{3-g}$-N(-$L^{1-a}$-$D^{1-a}$)($L^{2-j}$-$D^{2-cd}$),
Exemplification 6-633: Z-$L^{3-g}$-N(-$L^{1-a}$-$D^{1-a}$)($L^{2-j}$-$D^{2-ce}$),
Exemplification 6-634: Z-$L^{3-g}$-N(-$L^{1-a}$-$D^{1-a}$)($L^{2-j}$-$D2^{-cf}$),
Exemplification 6-635: Z-$L^{3-g}$-N(-$L^{1-a}$-$D^{1-a}$)($L^{2-k}$-$D^{2-cb}$),
Exemplification 6-636: Z-$L^{3-g}$-N(-$L^{1-a}$-$D^{1-a}$)($L^{2-1}$-$D^{2-ca}$),

Exemplification 6-637: $Z-L^{3-g}-N(-L^{1-a}-D^{1-a})(L^{2-l}-D^{2-cc})$,

Exemplification 6-638: $Z-L^{3-g}-N(-L^{1-a}-D^{1-a})(L^{2-l}-D^{2-cd})$,

Exemplification 6-639: $Z-L^{3-g}-N(-L^{1-a}-D^{1-a})(L^{2-l}-D^{2-ce})$,

Exemplification 6-640: $Z-L^{3-g}-N(-L^{1-a}-D^{1-a})(L^{2-l}-D^{2-cf})$,

Exemplification 6-641: $Z-L^{3-g}-N(-L^{1-a}-D^{1-a})(L^{2-m}-D^{2-ca})$,

Exemplification 6-642: $Z-L^{3-g}-N(-L^{1-a}-D^{1-a})(L^{2-m}-D^{2-cc})$,

Exemplification 6-643: $Z-L^{3-g}-N(-L^{1-a}-D^{1-a})(L^{2-m}-D^{2-cd})$,

Exemplification 6-644: $Z-L^{3-g}-N(-L^{1-a}-D^{1-a})(L^{2-m}-D^{2-ce})$,

Exemplification 6-645: $Z-L^{3-g}-N(-L^{1-a}-D^{1-a})(L^{2-m}-D^{2-cf})$,

Exemplification 6-646: $Z-L^{3-g}-N(-L^{1-a}-D^{1-a})(L^{2-n}-D^{2-cb})$,

Exemplification 6-647: $Z-L^{3-g}-N(-L^{1-a}-D^{1-a})(L^{2-b}-D^{2-cg})$,

Exemplification 6-648: $Z-L^{3-g}-N(-L^{1-a}-D^{1-a})(L^{2-b}-D^{2-ch})$,

Exemplification 6-649: $Z-L^{3-g}-N(-L^{1-a}-D^{1-a})(L^{2-d}-D^{2-cg})$,

Exemplification 6-650: $Z-L^{3-g}-N(-L^{1-a}-D^{1-a})(L^{2-d}-D2^{-ch})$,

Exemplification 6-651: $Z-L^{3-g}-N(-L^{1-a}-D^{1-b})(L^{2-i}-D^{2-ca})$,

Exemplification 6-652: $Z-L^{3-g}-N(-L^{1-a}-D^{1-b})(L^{2-i}-D^{2-cc})$,

Exemplification 6-653: $Z-L^{3-g}-N(-L^{1-a}-D^{1-b})(L^{2-i}-D^{2-cd})$,

Exemplification 6-654: $Z-L^{3-g}-N(-L^{1-a}-D^{1-b})(L^{2-i}-D^{2-ce})$,

Exemplification 6-655: $Z-L^{3-g}-N(-L^{1-a}-D^{1-b})(L^{2-i}-D^{2-cf})$,

Exemplification 6-656: $Z-L^{3-g}-N(-L^{1-a}-D^{1-b})(L^{2-3}-D^{2-ca})$,

Exemplification 6-657: $Z-L^{3-g}-N(-L^{1-a}-D^{1-b})(L^{2-j}-D^{2-cc})$,

Exemplification 6-658: $Z-L^{3-g}-N(-L^{1-a}-D^{1-b})(L^{2-j}-D^{2-cd})$,

Exemplification 6-659: $Z-L^{3-g}-N(-L^{1-a}-D^{1-b})(L^{2-j}-D^{2-ce})$,

Exemplification 6-660: $Z-L^{3-g}-N(-L^{1-a}-D^{1-b})(L^{2-j}-D2^{-cf})$,

Exemplification 6-661: $Z-L^{3-g}-N(-L^{1-a}-D^{1-b})(L^{2-k}-D^{2-cb})$,

Exemplification 6-662: $Z-L^{3-g}-N(-L^{1-a}-D^{1-b})(L^{2-1}-D^{2-cb})$,

Exemplification 6-663: $Z-L^{3-g}-N(-L^{1-a}-D^{1-b})(L^{2-l}-D^{2-cc})$,

Exemplification 6-664: $Z-L^{3-g}-N(-L^{1-a}-D^{1-b})(L^{2-l}-D^{2-cd})$,

Exemplification 6-665: $Z-L^{3-g}-N(-L^{1-a}-D^{1-b})(L^{2-l}-D^{2-ce})$,

Exemplification 6-666: $Z-L^{3-g}-N(-L^{1-a}-D^{1-b})(L^{2-l}-D^{2-cf})$,

Exemplification 6-667: $Z-L^{3-g}-N(-L^{1-a}-D^{1-b})(L^{2-m}-D^{2-ca})$,

Exemplification 6-668: $Z-L^{3-g}-N(-L^{1-a}-D^{1-b})(L^{2-m}-D^{2-cc})$,

Exemplification 6-669: $Z-L^{3-g}-N(-L^{1-a}-D^{1-b})(L^{2-m}-D^{2-cd})$,

Exemplification 6-670: $Z-L^{3-g}-N(-L^{1-a}-D^{1-b})(L^{2-m}-D^{2-ce})$,

Exemplification 6-671: $Z-L^{3-g}-N(-L^{1-a}-D^{1-b})(L^{2-m}-D^{2-cf})$,

Exemplification 6-672: $Z-L^{3-g}-N(-L^{1-a}-D^{1-b})(L^{2-n}-D^{2-cb})$,

Exemplification 6-673: $Z-L^{3-g}-N(-L^{1-a}-D^{1-b})(L^{2-b}-D^{2-cg})$,

Exemplification 6-674: $Z-L^{3-g}-N(-L^{1-a}-D^{1-b})(L^{2-b}-D^{2-ch})$,

Exemplification 6-675: $Z-L^{3-g}-N(-L^{1-a}-D^{1-b})(L^{2-d}-D^{2-cg})$,

Exemplification 6-676: $Z-L^{3-g}-N(-L^{1-a}-D^{1-b})(L^{2-d}-D2^{-ch})$,

Exemplification 6-677: $Z-L^{3-g}-N(-L^{1-b}-D^{1-a})(L^{2-i}-D^{2-ca})$,

Exemplification 6-678: $Z-L^{3-g}-N(-L^{1-b}-D^{1-a})(L^{2-i}-D^{2-cc})$,

Exemplification 6-679: $Z-L^{3-g}-N(-L^{1-b}-D^{1-a})(L^{2-i}-D^{2-cd})$,

Exemplification 6-680: $Z-L^{3-g}-N(-L^{1-b}-D^{1-a})(L^{2-i}-D^{2-ce})$,

Exemplification 6-681: $Z-L^{3-g}-N(-L^{1-b}-D^{1-a})(L^{2-i}-D^{2-cf})$,

Exemplification 6-682: $Z-L^{3-g}-N(-L^{1-b}-D^{1-a})(L^{2-3}-D^{2-ca})$,

Exemplification 6-683: $Z-L^{3-g}-N(-L^{1-b}-D^{1-a})(L^{2-j}-D^{2-cc})$,

Exemplification 6-684: $Z-L^{3-g}-N(-L^{1-b}-D^{1-a})(L^{2-j}-D^{2-cd})$,

Exemplification 6-685: $Z-L^{3-g}-N(-L^{1-b}-D^{1-a})(L^{2-j}-D^{2-ce})$,

Exemplification 6-686: $Z-L^{3-g}-N(-L^{1-b}-D^{1-a})(L^{2-j}-D^{2-cf})$,

Exemplification 6-687: $Z-L^{3-g}-N(-L^{1-b}-D^{1-a})(L^{2-k}-D^{2-cb})$,

Exemplification 6-688: $Z-L^{3-g}-N(-L^{1-b}-D^{1-a})(L^{2-l}-D^{2-ca})$,

Exemplification 6-689: $Z-L^{3-g}-N(-L^{1}-b-D^{1-a})$ $(L^{2-l}-D^{2-cc})$,

Exemplification 6-690: $Z-L^{3-g}-N(-L^{1-b}-D^{1-a})(L^{2-l}-D^{2-cd})$,

Exemplification 6-691: $Z-L^{3-g}-N(-L^{1}-b-D^{1-a})$ $(L^{2-l}-D^{2-ce})$,

Exemplification 6-692: $Z-L^{3-g}-N(-L^{1}-b-D^{1-a})$ $(L^{2-l}-D^{2-cf})$,

Exemplification 6-693: $Z-L^{3-g}-N(-L^{1-b}-D^{1-a})(L^{2-m}-D^{2-ca})$,

Exemplification 6-694: $Z-L^{3-g}-N(-L^{1-b}-D^{1-a})(L^{2-m}-D^{2-cc})$,

Exemplification 6-695: $Z-L^{3-g}-N(-L^{1-b}-D^{1-a})(L^{2-m}-D^{2-cd})$,
Exemplification 6-696: $Z-L^{3-g}-N(-L^{1-b}-D^{1-a})(L^{2-m}-D^{2-ce})$,
Exemplification 6-697: $Z-L^{3-g}-N(-L^{1}-b-D^{1-a})$ $(L^{2-m}-D^{2-cf})$,
Exemplification 6-698: $Z-L^{3-g}-N(-L^{1-b}-D^{1-a})(L^{2-n}-D^{2-cb})$,
Exemplification 6-699: $Z-L^{3-g}-N(-L^{1-b}-D^{1-a})(L^{2-b}-D^{2-cg})$,
Exemplification 6-700: $Z-L^{3-g}-N(-L^{1-b}-D^{1-a})(L^{2-b}-D^{2-ch})$,
Exemplification 6-701: $Z-L^{3-g}-N(-L^{1-b}-D^{1-a})(L^{2-d}-D^{2-cg})$,
Exemplification 6-702: $Z-L^{3-g}-N(-L^{1-b}-D^{1-a})(L^{2-d}-D2^{-ch})$,
Exemplification 6-703: $Z-L^{3-g}-N(-L^{1-b}-D^{1-b})(L^{2-i}-D^{2-ca})$,
Exemplification 6-704: $Z-L^{3-g}-N(-L^{1-b}-D^{1-b})(L^{2-i}-D^{2-cc})$,
Exemplification 6-705: $Z-L^{3-g}-N(-L^{1-b}-D^{1-b})(L^{2-i}-D^{2-cd})$,
Exemplification 6-706: $Z-L^{3-g}-N(-L^{1-b}-D^{1-b})(L^{2-i}-D^{2-ce})$,
Exemplification 6-707: $Z-L^{3-g}-N(-L^{1-b}-D^{1-b})(L^{2-i}-D^{2-cf})$,
Exemplification 6-708: $Z-L^{3-g}-N(-L^{1-b}-D^{1-b})(L^{2-j}-D^{2-ca})$,
Exemplification 6-709: $Z-L^{3-g}-N(-L^{1-b}D^{1-b})(L^{2-j}-D^{2-cc})$,
Exemplification 6-710: $Z-L^{3-g}-N(-L^{1-b}-D^{1-b})(L^{2-j}-D^{2-cd})$,
Exemplification 6-711: $Z-L^{3-g}-N(-L^{1-b}-D^{1-b})(L^{2-j}-D^{2-ce})$,
Exemplification 6-712: $Z-L^{3-g}-N(-L^{1-b}-D^{1-b})(L^{2-j}-D2^{-cf})$,
Exemplification 6-713: $Z-L^{3-g}-N(-L^{1-b}-D^{1-b})(L^{2-k}-D^{2-cb})$,
Exemplification 6-714: $Z-L^{3-g}-N(-L^{1-b}-D^{1-b})(L^{2-1}-D^{2-cb})$,
Exemplification 6-715: $Z-L^{3-g}-N(-L^{1-b}-D^{1-b})(L^{2-l}-D^{2-cc})$,
Exemplification 6-716: $Z-L^{3-g}-N(-L^{1-b}-D^{1-b})(L^{2-l}-D^{2-cd})$,
Exemplification 6-717: $Z-L^{3-g}-N(-L^{1-b}-D^{1-b})(L^{2-l}-D^{2-ce})$,
Exemplification 6-718: $Z-L^{3-g}-N(-L^{1-b}-D^{1-b})(L^{2-l}-D^{2-cf})$,
Exemplification 6-719: $Z-L^{3-g}-N(-L^{1-b}-D^{1-b})(L^{2-m}-D^{2-ca})$,
Exemplification 6-720: $Z-L^{3-g}-N(-L^{1-b}-D^{1-b})(L^{2-m}-D^{2-cc})$,
Exemplification 6-721: $Z-L^{3-g}-N(-L^{1-b}-D^{1-b})(L^{2-m}-D^{2-cd})$,
Exemplification 6-722: $Z-L^{3-g}-N(-L^{1-b}-D^{1-b})(L^{2-m}-D^{2-ce})$,
Exemplification 6-723: $Z-L^{3-g}-N(-L^{1-b}-D^{1-b})(L^{2-m}-D^{2-cf})$,
Exemplification 6-724: $Z-L^{3-g}-N(-L^{1-b}-D^{1-b})(L^{2-n}-D^{2-cb})$,
Exemplification 6-725: $Z-L^{3-g}-N(-L^{1-b}-D^{1-b})(L^{2-b}-D^{2-cg})$,
Exemplification 6-726: $Z-L^{3-g}-N(-L^{1-b}-D^{1-b})(L^{2-b}-D^{2-ch})$,
Exemplification 6-727: $Z-L^{3-g}-N(-L^{1-b}-D^{1-b})(L^{2-d}-D^{2-cg})$,
Exemplification 6-728: $Z-L^{3-g}-N(-L^{1-b}-D^{1-b})(L^{2-d}-D2^{-ch})$,
Exemplification 6-729: $Z-L^{3-h}-N(-L^{1-a}-D^{1-a})(L^{2-i}-D^{2-ca})$,
Exemplification 6-730: $Z-L^{3-h}-N(-L^{1-a}-D^{1-a})(L^{2-i}-D^{2-cc})$,
Exemplification 6-731: $Z-L^{3-h}-N(-L^{1-a}-D^{1-a})(L^{2-i}-D^{2-cd})$,
Exemplification 6-732: $Z-L^{3-h}-N(-L^{1-a}-D^{1-a})(L^{2-i}-D^{2-ce})$,
Exemplification 6-733: $Z-L^{3-h}-N(-L^{1-a}-D^{1-a})(L^{2-i}-D^{2-cf})$,
Exemplification 6-734: $Z-L^{3-h}-N(-L^{1-a}-D^{1-a})(L^{2-3}-D^{2-ca})$,
Exemplification 6-735: $Z-L^{3-h}-N(-L^{1-a}-D^{1-a})(L^{2-j}-D^{2-cc})$,
Exemplification 6-736: $Z-L^{3-h}-N(-L^{1-a}-D^{1-a})(L^{2-j}-D^{2-cd})$,
Exemplification 6-737: $Z-L^{3-h}-N(-L^{1-a}-D^{1-a})(L^{2-j}-D^{2-ce})$,
Exemplification 6-738: $Z-L^{3-h}-N(-L^{1-a}-D^{1-a})(L^{2-j}-D2^{-cf})$,
Exemplification 6-739: $Z-L^{3-h}-N(-L^{1-a}-D^{1-a})(L^{2-k}-D^{2-cb})$,
Exemplification 6-740: $Z-L^{3-h}-N(-L^{1-a}-D^{1-a})(L^{2-1}-D^{2-cb})$,
Exemplification 6-741: $Z-L^{3-h}-N(-L^{1-a}-D^{1-a})(L^{2-l}-D^{2-cc})$,
Exemplification 6-742: $Z-L^{3-h}-N(-L^{1-a}-D^{1-a})(L^{2-l}-D^{2-cd})$,
Exemplification 6-743: $Z-L^{3-h}-N(-L^{1-a}-D^{1-a})(L^{2-l}-D^{2-ce})$,
Exemplification 6-744: $Z-L^{3-h}-N(-L^{1-a}-D^{1-a})(L^{2-l}-D^{2-cf})$,
Exemplification 6-745: $Z-L^{3-h}-N(-L^{1-a}-D^{1-a})(L^{2-m}-D^{2-ca})$,
Exemplification 6-746: $Z-L^{3-h}-N(-L^{1-a}-D^{1-a})(L^{2-m}-D^{2-cc})$,
Exemplification 6-747: $Z-L^{3-h}-N(-L^{1-a}-D^{1-a})(L^{2-m}-D^{2-cd})$,
Exemplification 6-748: $Z-L^{3-h}-N(-L^{1-a}-D^{1-a})(L^{2-m}-D^{2-ce})$,
Exemplification 6-749: $Z-L^{3-h}-N(-L^{1-a}-D^{1-a})(L^{2-m}-D^{2-cf})$,
Exemplification 6-750: $Z-L^{3-h}-N(-L^{1-a}-D^{1-a})(L^{2-n}-D^{2-cb})$,
Exemplification 6-751: $Z-L^{3-h}-N(-L^{1-a}-D^{1-a})(L^{2-b}-D^{2-cg})$,
Exemplification 6-752: $Z-L^{3-h}-N(-L^{1-a}-D^{1-a})(L^{2-b}-D^{2-ch})$,

Exemplification 6-753: $Z-L^{3-h}-N(-L^{1-a}-D^{1-a})(L^{2-d}-D^{2-cg})$,
Exemplification 6-754: $Z-L^{3-h}-N(-L^{1-a}-D^{1-a})(L^{2-d}-D2^{-ch})$,
Exemplification 6-755: $Z-L^{3-h}-N(-L^{1-a}-D^{1-b})(L^{2-i}-D^{2-ca})$,
Exemplification 6-756: $Z-L^{3-h}-N(-L^{1-a}-D^{1-b})(L^{2-i}-D^{2-cc})$,
Exemplification 6-757: $Z-L^{3-h}-N(-L^{1-a}-D^{1-b})(L^{2-i}-D^{2-cd})$,
Exemplification 6-758: $Z-L^{3-h}-N(-L^{1-a}-D^{1-b})(L^{2-i}-D^{2-ce})$,
Exemplification 6-759: $Z-L^{3-h}-N(-L^{1-a}-D^{1-b})(L^{2-i}-D^{2-cf})$,
Exemplification 6-760: $Z-L^{3-h}-N(-L^{1-a}-D^{1-b})(L^{2-3}-D^{2-ca})$,
Exemplification 6-761: $Z-L^{3-h}-N(-L^{1-a}-D^{1-b})(L^{2-j}-D^{2-cc})$,
Exemplification 6-762: $Z-L^{3-h}-N(-L^{1-a}-D^{1-b})(L^{2-j}-D^{2-cd})$,
Exemplification 6-763: $Z-L^{3-h}-N(-L^{1-a}-D^{1-b})(L^{2-j}-D^{2-ce})$,
Exemplification 6-764: $Z-L^{3-h}-N(-L^{1-a}-D^{1-b})(L^{2-j}-D2^{-cf})$,
Exemplification 6-765: $Z-L^{3-h}-N(-L^{1-a}-D^{1-b})(L^{2-k}-D^{2-cb})$,
Exemplification 6-766: $Z-L^{3-h}-N(-L^{1-a}-D^{1-b})(L^{2-1}-D^{2-cb})$,
Exemplification 6-767: $Z-L^{3-h}-N(-L^{1-a}-D^{1-b})(L^{2-l}-D^{2-cc})$,
Exemplification 6-768: $Z-L^{3-h}-N(-L^{1-a}-D^{1-b})(L^{2-l}-D^{2-cd})$,
Exemplification 6-769: $Z-L^{3-h}-N(-L^{1-a}-D^{1-b})(L^{2-l}-D^{2-ce})$,
Exemplification 6-770: $Z-L^{3-h}-N(-L^{1-a}-D^{1-b})(L^{2-l}-D^{2-cf})$,
Exemplification 6-771: $Z-L^{3-h}-N(-L^{1-a}-D^{1-b})(L^{2-m}-D^{2-ca})$,
Exemplification 6-772: $Z-L^{3-h}-N(-L^{1-a}-D^{1-b})(L^{2-m}-D^{2-cc})$,
Exemplification 6-773: $Z-L^{3-h}-N(-L^{1-a}-D^{1-b})(L^{2-m}-D^{2-cd})$,
Exemplification 6-774: $Z-L^{3-h}-N(-L^{1-a}-D^{1-b})(L^{2-m}-D^{2-ce})$,
Exemplification 6-775: $Z-L^{3-h}-N(-L^{1-a}-D^{1-b})(L^{2-m}-D^{2-cf})$,
Exemplification 6-776: $Z-L^{3-h}-N(-L^{1-a}-D^{1-b})(L^{2-n}-D^{2-cb})$,
Exemplification 6-777: $Z-L^{3-h}-N(-L^{1-a}-D^{1-b})(L^{2-b}-D^{2-cg})$,
Exemplification 6-778: $Z-L^{3-h}-N(-L^{1-a}-D^{1-b})(L^{2-b}-D^{2-ch})$,
Exemplification 6-779: $Z-L^{3-h}-N(-L^{1-a}-D^{1-b})(L^{2-d}-D^{2-cg})$,
Exemplification 6-780: $Z-L^{3-h}-N(-L^{1-a}-D^{1-b})(L^{2-d}-D2^{-ch})$,
Exemplification 6-781: $Z-L^{3-h}-N(-L^{1-b}-D^{1-a})(L^{2-i}-D^{2-ca})$,
Exemplification 6-782: $Z-L^{3-h}-N(-L^{1-b}-D^{1-a})(L^{2-i}-D^{2-cc})$,
Exemplification 6-783: $Z-L^{3-h}-N(-L^{1-b}-D^{1-a})(L^{2-i}-D^{2-cd})$,
Exemplification 6-784: $Z-L^{3-h}-N(-L^{1-b}-D^{1-a})(L^{2-i}-D^{2-ce})$,
Exemplification 6-785: $Z-L^{3-h}-N(-L^{1-b}-D^{1-a})(L^{2-i}-D^{2-cf})$,
Exemplification 6-786: $Z-L^{3-h}-N(-L^{1-b}-D^{1-a})(L^{2-3}-D^{2-ca})$,
Exemplification 6-787: $Z-L^{3-h}-N(-L^{1-b}-D^{1-a})(L^{2-j}-D^{2-cc})$,
Exemplification 6-788: $Z-L^{3-h}-N(-L^{1-b}-D^{1-a})(L^{2-j}-D^{2-cd})$,
Exemplification 6-789: $Z-L^{3-h}-N(-L^{1-b}-D^{1-a})(L^{2-j}-D^{2-ce})$,
Exemplification 6-790: $Z-L^{3-h}-N(-L^{1-b}-D^{1-a})(L^{2-j}-D2^{-cf})$,
Exemplification 6-791: $Z-L^{3-h}-N(-L^{1-b}-D^{1-a})(L^{2-k}-D^{2-cb})$,
Exemplification 6-792: $Z-L^{3-h}-N(-L^{1-b}-D^{1-a})(L^{2-1}-D^{2-cb})$,
Exemplification 6-793: $Z-L^{3-h}-N(-L^{1-b}-D^{1-a})(L^{2-l}-D^{2-cc})$,
Exemplification 6-794: $Z-L^{3-h}-N(-L^{1-b}-D^{1-a})(L^{2-l}-D^{2-cd})$,
Exemplification 6-795: $Z-L^{3-h}-N(-L^{1-b}-D^{1-a})(L^{2-l}-D^{2-ce})$,
Exemplification 6-796: $Z-L^{3-h}-N(-L^{1-b}-D^{1-a})(L^{2-l}-D^{2-cf})$,
Exemplification 6-797: $Z-L^{3-h}-N(-L^{1-b}-D^{1-a})(L^{2-m}-D^{2-ca})$,
Exemplification 6-798: $Z-L^{3-h}-N(-L^{1-b}-D^{1-a})(L^{2-m}-D^{2-cc})$,
Exemplification 6-799: $Z-L^{3-h}-N(-L^{1-b}-D^{1-a})(L^{2-m}-D^{2-cd})$,
Exemplification 6-800: $Z-L^{3-h}-N(-L^{1-b}-D^{1-a})(L^{2-m}-D^{2-ce})$,
Exemplification 6-801: $Z-L^{3-h}-N(-L^{1-b}-D^{1-a})(L^{2-m}-D^{2-cf})$,
Exemplification 6-802: $Z-L^{3-h}-N(-L^{1-b}-D^{1-a})(L^{2-n}-D^{2-cb})$,
Exemplification 6-803: $Z-L^{3-h}-N(-L^{1-b}-D^{1-a})(L^{2-b}-D^{2-cg})$,
Exemplification 6-804: $Z-L^{3-h}-N(-L^{1-b}-D^{1-a})(L^{2-b}-D^{2-ch})$,
Exemplification 6-805: $Z-L^{3-h}-N(-L^{1-b}-D^{1-a})(L^{2-d}-D^{2-cg})$,
Exemplification 6-806: $Z-L^{3-h}-N(-L^{1-b}-D^{1-a})(L^{2-d}-D^{2-ch})$,
Exemplification 6-807: $Z-L^{3-h}-N(-L^{1-b}-D^{1-b})(L^{2-i}-D^{2-ca})$,
Exemplification 6-808: $Z-L^{3-h}-N(-L^{1-b}-D^{1-b})(L^{2-i}-D^{2-cc})$,
Exemplification 6-809: $Z-L^{3-h}-N(-L^{1-b}-D^{1-b})(L^{2-i}-D^{2-cd})$,
Exemplification 6-810: $Z-L^{3-h}-N(-L^{1-b}-D^{1-b})(L^{2-i}-D^{2-ce})$,

Exemplification 6-811: $Z-L^{3-h}-N(-L^{1-b}-D^{1-b})(L^{2-i}-D^{2-cf})$,

Exemplification 6-812: $Z-L^{3-h}-N(-L^{1-b}-D^{1-b})(L^{2-3}-D^{2-ca})$,

Exemplification 6-813: $Z-L^{3-h}-N(-L^{1-b}-D^{1-b})(L^{2-j}-D^{2-cc})$,

Exemplification 6-814: $Z-L^{3-h}-N(-L^{1-b}-D^{1-b})(L^{2-j}-D^{2-cd})$,

Exemplification 6-815: $Z-L^{3-h}-N(-L^{1-b}-D^{1-b})(L^{2-3}-D^{2-ce})$,

Exemplification 6-816: $Z-L^{3-h}-N(-L^{1-b}-D^{1-b})(L^{2-j}-D^{2-cf})$,

Exemplification 6-817: $Z-L^{3-h}-N(-L^{1-b}-D^{1-b})(L^{2-k}-D^{2-cb})$,

Exemplification 6-818: $Z-L^{3-h}-N(-L^{1-b}-D^{1-b})(L^{2-1}-D^{2-cb})$,

Exemplification 6-819: $Z-L^{3-h}-N(-L^{1-b}-D^{1-b})(L^{2-l}-D^{2-cc})$,

Exemplification 6-820: $Z-L^{3-h}-N(-L^{1-b}-D^{1-b})(L^{2-l}-D^{2-cd})$,

Exemplification 6-821: $Z-L^{3-h}-N(-L^{1-b}-D^{1-b})(L^{2-l}-D^{2-ce})$,

Exemplification 6-822: $Z-L^{3-h}-N(-L^{1-b}-D^{1-b})(L^{2-l}-D^{2-cf})$,

Exemplification 6-823: $Z-L^{3-h}-N(-L^{1-b}-D^{1-b})(L^{2-m}-D^{2-ca})$,

Exemplification 6-824: $Z-L^{3-h}-N(-L^{1-b}-D^{1-b})(L^{2-m}-D^{2-cc})$,

Exemplification 6-825: $Z-L^{3-h}-N(-L^{1-b}-D^{1-b})(L^{2-m}-D^{2-cd})$,

Exemplification 6-826: $Z-L^{3-h}-N(-L^{1-b}-D^{1-b})(L^{2-m}-D^{2-ce})$,

Exemplification 6-827: $Z-L^{3-h}-N(-L^{1-b}-D^{1-b})(L^{2-m}-D^{2-cf})$,

Exemplification 6-828: $Z-L^{3-h}-N(-L^{1-b}-D^{1-b})(L^{2-n}-D^{2-cb})$,

Exemplification 6-829: $Z-L^{3-h}-N(-L^{1-b}-D^{1-b})(L^{2-b}-D^{2-cg})$,

Exemplification 6-830: $Z-L^{3-h}-N(-L^{1-b}-D^{1-b})(L^{2-b}-D^{2-ch})$,

Exemplification 6-831: $Z-L^{3-h}-N(-L^{1-b}-D^{1-b})(L^{2-d}-D^{2-cg})$,

Exemplification 6-832: $Z-L^{3-h}-N(-L^{1-b}-D^{1-b})(L^{2-d}-D2^{-ch})$,

Exemplification 6-833: $Z-L^{3-i}-N(-L^{1-a}-D^{1-a})(L^{2-i}-D^{2-ca})$,

Exemplification 6-834: $Z-L^{3-i}-N(-L^{1-a}-D^{1-a})(L^{2-i}-D^{2-cc})$,

Exemplification 6-835: $Z-L^{3-i}-N(-L^{1-a}-D^{1-a})(L^{2-i}-D^{2-cd})$,

Exemplification 6-836: $Z-L^{3-i}-N(-L^{1-a}-D^{1-a})(L^{2-i}-D^{2-ce})$,

Exemplification 6-837: $Z-L^{3-i}-N(-L^{1-a}-D^{1-a})(L^{2-i}-D^{2-cf})$,

Exemplification 6-838: $Z-L^{3-i}-N(-L^{1-a}-D^{1-a})(L^{2-3}-D^{2-ca})$,

Exemplification 6-839: $Z-L^{3-i}-N(-L^{1-a}-D^{1-a})(L^{2-j}-D^{2-cc})$,

Exemplification 6-840: $Z-L^{3-i}-N(-L^{1-a}-D^{1-a})(L^{2-j}-D^{2-cd})$,

Exemplification 6-841: $Z-L^{3-i}-N(-L^{1-a}-D^{1-a})(L^{2-j}-D^{2-ce})$,

Exemplification 6-842: $Z-L^{3-i}-N(-L^{1-a}-D^{1-a})(L^{2-j}-D2^{-cf})$,

Exemplification 6-843: $Z-L^{3-i}-N(-L^{1-a}-D^{1-a})(L^{2-k}-D^{2-cb})$,

Exemplification 6-844: $Z-L^{3-i}-N(-L^{1-a}-D^{1-a})(L^{2-1}-D^{2-cb})$,

Exemplification 6-845: $Z-L^{3-i}-N(-L^{1-a}-D^{1-a})(L^{2-1}-D^{2-cc})$,

Exemplification 6-846: $Z-L^{3-i}-N(-L^{1-a}-D^{1-a})(L^{2-1}-D^{2-cd})$,

Exemplification 6-847: $Z-L^{3-i}-N(-L^{1-a}-D^{1-a})(L^{2-1}-D^{2-ce})$,

Exemplification 6-848: $Z-L^{3-i}-N(-L^{1-a}-D^{1-a})(L^{2-1}-D^{2-cf})$,

Exemplification 6-849: $Z-L^{3-i}-N(-L^{1-a}-D^{1-a})(L^{2-m}-D^{2-ca})$,

Exemplification 6-850: $Z-L^{3-i}-N(-L^{1-a}-D^{1-a})(L^{2-m}-D^{2-cc})$,

Exemplification 6-851: $Z-L^{3-i}-N(-L^{1-a}-D^{1-a})(L^{2-m}-D^{2-cd})$,

Exemplification 6-852: $Z-L^{3-i}-N(-L^{1-a}-D^{1-a})(L^{2-m}-D^{2-ce})$,

Exemplification 6-853: $Z-L^{3-i}-N(-L^{1-a}-D^{1-a})(L^{2-m}-D^{2-cf})$,

Exemplification 6-854: $Z-L^{3-i}-N(-L^{1-a}-D^{1-a})(L2^{-n}-D^{2-cb})$,

Exemplification 6-855: $Z-L^{3-i}-N(-L^{1-a}-D^{1-a})(L^{2-b}-D^{2-cg})$,

Exemplification 6-856: $Z-L^{3-i}-N(-L^{1-a}-D^{1-a})(L^{2-b}-D^{2-ch})$,

Exemplification 6-857: $Z-L^{3-i}-N(-L^{1-a}-D^{1-a})(L^{2-d}-D^{2-cg})$,

Exemplification 6-858: $Z-L^{3-i}-N(-L^{1-a}-D^{1-a})(L^{2-d}-D^{2-ch})$,

Exemplification 6-859: $Z-L^{3-i}-N(-L^{1-a}-D^{1-b})(L^{2-i}-D^{2-ca})$,

Exemplification 6-860: $Z-L^{3-i}-N(-L^{1-a}-D^{1-b})(L^{2-i}-D^{2-cc})$,

Exemplification 6-861: $Z-L^{3-i}-N(-L^{1-a}-D^{1-b})(L^{2-i}-D^{2-cd})$,

Exemplification 6-862: $Z-L^{3-i}-N(-L^{1-a}-D^{1-b})(L^{2-i}-D^{2-ce})$,

Exemplification 6-863: $Z-L^{3-i}-N(-L^{1-a}-D^{1-b})(L^{2-i}-D^{2-cf})$ ,

Exemplification 6-864: $Z-L^{3-i}-N(-L^{1-a}-D^{1-b})(L^{2-j}-D^{2-ca})$,

Exemplification 6-865: $Z-L^{3-i}-N(-L^{1-a}-D^{1-b})(L^{2-j}-D^{2-cc})$,

Exemplification 6-866: $Z-L^{3-i}-N(-L^{1-a}-D^{1-b})(L^{2-j}-D^{2-cd})$,

Exemplification 6-867: $Z-L^{3-i}-N(-L^{1-a}-D^{1-b})(L^{2-j}-D^{2-ce})$,

Exemplification 6-868: $Z-L^{3-i}-N(-L^{1-a}-D^{1-b})(L^{2-j}-D^{2-cf})$,

Exemplification 6-869: Z-L$^{3-i}$-N(-L$^{1-a}$-D$^{1-b}$)(L$^{2-k}$-D$^{2-cb}$),
Exemplification 6-870: Z-L$^{3-i}$-N(-L$^{1-a}$-D$^{1-b}$)(L$^{2-l}$-D$^{2-ca}$),
Exemplification 6-871: Z-L$^{3-i}$-N(-L$^{1-a}$-D$^{1-b}$)(L$^{2-1}$-D$^{2-cc}$),
Exemplification 6-872: Z-L$^{3-i}$-N(-L$^{1-a}$-D$^{1-b}$)(L$^{2-l}$-D$^{2-cd}$),
Exemplification 6-873: Z-L$^{3-i}$-N(-L$^{1-a}$-D$^{1-b}$)(L$^{2-1}$-D$^{2-ce}$),
Exemplification 6-874: Z-L$^{3-i}$-N(-L$^{1-a}$-D$^{1-b}$)(L$^{2-1}$-D$^{2-cf}$),
Exemplification 6-875: Z-L$^{3-i}$-N(-L$^{1-a}$-D$^{1-b}$)(L$^{2-m}$-D$^{2-ca}$),
Exemplification 6-876: Z-L$^{3-i}$-N(-L$^{1-a}$-D$^{1-b}$)(L$^{2-m}$-D$^{2-cc}$),
Exemplification 6-877: Z-L$^{3-i}$-N(-L$^{1-a}$-D$^{1-b}$)(L$^{2-m}$-D$^{2-cd}$),
Exemplification 6-878: Z-L$^{3-i}$-N(-L$^{1-a}$-D$^{1-b}$)(L$^{2-m}$-D$^{2-ce}$),
Exemplification 6-879: Z-L$^{3-i}$-N(-L$^{1-a}$-D$^{1-b}$)(L$^{2-m}$-D$^{2-cf}$),
Exemplification 6-880: Z-L$^{3-i}$-N(-L$^{1-a}$-D$^{1-b}$)(L2-$^{n}$-D$^{2-cb}$),
Exemplification 6-881: Z-L$^{3-i}$-N(-L$^{1-a}$-D$^{1-b}$)(L$^{2-b}$-D$^{2-cg}$),
Exemplification 6-882: Z-L$^{3-i}$-N(-L$^{1-a}$-D$^{1-b}$)(L$^{2-b}$-D$^{2-ch}$),
Exemplification 6-883: Z-L$^{3-i}$-N(-L$^{1-a}$-D$^{1-b}$)(L$^{2-d}$-D$^{2-cg}$),
Exemplification 6-884: Z-L$^{3-i}$-N(-L$^{1-a}$-D$^{1-b}$)(L$^{2-d}$-D$^{2-ch}$),
Exemplification 6-885: Z-L$^{3-i}$-N(-L$^{1-b}$-D$^{1-a}$)(L$^{2-i}$-D$^{2-ca}$),
Exemplification 6-886: Z-L$^{3-i}$-N(-L$^{1-b}$-D$^{1-a}$)(L$^{2-i}$-D$^{2-cc}$),
Exemplification 6-887: Z-L$^{3-i}$-N(-L$^{1-b}$-D$^{1-a}$)(L$^{2-i}$-D$^{2-cd}$),
Exemplification 6-888: Z-L$^{3-i}$-N(-L$^{1-b}$-D$^{1-a}$)(L$^{2-i}$-D$^{2-ce}$),
Exemplification 6-889: Z-L$^{3-i}$-N(-L$^{1-b}$-D$^{1-a}$)(L$^{2-i}$-D$^{2-cf}$),
Exemplification 6-890: Z-L$^{3-i}$-N(-L$^{1-b}$-D$^{1-a}$)(L$^{2-j}$-D$^{2-ca}$),
Exemplification 6-891: Z-L$^{3-i}$-N(-L$^{1-b}$-D$^{1-a}$)(L$^{2-j}$-D$^{2-cc}$),
Exemplification 6-892: Z-L$^{3-i}$-N(-L$^{1-b}$-D$^{1-a}$)(L$^{2-j}$-D$^{2-cd}$),
Exemplification 6-893: Z-L$^{3-i}$-N(-L$^{1-b}$-D$^{1-a}$)(L$^{2-j}$-D$^{2-ce}$),
Exemplification 6-894: Z-L$^{3-i}$-N(-L$^{1-b}$-D$^{1-a}$)(L$^{2-j}$-D$^{2-cf}$),
Exemplification 6-895: Z-L$^{3-i}$-N(-L$^{1-b}$-D$^{1-a}$)(L$^{2-k}$-D$^{2-cb}$,
Exemplification 6-896: Z-L$^{3-i}$-N(-L$^{1-b}$-D$^{1-a}$)(L$^{2-l}$-D$^{2-ca}$),
Exemplification 6-897: Z-L$^{3-i}$-N(-L$^{1-b}$-D$^{1-a}$)(L$^{2-1}$-D$^{2-cc}$),
Exemplification 6-898: Z-L$^{3-i}$-N(-L$^{1-b}$-D$^{1-a}$)(L$^{2-l}$-D$^{2-cd}$),
Exemplification 6-899: Z-L$^{3-i}$-N(-L$^{1-b}$-D$^{1-a}$)(L$^{2-1}$-D$^{2-ce}$),
Exemplification 6-900: Z-L$^{3-i}$-N(-L$^{1-b}$-D$^{1-a}$)(L$^{2-1}$-D$^{2-cf}$),
Exemplification 6-901: Z-L$^{3-i}$-N(-L$^{1-b}$-D$^{1-a}$)(L$^{2-m}$-D$^{2-ca}$),
Exemplification 6-902: Z-L$^{3-i}$-N(-L$^{1-b}$-D$^{1-a}$)(L$^{2-m}$-D$^{2-cc}$),
Exemplification 6-903: Z-L$^{3-i}$-N(-L$^{1-b}$-D$^{1-a}$)(L$^{2-m}$-D$^{2-cd}$),
Exemplification 6-904: Z-L$^{3-i}$-N(-L$^{1-b}$-D$^{1-a}$)(L$^{2-m}$-D$^{2-ce}$),
Exemplification 6-905: Z-L$^{3-i}$-N(-L$^{1-b}$-D$^{1-a}$)(L$^{2-m}$-D$^{2-cf}$),
Exemplification 6-906: Z-L$^{3-i}$-N(-L$^{1-b}$-D$^{1-a}$)(L$^{2-n}$-D2-$^{cb}$),
Exemplification 6-907: Z-L$^{3-i}$-N(-L$^{1-b}$-D$^{1-a}$)(L$^{2-b}$-D$^{2-cg}$),
Exemplification 6-908: Z-L$^{3-i}$-N(-L$^{1-b}$-D$^{1-a}$)(L$^{2-b}$-D$^{2-ch}$),
Exemplification 6-909: Z-L$^{3-i}$-N(-L$^{1-b}$-D$^{1-a}$)(L$^{2-d}$-D$^{2-cg}$),
Exemplification 6-910: Z-L$^{3-i}$-N(-L$^{1-b}$-D$^{1-a}$)(L$^{2-d}$-D$^{2-ch}$),
Exemplification 6-911: Z-L$^{3-i}$-N(-L$^{1-b}$-D$^{1-b}$)(L$^{2-i}$-D$^{2-ca}$),
Exemplification 6-912: Z-L$^{3-i}$-N(-L$^{1-b}$-D$^{1-b}$)(L$^{2-i}$-D$^{2-cc}$),
Exemplification 6-913: Z-L$^{3-i}$-N(-L$^{1-b}$-D$^{1-b}$)(L$^{2-i}$-D$^{2-cd}$),
Exemplification 6-914: Z-L$^{3-i}$-N(-L$^{1-b}$-D$^{1-b}$)(L$^{2-i}$-D$^{2-ce}$),
Exemplification 6-915: Z-L$^{3-i}$-N(-L$^{1-b}$-D$^{1-b}$)(L$^{2-i}$-D$^{2-cf}$),
Exemplification 6-916: Z-L$^{3-i}$-N(-L$^{1-b}$-D$^{1-b}$)(L$^{2-j}$-D$^{2-ca}$),
Exemplification 6-917: Z-L$^{3-i}$-N(-L$^{1-b}$-D$^{1-b}$)(L$^{2-j}$-D$^{2-cc}$),
Exemplification 6-918: Z-L$^{3-i}$-N(-L$^{1-b}$-D$^{1-b}$)(L$^{2-j}$-D$^{2-cd}$),
Exemplification 6-919: Z-L$^{3-i}$-N(-L$^{1-b}$-D$^{1-b}$)(L$^{2-j}$-D$^{2-ce}$),
Exemplification 6-920: Z-L$^{3-i}$-N(-L$^{1-b}$-D$^{1-b}$)(L$^{2-j}$-D$^{2-cf}$) ,
Exemplification 6-921: Z-L$^{3-i}$-N(-L$^{1-b}$-D$^{1-b}$)(L$^{2-k}$-D$^{2-cb}$),
Exemplification 6-922: Z-L$^{3-i}$-N(-L$^{1-b}$-D$^{1-b}$)(L$^{2-l}$-D$^{2-ca}$),
Exemplification 6-923: Z-L$^{3-i}$-N(-L$^{1-b}$-D$^{1-b}$)(L$^{2-1}$-D$^{2-cc}$),
Exemplification 6-924: Z-L$^{3-i}$-N(-L$^{1-b}$-D$^{1-b}$)(L$^{2-l}$-D$^{2-cd}$),
Exemplification 6-925: Z-L$^{3-i}$-N(-L$^{1-b}$-D$^{1-b}$)(L$^{2-1}$-D$^{2-ce}$),
Exemplification 6-926: Z-L$^{3-i}$-N(-L$^{1-b}$-D$^{1-b}$)(L$^{2-1}$-D$^{2-cf}$),

Exemplification 6-927: Z-$L^{3-i}$-N(-$L^{1-b}$-$D^{1-b}$)($L^{2-m}$-$D^{2-ca}$),
Exemplification 6-928: Z-$L^{3-i}$-N(-$L^{1-b}$-$D^{1-b}$)($L^{2-m}$-$D^{2-cc}$),
Exemplification 6-929: Z-$L^{3-i}$-N(-$L^{1-b}$-$D^{1-b}$)($L^{2-m}$-$D^{2-cd}$),
Exemplification 6-930: Z-$L^{3-i}$-N(-$L^{1-b}$-$D^{1-b}$)($L^{2-m}$-$D^{2-ce}$),
Exemplification 6-931: Z-$L^{3-i}$-N(-$L^{1-b}$-$D^{1-b}$)($L^{2-m}$-$D^{2-cf}$),
Exemplification 6-932: Z-$L^{3-i}$-N(-$L^{1-b}$-$D^{1-b}$)(L2-$^{n}$-$D^{2-cb}$),
Exemplification 6-933: Z-$L^{3-i}$-N(-$L^{1-b}$-$D^{1-b}$)($L^{2-b}$-$D^{2-cg}$),
Exemplification 6-934: Z-$L^{3-i}$-N(-$L^{1-b}$-$D^{1-b}$)($L^{2-b}$-$D^{2-ch}$),
Exemplification 6-935: Z-$L^{3-i}$-N(-$L^{1-b}$-$D^{1-b}$)($L^{2-d}$-$D^{2-cg}$), and
Exemplification 6-936: Z-$L^{3-i}$-N(-$L^{1-b}$-$D^{1-b}$)($L^{2-d}$-$D^{2-ch}$).

The present invention also provides exemplification precursor compounds (Exemplification 7-1 to Exemplification 7-144) {in the following formula, Z, $L^{1-a}$ to $L^{1-b}$, $L^{2-o}$ to $L^{2-r}$, $L^{3-a}$ to $L^{3-e}$, $L^{3-j}$ to $L^{3-m}$, $D^{1-a}$ to $D^{1-b}$, and $D^{2-da}$ are as defined above}.

Exemplification 7-1: Z-$L^{3-a}$-N(-$L^{1-a}$-$D^{1-a}$)($L^{2-o}$-$D^{2-da}$),
Exemplification 7-2: Z-$L^{3-a}$-N(-$L^{1-a}$-$D^{1-a}$)($L^{2-p}$-$D^{2-da}$),
Exemplification 7-3: Z-$L^{3-a}$-N(-$L^{1-a}$-$D^{1-a}$)($L^{2-q}$-$D^{2-da}$),
Exemplification 7-4: Z-$L^{3-a}$-N(-$L^{1-a}$-$D^{1-a}$)($L^{2-r}$-$D^{2-da}$),
Exemplification 7-5: Z-$L^{3-a}$-N(-$L^{1-a}$-$D^{1-b}$)($L^{2-o}$-$D^{2-da}$),
Exemplification 7-6: Z-$L^{3-a}$-N(-$L^{1-a}$-$D^{1-b}$)($L^{2-p}$-$D^{2-da}$),
Exemplification 7-7: Z-$L^{3-a}$-N(-$L^{1-a}$-$D^{1-b}$)($L^{2-q}$-$D^{2-da}$),
Exemplification 7-8: Z-$L^{3-a}$-N(-$L^{1-a}$-$D^{1-b}$)($L^{2-r}$-$D^{2-da}$),
Exemplification 7-9: Z-$L^{3-a}$-N(-$L^{1-b}$-$D^{1-a}$)($L^{2-o}$-$D^{2-da}$),
Exemplification 7-10: Z-$L^{3-a}$-N(-$L^{1-b}$-$D^{1-a}$)($L^{2-p}$-$D^{2-da}$),
Exemplification 7-11: Z-$L^{3-a}$-N(-$L^{1-b}$-$D^{1-a}$)($L^{2-q}$-$D^{2-da}$),
Exemplification 7-12: Z-$L^{3-a}$-N(-$L^{1-b}$-$D^{1-a}$)($L^{2-r}$-$D^{2-da}$),
Exemplification 7-13: Z-$L^{3-a}$-N(-$L^{1-b}$-$D^{1-b}$)($L^{2-o}$-$D^{2-da}$),
Exemplification 7-14: Z-$L^{3-a}$-N(-$L^{1-b}$-$D^{1-b}$)($L^{2-p}$-$D^{2-da}$),
Exemplification 7-15: Z-$L^{3-a}$-N(-$L^{1-b}$-$D^{1-b}$)($L^{2-q}$-$D^{2-da}$),
Exemplification 7-16: Z-$L^{3-a}$-N(-$L^{1-b}$-$D^{1-b}$)($L^{2-r}$-$D^{2-da}$),
Exemplification 7-17: Z-$L^{3-b}$-N(-$L^{1-a}$-$D^{1-a}$)($L^{2-o}$-$D^{2-da}$),
Exemplification 7-18: Z-$L^{3-b}$-N(-$L^{1-a}$-$D^{1-a}$)($L^{2-p}$-$D^{2-da}$),
Exemplification 7-19: Z-$L^{3-b}$-N(-$L^{1-a}$-$D^{1-a}$)($L^{2-q}$-$D^{2-da}$),
Exemplification 7-20: Z-$L^{3-b}$-N(-$L^{1-a}$-$D^{1-a}$)($L^{2-r}$-$D^{2-da}$),
Exemplification 7-21: Z-$L^{3-b}$-N(-$L^{1-a}$-$D^{1-b}$)($L^{2-o}$-$D^{2-da}$),
Exemplification 7-22: Z-$L^{3-b}$-N(-$L^{1-a}$-$D^{1-b}$)($L^{2-p}$-$D^{2-da}$),
Exemplification 7-23: Z-$L^{3-b}$-N(-$L^{1-a}$-$D^{1-b}$)($L^{2-q}$-$D^{2-da}$),
Exemplification 7-24: Z-$L^{3-b}$-N(-$L^{1-a}$-$D^{1-b}$)($L^{2-r}$-$D^{2-da}$),
Exemplification 7-25: Z-$L^{3-b}$-N(-$L^{1-b}$-$D^{1-a}$)($L^{2-o}$-$D^{2-da}$),
Exemplification 7-26: Z-$L^{3-b}$-N(-$L^{1-b}$-$D^{1-a}$)($L^{2-p}$-$D^{2-da}$),
Exemplification 7-27: Z-$L^{3-b}$-N(-$L^{1-b}$-$D^{1-a}$)($L^{2-q}$-$D^{2-da}$),
Exemplification 7-28: Z-$L^{3-b}$-N(-$L^{1-b}$-$D^{1-a}$)($L^{2-r}$-$D^{2-da}$),
Exemplification 7-29: Z-$L^{3-b}$-N(-$L^{1-b}$-$D^{1-b}$)($L^{2-o}$-$D^{2-da}$),
Exemplification 7-30: Z-$L^{3-b}$-N(-$L^{1-b}$-$D^{1-b}$)($L^{2-p}$-$D^{2-da}$),
Exemplification 7-31: Z-$L^{3-b}$-N(-$L^{1-b}$-$D^{1-b}$)($L^{2-q}$-$D^{2-da}$),
Exemplification 7-32: Z-$L^{3-b}$-N(-$L^{1-b}$-$D^{1-b}$)($L^{2-r}$-$D^{2-da}$),
Exemplification 7-33: Z-$L^{3-c}$-N(-$L^{1-a}$-$D^{1-a}$)($L^{2-o}$-$D^{2-da}$),
Exemplification 7-34: Z-$L^{3-c}$-N(-$L^{1-a}$-$D^{1-a}$)($L^{2-p}$-$D^{2-da}$),
Exemplification 7-35: Z-$L^{3-c}$-N(-$L^{1-a}$-$D^{1-a}$)($L^{2-q}$-$D^{2-da}$),
Exemplification 7-36: Z-$L^{3-c}$-N(-$L^{1-a}$-$D^{1-a}$)($L^{2-r}$-$D^{2-da}$),
Exemplification 7-37: Z-$L^{3-c}$-N(-$L^{1-a}$-$D^{1-b}$)($L^{2-o}$-$D^{2-da}$),
Exemplification 7-38: Z-$L^{3-c}$-N(-$L^{1-a}$-$D^{1-b}$)($L^{2-p}$-$D^{2-da}$),
Exemplification 7-39: Z-$L^{3-c}$-N(-$L^{1-a}$-$D^{1-b}$)($L^{2-q}$-$D^{2-da}$),
Exemplification 7-40: Z-$L^{3-c}$-N(-$L^{1-a}$-$D^{1-b}$)($L^{2-r}$-$D^{2-da}$),
Exemplification 7-41: Z-$L^{3-c}$-N(-$L^{1-b}$-$D^{1-a}$)($L^{2-o}$-$D^{2-da}$),
Exemplification 7-42: Z-$L^{3-c}$-N(-$L^{1-b}$-$D^{1-a}$)($L^{2-p}$-$D^{2-da}$),
Exemplification 7-43: Z-$L^{3-c}$-N(-$L^{1-b}$-$D^{1-a}$)($L^{2-q}$-$D^{2-da}$),

Exemplification 7-44: $Z-L^{3-c}-N(-L^{1-b}-D^{1-a})(L^{2-r}-D^{2-da})$,
Exemplification 7-45: $Z-L^{3-c}-N(-L^{1-b}-D^{1-b})(L^{2-o}-D^{2-da})$,
Exemplification 7-46: $Z-L^{3-c}-N(-L^{1-b}-D^{1-b})(L^{2-p}-D^{2-da})$,
Exemplification 7-47: $Z-L^{3-c}-N(-L^{1-b}-D^{1-b})(L^{2-q}-D^{2-da})$,
Exemplification 7-48: $Z-L^{3-c}-N(-L^{1-b}-D^{1-b})(L^{2-r}-D^{2-da})$,
Exemplification 7-49: $Z-L^{3-d}-N(-L^{1-a}-D^{1-a})(L^{2-o}-D^{2-da})$,
Exemplification 7-50: $Z-L^{3-d}-N(-L^{1-a}-D^{1-a})(L^{2-p}-D^{2-da})$,
Exemplification 7-51: $Z-L^{3-d}-N(-L^{1-a}-D^{1-a})(L^{2-q}-D^{2-da})$,
Exemplification 7-52: $Z-L^{3-d}-N(-L^{1-a}-D^{1-a})(L^{2-r}-D^{2-da})$,
Exemplification 7-53: $Z-L^{3-d}-N(-L^{1-a}-D^{1-b})(L^{2-o}-D^{2-da})$,
Exemplification 7-54: $Z-L^{3-d}-N(-L^{1-a}-D^{1-b})(L^{2-p}-D^{2-da})$,
Exemplification 7-55: $Z-L^{3-d}-N(-L^{1-a}-D^{1-b})(L^{2-q}-D^{2-da})$,
Exemplification 7-56: $Z-L^{3-d}-N(-L^{1-a}-D^{1-b})(L^{2-r}-D^{2-da})$,
Exemplification 7-57: $Z-L^{3-d}-N(-L^{1-b}-D^{1-a})(L^{2-o}-D^{2-da})$,
Exemplification 7-58: $Z-L^{3-d}-N(-L^{1-b}-D^{1-a})(L^{2-p}-D^{2-da})$,
Exemplification 7-59: $Z-L^{3-d}-N(-L^{1-b}-D^{1-a})(L^{2-q}-D^{2-da})$,
Exemplification 7-60: $Z-L^{3-d}-N(-L^{1-b}-D^{1-a})(L^{2-r}-D^{2-da})$,
Exemplification 7-61: $Z-L^{3-d}-N(-L^{1-b}-D^{1-b})(L^{2-o}-D^{2-da})$,
Exemplification 7-62: $Z-L^{3-d}-N(-L^{1-b}-D^{1-b})(L^{2-p}-D^{2-da})$,
Exemplification 7-63: $Z-L^{3-d}-N(-L^{1-b}-D^{1-b})(L^{2-q}-D^{2-da})$,
Exemplification 7-64: $Z-L^{3-d}-N(-L^{1-b}-D^{1-b})(L^{2-r}-D^{2-da})$,
Exemplification 7-65: $Z-L^{3-e}-N(-L^{1-a}-D^{1-a})(L^{2-o}-D^{2-da})$,
Exemplification 7-66: $Z-L^{3-e}-N(-L^{1-a}-D^{1-a})(L^{2-p}-D^{2-da})$,
Exemplification 7-67: $Z-L^{3-e}-N(-L^{1-a}-D^{1-a})(L^{2-q}-D^{2-da})$,
Exemplification 7-68: $Z-L^{3-e}-N(-L^{1-a}-D^{1-a})(L^{2-r}-D^{2-da})$,
Exemplification 7-69: $Z-L^{3-e}-N(-L^{1-a}-D^{1-b})(L^{2-o}-D^{2-da})$,
Exemplification 7-70: $Z-L^{3-e}-N(-L^{1-a}-D^{1-b})(L^{2-p}-D^{2-da})$,
Exemplification 7-71: $Z-L^{3-e}-N(-L^{1-a}-D^{1-b})(L^{2-q}-D^{2-da})$,
Exemplification 7-72: $Z-L^{3-e}-N(-L^{1-a}-D^{1-b})(L^{2-r}-D^{2-da})$,
Exemplification 7-73: $Z-L^{3-e}-N(-L^{1-b}-D^{1-a})(L^{2-o}-D^{2-da})$,
Exemplification 7-74: $Z-L^{3-e}-N(-L^{1-b}-D^{1-a})(L^{2-p}-D^{2-da})$,
Exemplification 7-75: $Z-L^{3-e}-N(-L^{1-b}-D^{1-a})(L^{2-q}-D^{2-da})$,
Exemplification 7-76: $Z-L^{3-e}-N(-L^{1-b}-D^{1-a})(L^{2-r}-D^{2-da})$,
Exemplification 7-77: $Z-L^{3-e}-N(-L^{1-b}-D^{1-b})(L^{2-o}-D^{2-da})$,
Exemplification 7-78: $Z-L^{3-e}-N(-L^{1-b}-D^{1-b})(L^{2-p}-D^{2-da})$,
Exemplification 7-79: $Z-L^{3-e}-N(-L^{1-b}-D^{1-b})(L^{2-q}-D^{2-da})$,
Exemplification 7-80: $Z-L^{3-e}-N(-L^{1-b}-D^{1-b})(L^{2-r}-D^{2-da})$,
Exemplification 7-81: $Z-L^{3-j}-N(-L^{1-a}-D^{1-a})(L^{2-o}-D^{2-da})$,
Exemplification 7-82: $Z-L^{3-j}-N(-L^{1-a}-D^{1-a})(L^{2-p}-D^{2-da})$,
Exemplification 7-83: $Z-L^{3-j}-N(-L^{1-a}-D^{1-a})(L^{2-q}-D^{2-da})$,
Exemplification 7-84: $Z-L^{3-j}-N(-L^{1-a}-D^{1-a})(L^{2-r}-D^{2-da})$,
Exemplification 7-85: $Z-L^{3-j}-N(-L^{1-a}-D^{1-b})(L^{2-o}-D^{2-da})$,
Exemplification 7-86: $Z-L^{3-j}-N(-L^{1-a}-D^{1-b})(L^{2-p}-D^{2-da})$,
Exemplification 7-87: $Z-L^{3-j}-N(-L^{1-a}-D^{1-b})(L^{2-q}-D^{2-da})$,
Exemplification 7-88: $Z-L^{3-j}-N(-L^{1-a}-D^{1-b})(L^{2-r}-D^{2-da})$,
Exemplification 7-89: $Z-L^{3-j}-N(-L^{1-b}-D^{1-a})(L^{2-o}-D^{2-da})$,
Exemplification 7-90: $Z-L^{3-j}-N(-L^{1-b}-D^{1-a})(L^{2-p}-D^{2-da})$,
Exemplification 7-91: $Z-L^{3-j}-N(-L^{1-b}-D^{1-a})(L^{2-q}-D^{2-da})$,
Exemplification 7-92: $Z-L^{3-j}-N(-L^{1-b}-D^{1-a})(L^{2-r}-D^{2-da})$,
Exemplification 7-93: $Z-L^{3-j}-N(-L^{1-b}-D^{1-b})(L^{2-o}-D^{2-da})$,
Exemplification 7-94: $Z-L^{3-j}-N(-L^{1-b}-D^{1-b})(L^{2-p}-D^{2-da})$,
Exemplification 7-95: $Z-L^{3-j}-N(-L^{1-b}-D^{1-b})(L^{2-q}-D^{2-da})$,
Exemplification 7-96: $Z-L^{3-j}-N(-L^{1-b}-D^{1-b})(L^{2-r}-D^{2-da})$,
Exemplification 7-97: $Z-L^{3-k}-N(-L^{1-a}-D^{1-a})(L^{2-o}-D^{2-da})$,
Exemplification 7-98: $Z-L^{3-k}-N(-L^{1-a}-D^{1-a})(L^{2-p}-D^{2-da})$,
Exemplification 7-99: $Z-L^{3-k}-N(-L^{1-a}-D^{1-a})(L^{2-q}-D^{2-da})$,
Exemplification 7-100: $Z-L^{3-k}-N(-L^{1-a}-D^{1-a})(L^{2-r}-D^{2-da})$,
Exemplification 7-101: $Z-L^{3-k}-N(-L^{1-a}-D^{1-b})(L^{2-o}-D^{2-da})$,

Exemplification 7-102: Z-$L^{3-k}$-N(-$L^{1-a}$-$D^{1-b}$)($L^{2-p}$-$D^{2-da}$),
Exemplification 7-103: Z-$L^{3-k}$-N(-$L^{1-a}$-$D^{1-b}$)($L^{2-q}$-$D^{2-da}$),
Exemplification 7-104: Z-$L^{3-k}$-N(-$L^{1-a}$-$D^{1-b}$)($L^{2-r}$-$D^{2-da}$),
Exemplification 7-105: Z-$L^{3-k}$-N(-$L^{1-b}$-$D^{1-a}$)($L^{2-o}$-$D^{2-da}$),
Exemplification 7-106: Z-$L^{3-k}$-N(-$L^{1-b}$-$D^{1-a}$)($L^{2-p}$-$D^{2-da}$),
Exemplification 7-107: Z-$L^{3-k}$-N(-$L^{1-b}$-$D^{1-a}$)($L^{2-q}$-$D^{2-da}$),
Exemplification 7-108: Z-$L^{3-k}$-N(-$L^{1-b}$-$D^{1-a}$)($L^{2-r}$-$D^{2-da}$),
Exemplification 7-109: Z-$L^{3-k}$-N(-$L^{1-b}$-$D^{1-b}$)($L^{2-o}$-$D^{2-da}$),
Exemplification 7-110: Z-$L^{3-k}$-N(-$L^{1-b}$-$D^{1-b}$)($L^{2-p}$-$D^{2-da}$),
Exemplification 7-111: Z-$L^{3-k}$-N(-$L^{1-b}$-$D^{1-b}$)($L^{2-q}$-$D^{2-da}$),
Exemplification 7-112: Z-$L^{3-k}$-N(-$L^{1-b}$-$D^{1-b}$)($L^{2-r}$-$D^{2-da}$),
Exemplification 7-113: Z-$L^{3-l}$-N(-$L^{1-a}$-$D^{1-a}$)($L^{2-o}$-$D^{2-da}$),
Exemplification 7-114: Z-$L^{3-l}$-N(-$L^{1-a}$-$D^{1-a}$)($L^{2-p}$-$D^{2-da}$),
Exemplification 7-115: Z-$L^{3-l}$-N(-$L^{1-a}$-$D^{1-a}$)($L^{2-q}$-$D^{2-da}$),
Exemplification 7-116: Z-$L^{3-l}$-N(-$L^{1-a}$-$D^{1-a}$)($L^{2-r}$-$D^{2-da}$),
Exemplification 7-117: Z-$L^{3-l}$-N(-$L^{1-a}$-$D^{1-b}$)($L^{2-o}$-$D^{2-da}$),
Exemplification 7-118: Z-$L^{3-l}$-N(-$L^{1-a}$-$D^{1-b}$)($L^{2-p}$-$D^{2-da}$),
Exemplification 7-119: Z-$L^{3-l}$-N(-$L^{1-a}$-$D^{1-b}$)($L^{2-q}$-$D^{2-da}$),
Exemplification 7-120: Z-$L^{3-l}$-N(-$L^{1-a}$-$D^{1-b}$)($L^{2-r}$-$D^{2-da}$),
Exemplification 7-121: Z-$L^{3-l}$-N(-$L^{1-b}$-$D^{1-a}$)($L^{2-o}$-$D^{2-da}$),
Exemplification 7-122: Z-$L^{3-l}$-N(-$L^{1-b}$-$D^{1-a}$)($L^{2-p}$-$D^{2-da}$),
Exemplification 7-123: Z-$L^{3-l}$-N(-$L^{1-b}$-$D^{1-a}$)($L^{2-q}$-$D^{2-da}$),
Exemplification 7-124: Z-$L^{3-l}$-N(-$L^{1-b}$-$D^{1-a}$)($L^{2-r}$-$D^{2-da}$),
Exemplification 7-125: Z-$L^{3-l}$-N(-$L^{1-b}$-$D^{1-b}$)($L^{2-o}$-$D^{2-da}$),
Exemplification 7-126: Z-$L^{3-l}$-N(-$L^{1-b}$-$D^{1-b}$)($L^{2-p}$-$D^{2-da}$),
Exemplification 7-127: Z-$L^{3-l}$-N(-$L^{1-b}$-$D^{1-b}$)($L^{2-q}$-$D^{2-da}$),
Exemplification 7-128: Z-$L^{3-l}$-N(-$L^{1-b}$-$D^{1-b}$)($L^{2-r}$-$D^{2-da}$),
Exemplification 7-129: Z-$L^{3-m}$-N(-$L^{1-a}$-$D^{1-a}$)($L^{2-o}$-$D^{2-da}$),
Exemplification 7-130: Z-$L^{3-m}$-N(-$L^{1-a}$-$D^{1-a}$)($L^{2-p}$-$D^{2-da}$),
Exemplification 7-131: Z-$L^{3-m}$-N(-$L^{1-a}$-$D^{1-a}$)($L^{2-q}$-$D^{2-da}$),
Exemplification 7-132: Z-$L^{3-m}$-N(-$L^{1-a}$-$D^{1-a}$)($L^{2-r}$-$D^{2-da}$),
Exemplification 7-133: Z-$L^{3-m}$-N(-$L^{1-a}$-$D^{1-b}$)($L^{2-o}$-$D^{2-da}$),
Exemplification 7-134: Z-$L^{3-m}$-N(-$L^{1-a}$-$D^{1-b}$)($L^{2-p}$-$D^{2-da}$),
Exemplification 7-135: Z-$L^{3-m}$-N(-$L^{1-a}$-$D^{1-b}$)($L^{2-q}$-$D^{2-da}$),
Exemplification 7-136: Z-$L^{3-m}$-N(-$L^{1-a}$-$D^{1-b}$)($L^{2-r}$-$D^{2-da}$),
Exemplification 7-137: Z-$L^{3-m}$-N(-$L^{1-b}$-$D^{1-a}$)($L^{2-o}$-$D^{2-da}$),
Exemplification 7-138: Z-$L^{3-m}$-N(-$L^{1-b}$-$D^{1-a}$)($L^{2-p}$-$D^{2-da}$),
Exemplification 7-139: Z-$L^{3-m}$-N(-$L^{1-b}$-$D^{1-a}$)($L^{2-q}$-$D^{2-da}$),
Exemplification 7-140: Z-$L^{3-m}$-N(-$L^{1-b}$-$D^{1-a}$)($L^{2-r}$-$D^{2-da}$),
Exemplification 7-141: Z-$L^{3-m}$-N(-$L^{1-b}$-$D^{1-b}$)($L^{2-o}$-$D^{2-da}$),
Exemplification 7-142: Z-$L^{3-m}$-N(-$L^{1-b}$-$D^{1-b}$)($L^{2-p}$-$D^{2-da}$),
Exemplification 7-143: Z-$L^{3-m}$-N(-$L^{1-b}$-$D^{1-b}$)($L^{2-q}$-$D^{2-da}$), and
Exemplification 7-144: Z-$L^{3-m}$-N(-$L^{1-b}$-$D^{1-b}$)($L^{2-r}$-$D^{2-da}$).

[Examples]

Hereinafter, synthesis examples of the "antitumor drug molecule or an analog thereof, or a derivative thereof" used in the present invention will described as "production examples," synthesis examples of the conjugate precursor (I) and the conjugate precursor synthesis intermediates (II-1) and (II-2) of the present invention will be described as "examples," and a production example of an antibody-multidrug conjugate (AMDC) by a reaction of a conjugate precursor with an antibody and an evaluation test example of said AMDC will be respectively described as "Reference Example 1" and "Reference Example 2," but the present invention is not limited thereto.

In the synthesis examples of the conjugate precursor (I) and the conjugate precursor synthesis intermediates (II-1) and (II-2) (that is, examples), all stereoisomers of amino acid residues in the linker moieties are L-types, but they may be D-types independently in the present invention, and the reaction is carried out in the same manner even in the case of D-types.

In addition, reagents, solvents, and starting materials which are not specifically described in the production examples, examples, and reference examples are easily available from commercially available supply sources.

Hereinafter, the methods for producing the antibody-drug conjugates of the present invention as well as the methods for producing the starting materials and the intermediates are specifically described in detail as Examples or Reference Examples, but the present invention is not limited to them. Also, reagents, solvents, and starting materials which are not specifically defined in the present description are easily available from commercially available supply sources.

Abbreviations correspond to structures as follows.

[C241]

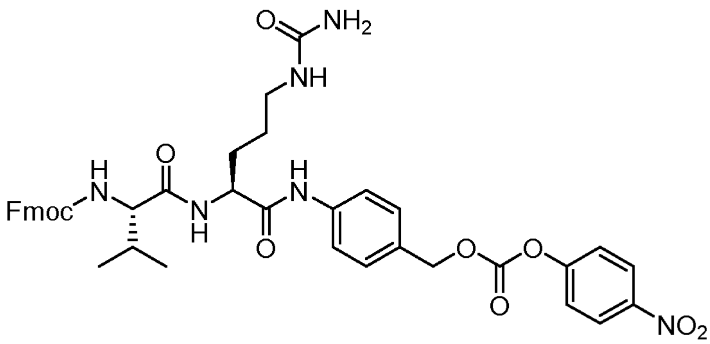

Fmoc- (L)-Val- (L)-Cit-PAB-PNP

[C242]

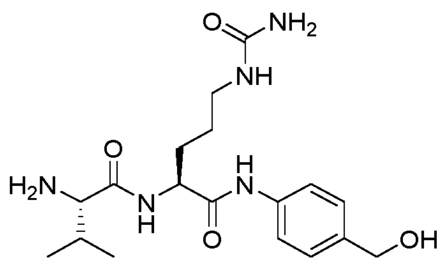

(L)-Val-(L)-Cit-PAB

[C243]

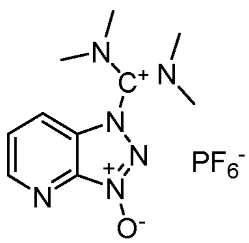

1-[bis(dimethylamino)methylene]-1H-1,2,3-triazolo[4,5-b]pyridinium 3-oxide hexafluorophosphate (hereinafter referred to as HATU)

[C244]

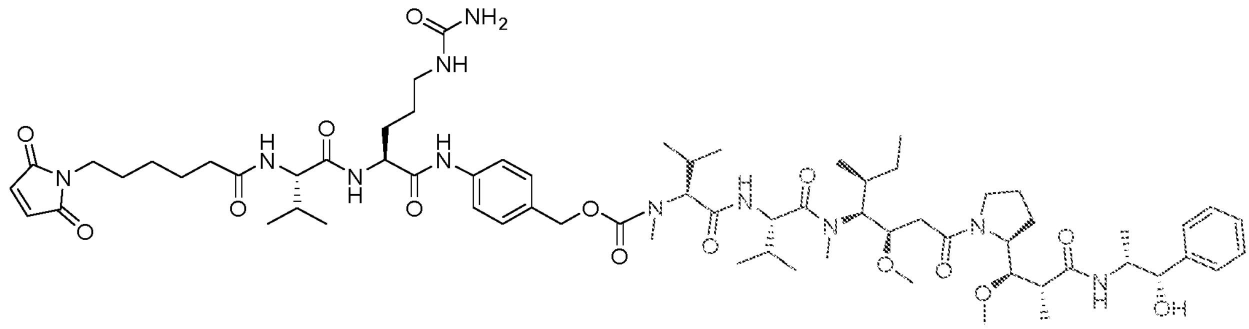

MC-Val-Cit-PAB-MMAE

[C245]

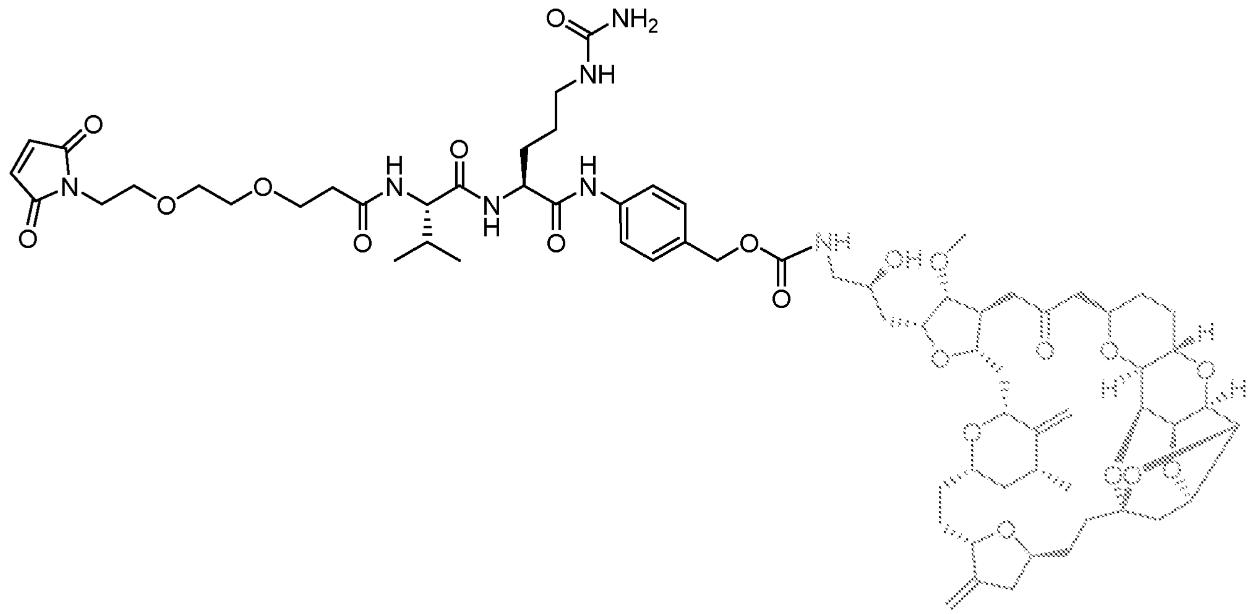

Mal-PEG2-Val-Cit-PAB-Eribulin

[Production examples]: Synthesis of antitumor drug molecule or analog thereof, or derivative thereof

Production Example 1. U-001

[C246]

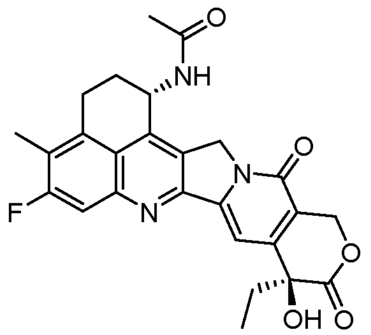

(U-001)

To a solution of exatecan mesylate (284.1 mg, 0.534 mmoL) and triethylamine (0.70 mL, 508.2 mg, 5.02 mmoL) in dichloromethane (5 mL) in a 30 mL cylindrical flask was added acetic anhydride (0.14 mL, 151.2 mg, 1.481 mmoL) under argon atmosphere with stirring at room temperature, and the resulting mixture was stirred at room temperature for 2 hours. After the reaction was completed, the resulting precipitates were filtered, and washed with dichloromethane to give U-001 (239.9 mg, yield: 94.0%) as light brown solids.
MS (ESI) m/z 478 $(M+H)^+$

Production example 2. U-002

[C247]

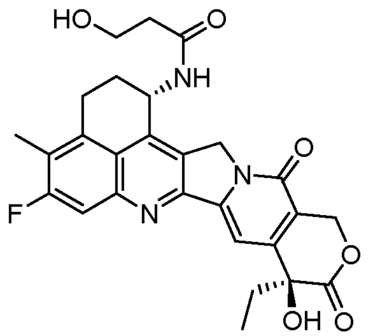

(U-002)

To a solution of exatecan mesylate (20.4 mg, 0.038 mmoL) in N,N-dimethylformamide (0.6 mL) in a 10 mL cylindrical flask were added 3-hydroxypropanoic acid (0.015 mL, 4.86 mg, 0.054 mmoL), 1-hydroxybenzotriazole (15.6 mg, 0.115 mmoL), 1-ethyl-3-(3-dimethylaminopropyl)carbodiimide hydrochloride (21.6 mg, 0.113 mmoL), and triethylamine (0.040 mL, 29.04 mg, 0.287 mmoL) under argon gas flow with stirring at room temperature, and the resulting mixture was stirred at room temperature for 3 hours. After the reaction was completed, to the reaction solution was added a saturated aqueous solution of sodium hydrogen carbonate, and the resulting mixed solution was subjected to extraction with ethyl acetate. The resulting organic layer was washed with saturated brine, dried over anhydrous magnesium sulfate, filtered, and concentrated under reduced pressure. The resulting residues were subjected to YAMAZEN medium pressure flash chromatography (Silica, M (16 g), ethyl acetate / methanol = 97/3 (V/V) → 60/40 (V/V)), and the fraction comprising the target compound (Rf value = 0.44 (ethyl acetate / methanol = 85/15 (V/V))) was concentrated under reduced pressure to give U-002 (9.8 mg, yield: 50.31%) as pale yellow solids.
MS (ESI) m/z 508 $(M+H)^+$

Production example 3. Synthesis of U-003 3-1. U-003-1

[C248]

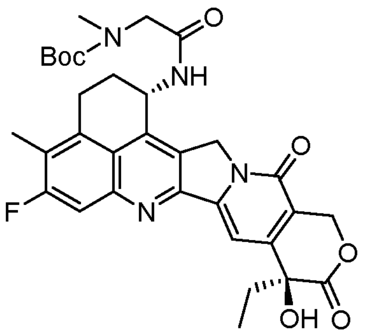

(U-003-1)

To a solution of exatecan mesylate (22 mg, 0.041 mmoL) in N,N-dimethylformamide (0.8 mL) in a 10 mL cylindrical flask were added N-(tert-butoxycarbonyl)-N-methylglycine (11 mg, 0.058 mmoL), 1-hydroxybenzotriazole (13 mg, 0.096 mmoL), 1-ethyl-3-(3-dimethylaminopropyl)carbodiimide hydrochloride (13 mg, 0.068 mmoL), and triethylamine (0.032 mL, 23.23 mg, 0.230 mmoL) under argon gas flow with stirring at room temperature, the resulting mixture was stirred at room temperature for 2 hours, and then left to stand overnight. After the reaction was completed, to the reaction solution was added a saturated aqueous solution of sodium hydrogen carbonate (5 mL × 2), and the resulting mixed solution was subjected to extraction with dichloromethane (15 mL). The resulting organic layer was washed with saturated brine (5 mL), dried over anhydrous magnesium sulfate, filtered, and concentrated under reduced pressure to give U-003-1 (29 mg, yield: quantitative).
MS (ESI) m/z 607 $(M+H)^+$

3-2. U-003

[C249]

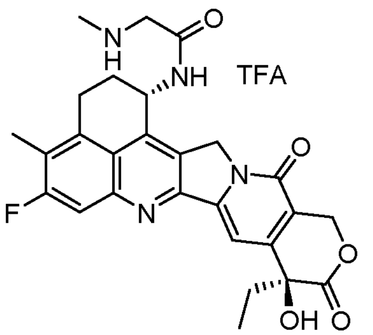

(U-003)

To a solution of U-003-1 (25 mg, 0.041 mmoL) in dichloromethane (0.6 mL) in a 20 mL cylindrical flask was added trifluoroacetic acid (0.050 mL, 74 mg, 0.649 mmoL) under argon gas flow with stirring at room temperature, the resulting mixture was stirred at room temperature for 1.5 hours, and then concentrated under reduced pressure. To the resulting residues was added methanol, the resulting mixture was concentrated under reduced pressure, then ethyl acetate was added thereto, and the resulting solids were separated by filtration. The resulting solids were dried under reduced pressure to give trifluoroacetate of U-003 (13 mg, yield: 50.83%) as brown solids.
MS (ESI) m/z 507 $(M+H)^+$

Production example 4. Synthesis of U-004 4-1. U-004-1

[C250]

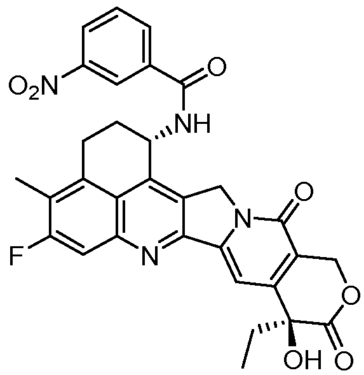

(U-004-1)

To a solution of exatecan mesylate (20.6 mg, 0.039 mmoL) and N,N-diisopropylethylamine (0.020 mL, 14.8 mg, 0.115 mmoL) in dichloromethane (1 mL) in a 10 mL cylindrical flask was added 3-nitrobenzoyl chloride (9.6 mg, 0.052 mmoL) with stirring at room temperature, and the resulting mixture was stirred at room temperature for 1 hour. After the reaction was completed, to the reaction solution was added water, and the resulting mixed solution was subjected to extraction with ethyl acetate. The resulting organic layer was concentrated under reduced pressure to give U-004-1 (22.6 mg, yield: 99.76%).

4-2. U-004

[C251]

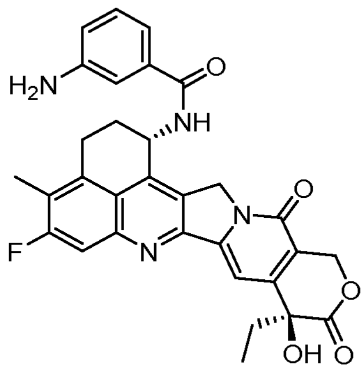

(U-004)

A solution of U-004-1 (22.6 mg, 0.039 mmoL) and 10% palladium carbon NX-Type (20.6 mg, 0.0968 mmoL) in a mixture of ethanol (1 mL) / dichloromethane (1 mL) in a 30 mL round-bottom flask was stirred under hydrogen atmosphere at room temperature for 2 hours. After the reaction was completed, the catalyst was separated by filtration, and the solvent was concentrated under reduced pressure. The resulting residues were subjected to YAMAZEN medium pressure flash chromatography (Silica, M (16 g), dichloromethane/methanol = 100/0 (V/V) → 94/6 (V/V)), and the fraction comprising the target compound was concentrated under reduced pressure to give U-004 (9.4 mg, yield: 43.84%) as slightly yellow solids. MS (DUIS) m/z 555 $(M+H)^+$

Production example 5. U-005

5-1. U-005-1

[C252]

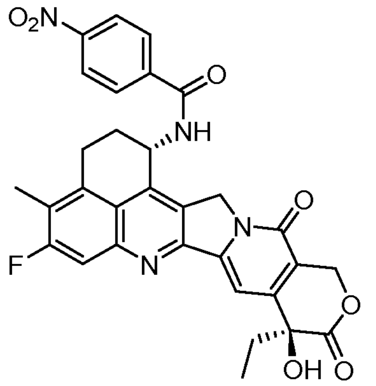

(U-005-1)

To a solution of exatecan mesylate (21.2 mg, 0.040 mmoL) and N,N-diisopropylethylamine (0.020 mL, 14.8 mg, 0.115 mmoL) in dichloromethane (1 mL) in a 10 mL cylindrical flask was added 4-nitrobenzoyl chloride (10.6 mg, 0.057 mmoL) with stirring at room temperature, and the resulting mixture was stirred at room temperature for 1 hour. Subsequently, 4-nitrobenzoyl chloride (10.6 mg, 0.057 mmoL) was added thereto under stirring at room temperature, and the resulting mixture was stirred at room temperature for 1 hour. After the reaction was completed, to the reaction solution were added water and tert-butyl methyl ether, the resulting mixture was stirred, the resulting solids were filtered, and sequentially washed with water and tert-butyl methyl ether to give U-005-1 (22.1 mg, yield: 94.79%).

5-2. U-005

[C253]

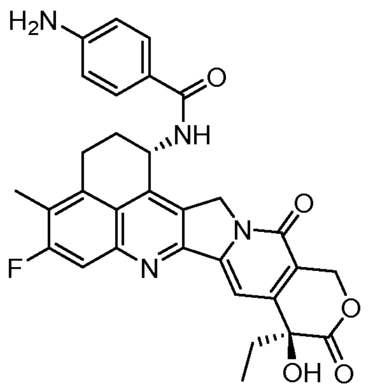

(U-005)

A solution of U-005-1 (22.6 mg, 0.039 mmoL) and 10% palladium carbon NX-Type (15.4 mg, 0.0724 mmoL) in a mixture of ethanol (1 mL) / dichloromethane (1 mL) in a 30 mL round-bottom flask was stirred under hydrogen atmosphere at room temperature for 2 hours. After the reaction was completed, the catalyst was separated by filtration, and the solvent was concentrated under reduced pressure. The resulting residues were subjected to YAMAZEN medium pressure flash chromatography (Silica, M (16 g), dichloroethane/methanol = 100/0 (V/V) → 94/6 (V/V)), and the fraction comprising the target compound was concentrated under reduced pressure to give crude products of U-005. The resulting crude products were subjected to preparative HPLC under the following conditions, the fraction comprising the target compound was neutralized by a saturated aqueous solution of sodium hydrogen carbonate, the resulting mixture was concentrated under reduced pressure, then to the precipitated solids was added water, the resulting insoluble matters were collected by filtration, and washed with water to give U-005 (7.1 mg, yield: 33.11%) as slightly yellow solids. MS (ESI) m/z 555 $(M+H)^+$

Column: Waters SunFire Prep C18 5 μm ODB 19*150 mm

Flow rate: 17 mL/min.
Elution solvent: 0.1% aqueous solution of formic acid (Solution A) - acetonitrile (Solution B)
Gradient (Solution B): 30% (0.00 min.) → 95% (10.00 min.)

Production example 6. U-006

6-1. U-006-1

[C254]

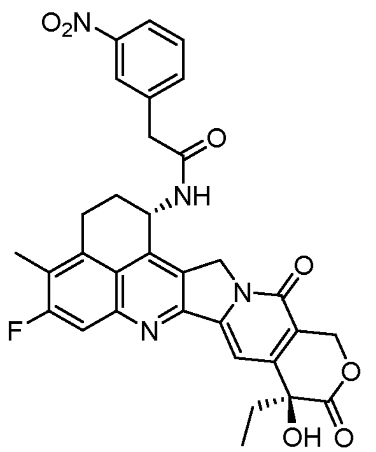

(U-006-1)

To a solution of exatecan mesylate (23.1 mg, 0.043 mmoL), 2-(3-nitrophenyl)acetic acid (15.7 mg, 0.087 mmoL), 1-ethyl-3-(3-dimethylaminopropyl)carbodiimide hydrochloride (16.7 mg, 0.087 mmoL), and 1-hydroxybenzotriazole (13.3 mg, 0.087 mmoL) in N,N-dimethylformamide in a 10 mL cylindrical flask was added triethylamine (0.012 mL, 8.71 mg, 0.086 mmoL) under argon atmosphere with stirring at room temperature, and the resulting mixture was stirred at room temperature for 1 hour. After the reaction was completed, to the reaction solution was added a saturated aqueous solution of sodium hydrogen carbonate, and the resulting mixed solution was subjected to extraction with ethyl acetate. The resulting organic layer was concentrated under reduced pressure to give crude products of U-006-1 (38.4 mg).
MS (ESI) m/z 599 $(M+H)^+$

6-2. U-006

[C255]

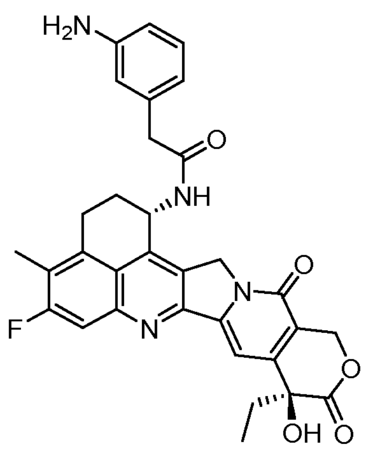

(U-006)

A solution of U-006-1 (38.4 mg, 0.044 mmoL) and 10% palladium carbon NX-Type (21.3 mg, 0.200 mmoL) in a mixture of ethanol (1 mL) / dichloromethane (1 mL) in a 30 mL round-bottom flask was stirred under hydrogen atmosphere at room temperature for 2 hours. After the reaction was completed, the catalyst was separated by filtration, and the solvent was concentrated under reduced pressure. The resulting residues were subjected to YAMAZEN medium pressure flash chromatography (Silica, M (16 g), dichloroethane/methanol = 100/0 (V/V) → 94/6 (V/V)), and the fraction comprising the target compound was concentrated under reduced pressure. To the resulting residues was added ethanol, the precipitated solids were collected by filtration, and washed with ethanol to give U-006 (16.6 mg, total yield of 6-1 and 6-2: 66.92%) as colorless solids.

MS (DUIS) m/z 569 $(M+H)^+$

Production example 7. U-007

7-1. U-007-1

[C256]

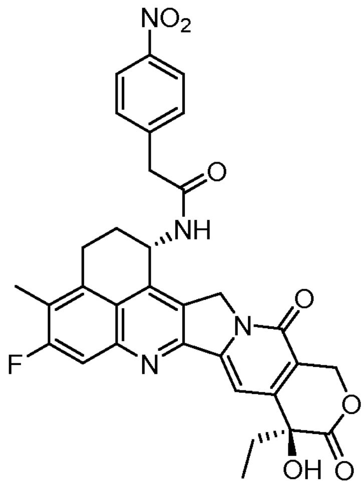

(U-007-1)

To a solution of exatecan mesylate (32.5 mg, 0.061 mmoL), 2-(4-nitrophenyl) acetic acid (22.1 mg, 0.122 mmoL), 1-ethyl-3-(3-dimethylaminopropyl)carbodiimide hydrochloride (23.4 mg, 0.122 mmoL), and 1-hydroxybenzotriazole (16.7 mg, 0.109 mmoL) in N,N-dimethylformamide (0.61 mL) in a 10 mL cylindrical flask was added triethylamine (0.017 mL, 12.34 mg, 0.122 mmoL) with stirring at room temperature, and the resulting mixture was stirred at room temperature for 1 hour. After the reaction was completed, to the reaction solution was added a saturated aqueous solution of sodium hydrogen carbonate, and the resulting mixed solution was subjected to extraction with ethyl acetate. The resulting organic layer was concentrated under reduced pressure to give U-007-1 (26.8 mg, yield: 73.23%).

MS (ESI) m/z 598 $(M+H)^+$

7-2. U-007

[C257]

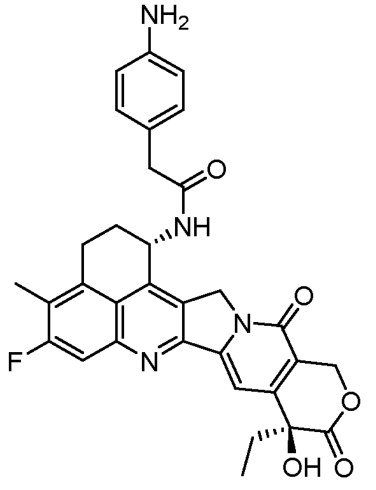

(U-007)

A solution of U-007-1 (26.8 mg, 0.045 mmoL) and 10% palladium carbon NX-Type (20.3 mg, 0.0954 mmoL) in a mixture of ethanol (1 mL) / dichloromethane (1 mL) in a 30 mL round-bottom flask was stirred under hydrogen atmosphere at room temperature for 2 hours. After the reaction was completed, the catalyst was separated by filtration, and the solvent was concentrated under reduced pressure. To the resulting residues was added ethanol, the precipitated solids were collected by filtration, and washed with ethanol to give U-007 (24.2 mg, yield: 95.06%) as light brown solids.
MS (DUIS) m/z 569 $(M+H)^+$

Production example 8. U-008

8-1. U-008-1

[C258]

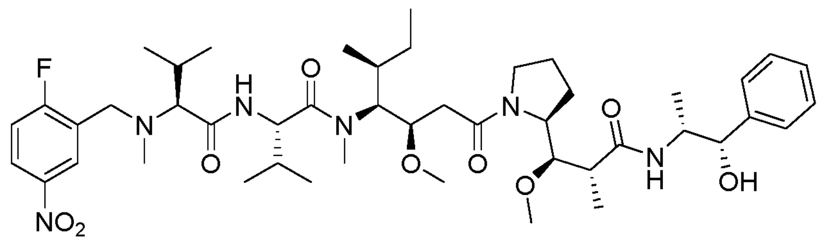

(U-008-1)

To a solution of MMAE (0.36 g, 0.501 mmoL) and 2-fluoro-5-nitrobenzaldehyde (0.25 g, 1.478 mmoL) in dichloromethane (5 mL) in a 30 mL cylindrical flask was added sodium triacetoxyborohydride (0.32 g, 1.510 mmoL) under argon atmosphere with stirring at room temperature, and the resulting mixture was stirred at room temperature for 1 hour. 2-fluoro-5-nitrobenzaldehyde (0.25 g, 1.478 mmoL) and sodium triacetoxyborohydride (0.32 g, 1.510 mmoL) were additionally added thereto, and the resulting mixture was stirred at room temperature for 1 hour. After the reaction was completed, to the reaction solution was added a saturated aqueous solution of sodium hydrogen carbonate, and the resulting mixed solution was subjected to extraction with dichloromethane. The resulting organic layer was dried over anhydrous magnesium sulfate, filtered, and concentrated under reduced pressure. The resulting residues were subjected to YAMAZEN medium pressure flash chromatography (Silica, M (16 g), ethyl acetate : methanol = 100/0 (V/V) → 90/10 (V/V)), and the fraction comprising the target compound was concentrated under reduced pressure to give U-008-1 (406.6 mg, yield: 93.09%) as pale yellow foam.
MS (DUIS) m/z 873 $(M+H)^+$

8-2.

[C259]

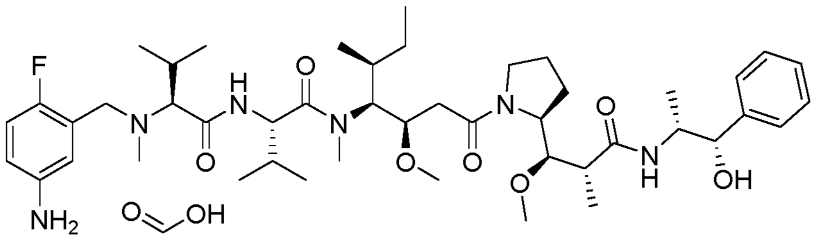

(U-008)

A solution of U-008-1 (45.1 mg, 0.052 mmoL) and zinc (89.3 mg, 1.366 mmoL) in acetic acid (0.2 mL) in a 30 mL cylindrical flask was stirred under argon atmosphere at room temperature for 2 hours. After the reaction was completed, to the reaction solution was added a saturated aqueous solution of sodium hydrogen carbonate, and the resulting mixed solution was subjected to extraction with ethyl acetate. The resulting organic layer was dried over anhydrous magnesium sulfate, filtered, and concentrated under reduced pressure. The resulting residues were subjected to YAMAZEN medium pressure flash chromatography (Silica, M (16 g), ethyl acetate / methanol = 100/0 (V/V) → 90/10 (V/V)), and the fraction comprising the target compound was concentrated under reduced pressure. The resulting residues were subjected to preparative HPLC under the following conditions, the fraction comprising the target compound was concentrated under reduced pressure to distill away acetonitrile, and then the resulting residues were freeze-dried to give formate of U-008 (13.3 mg, yield: 28.96%) as colorless foam.
MS (DUIS) m/z 841 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 95% (10.00 min.)

Production example 9. U-009

[C260]

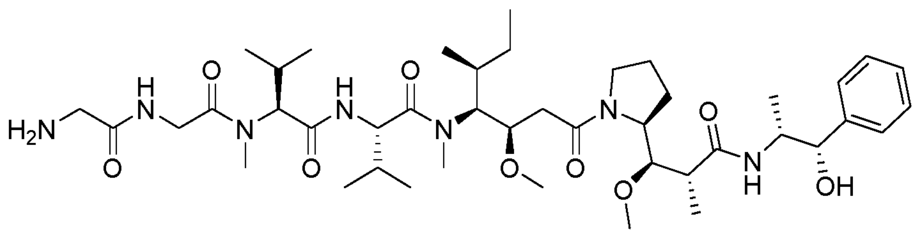

(U-009)

To a solution of (tert-butoxycarbonyl)glycylglycine (8.4 mg, 0.036 mmoL), 1-hydroxybenzotriazole (6.2 mg, 0.046 mmoL), and MMAE (22.3 mg, 0.031 mmoL) in N,N-dimethylformamide (0.1 mL) in a 10 mL cylindrical flask was added 1-ethyl-3-(3-dimethylaminopropyl)carbodiimide hydrochloride (8.8 mg, 0.046 mmoL) under argon gas flow with stirring at room temperature, and the resulting mixture was stirred at 60°C for 1 hour. The resulting reaction solution was subjected to preparative HPLC under the following conditions, and the fraction comprising the target compound was concentrated under reduced pressure to give a colorless oil.

Column: Waters SunFire Prep C18 5 μm ODB 19*150 mm
Flow rate: 17 mL/min.

Elution solvent: 0.1% aqueous solution of formic acid (Solution A) - acetonitrile (Solution B)
Gradient (Solution B): 30% (0.00 min.) → 95% (10.00 min.)

The resulting compound was dissolved in dichloromethane (1 mL) and trifluoroacetic acid (1 mL), the resulting mixture was stirred at room temperature for 1 hour, and then concentrated under reduced pressure. The resulting reaction solution was subjected to preparative HPLC under the following conditions, and the fraction comprising the target compound was concentrated under reduced pressure. Subsequently, the resulting residues were subjected to DNH silica gel column chromatography, eluted with dichloromethane/methanol (9/1 (V/V)), and the fraction comprising the target compound was concentrated under reduced pressure. Subsequently, the resulting residues were dissolved in a mixed solvent of water/acetonitrile, and freeze-dried to give the Production example 9 (U-009) (2.2 mg, yield: 8.51%) as colorless solids.
MS (ESI) m/z 833 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 95% (10.00 min.)

Production example 10. U-010

[C261]

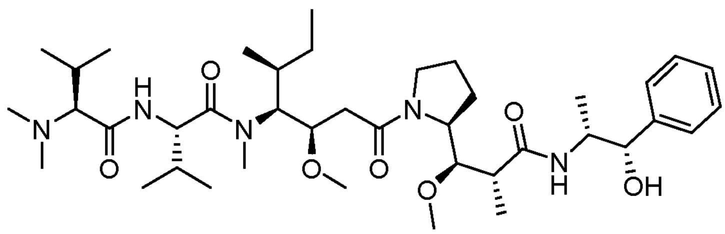

(U-010)

To a solution of MMAE (0.37 g, 0.515 mmoL) and a 37% aqueous solution of formaldehyde (0.37 mL, 0.4 g, 4.97 mmoL) in methanol (5 mL) in a 30 mL cylindrical flask was added sodium triacetoxyborohydride (0.32 g, 1.510 mmoL) under argon atmosphere with stirring at room temperature, and the resulting mixture was stirred at room temperature for 1 hour. After the reaction was completed, to the reaction solution was added a saturated aqueous solution of sodium hydrogen carbonate, and the resulting mixed solution was subjected to extraction with ethyl acetate. The resulting organic layer was dried over anhydrous magnesium sulfate, filtered, and concentrated under reduced pressure. The resulting residues were subjected to YAMAZEN medium pressure flash chromatography (Silica, M (16 g), dichloromethane/-methanol = 100/0 (V/V) → 90/10 (V/V)), and the fraction comprising the target compound was concentrated under reduced pressure to give U-010 (0.18 g, yield: 47.72%) as colorless foam.
MS (DUIS) m/z 732 $(M+H)^+$

Production example 11. U-011

11-1. U-011-1

[C262]

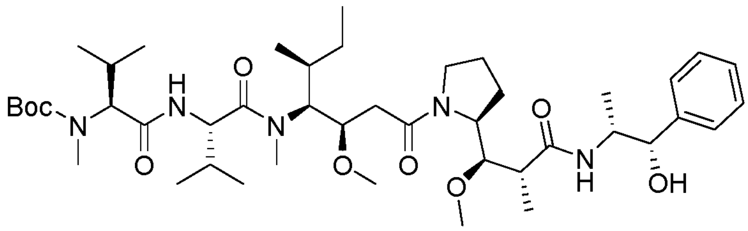

(U-011-1)

To a solution of MMAE (0.4996 g, 0.696 mmoL) and sodium carbonate (0.1503 g, 1.418 mmoL) in tetrahydrofuran (5 mL) in a 30 mL cylindrical flask was added water (5 mL), added di-tert-butyl dicarbonate (0.185 mL, 0.19 g, 0.848 mmoL) with stirring at room temperature, and the resulting mixture was stirred at room temperature for 18 hours and then stirred at 50°C for 1 hour. Di-tert-butyl dicarbonate (0.015 mL, 0.02 g, 0.069 mmoL) was added thereto, and the resulting mixture was stirred at 50°C for 1 hour. Di-tert-butyl dicarbonate (0.015 mL, 0.02 g, 0.069 mmoL) was additionally added thereto, and the resulting mixture was stirred at 50°C for 2 hours. After the reaction was completed, to the reaction solution were added ethyl acetate and saturated brine, and the resulting mixture was stirred at room temperature for a while. The resulting organic layer was dried over anhydrous magnesium sulfate, filtered, and concentrated under reduced pressure to give U-011-1 (570.5 mg, yield: quantitative) as white foam.
MS (ESI) m/z 818 $(M+H)^+$

11-2. U-011-2

[C263]

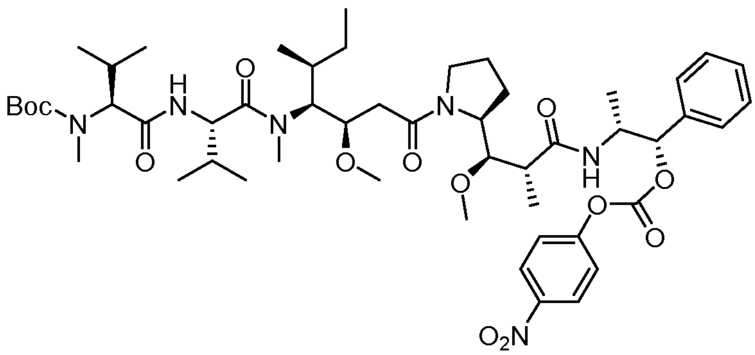

(U-011-2)

To a solution of U-011-1 (51.0 mg, 0.062 mmoL) and bis(4-nitrophenyl)carbonate (38.8 mg, 0.128 mmoL) in dichloromethane (1 mL) in a 0.2-0.5 mL microwave reaction container was added N,N-diisopropylethylamine (0.035 mL, 25.97 mg, 0.201 mmoL), the container was nitrogen-sealed, and the resulting mixture was stirred at room temperature for 3 hours. Subsequently, the mixture was subjected to a microwave reactor (Biotage), and reacted at 80°C for 1 hour. Subsequently, bis(4-nitrophenyl)carbonate (100.0 mg, 0.329 mmoL) and dichloromethane (0.5 mL) were additionally added thereto, and the resulting mixture was stirred at room temperature for 66 hours. The reaction solution was subjected to YAMAZEN medium pressure flash chromatography (Universal Premium silica gel, S (7 g) (biconnected), eluted with hexane / ethyl acetate (= 33/67 (V/V) → 12/88 (V/V))), and the fraction comprising the target compound was concentrated under reduced pressure to give U-011-2 (46.7 mg, yield: 76.19%) as slightly yellow foam.
MS (ESI) m/z 983 $(M+H)^+$

11-3. U-011-3

[C264]

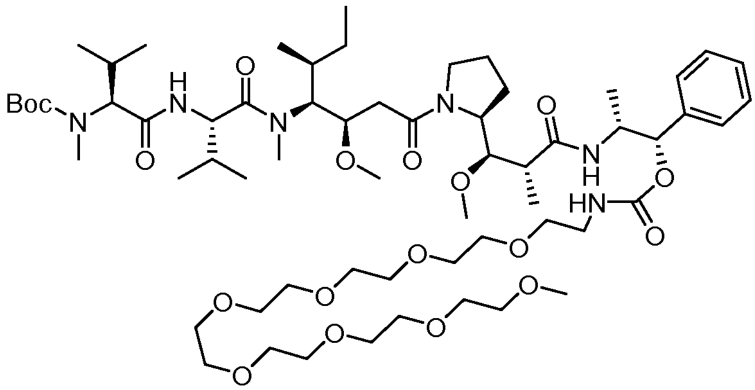

(U-011-3)

To a solution of U-011-2 (46.7 mg, 0.047 mmoL) and 3,6,9,12,15,18,21,24-octaoxapentacosan-1-amine (34.3 mg, 0.089 mmoL) in dichloromethane (2 mL) in a 20 mL pear-shaped flask was added N,N-diisopropylethylamine (0.030 mL, 22.26 mg, 0.172 mmoL), the resulting mixture was stirred under argon atmosphere at room temperature for 4 hours, and then left to stand at room temperature for 16.75 hours. The reaction solution was diluted with dichloromethane, packaged in Fuji Silysia Chromatorex Q-Pack DIOL-60 Size 20, eluted with hexane / ethyl acetate (= 0→100), and the fraction comprising the target compound was concentrated under reduced pressure to give U-011-3 (49.2 mg, yield: 84.38%) as a colorless oil.

MS (ESI) m/z 1227 $(M+H)^+$

11-4. U-011

[C265]

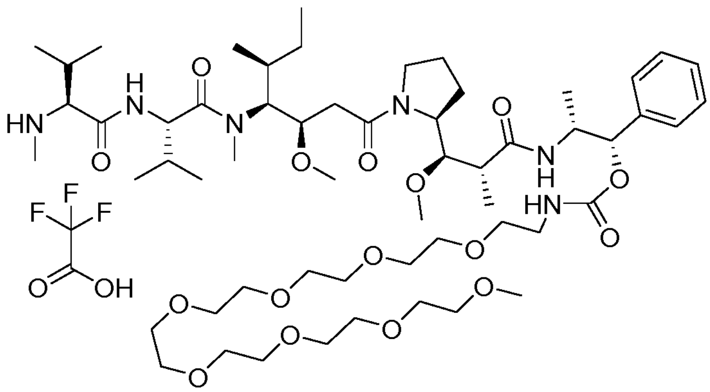

(U-011)

To a solution of U-011-3 (47 mg, 0.038 mmoL) in dichloromethane (2 mL) in a 30 mL pear-shaped flask was added trifluoroacetic acid (0.50 mL, 744.5 mg, 6.53 mmoL) at room temperature, and the resulting mixture was stirred at room temperature for 0.67 hour. Subsequently, the reaction solution was concentrated under reduced pressure. To the concentrated residues was added 0.1% formic acid solution in acetonitrile / water (= 1/9 (V/V)), and the resulting mixture was freeze-dried to give trifluoroacetate of U-011 (47.3 mg, yield: 99.51%) as white solids.

MS (ESI) m/z 1127 $(M+H)^+$

Production example 12. U-012

12-1. U-012-1

[C266]

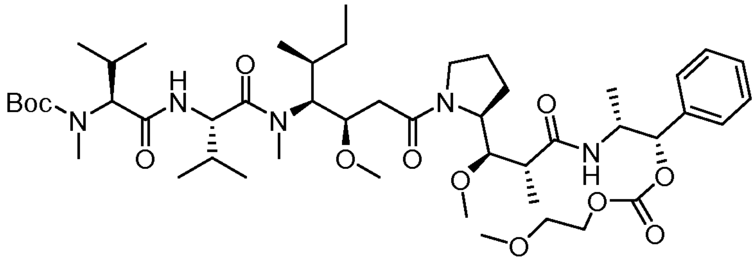

(U-012-1)

To a solution of U-011-1 (12.95 mg, 0.016 mmoL) and 4-dimethylaminopyridine (1.46 mg, 0.012 mmoL) in pyridine (0.3 mL) in a 0.2 mL - 0.5 mL microwave reaction container were added 2,4,6-trimethylpyridine (0.010 mL, 9.17 mg, 0.076 mmoL) and 2-methoxyethyl 2-chloroformate (0.050 mL, 59.6 mg, 0.43 mmoL), the container was nitrogen-sealed, the mixture was subjected to a microwave reactor (Biotage), and reacted at 100°C for 3 hours. The reaction solution was diluted with ethyl acetate, sequentially washed with water once, with a 5% aqueous solution of potassium hydrogen sulfate three times, with a saturated aqueous solution of sodium hydrogen carbonate once, and with saturated brine once, dried over anhydrous magnesium sulfate, filtered, and the resulting filtrate was concentrated under reduced pressure. The concentrated residues were dissolved in hexane / ethyl acetate (= 4/6 (V/V)), packaged in Fuji Silysia Chromatorex Q-Pack DIOL-60 Size 10, eluted with hexane / ethyl acetate (= 39/61 (V/V) → 18/82 (V/V)), and the fraction comprising the resulting target compound was concentrated under reduced pressure to give U-012-1 (9.4 mg, yield: 64.53%) as a colorless oil.
MS (ESI) m/z 920 $(M+H)^+$

12-2. U-012

[C267]

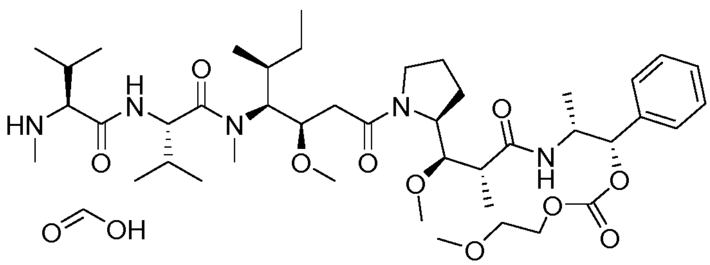

(U-012)

To a solution of U-012-1 (9.4 mg, 0.01022 mmoL) in dichloromethane (1 mL) in a 20 mL pear-shaped flask was added trifluoroacetic acid (0.050 mL, 74.45 mg, 0.653 mmoL) at room temperature, and the resulting mixture was stirred at room temperature for 1 hour. Subsequently, trifluoroacetic acid (0.450 mL, 670.05 mg, 5.88 mmoL) was additionally added thereto at room temperature, and the resulting mixture was stirred at room temperature for 0.5 hour. Subsequently, the reaction solution was concentrated under reduced pressure. The concentrated residues were fractionated by HPLC (Column: Waters SunFire Prep C18 5 μm ODB 19*150 mm, 0.1% formic acid solution in acetonitrile / water (V/V) = 30/70 → 95/5), and the fraction comprising the target compound was concentrated under reduced pressure. To the concentrated residues was added a 0.1% aqueous solution of formic acid, and the resulting mixture was freeze-dried to give formate of U-012 (3.8 mg, yield: 42.95%) as white solids.
MS (ESI) m/z 820 $(M+H)^+$

Production example 13. U-013

13-1. U-013-1

[C268]

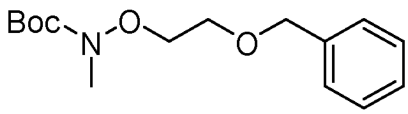

(U-013-1)

To a solution of tert-butyl hydroxy(methyl)carbamate (7.40 g, 50.3 mmoL) and potassium carbonate (13.84 g, 100 mmol) in acetonitrile (50 mL) in a 200 mL round-bottom flask was added ((2-bromoethoxy)methyl)benzene (11.36 g, 52.8 mmoL) under argon atmosphere with stirring at room temperature, and the resulting mixture was stirred at 80°C for 5 hours. After the reaction was completed, the reaction mixture was allowed to cool to room temperature, and concentrated under reduced pressure. To the resulting residues was added a saturated aqueous solution of sodium hydrogen carbonate, and the resulting mixture was subjected to extraction with diisopropyl ether twice. The resulting organic layers were combined, dried over anhydrous magnesium sulfate, filtered, and concentrated under reduced pressure. The resulting residues were subjected to YAMAZEN medium pressure flash chromatography (Silica, L (40 g), hexane / ethyl acetate = 95/5 (V/V) → 80/20 (V/V)), and the fraction comprising the target compound was concentrated under reduced pressure to give U-013-1 (15.11 g, yield: quantitative) as a colorless oil.

13-2. U-013-2

[C269]

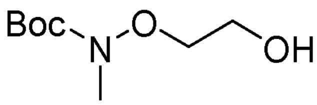

(U-013-2)

A solution of U-013-1 (2.8 g, 9.95 mmoL) and 10% palladium carbon (1.4 g, 0.658 mmoL) in ethanol (50 mL) in a 200 mL round-bottom flask was stirred under hydrogen atmosphere at room temperature for 4 hours. After the reaction was completed, the catalyst was separated by filtration, and the reaction solution was concentrated under reduced pressure to give U-013-2 (1.90 g, yield: 99.84%) as a pale yellow oil.

13-3. U-013-3

[C270]

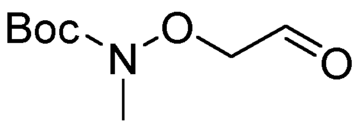

(U-013-3)

To a solution of U-013-2 (0.19 g, 0.994 mmoL) in dichloromethane (5 mL) in a 30 mL cylindrical flask was added Dess-Martin periodinane (0.51 g, 1.202 mmoL) with stirring at room temperature, and the resulting mixture was stirred at room temperature for 1 hour. After the reaction was completed, to the reaction solution was added a saturated aqueous solution of sodium hydrogen carbonate, further added an aqueous solution of sodium thiosulfate, the resulting mixture was

stirred at room temperature for 20 minutes, and then the resulting mixed solution was subjected to extraction with dichloromethane. The resulting organic layer was dried over anhydrous magnesium sulfate, filtered, and concentrated under reduced pressure to give U-013-3 (162.7 mg, yield: 86.54%) as a slightly yellow oil.

13-4. U-013-4

[C271]

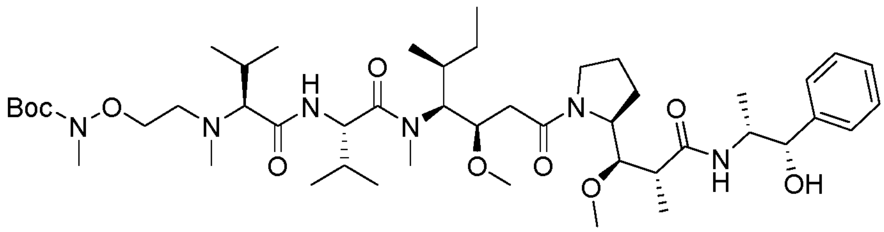

(U-013-4)

To a solution of MMAE (0.30 g, 0.418 mmoL) and U-013-3 (0.16 g, 0.846 mmoL) in dichloromethane (5 mL) in a 30 mL cylindrical flask was added sodium triacetoxyborohydride (0.18 g, 0.849 mmoL) under argon atmosphere with stirring at room temperature, and the resulting mixture was stirred at room temperature for 2 hours. After the reaction was completed, to the reaction solution was added a saturated aqueous solution of sodium hydrogen carbonate, and the resulting mixed solution was subjected to extraction with dichloromethane. The resulting organic layer was dried over anhydrous magnesium sulfate, filtered, and concentrated under reduced pressure to give crude products of U-013-4 as yellow solids. The resulting crude products were subjected to YAMAZEN medium pressure flash chromatography (Silica, M (16 g), ethyl acetate / methanol = 100/0 (V/V) → 95/5 (V/V)), and the fraction comprising the target compound was concentrated under reduced pressure to give U-013-4 (206.5 mg, yield: 55.46%) as colorless foam.
MS (DUIS) m/z 893 $(M+H)^+$

13-5. U-013

[C272]

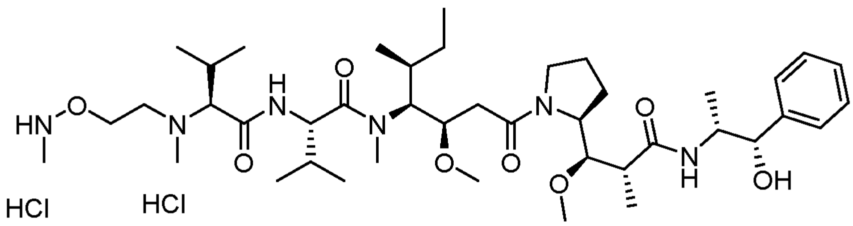

(hydrochloride of U-013)

To a solution of U-013-4 (45.3 mg, 0.051 mmoL) in dichloromethane (0.2 mL) in a 30 mL cylindrical flask was added a 4 M solution of hydrogen chloride in 1,4-dioxane (1 mL, 4 mmoL) under argon atmosphere with stirring at room temperature, and the resulting mixture was stirred at room temperature for 2 hours. After the reaction was completed, the reaction solution was concentrated under reduced pressure, then dissolved in a mixed solvent of water and acetonitrile, and freeze-dried to give hydrochloride of U-013 (44.3 mg, yield: quantitative) as slightly yellow foam.
MS (DUIS) m/z 791 $(M+H)^+$

Production example 14. U-014

14-1. U-014-1

[C273]

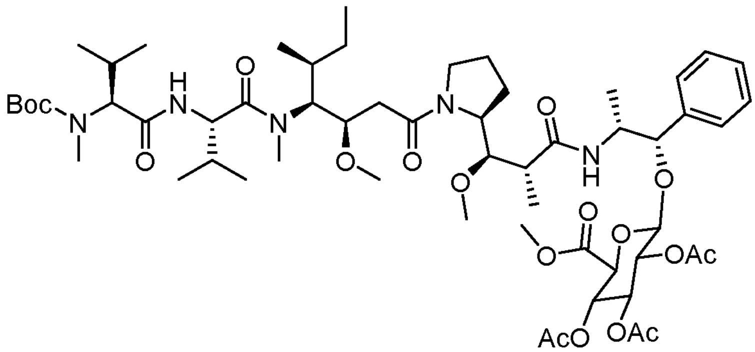

(U-014-1)

To a solution of U-011-1 (102.1 mg, 0.125 mmol) and (2R,3R,4S,5R,6S)-2-bromo-6-(methoxycarbonyl)tetrahydro-2H-pyran-3,4,5-triyl triacetate (463.2 mg, 1.17 mmoL) in dichloromethane (1.8 mL) in a 10 mL cylindrical flask was added 2,4,6-trimethylpyridine (0.255 mL, 233.8 mg, 1.93 mmoL) at room temperature. Under argon atmosphere, silver trifluoromethanesulfonate (387.2 mg, 1.93 mmoL) was added thereto, and then the resulting mixture was stirred at room temperature for 1 hour. After the reaction was completed, the reaction solution was diluted with ethyl acetate, filtered through Celite 545, and washed with ethyl acetate. The resulting filtrate and wash liquid were sequentially washed with a 5% aqueous solution of potassium hydrogen sulfate, a saturated aqueous solution of sodium hydrogen carbonate, and saturated brine, dried over anhydrous magnesium sulfate, filtered, and the resulting filtrate was concentrated under reduced pressure to give concentrated residues. The concentrated residues were purified under the following conditions to give U-014-1 (128 mg, yield: 90.4%) as white foam.
MS (DUIS) m/z 1134 $(M+H)^+$

[Preparative conditions]

Recycle preparative device (YMC Co., Ltd.): LC Forte/R Column (YMC Co., Ltd.): three columns of T-30000 (21.2 mmφ x 600 mm, 50 nm), T-4000 (21.2 mmφ × 600 mm, 10 nm) and T-2000 (21.2 mmφ × 600 mm, 5 nm) were connected and used.

Eluent: ethyl acetate
Temperature: room temperature

14-2. U-014

[C274]

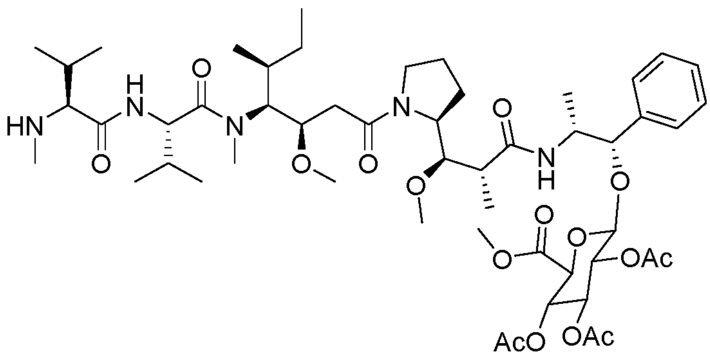

(U-014)

To a solution of U-014-1 (166 mg, 0.146 mmoL) and 2,6-dimethylpyridine (0.090 mL, 83.27 mg, 0.777 mmoL) in dichloromethane (5 mL) in a 100 mL round-bottom flask was added trimethylsilyl trifluoromethanesulfonate (0.135 mL,

166.05 mg, 0.747 mmoL) under argon atmosphere with stirring under ice-cooling, and the resulting mixture was stirred under ice-cooling for 1 hour. After the reaction was completed, to the reaction solution was added a saturated aqueous solution of sodium hydrogen carbonate under ice-cooling, and the resulting solution was separated. The resulting aqueous layer was subjected to extraction with dichloromethane, the resulting organic layers were combined, and dried over anhydrous sodium sulfate to give concentrated residues. The concentrated residues were purified under the following conditions, and the fraction comprising the target compound was concentrated under reduced pressure to give a colorless oil. Acetonitrile (2 mL) and distilled water (5 mL) were added thereto, and then the resulting mixture was freeze-dried to give U-014 (43.3 mg, yield: 28.61%) as a white powder.
MS (DUIS) m/z 1034 $(M+H)^+$

[Preparative conditions]

Recycle preparative device (YMC Co., Ltd.): LC Forte/R Column (YMC Co., Ltd.): three columns of T-30000 (21.2 mmφ × 600 mm, 50 nm), T-4000 (21.2 mmφ × 600 mm, 10 nm) and T-2000 (21.2 mmφ × 600 mm, 5 nm) were connected and used.

Eluent: ethyl acetate
Temperature: room temperature

Production example 15. U-015

15-1. U-015-1

[C275]

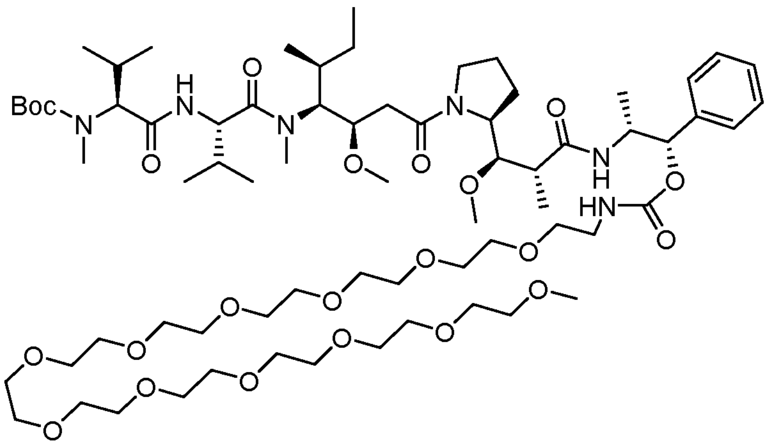

(U-015-1)

To a solution of U-011-2 (101.3 mg, 0.103 mmoL) and 2,5,8,11,14,17,20,23,26,29,32,35-dodecaoxaheptatriacontane-37-amine (98.1 mg, 0.175 mmoL) in dichloromethane (3 mL) in a 30 mL cylindrical flask was added N,N-diisopropylethylamine (0.060 mL, 44.52 mg, 0.344 mmoL), and the resulting mixture was stirred under argon atmosphere at room temperature for 7 hours. After the reaction was completed, the reaction mixture was concentrated under reduced pressure. The resulting residues were separated and purified under the following conditions, the resulting ethyl acetate solution (150 mL) was sequentially washed with water (50 mL) twice and with saturated brine (50 mL) once, dried over anhydrous magnesium sulfate, filtered, and the resulting filtrate was concentrated under reduced pressure to give U-015-1 (91.8 mg, yield: 63.47%) as a colorless oil.

MS (DUIS) m/z 702 $(M+2H)^{2+}$

[Preparative conditions]

Recycle preparative device (YMC Co., Ltd.): LC Forte/R
Column (YMC Co., Ltd.): triconnection of T-30000 (21.2 mmφ × 600 mm, 50 nm), T-4000 (21.2 mmφ × 600 mm, 10 nm), and T-2000 (21.2 mmφ × 600 mm, 5 nm).
Eluent: ethyl acetate
Temperature: room temperature

15-2. U-015

[C276]

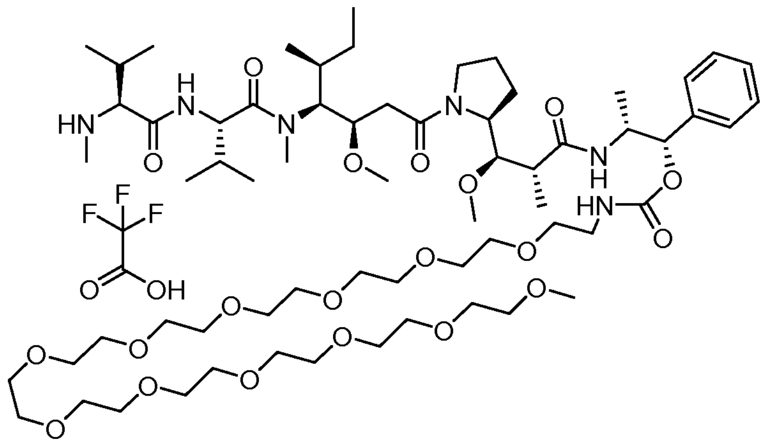

(U-015)

To a solution of U-015-1 (88.2 mg, 0.063 mmoL) in dichloromethane (5 mL) in a 50 mL round-bottom flask was added trifluoroacetic acid (0.48 mL, 714.72 mg, 6.27 mmoL) under ice-cooling, and the resulting mixture was stirred under ice-cooling for 1.25 hours and then stirred at room temperature for 1.5 hours. After the reaction was completed, the reaction solution was concentrated under reduced pressure. To the concentrated residues were added a 0.1% solution of formic acid in acetonitrile / water (= 1/9 (V/V) (5 mL)) solution and a 0.1% solution of formic acid in acetonitrile / water (= 5/5 (V/V) (5 mL)) solution, and the resulting mixture was freeze-dried to give trifluoroacetate of U-015 (96.1 mg, yield: quantitative) as a colorless oil.
MS (ESI) m/z 1304 $(M+H)^+$

Production example 16. U-016

16-1. U-016-1

[C277]

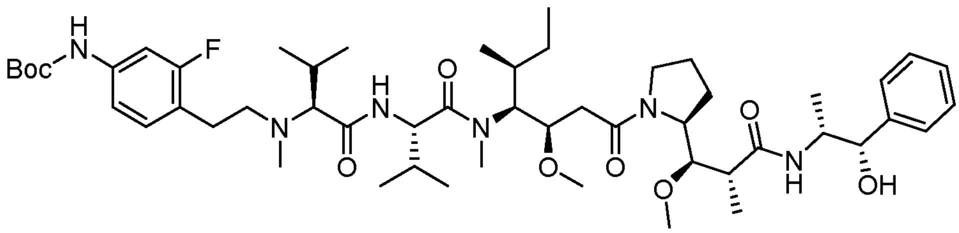

(U-016-1)

To a solution of tert-butyl (3-fluoro-4-(2-oxoethyl)phenyl)carbamate (57.0 mg, 0.225 mmoL) and MMAE (106.3 mg, 0.148 mmoL) in dichloromethane (2.5 mL) in a 50 mL round-bottom flask was added acetic acid (0.015 mL, 15.83 mg, 0.264 mmoL), then sodium triacetoxyborohydride (65.0 mg, 0.307 mmoL) was added thereto under ice-cooling under

argon atmosphere with stirring, and the resulting mixture was stirred at room temperature for 1.5 hours. After the reaction was completed, the reaction solution was diluted with ethyl acetate (5 mL), a saturated aqueous solution of sodium hydrogen carbonate (3 mL) was added thereto, the resulting mixture was stirred at room temperature for a while, and then the resulting solution was separated. The resulting organic layer was washed with saturated brine, dried over anhydrous magnesium sulfate, filtered, and the resulting filtrate was concentrated under reduced pressure. The concentrated residues were dissolved in hexane / ethyl acetate (= 50/50 (V/V)), subjected to Fuji Silysia Chromatorex Q-Pack DIOL-60 Size 10, eluted with hexane / ethyl acetate (= 50/50 (V/V) → 19/81 (V/V)), and the fraction comprising the target compound was concentrated under reduced pressure to give U-016-1 (140.2 mg, yield: 99.13%) as white foam.
MS (ESI) m/z 955 $(M+H)^+$

16-2. U-016

[C278]

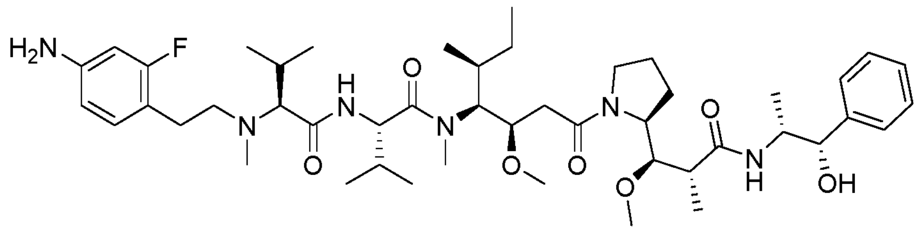

(U-016)

To a solution of U-016-1 (136 mg, 0.142 mmoL) in dichloromethane (3.6 mL) in a 50 mL round-bottom flask was added trifluoroacetic acid (0.545 mL, 811.51 mg, 7.12 mmoL) under argon atmosphere with stirring under ice-cooling, and the resulting mixture was stirred under ice-cooling for 0.5 hour and then stirred at room temperature for 1 hour. After the reaction was completed, the reaction mixture was concentrated under reduced pressure, dichloromethane (5 mL) and a saturated aqueous solution of sodium hydrogen carbonate (5 mL) were added thereto, and the resulting mixture was separated. The resulting organic layer was dried over anhydrous sodium sulfate, filtered, and the resulting filtrate was concentrated under reduced pressure to give concentrated residues. The concentrated residues were separated and purified under the following conditions, and the resulting fraction was concentrated under reduced pressure to give U-016 (93.9 mg, yield: 77.13%) as white foam.
MS (ESI) m/z 855 $(M+H)^+$

Recycle preparative device (YMC Co., Ltd.): LC Forte/R
Column (YMC Co., Ltd.): triconnection of T-30000 (21.2 mmφ × 600 mm, 50 nm), T-4000 (21.2 mmφ × 600 mm, 10 nm), and T-2000 (21.2 mmφ × 600 mm, 5 nm).
Eluent: acetonitrile
Temperature: room temperature

Production example 17. U-017

17-1.U-017-1

[C279]

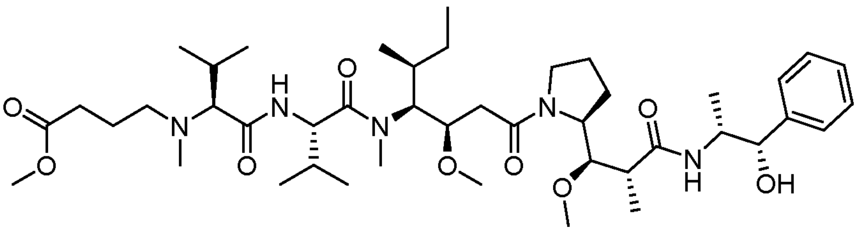

(U-017-1)

To a solution of MMAE (71.8 mg, 0.100 mmoL) and methyl 4-oxobutanoate (34.8 mg, 0.300 mmoL) in dichloromethane (1 mL) in a 30 mL cylindrical flask was added sodium triacetoxyborohydride (63.6 mg, 0.300 mmoL) under argon atmosphere with stirring at room temperature, and the resulting mixture was stirred at room temperature for 1 hour. After the reaction was completed, to the reaction solution was added a saturated aqueous solution of sodium hydrogen carbonate, and the resulting mixed solution was subjected to extraction with ethyl acetate. The resulting organic layer was dried over anhydrous magnesium sulfate, filtered, and concentrated under reduced pressure to give crude products of U-017-1 as yellow solids. The resulting solids were subjected to YAMAZEN medium pressure flash chromatography (Silica, M (16 g), dichloroethane/methanol = 100/0 (V/V) → 90/10 (V/V)), and the fraction comprising the target compound was concentrated under reduced pressure to give U-017-1 (20.2 mg, yield: 24.69%) as colorless foam.

17-2. U-017

[C280]

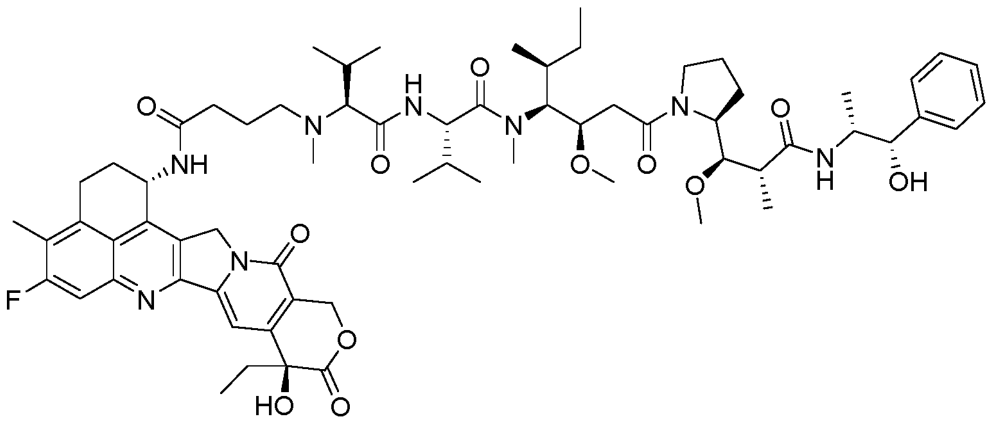

(U-017)

To a solution of U-017-1 (20.2 mg, 0.025 mmoL) in ethanol (0.4 mL) in a 10 mL cylindrical flask was added a 2N aqueous solution of sodium hydroxide (0.050 mL, 4 mg, 0.100 mmoL) under argon atmosphere with stirring at room temperature, and the resulting mixture was stirred at room temperature for 2 hours. 2N hydrochloric acid (0.2 mL) was added thereto to neutralize the mixture, and then the solvent was distilled away under reduced pressure. To the resulting residues was added N,N-dimethylformamide (0.4 mL), further added 1-ethyl-3-(3-dimethylaminopropyl)carbodiimide hydrochloride (7.5 mg, 0.039 mmoL), 1-hydroxybenzotriazole (6.5 mg, 0.042 mmoL), exatecan mesylate (13.1 mg, 0.025 mmoL), and triethylamine (0.01 mL, 7.26 mg, 0.072 mmoL), and the resulting mixture was stirred at room temperature for 14 hours. After the reaction was completed, to the reaction solution was added water, ethyl acetate was added thereto to separate the reaction solution, and then the resulting insoluble matters were separated by filtration. The resulting organic layer was subjected to YAMAZEN medium pressure flash chromatography (Silica, S (7 g), dichloroethane/methanol = 90/10 (V/V) → 80/20 (V/V)), and the fraction comprising the target compound (Rf value = 0.4 (dichloroethane/methanol = 90/10 (V/V))) was concentrated under reduced pressure. The resulting residues were subjected to preparative HPLC under the following conditions, the fraction comprising the target compound was neutralized by a saturated aqueous solution of sodium hydrogen carbonate, and then concentrated under reduced pressure. To the resulting residues was

added water, and then the resulting mixture was subjected to extraction with ethyl acetate twice. The resulting organic layer was dried over anhydrous magnesium sulfate, the solvent was distilled away under reduced pressure, then the resulting residues were dissolved in water/acetonitrile, and freeze-dried to give U-017 (3.8 mg, yield: 12.6%) as colorless solids. MS (DUIS) m/z 1222 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 50% (10.00 min.)

Production example 18. U-018

18-1. U-018-1

[C281]

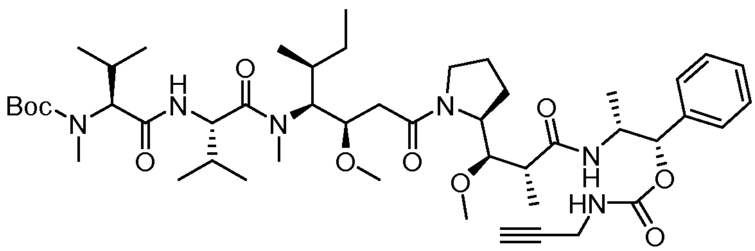

(U-018-1)

To a solution of U-011-2 (61.5 mg, 0.063 mmoL) in dichloromethane (0.4 mL) in a 20 mL cylindrical flask was added prop-2-yne-1-amine (0.008 mL, 6.88 mg, 0.125 mmoL) under argon gas flow with stirring at room temperature, and the resulting mixture was stirred at room temperature for 3 hours. The resulting residues were subjected to YAMAZEN medium pressure flash chromatography (Rf value = 0.25 (hexane / ethyl acetate = 20/80 (V/V)) (Silica, M (16 g))), and the fraction comprising the target compound was concentrated under reduced pressure to give U-018-1 (57.6 mg, yield: quantitative) as white solids.
MS (ESI) m/z 900 $(M+H)^+$

18-2. U-018-2

[C282]

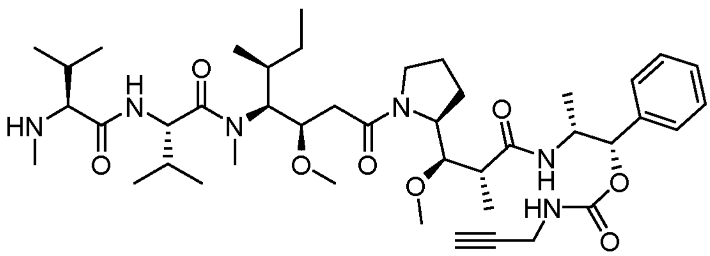

(U-018-2)

To a solution of U-018-1 (67.6 mg, 0.075 mmoL) in dichloromethane (0.2 mL) in a 20 mL cylindrical flask was added trifluoroacetic acid (0.064 mL, 95.36 mg, 0.836 mmoL) under argon gas flow with stirring at room temperature, and the resulting mixture was stirred at room temperature for 3 hours. After the reaction was completed, the reaction mixture was concentrated under reduced pressure, the resulting residues were subjected to preparative HPLC under the following conditions, and the fraction comprising the target compound was concentrated under reduced pressure to give U-018-2

(17.2 mg, yield: 28.63%) as white solids.
MS (ESI) m/z 799 $(M+H)^+$

Column: Waters SunFire Prep C18 5 μm ODB 19*150 mm
Flow rate: 17 mL/min.
Elution solvent: 0.1% aqueous solution of formic acid (Solution A) → 0.1% solution of formic acid in acetonitrile (Solution B)
Gradient (Solution B): 20% (0.00 min.) → 95% (10.00 min.)

18-3. U-018

[C283]

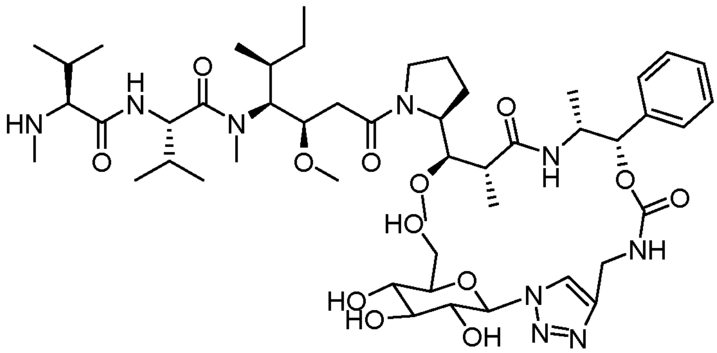

(U-018)

To a solution of U-018-2 (8.8 mg, 0.011 mmoL) in water (0.1 mL) in a 20 mL cylindrical flask were added (2R,3R,4S,5S,6R)-2-azide-6-(hydroxymethyl)tetrahydro-2H-pyran-3,4,5-triol (3.8 mg, 3.09 μmoL), copper(II) sulfate pentahydrate (3.5 mg, 0.022 mmoL), and sodium ascorbate (7.1 mg, 0.033 mmoL) under argon gas flow with stirring at room temperature, and the resulting mixture was stirred at room temperature for 5 minutes. The resulting residues were subjected to preparative HPLC under the following conditions, and the fraction comprising the target compound was freeze-dried to give U-018 (3.1 mg, yield: 28.03%) as white solids.
MS (DUIS) m/z 1046 $(M+HCOO)^-$

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 20% (0.00 min.) → 61% (6.00 min.)

Production example 19. U-019

[C284]

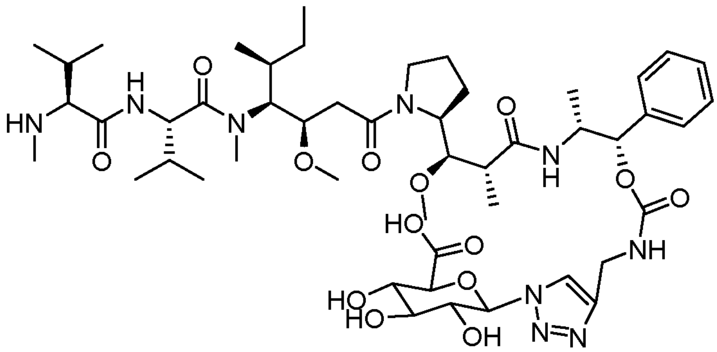

(U-019)

To a solution of the Production example 18-1 (U-018-1) (8.2 mg, 10.26 μmoL) in water (0.2 mL) in a 10 mL cylindrical flask were added (2R,3R,4S,5S,6R)-6-azide-3,4,5-trihydroxytetrahydro-2H-pyran-2-carboxylic acid (2.8 mg, 0.013 mmoL), copper(II) sulfate pentahydrate (2.0 mg, 0.013 mmoL), and sodium ascorbate (3.5 mg, 0.016 mmoL) under argon gas flow with stirring at room temperature, and the resulting mixture was stirred at room temperature for 5 minutes. The resulting residues were subjected to preparative HPLC under the following conditions, and the fraction comprising the target compound was freeze-dried to give U-019 (5.4 mg, yield: 51.68%) as white solids.
MS (ESI) m/z 1016 $(M-H)^-$

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 20% (0.00 min.) → 61% (7.00 min.)

Production example 20. U-020

20-1. U-020-1

[C285]

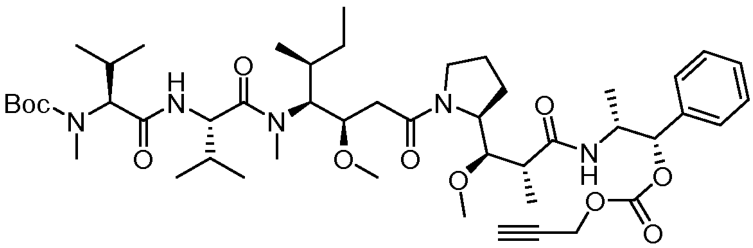

(U-020-1)

To a solution of U-011-2 (50.0 mg, 0.051 mmoL) in dichloromethane (0.2 mL) in a 20 mL cylindrical flask was added prop-2-yn-1-ol (0.00587 mL, 5.71 mg, 0.102 mmoL) under argon gas flow with stirring at room temperature, and the resulting mixture was stirred at room temperature for 45 minutes. Subsequently, 4-dimethylaminopyridine (0.8 mg, 6.55 μmoL) was added thereto under stirring at room temperature, and the resulting mixture was stirred at room temperature for 2.5 hours. Subsequently, prop-2-yn-1-ol (0.00587 mL, 5.71 mg, 0.102 mmoL) was additionally added thereto, and the resulting mixture was stirred for 14 hours. The resulting residues were subjected to YAMAZEN medium pressure flash chromatography (Rf value = 0.3 (hexane / ethyl acetate = 20/80 (V/V) (Silica, M (16 g)))), and the fraction comprising the target compound was concentrated under reduced pressure to give U-020-1 (37.8 mg, yield: 82.57%) as white solids.

MS (ESI) m/z 900 (M+H)+

20-2. U-020-2

[C286]

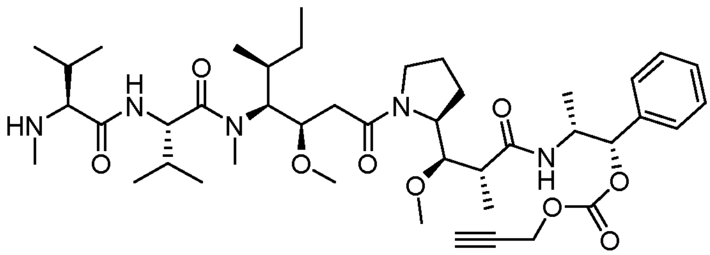

(U-020-2)

To a solution of U-020-1 (36.8 mg, 0.041 mmoL) in dichloromethane (0.2 mL) in a 20 mL cylindrical flask was added trifluoroacetic acid (0.069 mL, 102.81 mg, 0.902 mmoL) under argon gas flow with stirring at room temperature, and the resulting mixture was stirred at room temperature for 2.5 hours. After the reaction was completed, the reaction mixture was concentrated under reduced pressure, the resulting residues were subjected to preparative HPLC under the following conditions, the fraction comprising the target compound was concentrated under reduced pressure to distill away acetonitrile, and then the resulting residues were freeze-dried to give U-020-2 (18.2 mg, yield: 55.65%) as white solids. MS (ESI) m/z 800 (M+H)+

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 20% (0.00 min.) → 75% (6.50 min.)

20-3. U-020

[C287]

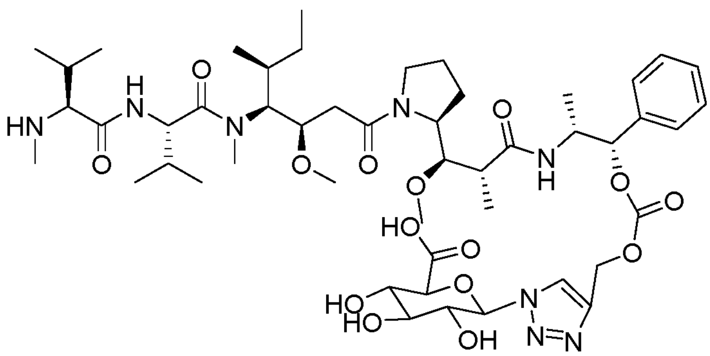

(U-020)

To a solution of U-020-2 (5.8 mg, 7.25 μmoL) in water (0.3 mL) in a 20 mL cylindrical flask were added (2R,3R,4S,5S,6R)-6-azide-3,4,5-trihydroxytetrahydro-2H-pyran-2-carboxylic acid (2.3 mg, 10.49 μmoL), copper(II) sulfate pentahydrate (0.6 mg, 2.403 μmoL), and sodium ascorbate (0.8 mg, 3.77 μmoL) under argon gas flow with stirring at room temperature, and the resulting mixture was stirred at room temperature overnight. The resulting residues were subjected to preparative HPLC under the following conditions, and the fraction comprising the target compound was freeze-dried to give U-020 (6.3 mg, yield: 85.26%) as white solids.

MS (ESI) m/z 1020 (M+H)+

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 20% (0.00 min.) → 61% (6.00 min.)

Production example 21. U-021

21-1. U-021-1

[C288]

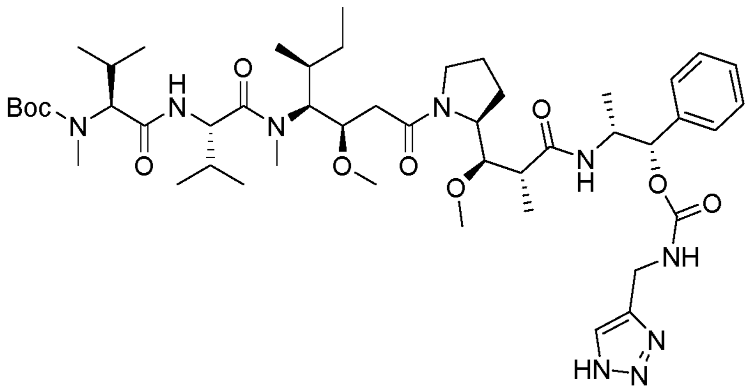

(U-021-1)

To a solution of U-011-2 (25 mg, 0.025 mmoL) in dichloromethane (2 mL) in a 20 mL cylindrical flask were added (1H-1,2,3-triazol-4-yl)methaneamine hydrochloride (7.0 mg, 0.052 mmoL) and triethylamine (0.00354 mL, 2.57 mg, 0.025 mmoL) under argon gas flow with stirring at room temperature, and the resulting mixture was stirred at room temperature for 1 hour. Subsequently, N,N-dimethylformamide (1 mL) was added thereto, and the resulting mixture was stirred for 1 hour. After the reaction was completed, to the reaction solution was added water, and the resulting mixed solution was subjected to extraction with ethyl acetate. The resulting organic layer was washed with saturated brine, dried over anhydrous sodium sulfate, filtered, and concentrated under reduced pressure to give crude products of U-021-1. The crude products were used in the next step without further purification.
MS (ESI) m/z 942 (M+H)+

21-2. U-021

[C289]

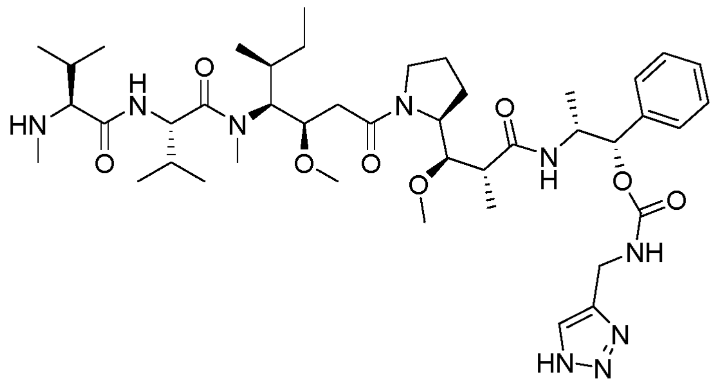

(U-021)

To a solution of the crude products of U-021-1 (284 mg (content: 8.5%)) in dichloromethane (0.5 mL) in a 20 mL cylindrical flask was added trifluoroacetic acid (0.10 mL, 149 mg, 1.307 mmoL) under argon gas flow with stirring at room temperature, and the resulting mixture was stirred at room temperature for 1.5 hours. After the reaction was completed, the reaction mixture was concentrated under reduced pressure. The resulting residues were subjected to preparative HPLC under the following conditions, the fraction comprising the target compound was concentrated under reduced pressure to distill away acetonitrile, and then the resulting residues were freeze-dried to give trifluoroacetate of U-021 (8.8 mg) as white solids.

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 5% (0.00 min.) → 95% (10.50 min.)

The resulting solids were dissolved in dichloromethane, washed with an aqueous solution of sodium hydrogen carbonate and saturated brine, and dried over anhydrous sodium sulfate. The resulting residues were concentrated under reduced pressure to give U-021 (7.0 mg, total yield of 21-1 and 21-2: 32.63%) as white solids.
MS (ESI) m/z 840 $(M-H)^-$

Production example 22. U-022

22-1. U-022-1

[C290]

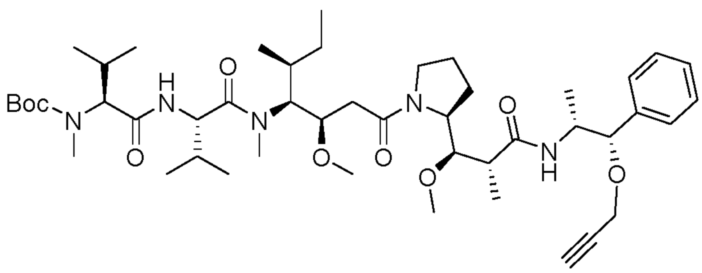

(U-022-1)

To a solution of U-011-1 (400 mg, 0.489 mmoL) in toluene (6 mL) in a 50 mL round-bottom flask were added 3-bromoprop-1-yne (0.11 mL, 173.8 mg, 1.461 mmoL), tetrabutylammonium bromide (33.2 mg, 0.103 mmoL), and 8N sodium hydroxide (0.061 mL, 19.52 mg, 0.488 mmoL) under argon gas flow with stirring at room temperature, and the resulting mixture was stirred at room temperature for 6 hours. After the reaction was completed, to the reaction solution

was added a saturated aqueous solution of ammonium chloride, and the resulting mixed solution was subjected to extraction with dichloromethane. The resulting residues were subjected to preparative HPLC under the following conditions, and the fraction comprising the target compound was concentrated under reduced pressure to give U-022-1 (142.1 mg, yield: 33.95%) as white solids.
MS (ESI) m/z 857 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 100% (10.00 min.)

22-2. U-022-2

[C291]

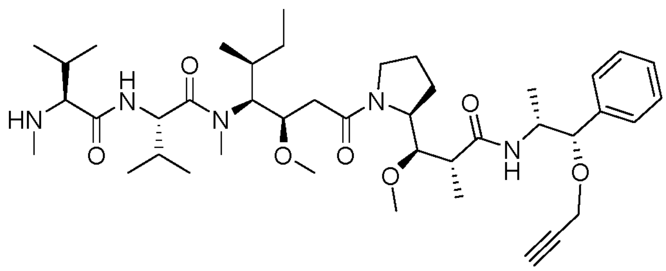

(U-022-2)

To a solution of U-022-1 (142.1 mg, 0.166 mmoL) in dichloromethane (0.4 mL) in a 20 mL cylindrical flask was added trifluoroacetic acid (0.254 mL, 378 mg, 3.32 mmoL) under argon gas flow with stirring at room temperature, and the resulting mixture was stirred at room temperature for 20 hours. After the reaction was completed, the reaction mixture was neutralized by triethylamine, and concentrated under reduced pressure. The resulting residues were subjected to preparative HPLC under the following conditions, the fraction comprising the target compound was concentrated under reduced pressure to distill away acetonitrile, and then the resulting residues were freeze-dried to give U-022-2 (69.6 mg, yield: 55.47%) as white solids.
MS (DUIS) m/z 757 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 0% (0.00 min.) → 50% (10.00 min.)

22-3. U-022

[C292]

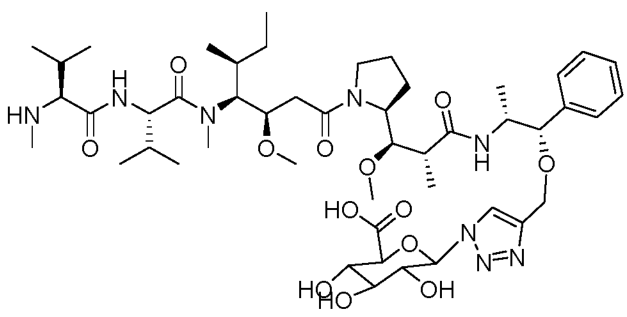

(U-022)

To a solution of U-022-2 (30 mg, 0.040 mmoL) in water (1 mL) in a 30 mL cylindrical flask were added (2S,3S,4S,5R,6R)-6-azide-3,4,5-trihydroxytetrahydro-2H-pyran-2-carboxylic acid (11.2 mg, 0.051 mmoL), copper sulfate pentahydrate (3.1 mg, 0.012 mmoL), and sodium ascorbate (12.7 mg, 0.060 mmoL) under argon gas flow with stirring at room temperature, and the resulting mixture was stirred at room temperature for 15 hours. The resulting residues were subjected to the following conditions, and the fraction comprising the target compound was concentrated under reduced pressure to give crude products of U-022 as yellow solids.

Column: Waters XSelect HSS C18 SB OBD 5 μm ODB 19*150 mm
Flow rate: 17 mL/min.
Elution solvent:

0.1% aqueous solution of formic acid / 0.1% solution of formic acid in acetonitrile = 90/10 (Solution A)

0.1% aqueous solution of formic acid / 0.1% solution of formic acid in acetonitrile = 10/90 (Solution B)

Gradient (Solution B): 0% (0.00 min.) → 0% (5.00 min.) → 50% (5.10 min.) → 50% (20.00 min.)

The resulting crude products of U-022 were purified under the following conditions, and the resulting residues were freeze-dried to give U-022 (18.8 mg, yield: 37.72%) as white solids.
MS (ESI) m/z 976 $(M+H)^+$

Column: Asahipak GS-510-20G, GS-310-20G, and GS-310-20G (triconnection) (shodex) 20*500 mm, 13 μm
Flow rate: 7.5 mL/min.
Elution solvent: 0.1% formic acid / 0.1% formic acid in acetonitrile = 90/10
Detection wavelength: 254 nm

Production example 23. U-023

23-1. U-023-1

[C293]

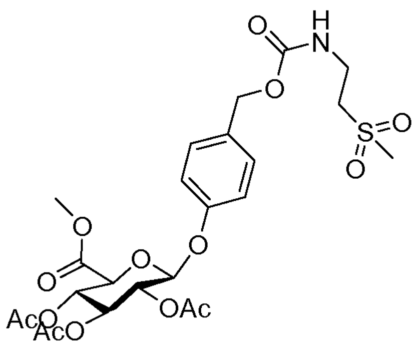

(U-023-1)

To a solution of (2S,3S,4S,5R,6S)-2-(methoxycarbonyl)-6-(4-((((4-nitrophenoxy)carbonyl)oxy)methyl)phenoxy) tetrahydro-2H-pyran-3,4,5-tolyl triacetate (0.7982 g, 1.318 mmoL) in dichloromethane (8 mL) in a 50 mL round-bottom flask were sequentially added N,N-diisopropylethylamine (1.20 mL, 0.86 g, 6.89 mmoL) and 2-(methylsulfonyl)ethan-1-amine hydrochloride (0.2105 g, 1.319 mmoL) under argon atmosphere with stirring at room temperature, and the resulting mixture was stirred at room temperature for 72 hours. After the reaction was completed, the reaction solution was concentrated under reduced pressure, the residues were diluted with ethyl acetate (30 mL), washed with a saturated aqueous solution of sodium hydrogen carbonate (10 mL) five times and then with saturated brine (20 mL), dried over anhydrous magnesium sulfate, filtered, and the resulting filtrate was concentrated under reduced pressure. The resulting residues were subjected to YAMAZEN medium pressure flash chromatography (Silica, L (40 g), hexane / ethyl acetate =

10/90 (V/V), (Rf = 0.51 (hexane / ethyl acetate = 10/90 (V/V)))), and the fraction comprising the target compound was concentrated under reduced pressure to give U-023-1 (677.8 mg, yield: 87.21%) as white foam.
MS (ESI) m/z 635 (M+HCOOH)+

23-2. U-023-2

[C294]

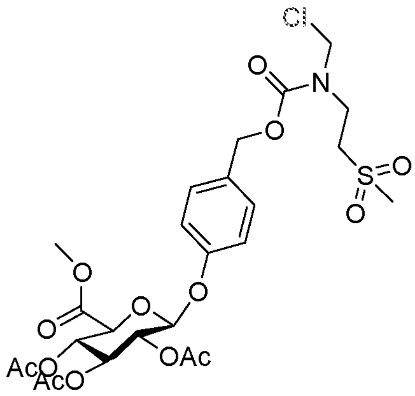

(U-023-2)

To a solution of U-023-1 (100.3 mg, 0.170 mmoL) in dichloromethane (1.5 mL) in a 20 mL cylindrical flask were sequentially added paraformaldehyde (9.30 mg, 0.310 mmoL) and trimethylsilyl chloride (33 μL, 28.25 mg, 0.260 mmoL) under argon atmosphere with stirring at room temperature, and the resulting mixture was stirred at room temperature for 5 hours. The reaction solution was filtered, washed with dichloromethane, and the resulting filtrate was concentrated under reduced pressure at room temperature to give U-023-2 (107.1 mg, yield: 98.67%) as white foam.
MS (ESI) m/z 651 $(M+H_2O)^+$

23-3. U-023-3

[C295]

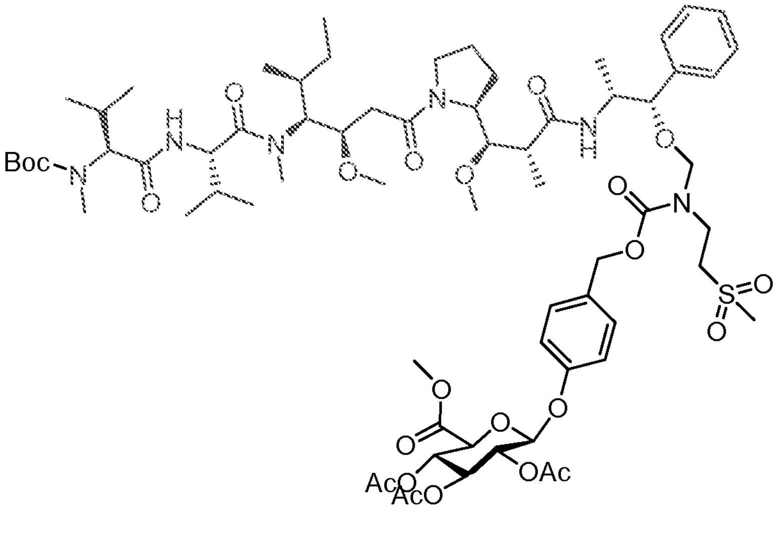

(U-023-3)

To a solution of U-011-1 (100.8 mg, 0.123 mmoL) in dichloromethane (0.5 mL) in a 20 mL cylindrical flask were

sequentially added N,N-diisopropylethylamine (150 μL, 111.3 mg, 0.861 mmoL) and a solution of U-023-2 (108.55 mg, 0.170 mmoL) in dichloromethane (1 mL) under argon atmosphere with stirring at room temperature, and the resulting mixture was stirred at room temperature for 22.5 hours. Subsequently, 2,4,6-trimethylpyridine (110 μL, 101.2 mg, 0.835 mmoL) was added thereto at room temperature, then a solution of U-023-2 (171.3 mg, 0.268 mmoL) in dichloromethane (2 mL) was added thereto, the resulting mixture was stirred at room temperature for 21 hours, and then to the reaction solution was sprayed nitrogen to distill away the solvent. Dichloromethane (0.75 mL) was added thereto, then N,N-diisopropylethylamine (250 μL, 185.5 mg, 1.435 mmoL) was added thereto, and the resulting mixture was stirred at room temperature for 2 hours. Subsequently, the mixture was ice-cooled, and a solution of U-023-2 (446.7 mg, 0.700 mmoL) in dichloromethane (3 mL) was added dropwise thereto. After the addition was completed, the resulting mixture was warmed to room temperature, and stirred for 18 hours. The reaction solution was concentrated, diluted with ethyl acetate, sequentially washed with a 5% by weight of aqueous solution of potassium hydrogen sulfate, a saturated aqueous solution of sodium hydrogen carbonate, and saturated brine, dried over anhydrous magnesium sulfate, filtered, and the resulting filtrate was concentrated under reduced pressure. The resulting residues were subjected to YAMAZEN medium pressure flash chromatography (Fuji Silysia Chromatorex Q-Pack DIOL-60 Size 60, hexane / ethyl acetate = 30/70 (V/V) (Rf = 0.30 (hexane / ethyl acetate = 30/70 (V/V)))), and the fraction comprising the target compound was concentrated under reduced pressure to give crude products of U-023-3 as white foam.

The resulting white foam was purified under the following conditions to give U-023-3 (46.0 mg, yield: 26.3%) as white solids.
MS (ESI) m/z 1420 (M+H)+

Column: YMC-GPC T-30000, T-4000, and T-2000 (triconnection) 21.2*600 mm, 10 μm
Flow rate: 15 mL/min.
Elution solvent: ethyl acetate
Temperature: room temperature
Detection wavelength: 254, 285 nm

23-4. U-023-4

[C296]

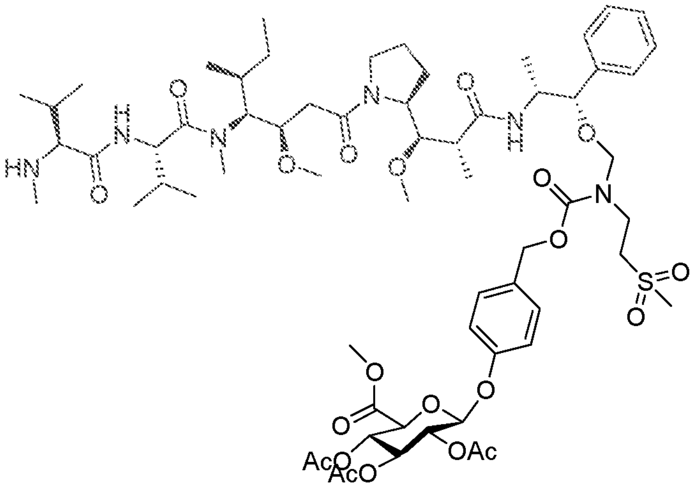

(U-023-4)

To a solution of U-023-3 (43.2 mg, 0.030 mmoL) and 2,6-dimethylpyridine (160 μL, 143 mg, 1.381 mmoL) in dichloromethane (20 mL) in a 100 mL round-bottom flask was added trimethylsilyl trifluoromethanesulfonate (240 μL, 295.2 mg, 1.328 mmoL) under argon atmosphere with stirring under ice-cooling, and the resulting mixture was stirred under ice-cooling for 20 hours. Subsequently, to the reaction solution was added a saturated aqueous solution of sodium hydrogen carbonate (20 mL) under ice-cooling, the resulting mixture was stirred at room temperature for a while, and then dichloromethane was added thereto to be subjected to extraction. The resulting aqueous layer was subjected to extraction with dichloromethane, the resulting organic layers were combined, dried over anhydrous sodium sulfate, and concentrated. The resulting residues were purified under the following conditions, and the fraction comprising the target

compound was concentrated under reduced pressure to give white foam. Acetonitrile (2 mL) and distilled water (4 mL) were added thereto, and then the resulting mixture was freeze-dried to give U-023-4 (18.3 mg, yield: 45.58%) as a white powder.
MS (ESI) m/z 1320 $(M+H)^+$

Column: Asahipak GS-510-20G, GS-310-20G, and GS-310-20G (triconnection) (shodex) 20*500 mm, 13 μm
Flow rate: 17 mL/min.
Elution solvent: acetonitrile
Detection wavelength: 220 nm

23-5. U-023

[C297]

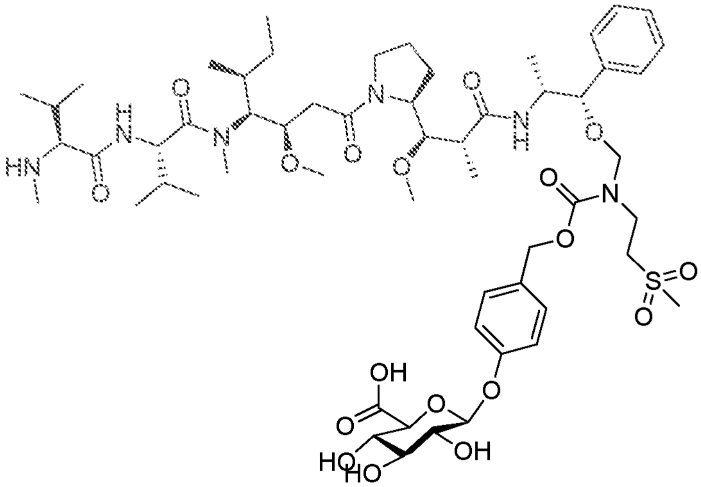

(U-023)

To a solution of U-023-4 (17.1 mg, 0.013 mmoL) in methanol (4 mL) in a 20 mL pear-shaped flask was added 1N lithium hydroxide (52 μL, 1.25 mg, 0.052 mmoL) under air atmosphere under ice-cooling with stirring, then the resulting mixture was warmed to room temperature, and stirred for 9.5 hours. Subsequently, a 50 mM aqueous solution of ammonium formate (4 mL, 12.61 mg, 0.200 mmoL) was added thereto. The resulting mixture was purified under the following conditions, and the fraction comprising the target compound was concentrated under reduced pressure. To the concentrated residues was added acetonitrile (15 mL), and then the resulting mixture was freeze-dried to give U-023 (13.10 mg, yield: 85.71%) as white solids.
MS (ESI) m/z 1180 $(M+H)^+$

Column: XSelect CSH, Prep Fluoro-Phenyl (Waters Corporation) 5 μm 19*150 mm
Flow rate: 17 mL/min.
Elution solvent: 10% aqueous solution of methanol (Solution A) - 90% aqueous solution of methanol (Solution B)
Gradient (Solution B): 50% (0.00 min.) → 50% (3.00 min.)
→ 62% (9.50 min.)
Detection wavelength: 220 nm

Production example 24.

[C298]

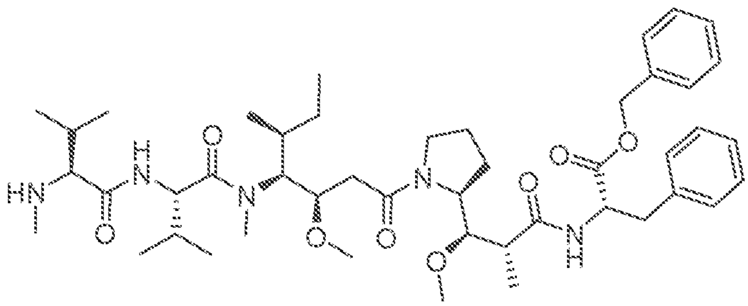

(U-024)

To a solution of MMAF (0.22 g, 0.301 mmoL) in dichloromethane (1 mL) in a 30 mL cylindrical flask was added methanesulfonic acid (58 μL, 0.09 g, 0.893 mmoL) under argon gas flow with stirring at room temperature, and the resulting mixture was stirred at 60°C for 4 hours. After the reaction was completed, to the reaction solution was added a saturated aqueous solution of sodium hydrogen carbonate, and the resulting mixed solution was subjected to extraction with ethyl acetate. The resulting organic layer was washed with water, dried over anhydrous magnesium sulfate, filtered, and concentrated under reduced pressure. The resulting residues were subjected to YAMAZEN medium pressure flash chromatography (Silica, M (16 g), ethyl acetate / methanol = 100/0 (V/V) → 60/40 (V/V)), and the fraction comprising the target compound was concentrated under reduced pressure to give U-024 (183.9 mg, yield: 74.61%) as colorless foam.
MS (ESI) m/z 821 $(M+H)^+$

Production example 25. U-025

25-1. U-025-1

[C299]

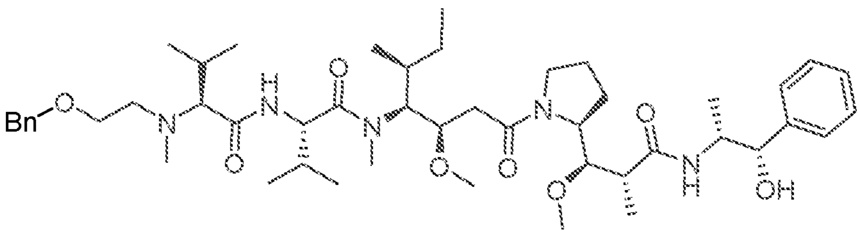

(U-025-1)

To a solution of MMAE (43.5 mg, 0.061 mmoL) in dichloromethane (1 mL) in a 5 mL sample bottle were sequentially added 2-(benzyloxy)acetoaldehyde (10.3 μL, 11.01 mg, 0.067 mmoL), acetic acid (5.2 μL, 5.49 mg, 0.091 mmol), and sodium triacetoxyborohydride (25.1 mg, 0.118 mmoL) with stirring at room temperature, and then the resulting mixture was stirred at room temperature for 1.5 hours. After the reaction was completed, a saturated aqueous solution of sodium hydrogen carbonate (1.5 mL) was added dropwise thereto with stirring at room temperature, and the resulting mixture was stirred at room temperature for 30 minutes. The resulting organic layers were separated, then the resulting aqueous layer was subjected to extraction with dichloromethane, the resulting organic layers were combined, dried over anhydrous sodium sulfate, and filtered. The resulting filtrate was concentrated, the resulting residues were subjected to YAMAZEN medium pressure flash chromatography (Silica, S (7 g), dichloromethane/2-propanol = 95/5 (V/V)) (Rf = 0.47 (dichloromethane/2-propanol = 95/5 (V/V))), and the fraction comprising the target compound was concentrated under reduced pressure to give U-025-1 (43.8 mg, yield: 84.84%) as white foam.
MS (ESI) m/z 853 $(M+H)^+$

25-2. Synthesis of U-025

[C300]

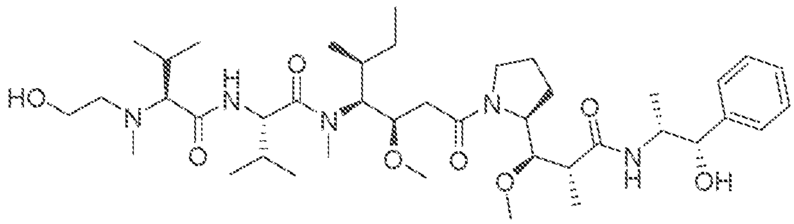

(U-025)

To a solution of U-025-1 (43.8 mg, 0.051 mmoL) in ethanol (2 mL) in a 50 mL pear-shaped flask was added 5% palladium carbon (ASCA-2 type, wetted with 50% water) (10.94 mg, 0.273 mg, 2.57 μmoL) under nitrogen atmosphere, the resulting mixture was degassed under reduced pressure, and then stirred under hydrogen atmosphere at room temperature for 1 hour. Subsequently, acetic acid (0.2 mL) was added thereto, and the resulting mixture was stirred at room temperature for 1 hour. Subsequently, the mixture was stirred with heating at a bath temperature of 60°C for 4.5 hours. After the reaction was completed, the reaction solution was allowed to cool, filtered using Celite 545 (trade name), washed with ethanol, and the resulting filtrate was concentrated under reduced pressure. The concentrated residues were diluted with dichloromethane, washed with a saturated aqueous solution of sodium hydrogen carbonate, dried over anhydrous sodium sulfate, filtered, and the resulting filtrate was concentrated under reduced pressure. To the concentrated residues were added acetonitrile (2 mL) and distilled water (3 mL), and the resulting mixture was freeze-dried to give U-025 (34.6 mg, yield: 88.34%) as white solids.
MS (ESI) m/z 763 $(M+H)^+$

Production example 26. U-026

[C301]

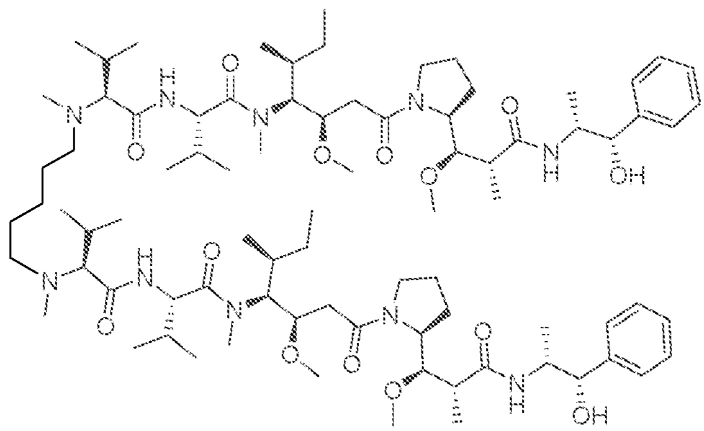

(U-026)

To a solution of MMAE (63.2 mg, 0.088 mmoL) in dichloromethane (1.5 mL) in a 5 mL sample bottle were sequentially added a 5.6 M aqueous solution of glutaraldehyde (8.00 μL, 4.49 mg, 0.045 mmoL), acetic acid (8.00 μL, 8.44 mg, 0.141 mmoL), and sodium triacetoxyborohydride (39.6 mg, 0.187 mmoL) with stirring at room temperature, and then the resulting mixture was stirred at room temperature for 2.5 hours. After the reaction was completed, a saturated aqueous solution of sodium hydrogen carbonate (2 mL) was added dropwise thereto with stirring at room temperature, and after the addition was completed, the resulting mixture was stirred at room temperature for 30 minutes. The resulting organic layers were separated, then the resulting aqueous layer was subjected to extraction with dichloromethane, the resulting dichloromethane layers were combined, dried over anhydrous sodium sulfate, filtered, and the resulting filtrate was concentrated under reduced pressure. The concentrated residues were purified under the following conditions, the fraction comprising the target compound was concentrated under reduced pressure, dissolved in dichloromethane, diethyl ether was added thereto, the resulting mixture was subjected to sonication, the resulting solids were collected by filtration under reduced pressure, washed with diethyl ether, and dried under reduced pressure to give U-026 (36.32 mg, yield:

53.9%) as white solids.
MS (ESI) m/z 753 $(M+2H)^{2+}$

Column: YMC-GPC T-30000, T-4000, and T-2000 (triconnection) 21.2*600 mm, 10 μm
Flow rate: 15 mL/min.
Elution solvent: ethyl acetate
Temperature: room temperature
Detection wavelength: 260 nm

Production example 27. U-027

27-1. U-027-1

[C302]

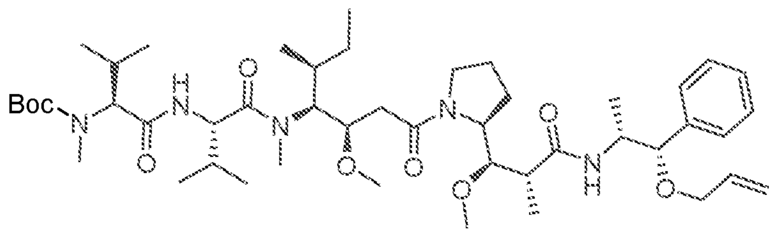

(U-027-1)

To a solution of U-011-1 (102.1 mg, 0.125 mmoL) in toluene (1.3 mL) in a 30 mL pear-shaped flask were added tetrabutylammonium hydrogen sulfate (2.16 mg, 6.36 μmoL) and 3-bromoprop-1-ene (16 μL, 22.37 mg, 0.185 mmoL) under air atmosphere with stirring at room temperature. Subsequently, a 50% by weight of aqueous solution of sodium hydroxide (230 μL, 345 mg, 4.31 mmoL) was added thereto at room temperature, and then the resulting mixture was stirred at room temperature for 5 hours. After the reaction was completed, to the reaction solution were added toluene (2 mL) and water (3 mL), the resulting solution was stirred, and then separated. The resulting organic layer was washed with water (2 mL). The resulting organic layer was diluted with ethyl acetate, sequentially washed with a 5% aqueous solution of potassium hydrogen sulfate, a saturated aqueous solution of sodium hydrogen carbonate, and saturated brine, dried over anhydrous magnesium sulfate, filtered, and the resulting filtrate was concentrated under reduced pressure to give white foam. To the resulting residues was added dichloromethane to dissolve the residues, the resulting solution was subjected to YAMAZEN medium pressure flash chromatography (Chromatorex_COOH_(LotNo. HU20999) MB100-40/75 (11.0 g), dichloromethane/2-propanol = 100/0 (V/V) → 90/10 (V/V), (Rf = 0.90 (dichloromethane/2-propanol = 80/20 (V/V)))), and the fraction comprising the target compound was concentrated under reduced pressure to give white foam. Acetonitrile (2 mL) and ultrapure water (2 mL) were added thereto, and the resulting mixture was freeze-dried to give U-027-1 (58.3 mg, yield: 54.44%) as white solids.
MS (ESI) m/z 859 $(M+H)^+$

27-2. U-027-2

[C303]

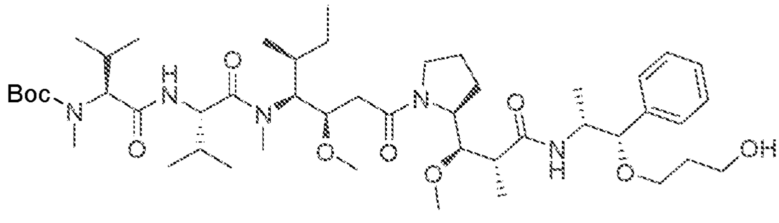

(U-027-2)

To a solution of U-027-1 (54.6 mg, 0.064 mmoL) in tetrahydrofuran (1 mL) in a 20 mL pear-shaped flask was added a 0.5 M solution of 9-BBN in tetrahydrofuran (640 μL, 39.05 mg, 0.320 mmoL) under argon atmosphere with stirring at room temperature, and the resulting mixture was stirred at room temperature for 2.33 hours. Subsequently, a 0.5 M solution of 9-BBN in tetrahydrofuran (360 μL, 21.96 mg, 0.180 mmoL) was additionally added thereto at room temperature, and the resulting mixture was stirred at room temperature for 2.33 hours. Subsequently, the mixture was ice-cooled, methanol (1 mL) was added thereto, then the resulting mixture was warmed to room temperature, stirred at room temperature for 1 hour, and concentrated under reduced pressure. To the concentrated residues were sequentially added tetrahydrofuran (1 mL), tetrahydrofuran (1 mL), and 1N sodium hydroxide (1.0 mL, 40 mg, 1.00 mmoL), then the resulting mixture was ice-cooled, a 30% by weight of aqueous solution of hydrogen peroxide (110 μL, 122.1 mg, 1.077 mmoL) was added thereto, and the resulting mixture was stirred under ice-cooling and then stirred at room temperature for 1 hour. Subsequently, water (5 mL) and ethyl acetate (5 mL) were added thereto to separate the mixture. The resulting organic layer was sequentially washed with a 5% by weight of aqueous solution of potassium hydrogen sulfate, a saturated aqueous solution of sodium hydrogen carbonate, and saturated brine, dried over anhydrous magnesium sulfate, filtered, and the resulting filtrate was concentrated under reduced pressure to give a colorless oil. To the resulting residues was added dichloromethane to dissolve the residues, the resulting solution was subjected to YAMAZEN medium pressure flash chromatography (Chromatorex_COOH_(LotNo. HU20999) MB100-40/75 (11.0 g), dichloromethane/2-propanol = 100/0 (V/V) → 90/10 (V/V)), and the fraction comprising the target compound was concentrated under reduced pressure to give a colorless oil. Acetonitrile (1 mL) and ultrapure water (1 mL) were added thereto, and the resulting mixture was freeze-dried to give U-027-2 (39.2 mg, yield: 70.32%) as white solids.
MS (ESI) m/z 877 $(M+H)^+$

27-2. U-027

[C304]

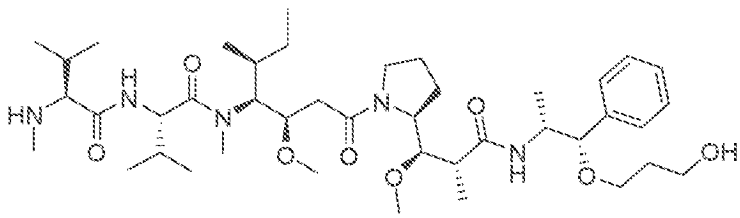

(U-027)

To a solution of U-027-2 (35.6 mg, 0.041 mmoL) in dichloromethane (3 mL) in a 30 mL pear-shaped flask were added 2,6-dimethylpyridine (99 μL, 91.6 mg, 0.856 mmoL) and trimethylsilyl trifluoromethanesulfonate (139 μL, 171 mg, 0.771 mmoL) under argon atmosphere under ice-cooling with stirring, and the resulting mixture was stirred under ice-cooling for 6 hours. To the reaction solution were added dichloromethane (4 mL) and a saturated aqueous solution of sodium hydrogen carbonate (8 mL) under ice-cooling to separate the solution. The resulting aqueous layer was subjected to extraction with dichloromethane, the resulting dichloromethane layers were combined, washed with saturated brine, dried over anhydrous sodium sulfate, filtered, and the resulting filtrate was concentrated under reduced pressure to give a pale yellow oil. The oil was purified under the following conditions, and the fraction comprising the target compound was concentrated under reduced pressure. To the concentrated residues were added acetonitrile and water, and then the resulting mixture was freeze-dried to give U-027 (21.69 mg, yield: 68.79%) as white solids.
MS (ESI) m/z 777 $(M+H)^+$

Column: Waters SunFire Prep C18 5 μm ODB 19*150 mm
Flow rate: 17 mL/min.
Elution solvent: acetonitrile
Detection wavelength: 220 nm

Production example 28. U-028

[C305]

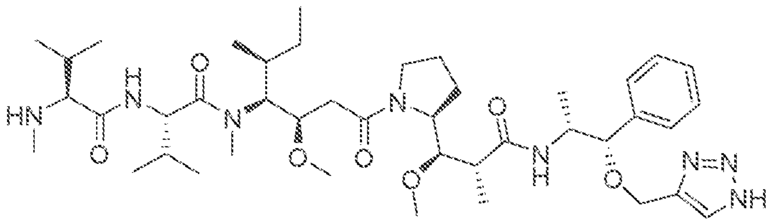

(U-028)

To a solution of U-022-2 (26.4 mg, 0.035 mmol), a solution of 4-methylbenzenesulfonylazide in 11 to 15% toluene (0.095 mL, 85.5 mg, 0.048 mmoL), and 2-aminophenol (4.0 mg, 0.037 mmoL) in acetonitrile (0.3 mL) in a 20 mL cylindrical flask was added copper(II) acetate (3.2 mg, 0.018 mmoL) under argon gas flow with stirring at room temperature, the resulting mixture was stirred at room temperature for 5 hours, and then left to stand for 15 hours. After the reaction was completed, acetonitrile was removed by a nitrogen blow, methanol (1 mL) was added thereto, and the resulting mixture was heated under reflux for 2 hours. The resulting residues were subjected to preparative HPLC under the following conditions, the fraction comprising the target compound was concentrated under reduced pressure to distill away acetonitrile, and then the resulting residues were freeze-dried to give crude products of U-028 as white solids.

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 100% (10.00 min.)

The resulting solids were purified under the following conditions, and then freeze-dried to give U-028 (2.1 mg, yield: 7.53%) as white solids.
MS (DUIS) m/z 799 $(M+H)^+$

Device: LC Forte/R (YMC Co., Ltd.)
Column: CHIRALPAK IB (20 × 250 mm, 5 μm) (Daicel Corporation)
Eluent: MeOH
Flow rate: 15 mL/min
Wavelength: 220, 254, 285 nm

Production example 29. U-029

[C306]

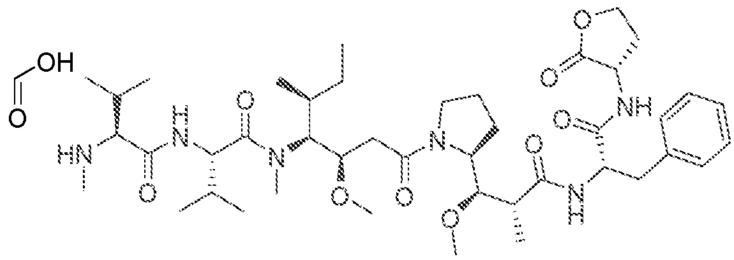

(U-029)

MMAF (49.7 mg, 0.068 mmoL) and (S)-(-)-α-amino-γ-butyrolactone hydrochloride (i.e., L-homoserine lactone hydrochloride) (14.1 mg, 0.102 mmoL) in a 30 mL cylindrical tube were dissolved in N,N-dimethylformamide (1.0 mL), and triethylamine (38 μL, 27.59 mg, 0.273 mmoL) was added thereto. Subsequently, HATU (28.6 mg, 0.075 mmol) was added thereto at room temperature, and the resulting mixture was stirred at room temperature for 12 hours. The reaction solution was concentrated under reduced pressure, the resulting residues were subjected to preparative HPLC under the following conditions, the fraction comprising the target compound was concentrated under reduced pressure to distill away

acetonitrile, and then the resulting residues were freeze-dried to give formate of U-029 (19.6 mg, yield: 33.52%) as colorless foam.
MS (ESI) m/z 816 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 25% (0.00 min.) → 38% (6.00 min.)

Production example 30. U-030

30-1. U-030-1

[C307]

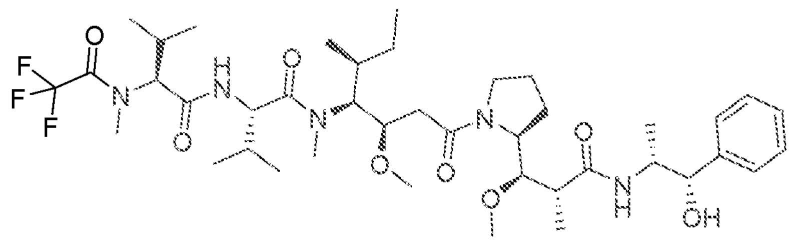

(U-030-1)

To a solution of MMAE (0.7545 g,1.051 mmol) in dichloromethane (15 mL) in a 50 mL pear-shaped flask were sequentially added triethylamine (0.73 mL, 0.53 g, 5.24 mmoL) and 2,2,2-trifluoroacetic anhydride (0.73 mL, 1.1 g, 5.25 mmoL) under argon atmosphere with stirring under ice-cooling, and the resulting mixture was stirred under ice-cooling for 1 hour. Subsequently, methanol (3.8 mL, 3.01 g, 94 mmoL) and triethylamine (0.38 mL, 0.28 g, 2.73 mmoL) were sequentially added thereto under ice-cooling, and the resulting mixture was stirred under ice-cooling for 1 hour. Subsequently, the mixture was concentrated under reduced pressure, ethyl acetate (30 mL) was added thereto, the resulting mixture was sequentially washed with a 5% by weight of aqueous solution of potassium hydrogen sulfate (30 mL), a saturated aqueous solution of sodium hydrogen carbonate (30 mL), and saturated brine (30 mL), dried over anhydrous magnesium sulfate, filtered, and the resulting filtrate was concentrated under reduced pressure. To the resulting residues were added acetonitrile (10 mL) and ultrapure water (8 mL), the resulting mixture was frozen, and freeze-dried to give U-030-1 (0.8320 g, yield: 97.27%) as white solids.
MS (ESI) m/z 815 $(M+H)^+$

30-2. U-030-2

[C308]

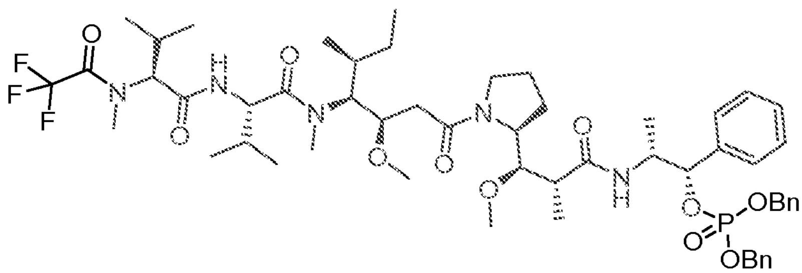

(U-030-2)

To a solution of U-030-1 (0.8011 g,0.984 mmol) and 1H-tetrazole (0.4840 g, 6.91 mmoL) in dichloromethane (30

mL) in a 50 mL pear-shaped flask was added dibenzyl diisopropylphosphoramidite (1.28 mL, 1.33 g, 3.85 mmoL) under argon atmosphere with stirring at room temperature, and the resulting mixture was stirred at room temperature for 2 hours. Subsequently, 30% by weight of hydrogen peroxide water (0.60 mL, 0.67 g, 5.87 mmoL) was added thereto under ice-cooling, and the resulting mixture was stirred under ice-cooling for 1.5 hours.

Subsequently, to sodium thiosulfate (12.75 g) was added a saturated aqueous solution of sodium hydrogen carbonate under ice-cooling to prepare a solution (100 mL), and 20 mL of said solution was added to the above mixture. The resulting organic layer was separated, then washed with saturated brine, dried over anhydrous magnesium sulfate, filtered, and the resulting filtrate was concentrated under reduced pressure.

The resulting residues were subjected to YAMAZEN medium pressure flash chromatography ((Fuji Silysia Chromatorex Q-PACK, DIOL-60), dichloromethane/isopropanol = 99/1 (V/V) → 95/5 (V/V)), and the fraction comprising the target compound was concentrated under reduced pressure to give crude products of U-030-2.

The resulting crude products were purified under the following conditions, and the fraction comprising the target compound was concentrated under reduced pressure. To the resulting residues were added acetonitrile (10 mL) and ultrapure water (8 mL), the resulting mixture was frozen, and freeze-dried to give U-030-2 (0.6915 g, yield: 65.41%) as white solids.
MS (ESI) m/z 1075 $(M+H)^+$

Device: LC Forte/R (YMC Co., Ltd.)
Column: YMC-GPC T-30000, T-4000, and T-2000 (triconnection) 21.2*600 mm, 10 μm
Temperature: room temperature
Eluent: acetonitrile
Flow rate: 25 mL/min
Wavelength: 220 nm

30-3. U-030-3

[C309]

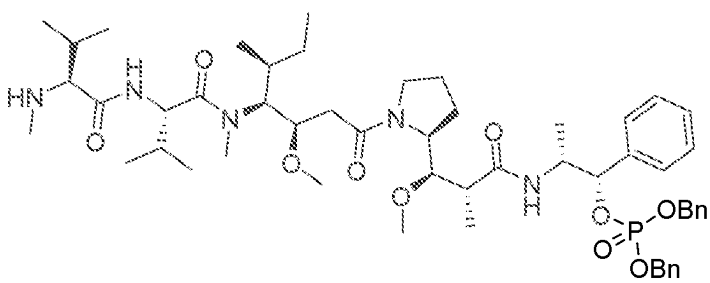

(U-030-3)

To a solution of U-030-2 (331 mg, 0.308 mmol) in methanol (5 mL) in a 50 mL pear-shaped flask was added sodium borohydride (34.6 mg, 0.915 mmol) under argon atmosphere with stirring under ice-cooling, and the resulting mixture was stirred under ice-cooling for 1.25 hours. Subsequently, sodium borohydride (107.4 mg, 2.838 mmol) was added dividedly (three parts) thereto under ice-cooling, and the resulting mixture was stirred under ice-cooling for 3 hours.

After the reaction was completed, dichloromethane (20 mL) and a saturated aqueous solution of sodium hydrogen carbonate (20 mL) were added thereto under ice-cooling, and the resulting organic layer was separated. The resulting aqueous layer was subjected to extraction with dichloromethane (20 mL) three times, the resulting organic layers were combined, dried over anhydrous sodium sulfate, filtered, and the resulting filtrate was concentrated under reduced pressure to give U-030-3 (325 mg, yield: quantitative) as a colorless oil.
MS (ESI) m/z 979 $(M+H)^+$

30-4. U-030

[C310]

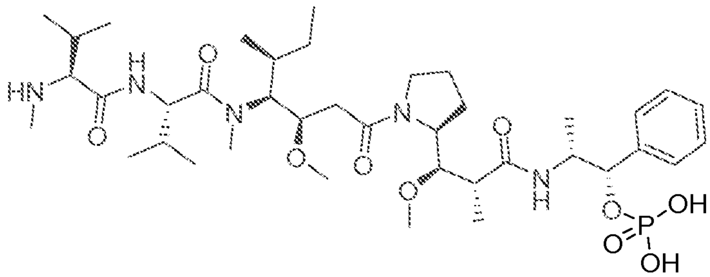

(U-030)

To a solution of U-030-3 (301 mg, 0.308 mmol) in ethanol (30 mL) in a 100 mL pear-shaped flask was added 5% by weight of palladium carbon STD type (wetted with 50% by weight of water, 82.3 mg, 0.019 mmoL) under nitrogen atmosphere with stirring, and the resulting mixture was stirred under hydrogen atmosphere at room temperature for 2 hours.

After the reaction was completed, the mixture was filtered through Celite 545 (trade name), washed with ethanol, and concentrated under reduced pressure. To the resulting residues was added acetonitrile, the resulting solids were collected by filtration, washed with ice-cooled acetonitrile, and dried under reduced pressure to give U-030 (220.3 mg, yield: 89.72%) as white solids.
MS (ESI) m/z 799 $(M+H)^+$

Production example 31. U-031

[C311]

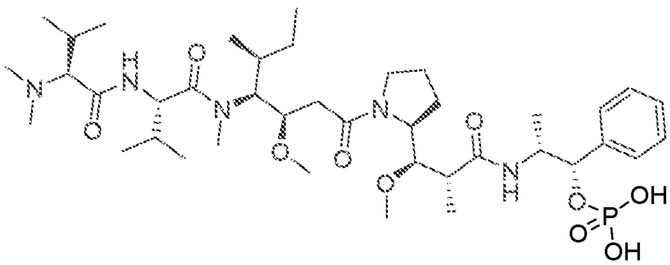

(U-031)

To a solution of the Production example 30 (100.4 mg, 0.126 mmoL) and a 37% aqueous solution of formaldehyde (0.093 mL, 101.37 mg, 1.249 mmoL) in methanol (1 mL) in a 20 mL cylindrical flask was added sodium triacetoxyborohydride (77.8 mg, 0.367 mmoL) under argon atmosphere with stirring at room temperature, and the resulting mixture was stirred at room temperature for 1 hour.

After the reaction was completed, the reaction mixture was concentrated under reduced pressure, and water was added thereto. The resulting aqueous solution was subjected to preparative HPLC under the following conditions, and the fraction comprising the target compound was concentrated under reduced pressure to give crudely purified products of U-031 (41.0 mg) as white solids.

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 10% (0.00 min.) → 30% (4.00 min.) → 30% (9.00 min.)

The resulting crudely purified products of U-031 (41.0 mg) were subjected to preparative HPLC under the following conditions, and the fraction comprising the target compound was freeze-dried to give U-031 (29.5 mg, yield: 28.87%) as white solids.
MS (ESI) m/z 810 $(M-H)^-$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 10% (0.00 min.) → 30% (5.00 min.) → 30% (10.00 min.)

Production example 32. U-032

[C312]

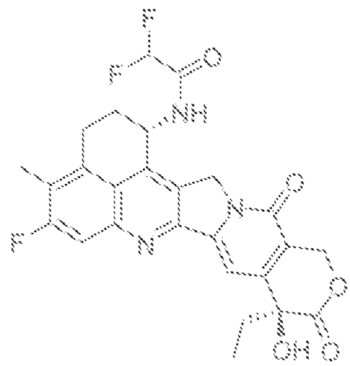

(U-032)

To a solution of exatecan mesylate (13.6 mg, 0.026 mmoL) in dichloromethane (1 mL) in a 10 mL cylindrical flask was added triethylamine (35 μL, 25.41 mg, 0.251 mmoL) under argon gas flow with stirring, and the resulting mixture was ice-cooled. 2,2-Difluoroacetic anhydride (10 μL, 16 mg, 0.092 mmoL) was added thereto under ice-cooling, and the resulting mixture was stirred at 0°C for 2 hours. Subsequently, triethylamine (17 μL, 12.34 mg, 0.122 mmoL) and 2,2-difluoroacetic anhydride (10 μL, 16 mg, 0.092 mmoL) were added thereto under stirring at room temperature, and the resulting mixture was stirred at room temperature for 1 hour. Subsequently, methanol (100 μL, 79.2 mg, 2.472 mmoL) was added thereto under stirring at room temperature, and the resulting mixture was stirred at room temperature for 0.5 hour. Subsequently, triethylamine (60 μL, 43.56 mg, 0.430 mmoL) was added thereto under stirring at room temperature, and the resulting mixture was stirred at room temperature for 0.5 hour.

After the reaction was completed, diethyl ether (2 mL) was added thereto, the resulting mixture was stirred for 5 minutes, filtered through a membrane filter, the resulting solids were washed with ethyl acetate and water, and dried under reduced pressure to give U-032 (7.9 mg, yield: 60.13%) as slightly yellow solids.
MS (ESI) m/z 514 $(M+H)^+$

Production example 33. U-033

33-1. U-033-1

[C313]

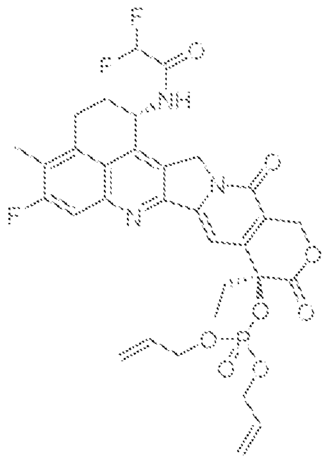

(U-033-1)

To a solution of U-032 (419 mg, 0.816 mmoL) in dichloromethane (12 mL) in a 100 mL cylindrical flask were sequentially added diallyl N,N-diisopropylphosphoramidite (820 μL, 760.96 mg, 3.10 mmoL) and 1H-tetrazole (373 mg, 5.32 mmoL) under argon atmosphere with stirring at room temperature, and the resulting mixture was stirred at room temperature for 5 hours. Subsequently, 30% by weight of hydrogen peroxide water (480 μL, 532.8 mg, 4.70 mmoL) was added thereto under ice-cooling, and the resulting mixture was stirred under ice-cooling for 1 hour. Subsequently, to sodium thiosulfate pentahydrate (20.0 g, 80.6 mmoL) and sodium hydrogen carbonate (7.0 g, 83.3 mmoL) was added water (100 mL) to prepare a solution, 6 mL of said solution and dichloromethane (6 mL) were added to the above mixture to separate it. The resulting aqueous layer was subjected to extraction with dichloromethane (6 mL), combined with the organic layer, dried over anhydrous magnesium sulfate (0.80 g), filtered, and the resulting filtrate was concentrated under reduced pressure to give concentrated residues (967 mg). To the resulting residues was added a 5% aqueous solution of acetonitrile, the resulting mixture was subjected to sonication, filtered under reduced pressure, and the resulting solids were dried to give U-033-1 (250 mg, yield: 45.48%) as brown solids.

MS (ESI) m/z 1348 $(2M+H)^+$

33-2. U-033

[C314]

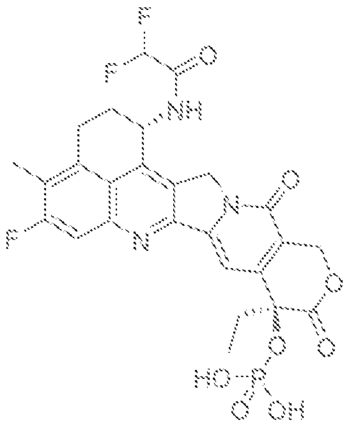

(U-033)

To a solution of U-033-1 (243 mg, 0.361 mmoL) in tetrahydrofuran (5 mL) in a 50 mL round-bottom flask were sequentially added N-methylaniline (84 μL, 83.16 mg, 0.776 mmoL) and tetrakis(triphenylphosphine)palladium (43 mg, 0.037 mmoL) under argon atmosphere with stirring at room temperature, and the resulting mixture was stirred at room temperature for 1 hour.

After the reaction was completed, tert-butyl methyl ether (15 mL) was added thereto, the resulting mixture was

stirred for 10 minutes, then the precipitated solids were filtered, washed with tert-butyl methyl ether, and dried under reduced pressure to give crude products of U-033 (256 mg).

The resulting solids were dissolved in a 30% aqueous solution of acetonitrile (15 mL) containing 2% by weight of ammonium acetate, the resulting solution was subjected to preparative HPLC under the following conditions, and the fraction comprising the target compound was freeze-dried to give U-033 (81 mg, yield: 37.83%) as pale yellow solids. (35905AL-028-2)

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 60% (6.00 min.) → 90% (6.50 min.) → 90% (9.00 min.)

MS (ESI) m/z 592 $(M-H)^-$

Production example 34. U-034

[C315]

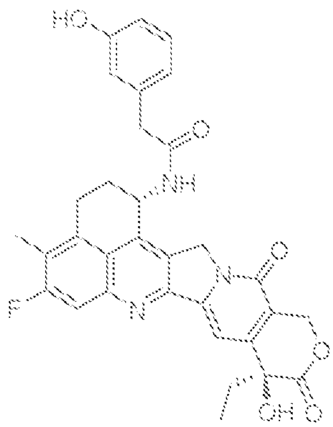

(U-034)

To a solution of exatecan mesylate (53.2 mg, 0.100 mmoL), 2-(3-hydroxyphenyl)acetic acid (18.3 mg, 0.120 mmoL), 1-ethyl-3-(3-dimethylaminopropyl)carbodiimide hydrochloride (38.2 mg, 0.199 mmoL), and 1-hydroxybenzotriazole (15.3 mg, 0.100 mmoL) in N,N-dimethylformamide (1 mL) in a 10 mL cylindrical flask was added triethylamine (28 μL, 20.33 mg, 0.201 mmoL) under argon atmosphere with stirring at room temperature, and the resulting mixture was stirred at room temperature for 2 hours.

After the reaction was completed, the resulting reaction solution was subjected to preparative HPLC under the following conditions, the fraction comprising the target compound was concentrated under reduced pressure, the precipitated solids were collected by filtration, and washed with water to give U-033 (25.4 mg, yield: 44.56%) as colorless solids.

MS (ESI) m/z 568 $(M-H)^-$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 95% (5.00 min.)

Production example 35. U-035

35-1. U-035-1

[C316]

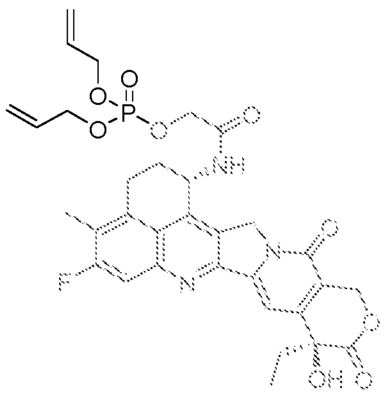

(U-033-1)

To a solution of DXd (162.6 mg, 0.329 mmoL) in N,N-dimethylformamide (4 mL) in a 50 mL pear-shaped flask was added trifluoromethanesulfonic acid (29.0 μL, 49.3 mg, 0.329 mmoL) under argon atmosphere with stirring, and the resulting mixture was stirred at room temperature for 30 minutes. Subsequently, diallyl N,N-diisopropylphosphoramidite (350 μL, 324.8 mg, 1.324 mmoL) and 1H-tetrazole (163.7 mg, 2.337 mmoL) were added thereto at room temperature, and the resulting mixture was stirred at room temperature for 2 hours.

Subsequently, a 70% aqueous solution of tert-butyl peroxide (270 μL, 253.8 mg, 1.971 mmoL) was added thereto at room temperature, and the resulting mixture was stirred at room temperature for 1.5 hours.

Subsequently, to the reaction solution was added diethyl ether (40 mL), and the resulting mixture was stirred under ice-cooling for a while. The resulting solids were collected by filtration under reduced pressure, washed with diethyl ether, and then dried under reduced pressure to give trifluoromethanesulfonate of U-035-1 (249.6 mg, yield: 94.26%) as light brown solids.

MS (ESI) m/z 654 $(M+H)^+$

35-2. U-035

[C317]

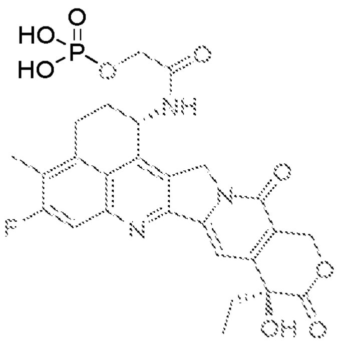

(U-035)

To a solution of trifluoromethanesulfonate of U-035-1 (51.4 mg, 0.064 mmoL) in tetrahydrofuran (5 mL) in a 20 mL pear-shaped flask were sequentially added N-methylaniline (22 μL, 21.78 mg, 0.203 mmoL) and tetrakis(triphenylphosphine)palladium (10.4 mg, 9.00 μmoL) under argon atmosphere with stirring at room temperature, and the resulting mixture was stirred at room temperature for 1.25 hours. Subsequently, tetrakis(triphenylphosphine)palladium (27.9 mg, 0.024 mmoL) was additionally added thereto, then N,N-dimethylformamide (0.5 mL) was added thereto, and the resulting mixture was stirred at room temperature for 0.75 hour.

The reaction solution was concentrated under reduced pressure. To the concentrated residues was added diethyl ether, the resulting mixture was subjected to sonication, the resulting solids were collected by filtration, washed with diethyl ether, and dried under reduced pressure to give gray-brown solids (55.2 mg).

The resulting gray-brown solids were subjected to the following recycle preparative, and the fraction comprising the target compound was freeze-dried to give U-035 (6.4 mg, yield: 17.45%) as pale yellow solids.
MS (ESI) m/z 574 $(M+H)^+$

Device: LC Forte/R (YMC Co., Ltd.)
Column: Xbridege C18, 30x150 mm, 5 μm
Eluent: 0.1% aqueous solution of formic acid (Solution A) - 0.1% solution of formic acid in acetonitrile (Solution B)
Solution composition (Solution B): 30%
Flow rate: 25 mL/min, 21 MPa
Wavelength: 254 nm

Synthesis of Example A1

[C318]

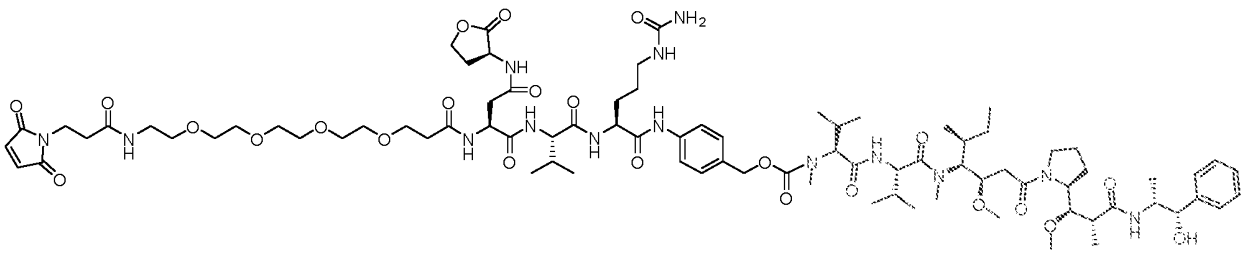

Example A1-1

[C319]

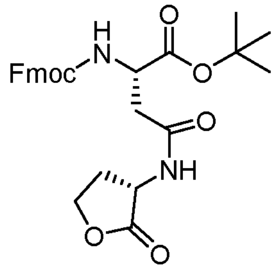

(S)-(-)-α-amino-γ-butyrolactone hydrochloride (i.e., L-homoserine lactone hydrochloride) (1.75 g, 12.72 mmoL) in a 200 mL round-bottom flask was dissolved in N,N-dimethylformamide (60 mL), and then Fmoc-(L)-Asp-tert-butyl (5.23 g, 12.71 mmoL) was added thereto, and triethylamine (3.65 mL, 2.65 g, 26.2 mmoL) was added thereto. Subsequently, HATU (4.83 g, 12.70 mmoL) was added thereto at room temperature, and the resulting mixture was stirred at room temperature for 2 hours. After the reaction was completed, to the reaction solution was added water, and the resulting mixed solution was subjected to extraction with ethyl acetate twice. The resulting organic layer was concentrated under reduced pressure to give the title compound (6.16 g, yield: 97.9%) as a colorless oil.

Example A1-2

[C320]

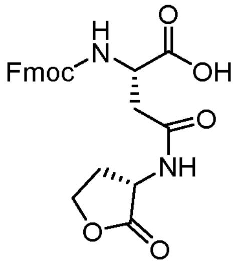

Example A1-1 (6.16 g, 12.46 mmoL) put into a 500 mL eggplant flask was dissolved in dichloromethane (60 mL), trifluoroacetic acid (60 mL) (1.421 g, 12.46 mmoL) was then added, the mixture was stirred at room temperature for 2 hours. After the reaction was completed, the solvent was distilled off under reduced pressure. The resulting residues were dissolved in acetonitrile (50 mL), and ethyl acetate (50 mL) was additionally added thereto. The precipitated solids were collected by filtration and washed with ethyl acetate to obtain a title compound (3.86 g, yield 70.68%) as a colorless solid.
MS(ESI)m/z 439(M+H)$^+$

Example A1-3

[C321]

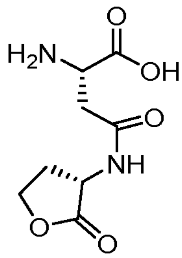

To a solution of Example A1-2 (298 mg, 0.680 mmoL) in N,N-dimethylformamide (4 mL) put into a 30 mL cylindrical flask, under argon airflow with stirring, piperidine (0.270 mL) (232.2 mg, 2.73 mmoL) was added at room temperature, and the mixture was stirred at room temperature for 1 hour.
After the reaction was completed, xylene was added, and the solvent was removed under reduced pressure.
To the resulting residues were added diethyl ether (4 mL) and ethyl acetate (8 mL), the resulting mixture was subjected to sonication, and the resulting solids were collected by filtration. To the collected solids was added a 20% aqueous solution of acetonitrile, and the resulting mixture was concentrated under reduced pressure.
To the resulting residues was added ethyl acetate, and the resulting mixture was filtered. To the resulting solids was added diethyl ether, the resulting mixture was filtered, and dried under reduced pressure to give the title compound (143 mg, yield: 97.32%) as white solids.
MS (ESI) m/z 215 (M-H)$^-$

Example A1-4

[C322]

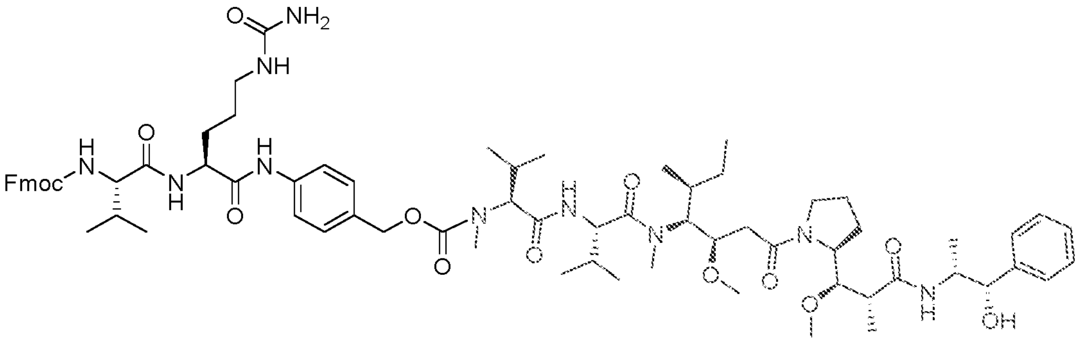

To a solution of Fmoc-(L)-Val-(L)-Cit-PAB-PNP (manufactured by Angene) (292.7 mg, 0.382 mmoL), MMAE (manufactured by MedChemExpress) (259.7 mg, 0.362 mmoL), and 1-hydroxy-7-azabenzotriazole (56.3 mg, 0.414 mmoL) in N,N-dimethylformamide (3 mL) in a 30 mL cylindrical flask was added N,N-diisopropylethylamine (70 μL, 51.94 mg, 0.402 mmoL) under nitrogen atmosphere with stirring, and the resulting mixture was stirred at room temperature for 19.5 hours. After the reaction was completed, the solvent was distilled away under reduced pressure. To the resulting residues was added diethyl ether (20 mL), the precipitated solids were collected by filtration, washed with water, and then washed with diethyl ether to give the title compound (415.8 mg, yield: 85.43%) as beige solids.
MS (DUIS) m/z 673 $(M+2H)^{2+}$

Example A1-5

[C323]

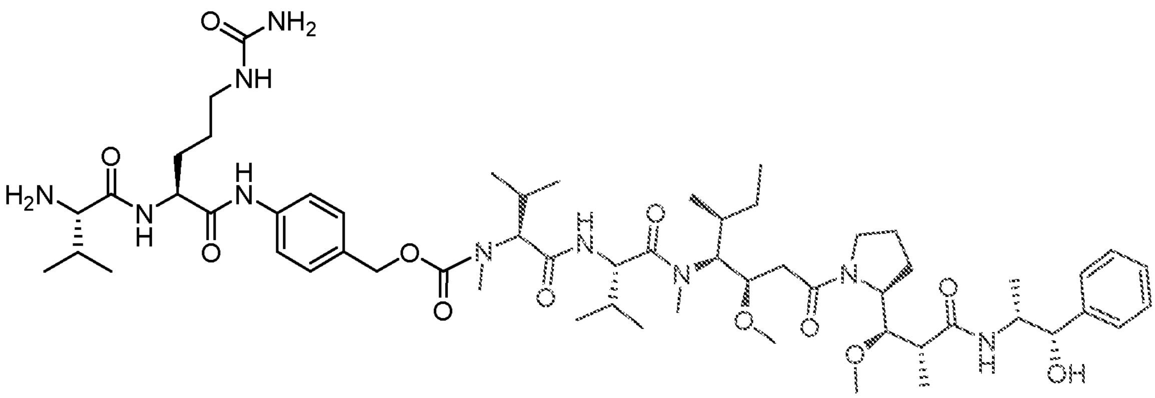

To a solution of the Example A1-4 (414.7 mg, 0.308 mmoL) in N,N-dimethylformamide (5 mL) in a 30 mL pear-shaped flask was added piperidine (0.244 mL, 210 mg, 2.465 mmoL) at room temperature, and then the resulting mixture was stirred at room temperature for 1 hour. The reaction solution was concentrated under reduced pressure. To the resulting residues were added ethyl acetate, acetonitrile, and dichloromethane to dissolve the residues. Then, diethyl ether was added thereto, the resulting mixture was subjected to sonication, the resulting solids were collected by filtration, washed with diethyl ether, and dried under reduced pressure to give the title compound (280.3 mg, yield: 80.96%) as pale yellow solids.
MS (ESI) m/z 1124 $(M+H)^+$

Example A1-6

[C324]

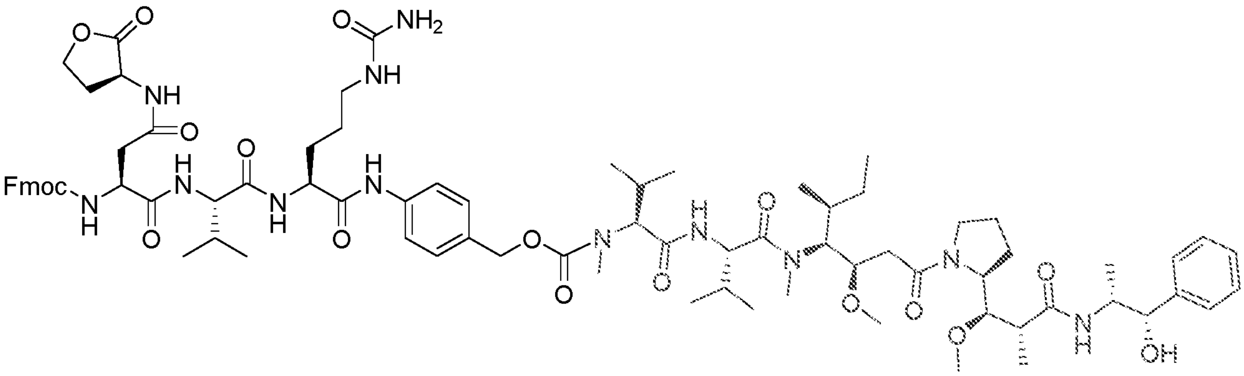

To a solution of the Example A1-2 (130.1 mg, 0.297 mmoL) and the Example A1-5 (309 mg, 0.275 mmoL) in N,N-dimethylformamide (5 mL) in a 30 mL cylindrical flask was added triethylamine (41 μL, 29.77 mg, 0.294 mmoL) at room temperature, then HATU (112.9 mg, 0.297 mmoL) was added thereto at room temperature, and then the resulting mixture was stirred at room temperature for 2 hours. The reaction solution was concentrated under reduced pressure, dichloromethane and 2-propanol were added thereto, and the resulting mixture was concentrated under reduced pressure. Acetonitrile (10 mL) and water (10 mL) were added thereto, the resulting mixture was subjected to sonication, then the resulting solids were collected by filtration, washed with acetonitrile/water (1/1 (V/V)) (10 mL), then washed with diethyl ether (20 mL), and the resulting solids were dried under reduced pressure to give the title compound (342.3 mg, yield: 80.61%) as white solids.
MS (ESI) m/z 773 $(M+2H)^{2+}$

Example A1-7

[C325]

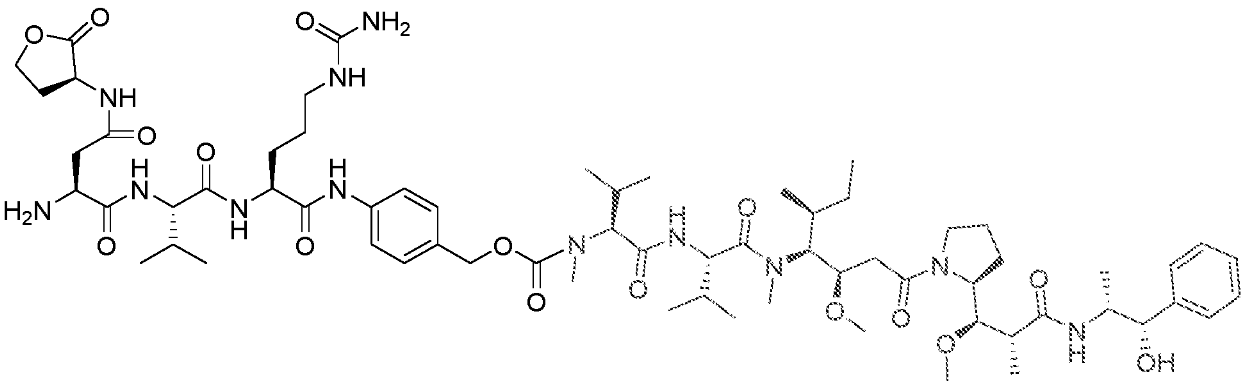

To a solution of the Example A1-6 (49.7 mg, 0.032 mmoL) in N,N-dimethylformamide (450 μL) in a 10 mL pear-shaped flask was added piperidine (10 μL, 8.62 mg, 0.101 mmoL) under argon gas flow with stirring at room temperature, the resulting mixture was stirred at room temperature for 50 minutes, then the solvent was removed under reduced pressure, the resulting residues were washed with diethyl ether (10 mL), and the resulting solids were dried under reduced pressure to give the title compound (36.4 mg, yield: 85.56%) as white solids.
MS (ESI) m/z 1323 $(M+H)^+$

Example A1

[C326]

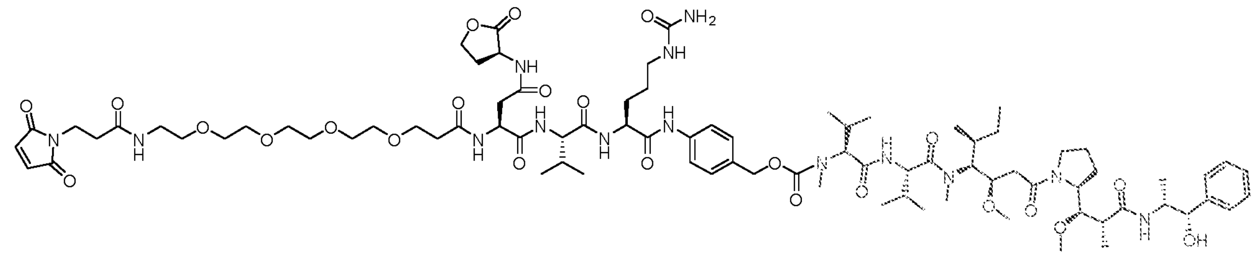

To a solution of the Example A1-7 (10.0 mg, 7.57 μmoL) in N,N-dimethylformamide (0.4 mL) in a 5 mL cylindrical flask was added a solution of N-succinimidyl 1-maleimide-3-oxo-7,10,13,16-tetraoxa-4-azanonadecanoate (8.2 mg, 0.027 mmoL) in acetonitrile (400 μL) under argon atmosphere with stirring, and the resulting mixture was stirred at room temperature for 1 hour.

The resulting reaction solution was subjected to preparative HPLC under the following conditions, and the fraction comprising the target compound was freeze-dried to give the Example A1 (3.2 mg, yield: 24.59%) as white solids. MS (ESI) m/z 861 $(M+2H)^{2+}$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 40% (0.00 min.) → 90% (10.00 min.)

Synthesis of Example A2

[C327]

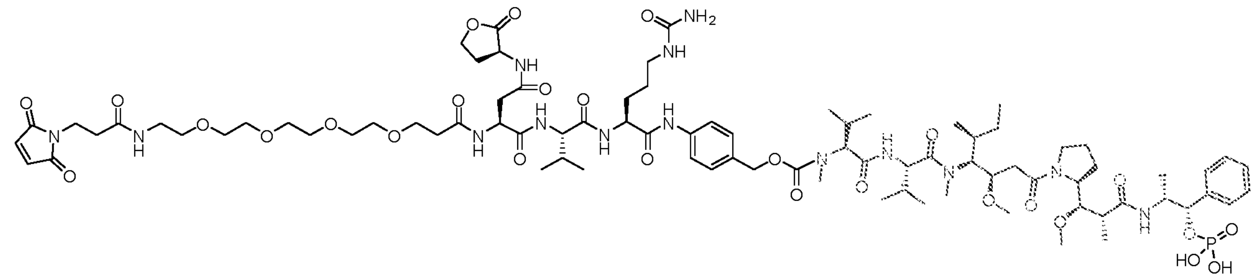

Example A2-1

[C328]

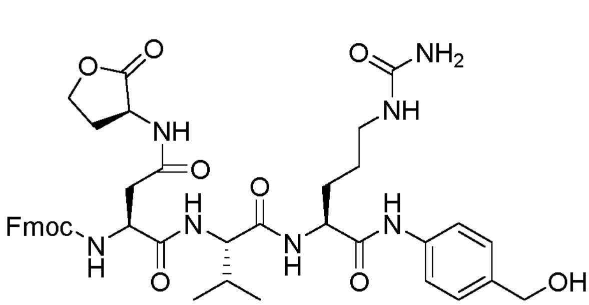

(L)-Val-(L)-Cit-PAB (manufactured by Angene) (1.90 g, 5.01 mmoL) and the Example A1-2 (2.24 g, 5.11 mmoL) in

a 200 mL round-bottom flask were suspended in N,N-dimethylformamide (50 mL), triethylamine (0.71 mL, 0.52 g, 5.09 mmoL) and then HATU (1.94 g, 5.10 mmoL) were added thereto, and the resulting mixture was stirred at room temperature for 1 hour. To the resulting reaction solution were added water (30 mL) and acetone (20 mL), the resulting mixture was stirred for 30 minutes, then filtered, washed with water, and then washed with acetone to give the title compound (3.37 g, yield: 84.13%) as slightly yellow solids.
MS (ESI) m/z 800 $(M+H)^+$

Example A2-2

[C329]

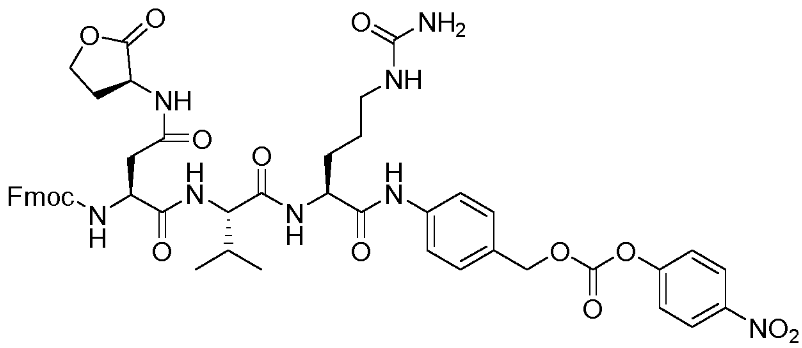

To a solution of the Example A2-1 (1.027 g, 1.284 mmoL) in N,N-dimethylformamide (12 mL) in a 100 mL round-bottom flask were added bis(4-nitrophenyl)carbonate (760 mg, 2.498 mmoL) and N,N-diisopropylethylamine (440 μL, 0.33 g, 2.52 mmoL) under argon gas flow with stirring at room temperature, and the resulting mixture was stirred at room temperature for 3 hours. Subsequently, N,N-dimethylformamide (8 mL) was added thereto under stirring at room temperature, the resulting mixture was stirred at room temperature for 0.5 hour, then the resulting insoluble matters were filtered, and the resulting filtrate was concentrated under reduced pressure. To the resulting residues were added diethyl ether and hexane, the resulting solids were filtered, washed with water four times, then washed with diethyl ether, and dried under reduced pressure to give the title compound (760 mg, yield: 61.34%) as slightly yellow solids.
MS (ESI) m/z 965 $(M+H)^+$

Example A2-3

[C330]

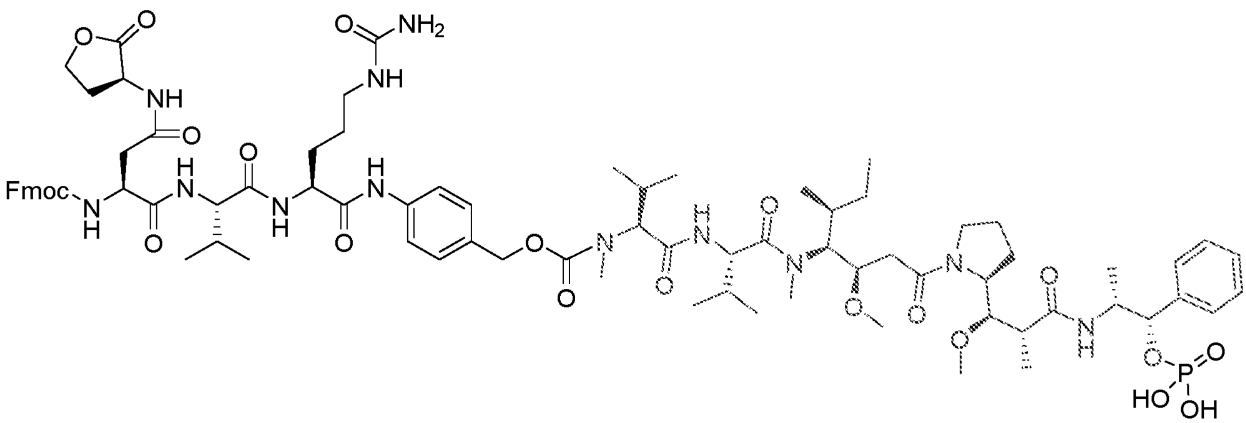

To a solution of the Example A2-2 (76.2 mg, 0.079 mmoL), the U-030 (55.1 mg, 0.069 mmoL), and 1-hydroxy-7-azabenzotriazole (13.0 mg, 0.096 mmoL) in N,N-dimethylformamide (1 mL) in a 10 mL pear-shaped flask was added N,N-diisopropylethylamine (40 μL, 29.68 mg, 0.230 mmoL) at room temperature, and then the resulting mixture was stirred at room temperature for 18 hours. Subsequently, the Example A2-2 (22.3 mg, 0.023 mmoL) was added thereto, and the resulting mixture was stirred at room temperature for 6.5 hours.

After the reaction was completed, the mixture was concentrated under reduced pressure. To the resulting residues was added diethyl ether, and the resulting solids were collected by filtration. To the resulting solids was added acetonitrile/water (1/1 (V/V)) (10 mL), the resulting insoluble matters were removed by filtration, and then freeze-dried to give the title compound (57.4 mg, yield: 51.19%) as white solids.
MS (ESI) m/z 812 $(M+2H)^{2+}$

Example A2-4

[C331]

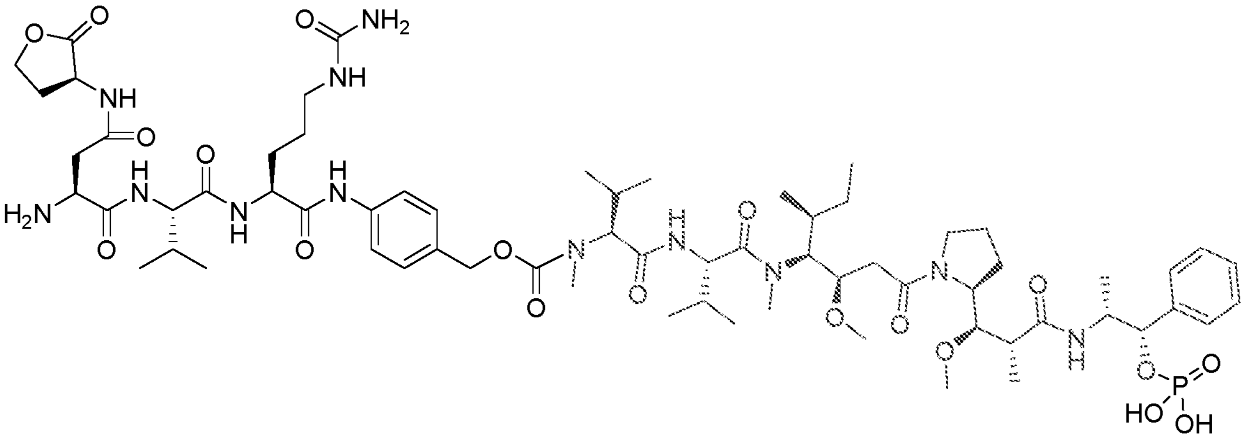

To a solution of the Example A2-3 (57.4 mg, 0.035 mmoL) in N,N-dimethylformamide (2 mL) in a 100 mL pear-shaped flask was added piperidine (11 μL, 9.48 mg, 0.111 mmoL) under argon gas flow with stirring at room temperature, the resulting mixture was stirred at room temperature for 1 hour, then the solvent was removed under reduced pressure, the resulting residues were washed with diethyl ether (10 mL), and the resulting solids were dried under reduced pressure to give the title compound (51.8 mg, yield: 93.23%) as pale yellow solids. MS (ESI) m/z 1402 $(M+H)^+$

Example A2

[C332]

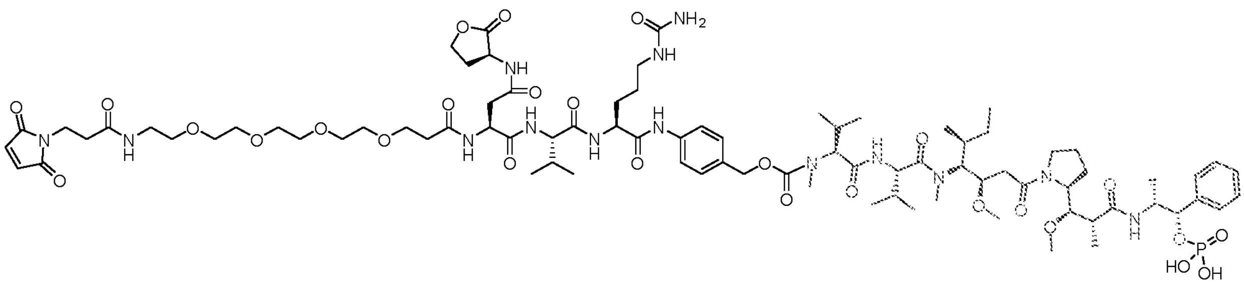

The Example A2-4 (18 mg, 13 μmoL) was reacted in the same manner as the Example A1 to give the title compound (5.2 mg, yield: 22.49%) as white solids.
MS (ESI) m/z 901 $(M+2H)^{2+}$

Synthesis of Example A3

[C333]

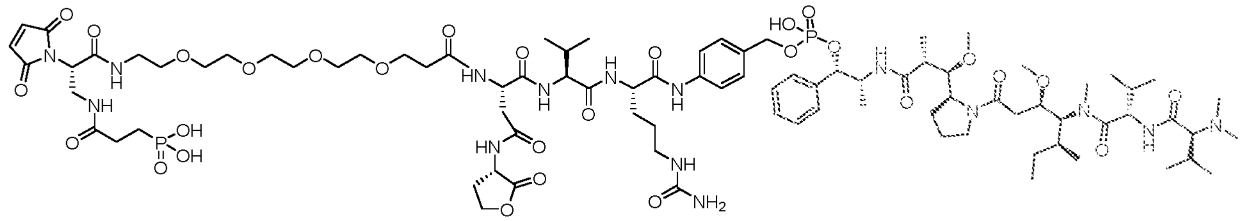

Example A3-1

[C334]

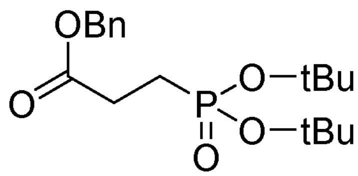

To a solution of di-tert-butyl phosphite (1.94 g, 9.99 mmoL) in acetonitrile (10 mL) in a 100 mL round-bottom flask was added benzyl acrylate (1.68 mL, 1.78 g, 10.98 mmoL) under nitrogen airflow with stirring at room temperature, and the resulting mixture was stirred at 80°C for 6 hours.

After the reaction was completed, the reaction mixture was concentrated under reduced pressure, the resulting residues were subjected to YAMAZEN medium pressure flash chromatography (Silica, L (40 g), hexane / ethyl acetate = 70/30 (V/V) → 50/50 (V/V)), and the fraction comprising the target compound was concentrated under reduced pressure to give the title compound (2.23 g, yield: 62.64%) as a colorless oil.
MS (ESI) m/z 357 $(M+H)^+$

Example A3-2

[C335]

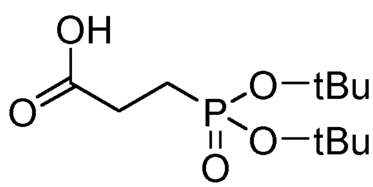

To a solution of the Example A3-1 (2.23 g, 6.26 mmol) in ethanol (22 mL) in a 200 mL round-bottom flask was added 10% by weight of palladium carbon NX-Type (wetted with 50% by weight of water, 1.30 g, 0.611 mmoL) under nitrogen atmosphere with stirring, and the resulting mixture was stirred under hydrogen atmosphere at room temperature for 2 hours.

After the reaction was completed, the reaction mixture was filtered through Celite 545 (trade name), washed with ethanol, and concentrated under reduced pressure to give the title compound (1.68 g, yield: quantitative) as a colorless oil.
MS (ESI) m/z 267 $(M+H)^+$

Example A3-3

[C336]

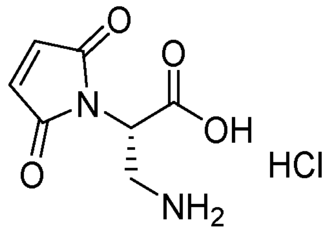

To a solution of (S)-3-((tert-butoxycarbonyl)amino)-2-(2,5-dioxo-2,5-dihydro-1H-pyrrol-1-yl)propanoic acid (i.e., (L)-3-((tert-butoxycarbonyl)amino)-2-(2,5-dioxo-2,5-dihydro-1H-pyrrol-1-yl)propanoic acid) (1.21 g, 4.26 mmoL) (disclosed in WO 2019/195665 pamphlet) in dichloromethane (4 mL) in a 100 mL round-bottom flask was added a 4 M solution of hydrogen chloride in 1,4-dioxane (21.3 mL, 3.11 g, 85 mmol) under argon gas flow with stirring at room temperature, and the resulting mixture was stirred at room temperature for 1 hour.

After the reaction was completed, the solvent was removed under reduced pressure. To the resulting residues was added ethyl acetate, and the resulting solids were filtered. The resulting solids were dissolved in methanol, and the resulting solution was concentrated under reduced pressure to give the title compound (1.17 g, yield: quantitative) as pale yellow foam.

Example A3-4

[C337]

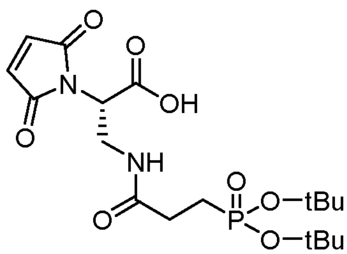

To a solution of the Example A3-2 (0.26 g, 0.976 mmoL) in N,N-dimethylformamide (3 mL) in a 30 mL cylindrical flask were added triethylamine (0.13 mL, 0.09 g, 0.933 mmoL) and HATU (0.37 g, 0.973 mmoL) under argon gas flow with stirring at room temperature, and the resulting mixture was stirred at room temperature for 1 hour to prepare a Solution A.

To a solution of the Example A3-3 (0.21 g, 0.952 mmoL) in N,N-dimethylformamide (3 mL) in a 20 mL cylindrical flask was added triethylamine (0.065 mL, 0.045 g, 0.466 mmoL) under argon gas flow with stirring at room temperature, and the resulting mixture was stirred at room temperature for 10 minutes.

To the above 20 mL cylindrical flask was added the Solution A at room temperature, and the resulting mixture was stirred at room temperature for 6 hours.

After the reaction was completed, to the reaction solution was added water, the resulting solution was filtered through a membrane filter, then the resulting solution was subjected to preparative HPLC under the following conditions, and the fraction comprising the target compound was freeze-dried to give the title compound (143.9 mg, yield: 34.96%) as colorless solids.
MS (ESI) m/z 433 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 36% (5.00 min.)

Example A3-5

[C338]

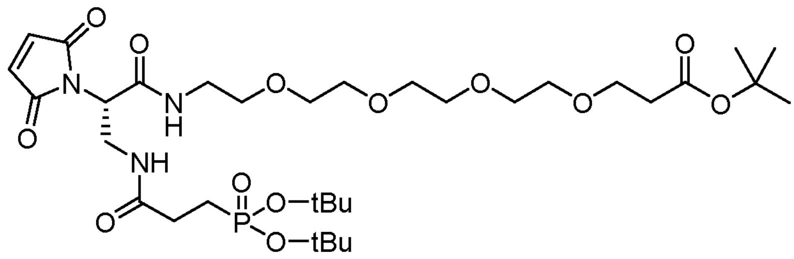

To a solution of the Example A3-4 (143.8 mg, 0.333 mmoL) in N,N-dimethylformamide (3 mL) in a 30 mL pear-shaped flask were added triethylamine (56 μL, 40.66 mg, 0.402 mmoL) and HATU (152.5 mg, 0.401 mmoL) under argon gas flow with stirring at room temperature, and the resulting mixture was stirred at room temperature for 2 minutes. Subsequently, a solution of tert-butyl 1-amino-3,6,9,12-tetraoxapentadecan-15-oate (112.5 mg, 0.350 mmoL) in N,N-dimethylformamide (0.5 mL) was added thereto at room temperature, and the resulting mixture was stirred at room temperature for 1 hour.

After the reaction was completed, the reaction solution was subjected to preparative HPLC under the following conditions, and the fraction comprising the target compound was freeze-dried to give the title compound (143.5 mg, yield: 58.64%) as colorless solids.
MS (ESI) m/z 734 $(M-H)^-$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 60% (8.00 min.)

Example A3-6

[C339]

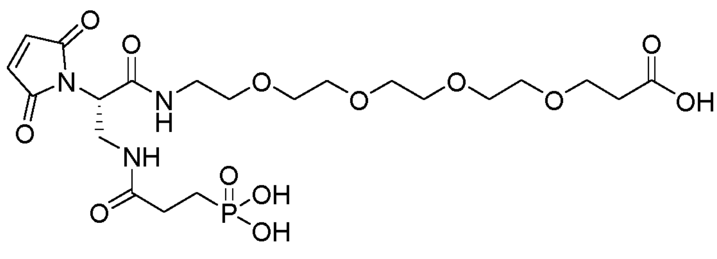

To a solution of the Example A3-5 (63.3 mg, 0.086 mmoL) in dichloromethane (1.5 mL) in a 30 mL pear-shaped flask was added trifluoroacetic acid (150 μL, 223.35 mg, 1.959 mmoL) under nitrogen airflow with stirring at room temperature, and the resulting mixture was stirred at room temperature for 2.5 hours. Subsequently, trifluoroacetic acid (0.5 mL, 744.5 mg, 6.530 mmoL) was added thereto at room temperature, and the resulting mixture was stirred at room temperature for 2 hours.

After the reaction was completed, the reaction solution was concentrated under reduced pressure. To the resulting residues was added acetonitrile/water (1/1 (V/V)) (2 mL), and the resulting mixture was freeze-dried to give the title compound (56.6 mg, yield: quantitative) as white solids.
MS (ESI) m/z 568 $(M+H)^+$

Example A3-7

[C340]

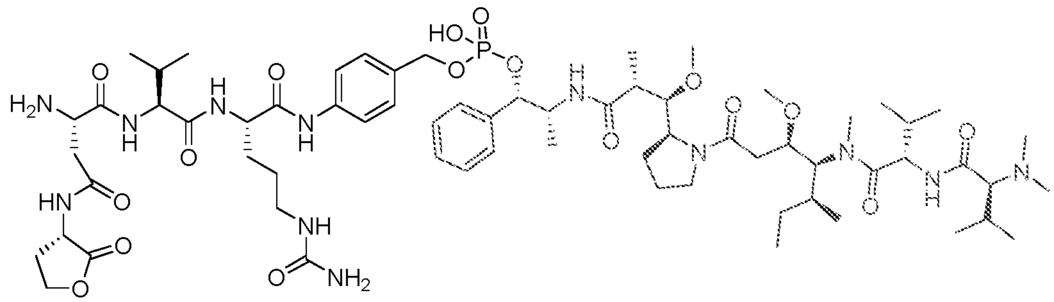

To a solution of the Example A2-1 (340.9 mg, 0.426 mmol) in N,N-dimethylformamide (6 mL) in a 100 mL pear-shaped flask were added 3-((bis(diisopropylamino)phosphino)oxy)propanenitrile (255 μL, 242.25 mg, 0.804 mmoL) and 1H-tetrazole (56.6 mg, 0.808 mmoL) under argon atmosphere with stirring at room temperature, and the resulting mixture was stirred at room temperature for 15 minutes. Subsequently, triisopropylsilyl trifluoromethanesulfonate (215 μL, 245.1 mg, 0.800 mmoL) was added thereto at room temperature, and the resulting mixture was stirred at room temperature for 30 minutes.

Subsequently, the U-010 (146 mg, 0.199 mmol) and 5-(ethylthio)-1H-tetrazole (109.4 mg, 0.840 mmoL) were added thereto, and the resulting mixture was stirred at room temperature for 1 hour. Subsequently, tert-butyl hydroperoxide (330 μL, 310.2 mg, 2.409 mmol) was added thereto under ice-cooling, and the resulting mixture was stirred at room temperature for 2 hours.

Subsequently, diazabicycloundecene (600 μL, 612 mg, 4.02 mmoL) was added thereto under ice-cooling, and the resulting mixture was stirred at room temperature for 1 hour.

After the reaction was completed, diethyl ether (50 mL) was added thereto, and the resulting supernatant solution was removed. Subsequently, diethyl ether (25 mL) was added thereto, and the resulting supernatant solution was removed. Subsequently, diethyl ether (25 mL) was added thereto, the resulting supernatant solution was removed, and dried under reduced pressure. The resulting residues were purified under the following recycle preparative conditions, and the fraction comprising the target compound was concentrated under reduced pressure to give the title compound (185.6 mg, yield: 67.8%) as a colorless oil.
MS (ESI) m/z 1372 $(M+H)^+$

Device: LC Forte/R (YMC Co., Ltd.)
Column: XSerect HSS C18 19*150 mm, 5 μm
Flow rate: 17 mL/min.
Elution solvent: 0.1% aqueous solution of formic acid (Solution A) - 0.1% solution of formic acid in acetonitrile (Solution B)
Solution composition (Solution B): 50%

Example A3

[C341]

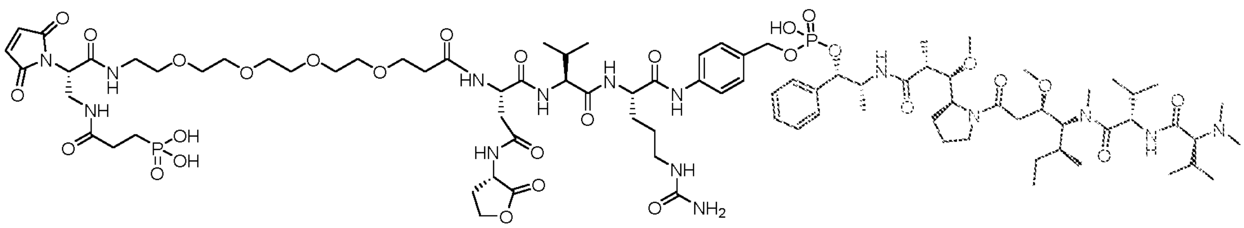

To a solution of the Example A3-6 (11.9 mg, 0.021 mmoL) in N,N-dimethylformamide (600 μL) in a 10 mL cylindrical flask was added triethylamine (9 μL, 6.53 mg, 0.065 mmoL) under argon gas flow with stirring, then HATU (8.7 mg, 0.023 mmoL) was added thereto, and the resulting mixture was stirred at room temperature for 5 minutes. Subsequently, a solution of the Example A3-7 (12.5 mg, 9.11 μmoL) in N,N-dimethylformamide (400 μL) was added thereto at room temperature, and the resulting mixture was stirred at room temperature for 3 hours.

After the reaction was completed, the reaction solution was concentrated under reduced pressure. The resulting residues were subjected to preparative HPLC under the following conditions, and the fraction comprising the target compound was freeze-dried to give the title compound (1.29 mg, yield: 7.37%) as white solids.
MS (ESI) m/z 961 $(M+2H)^{2+}$

Column: Waters XSELECT HSS T3 Prep 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 35% (6.00 min.) → 80% (6.50 min.) → 80% (9.00 min.)

Synthesis of Example A4

[C342]

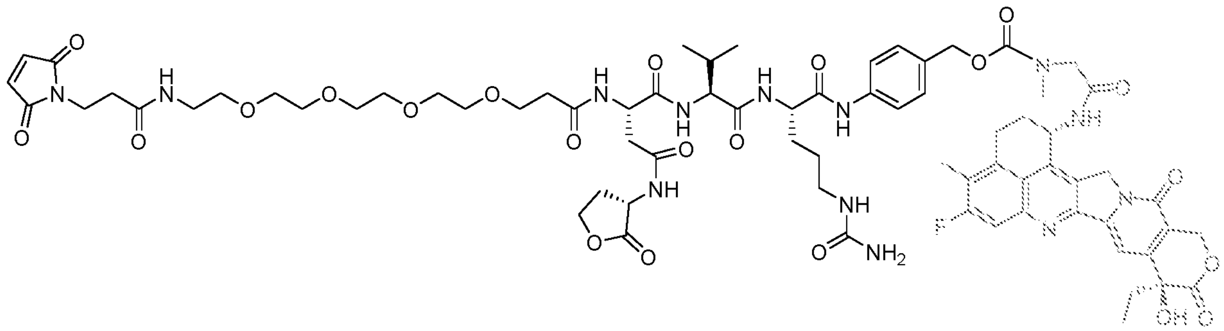

Example A4-1

[C343]

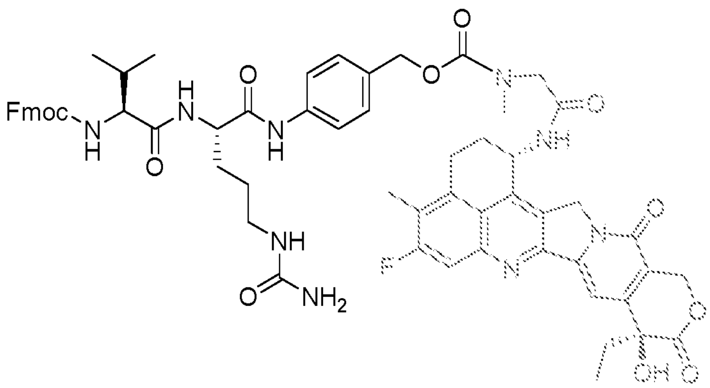

Fmoc-(L)-Val-(L)-Cit-PAB-PNP (manufactured by Angene) (263.5 mg, 0.344 mmoL) and formate of the free base of the U-003 (167.8 mg, 0.304 mmoL) instead of MMAE were reacted in the same manner as the Example A1-4 to give crude products of the title compound (390 mg, yield: quantitative) as light brown solids.
MS (ESI) m/z 1134 $(M+H)^+$

Example A4-2

[C344]

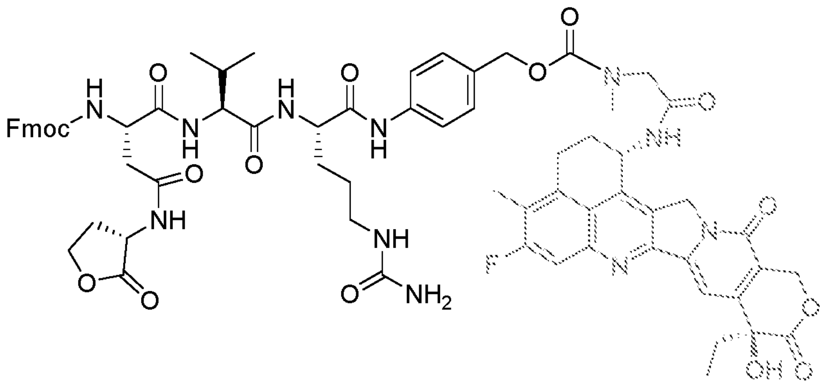

To a solution of the Example A4-1 (103.3 mg, 0.091 mmoL) in N,N-dimethylformamide (2 mL) in a 30 mL cylindrical flask was added piperidine (87 μL, 74.99 mg, 0.881 mmoL) under nitrogen airflow with stirring at room temperature, the resulting mixture was stirred at room temperature for 0.5 hour, and then the solvent was removed under reduced pressure.

To the resulting residues were added the Example A1-2 (115.5 mg, 0.263 mmoL), triethylamine (72 μL, 53.42 mg, 0.528 mmoL), N,N-dimethylformamide (2 mL), and HATU (106.4 mg, 0.280 mmoL) at room temperature, and the resulting mixture was stirred at room temperature for 10 minutes. Subsequently, the Example A1-2 (115.5 mg, 0.263 mmoL), triethylamine (72 μL, 53.42 mg, 0.528 mmoL), and HATU (106.4 mg, 0.280 mmoL) were added thereto at room temperature, and the resulting mixture was stirred at room temperature for 2 hours.

The reaction solution was subjected to preparative HPLC under the following conditions, and the fraction comprising the target compound was freeze-dried to give the title compound (57.1 mg, yield: 47.05%) as white solids. MS (ESI) m/z 1333 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 80% (10.00 min.)

Example A4

[C345]

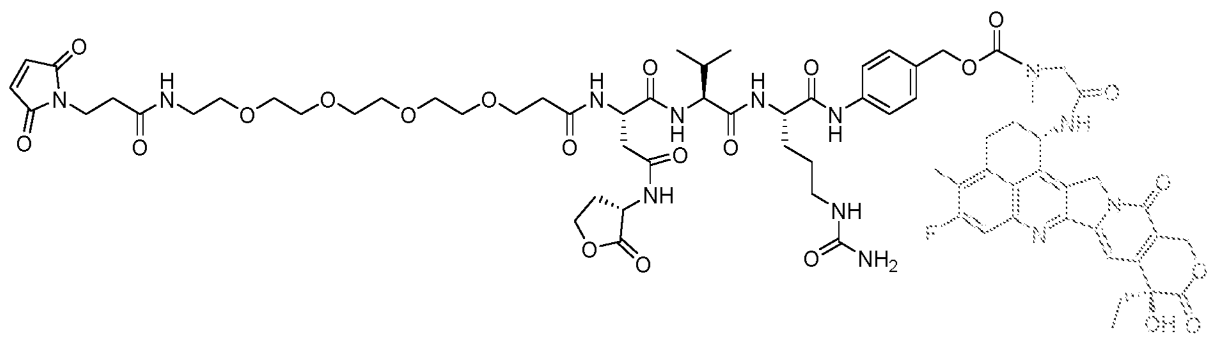

To a solution of the Example A4-2 (10.0 mg, 7.51 μmoL) in N,N-dimethylformamide (0.4 mL) in a 5 mL sample tube was added piperidine (4 μL, 3.44 mg, 0.040 mmoL) under nitrogen airflow with stirring at room temperature, the

resulting mixture was stirred at room temperature for 1 hour, and then the solvent was removed under reduced pressure.

To the resulting residues was added N,N-dimethylformamide (0.4 mL), a solution of N-succinimidyl 1-maleimide-3-oxo-7,10,13,16-tetraoxa-4-azanonadecanoate (15.4 mg, 0.030 mmoL) in acetonitrile (0.4 mL) was added thereto with stirring, and the resulting mixture was stirred for 1 hour. Subsequently, triethylamine (4 μL, 2.9 mg, 0.029 mmoL) was added thereto, and the resulting mixture was stirred for 1 hour.

After the reaction was completed, to the reaction solution was added a 50% aqueous solution of acetonitrile (3 mL), the resulting mixture was subjected to preparative HPLC under the following conditions, and the fraction comprising the target compound was freeze-dried to give the title compound (5.3 mg, yield: 46.81%) as colorless solids.
MS (ESI) m/z 755 $(M+2H)^{2+}$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 60% (10.00 min.)

Synthesis of Example A5

[C346]

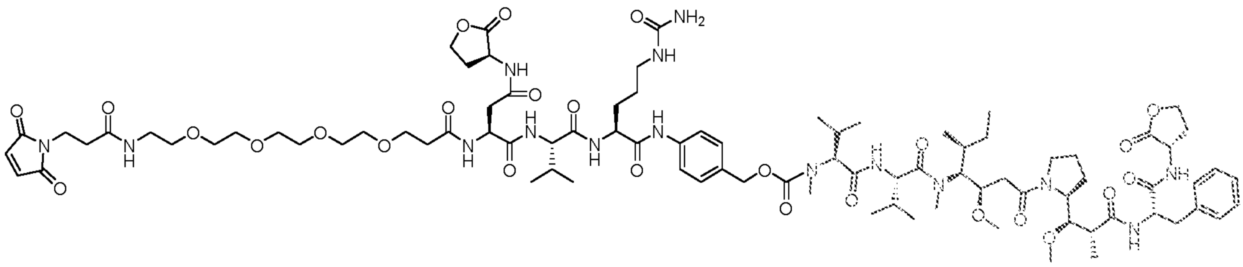

Example A5-1

[C347]

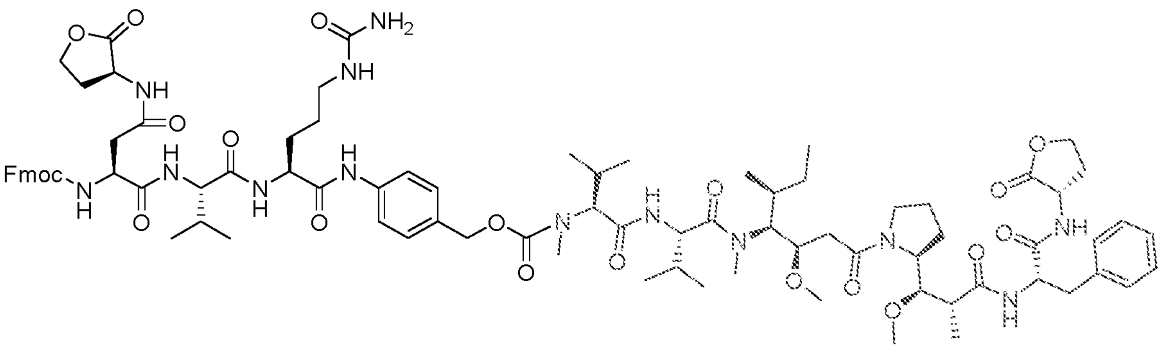

The Example A2-2 (343.0 mg, 0.356 mmoL) and the U-029 (290 mg, 0.356 mmoL) instead of the U-30 were reacted in the same manner as the Example A2-3 to give the title compound (49.7 mg, yield: 8.51%) as a colorless oil.
MS (DUIS) m/z 821 $(M+2H)^{2+}$

Example A5

[C348]

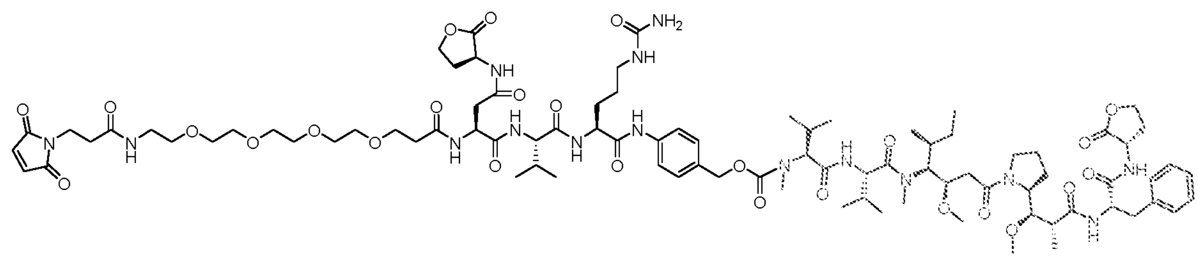

N-succinimidyl 1-maleimide-3-oxo-7,10,13,16-tetraoxa-4-azanonadecanoate (47 mg, 0.092 mmoL) and the Example A5-1 (25 mg, 0.015 mmoL) instead of the Example A4-2 were reacted in the same manner as the Example A4 to give the title compound (21.0 mg, yield: 75.86%) as white solids.
MS (DUIS) m/z 1815 $(M-H)^-$

Synthesis of Example A6

[C349]

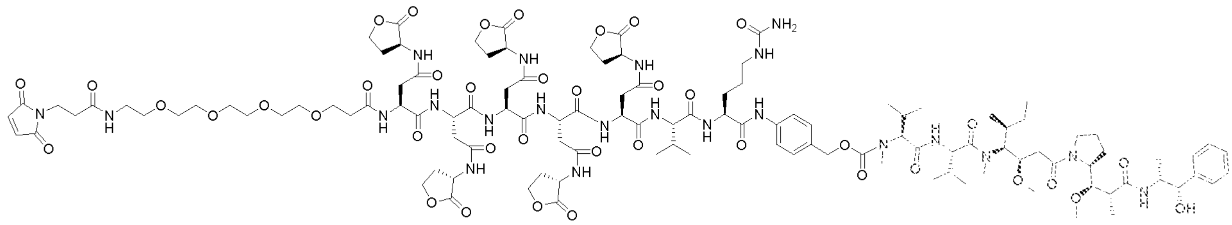

Example A6-1

[C350]

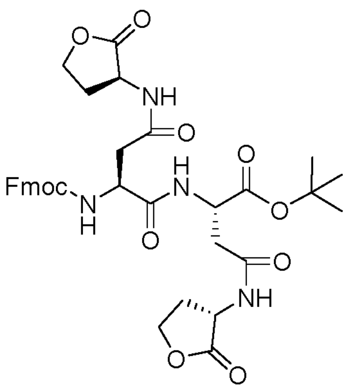

To a solution of the Example A1-1 (0.49 g, 0.991 mmoL) in N,N-dimethylformamide (5 mL) in a 20 mL cylindrical tube was added diazabicycloundecene (75 μL, 0.08 g, 0.498 mmoL) under nitrogen airflow with stirring at room temperature, and the resulting mixture was stirred at room temperature for 1 hour to prepare a Reaction solution A.

Separately, to a solution of the Example A1-2 (0.44 g, 1.004 mmoL) in N,N-dimethylformamide (3 mL) in a 20 mL cylindrical flask were added the above Reaction solution A, then triethylamine (0.28 mL, 0.2 g, 2.009 mmoL), and HATU (0.46 g, 1.210 mmoL) under nitrogen airflow with stirring at room temperature, and the resulting mixture was stirred at room temperature for 1 hour.

After the reaction was completed, to the reaction solution was added tert-butyl methyl ether (8 mL), 1N hydrochloric acid (2.5 mL) and then water (8 mL) were added thereto, and the resulting mixture was stirred at room temperature for 10 minutes. The resulting precipitates were filtered, washed with water, and washed with tert-butyl methyl ether to give the title compound (0.53 g, yield: 77.22%) as colorless solids.
MS (ESI) m/z 693 $(M+H)^+$

Example A6-2

[C351]

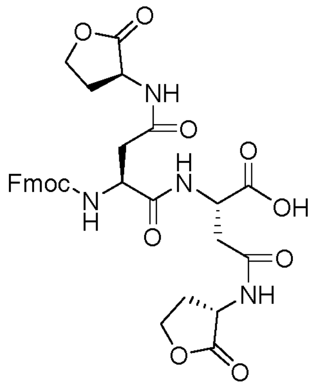

The Example A6-1 (0.21 g, 0.303 mmoL) in a 30 mL cylindrical tube was dissolved in dichloromethane (2 mL), then trifluoroacetic acid (2 mL) was added thereto, and the resulting mixture was stirred at room temperature for 1 hour.
After the reaction was completed, the solvent was distilled away under reduced pressure. To the resulting residues was added acetonitrile (3 mL), the precipitated solids were collected by filtration, and washed with acetonitrile to give the title compound (0.21 g, yield: quantitative) as colorless solids.
MS (ESI) m/z 637 $(M+H)^+$

Example A6-3

[C352]

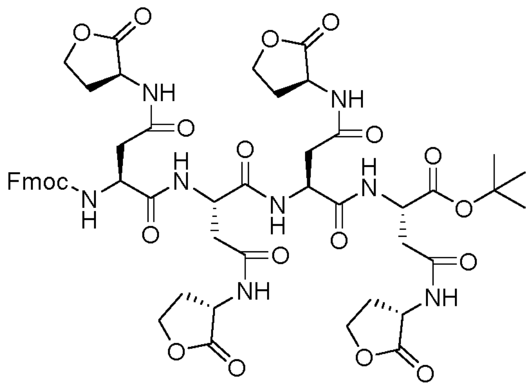

To a solution of the Example A6-1 (0.21 g, 0.303 mmoL) in N,N-dimethylformamide (2 mL) in a 20 mL cylindrical tube was added diazabicycloundecene (23 μL, 0.02 g, 0.153 mmoL) under nitrogen airflow with stirring, and the resulting mixture was stirred at room temperature for 1 hour to prepare a Reaction solution A.
To a solution of the Example A6-2 (0.22 g, 0.306 mmoL) in N,N-dimethylformamide (2 mL) in another 20 mL cylindrical tube were added the Reaction solution A, triethylamine (85 μL, 0.06 g, 0.610 mmoL), and HATU (0.14 g, 0.368 mmoL) under nitrogen airflow with stirring at room temperature, and the resulting mixture was stirred at room temperature

for 1 hour.

After the reaction was completed, to the reaction solution were added tert-butyl methyl ether (10 mL), 1N hydrochloric acid (0.76 mL), and water (10 mL), and the resulting mixture was stirred at room temperature for 10 minutes. The precipitated solids were filtered, washed with water and tert-butyl methyl ether, and dried to give the title compound (0.31 g, yield: 93.89%) as colorless solids.
MS (ESI) m/z 1089 $(M+H)^+$

Example A6-4

[C353]

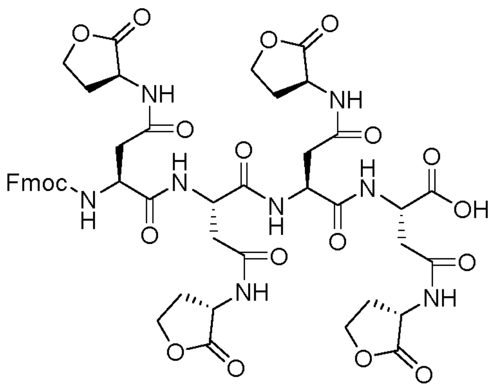

To the Example A6-3 (10.0 mg, 9.18 μmoL) in a 30 mL cylindrical tube was added trifluoroacetic acid (0.1 mL) under nitrogen airflow with stirring at room temperature, and the resulting mixture was stirred at room temperature for 1 hour.

After the reaction was completed, the solvent was concentrated under reduced pressure. To the resulting residues was added acetonitrile (0.3 mL), the precipitated solids were filtered, washed with acetonitrile, and dried to give the title compound (8.4 mg, yield: 88.56%) as colorless solids.
MS (ESI) m/z 1033 $(M+H)^+$

Example A6-5

[C354]

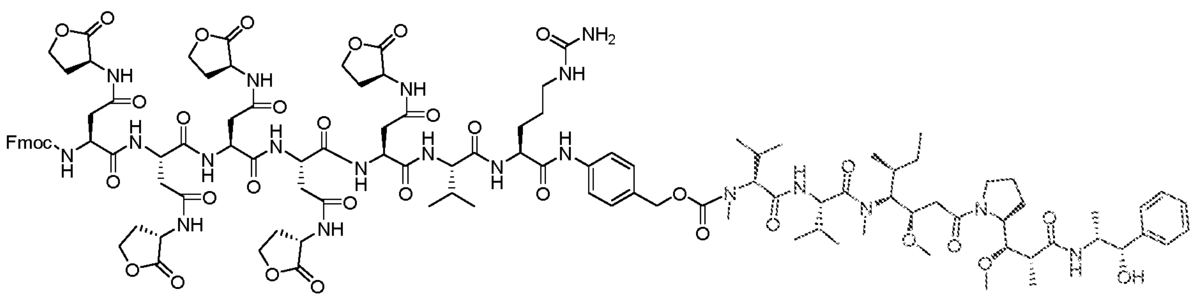

The Example A1-7 (6.9 mg, 5.22 μmoL) instead of the Example A1-5 and the Example A6-4 (6.6 mg, 6.39 μmoL) instead of the Example A1-2 were reacted in the same manner as the Example A1-7 to give the title compound (6.76 mg, yield: 55.41%) as colorless foam.
MS (ESI) m/z 1169 $(M+2H)^{2+}$

Example A6

[C355]

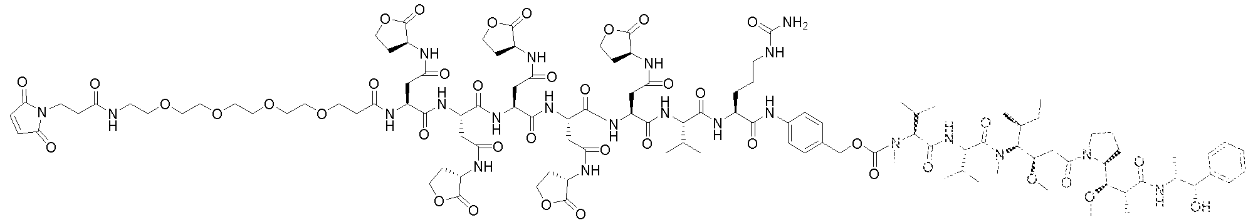

N-succinimidyl 1-maleimide-3-oxo-7,10,13,16-tetraoxa-4-azanonadecanoate (7.2 mg, 0.014 mmoL) and the Example A6-5 (5.3 mg, 2.268 μmoL) instead of the Example A4-2 were reacted in the same manner as the Example A4 to give the title compound (2.62 mg, yield: 45.97%) as colorless solids.
MS (ESI) m/z 1257 $(M+2H)^{2+}$

Synthesis of Example A7

[C356]

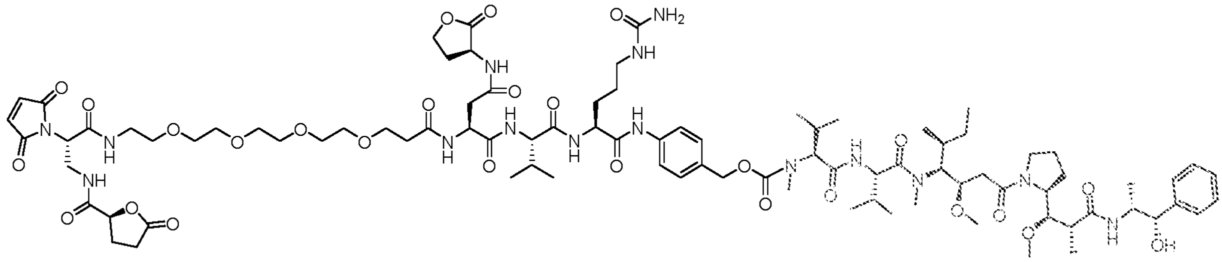

Example A7-1

[C357]

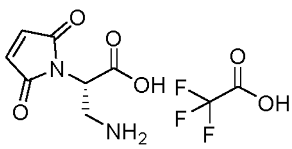

To a solution of (S)-3-((tert-butoxycarbonyl)amino)-2-(2,5-dioxo-2,5-dihydro-1H-pyrrol-1-yl)propanoic acid (i.e., (L)-3-((tert-butoxycarbonyl)amino)-2-(2,5-dioxo-2,5-dihydro-1H-pyrrol-1-yl)propanoic acid) (1.20 g, 4.22 mmoL) (disclosed in WO 2019/195665 pamphlet) in dichloromethane (50 mL) in a 100 mL round-bottom flask was added trifluoroacetic acid (3.24 mL) under nitrogen atmosphere with stirring at room temperature, and the resulting mixture was stirred at room temperature for 4 hours.

After the reaction was completed, the reaction solution was concentrated under reduced pressure, diethyl ether (20 mL) was added thereto, the precipitated solids were filtered, washed with diethyl ether, and dried to give the title compound (0.9734 g, yield: 77.33%) as beige solids.
MS (ESI) m/z 185 $(M+H)^+$

Example A7-2

[C358]

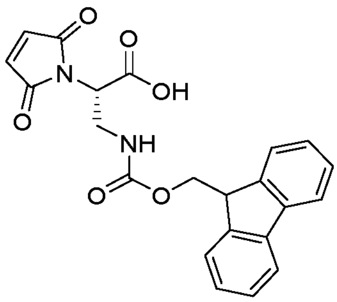

To a solution of the Example A7-1 (591 mg, 1.982 mmoL) and (9H-fluoren-9-yl)methyl carbonochloridate (536 mg, 2.072 mmoL) in tetrahydrofuran (10 mL) in a 100 mL round-bottom flask was added an aqueous solution (5 mL) containing sodium hydrogen carbonate (973 mg, 11.58 mmoL) under air atmosphere with stirring at 0°C, and the resulting mixture was stirred at room temperature for 1 hour.

After the reaction was completed, to the reaction solution was added a 10% by weight aqueous solution of citric acid, and the resulting mixture was subjected to extraction with ethyl acetate. The resulting residues were subjected to YAMAZEN medium pressure flash chromatography (Silica, L (40 g), ethyl acetate / methanol = 100/0 (V/V) → 60/40 (V/V) (Rf = 0.13 (ethyl acetate / methanol = 95/5 (V/V)))), and the fraction comprising the target compound was concentrated under reduced pressure to give the title compound (761 mg, yield: 94.48%) as white foam.
MS (ESI) m/z 407 $(M+H)^+$

Example A7-3

[C359]

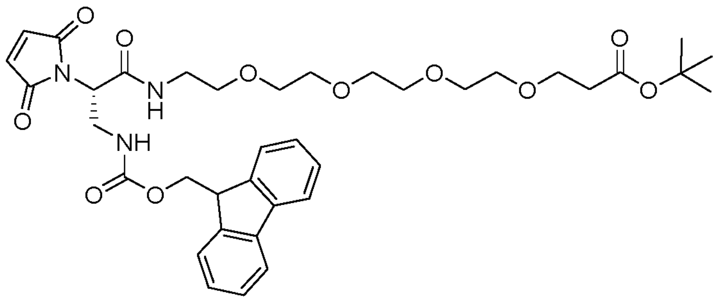

Tert-butyl 1-amino-3,6,9,12-tetraoxapentadecan-15-oate (477 mg, 1.484 mmoL) and the Example A7-2 (692 mg, 1.703 mmoL) instead of the Example A3-4 were reacted in the same manner as the Example A3-5 to give the title compound (918 mg, yield: 87.15%) as an orange oil.
MS (ESI) m/z 710 $(M+H)^+$

Example A7-4

[C360]

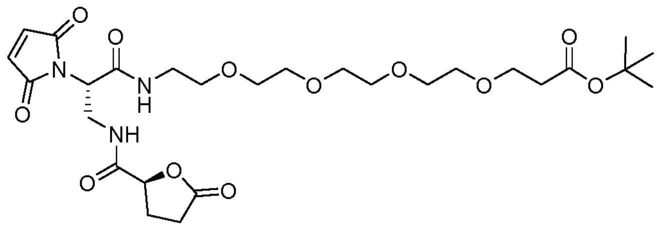

To a solution of the Example A7-3 (780 mg, 1.099 mmoL) in dichloromethane (12 mL) in a 30 mL cylindrical flask was added diazabicycloundecene (75 μL, 75.75 mg, 0.498 mmoL) under argon gas flow with stirring at room temperature, and the resulting mixture was stirred at room temperature for 40 minutes to prepare a Reaction solution A.

To a solution of (S)-5-oxotetrahydrofuran-2-carboxylic acid (211 mg, 1.622 mmoL) and 1-hydroxybenzotriazole (223 mg, 1.650 mmoL) in dichloromethane (12 mL) in another 100 mL round-bottom flask were added the above Reaction solution A and 1-ethyl-3-(3-dimethylaminopropyl)carbodiimide hydrochloride (320 mg, 1.669 mmoL) under argon gas flow with stirring at room temperature, and the resulting mixture was stirred at room temperature for 1 hour.

After the reaction was completed, to the reaction solution was added a 5% by weight aqueous solution of citric acid (10 mL), and the resulting mixture was subjected to extraction with methylene chloride. Subsequently, the reaction mixture was washed with saturated brine (5 mL), dried over anhydrous magnesium sulfate, filtered, and concentrated under reduced pressure.

The resulting residues were subjected to YAMAZEN medium pressure flash chromatography (COOH, M (16 g) (biconnected), dichloroethane/methanol = 100/0 (V/V) → 92/8 (V/V)), and the fraction comprising the target compound was concentrated under reduced pressure to give the title compound (187 mg, yield: 28.38%) as a light brown oil.
MS (ESI) m/z 598 $(M-H)^-$

Example A7-5

[C361]

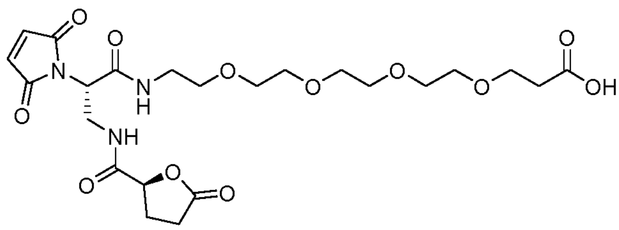

The Example A7-4 (21 mg, 0.035 mmoL) instead of the Example A3-5 was reacted in the same manner as the Example A3-6 to give the title compound (20 mg, yield: quantitative) as a slightly yellow oil.
MS (ESI) m/z 544 $(M+H)^+$

Example A7

[C362]

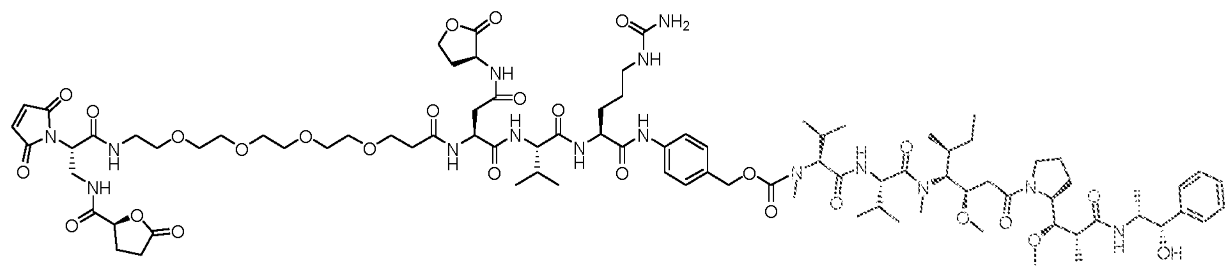

The Example A1-7 (18 mg, 0.014 mmoL) instead of the Example A3-7 and the Example A7-5 (20 mg, 0.037 mmoL) instead of the Example A3-6 were reacted in the same manner as the Example A3 to give the title compound (14.2 mg, yield: 56.44%) as white solids.
MS (ESI) m/z 924 $(M+2H)^{2+}$

Synthesis of Example A8

[C363]

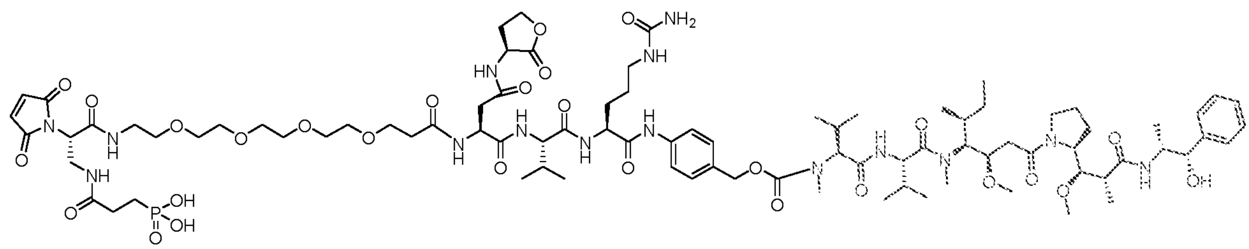

The Example A3-6 (6.10 mg, 10.75 μmoL) and the Example A1-7 (14.33 mg, 10.84 μmoL) instead of the Example A3-7 were reacted in the same manner as the Example A3 to give the title compound (5.91 mg, yield: 29.38%) as white solids.
MS (ESI) m/z 936 $(M+2H)^{2+}$

Synthesis of Example A9

[C364]

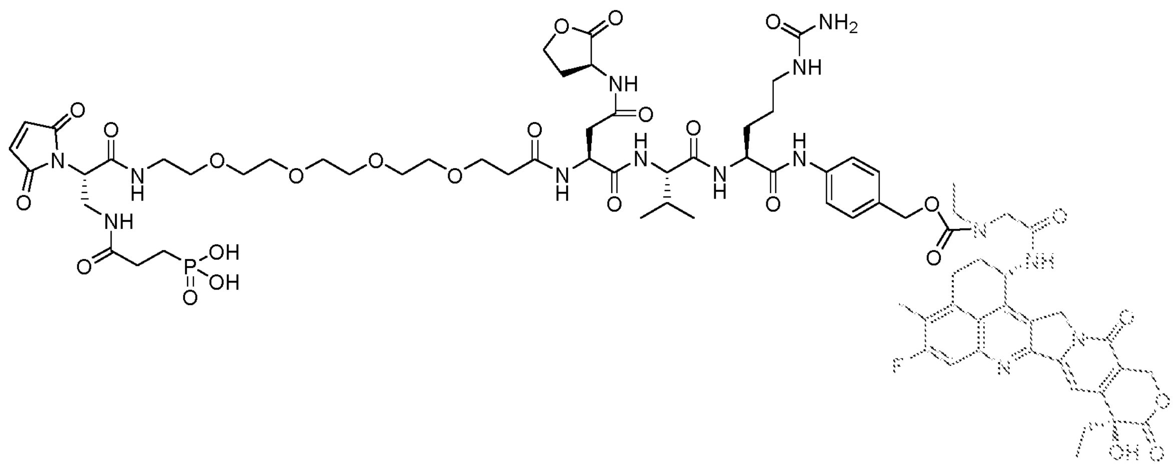

Example A9-1

[C365]

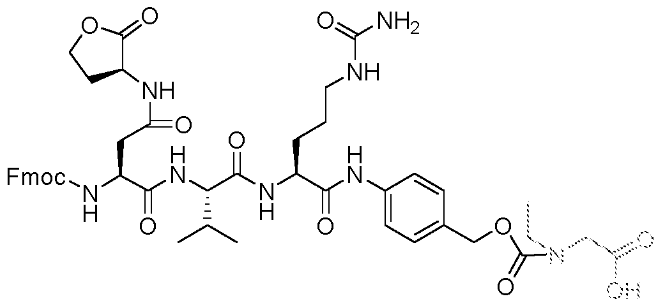

The Example A2-2 (198.0 mg, 0.205 mmoL) and ethylglycine (25.9 mg, 0.251 mmoL) instead of the U-030 were reacted in the same manner as the Example A2-3 to give the title compound (80.5 mg, yield: 42.23%) as slightly yellow solids.
MS (ESI) m/z 929 $(M+H)^+$

Example A9-2

[C366]

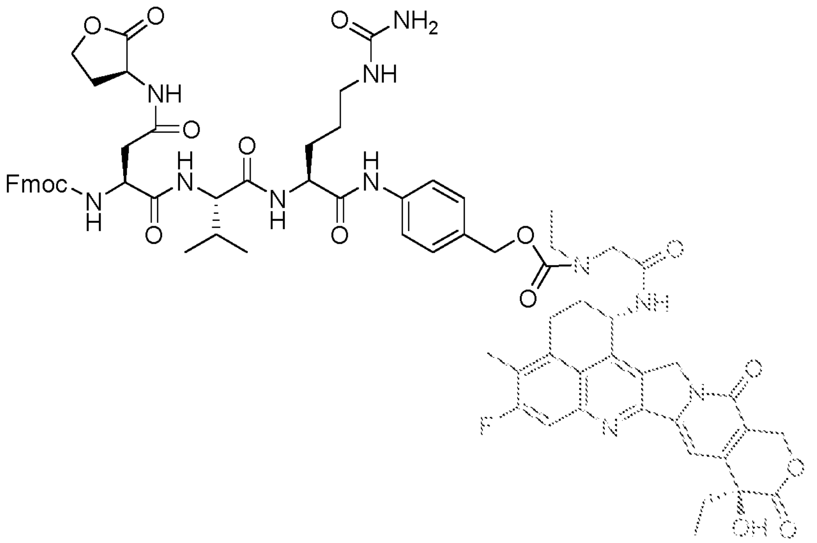

To a solution of exatecan mesylate (65.3 mg, 0.123 mmoL), the Example A9-1 (125 mg, 0.135 mmoL), 1-ethyl-3-(3-dimethylaminopropyl)carbodiimide hydrochloride (46.9 mg, 0.245 mmoL), and 1-hydroxybenzotriazole (18.7 mg, 0.122 mmoL) in N,N-dimethylformamide (0.2 mL) in a 10 mL cylindrical flask was added triethylamine (0.034 mL, 24.68 mg, 0.244 mmoL) under argon atmosphere with stirring at room temperature, and the resulting mixture was stirred at room temperature for 12 hours.
The resulting reaction solution was subjected to preparative HPLC under the following conditions, the fraction comprising the target compound was concentrated under reduced pressure to distill away acetonitrile, and then the resulting residues were freeze-dried to give the title compound (80.3 mg, yield: 48.55%) as colorless solids.
MS (DUIS) m/z 673 $(M+2H)^{2+}$

Column: Waters SunFire Prep C18 5 μm ODB 19*150 mm

Flow rate: 17 mL/min.

Elution solvent: 0.1% aqueous solution of formic acid (Solution A) - acetonitrile (Solution B)

Gradient (Solution B): 50% (0.00 min.) → 62% (6.00 min.)

Example A9

[C367]

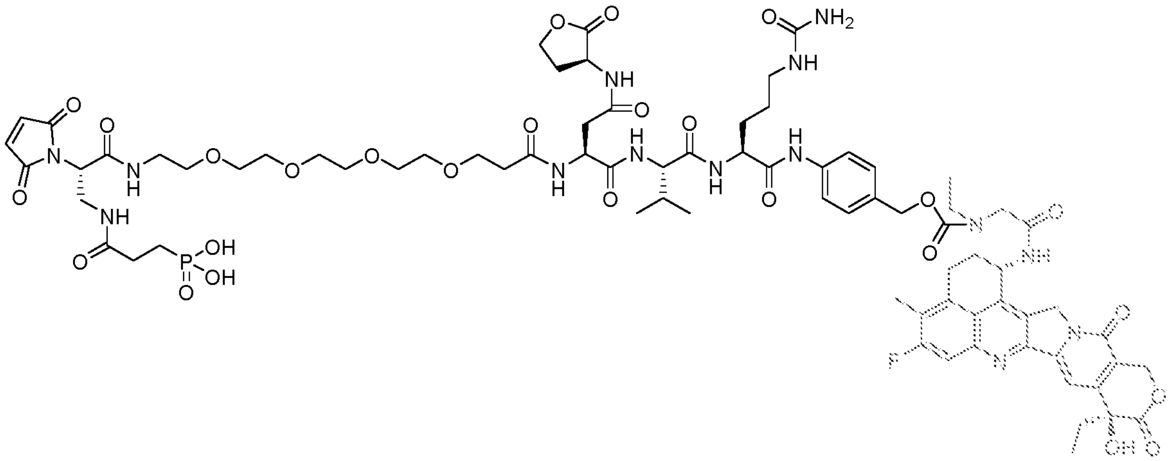

To a solution of the Example A9-2 (12.1 mg, 8.99 μmoL) in N,N-dimethylformamide (0.8 mL) in a 5 mL sample tube was added piperidine (1.74 μL, 1.5 mg, 0.018 mmoL) under airflow with stirring at room temperature, the resulting mixture was stirred at room temperature for 1 hour, and then the solvent was removed under reduced pressure.

To the resulting residues were added N,N-dimethylformamide (0.8 mL), the Example A3-6 (10.1 mg, 0.018 mmoL), HATU (8.2 mg, 0.022 mmoL), and N,N-diisopropylethylamine (11 μL, 8.14 mg, 0.063 mmoL) with stirring, and the resulting mixture was stirred at room temperature for 30 minutes.

After the reaction was completed, to the reaction solution was added a 50% aqueous solution of acetonitrile (5 mL), the resulting mixture was subjected to preparative HPLC under the following conditions, and the fraction comprising the target compound was freeze-dried to give the title compound (4.7 mg, yield: 31.25%) as white solids.
MS (ESI) m/z 837 $(M+2H)^{2+}$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 20% (2.00 min.) → 70% (8.00 min.)

Synthesis of Example A10

[C368]

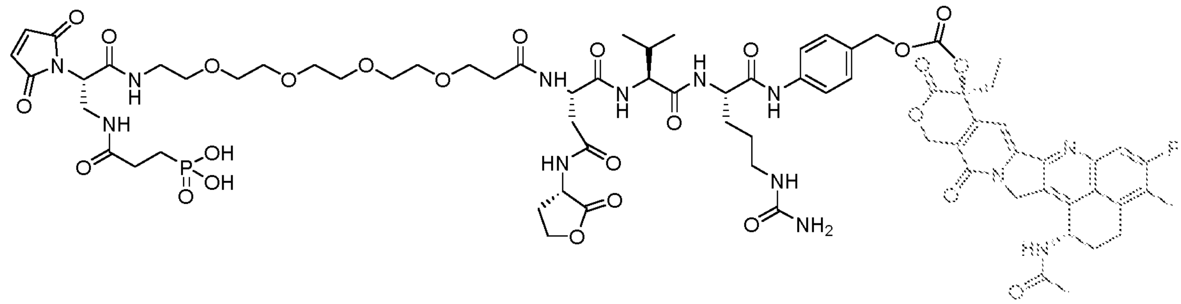

Example A10-1

[C369]

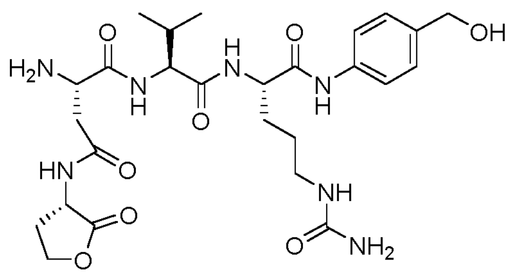

To a solution of the Example A2-1 (4.7 g, 5.88 mmoL) in N,N-dimethylformamide (50 mL) in a 200 mL round-bottom flask was added piperidine (1.45 mL, 1.25 g, 14.64 mmoL) under argon gas flow with stirring at room temperature, and the resulting mixture was stirred at room temperature for 1 hour. Subsequently, piperidine (0.29 mL, 0.25 g, 2.93 mmoL) was added thereto under stirring at room temperature, the resulting mixture was stirred at room temperature for 2 hours, and then the solvent was removed under reduced pressure.

After the reaction was completed, to the resulting residues were added ethyl acetate and diethyl ether, the resulting solids were collected by filtration, and dried under reduced pressure at 50°C for 4 hours to give the title compound (4.98 g, yield: quantitative) as white solids.
MS (ESI) m/z 578 $(M+H)^+$

Example A10-2

[C370]

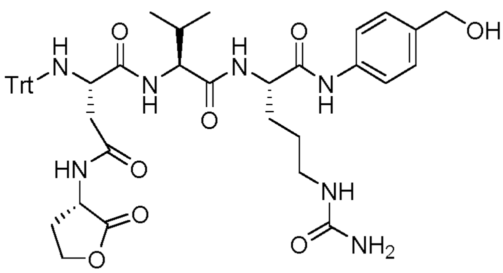

To a solution of the Example A10-1 (4.24 g, 7.34 mmoL) and potassium carbonate anhydrous (1.52 g, 11.00 mmoL) in N,N-dimethylformamide (60 mL) in a 300 mL round-bottom flask was added water (0.3 mL). Trityl chloride (1.03 g, 3.69 mmoL) was added thereto under air atmosphere with stirring at room temperature, and the resulting mixture was

stirred at room temperature for 0.5 hour. Subsequently, trityl chloride (1.03 g, 3.69 mmoL) was added thereto under stirring at room temperature, and the resulting mixture was stirred at room temperature for 0.5 hour. Subsequently, trityl chloride (1.01 g, 3.62 mmoL) was added thereto under stirring at room temperature, and the resulting mixture was stirred at room temperature for 0.5 hour. Subsequently, water (0.3 mL) and trityl chloride (0.496 g, 1.779 mmoL) were added thereto under stirring at room temperature, and the resulting mixture was stirred at room temperature for 15 minutes.

To the reaction solution were added ethyl acetate (100 mL), diethyl ether (50 mL), tetrahydrofuran (25 mL), then water (75 mL), and saturated brine (25 mL), the resulting mixture was stirred, and separated. The resulting aqueous layer was concentrated, and then the resulting residues were subjected to extraction with ethyl acetate again. The above resulting organic layers were combined, washed with saturated brine, dried over anhydrous magnesium sulfate, filtered, and concentrated under reduced pressure.

To the resulting residues were added tetrahydrofuran, ethyl acetate, diethyl ether, and hexane, the resulting solids were collected by filtration, and washed with tert-butyl methyl ether to give the title compound (2.69 g, yield: 44.69%) as white solids.

MS (ESI) m/z 820 $(M+H)^+$

Example A10-3

[C371]

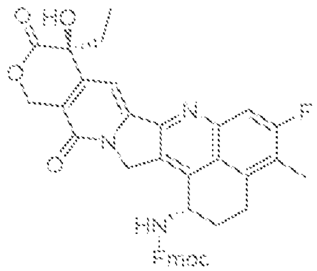

To a solution of exatecan mesylate (1.60 g, 3.01 mmoL) in a mixture of acetonitrile (90 mL) and water (30 mL) in a 300 mL round-bottom flask were added sodium hydrogen carbonate (1.26 g, 15.00 mmoL) and (9H-fluoren-9-yl)methyl carbonochloridate (0.93 g, 3.59 mmoL) under nitrogen atmosphere with stirring at room temperature, and the resulting mixture was stirred at room temperature for 2.5 hours.

After the reaction was completed, to the reaction solution was added water (150 mL), and the resulting mixture was stirred under ice-cooling for 10 minutes. The precipitated solids were separated, and dried under reduced pressure to give the title compound (1.91 g, yield: 96.48%) as slightly yellow solids.

MS (ESI) m/z 658 $(M+H)^+$

Example A10-4

[C372]

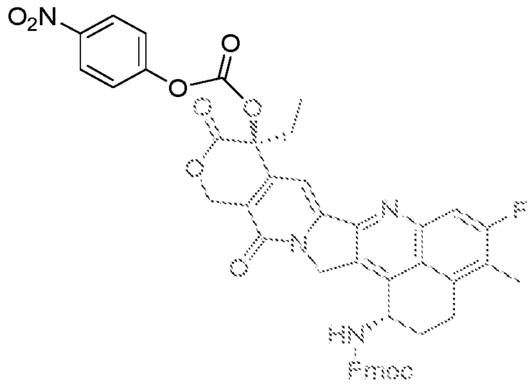

To a solution of the Example A10-3 (6.44 g, 79 mmoL) in dichloromethane (200 mL) in a 500 mL round-bottom flask were added 4-nitrophenyl carbonochloridate (5.92 g, 29.4 mmoL), triethylamine (5.45 mL, 3.96 g, 39.1 mmoL), and 4-dimethylaminopyridine (1.79 g, 14.65 mmoL) under nitrogen atmosphere with stirring at room temperature, and the resulting mixture was stirred at room temperature for 4 hours.

After the reaction was completed, to the reaction solution were added 0.2N hydrochloric acid (120 mL), dichloromethane (150 mL), and water (100 mL), the resulting mixture was filtered through Celite, and the resulting organic layer was separated.

The resulting organic layer was dried over anhydrous magnesium sulfate, filtered, and concentrated under reduced pressure. The resulting residues were subjected to YAMAZEN medium pressure flash chromatography (Silica, L (40 g), hexane / ethyl acetate = 50/50 (V/V) → 0/100 (V/V)), and the fraction comprising the target compound was concentrated under reduced pressure to give the title compound (5.06 g, yield: 62.81%) as brown solids.
MS (ESI) m/z 823 $(M+H)^+$

Example A10-5

[C373]

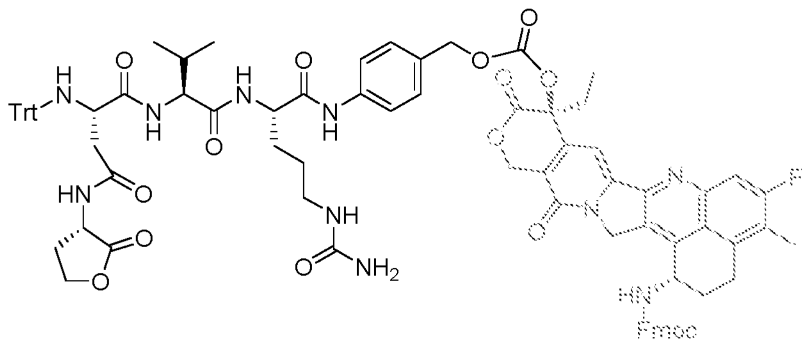

To a solution of the Example A10-4 (3.56 g, 4.33 mmoL) in dichloromethane (108 mL) in a 500 mL round-bottom flask were added the Example A10-2 (3.25 g, 3.96 mmoL), triethylamine (1.22 mL, 0.89 g, 8.75 mmoL), and 4-dimethylaminopyridine (1.02 g, 8.35 mmoL) under argon gas flow with stirring at room temperature, and the resulting mixture was stirred at room temperature for 7.5 hours.

After the reaction was completed, to the reaction solution was added saturated brine, the resulting mixed solution was subjected to extraction with methylene chloride, and the resulting organic layer was concentrated under reduced pressure.

The resulting residues were subjected to YAMAZEN medium pressure flash chromatography (Silica, 3L (135 g), ethyl acetate / methanol = 100/0 (V/V) → 70/30 (V/V) (Rf = 0.22 (ethyl acetate / methanol = 90/10 (V/V)))), and the fraction comprising the target compound was concentrated under reduced pressure to give the title compound (1.86 g, yield: 31.21%) as slightly yellow solids
MS (ESI) m/z 1262 $(M+H-Trt)^+$

Example A10-6

[C374]

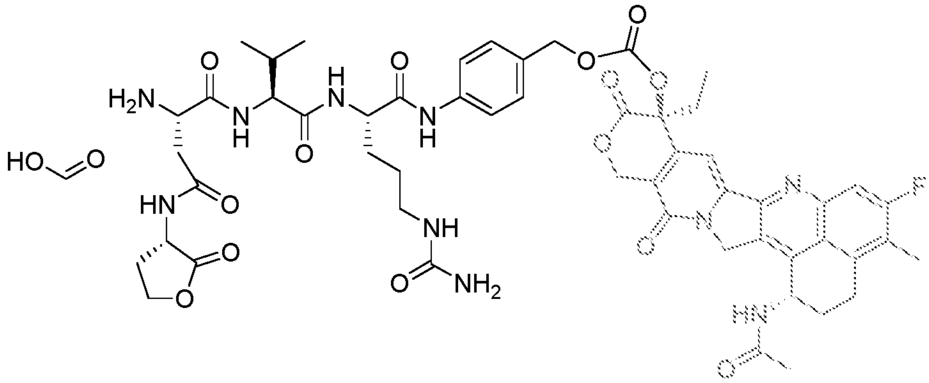

To a solution of the Example A10-5 (15 mg, 9.98 μmoL) in N,N-dimethylformamide (1 mL) in a 10 mL cylindrical flask was added piperidine (10 μL, 8.6 mg, 0.101 mmoL) under air atmosphere with stirring at room temperature, and the resulting mixture was stirred at room temperature for 1 hour.

The reaction solution was concentrated under reduced pressure. To the resulting residues was added dichloromethane (1 mL), triethylamine (4 μL, 2.9 mg, 0.029 mmoL) and then acetic anhydride (3.0 μL, 3.24 mg, 0.032 mmoL) were added thereto under stirring at room temperature, and the resulting mixture was stirred at room temperature for 36 hours.

The solvent was concentrated under reduced pressure, and to the resulting residues was added N,N-dimethylformamide (1 mL). Subsequently, formic acid (0.50 mL, 600 mg, 13.04 mmoL) was added thereto under stirring at room temperature, and the resulting mixture was stirred at room temperature for 1 hour.

The resulting residues were subjected to preparative HPLC under the following conditions, and the fraction comprising the target compound was freeze-dried to give the title compound (4.9 mg, yield: 43.58%) as colorless solids. MS (ESI) m/z 1081 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 60% (10.00 min.)

Example A10

[C375]

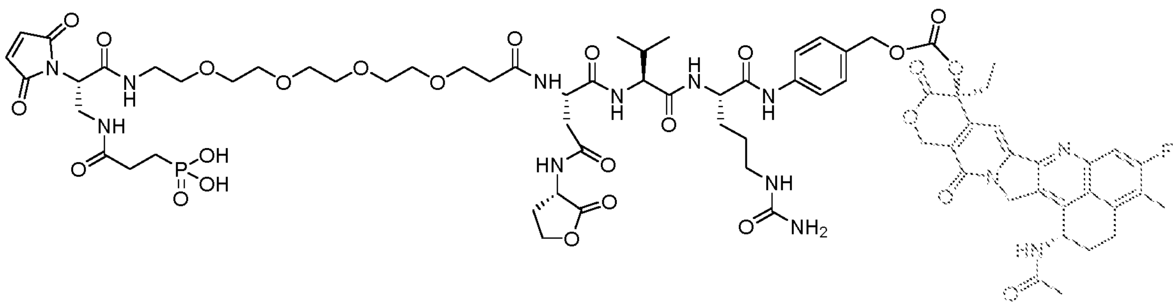

The Example A3-6 (16.1 mg, 0.028 mmoL) and the Example A10-6 (4.9 mg, 4.35 μmoL) instead of the Example A3-7 were reacted in the same manner as the Example A3 to give the title compound (4.1 mg, yield: 57.84%) as white

solids.
MS (ESI) m/z 816 $(M+2H)^{2+}$

Synthesis of Example A11

[C376]

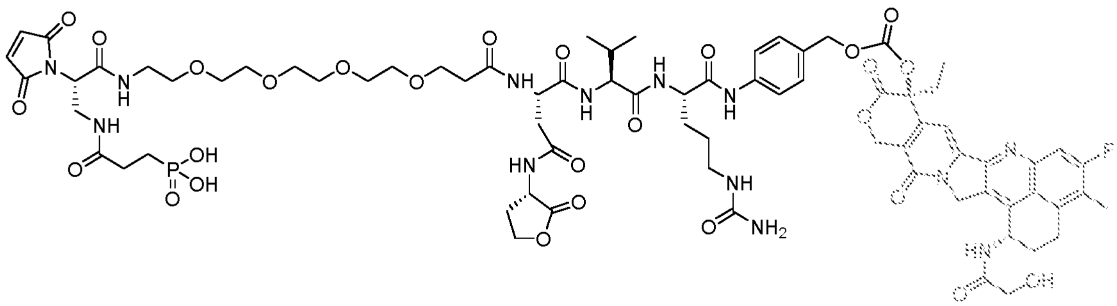

Example A11-1

[C377]

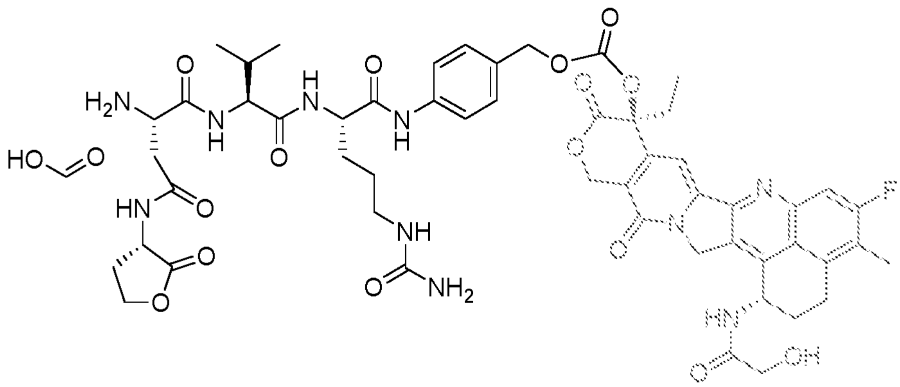

To a solution of the Example A10-5 (66.8 mg, 0.044 mmoL) in N,N-dimethylformamide (2 mL) in a 30 mL pear-shaped flask was added piperidine (22 μL, 18.92 mg, 0.222 mmoL) under air atmosphere with stirring at room temperature, and the resulting mixture was stirred at room temperature for 1 hour.

The reaction solution was concentrated under reduced pressure. To the resulting residues was added N,N-dimethylformamide (2 mL), triethylamine (19 μL, 13.79 mg, 0.136 mmoL), and then 2-hydroxyacetic acid (10.1 mg, 0.133 mmoL) and HATU (50.7 mg, 0.133 mmoL) were added thereto under stirring at room temperature, and the resulting mixture was stirred at room temperature for 3 hours.

Subsequently, formic acid (1 mL, 1200 mg, 26.1 mmoL) was added thereto at room temperature, and the resulting mixture was stirred at room temperature for 1 hour.

The resulting residues were subjected to preparative HPLC under the following conditions, and the fraction comprising the target compound was freeze-dried to give the title compound (30.6 mg, yield: 60.25%) as light brown solids.
MS (ESI) m/z 1097 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 60% (10.00 min.)

Example A11

[C378]

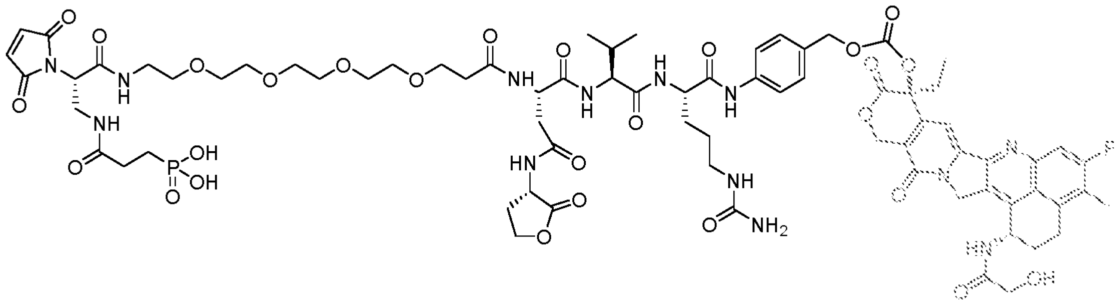

The Example A3-6 (16.5 mg, 0.029 mmoL) and the Example A11-1 (6.6 mg, 5.77 μmoL) instead of the Example A3-7 were reacted in the same manner as the Example A3 to give the title compound (3.6 mg, yield: 37.87%) as white solids.
MS (ESI) m/z 824 $(M+2H)^{2+}$

Synthesis of Example A12

[C379]

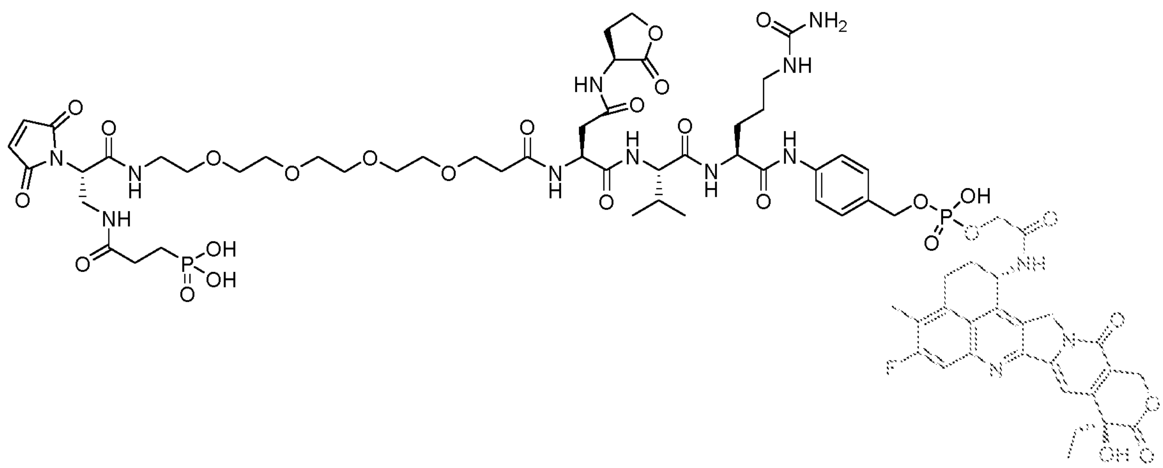

Example A12-1

[C380]

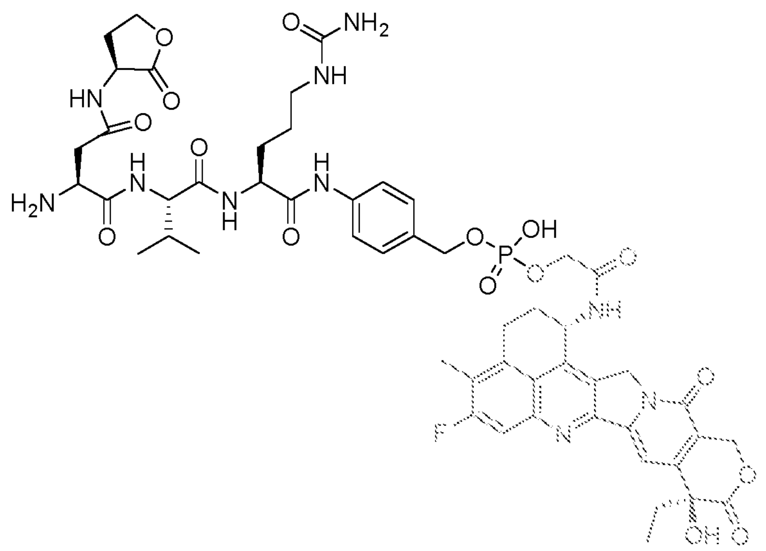

To a solution of the Example A2-1 (162.2 mg, 0.203 mmoL) in N,N-dimethylformamide (3 mL) in a 50 mL pear-shaped flask were added 2-cyanoethyl N,N,N',N'-tetraisopropylphosphordiamidite (130 μL, 123.5 mg, 0.410 mmoL) and 1H-tetrazole (29.8 mg, 0.425 mmoL) under argon atmosphere with stirring at room temperature, and the resulting mixture was stirred at room temperature for 15 minutes. Subsequently, triisopropylsilyl trifluoromethanesulfonate (110 μL, 125.4 mg, 0.409 mmoL) was added thereto at room temperature, and the resulting mixture was stirred at room temperature for 30 minutes.

Subsequently, DXd (100.7 mg, 0.204 mmoL) and 5-(ethylthio)-1H-tetrazole (53.8 mg, 0.413 mmoL) were added thereto, and the resulting mixture was stirred at room temperature for 1 hour. Subsequently, a 70% aqueous solution of tert-butyl hydroperoxide (170 μL, 159.8 mg, 1.241 mmoL) was added thereto at room temperature, and the resulting mixture was stirred at room temperature for 1.6 hours.

Subsequently, diazabicycloundecene (300 μL, 306 mg, 2.01 mmoL) was added thereto under ice-cooling, and the resulting mixture was stirred at room temperature for 1.5 hours.

After the reaction was completed, diethyl ether (30 mL) was added thereto, and the resulting supernatant solution was removed. Subsequently, diethyl ether (15 mL) was added thereto, and the resulting supernatant solution was removed. Subsequently, diethyl ether (15 mL) was added thereto, and the resulting supernatant solution was removed. Subsequently, acetonitrile (20 mL) was added thereto, and the resulting supernatant solution was removed. Subsequently, acetonitrile (10 mL) was added thereto, and the resulting supernatant solution was removed. Subsequently, diethyl ether (20 mL) was added thereto, the resulting supernatant solution was removed, and dried under reduced pressure to give crude products of the title compound. The crude products were purified under the following recycle preparative conditions, and the fraction comprising the target compound was concentrated under reduced pressure to give the title compound (39.8 mg, yield: 17.32%) as beige solids.
MS (ESI) m/z 1133 $(M+H)^+$

Device: LC Forte/R (YMC Co., Ltd.)
Column: XBridge Prep C18 5 μm ODB 19*150 mm
Flow rate: 25 mL/min.
Elution solvent: 0.1% aqueous solution of formic acid (Solution A) - 0.1% solution of formic acid in acetonitrile (Solution B)
Solution composition (Solution B): 40%

Example A12

[C381]

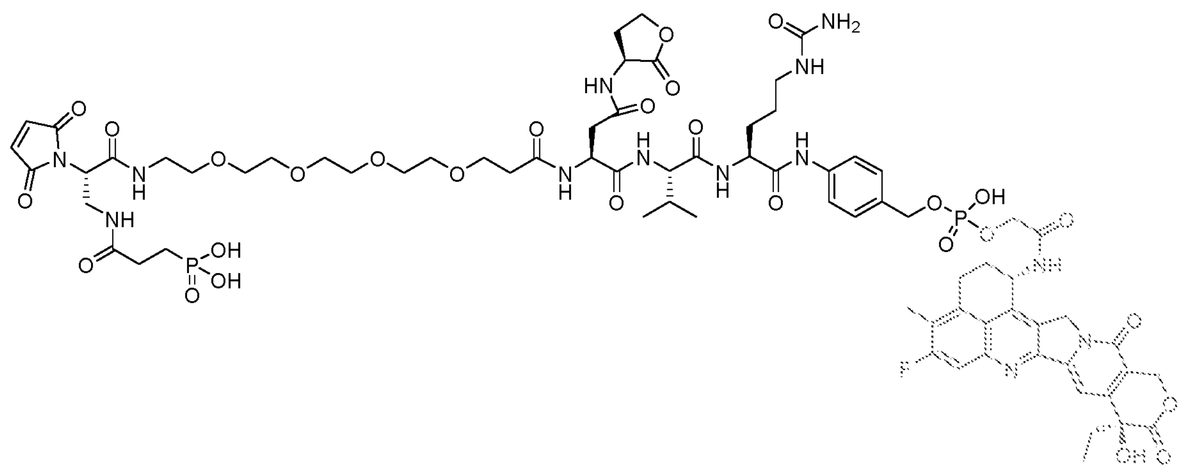

The Example A3-6 (13.2 mg, 0.023 mmoL) and the Example A12-1 (11.5 mg, 10.15 μmoL) instead of the Example A3-7 were reacted in the same manner as the Example A3 to give the title compound (5.78 mg, yield: 33.85%) as white solids.
MS (ESI) m/z 842 $(M+2H)^{2+}$

Synthesis of Example A13

[C382]

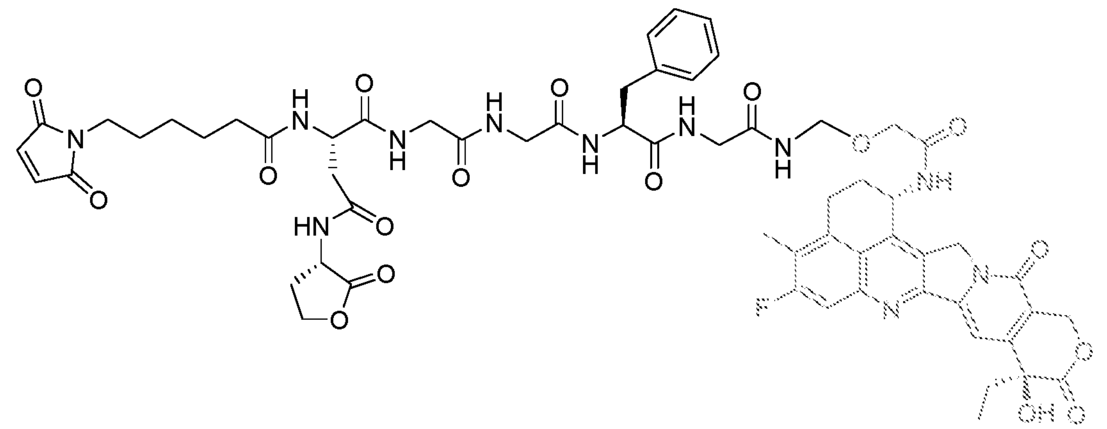

Example A13-1

[C383]

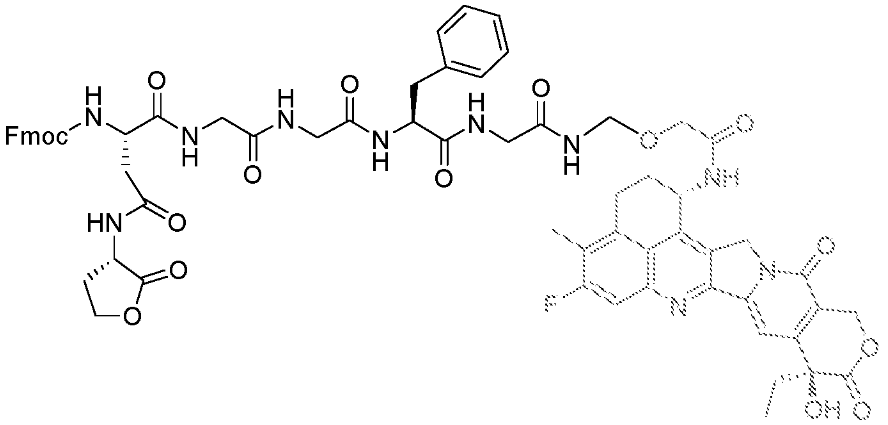

To a solution of N-[(9H-fluoren-9-ylmethoxy)carbonyl]glycylglycyl-L-phenylalanyl-N-[(2-1[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycineamide (compound disclosed in JP 6186045 B1) (102.8 mg, 0.040 mmoL, content: 41%) in N,N-dimethylformamide (0.3 mL) was added diazabicycloundecene (9 μL, 9.09 mg, 0.06 mmoL) under argon gas flow with stirring at room temperature, and the resulting mixture was stirred at room temperature for 12 hours. 2N hydrochloric acid (30 μL) was added thereto to prepare a Reaction solution A.

Separately, to a solution of the Example A1-2 (26.1 mg, 0.060 mmoL) in N,N-dimethylformamide (0.2 mL) in a 10 mL cylindrical flask were added triethylamine (8.5 μL, 6.17 mg, 0.061 mmoL) and HATU (22.8 mg, 0.060 mmoL) with stirring at room temperature, the resulting mixture was stirred at room temperature for 30 minutes, added to the above Reaction solution A, and the resulting mixture was stirred at room temperature for 1 hour.

The resulting reaction solution was subjected to preparative HPLC under the following conditions, the fraction comprising the target compound was concentrated under reduced pressure to distill away acetonitrile, and then the resulting residues were freeze-dried to give the title compound (17.9 mg, yield: 35.8%) as a colorless amorphous.
MS (ESI) m/z 1262 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 40% (0.00 min.) → 60% (8.00 min.)

Example A13

[C384]

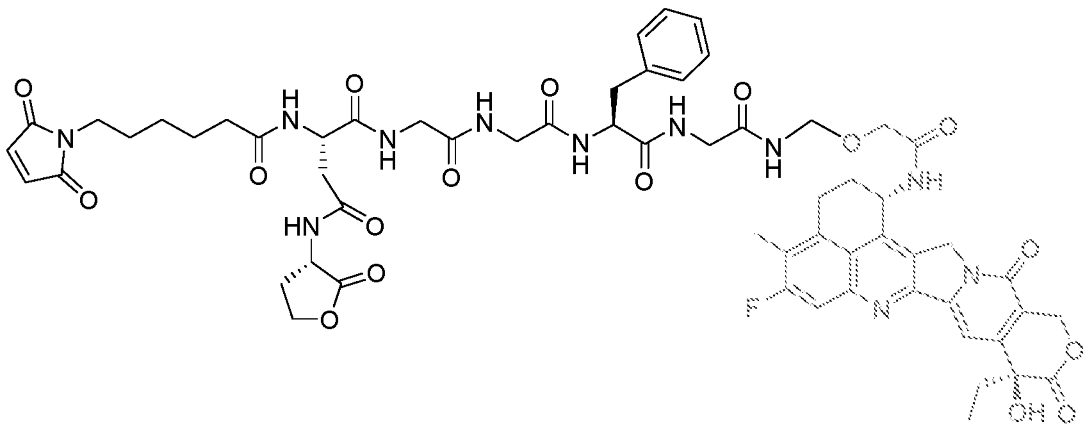

N-succinimidyl 6-maleimidohexanoate (8.75 mg, 0.028 mmoL) instead of N-succinimidyl 1-maleimide-3-oxo-7,10,13,16-tetraoxa-4-azanonadecanoate and the Example A13-1 (17.9 mg, 0.014 mmoL) instead of the Example A4-2 were reacted in the same manner as the Example A4 to give the title compound (3.6 mg, yield: 20.59%) as colorless solids.
MS (ESI) m/z 1233 $(M+H)^+$

Synthesis of Example A14

[C385]

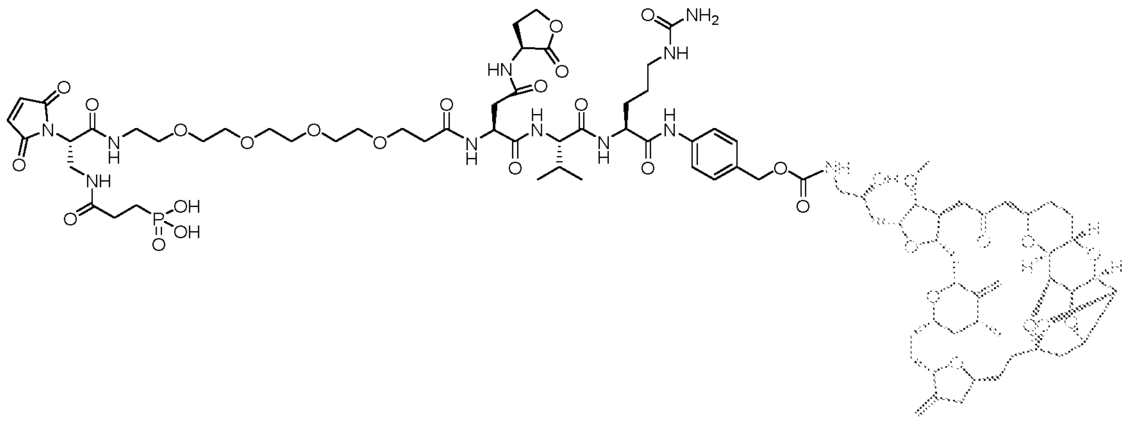

Example A14-1

[C386]

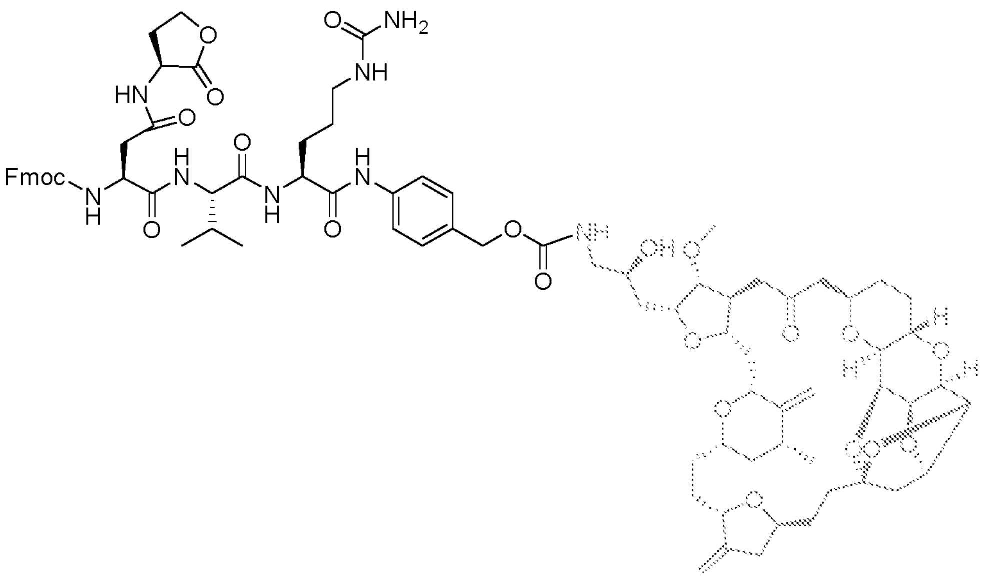

The Example A2-2 (23.1 mg, 0.024 mmoL) and eribulin mesylate (16.4 mg, 0.020 mmoL) instead of the U-030 were reacted in the same manner as the Example A2-3 to give the title compound (21.0 mg, yield: 67.98%) as a colorless amorphous.
MS (DUIS) m/z 1555 $(M+H)^+$

Example A14

[C387]

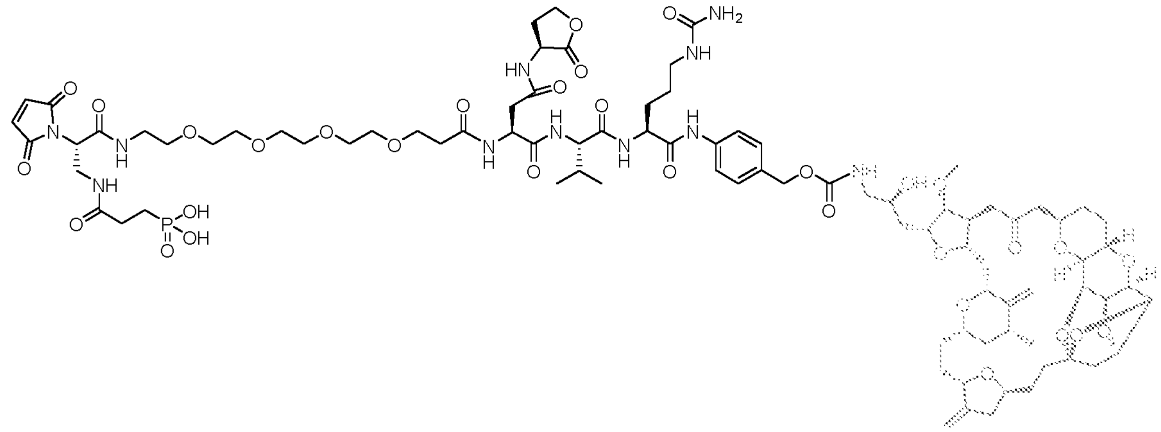

The Example A3-6 (11.7 mg, 0.021 mmoL) and the Example A14-1 (8.3 mg, 5.33 μmoL) instead of the Example A9-2 were reacted in the same manner as the Example A9 to give the title compound (6.9 mg, yield: 68.69%) as pale yellow solids.

MS (ESI) m/z 942 $(M+2H)^{2+}$

Synthesis of Example A15

[C388]

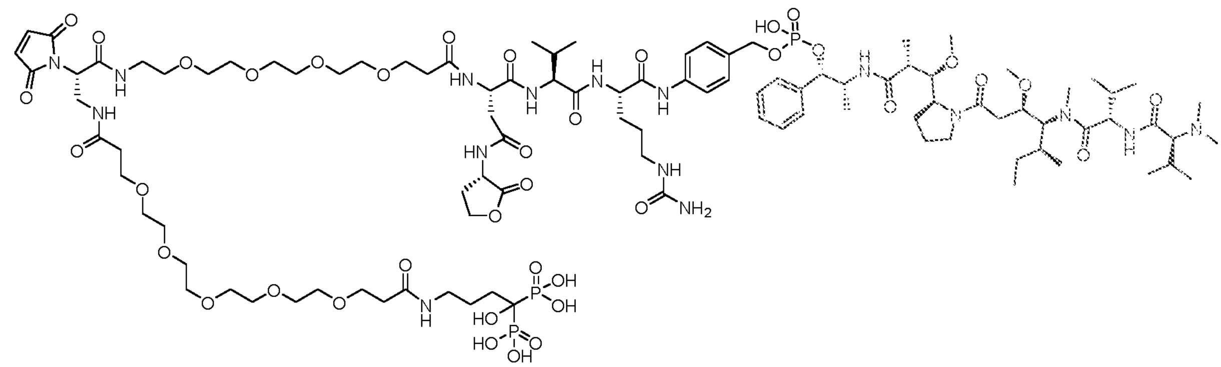

Example A15-1

[C389]

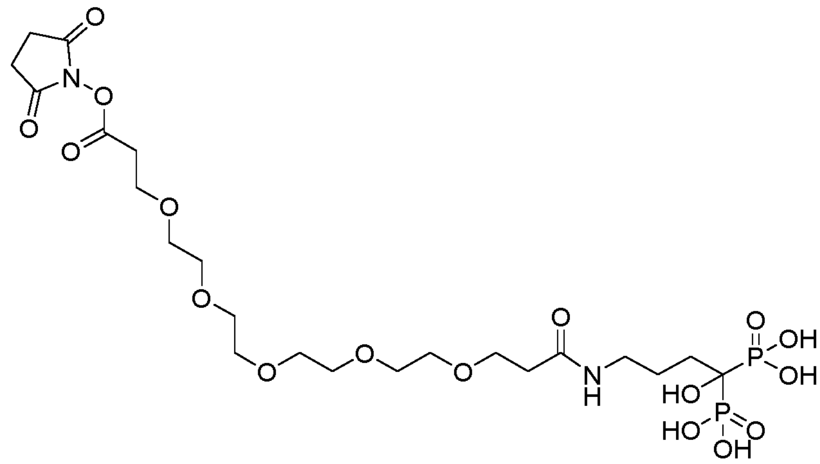

To a solution of bis(2,5-dioxopyrrolidin-1-yl) 4,7,10,13,16-pentaoxanonadecanedioate (1.08 g, 2.028 mmoL) and 1-hydroxypyrrolidine-2,5-dione (46.6 mg, 0.405 mmoL) in N,N-dimethylformamide (16 mL) in a 100 mL cylindrical flask were added dropwise 1-ethyl-3-(3-dimethylaminopropyl)carbodiimide hydrochloride (77.3 mg, 0.403 mmoL) and triethylamine (0.84 mL, 0.61 g, 6.03 mmoL) under argon gas flow with stirring at room temperature, and the resulting mixture was stirred at room temperature for 20 minutes. Subsequently, water (4 mL) was added thereto, then a solution of alendronic acid (0.50 g, 2.007 mmoL) and triethylamine (0.84 mL, 0.61 g, 6.03 mmoL) in a mixture of water (4 mL) / N,N-dimethylformamide (2 mL) was added dropwise thereto under stirring at room temperature, the resulting mixture was stirred at room temperature for 20 minutes, and then 2N hydrochloric acid (3 mL) was added thereto.

The resulting residues were subjected to preparative HPLC under the following conditions, and the fraction comprising the target compound was freeze-dried to give the title compound (0.31 g, yield: 23.17%) as a colorless oil. MS (ESI) m/z 667 $(M+H)^+$

Column: Waters XSelect HSS T3 OBD 5 μm (19*150 mm)
Flow rate: 17 mL/min.
Elution solvent: 0.1% aqueous solution of formic acid (Solution A) - acetonitrile (Solution B)
Gradient (Solution B): 5% (0.00 min.) → 30% (10.00 min.)

Example A15-2

[C390]

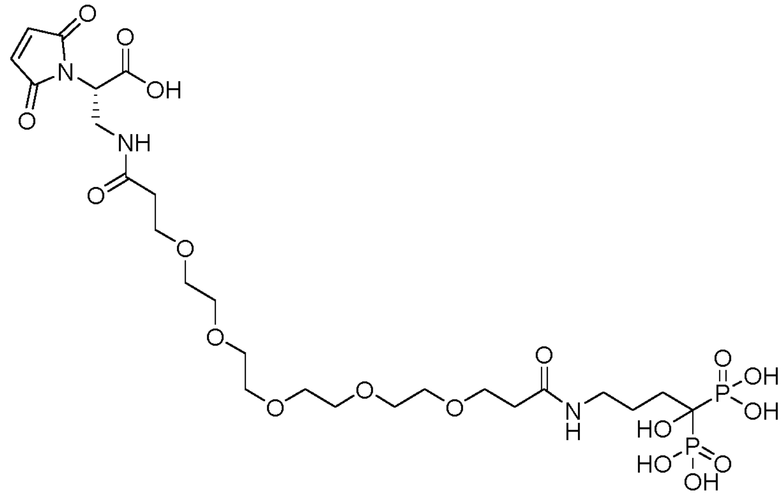

To a solution of the Example A15-1 (0.31 g, 0.465 mmoL) in N,N-dimethylformamide (9 mL) in a 20 mL cylindrical flask were added the Example A3-3 (0.21 g, 0.952 mmoL) and triethylamine (0.65 mL, 0.47 g, 4.66 mmoL) under argon gas flow with stirring at room temperature, the resulting mixture was stirred at room temperature for 1 hour, and then 2N hydrochloric acid (2.3 mL) was added thereto.

The resulting residues were subjected to preparative HPLC under the following conditions, the fraction comprising the target compound was concentrated under reduced pressure to distill away acetonitrile, and then the resulting residues were freeze-dried to give the title compound (272.7 mg, yield: 79.71%) as a colorless oil.
MS (ESI) m/z 736 $(M+H)^+$

Column: Waters XSelect HSS T3 OBD 5 μm (19*150 mm)
Flow rate: 17 mL/min.
Elution solvent: 0.1% aqueous solution of formic acid (Solution A) - acetonitrile (Solution B)
Gradient (Solution B): 5% (0.00 min.) → 20% (6.00 min.)

Example A15-3

[C391]

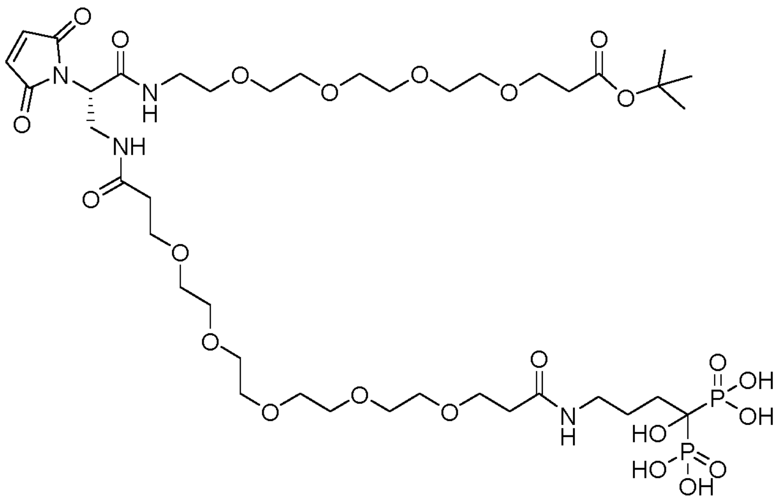

Tert-butyl 1-amino-3,6,9,12-tetraoxapentadecan-15-oate (17.5 mg, 0.054 mmoL) and the Example A15-2 (40.0 mg, 0.054 mmoL) instead of the Example A3-4 were reacted in the same manner as the Example A3-5 to give the title compound (20.4 mg, yield: 36.11%) as a colorless oil.
MS (ESI) m/z 1040 $(M+H)^+$

Example A15-4

[C392]

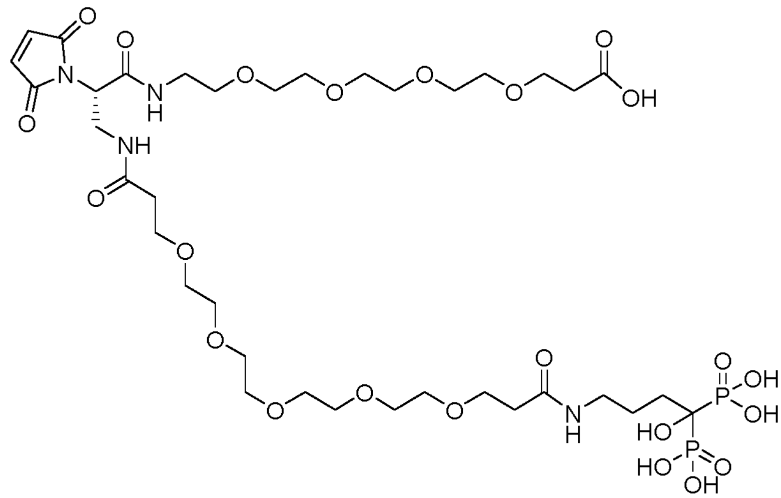

The Example A15-3 (20.3 mg, 0.020 mmoL) instead of the Example A3-5 was reacted in the same manner as the Example A3-6 to give the title compound (9.0 mg, yield: 46.87%) as a colorless oil.
MS (ESI) m/z 983 $(M+H)^+$

Example A15

[C393]

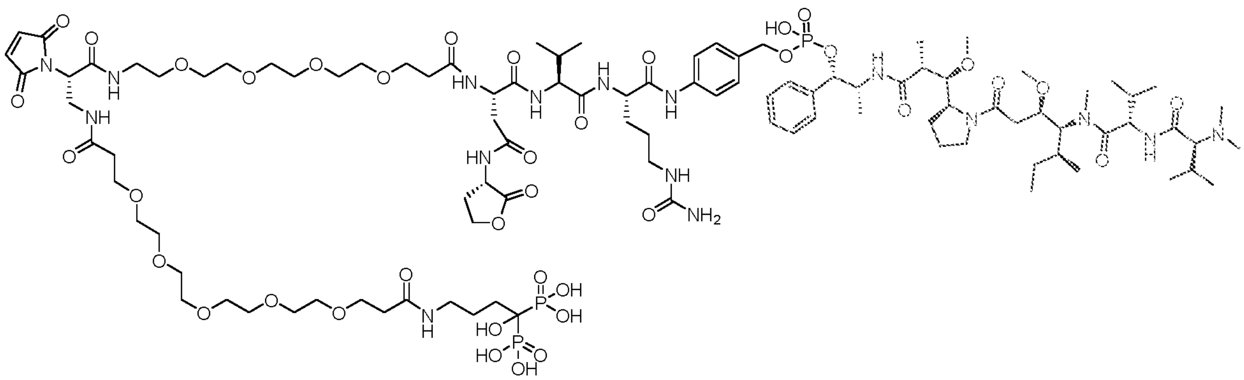

The Example A3-7 (8.4 mg, 6.12 μmoL) and the Example A15-4 (9.0 mg, 9.16 μmoL) instead of the Example A3-6 were reacted in the same manner as the Example A3 to give the title compound (9.97 mg, yield: 69.68%) as colorless solids.
MS (ESI) m/z 1169 $(M+2H)^{2+}$

Synthesis of Example A16

[C394]

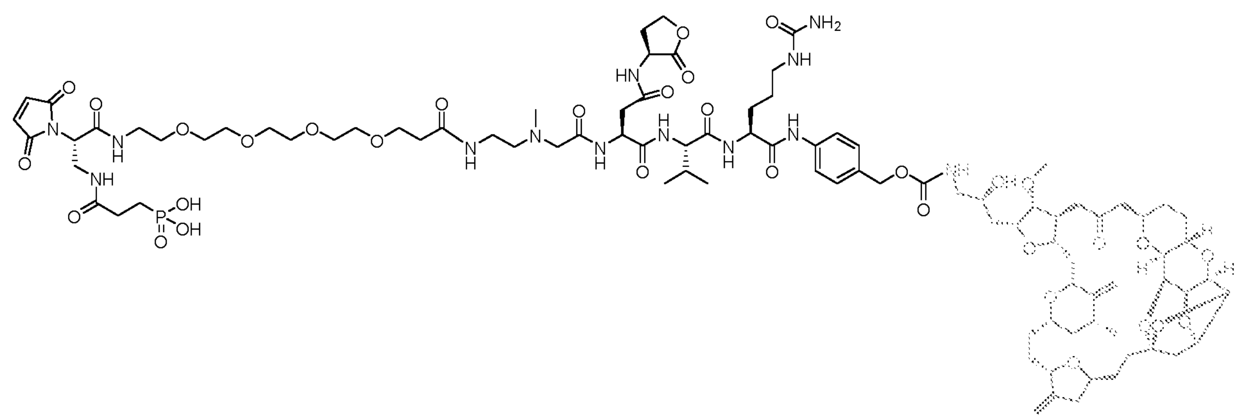

Example A16-1

[C395]

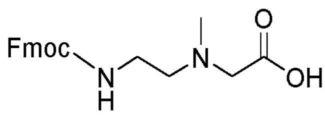

To a solution of (2-((((9H-fluoren-9-yl)methoxy)carbonyl)amino)ethyl)glycine (0.28 g, 0.823 mmoL) in a mixture of methanol (5 mL) / dichloromethane (5 mL) in a 30 mL cylindrical flask were added a 37% aqueous solution of formaldehyde (0.28 mL, 0.31 g, 3.76 mmoL), formic acid (0.26 mL, 0.31 g, 6.78 mmoL), and sodium triacetoxyborohydride (0.26 g, 1.227 mmoL) with stirring at room temperature, and the resulting mixture was stirred at room temperature for 1 hour. Subsequently, sodium triacetoxyborohydride (0.26 g, 1.227 mmoL) was added thereto.

After the reaction was completed, the solvent was concentrated under reduced pressure, then acetonitrile and water were added thereto, and the resulting mixture was filtered through a membrane filter.

The resulting solution was subjected to preparative HPLC under the following conditions, the fraction comprising the target compound was concentrated under reduced pressure to distill away acetonitrile, and then the resulting residues were freeze-dried to give the title compound (0.15 g, yield: 51.45%) as colorless solids.
MS (ESI) m/z 355 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 45% (6.00 min.)

Example A16-2

[C396]

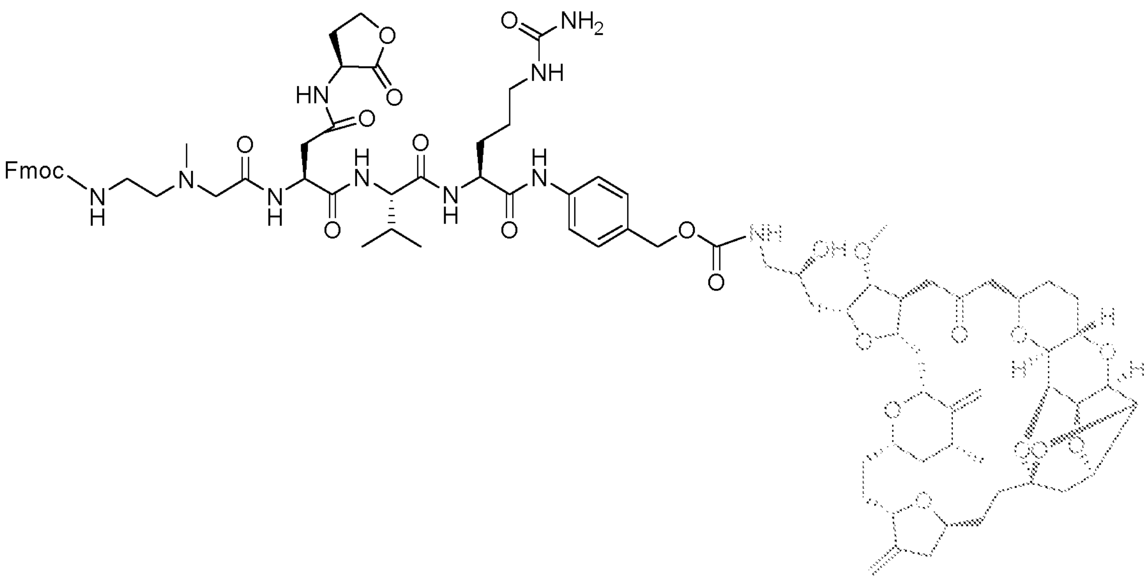

To a solution of the Example A14-1 (7.1 mg, 4.56 μmoL) in N,N-dimethylformamide (0.5 mL) in a 5 mL sample tube was added piperidine (5 μL, 4.3 mg, 0.050 mmoL) under air atmosphere with stirring at room temperature, the resulting mixture was stirred at room temperature for 1 hour, and then concentrated under reduced pressure to prepare Residues A.

To a solution of the Example A16-1 (3.4 mg, 9.59 μmoL) in N,N-dimethylformamide (0.5 mL) in another 5 mL sample tube were added dimethylbenzylamine (1.5 μL, 1.35 mg, 9.98 μmoL) and then HATU (3.4 mg, 8.94 μmoL) under argon atmosphere with stirring at room temperature, and the resulting mixture was stirred at room temperature for 1 hour.

The above reaction solution was added to the Residues A, and the resulting mixture was stirred at room temperature for 1 hour. Subsequently, HATU (1 mg, 2.63 μmoL) was added thereto.

The resulting reaction solution was subjected to preparative HPLC under the following conditions, and the fraction comprising the target compound was freeze-dried to give the title compound (4.5 mg, yield: 59.05%) as colorless solids.

MS (ESI) m/z 836 $(M+2H)^{2+}$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 80% (8.00 min.)

Example A16

[C397]

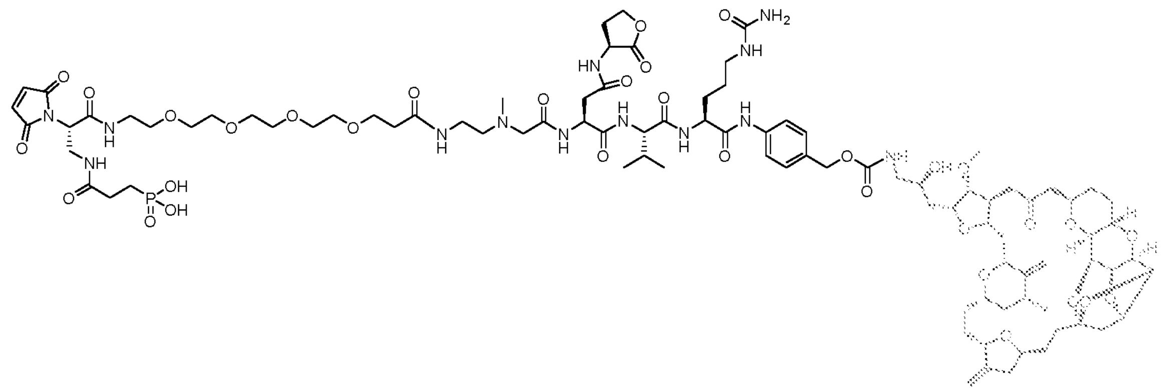

The Example A3-6 (6.0 mg, 10.57 μmoL) and the Example A16-2 (4.5 mg, 2.69 μmoL) instead of the Example A9-2 were reacted in the same manner as the Example A9 to give the title compound (2.40 mg, yield: 44.59%) as colorless solids.
MS (ESI) m/z 1000 $(M+2H)^{2+}$

Synthesis of Example A17

[C398]

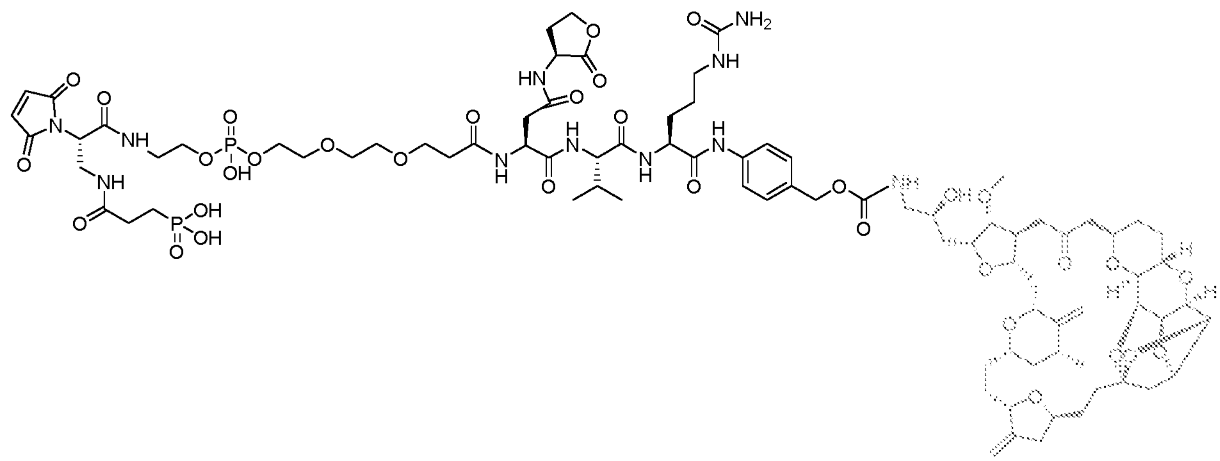

Example A17-1

[C399]

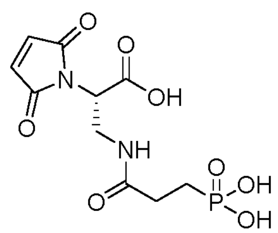

To a solution of the Example A3-2 (0.54 g, 1.470 mmoL) and triethylamine (0.41 mL, 0.3 g, 2.94 mmoL) in N,N-dimethylformamide (5 mL) in a 30 mL cylindrical flask was added HATU (0.59 g, 1.552 mmoL) under argon gas flow with stirring at room temperature, and the resulting mixture was stirred at room temperature for 0.5 hour to prepare a Solution A.

To a solution of the Example A3-3 (0.32 g, 1.451 mmoL) in N,N-dimethylformamide (5 mL) in a 20 mL cylindrical flask was added the Solution A under argon gas flow with stirring at room temperature, and the resulting mixture was stirred at room temperature for 6 hours.

After the reaction was completed, to the reaction solution was added water, the resulting solution was filtered through a membrane filter, then the resulting solution was subjected to preparative HPLC under the following conditions, and the fraction comprising the target compound was freeze-dried to give the Example A3-4 (62.5 mg) as a colorless oil.
MS (ESI) m/z 433 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 36% (5.00 min.)

The resulting Example A3-4 was left to stand at room temperature for one month to give the title compound as colorless solids.
MS (ESI) m/z 321 $(M+H)^+$

Example A17-2

[C400]

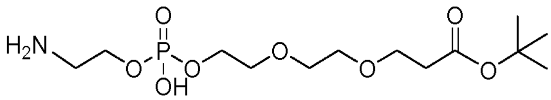

To a solution of tert-butyl 3-(2-(2-hydroxyethoxy)ethoxy)propanoate (0.21 g, 0.896 mmoL) in N,N-dimethylformamide (5 mL) in a 10 mL pear-shaped flask were sequentially added 2-cyanoethyl N,N,N',N'-tetraisopropylphosphordiamidite (0.32 mL, 0.3 g, 1.009 mmoL) and 1H-tetrazole (70.0 mg, 0.999 mmoL), and the resulting mixture was stirred at room temperature for 15 minutes.

Subsequently, (9H-fluoren-9-yl)methyl(2-hydroxyethyl)carbamate (0.31 g, 1.094 mmoL) was added thereto, then 5-(ethylthio)-1H-tetrazole (0.18 g, 1.383 mmoL) was added thereto, and the resulting mixture was stirred at room temperature for 20 minutes.

Subsequently, a 70% aqueous solution of tert-butyl hydroperoxide (0.75 mL, 0.71 g, 5.48 mmoL) was added thereto at room temperature, and then the resulting mixture was stirred at room temperature for 0.5 hour. Subsequently, diazabicycloundecene (1.09 mL, 1.11 g, 7.30 mmoL) was added thereto, and the resulting mixture was stirred for 10 minutes. Subsequently, a 50% aqueous solution of acetonitrile (2 mL) was added thereto, and then 6N hydrochloric acid (1.2 mL) was added thereto.

The resulting residues were subjected to preparative HPLC under the following conditions, and the fraction comprising the target compound was concentrated under reduced pressure to give the title compound (0.12 g, yield: 37.47%) as colorless solids.
MS (ESI) m/z 358 $(M+H)^+$

Column: Waters XSelect HSS T3 OBD 5 μm (19*150 mm)
Flow rate: 17 mL/min.
Elution solvent: 0.1% aqueous solution of formic acid (Solution A) - acetonitrile (Solution B)
Gradient (Solution B): 10% (0.00 min.) → 50% (8.00 min.)

Example A17-3

[C401]

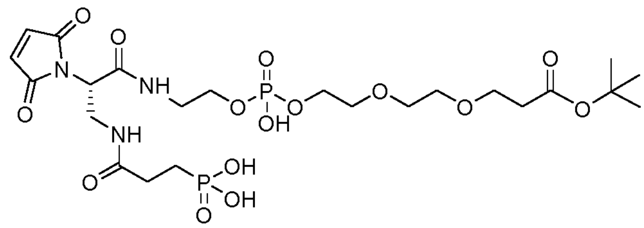

The Example A17-1 (10.4 mg, 0.032 mmoL) instead of the Example A3-4 and the Example A17-2 (15.6 mg, 0.044 mmoL) instead of tert-butyl 1-amino-3,6,9,12-tetraoxapentadecan-15-oate were reacted in the same manner as the Example A3-5 to give the title compound (10.3 mg, yield: 48.08%) as a colorless oil.
MS (ESI) m/z 660 $(M+H)^+$

Example A17-4

[C402]

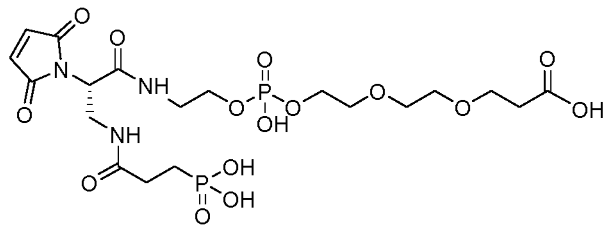

The Example A17-3 (10.3 mg, 0.016 mmoL) instead of the Example A3-5 was reacted in the same manner as the Example A3-6 to give the title compound (8.0 mg, yield: 84.89%) as a colorless oil.
MS (ESI) m/z 604 $(M+H)^+$

Example A17

[C403]

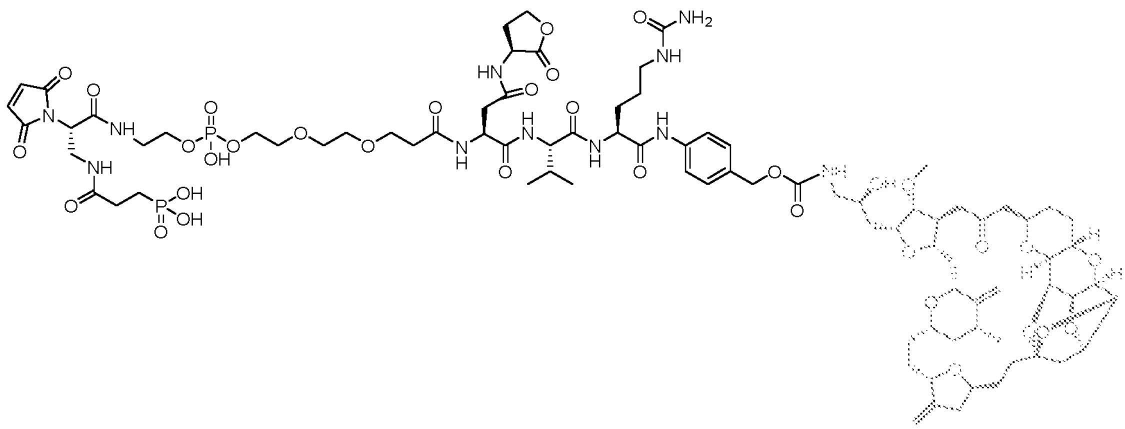

The Example A14-1 (4.4 mg, 2.83 μmoL) instead of the Example A9-2 and the Example A17-4 (8.5 mg, 0.014 mmoL) instead of the Example A3-6 were reacted in the same manner as the Example A9 to give the title compound (4.05 mg, yield: 74.63%) as colorless solids.

MS (ESI) m/z 960 $(M+2H)^{2+}$

Synthesis of Example A18

[C404]

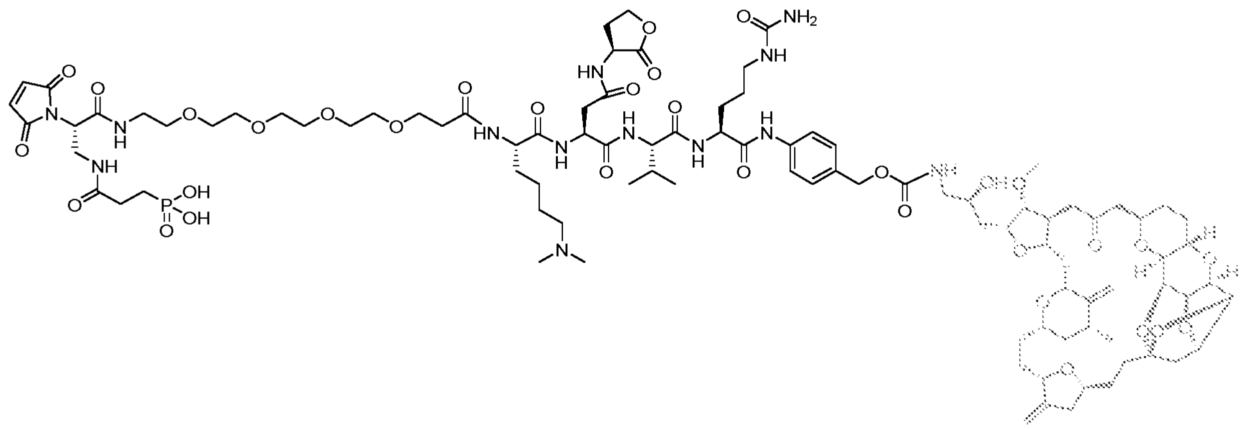

Example A18-1

[C405]

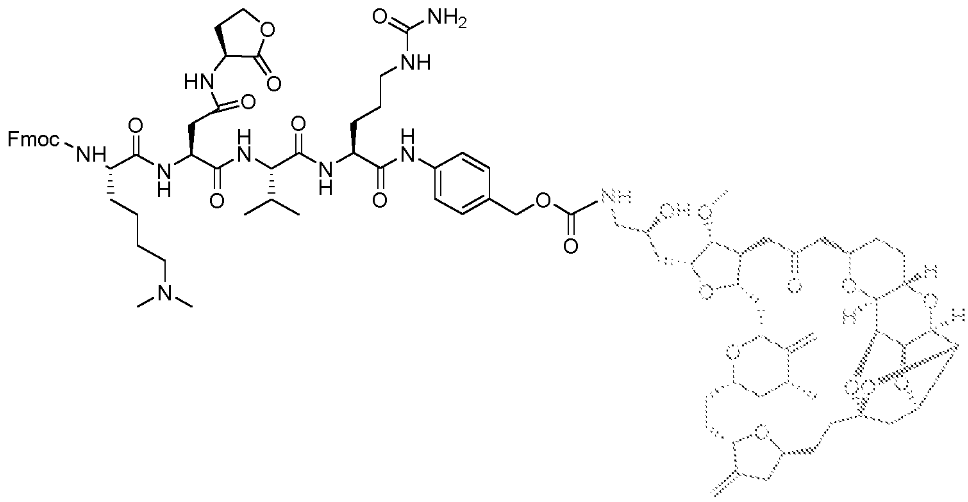

The Example A14-1 (9.5 mg, 6.11 μmoL), and N2-(((9H-fluoren-9-yl)methoxy)carbonyl)-N6,N6-dimethyl-L-lysinehydrochloride (5.3 mg, 0.012 mmoL) instead of the Example A16-1 were reacted in the same manner as the Example A16-2 to give the title compound (7.5 mg, yield: 71.74%) as colorless solids.
MS (ESI) m/z 857 $(M+2H)^{2+}$

Example A18

[C406]

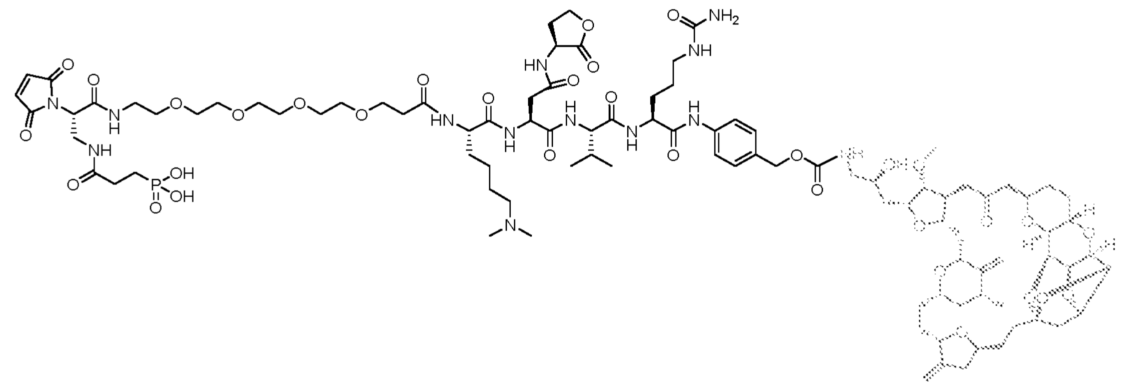

The Example A3-6 (5.0 mg, 8.81 μmoL) and the Example A18-1 (7.5 mg, 4.38 μmoL) instead of the Example A9-2 were reacted in the same manner as the Example A9 to give the title compound (3.8 mg, yield: 42.54%) as colorless solids.
MS (ESI) m/z 1021 $(M+2H)^{2+}$

Synthesis of Example A19

[C407]

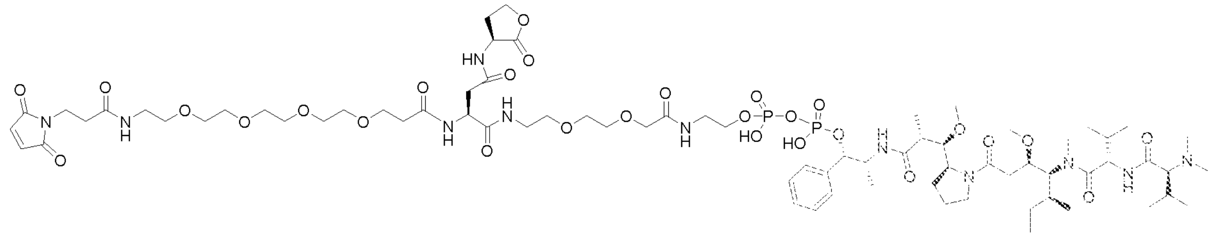

Example A19-1

[C408]

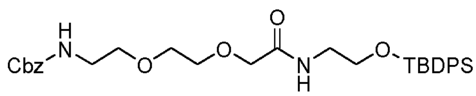

To 2-((tert-butyldiphenylsilyl)oxy)ethan-1-amine (10.78 g, 36.0 mmoL) in a 500 mL round-bottom flask was added a solution of 3-oxo-1-phenyl-2,7,10-trioxa-4-azadodecan-12-oic acid (11.28 g, 37.9 mmoL) in dichloromethane (100 mL) under water-cooling under argon atmosphere with stirring, then 1-ethyl-3-(3-dimethylaminopropyl)carbodiimide hydrochloride (7.28 g, 38.0 mmoL) and 1-hydroxybenzotriazole (1.11 g, 7.25 mmoL) were sequentially added thereto at room temperature, and the resulting mixture was stirred at room temperature for 2 hours.

To the reaction solution were added water (75 mL) and saturated brine (75 mL), the resulting mixture was separated, and the resulting aqueous layer was subjected to extraction with dichloromethane (30 mL) twice. The resulting organic layers were combined, dried over anhydrous magnesium sulfate (10 g), filtered, and the resulting filtrate was concentrated under reduced pressure to give orange syrupy residues (23.19 g).

To the resulting residues was added hexane / ethyl acetate (57/43 (V/V)) (100 mL) to uniformly dissolve the residues, the resulting solution was subjected to YAMAZEN medium pressure flash chromatography (Silica, 5L (3000 g), hexane / ethyl acetate = 40/60 (V/V) (Rf = 0.40 (hexane / ethyl acetate = 40/60 (V/V)))), and the fraction comprising the target compound was concentrated under reduced pressure to give the title compound (20.04 g, yield: 96.19%) as a colorless oil.

MS (ESI) m/z 579 $(M+H)^+$

Example A19-2

[C409]

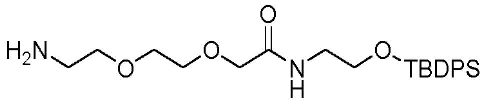

To a solution of the Example A19-1 (21.18 g, 36.6 mmoL) in ethanol (150 mL) in a 500 mL round-bottom flask was added a Pearlman's catalyst (20% Pd, wetted with 50% water, manufactured by Tokyo Chemical Industry Co., Ltd.) (1.28 g, 0.911 mmoL) under nitrogen atmosphere, and then the resulting mixture was stirred under hydrogen atmosphere at room temperature for 5 hours.

The reaction solution was subjected to nitrogen atmosphere, then filtered through Celite 545 (trade name), washed with ethanol, and the resulting filtrate and wash liquid were concentrated under reduced pressure to give the title compound (16.15 g, yield: 99.25%) as a colorless oil. MS (ESI) m/z 445 $(M+H)^+$

Example A19-3

[C410]

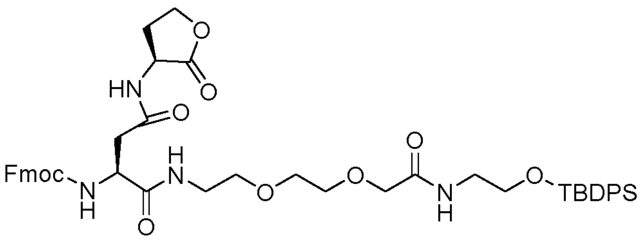

To a solution of the Example A1-2 (2.27 g, 5.11 mmoL) in dichloromethane (20 mL) in a 100 mL round-bottom flask was added the Example A19-2 (2.31 g, 5.27 mmoL) under argon atmosphere, then 1-ethyl-3-(3-dimethylaminopropyl) carbodiimide hydrochloride (1.08 g, 5.63 mmoL) and 1-hydroxybenzotriazole (0.1595 g, 1.042 mmoL) were sequentially added thereto at room temperature, and the resulting mixture was stirred at room temperature for 2 hours.

To the reaction solution were added water (10 mL) and saturated brine (10 mL), the resulting mixture was separated, and the resulting aqueous layer was subjected to extraction with dichloromethane (10 mL) twice. The resulting organic layers were combined, dried over anhydrous magnesium sulfate, filtered, and the resulting filtrate was concentrated under reduced pressure to give colorless solid residues (6.09 g).

To the resulting residues was added diethyl ether (50 mL), the resulting mixture was subjected to sonication, to the resulting solids was added diethyl ether (50 mL), the resulting mixture was subjected to sonication, and then collected by filtration. The resulting solids were washed with diethyl ether, and dried under reduced pressure to give the title compound (4.26 g, yield: 96.46%) as white solids.
MS (ESI) m/z 865 $(M+H)^+$

Example A19-4

[C411]

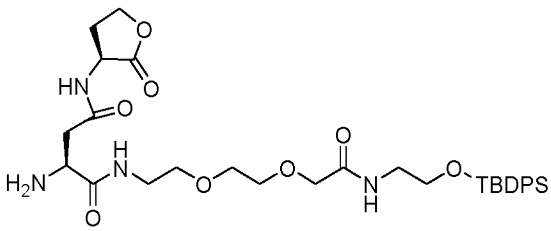

To a solution of the Example A19-3 (4.26 g, 4.92 mmoL) in N,N-dimethylformamide (20 mL) in a 100 mL pear-shaped flask was added piperidine (1.46 mL, 1.26 g, 14.78 mmoL) under argon atmosphere with stirring at room temperature, and the resulting mixture was stirred at room temperature for 0.5 hour.

The reaction solution was concentrated under reduced pressure to give white solid residues (7.72 g). To the resulting residues was added diethyl ether (50 mL), the resulting mixture was subjected to sonication, then left to stand at -20°C for 12 hours, and the resulting supernatant was removed. Subsequently, to the resulting residues was added diethyl ether (25 mL), the resulting mixture was subjected to sonication, then left to stand at -20°C, and the resulting supernatant was removed. Said operation was repeated once again, and then the resulting reaction mixture was dried under reduced pressure to give the title compound (3.05 g, yield: 96.35%) as a slightly yellow oil.
MS (ESI) m/z 643 $(M+H)^+$

Example A19-5

[C412]

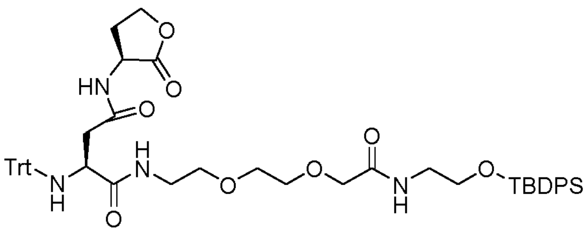

To a solution of the Example A19-4 (3.05 g, 4.74 mmoL) in N,N-dimethylformamide (20 mL) in a 100 mL pear-shaped flask were sequentially added potassium carbonate (1.0075 g, 7.29 mmoL) and trityl chloride (1.70 g, 6.10 mmoL) under argon atmosphere with stirring at room temperature, and then the resulting mixture was stirred at room temperature for 2 hours.

After the reaction was completed, the reaction solution was concentrated. To the concentrated residues was added tetrahydrofuran, the resulting mixture was subjected to sonication, the resulting insoluble matters were filtered, and the resulting filtered residues were washed with tetrahydrofuran. The resulting filtrate and wash liquid were combined, and the resulting mixture was concentrated under reduced pressure to give slightly yellow oily concentrated residues (5.61 g).

To the resulting residues was added dichloromethane to dissolve the residues, Fuji Silysia CHROMATOREX Q-PACK Diol-60 Size 60 (27.0 g) was added thereto, and the resulting mixture was dried under reduced pressure. The resulting residues were subjected to YAMAZEN medium pressure flash chromatography (Fuji Silysia CHROMATOREX Q-PACK Diol-60 (85.0 g), hexane / ethyl acetate = 7/93 (V/V) → 0/100 (V/V), (Rf = 0.50 (ethyl acetate))), and the fraction comprising the target compound was concentrated under reduced pressure to give the title compound (2.82 g, yield: 67.15%) as a colorless oil.
MS (ESI) m/z 885 $(M+H)^+$

Example A19-6

[C413]

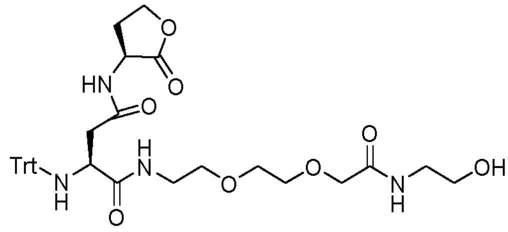

To a solution of the Example A19-5 (2.82 g, 3.19 mmoL) in tetrahydrofuran (50 mL) in a 300 mL round-bottom flask was added a 1 M solution of tetrabutylammonium fluoride in tetrahydrofuran (3.5 mL, 0.96 g, 3.50 mmoL) under argon atmosphere with stirring at room temperature, and the resulting mixture was stirred at room temperature for 2 hours.

The reaction solution was concentrated under reduced pressure, to the resulting residues (4.42 g) was added dichloromethane (50 mL) to dissolve the residues, and then the resulting solution was washed with a saturated aqueous solution of ammonium chloride (50 mL). The resulting aqueous layer was subjected to extraction with dichloromethane (25 mL) twice, the resulting organic layers were combined, dried over anhydrous sodium sulfate (10 g), filtered, and the resulting filtrate was concentrated under reduced pressure to give colorless oily residues (4.28 g).

To the resulting residues was added acetonitrile (45 mL) to dissolve the residues, the resulting solution was subjected to YAMAZEN medium pressure flash chromatography (Silica, L (40 g), A/B = 100/0 (V/V) → 50/50 (V/V), and then ethyl acetate / methanol = 100/0 (V/V) → 40/60 (V/V) (Rf = 0.47 (ethyl acetate)), Solution A; acetonitrile/water/-triethylamine = 950/50/1 (V/V/V), Solution B; acetonitrile/water/triethylamine = 850/150/5 (V/V/V)), and the fraction comprising the target compound was concentrated under reduced pressure to give crude products of the title compound (1.76 g) as white foam.

Subsequently, the crude products were subjected to recycle preparative under the following conditions, and the fraction comprising the target compound was concentrated under reduced pressure to give the title compound (1.58 g, yield: 71.62%) as white foam.
MS (ESI) m/z 647 $(M+H)^+$

- Device: LC Forte/R
- Column: GPC system
- Eluent: dichloromethane → acetonitrile
- Flow rate: 15 mL/min.
- Pressure: 12 MPa (dichloromethane), 8.4 MPa (acetonitrile)
- Wavelength: 220 nm

Example A19-7

[C414]

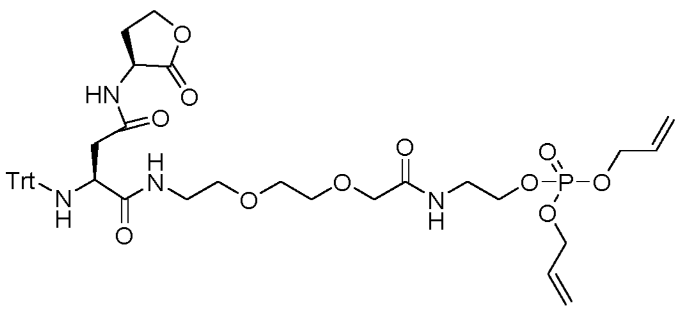

To a solution of the Example A19-6 (1.58 g, 2.282 mmoL) in dichloromethane (30 mL) in a 200 mL round-bottom flask were sequentially added diallyl N,N-diisopropylphosphoramidite (2.58 mL, 2.39 g, 9.76 mmoL) and 1H-tetrazole (1.20 g, 17.13 mmoL) under argon atmosphere with stirring at room temperature, and the resulting mixture was stirred at room temperature for 2 hours.

Subsequently, the mixture was ice-cooled, 30% by weight of hydrogen peroxide water (0.650 mL, 0.72 g, 6.36 mmoL) was added thereto, and the resulting mixture was stirred under ice-cooling for 1 hour.

Subsequently, to sodium thiosulfate pentahydrate (20.0 g, 80.6 mmoL) and sodium hydrogen carbonate (7.0 g, 83.3 mmoL) was added water (100 mL) to prepare a solution, 30 mL of said solution was added to the above mixture, and the resulting solution was separated. The resulting aqueous layer was subjected to extraction with dichloromethane (15 mL) twice, combined with the resulting organic layer, dried over anhydrous sodium sulfate (10.0 g), filtered, and the resulting filtrate was concentrated under reduced pressure to give slightly yellow oily concentrated residues (3.49 g).

To the residues was added dichloromethane (30 mL) to dissolve the residues, Fuji Silysia CHROMATOREX Q-PACK Diol-60 Size 60 (22.0 g) was added thereto, and the resulting mixture was dried under reduced pressure. The resulting residues were subjected to YAMAZEN medium pressure flash chromatography (Fuji Silysia CHROMATOREX Q-PACK Diol-60 (88.0 g), hexane / ethyl acetate = 0/100 (V/V), (Rf = 0.33 (ethyl acetate))), and the fraction comprising the target compound was concentrated under reduced pressure to give the title compound (1.55 g, yield: 84.19%) as white foam.

MS (ESI) m/z 807 $(M+H)^+$

Example A19-8

[C415]

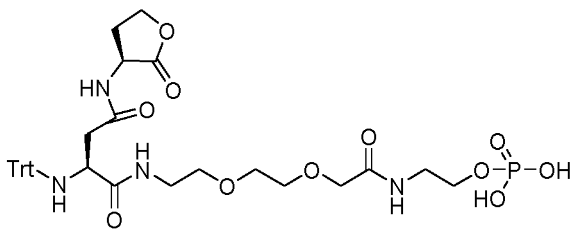

To a solution of the Example A19-7 (1.55 g, 1.921 mmoL) in tetrahydrofuran (33 mL) in a 300 mL round-bottom flask were sequentially added N-methylaniline (0.440 mL, 0.44 g, 4.07 mmoL) and tetrakis(triphenylphosphine)palladium (0.2263 g, 0.196 mmoL) under argon atmosphere with stirring at room temperature, and the resulting mixture was stirred at room temperature for 1 hour.

The reaction solution was concentrated under reduced pressure, then to the resulting pale yellow foam concentrated residues (2.38 g) was added diethyl ether (50 mL), the resulting mixture was subjected to sonication, the resulting solids were collected by filtration, washed with diethyl ether, and dried under reduced pressure to give a N-allyl-N-methylaniline salt of the title compound (1.89 g, yield: 96.34%) as pale yellow solids.

MS (ESI) m/z 727 $(M+H)^+$

Example A19-9

[C416]

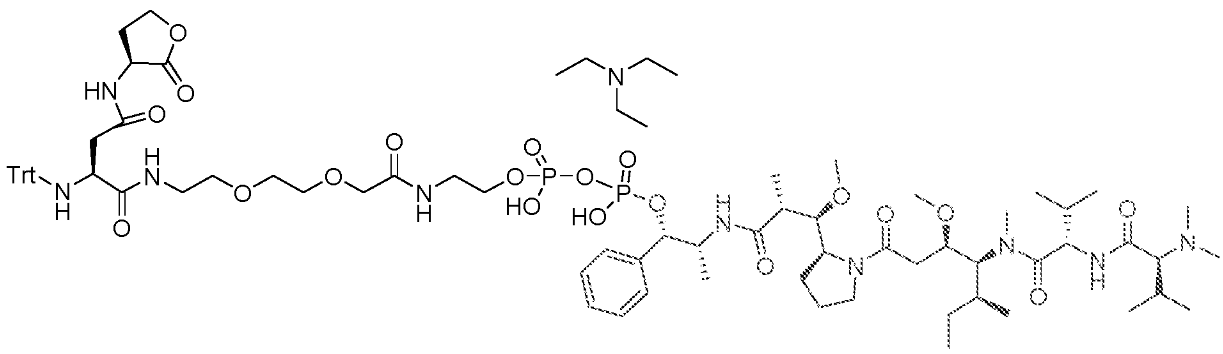

To a solution of the Example A19-8 (0.3958 g, 0.388 mmoL) in N,N-dimethylformamide (2.5 mL) in a 50 mL pear-shaped flask was added carbonyldiimidazole (0.1575 g, 0.971 mmoL) under argon atmosphere with stirring at room

temperature, and the resulting mixture was stirred at room temperature for 0.75 hour.

Subsequently, U-031 (0.3096 g, 0.381 mmoL) was added thereto, then the resulting mixture was ice-cooled, zinc chloride (0.4098 g, 3.01 mmoL) was added thereto, then the resulting mixture was warmed to room temperature, and stirred at room temperature for 6 hours.

To the reaction solution was added diethyl ether (25 mL), the resulting mixture was subjected to sonication, and then the resulting supernatant was removed. To the resulting residues was added diethyl ether (10 mL), and the resulting supernatant was removed. Said operation was repeated once again, and the resulting residues were dried under reduced pressure to give a pale yellow syrup (1.64 g).

To the syrup was added acetonitrile/water/triethylamine (= 850/150/5 (V/V/V)) (20 mL) to dissolve the syrup, the resulting solution was packaged in YAMAZEN injection column (silica gel) size L, subjected to YAMAZEN medium pressure flash chromatography (Silica, L (40 g), acetonitrile/water/triethylamine = 850/150/5 (V/V/V)), and the fraction comprising the target compound was concentrated under reduced pressure. To the resulting residues was added acetonitrile/water (1/1 (V/V)) (30 mL) to dissolve the residues, and then the resulting solution was freeze-dried to give the title compound (517 mg, yield: 83.6%) as white solids.

MS (ESI) m/z 761 $(M+2H)^{2+}$

Example A19-10

[C417]

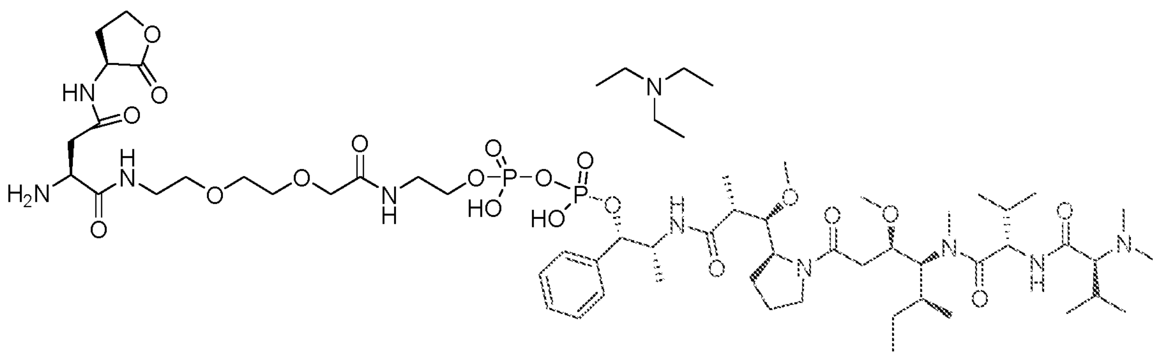

To a solution of the Example A19-9 (512.8 mg, 0.316 mmoL) in N,N-dimethylformamide (3 mL) in a 50 mL round-bottom flask was added formic acid (3 mL, 3660 mg, 80 mmoL) under argon atmosphere with stirring under ice-cooling, and then the resulting mixture was stirred at room temperature for 45 minutes.

Subsequently, diethyl ether (30 mL) was added thereto, the resulting mixture was subjected to sonication, and left to stand at -20°C for 3 hours. The resulting supernatant was removed, then to the resulting residues was added diethyl ether (15 mL), the resulting mixture was stirred for 5 minutes, and then the resulting supernatant was removed. Once again, to the resulting residues was added diethyl ether (15 mL), the resulting mixture was stirred for 5 minutes, and then the resulting supernatant was removed. The resulting residues were dried under reduced pressure to give a colorless syrup (0.7368 g).

To the syrup was added acetonitrile/water/triethylamine (= 850/150/5 (V/V/V)) (15 mL) to dissolve the syrup, then the resulting solution was packaged in YAMAZEN injection column (silica gel) size M, subjected to YAMAZEN medium pressure flash chromatography (Silica, M (16 g), acetonitrile/water/triethylamine = 850/150/5 (V/V/V)), and the fraction comprising the target compound was concentrated under reduced pressure. To the resulting residues was added acetonitrile/water (1/1 (V/V)) (20 mL) to dissolve the residues, and the resulting solution was freeze-dried to give the title compound (328.1 mg, yield: 75.22%) as white solids. MS (ESI) m/z 640 $(M+2H)^{2+}$

Example A19

[C418]

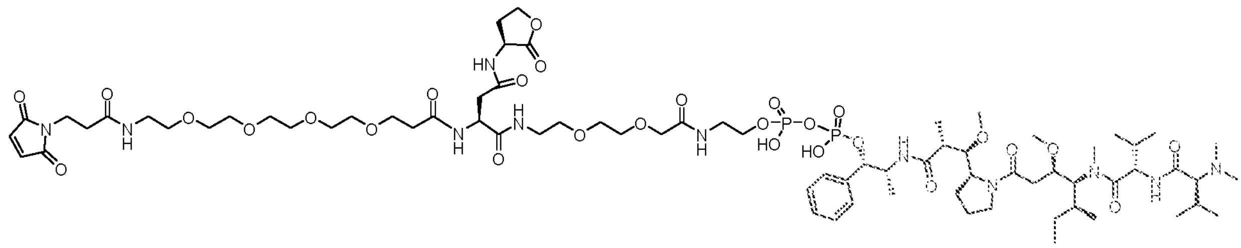

N-succinimidyl 1-maleimide-3-oxo-7,10,13,16-tetraoxa-4-azanonadecanoate (10.0 mg, 0.019 mmoL) and the Example A19-10 (9.5 mg, 6.89 μmoL) instead of the Example A1-7 were reacted in the same manner as the Example A1 to give the title compound (2.5 mg, yield: 21.65%) as white solids.
MS (ESI) m/z 839 $(M+2H)^{2+}$

Synthesis of Example A20

[C419]

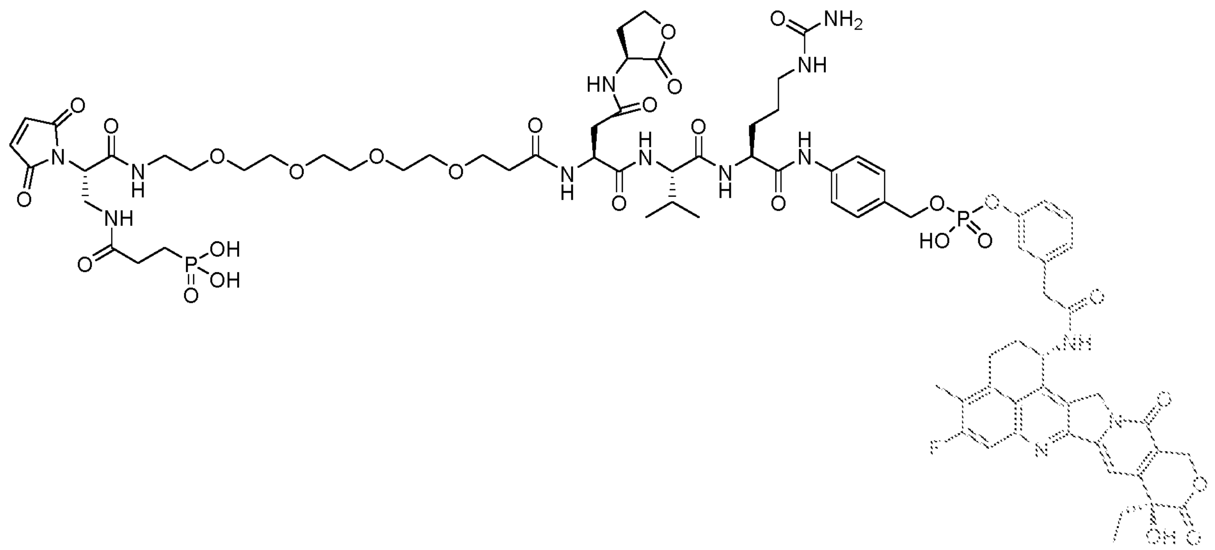

Example A20-1

[C420]

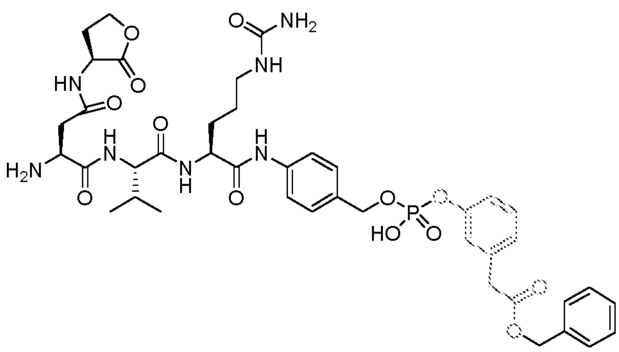

To a solution of benzyl 2-(3-hydroxyphenyl)acetate (72.2 mg, 0.298 mmoL) in N,N-dimethylformamide (2 mL) in a 10 mL pear-shaped flask were sequentially added 2-cyanoethyl N,N,N',N'-tetraisopropylphosphordiamidite (95 μL, 90.25 mg, 0.299 mmoL) and 1H-tetrazole (21.7 mg, 0.310 mmoL), and the resulting mixture was stirred at room temperature for 1 hour. The Example A2-1 (80.0 mg, 0.100 mmoL) was added thereto, then 5-(ethylthio)-1H-tetrazole (26.7 mg, 0.205 mmoL) was added thereto, and the resulting mixture was stirred at room temperature for 0.5 hour.

Subsequently, a 70% aqueous solution of tert-butyl hydroperoxide (41 μL, 38.54 mg, 0.299 mmoL) was added thereto at room temperature, and then the resulting mixture was stirred at room temperature for 0.5 hour. Subsequently, diazabicycloundecene (149 μL, 151.98 mg, 0.998 mmoL) was added thereto, and the resulting mixture was stirred at room temperature for 20 minutes. Formic acid (38 μL, 45.6 mg, 0.991 mmoL) was added thereto, and the resulting mixture was left to stand overnight.

To the resulting reaction solution was added a 50% aqueous solution of acetonitrile, then the resulting mixture was filtered, the resulting filtrate was subjected to preparative HPLC under the following conditions, the fraction comprising the target compound was concentrated under reduced pressure to distill away acetonitrile, and then the resulting residues were freeze-dried to give the title compound (16.9 mg, yield: 19.16%) as colorless foam.
MS (ESI) m/z 882 (M+H)$^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 25% (0.00 min.) → 46% (6.00 min.)

Example A20-2

[C421]

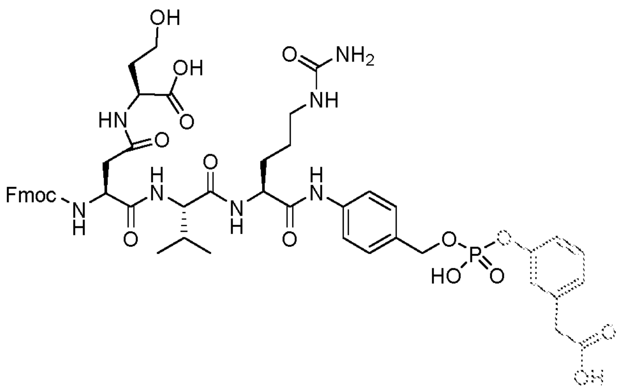

To a solution of the Example A20-1 (11.7 mg, 0.013 mmoL) in N,N-dimethylformamide (200 μL) in a 10 mL pear-shaped flask was added a 2N aqueous solution of sodium hydroxide (33 μL, 2.64 mg, 0.066 mmoL), and the resulting mixture was stirred at room temperature for 2 hours. Subsequently, 2N hydrochloric acid (33 μL) was added thereto.

Subsequently, N,N-dimethylbenzylamine (4 μL, 3.64 mg, 0.027 mmoL) and 9-fluorenylmethyl succinimidyl carbonate (9.0 mg, 0.027 mmoL) were added thereto, and the resulting mixture was stirred at room temperature for 1 hour. Subsequently, N,N-dimethylbenzylamine (4 μL, 3.64 mg, 0.027 mmoL) was added thereto, and the resulting mixture was stirred for 16 hours.

To the resulting reaction solution were added N,N-dimethylformamide and a 50% aqueous solution of acetonitrile, then the resulting mixture was filtered, the resulting filtrate was subjected to preparative HPLC under the following conditions, the fraction comprising the target compound was concentrated under reduced pressure, and then the resulting residues were freeze-dried to give the title compound (9.4 mg, yield: 68.65%) as colorless foam.
MS (ESI) m/z 1033 (M+H)$^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 25% (0.00 min.) → 60% (8.00 min.)

Example A20-3

[C422]

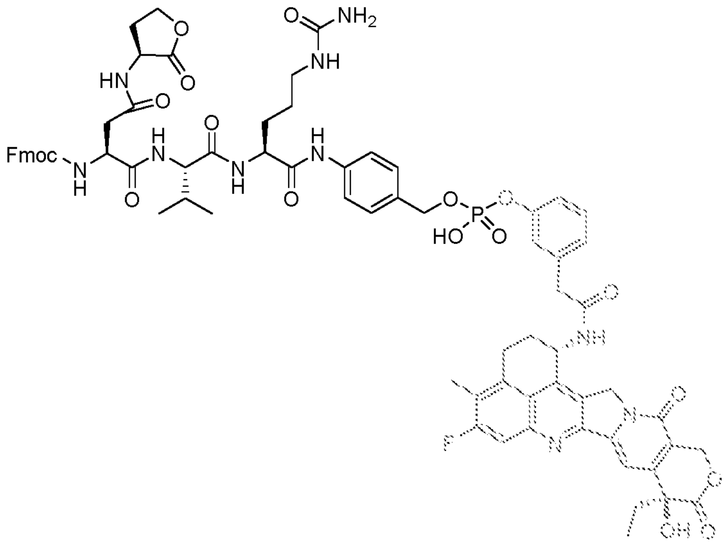

To a solution of exatecan mesylate (5.5 mg, 10.35 μmoL), the Example A20-2 (9.4 mg, 9.11 μmoL), and N,N-dimethylbenzylamine (5.71 μL, 5.2 mg, 0.038 mmoL) in N,N-dimethylformamide (500 μL) in a 10 mL cylindrical flask was added HATU (19.0 mg, 0.050 μmoL) under argon atmosphere with stirring at room temperature, and the resulting mixture was stirred at room temperature for 2 hours.

After the reaction was completed, the resulting reaction solution was subjected to preparative HPLC under the following conditions, and the fraction comprising the target compound was freeze-dried to give the title compound (6.8 mg, yield: 52.15%) as colorless solids.
MS (ESI) m/z 1432 (M+H)

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 25% (0.00 min.) → 65% (8.00 min.)

Example A20

[C423]

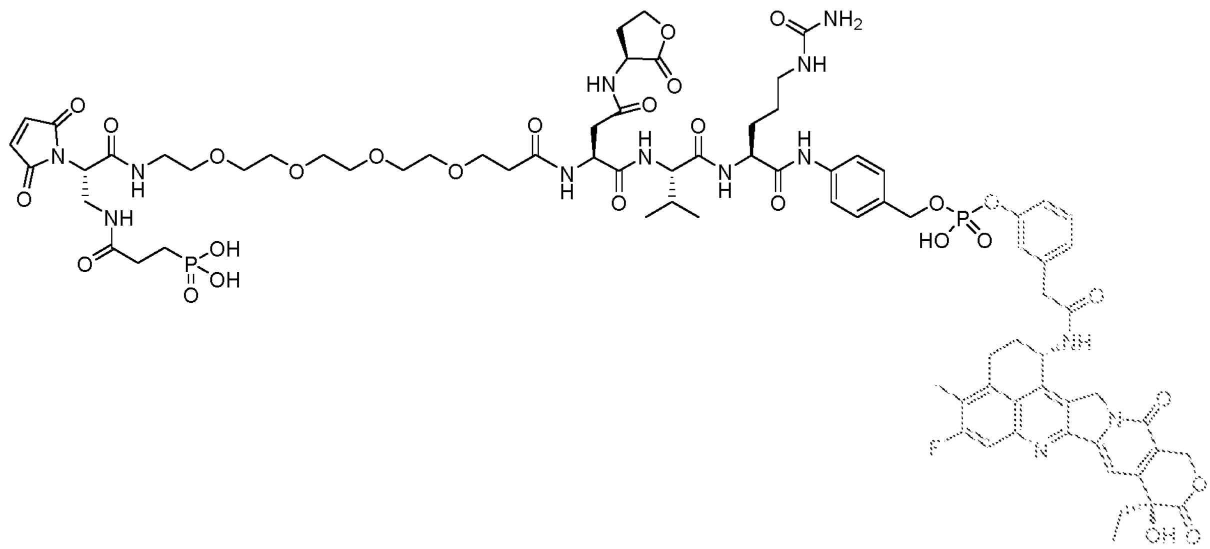

The Example A3-6 (10.8 mg, 0.019 mmoL) and the Example A20-3 (6.8 mg, 4.75 μmoL) instead of the Example A9-2 were reacted in the same manner as the Example A9 to give the title compound (5.16 mg, yield: 61.76%) as colorless solids.
MS (ESI) m/z 880 $(M+2H)^{2+}$

Synthesis of Example A21

Example A21

[C424]

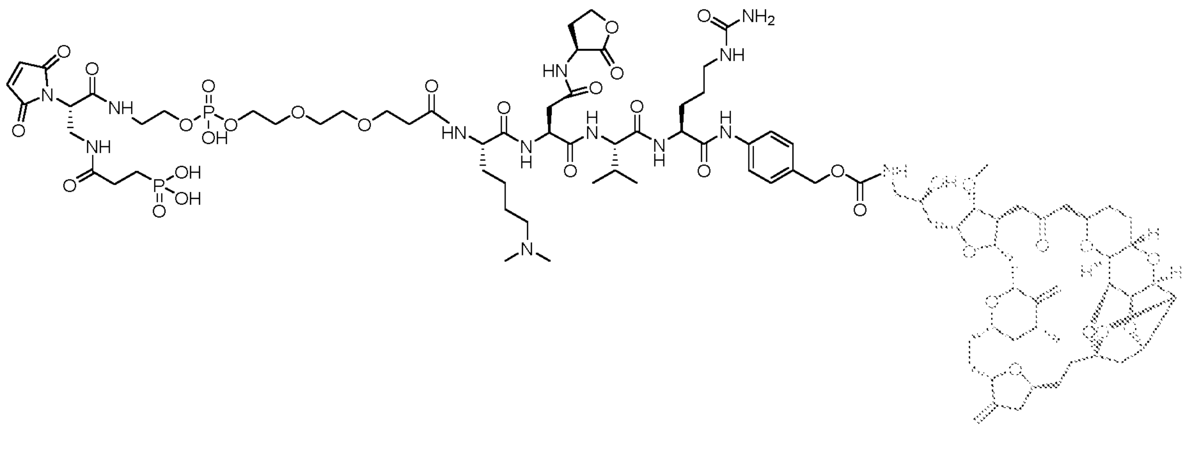

The Example A18-1 (8.0 mg, 4.67 μmoL) instead of the Example A9-2 and the Example A17-4 (15.2 mg, 0.025 mmoL) instead of the Example A3-6 were reacted in the same manner as the Example A9 to give the title compound (4.3 mg, yield: 44.34%) as a colorless amorphous.
MS (ESI) m/z 1036 $(M-2H)^{2-}$

Synthesis of Example A22

Example A22

[C425]

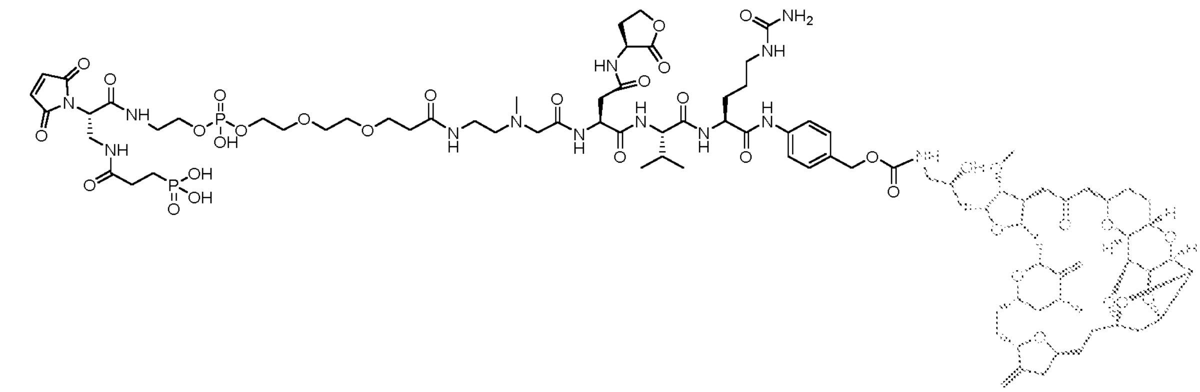

The Example A16-2 (6.8 mg, 4.07 μmoL) instead of the Example A9-2 and the Example A17-4 (6.2 mg, 10.27 μmoL) instead of the Example A3-6 were reacted in the same manner as the Example A9 to give the title compound (2.1 mg, yield: 25.37%) as colorless solids.
MS (ESI) m/z 1016 $(M+2H)^{2+}$

Synthesis of Example A23

[C426]

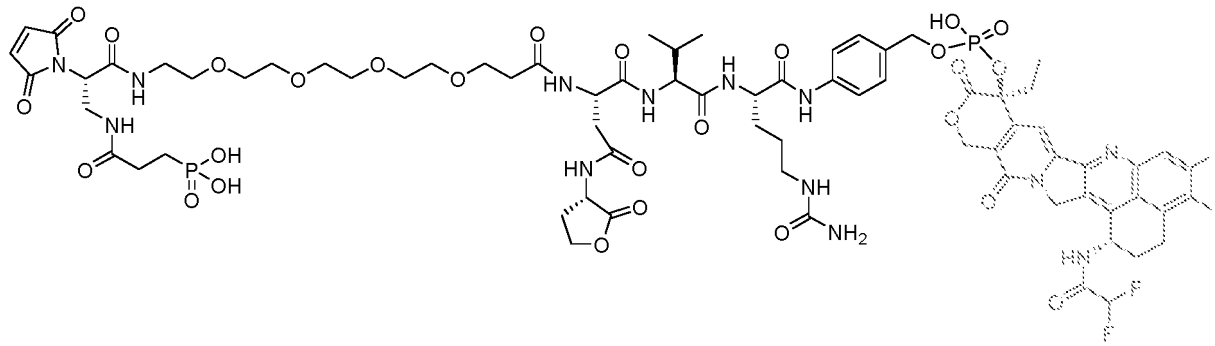

Example A23-1

[C427]

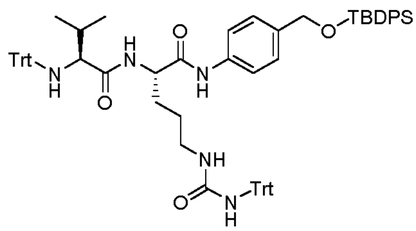

To a solution of (L)-Val-(L)-Cit-PAB (10.0 g, 26.4 mmoL) in tetrahydrofuran (100 mL) in a 500 mL round-bottom flask was added 1H-imidazole (3.59 g, 52.7 mmoL) under argon gas flow with stirring, then the resulting mixture was ice-cooled, tert-butyldiphenylsilyl chloride (7.45 mL, 7.97 g, 29.0 mmoL) was added thereto, and the resulting mixture was stirred at room temperature for 1.5 hours.

Subsequently, tert-butyldiphenylsilyl chloride (0.5 mL, 0.54 g, 1.946 mmoL) was added thereto under stirring under ice-cooling, and the resulting mixture was stirred at room temperature for 1 hour.

After the reaction was completed, to the reaction solution was added ethyl acetate (300 mL), the resulting insoluble matters were filtered, and washed with ethyl acetate (15 mL). To the resulting filtrate were added a saturated aqueous solution of sodium hydrogen carbonate (100 mL) and saturated brine (100 mL), the resulting mixture was stirred at room temperature for 15 minutes, then separated, and the resulting aqueous layer was subjected to extraction with ethyl acetate (20 mL) twice. The resulting organic layers were combined, dried over anhydrous sodium sulfate (5 g), filtered, and the resulting filtrate was concentrated under reduced pressure.

The resulting solids were added to tetrahydrofuran (150 mL), a saturated aqueous solution of ammonium chloride (150 mL) and water (20 mL) were added thereto, and the resulting mixture was separated. The resulting aqueous layer was subjected to extraction with ethyl acetate (20 mL) twice, washed with saturated brine (50 mL), the resulting organic layers were combined, dried over sodium sulfate (5 g), and then concentrated under reduced pressure.

To a solution of the resulting residues in dichloromethane (200 mL) were added triethylamine (36.7 mL, 26.64 g, 263 mmoL) and trityl chloride (36.7 g, 132 mmoL) under ice-cooling, and then the resulting mixture was stirred at room temperature for 1 hour. Subsequently, trityl chloride (36.7 g, 132 mmoL) was added thereto under stirring at room temperature, and the resulting mixture was stirred at room temperature for 1 hour.

Subsequently, triethylamine (36.7 mL, 26.64 g, 263 mmoL) and trityl chloride (36.7 g, 132 mmoL) were added thereto under stirring at room temperature, and the resulting mixture was stirred at room temperature for 1 hour.

Subsequently, trityl chloride (15.0 g, 53.8 mmoL) was added thereto under stirring at room temperature, and the resulting mixture was stirred at room temperature for 30 minutes.

After the reaction was completed, water (40 mL) was added thereto, the resulting mixture was separated, and the resulting aqueous layer was subjected to extraction with dichloromethane (20 mL) twice. The resulting organic layers were combined, washed with saturated brine, dried over anhydrous sodium sulfate, filtered, the resulting filtrate was concentrated under reduced pressure, and the precipitated solids were filtered to give a solution comprising the target compound.

The above solution was concentrated under reduced pressure, the concentration was stopped when white solids were precipitated, and the resulting mixture was stirred under ice-cooling for 30 minutes. The resulting solids were filtered, washed with a mixed solvent (50 mL) of hexane / ethyl acetate (= 70/30 (V/V)), and dried under reduced pressure to give the title compound (30.2459 g, yield: quantitative) as white solids.

MS (ESI) m/z 1103 $(M+H)^+$

Example A23-2

[C428]

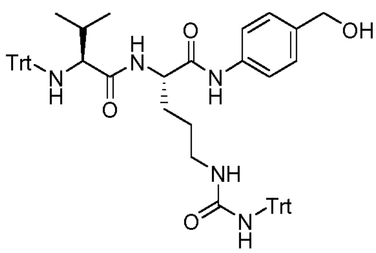

To a solution of the Example A23-1 (30.0 g, 27.2 mmoL) in tetrahydrofuran (270 mL) in a 500 mL round-bottom flask was added 1N tetra-n-butylammonium fluoride (77 mL) at room temperature, and then the resulting mixture was stirred at room temperature for 5 hours.

After the reaction was completed, the reaction solution was concentrated under reduced pressure, the resulting residues were dissolved in dichloromethane (30 mL), the resulting solution was subjected to YAMAZEN medium pressure flash chromatography (Silica, 3L (135 g), dichloromethane /ethyl acetate = 92/8 (V/V) → 44/56 (V/V)), and the fractions comprising the target compound (Rf = 0.45 (dichloromethane /ethyl acetate = 50/50 (V/V))) were collected. The precipitated solids were filtered, washed with dichloromethane, and dried under reduced pressure to give the title compound (16.2604 g, yield: 69.16%) as white solids.
MS (ESI) m/z 864 $(M+H)^+$

Example A23-3

[C429]

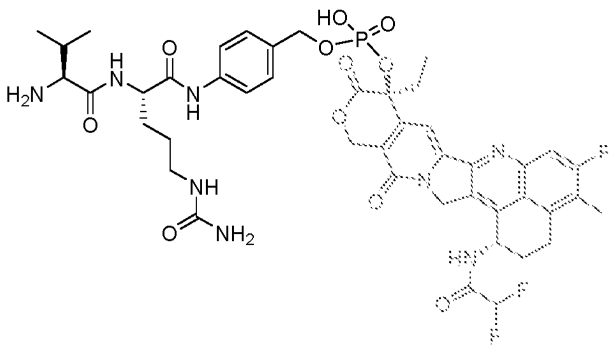

To a solution of the Example A23-2 (434 mg, 0.502 mmoL) in dichloromethane (15 mL) in a 50 mL round-bottom flask were sequentially added 1-allyloxy N,N,N',N'-tetraisopropylphosphinediamine (190 μL, 171.57 mg, 0.595 mmoL) and 1H-tetrazole (44 mg, 0.628 mmoL) under argon atmosphere with stirring at room temperature, and the resulting mixture was stirred at room temperature for 1 hour.

Subsequently, U-032 (99 mg, 0.193 mmoL) and 5-(ethylthio)-1H-tetrazole (80 mg, 0.615 mmoL) were added thereto, and the resulting mixture was stirred under heating at 43°C for 3 hours.

Subsequently, the mixture was ice-cooled, a 70% aqueous solution of tert-butyl hydroperoxide (145 μL, 136.3 mg, 1.059 mmoL) was added thereto under ice-cooling, and the resulting mixture was stirred under ice-cooling for 1 hour.

After the reaction was completed, to the reaction solution was added water (10 mL), and the resulting mixed solution was subjected to extraction with methylene chloride (15 mL). The resulting organic layer was washed with water, then dried over anhydrous magnesium sulfate, filtered, and concentrated under reduced pressure to give a pale yellow oil.

To a solution of the above pale yellow oil in tetrahydrofuran (15 mL) in a 100 mL round-bottom flask were sequentially added N-methylaniline (57 μL, 56.43 mg, 0.527 mmoL) and tetrakis(triphenylphosphine)palladium (47 mg, 0.041 mmoL) under argon atmosphere with stirring at room temperature, the resulting mixture was stirred at room temperature for 2 hours, and then the solvent was removed under reduced pressure.

To the resulting residues was added tert-butyl methyl ether, the resulting mixture was subjected to sonication, and the resulting supernatant was removed. Subsequently, to the resulting residues was added water, the resulting mixture was subjected to sonication, the resulting supernatant was removed, and dried under reduced pressure to give residues (770 mg).

To a solution of the above residues in dichloromethane (10 mL) in a 100 mL round-bottom flask was added trifluoroacetic acid (166 μL, 245.68 mg, 2.155 mmoL) at room temperature, and the resulting mixture was stirred at room temperature for 3 hours. Subsequently, trifluoroacetic acid (83 μL, 122.84 mg, 1.077 mmoL) was added thereto at room temperature, and the resulting mixture was stirred at room temperature for 1 hour.

After the reaction was completed, tert-butyl methyl ether (20 mL) was added thereto, and the resulting supernatant was removed. The resulting residues were dissolved in a 30% aqueous solution of acetonitrile, the resulting solution was subjected to preparative HPLC under the following conditions, and the fraction comprising the target compound was freeze-dried to give the title compound (65.1 mg, yield: 35.35%) as slightly yellow solids.
MS (ESI) m/z 955 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 60% (6.00 min.) - 90% (6.50 min.) → 90% (9.00 min.)

Example A23-4

[C430]

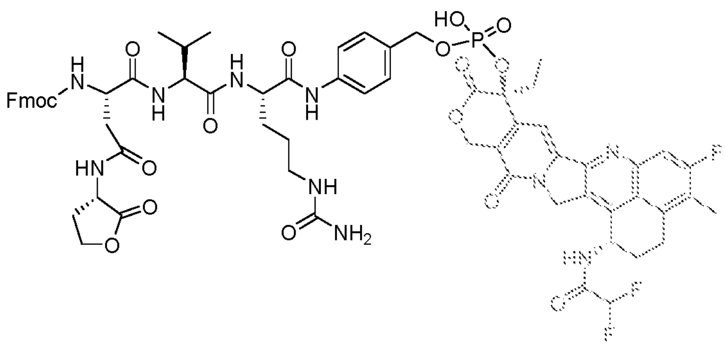

To a solution of the Example A1-2 (39 mg, 0.089 mmoL) in acetonitrile (1.2 mL) in a 20 mL cylindrical flask were added triethylamine (14 μL, 10.16 mg, 0.100 mmoL) and HATU (33 mg, 0.087 mmoL) under argon gas flow with stirring at room temperature, and the resulting mixture was stirred at room temperature for 15 minutes.

Subsequently, a solution of the Example A23-3 (65 mg, 0.068 mmoL) and triethylamine (10 μL, 7.26 mg, 0.072 mmoL) in N,N-dimethylformamide (1.8 mL) was added thereto under stirring at room temperature, and the resulting mixture was stirred at room temperature for 0.5 hour.

Subsequently, the Example A1-2 (12 mg, 0.027 mmoL), HATU (5.0 mg, 0.013 mmoL), and triethylamine (8 μL, 5.81 mg, 0.057 mmoL) were added thereto under stirring at room temperature, and the resulting mixture was stirred at room temperature for 0.5 hour.

After the reaction was completed, the reaction solution was concentrated under reduced pressure. To the resulting residues were added water (4 mL) and acetonitrile (3 mL), the precipitated solids were filtered, washed with ethyl acetate, and dried under reduced pressure to give the title compound (60 mg, yield: 64.09%) as pale yellow solids.
MS (ESI) m/z 1376 $(M+H)^+$

Example A23-5

[C431]

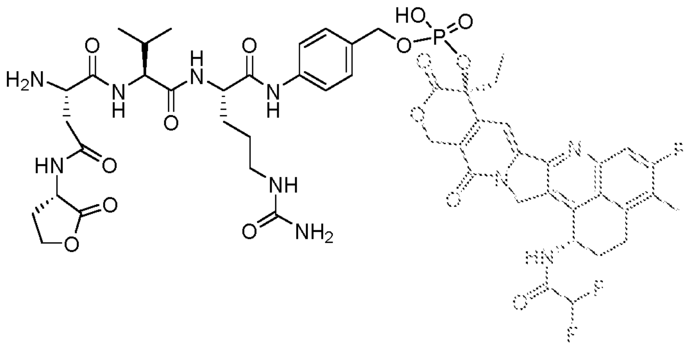

To a solution of the Example A23-4 (60 mg, 0.044 mmoL) in N,N-dimethylformamide (1 mL) in a 10 mL cylindrical flask was added piperidine (0.013 mL, 11.14 mg, 0.131 mmoL) under argon gas flow with stirring at room temperature, the resulting mixture was stirred at room temperature for 1 hour, and then the solvent was removed under reduced pressure.

The resulting residues were washed with ethyl acetate and then with diethyl ether, and dried under reduced pressure to give the title compound (45 mg, yield: 89.46%) as dark brown solids.
MS (ESI) m/z 1154 $(M+H)^+$

Example A23

[C432]

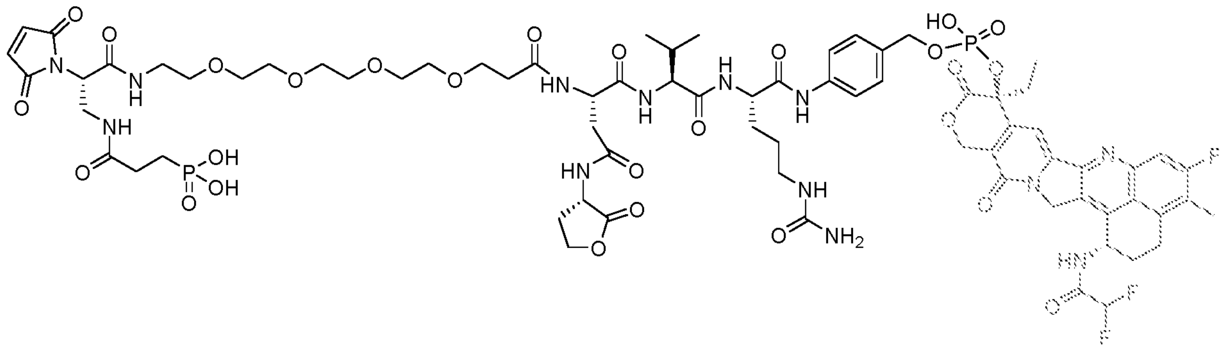

The Example A3-6 (12.5 mg, 0.022 mmoL) and the Example A23-5 (12 mg, 10.41 μmoL) instead of the Example A3-7 were reacted in the same manner as the Example A3 to give the title compound (2.8 mg, yield: 15.8%) as white solids.
MS (ESI) m/z 852 $(M+2H)^{2+}$

Synthesis of Example A24

[C433]

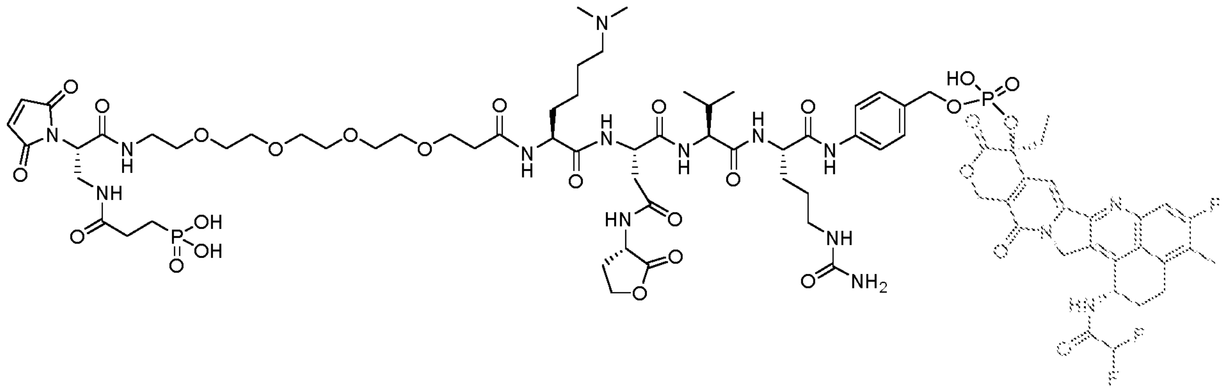

Example A24-1

[C434]

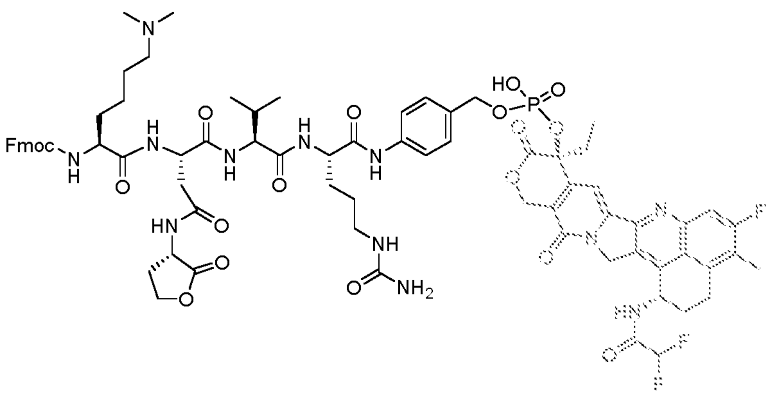

To a solution of the Example A23-4 (49 mg, 0.036 mmoL) in N,N-dimethylformamide (1 mL) in a 10 mL cylindrical flask was added piperidine (10 μL, 8.6 mg, 0.101 mmoL) under argon gas flow with stirring at room temperature, the resulting mixture was stirred at room temperature for 1 hour, then the solvent was removed under reduced pressure, the resulting residues were washed with ethyl acetate and then with diethyl ether, and the resulting solids were dried under reduced pressure.

To a solution of N2-(((9H-fluoren-9-yl)methoxy)carbonyl)-N6,N6-dimethyl-L-lysinehydrochloride (27 mg, 0.068 mmoL) in acetonitrile (1.2 mL) in a 10 mL cylindrical flask were added triethylamine (10 μL, 7.2 mg, 0.072 mmoL) and HATU (18 mg, 0.047 mmoL) under argon gas flow with stirring at room temperature, and the resulting mixture was stirred at room temperature for 15 minutes.

Subsequently, a solution of the above resulting solids and triethylamine (5 μL, 3.63 mg, 0.036 mmoL) in N,N-dimethylformamide (1 mL) was added thereto under stirring at room temperature, the resulting mixture was stirred at room temperature for 1 hour, and then the solvent was removed under reduced pressure. To the resulting residues was added diethyl ether, the resulting mixture was subjected to sonication, then the resulting supernatant was removed, and the resulting solids were dried under reduced pressure to give the title compound (68 mg, yield: quantitative) as dark brown solids. MS (ESI) m/z 767 $(M+2H)^{2+}$

Example A24-2

[C435]

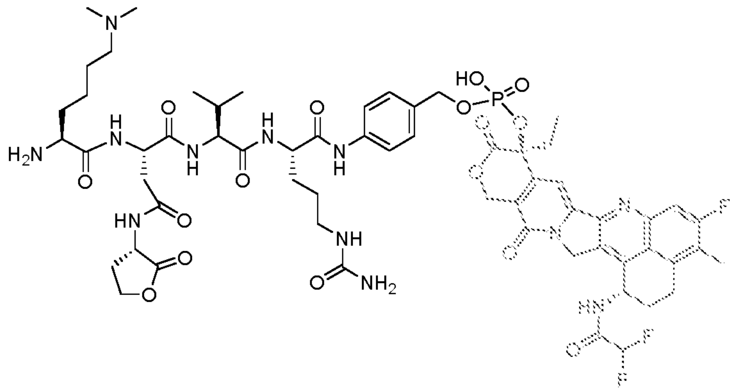

To a solution of the Example A24-1 (68 mg, 0.044 mmoL) in N,N-dimethylformamide (1 mL) in a 20 mL cylindrical flask was added piperidine (10 μL, 8.6 mg, 0.101 mmoL) under argon gas flow with stirring at room temperature, the resulting mixture was stirred at room temperature for 1 hour, and then the solvent was removed under reduced pressure. The resulting residues were subjected to preparative HPLC under the following conditions, and the fraction comprising the target compound was freeze-dried to give the title compound (12 mg, yield: 20.64%) as white solids.
MS (ESI) m/z 655 $(M+2H)^{2+}$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 50% (6.00 min.) - 90% (6.50 min.) → 90% (9.00 min.)

Example A24

[C436]

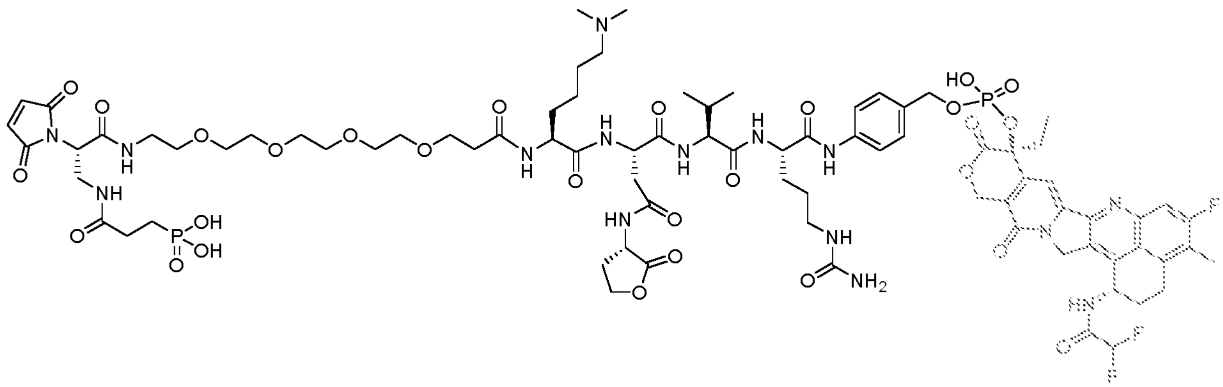

The Example A3-6 (13 mg, 0.023 mmoL) and the Example A24-2 (12 mg, 9.17 μmoL) instead of the Example A3-7 were reacted in the same manner as the Example A3 to give the title compound (8.6 mg, yield: 50.48%) as white solids.
MS (ESI) m/z 930 $(M+2H)^{2+}$

Synthesis of Example A25

[C437]

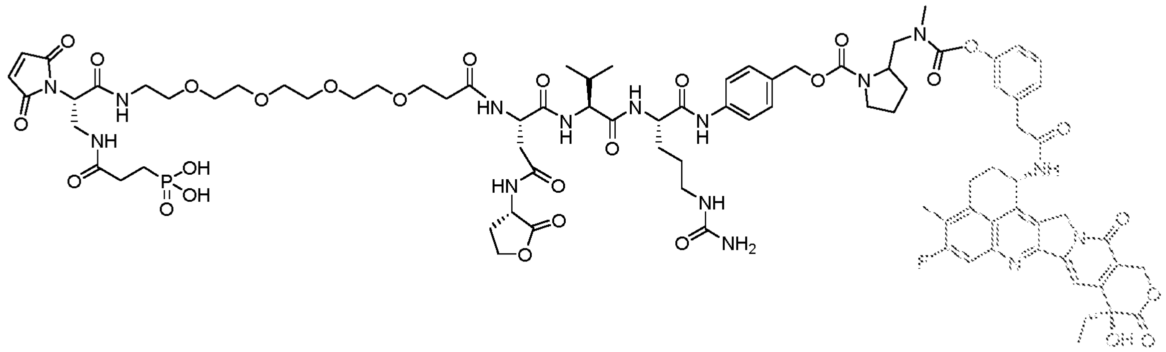

Example A25-1

[C438]

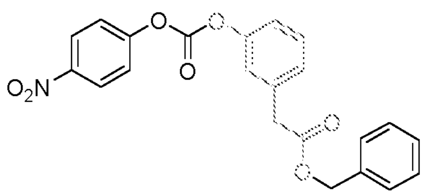

To a solution of benzyl 2-(3-hydroxyphenyl)acetate (3.40 g, 14.03 mmoL) in N,N-dimethylformamide (45 mL) in a 100 mL round-bottom flask were added bis(4-nitrophenyl)carbonate (4.70 g, 15.45 mmoL) and N,N-diisopropylethylamine (4.90 mL, 3.63 g, 28.1 mmoL) under argon gas flow with stirring at room temperature, and the resulting mixture was stirred at room temperature for 3 hours.

After the reaction was completed, the solvent was concentrated under reduced pressure. To the resulting residues was added water, and the resulting mixed solution was subjected to extraction with ethyl acetate. The resulting organic layer was washed with a saturated aqueous solution of sodium hydrogen carbonate and then with water three times, and concentrated under reduced pressure. To the resulting residues were added ethyl acetate and hexane, the precipitated solids were collected by filtration, and washed with a mixed solvent of hexane / ethyl acetate (= 4/1 (V/V)) to give the title compound (5.88 g, yield: quantitative) as slightly yellow solids.

Example A25-2

[C439]

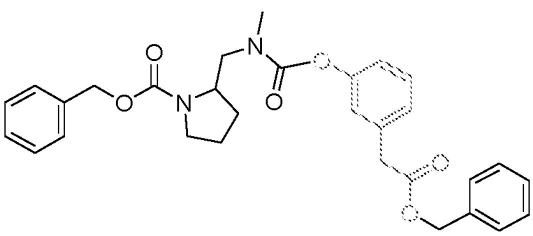

To a solution of the Example A25-1 (149.8 mg, 0.368 mmoL) and benzyl (S)-2-((methylamino)methyl)pyrrolidine-1-carboxylate trifluoroacetate (Angew Chem Int Ed Engl. 2020 Mar 2; 59(10): 4176-41) (112.3 mg, 0.294 mmoL) in dichloromethane (2 mL) in a 30 mL round-bottom flask was added N,N-diisopropylethylamine (0.22 mL, 162.8 mg, 1.260 mmoL) under argon gas flow with stirring at room temperature, and the resulting mixture was stirred at room temperature

for 2 hours.

After the reaction was completed, the reaction mixture was concentrated under reduced pressure. The resulting residues were dissolved in N,N-dimethylformamide (5 mL), the resulting solution was subjected to preparative HPLC under the following conditions, and the fraction comprising the target compound was concentrated under reduced pressure to give the title compound (45.5 mg, yield: 29.91%) as a colorless oil.
MS (ESI) m/z 517 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 95% (10.00 min.)

Example A25-3

[C440]

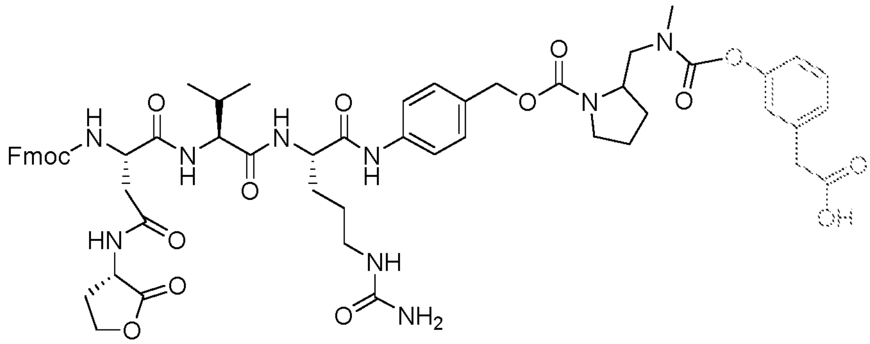

A solution of the Example A25-2 (45.6 mg, 0.088 mmoL), methanesulfonic acid (6.0 μL, 8.88 mg, 0.092 mmol), and 10% palladium carbon NX-Type (wetted with 50% water) (19.9 mg, 9.35 μmoL) in N,N-dimethylformamide (2 mL) in a 30 mL round-bottom flask was stirred under hydrogen atmosphere.

Subsequently, the Example A2-2 (101.1 mg, 0.105 mmoL) and N,N-dimethylformamide (2 mL) were added thereto, N,N-diisopropylethylamine (61 μL, 45.14 mg, 0.349 mmoL) was added thereto with stirring at room temperature, and the resulting mixture was stirred at room temperature for 2 hours.

The resulting reaction solution was subjected to preparative HPLC under the following conditions, and the fraction comprising the target compound was concentrated under reduced pressure to give the title compound (24.6 mg, yield: 24.92%) as a colorless oil.
MS (ESI) m/z 1119 $(M+H)^+$

Column: Waters XBridge Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 30% (0.00 min.) → 80% (10.00 min.)

Example A25-4

[C441]

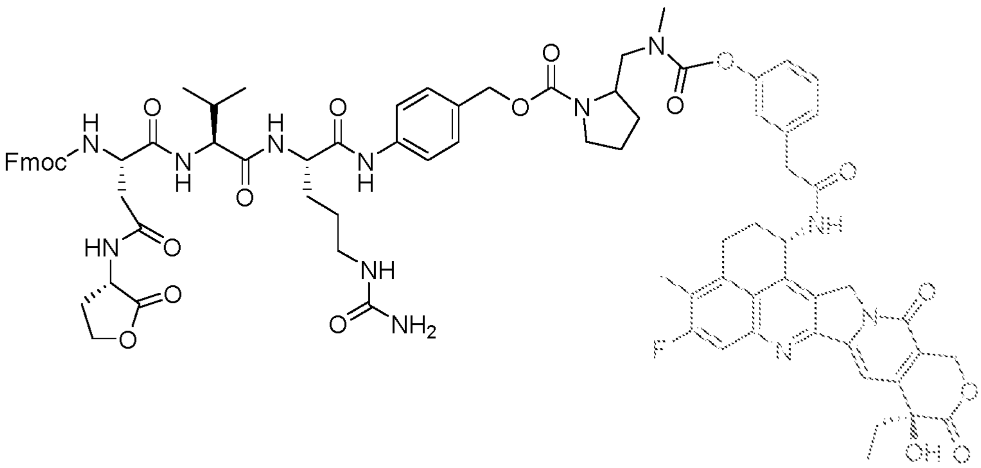

To a solution of the Example A25-3 (23.6 mg, 0.021 mmoL) and exatecan mesylate (10.5 mg, 0.020 mmoL) in N,N-dimethylformamide (2 mL) in a 30 mL round-bottom flask were added triethylamine (8 μL, 5.81 mg, 0.057 mmoL) and HATU (8.6 mg, 0.023 mmoL), and the resulting mixture was stirred at room temperature for 2 hours.

The resulting reaction solution was subjected to preparative HPLC under the following conditions, and the fraction comprising the target compound was concentrated under reduced pressure to give the title compound (26.2 mg, yield: 86.37%) as a colorless oil.
MS (ESI) m/z 769 $(M+2H)^{2+}$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 40% (0.00 min.) → 80% (8.00 min.)

Example A25

[C442]

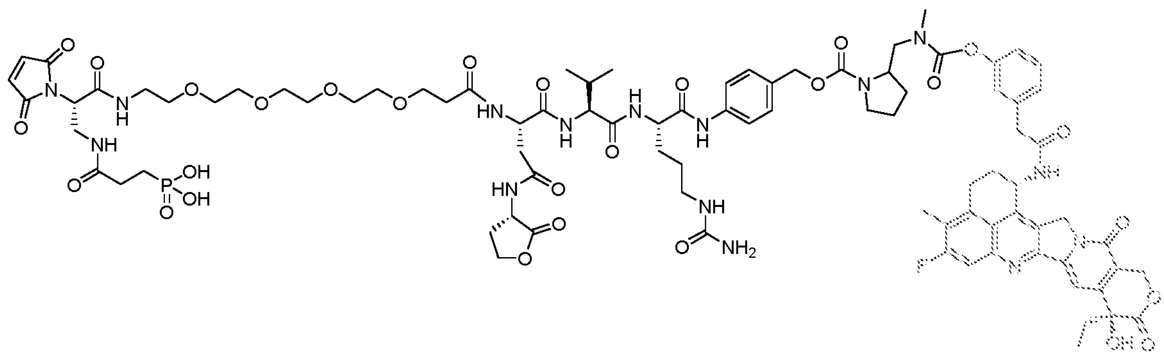

The Example A3-6 (11.9 mg, 0.021 mmoL) and the Example A25-4 (10.0 mg, 6.51 μmoL) instead of the Example A9-2 were reacted in the same manner as the Example A9 to give the title compound (3.8 mg, yield: 30.91%) as colorless solids.
MS (ESI) m/z 932 $(M+2H)^2$

Synthesis of Example A26

[C443]

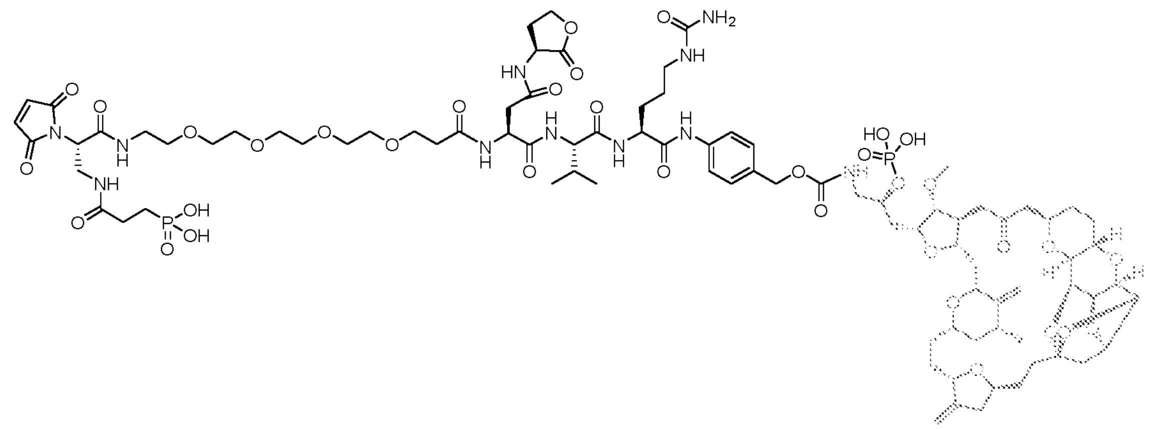

Example A26-1

[C444]

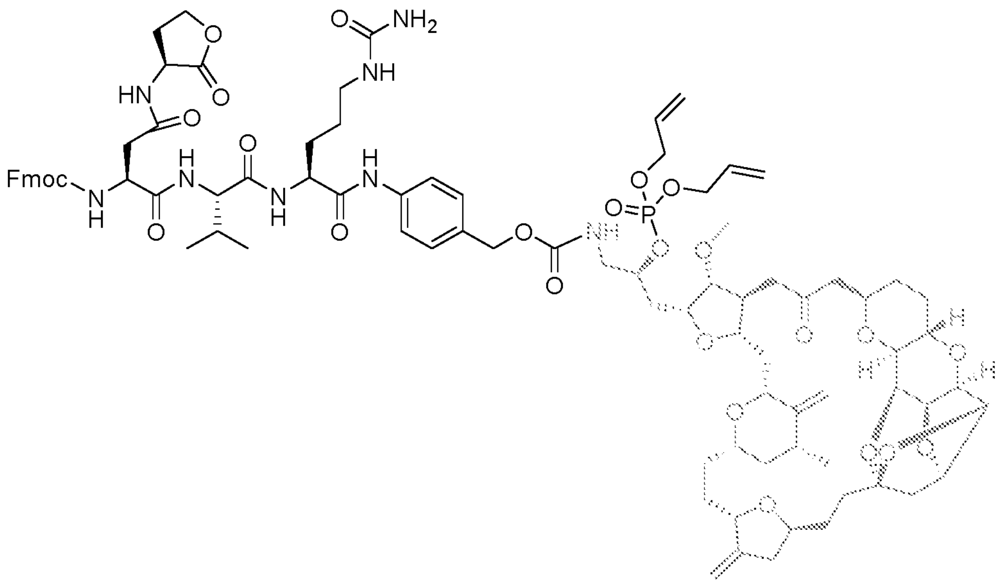

To a solution of the Example A14-1 (19.2 mg, 0.012 mmoL) in N,N-dimethylformamide (0.8 mL) in a 5 mL sample tube was added diallyl N,N-diisopropylphosphoramidite (49 μL, 45.47 mg, 0.185 mmoL) under argon atmosphere with stirring, and then added a solution of 1H-tetrazole (13.0 mg, 0.186 mmoL) in N,N-dimethylformamide (0.2 mL) at room temperature, and the resulting mixture was stirred at room temperature for 1 hour.

Subsequently, 30% by weight of hydrogen peroxide water (19 μL, 21.09 mg, 0.186 mmoL) was added thereto, and the resulting mixture was stirred at room temperature for 1 hour.

The resulting reaction solution was subjected to preparative HPLC under the following conditions, and the fraction comprising the target compound was freeze-dried to give the title compound (10.2 mg, yield: 48.17%) as colorless foam.

MS (ESI) m/z 859 (M+2H)2

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 95% (10.00 min.)

Example A26-2

[C445]

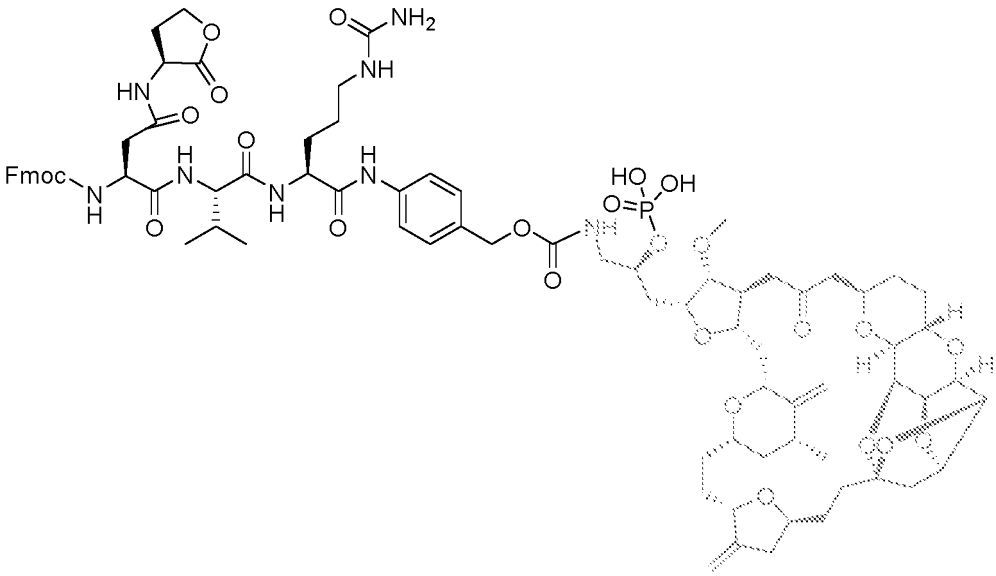

To a solution of the Example A26-1 (15.1 mg, 8.80 μmoL, including those produced by the same method as the Example A26-1) in N,N-dimethylformamide (0.5 mL) in a 5 mL sample tube were sequentially added N-methylaniline (3.5 μL, 3.47 mg, 0.032 mmoL) and tetrakis(triphenylphosphine)palladium (2.0 mg, 1.731 μmoL) under argon atmosphere with stirring at room temperature, and the resulting mixture was stirred at room temperature for 2 hours.

The resulting reaction solution was subjected to preparative HPLC under the following conditions, and the fraction comprising the target compound was freeze-dried to give the title compound (9.3 mg, yield: 64.61%) as colorless solids.

MS (ESI) m/z 819 (M+2H)2

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 70% (8.00 min.)

Example A26

[C446]

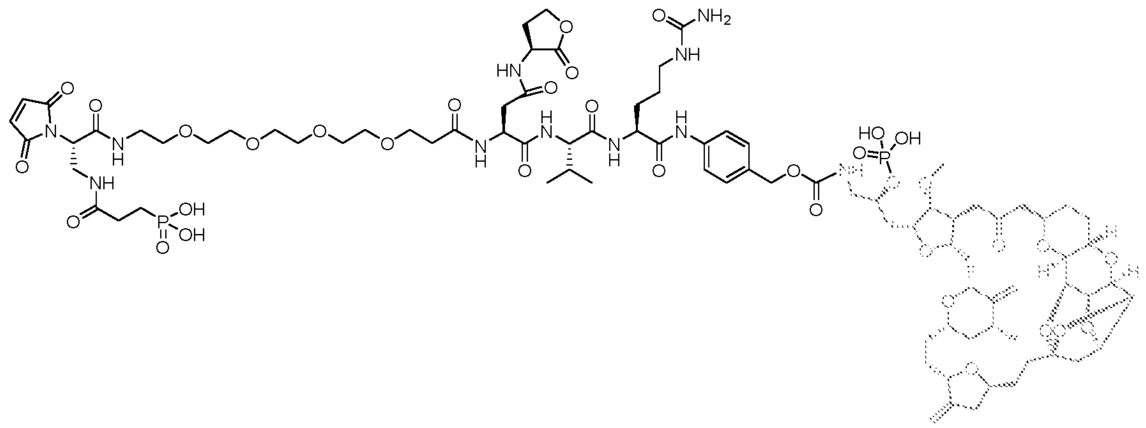

The Example A3-6 (11.9 mg, 0.021 mmoL) and the Example A26-2 (9.3 mg, 5.69 μmoL) instead of the Example A9-2 were reacted in the same manner as the Example A9 to give the title compound (5.4 mg, yield: 48.39%) as colorless solids.
MS (ESI) m/z 982 $(M+2H)^{2}$

Synthesis of Example A27

Example A27

[C447]

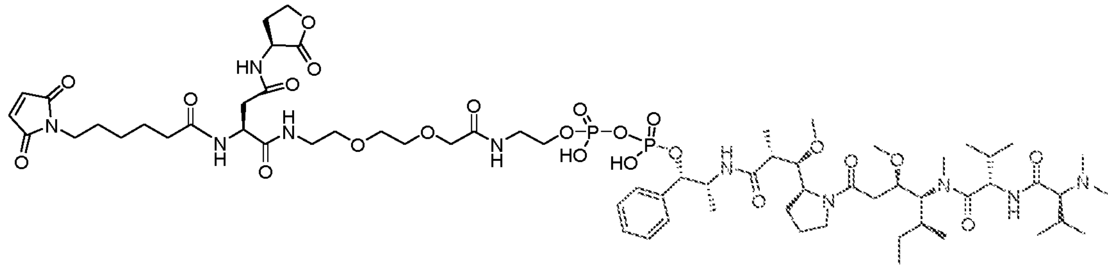

N-succinimidyl 6-maleimidohexanoate (9.7 mg, 0.031 mmoL) instead of N-succinimidyl 1-maleimide-3-oxo-7,10,13,16-tetraoxa-4-azanonadecanoate and the Example A19-10 (20 mg, 0.016 mmoL) instead of the Example A1-7 were reacted in the same manner as the Example A1 to give the title compound (11.1 mg, yield: 48.21%) as colorless solids.
MS (DUIS) m/z 1469 $(M-H)^{-}$

Synthesis of Example A28

Example A28

[C448]

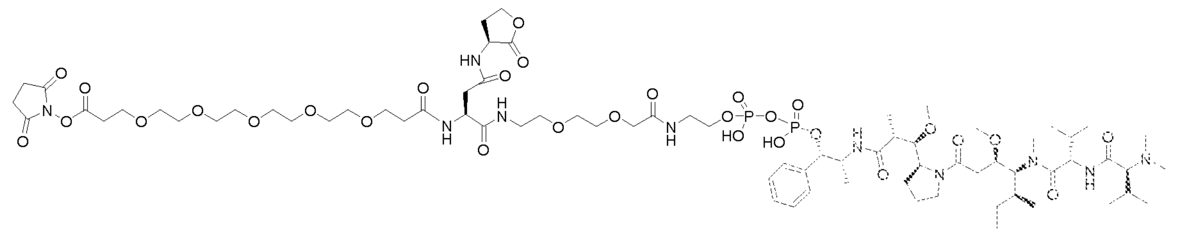

Bis(2,5-dioxopyrrolidin-1-yl) 4,7,10,13,16-pentaoxanonadecanedioate (16.6 mg, 0.031 mmoL) instead of N-succinimidyl 1-maleimide-3-oxo-7,10,13,16-tetraoxa-4-azanonadecanoate and the Example A19-10 (20 mg, 0.016 mmoL) instead of the Example A1-7 were reacted in the same manner as the Example A1 to give the title compound (3.9 mg, yield: 14.85%) as colorless solids.
MS (ESI) m/z 847 $(M-2H)^{2-}$

Synthesis of Example A29

Example A29

[C449]

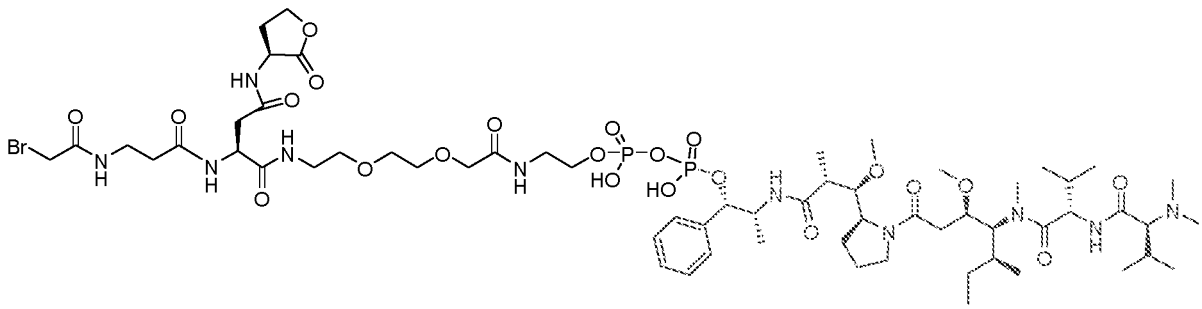

N-succinimidyl 3-(bromoacetoamide)propionate (9.61 mg, 0.031 mmoL) instead of N-succinimidyl 1-maleimide-3-oxo-7,10,13,16-tetraoxa-4-azanonadecanoate and the Example A19-10 (20 mg, 0.016 mmoL) instead of the Example A1-7 were reacted in the same manner as the Example A1 to give the title compound (3.9 mg, yield: 16.95%) as white solids.
MS (DUIS) m/z 736 $(M+2H)^{2+}$

[Examples]: Synthesis of conjugate precursor (I) and conjugate precursor synthesis intermediates (II-1) and (II-2)

Synthesis of Example 1

[C450]

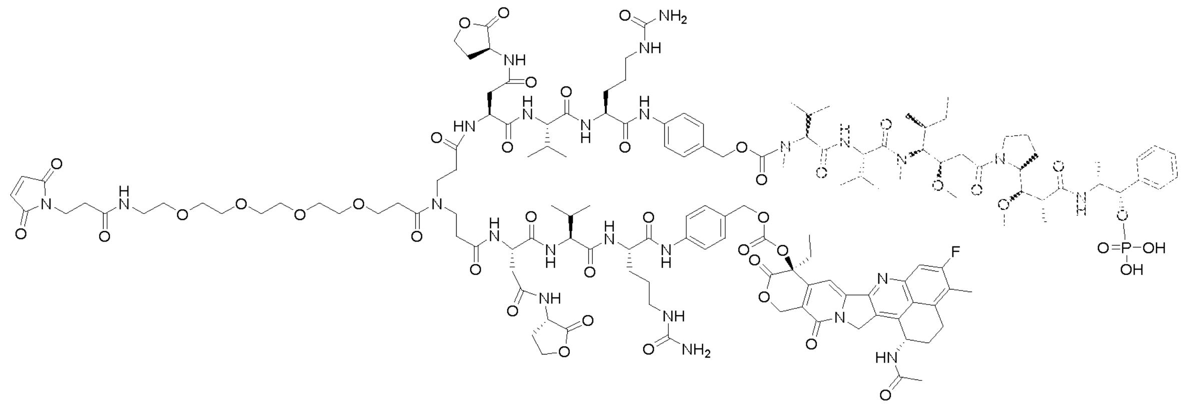

Example 1-1

[C451]

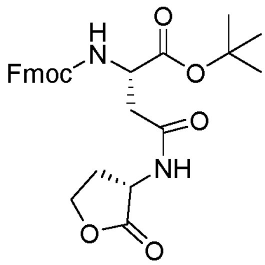

(S)-(-)-α-amino-gamma-butyrolactone hydrochloride (1.75 g, 12.72 mmoL) put into a 200 mL eggplant flask was dissolved in N,N-dimethylformamide (60 mL), Fmoc-Asp-tert-butyl (5.23 g, 12.71 mmoL) was then added, and triethylamine (3.65 mL) (2.65 g, 26.2 mmoL) was added. Next, HATU (4.83 g, 12.70 mmoL) was added at room temperature, and the mixture was stirred at room temperature for 2 hours. After the reaction was completed, water was added to the reaction solution, and the mixed solution was extracted twice with ethyl acetate. The organic layer was concentrated under reduced pressure to obtain a title compound (6.16 g, yield 97.9%) as a colorless oil.

Example 1-2

[C452]

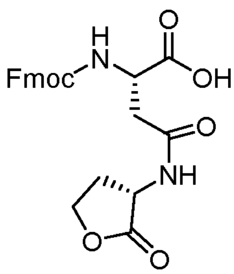

Example 1-1 (6.16 g, 12.46 mmoL) put into a 500 mL eggplant flask was dissolved in dichloromethane (60 mL), trifluoroacetic acid (60 mL) (1.421 g, 12.46 mmoL) was then added, the mixture was stirred at room temperature for 2 hours, and after the reaction was completed, the solvent was distilled off under reduced pressure. The resulting residues

were dissolved in acetonitrile (50 mL), and ethyl acetate (50 mL) was additionally added thereto. The precipitated solids were collected by filtration and washed with ethyl acetate to obtain a title compound (3.86 g, yield 70.68%) as a colorless solid.
MS(ESI)m/z 439$(M+H)^+$

Example 1-3

[C453]

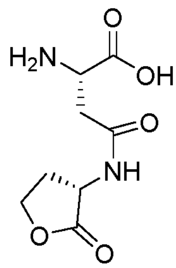

To a solution of Example 1-2 (298 mg, 0.680 mmoL) in N,N-dimethylformamide (4 mL) put into a 30 mL cylindrical flask, under argon airflow with stirring, piperidine (0.270 mL) (232.2 mg, 2.73 mmoL) was added at room temperature, and the mixture was stirred at room temperature for 1 hour.

After the reaction was completed, xylene was added, and the solvent was removed under reduced pressure. Diethyl ether (4 mL) and ethyl acetate (8 mL) were added to the residues, the mixture was subjected to sonication, and solids were collected by filtration. A 20% acetonitrile aqueous solution was added to the collected solids, and the mixture was concentrated under reduced pressure. Ethyl acetate was added to the resulting residue and filtering was performed. Diethyl ether was added to the resulting solids, and the mixture was filtered and dried under reduced pressure to obtain a title compound (143 mg, yield 97.32%) as a white solid.
MS(ESI)m/z 215 (M-H)

·Example 1-4

[C454]

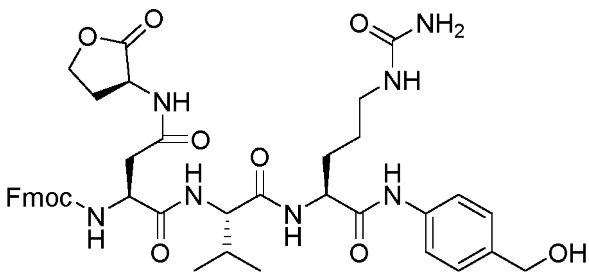

Val-Cit-PAB (1.90 g, 5.01 mmoL) and Example 1-2 (2.24 g, 5.11 mmoL) put into a 200 mL eggplant flask were suspended in N,N-dimethylformamide (50 mL), triethylamine (0.71 mL) (0.52 g, 5.09 mmoL) was added, HATU (1.94 g, 5.10 mmoL) was then added, and the mixture was stirred at room temperature for 1 hour. Water (30 mL) and acetone (20 mL) were added to the resulting reaction solution, the mixture was stirred for 30 minutes, then filtered, washed with water, and then washed with acetone to obtain a title compound (3.37 g, yield 84.13%) as a slightly yellow solid.
MS(ESI)m/z 800$(M+H)^+$

Example 1-5

[C455]

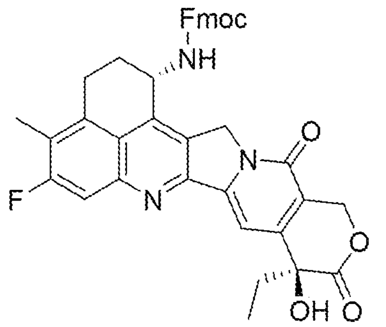

To a suspension of exatecan mesylate (200 mg, 0.376 mmoL) in acetonitrile (7.5 mL)/water (3.2 mL) put into a 50 mL eggplant flask, under argon airflow with stirring, sodium bicarbonate (152 mg, 1.809 mmoL) and 9-fluorenylmethyl chloroformate (117 mg, 0.452 mmoL) were added at room temperature, and the mixture was stirred at room temperature for 3 hours. After the reaction was completed, water (28 mL) was added to the reaction solution, and the mixture was stirred under ice-cooling 2 hours. The precipitated solids were separated and dried under reduced pressure to obtain a title compound (235 mg, yield 94.96%) as a slightly yellow solid.
MS(ESI)m/z 658$(M+H)^+$

Example 1-6

[C456]

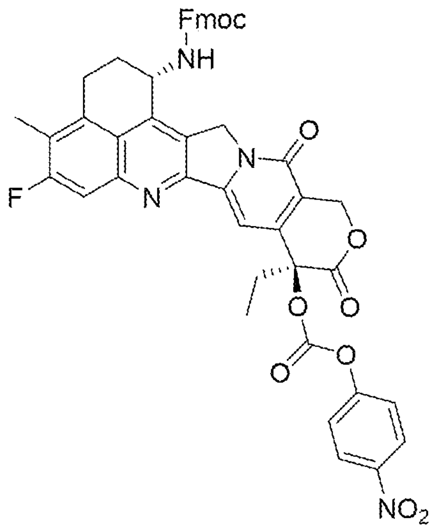

To a solution of Example 1-5 (235 mg, 0.357 mmoL) in dichloromethane (6 mL) put into a 30 mL cylindrical flask, under argon airflow with stirring, 4-nitrophenyl chloroformate (182 mg, 0.903 mmoL), triethylamine (0.149 mL) (108.17 mg, 1.069 mmoL), and 4-dimethylaminopyridine (65 mg, 0.532 mmoL) were added at room temperature, and the mixture was stirred at room temperature for 4 hours. After the reaction was completed, 0.05N hydrochloric acid (20 mL) was added to the reaction solution, and the mixed solution was extracted with dichloromethane (30 mL). The organic layer was washed with saturated brine, dried with anhydrous magnesium sulfate, filtered, and concentrated under reduced pressure. The resulting residues were subjected to YAMAZEN medium pressure flash chromatography (Rf value=0.29 (hexane/ethyl acetate=1/1(V/V)), (silica, M(16 g))), and fractions containing a target compound were concentrated under reduced pressure to obtain a title compound (247 mg, yield 84.02%) as a slightly yellow solid.
MS(ESI)m/z 823$(M+H)^+$

Example 1-7

[C457]

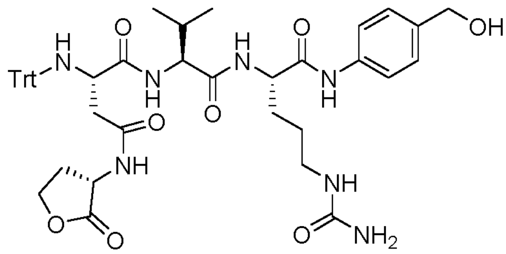

To a solution of Example 1-4 (1.496 g, 1.870 mmoL) in N,N-dimethylformamide (14 mL) put into a 100 mL eggplant flask, under argon airflow with stirring, piperidine (570 μL) (0.49 g, 5.76 mmoL) was added at room temperature, the mixture was stirred at room temperature for 1.5 hours, and the solvent was then removed under reduced pressure. The residues were washed with diethyl ether and dried under reduced pressure. To a solution of the residues in N,N-dimethylformamide (21 mL) put into a 100 mL eggplant flask, under argon airflow with stirring, potassium carbonate (519 mg, 3.76 mmoL) was added at room temperature, and the mixture was stirred at room temperature for 40 minutes. Next, trityl chloride (626 mg, 2.246 mmoL) was added at room temperature with stirring, and the mixture was stirred at room temperature for 1.5 hours. Next, trityl chloride (210 mg, 0.753 mmoL) was added at room temperature with stirring, and the mixture was stirred at room temperature for 1.5 hours. After the reaction was completed, the filtrate was concentrated under reduced pressure, the resulting residues were subjected to YAMAZEN medium pressure flash chromatography (silica, L(40 g), ethyl acetate/methanol=100/0(V/V)-+70/30(V/V), (Rf=0.18 (ethyl acetate/methanol=95/5(V/V)))), and fractions containing a target compound were concentrated under reduced pressure to obtain a title compound (886 mg, yield 57.77%) as a white solid.
MS(ESI)m/z 821(M+H)$^+$

Example 1-8

[C458]

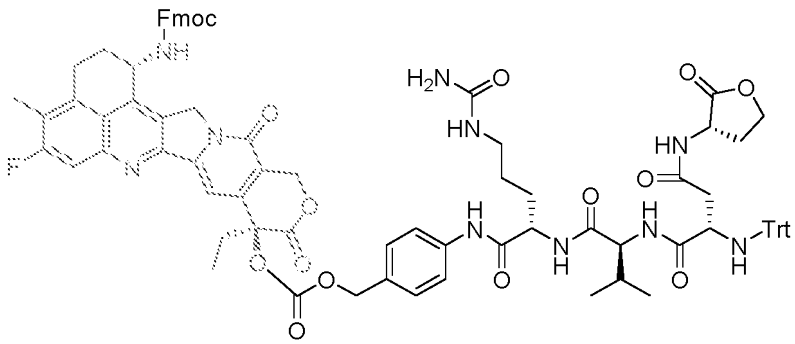

To a solution of Example 1-6 (758 mg, 0.921 mmoL) in dichloromethane (24 mL) put into a 50 mL eggplant flask, under argon airflow with stirring, Example 1-7 (886 mg, 1.081 mmoL), triethylamine (270 μL) (196.02 mg, 1.937 mmoL), and 4-dimethylaminopyridine (220 mg, 1.801 mmoL) were added at room temperature, and the mixture was stirred at room temperature for 9 hours. After the reaction was completed, saturated brine was added to the reaction solution, and the mixed solution was extracted with dichloromethane. The organic layer was dried with anhydrous magnesium sulfate, filtered, and concentrated under reduced pressure. The resulting residues were subjected to YAMAZEN medium pressure flash chromatography (silica, L(40 g), ethyl acetate/methanol=100/0(V/V)→70/30(V/V), (Rf=0.22 (ethyl acetate/methanol=95/5(V/V)))), and fractions containing a target compound were concentrated under reduced pressure to obtain a title compound (674 mg, yield 48.66%) as a slightly yellow solid.

MS(ESI)m/z 1262(M-Trt+H)+

Example 1-9

[C459]

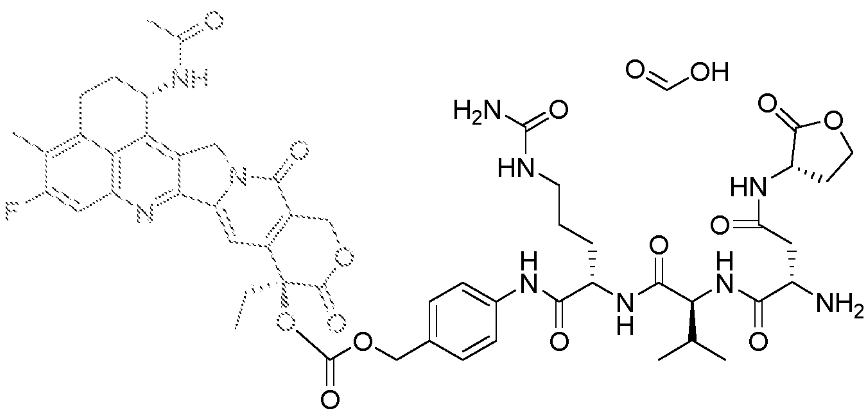

To a solution of Example 1-8 (15 mg, 9.98 μmoL) in N,N-dimethylformamide (1 mL) put into a 10 mL cylindrical flask, under air atmosphere with stirring, piperidine (10 μL) (8.6 mg, 101 μmoL) was added at room temperature, and the mixture was stirred at room temperature for 1 hour. After the reaction was completed, the mixture was concentrated under reduced pressure, dichloromethane (1 mL) was added to the residues, triethylamine 4 μL (2.9 mg, 0.029 mmoL) was added, acetic anhydride (3.0 μL) (3.24 mg, 0.032 mmoL) was then added at room temperature, and the mixture was stirred at room temperature for 36 hours. After the reaction was completed, the mixture was concentrated under reduced pressure, N,N-dimethylformamide (1 mL) was added to the residues, formic acid (0.5 mL) (600 mg, 27.6 mmoL) was then added, the mixture was stirred at room temperature for 1 hour, methanol (1 mL) was then added, and the mixture was concentrated under reduced pressure. The resulting residues were subjected preparative HPLC under the following conditions, and fractions containing a target compound were freeze-dried to obtain a title compound (4.9 mg, yield 43.58%) as a colorless solid.
MS(ESI)m/z 1081(M+H)+

Column: Waters XBrige Prep C18 5 μm ODB 19*150 mm
Flow rate: 17 mL/min.
Elution solvent: 0.1% formic acid aqueous solution (solution A)-acetonitrile (solution B)
Gradient (solution B): 20% (0.00 min.)→60% (10.00 min.)

Example 1-10

[C460]

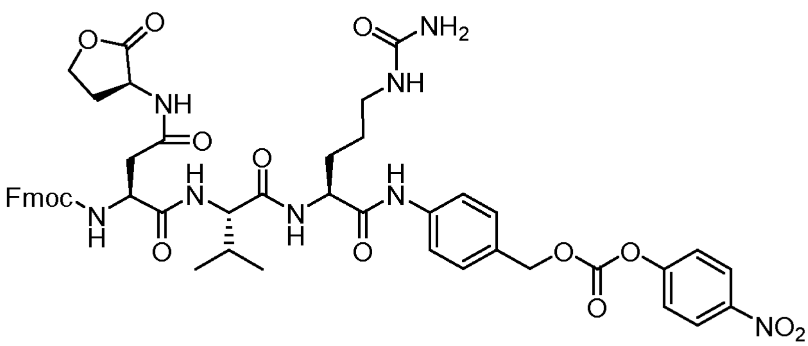

To a solution of Example 1-4 (1.027 g, 1.284 mmoL) in N,N-dimethylformamide (12 mL) put into a 100 mL eggplant flask, under argon airflow with stirring, bis(4-nitrophenyl)carbonate (760 mg, 2.498 mmoL) and N,N-diisopropylethylamine (440 μL) (0.33 g, 2.52 mmoL) were added at room temperature, and the mixture was stirred at room temperature for 3 hours. Next, N,N-dimethylformamide (8 mL) was added at room temperature with stirring, the mixture was stirred at room temperature for 0.5 hours, insoluble matters were then filtered, and the filtrate was concentrated under reduced pressure. Diethyl ether and hexane were added to the residues, the produced solids were filtered, washed with water four times and then washed with diethyl ether, and dried under reduced pressure to obtain a title compound (760 mg, yield 61.34%) as a slightly yellow solid.
MS(ESI)m/z 965$(M+H)^+$

Example 1-11

[C461]

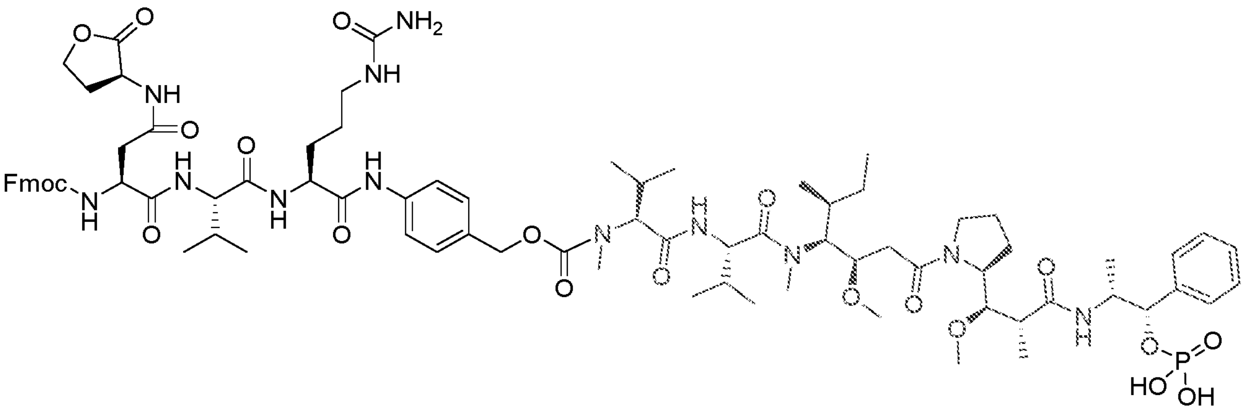

To a solution of Example 1-10 (76.2 mg, 0.079 mmoL), U-030 (55.1 mg, 0.069 mmoL), and 1-hydroxy-7-azabenzotriazole (13.0 mg, 0.096 mmoL) in N,N-dimethylformamide (1 mL) put into a 10 mL pear-shaped flask, N,N-diisopropylethylamine (40 μL) (29.68 mg, 0.230 mmoL) was added at room temperature, HATU (112.9 mg, 0.297 mmoL) was then added at room temperature, and the mixture was stirred at room temperature for 18 hours. Then, Example 1-10 (22.3 mg, 0.023 mmoL) was added, and the mixture was stirred at room temperature for 6.5 hours.

After the reaction was completed, the mixture was concentrated under reduced pressure, diethyl ether was added to the residues, and the produced solids were collected by filtration. Acetonitrile/water (1/1(V/V)) (10 mL) was added to the resulting solids, insoluble matters were removed by filtration, and freeze-drying was then performed to obtain a title compound (57.4 mg, yield 51.19%) as a white solid. MS(ESI)m/z 812$(M+2H)^{2+}$

Example 1-12

[C462]

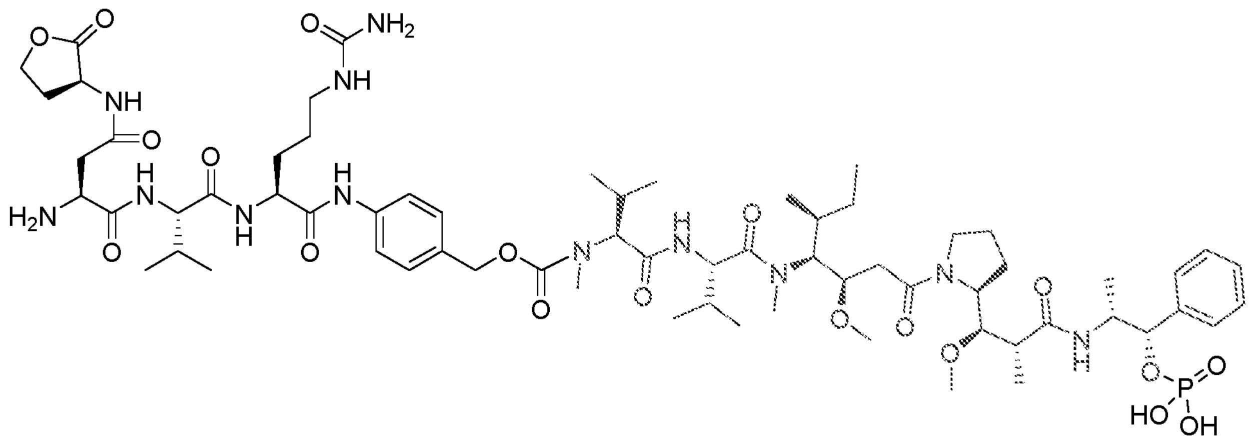

To a solution of Example 1-11 (57.4 mg, 0.035 mmoL) in N,N-dimethylformamide (2 mL) put into a 100 mL pear-shaped flask, under argon airflow with stirring, piperidine (11 μL) (9.48 mg, 0.111 mmoL) was added at room temperature, and the mixture was stirred at room temperature for 1 hour. The solvent was removed under reduced pressure, the residues were washed with diethyl ether (10 mL), and the resulting solids were dried under reduced pressure to obtain a title compound (51.8 mg, yield 93.23%) as a slightly yellow solid. MS(ESI)m/z 1402(M+H)+

Example 1-13

[C463]

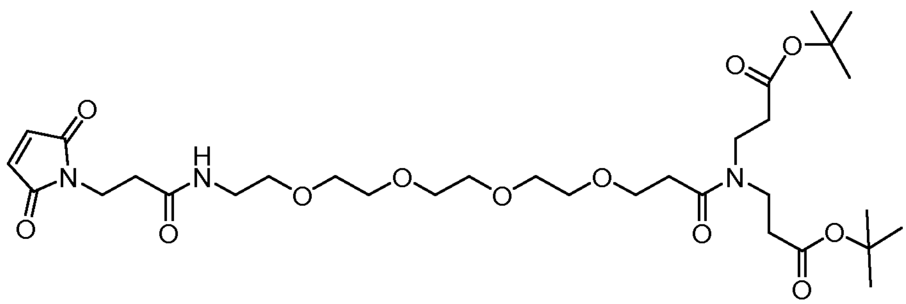

To a solution of 1-maleimido-3-oxo-7,10,13,16-tetraoxa-4-azanonadecanoic acid N-succinimidyl (53.3 mg, 0.104 mmoL) in dichloromethane (1 mL) put into a 5 mL sample tube, under argon airflow with stirring, di-tert-butyl 3,3'-iminodipropionate (27.3 mg, 0.100 mmoL) was added at room temperature, and the mixture was stirred at room temperature for 18 hours.

After the reaction was completed, the solvent was removed under reduced pressure, the resulting residues were subjected preparative HPLC under the following conditions, and fractions containing a target compound were freeze-dried to obtain a title compound (20.1 mg, yield 29.96%) as a colorless oil.
MS(ESI)m/z 672(M+H)+

Column: Waters XSelect HSS T3 OBD 5 μm 19*150 mm
Flow rate: 17 mL/min.
Elution solvent: 0.1% formic acid aqueous solution (solution A)-acetonitrile (solution B)
Gradient (solution B): 50% (0.00 min.)→95% (10.00 min.)

Example 1-14

[C464]

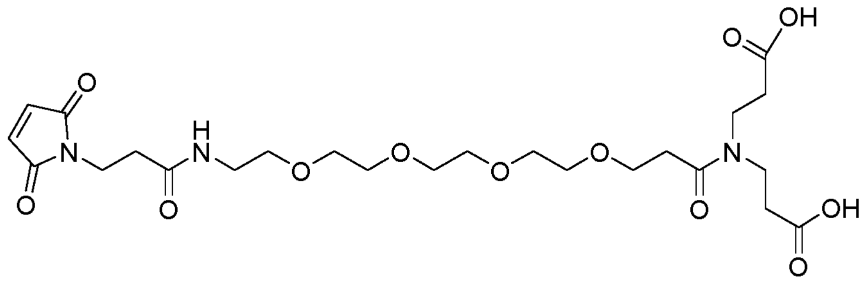

To a solution of Example 1-13 (20.1 mg, 0.030 mmoL) in dichloromethane (1 mL) put into a 20 mL pear-shaped flask, under nitrogen atmosphere with stirring, trifluoroacetic acid (1 mL) (1,489 mg, 13.06 mmoL) was added at room temperature, and the mixture was stirred at room temperature for 2.5 hours.

After the reaction was completed, the solvent was concentrated under reduced pressure, acetonitrile (1 mL) and water (1 mL) were added to the resulting residues and dissolved, and freeze-drying was then performed to obtain a title compound (20.0 mg, yield: quantitative) as a colorless oil.
MS(ESI)m/z 560(M+H)$^+$

Example 1-15

[C465]

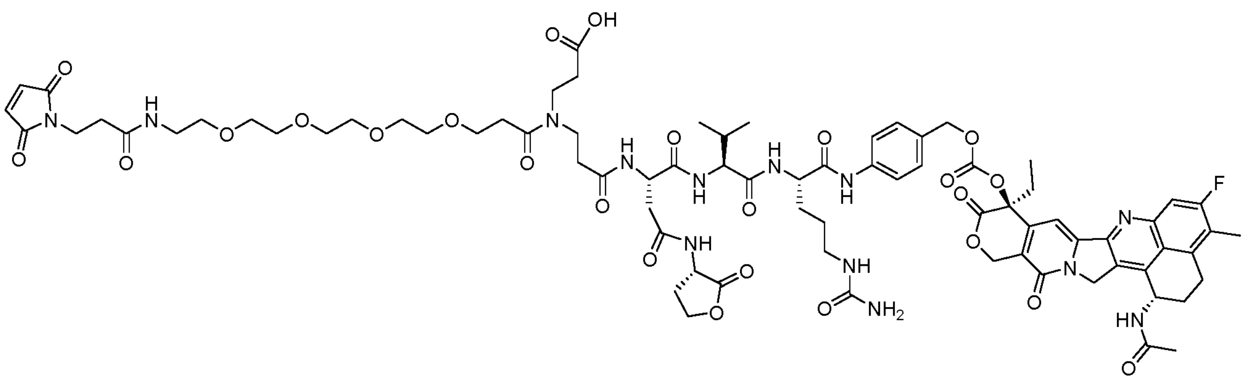

To a solution of Example 1-14 (8.7 mg, 0.016 mmoL) in N,N-dimethylformamide (0.4 mL) put into a 5 mL sample tube, under argon airflow with stirring, triethylamine (4.5 μL) (3.27 mg, 0.032 mmoL) was added, HATU (6.0 mg, 0.016 mmoL) was then added at room temperature, and the mixture was stirred at room temperature for 1 hour. Example 1-9 (8.8 mg, 7.81 μmoL) was added at room temperature to the reaction solution and the mixture was stirred at room temperature for 2 hours.

After the reaction was completed, the solvent was removed under reduced pressure, the resulting residues were subjected preparative HPLC under the following conditions, and fractions containing a target compound were freeze-dried to obtain a title compound (4.6 mg, yield 36.31%) as a colorless foam.
MS(ESI)m/z 812(M+2H)$^{2+}$

Column: Waters XBridge Prep C18 5 μm ODB 19*150 mm
Flow rate: 17 mL/min.
Elution solvent: 0.1% formic acid aqueous solution (solution A)-0.1% formic acid acetonitrile (solution B)
Gradient (solution B): 20% (0.00 min.)→60% (8.00 min.)

Example 1

[C466]

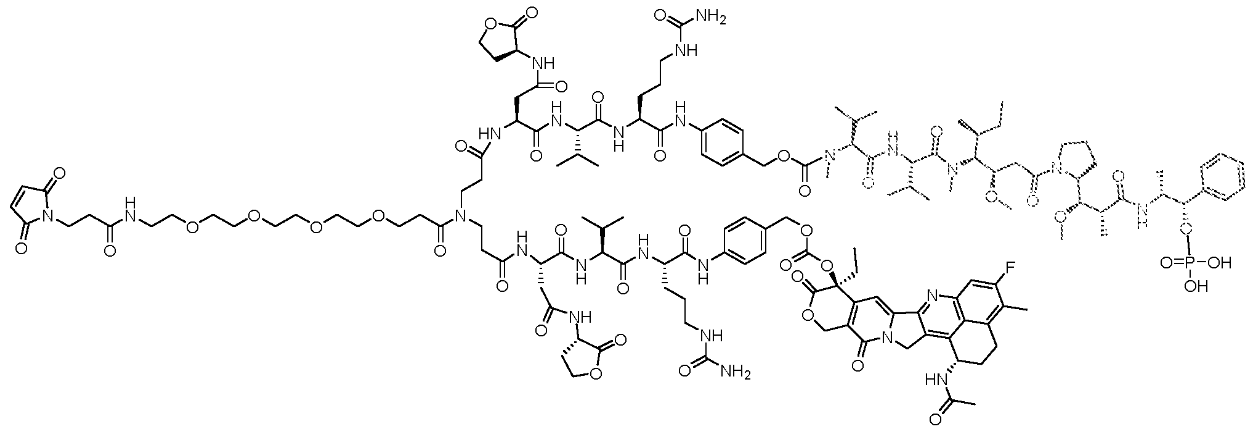

To a solution of Example 1-15 (4.6 mg, 2.83 μmoL) in N,N-dimethylformamide (0.4 mL) put into a 5 mL sample tube, under argon airflow with stirring, triethylamine (1.6 μL) (1.16 mg, 0.011 mmoL) was added, HATU (1.3 mg, 3.42 μmoL) was then added at room temperature, and the mixture was stirred at room temperature for 1 hour. Example 1-12 (5.0 mg, 3.57 μmoL) was added at room temperature to the reaction solution, and the mixture was stirred at room temperature for 1 hour.

After the reaction was completed, the resulting reaction solution was dissolved in a 50% acetonitrile aqueous solution, and subjected to preparative HPLC under the following conditions, and fractions containing a target compound were freeze-dried to obtain a title compound (3.55 mg, yield 34.54%) as a colorless foam.
MS(ESI)m/z 1003$(M+3H)^{3+}$

Column: Waters XBridge Prep C18 5 μm ODB 19*150 mm

Flow rate: 17 mL/min.

Elution solvent: 0.1% formic acid aqueous solution (solution A)-0.1% formic acid acetonitrile (solution B)

Gradient (solution B): 30% (0.00 min.)→65% (7.00 min.)

Synthesis of Example 2

[C467]

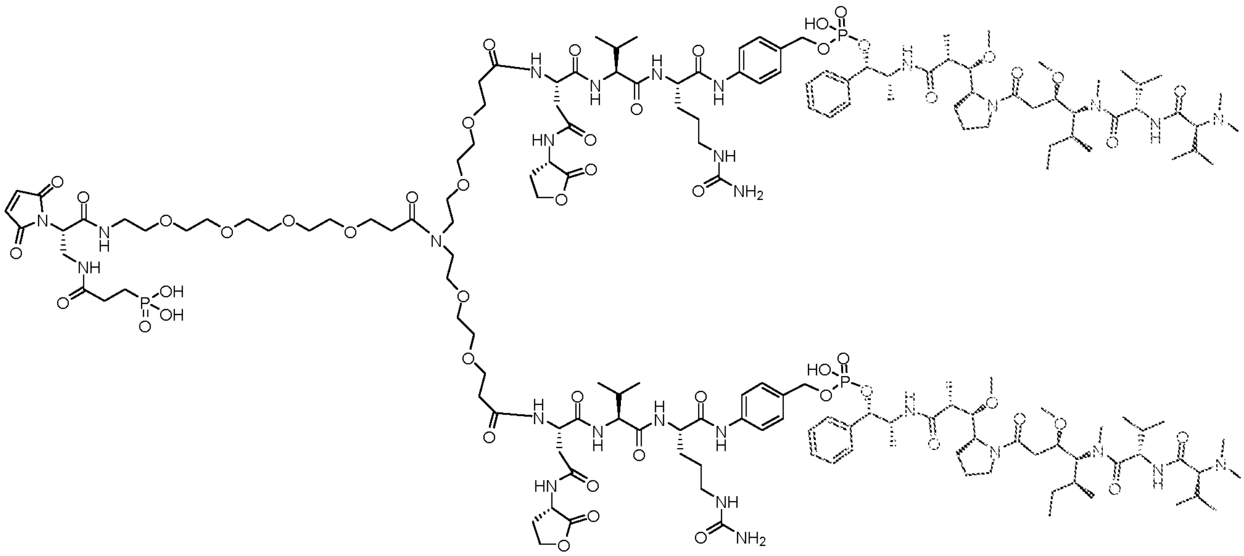

Example 2-1

[C468]

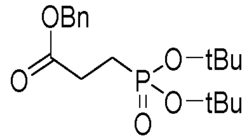

To a solution of di-tert-butyl phosphorous acid (1.94 g, 9.99 mmoL) in acetonitrile (10 mL) put into a 100 mL eggplant flask, under nitrogen airflow with stirring, benzyl acrylate (1.68 mL) (1.78 g, 10.98 mmoL) was added at room temperature, and the mixture was stirred at 80°C for 6 hours.

After the reaction was completed, the mixture was concentrated under reduced pressure, the resulting residues were subjected to YAMAZEN medium pressure flash chromatography (silica, L(40 g), hexane/ethyl acetate=70/30(V/V)→ 50/50(V/V)), and fractions containing a target compound were concentrated under reduced pressure to obtain a title compound (2.23 g, yield 62.64%) as a colorless oil.
MS(ESI)m/z 357(M+H)+

Example 2-2

[C469]

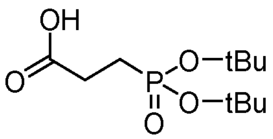

To a solution of Example 2-1 (2.23 g, 6.26 mmol) in ethanol (22 mL) put into a 200 mL eggplant flask, under nitrogen atmosphere with stirring, 10% by weight of palladium carbon NX type (50% by weight of water content, 1.30 g, 0.611 mmoL) was added, and under hydrogen atmosphere, and the mixture was stirred at room temperature for 2 hours.

After the reaction was completed, the mixture was filtered through Celite 545 (trade name), washed with ethanol,

and concentrated under reduced pressure to obtain a title compound (1.68 g, yield: quantitative) as a colorless oil. MS(ESI) m/z 267 $(M+H)^+$

Example 2-3

[C470]

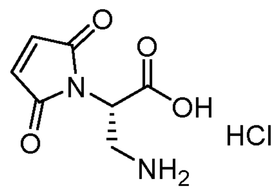

To a solution of (S)-3-((tert-butoxycarbonyl)amino)-2-(2,5-dioxo-2,5-dihydro-1H-pyrrol-1-yl)propanoic acid (1.21 g, 4.26 mmoL) (described in WO 2019/195665) in dichloromethane (4 mL) put into a 100 mL eggplant flask, under argon airflow with stirring, 4 M hydrogen chloride in a 1,4-dioxane solution (21.3 mL) (3.11 g, 85 mmol) was added at room temperature, and the mixture was stirred at room temperature for 1 hour.

After the reaction was completed, the solvent was removed under reduced pressure, ethyl acetate was added to the residues, and the produced solids were filtered. The resulting solids were dissolved in methanol, and concentrated under reduced pressure to obtain a title compound (1.17 g, yield: quantitative) as a slightly yellow foam.

Example 2-4

[C471]

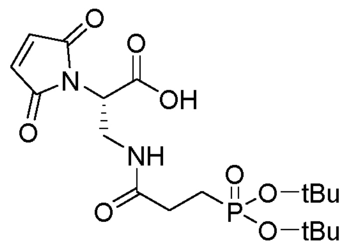

To a solution of Example 2-2 (0.26 g, 0.976 mmoL) in N,N-dimethylformamide (3 mL) put into a 30 mL cylindrical flask, under argon airflow with stirring, triethylamine (0.13 mL) (0.09 g, 0.933 mmoL) and HATU (0.37 g, 0.973 mmoL) were added at room temperature, and the mixture was stirred at room temperature for 1 hour. This was used as a solution A.

To a solution of Example 2-3 (0.21 g, 0.952 mmoL) in N,N-dimethylformamide (3 mL) put into a 20 mL cylindrical flask, under argon airflow with stirring, triethylamine (0.065 mL) (0.045 g, 0.466 mmoL) was added at room temperature, and the mixture was stirred at room temperature for 10 minutes. The solution A was put into the 20 mL cylindrical flask at room temperature, and the mixture was stirred at room temperature for 6 hours.

After the reaction was completed, water was added to the reaction solution, the mixture was filtered through a membrane filter, the resulting solution was then subjected to preparative HPLC under the following conditions, and fractions containing a target compound were freeze-dried to obtain a title compound (143.9 mg, yield 34.96%) as a colorless solid.
MS(ESI)m/z 433$(M+H)^+$

Column: Waters XBridge Prep C18 5 μm ODB 19*150 mm
Flow rate: 17 mL/min.
Elution solvent: 0.1% formic acid aqueous solution (solution A)-acetonitrile (solution B)
Gradient (solution B): 30% (0.00 min.)→36% (5.00 min.)

Example 2-5

[C472]

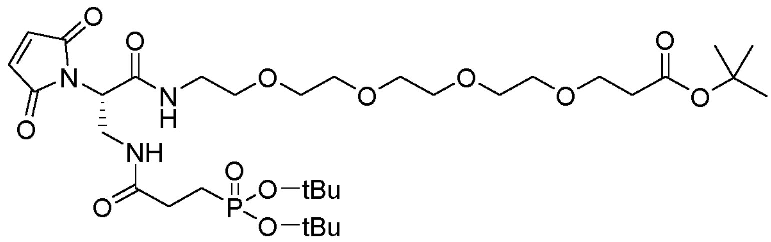

To a solution of Example 2-4 (143.8 mg, 0.333 mmoL) in N,N-dimethylformamide (3 mL) put into a 30 mL pear-shaped flask, under argon airflow with stirring, triethylamine (56 μL) (40.66 mg, 0.402 mmoL) and HATU (152.5 mg, 0.401 mmoL) were added at room temperature, and the mixture was stirred at room temperature for 2 minutes. Next, a solution of tert-butyl 1-amino-3,6,9,12-tetraoxapentadecan-15-oate (112.5 mg, 0.350 mmoL) in N,N-dimethylformamide (0.5 mL) was added at room temperature, and the mixture was stirred at room temperature for 1 hour.

After the reaction was completed, the reaction solution was subjected to preparative HPLC under the following conditions, and fractions containing a target compound were freeze-dried to obtain a title compound (143.5 mg, yield 58.64%) as a colorless solid.
MS(ESI)m/z 734$(M-H)^-$

Column: Waters SunFire Prep C18 5 μm ODB 19*150 mm
Flow rate: 17 mL/min.
Elution solvent: 0.1% formic acid aqueous solution (solution A)-acetonitrile (solution B)
Gradient (solution B): 20% (0.00 min.)→60% (8.00 min.)

Example 2-6

[C473]

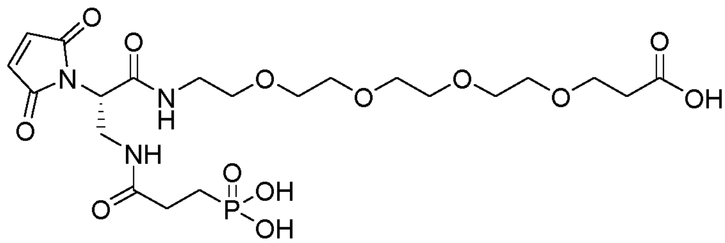

To a solution of Example 2-5 (63.3 mg, 0.086 mmoL) in dichloromethane (1.5 mL) put into a 30 mL pear-shaped flask, under nitrogen airflow with stirring, trifluoroacetic acid (150 μL) (223.35 mg, 1.959 mmoL) was added at room temperature, and the mixture was stirred at room temperature for 2.5 hours. Next, trifluoroacetic acid (0.5 mL) (744.5 mg, 6.530 mmoL) was added at room temperature, and the mixture was stirred at room temperature for 2 hours.

After the reaction was completed, the reaction solution was concentrated under reduced pressure, acetonitrile/-water (1/1(V/V)) (2 mL) was added to the residues, and freeze-drying was performed to obtain a title compound (56.6 mg, yield: quantitative) as a white solid.
MS(ESI)m/z 568$(M+H)^+$

Example 2-7

[C474]

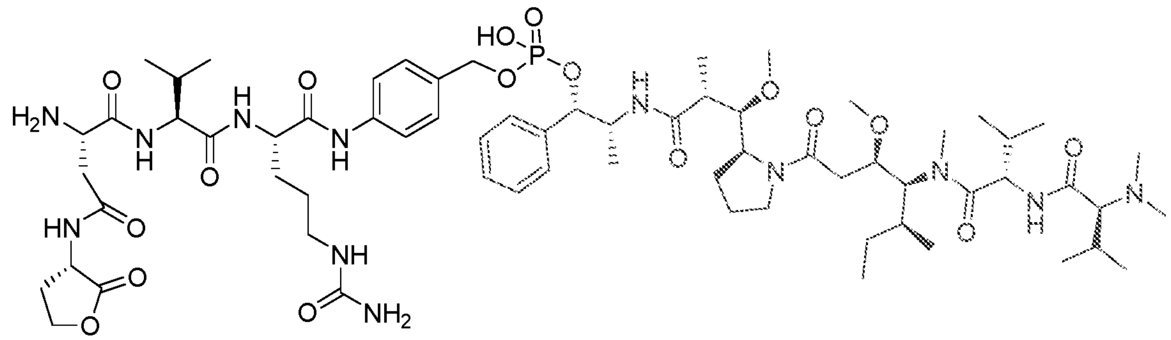

To a solution of Example 1-4 (340.9 mg, 0.426 mmol) in N,N-dimethylformamide (6 mL) put into a 100 mL pear-shaped flask, under argon atmosphere with stirring, 3-((bis(diisopropylamino)phosphino)oxy)propanenitrile (255 μL) (242.25 mg, 0.804 mmoL) and 1H-tetrazole (56.6 mg, 0.808 mmoL) were added at room temperature, and the mixture was stirred at room temperature for 15 minutes. Then, at room temperature, triisopropylsilyl trifluoromethanesulfonate (215 μL) (245.1 mg, 0.800 mmoL) was added, and the mixture was stirred at room temperature for 30 minutes. Then, U-010 (146 mg, 0.199 mmol) and 5-(ethylthio)-1H-tetrazole (109.4 mg, 0.840 mmoL) were added, and the mixture was stirred at room temperature for 1 hour. Next, under ice-cooling, tert-butyl hydroperoxide (330 μL) (310.2 mg, 2.409 mmol) was added, and the mixture was stirred at room temperature for 2 hours. Then, under ice-cooling, diazabicycloundecene (600 μL) (612 mg, 4.02 mmoL) was added, and the mixture was stirred at room temperature for 1 hour.

After the reaction was completed, diethyl ether (50 mL) was added, and the supernatant solution was removed. Next, diethyl ether (25 mL) was added, and the supernatant solution was removed. Next, diethyl ether (25 mL) was added, the supernatant solution was removed, and dried under reduced pressure. The residues were purified under the following recycle preparative conditions, and fractions containing a target compound were subjected to pressure reduction to obtain a title compound crude product (185.6 mg) as a colorless oil.

Device: LC Forte/R (YMC Co., Ltd.)
Column: Xserect HSS C18 19*150 mm, 5 μm
Flow rate: 17 mL/min.
Elution solvent: 0.1% formic acid aqueous solution (solution A)-0.1% formic acid acetonitrile (solution B)
Solution composition (solution B): 50%

The resulting crude product (185.6 mg) was purified under the following recycle preparative conditions, and fractions containing a target compound were subjected to pressure reduction to obtain a title compound (19.27 mg, yield 7.04%) as a white solid.
MS(ESI)m/z 1372(M+H)$^+$

Device: LC Forte/R (YMC Co., Ltd.)
Column: CSH Fluorophenyl Prep 19*150 mm, 5 μm
Flow rate: 17 mL/min.
Elution solvent: 0.1% formic acid aqueous solution (solution A)-0.1% formic acid acetonitrile (solution B)
Solution composition (solution B): 20%

Example 2-8

[C475]

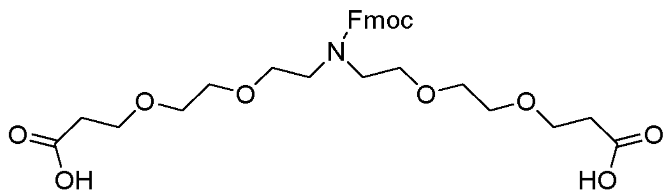

To a solution of di-tert-butyl 4,7,13,16-tetraoxa-10-azanonadecane dioate (0.45 g, 1.001 mmoL) and triethylamine (153 μL) (0.11 g, 1.098 mmoL) in dichloromethane (20 mL) put into a 100 mL pear-shaped flask, under nitrogen airflow with stirring, 9-fluorenylmethyl chloroformate (0.29 g, 1.121 mmoL) was added at room temperature, and the mixture was stirred at room temperature for 1 hour. Next, trifluoroacetic acid (10 mL) was added at room temperature, and the mixture was stirred at room temperature for 1 hour.

After the reaction was completed, the reaction solution was concentrated under reduced pressure. Water was added to the residues, and extraction with ethyl acetate was performed. The organic layer was dried with anhydrous magnesium sulfate, and ethyl acetate was distilled off under reduced pressure. The resulting residues were subjected to preparative HPLC under the following conditions, and fractions containing a target compound were concentrated under reduced pressure to obtain a title compound (0.49 g, yield 87.48%) as a colorless oil.
MS(ESI)m/z 560(M+H)$^+$

Column: Waters XBridge Prep C18 5 μm ODB 19*150 mm

Flow rate: 17 mL/min.

Elution solvent: 0.1% formic acid aqueous solution (solution A)-acetonitrile (solution B)

Gradient (solution B): 40% (0.00 min.)→70% (8.00 min.)

Example 2-9

[C476]

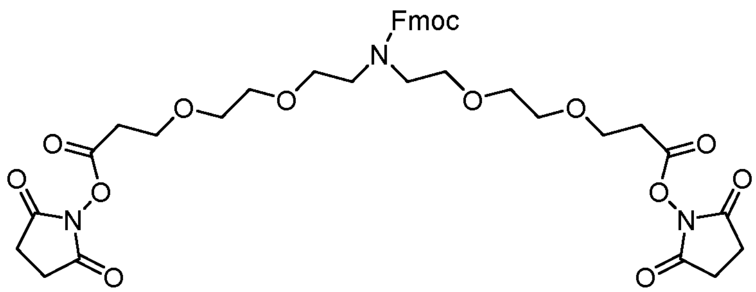

To a solution of Example 2-8 (56.0 mg, 0.100 mmoL) and N-hydroxysuccinimide (25.3 mg, 0.220 mmoL) in N,N-dimethylformamide (0.5 mL) put into a 5 mL sample tube, under air atmosphere with stirring, 1-ethyl-3-(3-dimethylaminopropyl)carbodiimide hydrochloride (47.9 mg, 0.250 mmoL) was added at room temperature, and the mixture was stirred at room temperature for 3 hours.

After the reaction was completed, acetonitrile was added, and the mixture was subjected to preparative HPLC under the following conditions, and fractions containing a target compound were freeze-dried to obtain a title compound (59.5 mg, yield 78.88%) as a colorless oil.
MS(ESI)m/z 754(M+H)$^+$

Column: Waters XBridge Prep C18 5 μm ODB 19*150 mm
Flow rate: 17 mL/min.
Elution solvent: 0.1% formic acid aqueous solution (solution A)-acetonitrile (solution B)

Gradient (solution B): 50% (0.00 min.)→80% (8.00 min.)

Example 2-10

[C477]

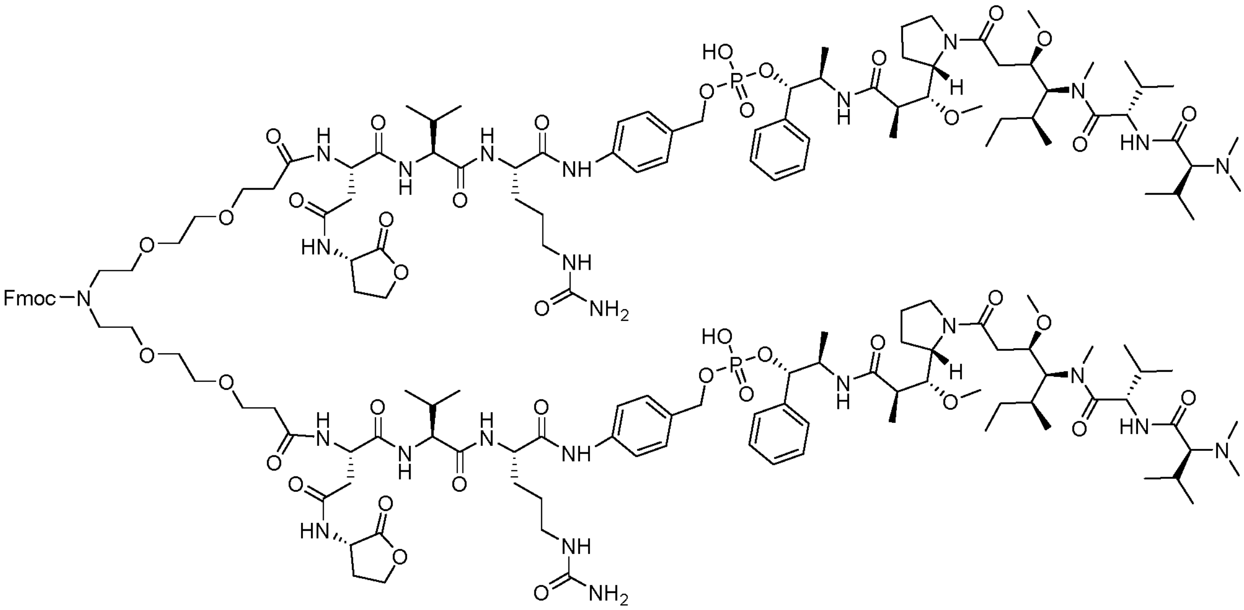

To a solution of Example 2-7 (7.32 mg, 5.34 μmoL) in N,N-dimethylformamide (125 μL) put into a 5 mL sample tube, under argon atmosphere with stirring, N,N-dimethylformamide (7.5 μL) containing triethylamine (0.75 μL) (0.54 mg, 5.38 μmoL) was added, N,N-dimethylformamide (10 μL) containing Example 2-9 (2.0 mg, 2.65 μmoL) was then added at room temperature, and the mixture was stirred at room temperature for 0.5 hours. Next, N,N-dimethylformamide (5 μL) containing HATU (0.35 mg, 2.57 μmoL) was added at room temperature, and the mixture was stirred at room temperature for 20 hours.

After the reaction was completed, a 50% acetonitrile aqueous solution was added, the mixture was subjected to preparative HPLC under the following conditions, and fractions containing a target compound were freeze-dried to obtain a title compound (4.0 mg, yield 46.15%) as a colorless foam.

MS(ESI)m/z 1090$(M+3H)^{3+}$

Column: Waters XSelect CSH 5 μm ODB 19 mm × 150 mm

Flow rate: 17 mL/min.

Elution solvent: 0.1% formic acid aqueous solution (solution A)-acetonitrile (solution B)

Gradient (solution B): 30% (0.00 min.)→70% (8.00 min.)

Example 2

[C478]

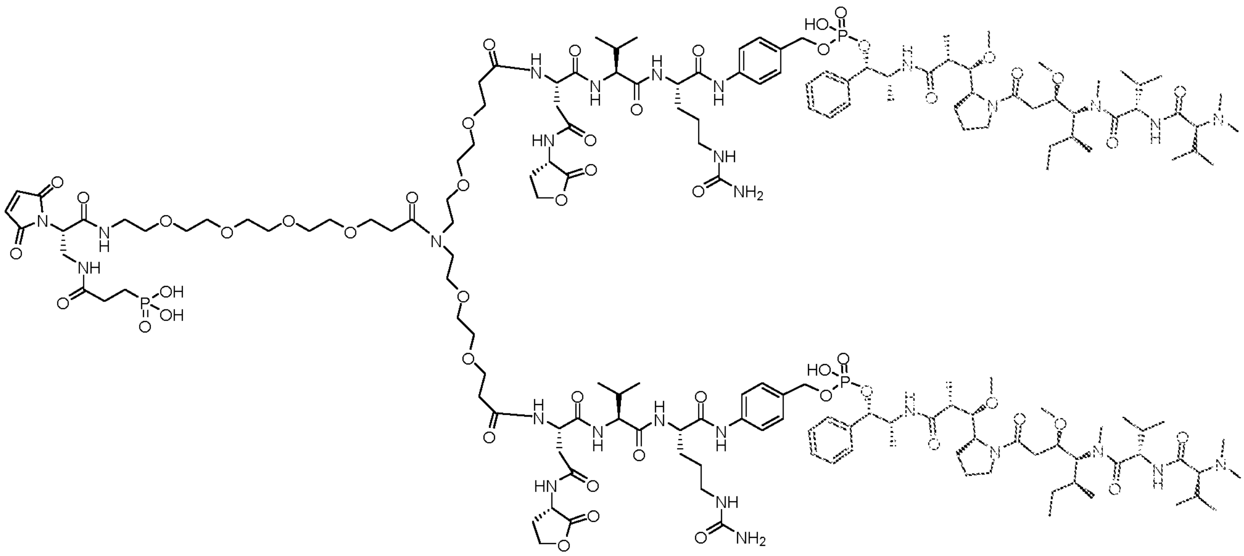

To a solution of Example 2-10 (5.6 mg, 1.714 μmoL) in N,N-dimethylformamide (0.2 mL) put into a 5 mL sample tube, under argon atmosphere with stirring, N,N-dimethylformamide (15 μL) containing diazabicycloundecene (1.5 μL) (1.52 mg, 9.95 μmoL) was added at room temperature, the mixture was stirred at room temperature for 0.5 hours, and 2N hydrochloric acid 5.1 μL was then added. This was used as a solution A.

To a solution of Example 2-6 (10.9 mg, 0.019 mmoL) in N,N-dimethylformamide (0.2 mL) put into a 5 mL sample tube, under argon atmosphere with stirring, triethylamine (8.0 μL) (5.81 mg, 0.057 mmoL) was added, HATU (7.2 mg, 0.019 μmoL) was then added at room temperature, and the mixture was stirred at room temperature for 1 hour. The reaction solution was added to the A solution, and the mixture was stirred at room temperature for 2 hours.

The resulting reaction solution was subjected to preparative HPLC under the following conditions, and fractions containing a target compound were concentrated under reduced pressure and then freeze-dried to obtain a title compound crude product (3.8 mg) as a colorless solid.

Column: Waters XSelect CSH 5 μm ODB 19 mm × 150 mm
Flow rate: 17 mL/min.
Elution solvent: 0.1% formic acid aqueous solution (solution A)-acetonitrile (solution B)
Gradient (solution B): 20% (0.00 min.)→50% (10.00 min.)

Next, the crude product was subjected to preparative HPLC under the following conditions, and fractions containing a target compound were freeze-dried to obtain a title compound (1.42 mg, yield 23.05%) as a colorless solid. MS(ESI)m/z 1199$(M+3H)^{3+}$

Column: Waters XSelect CSH 5 μm ODB 19 mm × 150 mm
Flow rate: 17 mL/min.
Elution solvent: 0.1% formic acid aqueous solution (solution A)-acetonitrile (solution B)
Gradient (solution B): 30% (0.00 min.)→60% (10.00 min.)

Synthesis of Example 3

[C479]

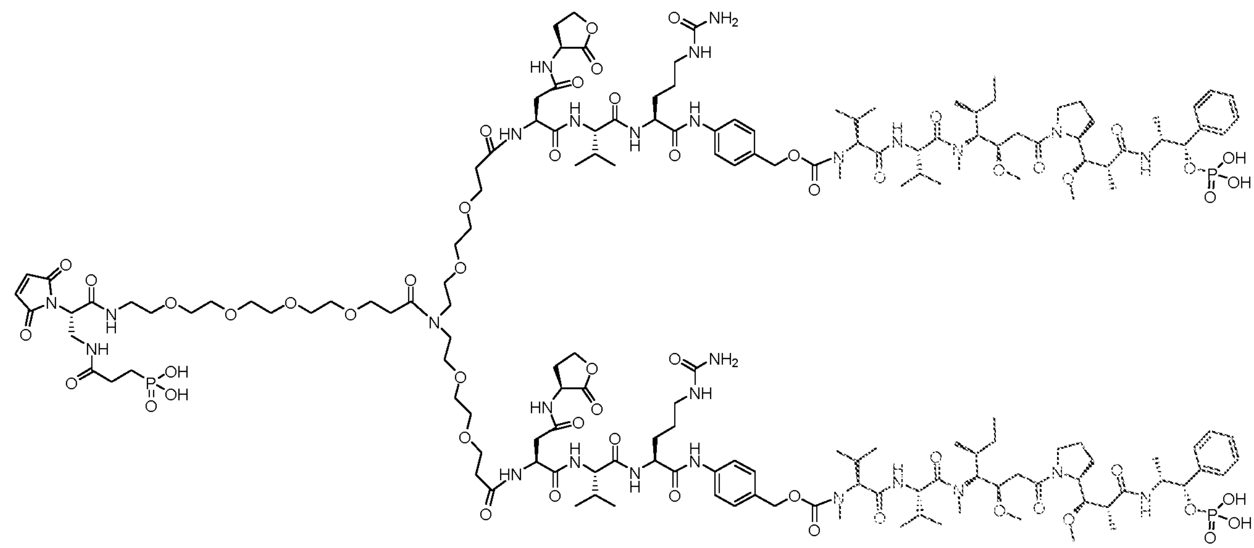

Example 3-1

[C480]

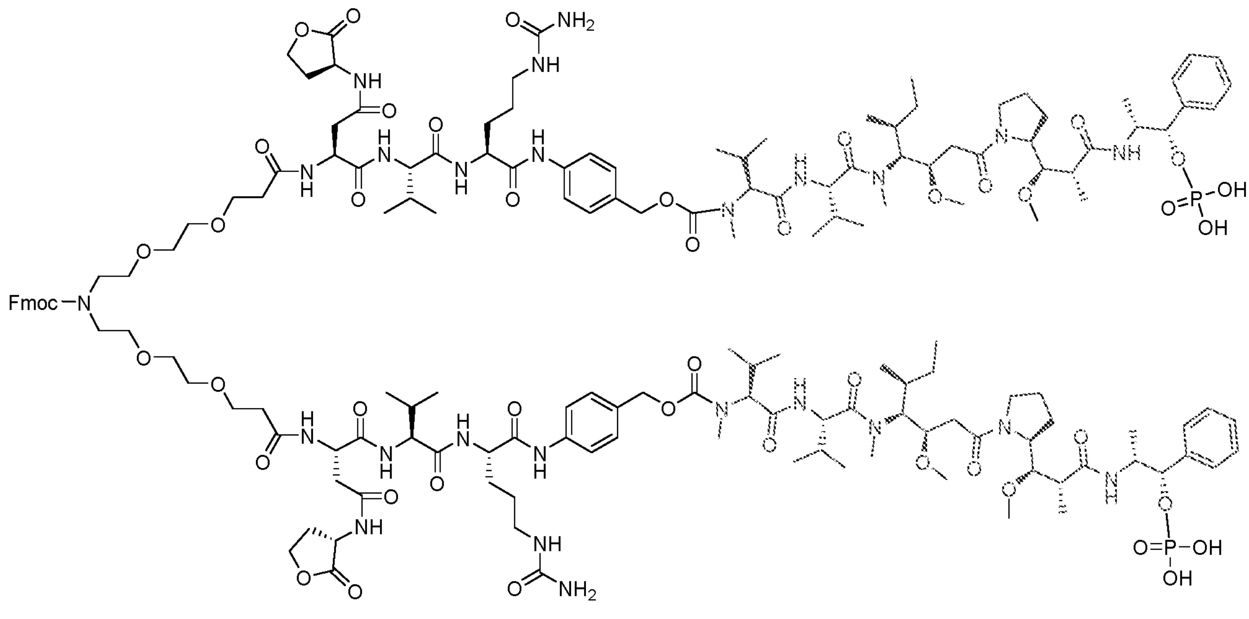

Example 1-12 (30.8 mg, 21.97 μmoL) instead of Example 2-9 (7.5 mg, 9.95 μmoL) and Example 2-7 was used for a reaction in the same manner as in Example 2-10 to obtain a title compound (9.3 mg, yield 28.09%) as a colorless solid. MS(ESI)m/z 1110$(M+3H)^{3+}$

Example 3

[C481]

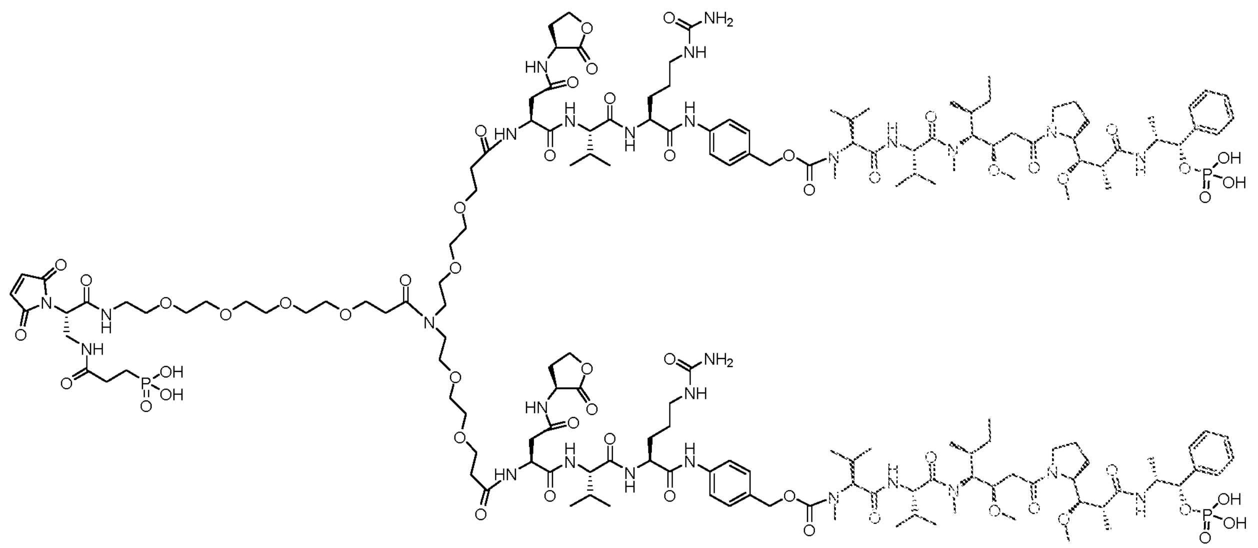

The reaction was performed in the same manner as in Example 2 except that Example 3-1 (5.24 mg, 1.668 μmoL) was used instead of Example 2-6 (11.0 mg, 0.019 mmoL) and Example 2-10 to obtain a title compound (3.98 mg, yield 65.28%) as a colorless solid.
MS(ESI)m/z 1219$(M+3H)^{3+}$

Synthesis of Example 4

[C482]

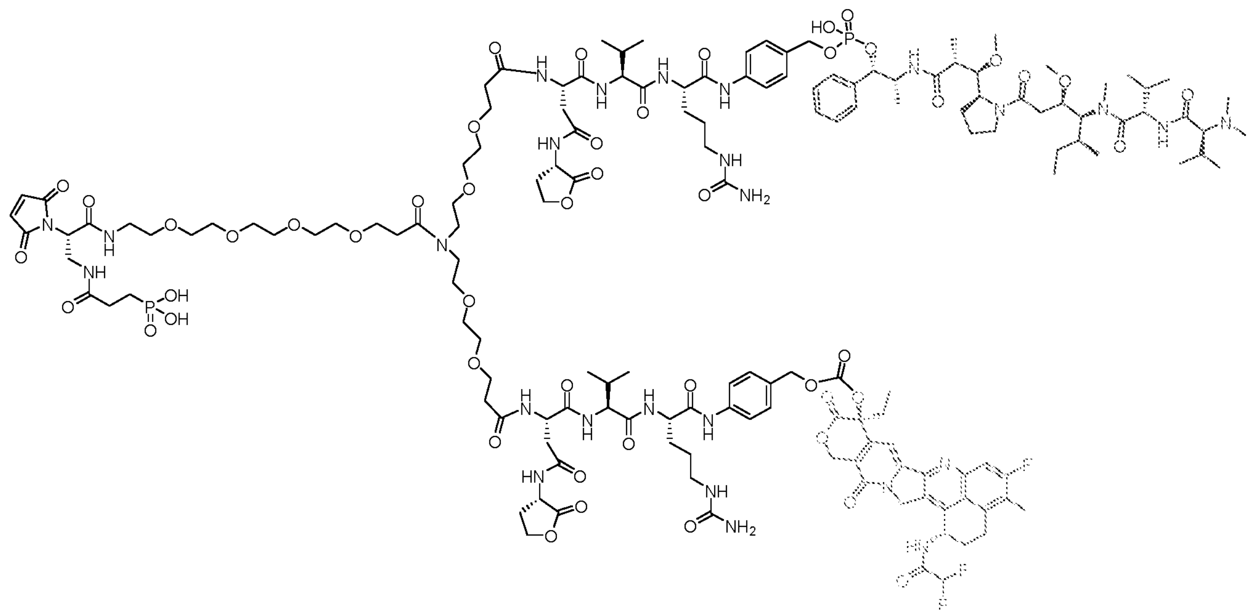

Example 4-1

[C483]

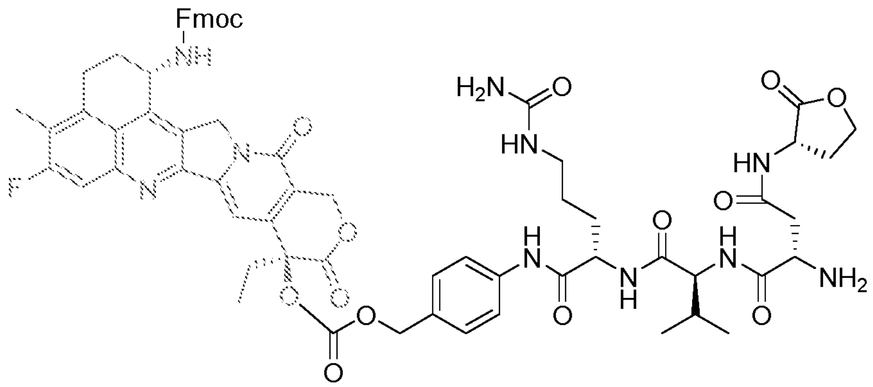

To a solution of Example 1-8 (1.33 g, 0.885 mmoL) in dichloromethane (20 mL) put into a 300 mL eggplant flask, under argon airflow with stirring, trifluoroacetic acid (102 μL) (0.15 g, 1.324 mmoL) was added at room temperature, and the mixture was stirred at room temperature for 1.5 hours. Next, trifluoroacetic acid (34 μL) (0.05 g, 0.441 mmoL) was added at room temperature with stirring, and the mixture was stirred at room temperature for 1 hour.

After the reaction was completed, N,N-diisopropylethylamine (600 μL) (0.44 g, 3.44 mmoL) was added to the reaction solution, and diethyl ether (40 mL) was added. The produced solids were filtered and sequentially washed with water, ethyl acetate, and diethyl ether, and then dried under reduced pressure to obtain a title compound (1.00 g, yield 89.63%) as a slightly yellow solid.

MS(ESI)m/z 1262$(M+H)^+$

Example 4-2

[C484]

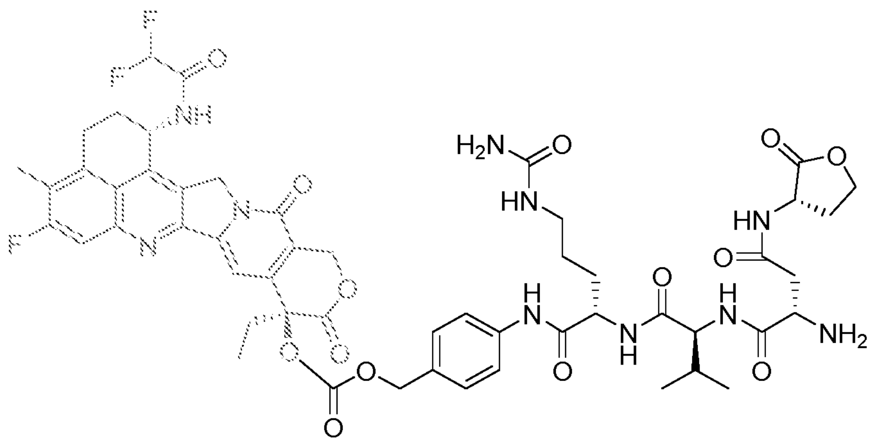

To a solution of Example 4-1 (24.3 mg, 0.019 mmoL) in N,N-dimethylformamide (800 μL) put into a 10 mL cylindrical flask, under argon airflow with stirring, N,N-diisopropylethylamine (8.5 μL) (6.29 mg, 0.049 mmoL) and trityl chloride (7.1 mg, 0.025 mmoL) were added at room temperature, and the mixture was stirred at room temperature for 1 hour. Next, N,N-diisopropylethylamine (5.6 μL) (4.14 mg, 0.032 mmoL) and trityl chloride (7.3 mg, 0.026 mmoL) were added at room temperature with stirring, and the mixture was stirred at room temperature for 1.5 hours. Next, piperidine (5 μL) (4.3 mg, 0.050 mmoL) was added at room temperature with stirring, and the mixture was stirred at room temperature for 1.5 hours. Next, piperidine (4 μL) (3.44 mg, 0.040 mmoL) was added at room temperature with stirring, and the mixture was stirred at room temperature for 1 hour.

After the reaction was completed, the solvent was removed under reduced pressure, and washing with diethyl ether was performed. To a solution of the resulting residues in dichloromethane (800 μL), under argon airflow with stirring, N,N-diisopropylethylamine (13 μL) (9.62 mg, 0.074 mmoL) was added, 2,2-difluoroacetic anhydride (3 μL) (4.8 mg, 0.028

mmoL) was then added at 0°C, and the mixture was stirred at room temperature for 0.5 hours. Next, N,N-diisopropylethylamine (5 μL) (3.7 mg, 0.029 mmoL) and 2,2-difluoroacetic anhydride (3 μL) (4.8 mg, 0.028 mmoL) were added at room temperature with stirring, the mixture was stirred at room temperature for 0.5 hours, and the solvent was then removed under reduced pressure. Acetonitrile (4 mL), water (2 mL), and formic acid (120 μL) (144 mg, 3.13 mmoL) were added to the resulting residues, and the mixture was stirred at room temperature for 2 hours.

The reaction solution was subjected to preparative HPLC under the following conditions, and fractions containing a target compound were freeze-dried to obtain a title compound (5.1 mg, yield 23.7%) as a white solid.
MS(ESI)m/z 1118(M+H)$^+$

Column: Waters XBridge Prep C18 5 μm ODB 19*150 mm
Flow rate: 17 mL/min.
Elution solvent: 0.1% formic acid aqueous solution (solution A)-acetonitrile (solution B)
Gradient (solution B): 40% (0.00 min.)→65% (6.00 min.)→95%
(6.50 min.)→95% (9.00 min.)

Example 4-3

[C485]

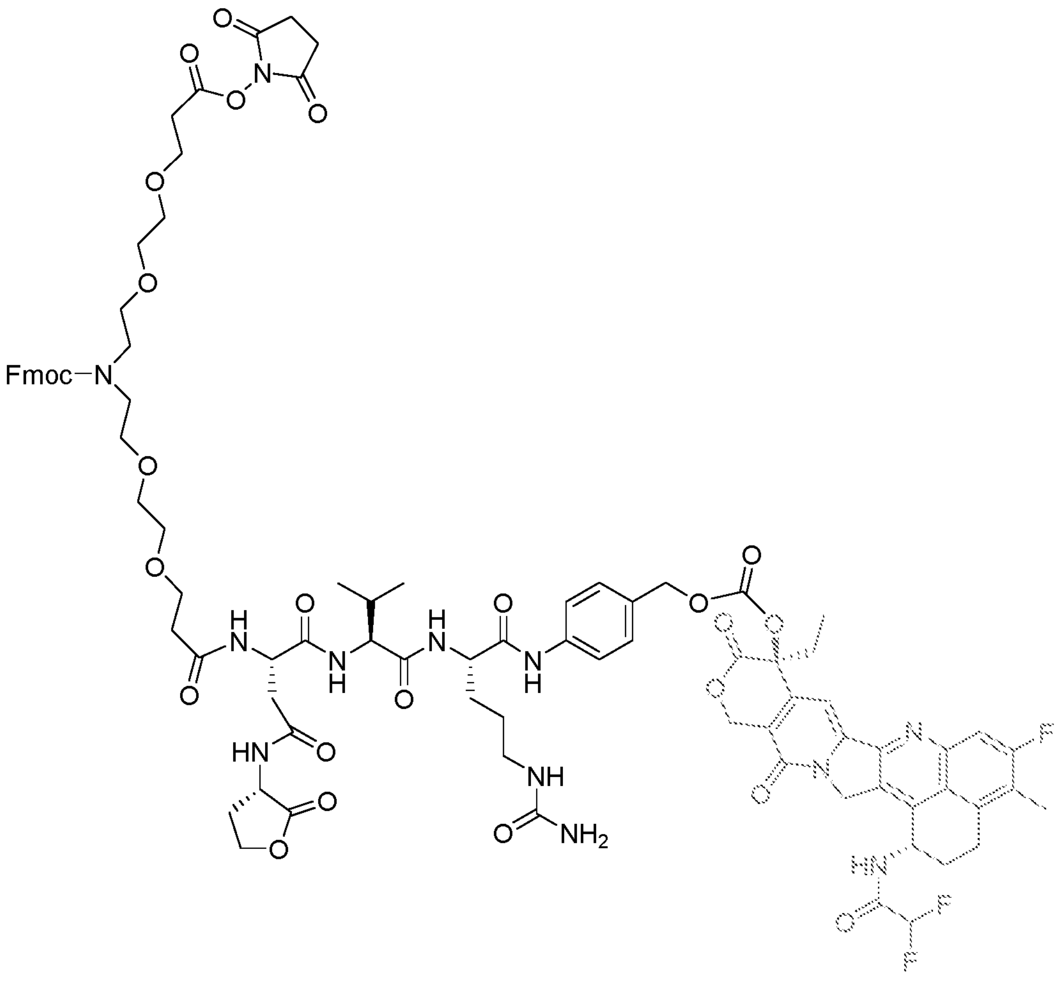

To a solution of Example 2-9 (28.4 mg, 0.038 mmoL) in N,N-dimethylformamide (1 mL) put into a 10 mL cylindrical flask, under argon atmosphere with stirring, Example 4-2 (15.9 mg, 0.014 mmoL) was added, N,N-diisopropylethylamine (7 μL) (5.18 mg, 0.040 mmoL) was then added at room temperature, and the mixture was stirred at room temperature for 3 hours. A 40% acetonitrile aqueous solution (5 mL) was added to the resulting reaction solution, the mixture was subjected

to preparative HPLC under the following conditions, and fractions containing a target compound were freeze-dried to obtain a title compound (7.2 mg, yield 28.81%) as a colorless foam.
MS(ESI)m/z 879(M+2H)$^{2+}$

Column: Waters XBridge Prep C18 5 μm ODB 19*150 mm
Flow rate: 17 mL/min.
Elution solvent: 0.1% formic acid aqueous solution (solution A)-acetonitrile (solution B)
Gradient (solution B): 30% (0.00 min.)→60% (10.00 min.)

Example 4-4

[C486]

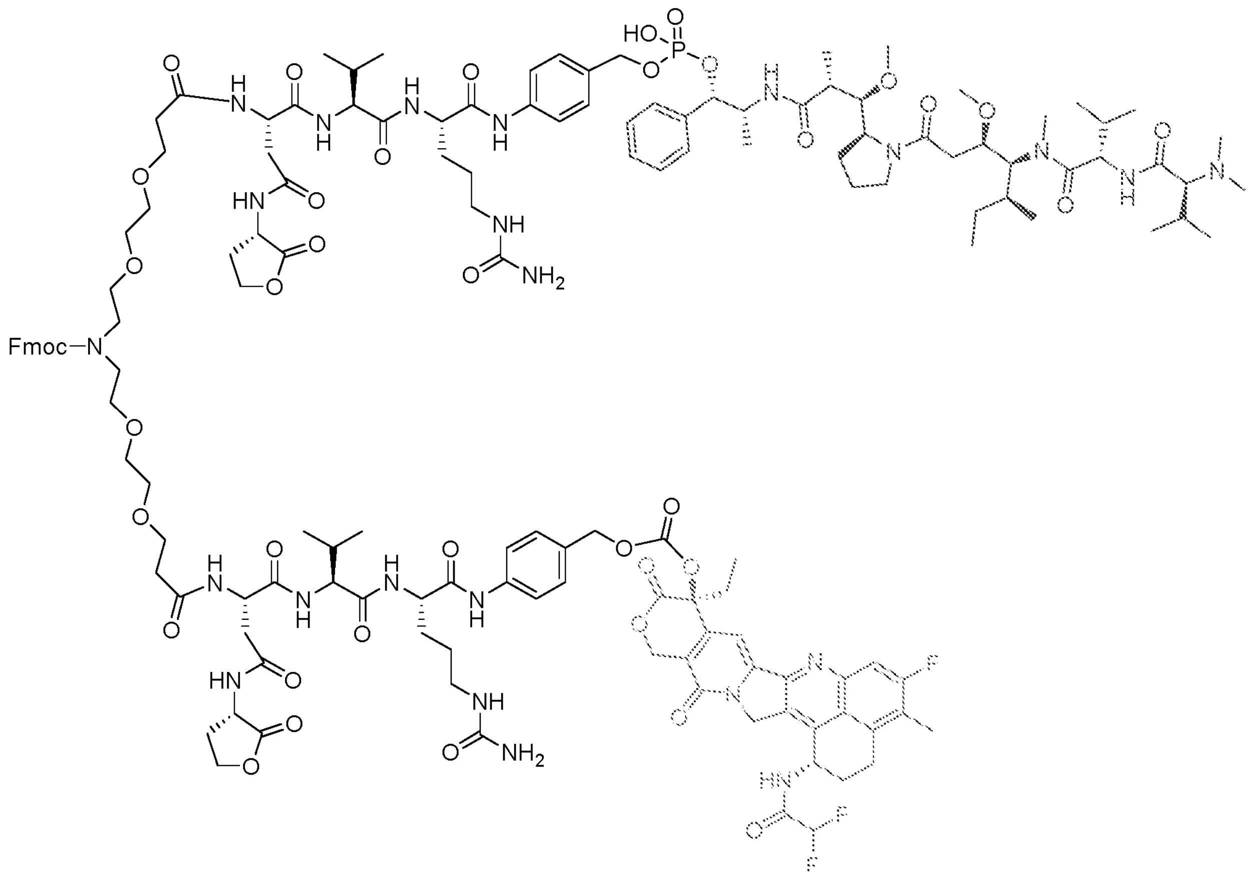

To a solution of Example 4-3 (7.2 mg, 4.10 μmoL), and Example 2-7 (7.2 mg, 5.25 μmoL) in N,N-dimethylformamide (0.8 mL) put into a 20 mL cylindrical flask, under argon atmosphere with stirring, N,N-diisopropylethylamine (3.2 μL) (2.37 mg, 0.018 mmoL) was added at room temperature, and the mixture was stirred at room temperature for 4 hours. Next, 1-hydroxy-7-azabenzotriazole (1.0 mg, 7.35 μmoL), 1-ethyl-3-(3-dimethylaminopropyl)carbodiimide hydrochloride (1.5 mg, 7.82 μmoL), and N,N-diisopropylethylamine (1 μL) (0.74 mg, 5.73 μmoL) were added at room temperature with stirring, and the mixture was stirred at room temperature for 3 hours. A 60% acetonitrile aqueous solution (5 mL) was added to the resulting reaction solution, the mixture was subjected to preparative HPLC under the following conditions, and fractions containing a target compound were freeze-dried to obtain a title compound (3.4 mg, yield 27.52%) as a colorless foam.
MS(ESI)m/z 1005(M+3H)$^{3+}$

Column: Waters XBridge Prep C18 5 μm ODB 19*150 mm
Flow rate: 17 mL/min.
Elution solvent: 0.1% formic acid aqueous solution (solution A)-acetonitrile (solution B)

Gradient (solution B): 30% (0.00 min.)→60% (10.00 min.)

Example 4

[C487]

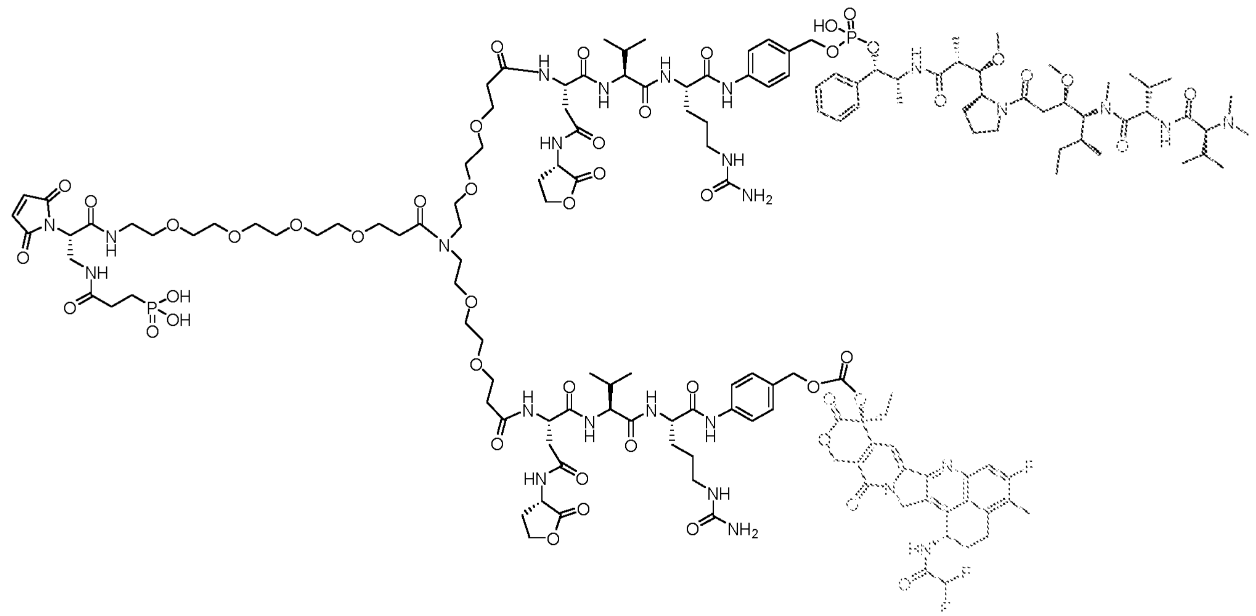

The reaction was performed in the same manner as in Example 2 except that Example 4-4 (12 mg, 3.98 μmoL) was used instead of Example 2-6 (5.1 mg, 8.99 μmoL) and Example 2-10 to obtain a title compound (3.75 mg, yield 28.19%) as a white solid.
MS(ESI)m/z 1114$(M+3H)^{3+}$

Synthesis of Example 5

[C488]

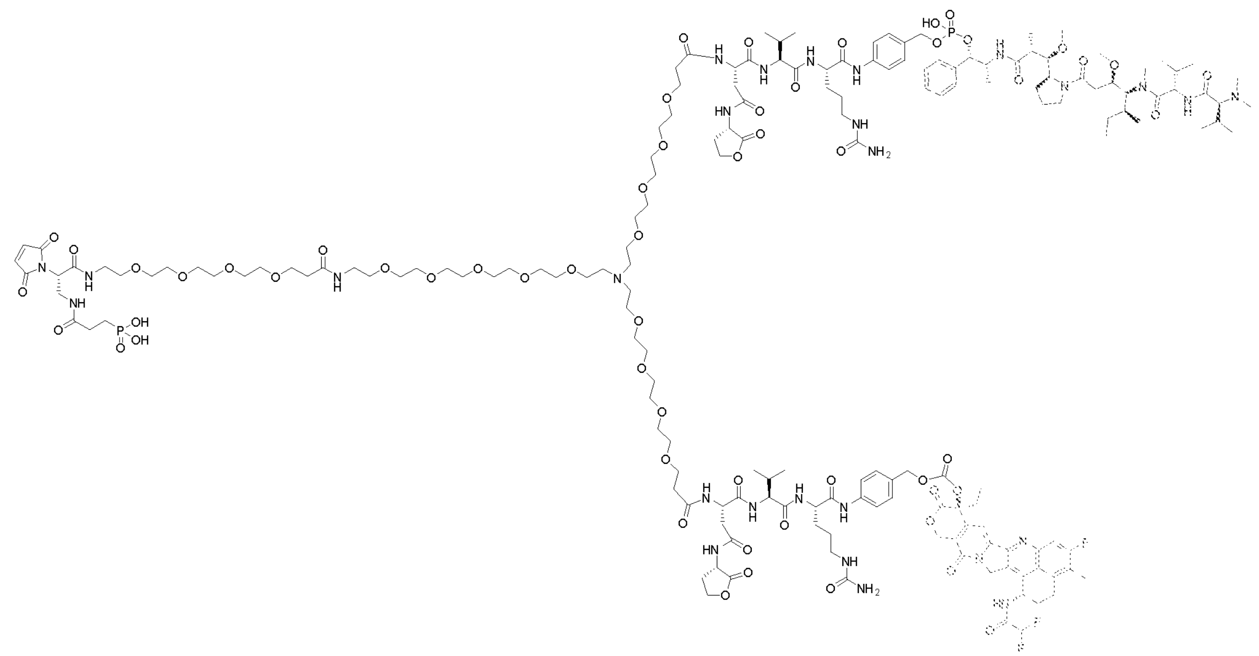

Example 5-1

[C489]

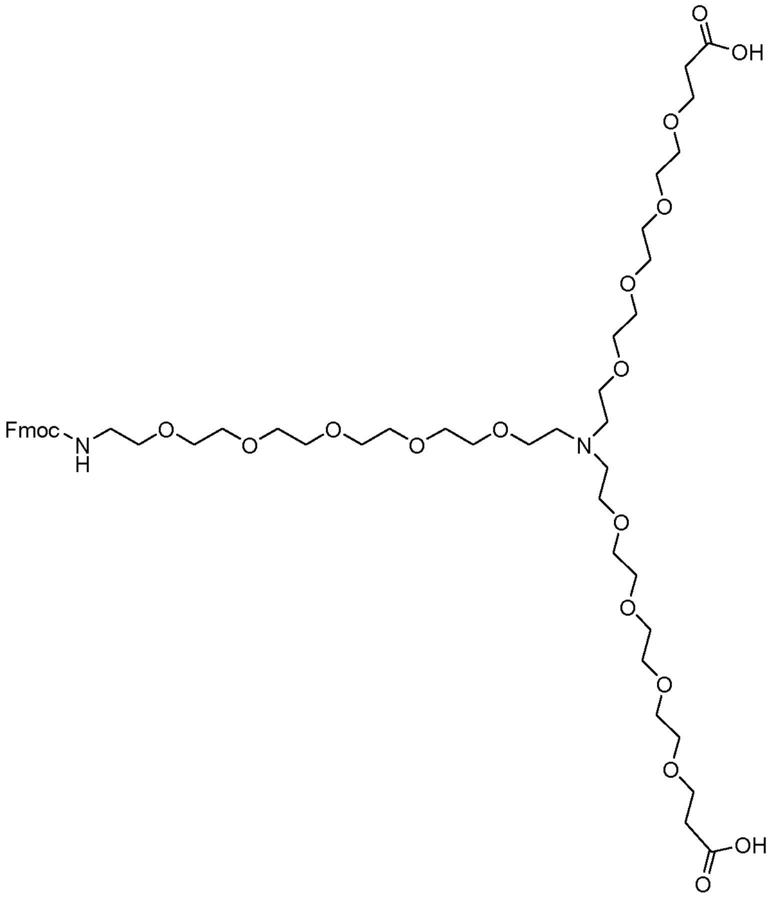

To a solution of 16-(17-amino-3,6,9,12,15-pentaoxaheptadecyl)-4,7,10,13,19,22,25,28-octaoxa-16-azahentriacontan dioic acid (146 mg, 0.188 mmol) in acetonitrile/water=1/1(V/V) (4 mL) put into a 30 mL cylindrical flask, under air atmosphere with stirring, sodium bicarbonate (80 mg, 0.952 mmoL) and 9-fluorenylmethyl chloroformate (62 mg, 0.240 mmoL) were added at room temperature, and the mixture was stirred at room temperature for 2.5 hours.

After the reaction was completed, water (2 mL) was added to the reaction solution, the mixture was subjected to preparative HPLC under the following conditions, and fractions containing a target compound were concentrated under reduced pressure to obtain a title compound (122 mg, yield 64.98%) as a slightly yellow oil.
MS(ESI)m/z 1000(M+H)+

Column: Waters XBridge Prep C18 5 μm ODB 19*150 mm

Flow rate: 17 mL/min.

Elution solvent: 0.1% formic acid aqueous solution (solution A)-acetonitrile (solution B)

Gradient (solution B): 40% (0.00 min.)→65% (6.00 min.)→95%

(6.50 min.)→95% (9.00 min.)

Example 5-2

[C490]

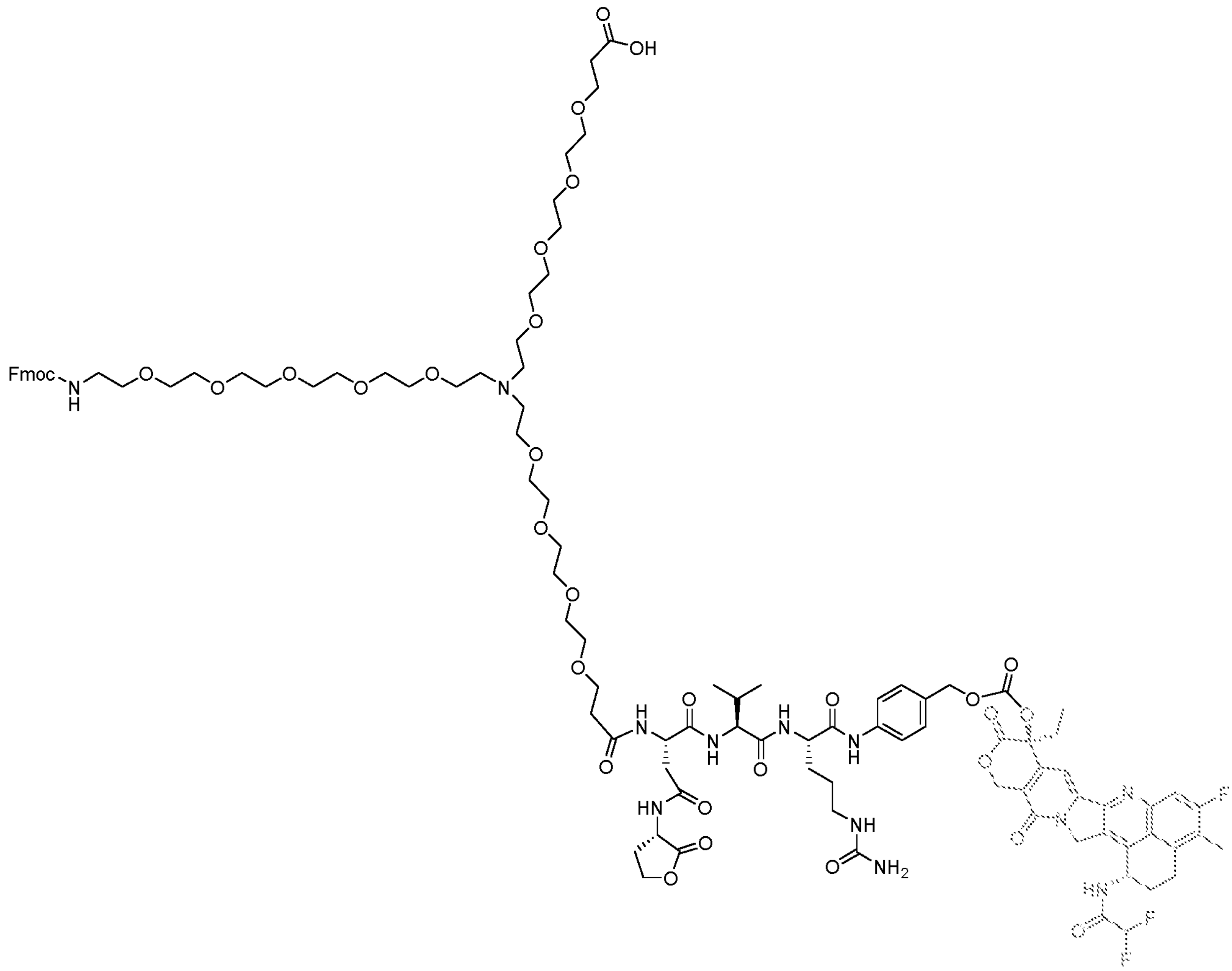

To a solution of Example 5-1 (68 mg, 0.068 mmoL) in acetonitrile (2 mL) put into a 20 mL cylindrical flask, under argon airflow with stirring, N,N-diisopropylethylamine (25 μL) (18.5 mg, 0.143 mmoL) was added, HATU (33 mg, 0.087 mmoL) was then added at room temperature, and the mixture was stirred at room temperature for 10 minutes. Next, a solution of Example 4-2 (40 mg, 0.036 mmoL) in N,N-dimethylformamide (3 mL) was added at room temperature with stirring, and the mixture was stirred at room temperature for 1 hour.

After the reaction was completed, water (7 mL) was added, the mixture was subjected to preparative HPLC under the following conditions, and fractions containing a target compound were concentrated under reduced pressure to obtain a title compound (45 mg, yield 59.89%) as a dark brown oil.
MS(ESI)m/z 1050$(M+2H)^{2+}$

Column: Waters XBridge Prep C18 5 μm ODB 19*150 mm

Flow rate: 17 mL/min.

Elution solvent: 0.1% formic acid aqueous solution (solution A)-acetonitrile (solution B)

Gradient (solution B): 40% (0.00 min.)→65% (6.00 min.)→95%

(6.50 min.)→95% (9.00 min.)

Example 5-3

[C491]

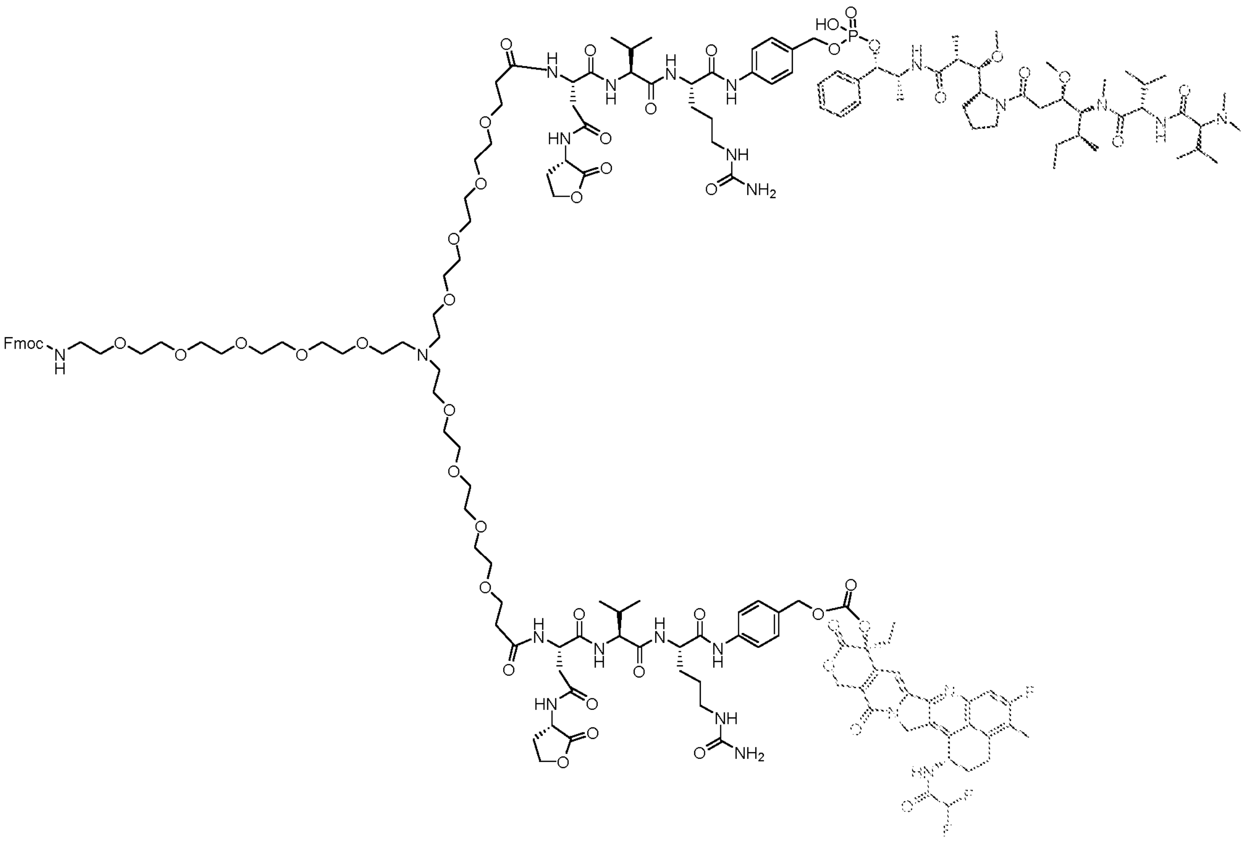

To a solution of Example 5-2 (45 mg, 0.021 mmoL) in acetonitrile (2 mL) put into a 20 mL cylindrical flask, under argon airflow with stirring, N,N-diisopropylethylamine (16 μL) (11.84 mg, 0.092 mmoL) was added, HATU (11 mg, 0.029 mmoL) was then added at room temperature, and the mixture was stirred at room temperature for 10 minutes. Next, a solution of Example 2-7 (18 mg, 0.013 mmoL) in N,N-dimethylformamide (4 mL) was added at room temperature with stirring, and the mixture was stirred at room temperature for 45 minutes. Next, water was added, and the solvent was removed under reduced pressure. A 40% acetonitrile aqueous solution (10 mL) was added to the resulting residues, the mixture was subjected to preparative HPLC under the following conditions, and fractions containing a target compound were freeze-dried to obtain a title compound (29 mg, yield 64.02%) as a white solid.
MS(ESI)m/z 1152$(M+3H)^{3+}$

Column Waters XBridge Prep C18 5 μm ODB 19*150 mm

Flow rate: 17 mL/min.

Elution solvent: 0.1% formic acid aqueous solution (solution A)-acetonitrile (solution B)

Gradient (solution B): 40% (0.00 min.)→65% (6.00 min.)→95%

(6.50 min.)→95% (9.00 min.)

Example 5

[C492]

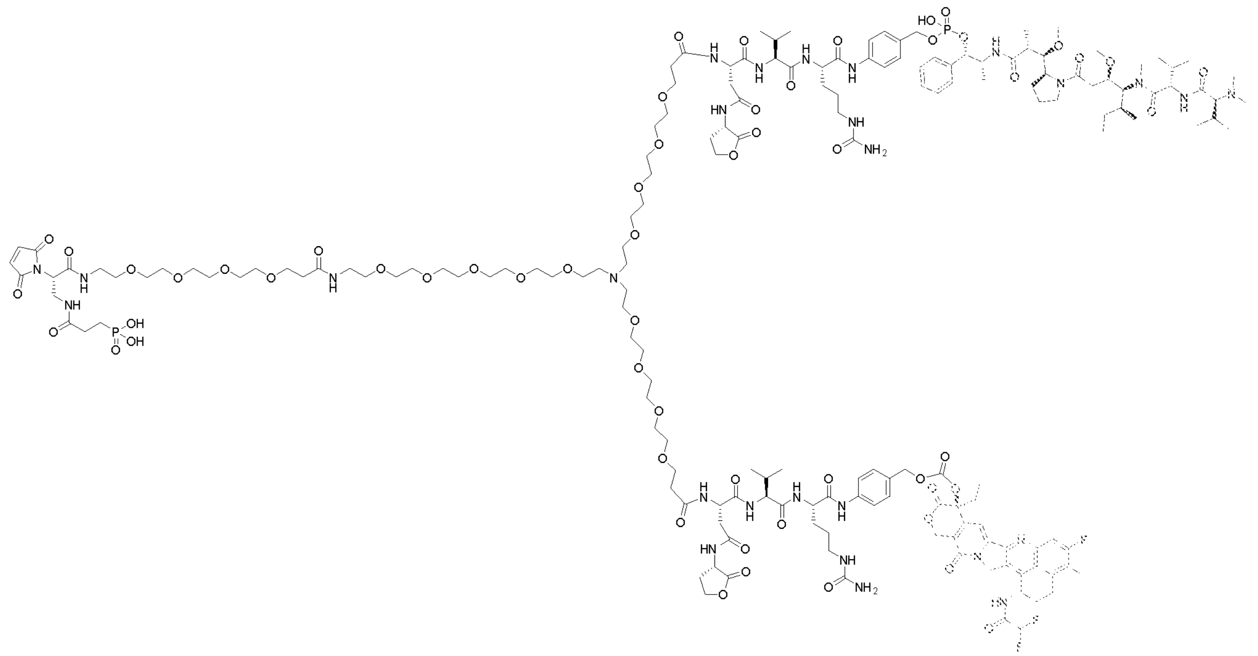

The reaction was performed in the same manner as in Example 2 except that Example 5-3 (15 mg, 4.35 μmoL) was used instead of Example 2-6 (6.3 mg, 0.011 mmoL) and Example 2-10 to obtain a title compound (5.2 mg, yield 31.66%) as a white solid.
MS(ESI)m/z 1261(M+3H)$^{3+}$

Synthesis of Example 6

[C493]

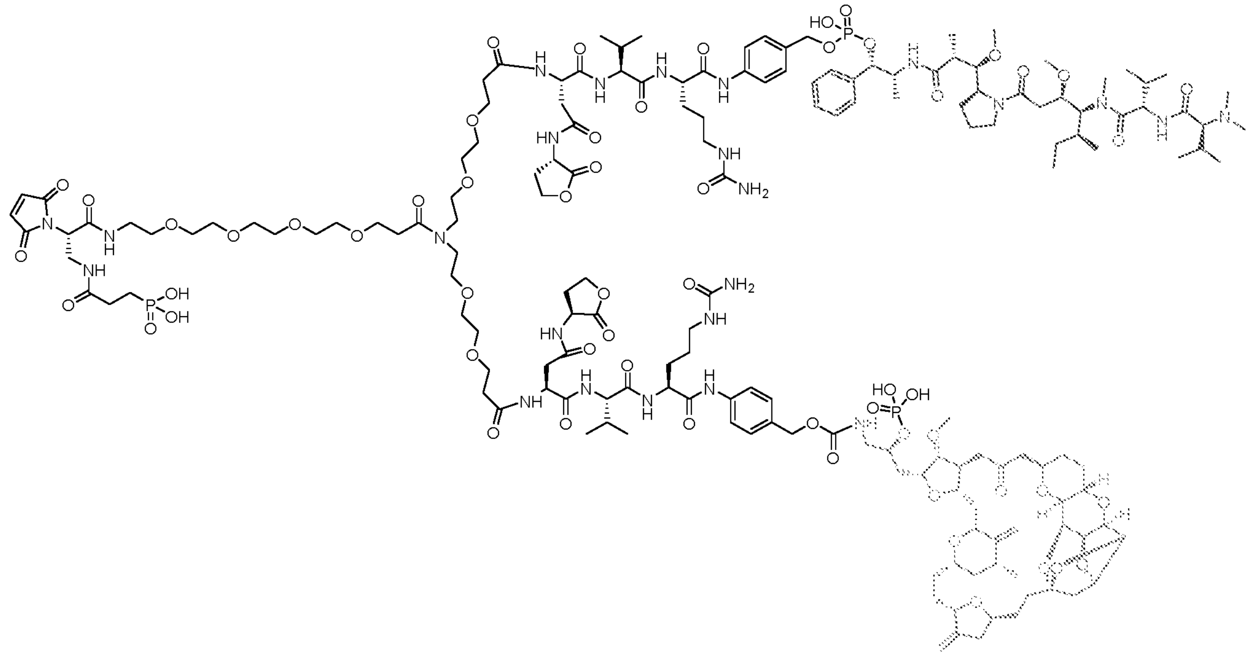

Example 6-1

[C494]

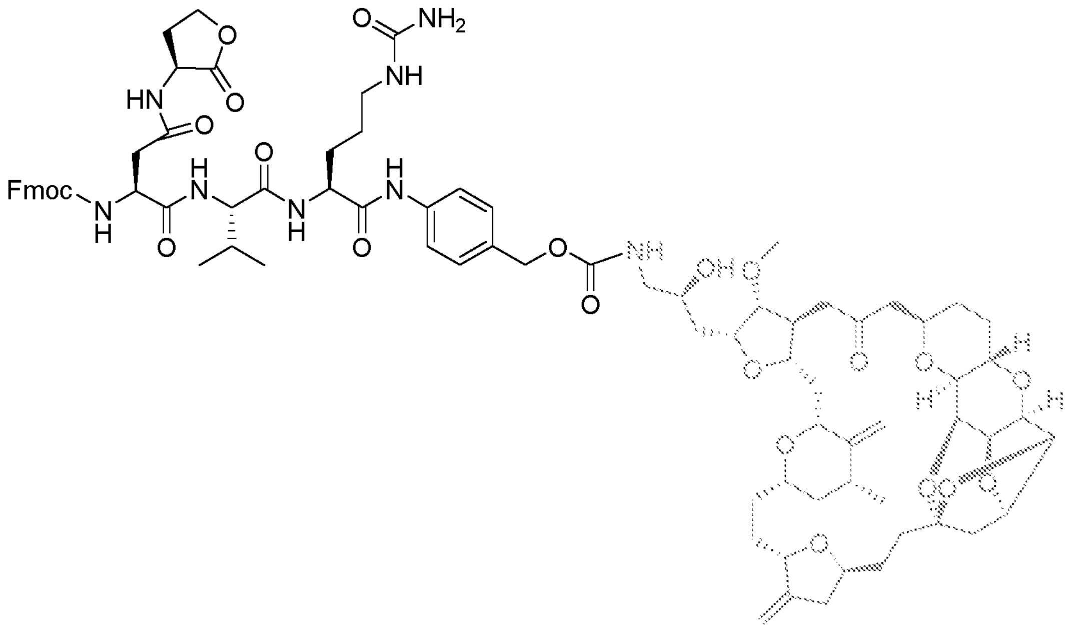

To a solution of Example 1-10 (23.1 mg, 0.024 mmoL) and eribulin mesylate (16.4 mg, 0.020 mmoL) in N,N-dimethylformamide (0.3 mL) put into a 10 mL pear-shaped flask, N,N-diisopropylethylamine (10 μL) (7.42 mg, 0.057 mmoL) and 4-dimethylaminopyridine (0.25 mg, 2.046 μmoL) were sequentially added, and the mixture was stirred at room temperature for 30 minutes.

The resulting residues were subjected to preparative HPLC under the following conditions, and fractions containing a target compound were freeze-dried to obtain a title compound (21.0 mg, yield 67.98%) as a colorless amorphous component.

MS(DUIS)m/z 1555(M+H)$^+$

Column Waters XBridge Prep C18 5 μm ODB 19*150 mm
Flow rate: 17 mL/min.
Elution solvent: 0.1% formic acid aqueous solution (solution A)-acetonitrile (solution B)
Gradient (solution B): 40% (0.00 min.)→84% (8.00 min.)

Example 6-2

[C495]

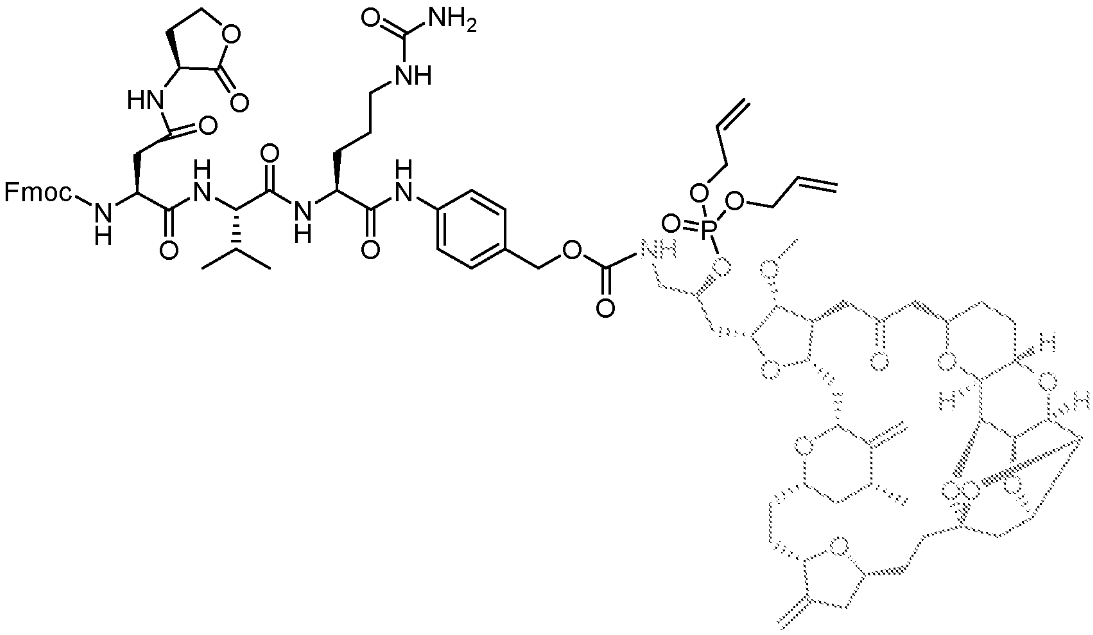

To a solution of Example 6-1 (19.2 mg, 0.012 mmoL) in N,N-dimethylformamide (0.8 mL) put into a 5 mL sample tube, under argon atmosphere with stirring, diallyl N,N-diisopropyl phosphoramidite (49 μL) (45.47 mg, 0.185 mmoL) was added, a solution of 1H-tetrazole (13.0 mg, 0.186 mmoL) of N,N-dimethylformamide (0.2 mL) was then added at room temperature, and the mixture was stirred at room temperature for 1 hour.

Then, 30% by weight of hydrogen peroxide (19 μL) (21.09 mg, 0.186 mmoL) was added, and the mixture was stirred at room temperature for 1 hour.

The resulting reaction solution was subjected to preparative HPLC under the following conditions, and fractions containing a target compound were freeze-dried to obtain a title compound (10.2 mg, yield 48.17%) as a colorless foam. MS(ESI)m/z 859(M+2H)$^2$

Column: Waters XBridge Prep C18 5 μm ODB 19*150 mm
Flow rate: 17 mL/min.
Elution solvent: 0.1% formic acid aqueous solution (solution A)-acetonitrile (solution B)
Gradient (solution B): 50% (0.00 min.)→95% (10.00 min.)

Example 6-3

[C496]

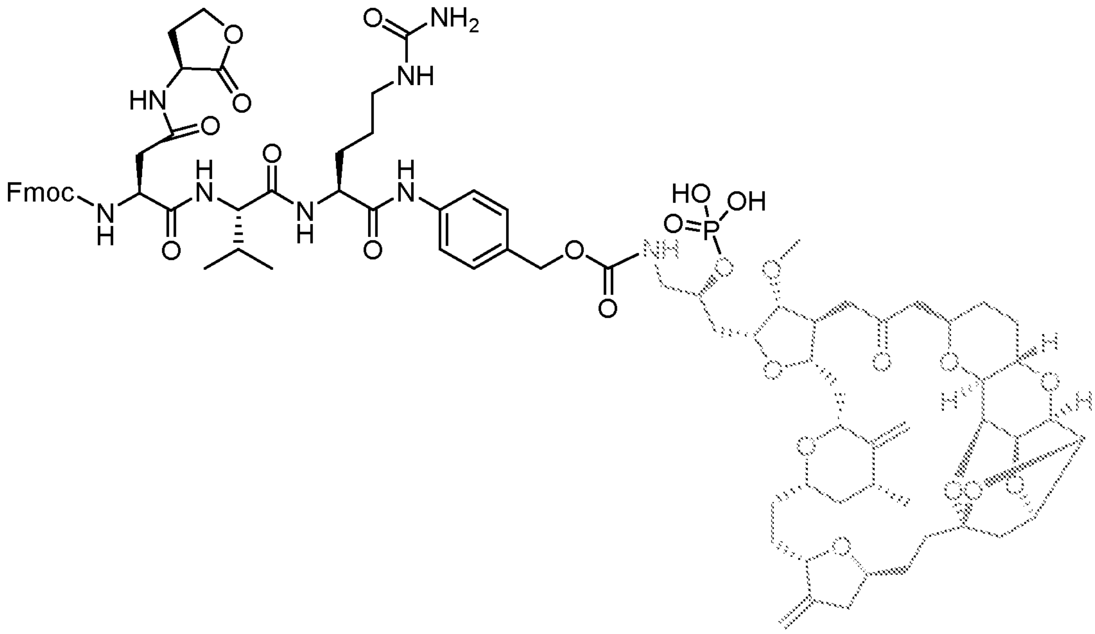

To a solution of Example 6-2 (15.1 mg, 8.80 μmoL, including one obtained in the same method as in Example 26-1) in N,N-dimethylformamide (0.5 mL) put into a 5 mL sample tube, under argon atmosphere with stirring, N-methylaniline (3.5 μL) (3.47 mg, 0.032 mmoL) and tetrakistriphenylphosphine palladium (2.0 mg, 1.731 μmoL) were sequentially added at room temperature, and the mixture was stirred at room temperature for 2 hours.

The resulting reaction solution was subjected to preparative HPLC under the following conditions, and fractions containing a target compound were freeze-dried to obtain a title compound (9.3 mg, yield 64.61%) as a colorless solid. MS(ESI)m/z 819(M+2H)$^2$

Column: Waters XBridge Prep C18 5 μm ODB 19*150 mm
Flow rate: 17 mL/min.
Elution solvent: 0.1% formic acid aqueous solution (solution A)-acetonitrile (solution B)
Gradient (solution B): 30% (0.00 min.)→70% (8.00 min)

Example 6-4

[C497]

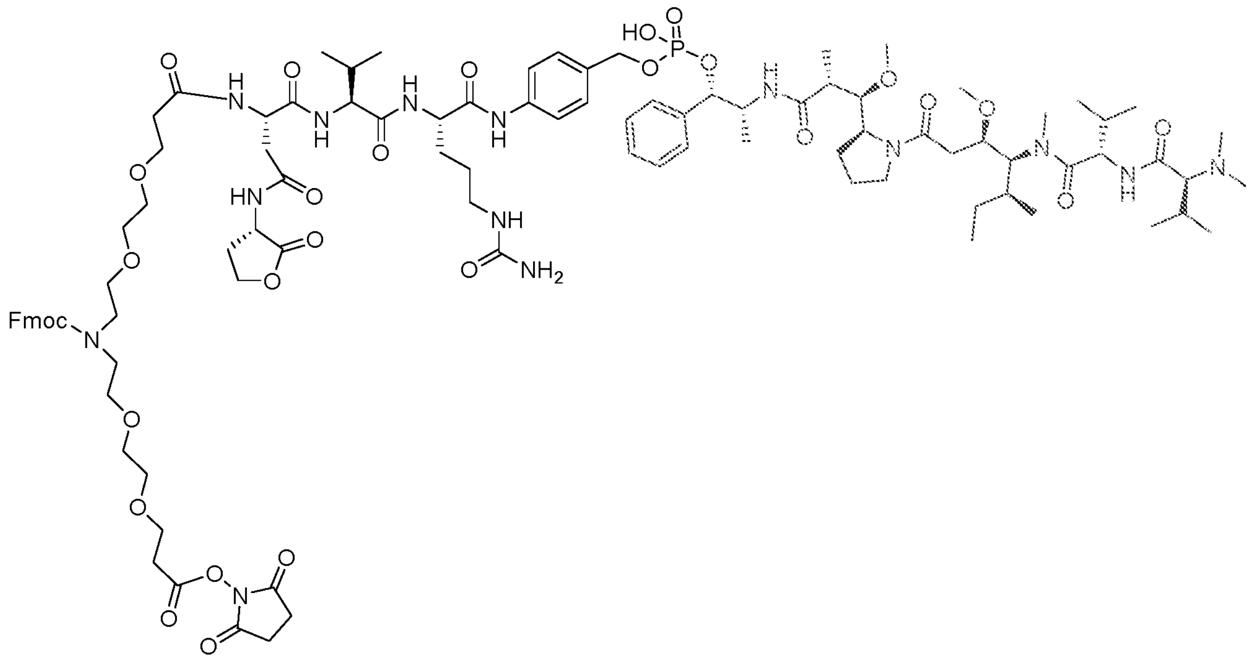

To a solution of Example 2-7 (99.0 mg, 0.072 mmoL) in N,N-dimethylformamide (1 mL) put into a 5 ml sample tube, under argon atmosphere with stirring, Example 2-9 (42.0 mg, 0.056 mmoL), and 1-hydroxybenzotriazole (4.9 mg, 0.036 mmoL) were added at room temperature, and the mixture was stirred at room temperature for 90 minutes. Next, N,N-dimethylbenzylamine (32 μL) (29.12 mg, 0.215 mmoL), and Example 2-7 (11.9 mg, 8.65 μmoL) were added, and the mixture was stirred at room temperature for 16 hours.

The resulting reaction solution was dissolved in N,N-dimethylformamide and subjected to preparative HPLC under the following conditions, and fractions containing a target compound (Rt=about 7.7 min.) were freeze-dried to obtain a title compound (26.1 mg, yield 43.12%) as a colorless foam.
MS(ESI)m/z 1006$(M+2H)^{2+}$

Column: Waters Xbridge Amide 5 μm ODB 19*150 mm
Flow rate: 17 ml/min.
Elution solvent: 0.1% formic acid aqueous solution (solution A)- acetonitrile (solution B)
Gradient (solution B): 95% (0.00 min.)-50% (10.00 min.) (same as Example 2-10, Rt of Example 2-10 was 9.0 min)

Example 6-5

[C498]

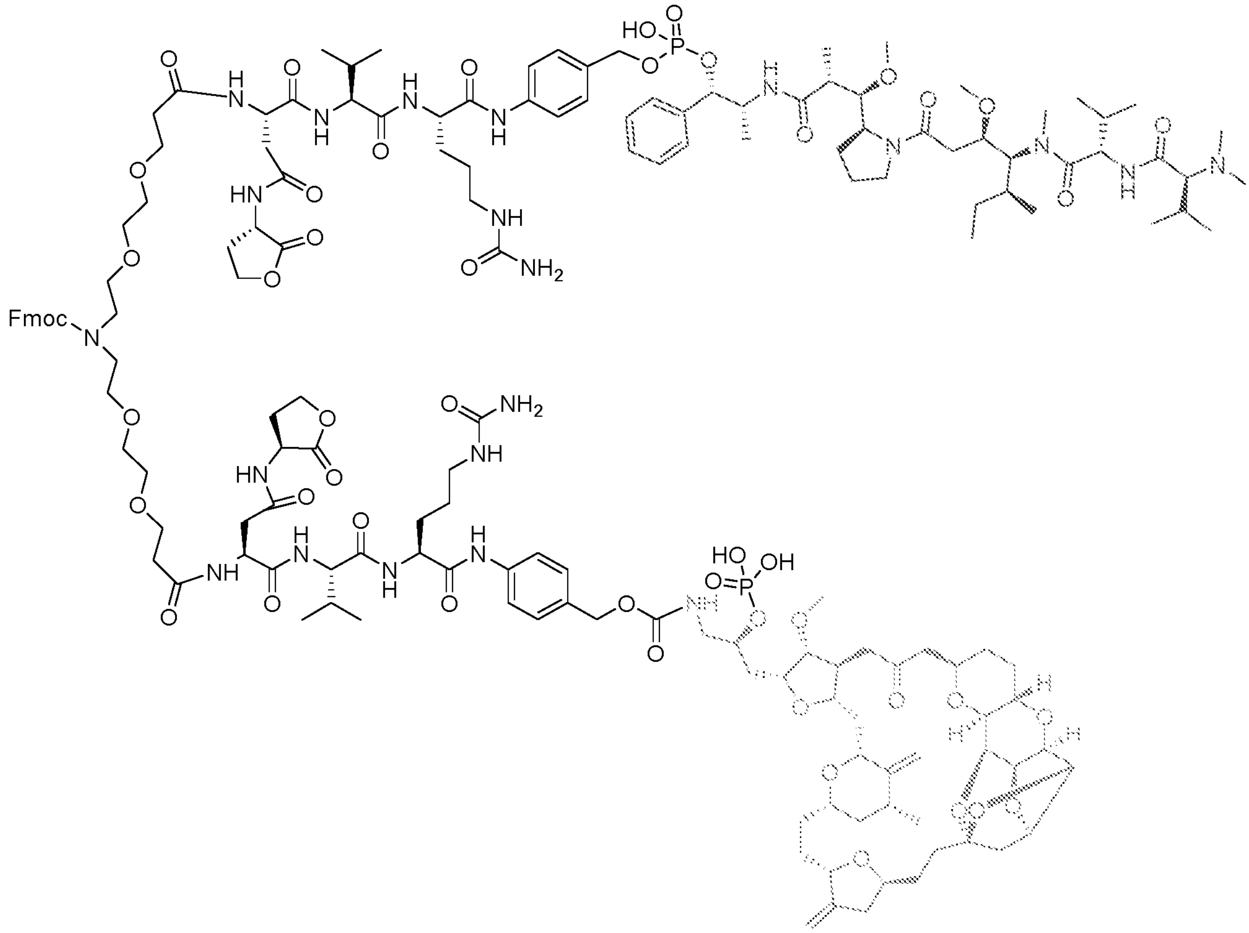

To a solution of Example 6-3 (7.8 mg, 4.77 μmoL) in N,N-dimethylformamide (0.15 mL) put into a 5 ml sample tube, under air atmosphere with stirring, diazabicycloundecene (1.5 μL) (1.52 mg, 9.95 μmoL) was added at room temperature, and the mixture was stirred at room temperature for 1 hour. Methanesulfonic acid (0.65 μL) (0.96 mg, 10.01 μmoL), triethylamine (3.5 μL) (2.54 mg, 0.025 mmoL), and Example 6-4 (9.9 mg, 4.92 μmoL) were then sequentially added, and the mixture was stirred at room temperature for 1 hour. Next, HATU (1.5 mg, 3.94 μmoL) was added, and the mixture was stirred at room temperature for 12 hours.

Formic acid (2.5 μL) (3 mg, 0.065 mmoL) was added to the resulting reaction solution, the mixture was diluted with N,N-dimethylformamide and subjected to preparative HPLC under the following conditions, and fractions containing a target compound were freeze-dried to obtain a title compound (2.8 mg, yield 17.75%) as a colorless solid.
MS(ESI)m/z 1104$(M+3H)^{3+}$

Column: Waters XBridge Prep C18 5 μm ODB 19*150 mm
Flow rate: 17 ml/min.
Elution solvent: 0.1% formic acid aqueous solution (solution A)-acetonitrile (solution B)
Gradient (solution B): 30% (0.00 min.)-80% (10.00 min.)

Example 6

[C499]

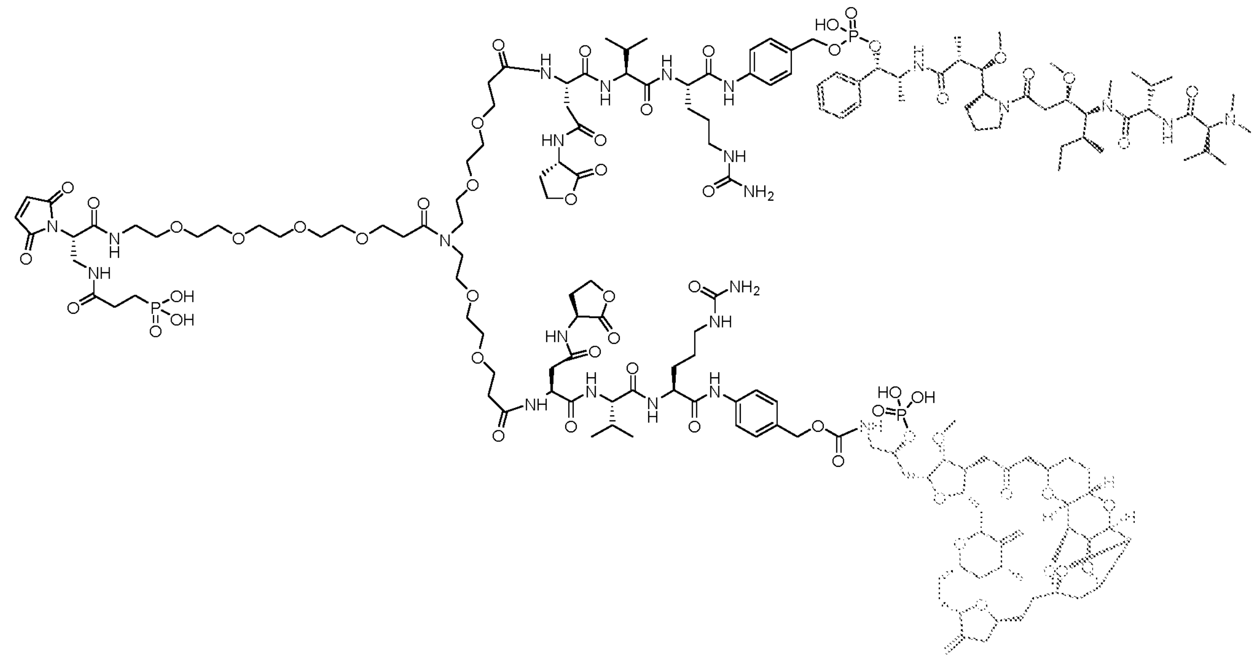

The reaction was performed in the same manner as in Example 2 except that Example 6-5 (2.8 mg, 0.846 μmoL) was used instead of Example 2-6 (10.8 mg, 0.019 mmoL) and Example 2-10 to obtain a title compound (1.72 mg, yield 55.9%) as a colorless solid.
MS(ESI)m/z 1213(M+3H)$^{3+}$

Synthesis of Example 7

[C500]

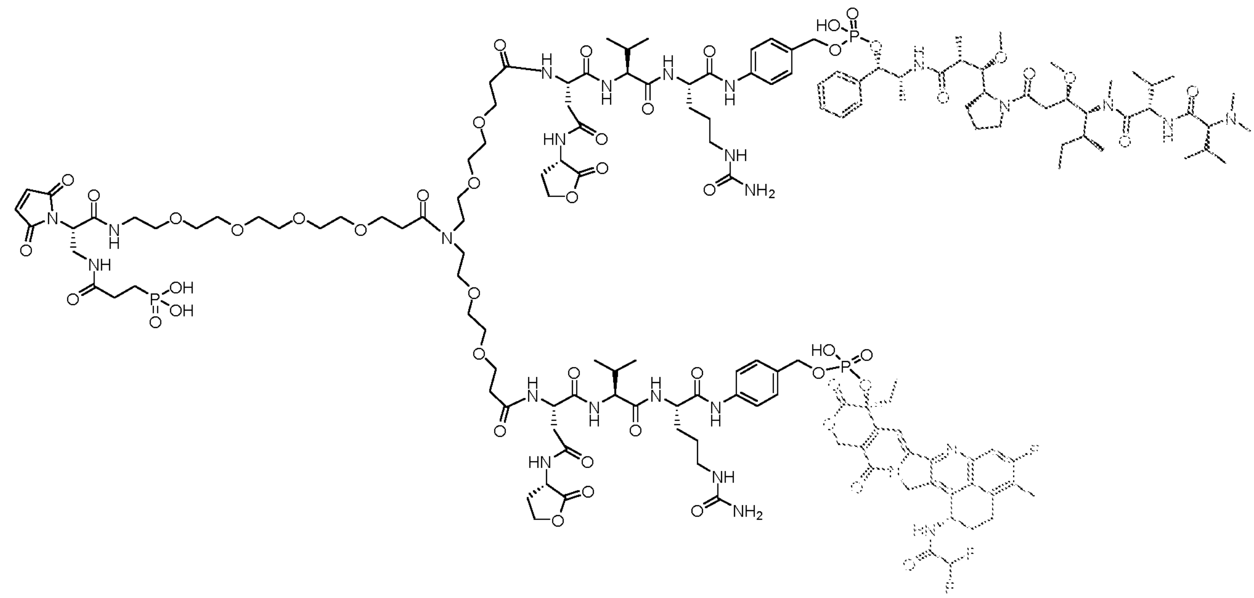

Example 7-1

[C501]

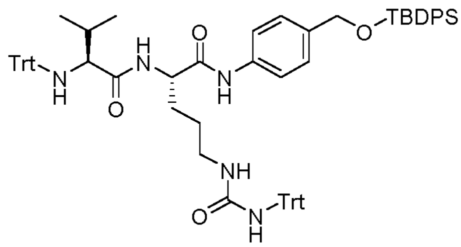

To a solution of (L)-Val-(L)-Cit-PAB(10.0 g, 26.4 mmoL) in tetrahydrofuran (100 mL) put into a 500 mL eggplant flask, under argon airflow with stirring, 1H-imidazole (3.59 g, 52.7 mmoL) was added, the mixture was then cooled on ice, tert-butyldiphenylsilyl chloride (7.45 mL) (7.97 g, 29.0 mmoL) was added, and the mixture was stirred at room temperature for 1.5 hours.

Next, tert-butyldiphenylsilyl chloride (0.5 mL) (0.54 g, 1.946 mmoL) was added with stirring under ice-cooling, and the mixture was stirred at room temperature for 1 hour.

After the reaction was completed, ethyl acetate (300 mL) was added to the reaction solution, insoluble matters were filtered, and washing with ethyl acetate (15 mL) was performed. A saturated sodium bicarbonate aqueous solution (100 mL) and saturated brine (100 mL) were added to the resulting filtrate, the mixture was stirred at room temperature for 15 minutes, and then separated, and the aqueous layer was extracted twice with ethyl acetate (20 mL). The organic layers were combined, dried with anhydrous sodium sulfate (5 g), and filtered, and the filtrate was concentrated under reduced pressure.

The resulting solid was added to tetrahydrofuran (150 mL), a saturated ammonium chloride aqueous solution (150 mL) and water (20 mL) were added, and separation was performed. The aqueous layer was extracted twice with ethyl acetate (20 mL) and washed with saturated brine (50 mL), and the organic layers were combined and dried with sodium sulfate (5 g) and then concentrated under reduced pressure.

To a solution of the resulting residues in dichloromethane (200 mL), under ice-cooling, triethylamine (36.7 mL) (26.64 g, 263 mmoL) and trityl chloride (36.7 g, 132 mmoL) were added, and the mixture was then stirred at room temperature for 1 hour. Next, trityl chloride (36.7 g, 132 mmoL) was added at room temperature with stirring, and the mixture was stirred at room temperature for 1 hour.

Next, triethylamine 36.7 mL (26.64 g, 263 mmoL) and trityl chloride (36.7 g, 132 mmoL) were added at room temperature with stirring, and the mixture was stirred at room temperature for 1 hour.

Next, trityl chloride (15.0 g, 53.8 mmoL) was added at room temperature with stirring, and the mixture was stirred at room temperature for 30 minutes.

After the reaction was completed, water (40 mL) was added, separation was performed, and the aqueous layer was extracted twice with dichloromethane (20 mL). The organic layers were combined, washed with saturated brine, dried with anhydrous sodium sulfate, and filtered, the filtrate was concentrated under reduced pressure, and the precipitated solids were filtered to obtain a solution containing a target compound.

The solution was concentrated under reduced pressure, and when white solids were precipitated, concentration was stopped, and the mixture was stirred for 30 minutes under ice-cooling. The solids were filtered, washed with a mixed solvent (50 mL) of hexane/ethyl acetate=70/30(V/V), and dried under reduced pressure to obtain a title compound (30.2459 g, yield: quantitative) as a white solid.
MS(ESI)m/z 1103(M+H)$^+$

Example 7-2

[C502]

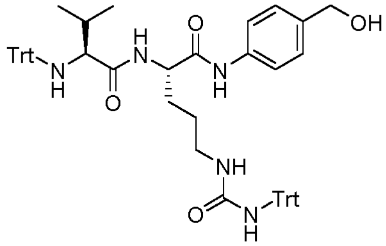

To a solution of Example 7-1 (30.0 g, 27.2 mmoL) in tetrahydrofuran (270 mL) put into a 500 mL eggplant flask, 1N tetra-n-butylammonium fluoride (77 mL) was added at room temperature, and the mixture was then stirred at room temperature for 5 hours.

After the reaction was completed, the reaction solution was concentrated under reduced pressure, the resulting residues were dissolved in dichloromethane (30 mL), subjected to YAMAZEN medium pressure flash chromatography (silica, 3L (135 g), dichloromethane/ethyl acetate=92/8(V/V)-44/56 (V/V), fractions containing a target compound (Rf=0.45 (dichloromethane/ethyl acetate=50/50(V/V)) were collected, and the precipitated solids were filtered, washed with dichloromethane, and dried under reduced pressure to obtain a title compound (16.2604 g, yield 69.16 %) as a white solid. MS(ESI)m/z 864$(M+H)^+$

Example 7-3

[C503]

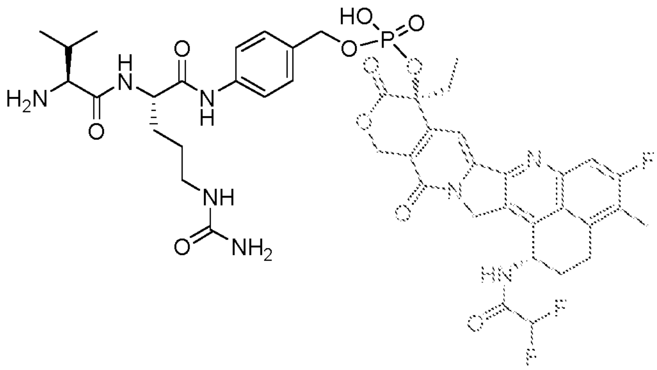

To a solution of Example 7-2 (434 mg, 0.502 mmoL) in dichloromethane (15 mL) put into a 50 mL eggplant flask, under argon atmosphere with stirring, 1-allyloxy N,N,N',N'-tetraisopropylphosphanediamine (190 μL) (171.57 mg, 0.595 mmoL) and 1H-tetrazole (44 mg, 0.628 mmoL) were sequentially added at room temperature, and the mixture was stirred at room temperature for 1 hour.

Next, U-032 (99 mg, 0.193 mmoL) and 5-(ethylthio)-1H-tetrazole (80 mg, 0.615 mmoL) were added, and the mixture was heated and stirred at 43°C for 3 hours.

Next, ice-cooling was performed, a 70% tert-butyl hydroperoxide aqueous solution (145 μL) (136.3 mg, 1.059 mmoL) was added under ice-cooling, and the mixture was stirred under ice-cooling for 1 hour.

After the reaction was completed, water (10 mL) was added to the reaction solution, the mixed solution was extracted with methylene chloride (15 mL), and the organic layer was washed with water, then dried with anhydrous magnesium sulfate, filtered, and concentrated under reduced pressure to obtain a slightly yellow oil.

To a solution of the slightly yellow oil in tetrahydrofuran (15 mL) put into a 100 mL eggplant flask, under argon atmosphere with stirring, N-methylaniline (57 μL) (56.43 mg, 0.527 mmoL) and tetrakistriphenylphosphine palladium (47 mg, 0.041 mmoL) were sequentially added at room temperature, the mixture was stirred at room temperature for 2 hours, and the solvent was then removed under reduced pressure.

Tert-butyl methyl ether was added to the residues, sonication was performed, and the supernatant was removed. Next, water was added to the residues, sonication was performed, the supernatant was removed, and the mixture was dried under reduced pressure to obtain residues (770 mg).

To a solution of the residues in dichloromethane (10 mL) put into a 100 mL eggplant flask, trifluoroacetic acid (166 μL) (245.68 mg, 2.155 mmoL) was added at room temperature, and the mixture was stirred at room temperature for 3

hours. Next, trifluoroacetic acid (83 μL) (122.84 mg, 1.077 mmoL) was added at room temperature, and the mixture was stirred at room temperature for 1 hour.

After the reaction was completed, tert-butyl methyl ether (20 mL) was added, the supernatant was removed, the residues were dissolved in a 30% acetonitrile aqueous solution and subjected to preparative HPLC under the following conditions, and fractions containing a target compound were freeze-dried to obtain a title compound (65.1 mg, yield 35.35%) as a slightly yellow solid.
MS(ESI)m/z 955(M+H)+

Column: Waters XBridge Prep C18 5 μm ODB 19*150 mm

Flow rate: 17 mL/min.

Elution solvent: 0.1% formic acid aqueous solution (solution A)-acetonitrile (solution B)

Gradient (solution B): 30% (0.00 min.)→60% (6.00 min.)-90%

(6.50 min.)→90% (9.00 min.)

Example 7-4

[C504]

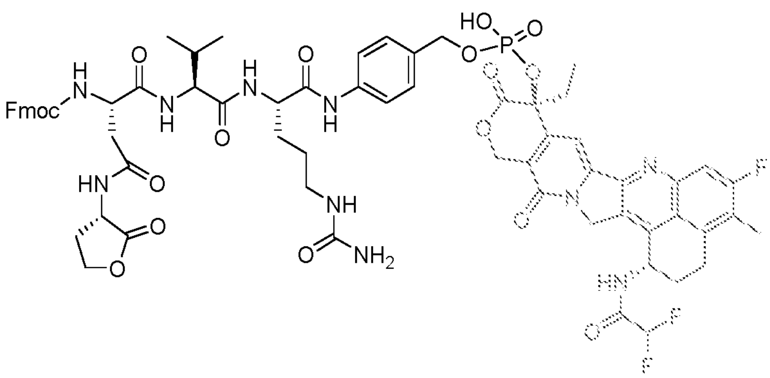

To a solution of Example 1-2 (39 mg, 0.089 mmoL) in acetonitrile (1.2 mL) put into a 20 mL cylindrical flask, under argon airflow with stirring, triethylamine (14 μL) (10.16 mg, 0.100 mmoL) and HATU (33 mg, 0.087 mmoL) were added at room temperature, and the mixture was stirred at room temperature for 15 minutes.

Next, a solution of Example 7-3 (65 mg, 0.068 mmoL) and triethylamine (10 μL) (7.26 mg, 0.072 mmoL) in N,N-dimethylformamide (1.8 mL) was added at room temperature with stirring, and the mixture was stirred at room temperature for 0.5 hours.

Next, Example 1-2 (12 mg, 0.027 mmoL), HATU (5.0 mg, 0.013 mmoL) and triethylamine (8 μL) (5.81 mg, 0.057 mmoL) were added at room temperature with stirring, and the mixture was stirred at room temperature for 0.5 hours.

After the reaction was completed, the reaction solution was concentrated under reduced pressure, water (4 mL) and acetonitrile (3 mL) were added to the residues, and the precipitated solids were filtered, washed with ethyl acetate, and dried under reduced pressure to obtain a title compound (60 mg, yield 64.09%) as a slightly yellow solid.
MS(ESI)m/z 1376(M+H)+

Example 7-5

[C505]

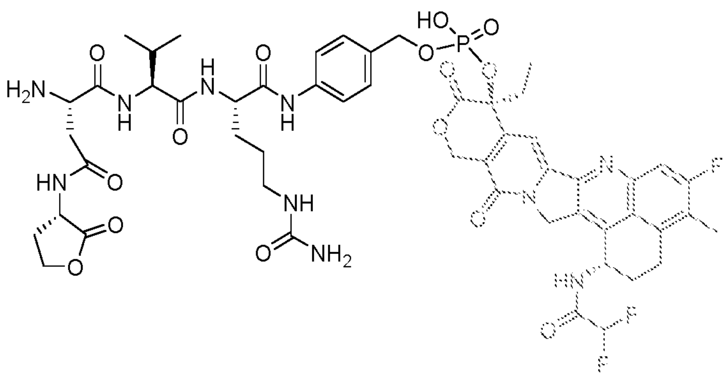

To a solution of Example 7-4 (60 mg, 0.044 mmoL) in N,N-dimethylformamide (1 mL) put into a 10 mL cylindrical flask, under argon airflow with stirring, piperidine (0.013 mL) (11.14 mg, 0.131 mmoL) was added at room temperature, the mixture was stirred at room temperature for 1 hour, and the solvent was then removed under reduced pressure.

The resulting residues were washed with ethyl acetate and then diethyl ether, and dried under reduced pressure to obtain a title compound (45 mg, yield 89.46%) as a dark brown solid. MS(ESI)m/z 1154$(M+H)^+$

Example 7-6

[C506]

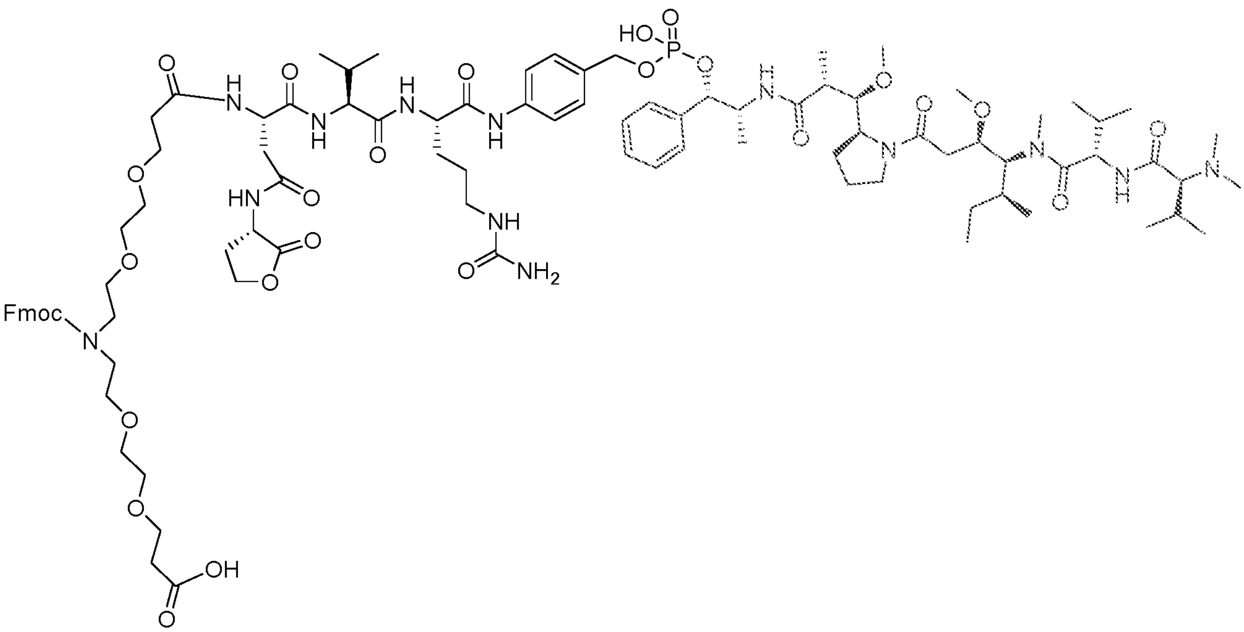

To a solution of Example 2-8 (75 mg, 0.134 mmoL) in acetonitrile (1 mL) put into a 20 mL cylindrical flask, under argon airflow with stirring, N,N-diisopropylethylamine (30 μL) (22.2 mg, 0.172 mmoL) was added, HATU (50 mg, 0.131 mmoL) was then added at room temperature, and the mixture was stirred at room temperature for 10 minutes.

Next, a solution of Example 2-7 (52 mg, 0.038 mmoL) in N,N-dimethylformamide (2 mL) was added at room temperature with stirring, the mixture was stirred at room temperature for 1 hour, water was then added, and the solvent was removed under reduced pressure.

A 40% acetonitrile aqueous solution (12 mL) was added to the resulting reaction solution, the mixture was subjected to preparative HPLC under the following conditions, and fractions containing a target compound were freeze-dried to obtain a title compound (41 mg, yield 56.53%) as a white solid.
MS(ESI)m/z 957$(M+2H)^{2+}$

Column: Waters XBridge Prep C18 5 μm ODB 19*150 mm
Flow rate: 17 ml/min.
Elution solvent: 0.1% formic acid aqueous solution (solution A)-acetonitrile (solution B)
Gradient (solution B): 40% (0.00 min.)-65% (6.00 min.)-95% (6.50 min.)-95% (9.00 min.)

Example 7-7

[C507]

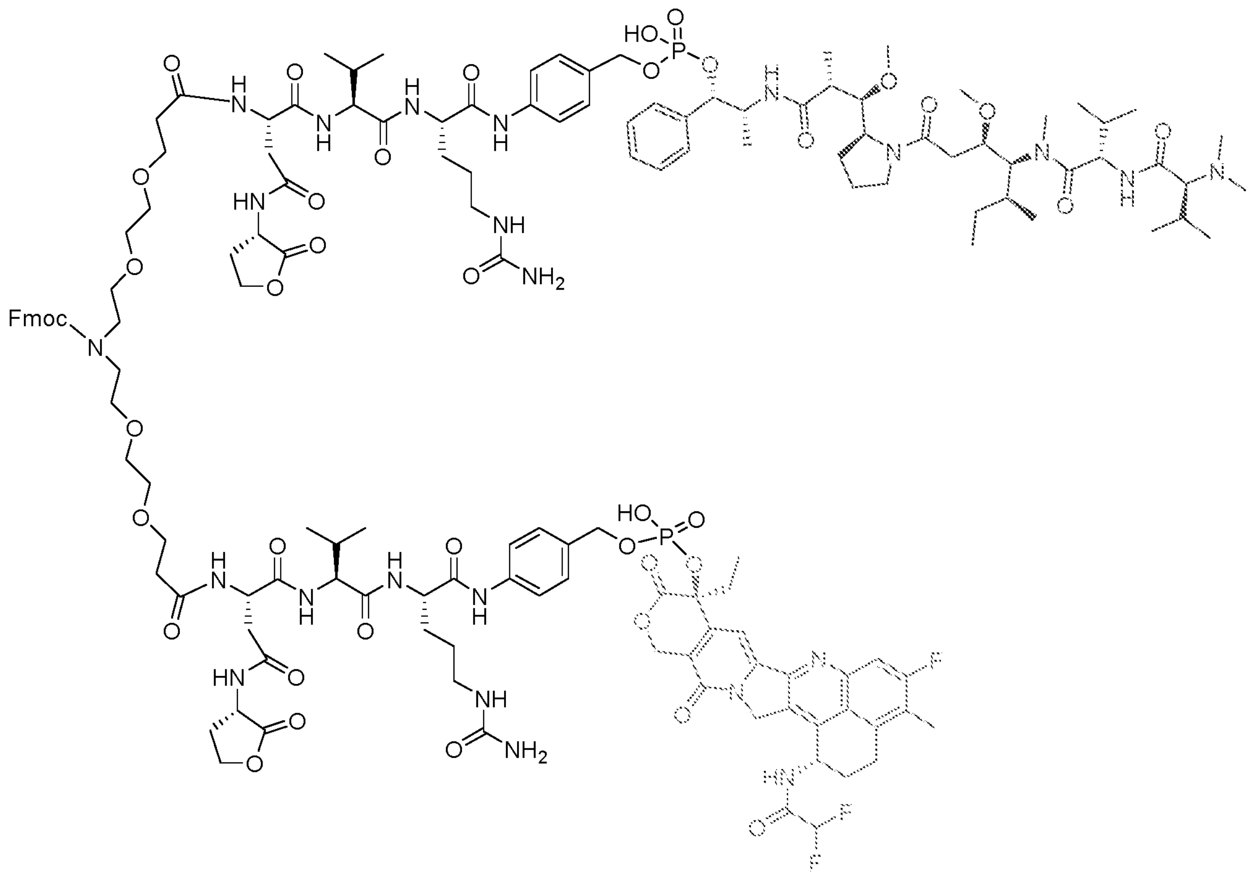

To a solution of Example 7-6 (22 mg, 0.011 mmoL) in N,N-dimethylformamide (0.6 mL) put into a 20 mL cylindrical flask, under argon airflow with stirring, N,N-diisopropylethylamine (3.6 μL) (2.66 mg, 0.021 mmol) was added, HATU (5.5 mg, 0.014 mmoL) was then added at room temperature, and the mixture was stirred at room temperature for 5 minutes.
Next, a solution of Example 7-5 (12 mg, 10.41 μmoL) in N,N-dimethylformamide (0.6 mL) and N,N-diisopropylethylamine (3.6 μL) (2.66 mg, 0.021 mmol) were added at room temperature with stirring, the mixture was stirred at room temperature for 1 hour, and the solvent was the removed under reduced pressure.
A 40% acetonitrile aqueous solution (5 mL) was added to the resulting reaction solution, the mixture was subjected to preparative HPLC under the following conditions, and fractions containing a target compound were freeze-dried to obtain a title compound (15.5 mg, yield 48.86%) as a white solid.
MS(ESI)m/z 1017(M+3H)$^{3+}$

Column: Waters XBridge Prep C18 5 μm ODB 19*150 mm
Flow rate: 17 ml/min.
Elution solvent: 0.1% formic acid aqueous solution (solution A)-acetonitrile (solution B)
Gradient (solution B): 40% (0.00 min.)-60% (6.00 min.)-90% (6.50 min.)-90% (9.00 min.)

Example 7

[C508]

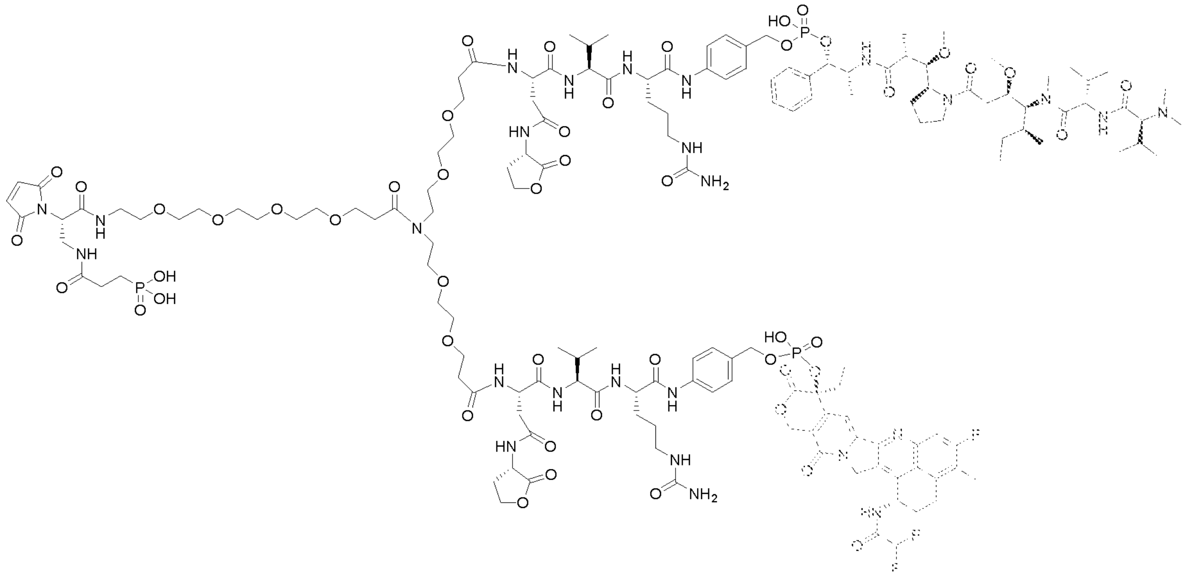

A title compound (6.5 mg, yield: 39.13%) was obtained as a pale yellow solid by carrying out the same reaction as that in Example 2 except that Example 7-6 (15 mg, 4.92 μmoL) was used instead of Example 2-6 (28 mg, 0.049 mmol) and Example 2-10.

MS (ESI) m/z 1126 $(M+3H)^{3+}$

Further, when the linker moieties or the antitumor drug residue moieties of the conjugate precursor (I) and the conjugate precursor synthesis intermediates (II-1) and (II-2) of the present invention includes partial structure(s) having asymmetric center(s) and optical isomer(s) may be generated, both D-type and L-type optical isomers are also encompassed by the present invention.

In addition, the present invention also encompasses compounds labeled with various radioactive or non-radioactive isotopes. One or more atoms constituting the conjugate precursor (I) and the conjugate precursor synthesis intermediates (II-1) and (II-2) of the present invention may also include unnatural proportion(s) of atomic isotope(s). Examples of atomic isotopes include deuterium ($^2H$), tritium ($^3H$), iodine-125 ($^{125}I$), and carbon-14 ($^{14}C$). In addition, conjugate precursor (I) and the conjugate precursor synthesis intermediates (II-1) and (II-2) of the present invention may be radioactively labeled with radioactive isotopes such as tritium ($^3H$), iodine-125 ($^{125}I$), and carbon-14 ($^{14}C$). All isotopic variants of all of the conjugate precursor (I) and the conjugate precursor synthesis intermediates (II-1) and (II-2) of the present invention are encompassed within the scope of the present invention, regardless of whether they are radioactive or not.

[Examples]: Production of antibody-multidrug conjugate (AMDC) by reaction of conjugate precursor with antibody (Example 1) and evaluation test of AMDC (Example 2) Example 1. Production of antibody-multidrug conjugate (AMDC)

An AMDC produced by reacting the conjugate precursor (I) of the present with an "antibody" is represented by General Formula (III) below.

[C509]

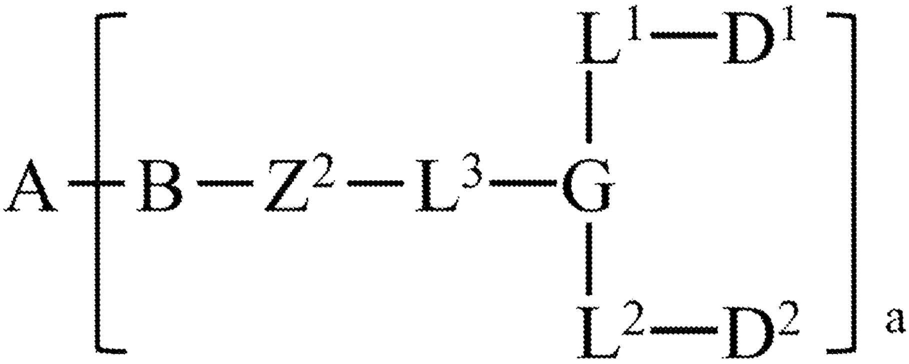

(III)

In General Formula (III) above, "A-" is a residue of the above-defined antibody, "-B-" is a "divalent residue derived from a functional group in an antibody" produced by a reaction of the "above-defined functional group in an antibody" with the "above-defined reactive group," $Z^2$ is a "divalent residue derived from a reactive group" produced by a reaction of "said reactive group" with "said functional group in an antibody," G, $L^1$, $L^2$, $L^3$, $D^1$, and $D^2$ are synonymous with the above, and a is an integer of 1 to 10.

A group of the following formula in which "A-B-$Z^2$-$L^3$-" is removed from Formula (III) above may be referred to as a "multidrug unit (MDU)."

[C510]

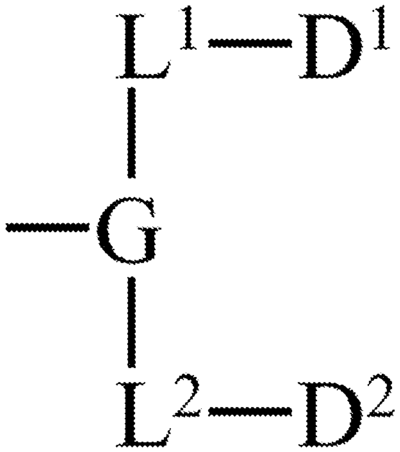

(MDU)

[in the formula, the symbols are synonymous with the above.]

A specific example of an AMDC produced by reacting the conjugate precursor (I) of the present invention with an antibody is shown as Exemplification 8, but the present invention is not limited thereto.

Exemplification 8. "Specific example of AMDC produced using conjugate precursor (I)"

Exemplification 8-1

[C511]

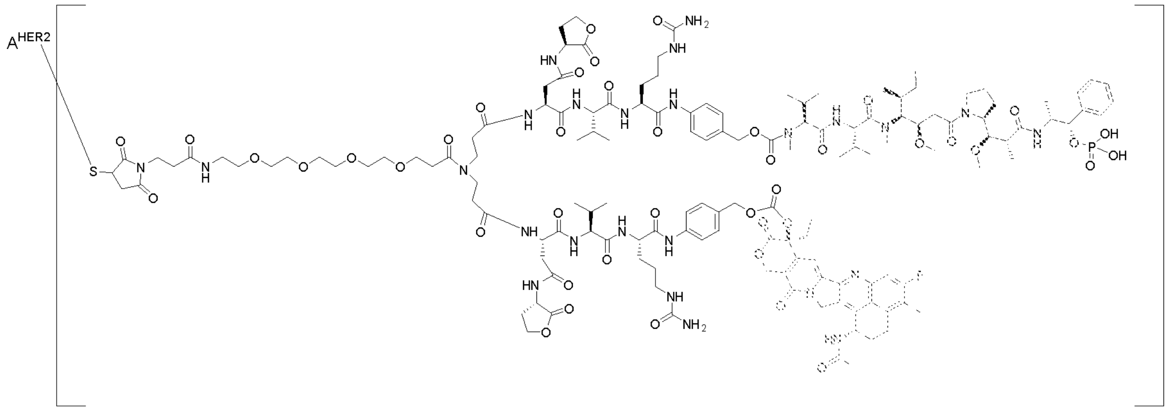

a

Exemplification 8-2

[C512]

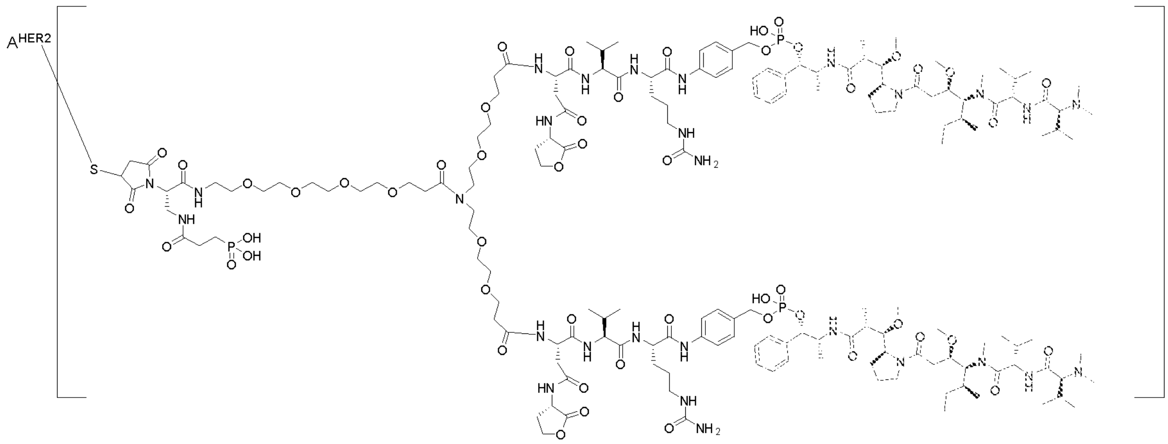

a

Exemplification 8-3

[C513]

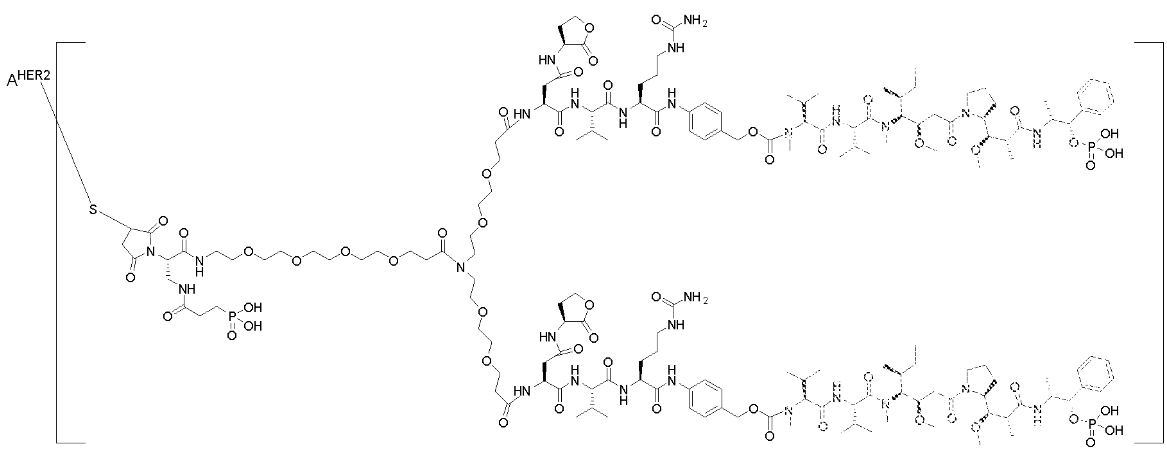

a

Exemplification 8-4

[C514]

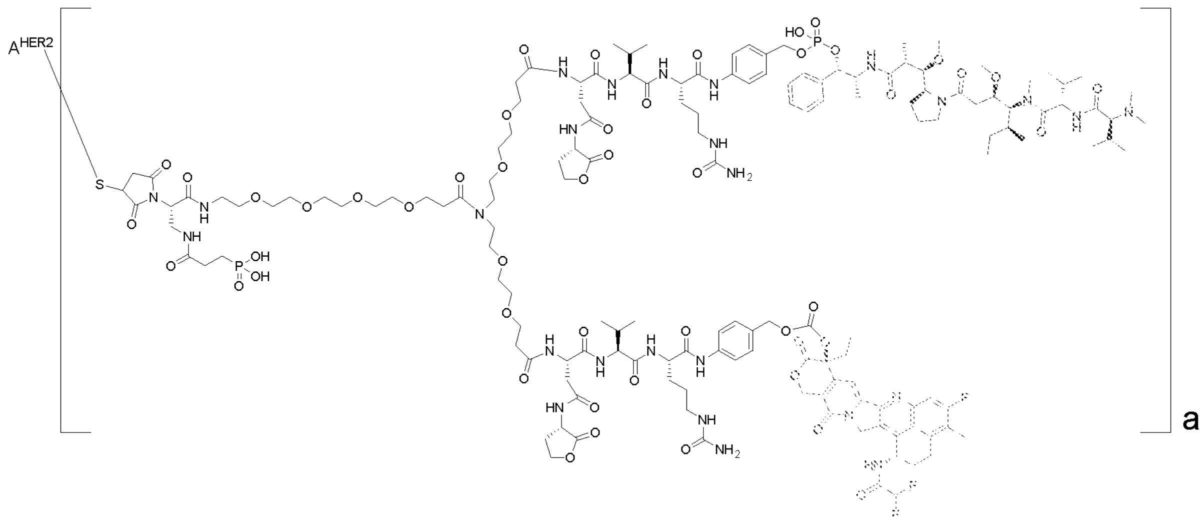

Exemplification 8-5

[C515]

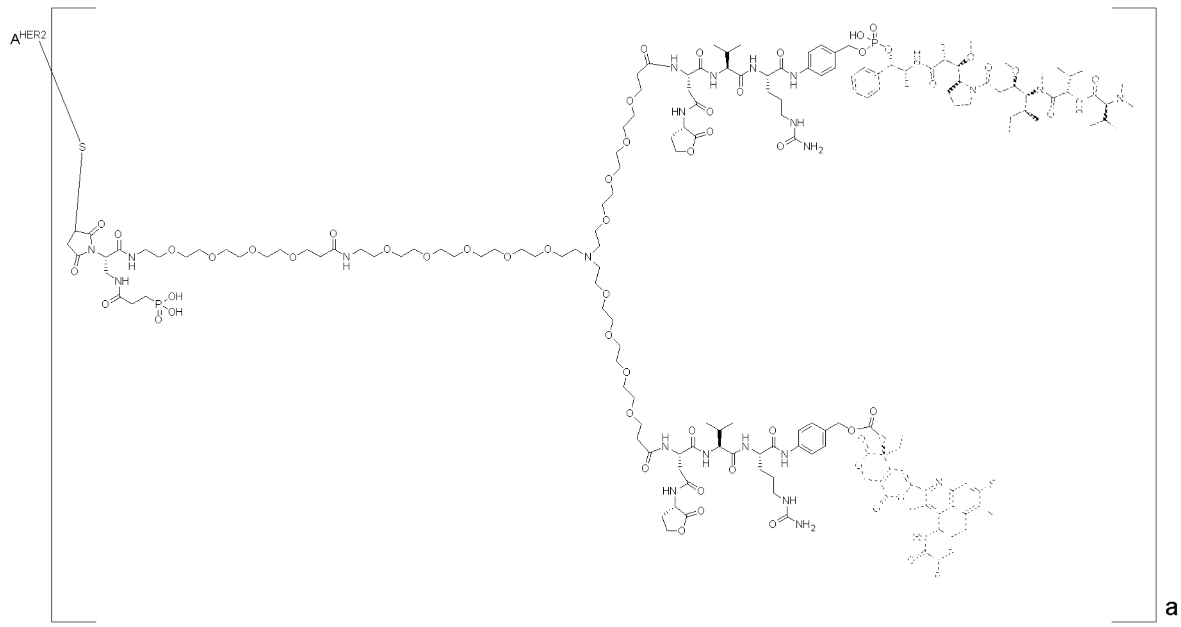

Exemplification 8-6

[C516]

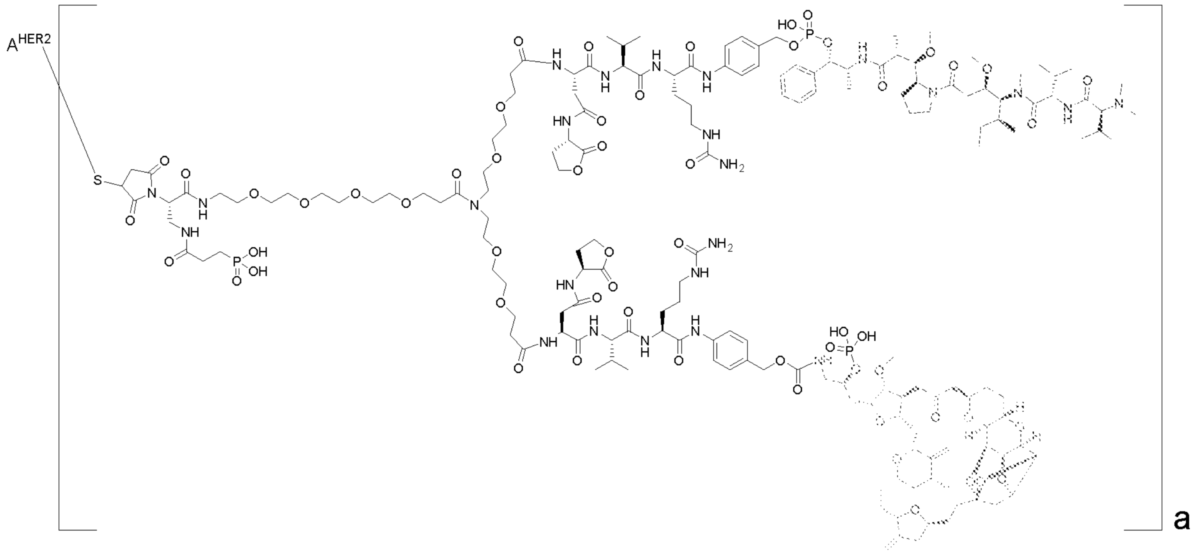

Exemplification 8-7

[C517]

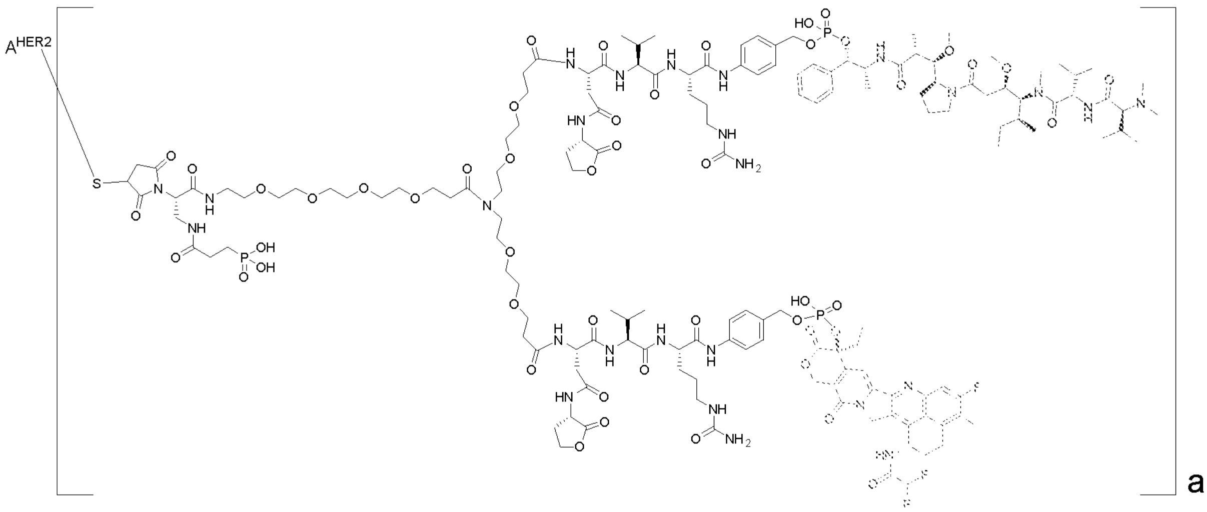

[a in the formulae is an integer of 1 to 10.]

Specific examples of ADCs produced by reacting the compounds of the Example A series of the present invention are shown as ADCs of Exemplification A8 series, but the present invention is not limited thereto.

Exemplification A8. "Specific examples of ADCs of A8 series produced using compounds of Example A series"

Exemplification A8-1

[C518]

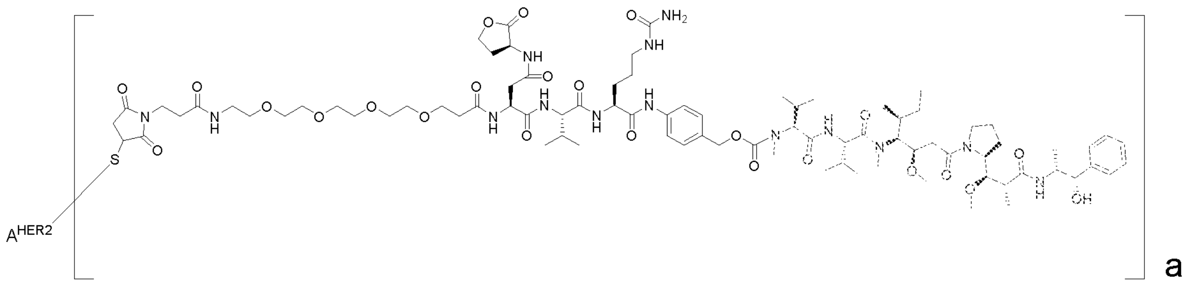

Exemplification A8-2

[C519]

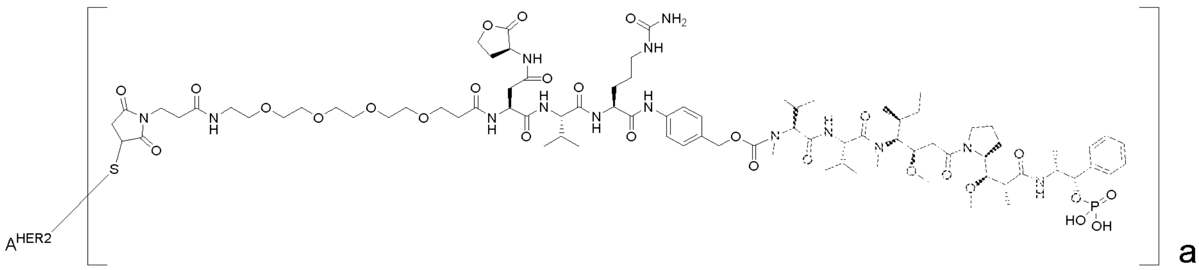

Exemplification A8-3

[C520]

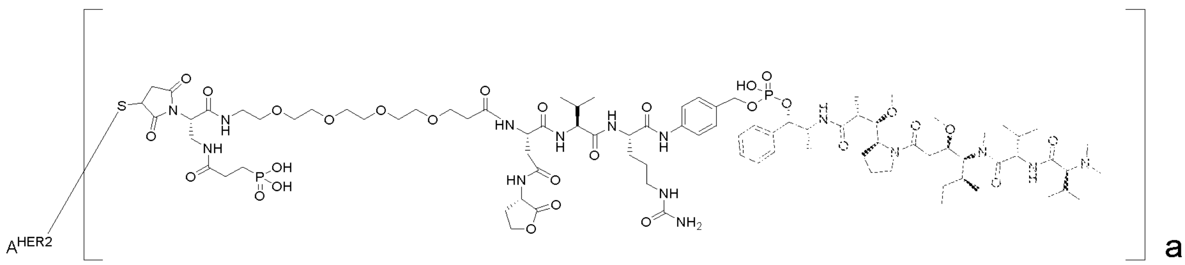

Exemplification A8-3b

[C521]

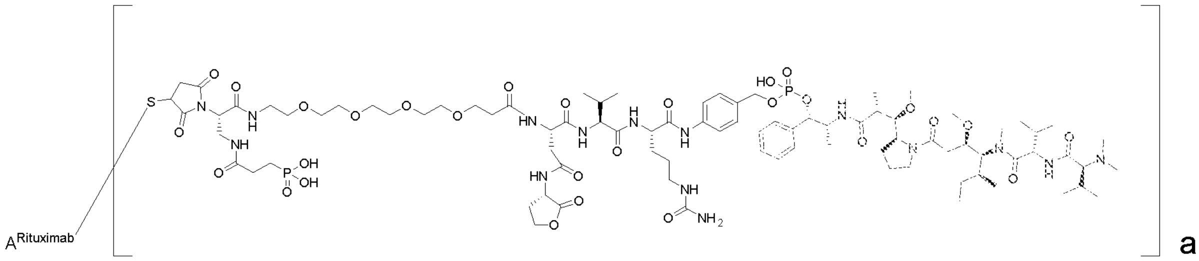

Exemplification A8-3c

[C522]

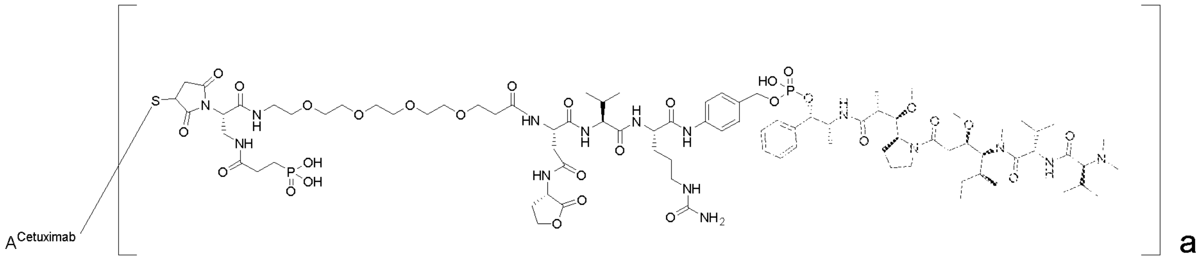

Exemplification A8-4

[C523]

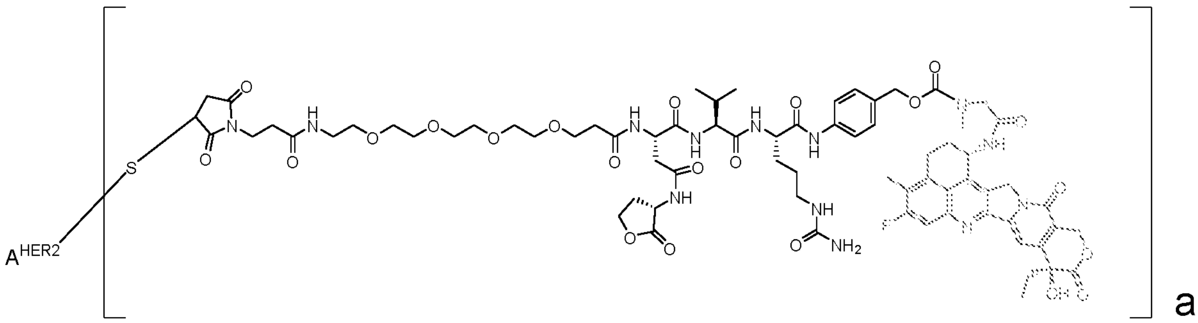

Exemplification A8-5

[C524]

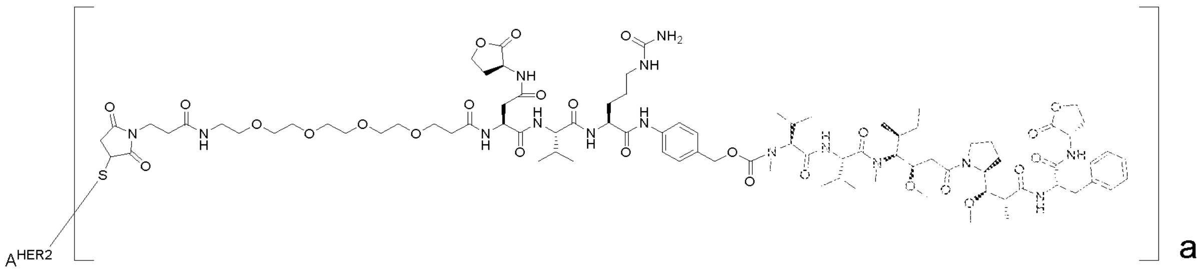

Exemplification A8-6

[C525]

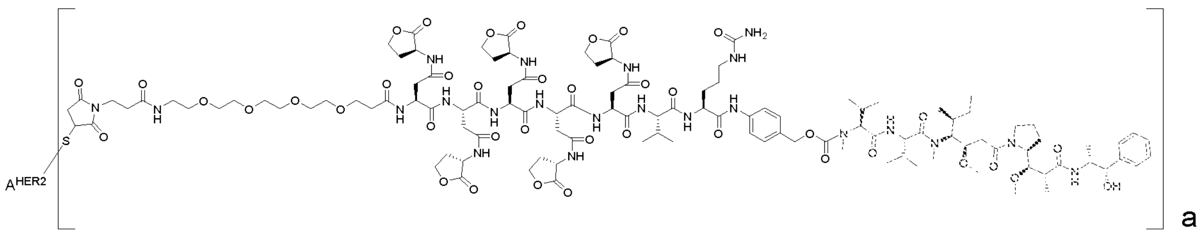

Exemplification A8-7

[C526]

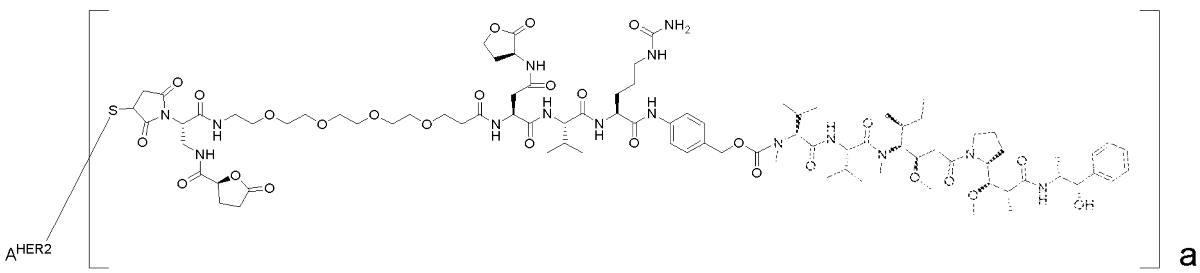

Exemplification A8-8

[C527]

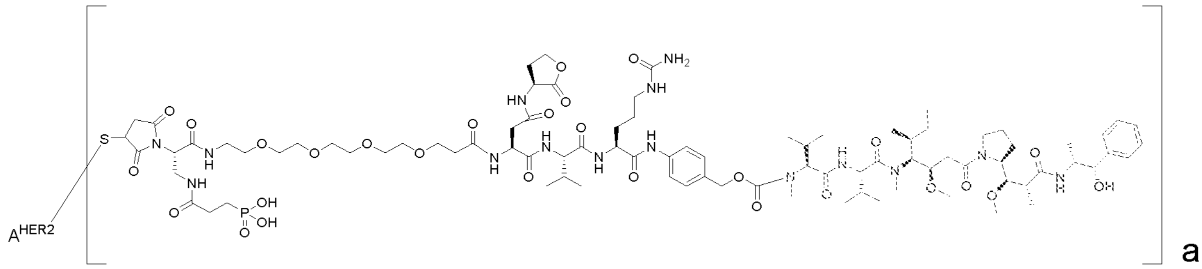

Exemplification A8-9

[C528]

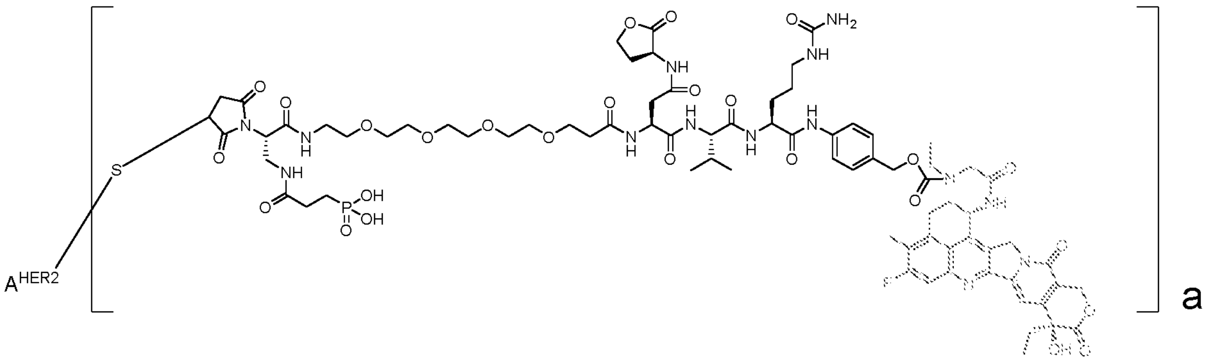

Exemplification A8-10

[C529]

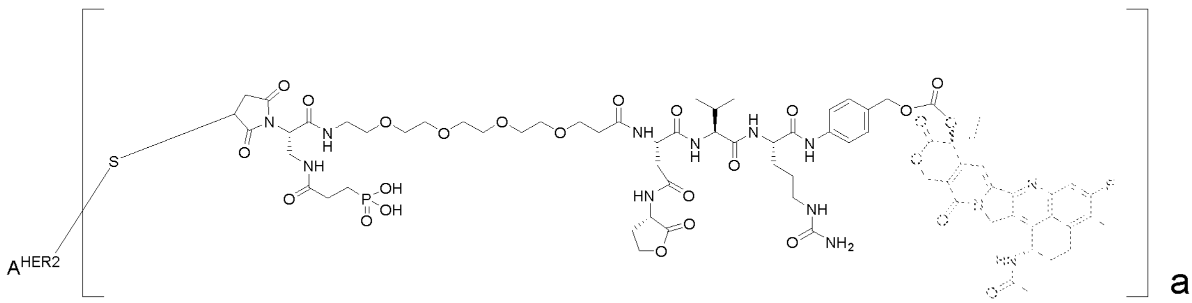

Exemplification A8-11

[C530]

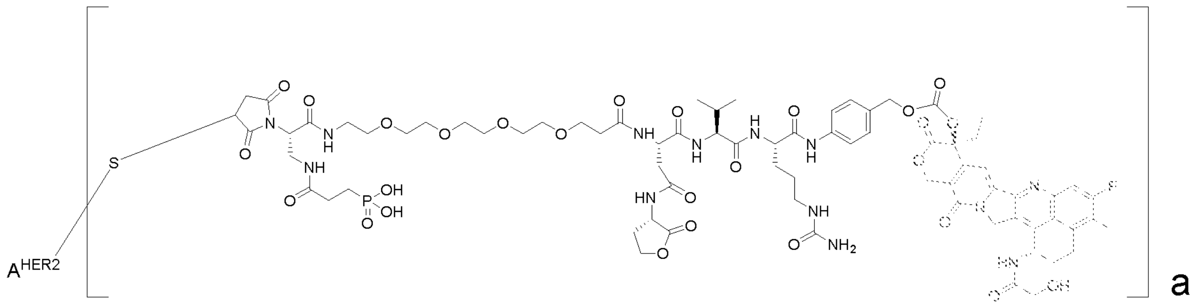

Exemplification A8-12

[C531]

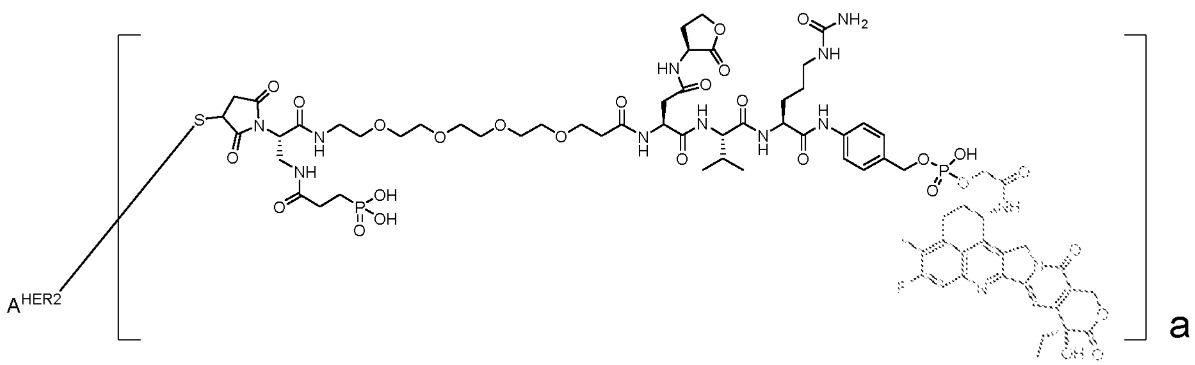

Exemplification A8-13

[C532]

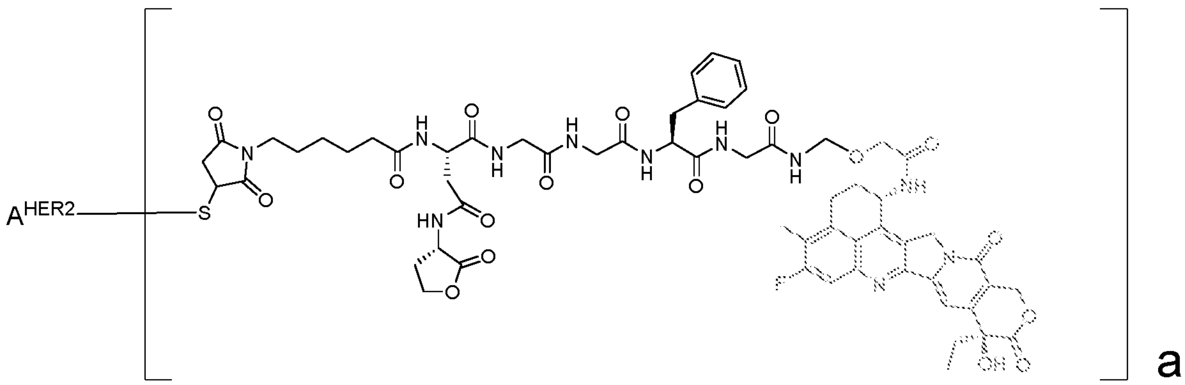

Exemplification A8-14

[C533]

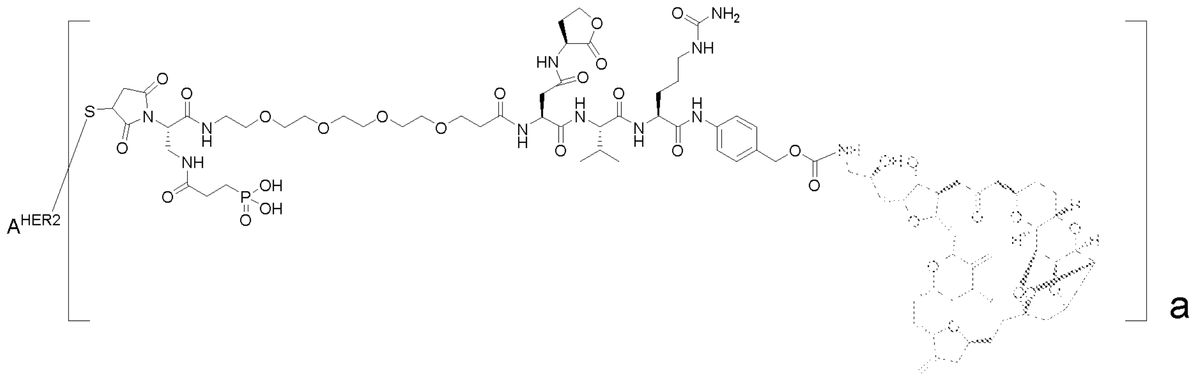

Exemplification A8-15

[C534]

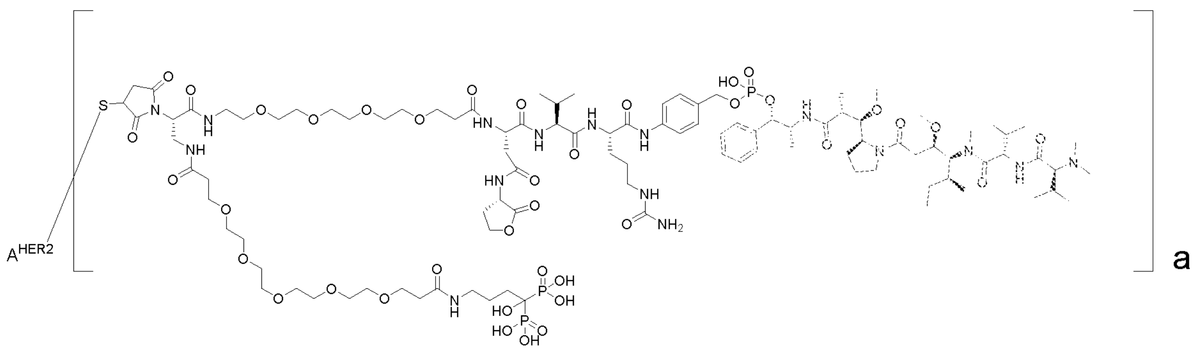

Exemplification A8-16

[C535]

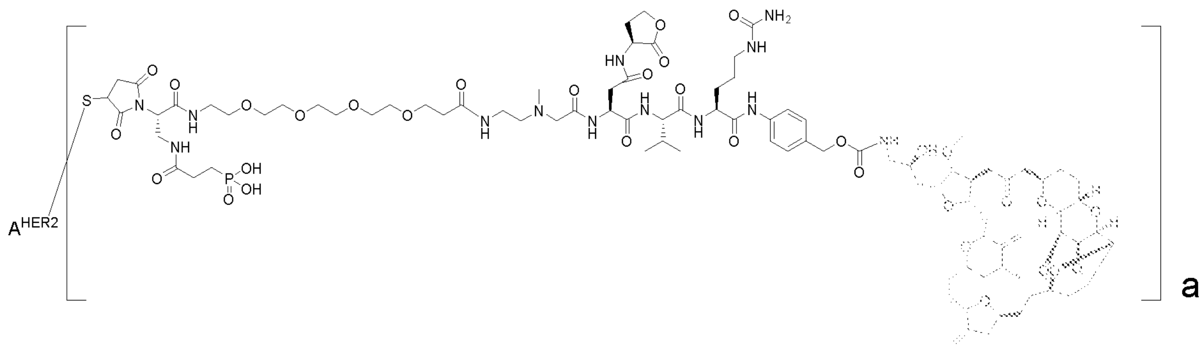

Exemplification A8-17

[C536]

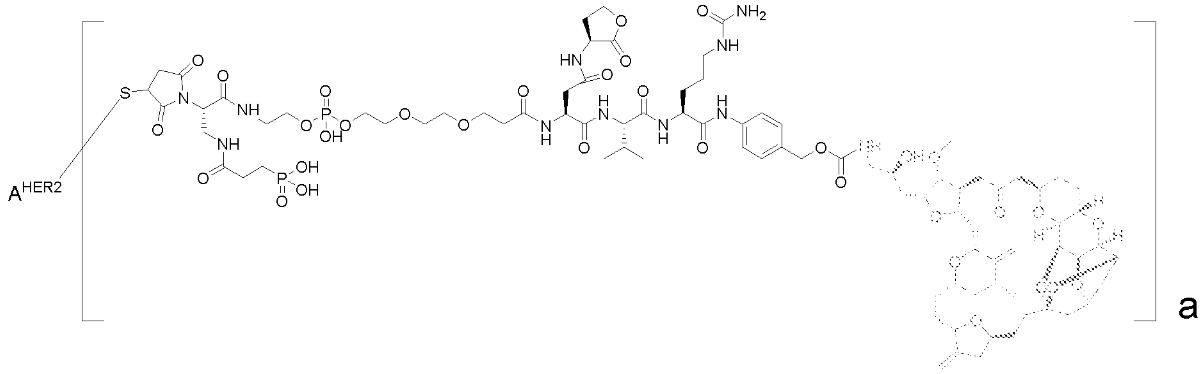

Exemplification A8-18

[C537]

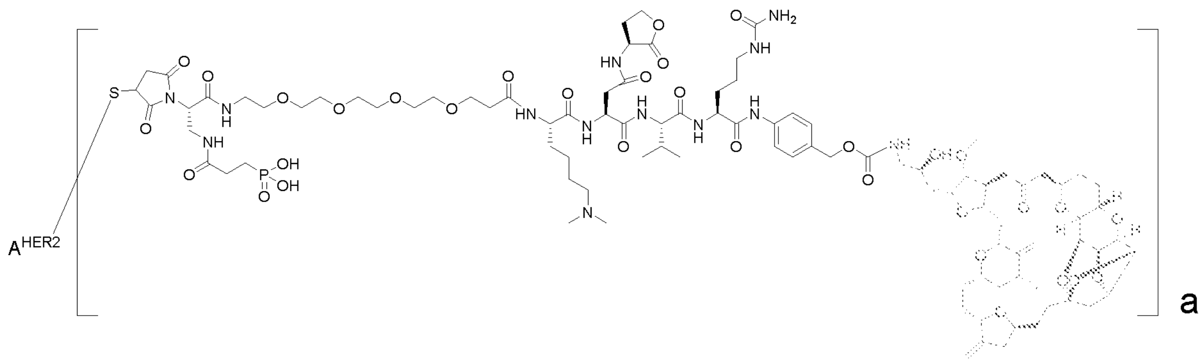

Exemplification A8-19

[C538]

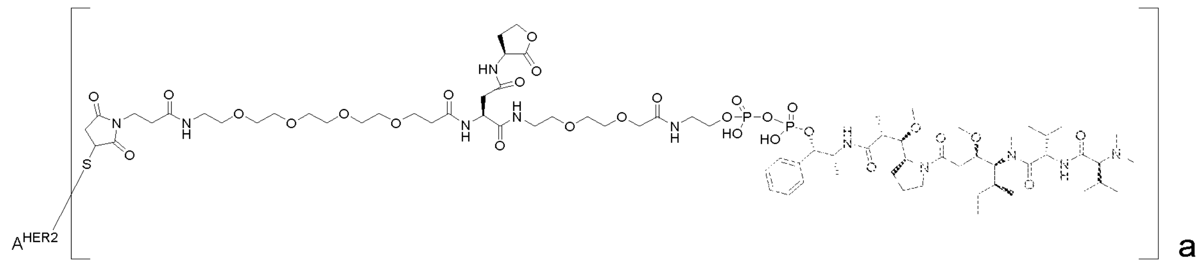

Exemplification A8-20

[C539]

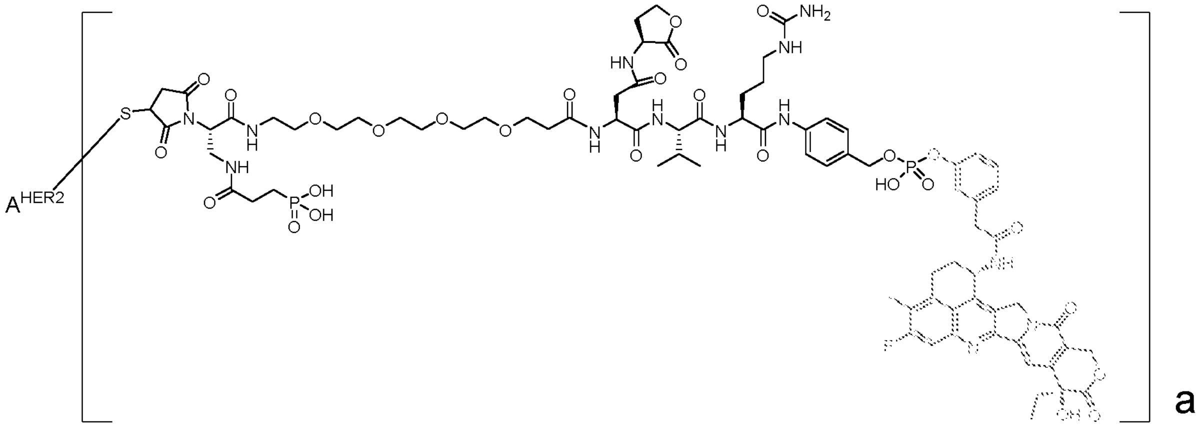

Exemplification A8-21

[C540]

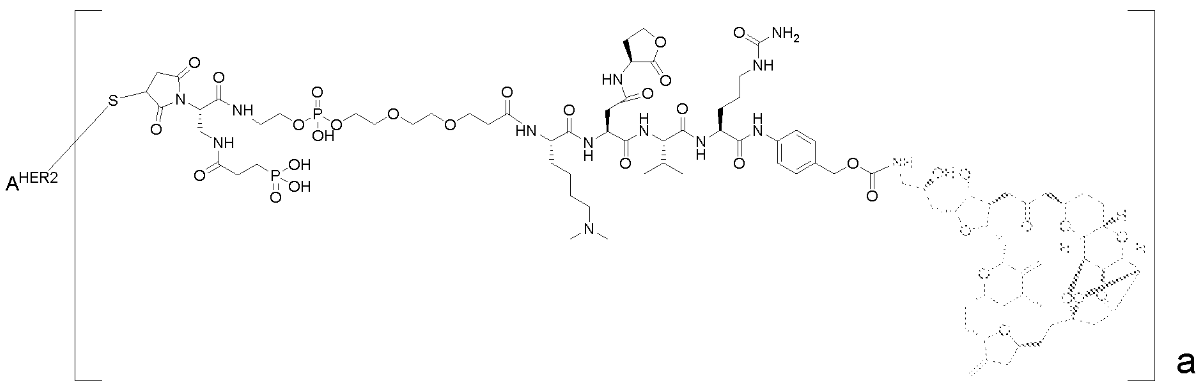

Exemplification A8-22

[C541]

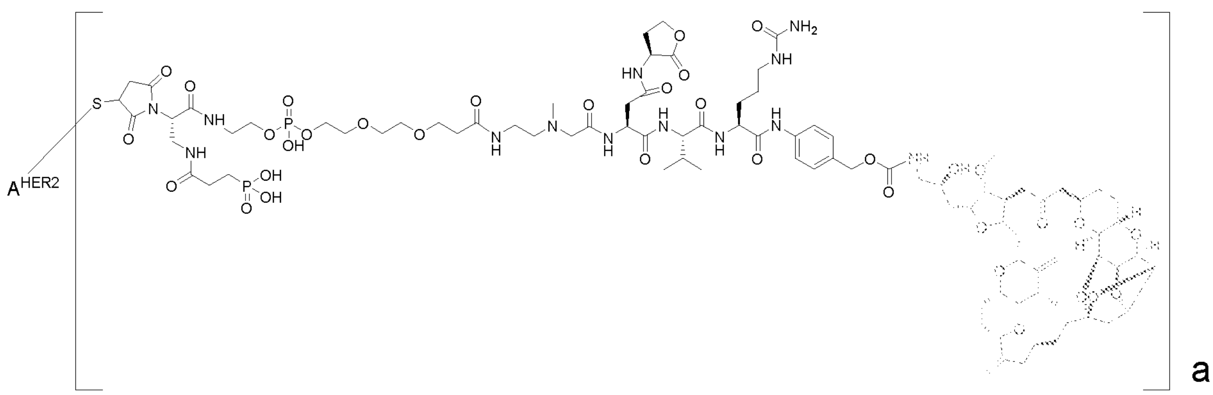

Exemplification A8-23

[C542]

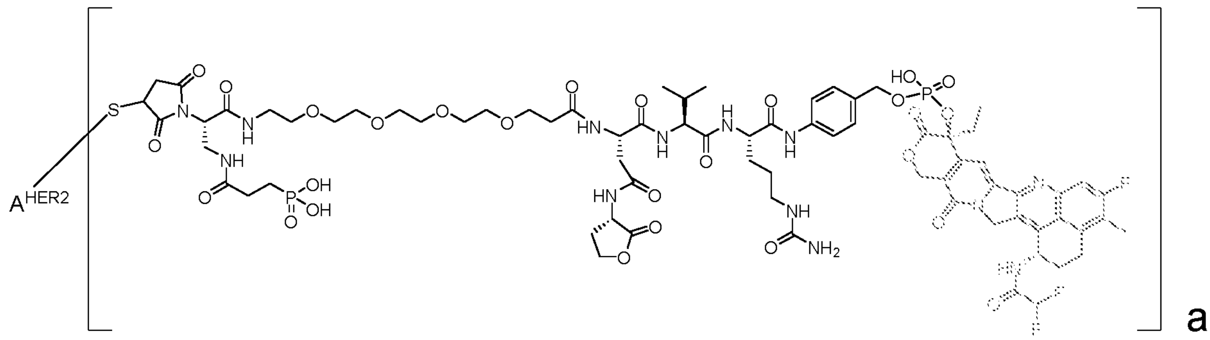

Exemplification A8-24

[C543]

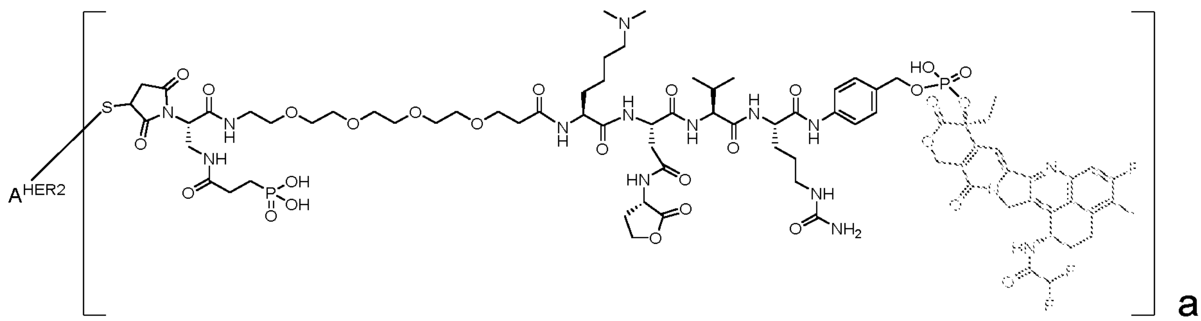

Hereinafter, general methods for producing said AMDCs will be specifically described, but basically, general-purpose, well-known methods in the production of ADCs or chemical modifications of proteins are appropriately selected and applied mutatis mutandis (refer to patents and literature below).

- · Japanese Patent No. 6186045
- · Synthetic Organic Chemistry, Vol. 42, No. 4 (1984)
- · Drug Delivery System, 34-1, 2019
- · Nat. Commun., 2018, 9, 2512
- · YAKUGAKU ZASSHI, 139, No.2 (2019)
- · Angew. Chem. Int. Ed. 2019, 58, 11631-11636 ... Phosphonamidate literature
- · Chem. Rev. 2015, 115, 2174-2195
- · Analytical Sciences, January 2018, Vol. 35, 5-27

1. Production Method-1

An AMDC (General Formula (III-1) in which B in the AMDC (General Formula (III)) produced using the conjugate precursor (I) of the present invention is -S- (thioether bond) is produced according to, for example, a method represented by the following formula.

Method for producing ADC represented by General Formula (III-1)

[C544]

**$A\text{-}(SH)_a$ +** Conjugate **precursor (I-1) → $A\text{-}(S\text{-}Z^2\text{-}L^3\text{-}MDU)_a$ General Formula (II-1)**

[in the formula, A-(SH)a is the above-defined "antibody" having one or more sulfhydryl groups (-SH),

the conjugate precursor (I-1) shows the conjugate precursor (I) in which Z in General Formula (I) above is a maleimidyl group (Formula (v) above), an α-halogenomethylcarbonyl group (Formula (vi) above), or an ethynylphosphonamidate group (Formula (vii) above) (Angew. Chem. Int. Ed. 2019, 58, 11631-11636),
$Z^2$ indicates a divalent residue (a succinimidylene group, a $-CH_2-C(=O)-$ group, or a cis-ethenylphosphonamidate group) produced by a reaction of the above-defined Z with the SH group(s) in the antibody,
the succinimidylene group is the following formula:

[C545]

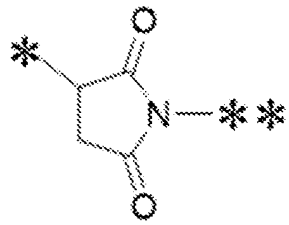

[in the formula, * is a point of attachment to the "antibody-S-" and ** is a point of attachment to "$L^3$"],
the cis-ethenylphosphonamidate group is represented by the following formula:

[C546]

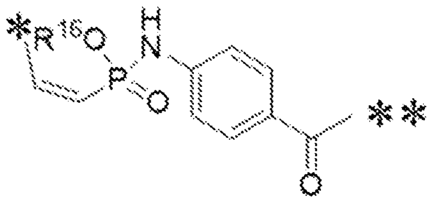

[in the formula, $R^{16}$ is a methyl group, an ethyl group, a $-CH_2CH_2OCH_2CH_2OH$ group, * is a point of attachment to the "antibody-S-" and ** is a point of attachment to "$L^3$"], and
$L^3$, MDU, and a are synonymous with the above.]

The above-described production method can be specifically described as follows.

(1)
[C547]

**$A-(SH)_a$ + Maleimidyl-$L^3$-MDU → $A-(S-Succinimidylene\ -L^3-MDU)_a$ General Formula (III-1-1)**

or
(2)
[C548]

**$A-(SH)_a + Hal-CH_2-C(=O)-L^3-MDU \rightarrow A-(S-CH_2-C(=O)-L^3-MDU)_a$ General Formula (III-1-2)**

or
(3)
[C549]

**$A-(SH)_a$ + Ethenylphosphonamidate $L^3$-MDU → A-(S-cis- Ethenylphosphonamidate $L^3$-**

**MDU)$_a$** **General Formula (III-1-3)**

[in the formula,

A-(SH)a, $L^3$, MDU, a, and Hal are synonymous with the above,
the succinimidylene group is the following formula:

[C550]

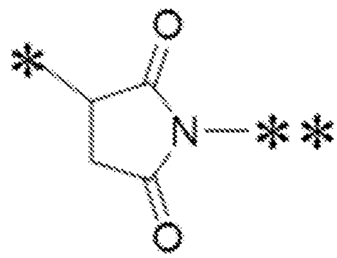

[in the formula, * is a point of attachment to the "antibody-S-" and ** is a point of attachment to "$L^3$"], and
the cis-ethenylphosphonamidate group is represented by the following formula:

[C551]

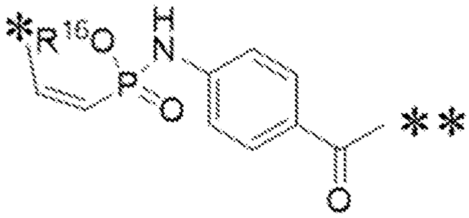

[in the formula, $R^{16}$ is synonymous with the above, * is a point of attachment to the "antibody-S-," and ** is a point of attachment to "$L^3$"].]

In this manner, an AMDC (General Formula (III-1)) can be produced by reacting the antibody A-(SH)a having sulfhydryl group(s) with the conjugate precursor (I) synthesized according to the methods described in the examples.

The antibody A-(SH)a having sulfhydryl group(s) can be obtained through a method well-known to those skilled in the art (Hermanson, G. T., Bioconjugate Techniques, pp. 56-136, 456-493, Academic Press (1996)).

For example, examples thereof include, but are not limited to, allowing a Traut's reagent to act on amino group(s) of an antibody; allowing N-succinimidyl-S-acetylthioalkanoates to act on amino group(s) of an antibody, and then allowing hydroxylamine to act thereon; allowing N-succinimidyl-3-(pyridyldithio)propionate to act, and then allowing a reducing agent to act; and allowing a reducing agent such as dithiothreitol, 2-mercaptoethanol, and tris(2-carboxyethyl)phosphine hydrochloride (TCEP) to act on an antibody to reduce disulfide bond(s) at the hinge moiety(ies) in the antibody and produce sulfhydryl group(s).

Specifically, TCEP as a reducing agent may be used at 0.3 to 3 molar equivalents per disulfide at a hinge moiety in an antibody and reacted with an antibody in a buffer solution containing a chelating agent to obtain an antibody in which disulfide(s) at hinge moiety(ies) in the antibody is/are partially or completely reduced. Examples of the chelating agent include ethylenediaminetetraacetic acid (EDTA) and diethylenetriaminepentaacetic acid (DTPA). The chelating agent may be used at a concentration of 1 mM to 20 mM. Examples of the buffer solution which may be used include a sodium phosphate, sodium borate, or sodium acetate solution, and phosphate buffered saline. In a specific example, an antibody may be reacted with TCEP at 4°C to 37°C for 1 to 4 hours to obtain an antibody A-(SH)a having partially or completely reduced sulfhydryl group(s). In addition, 2 to 20 molar equivalents of the conjugate precursor (I-1) can be used per antibody A-(SH)a having sulfhydryl group(s) to produce an AMDC (Formula (III-1)) in which 1 to 10 linker-drug units ("-$Z^2$-$L^3$-MDU" moiety(ies) in General Formula (III) above) are bound per antibody.

Specifically, a solution in which the conjugate precursor (I-1) is dissolved is added to a buffer solution containing an antibody A-(SH)a having sulfhydryl group(s) to cause a reaction. Here, Examples of the buffer solution which may be

used include a sodium phosphate, sodium borate, or sodium acetate solution, and phosphate buffered saline. The reaction is caused at pH 5 to 9, and more suitably at about pH 7. Examples of the solvent to be used for dissolving the conjugate precursor (I-1) include organic solvents such as dimethyl sulfoxide (DMSO), dimethylformamide (DMF), dimethylacetamide (DMA), and N-methyl-2-pyridone (NMP). An organic solvent solution in which the conjugate precursor (I-1) is dissolved is added to a buffer solution containing the antibody A-(SH)a having sulfhydryl group(s) at 1% v/v to 20% v/v to cause a reaction. The reaction temperature is 0°C to 37°C and more suitably 10°C to 25°C, and the reaction time is 0.5 to 2 hours. The reaction may be terminated by inactivating unreacted reactive group(s) of the conjugate precursor (I-1) using a thiol-containing reagent. Examples of the thiol-containing reagent include cysteine and N-acetyl-L-cysteine (NAC). More specifically, NAC may be added thereto at 1 to 2 molar equivalents relative to the conjugate precursor (I-1) used, and the resulting mixture may be incubated at room temperature for 10 to 30 minutes to terminate the reaction.

2. Production Method-2

An AMDC (General Formula (III-2) in which B in the AMDC (General Formula (III)) produced using the conjugate precursor (I) of the present invention is -NH- (thioether bond) is produced according to, for example, a method represented by the following formula.

Method for producing ADC represented by General Formula (III-2)

[C552]

**$A\text{-}(NH_2)_a$ + Conjugate precursor (I-2) → $A\text{-}(NH\text{-}Z^2\text{-}L^3\text{-}MDU)_a$ General Formula (III-2)**

[in the formula, $A\text{-}(NH_2)a$ is an antibody having one or more amino groups ($-NH_2$), the conjugate precursor (I-2) indicates a conjugate precursor in which Z in General Formula (I) above is a carboxy group or an active ester thereof, for example, an N-hydroxysuccinimidyl ester group, $Z^2$ indicates a divalent residue (-C(=O)- group) produced by a reaction of the above-defined Z with the $NH_2$ group(s) in the antibody, and $L^3$, MDU, and a are synonymous with the above definition.]

The above-described production method can be specifically described as follows.

(1)
[C553]

**$A\text{-}(NH_2)_a$ + $HOOC\text{-}L^3$-MDU → $A\text{-}(NH\text{-}C(=O)\text{-}L^3\text{-}MDU)_a$ General Formula (III-2-1)**

or
(2)
[C554]

**A-(NH2),+ N-Hydroxysuccinimidyl ester -$L^3$-MDU → $A\text{-}(NH\text{-}C(=O)\text{-}L^3\text{-}MDU)_a$ General Formula (III-2-2)**

[in the formula, $A\text{-}(NH_2)a$ is an antibody having one or more amino groups ($-NH_2$), and $L^3$, MDU, and a are synonymous with the above.]

In this manner, an AMDC (General Formula (III-2)) can be produced by reacting the antibody $A\text{-}(NH_2)a$ having amino group(s) ($-NH_2$) with the conjugate precursor (I-2) synthesized according to the methods described in the examples.

Active esters of the conjugate precursor (I-2) that can be used include, in addition to the N-hydroxysuccinimidyl ester) exemplified above, other active esters, for example, sulfosuccinimidyl ester, N-hydroxyphthalimidyl ester, N-hydroxysulfophthalimidyl ester, ortho-nitrophenyl ester, para-nitrophenyl ester, 2,4-dinitrophenyl ester, 3-sulfonyl-4-nitrophenyl ester, 3-carboxy-4-nitrophenyl ester, and pentafluorophenyl ester.

Specifically, 2 to 20 molar equivalents of the conjugate precursor (I-2) can be used per antibody ($A\text{-}(NH_2)a$) having amino group(s) to produce an AMDC (General Formula (III-2)) in which 1 to 10 linker-multidrug units ("-$L^3$-MDU" moiety(ies) in General Formula (III) above) are bound per antibody.

Specifically, the AMDC (General Formula (III-2)) can be produced by reacting a buffer solution containing an antibody ($A\text{-}(NH_2)a$) with a solution in which the conjugate precursor (I-2) is dissolved. Here, Examples of the buffer solution which may be used include a sodium phosphate, sodium borate, or sodium acetate solution, and phosphate buffered saline. The reaction may be caused at pH 5 to 9, and more suitably at about pH 7. Examples of the solvent that can be used for dissolving the conjugate precursor (I-2) include organic solvents such as dimethyl sulfoxide (DMSO),

dimethylformamide (DMF), dimethylacetamide (DMA), and N-methyl-2-pyridone (NMP). An organic solvent solution in which the conjugate precursor (I-2) is dissolved may be added to a buffer solution containing the antibody (A-($NH_2$)a) at 1% v/v to 20% v/v to cause a reaction. The reaction temperature is 0°C to 37°C and more suitably 10°C to 25°C, and the reaction time is 0.5 to 20 hours.

3. Common operations in production of antibody-multidrug conjugate (AMDC)

The antibody-multidrug conjugates produced according to the general production methods described above can be identified by carrying out concentration, buffer exchange, purification, measurement of antibody concentration, and measurement of the average number of "linker-multidrug units (-$Z^2$-$L^3$-MDU)" bound per antibody molecule, through the following common operations.

Common operation A: Concentration of antibody or antibody-multidrug conjugate aqueous solution

The antibody or antibody-multidrug conjugate solution is placed in a container of Amicon Ultra (50,000 MWCO, Millipore Corporation) and concentrated through a centrifugation operation (centrifugation for 5 to 20 minutes at 2000 G to 3800 G) using a centrifuge (Universal Refrigerated Centrifuge MODEL 5922, Kubota Corporation).

Common operation B: Measurement of antibody concentration

The antibody concentration was measured using a plate reader (FlexStation 3, Molecular Devices, LLC) according to the manufacturer's specified method. At this time, 280 nm absorption coefficients differing among antibodies (1.3 mLmg-1cm- to 1.8 mLmg-1cm-1) are used.

Common operation C: Buffer exchange for antibody

A NAP-25 column (Cat. No. 17-0854-02, GE Healthcare Japan Corporaton) using a Sephadex G-10 carrier is equilibrated with a phosphate buffer solution (pH 7.4) (referred to as PBS6.0/EDTA in the present specification) according to the method described in the manufacturer's instruction. 1.0 mL of the antibody aqueous solution is applied thereto per said NAP-10 column, and then a fraction (1.5 mL) eluted with 1.5 mL of PBS is separated. Said fraction is concentrated according to the common operation A, the antibody concentration is measured using the common operation B, and then the antibody concentration is adjusted using PBS.

Common operation D: Purification of antibody-multidrug conjugate

Any one of buffer solutions of a commercially available phosphate buffer solution (PBS 7.4, Cat. No. 10010-023, Invitrogen), a sodium phosphate buffer solution containing sodium chloride (137 mM) (10 mM, pH 6.0; referred to as "PBS 6.0" in the present specification), or an acetate buffer solution (10 mM, pH 5.5; referred to as "ABS" in the present specification) containing Sorbitol (5%) is used to equilibrate a NAP-10 column. An aqueous reaction solution of an antibody-drug conjugate (about 1.0 mL) is applied to said NAP-10 column, and eluted with the buffer solution in an amount specified by the manufacturer to separate an antibody fraction. A gel filtration purification operation, in which said isolated fraction is applied to the NAP-10 column again and eluted with the buffer solution, is repeated 2 to 3 times in total to obtain an antibody-multidrug conjugate in which unbound drug linkers and low molecular weight compounds (tris(2-carboxyethyl)phosphine hydrochloride (TCEP), N-acetyl-L-cysteine (NAC), and dimethyl sulfoxide) are removed.

Common operation E: Calculation of antibody concentration in antibody-drug conjugates (III) and average number of "linker-multidrug units" bound per antibody molecule (DAR) by absorbance

The bound drug concentration in antibody-multidrug conjugate can be calculated by measuring the UV absorbance of an aqueous solution of the antibody-multidrug conjugate at two wavelengths of 280 nm and 370 nm and then carrying out the following calculation.

The total absorbance at a certain wavelength is equal to the sum of the absorbance of all absorptive chemical species present in the system [additivity of absorbance]. Thus, assuming that the molar extinction coefficients of the antibody and drugs do not change before and after conjugating the antibody and drugs, the antibody concentration and drug concentration in the antibody-multidrug conjugate are represented by the following relation equations.

$$A280 = AD,280 + AA,280 = \varepsilon D,280CD + \varepsilon A,280CA \ ... \ Equation\ (1)$$

$$A370 = AD,370 + AA,370 = \varepsilon D,370CD + \varepsilon A,370CA \ ... \ Equation\ (2)$$

Here, A280 represents the absorbance of the antibody-drug conjugate aqueous solution at 280 nm, A370 represents the absorbance of the antibody-drug conjugate aqueous solution at 370 nm, AA,280 represents the absorbance of the antibody at 280 nm, AA,370 represents the absorbance of the antibody at 370 nm, AD,280 represents the absorbance of a conjugate precursor (the compound of General Formula (I) above; the same applies hereinafter) at 280 nm, AD,370 represents the absorbance of the conjugate precursor at 370 nm, εA,280 represents the molar extinction coefficient of the antibody at 280 nm, εA,370 represents the molar extinction coefficient of the antibody at 370 nm, εD,280 represents the molar extinction coefficient of the conjugate precursor at 280 nm, εD,370 represents the molar extinction coefficient of the conjugate precursor at 370 nm, CA represents the antibody concentration in the antibody-multidrug conjugate, and CD represents the drug concentration in the antibody-multidrug conjugate.

Here, values prepared in advance (calculated estimated values or measured value obtained from UV measurement of the compound) are used as εA,280, εA,370, εD,280, and εD,370. For example, εA,280 can be estimated from the amino acid sequence of the antibody according to a known calculation method (Protein Science, 1995, Vol. 4, 2411-2423). εA,370 is usually zero. εD,280 and εD,370 can be obtained using Lambert-Beer law (absorbance = molar concentration × molar extinction coefficient × cell optical path length) by measuring the absorbance of a solution in which a conjugate precursor to be used is dissolved at a certain molar concentration. CA and CD can be determined by measuring A280 and A370 of the antibody-drug conjugate aqueous solution and substituting these values into Equations (1) and (2) to solve the simultaneous equations. Furthermore, CD may be divided by CA to determine the average number of "linker-multidrug units" bound per antibody.

Common operation F: Calculation of DAR in antibody-multidrug conjugate by cysteine reactivity evaluation

The DAR in the antibody-multidrug conjugate (general formula; III-1) in which "-B-" in General Formula (III) is "-S-" can be calculated and estimated by evaluating the reactivity of the conjugate precursor (I-1)) with L-cysteine. In other words, if the conjugate precursor (I-1) is reacted with L-cysteine under the same conditions as those of the conjugating reaction and L-cysteine is completely consumed, the DAR is estimated to be 8. A specific method will be described below.

100 μL of a 1.51 mM conjugate precursor (I-1)) dimethyl sulfoxide solution to be used in conjugation was added to 900 μL of a 134 μM L-cysteine PBS/EDTA solution. Then, the resulting mixture is reacted on ice for 1 hour (under this condition, the amount of the conjugate precursor is 10 equivalents relative to 8 equivalents of L-cysteine). 480 μL of the resulting solution after the reaction is added to a microtube, and 20 μL of a 10 mM ethanol solution of 5, 5'-dithiobis(2-nitrobenzoic acid) (manufactured by FUJIFILM Wako Pure Chemical Corporation, 047-16401) is added thereto. The resulting mixture is reacted at room temperature for 15 minutes. The solution after the reaction is added to a 96-well plate, and a microplate reader (FlexStation 3, manufactured by Molecular Device, LLC) is used to measure the absorbance (412 nm). The same procedures are carried out using an L-cysteine solution having a known concentration, and a calibration curve is prepared to calculate the L-cysteine concentration in a sample.

An L-cysteine residual rate (%) in a sample is calculated by the following equation.

$$\text{L-cysteine residual rate (\%) in sample} = (a/121) \times 100$$

a: L-cysteine concentration (μM) in sample

The DAR of an antibody-multidrug conjugate (III-1) obtained using the conjugate precursor (I-1) in the same lot as that subjected to the above-described cysteine reactivity evaluation is calculated and estimated by the following equation.

$$DAR = 8 \times ((100-b)/100)$$

b: L-cysteine residual rate (%) in sample

In the antibody-multidrug conjugate (III) obtained from the conjugate precursor of the present invention, since two drugs are bound to each "linker-multidrug unit," the numerical value calculated by the above-described method of multiplying the "average number of "linker-multidrug units" bound per antibody" by "2" becomes the average total number of drugs bound to one antibody. Therefore, if the "average number of "linker-multidrug units" bound per antibody" of the antibody-multidrug conjugate obtained from the "conjugate precursor (III-1) to which two drugs are bound" is calculated to be "7," the average total number of drugs bound to one antibody is "$2 \times 7 = 14$."

In addition, in order to ensure an adequate amount of conjugate, conjugates having the same level of average total number of drugs (for example, ±1 level) prepared and obtained under the same conditions may be mixed with each other to prepare a new lot. In that case, the resulting average total number of drugs falls within the range of the average total number of drugs before mixing.

The antibody-multidrug conjugate or a salt thereof produced using the conjugate precursor (I) of the present invention may be allowed to stand in atmospheric air or recrystallized to absorb moisture, have adsorbed water attached thereto, or become a hydrate, and such an antibody-multidrug conjugate or a salt thereof containing water is also encompassed by the present invention.

4. Method of using antibody-multidrug conjugate (AMDC)

The antibody-multidrug conjugate (III) produced using the conjugate precursor (I) of the present invention is useful for inhibiting growth of tumor cells or cancer cells, inducing cell death in tumor cells or cancer cells, or treating cancer in patients. Said antibody-multidrug conjugate can be used in various conditions for the treatment of cancer. Said antibody-multidrug conjugate can be used for delivering drugs to tumor cells or cancer cells.

In one embodiment, an antibody in said antibody-multidrug conjugate binds to or associates with a cancer cell-associated antigen or a tumor cell-associated antigen, and the antibody-multidrug conjugate can be incorporated (internalized) into tumor cells or cancer cells through receptor-mediated endocytosis or other internalization mechanisms. An antigen can bind to a tumor cell or a cancer cell or can be an extracellular matrix protein associated with a tumor cell or a cancer cell. Once inside a cell, the drug is released into the cell via a cleavage mechanism. In addition, the drug may also be cleaved from the antibody-multidrug conjugate outside the tumor cell or cancer cell, followed by penetration of the drug or drug unit into the cell.

In one embodiment, an antibody binds to a tumor cell or a cancer cell.

In another embodiment, an antibody binds to a tumor cell or cancer cell antigen on the surface of the tumor cell or cancer cell.

In one embodiment, an antibody binds to a tumor cell or cancer cell antigen, which is an extracellular matrix protein associated with a tumor cell or a cancer cell.

The specificity of an antibody to a particular tumor cell or cancer cell can be important in determining which tumor or cancer is most effectively treated. For example, said antibody-drug conjugate targeting a cancer cell antigen present in hematopoietic cancer may be useful for treating hematologic malignancies (for example, anti-CD30, anti-CD70, anti-CD19, and anti-CD33 binding antibodies may be useful for treating hematologic malignancies). Said antibody-drug conjugate targeting a cancer cell antigen present on a solid tumor may be useful for treating such a solid tumor.

Cancer that can be treated using the antibody-drug conjugate (III) obtained from the conjugate precursor (I) of the present invention is not limited, but examples thereof include lymphomas (Hodgkin's lymphoma and non-Hodgkin's lymphoma), hematopoietic cancer such as leukemia, and solid tumors. Examples of hematopoietic cancer include follicular lymphoma, anaplastic large-cell lymphoma, mantle-cell lymphoma, acute myeloblastic leukemia, chronic myeloid leukemia, chronic lymphocytic leukemia, diffuse large B-cell lymphoma, and multiple myeloma. Examples of the solid tumors include fibrosarcoma, myxosarcoma, liposarcoma, chondrosarcoma, osteosarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, gastric cancer, oral cancer, nasal cavity cancer, throat cancer, flat epithelial cancer, basal cell cancer, adenocarcinoma, sweat gland cancer, sebaceous carcinoma, papillary carcinoma, papillary adenocarcinoma, cystadenocarcinoma, medullary cancer, bronchogenic cancer, renal cell cancer, liver cancer, biliary tract cancer, choriocarcinoma, seminoma, embryonal cancer, Wilms' tumor, cervical cancer, uterine cancer, testicular tumor, small cell lung cancer, bladder cancer, lung cancer, epithelial cancer, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

5. Production examples of antibody-multidrug conjugates (AMDCs)

Antibody-multidrug conjugates (AMDCs) were produced using the conjugate precursor (I) of the present invention through the following methods. Antibody-multidrug conjugates acquired in each of the following production examples are represented as General Formula (III').

General Formula (III')

[C555]

$$A\text{-}(B\text{-}Z^2\text{-}L^3\text{-}MDU)_{a'} \qquad \text{General Formula (III')}$$

(In the formula, A, B, $Z^2$, $L^3$, and MDU are synonymous with the above, and a' is an average number of linker-multidrug units ($-Z^2-L^3$-MDU) bound per antibody molecule in each antibody-multidrug conjugate acquired in each of the following production examples (hereinafter this number is referred to as "DAR" with respect to AMDCs), and a number from 1 to 10, which may include decimal fractions, and is displayed up to the first decimal place in the present specification.)

AMDC Production Example 1. Production of AMDC represented by Exemplification 8-1

1-1. Preparation of anti-HER2 antibody (trastuzumab) by purifying Herceptin

Trastuzumab was prepared by purifying Herceptin (manufactured by Chugai Pharmaceutical Co., Ltd.). EDTA (manufactured by Thermo Fisher Scientific, AM9260G) was added to phosphate buffered saline (hereinafter referred to as "PBS") (pH 7.2, manufactured by Thermo Fisher Scientific, 20012-043) so that the final concentration would become 10 mM (hereinafter referred to as "PBS/EDTA"). NAP-10 columns (manufactured by GE Healthcare, 17085402) were equilibrated with PBS/EDTA according to the manufacturer's specified method. A solution of Herceptin in PBS/EDTA (1 mL) was applied to the NAP-10 column, and then fraction (1.5 mL) eluted with PBS/EDTA (1.5 mL) was collected. The absorbance at 280 nm of this fraction was measured by using a microplate reader (FlexStation 3, manufactured by Molecular Device) to measure the antibody concentration (1.47 $mLmg^{-1}cm^{-1}$ was used as the extinction coefficient at 280 nm).

1-2. Reduction of trastuzumab:

The concentration of the trastuzumab solution obtained in the above 1-1. was adjusted to 1 to 10 mg/mL using PBS/EDTA. This solution (0.99 mL) was put into a 1.5 mL microtube, and a solution of TCEP hydrochloride (209-19861, manufactured by FUJIFILM Wako Pure Chemical Corporation) in PBS/EDTA (10 μL, 6.9 equivalents relative to one antibody molecule) was added thereto. The resulting mixture was incubated at 37°C for 2 hours to reduce the disulfide bond at the hinge moiety in the antibody.

1-3. Conjugate formation of reduced trastuzumab and conjugate precursor (I):

To the reduced trastuzumab solution (900 μL) was added a dimethyl sulfoxide solution (manufactured by FUJIFILM Wako Pure Chemical Corporation, 043-07216) containing the compound obtained in the Example 1 (Exemplification No. 2-1) (100 μL, 10 equivalent relative to one antibody molecule), and the resulting mixture was reacted on ice for 1 hour. Next, a solution of L-Cysteine (manufactured by FUJIFILM Wako Pure Chemical Corporation, 039-20652) in PBS/EDTA (10 equivalent relative to one antibody molecule) was added thereto, and the reaction was further carried out for 15 minutes to stop the reaction of conjugate formation.

1-4. Purification of antibody multidrug conjugate:

NAP-10 columns were equilibrated with PBS (pH 7.4, Thermo Fisher Scientific, 10010-023) according to the manufacturer's specified method. To this NAP-10 column was applied an aqueous reaction solution of the antibody multidrug conjugate obtained in the above 1-3 (1 mL), and the fraction (1.5 mL) eluted with PBS (1.5 mL, pH 7.4) was collected. This collected fraction was applied to the NAP-10 column again, and the similarly eluted fraction was collected.

1-5. Measurement of antibody concentration in antibody multi-drug conjugate and average number of bound linker multidrug units ($-Z^2-L^3$-MDU) per one antibody molecule:

The DAR of the antibody-multidrug conjugate obtained in the above 1-4 was measured and calculated according to "Common operation E" among the above common operations described in relation to the production method. As a result, the DAR of the antibody-multidrug conjugate of Example 1 was 7.5.

AMDC Production Examples 2 to 7

The same reaction and treatment operation as in AMDC Production Example 1 were performed using various conjugate precursors (I) to obtain antibody-multidrug conjugates of AMDC Production Examples 2 to 7. The results are summarized in the following Table 2 together with those of AMDC Production Example 1.

[Table 2]

Table 2

| AMDC production example | Structure of AMDC | DAR | |
|---|---|---|---|
| | | Common operation | a' |
| 1 | Exemplification 8-1 | E | 7.5 |
| 2 | Exemplification 8-2 | F | 7.1 |
| 3 | Exemplification 8-3 | F | 8.0 |
| 4 | Exemplification 8-4 | E | 6.7 |
| 5 | Exemplification 8-5 | E | 7.3 |
| 6 | Exemplification 8-6 | | |
| 7 | Exemplification 8-7 | F | 7.9 |

Antibody-drug conjugates (ADC Production Examples 1 to 24) were synthesized by the following methods. Antibody-drug conjugates acquired in the following ADC Production Examples 1 to 24 are represented as General Formula (I'-1). General Formula (I'-1):
[C556]

$$A\text{-}(B\text{-}L\text{-}D)_{a'} \qquad (I'\text{-}1)$$

(In the formula, A, B, L, and D are synonymous with the above, and a' is an average number of linker-drug (L-D) units bound per antibody molecule (DAR) in the antibody-drug conjugates acquired in examples, and a number of 1 to 10, which may include decimal fractions, and is displayed up to the first decimal place in the present specification.) In ADC Production Examples 1 to 24, Examples A1 to A24 were used as the linker-drug units (that is, -L-D).

ADC Production Example 1. Synthesis of ADC Production Example 1 (Structure of Exemplification A8-1) 1-1. Preparation of anti-HER2 antibody (trastuzumab) by purifying Herceptin

Trastuzumab was prepared by purifying Herceptin (manufactured by Chugai Pharmaceutical Co., Ltd.). EDTA (AM9260G, manufactured by Thermo Fisher Scientific) was added to a phosphate buffer solution (hereinafter referred to as PBS) (pH 7.2, 20012-043, manufactured by Thermo Fisher Scientific) so that the concentration became 10 mM (hereinafter referred to as PBS/EDTA). A NAP-10 column (17085402, manufactured by GE Healthcare) was equilibrated with PBS/EDTA according to the method in the manufacturer's instructions. A solution of Herceptin in PBS/EDTA (1 mL) was loaded onto each NAP-10 column, and fractions (1.5 mL) eluted with PBS/EDTA (1.5 mL) were then collected. The absorbance of the fractions at 280 nm was measured using a microplate reader (FlexStation3, Manufactured by Molecular Device), and thus the antibody concentration was measured (1.47 $mLmg^{-1}cm^{-1}$ was used as an extinction coefficient at 280 nm).

1-2. Reduction of trastuzumab:

The concentration of the trastuzumab solution obtained in the above 1-1 was adjusted to 1 to 10 mg/mL using PBS/EDTA. This solution (0.99 mL) was put into a 1.5 mL microtube, and a solution of TCEP hydrochloride (209-19861, manufactured by FUJIFILM Wako Pure Chemical Corporation) in PBS/EDTA (10 μL, 6.9 equivalents relative to one antibody molecule) was then added thereto. The resulting mixture was incubated at 37°C for 2 hours to reduce the disulfide bond at the hinge moiety in the antibody.

1-3. conjugate formation of reduced trastuzumab and reactive precursor (Example A1):

To the reduced trastuzumab solution (900 μL), a solution of dimethyl sulfoxide (043-07216, manufactured by FUJIFILM Wako Pure Chemical Corporation) containing the compound obtained in Example A1 (100 μL, 10 equivalents or 20 equivalents relative to one antibody molecule) was added, and the mixture was reacted on ice for 1 hour. Next, a solution of L-Cysteine (039-20652, manufactured by FUJIFILM Wako Pure Chemical Corporation) in PBS/EDTA (10 μL, 10 equivalents relative to one antibody molecule) was added, the mixture was additionally reacted for 15 minutes, and the reaction of the conjugate formation was stopped.

1-4. Purification of antibody-drug conjugate:

A NAP-10 column was equilibrated with PBS (pH 7.4, 10010-023, manufactured by Thermo Fisher Scientific) according to the method in the manufacturer's instructions. A reaction aqueous solution (1 mL) of the antibody-drug conjugate obtained in the above 1-3 was loaded onto this NAP-10 column, and fractions (1.5 mL) eluted with PBS (1.5 mL, pH 7.4) were collected. The preparative fractions were loaded onto the NAP-10 column again, and eluted fractions were collected in the same manner.

1-5. Calculation of average number of linker-drug units bound per antibody molecule (DAR) and antibody concentration in antibody-drug conjugate:

The DAR of the antibody-drug conjugate obtained in the above 1-4 was measured according to "Conjugate treatment step E" in the above general conjugate treatment steps described in connection with the production method or estimated according to "Conjugate treatment step F". As a result, the DAR of the antibody-drug conjugate of the present Example A1 was 8.0.

Regarding Examples A3b and 3c, antibody-drug conjugate was produced according to the same method as Example A1, except for an anti-CD20 antibody (Rituximab) purified from rituximab BS (Kyowa Kirin Co., Ltd.) or an anti-EGFR antibody (Cetuximab) purified from Erbitux (Merck BioPharma Co., Ltd.) was used instead of the anti-HER2 antibody used in the Preparation A1-1 of Example A1.

The present ADC Production examples and Comparative compounds were produced by using each reactive precursor (Examples A1 to A24), MC-Val-Cit-PAB-MMAE (MedChemExpress, CAS no. 646502-53-6), and Mal-PEG2-Val-Cit-PAB-Eribulin (Chem Scene, CAS no. 2130869-18-8) and performing the same reactions and treatment operations as in ADC Production Example 1 (regarding Production Example 11, Example A11, which is a reactive precursor, was used in 20 equivalents relative to the antibody). Results of the DAR of the Production Examples and Comparative compounds are summarized in the following Table 1 together with Production Example 1.

The Comparative compound-1 is an antibody-drug conjugate obtained using MC-Val-Cit-PAB-MMAE as a reactive precursor, and the Comparative compound-2 is an antibody-drug conjugate obtained using Mal-PEG2-VAl-Cit-PAB-Eribulin as a reactive precursor.

[Table 3]

Table 3

| Example number (Reactive precursor) | ADC production examples (Structure thereof) | A | B | DAR | |
|---|---|---|---|---|---|
| | | | | Common operation | a' |
| A1 | ADC Production Example 1 (Exemplification A8-1) | Anti-HER2 antibody residue | - S- | F | 8.0 |

(continued)

| Example number (Reactive precursor) | ADC production examples (Structure thereof) | A | B | DAR | |
|---|---|---|---|---|---|
| | | | | Common operation | a' |
| A2 | ADC Production Example 2 (Exemplification A8-2) | Anti-HER2 antibody residue | - S- | F | 7.9 |
| A3 | ADC Production Example 3 (Exemplification A8-3) | Anti-HER2 antibody residue | - S- | F | 8.0 |
| A3 | ADC Production Example 3b (Exemplification A8-3b) | Anti-CD20 antibody residue | - S- | F | 8.0 |
| A3 | ADC Production Example 3c (Exemplification A8-3c) | Anti-EGFR antibody residue | - S- | F | 8.0 |
| A4 | ADC Production Example 4 (Exemplification A8-4) | Anti-HER2 antibody residue | - S- | E | 7.0 |
| A5 | ADC Production Example 5 (Exemplification A8-5) | Anti-HER2 antibody residue | - S- | F | 8.0 |
| A6 | ADC Production Example 6 (Exemplification A8-6) | Anti-HER2 antibody residue | - S- | F | 8.0 |
| A7 | ADC Production Example 7 (Exemplification A8-7) | Anti-HER2 antibody residue | - S- | F | 8.0 |
| A8 | ADC Production Example 8 (Exemplification A8-8) | Anti-HER2 antibody residue | - S- | F | 7.1 |
| A9 | ADC Production Example 9 (Exemplification A8-9) | Anti-HER2 antibody residue | - S- | E | 6.7 |
| A10 | ADC Production Example 10 (Exemplification A8-10) | Anti-HER2 antibody residue | - S- | E | 7.1 |
| A11 | ADC Production Example 11 (Exemplification A8-11) | Anti-HER2 antibody residue | S- | E | 8.0 |
| A12 | ADC Production Example 12 (Exemplification A8-12) | Anti-HER2 antibody residue | - S- | E | 7.0 |
| A13 | ADC Production Example 13 (Exemplification A8-13) | Anti-HER2 antibody residue | S- | E | 6.8 |
| A14 | ADC Production Example 14 (Exemplification A8-14) | Anti-HER2 antibody residue | - S- | F | 7.9 |
| A15 | ADC Production Example 15 (Exemplification A8-15) | Anti-HER2 antibody residue | - S- | F | 7.3 |
| A16 | ADC Production Example 16 (Exemplification A8-16) | Anti-HER2 antibody residue | - S- | F | 7.3 |
| A17 | ADC Production Example 17 (Exemplification A8-17) | Anti-HER2 antibody residue | S- | F | 8.0 |
| A18 | ADC Production Example 18 (Exemplification A8-18) | Anti-HER2 antibody residue | - S- | F | 6.6 |
| A19 | ADC Production Example 19 (Exemplification A8-19) | Anti-HER2 antibody residue | - S- | F | 6.7 |
| A20 | ADC Production Example 20 (Exemplification A8-20) | Anti-HER2 antibody residue | S- | E | 4.8 |
| A21 | ADC Production Example 21 (Exemplification A8-21) | Anti-HER2 antibody residue | - S- | F | 7.9 |

(continued)

| Example number (Reactive precursor) | ADC production examples (Structure thereof) | A | B | DAR | |
|---|---|---|---|---|---|
| | | | | Common operation | a’ |
| A22 | ADC Production Example 22 (Exemplification A8-22) | Anti-HER2 antibody residue | - S- | F | 8.0 |
| A23 | ADC Production Example 23 (Exemplification A8-23) | Anti-HER2 antibody residue | S- | E | 8.8 |
| A24 | ADC Production Example 24 (Exemplification A8-24) | Anti-HER2 antibody residue | - S- | E | 9.3 |
| Comparative Compound-1 | Comparative ADC Production Example-1 | Anti-HER2 antibody residue | S- | F | 8.1 |
| Comparative Compound-2 | Comparative ADC Production Example-2 | Anti-HER2 antibody residue | S- | F | 8.0 |

Test Examples

[Test Example 1: Tubulin polymerization inhibition test]

For measuring the tubulin polymerization inhibitory action, Tubulin Polymerization Assay Kit (manufactured by Cytoskeleton, Inc., BK011P) was used. A PIPES buffer containing porcine tubulin, GTP, $MgCl_2$, EGTA, Glycerol, and a fluorescent reporter was prepared under ice-cooling as a reaction solution. The reaction solution was ice-cooled until it was used. A test compound solution was prepared by an aqueous solution containing 1.5% DMSO, and added to a 96 well plate (manufactured by Corning Inc., 3686) at 5 μL/well. The reaction solution was added thereto at 50 μL/well, the plate was placed in a microplate reader (FlexStation 3, manufactured by Molecular Device, LLC) in which the internal temperature was set to 37°C in advance, and the fluorescence intensity measurement (excitation wavelength: 360 nm, detection wavelength: 420 nm) of each well was started. The fluorescence intensity was measured once every minute, and the measurement was continued for 60 minutes.

The tubulin polymerization inhibition rate (%) was calculated by the following equation.

$$\text{Tubulin polymerization inhibition rate (\%)} = ((a-b)/a)\times 100$$

a: average amount of fluorescence intensity increase in vehicle-treated well (n = 2)
b: average amount of fluorescence intensity increase in test compound-treated well (n = 2)

Amount of fluorescence intensity increase: (average fluorescence intensity after 51 to 60 minutes from measurement start) - (average fluorescence intensity after 1 to 6 minutes from measurement start)

EXSUS (manufactured by CAC Croit Corporation) was used to represent the relationship between the tubulin polymerization inhibition rate and the test compound concentration (sigmoid curve regression), and the test compound concentration in which the tubulin polymerization inhibition rate became 50% ($IC_{50}$ value) was calculated.

In this test, "antitumor drug molecules or analogs thereof, or derivatives thereof (exemplified as U-compounds in the present description)" disclosed in the present description showed excellent tubulin polymerization inhibition activities. For example, each $IC_{50}$ value of U-008 (Production example 8), U-009 (Production example 9), U-010 (Production example 10), U-011 (Production example 11), U-012 (Production example 12), U-013 (Production example 13), U-014 (Production example 14), U-015 (Production example 15), U-016 (Production example 16), U-017 (Production example 17), U-018 (Production example 18), U-019 (Production example 19), U-020 (Production example 20), U-021 (Production example 21), U-030 (Production example 30), and U-031 (Production example 31) was 3 μM or less, and almost the same level as MMAE tested under the same conditions.

[Test Example 2: Topoisomerase I activity inhibition test]

For measuring topoisomerase I activity inhibitory actions, Human DNA Topoisomerase I Assay Kit (manufactured by ProFoldin, HRA100K) was used. A human topoisomerase I (manufactured by Sigma, T9069) was added to a Tris-HCl buffer containing KCl, DTT, $MgCl_2$, EDTA, and BSA to prepare a reaction solution. The reaction solution (35.2 μL) and

a test compound solution (4.4 μL) prepared by an aqueous solution containing 10% DMSO were mixed in a 1.5 mL microtube, and the resulting mixture was reacted at 37°C for 10 minutes. Subsequently, a supercoiled plasmid DNA (4.4 μL) was added thereto, and the resulting mixture was reacted at 37°C for 60 minutes. After the reaction, the solution was added to a 96 well plate (manufactured by Corning Inc., 3904) at 40 μL/well, and a Dye H19 solution (250 μL) diluted 10 times with the buffer included with the kit was added thereto. A microplate reader (Infinite F500, manufactured by TECAN) was used to measure the fluorescence intensity (excitation wavelength: 485 nm, detection wavelength: 535 nm) of each well.

The topoisomerase I activity inhibition rate (%) was calculated by the following equation.

Topoisomerase I activity inhibition rate (%) = ((a-b)/(c-b))×100

a: fluorescence intensity in test compound-treated well
b: fluorescence intensity in vehicle-treated well
c: fluorescence intensity in topoisomerase I-free well

EXSUS (manufactured by CAC Croit Corporation) was used to represent the relationship between the topoisomerase I activity inhibition rate and the test compound concentration (sigmoid curve regression), and the test compound concentration in which the topoisomerase I activity inhibition rate became 50% ($IC_{50}$ value) was calculated.

In this test, "antitumor drug molecules or analogs thereof, or derivatives thereof (exemplified as U-compounds in the present description)" disclosed in the present description showed excellent topoisomerase I inhibitory activities. For example, each $IC_{50}$ value of U-001 (Production example 1), U-002 (Production example 2), U-003 (Production example 3), U-004 (Production example 4), U-005 (Production example 5), U-006 (Production example 6), and U-007 (Production example 7) was 15 μM or less, and almost the same level as exatecan tested under the same conditions.

[Test Example 3: Cell growth inhibition test] 3-1. Growth inhibition test of HER2-positive human gastric cancer cell line (NCI-N87)

NCI-N87 is generally used as a HER2-overexpressing cell line (Clin Cancer Res; 22(20) October 15,2016). NCI-N87 (ATCC, CRL-5822) was cultured in RPMI 1640 Medium (ATCC Modification) (manufactured by Thermo Fisher Scientific, A10491-01) containing 10% fetal bovine serum (manufactured by Corning Inc., 35-011-CV) and 1% penicillin/streptomycin (manufactured by Thermo Fisher Scientific, 15140-122). The cells were seeded to a 96 well plate (manufactured by Corning Inc., 353377) at $1.0\times10^3$ cells / 100 μL / well. The cells were cultured under 5% $CO_2$ at 37°C overnight, then the medium was exchanged with new one (95 μL/well), and the cells were treated with a test compound or an anti-HER2 antibody (trastuzumab), which was adjusted to 2 μg/mL with PBS (manufactured by Thermo Fisher Scientific, 10010-023), at 5 μL/well (treatment concentration: 100 ng/mL). The cells were cultured under 5% $CO_2$ at 37°C for 6 days, then CellTiter-Glo (manufactured by Promega, G9241) was added thereto at 100 μL/well, and a microplate reader (FlexStation 3, manufactured by Molecular Device, LLC) was used to measure the luminescence amount of each well.

The growth inhibition rate (%) was calculated by the following equation.

Growth inhibition rate (%) = ((a-b)/(a-c))×100

a: average luminescence amount in vehicle-treated well (n = 3)
b: average luminescence amount in test compound-treated well (n = 3)
c: average luminescence amount before test compound treatment (n = 3)

As shown in Table 4, in this test, the AMDC of the present invention in Production Examples 1, 2, 3, 4, 5 and 7 exhibited better cell growth inhibitory activity than anti-HER2 antibodies.

[Table 4]

Table 4

| AMDC Production Example No. | Inhibition rate (%) by AMDC | Inhibition rate (%) by anti-HER2 antibody |
|---|---|---|
| 1 | 95 | 25 |
| 2 | 102 | 14 |
| 3 | 97 | 14 |
| 4 | 100 | 22 |
| 5 | 101 | 25 |
| 6 | | |
| 7 | 107 | 23 |

In addition, in this test, compounds of ADC production examples exhibited cell growth inhibitory activity, and among them, the compounds of ADC Production Examples 1, 2, 3, 5, 6, 7, 8, 14, 15, 16, 17, 18, 19, 21, and 22 exhibited a cell growth inhibitory activity which was extremely superior to that of anti-HER2 antibodies. In this test, the cell growth inhibitory activity of anti-HER2 antibodies was 14 to 35%.

3-2. Growth inhibition test of HER2-negative human breast cancer cell line (MDA-MB-231)

MDA-MB-231 is generally used as a triple-negative breast cancer cell line, and known to not express or weakly express HER2 (Breast Cancer (Auckl). 2010 May 20; 4: 35-41). MDA-MB-231 (ECACC, 92020424) was cultured in Leibovitz's L-15 Medium (ATCC Modification) (manufactured by Thermo Fisher Scientific, 11415-064) containing 10% fetal bovine serum (manufactured by Corning Inc., 35-011-CV) and 1% penicillin/streptomycin (manufactured by Thermo Fisher Scientific, 15140-122). The cells were seeded to a 96 well plate (manufactured by Corning Inc., 353377) at $1.0\times10^3$ cells / 100 $\mu$L / well. The cells were cultured at 37°C overnight, then the medium was exchanged with new one (95 $\mu$L/well), and the cells were treated with a test compound, which was adjusted to 2 $\mu$g/mL with PBS (manufactured by Thermo Fisher Scientific, 10010-023), at 5 $\mu$L/well (treatment concentration: 100 ng/mL). The cells were cultured at 37°C for 6 days, then CellTiter-Glo (manufactured by Promega, G9241) was added thereto at 100 $\mu$L/well, and a microplate reader (FlexStation 3, manufactured by Molecular Device, LLC) was used to measure the luminescence amount of each well.

The growth inhibition rate (%) was calculated in the same manner as the Test Example 3-1.

In this test, the growth inhibition rates of the AMDC of production Examples 1, 2, 3, 4, 5 and 7 were 10% or less, and they exhibited almost no cell growth inhibitory activity.

In the above Test Example 5 (3-1 and 3-2), the AMDC of Production Examples 1, 2, 3, 4, 5 and 7 exhibited almost no cell growth inhibitory action on HER2-negative cells, but exhibited a clearly stronger cell growth inhibitory action on HER2-positive cells than anti-HER2 antibody. This result suggests that the AMDC of Production Examples 1, 2, 3, 4, 5 and 7 were specifically bound to HER2-positive cells, and then incorporated into the cells, the antitumor drugs bound to the conjugates were released in the cells, and the drugs contributed to cell growth inhibitory actions.

[Test Example 4: Tumor growth suppression test in nude mouse with subcutaneously implanted HER2-overexpessing human gastric cancer cell line (NCI-N87)]

A human gastric cancer cell line (NCI-N87) (manufactured by ATCC, Code No. CRL-5822) was cultured in RPMI 1640 medium (manufactured by Thermo Fisher Scientific, A10491-01) containing 10% FBS (manufactured by CORNING Inc., 35-011-CV) and 1% penicillin/streptomycin (manufactured by Thermo Fisher Scientific, 15140-122). A mixed solvent of PBS (manufactured by Thermo Fisher Scientific, 10010-023) and Matrigel (manufactured by CORNING Inc., 356231) (final volume 1:1) was used to prepare a cell suspension of $5.0\times10^7$ cells / mL. The prepared cell suspension was subcutaneously injected to the right flank region of BALB/c-nu/nu mice (female, provided by Charles River Laboratories Japan, Inc.) at 0.1 mL per mouse. After the mice were bred for a prescribed time period, the long diameter (mm) and the short diameter (mm) of the tumor was measured by an electronic caliper (manufactured by Mitutoyo Corporation), and the tumor volume was calculated by the following equation.

Tumor volume (mm$^3$) = (long diameter)$\times$(short diameter)$\times$(short diameter)$\times$0.5

Individuals having a tumor volume within a range of 50 to 500 mm$^3$ were selected, divided into groups having almost the same level of tumor volume, and then a test compound or the solvent alone was intravenously administered to them. The first day of the administration start was set as Day 0, and the tumor volume was measured at Day 21. The tumor

volume increase from Day 0 in the solvent-administered control group was set as 100%, and the tumor volume inhibition rate (%) in each administered dose of a test compound was calculated.

In this test, at 1 mg/kg administration, AMDC Production Example 2 showed a tumor volume inhibition rate of 100% or more as shown in Fig. 1, and AMDC Production Examples 3, 4, 5 and 6 showed a tumor volume inhibition rate of 50% or more as shown in Figs. 2, 3, 4 and 5. In addition, in this test, at 3 mg/kg administration, AMDC Production Examples 4 and 5 showed a tumor volume inhibition rate of 100% or more as shown in Figs. 6 and 7. Based on these results, it was confirmed that the antibody-multidrug conjugates of the present invention had a clear tumor suppression or tumor regression effect.

In addition, in this test, the compounds of ADC Production Examples 5, 10, 11, 14, 16, and 17 showed a tumor volume inhibition rate of 50% or more at a dose of 3 mg/kg, the compounds of ADC Production Examples 1, 2, 3, 4, 6, 7, 8 and 18 showed a tumor volume inhibition rate of 100% or more at a dose of 3 mg/kg, and the compounds of ADC production Examples 2, 3, 15, and 19 showed a tumor volume inhibition rate of 100% or more at a dose of 1 mg/kg. Based on these results, it was confirmed that the antibody-drug conjugates of the present invention had a clear tumor suppression or tumor regression effect.

[Test Example 5: Metabolism test using human liver lysosomal fraction]

To a reaction composition solution (X10 Catabolic Buffer (manufactured by SEKISUI-Xenotech, LLC., K5200)) (60 μL) in which human liver lysosome (manufactured by SEKISUI-Xenotech, LLC., Cat No. H610.L 2.5 mg/mL) (corresponding to 0.125 mg of protein) was suspended and distilled water (manufactured by FUJIFILM Wako Pure Chemical Corporation, for HPLC) (10 μL) was added 1.2 μM test compound (AMDC produced in AMDC Production Examples) (25 μL) dissolved in PBS to prepare a reaction solution. After the reaction solution was incubated at 37°C for 24 hours, "an antitumor drug molecule or an analog thereof, or a derivative thereof" released from the AMDC into the reaction solution was quantified by LC-MS/MS (high performance liquid chromatography / mass spectrometer system) under the following conditions.

LC-MS/MS analysis conditions
LC: LC20 or LC30 HPLC system (Shimadzu Corporation)
Column: Phenomenex Kinetex C18 (50x2.1 mm, 2.6 μm)
Column temperature: 40°C
Flow rate: 0.4 mL/min
Mobile phase A: 0.1% formic acid aqueous solution, Mobile phase B: 0.1% formic acid, 50% acetonitrile/methanol mixture Gradient: 0 to 1 min; A/B=90/10-+10/90, 1 to 3 min; A/B=10/90, 3 to 5 min; A/B=90/10
MS: 3200QTRAP (SCIEX)
Ionization: ESI
Mode: Positive or Negative

The release rate (%) of drug was calculated by the following equation.

[Math. 1]

$$\text{Free ratio (\%)} = \frac{\text{Quantitative value (nM) of antitumor drug molecule}}{\text{Concentration (nM) of test compound in reaction solution (nM)} \times \text{DAR}} \times 100$$

When multiple molecules of the same antitumor drug were loaded, since it was not possible to determine the position at which the released drug was loaded, the calculated release rate (%) was divided by the number of molecules loaded and expressed as a numerical value.

As shown in the following Table 5, it was confirmed that multiple "antitumor drug molecules or analogs thereof, or derivatives thereof" bound to the AMDCs obtained in AMDC Production Examples 1, 2, 3, 4, 5 and 7 were released. Here, the release rate (%) of each drug is the numerical value shown in parentheses after the name of each drug, where drug 1 indicates an antitumor drug molecule corresponding to $D^1$ or an analog thereof, or a derivative thereof, and drug 2 indicates an antitumor drug molecule corresponding to $D^2$ or an analog thereof, or a derivative thereof.

[Table 5]

Table 5

| AMDC Production Example No. | Release rate (%) | |
|---|---|---|
| | Drug 1 | Drug 2 |
| 1 | U-030 (36) | U-001 (29) |
| 2 | U-031 (73) | |
| 3 | U-030 (80) | |
| 4 | U-031 (81) | U-032 (92) |
| 5 | U-031 (77) | U-032 (85) |
| 6 | | |
| 7 | U-031 (112) | U-033 (106) |

[Table 6]

Table 6

| ADC Production Example No. | Drug | Release rate (%) |
|---|---|---|
| 1 | MMAE | 68 |
| 2 | U-030 | 96 |
| 3 | U-031 | 81 |
| 4 | U-003 | 76 |
| 5 | U-029 | 7.3 |
| 6 | MMAE | 78 |
| 7 | MMAE | 86 |
| 8 | MMAE | 60 |
| 9 | U-036 | 98 |
| 10 | U-001 | 61 |
| 11 | DXd | 64 |
| 12 | U-035 | 3.5 |
| 13 | DXd | 76 |
| 14 | Eribulin | 61 |
| 15 | U-031 | 84 |
| 16 | Eribulin | 72 |
| 17 | Eribulin | 56 |
| 18 | Eribulin | 75 |
| 19 | U-031 | 1.1 |
| 20 | U-034 | 54 |

[Test Example 6: Metabolism test using human liver Cathepsin B]

To human liver Cathepsin B (manufactured by Sigma-Aldrich Co. LLC, Cat No. C8571 25 μg/vial) (corresponding to 400 Unit/mL) (5 μL), X10 Catabolic Buffer (manufactured by SEKISUI-Xenotech, LLC., K5200) (10 μL), 2.5 mM Bovine Serum Albumin (manufactured by FUJIFILM Wako Pure Chemical Corporation, Cat No. 012-15093) (5 μL), and distilled water (manufactured by FUJIFILM Wako Pure Chemical Corporation, for HPLC) (55 μL) was added 1.2 μM test compound (AMDC produced in AMDC Production Examples) (25 μL) dissolved in PBS to prepare a reaction solution. After the

reaction solution was incubated at 37°C for 24 hours, "an antitumor drug molecule or an analog thereof, or a derivative thereof" released from the AMDS into the reaction solution was quantified by LC-MS/MS (high performance liquid chromatography / mass spectrometer system) under the following conditions.

LC-MS/MS analysis conditions
LC: LC20 or LC30 HPLC system (Shimadzu Corporation)
Column: Phenomenex Kinetex C18 (50x2.1 mm, 2.6 μm)
Column temperature: 40°C
Flow rate: 0.4 mL/min
Mobile phase A: 0.1% formic acid aqueous solution, Mobile phase B: 0.1% formic acid, 50% acetonitrile/methanol mixture Gradient: 0 to 1 min; A/B=90/10-+10/90, 1 to 3 min; A/B=10/90, 3 to 5 min; A/B=90/10
MS: 3200QTRAP (SCIEX)
Ionization: ESI
Mode: Positive or Negative

The release rate (%) of drug was calculated by the following equation.

[Math. 2]

$$\text{Free ratio (\%)} = \frac{\text{Quantitative value (nM) of antitumor drug molecule}}{\text{Concentration (nM) of test compound in reaction solution} \times \text{DAR}} \times 100$$

When multiple molecules of the same antitumor drug molecule were loaded, since it was not possible to determine the position at which the released drug was loaded, the calculated release rate (%) was divided by the number of molecules loaded and expressed as a numerical value.

As shown in the following Table 7, it was confirmed that multiple "antitumor drug molecules or analogs thereof, or derivatives thereof" bound to the AMDC obtained in AMDC Production Examples 1, 2, 3, 4, 5 and 7 were released. Here, the release rate (%) of the drug is the numerical value shown in parentheses after the name of the drug, where drug 1 is an antitumor drug molecule corresponding to $D^1$ or an analog thereof, or a derivative thereof, and drug 2 indicates an antitumor drug molecule corresponding to $D^2$ or an analog thereof, or a derivative thereof.

[Table 7]

Table 7

| Example No. | Release rate (%) | |
|---|---|---|
| | Drug 1 | Drug 2 |
| 1 | U-030 (44) | U-001(30) |
| 2 | U-031 (91) | |
| 3 | U-030 (71) | |
| 4 | U-031 (75) | U-032 (83) |
| 5 | U-031 (85) | U-032 (107) |
| 6 | | |
| 7 | U-031 (139) | U-033 (121) |

[Table 8]

Table 8

| Example No. | Drug | Release rate (%) |
|---|---|---|
| 1 | MMAE | 71 |
| 2 | U-030 | 96 |
| 3 | U-031 | 97 |
| 4 | U-003 | 97 |
| 5 | U-029 | 8.3 |
| 6 | MMAE | 67 |
| 7 | MMAE | 65 |
| 8 | MMAE | 41 |
| 9 | U-036 | 40 |
| 10 | U-001 | 28 |
| 11 | DXd | 30 |
| 12 | U-035 | 106 |
| 13 | DXd | 24 |
| 14 | Eribulin | 50 |
| 15 | U-031 | 102 |
| 16 | Eribulin | 62 |
| 17 | Eribulin | 58 |
| 18 | Eribulin | 64 |
| 19 | U-031 | 0.87 |
| 20 | U-034 | 11 |

[Test Example 7: Pharmacokinetic test]

Trastuzumab or a test compound was intravenously administered to a female mouse (BALB/c, Charles River Laboratories Japan, Inc.). After a prescribed time period, the blood was collected from the jugular vein or abdominal vein, and centrifuged to prepare a serum. The resulting serum was cryopreserved until the measurement.

The serum trastuzumab concentration was measured using SHIKARI Q-TRAS (manufactured by MATRIKS BIOTEK) according to the manufacturer's specified method.

The serum trastuzumab residual rate after 3 days from administration (%) was calculated by the following equation.

Serum trastuzumab residual rate after 3 days (%) = $(a/b)\times 100$

a: serum trastuzumab concentration after 3 days from administration (ng/mL)
b: serum trastuzumab concentration after 5 minutes from administration (ng/mL)

The residual rates of the antibody-multidrug conjugates obtained in AMDC Production Examples 2, 4 and 5 were 30% or more, which was equal to or higher than that of trastuzumab.

The residual rates of the antibody-drug conjugates obtained in ADC Production Examples 1 to 24 are shown in Table 4, and all were equal to or higher than that of trastuzumab. On the other hand, the residual rate of the antibody-drug conjugate (Comparative Control Compound-1) obtained from MC-Val-Cit-PAB-MMAE (manufactured by MedChemExpress, CAS Number 646502-53-6) was 5.4 to 13%.

[Table 9]

Table 9

| ADC production Example No. | Serum trastuzumab residual rate (%) 3 days after administration |
|---|---|
| 1 | 35 |
| 2 | 27 |
| 3 | 32 |
| 4 | 31 |
| 5 | 37 |
| 6 | 57 |
| 7 | 34 |
| 8 | 24 |
| 9 | 20 |
| 10 | 23 |
| 11 | 28 |
| 12 | 18 |
| 13 | 25 |
| 14 | 59 |
| 15 | 28 |
| 16 | 64 |
| 17 | 69 |
| 18 | 73 |
| 19 | 28 |
| 20 | 25 |
| 21 | 46 |
| 22 | 47 |
| 23 | 21 |
| 24 | 31 |
| Trastuzumab | 21 to 33 |
| Comparative ADC Production Example-1 | 5.4 to 13 |

[Test Example 8: Size Exclusion Chromatography analysis]

As an index of stability of AMDC Production Example compounds, Size Exclusion Chromatography analysis (hereinafter referred to as "SEC analysis") was carried out.
SEC analysis conditions:

Device: HITACHI LC-2000
Column: Agilent AdvanceBio SEC 300Å, 7.8x300 mm, 2.7 μm
Eluent: phosphate buffered saline (PBS, manufactured by WAKO)
Temperature: room temperature
Flow rate: 0.8 mL/min
Detection wavelength: 220 nm
Injection volume: 5 μL

Analysis time: 25 min

Fig. 8 shows an SEC analysis HPLC chart of AMDC Production Example 2.

The antibody-drug conjugate of AMDC Production Example 2 was observed to have a sharp main peak, and there was a trace amount of peaks thought to be aggregates, suggesting that it was stable. For example, the same applies to AMDC Production Examples 3, 4, 6, and 7.

[Industrial Applicability]

The antibody-multidrug conjugate produced using the conjugate precursor (I) or a salt thereof of the present invention has been confirmed to be promising as cancer therapeutic agents having excellent antitumor effects and safety because it is selectively delivered to a target tumor cell after administered to a living body, and antitumor drugs released into said cell exert each function. Therefore, the conjugate precursor (I) or a salt thereof of the present invention is useful for producing excellent antibody-multidrug conjugates, and the conjugate precursor synthesis intermediates (II-1) and (II-2) or a salt thereof of the present invention are useful as synthetic intermediates of said conjugate precursor (I).

## Claims

**1.** An antibody-multidrug conjugate precursor represented by General Formula (I) below:

[C1]

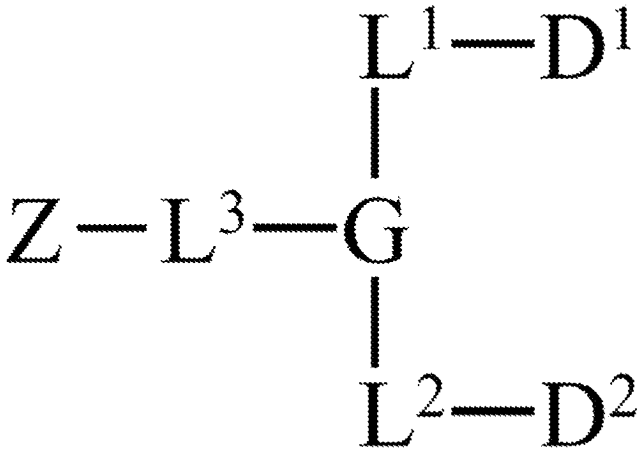

(I)

[in the formula,

Z is a reactive group capable of reacting with a functional group present in an antibody (a functional group in an antibody),
G is a group represented by Formula (i), (ii), (iii), or (iv) below,

[C2]

(i) ; 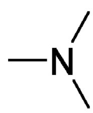

[C3]

(ii) ; 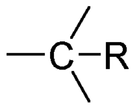

[C4]

(iii) ; 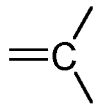

[C5]

(iv) ; 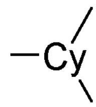

[in the formulae, R is a hydrogen atom, a hydroxyl group, an amino group, an alkyl group, or an alkyloxy group, and Cy is a cycloalkyl ring, a cycloalkenyl ring, an aryl ring, a heteroaryl ring, or a heterocyclyl ring],
$L^1$ is a linker linking G to $D^1$,
$L^2$ is a linker linking G to $D^2$,
$L^3$ is a linker linking G to Z,
$L^1$, $L^2$, and $L^3$ may be identical to or different from each other,
$D^1$ and $D^2$ are antitumor drug residues in which one hydrogen atom or one hydroxyl group is removed from any position of an antitumor drug molecule or an analog thereof, or a derivative thereof, and which may be identical to or different from each other,
one or more lactonyl groups and one or more phosphoryls are independently present as substituents or protecting groups at any position of the antitumor drug residues or $L^1$, $L^2$, or $L^3$, a total number of said lactonyl groups is 1 to 10, the lactonyl groups may be identical to or different from each other when said number of the lactonyl groups is two or more, and a total number of said phosphoryl groups is 1 to 10], or a salt thereof.

**2.** The antibody-multidrug conjugate precursor according to claim 1, or a salt thereof, wherein
Z is a maleimidyl group (Formula (v) below), an α-halogenomethylcarbonyl group (Formula (vi) below), an ethynylphosphonamidate group (Formula (vii) below), a carboxy group, an active ester of a carboxy group, a sulfhydryl group, a hydroxyl group, an amino group, an alkynyl group, a cycloalkynyl group, or an azide group ($-N_3$ group),

Formula (v)

[C6]

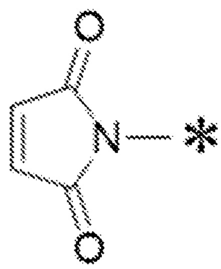

Formula (vi)

[C7] $Hal-CH_2-C(=O)-*$

Formula (vii)

[C8]

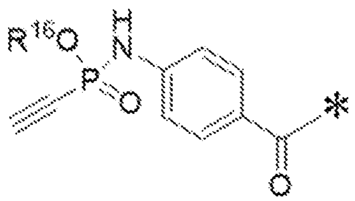

[in the formulae, * is a point of attachment to $L^3$, Hal is a fluorine atom, a chlorine atom, a bromine atom, or an iodine atom, and $R^{16}$ is a methyl group, an ethyl group, or a - $CH_2CH_2OCH_2CH_2OH$ group].

**3.** The antibody-multidrug conjugate precursor according to claim 1 or 2, or a salt thereof, wherein Z is a maleimidyl group (the Formula (v)), an α-halogenomethylcarbonyl group (the Formula (vi)), an ethynylphosphonamidate group (the Formula (vii)), a carboxy group, or an active ester of a carboxy group.

**4.** The antibody-multidrug conjugate precursor according to any one of claims 1 to 3, or a salt thereof, wherein Z is a maleimidyl group (the Formula (v)), an α-halogenomethylcarbonyl group (the Formula (vi)), a carboxy group, or an active ester of a carboxy group.

**5.** The antibody-multidrug conjugate precursor according to any one of claims 1 to 4, or a salt thereof, wherein G is a group represented by Formula (i), Formula (ii), or Formula (iii).

**6.** The antibody-multidrug conjugate precursor according to any one of claims 1 to 5, or a salt thereof, wherein G is a group represented by Formula (i).

**7.** The antibody-multidrug conjugate precursor according to any one of claims 1 to 6, or a salt thereof, wherein

$L^1$, $L^2$, and $L^3$ are each independently an optionally substituted alkylene group;
one or more methylene groups in the chain of the alkylene group are optionally replaced with one or more divalent groups independently selected from the group consisting of - $C(R^1)(R^2)$-; -O-; -$N(R^3)$-; -$N(R^3)$-N ($R^3$)-; -S-; -Se-; -$Si(R^4)(R^5)$-; -S-S-; -Se-Se-; -SOm-; -SeOn-; -C(=C($R^6$)($R^7$))-; -C(=O)-; - C (=S) -; -C(=N($R^8$)) -; -C(=N-O$R^9$)-; -C (=N-N($R^{10}$)($R^{11}$))-; - P(=O)($R^{12}$)-; -P(=O)(O$R^{13}$)-; -O-P(=O)($R^{12}$)-O-; -O-P(=O)(O$R^{13}$)-O-; -C($R^{14}$)=; =C($R^{14}$)-; -C($R^{14}$)=C($R^{14}$)-; -N=;=N-; -C≡C-; $-(O-C(R^1)(R^2)-C(R^1)(R^2))_{1-30}-$; $-(C(R^1)(R^2)-C(R^1)(R^2)-O)_{1-30}-$; an optionally substituted alkenylene group; an optionally substituted alkynylene group; an optionally substituted cycloalkylene group; an optionally substituted cycloalkenylene group; an optionally substituted arylene group; an optionally substituted heteroarylene group; and an optionally substituted heterocyclylene group,
$R^1$, $R^2$, $R^3$, $R^4$, $R^5$, $R^6$, $R^7$, $R^8$, $R^9$, $R^{10}$, $R^{11}$, $R^{12}$, $R^{13}$, and $R^{14}$ are each independently a group selected from the group consisting of a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, and an optionally substituted heterocyclyl group, and when $R^3$ is an alkyl group, the alkyl group is optionally combined with an alkyl group on an adjacent methylene group to form a cyclic structure, and
m and n are each independently an integer of 0 to 2.

**8.** The antibody-multidrug conjugate precursor according to claim 7, or a salt thereof, wherein

$L^1$, $L^2$, and $L^3$ are each independently an optionally substituted alkylene group; and
one or more methylene groups in the chain of the alkylene group are replaced with one or more divalent groups independently selected from a following formula group:
formula group

-C($R^1$)(alkyl) -;

-C($R^{14}$)=;
-O-;
-N($R^3$)-;
-N=;
-N($R^3$)-C($R^1$)($R^2$)- (where, when $R^3$, and $R^1$ or $R^2$ are alkyl groups, $R^3$ and $R^1$ or $R^2$ are optionally combined to form a cyclic structure);
-N($R^3$)-C($R^1$)($R^2$)-C(=O)- (wherein, when $R^3$, and $R^1$ or $R^2$ are alkyl groups, $R^3$ and $R^1$ or $R^2$ are optionally combined to form a cyclic structure);
-O-C($R^1$)($R^2$)-;
-S-C($R^1$)($R^2$)-;
-N($R^3$)-N($R^3$)-;
-N($R^3$)-O-C($R^1$)($R^2$)-;
-S-;
-Si($R^4$)($R^5$)-;
-S-S-;
-SOm-;
-C(=O)-;
-C($R^1$)($R^2$)-C(=O)-;
-C(=C($R^6$)($R^7$))-:
-C(=N($R^8$))-:
-C(=N-O$R^9$)-;
-C(=N-N($R^{10}$)($R^{11}$))-;
-P(=O)($R^{12}$)-;
-P(=O)($R^{12}$)-O-;
-O-P(=O)($R^{12}$)-O-;
-P(=O)(O$R^{13}$)-O-;
-P(=O)(O$R^{13}$)-N($R^3$)-;
-O-P(=O)(O$R^{13}$)-O-;
-C($R^{14}$)=C($R^{14}$)-;
-C($R^{14}$)=C($R^{14}$)-P(=O) (O$R^{13}$)-N($R^3$)-;
-C($R^{14}$)=N-;
-C($R^{14}$)=N-N($R^3$)-;
-C≡C-;
-O-C($R^1$)($R^2$)-C($R^1$)($R^2$)-;
-O-C($R^1$)($R^2$)-C($R^1$)($R^2$)-O-;
-(O-C($R^1$)($R^2$)-C($R^1$)($R^2$))$_{2-30}$-;
-C(=O)-O-;
-C(=O)-S-;
-C(=O)-N($R^3$)-;
-C(=O)-N($R^3$)-O-;
-C(=O)-N($R^3$)-C($R^1$)($R^2$)- (where, when $R^3$, and $R^1$ or $R^2$ are alkyl groups, $R^3$ and $R^1$ or $R^2$ are optionally combined to form a cyclic structure);
-C(=S)-O-;
-C(=S)-S-;
-O-C(=O)-O-;
-O-C(=O)-N($R^3$)-;
-S-C(=O)-N($R^3$)-;
-N($R^3$)-C(=O)-N($R^3$)-;
an optionally substituted arylene group;
an optionally substituted heteroarylene group; and
an optionally substituted heterocyclylene group.

**9.** The antibody-multidrug conjugate precursor according to claim 7, or a salt thereof, wherein

$L^1$, $L^2$, and $L^3$ are each independently an optionally substituted alkylene group; and
one or more methylene groups in the chain of the alkylene group are replaced with one or more divalent groups independently selected from a following formula group:
formula group

-C(H or $C_1$-$C_4$ alky)=;
-O-;
-N(H or $C_1$-$C_4$ alky) -;
-N=;
-S-;
-C(=O)-;
-O-Si($CH_3$)($CH_3$)-O-;
-$CH_2$-C(=O)-;
-C(=O)-NH-;
-C(=O)-O-;
-C(=O)-S-;
-O-C(=O)-O-;
-N(H or $C_1$-$C_4$ alky)-C(=O)-O-;
-NH-NH-;
-N(H or $C_1$-$C_4$ alky) -O-$CH_2$-;
-C(=O)-$(CH_2)_{1-20}$-C(=O)-;
-C(=O)-$(CH_2)_{1-10}$-O-$(CH_2)_{1-10}$-C(=O)-;
-C(=O)-$CH_2$-$(O-CH_2CH_2)_{1-20}$-O-$CH_2$-C(=O)-;
-$(O-CH_2CH_2)_{1-20}$-;
-C(=O)-N(H or H or $C_1$-$C_4$ alky)-$CH_2CH_2$-N(H or H or $C_1$-$C_4$ alky-C(=O)-;

[C9]

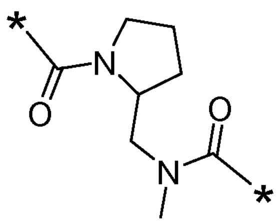

[wherein * is a point of attachment to an adjacent group];
-C(=O)-N(H or H or $C_1$-$C_4$ alky) -$CH_2$-;
-P(=O)(OH)-O-;
-O-P(=O)(OH)-O-;
-O-P(=O)(OH)-O-P(=O) (OH)-O-;
-O-P(=O)(OH)-O-P(=O)(OH)-O-P(=O)(OH)-O-;
-CH($CH_2$-$NH_2$ -;
-CH($CH_2$-NH-C(=O))-;
-CH($CH_2$-NH-C(=O)-$(CH_2CH_2\text{-}O\text{-})_{1-20}$-$CH_3$)-;
-CH($CH_2$-NH-C(=O)-$(CH_2)_{0-20}$-lactonyl)-;
-CH ($CH_2$-NH-C(=O)-$(CH_2CH_2\text{-}O\text{-})_{1-20}$-$CH_2$-lactonyl)-;
-CH ($CH_2$-NH-C(=O)-$(CH_2)_{1-20}$-phosphoryl)-;
-CH($CH_2$-NH-C(=O)-$(CH_2CH_2\text{-}O\text{-})_{1-20}$-$CH_2$-phosphoryl)-;
-CH($CH_2$-NH-$(CH_2)_{1-20}$-phosphoryl)-; - (cis)-CH=CH-P(=O)(O-$CH_2CH_3$ or O-$CH_2CH_2$-OH)-NH-(phenylene)-C(=O)-;
-C(=O)-(cyclohexylene)-;
-(succinimidylene)-;
-Gly-;
-Ala-;
-Val-;
-Leu-;
-Ile-;
-Phe-;
-Ser-;
-Cys-;
-Asp-;

-Glu-;
-Orn-;
-Lys-;
-Cit-;
-Arg-;
-His-;
-Pro-;
-GlyGly-;
-PheLys-;
-ValLys-;
-ValCit-;
-ValAla-;
-AspValCit-;
-GluValCit-;
-LysValCit-;
-SerValCit-;
-AspValAla-;
-GluValAla-;
-LysValAla-;
-SerValAla-;
-GlyGlyPheGly-;
-AspGlyGlyPheGly-;
-GluGlyGlyPheGly-;
-LysGlyGlyPheGly-;
-SerGlyGlyPheGly-;
-AspAspAspAspAsp-;

(in the amino acids and amino acid residues in peptides, carboxy groups in the side chains of Asp and Glu optionally form a lactonyl ester, lactonyl alkyl ester, or phosphoryl alkyl ester; or optionally form an unsubstituted amide, monoalkylamide, or dialkylamide, and the amino group of the amide moiety optionally has, as a substituent, a lactonyl group, a lactonylalkyl group, or a phosphorylalkyl group;
amino groups in the side chains of Lys and Orn optionally have, as a substituent, a lactonyl group, a lactonylalkyl group, a lactonylcarbonyl group, a lactonylalkylcarbonyl group, a phosphoryl group, a phosphorylalkyl group, or a phosphorylalkylcarbonyl group,; and
a hydroxy group in the side chain of Ser and a sulfhydryl group in the side chain of Cys optionally have, as a substituent, a lactonyl group, a lactonylalkyl group, a lactonylcarbonyl group, a lactonylalkylcarbonyl group, a phosphoryl group, a phosphorylalkyl group, or a phosphorylalkylcarbonyl group)

-N(H or $C_1$-$C_4$ alkyl)-(optionally substituted phenylene)-$CH_2$-O-C(=O)-;
-O-(optionally substituted phenylene)-$CH_2$-O-C(=O)-;
-N(H or $C_1$-$C_4$ alkyl)-(optionally substituted pyridylene)-$CH_2$-O-C(=O)-;

[C10]

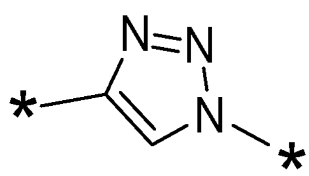

;

[C11]

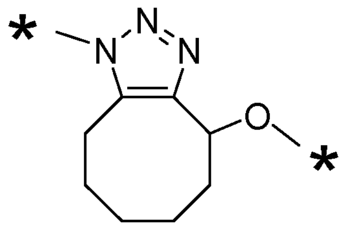

;

[C12]

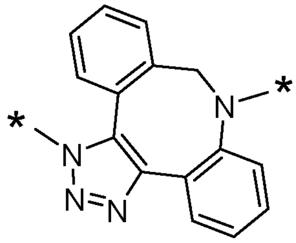

;

[C13]

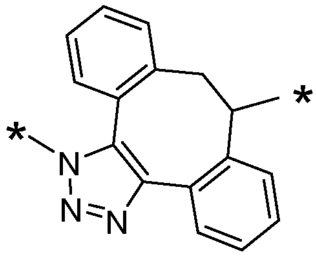

;

and

[C14]

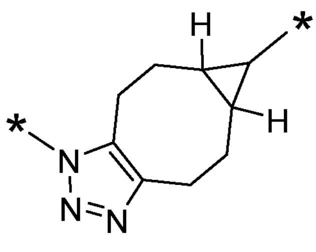

[in the formulae, * is a point of attachment to an adjacent group].

**10.** The antibody-multidrug conjugate precursor according to claim 7, or a salt thereof, wherein

$L^1$, $L^2$, and $L^3$ are each independently an optionally substituted alkylene group; and one or more methylene groups in the chain of said alkylene group are substituted with one or more divalent groups independently selected from a formula group below, formula group

$-CH_2-N(CH_3)-CH_2-$;
$-CH_2-C(=O)-$;

$-C(=O)-NH-CH_2-$;
$-C(=O)-(CH_2)_{1-10}-(O-CH_2CH_2)_{1-20}-O-(CH_2)_{1-10}-C(=O)-$;
$-C(=O)-(CH_2)_{1-10}-(O-CH_2CH_2)_{1-20}-O-(CH_2)_{1-10}-CH_2-NH-$;
$-NH-CH_2-(CH_2)_{1-10}-(O-CH_2CH_2)_{1-20}-O-(CH_2)_{1-10}-CH_2-NH-$;
$-C(=O)-N(CH_3)-CH_2CH_2-N(CH_3)-C(=O)-$;

[C15]

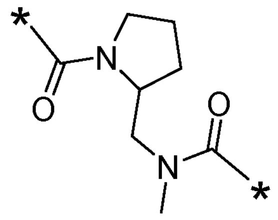

[in the formulae, * is a point of attachment to an adjacent group] ;
-P(=O)(OH)-O-;
-O-P (=O)(OH)-O-;
-O-P(=O)(OH)-O-P(=O) (OH)-O-;
-O-P(=O)(OH)-O-P(=O)(OH)-O-P(=O)(OH)-O-;
$-CH(CH_2-NH_2)-$;
$-CH(CH_2-NH(CH_3))-$;
$-CH(CH_2-N(CH_3)_2)-$;
$-CH(CH_2-NH-C(=O)-(CH_2CH_2-O-)_{0-20}-CH_3)-$;
$-CH(CH_2-NH-C(=O)-(CH_2)_{0-20}$-lactonyl)-;
$-CH(CH_2-NH-C(=O)-(CH_2CH_2-O-)_{1-20}-(CH_2)_{1-10}-CH_2$-lactonyl)-;
$-CH(CH_2-NH-C(=O)-(CH_2CH_2-O-)_{1-20}-(CH_2)_{1-10}-CH_2$-NH-lactonyl)-;
$-CH(CH_2-NH-C(=O)-(CH_2CH_2-O-)_{1-20}-(CH_2)_{1-10}-CH_2$-NH-C(=O)-lactonyl)-;
$-CH(CH_2-NH-C(=O)-(CH_2CH_2-O-)_{1-20}-(CH_2)_{1-10}$-C(=O)-NH-lactonyl)-;
$-CH(CH_2-NH-(CH_2)_{1-20}-(O)_{0-1}$-phosphoryl)-;
$-CH(CH_2-NH-C(=O)-(CH_2)_{1-20}$-phosphoryl)-;
$-CH(CH_2-NH-C(=O)-(CH_2)_{1-20}-C(=O)-NH-(CH_2)_{1-10}$-phosphoryl)-;
-CH $(CH_2-NH-C(=O)-(CH_2CH_2-O-)_{1-20}-(CH_2)_{1-10}-CH_2$-phosphoryl)-;
$-CH(CH_2-NH-C(=O)-(CH_2CH_2-O-)_{1-20}-(CH_2)_{1-10}-C(=O)-NH-(CH_2)_{1-10}$-phosphoryl) -;
$-CH(CH_2-NH-C(=O)-(CH_2CH_2-O-)_{1-20}-(CH_2)_{1-10}-C(=O)-NH-(CH_2)_{1-10}$-C(H, OH, Cl, $NH_2$, or $C_1$-$C_4$ alkyl) $(P(=O)(OH)_2)_2)-$;
- (cis)-CH=CH-P(=O)$(O-CH_2CH_3)$-NH- (phenylene)-C(=O)-;
-C(=O)-(cyclohexylene)-;
-(succinimidylene)-;
-Ser-;
-Cys-;
-Asp-;
-Glu-;
-Orn-;
-Lys-;
-ValLys-;
-ValCit-;
-ValAla-;
-GlyGly-C(=O)-NH-(optionally substituted phenylene)-$CH_2$-;
-PheLys-C(=O)-NH-(optionally substituted phenylene)-$CH_2$-;
-ValLys-C(=O)-NH-(optionally substituted phenylene)-$CH_2$-;
-ValCit-C(=O)-NH-(optionally substituted phenylene)-$CH_2$-;
-ValAla-C(=O)-NH-(optionally substituted phenylene)-$CH_2$-;
-AspValCit-C(=O)-NH-(optionally substituted phenylene)-$CH_2$-;
-GluValCit-C(=O)-NH-(optionally substituted phenylene)-$CH_2$-;
-LysValCit-C(=O)-NH-(optionally substituted phenylene)-$CH_2$-;

-SerValCit-C(=O)-NH-(optionally substituted phenylene)-$CH_2$-;
-AspValAla-C(=O)-NH-(optionally substituted phenylene)-$CH_2$-;
-GluValAla-C(=O)-NH-(optionally substituted phenylene)-$CH_2$-;
-LysValAla-C(=O)-NH-(optionally substituted phenylene)-$CH_2$-;
-SerValAla-C(=O)-NH-(optionally substituted phenylene)-$CH_2$-;
-GlyGlyPheGly-C(=O)-NH-(optionally substituted phenylene) -$CH_2$-;
-AspGlyGlyPheGly-C(=O)-NH-(optionally substituted phenylene) -$CH_2$-;
-GluGlyGlyPheGly-C(=O)-NH-(optionally substituted phenylene) -$CH_2$-;
-LysGlyGlyPheGly-C(=O)-NH-(optionally substituted phenylene) -$CH_2$-;
-SerGlyGlyPheGly-C(=O)-NH-(optionally substituted phenylene) -$CH_2$-;
-AspAspAspAspAsp-C(=O)-NH-(optionally substituted phenylene) -$CH_2$-;
-GlyGly-C(=O)-NH-(optionally substituted phenylene)-$CH_2$-O-C(=O)-;
-PheLys-C(=O)-NH-(optionally substituted phenylene)-$CH_2$-O-C(=O)-;
-ValLys-C(=O)-NH-(optionally substituted phenylene)-$CH_2$-O-C(=O)-;
-ValCit-C(=O)-NH-(optionally substituted phenylene)-$CH_2$-O-C(=O)-;
-ValAla-C(=O)-NH-(optionally substituted phenylene)-$CH_2$-O-C(=O)-;
-AspValCit-C(=O)-NH-(optionally substituted phenylene)-$CH_2$-O-C(=O)-;
-GluValCit-C(=O)-NH-(optionally substituted phenylene)-$CH_2$-O-C(=O)-;
-LysValCit-C(=O)-NH-(optionally substituted phenylene)-$CH_2$-O-C(=O)-;
-SerValCit-C(=O)-NH-(optionally substituted phenylene)-$CH_2$-O-C(=O)-;
-AspValAla-C(=O)-NH-(optionally substituted phenylene)-$CH_2$-O-C(=O)-;
-GluValAla-C(=O)-NH-(optionally substituted phenylene)-$CH_2$-O-C(=O)-;
-LysValAla-C(=O)-NH-(optionally substituted phenylene)-$CH_2$-O-C(=O)-;
-SerValAla-C(=O)-NH-(optionally substituted phenylene)-$CH_2$-O-C(=O)-;
-GlyGlyPheGly-C(=O)-NH-(optionally substituted phenylene)-$CH_2$-O-C(=O)-;
-AspGlyGlyPheGly-C(=O)-NH-(optionally substituted phenylene)-$CH_2$-O-C(=O)-;
-GluGlyGlyPheGly-C(=O)-NH-(optionally substituted phenylene)-$CH_2$-O-C(=O)-;
-LysGlyGlyPheGly-C(=O)-NH-(optionally substituted phenylene)-$CH_2$-O-C(=O)-;
-SerGlyGlyPheGly-C(=O)-NH-(optionally substituted phenylene)-$CH_2$-O-C(=O)-;
-AspAspAspAspAsp-C(=O)-NH-(optionally substituted phenylene)-$CH_2$-O-C(=O)-;
-GlyGlyPheGly-C(=O)-NH-$CH_2$-;
-AspGlyGlyPheGly-C(=O)-NH-$CH_2$-

(in said amino acid residues and the amino acid residues in the peptides,
the carboxy group(s) in the side chain(s) of Asp and Glu is/are converted into O-(lactonyl) ester, O-(lactonylalkyl) ester, O-(phosphorylalkyl) ester, N-(lactonyl)amide, N-(lactonylalkyl)amide, or N-(phosphorylalkyl)amide,
the amino group(s) in the side chain(s) of Lys and Orn has/have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s), and
the hydroxyl group in the side chain of Ser and the sulfhydryl group in the side chain of Cys optionally have lactonyl group(s), lactonylalkyl group(s), lactonylcarbonyl group(s), lactonylalkylcarbonyl group(s), phosphoryl group(s), phosphorylalkyl group(s), or phosphorylalkylcarbonyl group(s) as substituent(s));

[C16]

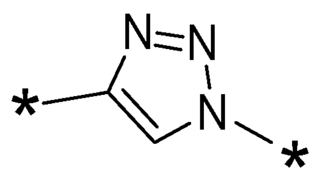

;

[C17]

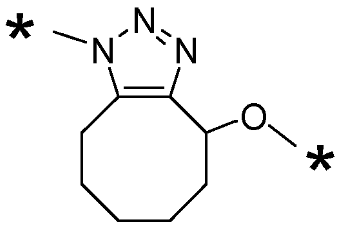

;

[C18]

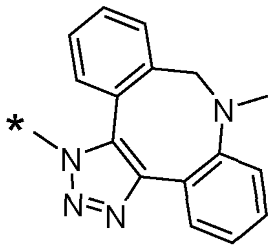

;

[C19]

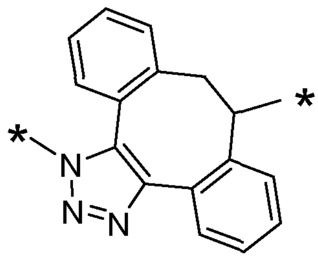

;

and

[C20]

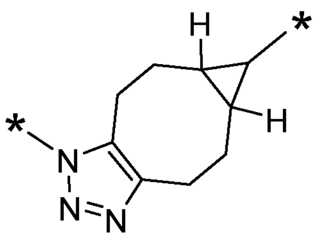

[in the formulae, * is a point of attachment to an adjacent group].

**11.** The antibody-multidrug conjugate precursor according to any one of claims 1 to 10, or a salt thereof, wherein $D^1$ and $D^2$ are residues of antitumor drug molecules independently selected from the group consisting of camptothecin; MMAE; maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; ciprofloxacin; and cadrofloxacin (CS-940); or analogs or derivatives thereof; or derivatives of said analogs.

**12.** The antibody-multidrug conjugate precursor according to any one of claims 1 to 11, or a salt thereof, wherein

$D^1$ and $D^2$ are residues of antitumor drug molecules independently selected from the group consisting of camptothecin; MMAE; maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; and superdox; or analogs or derivatives thereof; or derivatives of said analogs.

**13.** The antibody-multidrug conjugate precursor according to any one of claims 1 to 12, or a salt thereof, wherein $D^1$ and $D^2$ are residues of antitumor drug molecules independently selected from the group consisting of camptothecin; MMAE; maytansine; PBD (parabenzodiazepine) dimer; and eribulin; or analogs or derivatives thereof; or derivatives of said analogs.

**14.** The antibody-multidrug conjugate precursor according to any one of claims 1 to 13, or a salt thereof, wherein $D^1$ and $D^2$ are residues of identical antitumor drugs or analogs or derivatives thereof; or derivatives of said analogs.

**15.** The antibody-multidrug conjugate precursor according to any one of claims 1 to 13, or a salt thereof, wherein $D^1$ and $D^2$ are residues of different antitumor drugs or analogs or derivatives thereof; or derivatives of said analogs.

**16.** The antibody-multidrug conjugate precursor according to any one of claims 1 to 15, or a salt thereof, wherein at least one of $D^1$ and $D^2$ is a residue of an antitumor drug or an analog or derivative thereof which has a hydroxyl group in the molecule and in which at least one hydroxyl group is phosphorylated; or a derivative of said analog.

**17.** The antibody-multidrug conjugate precursor according to any one of claims 1 to 16, or a salt thereof, wherein one or more lactonyl groups and one or more phosphoryls are independently present as substituents or protecting groups at any position of the antitumor drug residues or $L^1$, $L^2$, or $L^3$, the total number of said lactonyl groups is 1 to 8, the lactonyl groups may be identical to or different from each other when said number of the lactonyl groups is two or more, and the total number of said phosphoryl groups is 1 to 4.

**18.** The antibody-multidrug conjugate precursor according to any one of claims 1 to 17, or a salt thereof, wherein

one or more lactonyl groups and one or more phosphoryls are independently present as substituents or protecting groups at any position of the antitumor drug residues or $L^1$, $L^2$, or $L^3$, and
apart from the above, when at least any one of the antitumor drug residues and $L^1$, $L^2$, or $L^3$ has one or more groups selected from, as substituents or protecting groups, a phosphorylalkylcarbonyl group (-C(=O)-alkylene-P(=O)(OH)$_2$); a dialkylamino group; and a cyclic amino group that forms a ring together with the dialkyl group at any position independently and there are a plurality of said groups in total, the groups may be identical to or different from each other.

**19.** An antibody-multidrug conjugate precursor selected from a group consisting of

Example 1

[C22]

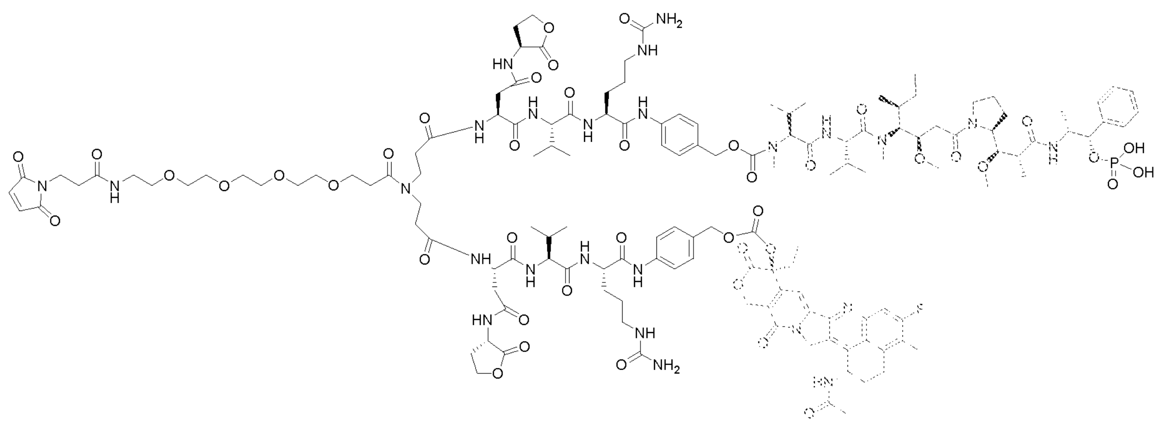

Example 2

[C23]

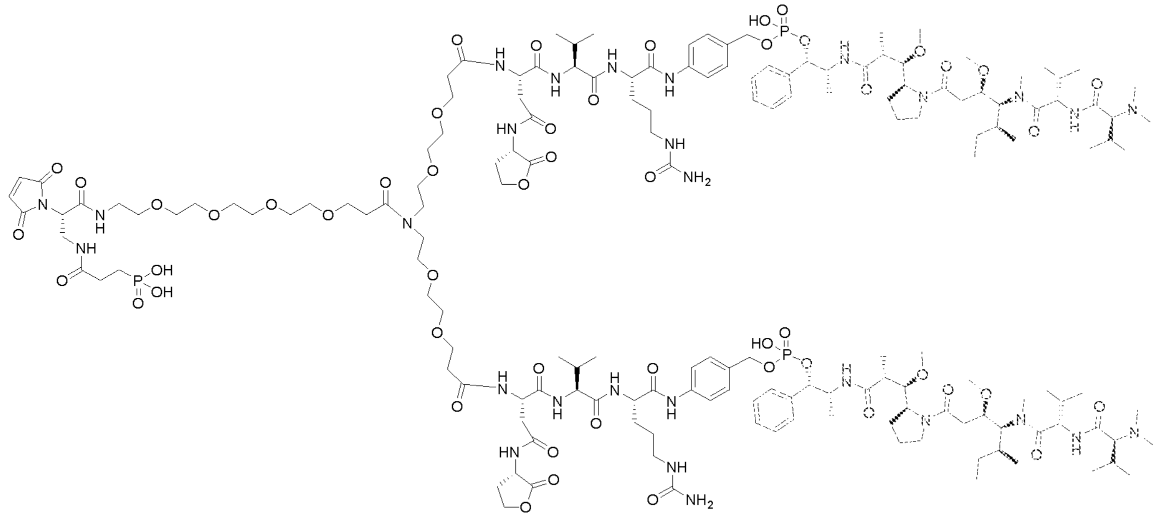

Example 3

[C24]

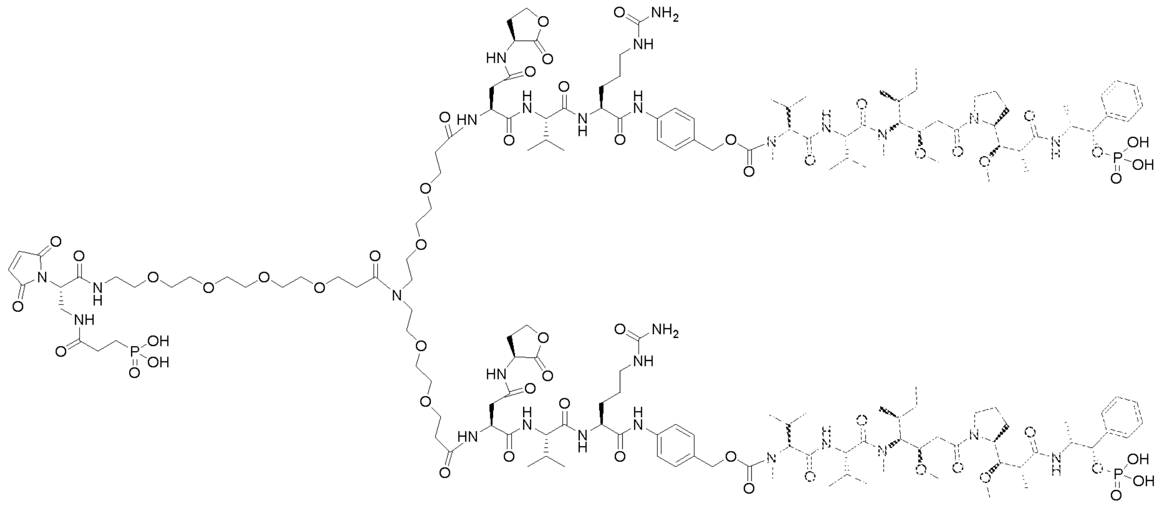

Example 4

[C25]

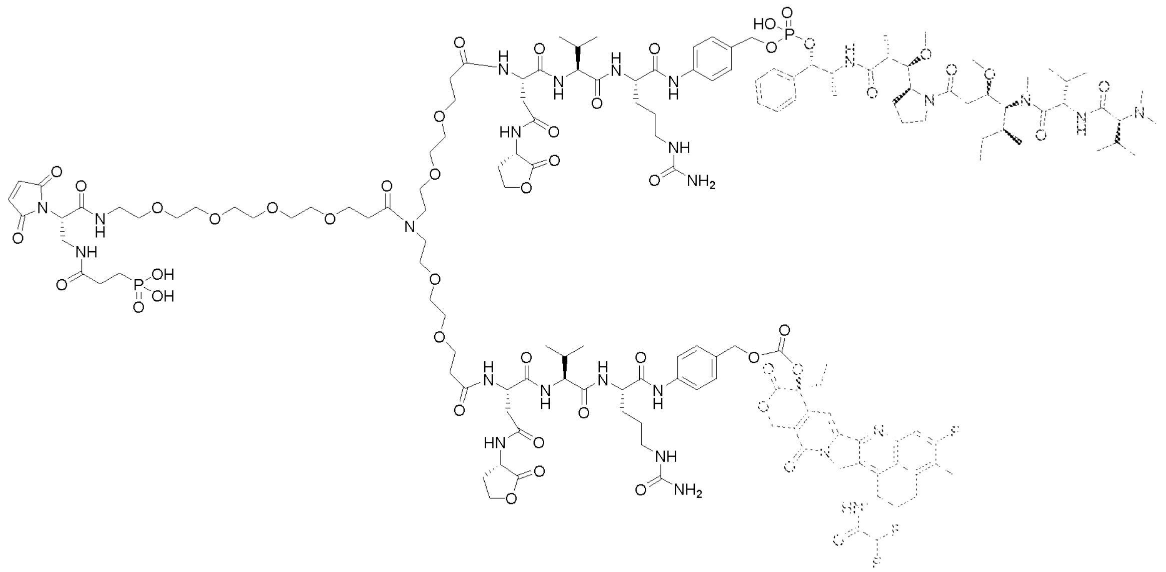

Example 5

[C26]

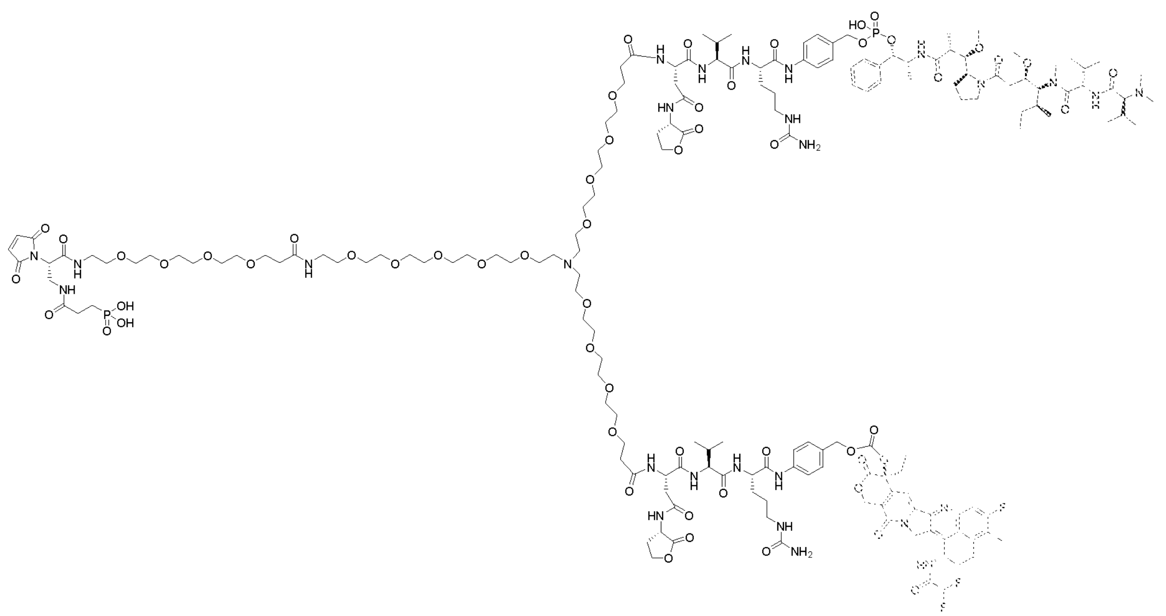

Example 6

[C00], and

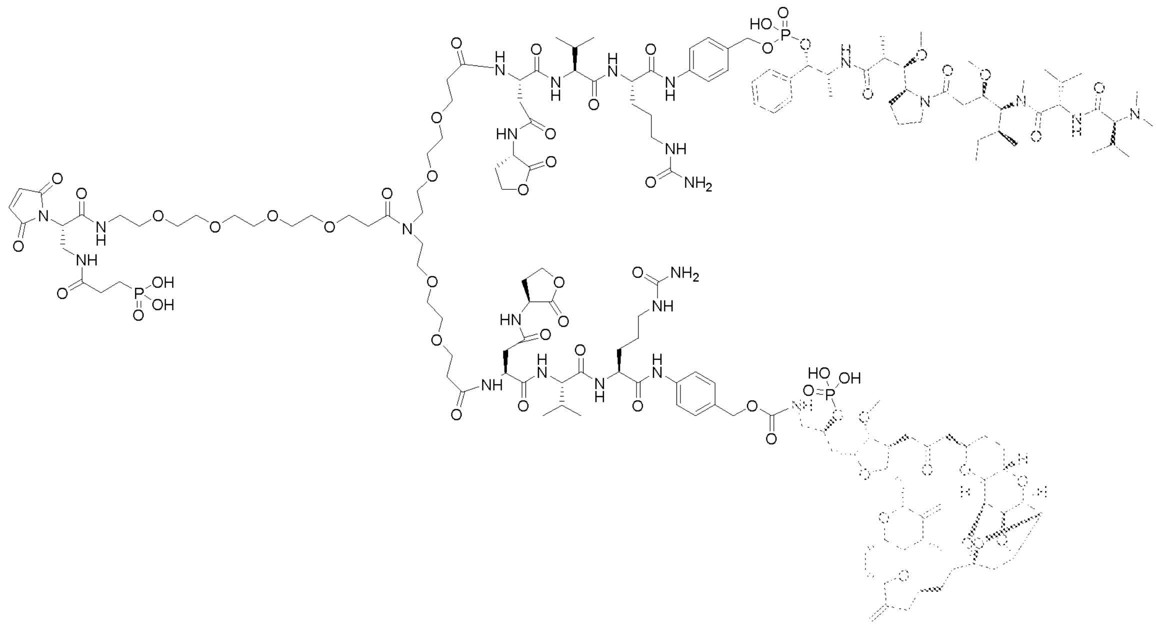

Example 7

[C00]

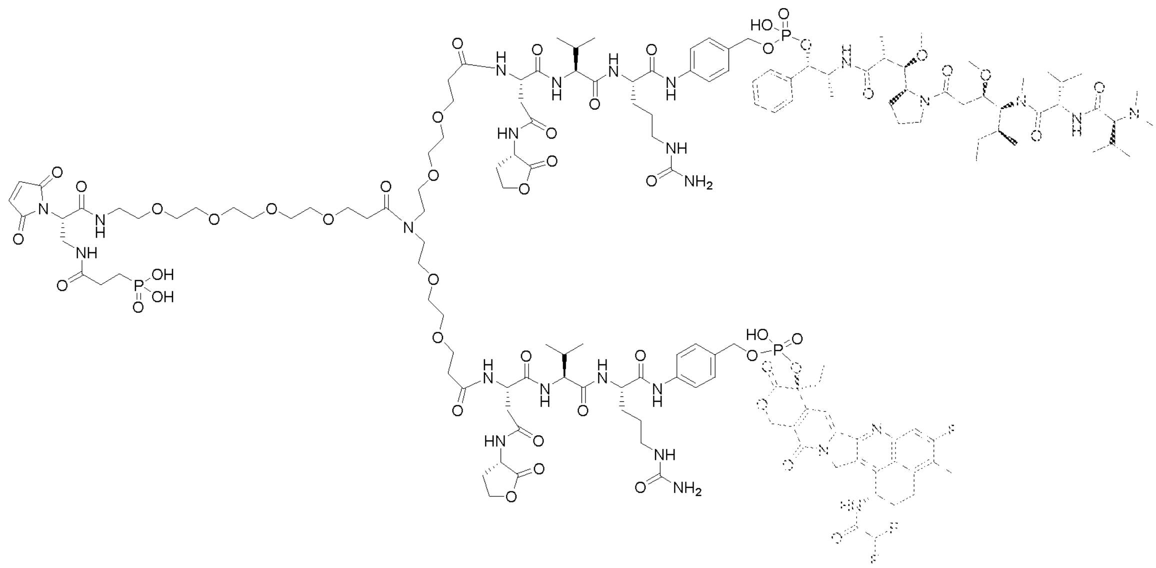

[in the formulae, steric configurations of the amino acid residues in the linkers are all L-shaped], or a salt thereof.

**20.** A synthetic intermediate (II-1) of an antibody-multidrug conjugate precursor represented by General Formula (II-1) below:

[C27]

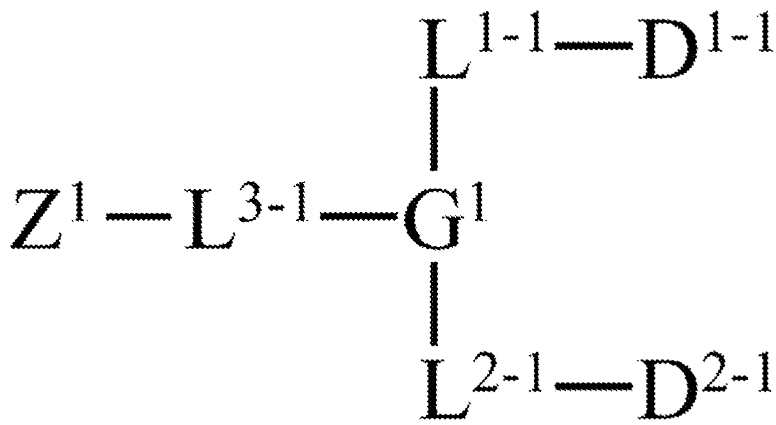

(II-1)

[in the formula,

$Z^1$ is the reactive group as defined in claim 1; or a bonding group for bonding said reactive group or the antitumor drug residue as defined in claim 1 to the linker, and the bonding group may be a protective body protected by a protecting group,
$G^1$ is a group represented by the Formula (i), (ii), (iii), or (iv),
$L^{1-1}$ is a linker linking $G^1$ to $D^{1-1}$,
$L^{2-1}$ is a linker linking $G^1$ to $D^{2-1}$,
$L^{3-1}$ is a linker linking $G^1$ to $Z^1$,
$L^{1-1}$, $L^{2-1}$, and $L^{3-1}$ may be identical to or different from each other,
$D^{1-1}$ and $D^{2-1}$ are antitumor drug residues as defined in claim 1 or

bonding groups or protective bodies thereof,

$D^{1-1}$ and $D^{2-1}$ may be identical to or different from each other,
when there are one or more groups independently selected from a lactonyl group; a phosphoryl group; a phosphorylalkylcarbonyl group ($-C(=O)$-alkylene-$P(=O)(OH)_2$); dialkylamino group; and a cyclic amino group that forms a ring together with the dialkyl group at any position(s) of the antitumor drug residues or $L^{1-1}$, $L^{2-1}$, or $L^{3-1}$ and there are a plurality of said groups in total, the groups may be identical to or different from each other, and when $Z^1$ is a reactive group, at least one of $D^{1-1}$ and $D^{2-1}$ is a bonding group],
or a salt thereof.

**21.** The synthetic intermediate (II-1) of the conjugate precursor according to claim 20, or a salt thereof, wherein $G^1$ is a group selected from the formulae according to claim 5 or 6.

**22.** The synthetic intermediate (II-1) of the conjugate precursor according to claim 20 or 21, or a salt thereof, wherein

$L^{1-1}$, $L^{2-1}$, and $L^{3-1}$ are each independently an optionally substituted alkylene group; and
one or more methylene groups in the chain of said alkylene group are each independently substituted with one or more groups selected from the divalent groups according to any one of claims 7 to 10.

**23.** The synthetic intermediate (II-1) of the conjugate precursor according to any one of claims 20 to 22, or a salt thereof, wherein
$Z^1$ is a bonding group which is a hydroxyl group, a nitro group, a cyano group, an amide group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.

**24.** The synthetic intermediate (II-1) of the conjugate precursor according to any one of claims 20 to 23, or a salt thereof, wherein

$Z^1$ is a bonding group which is a hydroxyl group, a nitro group, a cyano group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.

**25.** The synthetic intermediate (II-1) of the conjugate precursor according to any one of claims 20 to 24, or a salt thereof, wherein
$Z^1$ is a bonding group which is a hydroxyl group, a nitro group, an oxo group, an azide group, an amino group, a monoalkylamino group which may have a substituent, an imino group (also including -N= and an imino group of a cyclic amine), a carboxy group, a phosphoryl group, an alkynyl group, a cycloalkynyl group, or a sulfhydryl group; or a protective body thereof.

**26.** The synthetic intermediate (II-1) of the conjugate precursor according to any one of claims 20 to 25, or a salt thereof, wherein
$Z^1$ is a bonding group which is a hydroxyl group, a nitro group, an oxo group, an azide group, an amino group, a monoalkylamino group which may have a substituent, an imino group (also including -N= and an imino group of a cyclic amine), a carboxy group, a phosphoryl group, an alkynyl group, or a cycloalkynyl group; or a protective body thereof.

**27.** The synthetic intermediate (II-1) of the conjugate precursor according to any one of claims 20 to 26, or a salt thereof, wherein

$Z^1$ is a group selected from the bonding groups according to any one of claims 23 to 26, and
one of $D^{1-1}$ and $D^{2-1}$ is a group selected from the antitumor drug residues according to any one of claims 11 to 16, and the other is a group selected from the bonding groups according to any one of claims 23 to 26.

**28.** The synthetic intermediate (II-1) of the conjugate precursor according to any one of claims 20 to 26, or a salt thereof, wherein

$Z^1$ is a group selected from the bonding groups according to any one of claims 23 to 26, and
both of $D^{1-1}$ and $D^{2-1}$ are each independently a group selected from the antitumor drug residues according to any one of claims 11 to 16.

**29.** The synthetic intermediate (II-1) of the conjugate precursor according to any one of claims 20 to 26, or a salt thereof, wherein
all of $Z^1$, $D^{1-1}$, and $D^{2-1}$ are each independently a group selected from the bonding groups according to any one of claims 23 to 26.

**30.** The synthetic intermediate (II-1) of the conjugate precursor according to any one of claims 20 to 22, or a salt thereof, wherein

$Z^1$ is the reactive group as defined in claim 1, and
one of $D^{1-1}$ and $D^{2-1}$ is a group selected from the antitumor drug residues according to any one of claims 11 to 16, and the other is a group selected from the bonding groups according to any one of claims 23 to 26.

**31.** The synthetic intermediate (II-1) of the conjugate precursor according to any one of claims 20 to 22, or a salt thereof, wherein

$Z^1$ is the reactive group as defined in claim 1, and
both of $D^{1-1}$ and $D^{2-1}$ are each independently a group selected from the bonding groups according to any one of claims 23 to 26.

**32.** The synthetic intermediate (II-1) of the conjugate precursor according to any one of claims 20 to 31, or a salt thereof, wherein
one or more lactonyl groups and one or more phosphoryl groups are independently present as substituents or protecting groups at any position of the antitumor drug residues or $L^{1-1}$, $L^{2-1}$, or $L^{3-1}$, a total number of said lactonyl groups is 1 to 8, the lactonyl groups may be identical to or different from each other when said number of the lactonyl groups is two or more, and a total number of said phosphoryl groups is 1 to 4.

**33.** The synthetic intermediate (II-1) of the conjugate precursor according to any one of claims 20 to 32, or a salt thereof, wherein

one or more lactonyl groups and one or more phosphoryls are independently present as substituents or protecting groups at any position of the antitumor drug residues or $L^{1-1}$, $L^{2-1}$, or $L^{3-1}$, and
apart from the above, when there are one or more groups each independently selected from a phosphorylalkylcarbonyl group (-C(=O)-alkylene-P(=O)$(OH)_2$); dialkylamino group; and a cyclic amino group that forms a ring together with the dialkyl group at any position(s) of the antitumor drug residues or $L^{1-1}$, $L^{2-1}$, or $L^{3-1}$ and there are a plurality of said groups in total, the groups may be identical to or different from each other.

**34.** A synthetic intermediate (II-2) of a conjugate precursor represented by General Formula (II-2) below:

[C28]

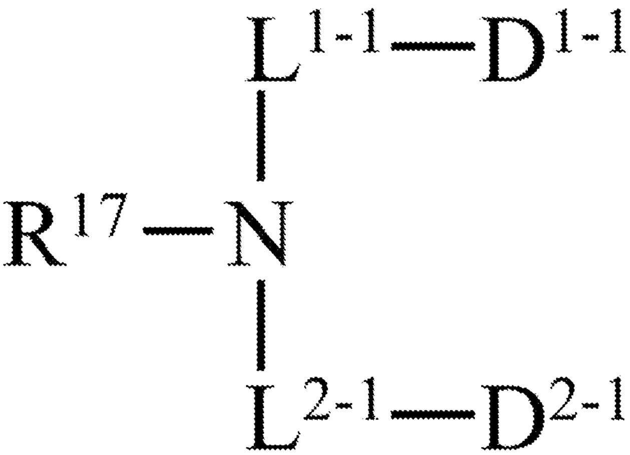

(II-2)

[in the formula,

$L^{1-1}$ and $L^{2-1}$ are each independently an optionally substituted alkylene group, and one or more methylene groups in the chain of said alkylene group are each independently substituted with one or more groups selected from the divalent groups according to any one of claims 7 to 10,
$D^{1-1}$ and $D^{2-1}$ are groups selected from the antitumor drug residues according to any one of claims 11 to 16 or groups selected from the bonding groups according to any one of claims 23 to 26, and $D^{1-1}$ and $D^{2-1}$ may be identical to or different from each other,
when there are one or more groups independently selected from a lactonyl group; a phosphoryl group; a phosphorylalkylcarbonyl group (-C(=O)-alkylene-P(=O)$(OH)_2$); dialkylamino group; and a cyclic amino group that forms a ring together with the dialkyl group as substituents or protecting groups at any position(s) of said $L^{1-1}$ and $L^{2-1}$ or antitumor drug residues and there are a plurality of said groups in total, the groups may be identical to or different from each other, and
$R^{17}$ is a hydrogen atom or a protecting group], or a salt thereof.

**35.** Conjugate precursor synthesis intermediates (II-1) and (II-2) selected from the group consisting of

Example 1-15

[C41]

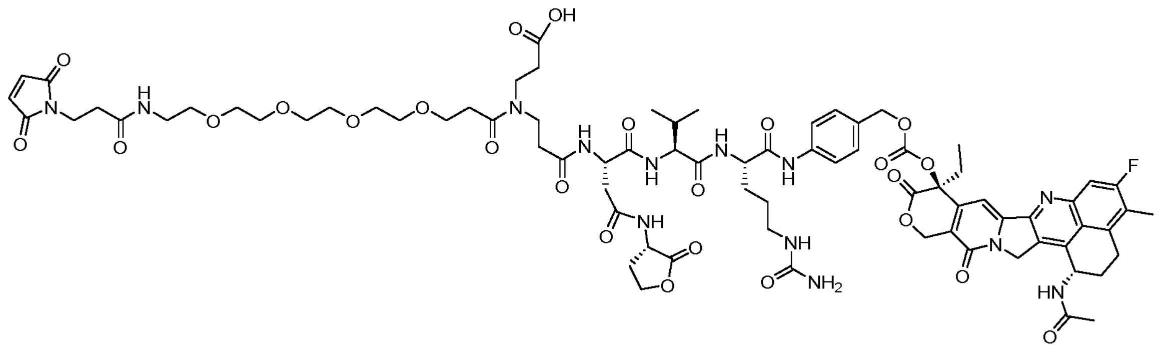

Example 2-10

[C46]

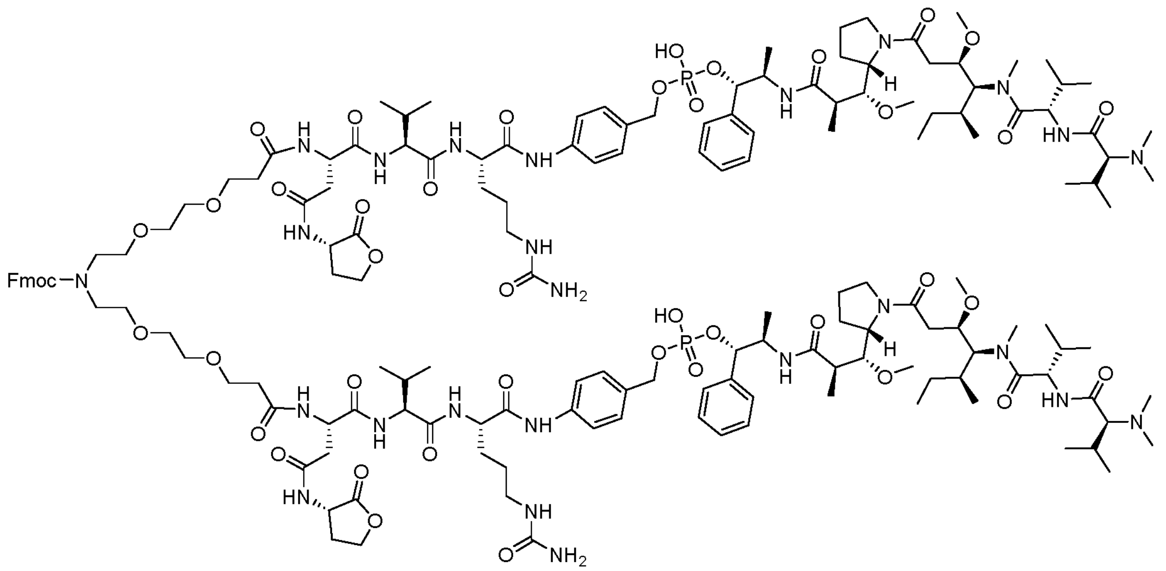

Example 3-1

[C47]

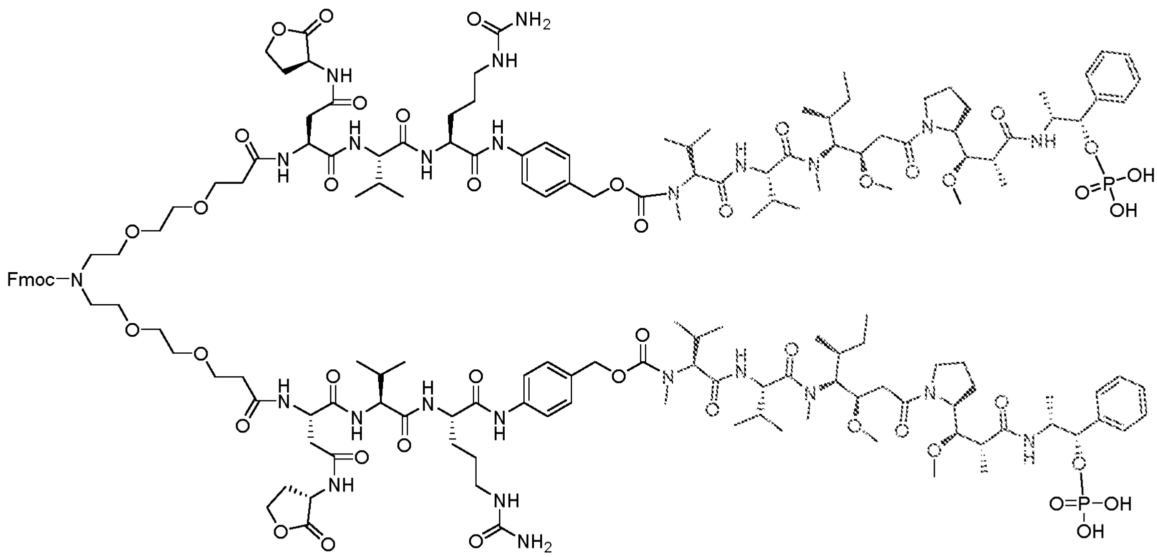

Example 4-3

[C50]

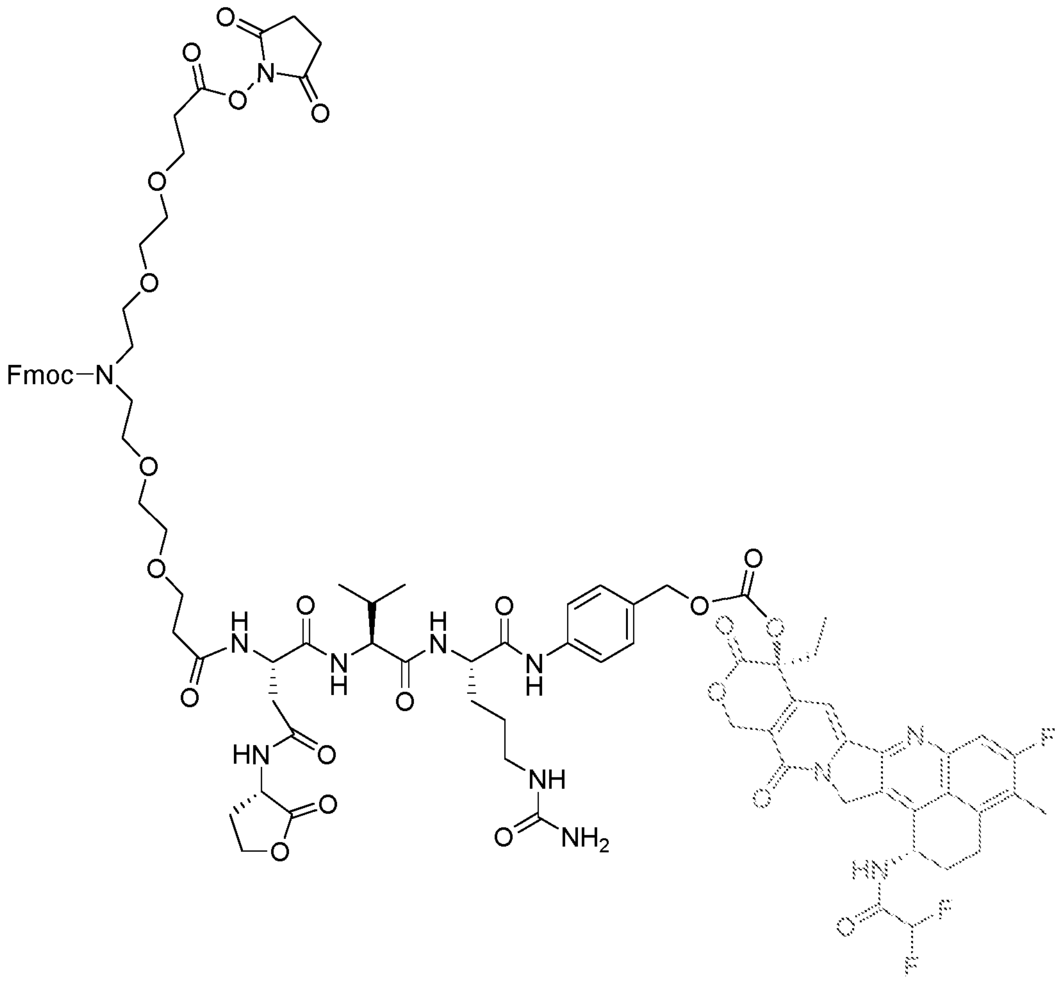

Example 4-4

[C51]

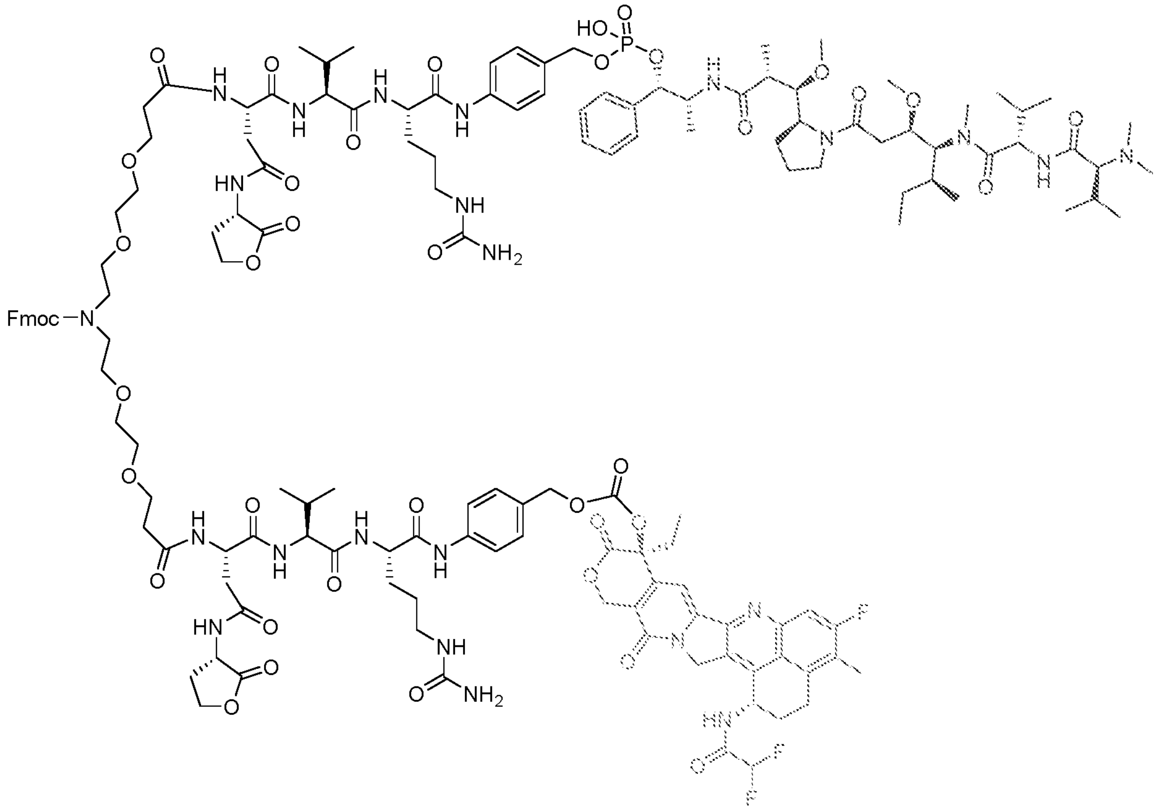

Example 5-2

[C52]

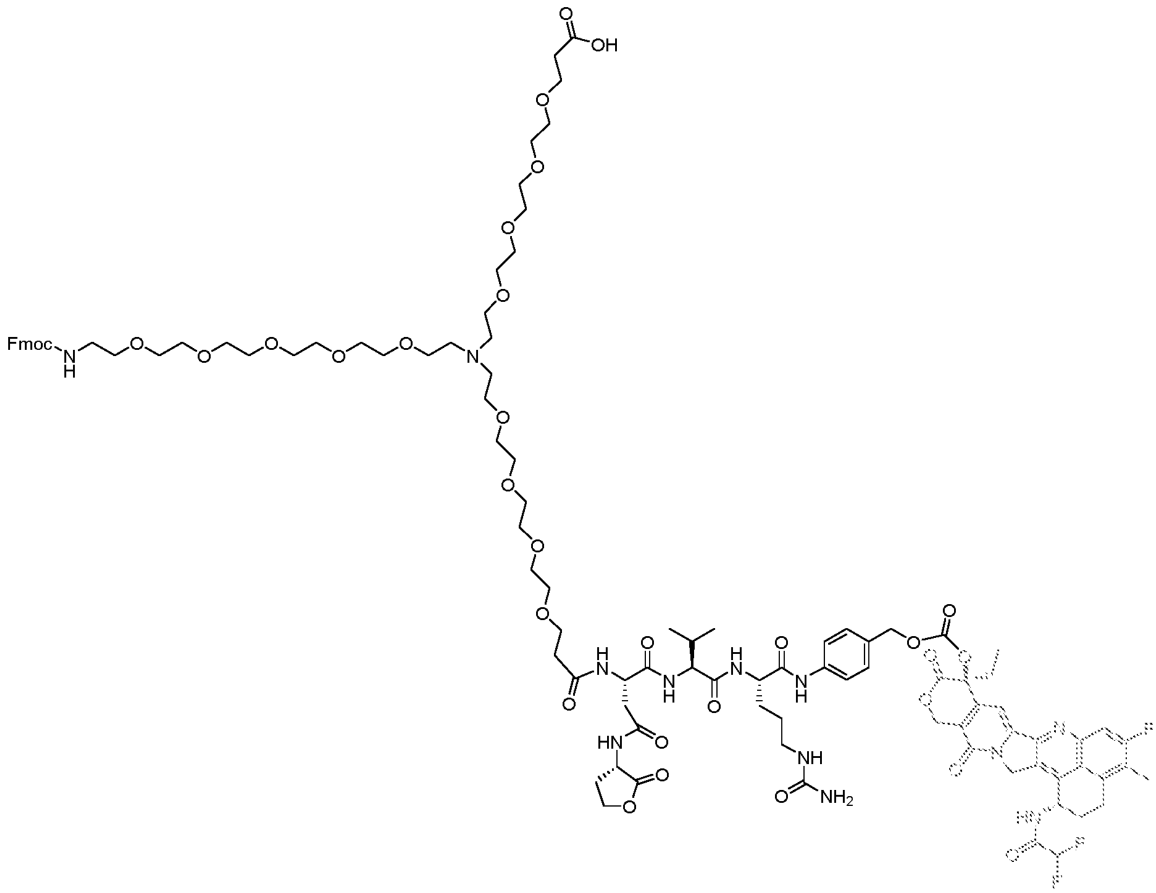

Example 5-3

[C53]

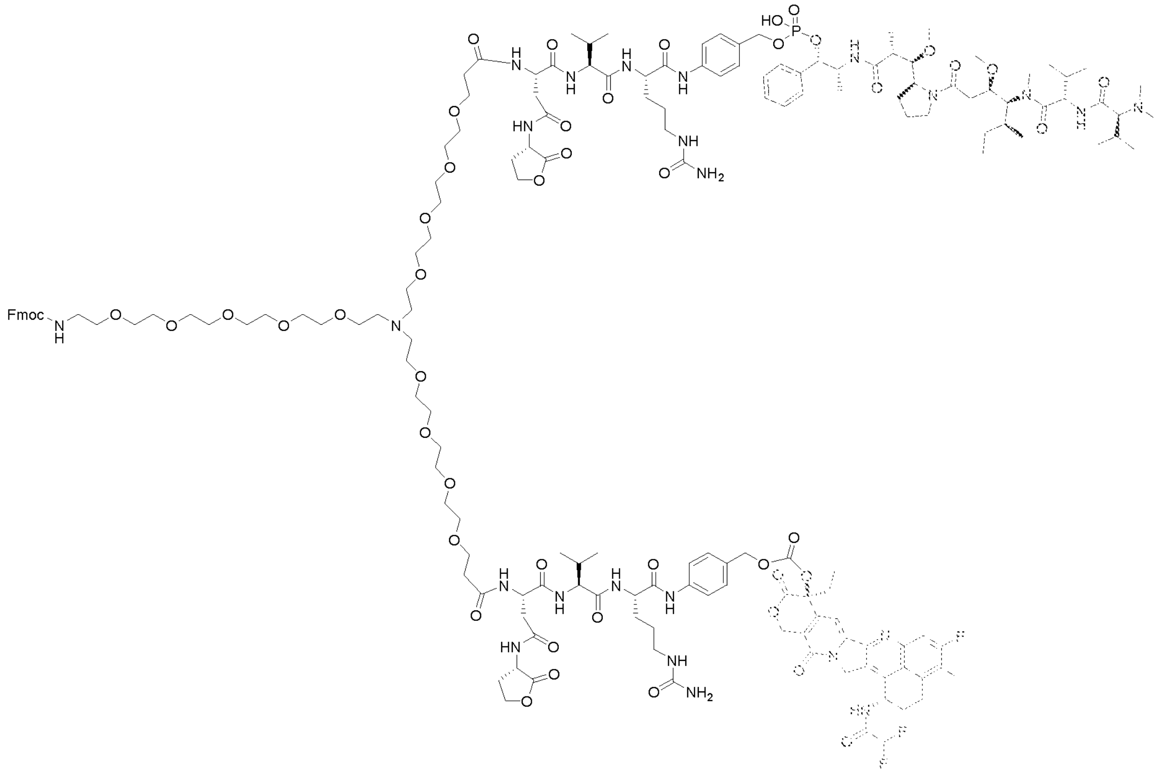

Example 6-4

[C557]

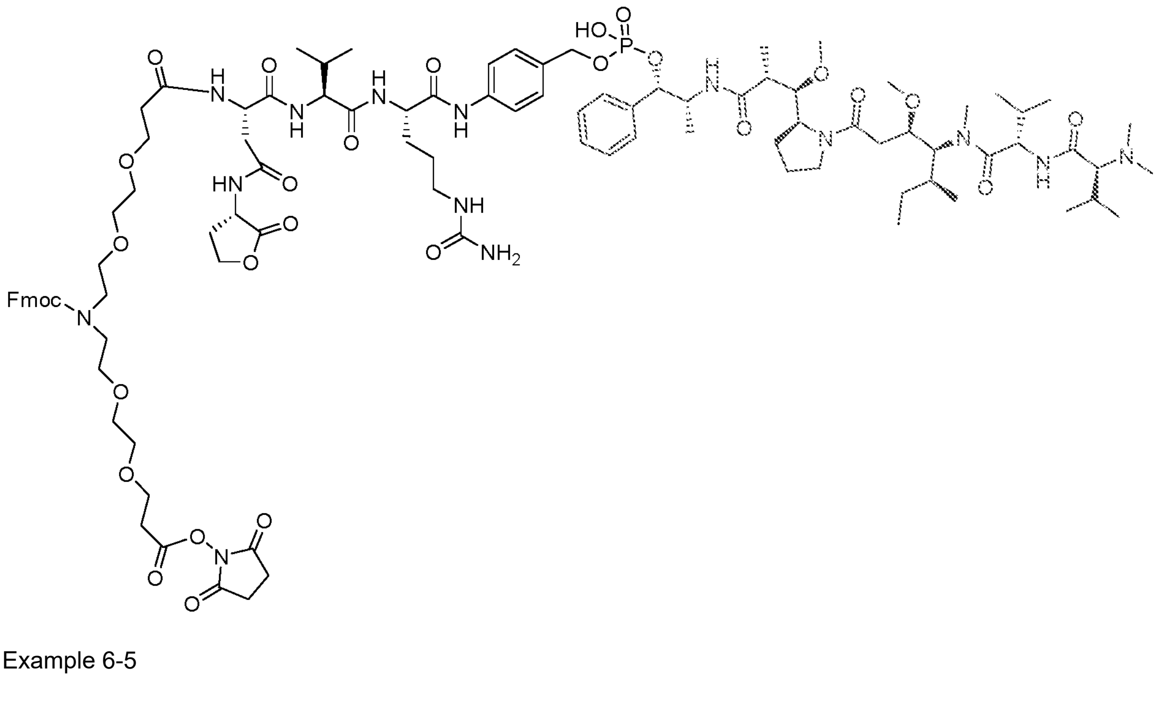

Example 6-5

[C558]

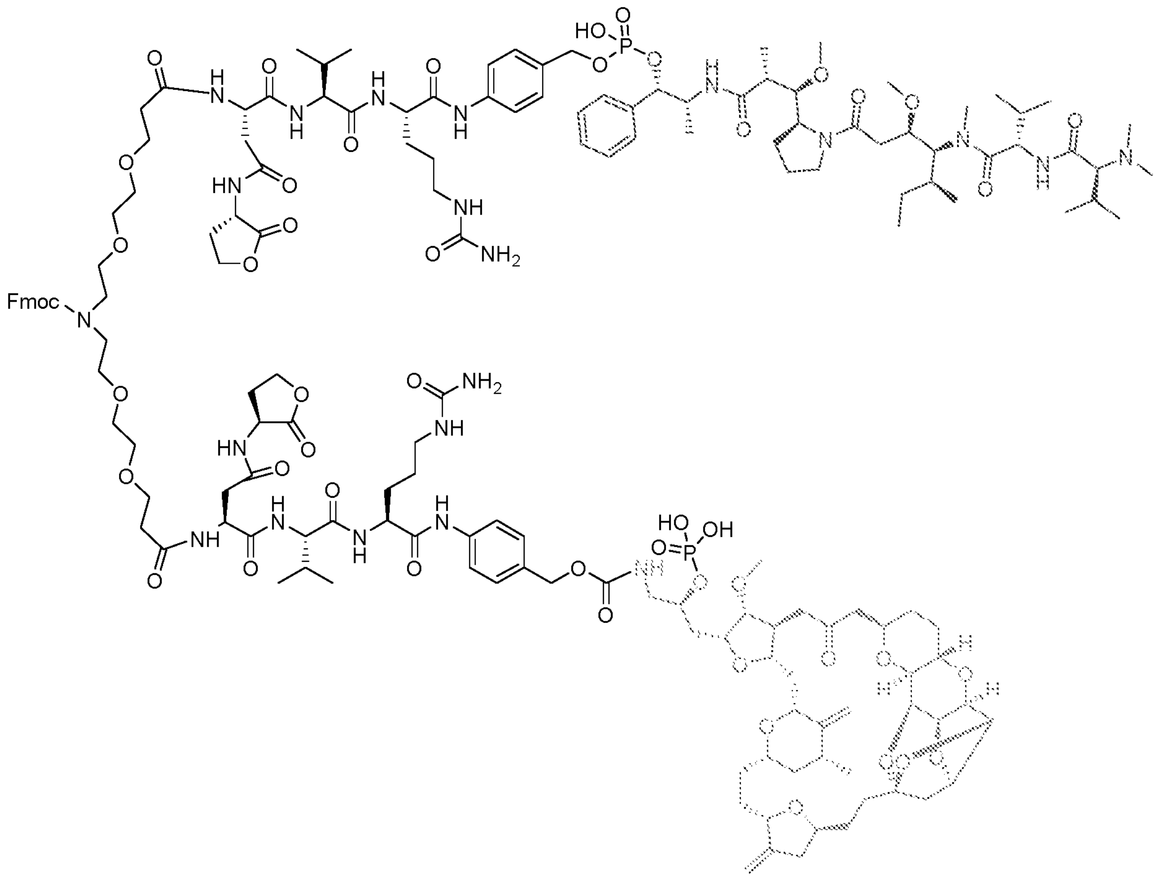

Example 7-6

[C559]

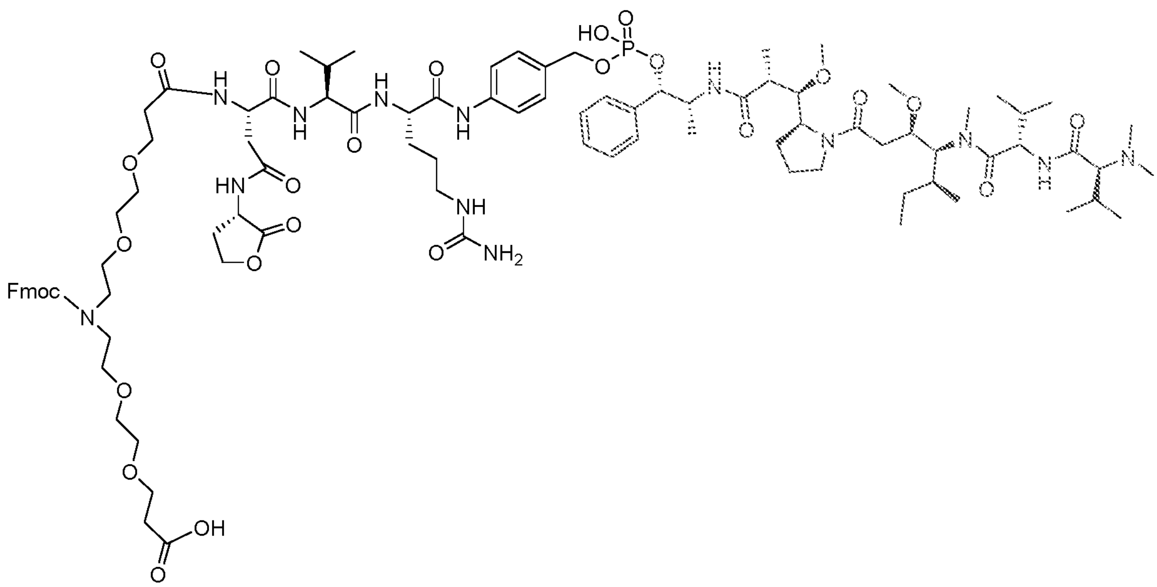

Example 7-7

[C560]

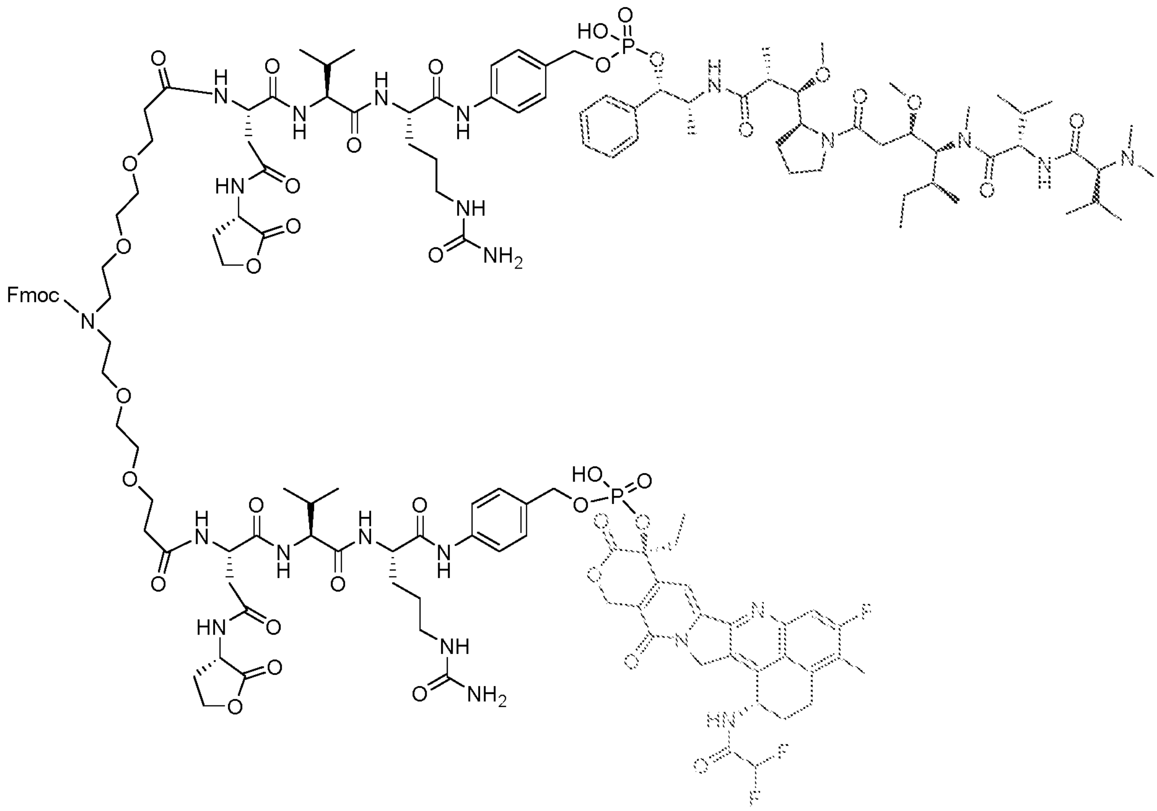

[in the formulae, steric configurations of amino acid residues in linkers are all L-shaped], or a salt thereof.

**36.** A precursor of an antibody-drug conjugate (ADC) containing an antibody and a drug, or a salt thereof, wherein

the ADC is one in which an antibody is directly or indirectly linked to a drug via a linker,
the precursor has a linker and a drug, and
the linker has a reactive group (a linkage moiety with an antibody) that can react with a functional group (for instance, a thiol group) in an antibody, and is modified by a solubilizing group, for instance, a group selected from the group consisting of a lactonyl group, an optionally substituted $C_1$-$C_6$ aminoalkyl group, and a phosphoryl group (especially a solubilizing group).

**37.** A compound having Formula (I) above, or a salt thereof, wherein
the formula has $R^{18}$ and $R^{19}$ instead of $D^1$ and $D^2$, respectively, and $R^{18}$ and $R^{19}$ are independently hydrogen atoms, protecting groups, or bonding groups.

**38.** A compound having Formula (II-1) above, or a salt thereof, wherein
the formula has $R^{18}$ and $R^{19}$ instead of $D^{1-1}$ and $D^{2-1}$, respectively, and $R^{18}$ and $R^{19}$ are independently hydrogen atoms, protecting groups, or bonding groups.

**39.** A compound having Formula (II-2) above, or a salt thereof, wherein
the formula has $R^{18}$ and $R^{19}$ instead of $D^{1-1}$ and $D^{2-1}$, respectively, and $R^{18}$ and $R^{19}$ are independently hydrogen atoms, protecting groups, or bonding groups.

**40.** A method for producing an antibody-drug conjugate (ADC) containing an antibody and a drug, the method comprising: reacting the compound or salt thereof according to any one of claims 37 to 39 with an antibody and an antitumor drug molecule, an analog or derivative thereof, and a derivative of said analog to obtain an ADC.

**41.** A method for producing an antibody-drug conjugate (ADC) containing an antibody and a drug, the method comprising: reacting an antibody with the precursors or salts thereof according to any one of claims 1 to 19 to obtain an ADC.

Fig. 1

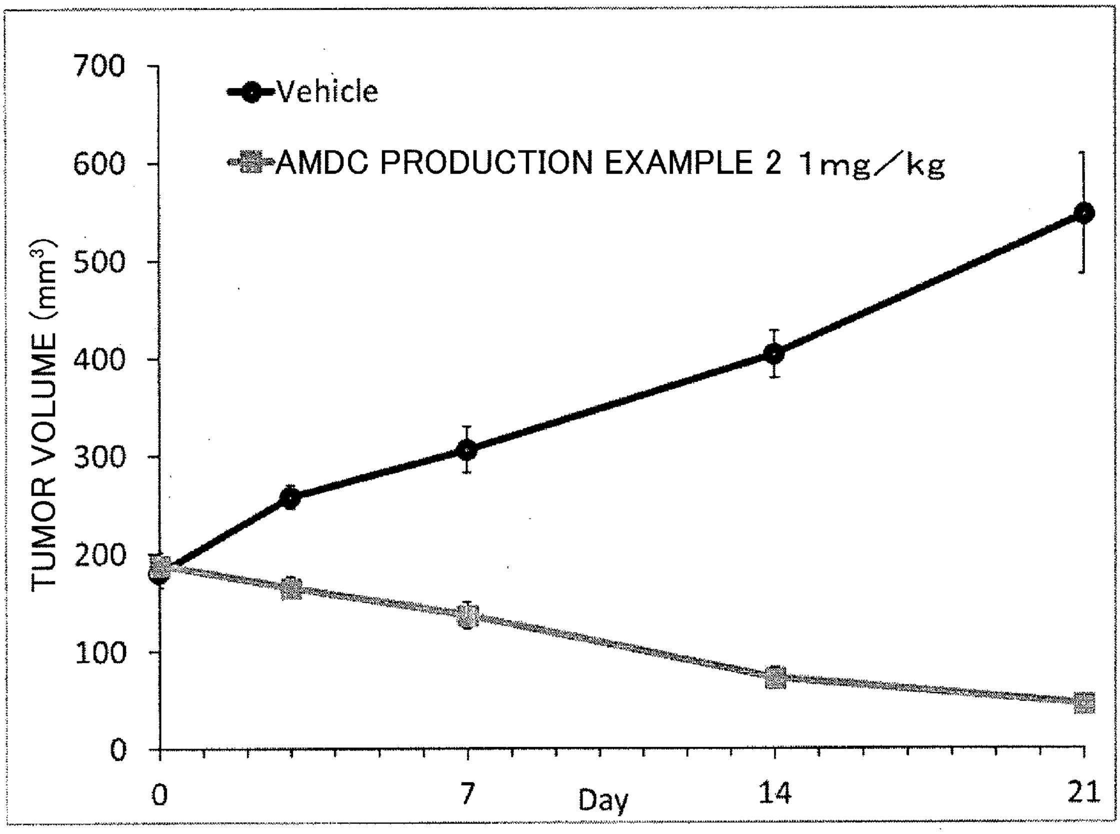
700
600
500
400
300
200
100
0
TUMOR VOLUME (mm3)
Vehicle
AMDC PRODUCTION EXAMPLE 2 1mg／kg
0
7
Day
14
21

Fig. 2

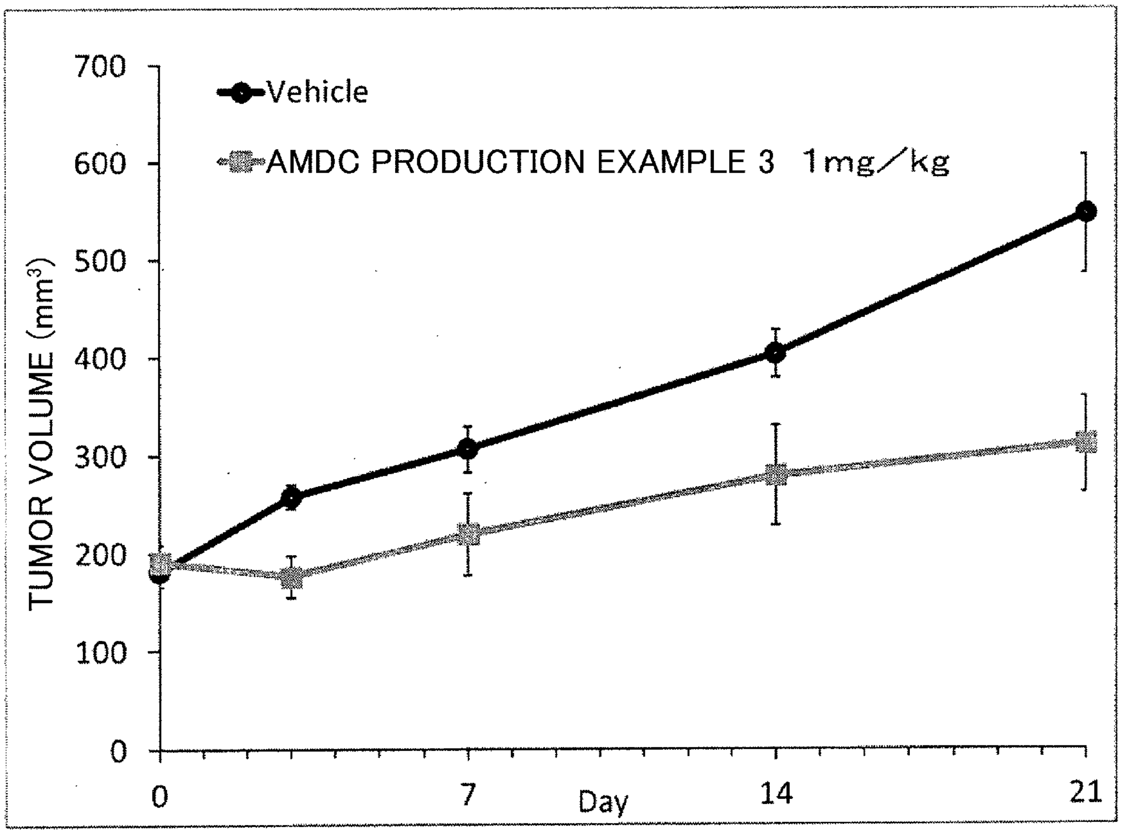
700
600
500
400
300
200
100
0
TUMOR VOLUME (mm³)
Vehicle
AMDC PRODUCTION EXAMPLE 3 1mg/kg
0
7
Day
14
21

Fig. 3

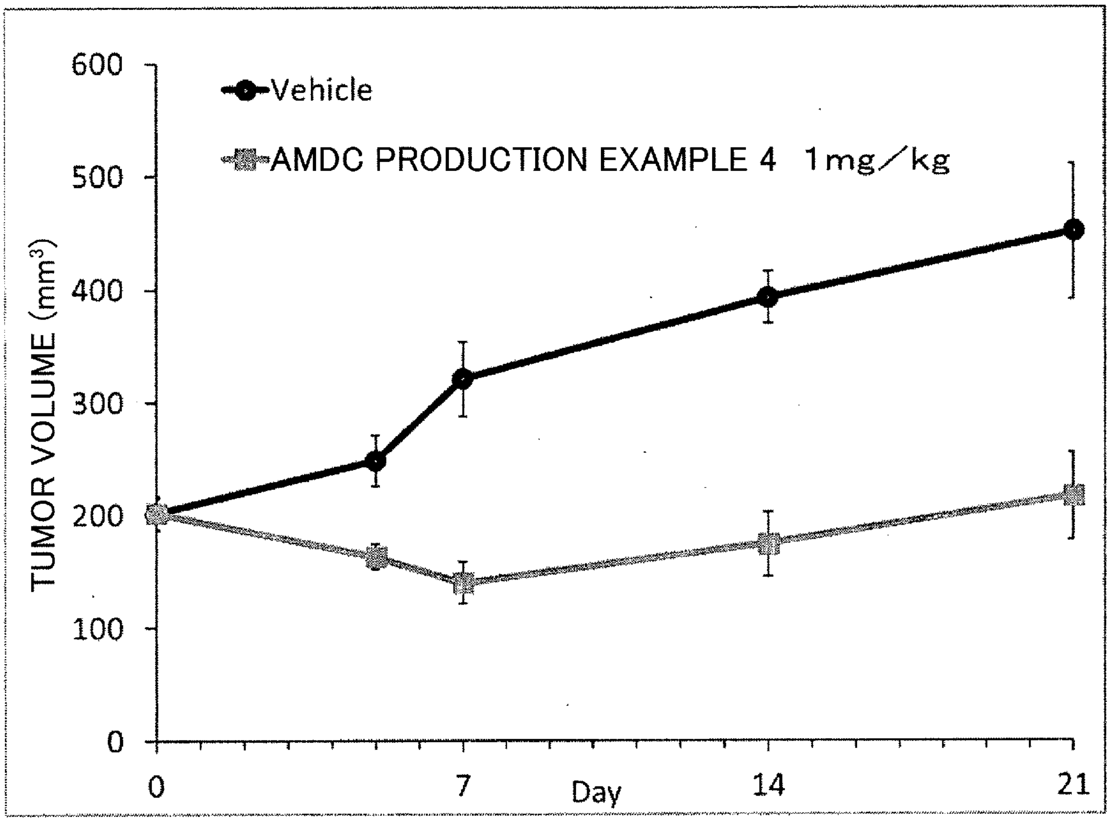
600
500
400
300
200
100
0
TUMOR VOLUME (mm3)
Vehicle
AMDC PRODUCTION EXAMPLE 4 1mg／kg
0
7
Day
14
21

Fig. 4

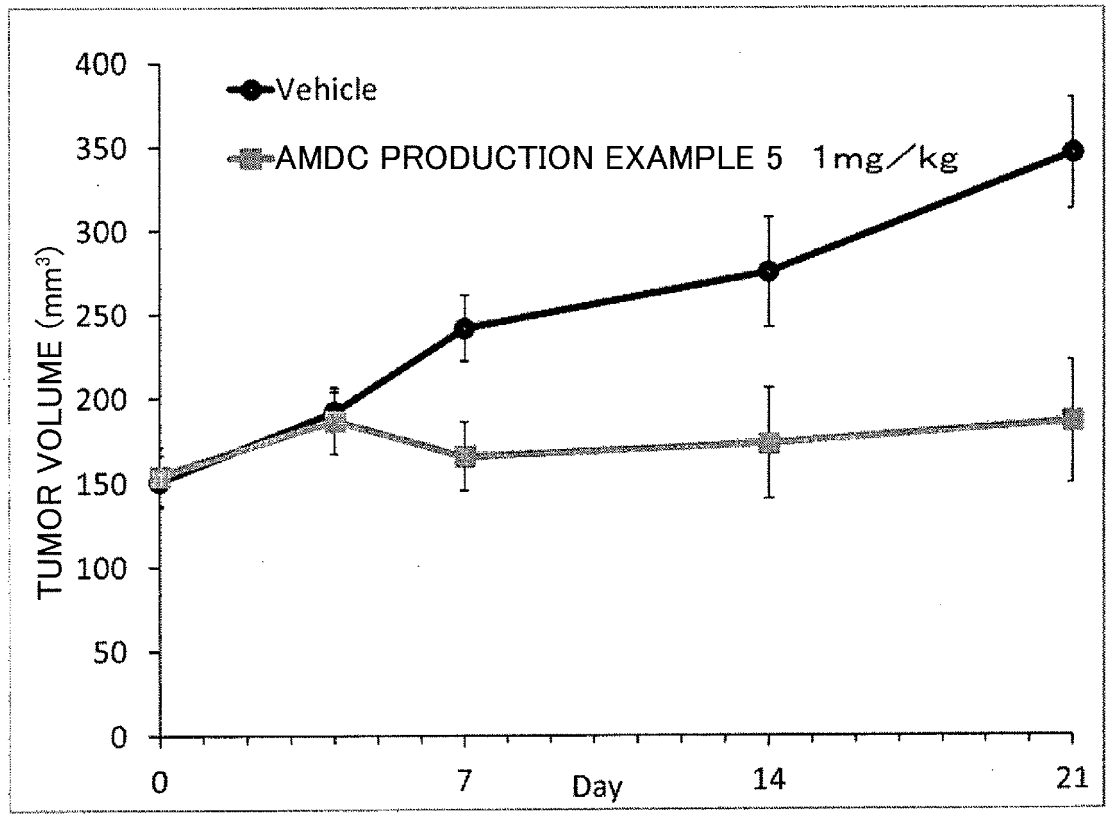
Vehicle
AMDC PRODUCTION EXAMPLE 5 1mg/kg
TUMOR VOLUME (mm3)
400
350
300
250
200
150
100
50
0
0
7
Day
14
21

Fig. 5

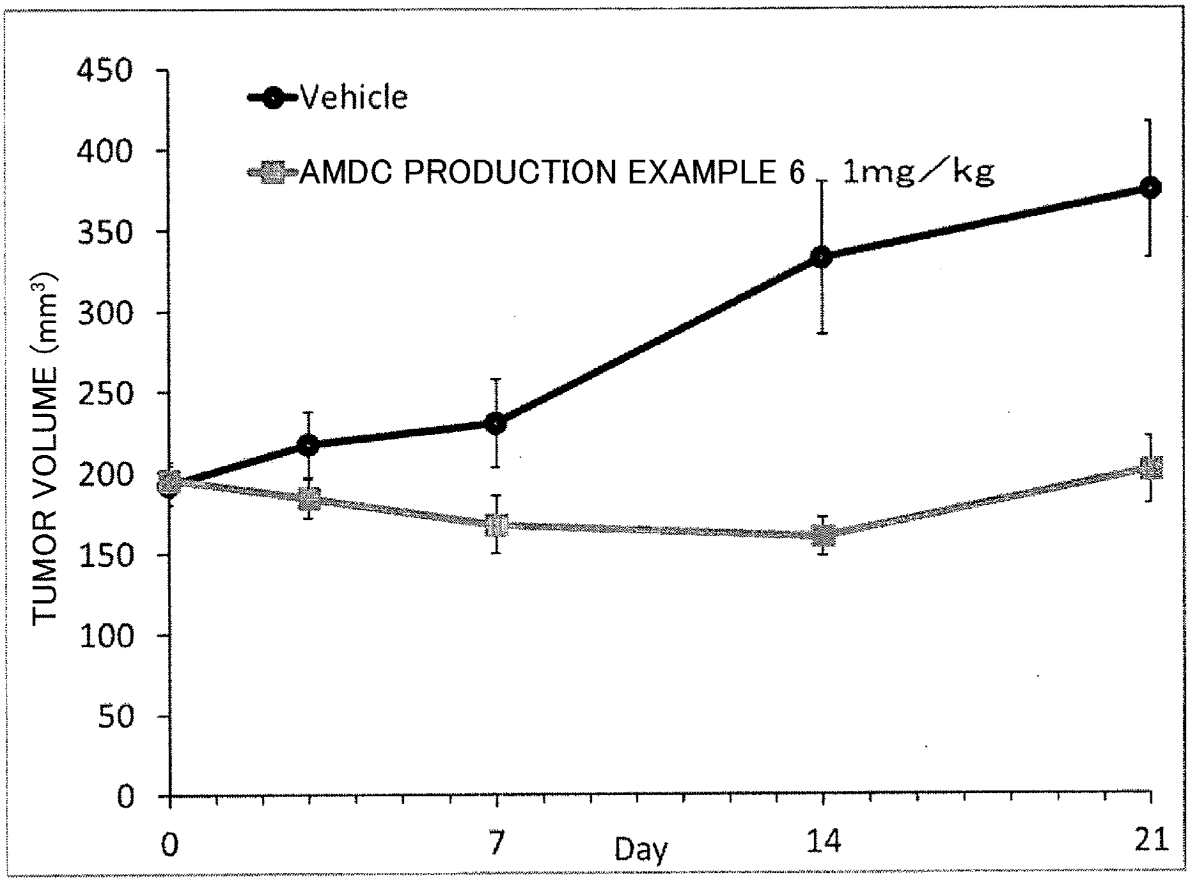

450
400
350
300
250
200
150
100
50
0
TUMOR VOLUME (mm3)
Vehicle
AMDC PRODUCTION EXAMPLE 6 1mg/kg
0
7
Day
14
21

Fig. 6

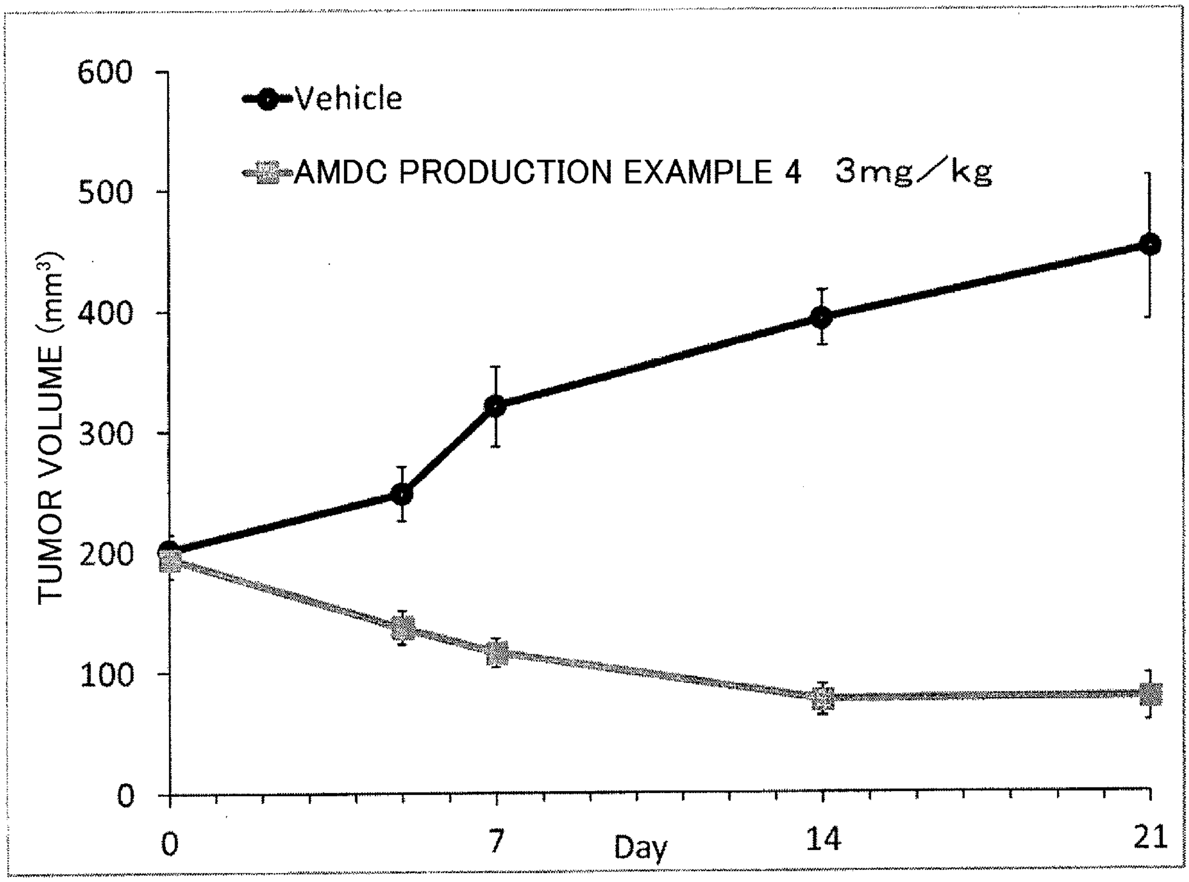
600
500
400
300
200
100
0
TUMOR VOLUME (mm3)
Vehicle
AMDC PRODUCTION EXAMPLE 4 3mg／kg
0
7
Day
14
21

Fig. 7

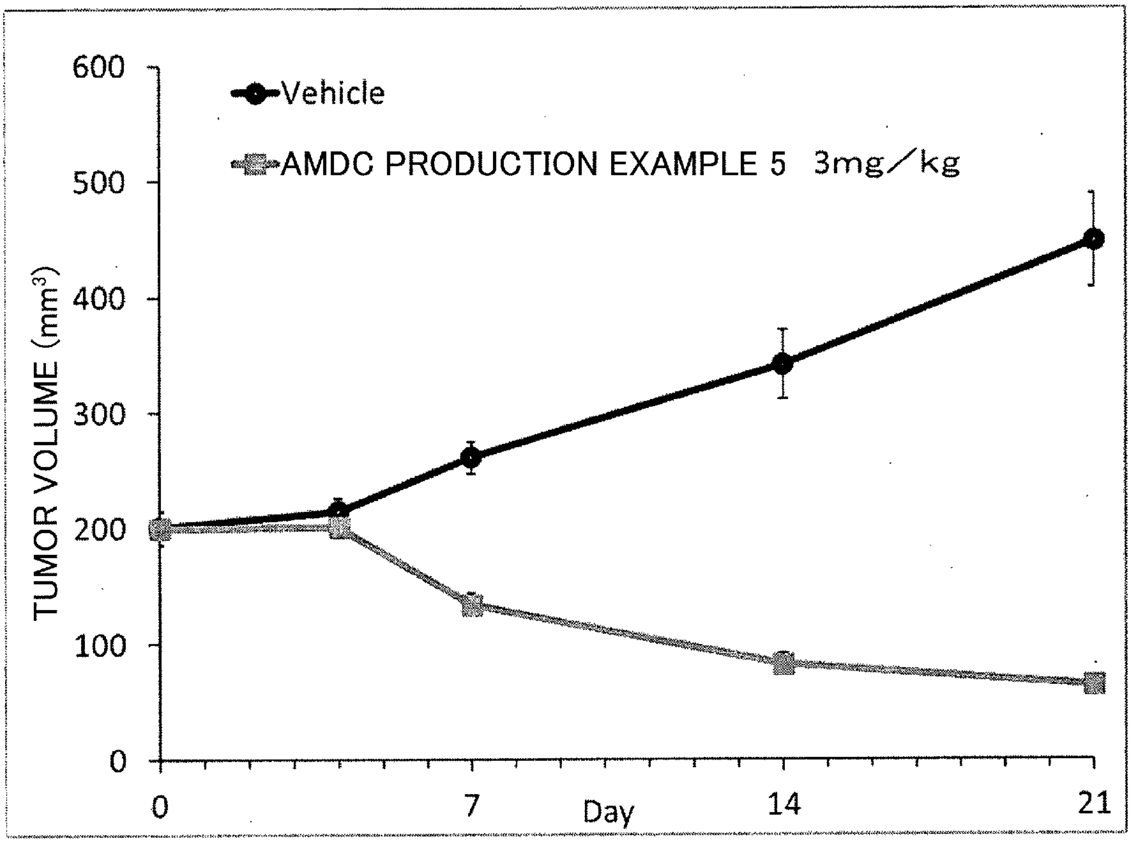
600
500
400
300
200
100
0
TUMOR VOLUME (mm³)
Vehicle
AMDC PRODUCTION EXAMPLE 5 3mg/kg
0
7
Day
14
21

Fig. 8

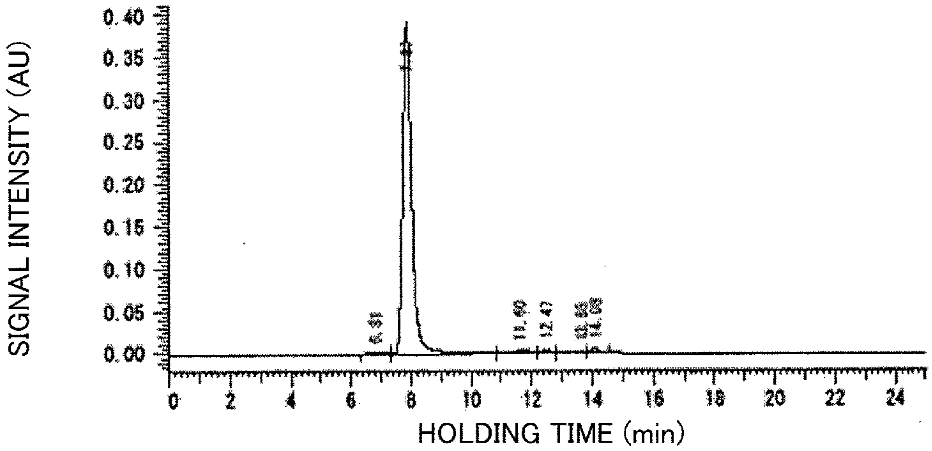


| NO | RT | AREA | CONCENTRATION 1 | BC |
|---|---|---|---|---|
| 1 | 6.81 | 17852 | 0.424 | BV |
| 2 | 7.87 | 4038308 | 95.945 | VV |
| 3 | 11.60 | 52238 | 1.241 | TBV |
| 4 | 12.47 | 28000 | 0.665 | TVB |
| 5 | 13.65 | 27383 | 0.651 | TBV |
| 6 | 14.08 | 45214 | 1.074 | TVB |
| | | 4208995 | 100.000 | |

**INTERNATIONAL SEARCH REPORT**

International application No. **PCT/JP2023/021638**

**A. CLASSIFICATION OF SUBJECT MATTER**

***A61K 47/68***(2017.01)i; ***A61K 31/337***(2006.01)i; ***A61K 31/357***(2006.01)i; ***A61K 31/407***(2006.01)i; ***A61K 31/4375***(2006.01)i; ***A61K 31/4745***(2006.01)i; ***A61K 31/475***(2006.01)i; ***A61K 31/513***(2006.01)i; ***A61K 31/537***(2006.01)i; ***A61K 31/5513***(2006.01)i; ***A61K 31/5517***(2006.01)i; ***A61K 31/675***(2006.01)i; ***A61K 31/7034***(2006.01)i; ***A61K 31/7036***(2006.01)i; ***A61K 31/706***(2006.01)i; ***A61K 45/00***(2006.01)i; ***A61P 35/00***(2006.01)i

FI: A61K47/68; A61K31/337; A61K31/357; A61K31/407; A61K31/4375; A61K31/4745; A61K31/475; A61K31/513; A61K31/537; A61K31/5513; A61K31/5517; A61K31/675; A61K31/7034; A61K31/7036; A61K31/706; A61K45/00; A61P35/00

According to International Patent Classification (IPC) or to both national classification and IPC

**B. FIELDS SEARCHED**

Minimum documentation searched (classification system followed by classification symbols)

A61K47/68; A61K31/337; A61K31/357; A61K31/407; A61K31/4375; A61K31/4745; A61K31/475; A61K31/513; A61K31/537; A61K31/5513; A61K31/5517; A61K31/675; A61K31/7034; A61K31/7036; A61K31/706; A61K45/00; A61P35/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | XIAO, Dian et al. A bifunctional molecule-based strategy for the development of theranostic antibody-drug conjugate. Theranostics, 2021. 11(6), 2550-2563<br>abstract, fig. 1-2, p. 2552, right column, lines 15-32 | 1-18, 20-24, 26-34, 36-41 |
| A | abstract, fig. 1-2, p. 2552, right column, lines 15-32 | 19, 25, 35 |
| Y | SU, Zheng et al. Antibody-drug conjugates: Recent advances in linker chemistry. Acta Pharmaceutica Sinica B. 2021, 11(12), 3889-3907<br>fig. 7 | 1-18, 20-24, 26-34, 36-41 |
| A | fig. 7 | 19, 25, 35 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 August 2023** | **29 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

## INTERNATIONAL SEARCH REPORT

International application No.
**PCT/JP2023/021638**

### C. DOCUMENTS CONSIDERED TO BE RELEVANT

| Category* | Citation of document, with indication, where appropriate, of the relevant passages | Relevant to claim No. |
|---|---|---|
| Y<br><br>A | LIU, Lianqi et al. Synthesis and evaluation of highly releasable and structurally stable antibody-SN-38-conjugates. DRUG DELIVERY. 2021, 28(1), 2603-2617<br>abstract, fig. 1, scheme 1, p. 2605, left column, lines 1-3<br>abstract, fig. 1, scheme 1, p. 2605, left column, lines 1-3 | 1-18, 20-24, 26-34, 36-41<br><br>19, 25, 35 |
| Y<br><br>A | LAU, Uland Y. et al. Lactone Stabilization is Not a Necessary Feature for Antibody Conjugates of Camptothecins. Mol. Pharmaceutics. 2018, 15(9), 4063-4072<br>abstract, p. 4065, right column, lines 1-29, p. 4069, lines 11-15<br>abstract, p. 4065, right column, lines 1-29, p. 4069, lines 11-15 | 1-18, 20-24, 26-34, 36-41<br><br>19, 25, 35 |
| Y<br><br>A | WO 2014/061277 A1 (DAIICHI SANKYO COMPANY, LIMITED) 02 April 2014 (2014-04-02)<br>claims, examples, in particular, examples 17, 32-35<br>claims, examples, in particular, examples 17, 32-35 | 1-18, 20-24, 26-34, 36-41<br><br>19, 25, 35 |
| Y<br><br>A | 眞鍋史乃, 抗体—薬物複合体開発の発展と現状, Drug Delivery System. 2019, 34(1), 10-21<br>fig. 13<br>fig. 13, (MANABE, Shino. Development and Current Status of Antibody-drug conjugate (ADC).) | 1-18, 20-24, 26-34, 36-41<br><br>19, 25, 35 |
| Y<br><br>A | KERN, C. J. et al. Novel phosphate modified Cathepsin B linkers: Improving aqueous solubility and enhancing payload scope of ADCs. Bioconjugate Chem. 2016, 27(9), 2081-2088<br>fig. 1-4<br>fig. 1-4 | 1-18, 20-24, 26-34, 36-41<br><br>19, 25, 35 |
| Y<br><br>A | WO 2020/236817 A2 (NOVARTIS AG) 26 November 2020 (2020-11-26)<br>claims, examples<br>claims, examples | 1-18, 20-24, 26-34, 36-41<br><br>19, 25, 35 |
| Y<br><br>A | WO 2022/115451 A1 (NOVARTIS AG) 02 June 2022 (2022-06-02)<br>for example, claims, paragraphs [0014], [0074], [0098], table A<br>for example, claims, paragraphs [0014], [0074], [0098], table A | 1-18, 20-24, 26-34, 36-41<br><br>19, 25, 35 |
| Y<br><br>A | JP 2013-136590 A (GENENTECH INC) 11 July 2013 (2013-07-11)<br>paragraph [0318]<br>paragraph [0318] | 1-18, 20-24, 26-34, 36-41<br><br>19, 25, 35 |

Form PCT/ISA/210 (second sheet) (January 2015)

**INTERNATIONAL SEARCH REPORT**
**Information on patent family members**

International application No.
**PCT/JP2023/021638**

| Patent document cited in search report | Publication date (day/month/year) | Patent family member(s) | Publication date (day/month/year) |
|---|---|---|---|
| WO 2014/061277 A1 | 02 April 2014 | US 2015/0352224 A1<br>claims, examples<br>EP 2910573 A1 | |
| WO 2020/236817 A2 | 26 November 2020 | US 2023/81720 A1<br>claims, examples | |
| WO 2022/115451 A1 | 02 June 2022 | (Family: none) | |
| JP 2013-136590 A | 11 July 2013 | WO 2009/012268 A1<br>paragraph [0484]<br>US 2009/28856 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003043583 A
- JP 2016196484 A
- WO 2022022649 A
- CN 113941007
- WO 2021148500 A
- WO 2021209007 A
- WO 2021212638 A
- WO 2022199429 A
- WO 9954342 A
- WO 0061739 A
- WO 0231140 A
- WO 2019195665 A
- JP 6186045 B

### Non-patent literature cited in the description

- *Bioconjugate Chem.*, 2010, vol. 21, 5-13
- *Current Opin. Chem. Biol.*, 2010, vol. 14, 529-537
- *Expert Opin. Biol. Ther.*, 2004, vol. 4, 1445-1452
- *Biotechnol Lett.*, 2016, vol. 38, 1655-1664
- *Chem Pharm Bull.*, 2019, vol. 67, 173-185
- *N Engl J Med.*, 2012, vol. 367, 1783-1791
- *Expert Opin. Biol. Ther.*, 2020, vol. 8, 871-875
- *Drugs Today*, 2021, vol. 57, 653-663
- *Drugs*, 2021, vol. 81, 2141-2147
- *Drugs*, 2021, vol. 81, 1229-1233
- *Cancer Res.*, 2019, vol. 25, 5441-5448
- *Molecules*, 2020, vol. 25, 4764
- *Methods Mol Biol.*, 2020, vol. 2078, 1-22
- *Nat. Rev. Drug Discov.*, 2017, vol. 16, 315
- *Nat.Biotecnol.*, 2015, vol. 33, 733-736
- *N Engl J Med.*, 2018, vol. 378, 331-344
- *Bioconjugate Chem.*, 2016, vol. 27, 1030-1039
- *Org. Biomol. Chem.*, 2016, vol. 14, 6165-6178
- *Nat. Commun.*, 2015, vol. 6, 6645
- *Angew. Chem. Int. Ed.*, 2017, vol. 56, 733-737
- *Bioorg. Med. Chem. Lett.*, 2018, vol. 28, 3617-3621
- *Brief*, 2018, vol. 21, 2208-2220
- *Antib. Ther.*, October 2019, vol. 2 (4), 71-78
- *Nat. Commun.*, 2018, vol. 9, 2512
- **P. G. M. WUTS**. Greene's Protective Groups in Organic Synthesis. JohnWiley & Sons Inc., 2014
- **HANS KAMERLING**. *Comprehensive Glycoscience From Chemistry to Systems Biology*, 2007
- *Angew Chem Int Ed Engl.*, 02 March 2020, vol. 59 (10), 4176-41
- *Synthetic Organic Chemistry*, 1984, vol. 42 (4)
- *Drug Delivery System*, 2019, 34-1
- *YAKUGAKU ZASSHI*, 2019, vol. 139 (2)
- *Angew. Chem. Int. Ed.*, 2019, vol. 58, 11631-11636
- *Chem. Rev.*, 2015, vol. 115, 2174-2195
- *Analytical Sciences*, January 2018, vol. 35, 5-27
- **HERMANSON, G. T.** Bioconjugate Techniques. Academic Press, 1996, 56-136, 456-493
- *Protein Science*, 1995, vol. 4, 2411-2423
- *CHEMICAL ABSTRACTS*, 646502-53-6
- *CHEMICAL ABSTRACTS*, 2130869-18-8
- *Clin Cancer Res*, 15 October 2016, vol. 22 (20)
- *Breast Cancer (Auckl).*, 20 May 2010, vol. 4, 35-41